# NEW EUROPEAN PATENT SPECIFICATION

(11) **EP 1 836 174 B2**
(45) Date of publication and mention of the opposition decision: **02.10.2019**
(45) Mention of the grant of the patent: 27.02.2013
(21) Application number: 05818381.5
(22) Date of filing: 03.10.2005
(51) Int. Cl.: C07D 239/84

(54) **ARYL NITROGEN-CONTAINING BICYCLIC COMPOUNDS AND THEIR USE AS KINASE INHIBITORS**
ARYLNITROGENHALTIGE BIZYKLISCHE VERBINDUNGEN UND IHRE VERWENDUNG ALS KINASEINHIBITOREN
COMPOSES BICYCLIQUES QUI CONTIENNENT UN AZOTE ARYLIQUE ET LEUR UTILISATION COMME INHIBITEURS DES KINASES

(30) Priority: 01.10.2004 US 615535 P; 30.09.2005 US 240590
(43) Date of publication of application: 26.09.2007
(73) Proprietor: AMGEN INC., Thousand Oaks, CA 91320-1799 (US)
(72) Inventor: PATEL, Vinod F., Acton, Massachusetts 01720 (US); KIM, Joseph L., Wayland, Massachusetts 01778 (US); GEUNS-MEYER, Stephanie D., Medford, Massachusetts 02155 (US); CHAFFEE, Stuart C., Philadelphia, Pennsylvania 19103 (US); CEE, Victor J., Mansfield, Massachusetts 02048 (US); HODOUS, Brian L., Cambridge, Massachusetts 02139 (US); BELLON, Steven, Wellesley, Massachusetts 02139 (US); HARMANGE, Jean-christophe, Andover, Massachusetts 01810 (US); OLIVIERI, Philip R., Medford, Massachusetts 02155 (US); THAMAN, Maya C., Ventura, California 93001 (US); DIMAURO, Erin F., Cambridge, Massachusetts 02139 (US); BUCHANAN, John L., Brookline, Massachusetts 02446 (US); MCGOWAN, David C., B-1150 Pierre (BE); ALBRECHT, Brian K., Cambridge, Massachusetts 02139 (US); DEAK, Holly L., Brookline, Massachusetts 02446 (US); BEMIS, Jean E., Arlington, Massachusetts 02476 (US); WHITE, Ryan, Somerville, Massachusetts 02144 (US); MARTIN, Matthew W., Cambridge, Massachusetts 02140 (US); HABGOOD, Gregory J., Merrimac, Massachusetts 01860 (US); MASSE, Craig E., Cambridge, Massachusetts 02139 (US); BUCKNER, William H., Arlington, Massachusetts 02476 (US); TEMPEST, Paul A., Brookline, Massachusetts 02139 (US); HERBERICH, Bradley J., Newbury Park, California 91320 (US); GRACEFFA, Russell, Hampton, New Hampshire 03842 (US); ZHANG, Dawei, Thousand Oaks, California 91362 (US); XU, Shimin, Santa Barbara, California 93117 (US); SHAM, Kelvin, Thousand Oaks, California 91362 (US); RZASA, Robert M., Ventura, California 93003 (US); FALSEY, James Richard, Moorpark, California 93021 (US); CHAKRABARTI, Partha P., Simi Valley, California 93065 (US); CAO, Guo-giang, Thousand Oaks, California 91320 (US); TOMLINSON, Susan Ann, Boston, Massachusetts 02118 (US); PETTUS, Liping H., Thousand Oaks, California 91360 (US); SMITH, Adrian Leonard, Simi Valey, California 93065 (US); PARAS, Nick A., Santa Monica, California 90402 (US); LIU, Gang, Oak Park, California 91377 (US); DEMORIN, Frenel F., Thousand Oaks, California 91360 (US); TASKER, Andrew, Simi Valey, California 93065 (US); REED, Anthony, Oxnard, CA 93035 (US)
(74) Representative: Grünecker Patent- und Rechtsanwälte PartG mbB
(86) International application number: PCT/US2005/035873
(87) International publication number: WO 2006/039718

(56) References cited:
- EP-A- 0 875 506
- WO-A-01/19828
- WO-A-01/21594
- WO-A-01/53268
- WO-A-02/16351
- WO-A-99/09016
- WO-A-2004/033435
- WO-A-2004/037814
- WO-A-2005/037285
- WO-A1-2006/015859
- WO-A1-2006/118256
- WO-A2-96/15128
- WO-A2-2005/034869
- US-A- 5 874 579
- US-A1- 2001 014 679
- US-A1- 2002 147 214
- E.A. COATS ET. AL.: "Comparative QSAR of antibacterial dihydrofolate reductase inhibitors, pages 71-85" 1984, PROUS , BARCELONA, ES , XP009020318 Table 1, compounds 3,4; Table 2, compounds 1,17-19, 36-45,47,48, 51-53,57-59,61-68,80,88-90.
- P. VICTORY ET. AL.: "Synthesis of 4-Amino-8-cyanoquinazolines from enones and enals." HETEROCYCLES, vol. 36, no. 10, 1993, pages 2273-2280, XP009065422
- H. MATTER ET. AL.: "Structural Requirements for Inhibition of the Neuronal Nitric Oxide Synthase (NOS-1): 3D QSAR Analysis of 4-Oxo- and 4-Amino-Pteridine Based Inhibitors." JOURNAL OF MEDICINAL CHEMISTRY, vol. 45, 2002, pages 2923-2941, XP002377951
- L. G. FRÖHLICH ET. AL.: "Inhibition of Neuronal Nitric Oxide Synthase by 4-Amino Pteridine Derivatives: Structure Activity Relationship of Antagonists of (6R)-5,6,7,8-Tetrahydrobiopterin Cofactor." JOURNAL OF MEDICINAL CHEMISTRY, vol. 42, 1999, pages 4108-4121, XP002377952
- A. GAZIT ET. AL.: "Tyrphostins. 5. Potent Inhibitors of Platelet Derived Growth Factor Receptor Tyrosine Kinase: Structure Activity Relationships in Quinoxalines, Quinolines and Indole Tyrphostins" JOURNAL OF MEDICINAL CHEMISTRY, vol. 39, 1996, pages 2170-2177, XP002377953
- B.S. HURLBERT ET. AL.: "Studies of Condensed Pyrimidine Sytems. XXIV. The Condensation of 2,4,6-Triaminopyrimidine with Malondialdehyde Derivatives." JOURNAL OF MEDICINAL CHEMISTRY, vol. 11, 1968, pages 708-710, XP002377954
- E.C. TAYLOR ET. AL.: "Pteridines. XXIX. An Unequivocal Route to 2,4-Diamino-6-substituted Pteridines." JOURNAL OF THE AMERICAN CHEMICAL SOCIETY, vol. 95, 1973, pages 6413-6418, XP002377955
- S. TAGHAVI-MOGHADAM ET. AL.: "A New, General and Regioselective Method for the Synthesis of 2,6-Disubstituted 4-Aminopteridines." TETRAHEDRON LETTERS, vol. 38, 1997, pages 6835-6836, XP002377956
- C.B. STORM ET. AL.: "Preparation of 6-Substituted Pterins via the Isay Reaction." JOURNAL OF ORGANIC CHEMISTRY, vol. 36, no. 25, 1971, pages 3925-3927, XP002377957
- L. N. PRIDGEN ET. AL.: "Versatile and Efficient Synthesis of Aryl-1,2,3,4-tetrahydroisoquinolines: Nickel(II) Phosphine Ligand Cytalysed Coupling of Arylmagnesium Halides to Haloisoquinolines." JOURNAL OF HETEROCYCLIC CHEMISTRY, vol. 17, 1980, pages 1289-1291, XP002377958
- SCHROEDER M C; ET AL: "SOLUBLE 2-SUBSTITUTED AMINOPYRIDOÄ2,3-DÜPYRIMIDIN-7-YL UREAS. STRUCTURE-ACTIVITY RELATIONSHIPS AGAINST SELECTED TYROSINE KINASES AND EXPLORATION OF IN VITRO AND IN VIVO ANTICANCER ACTIVITY", JOURNAL OF MEDICINAL CHEMISTRY, vol. 44, no. 12, 7 June 2001 (2001-06-07), pages 1915-1926, XP001152609,
- MOL, C. et al: Current Opinion in Drug Discovery & Development, vol. 7, no. 5, 2004, pages 639-648,

## Description

The invention generally relates to the field if pharmaceutical agents and, more specifically, to compounds, intermediates, methods for making the compounds and intermediates, compositions, uses for modulating protein kinases and for treating protein kinase-mediated diseases.

Protein kinases represent a large family of enzymes, which catalyze the phosphorylation of target protein substrates. The phosphorylation is usually a transfer reaction of a phosphate group from ATP to the protein substrate. Common points of attachment for the phosphate group to the protein substrate include, for example, a tyrosine, serine or threonine residue. For example, protein tyrosine kinases (PTKs) are enzymes, which catalyze the phosphorylation of specific tyrosine residues in cellular proteins. Examples of kinases in the protein kinase family include, without limitation, ab1, Akt, bcr-ab1, Blk, Brk, Btk, c-kit, c-Met, c-src, c-fms, CDK1, CDK2, CDK3, CDK4, CDKS, CDK6, CDK7, CDK8, CDK9, CDK10, cRaf1, CSF1R, CSK, EGFR, ErbB2, ErbB3, ErbB4, Erk, Fak, fes, FGFR1, FGFR2, FGFR3, FGFR4, FGFR5, Fgr, flt-1, Fps, Frk, Fyn, Hck, IGF-1R, INS-R, Jak, KDR, Lck, Lyn, MEK, p38, PDGFR, PIK, PKC, PYK2, ros, tie, tie2, TRK, Yes, and Zap70. Due to their activity in numerous cellular processes, protein kinases have emerged as important therapeutic targets.

Protein kinases play a central role in the regulation and maintenance of a wide variety of cellular processes and cellular function. For example, kinase activity acts as molecular switches regulating cell proliferation, activation, and/or differentiation. Uncontrolled or excessive kinase activity has been observed in many disease states including benign and malignant proliferation disorders as well as diseases resulting from inappropriate activation of the immune system (autoimmune disorders), allograff rejection, and graft vs host disease. In addition, endothelial cell specific receptor PTKs, such as VEGF-2 and Tie-2, mediate the angiogenic process and are involved in supporting the progression of cancers and other diseases involving uncontrolled vascularization.

Angiogenesis is the process of developing new blood vessels, particularly capillaries, from pre-existing vasculature and is an essential component of embryogenesis, normal physiological growth, repair, and tumor expansion. Angiogenesis remodels small vessels into larger conduit vessels, a physiologically important aspect of vascular growth in adult tissues. Vascular growth is required for beneficial processes such as tissue repair, wound healing, recovery from tissue ischemia and menstrual cycling.

Certain diseases and/or pathological conditions develop as a result of, or are known to be associated with, the regulation and/or deregulation of angiogenesis. For example, ocular neovascularisation such as retinopathies (including diabetic retinopathy), age-related macular degeneration, psoriasis, hemangioblastoma, hemangioma, and arteriosclerosis have been found to be caused, in part, due the loss of regulation and/or maintenance of vascular growth. Inflammatory diseases such as a rheumatoid or rheumatic inflammatory disease, and especially arthritis (including rheumatoid arthritis) where new capillary blood vessels invade the joint and destroy cartilage, have been associated with angiogenesis. In addition, chronic inflammatory disorders such as chronic asthma, arterial or post-transplantational atherosclerosis, endometriosis, and neoplastic diseases including so-called solid tumors and liquid tumors (for example, leukemias), have been found to be linked to the regulation and control of angiogenesis.

The involvement of angiogenesis in major diseases has led to the identification and development of various targets for inhibiting angiogenesis. These targets relate to various receptors, enzymes, and other proteins in the angiogenic process or cascade of events leading to angiogenesis, such as, for example, activation of endothelial cells by an angiogenic signal, synthesis and release of degradative enzymes, endothelial cell migration, proliferation of endothelial cells, and formation of capillary tubules.

One target identified in the cascade of events leading to angiogenesis is the Tie receptor family. The Tie-1 and Tie-2 receptors are single-transmembrane, tyrosine kinase receptors (Tie stands for tyrosine kinase receptors with immunoglobulin and EGF homology domains). Tie-2 is an endothelial cell specific receptor tyrosine kinase, which is involved in angiogenic processes, such as vessel branching, sprouting, remodeling, maturation and stability. Tie-2 is the first mammalian receptor for which both agonist ligand(s) (for example, Angiopoietin-1 ("Angl") which binds to and stimulates phosphorylation and signal transduction of Tie-2), and context dependent agonist/antagonist ligand(s) (for example, Angiopoietin-2 ("Ang2")) have been identified. Knock out and transgenic manipulation of the expression of Tie-2 and its ligands indicates that tight spacial and temporal control of Tie-2 signaling is important for the proper development of new vascularization.

Biological models suggest that the stimulation of Tie-2 by the Ang1 ligand is directly involved in the branching, sprouting and outgrowth of new vessels, and recruitment and interaction of periendothelial support cells important in maintaining vessel integrity and inducing quiescence. The absence of Ang1 stimulation of Tie-2 or the inhibition of Tie-2 autophosphorylation by Ang2, which is produced at high levels at sites of vascular regression, may cause a loss in vascular structure and matrix contacts resulting in endothelial death, especially in the absence of growth/survival stimuli.

Recently, upregulation of Tie-2 expression has been found in the vascular synovial pannus of arthritic joints of humans, consistent with the role in inappropriate neovasculariation. This finding suggests that Tie-2 plays a role in the progression of rheumatoid arthritis. Point mutations producing constitutively activated forms of Tie-2 have been identified in association with human venous malformation disorders. Tie-2 inhibitors would, therefore, be useful in treating such disorders, as well as in other instances of improper neovasacularization. However, with the recent recognition of Ang3 and Ang4 as additional Tie-2 binding ligands, targeting a Tie-2 ligand-receptor interaction as an anti-angiogenic therapeutic approach is less favorable. Accordingly, a Tie-2 receptor kinase inhibition approach has become the strategy of choice.

Another angiogenic factor responsible for regulating the growth and differentiation of the vascular system and its components, both during embryonic development and normal growth, as well as in a wide number of pathological anomalies and diseases, is Vascular Endothelial Growth Factor ("VEGF"; originally termed "Vascular Permeability Factor", VPF), along with its cellular receptors (see G. Breier et al., Trends in Cell Biology, 6:454-456 (1996)).

VEGF is a dimeric, disulfide-linked 46-kDa glycoprotein related to "Platelet-Derived Growth Factor" (PDGF). It is produced by normal cell lines and tumor cell lines; is an endothelial cell-specific mitogen; shows angiogenic activity in *in vivo* test systems (e.g. rabbit cornea); is chemotactic for endothelial cells and monocytes; and induces plasminogen activators in endothelial cells, which are involved in the proteolytic degradation of extracellular matrix during the formation of capillaries. A number of isoforms of VEGF are known, which show comparable biological activity, but differ in the type of cells that secrete them and in their heparin-binding capacity. In addition, there are other members of the VEGF family, such as "Placenta Growth Factor"(PlGF) and VEGF-C.

VEGF receptors (VEGFR) are also transmembrane receptor tyrosine kinases. They are characterized by an extracellular domain with seven immunoglobulin-like domains and an intracellular tyrosine kinase domain. Various types of VEGF receptor are known, e.g. VEGFR-1 (also known as flt-1), VEGFR-2 (also known as KDR), and VEGFR-3.

A large number of human tumors, especially gliomas and carcinomas, express high levels of VEGF and its receptors. This has led to the belief that the VEGF released by tumor cells stimulates the growth of blood capillaries and the proliferation of tumor endothelium in a paracrine manner, and through the improved blood supply, accelerate tumor growth. Increased VEGF expression could explain the occurrence of cerebral edema in patients with glioma. Direct evidence of the role of VEGF as a tumor angiogenesis factor *in vivo* has been shown in studies in which VEGF expression or VEGF activity was inhibited. This was achieved with anti-VEGF antibodies, with dominant-negative VEGFR-2 mutants, which inhibited signal transduction, and with antisense-VEGF RNA techniques. All approaches led to a reduction in the growth of glioma cell lines or other tumor cell lines *in vivo* as a result of inhibited tumor angiogenesis.

Inflammatory cytokines stimulate VEGF production. Hypoxia results in a marked upregulation of VEGF in numerous tissues, hence situations involving infarct, occlusion, ischemia, anemia, or circulatory impairment typically invoke VEGF/VPF-mediated responses. Vascular hyperpermeability, associated edema, altered transendothelial exchange and macromolecular extravasation, which is often accompanied by diapedesis, can result in excessive matrix deposition, aberrant stromal proliferation, fibrosis, etc. Hence, VEGF-mediated hyperpermeability can significantly contribute to disorders with these etiologic features. As such, the regulation of angiogenesis via the VEGF receptor activity has become an important therapeutic target.

Angiogenesis is regarded as an absolute prerequisite for tumors that grow beyond a diameter of about 1-2 mm. Up to this size, oxygen and nutrients may be supplied to the tumor cells by diffusion. Every tumor, regardless of its origin and its cause, is thus dependent on angiogenesis for its growth after it has reached a certain size.

Three principal mechanisms play an important part in the activity of angiogenesis inhibitors against tumors: 1) Inhibition of the growth of vessels, especially capillaries, into vascular resting tumors, with the result that there is no net tumor growth owing to the balance that is achieved between cell death and proliferation; 2) Prevention of the migration of tumor cells owing to the absence of blood flow to and from tumors; and 3) Inhibition of endothelial cell proliferation, thus avoiding the paracrine growth-stimulating effect exerted on the surrounding tissue by the endothelial cells which normally line the vessels. See R. Connell and J. Beebe, Exp. Opin. Ther. Patents, 11:77-114 (2001).

The inhibition of vascular growth in this context has also shown beneficial effects in preclinical animal models. For example, inhibition of angiogenesis by blocking vascular endothelial growth factor or its receptor has resulted in inhibition of tumor growth and in retinopathy. Also, the development of pathological pannus tissue in rheumatoid arthritis involves angiogenesis and might be blocked by inhibitors of angiogenesis.

The ability to stimulate vascular growth has potential utility for treatment of ischemia-induced pathologies such as myocardial infarction, coronary artery disease, peripheral vascular disease, and stroke. The sprouting of new vessels and/or the expansion of small vessels in ischemic tissues prevents ischemic tissue death and induces tissue repair. Regulating angiogenesis by inhibiting certain recognized pathways in this process would, therefore, be useful in treating diseases, such as ocular neovascularization, including retinopathy, age-related macular degeneration, psoriasis, hemangioblastoma, hemangioma, arteriosclerosis, inflammatory disease rheumatoid arthritis, chronic inflammatory disorders such as chronic asthma, arterial or post-transplantational atherosclerosis, endometriosis, and neoplastic diseases such as leukemias, otherwise known to be associated with deregulated angiogenesis. Treatment of malaria and related viral diseases may also be mediated by HGF and cMet.

Other receptor tyrosine kinases such as FGFR-1, PDGFR, FLK-1 (Fetal Liver Kinase-1) and c-Met have also been suggested to play a role in angiogenesis. C-met is a unique receptor tyrosine kinase, which comprises, in its native form, a 190 kDa heterodimeric (a disulfide-linked 50 kDa α-chain and a 145 kDa ß-chain) membrane-spanning tyrosine kinase protein (Proc. Natl. Acad. Sci. USA, 84:6379-6383 (1987)). C-Met is mainly expressed in epithelial cells and stimulation of c-Met leads to scattering, angiogenesis, proliferation and metastasis. (See Cytokine and Growth Factor Reviews, 13:41-59 (2002)). The ligand for c-Met is hepatocyte growth factor (also known as scatter factor, HGF and SF). HGF is a heterodimeric protein secreted by cells of mesodermal origin (Nature, 327:239-242 (1987); J. Cell Biol., 111:2097-2108 (1990)).

Various biological activities have been described for HGF through interaction with c-met (Hepatocyte Growth Factor-Scatter Factor (HGF-SF) and the c-Met Receptor, Goldberg and Rosen, eds., Birkhauser Verlag-Basel, 67-79 (1993). HGF induces a spectrum of biological activities in epithelial cells, including mitogenesis, stimulation of cell motility and promotion of matrix invasion (Biochem. Biophys. Res. Comm., 122:1450-1459 (1984); Proc. Natl. Acad. Sci. U.S.A., 88:415-419 (1991)). It stimulates the motility and invasiveness of carcinoma cells. Therefore, HGF is thought to be important in tumor invasion. Goldberg and Rosen, eds., Birkhauser Verlag-Basel, 131-165 (1993).

Recent work on the relationship between inhibition of angiogenesis and the suppression or reversion of tumor progression shows great promise in the treatment of cancer (Nature, 390:404-407 (1997)), especially the use of multiple angiogenesis inhibitors compared to the effect of a single inhibitor. Angiogenesis can be stimulated by HGF, as well as vascular endothelial growth factor (VEGF) and basic fibroblast growth factor (bFGF). Thus, a compound that reduces the effect of HGF would be a useful compound.

Non-receptor tyrosine kinases represent a collection of cellular enzymes, which lack extracellular activity and transmembrane sequences. Examples of non-receptor tyrosine kinases identified include over twenty-four individual kinases, comprising eleven (11) subfamilies (Src, Frk, Btk, Csk, Abl, Zap70, Fes/Fps, Fak, jak, Ack, and LIMK). Src is thought to be the largest family including Src, Lck, Fyn(B), Fyn(T), Lyn, Yes, Hck, Fgr and Blk (for review see: Bolen, JB, and Brugge, JS Annu. Rev. Immunol, 15, 371, 1997). The Src subfamily has been linked to oncogenesis and immune responses (See Bohlen, Oncogene, 8:2025-2031, 1993). These kinases have also been found to be involved in cellular signaling pathways in numerous pathogenic conditions, including cancer, psoriasis, and other hyper-proliferative disorders or hyper-immune responses. Thus, it would be useful to inhibit the activity of non-receptor kinases as well.

Members of the Src-family of tyrosine kinases, in particular, have been shown to be important in cell signal transduction as it relates to inflammatory response and inflammation-related conditions. Gene disruption studies suggest that inhibition of some members of the src family of kinases would potentially lead to a therapeutic benefit. Src(-/-) mice have abnormalities in bone remodeling or osteopetrosis (Soriano, P. Cell 1991, 64, 693), suggesting that inhibition of this kinase might be useful in diseases of bone resorption, such as osteoporosis. Lck(-/-) mice have defects in T cell maturation and activation (Anderson, SJ et al. Adv. Immunol. 1994, 56, 151), suggesting that inhibition of this kinase might be useful in diseases of T cell mediated inflammation. In addition, human patients have been identified with mutations affecting Lck kinase activity (Goldman, FD et al. J. Clin. Invest. 1998, 102, 421). These patients suffer from a severe combined immunodeficiency disorder (SCID).

T cells play a pivotal role in the regulation of immune responses and are important for establishing immunity to pathogens. In addition, T cells are often activated during inflammatory autoimmune diseases, such as rheumatoid arthritis, inflammatory bowel disease, type I diabetes, multiple sclerosis, Sjogren's disease, myasthenia gravis, psoriasis, and lupus. T cell activation is also an important component of transplant rejection, allergic reactions, and asthma.

T cells are activated by specific antigens through the T cell receptor (TCR), which is expressed on the cell surface. This activation triggers a series of intracellular signaling cascades mediated by enzymes expressed within the cell (Kane, LP et al. Current Opinion in Immunol. 12, 242, 2000). These cascades lead to gene regulation events that result in the production of cytokines, like interleukin-2 (IL-2). IL-2 is a necessary cytokine in T cell activation, leading to proliferation and amplification of specific immune responses.

Src-family kinases are also important for signaling downstream of other immune cell receptors. Fyn, like Lck, is involved in TCR signaling in T cells (Appleby, MW et al. Cell, 70, 751, 1992). Hck and Fgr are involved in Fcγ receptor signaling leading to neutrophil activation (Vicentini, L. et al. J. Immunol. 2002, 168, 6446). Lyn and Src also participate in Fcγ receptor signaling leading to release of histamine and other allergic mediators (Turner, H. and Kinet, J-P Nature 1999, 402, B24). These findings suggest that Src family kinase inhibitors may be useful in treating allergic diseases and asthma.

Src kinases are also activated in tumors including sarcoma, melanoma, breast, and colon cancers suggesting that Src kinase inhibitors may be useful anti-cancer agents (Abram, CL and Courtneidge, SA Exp. Cell Res., 254, 1, 2000). Src kinase inhibitors have also been reported to be effective in an animal model of cerebral ischemia (R. Paul et al. Nature Medicine, 7, 222, 2001), suggesting that Src kinase inhibitors may be effective at limiting brain damage following stroke.

Protein kinases, such as p38, are also involved in the regulation of inflammatory cytokines. Interleukin-1 (IL-1) and Tumor Necrosis Factor α (TNF-α) are pro-inflammatory cytokines secreted by a variety of cells, including monocytes and macrophages, in response to many inflammatory stimuli (*e.g.*, lipopolysaccharide - LPS) or external cellular stress (*e.g.*, osmotic shock and peroxide).

Elevated levels of TNF-α over basal levels have been implicated in mediating or exacerbating a number of disease states including rheumatoid arthritis; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; Reiter's syndrome; type II diabetes; bone resorption diseases; graft vs. host reaction; ischemia reperfusion injury; atherosclerosis; brain trauma; multiple sclerosis; cerebral malaria; sepsis; septic shock; toxic shock syndrome; fever, and myalgias due to infection. HIV-1, HIV-2, HIV-3, cytomegalovirus (CMV), influenza, adenovirus, the herpes viruses (including HSV-1, HSV-2), and herpes zoster are also exacerbated by TNF-α.

It has been reported that TNF-α plays a role in head trauma, stroke, and ischemia. For instance, in animal models of head trauma (rat), TNF-α levels increased in the contused hemisphere (Shohami et al., J. Cereb. Blood Flow Metab. 14, 615 (1994)). In a rat model of ischemia wherein the middle cerebral artery was occluded, the levels of TNF-α mRNA of TNF-α increased (Feurstein et al., Neurosci. Lett. 164, 125 (1993)). Administration of TNF-α into the rat cortex has been reported to result in significant neutrophil accumulation in capillaries and adherence in small blood vessels. TNF-α promotes the infiltration of other cytokines (IL-1ß, IL-6) and also chemokines, which promote neutrophil infiltration into the infarct area (Feurstein, Stroke 25, 1481 (1994)).

TNF-α appears to play a role in promoting certain viral life cycles and disease states associated with them. For instance, TNF-α secreted by monocytes induced elevated levels of HIV expression in a chronically infected T cell clone (Clouse et al., J. Immunol. 142, 431 (1989)). Lahdevirta et al., (Am. J. Med. 85, 289 (1988)) discussed the role of TNF-α in the HIV associated states of cachexia and muscle degradation.

TNF-α is upstream in the cytokine cascade of inflammation. As a result, elevated levels of TNF-α may lead to elevated levels of other inflammatory and proinflammatory cytokines, such as IL-1, IL-6, and IL-8. Elevated levels of IL-1 over basal levels have been implicated in mediating or exacerbating a number of disease states including rheumatoid arthritis; osteoarthritis; rheumatoid spondylitis; gouty arthritis; inflammatory bowel disease; adult respiratory distress syndrome (ARDS); psoriasis; Crohn's disease; ulcerative colitis; anaphylaxis; muscle degeneration; cachexia; Reiter's syndrome; type II diabetes; bone resorption diseases; ischemia reperfusion injury; atherosclerosis; brain trauma; multiple sclerosis; sepsis; septic shock; and toxic shock syndrome. Viruses sensitive to TNF-α inhibition, *e.g.*, HIV-1, HIV-2, HIV-3, are also affected by IL-1.

In rheumatoid arthritis models in animals, multiple intra-articular injections of IL-1 have led to an acute and destructive form of arthritis (Chandrasekhar et al., Clinical Immunol Immunopathol. 55, 382 (1990)). In studies using cultured rheumatoid synovial cells, IL-1 is a more potent inducer of stromelysin than is TNF-α (Firestein, Am. J. Pathol. 140, 1309 (1992)). At sites of local injection, neutrophil, lymphocyte, and monocyte emigration has been observed. The emigration is attributed to the induction of chemokines (*e.g.*, IL-8), and the up-regulation of adhesion molecules (Dinarello, Eur. Cytokine Netw. 5, 517-531 (1999)).

IL-1 also appears to play a role in promoting certain viral life cycles. For example, cytokine-induced increase of HIV expression in a chronically infected macrophage line has been associated with a concomitant and selective increase in IL-1 production (Folks et al., J. Immunol. 136, 40 (1986)). Beutler et al. (J. Immunol. 135, 3969 (1985)) discussed the role of IL-1 in cachexia. Baracos et al. (New Eng. J. Med. 308, 553 (1983)) discussed the role of IL-1 in muscle degeneration.

In rheumatoid arthritis, both IL-1 and TNF-α induce synoviocytes and chondrocytes to produce collagenase and neutral proteases, which leads to tissue destruction within the arthritic joints. In a model of arthritis (collagen-induced arthritis (CIA) in rats and mice), intra-articular administration of TNF-α either prior to or after the induction of CIA led to an accelerated onset of arthritis and a more severe course of the disease (Brahn et al., Lymphokine Cytokine Res. 11, 253 (1992); and Cooper, Clin. Exp. Immunol. 898, 244 (1992)).

IL-8 has been implicated in exacerbating and/or causing many disease states in which massive neutrophil infiltration into sites of inflammation or injury (*e.g.*, ischemia) is mediated by the chemotactic nature of IL-8, including, but not limited to, the following: asthma, inflammatory bowel disease, psoriasis, adult respiratory distress syndrome, cardiac and renal reperfusion injury, thrombosis and glomerulonephritis. In addition to the chemotaxis effect on neutrophils, IL-8 also has the ability to activate neutrophils. Thus, reduction in IL-8 levels may lead to diminished neutrophil infiltration.

Many classes of compounds generally modulate or specifically inhibit kinase activity, for use to treat related conditions and disorders. For example, the PCT publication, WO 04/030635, published on April 15, 2004, describes various classes of compounds as vasculostatic agents; PCT publication, WO 04/013141, published on February 12, 2004, describes condensed pyridines and pyrimidines with Tie-2 activity; PCT publication, WO 04/010995, published on February 05, 2004, describes fused heteroaryl derivatives for use as P38 kinase inhibitors in the treatment of I.A. rheumatoid arthritis; PCT publication, WO 04/054585, published on July 1, 2004, describes anilino-substituted heterocyclic compounds for the treatment of abnormal cell growth; U.S. Patent No. 6,395,733, issued May 28, 2002, describes heterocyclic ring-fused pyrimidine derivatives, useful in the treatment of hyperpoliferative diseases; U.S. Patent No. 6,465,449, issued October 15, 2002, describes heteroaromatic bicyclic derivatives useful as anticancer agents; U.S. Patent Publication No. 2003/0018029, published January 23, 2003, describes heterocyclic compounds useful in the treatment of poliferative diseases such as cancer; U.S. Patent Publication No. 2003/0004165, published January 2, 2003, describes polyazanaphthalene compounds and pharmaceutical use thereof; U.S. Publication No. 2003/0139398, published July 24, 2003, describes quinazoline-like compounds for the treatment of integrin-mediated disorders and U.S. Patent No. 6,635,644, issued October 21, 2003, describes fused nitrogen-containing bicyclic ring systems as P38 inhibitors. Other publications, such as US Patent Nos. 6,143,764, 5,523,367 and 5,639,753, and PCT publication Nos. WO 02/32872, WO 02/085908 and WO 00/47212, generally describe quinoline, quinazoline or quinoline-like or quinazoline-like compounds.

WO 96/15128 discloses 6-aryl pyrido[2,3-d]pyrimidines and naphthyridines for inhibiting protein tyrosine kinase mediated cellular proliferation.

The present invention provides new aryl nitrogen-containing bicyclic compounds according to claim 1 useful in treating pathological conditions and/or disease states related to kinase activity. Particularly, the compounds are useful for treating various diseases, such as cancer, inflammation and related disorders and conditions including rheumatoid arthritis. The compounds are useful by virtue of their ability to regulate active angiogenesis, cell-signal transduction and related pathways, for example, through kinase modulation. The compounds provided by the invention, including stereoisomers, tautomers, solvates, or pharmaceutically acceptable salts thereof, are defined by general Formula II according to claim 1.

The invention also discloses procedures for making compounds of Formula II, as well as intermediates useful in such procedures.

The compounds provided by the invention are capable of modulating various kinase activity. For example, in one embodiment, the compounds are capable of modulating one or more kinase enzymes, such as ab1, Akt, bcr-ab1, Blk, Brk, Btk, c-kit, c-Met, c-src, c-fms, CDK1, CDK2, CDK3, CDK4, CDK5, CDK6, CDK7, CDK8, CDK9, CDK10, cRaf1, CSF1R, CSK, EGFR, ErbB2, ErbB3, ErbB4, Erk, Fak, fes, FGFR1, FGFR2, FGFR3, FGFR4, FGFR5, Fgr, flt-1, Fps, Frk, Fyn, Hck, IGF-1R, INS-R, Jak, KDR, Lck, Lyn, MEK, p38, PDGFR, PIK, PKC, PYK2, ros, tie, tie2, TRK, Yes or Zap70. In particular, the compounds are capable of inhibiting various kinase enzymes, including Tie-2, Lck, KDR and P38.

To this end, the invention further provides for the use of these compounds for therapeutic, prophylactic, acute and/or chronic treatment of kinase mediated diseases, such as those described herein. For example, the invention provides the use and preparation of a medicament, containing one or more of the compounds, useful to attenuate, alleviate, or treat disorders through inhibition of Tie-2, Lck, KDR and/or P38. These compounds.are also useful in the treatment of an angiogenesis- or T-cell activation- or proliferation- mediated disease or condition. Accordingly, these compounds are useful in the manufacture of anti-cancer and anti-inflammation medicaments. In one embodiment, the invention provides a pharmaceutical composition comprising an effective dosage amount of a compound of Formula II in association with a least one pharmaceutically acceptable carrier, adjuvant or diluent.

Further, the invention discloses a method of treating kinase mediated disorders, such as treating angiogenesis related or T-cell activation related disorders in a subject inflicted with, or susceptible to, such disorder. The method comprises administering to the subject an effective dosage amount of a compound of Formula II. In other embodiments, the invention provides a compound for use in reducing tumor size, blood flow to and from a tumor, and treating or alleviating various inflammatory responses, including arthritis, organ transplantation or rejection, and many others as described herein.

In one embodiment of the invention, aryl nitrogen-containing bicyclic compounds useful for treating angiogenesis- and/or T-cell proliferation-related disorders, including cancer and inflammation are provided. The compounds, including stereoisomers, tautomers, solvates, or pharmaceutically acceptable salts thereof, are defined by general Formula II: 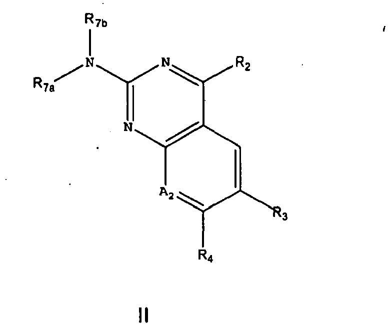 wherein
A² is CR⁵ or N;
R² is H, NO₂, CN, OR^{7b}, SR^{7b}, C₁₋₁₀-alkyl or C₃₋₁₀-cycloalkyl;
R³ is 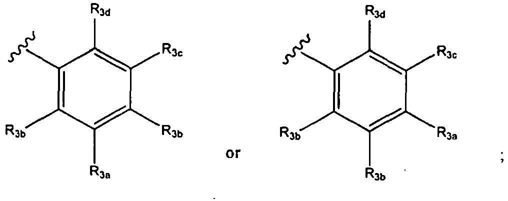 wherein
   R^{3a} is C(O)R¹⁰, COOR¹⁰, C(O)R¹¹, COOR¹¹, C(O)SR¹⁰, C(O)SR¹¹, C(O)NR¹⁰R¹⁰, C(S)NR¹⁰R¹⁰, C(O)NR¹⁰R¹¹, C(S)NR¹⁰R¹¹, NR¹⁰C(O)R¹⁰, NR¹⁰C(S)R¹⁰, NR¹⁰C(O)R¹¹, NR¹⁰C(S)R¹¹, NR¹⁰C(O)NR¹⁰R¹⁰, NR¹⁰C(O)NR¹⁰R¹¹, NR¹⁰C(S)NR¹⁰R¹⁰, NR¹⁰C(S)NR¹⁰R¹¹, NR¹⁰(COOR¹⁰), NR¹⁰(COOR¹¹), S(O)₂R¹¹, S(O)₂NR¹⁰R¹⁰, S(O)₂NR¹⁰R¹¹, NR¹⁰S(O)₂NR¹⁰R¹¹, NR¹⁰S(O)₂R¹⁰ or NR¹⁰S(O)₂R¹¹;
   R^{3b} is H, F, Cl, Br, I, CF₃, CF₂CF₃, OCF₃, OCF₂CF₃, CN, NO₂, NH₂, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl;
   R^{3c} is H, F, Cl, Br, I, CF₃, CF₂CF₃, OCF₃, OCF₂CF₃, CN, NO₂, NH₂, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl;
   R^{3d} is H, F, Cl, Br, I, CF₃, CF₂CF₃, OCF₃, OCF₂CF₃, CN, NO₂, NH₂, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl;
   R⁴ is H, F, Cl, Br, I, CF₃, CF₂CF₃, OCF₃, CN, methyl, ethyl, propyl, isopropyl, n-butyl, sec-butyl, tert-butyl, OH, methoxyl, ethoxyl, propoxyl;
   R⁵ is H, F, Cl, Br, I, CF₃, CF₂CF₃, OCF₃, CN, methyl, ethyl, propyl, isopropyl, n-butyl, sec-butyl, tert-butyl, OH, methoxyl, ethoxyl, propoxyl;
   R⁶ is H, CN, methyl, ethyl, propyl, isopropyl or n-butyl;
   R^{7a} is H, C(O)R⁸, COOR⁸, C(O)R⁹, COOR⁹, C(O)NR⁸R⁹, C(O)NR⁹R⁹, S(O)₂R⁸, S(O)₂NR⁹R⁹, S(O)₂R⁹, S(O)₂NR⁹R⁹, C₁₋₁₀-alkyl, C₂₋₁₀-alkenyl, C₂₋₁₀-alkynyl or C₃₋₁₀-cycloalkyl, each of the C₁₋₁₀-alkyl, C₂₋₁₀-alkenyl, C₂₋₁₀-alkynyl and C₃₋₁₀-cycloalkyl optionally comprising 1-4 heteroatoms selected from N, O and S and optionally substituted with one or more substituents of NR⁸R⁹, NR⁹R⁹, OR⁸, SR⁸, OR⁹, SR⁹, C(O)R⁸, C(O)R⁹, C(O)NR⁸R⁹, C(O)NR⁹R⁹, NR⁹C(O)R⁹, NR⁹C(O)R⁹, NR⁹C(O)NR⁸R⁹, NR⁹C(O)NR⁹R⁹, NR⁹(COOR⁸), NR⁹(COOR⁹), S(O)₂R⁸, S(O)₂NR⁸R⁹, S(O)₂R⁹, S(O)₂NR⁹R⁹, NR⁹S(O)₂NR⁸R⁹, NR⁹S(O)₂NR⁹R⁹, NR⁹S(O)₂R⁹, NR⁹S(O)₂R⁹, R⁸ or R⁹;
   R^{7b} is H;
   R⁸ is a ring system selected from phenyl, naphthyl, pyridyl, piperazinyl, triazinyl, quinolinyl, isoquinolinyl, quinazolinyl, isoquinazolinyl, thiophenyl, furyl, pyrrolyl, imidazolyl, triazolyl, thiazolyl, oxazolyl, isoxazolyl, isothiazolyl, indolyl, isoindolyl, benzofuranyl, benzothiophenyl, benzimidazolyl, tetrahydrofuranyl, pyrrolidinyl, oxazolinyl, isoxazolinyl, thiazolinyl, pyrazolinyl, morpholinyl, piperidinyl, piperazinyl, pyranyl, dioxozinyl, cyclopropyl, cyclobutyl, cyclopentyl, cyclohexyl and cycloheptyl, wherein the ring system is optionally substituted independently with 1-3 substituents of R⁹, oxo, NR⁹R⁹, OR⁹; SR⁹, C(O)R⁹, phenyl, pyridyl, piperidyl, piperazinyl or morpholinyl;
   R⁹ is H, halo, haloalkyl, CN, OH, NO₂, NH₂, acetyl, C₁₋₁₀-alkyl, C₂₋₁₀-alkenyl, C₂₋₁₀-alkynyl, C₃₋₁₀-cycloalkyl, C₁₋₁₀-alkylamino-, C₁₋₁₀-dialkylamino-, C₁₋₁₀-alkoxyl, C₁₋₁₀-thioalkoxyl or a ring system selected from phenyl, naphthyl, pyridyl, piperazinyl, triazinyl, quinolinyl, isoquinolinyl, quinazolinyl, isoquinazolinyl, thiophenyl, furyl, pyrrolyl, imidazolyl, triazolyl, thiazolyl, oxazolyl, isoxazolyl, isothiazolyl, indolyl, isoindolyl, benzofuranyl, benzothiophenyl, benzimidazolyl, tetrahydrofuranyl, pyrrolidinyl, oxazolinyl, isoxazolinyl, thiazolinyl, pyrazolinyl, morpholinyl, piperidinyl, piperazinyl, pyranyl and dioxozinyl, each of the C₁₋₁₀-alkyl, C₂₋₁₀-alkenyl, C₂₋₁₀-alkynyl, C₃₋₁₀-cycloalkyl, C₁₋₁₀-alkylamino-, C₁₋₁₀-dialkylamino-, C₁₋₁₀-alkoxyl, C₁₋₁₀-thioalkoxyl and ring system optionally substituted independently with 1-3 substituents of halo, haloalkyl, CN, NO₂, NH₂, OH, oxo, methyl, methoxyl, ethyl, ethoxyl, propyl, propoxyl, isopropyl, cyclopropyl, butyl, isobutyl, tert-butyl, methylamino, dimethylamino, ethylamino, diethylamino, propylamine, isopropylamine, dipropylamine, diisopropylamine, benzyl or phenyl;
   R¹⁰ is H, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl; each of which is optionally substituted with one or more substituents of R¹¹, R¹² or R¹⁶;
   R¹¹ is phenyl, naphthyl, pyridyl, quinolinyl, tetrahydroquinolinyl, oxo-tetrahydroquinolinyl, isoquinolinyl, oxo-tetrahydroisoquinolinyl, tetrahydroisoquinolinyl, thiophenyl, tetrahydrofuranyl, thieno-pyrazolyl, tetrahydropentapyrazolyl, imidazolyl, thiazolyl, thiadiazolyl, benzothiazolyl, oxazolyl, oxadiazolyl, benzoxazolyl, benzoxadiazolyl, isoxazolyl, isothiazolyl, indolyl, 2,3-dihydroindolyl, benzofuranyl, benzothiophenyl, benzimidazolyl, 2,3-dihydro-1,4-benzoxazinyl, 1,3-benzodioxolyl, cyclopropyl, cyclobutyl, cyclopentyl and cyclohexyl, said ring system optionally substituted independently with 1-3 substituents of R¹², R¹³, R¹⁴ or R¹⁶;
   R¹² is H, C₁₋₁₀-alkyl, C₂₋₁₀-alkenyl, C₂₋₁₀-alkynyl, C₃₋₁₀-cycloalkyl, C₄₋₁₀-cycloalkenyl, C₁₋₁₀-alkylamino-, C₁₋₁₀-dialkylamino- or C₁₋₁₀-alkoxyl, each of which is optionally substituted independently with 1-3 substituents of R¹³, R¹⁴ R¹⁵ or R¹⁶;
   R¹³ is NR¹⁴R¹⁵, NR¹⁵R¹⁵, OR¹⁴; SR¹⁴, OR¹⁵; SR¹⁵, C(O)R¹⁴, OC(O)R¹⁴, COOR¹⁴, C(O)R¹⁵, OC(O)R¹⁵, COOR¹⁵, C(O)NR¹⁴R¹⁵, C(O)NR¹⁵R¹⁵, NR¹⁴C(O)R¹⁴, NR¹⁵C(O)R¹⁴, NR¹⁴C(O)R¹⁵, NR¹⁵C(O)R¹⁵, NR¹⁵C(O)NR¹⁴R¹⁵, NR¹⁵C(O)NR¹⁵R¹⁵, NR¹⁵(COOR¹⁴), NR¹⁵(COOR¹⁵), OC(O)NR¹⁴R¹⁵, OC(O)NR¹⁵R¹⁵, S(O)₂R¹⁴, S(O)₂R¹⁵, S(O)₂NR¹⁴R¹⁵, S(O)₂NR¹⁵R¹⁵, NR¹⁴S(O)₂NR¹⁴R¹⁵, NR¹⁵S(O)₂NR¹⁵R¹⁵, NR¹⁴S(O)₂R¹⁴ or NR¹⁵S(O)₂R¹⁵;
   R¹⁴ is phenyl, pyridyl, pyrimidinyl, thiophenyl, furyl, tetrahydrofuryl, pyrrolyl, pyrazolyl, thieno-pyrazolyl, imidazolyl, triazolyl, thiazolyl, thiadiazolyl, benzothiazolyl, oxazolyl, oxadiazolyl, benzoxazolyl, benzoxadiazolyl, isoxazolyl, isothiazolyl, indolyl, azaindolyl, isoindolyl, indazolyl, benzofuranyl, benzothiophenyl, benzimidazolyl, pyrrolidinyl, pyrazolinyl, morpholinyl, piperidinyl, piperazinyl, 2,3-dihydro-1,4-benzoxazinyl, 1,3-benzodioxolyl, cyclopropyl, cyclobutyl, azetidinyl, cyclopentyl and cyclohexyl, each of which is optionally substituted independently with 1-3 substituents of R¹⁵ or R¹⁶;
   R¹⁵ is H or C₁₋₁₀-alkyl, C₂₋₁₀-alkenyl, C₂₋₁₀-alkynyl, C₃₋₁₀-cycloalkyl, C₄₋₁₀-cycloalkenyl, C₁₋₁₀-alkylamino-, C₁₋₁₀-dialkylamino-, C₁₋₁₀-alkoxyl or C₁₋₁₀-thioalkoxyl, each of which is optionally substituted independently with 1-3 substituents of R¹⁶; and
   R¹⁶ is H, halo, haloalkyl, CN, OH, NO₂, NH₂, OH, methyl, methoxyl, ethyl, ethoxyl, propyl, propoxyl, isopropyl, cyclopropyl, butyl, isobutyl, tert-butyl, methylamino, dimethylamino, ethylamino, diethylamino, isopropylamino, oxo, acetyl, benzyl or a ring system selected from phenyl, pyridyl, thiophenyl, furyl, tetrahydrofuryl, pyrrolyl, pyrazolyl, thieno-pyrazolyl, imidazolyl, triazolyl, thiazolyl, thiadiazolyl, benzothiazolyl, oxazolyl, oxadiazolyl, benzoxazolyl, benzoxadiazolyl, isoxazolyl, isothiazolyl, indolyl, azaindolyl, isoindolyl, indazolyl, benzofuranyl, benzothiophenyl, benzimidazolyl, pyrrolidinyl, pyrazolinyl, morpholinyl, piperidinyl, piperazinyl, cyclopropyl, cyclobutyl, azetidinyl, cyclopentyl and cyclohexyl, said ring system optionally substituted independently with 1-3 substituents of halo, haloalkyl, CN, NO₂, NH₂, OH, methyl, methoxyl, ethyl, ethoxyl, propyl, propoxyl, isopropyl, cyclopropyl, butyl, isobutyl, tert-butyl, methylamino, dimethylamino, ethylamino, diethylamino, isopropylamino, benzyl or phenyl;
provided that
   (1) when A² is N, then R⁴ is not halo or hydroxyl.

Preferred embodiments are set forth in the subclaims.

In other embodiments, Formula II includes the various of the exemplary compounds described in the Experimentals Methods section hereinbelow.

### DEFINITIONS

The following definitions should assist in understanding the invention described herein.

The terms "agonist" and "agonistic" when used herein refer to or describe a molecule which is capable of, directly or indirectly, substantially inducing, promoting or enhancing biological activity of a biological molecule, such as an enzyme or receptor, including Tie-2 and Lck.

The term "comprising" is meant to be open ended, including the indicated component(s), but not excluding other elements.

The term "H" denotes a single hydrogen atom. This radical may be attached, for example, to an oxygen atom to form a hydroxyl radical.

The term "C_{α-β}alkyl", when used either alone or within other terms such as "haloalkyl" and "alkylamino", embraces linear or branched radicals having α to β number of carbon atoms (such as C₁-C₁₀). The term "alkyl" radicals include "lower alkyl" radicals having one to about six carbon atoms. Examples of such radicals include methyl, ethyl, n-propyl, isopropyl, n-butyl, isobutyl, *sec*-butyl, *tert*-butyl, pentyl, isoamyl, hexyl and the like. The term "alkylenyl" embraces bridging divalent alkyl radicals such as methylenyl and ethylenyl.

The term "alkenyl", when used alone or in combination, embraces linear or branched radicals having at least one carbon-carbon double bond in a moiety having between two and ten carbon atoms. Included within alkenyl radicals are "lower alkenyl" radicals having two to about six carbon atoms and, for example, those radicals having two to about four carbon atoms. Examples of alkenyl radicals include, without limitation, ethenyl, propenyl, allyl, propenyl, butenyl and 4-methylbutenyl. The terms "alkenyl" and "lower alkenyl", embrace radicals having "cis" and "trans" orientations, or alternatively, "E" and "Z" orientations, as appreciated by those of ordinary skill in the art.

The term "alkynyl", when used alone or in combination, denotes linear or branched radicals having at least one carbon-carbon triple bond and having two to ten carbon atoms. Examples of alkynyl radicals include "lower alkynyl" radicals having two to about six carbon atoms and, for example, lower alkynyl radicals having two to about four carbon atoms. Examples of such radicals include, without limitation, ethynyl, propynyl (propargyl), butynyl, and the like.

The term "alkoxy" or "alkoxyl", when used alone or in combination, embraces linear or branched oxygen-containing radicals each having alkyl portions of one or more carbon atoms. The term alkoxy radicals include "lower alkoxy" radicals having one to six carbon atoms. Examples of such radicals include methoxy, ethoxy, propoxy, butoxy and tert-butoxy. Alkoxy radicals may be further substituted with one or more halo atoms, such as fluoro, chloro or bromo, to provide "haloalkoxy" radicals. Examples of such radicals include fluoromethoxy, chloromethoxy, trifluoromethoxy, trifluoroethoxy, fluoroethoxy and fluoropropoxy.

The term "aryl", when used alone or in combination, means a carbocyclic aromatic moiety containing one, two or even three rings wherein such rings may be attached together in a fused manner. Every ring of an "aryl" ring system need not be aromatic, and the ring(s) fused to the aromatic ring may be partially or fully unsaturated and include one or more heteroatoms selected from nitrogen, oxygen and sulfur. Thus, the term "aryl" embraces aromatic radicals such as phenyl, naphthyl, indenyl, tetrahydronaphthyl, dihydrobenzafuranyl, anthracenyl, indanyl, benzodioxazinyl, and the like. The "aryl" group may be subsitituted, such as with 1 to 5 substituents including lower alkyl, hydroxyl, halo, haloalkyl, nitro, cyano, alkoxy and lower alkylamino, and the like. Phenyl substituted with -O-CH₂-O- or -O-CH₂-CH₂-O- forms an aryl benzodioxolyl substituent.

The term "carbocyclic", also referred to herein as "cycloalkyl", when used alone or in combination, means a partially or fully saturated ring moiety containing one ("monocyclic"), two ("bicyclic") or even three ("tricyclic") rings wherein such rings may be attached together in a fused manner and formed from carbon atoms. Examples of saturated carbocyclic radicals include saturated 3 to 6-membered monocyclic groups such as cyclopropane, cyclobutane, cyclopentane and cyclohexane.

The terms "ring" and "ring system" refer to a ring comprising the delineated number of atoms, the atoms being carbon or, where indicated, a heteroatom such as nitrogen, oxygen or sulfur. The ring itself, as well as any substitutents thereon, may be attached at any atom that allows a stable compound to be formed. The term "nonaromatic" ring or ring system refers to the fact that at least one, but not necessarily all, rings in a bicyclic or tricyclic ring system is nonaromatic.

The term "cycloalkenyl", when used alone or in combination, means a partially or fully saturated cycloalkyl containing one, two or even three rings in a structure having at least one carbon-carbon double bond in the structure. Examples of cycloalkenyl groups include C₃-C₆ rings, such as compounds including, without limitation, cyclopropene, cyclobutene, cyclopentene and cyclohexene. The term also includes carbocyclic groups having two or more carbon-carbon double bonds such as "cycloalkyldienyl" compounds. Examples of cycloalkyldienyl groups include, without limitation, cyclopentadiene and cycloheptadiene.

The term "halo", when used alone or in combination, means halogens such as fluorine, chlorine, bromine or iodine atoms.

The term "haloalkyl", when used alone or in combination, embraces radicals wherein any one or more of the alkyl carbon atoms is substituted with halo as defined above. For example, this term includes monohaloalkyl, dihaloalkyl and polyhaloalkyl radicals such as a perhaloalkyl. A monohaloalkyl radical, for example, may have either an iodo, bromo, chloro or fluoro atom within the radical. Dihalo and polyhaloalkyl radicals may have two or more of the same halo atoms or a combination of different halo radicals. "Lower haloalkyl" embraces radicals having 1-6 carbon atoms and, for example, lower haloalkyl radicals having one to three carbon atoms. Examples of haloalkyl radicals include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. "Perfluoroalkyl", as used herein, refers to alkyl radicals having all hydrogen atoms replaced with fluoro atoms. Examples include trifluoromethyl and pentafluoroethyl.

The term "heteroaryl", as used herein, either alone or in combination, means a fully unsaturated (aromatic) ring moiety formed from carbon atoms and having one or more heteroatoms selected from nitrogen, oxygen and sulfur. The ring moiety or ring system may contain one ("monocyclic"), two ("bicyclic") or even three ("tricyclic") rings wherein such rings are attached together in a fused manner. Every ring of a "heteroaryl" ring system need not be aromatic, and the ring(s) fused thereto (to the heteroaromatic ring) may be partially or fully saturated and optionally include one or more heteroatoms selected from nitrogen, oxygen and sulfur. The term "heteroaryl" does not include rings having ring members of -O-O-,-O-S- or -S-S-.

Examples of unsaturated heteroaryl radicals, include unsaturated 5- to 6- membered heteromonocyclyl groups containing 1 to 4 nitrogen atoms, including for example, pyrrolyl, imidazolyl, pyrazolyl, 2-pyridyl, 3-pyridyl, 4-pyridyl, pyrimidyl, pyrazinyl, pyridazinyl, triazolyl [e.g., 4H-1,2,4-triazolyl, 1H-1,2,3-triazolyl, 2H-1,2,3-triazolyl] and tetrazole; unsaturated 7- to 10- membered heterobicyclyl groups containing 1 to 4 nitrogen atoms, including for example, quinolinyl, isoquinolinyl, quinazolinyl, isoquinazolinyl, aza-quinazolinyl, and the like; unsaturated 5- to 6-membered heteromonocyclic group containing an oxygen atom, for example, pyranyl, 2-furyl, 3-furyl, benzofuryl, etc.; unsaturated 5 to 6-membered heteromonocyclic group containing a sulfur atom, for example, 2-thienyl, 3-thienyl, benzothienyl, etc.; unsaturated 5- to 6-membered heteromonocyclic group containing 1 to 2 oxygen atoms and 1 to 3 nitrogen atoms, for example, oxazolyl, isoxazolyl, oxadiazolyl [e.g., 1,2,4-oxadiazolyl, 1,3,4-oxadiazolyl, 1,2,5-oxadiazolyl]; unsaturated 5 to 6-membered heteromonocyclic group containing 1 to 2 sulfur atoms and 1 to 3 nitrogen atoms, for example, thiazolyl, isothiazolyl, thiadiazolyl [e.g., 1,2,4-thiadiazolyl, 1,3,4-thiadiazolyl, 1,2,5-thiadiazolyl].

The term "heterocyclic", when used alone or in combination, means a partially or fully saturated ring moiety containing one, two or even three rings wherein such rings may be attached together in a fused manner, formed from carbon atoms and including one or more heteroatoms selected from N, O or S. Examples of saturated heterocyclic radicals include saturated 3 to 6-membered heteromonocyclic groups containing 1 to 4 nitrogen atoms [e.g. pyrrolidinyl, imidazolidinyl, piperidinyl, pyrrolinyl, piperazinyl]; saturated 3 to 6-membered heteromonocyclic group containing 1 to 2 oxygen atoms and 1 to 3 nitrogen atoms [e.g. morpholinyl]; saturated 3 to 6-membered heteromonocyclic group containing 1 to 2 sulfur atoms and 1 to 3 nitrogen atoms [e.g., thiazolidinyl]. Examples of partially saturated heterocyclyl radicals include dihydrothienyl, dihydropyranyl, dihydrofuryl and dihydrothiazolyl.

The term "heterocycle" also embraces radicals where heterocyclic radicals are fused/condensed with aryl radicals: unsaturated condensed heterocyclic group containing 1 to 5 nitrogen atoms, for example, indolyl, isoindolyl, indolizinyl, benzimidazolyl, quinolyl, isoquinolyl, indazolyl, benzotriazolyl, tetrazolopyridazinyl [e.g., tetrazolo [1,5-b]pyridazinyl]; unsaturated condensed heterocyclic group containing 1 to 2 oxygen atoms and 1 to 3 nitrogen atoms [e.g. benzoxazolyl, benzoxadiazolyl]; unsaturated condensed heterocyclic group containing 1 to 2 sulfur atoms and 1 to 3 nitrogen atoms [e.g., benzothiazolyl, benzothiadiazolyl]; and saturated, partially unsaturated and unsaturated condensed heterocyclic group containing 1 to 2 oxygen or sulfur atoms [e.g. benzofuryl, benzothienyl, 2,3-dihydro-benzo[1,4]dioxinyl and dihydrobenzofuryl]. Examples of heterocyclic radicals include five to ten membered fused or unfused radicals.

Examples of partially saturated and saturated heterocyclyl include, without limitation, pyrrolidinyl, imidazolidinyl, piperidinyl, pyrrolinyl, pyrazolidinyl, piperazinyl, morpholinyl, tetrahydropyranyl, thiazolidinyl, dihydrothienyl, 2,3-dihydrobenzo[1,4]dioxanyl, indolinyl, isoindolinyl, dihydrobenzothienyl, dihydrobenzofuryl, isochromanyl, chromanyl, 1,2-dihydroquinolyl, 1,2,3,4-tetrahydro-isoquinolyl, 1,2,3,4-tetrahydro-quinolyl, 2,3,4,4a,9,9a-hexahydro-1H-3-aza-fluorenyl, 5,6,7-trihydro-1,2,4-triazolo[3,4-a]isoquinolyl, 3,4-dihydro-2H-benzo[1,4]oxazinyl, benzo[1,4]dioxanyl, 2,3-dihydro-1H-1λ'-benzo[d]isothiazol-6-yl, dihydropyranyl, dihydrofuryl and dihydrothiazolyl, and the like.

The term "alkylamino" includes "N-alkylamino" where amino radicals are independently substituted with one alkyl radical. Preferred alkylamino radicals are "lower alkylamino" radicals having one to six carbon atoms. Even more preferred are lower alkylamino radicals having one to three carbon atoms. Examples of such lower alkylamino radicals include N-methylamino, and N-ethylamino, N-propylamino, N-isopropylamino and the like.

The term "dialkylamino" includes "N, N-dialkylamino" where amino radicals are independently substituted with two alkyl radicals. Preferred alkylamino radicals are "lower alkylamino" radicals having one to six carbon atoms. Even more preferred are lower alkylamino radicals having one to three carbon atoms. Examples of such lower alkylamino radicals include N,N-dimethylamino, N,N-diethylamino, and the like.

The terms "carboxy" or "carboxyl", whether used alone or with other terms, such as "carboxyalkyl", denotes -CO₂H.

The term "carbonyl", whether used alone or with other terms, such as "aminocarbonyl", denotes -(C=O)-.

The term "aminocarbonyl" denotes an amide group of the formula -C(=O)NH₂.

The term "alkylthio" embraces radicals containing a linear or branched alkyl radical, of one to ten carbon atoms, attached to a divalent sulfur atom. An example of "alkylthio" is methylthio, (CH₃S-).

The term "haloalkylthio" embraces radicals containing a haloalkyl radical, of one to ten carbon atoms, attached to a divalent sulfur atom. An example of "haloalkylthio" is trifluoromethylthio.

The term "aminoalkyl" embraces linear or branched alkyl radicals having one to about ten carbon atoms any one of which may be substituted with one or more amino radicals. Examples of aminoalkyl radicals include "lower aminoalkyl" radicals having one to six carbon atoms and one or more amino radicals. Examples of such radicals include aminomethyl, aminoethyl, aminopropyl, aminobutyl and aminohexyl. Even more preferred are lower aminoalkyl radicals having one to three carbon atoms.

The term "alkylaminoalkyl" embraces alkyl radicals substituted with alkylamino radicals. Examples of alkylaminoalkyl radicals include "lower alkylaminoalkyl" radicals having alkyl radicals of one to six carbon atoms. Suitable alkylaminoalkyl radicals may be mono or dialkyl substituted, such as N-methylaminomethyl, N,N-dimethylaminoethyl, N,N-diethylaminomethyl and the like.

The term "alkylaminoalkoxy" embraces alkoxy radicals substituted with alkylamino radicals. Examples of alkylaminoalkoxy radicals include "lower alkylaminoalkoxy" radicals having alkoxy radicals of one to six carbon atoms. Suitable alkylaminoalkoxy radicals may be mono or dialkyl substituted, such as N-methylaminoethoxy, N,N-dimethylaminoethoxy, N,N-diethylaminoethoxy and the like.

The term "Formula II" includes any sub formulas.

The term "pharmaceutically-acceptable" when used with reference to a compound of Formula II is intended to refer to a form of the compound that is safe for administration. For example, a salt form, a solvate, a hydrate or derivative form of a compound of Formula II, which has been approved for mammalian use, via oral ingestion or other routes of administration, by a governing body or regulatory agency, such as the Food and Drug Administration (FDA) of the United States, is pharmaceutically acceptable.

Included in the compounds of Formula II are the pharmaceutically acceptable salt forms of the free-base compounds. The term "pharmaceutically-acceptable salts" embraces salts commonly used to form alkali metal salts and to form addition salts of free acids or free bases. As appreciated by those of ordinary skill in the art, salts may be formed from ionic associations, charge-charge interactions, covalent bonding, complexation, coordination, etc. The nature of the salt is not critical, provided that it is pharmaceutically acceptable.

Suitable pharmaceutically acceptable acid addition salts of compounds of Formula II may be prepared from an inorganic acid or from an organic acid. Examples of such inorganic acids are hydrochloric, hydrobromic, hydroiodic, hydrofluoric, nitric, carbonic, sulfuric and phosphoric acid. Appropriate organic acids may be selected from aliphatic, cycloaliphatic, aromatic, arylaliphatic, heterocyclic, carboxylic and sulfonic classes of organic acids, examples of which include, without limitation, formic, acetic, adipic, butyric, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, ethanedisulfonic, benzenesulfonic, pantothenic, 2-hydroxyethanesulfonic, toluenesulfonic, sulfanilic, cyclohexylaminosulfonic, camphoric, camphorsulfonic, digluconic, cyclopentanepropionic, dodecylsulfonic, glucoheptanoic, glycerophosphonic, heptanoic, hexanoic, 2-hydroxy-ethanesulfonic, nicotinic, 2-naphthalenesulfonic, oxalic, palmoic, pectinic, persulfuric, 2-phenylpropionic, picric, pivalic propionic, succinic, thiocyanic, undecanoic, stearic, algenic, β-hydroxybutyric, salicylic, galactaric and galacturonic acid. Suitable pharmaceutically-acceptable base addition salts of compounds of Formula II include metallic salts, such as salts made from aluminum, calcium, lithium, magnesium, potassium, sodium and zinc, or salts made from organic bases including, without limitation, primary, secondary and tertiary amines, substituted amines including cyclic amines, such as caffeine, arginine, diethylamine, N-ethyl piperidine, histidine, glucamine, isopropylamine, lysine, morpholine, N-ethyl morpholine, piperazine, piperidine, triethylamine, disopropylethylamine and trimethylamine. All of these salts may be prepared by conventional means from the corresponding compound of the invention by reacting, for example, the appropriate acid or base with the compound of Formula II.

Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, hydrobromic acid, citric acid, sulphuric acid and phosphoric acid and such organic acids as oxalic acid, stearic and, salicylic acid, pamoic acid, gluconic acid, ethanesulfonic acid, methanesulfonic acid, toluenesulfonic acid, tartaric acid, fumaric acid, medronic acid, napsylic acid, maleic acid, succinic acid and citric acid. Other examples include salts with alkali metals or alkaline earth metals such as sodium, potassium, calcium or magnesium, or with organic bases.

Additional examples of such salts can be found in Berge et al., J. Pharm. Sci., 66, 1 (1977). Conventional methods may be used to form the salts. For example, a phosphate salt of a compound of the invention may be made by combining the desired compound free base in a desired solvent, or combination of solvents, with phosphoric acid in a desired stoichiometric amount, at a desired temperature, typically under heat (depending upon the boiling point of the solvent). The salt can be precipitated upon cooling (slow or fast) and may crystallize (i.e., if crystalline in nature), as appreciated by those of ordinary skill in the art. Further, hemi-, mono-, di, tri- and poly-salt forms of the compounds of the present invention are also contemplated herein. Similarly, hemi-, mono-, di, tri- and poly-hydrated forms of the compounds, salts and derivatives thereof, are also contemplated herein.

The compound(s) of Formula II may be used to treat a subject by administering the compound(s) as a pharmaceutical composition. To this end, the compound(s) can be combined with one or more carriers, diluents or adjuvants to form a suitable composition, which is described in more detail herein.

The term "carrier", as used herein, denotes any pharmaceutically acceptable additive, excipient, adjuvant, or other suitable ingredient, other than the active pharmaceutical ingredient (API), which is typically included for formulation and/or administration purposes. "Diluent" and "adjuvant" are defined hereinafter.

The terms "treat", "treating," "treatment," and "therapy" as used herein refer to therapy, including without limitation, curative therapy, prophylactic therapy, and preventative therapy. Prophylactic treatment generally constitutes either preventing the onset of disorders altogether or delaying the onset of a pre-clinically evident stage of disorders in individuals.

The phrase "effective dosage amount" is intended to quantify the amount of each agent, which will achieve the goal of improvement in disorder severity and the frequency of incidence over treatment of each agent by itself, while avoiding adverse side effects typically associated with alternative therapies. For example, effective neoplastic therapeutic agents prolong the survivability of the patient, inhibit the rapidly-proliferating cell growth associated with the neoplasm, or effect a regression of the neoplasm.

The term "leaving groups" generally refer to groups that are displaceable by a nucleophile. Such leaving groups are known in the art. Examples of leaving groups include, but are not limited to, halides (e.g., I, Br, F, Cl), sulfonates (e.g., mesylate, tosylate), sulfides (e.g., SCH₃), N-hydroxsuccinimide, N-hydroxybenzotriazole, and the like. Nucleophiles are species that are capable of attacking a molecule at the point of attachment of the leaving group causing displacement of the leaving group. Nucleophiles are known in the art. Examples of nucleophilic groups include, but are not limited to, amines, thiols, alcohols, Grignard reagents, anionic species (e.g., alkoxides, amides, carbanions) and the like.

The term "angiogenesis" is defined as any alteration of an existing vascular bed or the formation of new vasculature which benefits tissue perfusion. This includes the formation of new vessels by sprouting of endothelial cells from existing blood vessels or the remodeling of existing vessels to alter size, maturity, direction and/or flow properties to improve blood perfusion of tissue.

The terms "cancer" and "cancerous" when used herein refer to or describe the physiological condition in mammals that is typically characterized by unregulated cell growth. Examples of cancer include carcinoma, lymphoma, sarcoma, blastoma and leukemia. More particular examples of such cancers include squamous cell carcinoma, lung cancer, pancreatic cancer, cervical cancer, bladder cancer, hepatoma, breast cancer, colon carcinoma, and head and neck cancer. While the term "cancer" as used herein is not limited to any one specific form of the disease, it is believed that the compounds for use of the invention will be particularly effective for cancers which are found to be accompanied by unregulated levels of Tie-2, and similar kinases, in the mammal.

### GENERAL SYNTHETIC PROCEDURES

The present invention further comprises procedures for the preparation of a compound of Formul II. The compound of Formula II can be synthesized according to the procedures described in the following Schemes 1-8, wherein the substituents are as defined for Formula II, except where further noted. The synthetic methods described below are merely exemplary, and the compounds of the invention may be synthesized by alternate routes as appreciated by persons of ordinary skill in the art.

The following list of abbreviations used throughout the specification represent the following and should assist in understanding the invention:

| | | |
|---|---|---|
| ACN, MeCN | - | acetonitrile |
| BSA | - | bovine serum albumin |
| Cs₂CO₃ | - | cesium carbonate |
| CHCl₃ | - | chloroform |
| CH₂Cl₂, DCM | - | dichloromethane, methylene chloride |
| CuBr | - | copper bromide |
| CuI | - | copper iodide |
| DIBAL | - | diisobutylaluminum hydride |
| DIC | - | 1,3-diisopropylcarbodiimide |
| DIEA,(iPr)₂NEt | - | diisopropylethylamine |
| DME | - | dimethoxyethane |
| DMF | - | dimethylformamide |
| DMAP | - | 4-dimethylaminopyridine |
| DMSO | - | dimethylsulfoxide |
| EDC | - | 1-(3-dimethylaminopropyl)-3-ethylcarbodiimide |
| Et₂O | - | diethyl ether |
| EtOAc | - | ethyl acetate |
| FBS | - | fetal bovine serum |
| G, gm | - | gram |
| h, hr | - | hour |
| H₂ | - | hydrogen |
| HATU | - | O-(7-azabenzotriazol-1-yl)-N,N,N',N'-tetramethyluroniumhexafluorophosphate |
| HBr | - | hydrobromic acid |
| HCl | - | hydrochloric acid |
| HOBt | - | 1-hydroxybenzotriazole hydrate |
| HPLC | - | high pressure liquid chromatography |
| IPA, IpOH | - | isopropyl alcohol |
| K₂CO₃ | - | potassium carbonate |
| KI | - | potassium iodide |
| MgSO₄ | - | magnesium sulfate |
| MeOH | - | methanol |
| N₂ | - | nitrogen |
| NaCNBH₃ | - | sodium cyanoborohydride |
| NaHCO₃ | - | sodium bicarbonate |
| NaH | - | sodium hydride |
| NaOCH₃ | - | sodium methoxide |
| NaOH | - | sodium hydroxide |
| Na₂SO₄ | - | sodium sulfate |
| NH₄Cl | - | ammonium chloride |
| NH₄OH | - | ammonium hydroxide |
| NMP | - | N-methylpyrrolidinone |
| P(t-bu)₃ | - | tri(tert-butyl)phosphine |
| PBS | - | phospate buffered saline |
| Pd/C | - | palladium on carbon |
| Pd(PPh₃)₄ | - | palladium(0)triphenylphosphine tetrakis |
| Pd(dppf)Cl₂ | - | palladium(1,1-bisdiphenylphosphinoferrocene) II chloride |
| Pd(PhCN)₂Cl₂ | - | palladium di-cyanophenyl dichloride |
| Pd(OAc)₂ | - | palladium acetate |
| Pd₂(dba)₃ | - | bis(dibenzylideneacetone) palladium |
| PyBop | - | benzotriazol-1-yl-oxy-tripyrrolidino-phosphonium hexafluorophosphate |
| RT | - | room temperature |
| TBTU | - | O-benzotriazol-1-yl-N,N,N',N'-tetramethyluronium tetrafluoroborate |
| TEA, Et₃N | - | triethylamine |
| TFA | - | trifluoroacetic acid |
| THF | - | tetrahydrofuran |

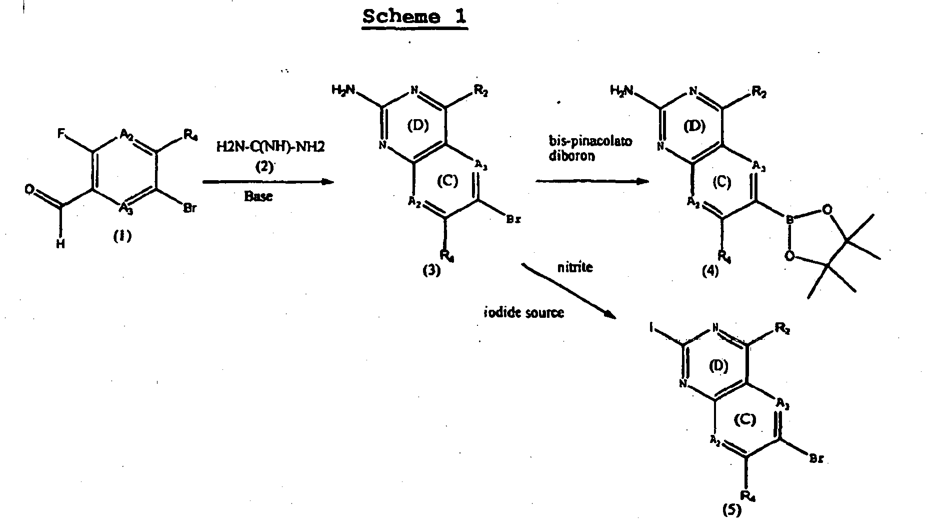

A 2-amino-6-halo or boron substituted-aryl nitrogen-containing bicyclic ring **3** or **4**, respectively, a quinoline where one of the nitrogens on the D ring is carbon (not shown), a quinazoline ring system where A² and A³ are both carbon, an aza-quinazoline ring system where either of A² or A³ are nitrogen or a diaza-quinazoline ring system where both of A² or A³ are nitrogen, and which are generally referred to herein as the C-D ring portion of the compounds of Formula II, can be prepared according to the method generally described in Scheme **1**. As shown, a halo-arylcarboxaldehyde **1** can be treated with guanidine **2** in the presence of a suitable solvent and a mild base, such as a tertiary amine base such as DIEA and/or NMP, to form the 2-amino-6-bromo nitrogen-containing bicyclic ring **3**. 2-amino-6-bromo nitrogen-containing bicyclic ring **3** can then be treated with bis(pinacolato)diboron to form the corresponding 6-dioxaborolane **4**. Alternatively, the 2-amino of compound **3** can be converted to the corresponding 2-iodo shown in compound **5**, by first transforming the NH₂ to the corresponding diazonium intermediate (not shown). The diazonium ion can then be replaced by addition of an iodide ion, provided from a suitable iodide source such as iodine or diiodomethane. The reaction occurs by initial elimination of the diazide cation followed by addition of the iodide anion in S_{N}1 mechanistic fashion. Compound **3** where R² is NH₂ and A² is N can be prepared using a method described in J. Med Chem. 40, 470, 1997. The bromide **3**, the boron compound **4** and iodo compound **5** are useful intermediates for coupling the R³ ring system, with or without a "B" linker, as illustrated in Formula II.

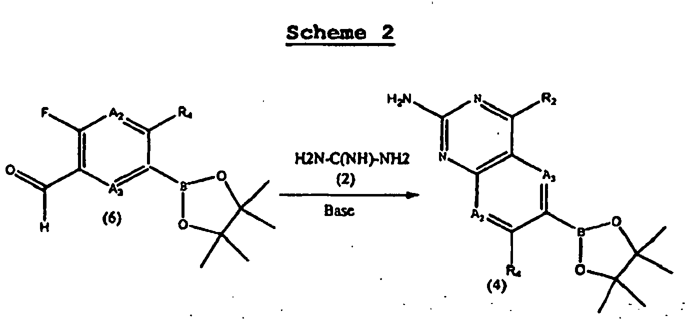

Alternatively, 2-amino-6-dioxaborolan-2-yl-aryl nitrogen-containing bicyclic ring **4**, can be prepared according to the method generally described in Scheme 2. As shown, a 2-halo-5-(4,4,5,5-tetramethyl-1,2,3-diboroxalan-2-yl) arylcarboxaldehyde **6** can be treated with guanidine **2** in the presence of a suitable solvent under suitable heat, such as in a microwave reactor, to form the 2-amino-6-dioxaborolane nitrogen-containing bicyclic ring **4**.

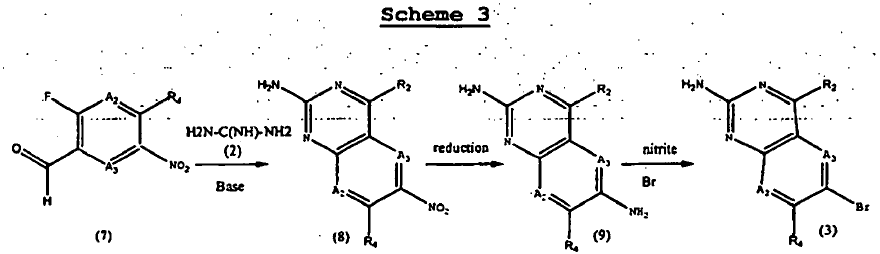

Alternatively, the 2-amino-6-bromo nitrogen-containing bicyclic ring **3** can then be made by a different route, such as by the route described in Scheme 3. As shown, 2-fluoro-5-nitro-arylcarboxaldehyde **7** can be treated with guanidine 2 in the presence of a suitable base, such as a carbonate base, to form the corresponding 6-nitro compound **8**. The nitro group of compound **8** can be reduced to the corresponding amino shown in compound **9** by methods well known to those skilled in the art, such as by hydrogenation or tin or zinc metal/acid reduction methods. The amino group of compound **9** can be treated with nitrite to form the diazonium intermediate (not shown), which can then be replaced with bromine, from a suitable bromide source such as HBr, to form the useful 6-bromo-intermediate compound **3**, as similarly described in Scheme 1.

The methods of Schemes 1-3 also readily apply to synthesis of the 4-NH₂ substituted quinazolines, aza-quinazolines and diazaquinazolines, as appreciated by the skilled artisan.

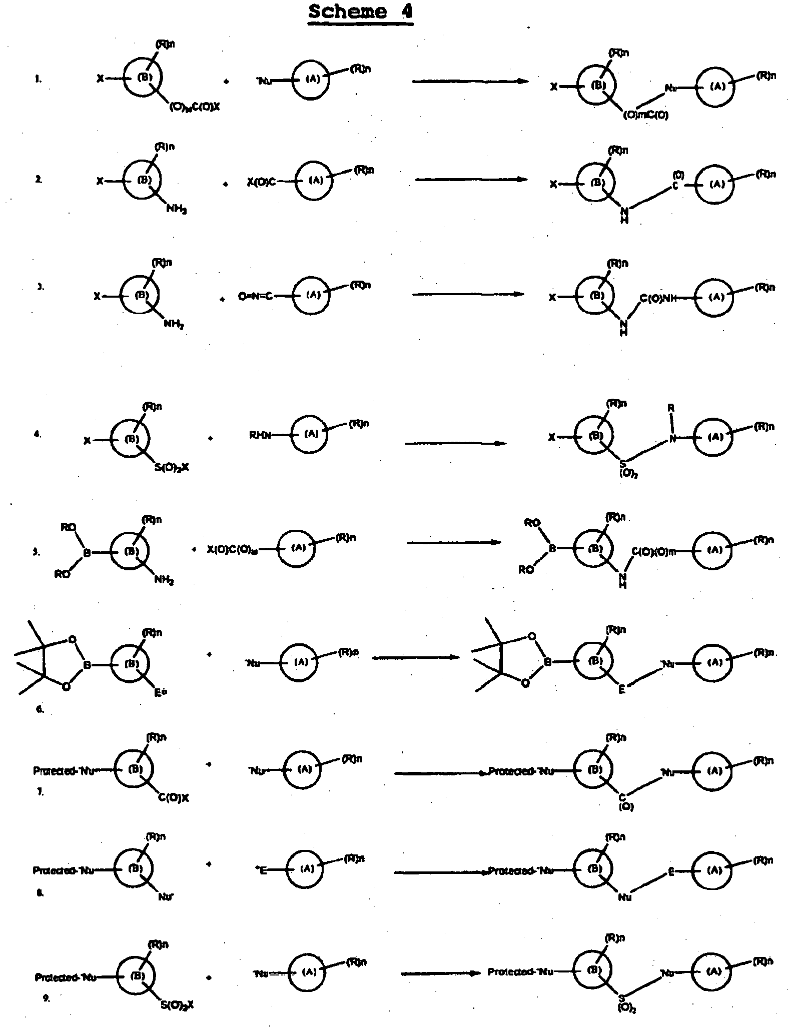

R³ ring systems, generally designated and referred to in Scheme 4, and throughout the specification, as the "B" ring may be substituted with various substitutions including R¹¹ ring systems, generally designated and referred to in Scheme 4, and throughout the specification, as the "A" ring system, by various coupling methods as described in Scheme 4. Each of the nine sub-schemes, numbered 1-9 above and described below, utilize the following meanings for (R)ₙ, X, Nu⁻, E⁺ and m: (R)ₙ refers to n number of R¹⁰, R¹¹ and R¹⁶ substitutions wherein n is an integer from 0-9; X refers generally to a "leaving group" such as a halide (bromine, chlorine, iodine or fluorine), alkylsulfonate and other known groups (also see definitions herein); Nu⁻ refers generally to a nucleophilic species such as a primary or secondary amine, an oxygen, a sulfur or a anionic carbon species - examples of nucleophiles include, without limitation, amines, hydroxides, alkoxides and the like; E⁺ refers generally to an electrophilic species, such as the carbon atom of a carbonyl, which is susceptible to nucleophilic attack or readily eliminates - examples of suitable electrophilic carbonyl species include, without limitation, acid halides, mixed anhydrides, aldehydes, carbamoyl-chlorides, sulfonyl chlorides, acids activated with activating reagents such as TBTU, HBTU, HATU, HOBT, BOP, PyBOP and carbodiimides (DCC, EDC and the like), and other electrophilic species including halides, isocyanates, daizonium ions and the like; and m is either 0 or 1.

The coupling of rings B and A, as shown as products in sub-schemes 1-9, can be brought about using various conventional methods to link rings B and A together. For example, an amide or a sulfonamide linkage, as shown in sub-schemes 2 and 4, and 7 and 9 where the Nu- is an amine, respectively, can be made utilizing an amine on either the B or A rings and an acid chloride or sulfonyl chloride on the other of either the B or A rings. The reaction proceeds generally in the presence of a suitable solvent and/or base. Suitable solvents include, without limitation, generally non-nucleophilic, anhydrous solvents such as toluene, CH₂Cl₂, THF, DMF, DMSO, N,N-dimethylacetamide and the like, including solvent combinations thereof. The solvent may range in polarity, as appreciated by those skilled in the art. Suitable bases include, for example, tertiary amine bases such as DIEA, TEA, carbonate bases such as Na₂CO₃, K₂CO₃, Cs₂CO₃, hydrides such as NaH, KH, borohydrides, cyanoborohydrides and the like, alkoxides such as NaOCH₃, and the like. The base itself may also serve as a solvent. The reaction may optionally be run neat, i.e., without any base and/or solvent. These coupling reactions are generally fast and conversion occurs typically in ambient conditions. However, depending upon the particular substrate, such reactions may require heat, as appreciated by those skilled in the art.

Similarly, carbamates as illustrated in sub-schemes 5 and 1 where Nu- is an amine, anhydrides as illustrated in sub-scheme 1 where Nu- is an oxygen, reverse amides as generally illustrated in sub-scheme 8 where Nu- is an amine and E+ is an acid chloride, ureas as illustrated in sub-scheme 3, thioamides and thioureas where the respective carbonyl oxygen is a sulfur, thiocarbamates where the respective carbonyl oxygen and/or carbamate oxygen is a sulfur, and the like. While the above methods are so described, they are not exhaustive, and other methods for linking rings A and B together may be utilized as appreciated by those skilled in the art.

Although sub-schemes 1-9 are illustrated as having the nucleophilic and electrophilic coupling groups, such as the amino group and acid chloride groups illustrated in sub-scheme 2, directly attached to the substrate, either the A or B ring, in question, the invention is not so limited. It is contemplated herein that these nucleophilic and/or electrophilic coupling groups may be tethered from their respective ring. For example, the amine group on the B ring, and/or the acid halide group on the A ring, as illustrated in sub-scheme 2, may be removed from direct attachment to the ring by a one or more atom spacer, such as by a methylene, ethylene spacer or the like. As appreciated by those skilled in the art, such spacer may or may not affect the coupling reactions described above, and accordingly, such reaction conditions may need to be modified to effect the desired transformation.

The coupling methods described in sub-schemes 1-9 of scheme 4 are also applicable for coupling desired A rings to desired DC-B intermediates, to synthesize desired compounds od Formula II.

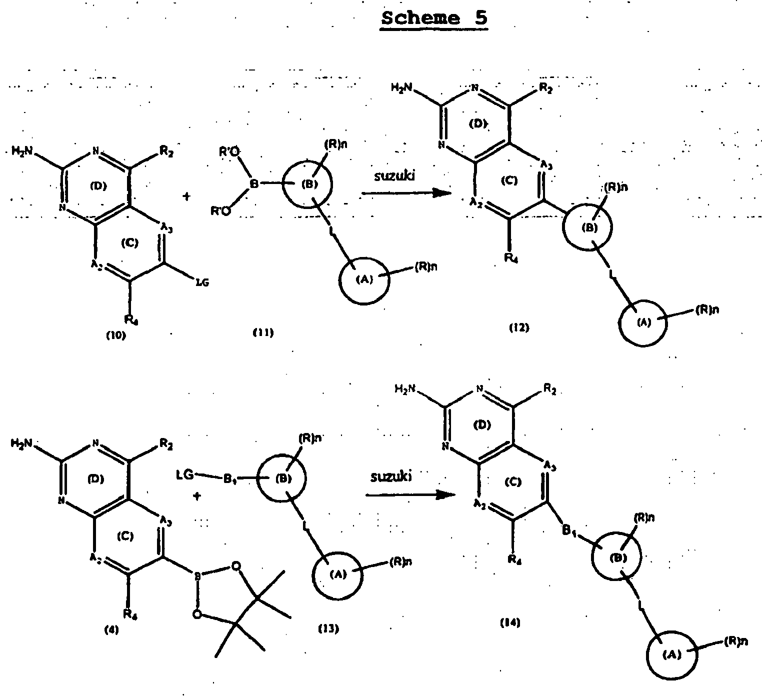

A compound according to Formula II can be prepared by the general method described in Scheme 5, utilizing the various intermediates described in Schemes 1-4. As shown, a Suzuki type coupling method may be employed to attach the desired C-D ring system to the desired B-A ring system, or simply to a desirably substituted B-ring system (not shown; see Experimental Methods herein). Other known metal coupling chemistry, such Stille, Kumada, Negishi coupling methods, and the like, may be employed to couple ring systems C-D to ring B, and to couple ring systems C-D to intermediates B-A. Note that the B-A ring system is connected through a linker "L". "L" may be any linker generally defined by the R³ substitutions in Formula II, and particularly, it includes, without limitation, an amide, a urea, a thiourea, a thioamide, a carbamate, an anhydride, a sulfonamide and the like, allowing for spacer atoms either between ring B and L and/or between ring A and L, as described in Scheme 4 above.

The Suzuki method is a reaction using a borane reagent, such as a dioxaborolane intermediate **4** previously described or a borane B-A intermediate **11** (R'= H or alkyl), and a suitable leaving group containing reagent, such as the 6-LG-C-D ring compound **10** or the halo-B-A ring compound **13** (LG = X = I, Br, Cl). As appreciated to one of ordinary skill in the art, Suzuki reactions also use palladium as a catalyst, in the presence of a suitable base, such as a carbonate base, bicarbonate or an acetate base, in a suitable solvent, such as toluene, acetonitrile, DMF or an aqueous-organic solvent combination or a biphasic system of solvents. Suitable palladium reagents include Pd(PPh₃)₄, Pd(OAc)₂ or Pd(dppf)Cl₂. Where LG is a halide, the halide may be an iodide, a bromide or even a chloride (chloro-pyridyl or chloro-picolinyl B rings undergo suzuki reactions in the presence of Pd(OAc)₂). In addition, a corresponding halo intermediate, the C-D ring piece or the B-A ring piece, may be converted to the borane, such as the dioxaborolane as described in Scheme 1. Other LGS are also suitable. For example, Suzuki couplings are known to occur with a sulfonate, such as trifluoromethanesulfonate, as the leaving group.

Compounds **13** and **14** further include a spacer, which is optional and generally designated as B¹ in Scheme 5, and as "B" in Formula II (B may also be a direct bond). This spacer may be a carbon spacer, such as that previously described, a nitrogen, an oxygen, a sulfur or any combination thereof. The spacer may not only be positioned between the halide and B ring, as illustrated, but also between the boron atom and the C-D ring as well. Accordingly, via the procedure described in Scheme 4, the C-D ring system can be coupled to the B-A ring system having a B linker or spacer so long as the particular coupling groups are available to react, i.e., the boronate ester and halide in this instance are available for a Suzuki reaction.

The coupling methods described in Scheme 5 may also be used to couple a C-D ring to a desired B ring, without having the A ring in place. Halo-NH₂-B rings may be coupled via a Suzuki reaction to a dioxaborolane C-D ring, and the amine group may then be converted to an isocyanate, for example, or any other desired group for coupling the A ring via the desired linker. Further, the amine may be protected, such as with BOC-ON, while further substituents are coupled to the B ring, prior to coupling the B ring to an A ring (see Scheme 7).

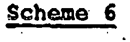

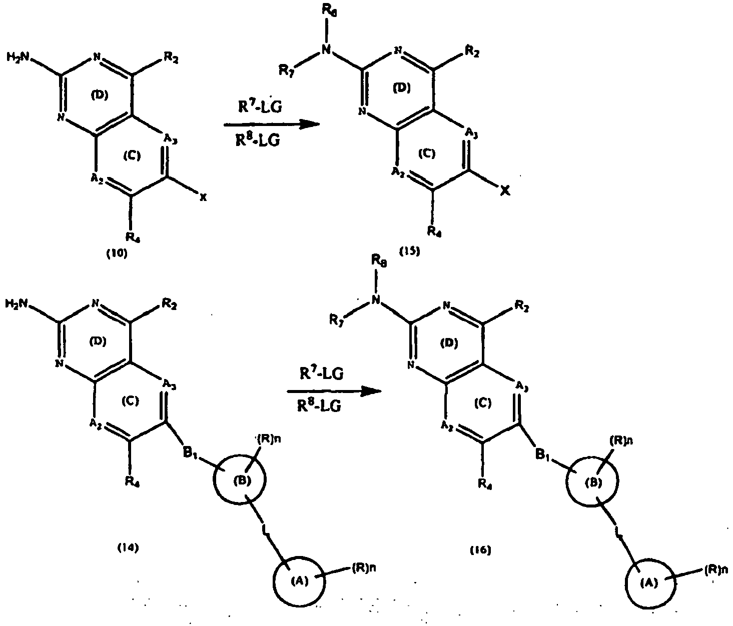

Various R⁷ and R⁸ substitutions can be installed in the C-D ring portion, at either the 2 or 4 position of the C-D ring of the compounds of Formula II, with or without the B-A ring system attached, as described in Scheme 6. For instance, compounds **15** and **16** may be made by the method described in Scheme 6. As shown, amino substitutions R⁷ and R⁸ may be made by reacting the amino aryl nitrogen containing bicyclic compound **10** or **14** with a desired R group having a leaving group ("LG"), suitable for reaction with an aryl NH₂. For example, a methyl group may be covalently bound to the amine via reaction with methyl iodide. Similarly, a 2-dimethylamino substitution may be obtained via excess methyl iodide, or similar methylating reagent. Base may or may nor be needed, as appreciated by those skilled in the art. Similarly, amide or sulfonamide linkers may be obtained where R⁷ or R⁸ is an activated carbonyl or sulfonyl species, such as an acid or sulfonyl chloride and the like. X on compound **10** is generally a halide, such as I, Br, or Cl as previously described, or a boronate. However, X may also be a suitable leaving group ("LG"), which may need to be protected during the reaction to install the R⁷ and/or R⁸ group, and later deprotected to couple the desired C-D ring system to the desired B-A ring system, utilizing methods described in Scheme 5. Such is readily appreciated by those skilled in the art.

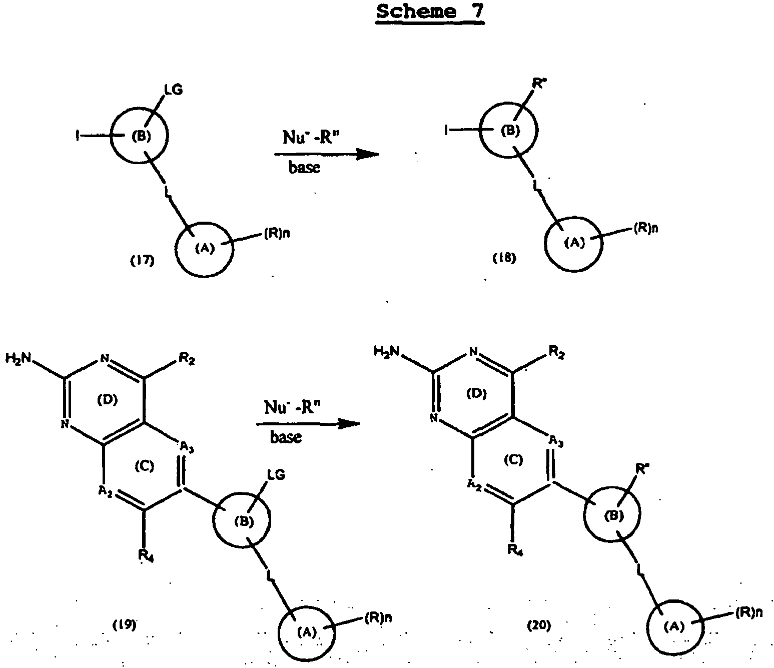

Various R¹⁰, R¹¹ and R¹⁶ substitutions (designated generally as R" groups in compounds **18** and **20**) can be installed on the B ring of Formula II, with or without the C-D ring system attached, as described in Scheme 7. For instance, compounds **18** and **20** may be made by the method described in Scheme 7. As shown, iodinated aryl B ring compounds **17** and compounds **19** may contain suitable leaving groups, such as a fluoride, at a desired position for substitution. These intermediates (compounds **17** and **19**) may be reacted with desirable nucleophilic R" groups (R¹⁰, R¹¹ and R¹⁶ substitutions), such as alkoxides, amines and the like, in the presence of a suitable base, such as a hydride or borohydride, to covalently bind the R" group to the B ring. Alternatively, the B ring may have a nucleophile, such as a hydroxide or an amine, which may be further functionalized as desired via standard chemical methodology, as appreciated by those skilled in the art.

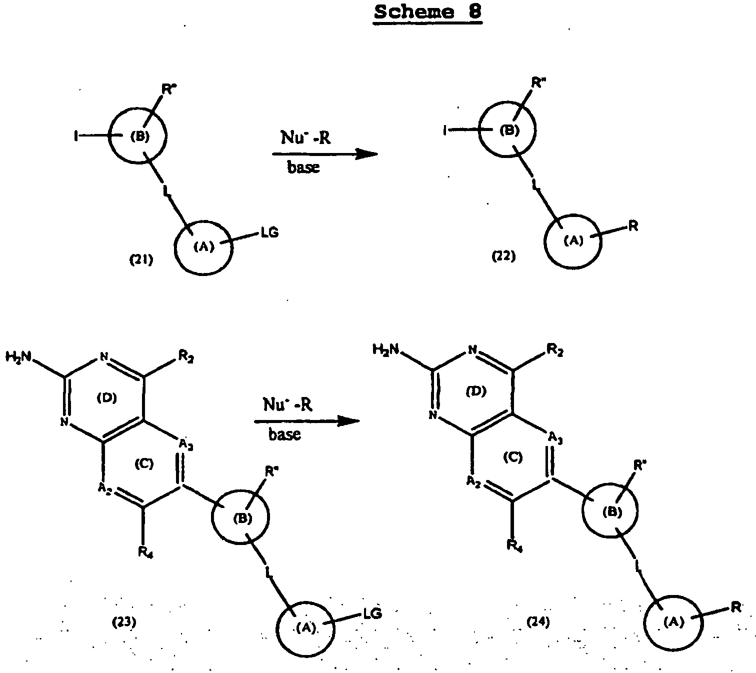

Various R¹², R¹³, R¹⁴ and R¹⁶ substitutions (designated generally as R groups in compounds **22** and **24**) can be installed on the A ring of Formula II, with or without the C-D ring system attached, as described in Scheme 8. For instance, compounds **22** and **24** may be made by the method described in Scheme 8. As shown, iodinated (or amino-protected, which is not shown) aryl B ring compounds **21** and compounds **23** may contain suitable leaving groups on the A ring, such as a halide, sulfonate, activated acid, ester, hydroxide and the like, at a desired position for substitution. These intermediates (compounds **21** and **23**) may be reacted with desirable nucleophilic R groups (R¹², R¹³, R¹⁴ and R¹⁶ substitutions), such as alkoxides, amines and the like, in the presence of a suitable base, such as a tertiary amine base, carbonate or bicarbonate bases, hydride or borohydride bases, hydroxide and alkoxide bases, and stronger bases as necessary, to covalently bind the R group to the A ring. Other R groups such as aryl rings, acetylene groups, and the like may be attached utilizing Suzuki methods or other metal chemistry as appreciated by the skilled artisan. Alternatively, the A ring may have a nucleophile, such as a hydroxide or an amine, which may be further functionalized as desired via standard chemical methodology, as appreciated by those skilled in the art.

To enhance the understanding and appreciation of the present invention, the following exemplary methods and specific examples (starting reagents, intermediates and compounds of Formula II) are set forth. It should be appreciated that these methods and examples are merely for illustrative purposes only and are not to be construed as limiting the scope of this invention in any manner.

### Analytical methods:

Unless otherwise indicated, all HPLC analyses were run on a Agilent Model 1100 system with an Agilent Technologies Zorbax SB-C₈(5 µ) reverse phase column (4.6 x 150 mm; Part no. 883975-906) run at 30 °C with a flow rate of about 1.50 mL/min. The mobile phase used solvent A (H₂O/0.1% TFA) and solvent B (ACN/0.1% TFA) with a 11 min gradient from 5% to 100% ACN. The gradient was followed by a 2 min. return to 5% ACN and about a 2.5 min. re-equilibration (flush).

### LC-MS Method:

Samples were run on an Agilent model-1100 LC-MSD system with an Agilent Technologies XDB-C₈ (3.5 µ) reverse phase column (4.6 x 75 mm) at 30 °C. The flow rate was constant and ranged from about 0.75 mL/min to about 1.0 mL/min.

The mobile phase used a mixture of solvent A (H₂O/0.1% HOAc) and solvent B (ACN/0.1% HOAc) with a 9 min time period for a gradient from 10% to 90% solvent B. The gradient was followed by a 0.5 min period to return to 10% solvent B and a 2.5 min 10% solvent B re-equilibration (flush) of the column.

### Preparative HPLC Method:

Where indicated, compounds of interest were purified via reverse phase HPLC using a Gilson workstation utilizing one of the following two columns and methods:
(A) Using a 50 x 100 mm column (Waters, Exterra, C18, 5 microns) at 50 mL/min. The mobile phase used was a mixture of solvent A (H₂O/10 mM ammonium carbonate at pH about 10, adjusted with conc. NH₄OH) and solvent B (85:15 ACN/water, 10 mM ammonium carbonate at pH of about 10 adjusted with conc. NH₄OH). Each purification run utilized a 10 minute gradient from 40% to 100% solvent B followed by a 5 minute flow of 100% solvent B. The gradient was followed by a 2 min return to 40% solvent B.
(B) Using a 20 x 50 mm column at 20 mL/min. The mobile phase used was a mixture of solvent A (H₂O/0.1% TFA) and solvent B (ACN/0.1% TFA) with a 10 min gradient from 5% to 100% solvent B. The gradient is followed by a 2 min return to 5% ACN.

### Proton NMP Spectra :

Unless otherwise indicated, all ¹H NMR spectra were run on a Varian series Mercury 300 MHz instrument or a Bruker series 400MHz instrument. Where so characterized, all observed protons are reported as parts-per-million (ppm) downfield from tetramethylsilane (TMS) or other internal reference in the appropriate solvent indicated.

### Mass Spectra (MS)

Unless otherwise indicated, all mass spectral data for starting materials, intermediates and/or exemplary compounds are reported as mass/charge (m/z), having an (M+H⁺) molecular ion. The molecular ion reported was obtained by electrospray detection method. Compounds having an isotopic atom, such as bromine and the like, are reported according to the detected isotopic pattern, as appreciated by those skilled in the art.

The following examples represent various starting materials and intermediates, which should assist in better understanding and appreciating the exemplary methods of synthesizing compounds of Formula II.

Various experimental methods have been employed to synthesize compounds of Formula II, as more generally described in schemes 1-8 above, and further described in more detail by the representative examples below.

### Experimental Method A1

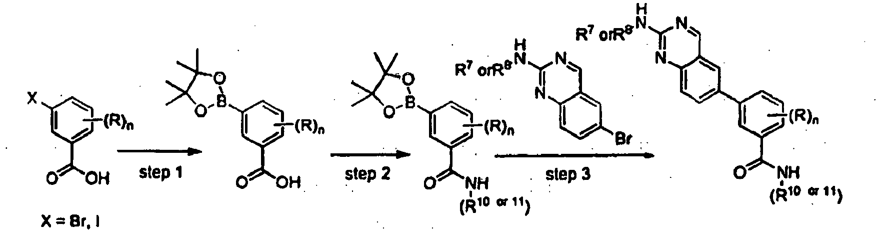

Compounds falling within claims 1 and 2 are according to the invention. All other compounds are reference compounds.

### Example 1

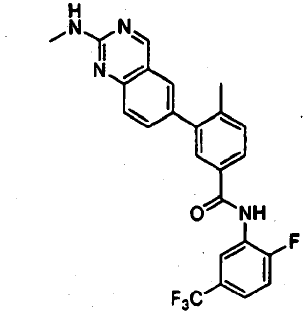

### Synthesis of N-(2-fluoro-5-(trifluoromethyl)phenyl)-4-methyl-3-(2-(methylamino)quinazolin-6-yl)benzamide:

### Step 1: Preparation of 9-methyl-3-(9,4,5,5-tetramethyl-1,3,2-dioxaborolan-2-yl)benzoic acid:

3-Iodo-4-methylbenzoic acid (10.0 g, 38.2 mmol), bis(pinacolato)diboron (12.6 g, 49.6 mmol), [1,1'-bis(diphenylphosphino)ferrocene]palladium (II) chloride (2.8 g, 3.82 mmol), and KOAc (13.1 g, 134 mmol) were added to a screw cap sealed tube. The tube was purged with N₂, DMF (116 mL) was added, and the tube was sealed. The reaction mixture was stirred at 70°C overnight. The reaction mixture was allowed to cool to RT and was concentrated *in vacuo.* The residue was diluted with 2N NaOH and ethyl acetate. The aqueous layer was extracted 5 times with ethyl acetate, then acidified to pH 3 with 6N HCl. 4-methyl-3-(4,4,5,5-tetramethyl-1,3,2-dioxaborolan-2-yl)benzoic acid precipitated as a pale pink solid. The solid was filtered, rinsed with water, and dried under high vacuum overnight to provide 4-methyl-3-(4,4,5,5-tetramethyl-1,3,2-dioxaborolan-2-yl)benzoic acid. MS m/z = 263 [M+1]⁺. Calc'd for C₁₄H₁₉BO₄: 262

### Step 2: Preparation of N-(2-fluoro-5-(trifluoromethyl)phenyl)-4-methyl-3-(4,9,5,5-tetramethyl-1,3,2-dioxaborolan-2-yl)benzamide:

4-Methyl-3-(4,4,5,5-tetramethyl-1,3,2-dioxaborolan-2-yl)benzoic acid (300 mg, 1.14 mmol) and thionyl chloride (2.34 mL, 32.0 mmol) were heated to 80°C in a screw cap sealed tube for 1 hour. The reaction mixture was allowed to cool to room temperature and then concentrated *in vacuo,* followed by azeotropic removal of water with toluene. To the crude acid chloride was added 2-fluoro-5-(trifluoromethyl)benzenamine (0.173 mL, 1.37 mmol), dichloromethane (5.7 mL), and triethylamine (several drops). After stirring at room temperature for 1 hour, the reaction mixture was diluted with ethyl acetate and 1N NaOH. The aqueous layer was extracted with ethyl acetate 3 times, and the combined organic extracts were washed with brine, dried over MgSO₄, and concentrated *in vacuo* to yield N-(2-fluoro-5-(trifluoromethyl)phenyl)-4-methyl-3-(4,4,5,5-tetramethyl-1,3,2-dioxaborolan-2-yl)benzamide. MS *m*/*z* = 424 [M+1]⁺. Calc'd for C₂₁H₂₂BF₄NO₃: 423.

### Step 3: N-(2-fluoro-5-(trifluoromethyl)phenyl)-4-methyl-3-(2-(methylamino)quinazolin-6-yl)benzamide:

N-(2-Fluoro-5-(trifluoromethyl)phenyl)-4-methyl-3-(4,4,5,5-tetramethyl-1,3,2-dioxaborolan-2-yl)benzamide (135 mg, 0.321 mmol), 6-bromo-N-methylquinazolin-2-amine (84 mg, 0.353 mmol), [1,1'-bis(diphenylphosphino) ferrocene]palladium(II)chloride (23.5 mg, 0.0321 mmol), Na₂CO₃ (2M in water, 0.482 mL), and 1,4-dioxane (2.35 mL) were combined in a screw cap sealed tube and stirred at 80 °C overnight. The reaction mixture was allowed to cool to room temperature and diluted with water and ethyl acetate. The water layer was extracted with ethyl acetate twice, then CH₂Cl₂ once. The combined organic extracts were washed with brine, dried over MgSO₄, filtered, and concentrated *in vacuo.* The residue was purified by automated flash chromatography (CH₂Cl₂ and 90:10:1 CH₂Cl₂:CH₃OH:NH₄OH gradient) to provide N-(2-fluoro-5-(trifluoromethyl)phenyl)-4-methyl-3-(2-(methylamino)quinazolin-6-yl)benzamide. MS *m*/*z* = 455 [M+1]⁺. Calc'd for C₂₄H₁₈F₄N₄O: 454.

### Example 2

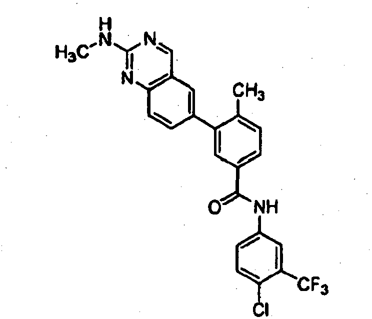

### Synthesis of N-(4-chloro-3-(trifluoromethyl)phenyl)-4-methyl-3-(2-(methylamino)quinazolin-6-yl)benzamide:

### Step 1: Preparation of 4-methyl-3-(4,4,5,5-tetramethyl-1,3,2-dioxaborolan-2-yl)benzoic acid

3-Iodo-4-methylbenzoic acid (10.0 g, 38.2 mmol), bis(pinacolato)diboron (12.6 g, 49.6 mmol), [1,1'-bis(diphenylphosphino)ferrocene]palladium (II) chloride (2.8 g, 3.82 mmol), and KOAc (13.1 g, 134 mmol) were added to a screw cap sealed tube. The tube was purged with N₂, DMF (116 mL) was added, and the tube was sealed. The reaction mixture was stirred at 70 °C overnight. The reaction mixture was allowed to cool to RT and was concentrated *in vacuo.* The residue was diluted with 2N NaOH and ethyl acetate. The aqueous layer was extracted 5 times with ethyl actetate, then acidified to pH 3 with 6N HCl. 4-methyl-3-(4,4,5,5-tetramethyl-1,3,2-dioxaborolan-2-yl)benzoic acid precipitated as a pale pink solid. The solid was filtered, rinsed with water, and dried under high vacuum overnight to provide 4-methyl-3-(4,4,5,5-tetramethyl-1,3,2-dioxaborolan-2-yl)benzoic acid. MS *m*/*z* = 263 [M+1]⁺; Calc'd for C₁₄H₁₉BO₄: 262.

### Step 2: Preparation of N-(4-chloro-3-(trifluoromethyl)phenyl)-4-methyl-3-(4,4,5,5-tetramethyl-1,3,2-dioxaborolan-2-yl)benzamide:

4-Methyl-3-(4,4,5,5-tetramethyl-1,3,2-dioxaborolan-2-yl)benzoic acid (200 mg, 0.763 mmol) was dissolved in DMF (1.84 mL), and 4-chloro-3-(trifluoromethyl)benzenamine (137 mg, 0.700 mmol), N,N-diisopropylethylamine (0.300 mL, 1.72 mmol), and HATU (279 mg, 0.735 mmol) were added successively. The mixture was allowed to stir at room temperature overnight. The solvent was removed *in vacuo,* and the residue was partitioned between ethyl acetate and saturated aqueous NaHCO₃. The aqueous layer was extracted with ethyl acetate, and the combined organic extracts were dried over MgSO₄, filtered, and concentrated *in vacuo*. The residue was purified by automated flash chromatography (100% hexanes to 4:1 hexanes:ethyl acetate) to provide N-(4-chloro-3-(trifluoromethyl)phenyl)-4-methyl-3-(4,4,5,5-tetramethyl-1,3,2-dioxaborolan-2-yl)benzamide. MS *m*/*z* = 440; Calc'd for C₂₁H₂₂BClF₃NO₃: 440.

### Step 3: Preparation of N-(4-chloro-3-(trifluoromethyl)phenyl)-4-methyl-3-(2-(methylamino)quinazolin-6-yl)benzamide:

N-(4-chloro-3-(trifluoromethyl)phenyl)-4-methyl-3-(4,4,5,5-tetramethyl-1,3,2-dioxaborolan-2-yl)benzamide (180 mg, 0.409 mmol), 6-bromo-N-methylquinazolin-2-amine (107 mg, 0.450 mmol), [1,1'-bis(diphenylphosphino) ferrocene]palladium(II)chloride (30 mg, 0.0409 mmol), Na₂CO₃ (2M in water, 0.614 mL), and 1,4-dioxane (2.73 mL) were combined in a screw cap sealed tube and stirred at 80 °C overnight. The reaction mixture was allowed to cool to room temperature and then diluted with water and ethyl acetate. The water layer was extracted with ethyl acetate twice, then CH₂Cl₂ once. The combined organic extracts were washed with brine, dried over MgSO₄, filtered, and concentrated in vacuo. The residue was purified by automated chromatography (CH₂Cl₂ and 90:10:1 CH₂Cl₂ : CH₃OH : NH₄OH gradient) to provide N-(4-chloro-3-(trifluoromethyl)phenyl)-4-methyl-3-(2-(methylamino)quinazolin-6-yl)benzamide. MS m/z = 471. Calc'd for C₂₄H₁₈ClF₃N₄O: 471.

The following Examples 3-8 were prepared by a method similar to that described in Experimental Method A1 and Example 1, utilizing an acid chloride method for step 2.

| Example No. | Structure | Name | Method | MW | (M+H⁺) |
|---|---|---|---|---|---|
| 3 | 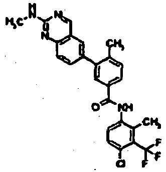 | N-(4-chloro-2-methyl-3-(trifluoromethyl)phenyl)-4-methyl-3-(2-(methylamino)-6-quinazolinyl)benzamide | A1 | 484.907 | 485 |
| 4 | 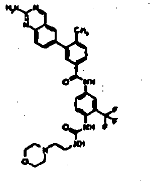 | 3-(2-amino-6-quinazolinyl)-4-methyl-N-(4-((((2-(4-morpholinyl)ethyl)amino)carbonyl)amino)-3-(trifluoromethyl)phenyl)benzamide | A1 | 593.607 | 594.2 |
| 5 | 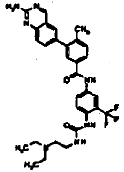 | 3-(2-amino-6-quinazolinyl)-N-(4-((((2-(diethylamino)ethyl)amino)carbonyl)amino)-3-(trifluoromethyl)phenyl)-4-methylbenzamide | A1 | 579.624 | 580.2 |
| 6 | 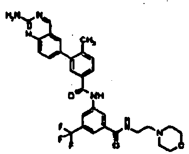 | 3-(2-amino-6-quinazolinyl)-4-methyl-N-(3-(((2-(4-morpholinyl)ethyl)amino)carbonyl)-5-(trifluoromethyl)phenyl)benzamide | A1 | 578.592 | 579.1 |
| 7 | 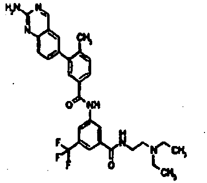 | 3-(2-amino-6-quinazolinyl)-N-(3-(((2-(diethylamino)ethyl)amino)carbonyl)-5-(trifluoromethyl)phenyl)-4-methylbenzamide | A1 | 564.609 | 565.1 |
| 8 | 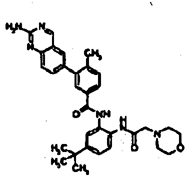 | 3-(2-amino-6-quinazolinyl)-N-(5-(1,1-dimethylethyl)-2-((4-morpholinylacetyl)amino)phenyl)-4-methylbenzamide | A1 | 552.675 | 553.2 |

The following Examples 9-14 were prepared by a method similar to that described in Experimental Method A1 and Example 2, utilizing a conventional acid-amine coupling reagent, such as HOBT, HATU, and the like, in step 2.

| Example No. | Structure | Name | Method | MW | (M+H⁺) |
|---|---|---|---|---|---|
| 9 | 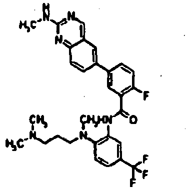 | N-(2-((3-(dimethylamino)propyl)(methyl)amino)-5-(trifluoromethyl)phenyl)-2-fluoro-5-(2-(methylamino)-6-quinazolinyl)benzamide | A1 | 554.589 | 555.3 |
| 10 | 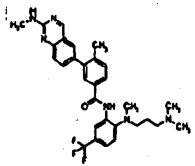 | N-(2-((3-(dimethylamino)propyl)(methyl)amino)-5-(trifluoromethyl)phenyl)-4-methyl-3-(2-(methylamino)-6-quinazolinyl)benzamide | A1 | 550.626 | 551 |
| 11 | 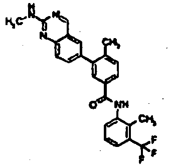 | 4-methyl-3-(2-(methylamino)-6-quinazolinyl)-N-(2-methyl-3-(trifluoromethyl)phenyl)benzamide | A1 | 450.462 | 451 |
| 12 | 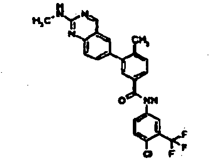 | N-(4-chloro-3-(trifluoromethyl)phenyl)-4-methyl-3-(2-(methylamino)-6-quinazolinyl)benzamide | A1 | 470.88 | 471 |
| 13 | 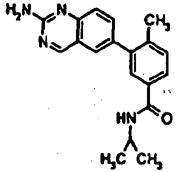 | 3-(2-amino-6-quinazolinyl)-4-methyl-N-(1-methylethyl)benzamide | A1 | 320.394 | 321.1 |
| 14 | 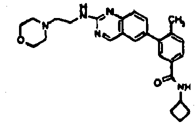 | N-cyclobutyl-4-methyl-3-(2-((2-(4-morpholinyl)ethyl)amino)-6-quinazolinyl)benzamide | A1 | 445.564 | 446.2 |

### Experimental Method A2

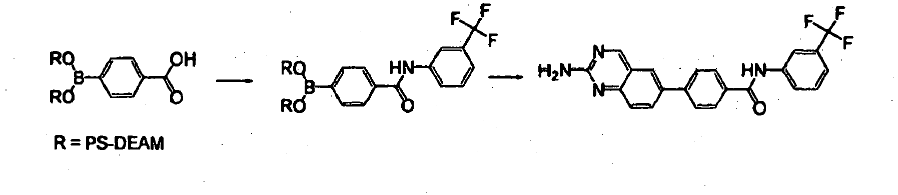

### Example 15

### Synthesis of 4-(2-amino-6-quinazolinyl)-N-(3-(trifluoromethyl)phenyl)benzamide

Polystyrene-supported diethanolamine (PS-DEAM, 1.64 mmol/g, 5.7 g, 9.3 mmol) was mixed with 4-boronobenzoic acid (2.0 g, 6.0 mmol) in 85 mL anhydrous THF. After 1 h, the THF was removed by filtration, and the solid rinsed three times with THF. A portion of the resulting solid (2.4 g) was mixed with 3-trifluoromethyl aniline (1.8 mL, 14 mmol), DIC (2.2 mL, 14 mmol), and HOBT (1.9 g, 14 mmol) in 15 mL NMP in a sealed fritted tube. The mixture was shaken for 12 h, and the solid was rinsed with three times with NMP, five times with THF, and five times with dichloromethane to give a yellow solid. A portion of this material (0.20 g) was mixed with 2-amino-6-bromo quinazoline (0.030 g, 0.13 mmol), tetrakis(triphenylphosphine) palladium (0) (0.009 g, 0.008 mmol), sodium carbonate (2.0 M solution in water, 0.27 mL, 0.54 mmol), 1.3 mL THF and 0.3 mL EtOH and heated to 70 °C for 24 h. The mixture was cooled and filtered, and the filtrate concentrated *in vacuo* to yield a crude solid. The crude mixture was purified by silica gel chromatography (acetone/methylene chloride + triethylamine) to give the desired product. MS (m/z): 409.0 (M+H)⁺. Calc'd for C₂₂H₁₅F₃N₄O- 408.38.

### Experiental Method B

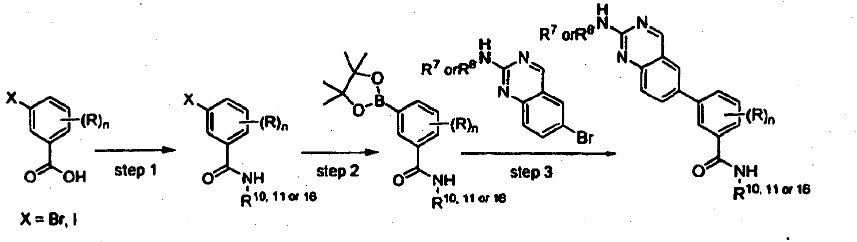

### Example 16

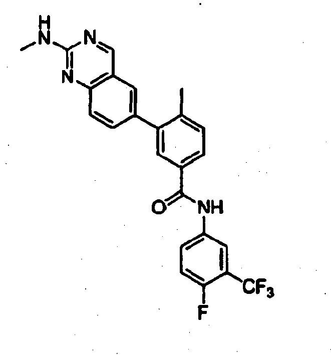

### Synthesis of N-(4-fluoro-3-(trifluoromethyl)phenyl)-4-methyl-3-(2-(methylamino)-6-quinazolinyl)benzamide:

### Step 1: Preparation of N-(4-fluoro-3-(trifluoromethyl)phenyl)-3-iodo-4-methylbenzamide:

3-Iodo-4-methyl benzoic acid (200 mg, 0.763 mmol) and thionyl chloride (4.5 mL, 612 mmol) were heated to 80°C in a round-bottom flask equipped with a reflux condenser for 1 hr. After cooling to room temperature, the reaction mixture was concentrated on the rotary evaporator and then high vacuum for 10 minutes. The residue was dissolved in CH₂Cl₂ (7.6 mL), and then the flask was charged with triethylamine (0.21 mL, 1.53 mmol) and 4-fluoro-3-(trifluoromethyl)benzenamine (0.916 mmol, 0.118 mL). The reaction mixture was allowed to stir at room temperature for 4 hrs. Upon completion, the reaction mixture was concentrated *in vacuo.* The residue was purified by automated chromatography (100% CH₂Cl₂) to yield N-(4-fluoro-3-(trifluoromethyl)phenyl)-3-iodo-4-methylbenzamide. MS *m*/*z* = 421 [M-2H]⁻, 422 [M-H]⁻. Calc'd for C₁₅H₁₀F₄INO₃: 423

### Step 2: Preparation of N-(4-fluoro-3-(trifluoromethyl)phenyl)-4-methyl-3-(4,4,5,5-tetramethyl-1,3,2-dioxaborolan-2-yl)benzamide:

N-(4-fluoro-3-(trifluoromethyl)phenyl)-3-iodo-4-methylbenzamide (346 mg, 0.818 mmol), bis(pinacolato)diboron (270 mg, 1.06 mmol), [1,1'-bis(diphenylphosphino)ferrocene]palladium (II) chloride (60 mg, 0.082 mmol), and KOAc (281 mg, 2.86 mmol) were combined in a screw cap sealed tube. The tube was purged with nitrogen, and then DMF or dimethylacetamide (2.5 mL; DMA) was added. The tube was sealed and the mixture was heated to 70 °C for 10 hrs. The reaction mixture was concentrated *in vacuo,* and the residue was dissolved in dichloromethane. The CH₂Cl₂ solution was washed twice with water, dried over Na₂SO₄, and concentrated *in vacuo.* The residue was purified by automated chromatography (100% CH₂Cl₂) to yield N-(4-fluoro-3-(trifluoromethyl)phenyl)-4-methyl-3-(4,4,5,5-tetramethyl-1,3,2-dioxaborolan-2-yl)benzamide. MS m/z = 424 [M+H]⁺. Calc'd for C₂₁H₂₂BF₄NO₃: 423.

### Step 3: N-(4-fluoro-3-(trifluoromethyl)phenyl)-4-methyl-3-(2-(methylamino)-6-quinazolinyl)benzamide:

N-(4-fluoro-3-(trifluoromethyl)phenyl)-4-methyl-3-(4,4,5,5-tetramethyl-1,3,2-dioxaborolan-2-yl)benzamide (140 mg, 0.331 mmol), 6-bromo-N-methylquinazolin-2-amine (63 mg, 0.265 mmol), [1,1'-bis(diphenylphosphino) ferrocene]palladium(II)chloride (19.4 mg, 0.0265 mmol), and K₂CO₃ (58.5 mg, 0.424 mmol) were combined in a screw cap sealed tube. The vessel was purged with nitrogen and then CH₃CN (2.65 mL) and water (0.83 mL) were added. The tube was sealed and the reaction mixture was stirred at 60°C for 2 hrs. The reaction mixture was concentrated *in vacuo* and diluted with CH₂Cl₂. The dichloromethane solution was washed with water, dried over Na₂SO₄, filtered, and concentrated *in vacuo*. The residue was purified by automated chromatography (9:1 to 1:1 hexanes:ethyl acetate gradient) to provide N-(4-fluoro-3-(trifluoromethyl)phenyl)-4-methyl-3-(2-(methylamino)-6-quinazolinyl)benzamide as a pale peach solid. MS *m*/*z* = 455 [M+H]⁺; Calc'd for C₂₄H₁₈F₄N₄O: 454.

The following Examples 17-49 were prepared by a method similar to that described in Experimental Method B and Example 16.

| Example No. | Structure | Name | Method | MW | (M+H⁺) |
|---|---|---|---|---|---|
| (Reference Example) 17 | 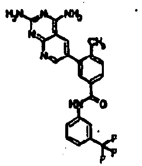 | 3-(2,4-diaminopyrido[2,3-d]pyrimidin-6-yl)-4-methyl-N-(3-(trifluoromethyl)phenyl)benzamide | B | 438.411 | 439.1 |
| 18 | 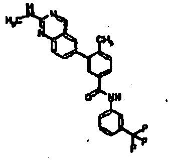 | 4-methyl-3-(2-(methylamino)-6-quinazolinyl)-N-(3-(trifluoromethyl)phenyl)benzamide | B | 436.435 | 437 |
| 19 | 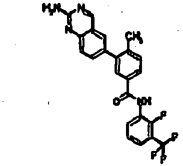 | 3-(2-amino-6-quinazolinyl)-N-(2-fluoro-3-(trifluoromethyl)phenyl)-4-methylbenzamide | B | 440.398 | 441 |
| 20 | 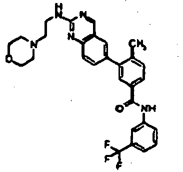 | 4-methyl-3-(2-((2-(4-morpholinyl)ethyl)amino)-6-quinazolinyl)-N-(3-(trifluoromethyl)phenyl)benzamide | B | 535.567 | 536 |
| 21 | 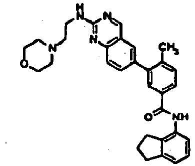 | N-(2,3-dihydro-1H-inden-4-yl)-4-methyl-3-(2-((2-(4-morpholinyl)ethyl)amino)-6-quinazolinyl)benzamide | B | 507.635 | 508 |
| 22 | 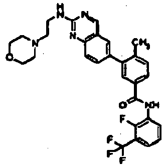 | N-(2-fluoro-3-(trifluoromethyl)phenyl)-4-methyl-3-(2-((2-(4-morpholinyl)eth-yl)amino)-6-quinazolinyl)benzamide | B | 553.557 | 554 |
| 23 | 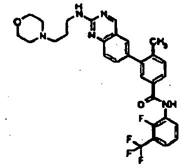 | N-(2-fluoro-3-(trifluoromethyl)phenyl)-4-methyl-3-(2-((3-(4-morpholinyl)propyl)amino)-6-quinazolinyl)benzamide | B | 567.584 | 568 |
| 24 | 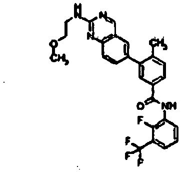 | N-(2-fluoro-3-(trifluoromethyl)phenyl)-4-methyl-3-(2-((2-(methyloxy)ethyl)amino)-6-quinazolinyl)benzamide | B | 498.478 | 499 |
| 25 | 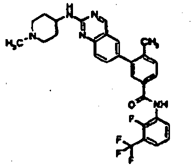 | N-(2-fluoro-3-(trifluoromethyl)phenyl)-4-methyl-3-(2-((1-methyl-4-piperidinyl)amino)-6-quinazolinyl)benzamide | B | 537.558 | 538 |
| 26 | 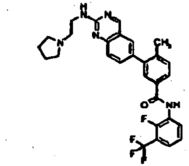 | N-(2-fluoro-3-(trifluoromethyl)phenyl)-4-methyl-3-(2-((2-(1-pyrrolidinyl)ethyl)amino)-6-quinazolinyl)benzamide | B | 537.558 | 538 |
| 27 | 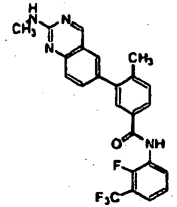 | N-(4-fluoro-3-(trifluoromethyl)phenyl)-4-methyl-3-(2-(methylamino)-6-quinazolinyl)benzamide | B | 454.425 | 455 |
| 28 | 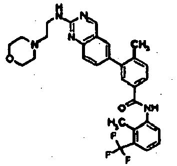 | 4-methyl-N-(2-methyl-3-(trifluoromethyl)phenyl)-3-(2-((2-(4-morpholinyl)ethyl)amino)-6-quinazolinyl)benzamide | B | 549.594 | 550 |
| 29 | 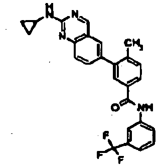 | 3-(2-(cyclopropylamino)-6-quinazolinyl)-4-methyl-N-(3-(trifluoromethyl)phenyl)benzamide | B | 462.473 | 463 |
| 30 | 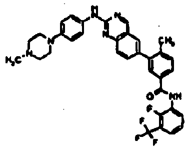 | N-(2-fluoro-3-(trifluoromethyl)phenyl)-4-methyl-3-(2-((4-(4-methyl-1-piperazinyl)phenyl)amino)-6-quinazolinyl)benzamide | B | 614.644 | 615 |
| 31 | 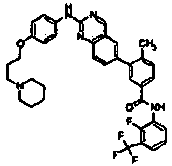 | N-(2-fluoro-3-(trifluoromethyl)phenyl)-4-methyl-3-(2-((4-((3-(1-piperidinyl)propyl)oxy)phenyl)amino)-6-quinazolinyl)benzamide | B | 657.708 | 658 |
| 32 | 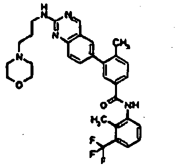 | 4-methyl-N-(2-methyl-3-(trifluoromethyl)phenyl)-3-(2-((3-(4-morpholinyl)propyl)amino)-6-quinazolinyl)benzamide | B | 563.621 | 564 |
| 33 | 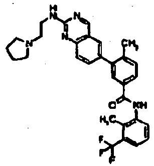 | 4-methyl-N-(2-methyl-3-(trifluoromethyl)phenyl)-3-(2-((2-(1-pyrrolidinyl)ethyl)amino)-6-quinazolinyl)benzamide | B | 533.595 | 534 |
| 34 | 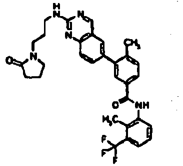 | 4-methyl-N-(2-methyl-3-(trifluoromethyl)phenyl)-3-(2-((3-(2-oxo-1-pyrrolidinyl)propyl)amino)-6-quinazolinyl)benzamide | B | 561.605 | 562 |
| 35 | 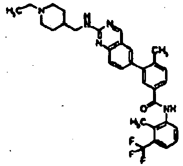 | 3-(2-(((1-ethyl-4-piperidinyl)methyl)amino)-6-quinazolinyl)-4-methyl-N-(2-methyl-3-(trifluoromethyl)phenyl)benzamide | B | 561.649 | 562 |
| 36 | 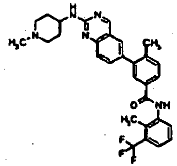 | 4-methyl-3-(2-((1-methyl-4-piperidinyl)amino)-6-quinazolinyl)-N-(2-methyl-3-(trifluoromethyl)phenyl)benzamide | B | 533.595 | 534 |
| 37 | 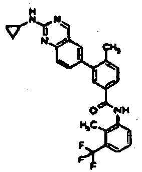 | 3-(2-(cyclopropylamino)-6-quinazolinyl)-4-methyl-N-(2-methyl-3-(trifluoromethyl)phenyl)benzamide | B | 476.5 | 477 |
| 38 | 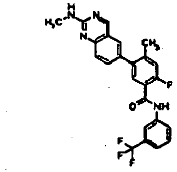 | 2-fluoro-4-methyl-5-(2-(methylamino)-6-quinazolinyl)-N-(3-(trifluoromethyl)phenyl)benzamide | B | 454.425 | 455 |
| 39 | 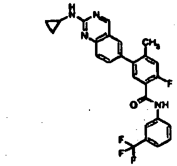 | 5-(2-(cyclopropylamino)-6-quinazolinyl)-2-fluoro-4-methyl-N-(3-(trifluoromethyl)phenyl)benzamide | B | 480.463 | 481 |
| 40 | 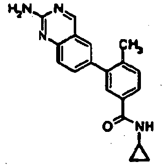 | 3-(2-amino-6-quinazolinyl)-N-cyclopropyl-4-methylbenzamide | B | 318.378 | 319.1 |
| 41 | 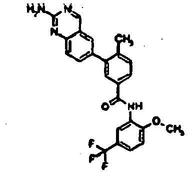 | 3-(2-amino-6-quinazolinyl)-4-methyl-N-(2-(methyloxy)-5-(trifluoromethyl)phenyl)benzamide | B | 452.434 | 453.3 |
| 42 | 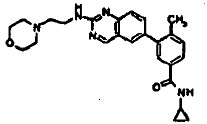 | N-cyclopropyl-4-methyl-3-(2-((2-(4-morpholinyl)ethyl)amino)-6-quinazolinyl)benzamide | B | 431.537 | 432.2 |
| 43 | 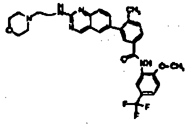 | 4-methyl-N-(2-(methyloxy)-5-(trifluoromethyl)phenyl)-3-(2-((2-(4-morpholinyl)ethyl)amino)-6-quinazolinyl)benzamide | B | 565.593 | 566.2 |
| 44 | 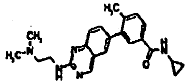 | N-cyclopropyl-3-(2-((2-(dimethylamino)ethyl)amino)-6-quinazolinyl)-4-methylbenzamide | B | 389.5 | 390.1 |
| 45 | 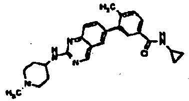 | N-cyclopropyl-4-methyl-3-(2-((1-methyl-4-piperidinyl)amino)-6-quinazolinyl)benzamide | B | 415.538 | 416.1 |
| 46 | 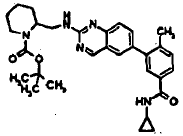 | 1,1-dimethylethyl 2-(((6-(5-((cyclopropylamino)carbonyl)-2-methylphenyl)-2-quinazolinyl)amino)methyl)-1-piperidinecarboxylate | B | 515.654 | 516.8 |
| 47 | 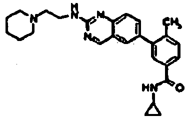 | N-cyclopropyl-4-methyl-3-(2-((2-(1-piperidinyl)ethyl)amino)-6-quinazolinyl)benzamide | B | 429.565 | 430.1 |
| 48 | 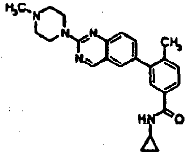 | N-cyclopropyl-4-methyl-3-(2-(4-methyl-1-piperazinyl)-6-quinazolinyl)benzamide | B | 401.511 | 402.2 |
| 49 | 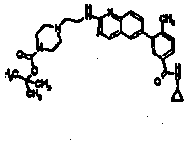 | 1,1-dimethylethyl 4-(2-((6-(5-((cyclopropylamino)carbonyl)-2-methylphenyl)-2-quinazolinyl)amino)ethyl)-1-piperazinecarboxylate | B | 530.669 | 531.3 |
| 49a | 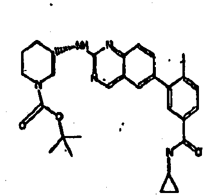 | (3S)-tert-butyl 3-(6-(5-(cyclopropylcarbamoyl)-2-methylphenyl)quinazolin-2-ylamino)piperidine-1-carboxylate | B | 501.4 | 502.1 |
| 50 | 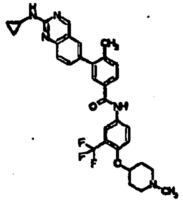 | 3-(2-(cyclopropylamino)-6-quinazolinyl)-4-methyl-N-(4-((1-methyl-4-piperidinyl)oxy)-3-(trifluoromethyl)phenyl)benzamide | B | 575.632 | 576 |

### Experimental Method C1

### Example 51 (Reference Example)

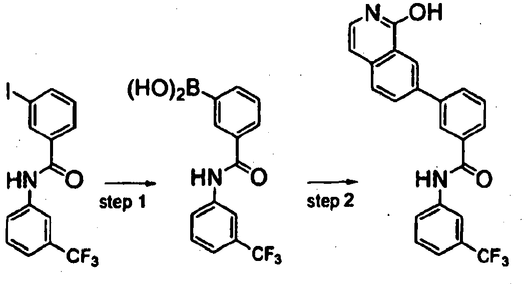

### Synthesis of 3-(1-hydroxy-7-isoquinolinyl)-N-(3-(trifluoromethyl)phenyl)benzamide

### Step 1. 3-iodo-N-(3-(trifluoromethyl)phenyl)benzamide:

The title compound was synthesized in a manner similar to that described in Example 16, step 1, to yield the title compound as a white solid. MS (ES⁺): 392 (M+H)⁺. Calc'd for C₁₄H₉F₃INO- 391.13.

### Step 2. 3-((3-(trifluoromethyl)phenyl)carbamoyl)phenylboronic acid:

To a solution of 3-iodo-N-(3-(trifluoromethyl)phenyl)benzamide (0.43 g, 1.1 mmol) in 30 mL dry THF under argon at 0 °C was added MeMgCl (3.0 M solution in THF, 1.9 mL, 5.6 mmol) slowly dropwise. The resulting light yellow solution was stirred for 45 min, and cooled to -78 °C, and t-BuLi (1.7 M solution in pentane, 3.3 mL, 5.6 mmol) was added slowly dropwise. The solution was allowed to stir for 5 minutes, and trimethoxyborane (1.2 mL, 10 mmol) was added slowly dropwise. The solution was allowed to stir for 90 min. and was then sealed and placed in a 0°C freezer overnight. The reaction was quenched by addition of 5 ml saturated aqueous sodium sulfite and 25 ml 10% aqueous sodium bisulfate. Additional saturated aqueous sodium sulfite was added until a yellow color disappeared. The aqueous material was extracted four times with EtOAc. The combined organic layers were dried with Na₂SO₄, filtered, and concentrated. Purification by silica gel chromatography (dichloromethane/methanol) provided the title compound as a yellow foamy solid. MS (ES⁺): 310.1 (M+H)⁺. Calc'd for C₁₄H₁₁BF₃NO₃: 309.05.

### Step 3. 3-(1-hydroxy-7-isoquinolinyl)-N-(3-(trifluoromethyl)phenyl)benzamide:

A mixture of 3-((3-(trifluoromethyl)phenyl)carbamoyl)phenylboronic acid (0.050 g, 0.16 mmol), 7-bromoisoquinolin-1-ol (0.036 g, 0.16 mmol), tetrakis(triphenylphosphine)palladium (0) (0.0056 g, 0.005 mmol), sodium carbonate (2.0 M solution in water, 0.16 mL, 0.32 mmol), 0.2 mL EtOH, and 1 mL toluene was heated in a sealed tube at 100°C for 16 h. The reaction was cooled to ambient temperature, and was added to EtOAc and 2.0 M aqueous sodium carbonate. The organic layer was washed once with brine, dried with Na₂SO₄, filtered, and concentrated. Purification by silica gel chromatography (dichloromethane/acetone) provided the title compound as a yellow solid. MS (ES⁺): 409.0 (M+H)⁺. Calc'd for C₂₃H₁₅F₃N₂O₂- 408.37.

The following Examples 52-53 were prepared by a method similar to that described in Experimental Method C1 and Example 51.

| Example No. | Structure | Name | Method | MW | (M+H⁺) |
|---|---|---|---|---|---|
| 52 | 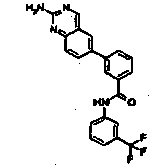 | 3-(2-amino-6-quinazolinyl)-N-(3-(trifluoromethyl)phenyl)benzamide | C1 | 408.382 | 409.1 |
| 53 | 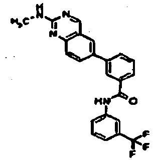 | 3-(2-(methylamino)-6-quinazolinyl)-N-(3-(trifluoromethyl)phenyl)benzamide | C1 | 422.408 | 423.1 |

### Experimental Method C2

### Example 54

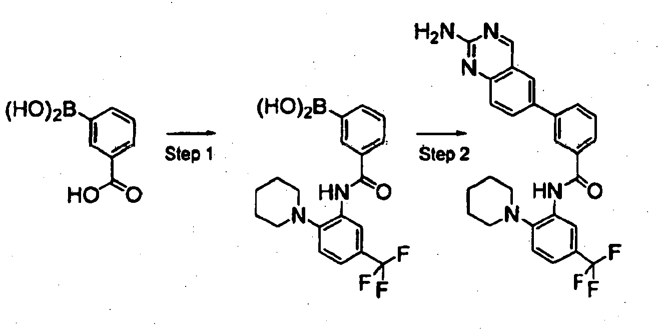

### Synthesis of 3-(2-amino-6-quinazolinyl)-N-(2-(1-piperidinyl)-5-(trifluoromethyl)phenyl)benzamide

### Step 1. 3-((2-(piperidin-1-yl)-5-(trifluoromethyl)phenyl)carbamoyl)phenylboronic acid:

A solution of 3-boronobenzoic acid (0.12 g, 0.72 mmol), 2-(piperidin-1-yl)-5-(trifluoromethyl)benzenamine (0.19 g, 0.80 mmol), EDC (0.15 g, 0.80 mmol), HOBT (0.11 g, 0.80 mmol) in 2.4 mL DMF was heated to 80 °C with a water-cooled reflux condensor for 17 h. The reaction was cooled to ambient temperature and the solvent was removed under reduced pressure. The residue was partitioned between 1N HCl and EtOAc. The organic layer was washed once with 1N HCl and brine, dried with Na₂SO₄, filtered, and concentrated. Purification by silica gel chromatography (dichloromethane/methanol) provided the title compound as an off-white powder. MS (ES⁺) : 393.2 (M+H)⁺. Calc'd for C₁₉H₂₀BF₃N₂O₃- 392.18.

### Step 2. 3-(2-amino-6-quinazolinyl)-N-(2-(1-piperidinyl)-5-(trifluoromethyl)phenyl)benzamide:

Synthesized in a manner similar to Example 50, Step 3 to yield the title compound as a light yellow solid. MS (ES⁺) : 492.2 (M+H)⁺. Calc'd for C₂₇H₂₄F₃N₅O- 491.51.

### Experimental Method C3

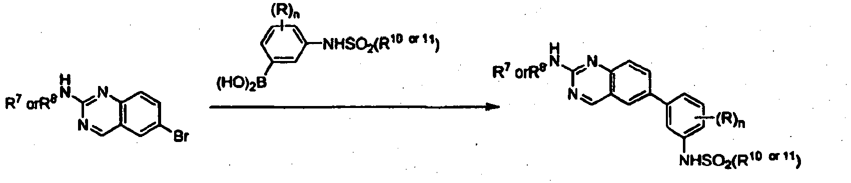

### Example 55

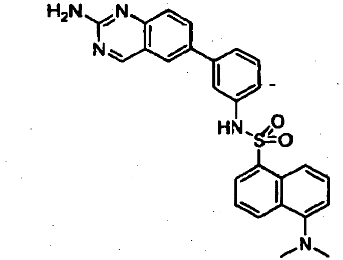

### N-(3-(2-amino-6-quinazolinyl)phenyl)-5-(dimethylamino)-1-naphthalenesulfonamide

Sodium carbonate (2 M in water, 1.1 mL, 2.19 mmol) was added to a solution of 6-bromoquinazolin-2-amine (0.164 g, 0.73 mmol) and 3-(5-dimethylaminonaphthalene-1-sulfonylamino)benzeneboronic acid (0.285 g, 0.77 mmol) in toluene (20 mL) and ethanol (4 mL). Tetrakis(triphenylphosphine)palladium (0) (0.046 g, 0.04 mmol) was added and the mixture was heated overnight at 80 °C under a nitrogen atmosphere. The reaction mixture was cooled to room temperature and concentrated. The crude material was purified via column chromatography on silica gel (gradient elution with 5-100% (90:10:1, dichloromethane/methanol/ammonium hydroxide)-dichloromethane) to afford N-(3-(2-amino-6-quinazolinyl)phenyl)-5-(dimethylamino)-1-naphthalenesulfonamide as a yellow solid. MS (MH⁺) 470.1; Cal'd for C₂₆H₂₃N₅O₂S - 469

### Experimental Method D1

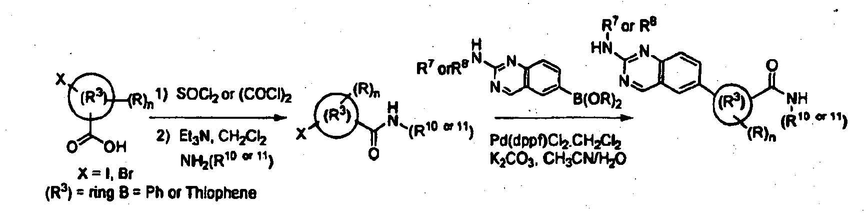

### Example 56

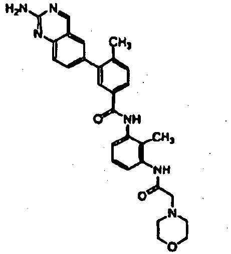

### Synthesis of 3-(2-Amino-6-quinazolinyl)-4-methyl-N-(2-methyl-3-((4-morpholinylacetyl)amino)phenyl)benzamide

### Step 1: 3-Iodo-4-methyl-N-(2-methyl-3-(2-morpholinoacetamido)phenyl)benzamide

A resealable tube was charged with 3-iodo-4-methylbenzoic acid (0.4 g 1.5 mmol) and thionyl chloride (4.0 ml, 46.2 mmol). The vessel was purged with argon and sealed. The mixture was heated at 80°C for 1 hour. The reaction was cooled and then concentrated to a brown solid. The solid was dissolved in 6 ml of CH₂Cl₂, and added to a solution of the N-(3-amino-2-methylphenyl)-2-morpholinoacetamide (0.41 g, 1.6 mmol) in 4 ml of CH₂Cl₂. To this was then added triethylamine (0.62 ml, 0.46 g, 4.5 mmol). The reaction was stirred at room temperature for 16 hours. The resulting precipitate was filtered, washed with CH₂Cl₂ and dried under high vacuum to afford 3-iodo-4-methyl-N-(2-methyl-3-(2-morpholinoacetamido)phenyl)benzamide as a white solid. MS m/z = 494.0 [M+H]⁺; Calc'd for C₂₁H₂₄IN₃O₃: 493.

### Step 2: 3-(2-Amino-6-quinazolinyl)-4-methyl-N-(2-methyl-3-((4-morpholinylacetyl)amino)phenyl)benzamide

To a suspension of 3-iodo-4-methyl-N-(2-methyl-3-(2-morpholinoacetamido)phenyl)benzamide (0.30g, 0.6 mmol) in 4 ml of CH₃CN and 4 ml of H₂O was added 6- (4, 4, 5, 5-tetramethyl-1,3,2-dioxoborolan-2-yl)quinazolin-2-amine (0.18 g, 0.7 mmol), potassium carbonate (0.42 g, 3.0 mmol) and dichloro[1,1'-bis(diphenylphosphino)ferrocene]palladium (II) dichloromethane adduct (0.044 g, 0.1 mmol). The reaction was stirred at 45°C for 2 hours and then at room temperature for 16 hours. The reaction mixture was partitioned between EtOAc and H₂O. The resulting emulsion was filtered through a scintered glass funnel, and the layers were separated. The aqueous layer was extracted with EtOAc, and the combined organic layers were washed with saturated NH₄Cl, H₂O and brine, and then dried over MgSO₄. The solvent was evaporated, and the residue was dissolved in a minimal amount of CH₂Cl₂ and the product was triturated with MeOH. The solid was filtered, washed with MeOH and dried to afford 3-(2-Amino-6-quinazolinyl)-4-methyl-N-(2-methyl-3-((4-morpholinylacetyl)amino)phenyl)benzamide as a tan solid. MS m/z = 511.2 [M+H]⁺; Calc'd for C₂₉H₃₀N₆O₃: 510.

### Example 57

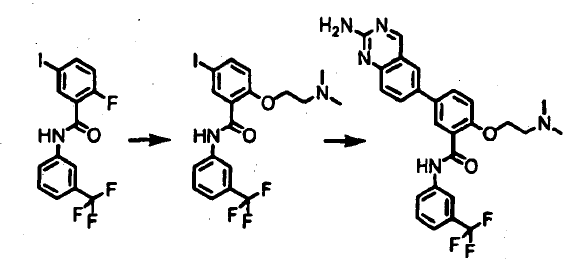

### Synthesis of 5-(2-amino-6-quinazolinyl)-2-((2-(dimethylamino)ethyl)oxy)-N-(3-(trifluoromethyl)phenyl)benzamide

### Step 1: 2-fluoro-5-iodo-N-(3-(trifluoromethyl)phenyl)benzamide

The title compound was synthesized in a manner similar to the method described in Example 161, step 1 (Experimental Method D2 - below) yielding 2-fluoro-5-iodo-N-(3-(trifluoromethyl)phenyl)benzamide as a white solid. MS (ES⁺): 408 (M-H)⁻. Calc'd for C₁₄H₈F₄INO- 409.12

### Step 2: 2-(2-(dimethylamino)ethoxy)-5-iodo-N-(3-(trifluoromethyl)phenyl)benzamide

To a mixture of NaH (60% in mineral oil, 0.022 g, 0.54 mmol) in DMF was added N,N-dimethylethanolamine (0.24 mL, 2.4 mmol) followed by 2-fluoro-5-iodo-N-(3-(trifluoromethyl)phenyl)benzamide (0.20 g, 0.49 mmol). The reaction was sealed and heated to 100 °C for 12 h. The reaction was cooled to ambient temperature, and water was added to give a precipitate. Filtration provided the title compound as a white solid. MS (ES⁺): 479 (M+H)⁺. Calc'd for C₁₈H₁₈IF₃N₂O₂- 478.25.

### Step 3: 5-(2-amino-6-quinazolinyl)-2-((2-(dimethylamino)ethyl)oxy)-N-(3-(trifluoromethyl)phenyl)benzamide

The title compound was prepared by a method similar to that described in Example 56 using bis(diphenylphosphino)ferrocene]palladium (II) dichloromethane adduct and aqueous sodium carbonate in dioxane, and afforded the title compound as an off-white solid. MS (ES⁺) : 496.2 (M+H)⁺. Calc'd for C₂₆H₂₂F₃N₅O₂- 495.50.

### Example 58

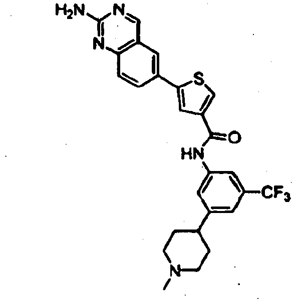

### Synthesis of 5-(2-amino-6-quinazolinyl)-N-(3-(1-methyl-4-piperidinyl)-5-(trifluoromethyl)phenyl)-3-thiophenecarboxamide

The title compound was prepared from 5-bromo-3-thiophenecarboxylic acid (prepared by a method similar to that described in "Substitution reactions of 3-thenoic acid" by Campaigne, E. E.; Bourgeois, R. C., J. Am. Chem. Soc. 76, 2445-2447, 1954) and 3-(1-methylpiperidin-4-yl)-5-(trifluoromethyl)benzenamine by a method similar to that described in Experimental Method D1 and Example 56. MS *m*/*z* = 512.2 [M+H]⁺. Calc'd for C₂₆H₂₄F₃N₅OS: 511.56.

The following Examples 59-160 were prepared by a method similar to that described in Experimental Method D1 and Examples 56, 57 and 58.

| Example No. | Structure | Name | Method | MW | (M+H⁺) |
|---|---|---|---|---|---|
| 59 | 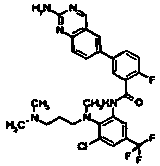 | 5-(2-amino-6-quinazolinyl)-N-(3-chloro-2-((3-(dimethylamino)propyl)(methyl)amino)-5-(trifluoromethyl)phenyl)-2-fluorobenzamide | D1 | 575.007 | 575.2 |
| 60 | 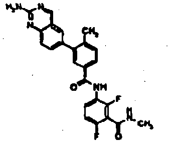 | 3-(((3-(2-amino-6-quinazolinyl)-4-methylphenyl)carbonyl)amino)-2,6-difluoro-N-methylbenzamide | D1 | 447.443 | 448.1 |
| 61 | 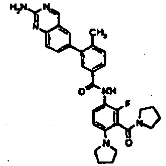 | 3-(2-amino-6-quinazolinyl)-N-(2-fluoro-4-(1-pyrrolidinyl)-3-(1-pyrrolidinylcarbonyl)phenyl)-4-methylbenzamide | D1 | 538.624 | 539.2 |
| 62 | 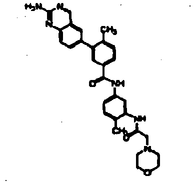 | 3-(2-amino-6-quinazolinyl)-4-methyl-N-(4-methyl-3-((4-morpholinylacetyl)amino)phenyl)benzamide | D1 | 510.595 | 511.2 |
| 63 | 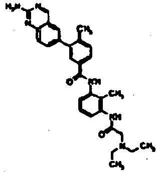 | 3-(2-amino-6-quinazolinyl)-N-(3-((N,N-diethylglycyl)amino)-2-methylphenyl)-4-methylbenzamide | D1 | 496.612 | 497.2 |
| 64 | 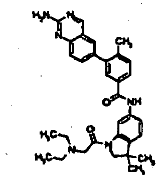 | 3-(2-amino-6-quinazolinyl)-N-(1-(N,N-diethylglycyl)-3,3-dimethyl-2,3-dihydro-1H-indol-6-yl)-4-methylbenzamide | D1 | 536.676 | 537.3 |
| 65 | 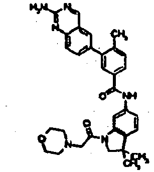 | 3-(2-amino-6-quinazolinyl)-N-(3,3-dimethyl-1-(4-morpholinylacetyl)-2,3-dihydro-1H-indol-6-yl)-4-methylbenzamide | D1 | 550.66 | 551.2 |
| 66 | 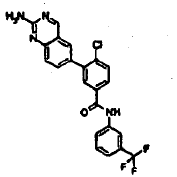 | 3-(2-amino-6-quinazolinyl)-4-chloro-N-(3-(trifluoromethyl)phenyl)benzamide | D1 | 442.827 | 443.4 |
| 67 | 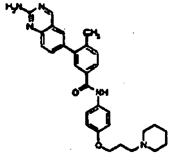 | 3-(2-amino-6-quinazolinyl)-4-methyl-N-(4-((3-(1-piperidinyl)propyl)oxy)phenyl)benzamide | D1 | 495.624 | 496.4 |
| 68 | 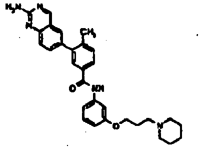 | 3-(2-amino-6-quinazolinyl)-4-methyl-N-(3-((3-(1-piperidinyl)propyl)oxy)phenyl)benzamide | D1 | 495.624 | 496.4 |
| 69 | 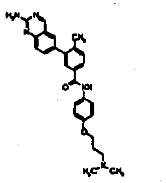 | 3-(2-amino-6-quinazolinyl)-N-(4-((3-(dimethylamino)propyl)oxy)phenyl)-4-methylbenzamide | D1 | 455.559 | 456.4 |
| 70 | 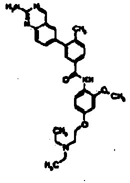 | 3-(2-amino-6-quinazolinyl)-N-(4-((2-(diethylamino)ethyl)oxy)-2-(methyloxy)phenyl)-4-methylbenzamide | D1 | 499.612 | 500.4 |
| 71 | 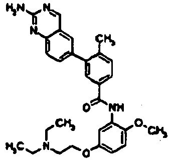 | 3-(2-amino-6-quinazolinyl)-N-(5-((2-(diethylamino)ethyl)oxy)-2-(methyloxy)phenyl)-4-methylbenzamide | D1 | 499.612 | 500.4 |
| 72 | 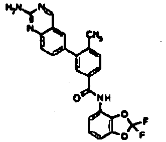 | 3-(2-amino-6-quinazolinyl)-N-(2,2-difluoro-1,3-benzodioxol-4-yl)-4-methylbenzamide | D1 | 434.4 | 435.1 |
| 73 | 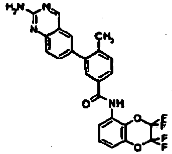 | 3-(2-amino-6-quinazolinyl)-4-methyl-N-(2,2,3,3-tetrafluoro-2,3-dihydro-1,4-benzodioxin-5-yl)benzamide | D1 | 484.407 | 485.2 |
| 74 | 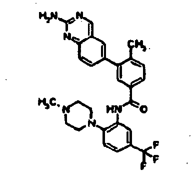 | 3-(2-amino-6-quinazolinyl)-4-methyl-N-(2-(4-methyl-1-piperazinyl)-5-(trifluoromethyl)phenyl)benzamide | D1 | 520.556 | 521 |
| 75 | 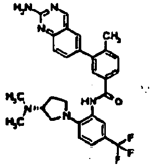 | 3-(2-amino-6-quinazolinyl)-N-(2-((3R)-3-(dimethylamino)-1-pyrrolidinyl)-5-(trifluoromethyl)phenyl)-4-methylbenzamide | D1 | 534.583 | 535 |
| 76 | 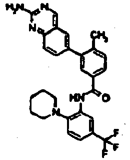 | 3-(2-amino-6-quinazolinyl)-4-methyl-N-(2-(1-piperidinyl)-5-(trifluoromethyl)phenyl)benzamide | D1 | 505.541 | 506 |
| 77 | 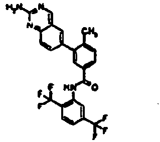 | 3-(2-amino-6-quinazolinyl)-N-(2,5-bis(trifluoromethyl)phenyl)-4-methylbenzamide | D1 | 490.405 | 490.9 |
| 78 | 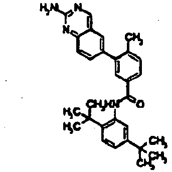 | 3-(2-amino-6-quinazolinyl)-N-(2,5-bis(1,1-dimethylethyl)phenyl)-4-methylbenzamide | D1 | 466.626 | 467.3 |
| 79 | 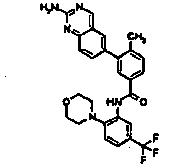 | 3-(2-amino-quinazolinyl)-4-methyl-N-(2-(4-morpholinyl)-5-(trifluoromethyl)phenyl)benzamide | D1 | 507.514 | 508 |
| 80 | 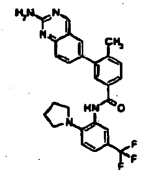 | 3-(2-amino-6-quinazolinyl)-4-methyl-N-(2-(1-pyrrolidinyl)-5-(trifluoromethyl)phenyl)benzamide | D1 | 491.515 | 492 |
| 81 | 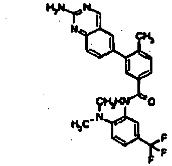 | 3-(2-amino-6-quinazolinyl)-N-(2-(dimethylamino)-5-(trifluoromethyl)phenyl)-4-methylbenzamide | D1 | 465.477 | 466 |
| 82 | 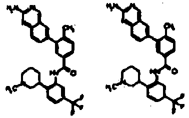 | 3-(2-amino-6-quinazolinyl)-4-methyl-N-(2-((3R)-1-methyl-3-piperidinyl)-5-(trifluoromethyl)phenyl)benzamide | D1 | 519.568 | 520 |
| 83 | 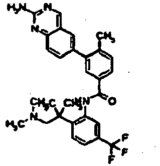 | 3-(2-amino-6-quinazolinyl)-N-(2-(2-(dimethylamino)-1,1-dimethylethyl)-5-(trifluoromethyl)phenyl)-4-methylbenzamide | D1 | 521.584 | 522.2 |
| 84 | 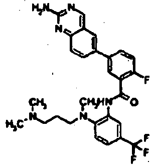 | 5-(2-amino-6-quinazolinyl)-N-(2-((3-(dimethylamino)propyl)(methyl)amino)-5-(trifluoromethyl)phenyl)-2-fluorobenzamide | D1 | 540.562 | 541.2 |
| 85 | 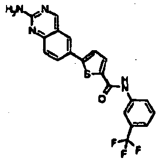 | 5-(2-amino-6-quinazolinyl)-N-(3-(trifluoromethyl)phenyl)-2-thiophenecarboxamide | D1 | 414.41 | 415 |
| 86 | 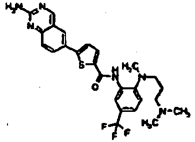 | 5-(2-amino-6-quinazolinyl)-N-(2-((3-(dimethylamino)propyl)(methyl)amino)-5-(trifluoromethyl)phenyl)-2-thiophenecarboxamide | D1 | 528.6 | 529.2 |
| 87 | 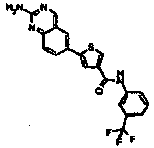 | 5-(2-amino-6-quinazolinyl)-N-(3-(trifluoromethyl)phenyl)-3-thiophenecarboxamide | D1 | 414.41 | 415 |
| 88 | 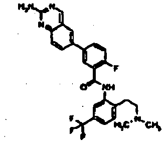 | 5-(2-amino-6-quinazolinyl)-N-(2-(2-(dimethylamino)ethyl)-5-(trifluoromethyl)phenyl)-2-fluorobenzamide | D1 | 497.494 | 498.2 |
| 89 | 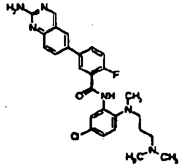 | 5-(2-amino-6-quinazolinyl)-N-(5-chloro-2-((3-(dimethylamino)propyl)(methyl)amino)phenyl)-2-fluorobenzamide | D1 | 507.01 | 507.2 |
| 90 | 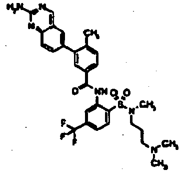 | 3-(2-amino-6-quinazolinyl)-N-(2-(((3-(dimethylamino)propyl)(methyl)amino)sulfonyl)-5-(trifluoromethyl)phenyl)-4-methylbenzamide | D1 | 600.663 | 601.2 |
| 91 | 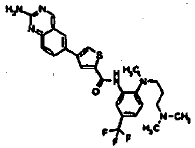 | 4-(2-amino-6-quinazolinyl)-N-(2-((3-(dimethylamino)propyl)(methyl)amino)-5-(trifluoromethyl)phenyl)-2-thiophenecarboxamide | D1 | 528.6 | 529.2 |
| 92 | 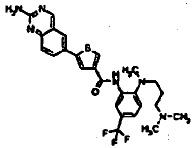 | 5-(2-amino-6-quinazolinyl)-N-(2-((3-(dimethylamino)propyl)(methyl)amino)-5-(trifluoromethyl)phenyl)-3-thiophenecarboxamide | D1 | 528.6 | 529.2 |
| 93 | 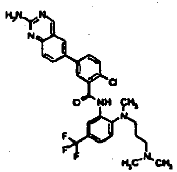 | 5-(2-amino-6-quinazolinyl)-2-chloro-N-(2-((3-(dimethylamino)propyl)(methyl)amino)-5-(trifluoromethyl)phenyl)benzamide | D1 | 557.017 | 557.2 |
| 94 | 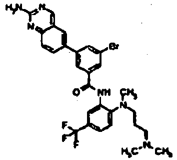 | 3-(2-amino-6-quinazolinyl)-5-bromo-N-(2-((3-(dimethylamino)propyl)(methyl)amino)-5-(trifluoromethyl)phenyl)benzamide | D1 | 601.468 | 603.1 |
| 95 | 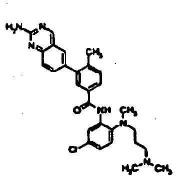 | 3-(2-amino-6-quinazolinyl)-N-(5-chloro-2-((3-(dimethylamino)propyl)(methyl)amino)phenyl)-4-methylbenzamide | D1 | 503.047 | 503.2 |
| 96 | 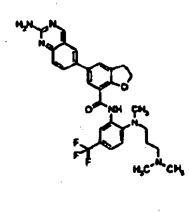 | 5-(2-amino-6-quinazolinyl)-N-(2-((3-(dimethylamino)propyl)(methyl)amino)-5-(trifluoromethyl)phenyl)-2,3-dihydro-1-benzofuran-7-carboxamide | D1 | 564.609 | 565.2 |
| 97 | 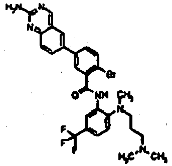 | 5-(2-amino-6-quinazolinyl)-2-bromo-N-(2-((3-(dimethylamino)propyl)(methyl)amino)-5-(trifluoromethyl)phenyl)benzamide | D1 | 601.468 | 603.1 |
| 98 | 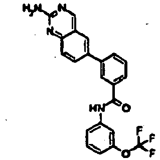 | 3-(2-amino-6-quinazolinyl)-N-(3-((trifluoromethyl)oxy)phenyl)benzamide | D1 | 424.38 | 425.1 |
| 99 | 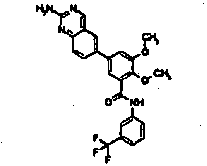 | 5-(2-amino-6-quinazolinyl)-2,3-bis(methyloxy)-N-(3-(trifluoromethyl)phenyl)benzamide | D1 | 468.433 | 469.1 |
| 100 | 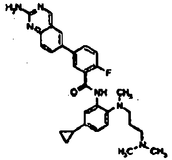 | 5-(2-amino-6-quinazolinyl)-N-(5-cyclopropyl-2-((3-(dimethylamino)propyl)(methyl)amino)phenyl)-2-fluorobenzamide | D1 | 512.63 | 513.2 |
| 101 | 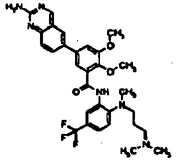 | 5-(2-amino-6-quinazolinyl)-N-(2-((3-(dimethylamino)propyl)(methyl)amino)-5-(trifluoromethyl)phenyl)-2,3-bis(methyloxy)benzamide | D1 | 582.624 | 583.2 |
| 102 | 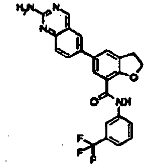 | 5-(2-amino-6-quinazolinyl)-N-(3-(trifluoromethyl)phenyl)-2,3-dihydro-1-benzofuran-7-carboxamide | D1 | 450.418 | 451.1 |
| 103 | 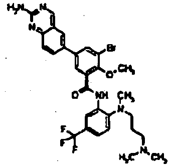 | 5-(2-amino-quinazolinyl)-3-bromo-N-(2-((3-(dimethylamino)propyl)(methyl)amino)-5-(trifluoromethyl)phenyl)-2-(methyloxy)benzamide | D1 | 631.494 | 633.1 |
| 104 | 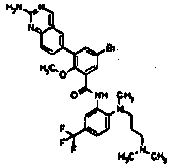 | 3-(2-amino-6-quinazolinyl)-5-bromo-N-(2-((3-(dimethylamino)propyl)(methyl)amino)-5-(trifluoromethyl)phenyl)-2-(methyloxy)benzamide | D1 | 631.494 | 631.1 |
| 105 | 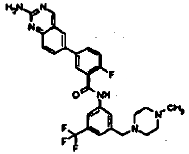 | 5-(2-amino-6-quinazolinyl)-2-fluoro-N-(3-((4-methyl-1-piperazinyl)methyl)-5-(trifluoromethyl)phenyl)benzamide | D1 | 538.546 | 539.2 |
| 106 | 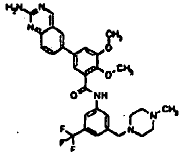 | 5-(2-amino-6-quinazolinyl)-2,3-bis(methyloxy)-N-(3-((4-methyl-1-piperazinyl)methyl)-5-(trifluoromethyl)phenyl)benzamide | D1 | 580.608 | 581.2 |
| 107 | 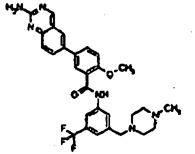 | 5-(2-amino-6-quinazolinyl)-2-(methyloxy)-N-(3-((4-methyl-1-piperazinyl)methyl)-5-(trifluoromethyl)phenyl)benzamide | D1 | 550.582 | 551.2 |
| 108 | 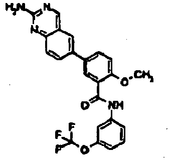 | 5-(2-amino-6-quinazolinyl)-2-(methyloxy)-N-(3-((trifluoromethyl)oxy)phenyl)benzamide | D1 | 454.406 | 455.1 |
| 109 | 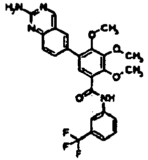 | 5-(2-amino-6-quinazolinyl)-2,3,4-tris(methyloxy)-N-(3-(trifluoromethyl)phenyl)benzamide | D1 | 498.459 | 499.1 |
| 110 | 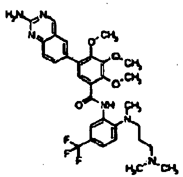 | 5-(2-amino-6-quinazolinyl)-N-(2-((3-(dimethylamino)propyl)(methyl)amino)-5-(trifluoromethyl)phenyl)-2,3,4-tris(methyloxy)benzamide | D1 | 612.649 | 613.2 |
| 111 | 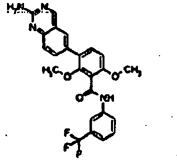 | 3-(2-amino-6-quinazolinyl)-2,6-bis(methyloxy)-N-(3-(trifluoromethyl)phenyl)benzamide | D1 | 468.433 | 469.1 |
| 112 | 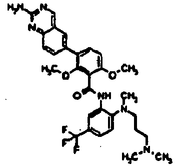 | 3-(2-amino-6-quinazolinyl)-N-(2-((3-(dimethylamino)propyl)(methyl)amino)-5-(trifluoromethyl)phenyl)-2,6-bis(methyloxy)benzamide | D1 | 582.624 | 583.2 |
| 113 | 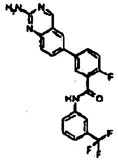 | 5-(2-amino-6-quinazolinyl)-2-fluoro-N-(3-(trifluoromethyl)phenyl)benzamide | D1 | 426.372 | 427 |
| 114 | 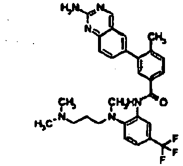 | 3-(2-amino-6-quinazolinyl)-N-(2-((3-(dimethylamino)propyl)(methyl)amino)-5-(trifluoromethyl)phenyl)-4-methylbenzamide | D1 | 536.599 | 537 |
| 115 | 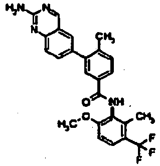 | 3-(2-amino-6-quinazolinyl)-4-methyl-N-(2-methyl-6-(methyloxy)-3-(trifluoromethyl)phenyl)benzamide | D1 | 466.461 | 467 |
| 116 | 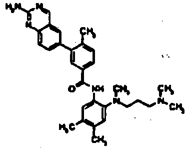 | 3-(2-amino-6-quinazolinyl)-N-(2-((3-(dimethylamino)propyl)(methyl)amino)-4,5-dimethylphenyl)-4-methylbenzamide | D1 | 496.655 | 497 |
| 117 | 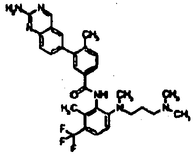 | 3-(2-amino-6-quinazolinyl)-N-(6-((3-(dimethylamino)propyl)(methyl)amino)-2-methyl-3-(trifluoromethyl)phenyl)-4-methylbenzamide | D1 | 550.626 | 551 |
| 118 | 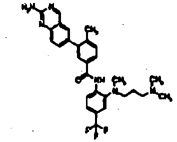 | 3-(2-amino-6-quinazolinyl)-N-(2-((3-(dimethylamino)propyl)(methyl)amino)-4-(trifluoromethyl)phenyl)-4-methylbenzamide | D1 | 536.599 | 537 |
| 119 | 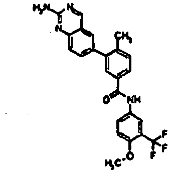 | 3-(2-amino-6-quinazolinyl)-4-methyl-N-(4-(methyloxy)-3-(trifluoromethyl)phenyl)benzamide | D1 | 452.434 | 453 |
| 120 | 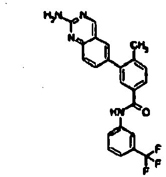 | 3-(2-amino-6-quinazolinyl)-4-methyl-N-(3-(trifluoromethyl)phenyl)benzamide | D1 | 422.408 | 423 |
| 121 | 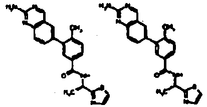 | 3-(2-amino-6-quinazolinyl)-4-methyl-N-((1S)-1-(1,3-thiazol-2-yl)ethyl)benzamide | D1 | 389.481 | 390 |
| 122 | 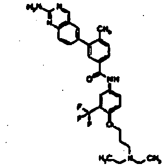 | 3-(2-amino-6-quinazolinyl)-N-(4-((3-(diethylamino)propyl)oxy)-3-(trifluoromethyl)phenyl)-4-methylbenzamide | D1 | 551.61 | 552 |
| 123 | 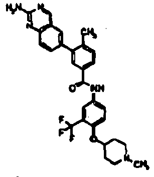 | 3-(2-amino-6-quinazolinyl)-4-methyl-N-(4-((1-methyl-4-piperidinyl)oxy)-3-(trifluoromethyl)phenyl)benzamide | D1 | 535.567 | 536 |
| 124 | 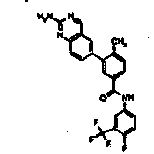 | 3-(2-amino-6-quinazolinyl)-N-(4-fluoro-3-(trifluoromethyl)phenyl)-4-methylbenzamide | D1 | 440.398 | 441 |
| 125 | 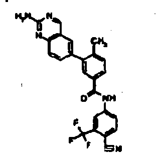 | 3-(2-amino-6-quinazolinyl)-N-(4-cyano-3-(trifluoromethyl)phenyl)-4-methylbenzamide | D1 | 447.418 | 448 |
| 126 | 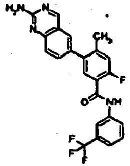 | 5-(2-amino-6-quinazolinyl)-2-fluoro-4-methyl-N-(3-(trifluoromethyl)phenyl)benzamide | D1 | 440.398 | 441 |
| 127 | 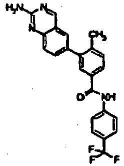 | 3-(2-amino-6-quinazolinyl)-4-methyl-N-(4-(trifluoromethyl)phenyl)benzamide | D1 | 422.408 | 423 |
| 128 | 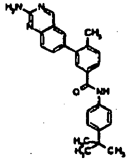 | 3-(2-amino-6-quinazolinyl)-N-(4-(1,1-dimethylethyl)phenyl)-4-methylbenzamide | D1 | 410.518 | 411 |
| 129 | 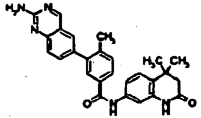 | 3-(2-amino-6-quinazolinyl)-N-(4,4-dimethyl-2-oxo-1,2,3,4-tetrahydro-7-quinolinyl)-4-methylbenzamide | D1 | 451.527 | 452 |
| 130 | 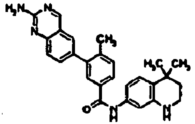 | 3-(2-amino-quinazolinyl)-N-(4,4-dimethyl-1,2,3,4-tetrahydro-7-quinolinyl)-4-methylbenzamide | D1 | 437.544 | 438 |
| 131 | 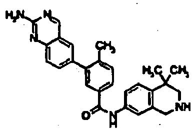 | 3-(2-amino-6-quinazolinyl)-N-(4,4-dimethyl-1,2,3,4-tetrahydro-7-isoquinolinyl)-4-methylbenzamide | D1 | 437.544 | 438 |
| 132 | 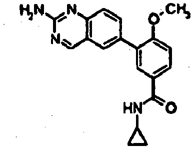 | 3-(2-amino-6-quinazolinyl)-N-cyclopropyl-4-(methyloxy)benzamide | D1 | 334.377 | 335.2 |
| 133 | 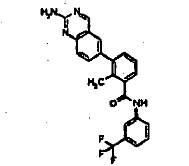 | 3-(2-amino-6-quinazolinyl)-2-methyl-N-(3-(trifluoromethyl)phenyl)benzamide | D1 | 422.408 | 423.1 |
| 134 | 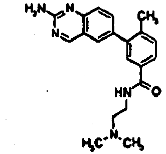 | 3-(2-amino-6-quinazolinyl)-N-(2-(dimethylamino)ethyl)-4-methylbenzamide | D1 | 349.436 | 350.1 |
| 135 | 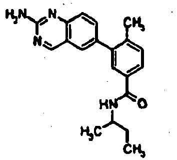 | 3-(2-amino-6-quinazolinyl)-4-methyl-N-(1-methylpropyl)benzamide | D1 | 334.421 | 335.2 |
| 136 | 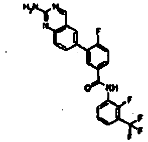 | 3-(2-amino-6-quinazolinyl)-4-fluoro-N-(2-fluoro-3-(trifluoromethyl)phenyl)benzamide | D1 | 444.362 | 445.1 |
| 137 | 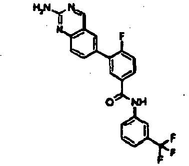 | 3-(2-amino-6-quinazolinyl)-4-fluoro-N-(3-(trifluoromethyl)phenyl)benzamide | D1 | 426.372 | 427.1 |
| 138 | 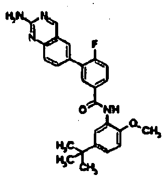 | 3-(2-amino-6-quinazolinyl)-N-(5-(1,1-dimethylethyl)-2-(methyloxy)phenyl)-4-fluorobenzamide | D1 | 444.508 | 445.1 |
| 139 | 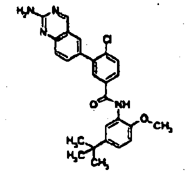 | 3-(2-amino-6-quinazolinyl)-4-chloro-N-(5-(1'1dimethylethyl)-2-(methyloxy)phenyl)benzamide | D1 | 460.962 | 461.1 |
| 140 | 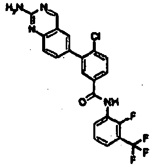 | 3-(2-amino-6-quinazolinyl)-4-chloro-N-(2-fluoro-3-(trifluoromethyl)phenyl)benzamide | D1 | 460.817 | 461.0 |
| 141 | 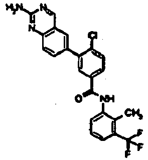 | 3-(2-amino-6-quinazolinyl)-4-chloro-N-(2-methyl-3-(trifluoromethyl)phenyl)benzamide | D1 | 456.853 | 457.1 |
| 142 | 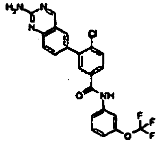 | 3-(2-amino-6-quinazolinyl)-4-chloro-N-(3-((trifluoromethyl)oxy)phenyl)benzamide | D1 | 458.826 | 459.1 |
| 143 | 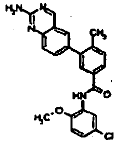 | 3-(2-amino-6-quinazolinyl)-N-(5-chloro-2-(methyloxy)phenyl)-4-methylbenzamide | D1 | 418.882 | 419 |
| 144 | 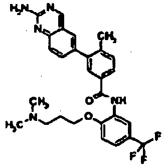 | 3-(2-amino-6-quinazolinyl)-N-(2-((3-(dimethylamino)propyl)oxy)-5-(trifluoromethyl)phenyl)-4-methylbenzamide | D1 | 523.556 | 522 (M-H⁺) |
| 145 | 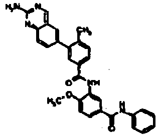 | 3-(2-amino-6-quinazolinyl)-4-methyl-N-(2-(methyloxy)-5-((phenylamino)carbonyl)phenyl)benzamide | D1 | 503.56 | 502 (M-H⁺) |
| 146 | 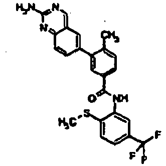 | 3-(2-amino-6-quinazolinyl)-4-methyl-N-(2-(methylsulfanyl)-5-(trifluoromethyl)phenyl)benzamide | D1 | 468.501 | 467 (M-H⁺) |
| 147 | 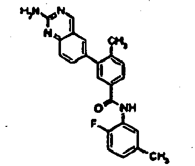 | 3-(2-amino-6-quinazolinyl)-N-(2-fluoro-5-methylphenyl)-4-methylbenzamide | D1 | 386.428 | 387 |
| 148 | 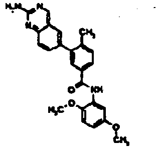 | 3-(2-amino-quinazolinyl)-N-(2,5-bis(methyloxy)phenyl)-4-methylbenzamide | D1 | 414.463 | 415 |
| 149 | 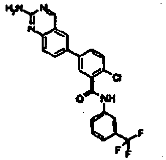 | 5-(2-amino-6-quinazolinyl)-2-chloro-N-(3-(trifluoromethyl)phenyl)benzamide | D1 | 442.827 | 443 |
| 150 | 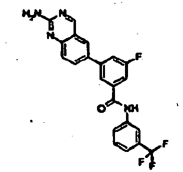 | 3-(2-amino-6-quinazolinyl)-5-fluoro-N-(3-(trifluoromethyl)phenyl)benzamide | D1 | 426.372 | 427 |
| 151 | 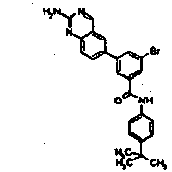 | 3-(2-amino-6-quinazolinyl)-5-bromo-N-(4-(1,1-dimethylethyl)phenyl)benzamide | D1 | 475.388 | 476 |
| 152 | 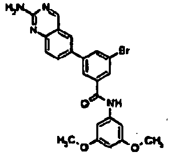 | 3-(2-amino-6-quinazolinyl)-N-(3,5-bis(methyloxy)phenyl)-5-bromobenzamide | D1 | 479.332 | 480 |
| 153 | 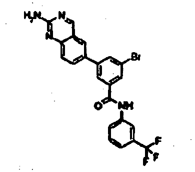 | 3-(2-amino-6-quinazolinyl)-5-bromo-N-(3-(trifluoromethyl)phenyl)benzamide | D1 | 487.278 | 489 |
| 154 | 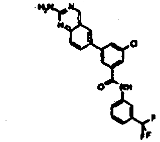 | 3-(2-amino-6-quinazolinyl)-5-chloro-N-(3-(trifluoromethyl)phenyl)benzamide | D1 | 442.827 | 443 |
| 155 | 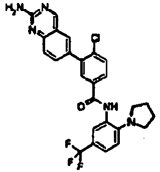 | 3-(2-amino-6-quinazolinyl)-4-chloro-N-(2-(1-pyrrolidinyl)-5-(trifluoromethyl)phenyl)benzamide | D1 | 511.933 | 512 |
| 156 | 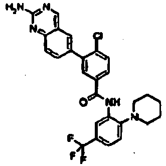 | 3-(2-amino-6-quinazolinyl)-4-chloro-N-(2-(1-piperidinyl)-5-(trifluoromethyl)phenyl)benzamide | D1 | 525.96 | 526 |
| 157 | 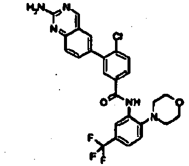 | 3-(2-amino-6-quinazolinyl)-4-chloro-N-(2-(4-morpholinyl)-5-(trifluoromethyl)phenyl)benzamide | D1 | 527.932 | 528 |
| 158 | 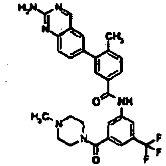 | 3-(2-amino-6-quinazolinyl)-4-methyl-N-(3-((4-methyl-1-piperazinyl)carbonyl)-5-(trifluoromethyl)phenyl)benzamide | D1 | 548.566 | 549.2 |
| 159 | 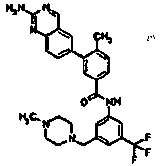 | 3-(2-amino-6-quinazolinyl)-4-methyl-N-(3-((4-methyl-1-piperazinyl)methyl)-5-(trifluoromethyl)phenyl)benzamide | D1 | 534.583 | 535.2 |
| 160 | 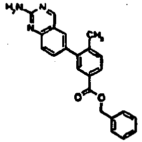 | phenylmethyl 3-(2-amino-6-quinazolinyl)-4-methylbenzoate | D-1 | 369.422 | 370.2 |

### Experimental Method D2

### Example 161

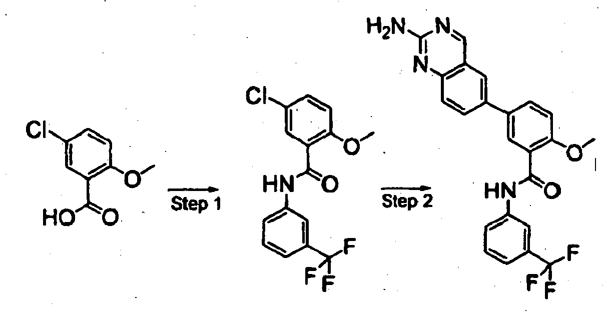

### Synthesis of 5-(2-amino-6-quinazolinyl)-2-(methyloxy)-N-(3-(trifluoromethyl)phenyl)benzamide

### Step 1. 5-chloro-2-methoxy-N-(3-(trifluoromethyl)phenyl)benzamide:

To a solution of 4-chloro-2-methoxybenzoic acid (2.5 g, 13 mmol) in 20 ml dichloromethane at 0 °C was added 2 drops of DMF followed by oxalyl chloride (1.5 ml, 17.5 mmol). After 4 h, the light yellow solution was concentrated in vacuo to give a light yellow solid. A portion of this material was treated with 3-(trifluoromethyl)benzenamine (0.28 ml, 2.25 mmol) in 1 ml dry THF. A thick precipitate formed. After 1 h, the mixture was partitioned between 1N HCl and EtOAc. The organic layer was dried with Na₂SO₄, filtered, and concentrated to give a solid. Trituration three times with MeOH provided the desired product as a white solid. MS (m/z): 330 (M+H)⁺. Calc'd for C₁₅H₁₁ClF₃NO₂: 329.70.

### Step 2. 5-(2-amino-6-quinazolinyl)-2-(methyloxy)-N-(3-(trifluoromethyl)phenyl)benzamide:

To a mixture of 6-(4,4,5,5-tetramethyl-1,3,2-dioxaborolan-2-yl)quinazolin-2-amine (0.18 g, 0.68 mmol), 2-dicyclohexylphosphino-2',6'-dimethoxy-1,1'-biphenyl (0.024 g, 0.060 mmol), Pd(OAc)₂ (0.015 g, 0.023 mmol), and K₃PO₄ (0.19 g, 0.91 mmol) in 2.3 mL toluene was added 5-chloro-2-methoxy-N-(3-(trifluoromethyl)phenyl)benzamide (0.15 g, 0.46 mmol) and 1 drop of water. The reaction vessel was sealed and heated to 100°C for 60 h. The reaction was cooled to ambient temperature, and was filtered through celite, rinsing with EtOAc. Removal of the solvent in vacuo gave a yellow residue which was purified by silica gel chromatography (dichloromethane/methanol). The resulting solid was mixed with MeOH, filtered, and collected to provide the title compound as a yellow solid. MS (m/z): 439.1 (M+H)⁺. Calc'd for C₂₃H₁₇F₃N₄O₂: 438.40.

### Example 162

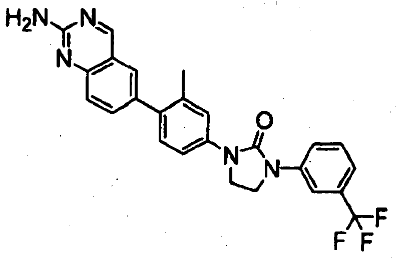

### Synthesis of 1-(4-(2-amino-6-quinazolinyl)-3-methylphenyl)-3-(3-(trifluoromethyl)phenyl)-2-imidazolidinone

### Step 1. Synthesis of 1-(4-bromo-3-methylphenyl)imidazolidin-2-one:

The title compound was prepared in a manner similar to to that described in J. Med. Chem. 3661, 2000. To a solution of 4-bromo-3-methylbenzenamine (3.0 g, 16 mmol) in 32 mL THF at 0 °C was added 1-chloro-2-isocyanatoethane (1.5 mL, 17 mmol). The reaction was allowed to warm to ambient temperature and stir for approximately 12h, and was then cooled to 0°C and NaH (60% in mineral oil, 1.4 g, 34 mmol) was added in small portions over 30 min. The mixture was heated to 70°C under nitrogen with a water-cooled reflux condenser. 20 ml THF was added to improve stirring. After a total of 1.5 h, the reaction was cooled to ambient temperature, and the solvent was removed in vacuo. The residue was added to water and dichloromethane, and the aqueous was acidified with 1N HCl until a pH of about 1. The aqueous mixture was extracted three times with dichloromethane, once with EtOAc, and was then filtered, rinsing the solid with MeOH, to afford the title compound as a white powder. MS (m/z): 254.9 (M+H)⁺; Calc'd for C₁₀H₁₁BrN₂O- 255.11.

### Step 2. 1-(4-bromo-3-methylphenyl)-3-(3-(trifluoromethyl)phenyl)imidazolidin-2-one:

To a mixture of 1-(4-bromo-3-methylphenyl)imidazolidin-2-one (0.20 g, 0.78 mmol), 9,9-dimethyl-4,5-bis(diphenylphosphino)xanthene (0.034 g, 0.060 mmol), Pd(OAc)₂ (0.026 g, 0.039 mmol), and Cs₂CO₃ (0.38 g, 1.2 mmol) in 1.5 mL dioxane was added 1-bromo-3-(trifluoromethyl)benzene (0.43 mL, 3.1 mmol). The reaction vessel was sealed and heated to 100 °C for 17 h. The reaction was cooled to ambient temperature, and was filtered through celite, rinsing with dichloromethane. Removal of the solvent in vacuo gave a brown residue which was purified by silica gel chromatography (methanol/dichloromethane) to provide the title compound as an off-white solid. MS (m/z): 400 (M+H)⁺; Calc'd for C₁₇H₁₄BrF₃N₂O- 399.21.

### Step 3. 1-(4-(2-amino-6-quinazolinyl)-3-methylphenyl)-3-(3-(trifluoromethyl)phenyl)-2-imidazolidinone:

The title compound was prepared by a method similar to that described in Example 161, step 2, except that filtration through celite was accompanied by 1:1 MeOH/CH₂Cl₂, to obtain the title compound as a yellow solid. MS (m/z): 464.1 (M+H)⁺; Calc'd for C₂₅H₂₀F₃N₅O: 463.45.

The following Examples 163-164 were prepared by a method similar to that described in Experimental Method D2 and Examples 161-162.

| Example No. | Structure | Name | Method | MW | (M+H⁺) |
|---|---|---|---|---|---|
| 163 | 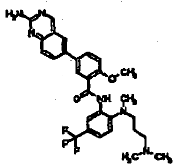 | 5-(2-amino-6-quinazolinyl)-N-(2-((3-(dimethylamino)propyl)(methyl)amino)-5-(trifluoromethyl)phenyl)-2-(methyloxy)benzamide | D2 | 552.598 | 553.2 |
| 164 | 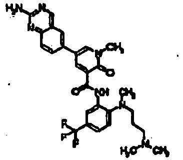 | 5-(2-amino-6-quinazolinyl)-N-(2-((3-(dimethylamino)propyl)(methyl)amino)-5-(trifluoromethyl)phenyl)-1-methyl-2-oxo-1,2-dihydro-3-pyridinecarboxamide | D2 | 553.586 | 554.2 |
| (Reference Example) | | | | | |

### Experimental Method D3

### Example 165

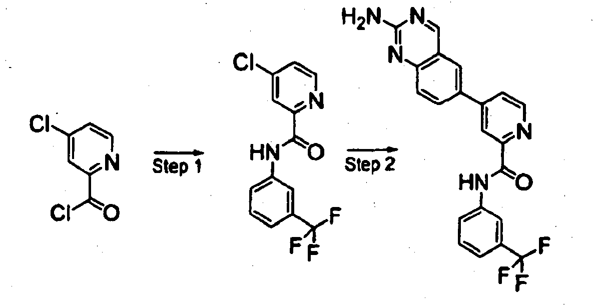

### Synthesis of 4-(2-amino-6-quinazolinyl)-N-(3-(trifluoromethyl)phenyl)-2-pyridinecarboxamide

### Step 1. 4-chloro-N-(3-(trifluoromethyl)phenyl)picolinamide:

To a solution of 4-chloropicolinyl chloride (prepared by a similar procedure described in Gudmundsson et al. Syn. Comm., 27, 861, 1997) (3.1 g, 18 mmol) in 10 mL THF at 0°C was added 3-(trifluoromethyl)benzenamine (2.2 ml, 18 mmol. The thick mixture was allowed to warm to ambient temperature. After 1 h, the mixture was partitioned between EtOAc and saturated aqueous sodium bicarbonate. The organic layer was dried with Na₂SO₄, filtered, and concentrated to give a solid. Purification by silica gel chromatography (EtOAc/hexanes) provided the desired title compound as a white solid. MS (m/z) : 301 (M+H)⁺; Calc'd for C₁₃H₈ClF₃N₂O-300.66.

### Step 2. 4-(2-amino-6-quinazolinyl)-N-(3-(trifluoromethyl)phenyl)-2-pyridinecarboxamide:

According to a method described in the literature (Synlett 1999, 45), to a mixture of 6-(4,4,5,5-tetramethyl-1,3,2-dioxaborolan-2-yl)quinazolin-2-amine (0.10 g, 0.37 mmol), 4-chloro-N-(3-(trifluoromethyl)phenyl)picolinamide (0.092 g, 0.31 mmol), and tri-o-tolylphospine (0.018 g, 0.061 mmol) in 1 mL DME was added potassium carbonate (2.0 M solution in water, 0.41 mL, 0.83 mmol) and Pd(OAc)₂ (0.010 g, 0.015 mmol). The reaction was sealed and heated to 100 °C for 48 h. The reaction was cooled to ambient temperature, diluted with EtOAc, water, and 1N NaOH and was filtered through celite, rinsing with EtOAc. The organic layer was dried with Na₂SO₄, filtered, and concentrated to give a solid. Purification by silica gel chromatography (DCM/MeOH) gave a solid which was suspended in MeOH and filtered to give the desired product as a yellow solid. MS (m/z): 410.1 (M+H)⁺; Calc'd for C₁₂H₁₄F₃N₅O: 409.36.

The following Example 166 was prepared by a method similar to that described in Example 165.

| Example No. | Structure | Name | Method | MW | (M+H⁺) |
|---|---|---|---|---|---|
| 166 | 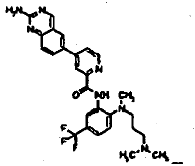 | 4-(2-amino-6-quinazolinyl)-N-(2-((3-(dimethylamino)propyl)(methyl)amino)-5-(trifluoromethyl)phenyl)-2-pyridinecarboxamide | D3 | 523.56 | 524.2 |

### Experimental Method E1

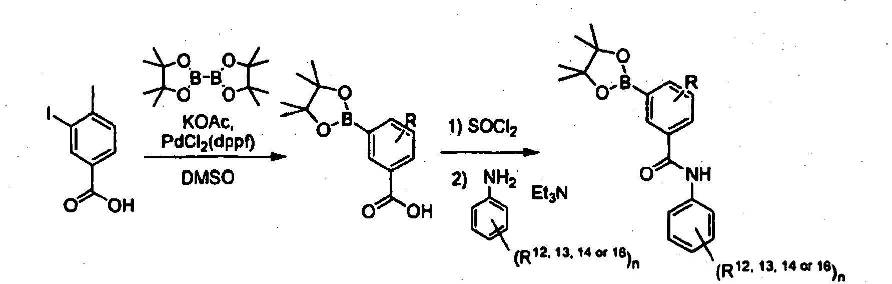

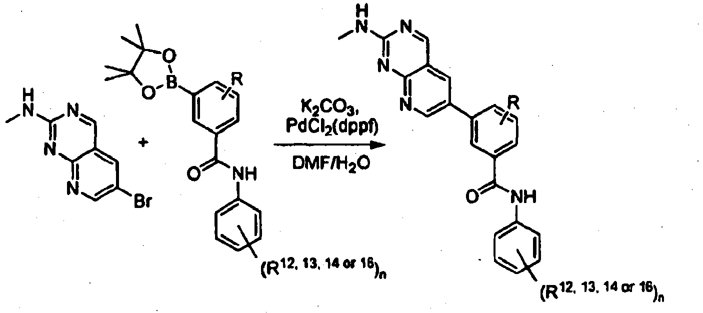

### Example 167

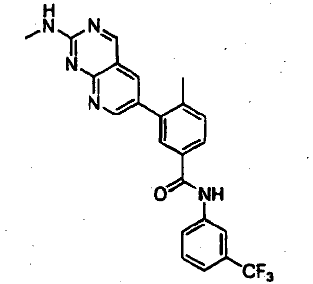

### Synthesis of 4-methyl-3-(2-(methylamino)pyrido[2,3-d]pyrimidin-6-yl)-N-(3-(trifluoromethyl)phenyl)benzamide

### Step 1: 9-methyl-3-(4,4,5,5-tetramethyl-1,3,2-dioxaborolan-2-yl)benzoic acid

To a mixture of 3-iodo-4-methylbenzoic acid (5.00 g, 19.1 mmol, 1.0 equiv), bis(pinacolato)diboron (5.80 g, 22.91 mmol, 1.2 equiv), and potassium acetate (5.60 g, 57.3 mmol, 3.0 equiv) in DMSO (70 ml), was added dichloro[1,1'-bis(diphenylphosphino)ferrocene] palladium dichloromethane adduct (421 mg, 0.573 mmol, 0.03 equiv). The mixture was heated at 80°C until the starting material was consumed. The solvent was removed *in vacuo* and the residue taken up in EtOAc (ca. 200 ml). After extracting with 2N NaOH, the aqueous fractions were combined and acidified with 6N HCl to pH 5-6. The resulting precipitate was filtered to provide 4-methyl-3-(4,4,5,5-tetramethyl-1,3,2-dioxaborolan-2-yl)benzoic acid. MS (M+H⁺) 263; Calc'd for C₁₄H₁₉BO₄: 262.1.

### Step 2: 4-methyl-3-(4,4,5,5-tetramethyl-1,3,2-dioxaborolan-2-yl)-N-(3-(trifluoromethyl)phenyl)benzamide

A mixture of 4-methyl-3-(4,4,5,5-tetramethyl-1,3,2-dioxaborolan-2-yl)benzoic acid (200 mg, 0.763 mmol, 1.0 equiv) and thionyl chloride (2.0 mL) was heated at 75°C for 1h. The solvent was removed *in vacuo* and the residue taken up in CH₂Cl₂ (5.0 mL). To the solution was added 3-(trifluoromethyl)benzenamine (104 µL, 0.840 mmol, 1.1 equiv) and triethylamine (319 µL, 2.29 mmol, 3.0 equiv). After the reaction was complete, the solution was diluted with CH₂Cl₂ (ca. 10 mL) and washed with water and brine. After drying with Na₂SO₄ and concentration *in vacuo,* the resulting 4-methyl-3-(4,4,5,5-tetramethyl-1,3,2-dioxaborolan-2-yl)-N-(3-(trifluoromethyl)phenyl)benzamide was advanced without further purification. MS (M+H⁺) 406; Calc'd for C₂₁H₂₃BF₃NO₃: 405.2.

### Step 3: 4-methyl-3-(2-(methylamino)pyrido[2,3-d]pyrimidin-6-yl)-N-(3-(trifluoromethyl)phenyl)benzamide

To a mixture of 4-methyl-3-(4,4,5,5-tetramethyl-1,3,2-dioxaborolan-2-yl)-N-(3-(trifluoromethyl)phenyl)benzamide (186 mg, 0.460 mmol, 1.1 equiv), 6-bromo-N-methylpyrido[2,3-d]pyrimidin-2-amine (100 mg, 0.418 mmol, 1.0 equiv), potassium carbonate (173 mg, 1.25 mmol, 3.0 equiv), and dichloro[1,1'-bis(diphenylphosphino)ferrocene] palladium dichloromethane adduct (31 mg, 0.0418 mmol, 0.1 equiv), was added DMF (5.0 mL) and H₂O (1.0 mL). The mixture was heated at 70°C. The solvent was removed *in vacuo* and the residue taken up in EtOAc (ca. 25 mL)). The organic portion was washed with water and brine, and dried with Na₂SO₄. After concentration *in vacuo,* the residue was purified by silica gel chromatography (1:3 hexanes:EtOAc to 100% EtOAc) to afford 4-methyl-3-(2-(methylamino)pyrido[2,3-d]pyrimidin-6-yl)-N-(3-(trifluoromethyl)phenyl)benzamide. MS (m/z) = 438.1 (M+H⁺); Calculated for C₂₃H₁₈F₃N₅O: 437.2

### Example 168

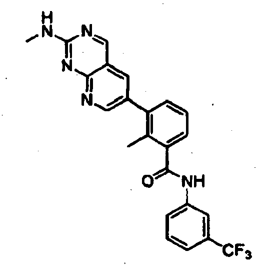

### Synthesis of 2-methyl-3-(2-(methylamino)pyrido[2,3-d)pyrimidin-6-yl)-N-(3-(trifluoromethyl)phenyl)benzamide

### Step 1: 2-methyl-3-(4,4,5,5-tetramethyl-1,3,2-dioxaborolan-2-yl)benzoic acid

To a mixture of 3-iodo-2-methylbenzoic acid (5.00 g, 19.1 mmol, 1.0 equiv), bis(pinacolato)diboron (5.80 g, 22.91 mmol, 1.2 equiv), and potassium acetate (5.60 g, 57.3 mmol, 3.0 equiv) in DMSO (70 mL), was added dichloro[1,1'-bis(diphenylphosphino)ferrocene] palladium dichloromethane adduct (421 mg, 0.573 mmol, 0.03 equiv). The mixture was heated at 80°C until the starting material was consumed. The solvent was removed *in vacuo* and the residue taken up in EtOAc (ca. 200 ml). After extracting with 2N NaOH, the aqueous fractions were combined and acidified with 6N HCl to pH 5-6. The resulting precipitate was filtered to provide 2-methyl-3-(4,4,5,5-tetramethyl-1,3,2-dioxaborolan-2-yl)benzoic acid. MS (m/z) = 263 (M+H⁺); Calc'd for C₁₄H₁₉BO₄ - 262.1.

### Step 2: 2-methyl-3-(4,4,5,5-tetramethyl-1,3,2-dioxaborolan-2-yl)-N-(3-(trifluoromethyl)phenyl)benzamide

A mixture of 2-methyl-3-(4,4,5,5-tetramethyl-1,3,2-dioxaborolan-2-yl)benzoic acid (200 mg, 0.763 mmol, 1.0 equiv) and thionyl chloride (2.0 mL) was heated at 75°C for 1h. The solvent was removed *in vacuo* and the residue taken up in CH₂Cl₂ (5.0 ml). To the solution was added 3-(trifluoromethyl)benzenamine (104 µL, 0.840 mmol, 1.1 equiv) and triethylamine (319 µL, 2.29 mmol, 3.0 equiv). After the reaction was complete, the solution was diluted with CH₂Cl₂ (ca. 10 ml) and washed with water and brine. After drying with Na₂SO₄ and concentration *in vacuo,* the resulting 2-methyl-3-(4,4,5,5-tetramethyl-1,3,2-dioxaborolan-2-yl)-N-(3-(trifluoromethyl)phenyl)benzamide was advanced without further purification. MS (m/z) = 406 (M+H⁺); Calculated for C₂₁H₂₃BF₃NO₃ - 405.2

### Step 3: 2-methyl-3-(2-(methylamino)pyrido[2,3-d]pyrimidin-6-yl)-N-(3-(trifluoromethyl)phenyl)benzamide

To a mixture of 2-methyl-3-(4,4,5,5-tetramethyl-1,3,2-dioxaborolan-2-yl)-N-(3-(trifluoromethyl)phenyl)benzamide (186 mg, 0.460 mmol, 1.1 equiv), 6-bromo-N-methylpyrido[2,3-d]pyrimidin-2-amine (100 mg, 0.418 mmol, 1.0 equiv), potassium carbonate (173 mg, 1.25 mmol, 3.0 equiv), and dichloro[1,1'-bis(diphenylphosphino)ferrocene] palladium dichloromethane adduct (31 mg, 0.0418 mmol, 0.1 equiv), was added DMF (5.0 mL) and H₂O (1.0 mL). The mixture was heated at 70°C. The solvent was removed *in vacuo* and the residue taken up in EtOAc (about 25 ml). The organic portion was washed with water and brine, and dried with Na₂SO₄. After concentration *in vacuo,* the residue was purified by silica gel chromatography (1:3 hexanes:EtOAc to 100% EtOAc) to afford 2-methyl-3-(2-(methylamino)pyrido[2,3-d]pyrimidin-6-yl)-N-(3-(trifluoromethyl)phenyl)benzamide. MS (m/z) = 438.1 (M+H⁺); Calculated for C₂₃H₁₈F₃N₅O - 437.2.

### Example 169

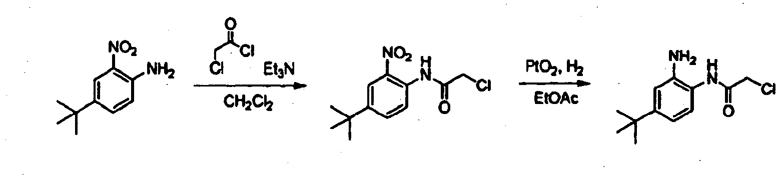

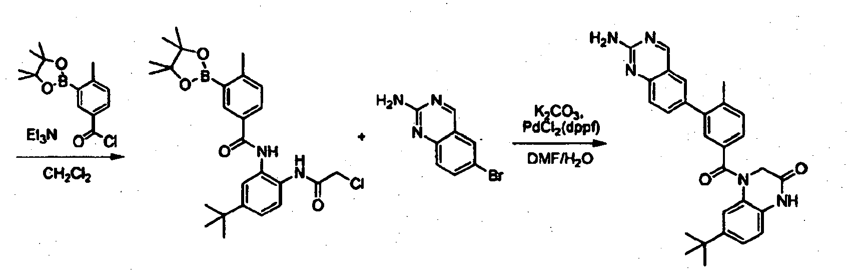

### Synthesis of '4-((3-(2-amino-6-quinazolinyl)-4-methylphenyl)carbonyl)-6-(1,1-dimethylethyl)-3,4-dihydro-2(1H)-quinoxalinone

### Step 1: N-(4-tert-butyl-2-nitrophenyl)-2-chloroacetamide

To a solution of 4-tert-butyl-2-nitrobenzenamine (3.04 g, 15.67 mmol, 1.0 equiv) in CH₂Cl₂ (90 mL) at 0°C was added chloroacetyl chloride (1.63 mL, 20.4 mmol, 1.3 equiv) followed by triethylamine (5.40 mL, 23.5 mmol, 2.5 equiv). After 1h, the solution was warmed to 25°C and allowed to stir until the reaction was complete. The solution was washed with water and dried with Na₂SO₄. The solvent was removed *in vacuo* and the residue purified by silica gel chromatography (9:1 hexanes:EtOAc) to afford N-(4-tert-butyl-2-nitrophenyl)-2-chloroacetamide. MS (MH⁺) 271; Calculated for C₁₂H₁₅ClN₂O₃: 270.1

### Step 2: N-(2-amino-4-tert-butylphenyl)-2-chloroacetamide

A mixture of N-(4-tert-butyl-2-nitrophenyl)-2-chloroacetamide (480 mg, 1.78 mmol, 1.0 equiv) and Adam's catalyst (20 mg) in EtOAc (15 ml) was exposed to an atmosphere of H₂ (balloon). Upon completion of the reduction, the reaction mixture was filtered through celite and concentrated *in vacuo* to afford N-(2-amino-4-tert-butylphenyl)-2-chloroacetamide, which was advanced without further purification. MS (MH⁺) 241; Calc'd for C₁₂H₁₇ClN₂O: 240.1

### Step 3: 7-benzyl-5-bromo-2,3-diphenylfuro[2,3-b]pyridin-4(7H)-one (2)

A mixture of 4-methyl-3-(4,4,5,5-tetramethyl-1,3,2-dioxaborolan-2-yl)benzoic acid (200 mg, 0.763 mmol, 1.0 equiv) and thionyl chloride (2.0 ml) was heated at 75°C for 1h. The solvent was removed *in vacuo* and the residue taken up in CH₂Cl₂ (5.0 ml). To the solution was added N-(2-amino-4-tert-butylphenyl)-2-chloroacetamide (202 mg, 0.840 mmol, 1.1 equiv) and triethylamine (319 µL, 2.29 mmol, 3.0 equiv). After the reaction was complete, the solution was diluted with CH₂Cl₂ (ca.10 ml) and washed with water and brine. After drying with Na₂SO₄ and concentration in vacuo, the resulting N-(5-tert-butyl-2-(2-chloroacetamido)phenyl)-4-methyl-3-(4,4,5,5-tetramethyl-1,3,2-dioxaborolan-2-yl)benzamide was advanced without further purification. MS (MH⁺) 485; Calculated for C₂₆H₃₄BClN₂O₄: 484.2

### Step 4: '4-((3-(2-amino-6-quinazolinyl)-4-methylphenyl)carbonyl)-6-(1,1-dimethylethyl)-3,4-dihydro-2(1H)-quinoxalinone

To a mixture of N-(5-tert-butyl-2-(2-chloroacetamido)phenyl)-4-methyl-3-(4,4,5,5-tetramethyl-1,3,2-dioxaborolan-2-yl)benzamide (223 mg, 0.460 mmol, 1.1 equiv), 6-bromoquinazolin-2-amine (93 mg, 0.418 mmol, 1.0 equiv), potassium carbonate (173 mg, 1.25 mmol, 3.0 equiv), and dichloro[1,1'-bis(diphenylphosphino)ferrocene] palladium dichloromethane adduct (31 mg, 0.0418 mmol, 0.1 equiv), was added DMF (5.0 mL) and H₂O (1.0 mL). The mixture was heated at 70°C. The solvent was removed *in vacuo* and the residue taken up in EtOAc (ca. 25 mL). The organic portion was washed with water and brine, and dried with Na₂SO₄. After concentration *in vacuo,* the residue was purified by silica gel chromatography (1:1 hexanes:EtOAc to 100% EtOAc) to afford '4-((3-(2-amino-6-quinazolinyl)-4-methylphenyl)carbonyl)-6-(1,1-dimethylethyl)-3,4-dihydro-2(1H)-quinoxalinone. MS (M+H⁺) 466.2; Calculated for C₂₈H₂₇N₅O₂ - 465.2.

The following Examples 170-192 were prepared by a method similar to that described in Experimental Method E1 and Examples 167-168.

| Example No. | Structure | Name | Method | MW | (M+H⁺) |
|---|---|---|---|---|---|
| 170 | 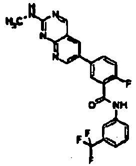 | 2-fluoro-5-(2-(methylamino)pyrido[2,3-d]pyrimidin-6-yl)-N-(3-(trifluoromethyl)phenyl)benzamide | E1 | 441.387 | 442 |
| 171 | 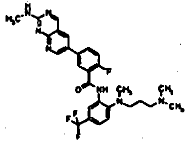 | N-(2-((3-(dimethylamino)propyl)(methyl)amino)-5-(trifluoromethyl)phenyl)-2-fluoro-5-(2-(methylamino)pyrido[2,3-d]pyrimidin-6-yl)benzamide | E1 | 555.577 | 556 |
| 172 | 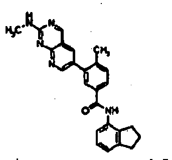 | N-(2,3-dihydro-1H-inden-4-yl)-4-methyl-3-(2-(methylamino)pyrido[2,3-d]pyrimidin-6-yl)benzamide | E1 | 409.491 | 410.1 |
| 173 | 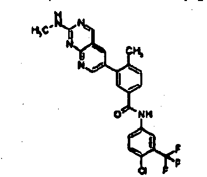 | N-(4-chloro-3-(trifluoromethyl)phenyl)-4-methyl-3-(2-(methylamino)pyrido[2,3-d]pyrimidin-6-yl)benzamide | E1 | 471.868 | 472.2 |
| 174 | 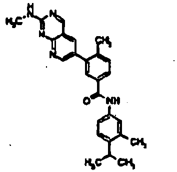 | 4-methyl-3-(2-(methylamino)pyrido[2,3-d]pyrimidin-6-yl)-N-(3-methyl-4-(1-methylethyl)phenyl)benzamide | E1 | 425.533 | 426.2 |
| 175 | 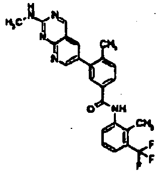 | 4-methyl-3-(2-(methylamino)pyrido[2,3-d]pyrimidin-6-yl)-N-(2-methyl-3-(trifluoromethyl)phenyl)benzamide | E1 | 451.45 | 452.1 |
| 176 | 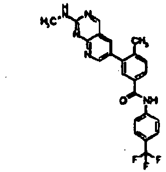 | 4-methyl-3-(2-(methylamino)pyrido[2,3-d]pyrimidin-6-yl)-N-(4-(trifluoromethyl)phenyl)benzamide | E1 | 437.423 | 438.1 |
| 177 | 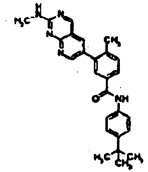 | N-(4-(1,1-dimethylethyl)phenyl)-4-methyl-3-(2-(methylamino)pyrido[2,3-d]pyrimidin-6-yl)benzamide | E1 | 425.533 | 426.2 |
| 178 | 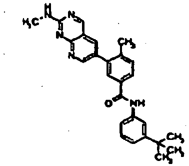 | N-(3-(1,1-dimethylethyl)phenyl)-4-methyl-3-(2-(methylamino)pyrido[2,3-d]pyrimidin-6-yl)benzamide | E1 | 425.533 | 426.2 |
| 179 | 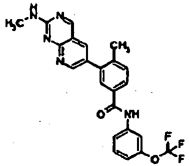 | 4-methyl-3-(2-(methylamino)pyrido[2,3-d]pyrimidin-6-yl)-N-(3-((trifluoromethyl)oxy)phenyl)benzamide | E1 | 453.422 | 454.1 |
| 180 | 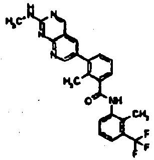 | 2-methyl-3-(2-(methylamino)pyrido[2,3-d]pyrimidin-6-yl)-N-(2-methyl-3-(trifluoromethyl)phenyl)benzamide | E1 | 451.45 | 452.2 |
| 181 | 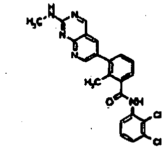 | N-(2,3-dichlorophenyl)-2-methyl-3-(2-(methylamino)pyrido[2,3-d]pyrimidin-6-yl)benzamide | E1 | 438.316 | 438.1 |
| 182 | 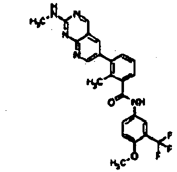 | 2-methyl-3-(2-(methylamino)pyrido[2,3-d]pyrimidin-6-yl)-N-(4-(methyloxy)-3-(trifluoromethyl)phenyl)benzamide | E1 | 467.449 | 468.1 |
| 183 | 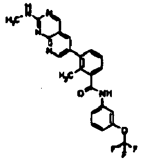 | 2-methyl-3-(2-(methylamino)pyrido[2,3-d]pyrimidin-6-yl)-N-(3-((trifluoromethyl)oxy)phenyl)benzamide | E1 | 453.422 | 454.1 |
| 184 | 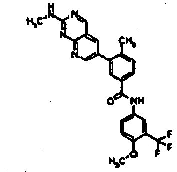 | 4-methyl-3-(2-(methylamino)pyrido[2,3-d]pyrimidin-6-yl)-N-(4-(methyloxy)-3-(trifluoromethyl)phenyl)benzamide | E1 | 467.449 | 468.1 |
| 185 | 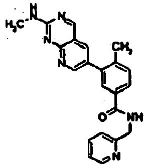 | 4-methyl-3-(2-(methylamino)pyrido[2,3-d]pyrimidin-6-yl)-N-(2-pyridinylmethyl)benzamide | E1 | 384.441 | 385.2 |
| 186 | 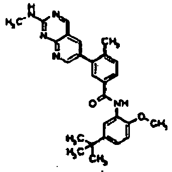 | N-(5-(1,1-dimethylethyl)-2-(methyloxy)phenyl)-4-methyl-3-(2-(methylamino)pyrido[2,3-d]pyrimidin-6-yl)benzamide | E1 | 455.559 | 456.2 |
| 187 | 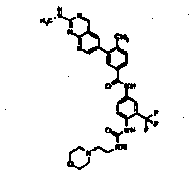 | 4-methyl-3-(2-(methylamino)pyrido[2,3-d]pyrimidin-6-yl)-N-(4-((((2-(4-morpholinyl)ethyl)amino)carbonyl)amino)-3-(trifluoromethyl)phenyl)benzamide | E1 | 608.622 | 609.1 |
| 188 | 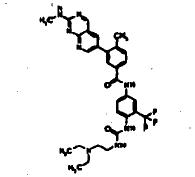 | N-(4-((((2-(diethylamino)ethyl)amino)carbonyl)amino)-3-(trifluoromethyl)phenyl)-4-methyl-3-(2-(methylamino)pyrido[2,3-d]pyrimidin-6-yl)benzamide | E1 | 594.639 | 595.1 |
| 189 | 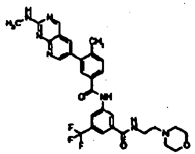 | 4-methyl-3-(2-(methylamino)pyrido[2,3-d]pyrimidin-6-yl)-N-(3-(((2-(4-morpholinyl)ethyl)amino)carbonyl)-5-(trifluoromethyl)phenyl)benzamide | E1 | 593.607 | 594.1 |
| 190 | 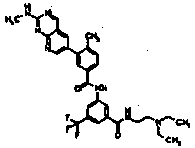 | N-(3-(((2-(diethylamino)ethyl)amino)carbonyl)-5-(trifluoromethyl)phenyl)-4-methyl-3-(2-(methylamino)pyrido[2,3-d)pyrimidin-6-yl)benzamide | E1 | 579.624 | 580.1 |
| 191 | 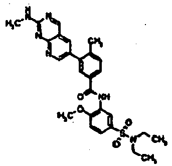 | N-(5-((diethylamino)sulfonyl)-2-(methyloxy)phenyl)-4-methyl-3-(2-(methylamino)pyrido[2,3-d]pyrimidin-6-yl)benzamide | E1 | 534.638 | 535.2 |
| 192 | 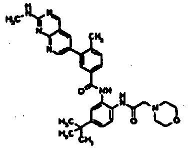 | N-(5-(1,1-dimethylethyl)-2-((4-morpholinylacetyl)amino)phenyl)-4-methyl-3-(2-(methylamino)pyrido[2,3-d]pyrimidin-6-yl)benzamide | E1 | 567.69 | 568.3 |

### Experimental Method E2

### Example 193

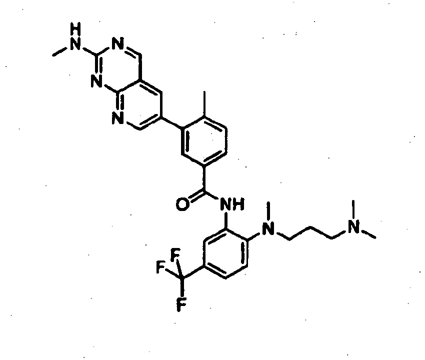

### N-(2-((3-(dimethylamino)propyl)(methyl)amino)-5-(trifluoromethyl)phenyl)-4-methyl-3-(2-(methylamino)pyrido[2,3-d]pyrimidin-6-yl)benzamide

### Step 1. Preparation of 6-bromo-N-methylpyrido[2,3-d]pyrimidin-2-amine

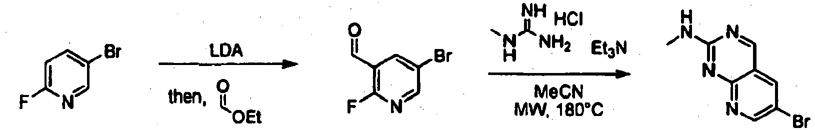

### Step a: 5-bromo-2-fluoronicotinaldehyde

To a solution of diisopropylamine (9.6 ml, 68.6 mmol, 1.1 equiv) in THF (90 ml) at 0°C, was added n-BuLi (27.9 ml, 2.5 M in hexanes). After 20 min, the solution was cooled to -78°C and diluted with THF (90 ml). A solution of 2-fluro-5-bromo-pyridine (11.1 g, 63.2 mmol, 1.0 equiv) in THF (90 ml) was added via addition funnel over ca. 15 min. After 1.5 h, ethyl formate (10.3 ml, 127 mmol, 2.0 equiv) was added dropwise and the solution was stirred for 1 h before quenching with a 1:1 mixture of saturated aqueous ammonium chloride and acetic acid (18 ml). The resulting slurry was warmed to 25°C and Na₂SO₄ (ca. 20 g) added. After filtering and concentration in vacuo, the resulting solid was recrystallized from CH₂Cl₂ to afford 5-bromo-2-fluoronicotinaldehyde. MS (MH⁺) 204; Calc'd for C₆H₃BrFNO: 204.0

### Step b: 6-bromo-N-methylpyrido[2,3-d]pyrimidin-2-amine

To a mixture of 5-bromo-2-fluoronicotinaldehyde (300 mg, 1.47 mmol, 1.0 equiv) and 1-methylguanidine hydrochloride (193 mg, 1.76 mmol, 1.2 equiv) in MeCN (18 ml) was added triethylamine (0.61 ml, 4.41 mmol, 3.0 equiv). The mixture was exposed to microwave radiation for 10 min at 180°C. After concentrating *in vacuo,* the resulting residue was taken up in CH₂Cl₂ (ca. 25 ml) and washed with water and brine. After drying the organic layer with Na₂SO₄, the solvent was removed *in vacuo* and residue purified by silica gel chromatography (1:3 hexanes:EtOAc to 100% EtOAc) to afford 6-bromo-N-methylpyrido[2,3-d]pyrimidin-2-amine. MS (MH⁺) 239; Calculated for C₈H₇BrN₄ - 239.1

### Step 2. Preparation of 4-methyl-3-(2-(methylamino)pyrido[2,3-d]pyrimidin-6-yl)benzoic acid

To 6-bromo-N-methylpyrido[2,3-d]pyrimidin-2-amine (115 mg, 0.49 mmol), 4-methyl-3-(4,4,5,5-tetramethyl-1,3,2-dioxaborolan-2-yl)benzoic acid (see Experimental Methods A or E1 for preparation of boronic ester) (128 mg, 0.49 mmmol), Pd(PPh₃)₄ (28 mg, 0.0025 mmol), and Na₂CO₃ (156 mg, 1.47 mmol) was added CH₃CN (1.8 mL) and water (1.8 mL). The mixture was stirred for 15 hours at 90 °C, diluted with saturated NaHCO₃ and extracted with EtOAc. The aqueous layer was acidified with TFA (pH-6) and the resulting solid was filtered to yield 4-methyl-3-(2-(methylamino)pyrido[2,3-d]pyrimidin-6-yl)benzoic acid as a yellow solid. MS m/z = 295 [M+1]⁺. Calc' d for C₁₆H₁₄N₄O₂: 294.32.

### Step 3. Preparation of N-(2-((3-(dimethylamino)propyl)(methyl)amino)-5-(trifluoromethyl)phenyl)-4-methyl-3-(2-(methylamino)pyrrido[2,3-d]pyrimidin-6-yl)benzamide

To 4-methyl-3-(2-(methylamino)pyrido[2,3-d]pyrimidin-6-yl)benzoic acid (90 mg, 0.31 mmol) was added SOCl₂ (2.5 mL). The mixture was stirred for 2.5 hours at 90 °C and concentrated. To the resulting acid chloride, N-(3-(dimethylamino)propyl)-N-methyl-4-(trifluoromethyl)benzene-1,2-diamine (83 mg, 0.30 mmol), and NaHCO₃ (large excess) was added CHCl₃ (1 ml). The mixture was stirred for 18 hours at RT. Purified the crude reaction by preparative TLC (90:10:1 CH₂Cl₂/MeOH/NH₄OH) to yield N-(2-((3-(dimethylamino)propyl)(methyl)amino)-5-(trifluoromethyl)phenyl)-4-methyl-3-(2-(methylamino)pyrido[2,3-d]pyrimidin-6-yl)benzamide as a light yellow solid. MS *m*/*z* = 552 [M+1]⁺. Calc'd for C₂₉H₃₂F₃N₇O: 551.62.

The following Examples 194-195 were prepared by a method similar to that described in Experimental Method E2 and Example 193.

| Example No. | Structure | Name | Method | MW | (M+H⁺) |
|---|---|---|---|---|---|
| 194 | 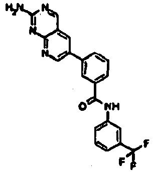 | 3-(2-aminopyrido[2,3-d]pyrimidin-6-yl)-N-(3-(trifluoromethyl)phenyl)benzamide | E2 | 409.37 | 410 |
| 195 | 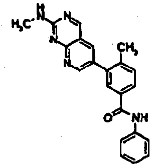 | 4-methyl-3-(2-(methylamino)pyrido[2,3-d]pyrimidin-6-yl)-N-phenylbenzamide | E2 | 369.426 | 370 |

### Experimental Method F1

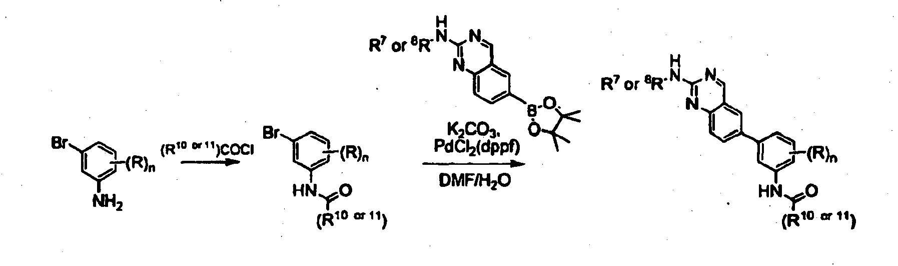

### Example 195a

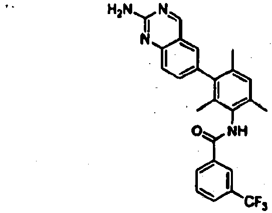

### Step 1: N-(3-bromo-2,4,6-trimethylphenyl)-3-(trifluoromethyl)benzamide

To a solution of 3-bromo-2,4,6-trimethylbenzenamine (200 mg, 0.934 mmol, 1.0 equiv) in CH₂Cl₂ (5.0 ml) was added 3-(trifluoromethyl)benzoyl chloride (214 mg, 1.03 mmol, 1.1 equiv) followed by triethylamine (390 µL, 2.80 mmol, 3.0 equiv). After the reaction was complete, the solution was diluted with CH₂Cl₂ (ca.10 ml) and washed with water and brine. After drying with Na₂SO₄ and concentration *in vacuo,* the resulting N-(3-bromo-2,4,6-trimethylphenyl)-3-(trifluoromethyl)benzamide was advanced without further purification. MS (MH⁺) 386; Calculated for C₂₁H₂₃BF₃NO₃: 385.0

### Step 2: 'N-(3-(2-amino-6-quinazolinyl)-2,4,6-trimethylphenyl)-3-(trifluoromethyl)benzamide

To a mixture of 6-(4,4,5,5-tetramethyl-1,3,2-dioxaborolan-2-yl)quinazolin-2-amine (186 mg, 0.460 mmol, 1.1 equiv), N-(3-bromo-2,4,6-trimethylphenyl)-3-(trifluoromethyl)benzamide (100 mg, 0.418 mmol, 1.0 equiv), potassium carbonate (173 mg, 1.25 mmol, 3.0 equiv), and dichloro[1,1'-bis(diphenylphosphino)ferrocene] palladium dichloromethane adduct (31 mg, 0.0418 mmol, 0.1 equiv), was added DMF (5.0 mL) and H₂O (1.0 mL). The mixture was heated at 70°C. The solvent was removed *in vacuo* and the residue taken up in EtOAc (ca. 25 ml). The organic portion was washed with water and brine, and dried with Na₂SO₄. After concentration *in vacuo,* the residue was purified by silica gel chromatography (1:3 hexanes:EtOAc to 100% EtOAc) to afford 'N-(3-(2-amino-6-quinazolinyl)-2,4,6-trimethylphenyl)-3-(trifluoromethyl)benzamide. MS (MH⁺) 451.1; Calculated for C₂₅H₂₁F₃N₄O: 450.2

### Experimental Method G1

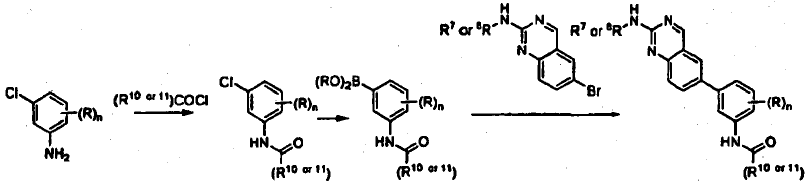

### Example 196

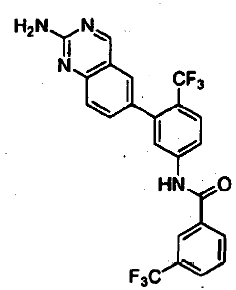

### Synthesis of 5-(2-amino-6-quinazolinyl)-4-(trifluoromethylphenyl)-N-(3-(trifluoromethyl)phenyl)benzamide

### Step 1: Preparation of N-(3-chloro-4-(trifluoromethyl)phenyl)-3-(trifluoromethyl)benzamide

3-(Trifluoromethyl)benzoyl chloride (1.1 ml, 7.31 mmol) was added to 3-chloro-4-(trifluoromethyl)benzenamine (1.1 g, 5.62 mmol) in CH₂Cl₂ (25 ml). The mixture was stirred at RT over night then NEt₃ (1.17 ml, 8.34 mmol) was added. After stirring for 8h, the mixture was concentrated and the crude residue was purified by flash chromatography on silica, eluting with 3 % MeOH/CH₂Cl₂ to afford the title compound as a white solid. MS *m*/*z* = 366 [M-H]⁻. Calc'd for C₁₅H₈ClF₆NO : 367.

### Step 2: N-(3-(4,4,5,5-tetramethyl-1,3,2-dioxaborolan-2-yl)-4-(trifluoromethyl)phenyl)-3-(trifluoromethyl)benzamide

This intermediate was prepared using the conditions for the preparation of 3-(4,4,5,5-tetramethyl-1,3,2-dioxaborolan-2-yl)-4-(trifluoromethyl)benzenamine, with N-(3-chloro-4-(trifluoromethyl)phenyl)-3-(trifluoromethyl)benzamide (from step 1 above) used in place of 3-chloro-4-(trifluoromethyl)benzenamine. MS *m*/*z* = 460 [M+H]⁺ Calc' d for C₂₁H₂₀BF₆NO₃: 359.

### Step 3 - Preparation of 5-(2-amino-6-quinazolinyl)-4-(trifluoromethylphenyl)-N-(3-(trifluoromethyl)phenyl)benzamide

The Suzuki reaction between N-(3-(4,4,5,5-tetramethyl-1,3,2-dioxaborolan-2-yl)-4-(trifluoromethyl)phenyl)-3-(trifluoromethyl)benzamide and 6-bromoquinazolin-2-amine (Example 722) was performed using DMF in place of CH₃CN(10% Pd(dppf)Cl₂, K₂CO₃, DMF/H₂O, 60 °C, 2.5 h). The reaction afforded the title compound as a peach-colored solid. MS *m*/*z* = 477 [M+H]⁴. Calc'd for C₂₃H₁₄F₆N₄O: 476.

### Experimental Method H1

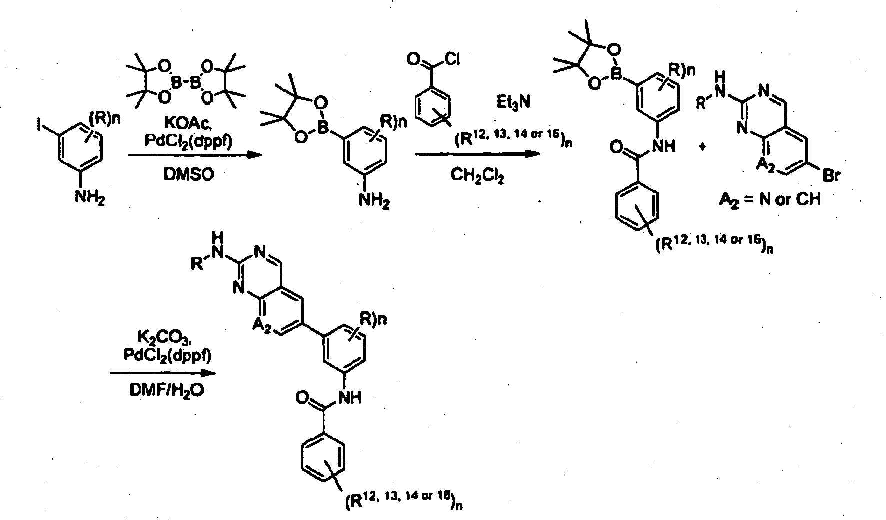

### Example 197

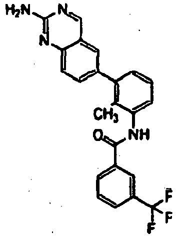

### Synthesis of N-(3-(2-amino-6-quinazolinyl)-2-methylphenyl)-3-(trifluoromethyl)benzamide

### Step 1: 2-methyl-3-(4,4,5,5-tetramethyl-1,3,2-dioxaborolan-2-yl)benzenamine

To a mixture of 3-iodo-2-methylbenzenamine (4.45 g, 19.1 mmol, 1.0 equiv), bis(pinacolato)diboron (5.80 g, 22.91 mmol, 1.2 equiv), and potassium acetate (5.60 g, 57.3 mmol, 3.0 equiv) in DMSO (70 mL), was added dichloro[1,1'-bis(diphenylphosphino)ferrocene] palladium dichloromethane adduct (421 mg, 0.573 mmol, 0.03 equiv). The mixture was heated at 80°C until the starting material was consumed. The solvent was removed *in vacuo* and the residue taken up in EtOAc (ca. 200 ml) and washed with water and brine. After drying with Na₂SO₄ and concentrating in vacuo, the residue was purified by silica gel chromatography (3:1 hexanes:EtOAc to 1:3 hexanes:EtOAc) to provide 2-methyl-3-(4,4,5,5-tetramethyl-1,3,2-dioxaborolan-2-yl)benzenamine. MS (M+H⁺) 234; Calculated for C₁₃H₂₀BNO₂: 233.2

### Step 2: N-(2-methyl-3-(4,4,5,5-tetramethyl-1,3,2-dioxaborolan-2-yl)phenyl)-3-(trifluoromethyl)benzamide

To a solution of 2-methyl-3-(4,4,5,5-tetramethyl-1,3,2-dioxaborolan-2-yl)benzenamine (178 mg, 0.763 mmol, 1.0 equiv) in CH₂Cl₂ (5.0 mL), was added 3-(trifluoromethyl)benzoyl chloride (214 mg, 1.03 mmol, 1.1 equiv) followed by triethylamine (390 µL, 2.80 mmol, 3.0 equiv). After the reaction was complete, the solution was diluted with CH₂Cl₂ (ca. 10 mL) and washed with water and brine. After drying with Na₂SO₄ and concentration *in vacuo,* the resulting N-(2-methyl-3-(4,4,5,5-tetramethyl-1,3,2-dioxaborolan-2-yl)phenyl)-3-(trifluoromethyl)benzamide was advanced without further purification. MS (M+H⁺) 406; Calculated for C₂₁H₂₃BF₃NO₃: 405.2

### Step 3: N-(3-(2-amino-6-quinazolinyl)-2-methylphenyl)-3-(trifluoromethyl)benzamide

To a mixture of N-(2-methyl-3-(4,4,5,5-tetramethyl-1,3,2-dioxaborolan-2-yl)phenyl)-3-(trifluoromethyl)benzamide (186 mg, 0.460 mmol, 1.1 equiv), 6-bromoquinazolin-2-amine (93 mg, 0.418 mmol, 1.0 equiv), potassium carbonate (173 mg, 1.25 mmol, 3.0 equiv), and dichloro[1,1'-bis(diphenylphosphino)ferrocene] palladium dichloromethane adduct (31 mg, 0.0418 mmol, 0.1 equiv), was added DMF (5.0 ml) and H₂O (1.0 ml). The mixture was heated at 70°C for about 6 hrs. The solvent was removed *in vacuo* and the residue taken up in EtOAc (ca. 25 ml). The organic portion was washed with water and brine, and dried with Na₂SO₄. After concentration *in vacuo,* the residue was purified by silica gel chromatography (1:3 hexanes:EtOAc to 100% EtOAc) to afford N-(3-(2-amino-6-quinazolinyl)-2-methylphenyl)-3-(trifluoromethyl)benzamide. MS (M+H⁺) 423.1; Calculated for C₂₃H₁₈F₃N₅O: 422.1

### Example 198

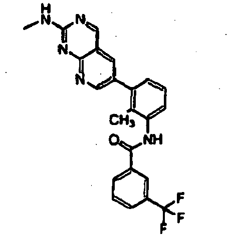

### Synthesis of N-2-methyl-3-(2-(methylamino)pyrido[2,3-d]pyrimidin-6-yl)-N-(3-(trifluoromethyl)phenyl)benzamide (A₂ = N)

To a mixture of N-(2-methyl-3-(4,4,5,5-tetramethyl-1,3,2-dioxaborolan-2-yl)phenyl)-3-(trifluoromethyl)benzamide (186 mg, 0.460 mmol, 1.1 equiv), 6-bromo-N-methylpyrido[2,3-d]pyrimidin-2-amine (100 mg, 0.418 mmol, 1.0 equiv), potassium carbonate (173 mg, 1.25 mmol, 3.0 equiv), and dichloro[1,1'-bis(diphenylphosphino)ferrocene] palladium dichloromethane adduct (31 mg, 0.0418 mmol, 0.1 equiv), was added DMF (5.0 ml) and H₂O (1.0 ml). The mixture was heated at 70°C for about 6 hrs. The solvent was removed *in vacuo* and the residue taken up in EtOAc (ca. 25 ml). The organic portion was washed with water and brine, and dried with Na₂SO₄. After concentration *in vacuo,* the residue was purified by silica gel chromatography (1:3 hexanes:EtOAc to 100% EtOAc) to afford N-2-methyl-3-(2-(methylamino)pyrido[2,3-d]pyrimidin-6-yl)-N-(3-(trifluoromethyl)phenyl)benzamide. MS (MH⁺) 438.1; Calculated for C₂₃H₁₈F₃N₅O - 437.2.

The following Examples 199-224 were prepared by a method similar to that described in Experimental Method H1 and Examples 200 and 201.

| Example No. | Structure | Name | Method | MW | (M+H⁺) |
|---|---|---|---|---|---|
| 199 | 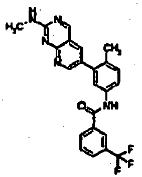 | N-(4-methyl-3-(2-(methylamino)pyrido[2,3-d]pyrimidin-6-yl)phenyl)-3-(trifluoromethyl)benzamide | H1 | 437.423 | 438.2 |
| 200 | 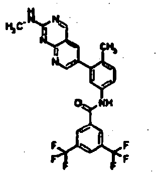 | N-(4-methyl-3-(2-(methylamino)pyrido[2,3-d]pyrimidin-6-yl)phenyl)-3,5-bis(trifluoromethyl)benzamide | H1 | 505.42 | 506.1 |
| 201 | 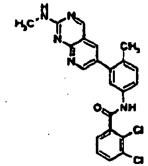 | 2,3-dichloro-N-(4-methyl-3-(2-(methylamino)pyrido[2,3-d]pyrimidin-6-yl)phenyl)benzamide | H1 | 438.316 | 438 |
| 202 | 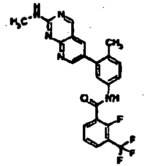 | 2-fluoro-N-(4-methyl-3-(2-(methylamino)pyrido[2,3-d]pyrimidin-6-yl)phenyl)-3-(trifluoromethyl)benzamide | H1 | 455.413 | 456.1 |
| 203 | 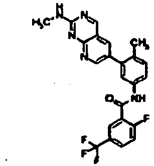 | 2-fluoro-N-(4-methyl-3-(2-(methylamino)pyrido[2,3-d]pyrimidin-6-yl)phenyl)-5-(trifluoromethyl)benzamide | H1 | 455.413 | 456.1 |
| 204 | 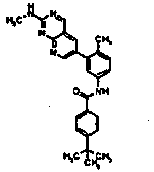 | 4-(1,1-dimethylethyl)-N-(4-methyl-3-(2-(methylamino)pyrido[2,3-d]pyrimidin-6-yl)phenyl)benzamide | H1 | 425.533 | 426.1 |
| 205 | 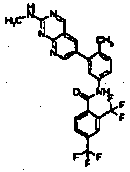 | N-(4-methyl-3-(2-(methylamino)pyrido[2,3-d]pyrimidin-6-yl)phenyl)-2,4-bis(trifluoromethyl)benzamide | H1 | 505.42 | 506.1 |
| 206 | 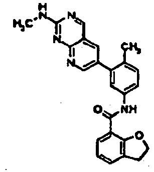 | N-(4-methyl-3-(2-(methylamino)pyrido[2,3-d]pyrimidin-6-yl)phenyl)-2,3-dihydro-1-benzofuran-7-carboxamide | H1 | 411.463 | 412.1 |
| 207 | 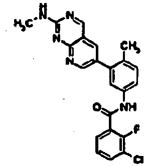 | 3-chloro-2-fluoro-N-(4-methyl-3-(2-(methylamino)pyrido[2,3-d]pyrimidin-6-yl)phenyl)benzamide | H1 | 421.861 | 422.1 |
| 208 | 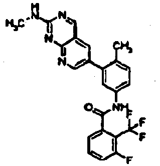 | 3-fluoro-N-(4-methyl-3-(2-(methylamino)pyrido[2,3-d]pyrimidin-6-yl)phenyl)-2-(trifluoromethyl)benzamide | H1 | 455.413 | 456.1 |
| 209 | 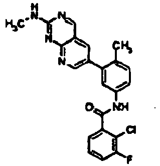 | 2-chloro-3-fluoro-N-(4-methyl-3-(2-(methylamino)pyrido[2,3-d]pyrimidin-6-yl)phenyl)benzamide | H1 | 421.861 | 422.1 |
| 210 | 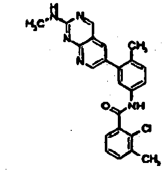 | 2-chloro-3-methyl-N-(4-methyl-3-(2-(methylamino)pyrido[2,3-d]pyrimidin-6-yl)phenyl)benzamide | H1 | 417.898 | 418.1 |
| 211 | 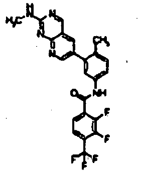 | 2,3-difluoro-N-(4-methyl-3-(2-(methylamino)pyrido[2,3-d]pyrimidin-6-yl)phenyl)-4-(trifluoromethyl)benzamide | H1 | 473.403 | 474.1 |
| 212 | 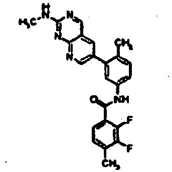 | 2,3-difluoro-4-methyl-N-(4-methyl-3-(2-(methylamino)pyrido[2,3-d]pyrimidin-6-yl)phenyl)benzamide | H1 | 419.433 | 420.2 |
| 213 | 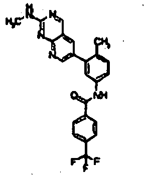 | N-(4-methyl-3-(2-(methylamino)pyrido[2,3-d]pyrimidin-6-yl)phenyl)-4-(trifluoromethyl)benzamide | H1 | 437.423 | 438.1 |
| 214 | 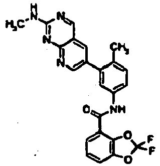 | 2,2-difluoro-N-(4-methyl-3-(2-(methylamino)pyrido[2,3-d]pyrimidin-6-yl)phenyl)-1,3-benzodioxole-4-carboxamide | H1 | 449.415 | 450.1 |
| 215 | 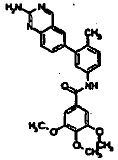 | N-(3-(2-amino-6-quinazolinyl)-4-methylphenyl)-3,4,5-tris(methyloxy)benzamide | H1 | 444.489 | 445.1 |
| 216 | 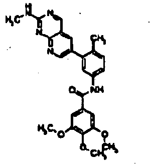 | N-(4-methyl-3-(2-(methylamino)pyrido[2,3-d]pyrimidin-6-yl)phenyl)-3,4,5-tris(methyloxy)benzamide | H1 | 459.504 | 460.2 |
| 217 | 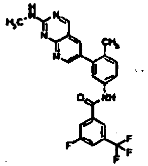 | 3-fluoro-N-(4-methyl-3-(2-(methylamino)pyrido[2,3-d]pyrimidin-6-yl)phenyl)-5-(trifluoromethyl)benzamide | H1 | 455.413 | 456.1 |
| 218 | 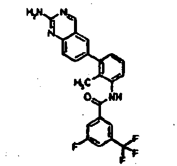 | N-(3-(2-amino-6-quinazolinyl)-2-methylphenyl)-3-fluoro-5-(trifluoromethyl)benzamide | H1 | 440.398 | 441.1 |
| 219 | 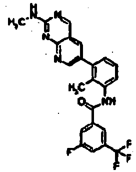 | 3-fluoro-N-(2-methyl-3-(2-(methylamino)pyrido[2,3-d]pyrimidin-6-yl)phenyl)-5-(trifluoromethyl)benzamide | H1 | 455.413 | 456.1 |
| 220 | 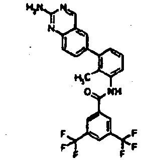 | N-(3-(2-amino-6-quinazolinyl)-2-methylphenyl)-3,5-bis(trifluoromethyl)benzamide | H1 | 490.405 | 491.1 |
| 221 | 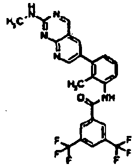 | N-(2-methyl-3-(2-(methylamino)pyrido[2,3-d]pyrimidin-6-yl)phenyl)-3,5-bis(trifluoromethyl)benzamide | H1 | 505.42 | 506.1 |
| 222 | 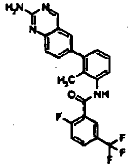 | N-(3-(2-amino-6-quinazolinyl)-2-methylphenyl)-2-fluoro-5-(trifluoromethyl)benzamide | H1 | 440.398 | 441.1 |
| 223 | 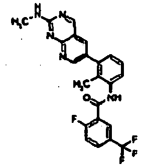 | 2-fluoro-N-(2-methyl-3-(2-(methylamino)pyrido[2,3-d]pyrimidin-6-yl)phenyl)-5-(trifluoromethyl)benzamide | H1 | 455.413 | 456.1 |
| 224 | 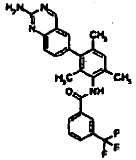 | N-(3-(2-amino-6-quinazolinyl)-2,4,6-trimethylphenyl)-3-(trifluoromethyl)benzamide | H1 | 450.462 | 451.1 |

### Experimental Method 11

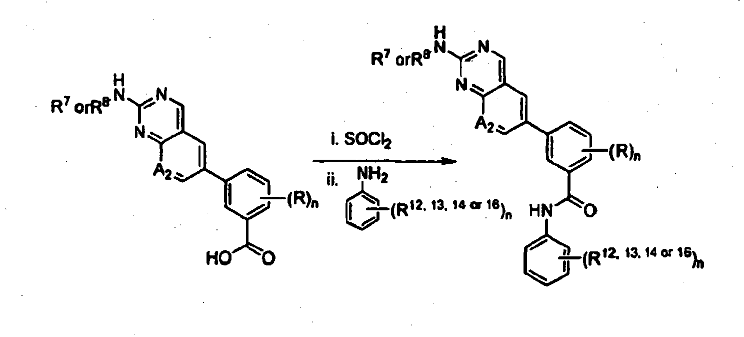

### Example 225

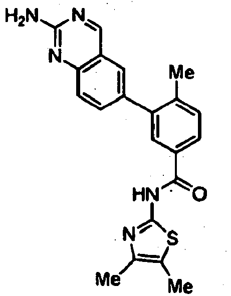

### Synthesis of 3-(2-amino-6-quinazolinyl)-N-(9,5-dimethyl-1,3-thiazol-2-yl)-4-methylbenzamide

3-(2-Aminoquinazolin-6-yl)-4-methylbenzoic acid (0.060 g, 0.21 mmol) was suspended in neat thionyl chloride (0.5 ml) at ambient temperature. The mixture was heated at reflux for 1 h, then cooled to ambient temperature. The resulting solution was concentrated *in vacuo,* then diluted with toluene and concentrated a second time. The crude solid was dried under high vacuum for 0.5 h. To a solution of the crude solid in anhydrous methylene chloride (2.1 ml), was added 2-amino-4,5-dimethylthiazole hydrochloride (0.036 g, 0.21 mmol) and triethylamine (0.15 ml, 1.10 mmol). The solution was stirred at ambient temperature for 15 h then concentrated *in vacuo.* Purification by silica chromatography (5% methanol/methylene chloride) afforded 3-(2-amino-6-quinazolinyl)-N-(4,5-dimethyl-1,3-thiazol-2-yl)-4-methylbenzamide as a white solid. MS (M+H⁺) 390.2; Calculated for C₂₁H₁₉N₅OS: 389.

The following Examples 226-374 were prepared by a method similar to that described in Experimental Method I1 and Example 225.

| Example No. | Structure | Name | Method | MW | (M+H⁺) |
|---|---|---|---|---|---|
| 226 | 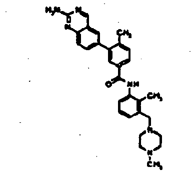 | 3-(2-amino-6-quinazolinyl)-4-methyl-N-(2-methyl-3-((4-methyl-1-piperazinyl)methyl)phenyl)benzamide | I1 | 480.613 | 481.2 |
| 227 | 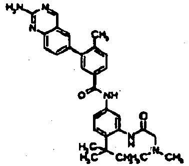 | 3-(2-amino-6-quinazolinyl)-N-(4-(1,1-dimethylethyl)-3-((N,N-dimethylglycyl)amino)phenyol)-4-methylbenzamide | I1 | 510.639 | 511.2 |
| 228 | 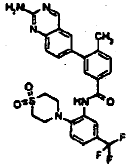 | 3-(2-amino-6-quinazolinyl)-N-(2-(1,1-dioxido-4-thiomorpholinyl)-5-(trifluoromethyl)phenyl)-4-methylbenzamide | I1 | 555.579 | 556 |
| 229 | 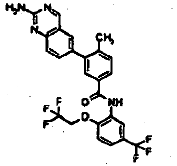 | 3-(2-amino-6-quinazolinyl)-4-methyl-N-(2-((2,2,2-trifluoroethyl)oxY)-5-(trifluoromethyl)phenyl)benzamide | I1 | 520.431 | 521.3 |
| 230 | 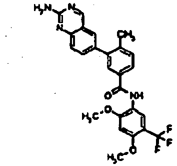 | 3-(2-amino-quinazolinyl)-N-(2,4-bis(methyloxy)-5-(trifluoromethyl)phenyl)-4-methylbenzamide | I1 | 482.46 | 483.3 |
| 231 | 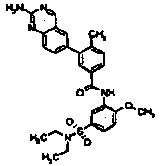 | 3-(2-amino-6-quinazolinyl)-N-(5-((diethylamino)sulfonyl)-2-(methyloxy)phenyl)-4-methylbenzamide | I1 | 519.623 | 520.1 |
| 232 | 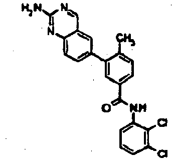 | 3-(2-amino-quinazolinyl)-N-(2,3-dichlorophenyl)-4-methylbenzamide | I1 | 423.301 | 423 |
| 233 | 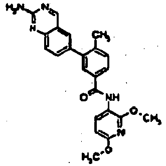 | 3-(2-amino-quinazolinyl)-N-(2,6-bis(methyloxy)-3-pyridinyl)-4-methylbenzamide | I1 | 415.451 | 416.1 |
| 234 | 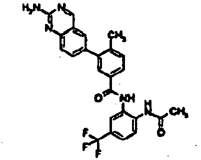 | N-(2-(acetylamino)-5-(trifluoromethyl)phenyl)-3-(2-amino-6-quinazolinyl)-4-methylbenzamide | I1 | 479.46 | 480 |
| 235 | 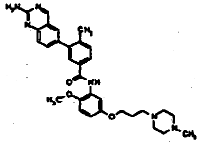 | 3-(2-amino-6-quinazolinyl)-4-methyl-N-(2-(methyloxy)-5-((3-(4-methyl-1-piperazinyl)propyl)oxy)phenyl)benzamide | I1 | 540.664 | 541.2 |
| 236 | 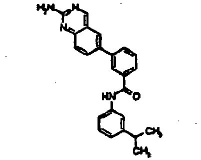 | 3-(2-amino-6-quinazolinyl)-N-(3-(1-methylethyl)phenyl)benzamide | I1 | 382.465 | 383.2 |
| 237 | 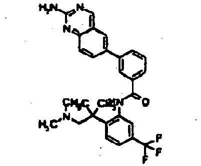 | 3-(2-amino-6-quinazolinyl)-N-(2-(2-(dimethylamino)-1.1-dimethylethyl)-5-(trifluoromethyl)phenyl)benzamide | I1 | 507.557 | 508.2 |
| 238 | 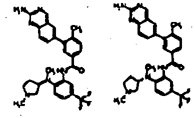 | 3-(2-amino-6-quinazolinyl)-4-methyl-N-(2-(methyl((3S)-1-methyl-3-pyrrolidinyl)amino)-5-(trifluoromethyl)phenyl)benzamide | I1 | 534.583 | 535.3 |
| 239 | 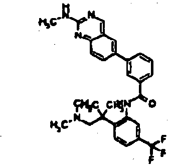 | N-(2-(2-(dimethylamino)-1,1-dimethylethyl)-5-(trifluoromethyl)phenyl)-3-(2-(methylamino)-6-quinazolinyl)benzamide | 11 | 521.584 | 522.2 |
| 240 | 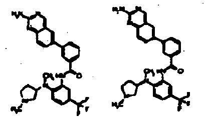 | 3-(2-amino-6-quinazolinyl)-N-(2-(methyl((3R)-1-methyl-3-pyrrolidinyl)amino)-5-(trifluoromethyl)phenyl)benzamide | I1 | 520.556 | 521.2 |
| 241 | 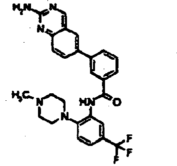 | 3-(2-amino-6-quinazolinyl)-N-(2-(4-methyl-1-piperazinyl)-5-(trifluoromethyl)phenyl)benzamide | I1 | 506.53 | 507.2 |
| 242 | 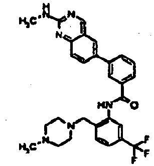 | 3-(2-(methylamino)-6-quinazolinyl)-N-(2-((4-methyl-1-piperazinyl)methyl)-5-(trifluoromethyl)phenyl)benzamide | I1 | 534.583 | 535.2 |
| 243 | 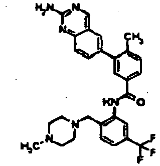 | 3-(2-amino-6-quinazolinyl)-4-methyl-N-(2-((4-methyl-1-piperazinyl)methyl)-5-(trifluoromethyl)phenyl)benzamide | I1 | 534.583 | 535.2 |
| 244 | 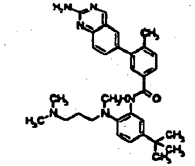 | 3-(2-amino-6-quinazolinyl)-N-(2-((3-(dimethylamino)propyl)(methyl)amino)-5-(1,1-dimethylethyl)phenyl)-4-methylbenzamide | I1 | 524.709 | 525.3 |
| 245 | 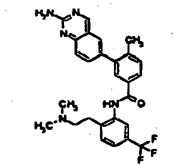 | 3-(2-amino-6-quinazolinyl)-N-(2-(2-(dimethylamino)ethyl)-5-(trifluoromethyl)phenyl)-4-methylbenzamide | I1 | 493.53 | 494.2 |
| 246 | 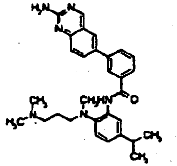 | 3-(2-amino-6-quinazolinyl)-N-(2-((3-(dimethylamino)propyl)(methyl)amino)-5-(1-methylethyl)phenyl)benzamide | I1 | 496.655 | 497.3 |
| 247 | 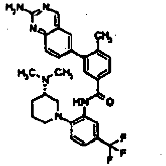 | 3-(2-amino-6-quinazolinyl)-N-(2-((3S)-3-(dimethylamino)-1-piperidinyl)-5-(trifluoromethyl)phenyl)-4-methylbenzamide | I1 | 548.61 | 549.3 |
| 248 | 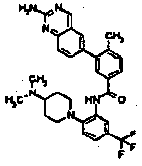 | 3-(2-amino-6-quinazolinyl)-N-(2-(4-(dimethylamino)-1-piperidinyl)-5-(trifluoromethyl)phenyl)-4-methylbenzamide | I1 | 548.61 | 549.2 |
| 249 | 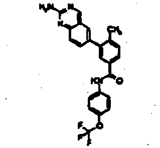 | 3-(2-amino-6-quinazolinyl)-4-methyl-N-(4-((trifluoromethyl)oxy)phenyl)benzamide | I1 | 438.407 | 439.2 |
| 250 | 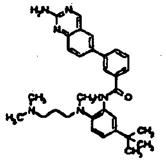 | propyl)(methyl)a3-(2-amino-6-quinazolinyl)-N-(2-((3-(dimethylamino)propyl)(methyl)amino)-5-(1,1-dimethylethyl)phenyl)benzamide | I1 | 510.682 | 511.4 |
| 251 | 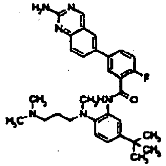 | 5-(2-amino-6-quinazolinyl)-N-(2-((3-(dimethylamino)propyl)(methyl)amino)-5-(1,1-dimethylethyl)phenyl)-2-fluorobenzamide | I1 | 528.672 | 529.2 |
| 252 | 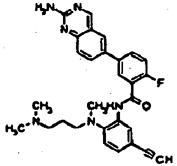 | 5-(2-amino-6-quinazolinyl)-N-(2-((3-(dimethylamino)propyl)(methyl)amino)-5-ethynylphenyl)-2-fluorobenzamide | I1 | 496.587 | 497.3 |
| 253 | 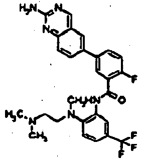 | 5-(2-amino-quinazolinyl)-N-(2-((2--(dimethylamino)ethyl)(methyl)amino)-5-(trifluoromethyl)phenyl)-2-fluorobenzamide | 11 | 526.535 | 527.2 |
| 254 | 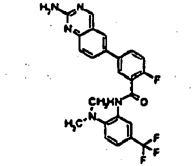 | 5-(2-amino-6-quinazolinyl)-N-(2-(dimethylamino)-5-(trifluoromethyl)phenyl)-2-fluorobenzamide | 11 | 469.44 | 470.2 |
| 255 | 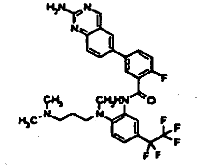 | 5-(2-amino-quinazolinyl)-N-(2-((3-(dimethylamino)propyl)(methyl)amino)-5-(pentafluoroethyl)phenyl)-2-fluorobenzamide | I1 | 590.569 | 591.3 |
| 256 | 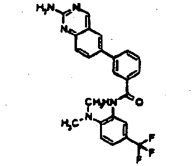 | 3-(2-amino-6-quinazolinyl)-N-(2-(dimethylamino)-5-(trifluoromethyl)phenyl)benzamide | I1 | 451.45 | 452 |
| 257 | 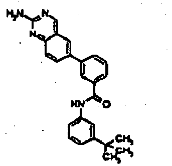 | 3-(2-amino-6-quinazolinyl)-N-(3-(1,1-dimethylethyl)phenyl)benzamide | I1 | 396.492 | 397 |
| 258 | 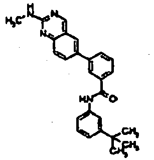 | N-(3-(1,1-dimethylethyl)phenyl)-3-(2-(methylamino)-6-quinazolinyl)benzamide | I1 | 410.518 | 411 |
| 259 | 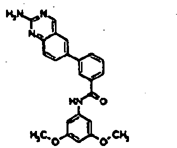 | 3-(2-amino-6-quinazolinyl)-N-(3,5-bis(methyloxy)phenyl)benzamide | I1 | 400.436 | 401 |
| 260 | 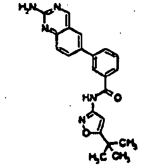 | 3-(2-amino-6-quinazolinyl)-N-(5-(1,1-dimethylethyl)-3-isoxazolyl)benzamide | I1 | 387.441 | 388 |
| 261 | 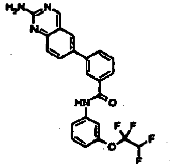 | 3-(2-amino-6-quinazolinyl)-N-(3-((1,1,2,2-tetrafluoroethyl)oxy)phenyl)benzamide | I1 | 456.397 | 456.9 |
| 262 | 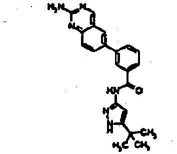 | 3-(2-amino-6-quinazolinyl)-N-(5-(1,1-dimethylethyl)-1H-pyrazol-3-yl)benzamide | I1 | 386.457 | 387 |
| 263 | 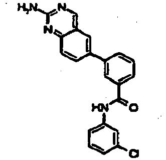 | 3-(2-amino-6-quinazolinyl)-N-(3-chlorophenyl)benzamide | I1 | 374.829 | 374.9 |
| 264 | 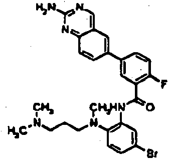 | 5-(2-amino-6-quinazolinyl)-N-(5-bromo-2-((3-(dimethylamino)propyl)(methyl)amino)phenyl)-2-fluorobenzamide | I1 | 551.461 | 552.9 |
| 265 | 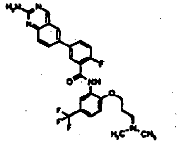 | 5-(2-amino-6-quinazolinyl)-N-(2-((3-(dimethylamino)propyl)oxy)-5-(trifluoromethyl)phenyl)-2-fluorobenzamide | I1 | 527.519 | 528.2 |
| 266 | 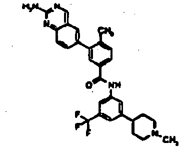 | 3-(2-amino-6-quinazolinyl)-4-methyl-N-(3-(1-methyl-4-piperidinyl)-5-(trifluoromethyl)phenyl)benzamide | I1 | 519.568 | 520.2 |
| 267 | 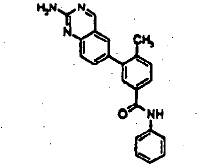 | 3-(2-amino-6-quinazolinyl)-4-methyl-N-phenylbenzamide | I1 | 354.411 | 355 |
| 268 | 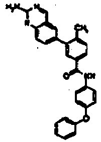 | 3-(2-amino-6-quinazolinyl)-4-methyl-N-(4-(phenyloxy)phe nyl)benzamide | I1 | 446.508 | 447 |
| 269 | 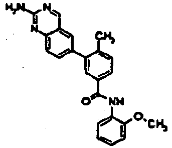 | 3-(2-amino-6-quinazolinyl)-4-methyl-N-(2-(methyloxy)phe nyl)benzamide | I1 | 384.437 | 385 |
| 270 | 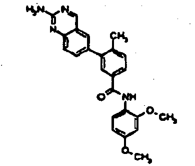 | 3-(2-amino-quinazolinyl)-N-(2,4-bis(methyloxy)phenyl)-4-methylbenzamide | I1 | 414.463 | 415 |
| 271 | 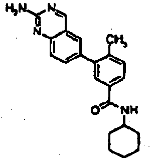 | 3-(2-amino-6-quinazolinyl)-N-cyclohexyl-4-methylbenzamide | I1 | 360.459 | 361 |
| 272 | 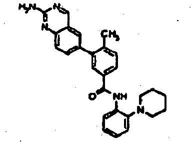 | 3-(2-amino-6-quinazolinyl)-4-methyl-Ni(2-(1-piperidinyl)phenyl)benzamide | I1 | 437.544 | 438 |
| 273 | 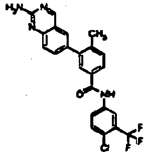 | 3-(2-amino-6-quinazolinyl)-N-(4-chloro-3-(trifluoromethyl)phenyl)-4-methylbenzamide | I1 | 456.853 | 457 |
| 274 | 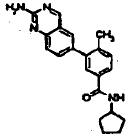 | 3-(2-amino-6-quinazolinyl)-N-cyclopentyl-4-methylbenzamide | I1 | 346.432 | 347 |
| 275 | 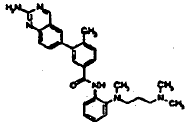 | 3-(2-amino-6-quinazolinyl)-N-(2-((3-(dimethylamino)propyl)(methyl)amino)phenyl)-4-methylbenzamide | I1 | 468.602 | 469 |
| 276 | 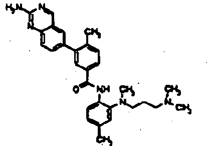 | 3-(2-amino-6-quinazolinyl)-N-(2-((3-(dimethylamino)propyl)(methyl)amino)-4-methylphenyl)-4-methylbenzamide | I1 | 482.629 | 483 |
| 277 | 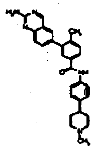 | 3-(2-amino-6-quinazolinyl)-4-methyl-N-(4-(1-methyl-4-piperidinyl)phenyl)benzamide | I1 | 451.571 | 452 |
| 278 | 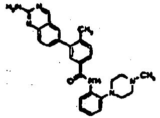 | 3-(2-amino-6-quinazolinyl)-4-methyl-N-(2-(4-methyl-1-piperazinyl)phenyl)benzamide | I1 | 452.559 | 453 |
| 279 | 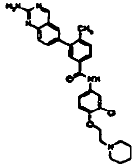 | 3-(2-amino-6-quinazolinyl)-N-(3-chloro-4-((2-(1-piperidinyl)ethyl)oxy)phenyl)-4-methylbenzamide | I1 | 516.042 | 516 |
| 280 | 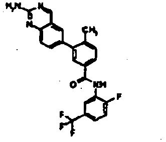 | 3-(2-amino-6-quinazolinyl)-N-(2-fluoro-5-(trifluoromethyl)phenyl)-4-methylbenzamide | I1 | 440.398 | 441 |
| 281 | 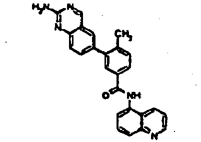 | 3-(2-amino-quinazolinyl)-4-methyl-N-(5-quinolinyl)benzamide | I1 | 405.459 | 406 |
| 282 | 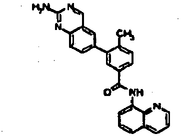 | 3-(2-amino-6-quinazolinyl)-4-methyl-N-(8-quinolinyl)benzamide | I1 | 405.459 | 406 |
| 283 | 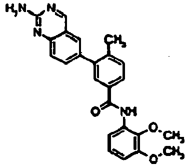 | 3-(2-amino-quinazolinyl)-N-(5,6-bis(methyloxy)-phenyl)-4-methylbenzamide | I1 | 416.479 | 416 |
| 284 | 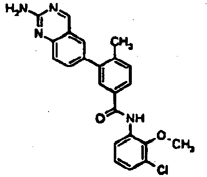 | 3-(2-amino-6-quinazolinyl)-N-(5-chloro-6-(methyloxy)-phenyl)-4-methylbenzamide | I1 | 420.898 | 421 |
| 285 | 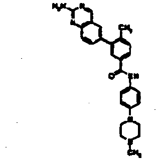 | 3-(2-amino-6-quinazolinyl)-4-methyl-N-(4-(4-methyl-1-piperazinyl)phenyl)benzamide | I1 | 452.559 | 453 |
| 286 | 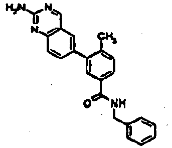 | 3-(2-amino-6-quinazolinyl)-4-methyl-N-(phenylmethyl)benzamide | I1 | 368.438 | 369 |
| 287 | 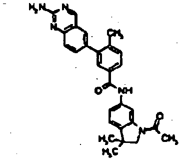 | N-(1-acetyl-3,3-dimethyl-2,3-dihydro-1H-indol-6-yl)-3-(2-amino-6-quinazolinyl)-4-methylbenzamide | I1 | 465.554 | 466 |
| 288 | 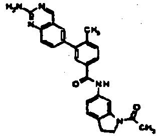 | N-(1-acetyl-2,3-dihydro-1H-indol-6-yl)-3-(2-amino-6-quinazolinyl)-4-methylbenzamide | I1 | 437.501 | 438 |
| 289 | 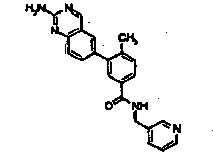 | 3-(2-amino-6-quinazolinyl)-4-methyl-N-(3-pyridinylmethyl)benzamide | I1 | 369.426 | 370 |
| 290 | 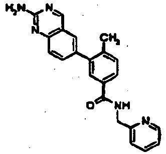 | 3-(2-amino-6-quinazolinyl)-4-methyl-N-(2-pyridinylmethyl)benzamide | I1 | 369.426 | 370 |
| 291 | 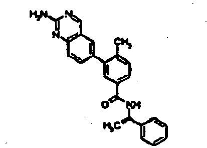 | 3-(2-amino-6-quinazolinyl)-4-methyl-N-((1R)-phenylethyl)benzamide | I1 | 382.465 | 383 |
| 292 | 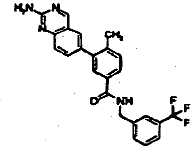 | 3-(2-amino-6-quinazolinyl)-4-methyl-N-((3-(trifluoromethyl)phenyl)methyl)benzamide | I1 | 436.435 | 437 |
| 293 | 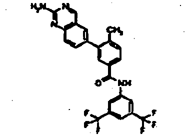 | 3-(2-amino-6-quinazolinyl)-N-(3,5-bis(trifluoromethyl)phenyl)-4-methylbenzamide | I1 | 490.405 | 491 |
| 294 | 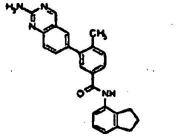 | 3-(2-amino-6-quinazolinyl)-N-(2,3-dihydro-1H-inden-4-yl)-4-methylbenzamide | I1 | 394.476 | 395 |
| 295 | 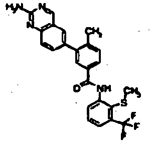 | 3-(2-amino-6-quinazolinyl)-4-methyl-N-(2-(methylsulfanyl)-3-(trifluoromethyl)phenyl)benzamide | I1 | 468.501 | 469 |
| 296 | 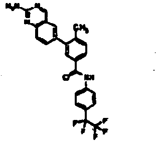 | 3-(2-amino-6-quinazolinyl)-4-methyl-N-(4-(pentafluoroethyl)phenyl)benzamide | I1 | 472.415 | 473 |
| 297 | 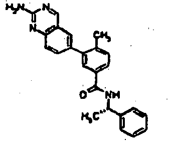 | 3-(2-amino-6-quinazolinyl)-4-methyl-N-((1S)-1-phenylethyl)benzamide | I1 | 382.465 | 383 |
| 298 | 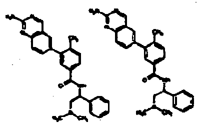 | 3-(2-amino-quinazolinyl)-N-((1R)-2-(dimethylamino)-1-phenylethyl)-4-methylbenzamide | I1 | 425.533 | 426 |
| 299 | 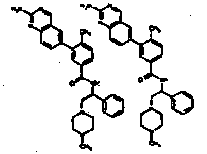 | 3-(2-amino-6-quinazolinyl)-4-methyl-N-((1R)-2-(4-methyl-1-piperazinyl)-1-phenylethyl)benzamide | I1 | 480.613 | 481 |
| 300 | 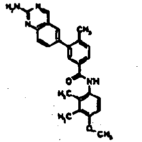 | 3-(2-amino-6-quinazolinyl)-N-(2,3-dimethyl-4-(methyloxy)phenyl)-4-methylbenzamide | I1 | 412.491 | 413 |
| 301 | 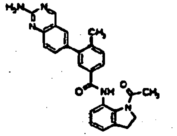 | N-(1-acetyl-2,3-dihydro-1H-indol-7-yl)-3-(2-amino-6-quinazolinyl)-4-methylbenzamide | I1 | 437.501 | 438 |
| 302 | 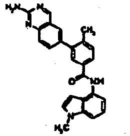 | 3-(2-amino-6-quinazolinyl)-4-methyl-N-(1-methyl-1H-indol-4-yl)benzamide | I1 | 407.475 | 408 |
| 303 | 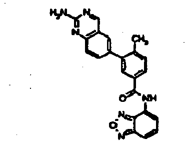 | 3-(2-amino-6-quinazolinyl)-N-(2,1,3-benzoxadiazol-4-yl)-4-methylbenzamide | I1 | 396.408 | 397 |
| 304 | 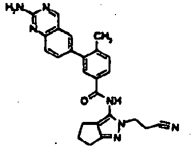 | 3-(2-amino-6-quinazolinyl)-N-(2-(2-cyanoethyl)-2,4,5,6-tetrahydrocyclo penta[c]pyrazol-3-yl)-4-methylbenzamide | I1 | 437.505 | 438 |
| 305 | 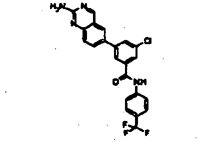 | 3-(2-amino-6-quinazolinyl)-5-chloro-N-(4-(trifluoromethyl)phenyl)benzamide | I1 | 442.827 | 443 |
| 306 | 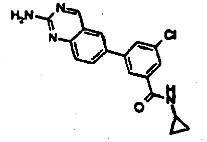 | 3-(2-amino-6-quinazolinyl)-5-chloro-N-cyclopropylbenzamide | I1 | 338.797 | 339 |
| 307 | 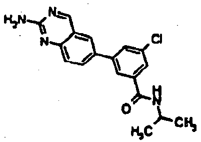 | 3-(2-amino-6-quinazolinyl)-5-chloro-N-(1-methylethyl)benzamide | I1 | 340.812 | 341 |
| 308 | 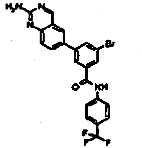 | 3-(2-amino-6-quinazolinyl)-5-bromo-N-(4-(trifluoromethyl)phenyl)benzamide | I1 | 487.278 | 489 |
| 309 | 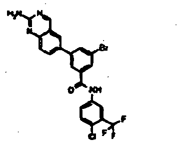 | 3-(2-amino-6-quinazolinyl)-5-bromo-N-(4-chloro-3-(trifluoromethyl)phenyl)benzamide | I1 | 521.723 | 523 |
| 310 | 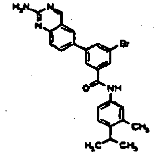 | 3-(2-amino-6-quinazolinyl)-5-bromo-N-(3-methyl-4-(1-methylethyl)phenyl)benzamide | I1 | 475.388 | 477 |
| 311 | 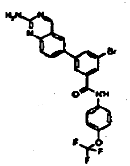 | 3-(2-amino-6-quinazolinyl)-5-bromo-N-(4-((trifluoromethyl)oxy)phenyl)benzamide | I1 | 503.277 | 505 |
| 312 | 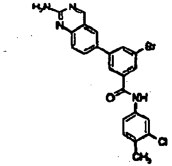 | 3-(2-amino-6-quinazolinyl)-5-bromo-N-(3-chloro-4-methylphenyl)benzamide | I1 | 467.752 | 469 |
| 313 | 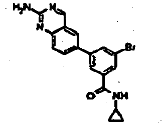 | 3-(2-amino-6-quinazolinyl)-5-bromo-N-cyclopropylbenzamide | I1 | 383.247 | 383 |
| 314 | 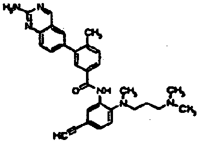 | 3-(2-amino-6-quinazolinyl)-N-(2-((3-(dimethylamino)propyl)(methyl)amino)-5-ethynylphenyl)-4-methylbenzamide | I1 | 492.624 | 493 |
| 315 | 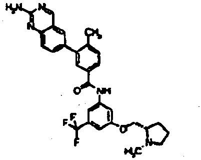 | 3-(2-amino-quinazolinyl)-4-methyl-N-(3-((((2S)-1-methyl-2-pyrrolidinyl)met hyl)oxy)-5-(trifluoromethyl)phenyl)benzamide | I1 | 535.567 | 536 |
| 316 | 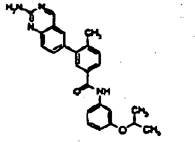 | 3-(2-amino-6-quinazolinyl)-4-methyl-N-(3-((1-methylethyl)oxy)phenyl)benzamide | I1 | 412.491 | 413 |
| 317 | 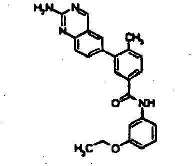 | 3-(2-amino-quinazolinyl)-N-(3-(ethyloxy)phenyl)-4-methylbenzamide | I1 | 398.464 | 399 |
| 318 | 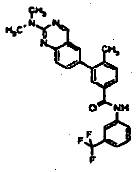 | 3-(2-(dimethylamino)-6-quinazolinyl)-4-methyl-N-(3-(trifluoromethyl)phenyl)benzamide | I1 | 450.462 | 451 |
| 319 | 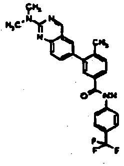 | 3-(2-(dimethylamino)-6-quinazolinyl)-4-methyl-N-(4-(trifluoromethyl)phenyl)benzamide | I1 | 450.462 | 451 |
| 320 | 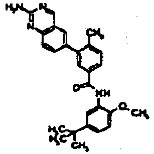 | 3-(2-amino-6-quinazolinyl)-N-(5-(1,1-dimethylethyl)-2-(methyloxy)phenyl)-4-methylbenzamide | I1 | 440.544 | 441.3 |
| 321 | 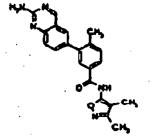 | 3-(2-amino-6-quinazolinyl)-N-(3,4-dimethyl-5-isoxazolyl)-4-methylbenzamide | I1 | 373.414 | 374 |
| 322 | 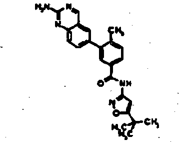 | 3-(2-amino-6-quinazolinyl)-N-(5-(1,1-dimethylethyl)-3-isoxazolyl)-4-methylbenzamide | I1 | 401.468 | 402 |
| 323 | 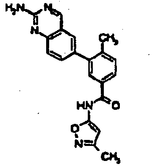 | 3-(2-amino-6-quinazolinyl)-4-methyl-N-(3-methyl-5-isoxazolyl)benzamide | I1 | 359.387 | 360 |
| 324 | 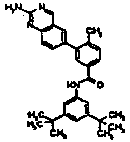 | 3-(2-amino-6-quinazolinyl)-N-(3,5-bis(1,1-dimethylethyl)phenyl)-4-methylbenzamide | I1 | 466.626 | 467 |
| 325 | 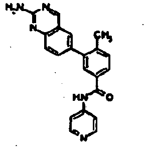 | 3-(2-amino-6-quinazolinyl)-4-methyl-N-(4-pyridinyl)benzamide | I1 | 355.399 | 356 |
| 326 | 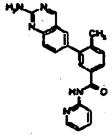 | 3-(2-amino-6-quinazolinyl)-4-methyl-N-(2-pyridinyl)benzamide | I1 | 355.399 | 356 |
| 327 | 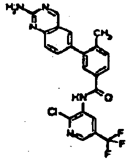 | 3-(2-amino-6-quinazolinyl)-N-(2-chloro-5-(trifluoromethyl)-3-pyridinyl)-4-methylbenzamide | I1 | 457.841 | 458 |
| 328 | 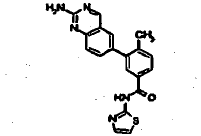 | 3-(2-amino-6-quinazolinyl)-4-methyl-N-(1,3-thiazol-2-yl)benzamide | I1 | 361.427 | 362 |
| 329 | 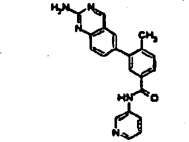 | 3-(2-amino-6-quinazolinyl)-4-methyl-N-(3-pyridinyl)benzamide | I1 | 355.399 | 356 |
| 330 | 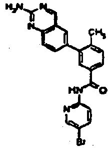 | 3-(2-amino-6-quinazolinyl)-N-(5-bromo-2-pyridinyl)-4-methylbenzamide | I1 | 434.295 | 434 |
| 331 | 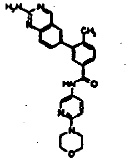 | 3-(2-amino-6-quinazolinyl)-4-methyl-N-(6-(4-morpholinyl)-3-pyridinyl)benzamide | I1 | 440.505 | 441 |
| 332 | 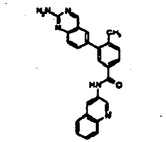 | 3-(2-amino-6-quinazolinyl)-4-methyl-N-(3-quinolinyl)benzamide | I1 | 405.459 | 406 |
| 333 | 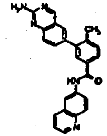 | 3-(2-amino-6-quinazolinyl)-4-methyl-N-(6-quinolinyl)benzamide | I1 | 405.459 | 406 |
| 334 | 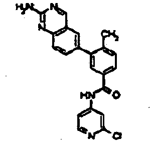 | 3-(2-amino-6-quinazolinyl)-N-(2-chloro-4-pyridinyl)-4-methylbenzamide | I1 | 389.844 | 390 |
| 335 | 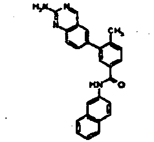 | 3-(2-amino-6-quinazolinyl)-4-methyl-N-(2-naphthalenyl)benzamide | I1 | 404.471 | 405 |
| 336 | 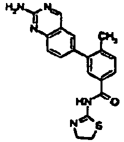 | 3-(2-amino-6-quinazolinyl)-N-(4,5-dihydro-1,3-thiazol-2-yl)-4-methylbenzamide | I1 | 363.443 | 364 |
| 337 | 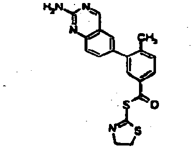 | S-(4,5-dihydro-1,3-thiazol-2-yl)3-(2-amino-6-quinazolinyl)-4-methylbenzenecarbothioate | I1 | 380.494 | 381 |
| 338 | 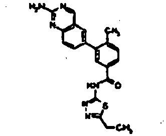 | 3-(2-amino-6-quinazolinyl)-N-(5-ethyl-1,3,4-thiadiazol-2-yl)-4-methylbenzamide | I1 | 390.469 | 391 |
| 339 | 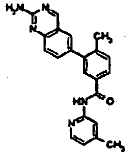 | 3-(2-amino-6-quinazolinyl)-4-methyl-N-(4-methyl-2-pyridinyl)benzamide | I1 | 369.426 | 370 |
| 340 | 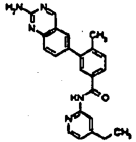 | 3-(2-amino-6-quinazolinyl)-N-(4-ethyl-2-pyridinyl)-4-methylbenzamide | I1 | 383.453 | 384 |
| 341 | 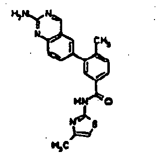 | 3-(2-amino-6-quinazolinyl)-4-methyl-N-(4-methyl-1,3-thiazol-2-yl)benzamide | I1 | 375.454 | 376 |
| 342 | 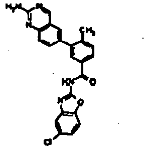 | 3-(2-amino-6-quinazolinyl)-N-(5-chloro-1,3-benzoxazol-2-yl)-4-methylbenzamide | I1 | 429.865 | 430 |
| 343 | 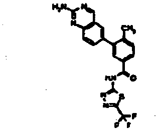 | 3-(2-amino-quinazolinyl)-4-methyl-N-(5-(trifluoromethyl)-1,3,4-thiadiazol-2-yl)benzamide | I1 | 430.413 | 431 |
| 344 | 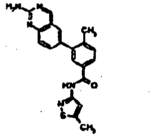 | 3-(2-amino-6-quinazolinyl)-4-methyl-N-(5-methyl-3-isothiazolyl)benzamide | I1 | 375.454 | 376 |
| 345 | 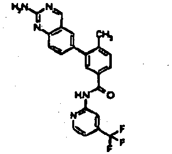 | 3-(2-amino-6-quinazolinyl)-4-methyl-N-(4-(trifluoromethyl)-2-pyridinyl)benzamide | I1 | 423.396 | 424 |
| 346 | 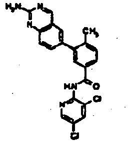 | 3-(2-amino-6-quinazolinyl)-N-(3,5-dichloro-2-pyridinyl)-4-methylbenzamide | I1 | 424.289 | 424 |
| 347 | 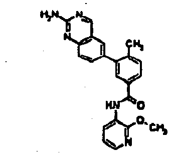 | 3-(2-amino-6-quinazolinyl)-4-methyl-N-(2-(methyloxy)-3-pyridinyl)benzamide | I1 | 385.425 | 386 |
| 348 | 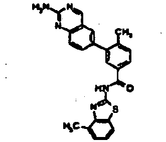 | 3-(2-amino-6-quinazolinyl)-4-methyl-N-(4-methyl-1,3-benzothiazol-2-yl)benzamide | I1 | 425.514 | 426 |
| 349 | 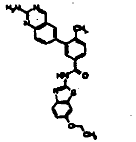 | 3-(2-amino-6-quinazolinyl)-N-(6-(ethyloxy)-1,3-benzothiazol-2-yl)-4-methylbenzamide | I1 | 455.54 | 456 |
| 350 | 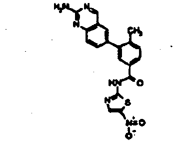 | 3-(2-amino-quinazolinyl)-4-methyl-N-(5-nitro-1,3-thiazol-2-yl)benzamide | I1 | 406.425 | 407 |
| 351 | 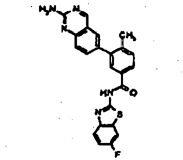 | 3-(2-amino-6-quinazolinyl)-N-(6-fluoro-1,3-benzothiazol-2-yl)-4-methylbenzamide | I1 | 429.477 | 430 |
| 352 | 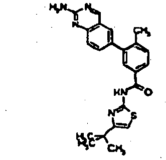 | 3-(2-amino-6-quinazolinyl)-N-(4-(1,1-dimethylethyl)-1,3-thiazol-2-yl)-4-methylbenzamide | I1 | 417.535 | 418 |
| 353 | 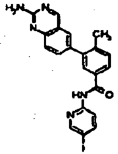 | 3-(2-amino-6-quinazolinyl)-N-(5-iodo-2-pyridinyl)-4-methylbenzamide | I1 | 481.291 | 482 |
| 354 | 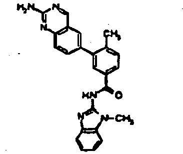 | 3-(2-amino-6-quinazolinyl)-4-methyl-N-(1-methyl-1H-benzimidazol-2-yl)benzamide | I1 | 408.463 | 409 |
| 355 | 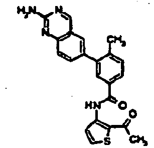 | N-(2-acetyl-3-thienyl)-3-(2-amino-6-quinazolinyl)-4-methylbenzamide | I1 | 402.476 | 403 |
| 356 | 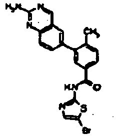 | 3-(2-amino-quinazolinyl)-N-(5-bromo-1,3-thiazol-2-yl)-4-methylbenzamide | I1 | 440.324 | 440 |
| 357 | 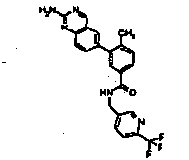 | 3-(2-amino-6-quinazolinyl)-4-methyl-N-((6-(trifluoromethyl)-3-pyridinyl)methyl)benzamide | I1 | 437.423 | 438 |
| 358 | 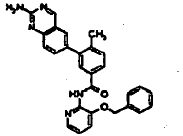 | 3-(2-amino-6-quinazolinyl)-4-methyl-N-(3-((phenylmethyl)oxy)-2-pyridinyl)benzamide | I1 | 461.523 | 462 |
| 359 | 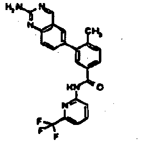 | 3-(2-amino-6-quinazolinyl)-4-methyl-N-(6-(trifluoromethyl)-2-pyridinyl)benzamide | I1 | 423.396 | 424 |
| 360 | 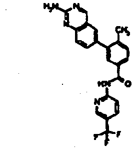 | 3-(2-amino-6-quinazolinyl)-4-methyl-N-(5-(trifluoromethyl)-2-pyridinyl)benzamide | I1 | 423.396 | 424 |
| 361 | 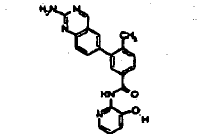 | 3-(2-amino-6-quinazolinyl)-N-(3-hydroxy-2-pyridinyl)-4-methylbenzamide | I1 | 371.398 | 372 |
| 362 | 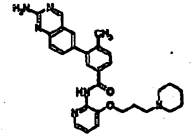 | 3-(2-amino-6-quinazolinyl)-4-methyl-N-(3-((3-(1-piperidinyl)propyl)oxy)-2-pyridinyl)benzamide | I1 | 496.612 | 497 |
| 363 | 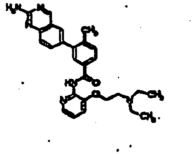 | 3-(2-amino-6-quinazolinyl)-N-(3-((2-(diethylamino)ethyl)oxy)-2-pyridinyl)-4-methylbenzamide | I1 | 470.574 | 471 |
| 364 | 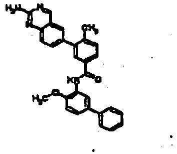 | 3-(2-amino-6-quinazolinyl)-4-methyl-N-(4-(methyloxy)-1,1'-biphenyl-3-yl)benzamide | I1 | 460.535 | 459 (M-H⁺) |
| 365 | 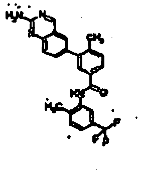 | 3-(2-amino-6-quinazolinyl)-4-methyl-N-(2-methyl-5-(trifluoromethyl)phenyl)benzamide | I1 | 436.435 | 435 (M-H⁺) |
| 366 (Reference Example) | 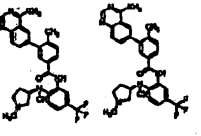 | 3-(4-amino-6-quinazolinyl)-4-methyl-N-(2-(methy)((3S)-1-methyl-3-pyrrolidinyl)amino)-5-(trifluoromethyl)phenyl)benzamide | I1 | 534.583 | 535 |
| 367 (Reference Example) | 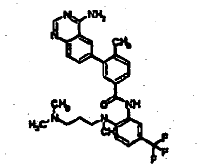 | 3-(4-amino-quinazolinyl)-N-(2-((3-(dimethylamino)propyl)(methyl)amino)-5-(trifluoromethyl)phenyl)-4-methylbenzamide | I1 | 536.599 | 537 |
| 368 (Reference Example) | 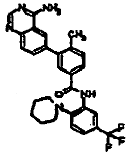 | 3-(4-amino-6-quinazolinyl)-4-methyl-N-(2-(1-piperidinyl)-5-(trifluoromethyl)phenyl)benzamide | I1 | 505.541 | 506 |
| 369 | 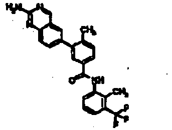 | 3-(2-amino-6-quinazolinyl)-4-methyl-N-(2-methyl-3-(trifluoromethyl)phenyl)benzamide | I-1 | 436.435 | 437.0 |
| 370 | 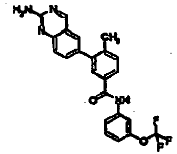 | 3-(2-amino-6-quinazolinyl)-4-methyl-N-(3-((trifluoromethyl)oxy)phenyl)benzamide | I-1 | 438.407 | 439.1 |
| 371 | 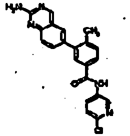 | 3-(2-amino-6-quinazolinyl)-N-(6-chloro-3-pyridinyl)-4-methylbenzamide | I-1 | 389.844 | 390.1 |
| 372 | 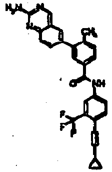 | 3-(2-amino-6-quinazolinyl)-N-(4-(cyclopropylethynyl)-3-(trifluoromethyl)phenyl)-4-methylbenzamide | I-1 | 486.495 | 487.2 |
| 373 | 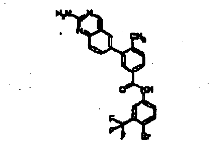 | 3-(2-amino-6-quinazolinyl)-N-(4-bromo-3-(trifluoromethyl)phenyl)-4-methylbenzamide | I-1 | 501.304 | 502.0 |
| 374 | 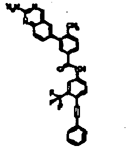 | 3-(2-amino-6-quinazolinyl)-4-methyl-N-(4-(phenylethynyl)-3-(trifluoromethyl)phenyl)benzamide | 1-1 | 522.528 | 523.1 |

Note that Examples 372 and 374 were synthesized using the aniline intermediates of Examples 563 and 564, respectively, described herein below:

### Experimental method 12 (Reference Method)

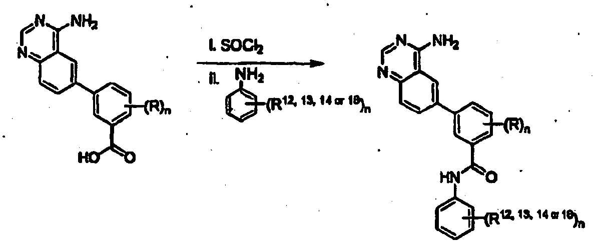

### Example 375 (Reference Example)

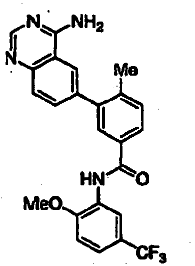

### Synthesis of 3-(4-amino-6-quinazolinyl)-4-methyl-N-(2-methoxy)-5-(trifluoromethyl)phenyl)benzamide

3-(4-Aminoquinazolin-6-yl)-4-methylbenzoic acid (0.032 g, 0.11 mmol) was suspended in neat thionyl chloride (0.5 mL) at ambient temperature. The mixture was heated at reflux for 1 h, then cooled to ambient temperature. The resulting solution was concentrated *in vacuo,* then diluted with toluene and concentrated a second time. The crude solid was dried under high vacuum for 0.5 h. To a solution of the crude solid in anhydrous methylene chloride (1.1 ml), was added 2-methoxy-5-trifluoromethylaniline (0.023 g, 0.11 mmol) and triethylamine (0.080 mL, 0.55 mmol). The solution was stirred at ambient temperature for 15 h then concentrated *in vacuo*. Purification by silica chromatography (5% methanol/methylene chloride) afforded 3-(4-amino-6-quinazolinyl)-4-methyl-N-(2-methoxy)-5-(trifluoromethyl)phenyl)benzamide as a white solid. MS (MH⁺) 453.4; Calculated for C₂₄H₁₉F₃N₄O₂ : 452.

The following Examples 376-408 were prepared by a method similar to that described in Experimental Method I1 and Example 375. **The following Examples 376 to 408 are Reference Examples.**

| Example No. | Structure | Name | Method | MW | (M+H⁺) |
|---|---|---|---|---|---|
| 376 | 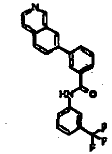 | 3-(7-isoquinolinyl)-N-(3-(trifluoromethyl)phenyl)benzamide | I2 | 392.379 | 393.1 |
| 377 | 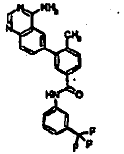 | 3-(4-amino-6-quinazolinyl)-4-methyl-N-(3-(trifluoromethyl)phenyl)benzamide | 12 | 422.408 | 423 |
| 378 | 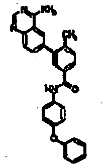 | 3-(4-amino-6-quinazolinyl)-4-methyl-N-(4-(phenyloxy)phenyl)benzamide | I2 | 446.508 | 447 |
| 379 | 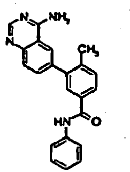 | 3-(4-amino-6-quinazolinyl)-4-methyl-N-phenylbenzamide | I2 | 354.411 | 355 |
| 380 | 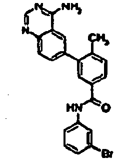 | 3-(4-amino-6-quinazolinyl)-N-(3-bromophenyl)-4-methylbenzamide | I2 | 433.307 | 433.3 |
| 381 | 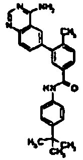 | 3-(4-amino-6-quinazolinyl)-N-(4-(1,1-dimethylethyl)phenyl)-4-methylbenzamide | 12 | 410.518 | 411 |
| 382 | 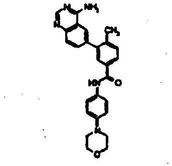 | 3-(4-amino-6-quinazolinyl)-4-methyl-N-(4-(4-morpholinyl)phenyl)benzamide | 12 | 439.517 | 440 |
| 383 | 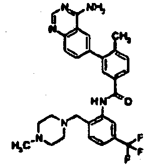 | 3-(4-amino-6-quinazolinyl)-4-methyl-N-(2-((4-methyl-1-piperazinyl)methyl)-5-(trifluoromethyl)phenyl)benzamide | 12 | 534.583 | 535 |
| 384 | 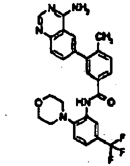 | 3-(4-amino-6-quinazolinyl)-4-methyl-N-(2-(4-morpholinyl)-5-(trifluoromethyl)phenyl)benzamide | I2 | 507.514 | 508 |
| 385 | 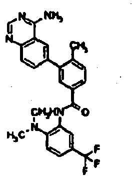 | 3-(4-amino-6-quinazolinyl)-N-(2-(dimethylamino)-5-(trifluoromethyl)phenyl)-4-methylbenzamide | I2 | 465.477 | 466 |
| 386 | 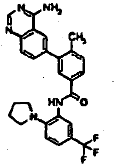 | 3-(4-amino-6-quinazolinyl)-4-methyl-N-(2-(1-pyrrolidinyl)-5-(trifluoromethyl)phenyl)benzamide | 12 | 491.515 | 492 |
| 387 | 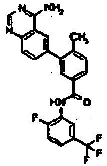 | 3-(4-amino-6-quinazolinyl)-N-(2-fluoro-5-(trifluoromethyl)phenyl)-4-methylbenzamide | 12 | 440.398 | 441 |
| 388 | 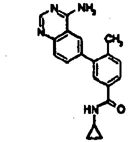 | 3-(4-amino-6-quinazolinyl)-N-cyclopropyl-4-methylbenzamide | I2 | 318.378 | 319 |
| 389 | 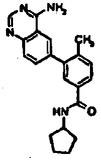 | 3-(4-amino-6-quinazolinyl)-N-cyclopentyl-4-methylbenzamide | 12 | 346.432 | 347 |
| 390 | 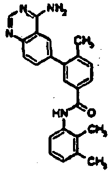 | 3-(4-amino-6-quinazolinyl)-N-(2,3-dimethylphenyl)-4-methylbenzamide | 12 | 382.465 | 383 |
| 391 | 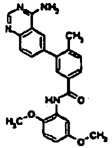 | 3-(4-amino-6-quinazolinyl)-N-(2,5-bis(methyloxy)phenyl)-4-methylbenzamide | I2 | 414.463 | 415 |
| 392 | 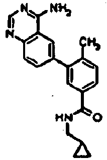 | 3-(4-amino-6-quinazolinyl)-N-(cyclopropylmethyl)-4-methylbenzamide | I2 | 332.405 | 333 |
| 393 | 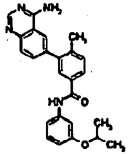 | 3-(4-amino-6-quinazolinyl)-4-methyl-N-(3-((1-methylethyl)oxy)phenyl)benzamide | I2 | 412.491 | 413 |
| 394 | 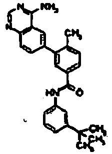 | 3-(4-amino-6-quinazolinyl)-N-(3-(1,1-dimethylethyl)phenyl)-4-methylbenzamide | I2 | 410.518 | 411 |
| 395 | 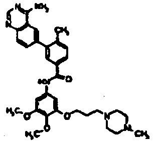 | 3-(4-amino-6-quinazolinyl)-N-(3,4-bis(methyloxy)-5-((3-(4-methyl-1-piperazinyl)propyl)oxy)phenyl)-4-methylbenzamide | I2 | 570.69 | 571 |
| 396 | 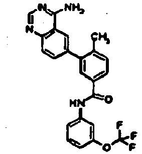 | 3-(4-amino-6-quinazolinyl)-4-methyl-N-(3-((trifluoromethyl)oxy)phenyl)benzamide | 12 | 438.407 | 439 |
| 397 | 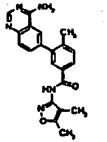 | 3-(4-amino-6-quinazolinyl)-N-(4,5-dimethyl-3-isoxazolyl)-4-methylbenzamide | I2 | 373.414 | 374 |
| 398 | 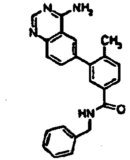 | 3-(4-amino-6-quinazolinyl)-4-methyl-N-(phenylmethyl)benzamide | I2 | 368.438 | 369 |
| 399 | 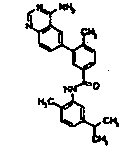 | 3-(4-amino-6-quinazolinyl)-4-methyl-N-(2-methyl-5-(1-methylethyl)phenyl)benzamide | 12 | 410.518 | 411 |
| 400 | 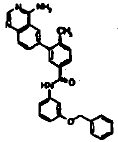 | 3-(4-amino-6-quinazolinyl)-4-methyl-N-(3-((phenylmethyl)oxy)phenyl)benzamide | I2 | 460.535 | 461 |
| 401 | 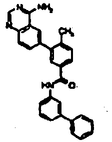 | 3-(4-amino-6-quinazolinyl)-N-(1,1'-biphenyl-3-yl)-4-methylbenzamide | I2 | 430.509 | 431 |
| 402 | 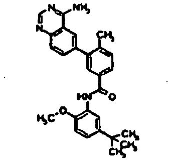 | 3-(4-amino-6-quinazolinyl)-N-(5-(1.1-dimethylethyl)-2-(methyloxy)phenyl)-4-methylbenzamide | 12 | 440.544 | 441 |
| 403 | 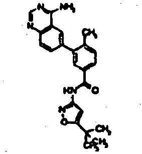 | 3-(4-amino-6-quinazolinyl)-N-(5-(1,1-dimethylethyl)-3-isoxazolyl)-4-methylbenzamide | I2 | 401.468 | 402 |
| 404 | 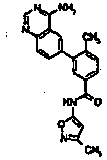 | 3-(4-amino-6-quinazolinyl)-4-methyl-N-(3-methyl-5-isoxazolyl)benzamide | 12 | 359.387 | 360 |
| 405 | 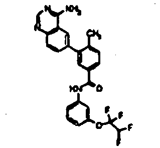 | 3-(4-amino-6-quinazolinyl)-4-methyl-N-(3-((1,1,2,2-tetrafluoroethyl)oxy)phenyl)benzamide | 12 | 470.424 | 471 |
| 406 | 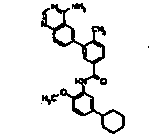 | 3-(4-amino-quinazolinyl)-N-(5-cyclohexyl-2-(methyloxy)phenyl)-4-methylbenzamide | 12 | 466.582 | 467 |
| 407 | 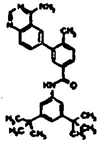 | 3-(4-amino-6-quinazolinyl)-N-(3,5-bis(1,1-dimethylethyl)phenyl)-4-methylbenzamide | I2 | 466.626 | 467 |
| 408 | 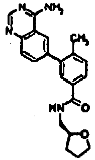 | 3-(4-amino-6-quinazolinyl)-4-methyl-N-((2S)-tetrahydro-2-furanylmethyl)benzamide | 12 | 362.431 | 363 |

### Experimental Method I3

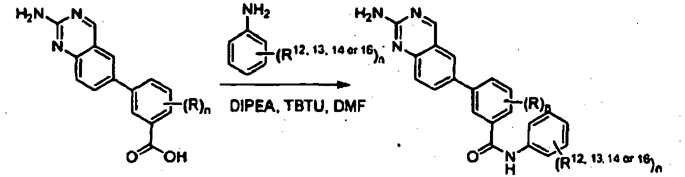

### Example 409

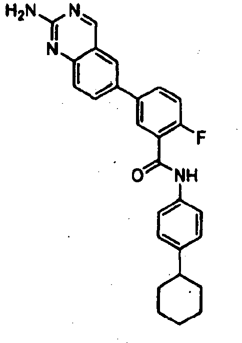

### Synthesis of '5-(2-amino-6-quinazolinyl)-N-(4-cyclohexylphenyl)-2-fluorobenzamide

4-cyclohexylbenzenamine(0.034 g, 0.194 mmol) was added to 5-(2-aminoquinazolin-6-yl)-2-fluorobenzoic acid (0.050 g, 0.177 mmol) in DMF (1 ml). TBTU (0.068 g, 0.212 mmol) was added, followed by DIPEA (0.045 g, 0.353 mmol). The mixture was stirred at RT overnight and then diluted with sodium bicarbonate and extracted (3 x 50 ml) with diethyl ether. The organic layer was washed with water (3 x 50 ml), dried over magnesium sulfate and concentrated. The residue was purified by flash chromatography on silica, eluting with 20% MeOH/EtOAc, to afford the title compound as a pale yellow solid. MS *m*/*z* = 441 [M+H]⁺; Calc'd for C₂₇H₂₅FN₄O: 440

### Example 410 (Reference Example)

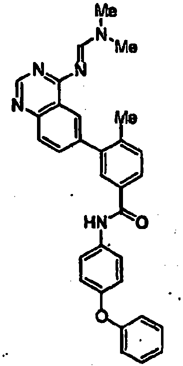

### Synthesis of 3-4(-(((1E)-(dimethylamino)methylidiene)amino)-6-quinazolinyl)-4-methyl-N-(4-phenloxy)phenyl)benzamide

To a suspension of 3-(4-aminoquinazolin-6-yl)-4-methylbenzoic acid (0.050 g, 0.18 mmol) in anhydrous methylene chloride (1.8 ml) at ambient temperature, was added oxalyl chloride (0.052 ml, 0.54 mmol) and anhydrous N,N-dimethylformamide (0.020 ml). The mixture was stirred at ambient temperature for 1 h then concentrated *in vacuo.* The crude solid was dried under high vacuum for 0.5 h. To a solution of the crude solid in anhydrous methylene chloride (1.8 ml), was added 4-phenoxyaniline (0.034 g, 0.18 mmol) and triethylamine (0.13 ml, 0.90 mmol). The solution was stirred at ambient temperature for 2 h then concentrated in vacuo. Purification by silica chromatography (2.5% methanol/methylene chloride) afforded 3-4(-(((1E)-(dimethylamino)methylidiene)amino)-6-quinazolinyl)-4-methyl-N-(4-phenloxy)phenyl)benzamide as a white solid. MS (M+H⁺) 502.5; Calculated for C₃₁H₂₇N₅O₂ - 501.

The following Examples 411-422 were prepared by a method similar to that described in Experimental Method I3 and Examples 409 and 410.

| Example No. | Structure | Name | Method | MW | MS Data |
|---|---|---|---|---|---|
| 411 | 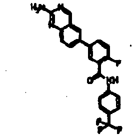 | 5-(2-amino-6-quinazolinyl)-2-fluoro-N-(4-(trifluoromethyl)phenyl)benzamide | I3 | 426.372 | 427 |
| 412 | 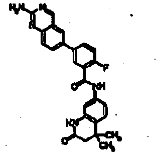 | 5-(2-amino-6-quinazolinyl)-N-(4,4-dimethyl-2-oxo-1,2,3,4-tetrahydro-7-quinolinyl)-2-fluorobenzamide | 13 | 455.491 | 456 |
| 413 | 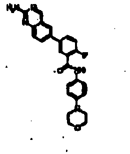 | 5-(2-amino-6-quinazolinyl)-2-fluoro-N-(4-(4-morpholinyl)phenyl)benzamide | 13 | 443.48 | 444 |
| 414 | 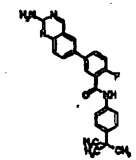 | 5-(2-amino-6-quinazolinyl)-N-(4-(1,1-dimethylethyl)phenyl)-2-fluorobenzamide | 13 | 414.482 | 415 |
| 415 (Reference Example) | 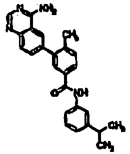 | 3-(4-amino-6-quinazolinyl)-4-methyl-N-(3-(1-methylethyl)phenyl)benzamide | I3 | 396.492 | 397 |
| 416 (Reference Example) | 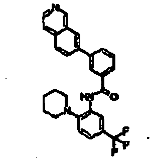 | 3-(7-isoquinolinyl)-N-(2-(1-piperidinyl)-5-(trifluoromethyl)phenyl)benzamide | I3 | 475.512 | 476.2 |
| 417 | 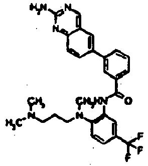 | 3-(2-amino-6-quinazolinyl)-N-(2-((3-(dimethylamino)propyl)(methyl)amino)-5-(trifluoromethyl)phenyl)benzamide | I3 | 522.572 | 523.2 |
| 418 | 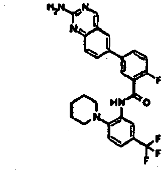 | 5-(2-amino-6-quinazolinyl)-2-fluoro-N-(2-(1-piperidinyl)-5-(trifluoromethyl)phenyl)benzamide | I3 | 509.505 | 510.2 |
| 419 | 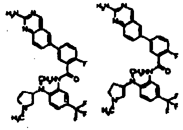 | 5-(2-amino-quinazolinyl)-2-fluoro-N-(2-(methyl((3R)-1-methyl-3-pyrrolidinyl)amino)-5-(trifluoromethyl)phenyl)benzamide | I3 | 538.546 | 539.2 |
| 420 | 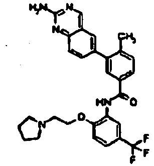 | 3-(2-amino-6-quinazolinyl)-4-methyl-N-(2-((2-(1-pyrrolidinyl)ethyl)oxy)-5-(trifluoromethyl)phenyl)benzamide | 13 | 535.567 | 536.3 |
| 421 | 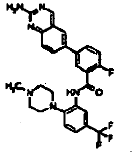 | 5-(2-amino-6-quinazolinyl)-2-fluoro-N-(2-(4-methyl-1-piperazinyl)-5-(trifluoromethyl)phenyl)benzamide | 13 | 524.52 | 525.2 |
| 422 | 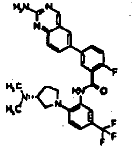 | 5-(2-amino-6-quinazolinyl)-N-(2-((3R)-3-(dimethylamino)-1-pyrrolidinyl)-5-(trifluoromethyl)phenyl)-2-fluorobenzamide | I3 | 538.546 | 539.3 |

### Experimental Method J

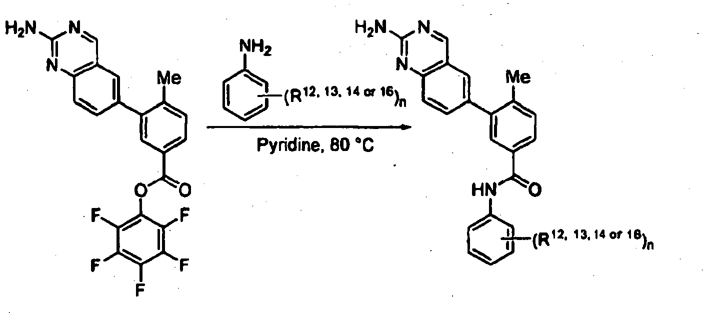

### Example 423

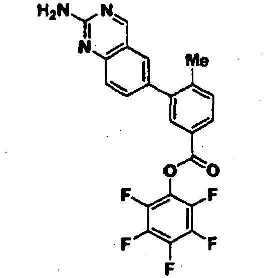

### Synthesis of Perfluorophenyl-3-(2-aminoquinazolin-6-yl)-4-methylbenzoate

To a solution of 3-(2-aminoquinazolin-6-yl)-4-methylbenzoic acid (0.50 g, 1.80 mmol) and pentafluorophenol (0.36 g, 1.98 mmol) in anhydrous N,N-dimethylformamide (18 ml), was added 1-(3-dimethylaminopropyl)-3-ethylcarbodimide hydrochloride (0.38 g, 1.98 mmol). The solution was stirred at ambient temperature for 15 h, then diluted with water (50 ml). The mixture was extracted with ethyl acetate and the organic extracts washed with water, saturated aqueous sodium bicarbonate, and saturated aqueous sodium chloride. The organic extracts were then dried over anhydrous magnesium sulfate, filtered, and concentrated *in vacuo.* Purification on silica (75% ethyl acetate/hexanes) afforded perfluorophenyl-3-(2-aminoquinazolin-6-yl)-4-methylbenzoate as a white solid. MS (MH⁺) 446.2; Calc'd for C₂₂H₁₂F₅N₃O₂ - 445.

### Example 424

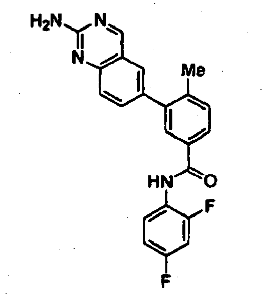

### Synthesis of 3-(2-amino-6-quinazolinyl)-N-(2,4-difluorophenyl)-4-methylbenzamide

To a solution of perfluorophenyl-3-(2-aminoquinazolin-6-yl)-4-methylbenzoate (0.041 g, 0.092 mmol) in anhydrous pyridine (1.0 ml), was added 2,4-difluoroaniline (0.012 ml, 0.11 mmol). The solution was heated to 80 °C for a period of 15 h, then cooled to ambient temperature and concentrated *in vacuo.* Purification by silica chromatography (2.5 % methanol/methylene chloride) afforded 3-(2-amino-6-quinazolinyl)-N-(2,4-difluorophenyl)-4-methylbenzamide as a white solid. MS (MH⁺) 391.0; Calculated for C₂₂H₁₆F₂N₄O: 390.

The following Examples 425-429 were prepared by a method similar to that described in Experimental Method J and Example 424.

| Example No. | Structure | Name | Method | MW | MS Data |
|---|---|---|---|---|---|
| 425 | 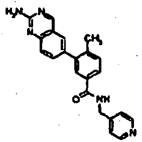 | 3-(2-amino-6-quinazolinyl)-4-methyl-N-(4-pyridinylmethyl)benzamide | J | 369.426 | 370 |
| 426 | 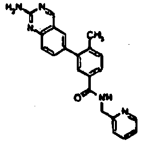 | 3-(2-amino-6-quinazolinyl)-4-methyl-N-(2-pyridinylmethyl)benzamide | J | 369.426 | 370 |
| 427 | 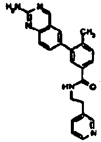 | 3-(2-amino-6-quinazolinyl)-4-methyl-N-(2-(3-pyridinyl)ethyl)benzamide | J | 383.453 | 384 |
| 428 | 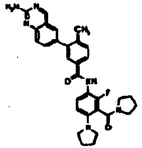 | 3-(2-amino-6-quinazolinyl)-N-(2-fluoro4-(1-pyrrolidinyl)-3-(1-pyrrolidinylcarbonyl)phenyl)-4-methylbenzamide | J | 538.624 | 539 |
| 429 | 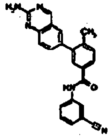 | 3-(2-amino-6-quinazolinyl)-N-(3-cyanophenyl)-4-methylbenzamide | J | 379.421 | 380 |

### Experimental Method K1

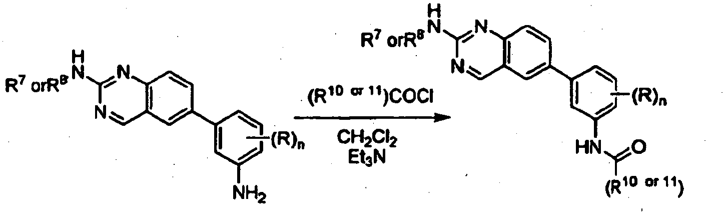

### Example 430

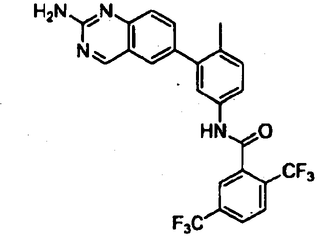

### Synthesis of N-(3-(2-amino-6-quinazolinyl)-4-methylphenyl)-2,5-bis(trifluoromethyl)benzamide

2,5-Bis(trifluromethyl)benzoyl chloride (0.058 g, 0.038 mL, 0.21 mmol) was added to a solution of 6-(5-amino-2-methylphenyl)quinazolin-2-amine (0.050 g, 0.20 mmol) in dichloromethane (2 ml), and the mixture stirred at room temperature for 3 h. Triethylamine (0.026 g, 0.036 ml, 0.26 mmol) was added and the solution stirred at room temperature for 15 minutes. The resulting suspension was concentrated to afford an off-white solid. Trituration with dichloromethane and filtering afforded N-(3-(2-amino-6-quinazolinyl)-4-methylphenyl)-2,5-bis(trifluoromethyl)benzamide as a white solid. MS (Mfi+) 491.1; Calculated for C₂₄H₁₆F₆N₄O: 490.

### Example 431

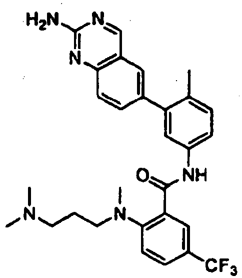

### Synthesis of N-(3-(2-amino-6-quinazolinyl)-4-methylphenyl)-2-((3-(dimethylamino)propyl)(methyl)amino)-5-(trifluoromethyl)benzamide

To a solution of N-(3-(2-aminoquinazolin-6-yl)-4-methylphenyl)-2-fluoro-5-(trifluoromethyl)benzamide (33.8 mg, 0.077 mmol, prepared using general method K1) in DMSO (3 ml) under N₂ was added *N*¹,*N*¹,*N*³-trimethylpropane-1,3-diamine (0.013 ml, 0.084 mmol). The resulting mixture was heated at 80°C for 20 h. The reaction was cooled to room temperature and poured onto water. The resulting white precipitate was collected via filtration and dried under vacuum to afford N-(3-(2-amino-6-quinazolinyl)-4-methylphenyl)-2-((3-(dimethylamino)propyl)(methyl)amino)-5-(trifluoromethyl)benzamide as a white solid: MS m/z = 537.2 [M+H]⁺. Calc'd for C₂₉H₃₁F₃N₆O: 536.6.

### Example 432

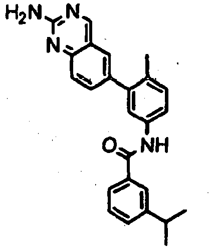

### Synthesis of N-(3-(2-aminoquinazolin-6-yl)-4-methylphenyl)-3-isopropylbenzamide

Step 1: To a solution of 4-methyl-3-(4,4,5,5-tetramethyl-1,3,2-dioxaborolan-2-yl)benzenamine (126 mg, 0.54 mmol) and 6-bromoquinazolin-2-amine (110 mg, 0.49 mmol) in toluene (5 ml) and ethanol (1 ml) was added Pd(PPh₃)₄ (113 mg, 0.10 mmol)and sodium carbonate (2M, aqueous, 0.736 mL, 1.47 mmol). The reaction vessel was purged with nitrogen and heated at 80°C for 12 hours. The solvent was removed under reduced pressure and the mixture was partitioned between ethyl acetate and water. The organics were washed with brine, dried with magnesium sulfate and filtered. Concentration, followed by chromatography on silica gel (5% MeOH/CH₂Cl₂) provided 6-(5-amino-2-methylphenyl)quinazolin-2-amine.
Step 2: To a suspension of 3-isopropylbenzoic acid (33 mg, 0.20 mmol) in methylene chloride (2 ml) was added DMF (1 drop) and oxalyl chloride (0.019 mL, 0.22 mmol). The reaction was stirred at room temperature for 1 hour, at which time the solvent was removed and replaced with THF (2 mL). The mixture was cooled to 0°C and 6-(5-amino-2-methylphenyl)quinazolin-2-amine (50 mg, 0.20 mmol) was added, forming a yellow precipitate. The reaction was allowed to warm to room temperature and was stirred for 2 hours. After removal of solvent under reduced pressure, the mixture was taken up in ethyl acetate, washed with 2N NaOH, then brine, and dried with magnesium sulfate. Filtration and concentration, followed by trituration with ether afforded the title compound. MS (m/z) = 397.2 (M+H+); calc'd for C₂₅H₂₄N₄O: 396.5

### Example 433

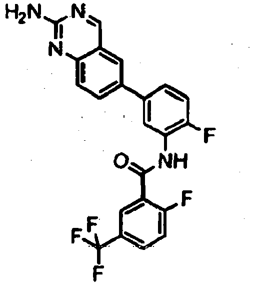

### Synthesis of N-(5-(2-aminoquinazolin-6-yl)-2-fluorophenyl)-2-fluoro-5-(trifluoromethyl)benzamide

To a solution of 6-(5-amino-2-methylphenyl)quinazolin-2-amine (500 mg, 1.97 mmol) in THF (15 ml) at room temperature was added 2-fluoro-5-(trifluoromethyl)benzoyl chloride (0.297 ml, 1.97 mmol). A white precipitate forms. The reaction is stirred at room temperature for 12 hours, at which point the solvent is removed under reduced pressure. The mixture was partitioned between ethyl acetate and aqueous saturated sodium bicarbonate. The organics were washed with brine and dried with magnesium sulfate. Filtration and concentration afforded the title compound. MS (m/z) = 445 (M+H+); calc'd for C₂₁H₁₃F₅N₄O: 444.37 Alternatively, the crude product, as an HCl salt, may be carried forward without a basic workup.

### Example 434

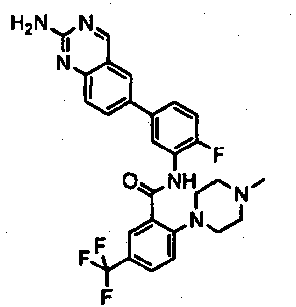

### Synthesis of N-(5-(2-aminoquinazolin-6-yl)-2-fluorophenyl)-2-(4-methylpiperazin-1-yl)-5-(trifluoromethyl)benzamide

This compound was prepared by a method similar to that described in Example 431. Specifically, to a suspension of N-(5-(2-aminoquinazolin-6-yl)-2-fluorophenyl)-2-fluoro-5-(trifluoromethyl)benzamide hydrochloride (100 mg, 0.23 mmol) in DMSO (1 ml) in a sealed tube was added N-methylpiperazine (0.050 mL, 0.45 mmol). The vessel was flushed with argon, sealed, and heated at 80°C for 4 hours. The reaction was cooled to room temperature and NaOH (2N, aqueous, 2 ml) was added. The mixture was extracted with ethyl acetate. The organics were then washed with brine, dried with magnesium sulfate and filtered. Removal of solvent under reduced pressure, followed by chromatography on silica gel (1% to 10% gradient MeOH in DCM) afforded the product. MS (m/z) = 525.2 (M+H+) ; calc'd for C₂₇H₂₄F₄N₆O: 524.53

The following Examples 435-511 were prepared by a method similar to that described in Experimental Method K1 and Examples 430-434.

| Example No. | Structure | Name | Method | MW | (M+H⁺) |
|---|---|---|---|---|---|
| 435 | 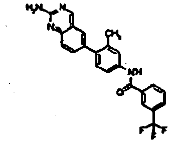 | N-(4-(2-amino-6-quinazolinyl)-3-methylphenyl)-3-(trifluoromethyl)benzamide | K1 | 422.408 | 423.1 |
| 436 | 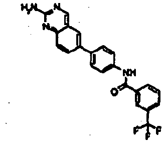 | N-(4-(2-amino-6-quinazolinyl)phenyl)-3-(trifluoromethyl)benzamide | K1 | 408.382 | 409.1 |
| 437 | 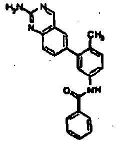 | N-(3-(2-amino-6-quinazolinyl)-4-methylphenyl)benzamide | K1 | 354.411 | 355 |
| 438 | 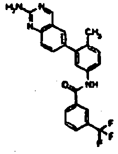 | N-(3-(2-amino-6-quinazolinyl)-4-methylphenyl)-3-(trifluoromethyl)benzamide | K1 | 422.408 | 423 |
| 439 | 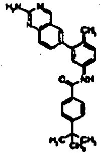 | N-(3-(2-amino-6-quinazolinyl)-4-methylphenyl)-4-(1,1-dimethylethyl)benzamide | K1 | 410.518 | 411 |
| 440 | 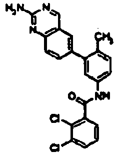 | N-(3-(2-amino-6-quinazolinyl)-4-methylphenyl)-2,3-dichlorobenzamide | K1 | 423.301 | 423 |
| 441 | 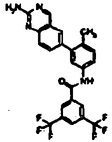 | N-(3-(2-amino-6-quinazolinyl)-4-methylphenyl)-3,5-bis(trifluoromethyl)benzamide | K1 | 490.405 | 491 |
| 442 | 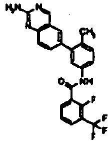 | N-(3-(2-amino-6-quinazolinyl)-4-methylphenyl)-2-fluoro-3-(trifluoromethyl)benzamide | K1 | 440.398 | 441 |
| 443 | 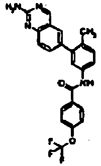 | N-(3-(2-amino-6-quinazolinyl)-4-methylphenyl)-4-((trifluoromethyl)oxy)benzamide | K1 | 438.407 | 439 |
| 444 | 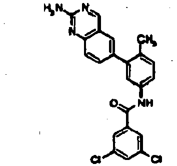 | N-(3-(2-amino-6-quinazolinyl)-4-methylphenyl)-3,5-dichlorobenzamide | K1 | 423.301 | 424 |
| 445 | 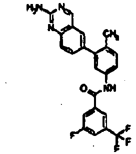 | N-(3-(2-amino-6-quinazolinyl)-4-methylphenyl)-3-fluoro-5-(trifluoromethyl)benzamide | K1 | 440.398 | 441 |
| 446 | 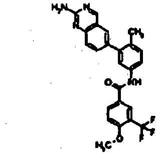 | N-(3-(2-amino-6-quinazolinyl)-4-methylphenyl)-4-(methyloxy)-3-(trifluoromethyl)benzamide | K1 | 452.434 | 453 |
| 447 | 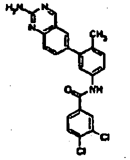 | N-(3-(2-amino-6-quinazolinyl)-4-methylphenyl)-2,3-dichlorobenzamide | K1 | 423.301 | 423 |
| 448 | 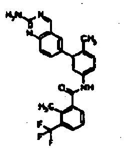 | N-(3-(2-amino-6-quinazolinyl)-4-methylphenyl)-2-methyl-3-(trifluoromethyl)benzamide | K1 | 436.435 | 437 |
| 449 | 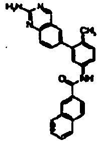 | N-(3-(2-amino-6-quinazolinyl)-4-methylphenyl)-2-naphthalenecarboxamide | K1 | 404.471 | 405 |
| 450 | 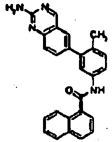 | N-(3-(2-amino-6-quinazolinyl)-4-methylphenyl)-1-naphthalenecarboxamide | K1 | 404.471 | 405 |
| 451 | 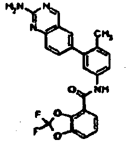 | N-(3-(2-amino-6-quinazolinyl)-4-methylphenyl)-2,2-difluoro-1,3-benzodioxole-4-carboxamide | K1 | 434.4 | 435 |
| 452 | 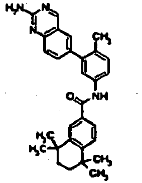 | N-(3-(2-amino-6-quinazolinyl)-4-methylphenyl)-5,5,8,8-tetramethyl-5,6,7,8-tetrahydro-2-naphthalenecarboxamide | K1 | 464.61 | 465 |
| 453 | 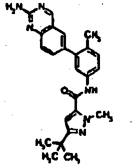 | N-(3-(2-amino-6-quinazolinyl)-4-methylphenyl)-3-(1,1-dimethylethyl)-1-methyl-1H-pyrazole-5-carboxamide | K1 | 414.51 | 415 |
| 454 | 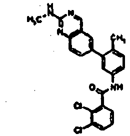 | 2,3-dichloro-N-(4-methyl-3-(2-(methylamino)-6-quinazolinyl)phenyl)benzamide | K1 | 437.328 | 437 |
| 455 | 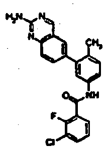 | N-(3-(2-amino-6-quinazolinyl)-4-methylphenyl)-3-chloro-2-fluorobenzamide | K1 | 406.846 | 407 |
| 456 | 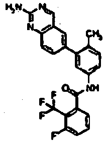 | N-(3-(2-amino-6-quinazolinyl)-4-methylphenyl)-3-fluoro-2-(trifluoromethyl)benzamide | K1 | 440.398 | 441 |
| 457 | 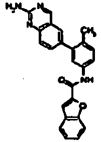 | N-(3-(2-amino-6-quinazolinyl)-4-methylphenyl)-1-benzofuran-2-carboxamide | K1 | 394.432 | 395 |
| 458 | 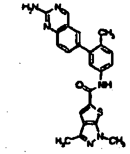 | N-(3-(2-amino-6-quinazolinyl)-4-methylphenyl)-1,3-dimethyl-1H-thieno[2,3-c]pyrazole-5-carboxamide | K1 | 428.518 | 429 |
| 459 | 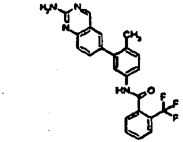 | N-(3-(2-amino-6-quinazolinyl)-4-methylphenyl)-2-(trifluoromethyl)benzamide | K1 | 422.408 | 423.1 |
| 460 | 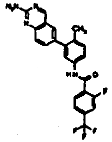 | N-(3-(2-amino-6-quinazolinyl)-4-methylphenyl)-2-fluoro-4-(trifluoromethyl)benzamide | K1 | 440.398 | 441.1 |
| 461 | 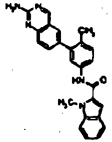 | N-(3-(2-amino-6-quinazolinyl)-4-methylphenyl)-1-methyl-1H-indole-2-carboxamide | K1 | 407.475 | 408.2 |
| 462 | 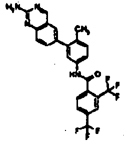 | N-(3-(2-amino-6-quinazolinyl)-4-methylphenyl)-2,4-bis(trifluoromethyl)benzamide | K1 | 490.405 | 491.1 |
| 463 | 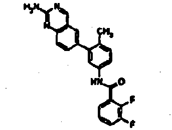 | N-(3-(2-amino-6-quinazolinyl)-4-methylphenyl)-2,3-difluorobenzamide | K1 | 390.391 | 391.1 |
| 464 | 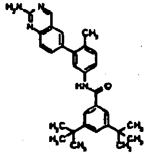 | N-(3-(2-amino-6-quinazolinyl)-4-methylphenyl)-3,5-bis(1,1-dimethylethyl)benzamide | K1 | 466.626 | 467.3 |
| 465 | 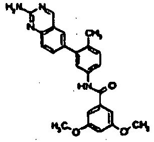 | N-(3-(2-amino-6-quinazolinyl)-4-methylphenyl)-3,5-bis(methyloxy)benzamide | K1 | 414.463 | 415.1 |
| 466 | 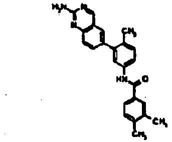 | N-(3-(2-amino-6-quinazolinyl)-4-methylphenyl)-3,4-dimethylbenzamide | K1 | 382.465 | 383.2 |
| 467 | 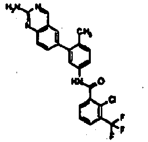 | N-(3-(2-amino-6-quinazolinyl)-4-methylphenyl)-2-chloro-3-(trifluoromethyl)benzamide | K1 | 456.853 | 457.0 |
| 468 | 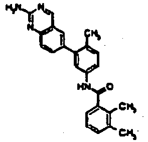 | N-(3-(2-amino-6-quinazolinyl)-4-methylphenyl)-2,3-dimethylbenzamide | K1 | 382.465 | 383.2 |
| 469 | 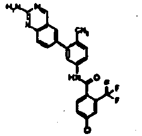 | N-(3-(2-amino-6-quinazolinyl)-4-methylphenyl)-4-chloro-2-(trifluoromethyl)benzamide | K1 | 456.853 | 457.1 |
| 470 | 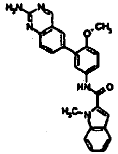 | N-(3-(2-amino-6-quinazolinyl)-4-(methyloxy)phenyl)-1-methyl-1H-indole-2-carboxamide | K1 | 423.474 | 424.2 |
| 471 | 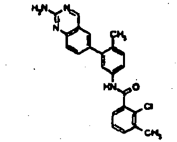 | N-(3-(2-amino-6-quinazolinyl)-4-methylphenyl)-2-chloro-3-methylbenzamide | K1 | 402.883 | 403.1 |
| 472 | 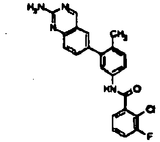 | N-(3-(2-amino-6-quinazolinyl)-4-methylphenyl)-2-chloro-3-fluorobenzamide | K1 | 406.846 | 407.1 |
| 473 | 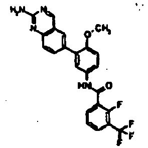 | N-(3-(2-amino-6-quinazolinyl)-4-(methyloxy)phenyl)-2-fluoro-3-(trifluoromethyl)benzamide | K1 | 456.397 | 457.1 |
| 474 | 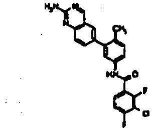 | N-(3-(2-amino-6-quinazolinyl)-4-methylphenyl)-3-chloro-2,4-difluorobenzamide | K1 | 424.836 | 425.1 |
| 475 | 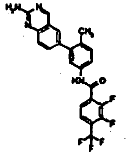 | N-(3-(2-amino-6-quinazolinyl)-4-methylphenyl)-2,3-difluoro-4-(trifluoromethyl)benzamide | K1 | 458.388 | 459.1 |
| 476 | 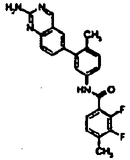 | N-(3-(2-amino-6-quinazolinyl)-4-methylphenyl)-2,3-difluoro-4-methylbenzamide | K1 | 404.418 | 405.1 |
| 477 | 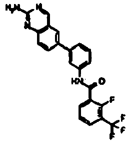 | N-(3-(2-amino-6-quinazolinyl)phenyl)-2-fluoro-3-(trifluoromethyl)benzamide | K1 | 426.372 | 427.1 |
| 478 | 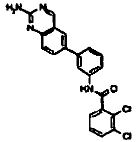 | N-(3-(2-amino-6-quinazolinyl)phenyl)-2,3-dichlorobenzamide | K1 | 409.275 | 409.1 |
| 479 | 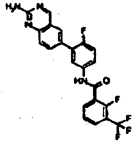 | N-(3-(2-amino-6-quinazolinyl)-4-fluorophenyl)-2-fluoro-3-(trifluoromethyl)benzamide | K1 | 444.362 | 445.1 |
| 480 | 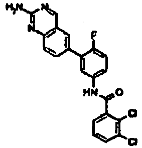 | N-(3-(2-amino-6-quinazolinyl)-4-fluorophenyl)-2,3-dichlorobenzamide | K1 | 427.265 | 427.0 |
| 481 | 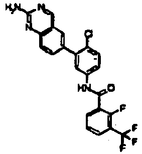 | N-(3-(2-amino-6-quinazolinyl)-4-chlorophenyl)-2-fluoro-3-(trifluoromethyl)benzamide | K1 | 460.817 | 461.0 |
| 482 | 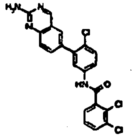 | N-(3-(2-amino-6-quinazolinyl)-4-chlorophenyl)-2,3-dichlorobenzamide | K1 | 443.72 | 443.0 |
| 483 | 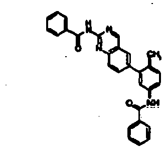 | N-(6-(2-methyl-5-((phenylcarbonyl)amino)phenyl)-2-quinazolinyl)benzamide | K1 (byproduct) | 458.519 | MH+ 459 |
| 484 | 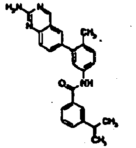 | N-(3-(2-amino-6-quinazolinyl)-4-methylphenyl)-3-(1-methylethyl)benzamide | K1 | 396.492 | 397 |
| 485 | 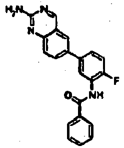 | N-(5-(2-amino-6-quinazolinyl)-2-fluorophenyl)benzamide | K1 | 358.375 | 359 |
| 486 | 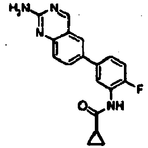 | N-(5-(2-amino-6-quinazolinyl)-2-fluorophenyl)cyclopropanecarboxamide | K1 | 322.341 | 323 |
| 487 | 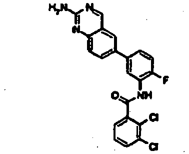 | N-(5-(2-amino-6-quinazolinyl)-2-fluorophenyl)-2,3-dichlorobenzamide | K1 | 427.265 | 428 |
| 488 | 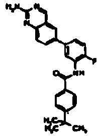 | N-(5-(2-amino-6-quinazolinyl)-2-fluorophenyl)-4-(1,1-dimethylethyl)benzamide | K1 | 414.482 | 415 |
| 489 | 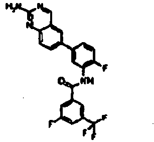 | N-(5-(2-amino-6-quinazolinyl)-2-fluorophenyl)-3-fluoro-5-(trifluoromethyl)benzamide | K1 | 444.362 | 445 |
| 490 | 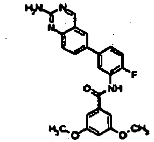 | N-(5-(2-amino-6-quinazolinyl)-2-fluorophenyl)-3,5-bis(methyloxy)benzamide | K1 | 418.426 | 419 |
| 491 | 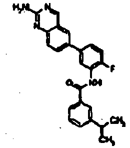 | N-(5-(2-amino-6-quinazolinyl)-2-fluorophenyl)-3-(1-methylethyl)benzamide | K1 | 400.455 | 401 |
| 492 | 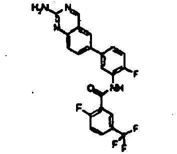 | N-(5-(2-amino-6-quinazolinyl)-2-fluorophenyl)-2-fluoro-5-(trifluoromethyl)benzamide | K1 | 444.362 | 445 |
| 493 | 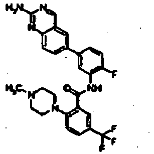 | N-(5-(2-amino-6-quinazolinyl)-2-fluorophenyl)-2-(4-methyl-1-piperazinyl)-5-(trifluoromethyl)benzamide | K1 | 524.52 | 525 |
| 494 | 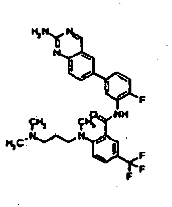 | N-(5-(2-amino-6-quinazolinyl)-2-fluorophenyl)-2-((3-(dimethylamino)propyl)(methyl)amino)-5-(trifluoromethyl)benzamide | K1 | 540.562 | 541 |
| 495 | 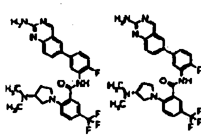 | N-(5-(2-amino-6-quinazolinyl)-2-fluorophenyl)-2-((3R)-3-(dimethylamino)-1-pyrrolidinyl)-5-(trifluoromethyl)benzamide | K1 | 538.546 | 539 |
| 496 | 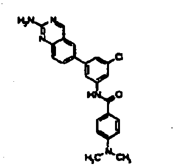 | N-(3-(2-amino-6-quinazolinyl)-5-chlorophenyl)-4-(dimethylamino)benzamide | K1-a | 417.898 | 418 |
| 497 | 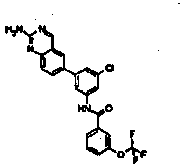 | N-(3-(2-amino-6-quinazolinyl)-5-chlorophenyl)-3-((trifluoromethyl)oxy)benzamide | K1-a | 458.826 | 459 |
| 498 | 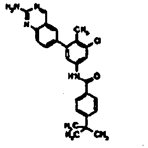 | N-(3-(2-amino-6-quinazolinyl)-5-chloro-4-methylphenyl)-4-(tert-butyl)benzamide | K1-a | 444.964 | 445 |
| 499 | 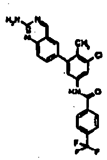 | N-(3-(2-amino-6-quinazolinyl)-5-chloro-4-methylphenyl)-4-(trifluoromethyl)benzamide | K1-a | 456.853 | 457.8 |
| 500 | 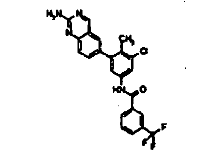 | N-(3-(2-amino-6-quinazolinyl)-5-chloro-4-methylphenyl)-3-(trifluoromethyl)benzamide | K1-a | 456.853 | 457 |
| 501 | 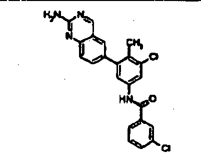 | N-(3-(2-amino-6-quinazolinyl)-5-chloro-4-methylphenyl)-3-chlorobenzamide | K1-a | 423.301 | 423 |
| 502 | 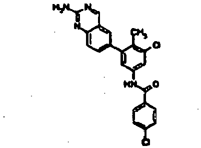 | N-(3-(2-amino-6-quinazolinyl)-5-chloro-4-methylphenyl)-4-chlorobenzamide | K1-a | 423.301 | 423 |
| 503 | 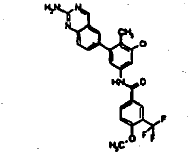 | N-(3-(2-amino-6-quinazolinyl)-5-chloro-4-methylphenyl)-4-(methyloxy)-3-(trifluoromethyl)benzamide | K1-a | 486.879 | 487 |
| 504 | 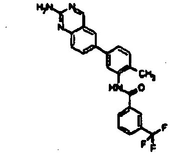 | N-(5-(2-amino-6-quinazolinyl)-2-methylphenyl)-3-(trifluoromethyl)benzamide | K1-b | 422.408 | 423 |
| 505 | 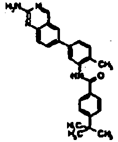 | N-(5-(2-amino-6-quinazolinyl)-2-methylphenyl)-4-(1,1-dimethylethyl)benzamide | K1-b | 410.518 | 411 |
| 506 | 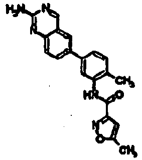 | N-(5-(2-amino-6-quinazolinyn-2-methylphenyl)-5-methyl-3-isoxazolecarboxamide | K1-b | 359.387 | 360 |
| 507 | 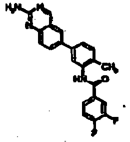 | N-(5-(2-amino-6-quinazolinyl)-2-methylphenyl)-3,4-difluorobenzamide | K1-b | 390.391 | 391 |
| 508 | 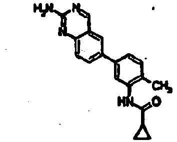 | N-(5-(2-amino-6-quinazolinyl)-methylphenyl)cyclopropanecarboxamide | K1-b | 318.378 | 319 |
| 509 | 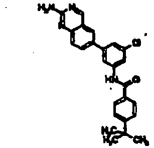 | N-(3-(2-amino-6-quinazolinyl)-5-chlorophenyl)-4-(1,1-dimethylethyl)benzamide | K1-b | 430.937 | 431 |
| 510 | 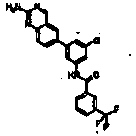 | N-(3-(2-amino-6-quinazolinyl)-5-chlorophenyl)-3-(trifluoromethyl)benzamide | K1-c | 442.827 | 443 |
| 511 (Reference Example) | 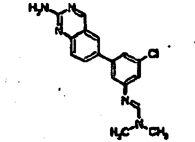 | N'-(3-(2-amino-6-quinazolinyl)-5-chlomphenyl)-N,N-dimethylformamadine | K1-c | 325.8 | 326 |

Note that Examples 496-503, 504-509 and 510-511 were synthesized by methods K1-a, K1-b and K1-c, respectively. K1-a followed the same method described in K1 except potassium carbonate was used instead of triethylamine as the base and N,N-dimethylacetamide/dichloromethane (2:5) was used instead of dichloromethane as the solvent. K1-b followed the same method described in K1 except potassium carbonate was used instead of triethylamine as the base. K1-c followed the same method described in K1 except potassium carbonate is used instead of triethylamine as the base and N,N-dimethylformamide is used instead dichloromethane as the solvent.

### Experimental Method K2

### Example 512

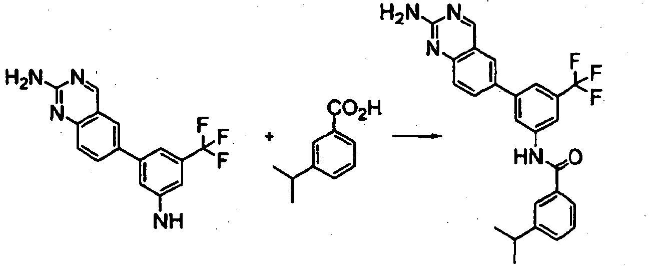

### Synthesis of N-(3-(2-amino-6-quinazolinyl)-S-(trifluoromethyl)phenyl)-3-(1-methylethyl)benzamide

A mixture of 6-(3-amino-5-(trifluoromethyl)phenyl)quinazolin-2-amine (0.032 g, 0.10 mmol), 3-isopropylbenzoic acid (0.017 g, 0.10 mmol), HATU (0.051 g, 0.135 mmol), iPr₂NEt (0.036 ml, 0.208 mmol), and 1 ml CHCl₃ was stirred at ambient temperature for 60 h, and was then sealed and heated to 70°C for 24 h. The reaction was concentrated *in vacuo* to yield a crude solid. The crude mixture was purified by reverse phase chromatography (acetonitrile/water/TFA) followed by preparative TLC (DCM/MeOH/conc. NH₄OH) to give the title compound. MS (ES⁺): 451.0 (M+H)⁺; Calc'd for C₂₅H₂₁F₃N₉O: 450.46.

### Experimental Method K3 (Reference Method)

### Example 513 (Reference Example)

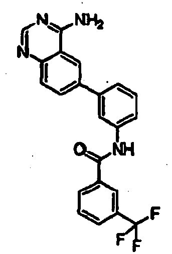

### Synthesis of N-(3-(4-amino-6-quinazolinyl)phenyl)-3-(trifluoromethyl)benzamide

### Step 1. Preparation of 6-(3-aminophenyl)quinazolin-4-amine

To 6-bromoquinazolin-4-amine (448 mg, 2.0 mmol), 3-aminophenylboronic acid (301 mg, 2.2 mmol), Pd(PPh₃)₄ (115 mg, 0.10 mmol), and Na₂CO₃ (1.00 g, 10.0 mmol) was added toluene (16 ml) and EtOH (4 ml). The mixture was stirred for 72 hours at 90°C, filtered and concentrated. The residue was diluted with EtOAc, washed with saturated NaCl, acidified with 6 N HCl, and extracted with EtOAc. The aqueous layer was then neutralized (pH∼5) with Na₂CO₃, back extracted with EtOAc and concentrated to yield 6-(3-aminophenyl)quinazolin-4-amine. MS *m*/*z* = 237 [M+1]⁺. Calc'd for C₁₄H₁₂N₄: 236.28.

### Step 2. Preparation of Resin-Bound TFP-3-trifluoromethylbenzoic acid

To the TFP-resin (1.37 mmol/g, 4.0 g, 5.5 mmol) deposited in a 60 ml Quest vessel, was added 3-trifluoromethylbenzoic acid (1.56 g, 8.25 mmol), DMAP (403 mg, 3.3 mmol). The vessel was filled with 4:1 CH₂Cl₂/DMF and mixed for 30 minutes. DIC (3.8 ml, 25 mmol) was added and the reaction was mixed for 4 hours, drained, rinsed with Et₂O and dried to yield the TFP-bound benzoate.

### Step 3. Preparation of N-(3-(4-amino-6-guinazolinyl)phenyl)-3-(trifluoromethyl)benzamide

To the TFP-resin bound 3-trifluoromethylbenzoate (200 mg) was added 6-(3-aminophenyl)quinazolin-4-amine (100 mg, 0.424 mmol) in DMF (2 ml). The mixture was shaken for 48 hours at RT, filtered and the resin was washed with 2 ml of DMF. After concentration, the residue was purified by Gilson reverse-phase HPLC (0.1% TFA in H₂O/CH₃CN) to yield N-(3-(4-amino-6-quinazolinyl)phenyl)-3-(trifluoromethyl)benzamide as a white solid. MS *m*/*z* = 409 [M+1]⁺. Calc'd for C₂₂H₁₅F₃N₄O: 408.39.

The following Examples 514-517 were prepared by a method similar to that described in Experimental Methods K2 and K3 and Examples 512 and 513.

| Example No. | Structure | Name | Method | MW | (M+H⁺) |
|---|---|---|---|---|---|
| 514 | 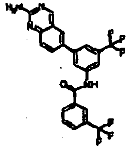 | N-(3-(2-amino-6-quinazolinyl)-5-(trifluoromethyl)phenyl)-3-(trifluoromethyl)benzamide | K3 | 476.379 | 476.9 |
| 515 (Reference Example) | 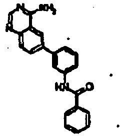 | N-(3-(4-amino-6-quinazolinyl)phenyl)benzamide | K3 | 340.384 | 341 |
| 516 (Reference Example) | 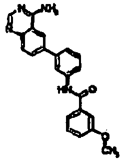 | N-(3-(4-amino-6-quinazolinyl)phenyl)-3-(methyloxy)benzamide | K3 | 370.41 | 371 |
| 517 (Reference Example) | 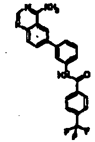 | N-(3-(4-amino-6-quinazolinyl)phenyl)-4-(trifluoromethyl)benzamide | K3 | 408.382 | 409 |

### Experimental Method L

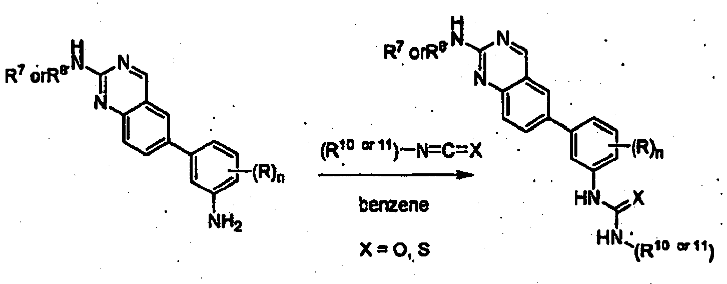

### Example 518

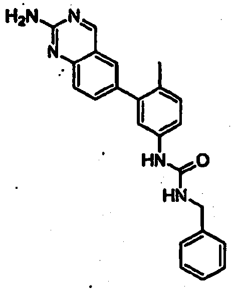

### Synthesis of N-(3-(2-amino-6-quinazolinyl)-4-methylphenyl)-N'-(phenylmethyl) urea

1-(Isocyanatomethyl)benzene (0.018 ml, 0.147 mmol)was added to 6-(5-amino-2-methylphenyl)quinazolin-2-amine (35 mg, 0.14 mmol) in benzene (2 l). The mixture was stirred at RT over night and filtered to afford the title compound as an off white solid. MS *m*/*z* = 384 [M+H]⁺. Calc'd for C₂₃H₂₁N₅O: 383

The following Examples 519-531 were prepared by a method similar to that described in Experimental Method L and Example 518.

| Example No. | Structure | Name | Method | MW | (M+H⁺) |
|---|---|---|---|---|---|
| 519 | 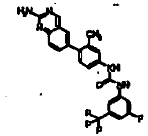 | N-(4-(2-amino-6-quinazolinyl)-methylphenyl)-N'-(3-fluoro-5-(trifluoromethyl)phenyl)urea | L | 455.413 | 456.1 |
| 520 | 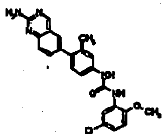 | N-(4-(2-amino-6-quinazolinyl)-3-methylphenyl)-N'-(5-chloro-2-(methyloxy)phenyl)urea | L | 433.897 | 434.1 |
| 521 | 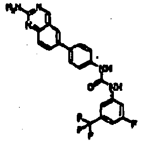 | N-(4-(2-amino-6-quinazolinyl)phenyl)-N'-(3-fluoro-5-(trifluoromethyl)phenyl)urea | L | 441.387 | 442.1 |
| 522 | 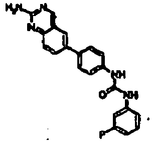 | N-(4-(2-amino-6-quinazolinyl)phenyl)-N'-(3-fluorophenyl)urea | L | 373.389 | 374.1 |
| 523 (Reference Example) | 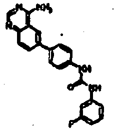 | N-(4-(4-amino-6-quinazolinyl)phenyl)-N'-(3-fluorophenyl)urea | L | 373.389 | 374.1 |
| 524 | 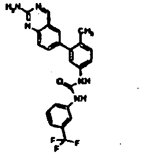 | N-(3-(2-amino-6-quinazolinyl)-4-methylphenyl)-N'-(3-(trifluoromethyl)phenyl)urea | L | 437.423 | 438 |
| 525 | 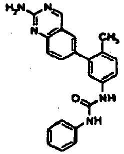 | N-(3-(2-amino-6-quinazolinyl)-4-methylphenyl)-N'-phenylurea | L | 369.426 | 370 |
| 526 | 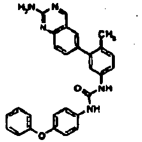 | N-(3-(2-amino-6-quinazolinyl)-4-methylphenyl)-N'-(4-(phenyloxy)phenyl)urea | L | 461.523 | 462 |
| 527 | 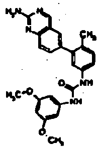 | N-(3-(2-amino-6-quinazolinyl)-4-methylphenyl)-N'-(3,5-bis(methyloxy)phenyl)urea | L | 429.478 | 430 |
| 528 | 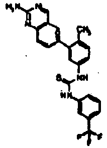 | N-(3-(2-amino-6-quinazolinyl)-4-methylphenyl)-N'-(3-(trifluoromethyl)phenyl)thiourea | L | 453.49 | 454 |
| 529 | 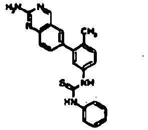 | N-(3-(2-amino-6-quinazolinyl)-4-methylphenyl)-N'-phenylthiourea | L | 385.493 | 386 |
| 530 | 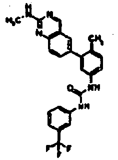 | N-(4-methyl-3-(2-(methylamino)-6-quinazolinyl)phenyl)-N'-(3-(trifluoromethyl)phenyl)urea | L | 451.45 | 453 |
| 531 | 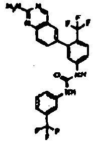 | N-(3-(2-amino-6-quinazolinyl)-4-(trifluoromethyl)phenyl)-N'-(3-(trifluoromethyl)phenyl)urea | L | 491.393 | 492 |

### Experimental Method M

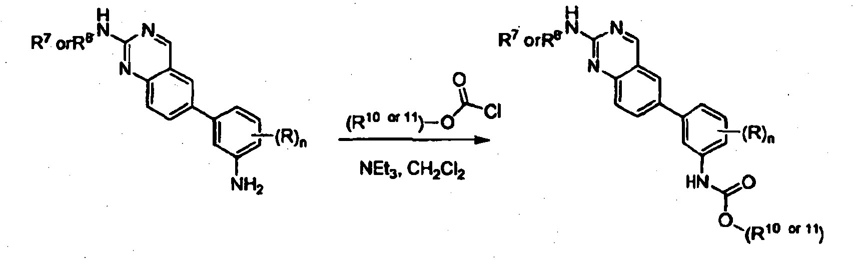

### Example 532

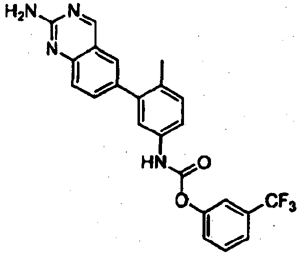

### Synthesis of 3-(trifluoromethyl)phenyl 3-(2-amino-6-quinazolinyl)-4-methylphenylcarbamate

Et₃N (0.029 ml, 0.208 mmol) and 3-(trifluoromethyl)phenyl carbonochloridate (0.028 ml, 0.176 mmol) were added to 6-(5-amino-2-methylphenyl)quinazolin-2-amine (40 mg, 0.16 mmol) in CH₂Cl₂ (1.6 mL). The mixture was allowed to stir at RT over night, then concentrated. The residue was purified by flash chromatography on silica, eluting with a gradient 0 to 10% MeOH/CH₂Cl₂, to afford 3-(trifluoromethyl)phenyl 3-(2-amino-6-quinazolinyl)-4-methylphenylcarbamate as a pale yellow solid. MS m/z = 439 [M+H]⁺. Calc'd for C₂₃H₁₇F₃N₄O₂: 438

### Experimental Method N

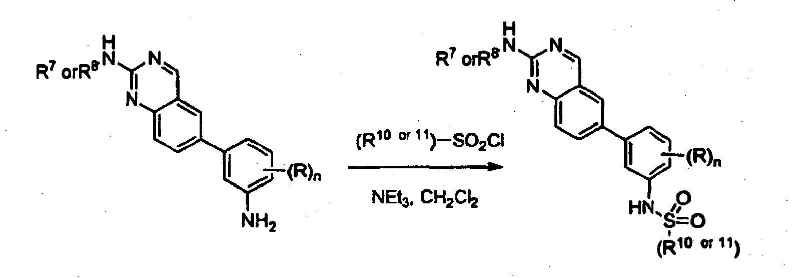

### Example 533

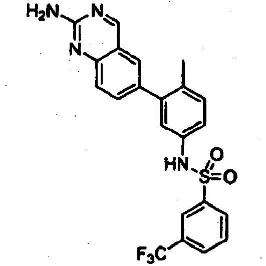

### Synthesis of N-(3-(2-amino-6-quinazolinyl)-4-methylphenyl)-3-(trifluoromethyl)benzenesulfonamide

3-(trifluoromethyl)benzene-1-sulfonyl chloride (0.031 mL, 0.20 mmol)was added to 6-(5-amino-2-methylphenyl)quinazolin-2-amine (50 mg, 0.20 mmol) in CH₂Cl₂ (2 mL). The mixture was stirred at RT over night then Et₃N (0.05 ml, 0.36 mmol) was added. The mixture was concentrated and the residue purified by flash chromatography on silica, eluting with a gradient 0 to 5% MeOH/CH₂Cl₂, to afford N-(3-(2-amino-6-quinazolinyl)-4-methylphenyl)-3-(trifluoromethyl)benzenesulfonamide as an off-white solid. MS *m*/*z* = 459 [M+H]⁺. Calc'd for C₂₂H₁₇F₃N₄O₂S : 458

### Exiperimental Method O

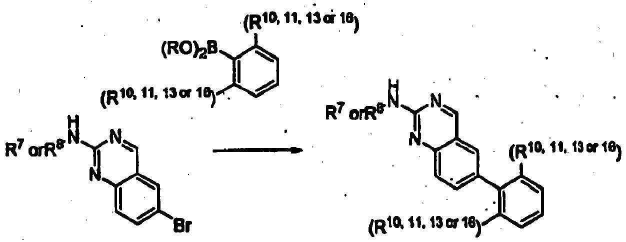

### Example 534 (Reference Example)

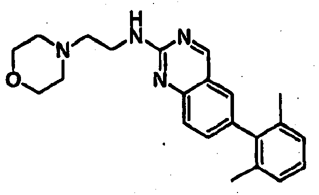

### Synthesis of 6-(2,6-dimethylphenyl)-N-(2-morpholinoethyl)quinazolin-2-amine

6-bromo-N-(2-morpholinoethyl)quinazolin-2-amine (84 mg, 0.248 mmol), 2,6-dimethylphenylboronic acid (47 mg, 0.311 mmol), K₂CO₃ (55 mg, 0.397 mmol) and Pd(dppf)Cl₂ (27 mg, 0.037 mmol) were taken up in CH₃CN (3.0 mL) and H₂O (1.0 ml) under an atmosphere of N₂. The mixture was heated in a sealed tube at 60°C for 3 h. After cooling, the mixture was taken up in CH₂Cl₂ and washed twice with H₂O, then dried over Na₂SO₄. Purification by flash chromatography on silica afforded (90/10/1 - DCM/MeOH/NH₃) 6-(2,6-dimethylphenyl)-N-(2-morpholinoethyl)quinazolin-2-amine as a pale yellow solid. MS m/z = 363 [M+H]⁺; Calc'd for C₂₂H₂₆N₄O: 362.

The following Examples 535-538 were prepared by a method similar to that described in Experimental Method O and Example 534; Examples 535 to 538 are Reference Examples.

| Example No. | Structure | Name | Method | MW | (M+H⁺) |
|---|---|---|---|---|---|
| 535 | 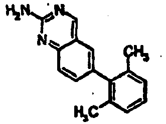 | 6-(2,6-dimethylphenyl)-2-quinazolinamine | O | 249.316 | 250.1 |
| 536 | 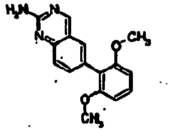 | 6-(2,6-bis(methyloxy)phenyl)-2-quinazolinamine | O | 281.314 | 282 |
| 537 | 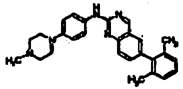 | 6-(2,6-dimethylphenyl)-N-(4-(4-methyl-1-piperazinyl)phe nyl)-2-quinazolinamlne | O | 423.561 | 424 |
| 538 | 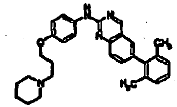 | 6-(2,6-dimethylphenyl)-N-(4-((3-(1-pipendinyl)propyl)oxy)phenyl)-2-quinazolinamine | O | 466.626 | 467 |

### Experimental Method P

### Example 539

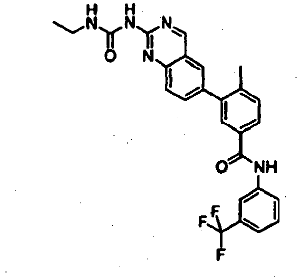

### 3-(2-(((ethylamino)carbonyl)amino)-6-quinazolinyl)-4-methyl-N-(3-(trifluoromethyl)phenyl)benzamide

### Step 1. Preparation of 3-(2-amino-6-quinazolinyl)-4-methyl-N-(3-(trifluoromethyl)phenyl)benzamide

The title compound was prepared by a method similar to that described in Experimental Method I1 and Example 228.

### Step 2. Preparation of 3-(2-(((ethylamino)carbonyl)amino)-6-quinazolinyl)-4-methyl-N-(3-(trifluoromethyl)phenyl)benzamide

To 3-(2-amino-6-quinazolinyl)-4-methyl-N-(3-(trifluoromethyl)phenyl)benzamide (63 mg, 0.15 mmol) in DMF (1.0 mL) was added ethyl isocyanate (1 ml). The mixture was stirred overnight at 80 °C. The reaction mixture was purified by preparative TLC (30% acetone/CH₂Cl₂) to yield a white solid, which was triturated with MeOH and dried to yield 3-(2-(((ethylamino)carbonyl)amino)-6-quinazolinyl)-4-methyl-N-(3-(trifluoromethyl)phenyl)benzamide as a white solid. MS *m*/*z* = 494 [M+H]⁺; Calc'd for C₂₆H₂₂F₃N₅O₂: 493.49.

The following Example 540 was prepared by a method similar to that described in Experimental Method P and Example 539.

| Example No. | Structure | Name | Method | MW | (M+H⁺) |
|---|---|---|---|---|---|
| 540 | 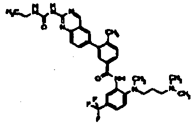 | N-(2-((3-(dimethylamino)propyl)(methyl)amino)-5-(trifluoromethyl)phenyl)-3-(2-(((ethylamino)carbonyl)amino)-6-quinazolinyl)-4-methylbenzamide | P | 607.677 | 608 |

### Experimental Method Q

### Example 541

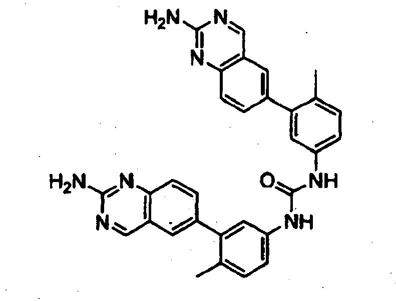

### Synthesis of 1,3-bis(3-(2-aminoquinazolin-6-yl)-4-methylphenyl)urea

6-(5-amino-2-methylphenyl)quinazolin-2-amine (100 mg, 0.40 mmol) was taken up in CH₂Cl₂ (8 ml) and saturated NaHCO₃ (4.2 mL). After 10 min, phosgene (20% solution in toluene, 0.317 ml, 0.60 mmol) was added to the organic layer. Stirring was resumed for 20 min. The mixture was then diluted with CH₂Cl₂/H₂O (1:1) (10 mL) and the organic layer was washed once with H₂O and dried over granular Na₂SO₄. 1,3-bis(3-(2-aminoquinazolin-6-yl)-4-methylphenyl) urea was then isolated as a pale yellow solid by filtration and removal of the granular Na₂SO₄. MS *m*/*z* = 527 [M+H]⁺; Calc'd for C₃₁H₂₆N₈O: 526.

### Experimental Method R

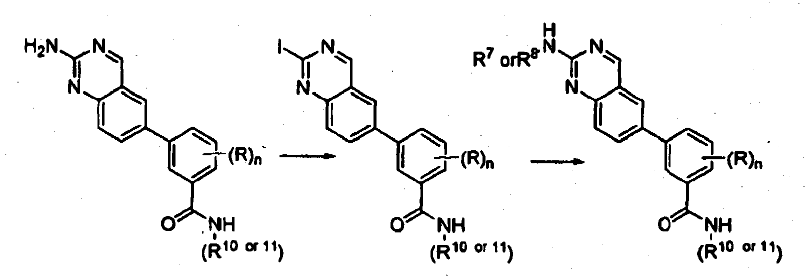

### Example 542

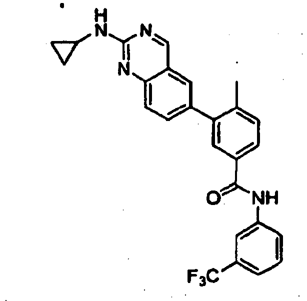

### Synthesis of 3-(2-(cyclopropylamino)quinazolin-6-yl)-4-methyl-N-(3-(trifluoromethyl)phenyl)benzamide

### Step 1 - Preparation of 3-(2-iodoquinazolin-6-yl)-4-methyl-N-(3-(trifluoromethyl)phenyl)benzamide

Isoamyl nitrite (0.284 ml, 1.81 mmol) was added to a mixture of 3-(2-aminoquinazolin-6-yl)-4-methyl-N-(3-(trifluoromethyl)phenyl)benzamide (250 mg, 0.592 mmol; prepared by Method D1), CuI (113 mg, 0.592 mmol), and CH₂I₂ (0.243 ml, 3.0 mmol) in THF (3 ml). The mixture was heated under an atmosphere of N₂, at 70°C for 1 h in a sealed tube. After cooling the mixture was poured into 50 ml EtOAc/1N HCl (1:1). The aqueous layer was extracted twice with EtOAc then the combined organics were dried over Na₂SO₄. Rapid purification by flash chromatography on silica, eluting with EtOAc/Hexanes followed by trituration from CH₂Cl₂ afforded the title compound as a pale yellow solid. MS *m*/*z* = 534 [M+H]⁺; Calc'd for C₂₃H₁₅F₃IN₃O: 533

### Step 2 - Preparation of 3-(2-(cyclopropylamino)quinazolin-6-yl)-4-methyl-N-(3-(trifluoromethyl)phenyl)benzamide

3-(2-(cyclopropylamino)quinazolin-6-yl)-4-methyl-N-(3-(trifluoromethyl)phenyl)benzamide was prepared in a manner similar to that described in Example 725, using 3-(2-iodoquinazolin-6-yl)-4-methyl-N-(3-(trifluoromethyl)phenyl)benzamide (from step 1 above) in place of 6-bromo-2-iodoquinazoline, to afford the title compound as a yellow solid. MS *m*/*z* = 463 [M+H]⁺. Calc'd for C₂₆H₂₁F₃N₄O: 462.

The following Example 543 was prepared by a method similar to that described in Experimental Method R and Example 542.

| Example No. | Structure | Name | Method | MW | (M+H⁺) |
|---|---|---|---|---|---|
| 543 | 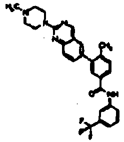 | 4-methyl-3-(2-(4-methyl-1-piperazinyl)-6-quinazolinyl)-N-(3-(trifluoromethyl)phenyl)benzamide | R | 505.541 | 506.2 |

### Experimental Method S

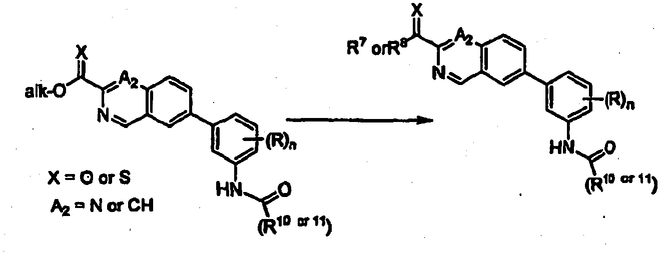

### Example 544 (Reference Example)

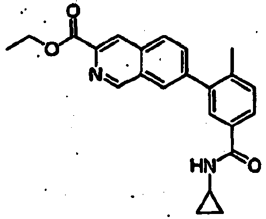

### Synthesis of Ethyl 7-(5-((cyclopropylamino)carbonyl)-2-methylphenyl)-3-isoquinolinecarboxylate

Sodium carbonate (2M, 1.61 ml) and toluene/EtOH (8 ml, 5/1) was added to ethyl 7-bromoisoquinoline-3-carboxylate (450 mg, 1.61 mmol), N-cyclopropyl-4-methyl-3-(4,4,5,5-tetramethyl-1,3,2-dioxaborolan-2-yl)benzamide (532 mg, 1.77 mmol) and tetrakis(triphenylphosphine)palladium (186 mg, 0.16 mmol) and heated to 80°C under N₂. After stirring overnight the reaction was cooled and concentrated. Sodium bicarbonate (sat.) was added and the mixture extracted with dichloromethane. The combined organic layers were dried over Na₂SO₄, filtered and concentrated. Purification of the crude product by column chromatography (3% MeOH in CH₂Cl₂) gave of ethyl 7-(5-((cyclopropylamino)carbonyl)-2-methylphenyl)-3-isoquinolinecarboxylate as a yellow solid. MS (m/z) : 375.1 (M+H⁺).

### Example 545 (Reference Example)

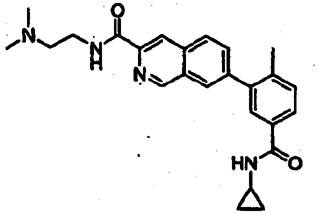

### Synthesis of 7-(5-((cyclopropylamino)carbonyl)-2-methylphenyl)-N-(2-(dimethylamino)ethyl)-3-isoquinolinecarboxamide

Step 1: To a solution of ethyl 7-(5-(cyclopropylcarbamoyl)-2-methylphenyl)isoquinoline-3-carboxylate (240 mg, 0.64 mmol) in THF/MeOH (4/1, 10 ml) was added 2M LiOH (1.60 ml) at 0°C. The reaction was allowed to warm up to RT. After 2h, the reaction was neutralized with 2M HCl and extracted with CH₂Cl₂/MeOH to give 7-(5-(cyclopropylcarbamoyl)-2-methylphenyl)isoquinoline-3-carboxylic acid as a white solid. MS(m/z) = 347.1 (M+H⁺).
Step 2: DIPEA (0.041 ml, 0.24 mmol) was added to a solution of 7-(5-(cyclopropylcarbamoyl)-2-methylphenyl)isoquinoline-3-carboxylic acid (33 mg, 0.095 mmol), N,N-dimethylethylenediamine (0.010 ml, 0.096 mmol), and HATU (36 mg, 0.095 mmol) in 1 ml of DMF at RT. After stirring overnight, water was added and the mixture was extracted with EtOAc and the combined organic layers dried over Na₂SO₄, filtered and concentrated. The crude product was purified by silica gel chromatography (10% MeOH in CH₂Cl₂) to give 7-(5-((cyclopropylamino)carbonyl)-2-methylphenyl)-N-(2-(dimethylamino)ethyl)-3-isoquinolinecarboxamide as a pale yellow solid. MS(m/z): 417.2 (M+H⁺)

### Example 546 (Reference Example)

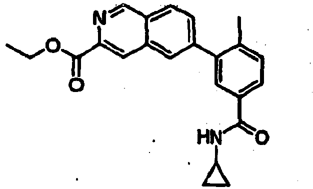

### Synthesis of ethyl 6-(5-((cyclopropylamino)carbonyl)-2-methylphenyl)-3-isoquinolinecarboxylate

Step 1: Sodium carbonate (2M, 1.61 ml) and toluene/EtOH (8 ml, 5/1) was added to ethyl 6-bromoisoquinoline-3-carboxylate (450 mg, 1.61 mmol), N-cyclopropyl-4-methyl-3-(4,4,5,5-tetramethyl-1,3,2-dioxaborolan-2-yl)benzamide (532 mg, 1.77 mmol) and tetrakis(triphenylphosphine)palladium (186 mg, 0.16 mmol) and heated to 80°C under N₂. After stirring overnight the reaction was cooled and concentrated. Sodium bicarbonate (sat.) was added and the mixture extracted with dichloromethane. The combined organic layers were dried over Na₂SO₄, filtered and concentrated. Purification of the crude product by silica gel chromatography (3% MeOH in CH₂Cl₂) gave ethyl 6-(5-((cyclopropylamino)carbonyl)-2-methylphenyl)-3-isoquinolinecarboxylate as a pale yellow solid. MS(m/z): 375.1 (M+H⁺).

### Example 547 (Reference Example)

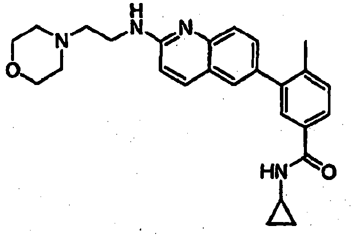

### Synthesis of N-cyclopropyl-4-methyl-3-(2-(2-morpholinoethylamino)quinolin-6-yl)benzamide

A solution of 6-bromo-2-chloroquinoline (100 mg, 0.41 mmol) and 4-(2-aminoethyl)morpholine (0.27 mL, 2.06 mmol) in 2 mL of EtOH was heated to 160°C by microwave irradiation in a sealed tube for 10 minutes. The reaction was concentrated and the crude product purified by silica gel chromatography (3% MeOH in CH₂Cl₂) to give 6-bromo-N-(2-morpholinoethyl)quinolin-2-amine. The 6-bromo-N-(2-morpholinoethyl)quinolin-2-amine was coupled with N-cyclopropyl-4-methyl-3-(4,4,5,5-tetramethyl-1,3,2-dioxaborolan-2-yl)benzamide, in a method similar to that described in Example 544, to yield the title compound. MS (m/z) : 431.2 (M+H+) ; calc'd for C₂₆H₃₀N₄O₂ = 430.24.

### Example 548 (Reference Example)

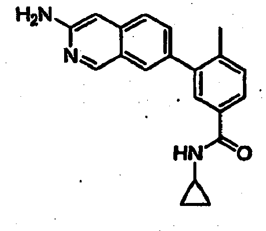

### Synthesis of 3-(3-aminoisoquinolin-7-yl)-N-cyclopropyl-4-methylbenzamide

5-Bromo-2-fluoro benzaldehyde (25 g, 0.12 moles) in NMP (25 ml) was added dropwise to a solution of acetamidine hydrochloride (15.1 g, 0.16 moles), DIPEA (55.7 ml, 0.32 mol) in NMP (250 ml) at 145°C. After the addition the reaction was cooled and water and ice were added. The mixture was extracted with EtOAc and the combined organic layers were dried over Na₂SO₄, filtered and concentrated. Purification of the crude product by column chromatography (3% MeOH in CH₂Cl₂) gave 7-bromoisoquinolin-3-amine. The 7-bromoisoquinolin-3-amine was coupled with N-cyclopropyl-4-methyl-3-(4,4,5,5-tetramethyl-1,3,2-dioxaborolan-2-yl)benzamide, in a method similar to that described in Example 533, to yield the title compound. MS (m/z) : **Mass Spec** ?? (M+H⁺).

The following Examples 549-552 were prepared by a method similar to that described in Experimental Method T and Examples 544-548. The following Examples 549 to 552 are Reference Examples.

| Example No. | Structure | Name | Method | MW | (M+H⁺) |
|---|---|---|---|---|---|
| 549 | 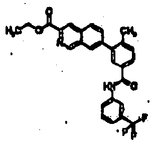 | ethyl 7-(2-methyl-5-(((3-(trifluoromethyl)phenyl)amino)carbonyl)phenyl)-3-isoquinolinecarboxylate | S | 478.468 | 479.2 |
| 550 | 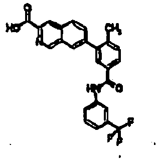 | 7-(2-methy)-5-(((3-(trifluoromethyl)phenyl)amino)carbonyl)phenyl)-3-isoquinolinecarboxylic acid | S | 450.414 | 451 |
| 551 | 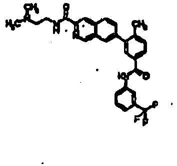 | N-(2-(dimethylamino)ethyl)-7-(2-methyl-5-(((3-(trifluoromethyl)phenyl)amino)carbonyl)phenyl)-3-isoquinolinecarboxamide | S | 520.552 | 521.2 |
| 552 | 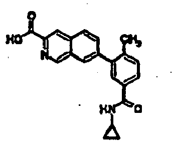 | 7-(5-((cyclopropylamino)carbonyl)-2-methylphenyl)-3-isoquinolinecarboxylic acid | S | 346.384 | 347.1 |

### Experimental Method T (Reference Example)

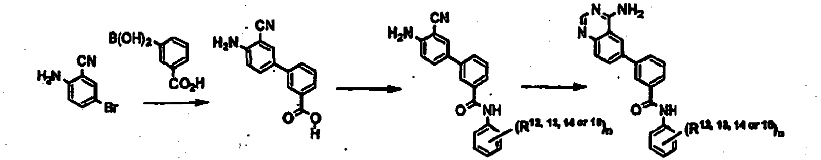

### Example 553 (Reference Example)

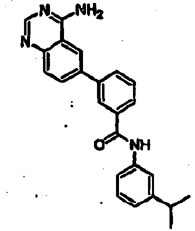

### Synthesis of 3-(4-amino-6-quinazolinyl)-N-(3-(1-methylethyl)phenyl)benzamide

### Step 1. Preparation of 4'-amino-3'-cyano-1,1'-biphenyl-3-carboxylic acid

To 2-amino-5-bromobenzonitrile (400 mg, 2.03 mmol), 3-boronobenzoic acid (332 mg, 2.00 mmol)), Pd (PPh₃) (115 mg, 0.100 mmol), and Na₂CO₃ (1.1 g, 10.0 mmol) was added CH₃CN (15 ml) and water (5 ml). The mixture was stirred at 100 °C. The resulting mixture was filtered, concentrated and purified by Gilson reverse-phase HPLC (0.1% TFA in H₂O/CH₃CN) to yield 4'-amino-3'-cyano-1,1'-biphenyl-3-carboxylic acid as a white solid.

### Step 2. Preparation of 4'-amino-3'-cyano-N-(3-(1-methylethyl)phenyl)-1,1'-biphenyl-3-carboxamide

4'-amino-3'-cyano-N-(3-(1-methylethyl)phenyl)-1,1'-biphenyl-3-carboxamide was prepared from 4'-amino-3'-cyano-1,1'-biphenyl-3-carboxylic acid in accordance with the method described in representative examples 409 and 410 of Experimental Method 13. MS *m*/*z* = 356 [M+H]⁺. Calc'd for C₂₃H₂₁N₃O: 355.44.

### Step 3. Preparation of 3-(4-amino-6-quinazolinyl)-N-(3-(1-methylethyl)phenyl)benzamide

After 20 minutes of stirring p-toluenesulfonyl chloride (400 mg, 2.10 mmol) in DMF, 4'-amino-3'-cyano-N-(3-(1-methylethyl)phenyl)-1,1'-biphenyl-3-carboxamide (40 mg, 0.11 mmol) was added. The mixture was stirred for 1 hour at RT. The DMF was removed and the residue was dissolved in EtOH (3 ml) before adding NH₄OH (0.30 ml) and heating to about 110 °C for 1 hour. The resulting mixture was concentrated and purified by Gilson reverse-phase HPLC. (0.1% TFA in H₂O/CH₃CN) The product fraction were collected, concentrated and free based to yield 3-(4-amino-6-quinazolinyl)-N-(3-(1-methylethyl)phenyl)benzamide. MS *m*/*z* = 383 [M+H]⁺; Calc'd for C₂₄H₂₂N₄: 382.47.

### Experimental Method U

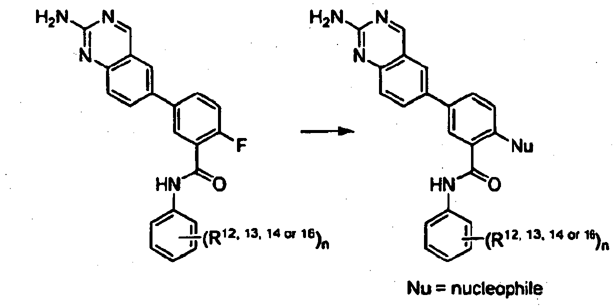

In addition to representative Examples 554 and 555 below, see also Example 57.

### Example 554

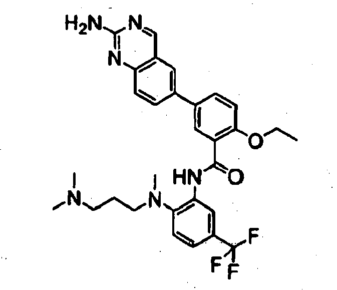

### Synthesis of 5-(2-amino-6-quinazolinyl)-N-(2-((3-(dimethylamino)propyl)(methyl)amino)-5-(trifluoromethyl)phenyl)-2-(ethyloxy)benzamide

A solution of 5-(2-amino-6-quinazolinyl)-N-(2-((3-(dimethylamino)propyl)(methyl)amino)-5-(trifluoromethyl)phenyl)-2-fluorobenzamide (0.064 g, 0.12 mmol) and KOt-Bu (0.040 g, 0.34 mmol) in 0.5 ml tert-butanol was heated to 100°C in a sealed tube for 6 h. The reaction was cooled to ambient temperature, and 0.5 ml ethanol was added. The vessel was resealed and heated for 12 h. The reaction was cooled to ambient temperature and partitioned between EtOAc and water. The organic layer was dried with Na₂SO₄, filtered, and concentrated to give a solid. This material was suspended in dichloromethane/diethyl ether with sonication, and was filtered to give the title compound as a white solid. MS (m/z): 567.2 (M+H)⁺; Calc'd for C₃₀H₃₃F₃N₆O₂: 566.62.

### Example 555

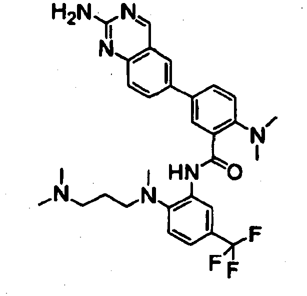

### Synthesis of 5-(2-amino-6-quinazolinyl)-2-(dimethylamino)-N-(2-((3-(dimethylamino)propyl)(methyl)amino)-5-(trifluoromethyl)phenyl)benzamide

A solution of 5-(2-amino-6-quinazolinyl)-N-(2-((3-(dimethylamino)propyl)(methyl)amino)-5-(trifluoromethyl)phenyl)-2-fluorobenzamide (0.066 g, 0.12 mmol), Me₂NH-HCl (0.10 g, 1.2 mmol), and DIPEA (0.21 ml, 1.2 mmol) in 0.8 ml DMF was heated to 100 °C in a sealed tube for 12 h. The reaction was cooled to ambient temperature and partitioned between EtOAc, water, and 1N NaOH. The organic layer was dried with Na₂SO₄, filtered, and concentrated to give a solid. This material was resubjected to the previous conditions with the following modifications: 0.5 ml of a 2.0 M solution of methylamine in THF was used, no DIPEA was used, and 0.5 ml DMF was used as the solvent. After three days the reaction was quenched and worked up as before. Purification by preparative TLC (MC/MeOH/conc. NH₄OH) provided the desired product as a yellow solid. MS (m/z): 566.2 (M+H)⁺; Calc'd for C₃₀H₃₄F₃N₇O: 565.63.

Building block starting materials and intermediates were made, where not commercially available, and utilized in Experimental Methods A-V above. Below are procedures and examples for building various of the exemplary building blocks.

Various different A rings (R¹¹ and R¹⁴ groups), which are contemplated herein, may be made by various methods, as represented by Examples 563-628 below.

### Example 563

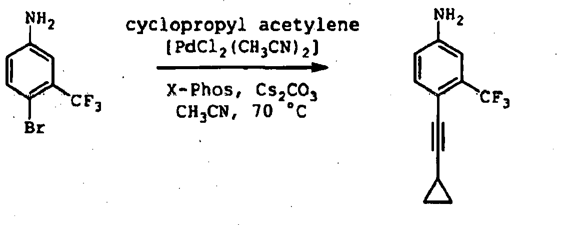

### Synthesis of 4-(2-cyclopropylethynyl)-3-(trifluoromethyl)benzenamine

The title compound was prepared in a manner similar to the procedure described in Gelman, D.; Buchwald, S. L. Angew. Chem. Int. Ed., 42, 5993, 2003. A 38 ml heavy-walled sealed tube was flame-dried and cooled to RT under a nitrogen stream, then charged with [PdCl₂(CH₃CN)₂] (0.0054 g, 0.0208 mmol), X-Phos (0.0298 g, 0.0625 mmol), cesium carbonate (1.76 g, 5.42 mmol) and acetonitrile (10 ml). The tube was then purged with a stream of argon. 4-bromo-3-(trifluoromethyl)benzenamine (0.500 g, 2.08 mmol) was added followed by an additional argon purge. The tube was sealed and the mixture stirred at room temperature for 25 min. Cyclopropyl acetylene (70 wt. % in toluene, 0.321 ml, 2.71 mmol) was then added via syringe followed by a quick argon purge. The tube was resealed, and the mixture was heated at 70°C for 3.25 h, and cooled to RT. The reaction mixture was partitioned between ethyl acetate and water. The aqueous phase was separated and extracted with ethyl acetate. The combined organic phases were dried over anhydrous sodium sulfate, filtered, and concentrated to afford a dark brown oil. The crude oil material was purified via medium pressure column chromatography on silica gel (ISCO, RediSep, gradient elution using 5-30% ethyl acetate in hexanes) to afford 4-(2-cyclopropylethynyl)-3-(trifluoromethyl)benzenamine as a brown oil. MS (M+H⁺) 226.0; Calculated for C₁₂H₁₀F₃N: 225.

### Example 564

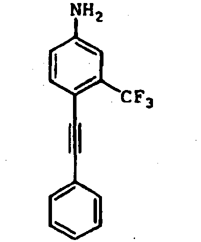

### Synthesis of 4-(2-phenylethynyl)-3-(trifluoromethyl)benzenamine

4-(2-phenylethynyl)-3-(trifluoromethyl)benzenamine was synthesized by a method similar to that described in Example 563, affording the title compound as a brown oil. MS (M+H⁴) 262.0; Calculated for C₁₅H₁₀F₃N: 261.

### Example 565

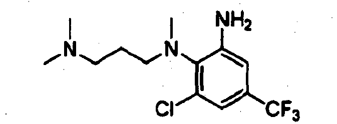

### Synthesis of 6-chloro-N¹-(3-(dimethylamino)propyl)-N¹-methyl-4-(trifluoromethyl)benzene-1,2-diamine

A heterogeneous mixture of 1-chloro-2-fluoro-3-nitro-5-(trifluoromethyl)benzene (1.25 mL, 8.2 mmol), K₂CO₃ (3.44 g, 24.6 mmol), *N*¹,*N*¹,*N*³-trimethylpropane-1,3-diamine (1.26 mL, 8.61 mmol)and THF were allowed to stir at room temperature for 45 min. The THF was removed under reduced pressure and reconstituted in EtOAc (50 ml). The organic layer was washed with water (20 ml), brine (20 ml), dried over anhydrous sodium sulfate, filtered and concentrated to an oil. The concentrated oil was taken up in EtOH (20 ml) to which Raney nickel (2.5 g wet, washed) was added. The reduction was monitored and after 1 h, another portion of Raney nickel (3.8 g, wet, washed) was added. The reaction was allowed to stir for an additional 30 min., and filtered through Celite, washed with EtOH (10 ml) and concentrated. The crude residue was purified via flash chromatography (silica gel, gradient elution 0 to 25% MeOH in CH₂Cl₂) to afford 6-chloro-*N*¹-(3-(dimethylamino)propyl)-*N*¹-methyl-4-(trifluoromethyl)benzene-1,2-diamine as a yellow oil. MS *m*/*z* = 310.1 [M+H]⁺. Calc'd for C₁₃H₁₉ClF₃N₃: 309.8.

### Example 566

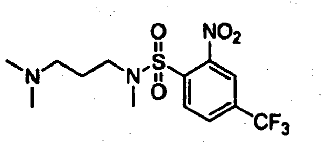

### Synthesis of N-(3-(dimethylamino)propyl)-N-methyl-2-nitro-4-(trifluoromethyl)benzenesulfonamide

To a solution of 2-nitro-4-(trifluoromethyl)benzene-1-sulfonyl chloride (500 mg, 1.73 mmol) in CH₂Cl₂ (5 ml) was added *N*¹, *N*¹, *N*³-trimethylpropane-1,3-diamine (0.26 ml, 1.8 mmol). The resulting mixture was allowed to stir at room temperature for 20 min. Diluted with CH₂Cl₂ (30 ml) and washed the organic layer with 9% aq. Na₂CO₃ (10 ml) and brine (10 ml). Dried over anhydrous sodium sulfate, filtered and concentrated to a white solid, which was used without further purification. MS *m*/*z* = 370.1 [M+H]⁺. Calc'd for C₁₃H₁₈F₃N₃O₄S: 369.4.

### Example 567

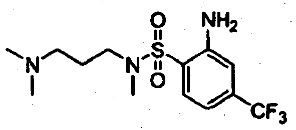

### Synthesis of 2-amino-N-(3-(dimethylamino)propyl)-N-methyl-4-(trifluoromethyl)benzenesulfonamide

To an argon purged solution of *N*-(3-(dimethylamino)propyl)-*N*-methyl-2-nitro-4-(trifluoromethyl)benzenesulfonamide (255 mg, 0.69 mmol) in EtOH (10 ml) was added Pd/C (73 mg, 0.069 mmol, 10%). The reaction mixture was placed under an atmosphere of H₂ gas and allowed to stir for 2 h. The reaction mixture was purged with argon and filtered through Celite. The reaction was washed with EtOH (10 ml) and concentrated under reduced pressure to afford 2-amino-*N*-(3-(dimethylamino)propyl)-*N*-methyl-4-(trifluoromethyl)benzenesulfonamide as a dark oil. MS *m*/*z* = 340.1 [M+H]⁺. Calc'd for C₁₃H₂₀F₃N₃OS: 339.4.

### Example 568

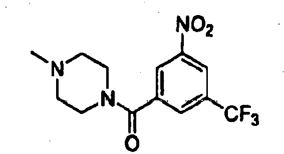

### Synthesis of (4-methylpiperazin-1-yl)(3-nitro-5-trifluoromethyl)phenyl)-methanone

A solution of thionyl chloride (30 ml) and 3-nitro-5-(trifluoromethyl)benzoic acid (10 g) was heated to reflux for 2 h. The reaction mixture was concentrated under reduced pressure and treated with toluene (10 ml) which was then removed under reduced pressure to afford 3-nitro-5-(trifluoromethyl)benzoyl chloride.

To a solution of 3-nitro-5-(trifluoromethyl)benzoyl chloride (2.35 g, 9.3 mmol) in CH₂Cl₂ (40 ml) at room temperature was added *N*-methylpiperazine (1.26 ml, 9.3 mmol) and the mixture was allowed to stir for 30 min. The reaction was concentrated under reduced pressure, taken up in 1 M HCl (50 ml) and the aqueous layer was washed with Et₂O (2 x 20 ml). The aqueous layer was basified to a pH of about 9 with 6 N NaOH, and the aqueous layer was extracted with Et₂O (3 x 50 ml). The organic extracts were combined and washed with water (1 x 20 ml) followed by brine (1 x 20 ml), and dried over anhydrous sodium sulfate, filtered and concentrated under reduced pressure to afford (4-methylpiperazin-1-yl)(3-nitro-5-trifluoromethyl)phenyl)-methanone as an tan oil, which was used without further purification.

### Example 569

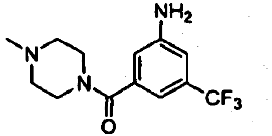

### Synthesis of (3-amino-5-(trifluoromethyl)phenyl)(4-methylpiperazin-1-yl)methanone

To an argon purged solution of (4-methylpiperazin-1-yl(3-nitro-5-trifluoromethyl)phenyl)-methanone (1.03 g, 3.25 mmol) was added Pd/C (344 mg, 0.32 mmol, 10%). The mixture was placed under an atmosphere of H₂ for 5 h. The reaction was purged with argon and filtered through Celite. The filtrate was concentrated under reduced pressure to afford (3-amino-5-(trifluoromethyl)phenyl)(4-methylpiperazin-1-yl)methanone as an off-white solid. MS *m*/*z* = 288.1 [M+H]⁺. Calc'd for C₁₃H₁₆F₃N₃O: 287.3.

### Example 570

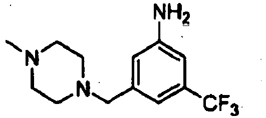

### Synthesis of 3-((4-methylpiperazin-1-yl)methyl)-5-(trifluoromethyl)-benzenamine

To LAH (1.84 g, 48.5 mmol) in THF (50 ml) at room temperature was added (4-methylpiperazin-1-yl)(3-nitro-5-trifluoromethyl)phenyl)-methanone (1.54 g, 4.85 mmol) in THF (10 ml). The resulting mixture was brought to reflux for 5 h. The reaction mixture was cooled to 0°C at which point water (1.84 ml), 15% aq. NaOH (1.84 ml and water (3.68 ml) were successively added. The resulting mixture was allowed to stir at room temperature for 1 h. The mixture was filtered through Celite, concentrated under reduced pressure and purified via flash chromatography (silica gel, 0 to 25% MeOH in CH₂Cl₂, gradient elution) to afford 3-((4-methylpiperazin-1-yl)methyl)-5-(trifluoromethyl)benzenamine as a colorless oil. MS *m*/*z* = 275.1 [M+H]⁺. Calc'd for C₁₃H₁₈F₃N₃: 273.3.

The following substituted aniline intermediates were prepared in a manner similar to the procedure described in Example 64 of co-pending patent Application serial no. 60/569,193:

| **Example** | **Structure** | **Name** | **Cal'd MS** | **M+H⁺** |
|---|---|---|---|---|
| 571 | 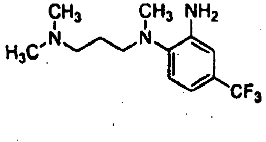 | N1-(3-(dimethylamino)propyl)-N1-methyl-4-(trifluoromethyl)benzene-1,2-diamine: | 275 | 276 .1 |
| 572 | 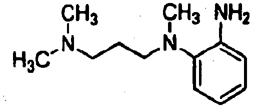 | N1-(3-(dimethylamino)propyl)-N1-methylbenzene-1,2-diamine | 207 | 208 |
| 573 | 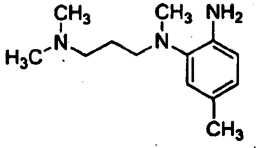 | N1-(3-(dimethylamino)propyl)-N1,5-dimethylbenzene-1,2-diamine: | 221 | 222 |
| 574 | 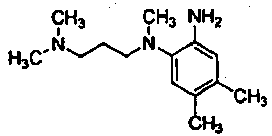 | No-(3-(dimethylamino)propyl)-N1,4,5-trimethylbenzene-1,2-diamine: | 235 | 236 |
| 575 | 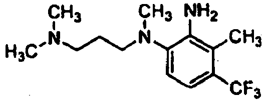 | N1-(3-(dimethylamino)propyl)-N1,3-dimethyl-4-(trifluoromethyl)benzene-1,2-diamine: | 289 | 290 |
| 576 | 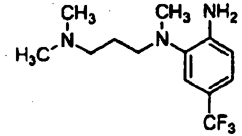 | N1-(3-(dimethylamino)propyl)-N1-methyl-5-(trifluoromethyl)benzene-1,2-diamine: | 275 | 276 |

### Example 577

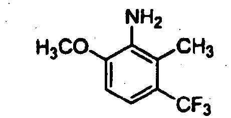

### Synthesis of 6-methoxy-2-methyl-3-(trifluoromethyl) benzenamine

### Step 1. Preparation of 1-methoxy-3-methyl-2-nitro-4-(trifluoromethyl)benzene

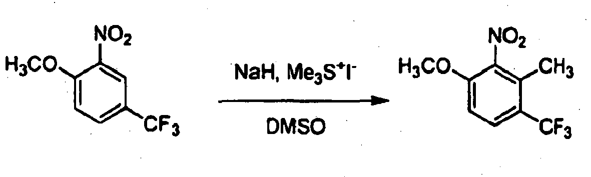

1-methoxy-3-methyl-2-nitro-4-(trifluoromethyl)benzene was prepared by a procedure similar to that described in "Synthesis of 3,6-Disubstituted 2-Nitrotoluenes by Methylation of Aromatic Nitro Compounds with Dimethylsulfonium Methylide", Kitano, Masafumi, Ohashi Naohito, Synthetic Communications, 30(23), 4247-4254, 2000. To a suspension of NaH (60% by wt. in mineral oil, 362 mg, 9.04 mmol) and trimethylsulfonium iodide (1.84 g, 9.04 mmol) in DMSO (17 ml) and THF (6.7 ml) was added 4-methoxy-3-nitrobenzotrifluoride (1.00 g, 4.52 mmol) as a solution in DMSO (2.7 ml). The reaction mixture was allowed to stir at 10-20°C for 5 hrs. The reaction mixture was quenched by addition to ice water. The aqueous layer was separated and extracted with toluene 7 times. The combined organic extracts were washed with brine, dried over MgSO₄, and filtered. The solvent was removed by distillation at reduced pressure. The residue was purified by automated (100% hexanes to 98:2 hexanes:ethylacetate) to provide 1-methoxy-3-methyl-2-nitro-4-(trifluoromethyl)benzene.

### Step 2. Preparation of 6-methoxy-2-methyl-3-(trifluoromethyl) benzenamine

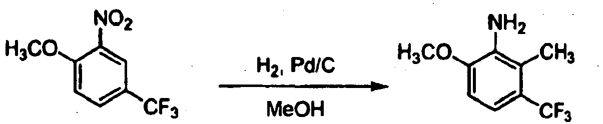

1-methoxy-3-methyl-2-nitro-4-(trifluoromethyl)benzene (258 mg, 1.10 mmol), methanol (11.0 mL), and palladium on carbon (77.4 mg) were combined in a N₂-purged round bottom flask. A balloon containing H₂ was affixed to the flask, and the solution was saturated with H₂ for 2 minutes. The reaction mixture was allowed to stir under H₂ atmosphere for 12 hrs. Upon completion, as judged by LCMS, the reaction mixture was filtered through a plug of Celite and the solvent was removed *in vacuo* to afford 6-methoxy-2-methyl-3-(trifluoromethyl) benzenamine. MS *m*/*z* = 206 [M+1]⁺. Calc'd for C₉H₁₀F₃NO: 205.

### Example 578

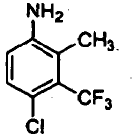

### Synthesis of 4-chloro-2-methyl-3-(trifluoromethyl)benzenamine

4-Chloro-2-methyl-3-(trifluoromethyl)benzenamine was prepared by a method similar to that described in "Preparation of Fused Succinimides as Modulators of Nuclear Hormone Receptor Function", Salvati, Mark E. et al,. PCT Patent Publication WO 2003062241. MS *m*/*z* = 210. Calc'd for C₉H₁₀F₃NO: 210.

### Example 579

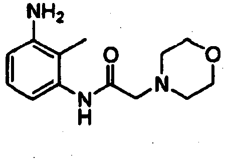

### Synthesis of N-(3-amino-2-methylphenyl)-2-morpholinoacetamide

### Step 1. Preparation of 2-bromo-N-(2-methyl-3-nitrophenyl)acetamide

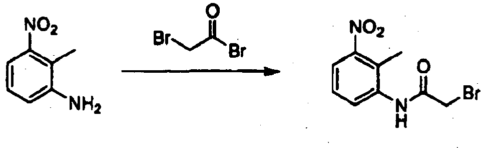

To a solution of 2-methyl-3-nitroaniline (5.0 g, 32.9 mmol) in 120 ml of CH₂Cl₂ was added 120 ml of saturated NaHCO₃ and bromoacetyl bromide (2.85 ml, 6.6 g, 32.9 mmol). The reaction was stirred at room temperature for 64 hours. The layers were separated, and the organic layer was washed with water, brine and then dried over MgSO₄. Solvent evaporation afforded 2-bromo-N-(2-methyl-3-nitrophenyl)acetamide as a yellow solid.

### Step 2. Preparation of N-(2-methyl-3-nitrophenyl)-2-morpholinoacetamide

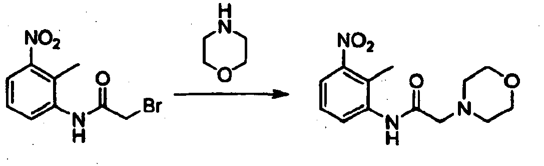

2-Bromo-N-(2-methyl-3-nitrophenyl)acetamide (0.5 g, 1.8 mmol) was dissolved in 15 ml of THF and to this was added morpholine (0.17g, 2.0 mmol) and diisopropylethylamine (0.71 g, 5.5 mmol). The reaction was stirred at room temperature for 16 hours. The reaction was then partitioned between EtOAc and H₂O. The aqueous mixture was extracted with EtOAc, and the combined organic layers were washed with H₂O, brine and then dried over MgSO₄. Solvent evaporation afforded N-(2-methyl-3-nitrophenyl)-2-morpholinoacetamide as a yellow solid.

### Step 3. Preparation of N-(3-amino-2-methylphenyl)-2-morpholinoacetamide

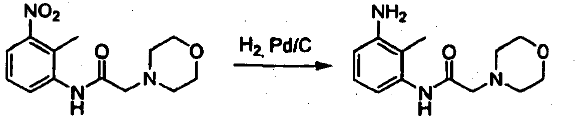

N-(2-Methyl-3-nitrophenyl)-2-morpholinoacetamide (0.25 g, 0.9 mmol) was dissolved in 20 ml of MeOH, and to this was added a slurry of 10 % Pd/C (0.025 g) in a minimal amount of EtOH. The reaction vessel was evacuated and purged with H₂, and the reaction was stirred at room temperature for 16 hours. The mixture was purged with N₂ for 30 minutes and then filtered through a pad of celite. Solvent evaporation afforded N-(3-amino-2-methylphenyl)-2-morpholinoacetamide as a gray solid. MS m/z = 250.1 [M+H]⁺ ; Calc'd for C₁₃H₁₉N₃O₂: 249.

Examples 580-583 were prepared by a method similar to the procedure described in Example 579 above.

| Example | Structure | Name |
|---|---|---|
| 580 | 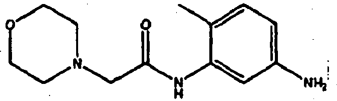 | N-(5-amino-2-methylphenyl)-2-morpholinoacetamide |
| 581 | 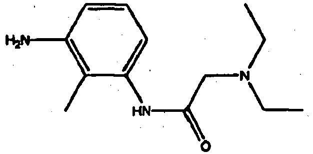 | N-(3-amino-2-methylphenyl-2-(diethylamino)acetamide |
| 582 | 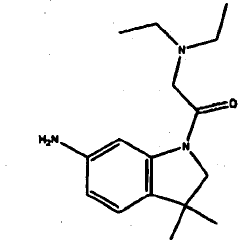 | 1-(6-amino-3,3-dimethylindolin-1-yl)-2-(diethylamino)ethanone |
| 583 | 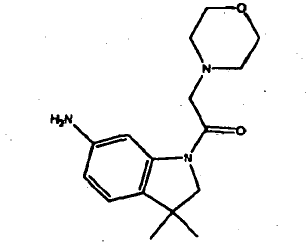 | 1-(6-amino-3,3-dimethylindolin-1-yl)-2-morpholinoethanone |

### Example 584

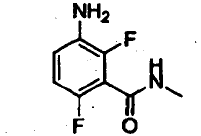

### Synthesis of 3-amino-2,6-difluoro-N-methylbenzamide

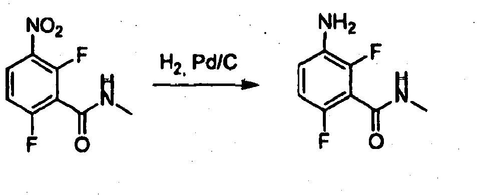

2,6-Difluoro-3-nitrophenylacetamide (0.5 g, 2.3 mmol) was dissolved in 20 ml of MeOH and to this was added a slurry of 10% Pd/C (0.050 g). The reaction vessel was evacuated and purged with H₂, and the reaction was stirred at room temperature for 3 hours. The mixture was purged with N₂, and then filtered through a pad of celite. Solvent evaporation afforded 3-amino-2,6-difluoro-N-methylbenzamide as a pink solid.

### Example 585

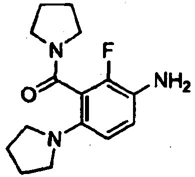

### (3-amino-2-fluoro-6-(pyrrolidin-1-yl)phenyl)(pyrrolidin-1-yl)methanone

Example 585 was prepared by a method similar to that described in Example 584 above.

### Example 586

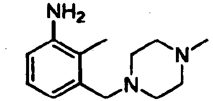

### Synthesis of 2-methyl-3-((4-methylpiperazin-1-yl)methyl)benzeneamine

### Step 1. Preparation of 1-(2-methyl-3-nitrobenzyl)-4-methylpiperazine

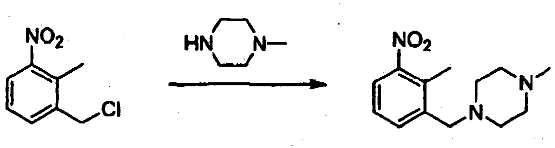

2-Methyl-3-nitrobenzylchloride (1.0 g, 5.4 mmol) was dissolved in 30 ml of THF, and to this was added 1-methylpiperazine (0.65 g, 6.5 mmol) and sodium bicarbonate (2.26 g, 26.9 mmol). The reaction mixture was stirred at 65 °C for 16 hours. The mixture was partitioned between EtOAc and H₂O. The aqueous layer was extracted with EtOAc, and the combined organic layers were washed with saturated NH₄Cl, H₂O, brine and dried over MgSO₄. Solvent evaporation afforded 1-(2-methyl-3-nitrobenzyl)-4-methylpiperazine.

### Step 2. Preparation of 2-methyl-3-((4-methylpiperazin-1-yl)methyl)benzeneamine

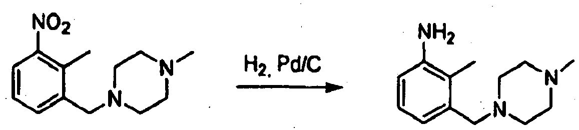

1-(2-Methyl-3-nitrobenzyl)-4-methylpiperazine (1.2 g, 4.8 mmol) was dissolved in 50 ml of MeOH, and to this was added a slurry of 10% Pd/C in a minimal amount of EtOH. The reaction mixture was evacuated and purged with H₂, and then stirred at room temperature for 3 hours. The mixture was purged with N₂ for 30 minutes and then filtered through a pad of celite. Solvent evaporation afforded 2-methyl-3-((4-methylpiperazin-1-yl)methyl)benzeneamine.

### Example 587

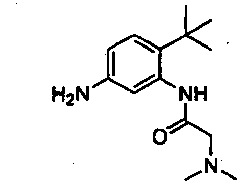

### Synthesis of N-(5-amino-2-tert-butylphenyl)-2-dimethylamino)acetamide

### Step 1. Preparation of 2-tert-butyl-5-nitrobenzenamine

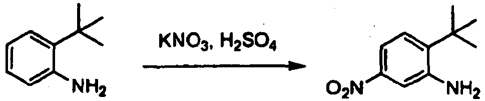

Concentrated sulfuric acid (1 L) was cooled to -10 °C with a dry ice-isopropanol bath in a 2 L 3-necked round bottom flask fitted with a mechanical stirrer and temperature probe. The 2-t-butylaniline (109 g, 730 mmol) was added, giving a clumpy solid. Once the temperature of the mixture was stabilized at -10 °C, the potassium nitrate (101 g, 1001 mmol) was added portion wise, as a solid, over a 4-hour period, maintaining the temperature between -20 and -5 °C. Once all of the potassium nitrate was added, the reaction was left to stir overnight with gradual warming to room temperature. The reaction was quenched by diluting with water and then extracting three times with EtOAc. The EtOAc extracts were washed multiple times with saturated NaHCO₃, until gas evolution ceased, then with brine. The ethyl acetate extracts were then combined, dried over anhydrous Na₂SO₄, filtered and concentrated under reduced pressure giving a black oil. The oil was eluted through a column of silica gel with EtOAc: hexanes gradient 5-50%. Solvent evaporation afforded 2-tert-butyl-5-nitrobenzenamine as a red solid.

### Step 2. Preparation of 2-bromo-N-(2-tert-butyl-5-nitrophenyl)acetamide

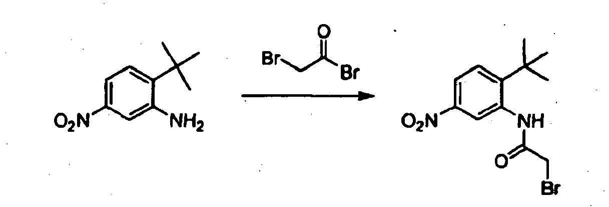

2-tert-Butyl-5-nitrobenzenamine (70 g, 359 mmol) and a catalytic amount of DMAP were dissolved into THF (1.5 L) under N₂. Triethylamine (109 g, 1077 mmol) was added and the solution was cooled to 0 °C. Bromoacetyl bromide (207 g, 1023 mmol) was then added and the reaction was stirred at room temperature for 16 hours. The reaction was then partially concentrated under reduced pressure, treated with water, and extracted three times with EtOAc. The EtOAc extracts were washed with brine, combined, dried over Na₂SO₄ and concentrated to a black oil. This oil was purified using silica chromatography, 95:5:0.5 CH₂Cl₂:MeOH:NH₄OH, giving 2-bromo-N-(2-tert-butyl-5-nitrophenyl)acetamide as a brown solid.

### Step 3. Preparation of N-(2-tert-butyl-5-nitrophenyl)-2-(dimethylamino)acetamide

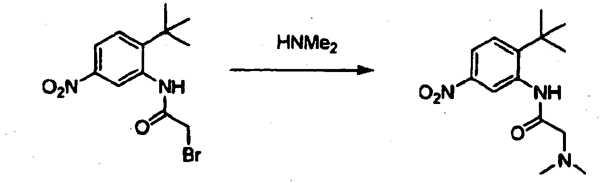

2-Bromo-N-(2-tert-butyl-5-nitrophenyl)acetamide (80 g, 253, mmol) and potassium carbonate (70 g, 506 mmol) were combined in THF (1.75 L), and the mixture was cooled to 0 °C. N,N-Dimethylamine (40 ml of a 2 M solution in THF, 800 mmol) was then added to the mixture through an addition funnel over a 30-minute period. The mixture was then stirred at room temperature for 16 hours. The mixture was then filtered and the filtrate was concentrated. The crude material was purified by silica chromatography using 50% EtOAc:hexanes as the eluent to give N-(2-tert-butyl-5-nitrophenyl)-2-(dimethylamino)acetamide as a brown solid.

### Step 4. Preparation of N-(5-amino-2-tert-butylphenyl)-2-dimethylamino)acetamide

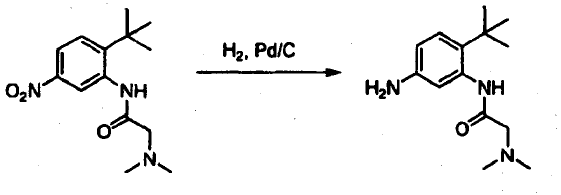

To a solution of N-(2-tert-butyl-5-nitrophenyl)-2-(dimethylamino)acetamide (25,8 g, 02 mmol) in 1,4-dioxane (200 ml) was added 10 % Pd/C (2.5 g) as a slurry in a minimal amount of EtOH. The mixture was evacuated and purged with H₂, and then stirred at room temperature for 16 hours. The reaction was then purged with N₂ and filtered through celite. The filtrate was concentrated and purified using silica chromatography, 97.5:2.5:0.25 to 95:5:0.5 CH₂Cl₂:MeOH:NH₄OH, to afford N-(5-amino-2-tert-butylphenyl)-2-dimethylamino)acetamide as a brown solid. MS (m/z) = 250.2 (M+H⁺) ; Calculated for C₁₄H₂₃N₃O: 249.4

### Example 588

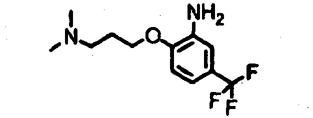

### Synthesis of N,N-dimethyl-3-(2-nitro-4-(trifluoromethyl)phenoxy)propan-1-amine

### Step 1. 2-(3-(dimethylamino)propoxy)-5-(trifluoromethyl)benzenamine

A suspension of NaHCO₃ (3.9 g, 48 mmol), 1-fluoro-2-nitro-4-trifluoromethylbenzene (4.0 g, 19 mmol), and 3-dimethylamino-1-propanol (2.5 ml, 21 mmol) in 38 mL dry THF was heated with a reflux condenser under nitrogen for 12 h. The mixture was filtered through a fritted funnel into a flask. The solution was cooled to 0 °C and was treated with potassium tert-butoxide (2.4 g, 21 mmol) resulting in an orange solution. The solution was warmed to ambient temperature and was allowed to stir for 1 h. The solvent was removed in vacuo, and the resulting brown oil was partitioned between saturated aqueous NaHCO₃ and methylene chloride. The aqueous layer was extracted three times with methylene chloride. The combined organic layers were dried with Na₂SO₄, filtered, and concentrated. The residue was purified by silica gel chromatography (MC/MeOH/conc. NH₄OH) to provide the desired compound as an orange oil. MS (m/z): 293.1 (M+H)⁺. Calc'd for C₁₂H₁₅F₃N₂O₃: 292.25.

### Step 2. N,N-dimethyl-3-(2-nitro-4-(trifluoromethyl)phenoxy)propan-1-amine

To 2-(3-(dimethylamino)propoxy)-5-(tri-fluoromethyl)benzenamine (1.6 g, 5.5 mmol) was added Pd/C (10%, 0.58 g) under nitrogen. Methanol (18 ml) was added via syringe, and H₂ gas was introduced and the mixture stirred vigorously under an atmosphere of H₂. After 23 h, the mixture was filtered through celite and concentrated to afford the title compound as a light brown solid. MS (m/z): 263 (M+H)⁺. Calc'd for C₁₂H₁₇F₃N₂O₃: 262.27.

### Example 589

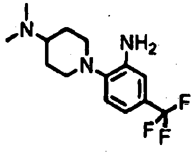

### Synthesis of 1-(2-amino-4-(trifluoromethyl)phenyl)-N,N-dimethylpiperidin-4-amine

### Step 1. 1-benzyl-N,N-dimethylpiperidin-4-amine dihydrochloride

To a mixture of 4-amino-1-benzyl piperidine (5.0 g, 26 mmol), NaBH₃CN (3.3 g, 53 mmol), AcOH (7.5 ml, 132 mmol) in 130 ml MeOH at 0 °C under nitrogen was added formaldehyde (37 wt % in water, 5.3 mL) as a solution in 15 ml MeOH slowly dropwise via a pressure-equalized addition funnel over 15 min. The resulting clear solution was allowed to warm to room temperature and was allowed to stir for approximately 60 h. The reaction was quenched by the addition of 20 ml saturated aqueous potassium carbonate. The mixture was concentrated in vacuo, and water and EtOAc was added. The organic layer was removed, and the aqueous layer was extracted twice with EtOAc. The combined organic layers were dried with Na₂SO₄, filtered, and concentrated to give a cloudy oil, which was dissolved in methylene chloride and filtered through a fritted funnel. The solvent was removed to give a waxy solid, which was purified by silica gel chromatography (MC/MeOH/conc. NH₄OH). The resulting material was dissolved in diethyl ether, cooled to 0°C and treated with 20 ml 4N HCl in dioxane. The solvent was removed in vacuo to give the desired product as a white solid. MS (m/z): 219.1 (M+H)⁺. Calc'd for C₁₄H₂₂N₂: 218.34.

### Step 2. N,N-dimethyl-1-(2-nitro-4-(trifluoromethyl)phenyl)piperidin-4-amine

To 1-benzyl-N,N-dimethylpiperidin-4-amine dihydrochloride (6.7 g, 23 mmol) was added Pd/C (10%, 2.4 g) under argon. Methanol (100 ml) was added via syringe, and H₂ gas was introduced and the mixture stirred vigorously under an atmosphere of H₂. After 48 h, the mixture was flushed with nitrogen, filtered through celite and concentrated to afford a mixture of starting material and N,N-dimethylpiperidin-4-amine dihydrochloride as a white solid. This solid was treated with 1-Fluoro-2-nitro-4-trifluoromethyl-benzene (3.2 ml, 22.9 mmol), triethylamine (12.7 ml, 92 mmol), and 50 ml dry THF. The mixture was heated to 75 °C with a water-cooled reflux condenser for 12 h. The mixture was allowed to cool to ambient temperature, was filtered through a fritted funnel, and concentrated to an orange oil. The residue was purified by silica gel chromatography (MC/MeOH/conc. NH₄OH) to give the desired product as an orange oil. MS (m/z): 318.1 (M+H)⁺. Calc'd for C₁₄H₁₉F₃N₃O₂: 317.31.

### Step 3. 1-(2-amino-4-(trifluoromethyl)phenyl)-N,N-dimethylpiperidin-4-amine

To N,N-dimethyl-1-(2-nitro-4-(trifluoromethyl)phenyl)piperidin-4-amine (3.4 g, 11 mmol) was added Pd/C (10%, 0.57 g) under nitrogen. Methanol (25 mL) was added via syringe, and H₂ gas was introduced and the mixture stirred vigorously under an atmosphere of H₂. After 96 h, the mixture was flushed with nitrogen, filtered through celite and concentrated. The residue was resubjected to the reaction conditions. After 12 h, the reaction was flushed with nitrogen, filtered through celite and concentrated. The resulting solid was triturated with methanol ten times to give the title compound as a pink solid. MS (m/z): 288.2 (M+H)⁺. Calc'd for C₁₄H₂₀F₃N₃: 287.32.

### Example 590

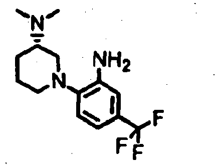

### Synthesis of (S)-1-(2-amino-4-(trifluoromethyl)phenyl)-N,N-dimethylpiperidin-3- amine

### Step 1. (S)-N,N-dimethyl-1-(2-nitro-4-(trifluoromethyl)phenyl)piperidin-3-amine

To a light yellow solution of (S)-tert-butyl 3-aminopiperidine-1-carboxylate (0.52 g, 2.6 mmol) in 25 ml MeOH was added sodium cyanoborohydride (0.33 g, 5.2 mmol), AcOH (0.74 ml, 13 mmol), and formaldehyde (37 wt.% solution in water, 1.0 ml). After stirring approximately 12 h, the reaction was quenched by the addition of 5 ml saturated aqueous sodium bicarbonate. The volatile organic solvents were removed in vacuo, and water and EtOAc was added. The organic layer was removed, and the aqueous layer was extracted twice with EtOAc. The combined organic layers were dried with Na₂SO₄, filtered, and concentrated to give a yellow oil. The resulting material was treated with 4 ml 4N HCl in dioxane at 0°C. After 2 h, the solution was concentrated in vacuo to give a light yellow solid. This solid was treated with 1-Fluoro-2-nitro-4-trifluoromethylbenzene (0.37 ml, 2.6 mmol), sodium bicarbonate (1.0 g, 13 mmol), and 5 ml dry THF. The mixture was heated to 75°C with a water-cooled reflux condenser for 12 h. The mixture was allowed to cool to ambient temperature, was filtered through a fritted funnel, and concentrated to give the desired product as an orange oil. MS (m/z): 318.0 (M+H)⁺. Calc'd for C₁₄H₁₈F₃N₃O₂: 317.31.

### Step 2. (S)-1-(2-amino-4-(trifluoromethyl)phenyl)-N,N-dimethylpiperidin-3-amine

(S)-N,N-Dimethyl-1-(2-nitro-4-(trifluoromethyl)phenyl)piperidin-3-amine (0.82 g, 2.6 mmol) was reduced with Pd/C (10%, 0.27 g) in 10 ml methanol in a manner similar to Example 589 - Step 3 to give the title compound as an orange-red oil. MS (m/z): 288.2 (M+H)⁺. Calc'd for C₁₄H₂₀F₃N₃: 287.32.

### Example 591

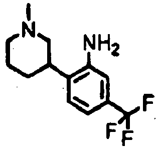

### Synthesis of 2-(1-methylpiperidin-3-yl)-5-(trifluoromethyl)benzenamine

### Step 1. 3-(2-nitro-4-(trifluoromethyl)phenyl)pyridine

A mixture of pyridin-3-ylboronic acid (0.99 g, 8.1 mmol), 2-bromo-5-(trifluoromethyl)benzenamine (1.2 ml, 8.1 mmol), tetrakis(triphenylphosphine)palladium (0.28 g, 0.24 mmol), sodium carbonate (2.0 M solution in water, 8.0 ml, 16 mmol), 4 ml ethanol, and 20 ml toluene was heated to 90 °C under nitrogen with a water-cooled reflux condenser. After 12 h, mixture was cooled to ambient temperature, and was partitioned between EtOAc and 1N NaOH. The organic layer was washed once with brine, dried with Na₂SO₄, filtered, and concentrated to give a brown oil, which was further purified by silica gel chromatography (EtOAc/hexanes) to give the desired product as a waxy orange solid. MS (m/z): 269.0 (M+H)⁺. Calc'd for C₁₂H₇F₃N₂O₂: 268.19.

### Step 2. 2-(1-methylpiperidin-3-yl)-5-(trifluoromethyl)benzenamine

To an orange solution of 3-(2-nitro-4-(trifluoromethyl)phenyl)pyridine (1.4 g, 5.2 mmol) in 2 ml acetone and 1 mL benzene was added iodomethane (1.0 ml, 16 mmol). The solution was allowed to stand for 5 days, and was concentrated in vacuo to give an orange solid. A portion of this material was treated with platinum (IV) oxide (0.11 g, 0.49 mmol) in 5 ml MeOH under an atmosphere of hydrogen for approximately 24 h. The reaction was flushed with nitrogen, filtered through celite, and concentrated. Purification by silica gel chromatography (MC/MeOH/conc. NH₄OH) provided the desired product. MS (m/z): 259.0 (M+H)⁺. Calc'd for C₁₃H₁₇F₃N₂: 258.28.

### Example 592

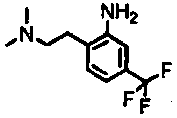

### Synthesis of 2-(2-(dimethylamino)ethyl)-5-(trifluoromethyl)benzenamine

The title compound was synthesized in a manner similar to that described in Example 58 of pending U.S. Patent Application No. 60/569,193. MS (m/z): 233.1 (M+H)⁺. Calc'd for C₁₁H₁₅F₃N₂: 232.25.

### Example 593

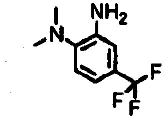

### Synthesis of N1,N1-dimethyl-4-(trifluoromethyl)benzene-1,2-diamine

The title compound was synthesized in a manner similar to Example 55 of pending U.S. Patent Application No. 60/569,193. MS (m/z): 205.1 (M+H)⁺. Calc'd for C₉H₁₁F₃N₂: 204.19.

### Example 594

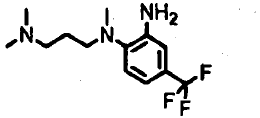

### Synthesis of N1-(3-(dimethylamino)propyl)-N1-methyl-4-(trifluoromethyl)benzene-1,2-diamine

The title compound was synthesized in a manner similar to Example 55 of pending U.S. Patent Application No. 60/569, 193. MS (ES⁺) : 276.1 (M+H)⁺. Calc' d for C₁₃H₂₀F₃N₃-275.31.

### Example 595

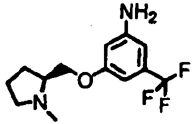

### Synthesis of (S)-3-((1-methylpyrrolidin-2-yl)methoxy)-5-(trifluoromethyl)benzenamine

The title compound was synthesized by a method similar to that described in WO 2002066470 A1.

### Example 596

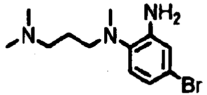

### Synthesis of 4-bromo-N1-(3-(dimethylamino)propyl)-N1-methylbenzene-1,2-diamine

To *N*-(4-Bromo-2-nitro-phenyl)-*N*, *N'*,*N'*-trimethyl-propane-1,3-diamine (Example 619, Step 1) (0.54 g, 1.7 mmol) in 20 ml EtOH was added SnCl₂ (0.51 g, 2.67 mmol). The mixture was sealed and was heated to 80°C for 12 h. An additional amount of SnCl₂ (0.51 g, 2.67 mmol) was added and heating continued for 12 h. The reaction was cooled to ambient temperature, and was poured into a mixture of EtOAc and saturated aqueous sodium bicarbonate. The mixture was filtered through celite, and the organic layer was removed. The aqueous layer was extracted twice with EtOAc, and the combined organic layers were dried with Na₂SO₄, filtered, and concentrated to give a cloudy oil. This material was filtered through silica gel with 90/10/1 dichloromethane/MeOH/conc. NH₄OH and concentrated in vacuo to give the title compound as a red oil. MS (ES⁺) : 285.9 (M)⁺. Calc'd for C₁₂H₂₀BrN₃- 286.21.

Examples 597-607 were prepared by methods similar to the procedures described in pending U.S. Patent Application No. 60/569,193.

| Example No | Structure | Example | No. Structure |
|---|---|---|---|
| 597 | 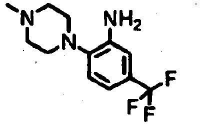 | 602 | 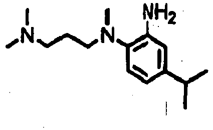 |
| 598 | 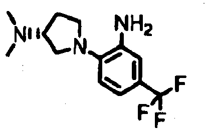 | 603 | 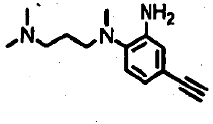 |
| 599 | 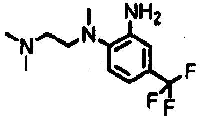 | 604 | 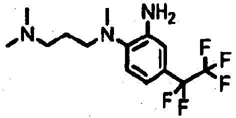 |
| 600 | 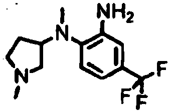 | 605 | 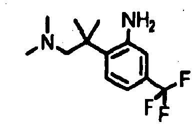 |
| 601 | 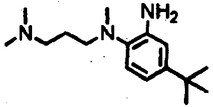 | 606 | 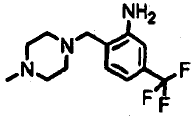 |
| | | 607 | 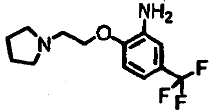 |

### Example 608

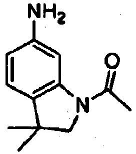

### Synthesis of 1-(6-amino-3,3-dimethylindolin-1-yl)ethanone

The title compound was prepared according to a procedure described in U.S. Patent Publication No. 2003/0203922.

### Example 609

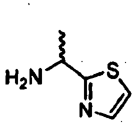

### Synthesis of 1-(thiazol-2-yl)ethanamine

The title compound was prepared by a procedure silimar to that described in J. Chem. Soc. Perkin trans., 2, 1339, 2000. NH₄OAc (38.54 g, 500 mmol) was added to 1-(thiazol-2-yl)ethanone (5.0 g, 39.3 mmol) in MeOH (100 ml). The mixture was stirred at RT for 15 min. NaCNBH₄ (1.76 g, 200 mmol) was added and the mixture was stirred for 4 d. 30 ml 6N HCl was added dropwise with the formation of a solid precipitate. The white solid was isolated by filtration then taken up in H₂O and washed with Et₂O. The aqueous solution was then basified to pH of about 10 with NaOH, Extracted with EtOAc and dried over Na₂SO₄. Purification by silica chromatography eluting with 5% MeOH/CH₂Cl₂ afforded 1-(thiazol-2-yl)ethanamine.

### Example 610

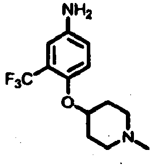

### Synthesis of 4-(1-methylpiperidin-4-yloxy)-3-(trifluoromethyl)benzenamine

The title compound was synthesized in a manner similar to Example 56 of pending U.S. Patent Application No. 60/569,193.

### Example 611

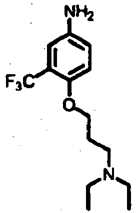

### Synthesis of 4-(3-(diethylamino)propoxy)-3-(trifluoromethyl)benzenamine

The title compound was synthesized in a manner similar to Example 610 above.

### Example 612

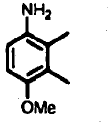

### Synthesis of 4-methoxy-2,3-dimethylbenzenamine

The title compound was synthesized in a manner similar to Example 610 above.

### Example 613

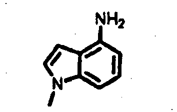

### Synthesis of 1-methyl-1H-indol-4-amine

The title compound was synthesized in a manner similar to Example 610 above.

### Example 614

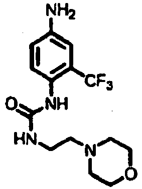

### Synthesis of 1-(4-amino-2-(trifluoromethyl)phenyl)-3-(2-morpholinoethyl)urea

### Step 1: 1-(2-morpholinoethyl)-3-(4-nitro-2-(trifluoromethyl)phenyl)urea

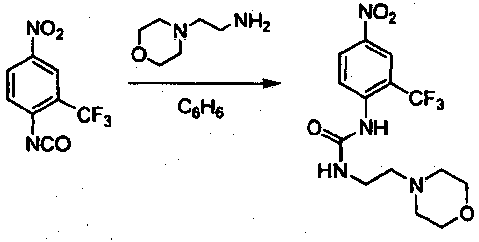

To a solution of 1-isocyanato-4-nitro-2-(trifluoromethyl)benzene (339 µL, 2.21 mmol, 1.0 equiv) in benzene (3.0 mL), was added 2-morpholinoethanamine (316 mg, 2.43 mmol, 1.0 equiv). The resulting precipitant was filtered and washed with hexanes to provide 1-(2-morpholinoethyl)-3-(4-nitro-2-(trifluoromethyl)phenyl)urea, which was advanced without further purification. MS (MH⁺) 363; Calculated for C₁₄H₁₇F₃N₄O₄: 362.1

### Step 2: 1-(4-amino-2-(trifluoromethyl)phenyl)-3-(2-morpholinoethyl)urea

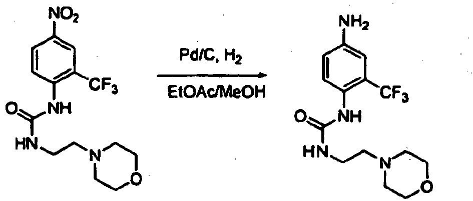

A mixture of 1-(2-morpholinoethyl)-3-(4-nitro-2-(trifluoromethyl)phenyl)urea (651 mg, 1.80 mmol, 1.0 equiv) and 10% Pd/C (20 mg) in EtOAc (25 mL) and MeOH (2 mL) was exposed to an atmosphere of H₂ (balloon). Upon completion of the reduction, the reaction mixture was filtered through celite and concentrated *in vacuo* to afford 1-(4-amino-2-(trifluoromethyl)phenyl)-3-(2-morpholinoethyl)urea, which was advanced without further purification. MS (MH⁺) 333; Calculated for C₁₄H₁₉F₃N₄O₂: 332.2

### Example 615

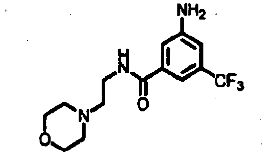

### Synthesis of 3-amino-N-(2-morpholinoethyl)-5-(trifluoromethyl)benzamide

### Step 1: N-(2-morpholinoethyl)-3-nitro-5-(trifluoromethyl)benzamide

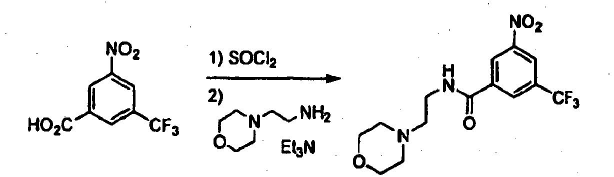

A mixture of 3-nitro-5-(trifluoromethyl)benzoic acid (300 mg, 1.29 mmol, 1.0 equiv) and thionyl chloride (2.0 ml) was heated at 75°C for 1h. The solvent was removed *in vacuo* and the residue taken up in CH₂Cl₂ (5.0 ml). To the solution was added 2-morpholinoethanamine (185 mg, 1.42 mmol, 1.1 equiv) and triethylamine (0.54 ml, 3.86 mmol, 3.0 equiv). After the reaction was complete, the solution was diluted with CH₂Cl₂ (ca.10 ml) and washed with water and brine. After drying with Na₂SO₄ and concentration *in vacuo,* the resulting N-(2-morpholinoethyl)-3-nitro-5-(trifluoromethyl)benzamide was advanced without further purification. MS (MH⁺) 348; Calculated for C₁₄H₁₆F₃N₃O₄: 347.1

### Step 2: 3-amino-N-(2-morpholinoethyl)-5-(trifluoromethyl)benzamide

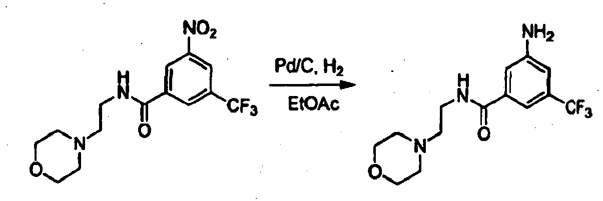

A mixture of N-(2-morpholinoethyl)-3-nitro-5-(trifluoromethyl)benzamide (300 mg, 0.865 mmol, 1.0 equiv) and 10% Pd/C (20 mg) in EtOAc (25 ml) and MeOH (2 mL) was exposed to an atmosphere of H₂ (balloon). Upon completion of the reduction, the reaction mixture was filtered through celite and concentrated *in vacuo* to afford 3-amino-N-(2-morpholinoethyl)-5-(trifluoromethyl)benzamide, which was advanced without further purification. MS (MH⁺) 318; Calculated for C₁₄H₁₈F₃N₃O₂: 317.1

### Example 616

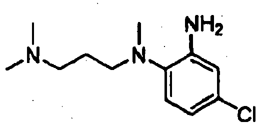

### Synthesis of 4-chloro-N1-(3-(dimethylamino)propyl)-N1-methylbenzene-1,2-diamine

### Step 1. Preparation of 4-chloro-N-(3-(dimethylamino)propyl)-N-methyl-2-nitrobenzenamine

To 2,5-dichloronitrobenzene (3.0 g, 16 mmol) was added N1,N1,N3-trimethylpropane-1,3-diamine (2.2 g, 19 mmol). The mixture was stirred for 2.5 days at RT, diluted with 0.01 N HCl and extracted with EtOAc. The aqueous layer was made basic with Na₂CO₃ and extracted with EtOAc. The organic layer was dried over anhydrous Na₂SO₄, filtered and concentrated to yield 4-chloro-N-(3-(dimethylamino)propyl)-N-methyl-2-nitrobenzenamine as an orange oil. MS m/z = 272 [M+1]⁺. Calc'd for C₁₂H₁₈ClN₃O₂: 271.75.

### Step 2. Preparation of 4-chloro-N1-(3-(dimethylamino)propyl)-N1-methylbenzene-1,2-diamine

To 4-chloro-N-(3-(dimethylamino)propyl)-N-methyl-2-nitrobenzenamine (4.0 g, 15 mmol) in EtOH (80 ml) and water (10 ml) was added Raney-Ni (10 g). The mixture was stirred for 5 hours at RT, filtered through a pad of Celite and concentrated to yield 4-chloro-N1-(3-(dimethylamino)propyl)-N1-methylbenzene-1,2-diamine as a deep red oil. MS m/z = 242 [M+1]⁺. Calc'd for C₁₂H₂₀ClN₃: 241.77.

### Example 617

### Synthesis of 3-amino-4-deuteromethoxy(-d₃)benzotrifluoride

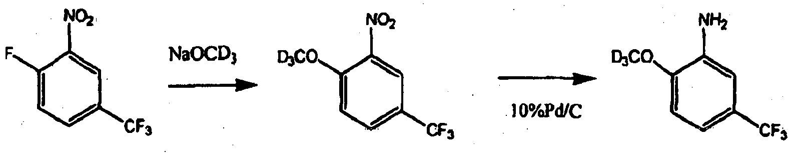

Step 1: To 10 g of deuterated methanol over an ice bath was added sodium metal until a cloudy solution formed. 4-Chloro-3-nitrobenzotrifluoride (2.25 g, 1.46 mL, 0.01 mol), was added to the solution dropwise over an ice bath. The reaction mixture was allowed to stir 24 hours at room temperature. The orange solution is brought to pH 6 (turns yellow) with acetic acid added dropwise over an ice bath.
Step 2: 10% Palladium on carbon (0.05 g) was added to a reaction mixture of the nitroaniline (0.01 mol) allowed to stir at room temperature under a H₂ (g) atmosphere (via balloon). The reaction mixture was then filtered through celite. The filtrate was concentrated to afford a yellow oil that was reconstituted in dichloromethane (5 ml) and purified by flash silica column using isocratic 90/10/1 : CH₂Cl₂/CH₃OH/NH₄OH. A very pale yellow solid is isolated. LC-MS (+) revealed a mass of 195 (M+H⁺) ; calc'd for C₈H₅D₃F₃NO: 194.17.

### Example 618.

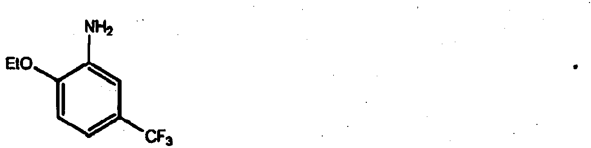

### Synthesis of 3-amino-4-ethoxybenzotrifluoride

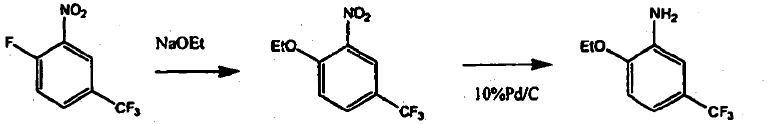

The title compound was prepared by a method similar to Example 617, using ethanol in place of deuteromethanol and purified by flash silica column using isocratic 90/10/1 : CH₂Cl₂/CH₃OH/NH₄OH. A very pale yellow solid was isolated. LC-MS (+) revealed a mass of 206 (M+H⁺) ; calc'd for C₉H₁₀F₃NO: 205.18.

### Example 619

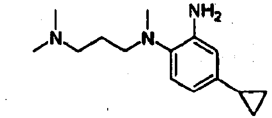

### Synthesis of 4-cyclopropyl-N1-(3-(dimethylamino)propyl)-N1-methylbenzene-1,2-diamine

### Step 1. N-(4-Bromo-2-nitro-phenyl)-N, N', N'-trimethyl-propane-1,3-diamine

To a round bottom flask at 0°C was added 4-Bromo-1-fluoro-2-nitrobenzene (10 g, 45.46mmol) and *N*, *N*, *N'-*Trimethyl-propane-1,3-diamine (6.99ml, 47.73mmol). The reaction was allowed to warm to RT and stirred for 16h. The reaction was extracted into EtOAc, washed once with saturated aqueous NaHCO₃, twice with water, and then dried over Mg₂SO₄. The organic layer was filtered and concentrated to yield the title compound as a bright orange solid.
MS (M+H⁺) = 316, 318; Calc'd for C₁₂H₁₈BrN₃O₂ = 316.19.

### Step 2. 4-cyclopropyl-N-(3-(dimethylamino)propyl)-N-methyl-2-nitrobenzenamine

To a pressure vessel was added 2-cyclopropyl-4,4,5,5-tetramethyl-1,3,2-dioxaborolane (900mg, 5.36mmol), potassium phosphate (3.0g, 14.42mmol), and 0.82mL water. After stirring at RT for 15 minutes, *N*-(4-Bromo-2-nitrophenyl)-*N*, *N*,*N*-trimethyl-propane-1,3-diamine (Step 1, 1.30g, 4.12mmol), palladium acetate (92mg, 0.412mmol), tricyclohexylphosphine (231mg 0.824mmol), and 21 ml toluene were added. The reaction was sealed and stirred at 80°C for 19h. The reaction was then cooled to RT, quenched with EtOAc and extracted into water, washed once with brine, and then dried over Mg₂SO₄. The crude mixture was then purified by reverse phase chromatography to yield the title compound as a dark red-brown oil. MS (M+H⁺) = 278; Calc'd for C₁₅H₂₃N₃O₂ = 277.36.

### Step 3. 4-cyclopropyl-N1-(3-(dimethylamino)propyl)-N1-methylbenzene-1,2-diamine

4-cyclopropyl-N-(3-(dimethylamino)propyl)-N-methyl-2-nitrobenzenamine (Step 2, 600mg, 2.16mmol) was dissolved in 22mL MeOH. Palladium (115mg, 0.108mmol, 10% w/w on carbon) was added, a balloon containing hydrogen was inserted, and the reaction was stirred at RT for 18h. The solution was then filtered through a pad of Celite and concentrated, yielding the title compound as viscous red-brown oil. MS (M+H⁺) = 248; Calc'd for C₁₅H₂₅N₃ = 247.38.

### Example 620

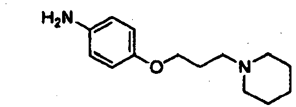

### Synthesis of 4-(3-Piperidin-1-yl-propoxy)aniline

### Step 1: 1-(3-Chloropropyl)piperidine

A mixture of 1-bromo-3-chloropropane (65.6 g, 0.417 mol) and piperidine (62 ml, 0.625 mol) in anhydrous THF (200 ml) was heated to reflux for 24 h. The mixture was cooled to RT and filtered to remove solids. The organics were concentrated under in vacuo. The resultant residue was taken up in 2N HCl and washed twice with ethyl acetate. The aqueous layer was basicified with 2N NaOH to pH 14. The compound was extracted three times with ethyl acetate and the combined organics dried over anhydrous magnesium sulfate. The solution was then concentrated under reduced pressure to give the desired compound as a yellowish oil.

### Step 2: 1-[3-(4-nitrophenoxy)propyl]piperidine

In a three-necked flask fitted with an overhead mechanical stirrer, a mixture of 1-(3-chloropropyl)piperidine (49.8 g, 0.308 mol), 4-nitrophenol (42.8 g, 0.308 mol) and potassium carbonate (212 g, 1.53 mol), in anhydrous DMF (200 mL) was heated to 94°C and stirred for 18 h. The mixture was cooled to room temperature, then diluted with 2 L water. The organics were taken up in ethyl acetate and washed twice with 2N sodium hydroxide and then brine. The combined organics were dried over magnesium sulfate then concentrated under reduced pressure to give the title compound as a yellowish oil.

### Step 3: 4-(3-Piperidin-1-ylpropoxy)aniline

A mixture of 1-[3-(4-nitrophenoxy)propyl]piperidine (15.5 g, 58.6 mmol) and 10% Pd/C (12.5g) in 150 mL of EtOH was placed under a balloon of H₂. The mixture was stirred for 18 h. The catalyst was removed by suction filtration and the organics concentrated to give the title compound as a yellowish oil. MS (m/z) = 235.2 (M+H⁴); Calc'd for C₁₄H₂₂N₂O = 234.34.

### Example 621

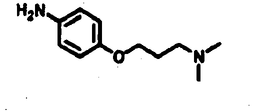

### Synthesis of 4-(3-(dimethylamino)propoxy)aniline

### Step 1: 1-(3-chloropropoxy)-4-nitrobenzene

A solution of 4-Nitrophenol (10 g, 72 mmol) dissolved in acetonitrile (100 ml was charged with potassium carbonate (24.9 g, 180 mmol) and 1-bromo-3-chloropropane (113.2 g, 720 mmol). The mixture was heated and stirred at reflux overnight. The reaction was cooled to room temperature, the solids filtered off and the solvent evaporated under reduced pressure to give the title compound.

### Step 2: 4-(3-(dimethylamino)propoxy)nitrobenzene

A mixture of 1-(3-chloropropoxy)-4-nitrobenzene (2 g, 9.27 mmol), potassium carbonate (7.69 g, 46.4 mmol) and acetonitrile (15 ml) was prepared and stirred in a tube. To the stirring solution dimethylamine hydrochloride (3.78 g, 46.4 mmol) was added quickly. The tube was sealed and the mixture was stirred while heating overnight at 80°C. The mixture was cooled well before opening the pressure tube, then water and dichloromethane were added and the aqueous layer was extracted with dichloromethane. The combined organics were dried and evaporated giving the title product.

### Step 3: 4-(3-(dimethylamino)propoxy)aniline

4-(3-(dimethylamino)propoxy)nitrobenzene (4.4 g, 19.6 mmol) was hydrogenated over Pd (10% on C, 0.4 g) in ethanol (50 ml) for 16 h. The catalyst was filtered off and the solvent removed under reduced pressure to afford the title compound as a brown oil. MS (m/z) = 195.3 (M+H⁺) ; Calc'd for C₁₁H₁₈N₂O = 194.28.

### Example 622

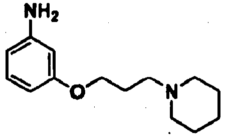

### Synthesis of 3-(3-Piperidin-1-yl-propoxy)aniline

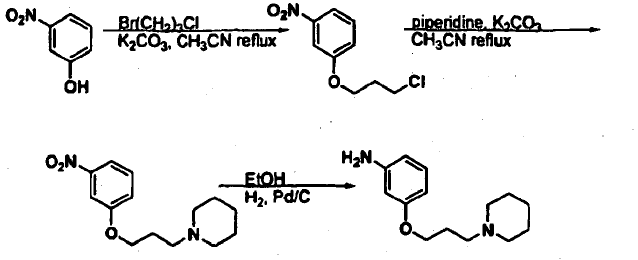

The title compound was prepared by a method similar to that described in Example 621 above, wherein 3-nitrophenol was substituted for 4-nitrophenol in Step 1 and piperidine for dimethylamine hydrochloride in Step 2. MS (m/z) = 235.2 (M+H⁺) ; Calc'd for C₁₄H₂₃N₂O = 234.34.

### Example 623

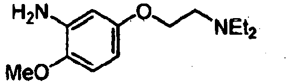

### Synthesis of 5-(2-(diethylamino)ethoxy)-2-methoxyaniline

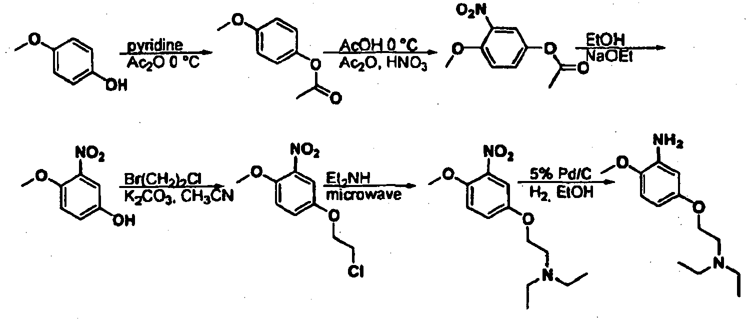

### Step 1: 4-Methoxyphenylacetate

4-Methoxyphenol (2 g, 16 mmol) was dissolved in anhydrous pyridine (6.5 ml) and stirred while cooling at 0°C under a nitrogen atmosphere. Acetic anhydride (7.5 ml, 80 mmol) was added. The reaction was allowed to warm to room temperature, where it was stirred for 16 h. The reaction was cooled in an ice bath before quenching with ice. The solution was neutralized with saturated aqueous sodium bicarbonate solution and then extracted with ethyl acetate. The combined organic extracts were washed twice with 2M HCl, then with saturated aqueous copper sulfate solution to remove residual pyridine. The organic extract was further washed with 5M aqueous sodium hydroxide solution and brine, then dried over sodium sulfate and concentrated under reduced pressure to afford a clear oil, which crystallized to give the title compound as a white solid.

### Step 2: 4-Methoxy-3-nitrophenylacetate

4-Methoxyphenylacetate (2.37g, 14.3 mmol) was dissolved in glacial acetic acid (4 ml) and cooled to 5-10 °C. A chilled mixture of glacial acetic acid (1.3 ml), fuming nitric acid (0.9 ml) and acetic anhydride (1.3 ml) was added dropwise as the temperature gradually increased to 25 °C. The reaction was stirred for 1h, then quenched with ice and diluted with water. The resulting precipitate was isolated by filtration, rinsed with water and dried in vacuo to afford the title compound as a fine crystalline yellow solid.

### Step 3: 4-Methoxy-3-nitrophenol

4-Methoxy-3-nitrophenylacetate (2.46 g, 11.7 mmol) was dissolved in anhydrous ethanol (80 ml) and sodium ethoxide (1.19 g, 17.5 mmol) was added. The reaction was stirred at room temperature for 0.5 h. The dark red solution was acidified with 2M HCl and concentrated under reduced pressure. The residue was taken up into water and extracted with dichloromethane. The combined organics were washed with 2M HCl and brine, then dried over sodium sulfate. Evaporation of the solvent under reduced pressure gave the title compound as a yellow solid.

### Step 4: 4-(2-chloroethoxy)-1-methoxy-2-nitrobenzene

4-Methoxy-3-nitrophenol (0.8 g, 4.7 mmol) was dissolved in acetonitrile (13 ml). Potassium carbonate (1.63g, 11.8 mmol) was added, followed by 1-bromo-2-chloroethane (3.93 ml, 47.2 mmol). The reaction was heated and stirred at reflux for 20h. The reaction was cooled to room temperature, the solid was then filtered off and the solvent evaporated under reduced pressure to give the title compound.

### Step 5: N,N-Diethyl-2-(4-methoxy-3-nitrophenoxy)ethylamine

4-(2-chloroethoxy)-1-methoxy-2-nitrobenzene (0.15 g, 0.67 mmol) was dissolved in acetonitrile (1 ml). Excess diethylamine (1.5 ml, 17.7 mmol) was added and the reaction heated in the microwave (T= 120 °C, 40 min) to complete conversion. The reaction mixture was diluted with dichloromethane, then washed with 5M sodium hydroxide and brine, then dried over sodium sulfate. Evaporation of the solvent under reduced pressure gave the title compound as an orange oil. MS found: 239 (M+H⁺) Calc'd for _ _ _ _ _:

### Step 6: 5-(2-(diethylamino)ethoxy)-2-methoxyphenylamine

*N*,*N*-diethyl-2-(4-methoxy-3-nitrophenoxy)ethylamine (0.29 g, 1.1 mmol) was hydrogenated over Pd (5% on C, 50% wet, 0.12 g) in ethanol (5 ml) for 16 hours. The catalyst was filtered off and the solvent removed under reduced pressure to afford the title compound as a red oil. MS (m/z) = 239 (M+H⁺) ; Calc'd for C₁₃H₂₂N₂O₂ = 238.33.

### Example 624

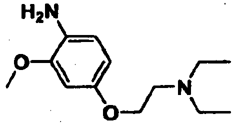

### Synthesis of 4-(2-(diethylamino)ethoxy)-2-methoxyaniline

### Step 1: 4-Fluoro-2-methoxynitrobenzene

5-Fluoro-2-nitrophenol (6 g, 38.2 mmol) was dissolved in anhydrous DMF (20 ml). Potassium carbonate (5.3g, 38.2 mmol) was added, followed by iodomethane (2.28 ml, 38.2 mmol). The reaction was stirred at room temperature for 16h, then partitioned between dichloromethane and water. The organic layer was washed three times with 1M sodium hydroxide and once with brine, then dried over sodium sulfate. Removal of the solvent in vacuo afforded the title compound as a yellow oil, which solidified upon standing.

### Step 2: 3-Methoxy-4-nitrophenol

4-Fluoro-2-methoxynitrobenzene (4.68g, 27.4mmol) was suspended in a 5M potassium hydroxide solution (50 ml) and heated to 90°C for 5h. The red solution was cooled to room temperature and acidified to pH 6 with 1M HCl. The aqueous solution was extracted three times with ethyl acetate and the combined organics were washed with brine and dried over sodium sulfate. Removal of the solvent under reduced pressure, followed by purification by flash column chromatography (1:1 hexane/ethyl acetate) afforded the title compound as a yellow solid.

### Step 3: 4-(2-chloroethoxy)-2-methoxy-1-nitrobenzene

3-Methoxy-4-nitrophenol (0.6 g, 3.6 mmol) was dissolved in acetonitrile (15 ml). Potassium carbonate (1.3 g, 9.1 mmol) was added, followed by 1-bromo-2-chloroethane (5.1 g, 35.5 mmol). The reaction was stirred in a sealed pressure tube at 80°C for 20h. The reaction was cooled to room temperature, the solid was then filtered off and the solvent evaporated under reduced pressure. The residue was then taken up into ethyl acetate and washed with 1M sodium hydroxide, brine, and then dried over sodium sulfate. Evaporation of the solvent afforded the title compound as a yellow solid.

### Step 4: N,N-Diethyl-2-(3-methoxy-4-nitrophenoxy)ethylamine

4-(2-Chloroethoxy)-2-methoxy-1-nitrobenzene (0.22 g, 0.9 mmol) was dissolved in acetonitrile (1 ml). Diethylamine (0.14 ml, 2.6 mmol) and potassium carbonate (0.31g, 2.2 mmol) were added and the reaction was heated in a sealed pressure tube to 80 °C for 20h. The reaction mixture was diluted with dichloromethane, then washed with 1M sodium hydroxide and brine, then dried over sodium sulfate.
Evaporation of the solvent under reduced pressure gave the title compound as a brown oil.

### Step 5: N,N-Diethyl-2-(4-amino-3-methoxyphenoxy)ethylamine

N,N-Diethyl-2-(3-methoxy-4-nitrophenoxy)ethylamine (140 mg, 0.5 mmol) was hydrogenated over Pd (5% on C, 50% wet, 40 mg) in ethanol (5 ml) for 16 hours. The catalyst was filtered off and the solvent removed under reduced pressure to afford the title compound as a brown oil. MS (m/z) = 239 (M+H⁺) ; Calc'd for C₁₃H₂₂N₂O₂ = 238.33

### Example 625

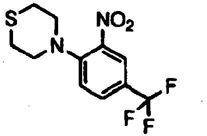

### Synthesis of 4-(2-nitro-4-(trifluoromethyl)phenyl)thiomorpholine

To a solution of 1-fluoro-2-nitro-4-(trifluoromethyl)benzene (7.00 g, 33.48 mmol) in THF (250 ml) at room temperature was added thiomorpholine (3.45 g, 33.48 mmol) and sodium bicarbonate (3.66 g, 43.52 mmol). The vessel was purged with nitrogen and stirred at room temperature for 48 hours. After removal of solvent under reduced pressure, the mixture was taken up in ethyl acetate and filtered. The organics were washed with water, then brine and dried with magnesium sulfate. Filtration and concentration provided the title compound as a bright orange solid. MS m/z: 293.1 (M+H⁺); calc MW = 292.28.

### Example 626

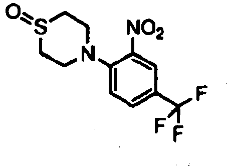

### Synthesis of the sulfoxide of 4-(2-nitro-4-(trifluoromethyl)phenyl)thiomorpholine

To a solution of 4-(2-nitro-4-(trifluoromethyl)phenyl)thiomorpholine (2.0 g, 6.84 mmol) in methanol (60 ml) and water (15 ml) was added NaIO₄ (1.61 g, 7.53 mmol). The mixture was allowed to stir at room temperature for 12 hours, at which time it was filtered to remove white solid precipitates. Concentration afforded the title compound as an orange solid. MS m/z: 309 (M+H⁺); calc'd MW = 308.28.

### Example 627

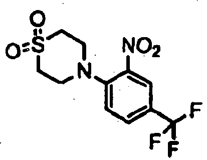

### Synthesis of the sulfone of 4-(2-nitro-4-(trifluoromethyl)phenyl)thiomorpholine

To a solution of the sulfoxide of 4-(2-nitro-4-(trifluoromethyl)phenyl)thiomorpholine (170 mg, 0.55 mmol) in methanol (50 ml) was added KMNO₄ (96 mg, 0.61 mmol). The reaction was stirred at room temperature for 15 minutes and then quenched by the addition of aqueous saturated sodium bisulfate (20 ml). The reaction was filtered and concentrated to provide the sulfone product. MS m/z: 325 (M+H⁺) ; calc'd MW = 324.28.

The nitro groups of Examples 625-627 were reduced to the corresponding amine by conventional methods, such as be hydrogenation in the presence of a palladium catalyst. The reduction product of Example 625 was found to have a MS (m/z) = 263.1 (M+H⁺); calc'd MW = 262.30, and the reduction product of Example 627 was found to have a MS (m/z) = 295.1 (M+H⁺); calc'd MW = 294.30.

### Example 628

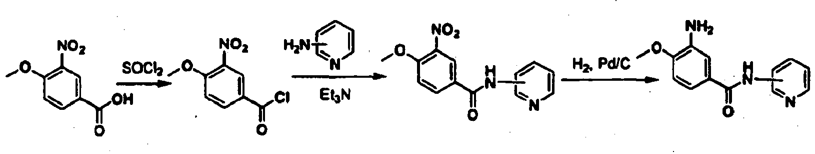

### Synthesis of 3-amino-4-methoxy-N-(pyridine-3-yl)benzamide.

Step 1: 4-methoxy-3-nitrobenzoic acid (10.0 g, 0.051 mol), and thionyl chloride (10.0 g, 0.051 mol), were refluxed together for 24 hours. The reaction mixture was cooled to room temperature and concentrated. The off-white solid was carried onto the next step.
Step 2: The acid chloride intermediate was stirred with pyridyl amine in TEA to form the amide.
Step 3: The nitro-amide intermediate can be reduced to the corresponding amine by conventional methods such as with tin or zinc in the presence of acid, to afford the title compound.

Various different B rings (R³ groups), which are contemplated herein, may be commercially purchased or made by various methods, as represented by Examples 629-632.

### Example 629

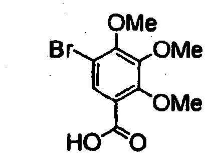

### Synthesis of 5-bromo-2,3,4-trimethoxybenzoic acid

To a solution of 2,3,4-trimethoxybenzoic acid (4.7 g, 22 mmol) and NaOAc (5.5 g, 40 mmol) in 35 ml AcOH was added a solution of bromine (1.5 ml, 29 mmol) in 35 ml AcOH. The reaction became red in color, which quickly faded. The mixture was heated to 80°C for 1 h, at which point it was cooled to ambient temperature. The material was partitioned between dichloromethane and water. The organic layer was removed and the aqueous layer was extracted once with dichloromethane. The combined organic layers were dried with Na₂SO₄, filtered, and concentrated to give an oil which solidified on standing. The material was dissolved in diethyl ether and hexanes. Concentration to ½ the volume resulted in precipitation of a white solid. Filtration provided the title compound as a white solid. MS m/z: 293 (M+H⁺) ; calc'd for C₁₀H₁₁BrO₅: 396.5

### Example 630

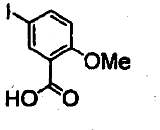

### Synthesis of 5-iodo-2-methoxybenzoic acid

### Step 1. Synthesis of methyl 5-iodo-2-methoxy benzoate

To a solution of 5-iodosalicylic acid (10.0 g, 38 mmol) in 189 ml acetone was added potassium carbonate (23 g, 169 mmol). The mixture was cooled to 0 °C and dimethyl sulfate (7.7 ml, 80 mmol) was added. The mixture was heated to reflux overnight and was cooled to ambient temperature and concentrated under reduced pressure. The residue was partitioned between EtOAc and water, and the aqueous layer was extracted three times with EtOAc. The combined organic layers were dried with Na₂SO₄, filtered, and concentrated to give a white solid. This material was heated with hexanes and allowed to stand for 60 h, resulting in the formation of crystals. Filtration provided the title compound as white needles. MS (ES⁺) : 292.9 (M+H)⁺. Calc'd for C₉H₉IO₃: 292.07.

### Step 2. Synthesis of 5-iodo-2-methoxybenzoic acid

A mixture of methyl 5-iodo-2-methoxy benzoate (6.0 g, 21 mmol) and 23 ml each MeOH and IN NaOH was heated with a water-cooled reflux condensor to 90 °C for 2 h. The reaction was cooled to ambient temperature, 100 mL water was added, and the solution adjusted to pH 1 with 6N HCl. A thick white precipitate formed which was collected by filtration to give the title compound as a white solid. MS (ES⁺) : 278. 9 (M+H)⁺. Calc'd for C₈H₇IO₃: 278. 04 .

### Example 631

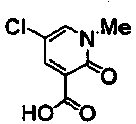

### Synthesis of 5-chloro-1-methyl-2-oxo-1,2-dihydropyridine-3-carboxylic acid

### Step 1. Synthesis of methyl 5-chloro-1-methyl-2-oxo-1,2-dihydropyridine-3-carboxylate

To a suspension of 5-chloro-2-hydroxynicotinic acid (2.0 g, 12 mmol) and cesium carbonate (8.2 g, 26 mmol) in 50 mL DMF was added MeI (1.6 ml, 26 mmol). The reaction was allowed to stir for approximately 12 h. The cloudy yellow mixture was added to EtOAc/water. The organic layer was removed and the aqueous layer was extracted three times with EtOAc. The combined organic layers were washed once with water and brine, dried with Na₂SO₄, filtered, and concentrated to give an orange-yellow solid. The material was partitioned between IN HCl and EtOAc. The organic layer was washed twice with 1N HCl, dried with Na₂SO₄, filtered, and concentrated to give the desired product as an orange solid. MS (ES⁺) : 202.0 (M+H)⁺. Calc'd for C₈H₈ClNO₃: 201.61.

### Step 2. Synthesis of 5-chloro-1-methyl-2-oxo-1,2-dihydropyridine-3-carboxylic acid

A mixture of methyl-5-chloro-1-methyl-2-oxo-1,2-dihydropyridine-3-carboxylate (0.36 g, 1.8 mmol) and 2.0 mL each MeOH and 1N NaOH was heated in a sealed vial to 80 °C for 1h. The reaction was cooled to ambient temperature, and the methanol was removed by a stream of nitrogen. Water (2 ml) was added and the solution was adjusted to pH 1 with 6N HCl. A thick white precipitate formed which was partitioned between water and dichloromethane. The organic layer was removed and the aqueous layer was extracted twice with dichloromethane. The combined organic layers were dried with Na₂SO₄, filtered, and concentrated to give an orange-yellow solid. The material was partitioned between 1N HCl and EtOAc. The organic layer was washed twice with 1N HCl, dried with Na₂SO₄, filtered, and concentrated to give the desired product as a light orange solid. MS (ES⁺): 188.0 (M+H)⁺. Calc' d for C₇H₆ClNO₃: 187 . 58 .

### Example 632

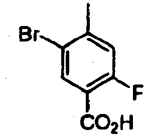

5-bromo-2-fluoro-4-methylbenzoic acid was prepared by a method described in PCT Patent Publication WO 2003/032972.

### Example 633

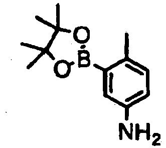

### Synthesis of 4-methyl-3-(4,4,5,5-tetramethyl-1,3,2-dioxaborolan-2-yl)benzenamine

To a solution of 3-iodo-4-methylaniline (5.0 g, 21.45 mmol) in DMSO (60 ml) was added bis-pinacolborane (6.0 g, 23.60 mmol), Pd(dppf)Cl₂ (471 mg, 0.64 mmol) and potassium acetate (6.32 g, 64.36 mmol). The reaction vessel was purged with nitrogen and heated at 80°C for 12 hours. The solvent was removed under reduced pressure and the mixture was taken up in ethyl acetate. The resulting suspension was filtered and the solid washed several times with ethyl acetate. The combined organics were washed with water, then brine, and then dried with magnesium sulfate. After filtration and concentration, the mixture was chromatographed on silica gel (0 to 5% MeOH/CH₂Cl₂ gradient) to provide the title compound. MS (m/z) = 234(M+H+); calc'd for C₁₃H₂₀BNO₂ :233.12

Various different A-B linked ring intermediates (substituted R³ groups), which are contemplated herein, may be made by various methods, such as with A-B amide linked rings, as represented by Examples 634-640.

### Example 634

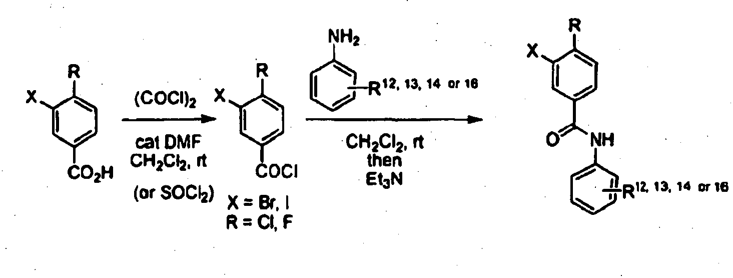

### Synthesis of 3-bromo-4-fluoro-N-(2-fluoro-3-(trifluoromethyl)phenyl)benzamide

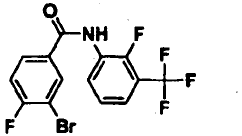

### Step 1: 3-Bromo-4-fluorobenzoyl chloride

Oxalyl chloride (1.739 g, 1.20 ml, 13.7 mmol) was added dropwise to a solution of 3-bromo-4-fluorobenzoic acid (0.600 mg, 2.74 mmol) and dichloromethane (9 ml). N,N-Dimethylformamide (1 drop) was added and the colorless solution stirred at rt for 1 h. The solution was concentrated to afford 3-bromo-4-fluorobenzoyl chloride an off-white solid which was used directed without purification.

### Step 2: 3-Bromo-4-fluoro-N-(2-fluoro-3-(trifluoromethyl)phenyl)benzamide

2-Fluoro-3-(trifluromethyl)aniline (0.515 g, 0.37 mL, 2.88 mmol) was added to a solution of 3-bromo-4-fluorobenzoyl chloride (0.650 g, 2.74 mmol) in dichloromethane (5 ml), and the mixture stirred at room temperature for 30 min. Triethylamine (0.360 g, 0.50 ml, 3.56 mmol) was added and the solution stirred at room temperature for 1 h. The reaction mixture was partitioned between dichloromethane and saturated aqueous sodium bicarbonate solution. The aqueous phase was separated and extracted with dichloromethane. The combined organic phases were washed with water, brine, dried over anhydrous sodium sulfate, filtered, and concentrated to afford a light yellow solid. Trituration with dichloromethane and filtering afforded 3-bromo-4-fluoro-N-(2-fluoro-3-(trifluoromethyl)phenyl)benzamide as a white solid. MS (M-1) 377.9; Calculated for C₁₄H₇BrF₅NO: 379.

### Example 635

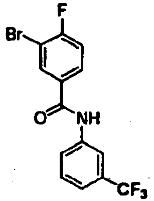

### Synthesis of 3-Bromo-4-fluoro-N-(3-(trifluoromethyl)phenyl)benzamide

3-Bromo-4-fluoro-N-(3-(trifluoromethyl)phenyl)benzamide was synthesized from 3-trifluoromethylaniline and 3-bromo-4-fluorobenzoyl chloride according to the procedure described in Example 634, affording the title compound as a white solid. MS (M-1) 360.0; Calculated for C₁₄H₈BrF₄NO: 361.

### Example 636

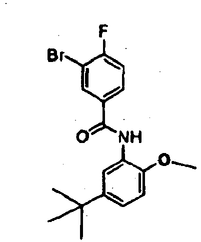

### Synthesis of 3-Bromo-N-(5-tert-butyl-2-methoxyphenyl)-4-fluorobenzamide

3-Bromo-N-(5-tert-butyl-2-methoxyphenyl)-4-fluorobenzamide was synthesized from 5-tert-butyl-o-anisidine and 3-bromo-4-fluorobenzoyl chloride according to the procedure described in Example 634, affording the title compound as an off-white solid. MS (MH+) 380.0; Calculated for C₁₈H₁₉BrFNO₂: 379.

### Example 637

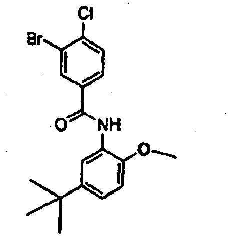

### Synthesis of 3-Bromo-N-(5-tert-butyl-2-methoxyphenyl)-4-chlorobenzamide

### Step 1: 3-Bromo-4-chlorobenzoyl chloride

3-Bromo-4-chlorobenzoyl chloride was prepared from 3-bromo-4-chlorobenzoic acid according to the procedure described in Example 634 for the synthesis of 3-bromo-4-fluorobenzoyl chloride.

### Step 2: 3-Bromo-N-(5-tert-butyl-2-methoxyphenyl)-4-chlorobenzamide

3-Bromo-N-(5-tert-butyl-2-methoxyphenyl)-4-chlorobenzamide was synthesized from 5-tert-butyl-o-anisidine and 3-bromo-4-chlorobenzoyl chloride according to the procedure described in Example 634, step 2. 3-Bromo-N-(5-tert-butyl-2-methoxyphenyl)-4-chlorobenzamide was obtained as an off-white solid. MS (M-1) 394.0; Calculated for C₁₈H₁₉BrClNO₂: 395.

### Example 638

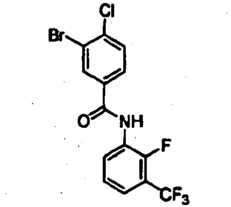

### Synthesis of 3-Bromo-4-chloro-N-(2-fluoro-3-(trifluoromethyl)phenyl)benzamide

3-Bromo-4-chloro-N-(2-fluoro-3-(trifluoromethyl)phenyl)benzamide was synthesized from 2-fluoro-3-(trifluromethyl)aniline and 3-bromo-4-chlorobenzoyl chloride according to the procedure described in Example 634. 3-Bromo-4-chloro-N-(2-fluoro-3-(trifluoromethyl)phenyl)benzamide was obtained as a red-orange solid. MS (M-1) 393.9; Calc' d for C₁₄H₇BrClF₄NO: 395.

### Example 639

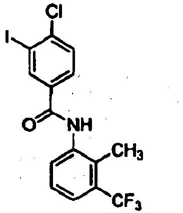

### Synthesis of 4-Chloro-3-iodo-N-(2-methyl-3-(trifluoromethyl)phenyl)benzamide

### Step 1: 4-Chloro-3-iodobenzoylchloride

4-Chloro-3-iodobenzoylchloride was prepared from 4-chloro-3-iodobenzoic acid according to the procedure described in Example 634 for the synthesis of 3-bromo-4-fluorobenzoyl chloride.

### Step 2: 4-Chloro-3-iodo-N-(2-methyl-3-(trifluoromethyl)phenyl)benzamide

4-Chloro-3-iodo-N-(2-methyl-3-(trifluoromethyl)phenyl)benzamide was synthesized from 2-methyl-3-(trifluromethyl)aniline and 4-chloro-3-iodobenzoyl chloride according to the procedure dscribed in Example 634. 4-Chloro-3-iodo-N-(2-methyl-3-(trifluoromethyl)phenyl)benzamide was obtained as a white solid. MS (M-1) 437.8; Calculated for C₁₅H₁₀ClF₃INO: 439.

### Example 640

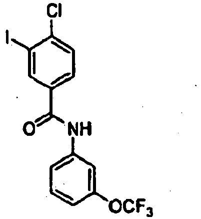

### Synthesis of 4-Chloro-3-iodo-N-(3-(trifluoromethoxy)phenyl)benzamide

4-Chloro-3-iodo-N-(3-(trifluoromethoxy)phenyl)benzamide was synthesized from 3-(trifluoromethoxy)aniline and 4-chloro-3-iodobenzoylchloride according to the procedure described in Example 634, affording the title compound as a white solid. MS (M-1) 439.8; Calculated for C₁₄H₈ClF₃INO₂: 441.

The following A-B amide linked ring intermediates. Examples 641-721, were made by methods similar to that described in Examples 169 and 634.

| Example No. | Structure | Example No. | Structure |
|---|---|---|---|
| 641 | 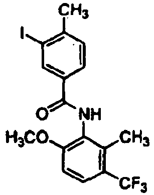 | 684 | 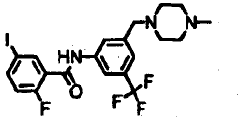 |
| 642 | 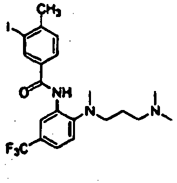 | 685 | 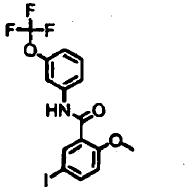 |
| 643 | 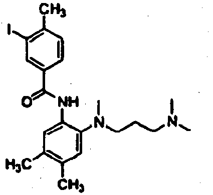 | 686 | 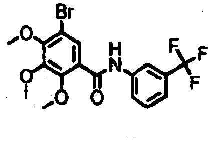 |
| 644 | 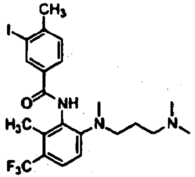 | 687 | 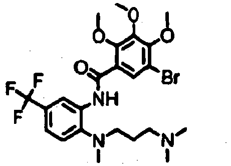 |
| 645 | 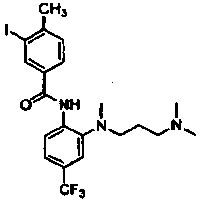 | 688 | 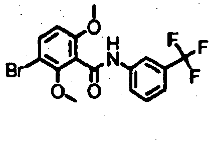 |
| 646 | 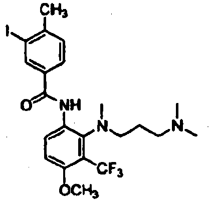 | 689 | 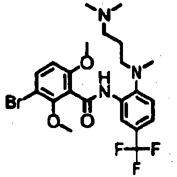 |
| 647 | 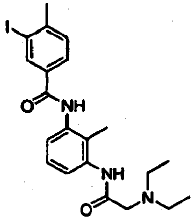 | 690 | 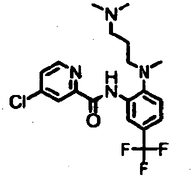 |
| 648 | 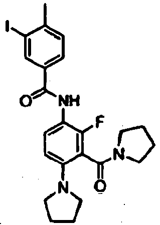 | 691 | 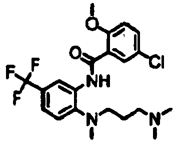 |
| 649 | 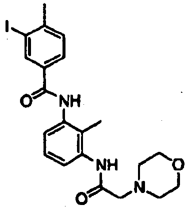 | 692 | 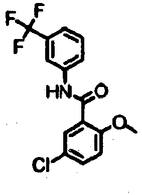 |
| 650 | 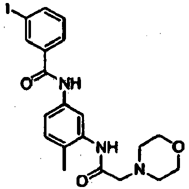 | 693 | 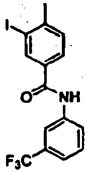 |
| 651 | 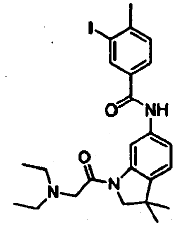 | 694 | 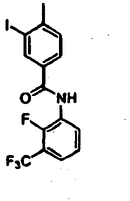 |
| 652 | 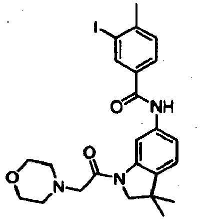 | 695 | 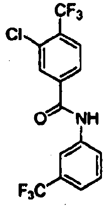 |
| 653 | 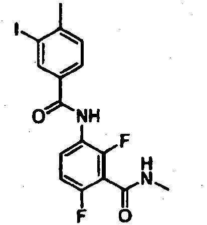 | 696 | 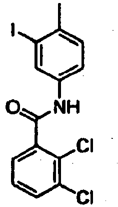 |
| 654 | 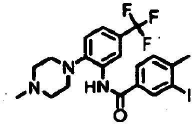 | 697 | 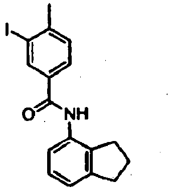 |
| 655 | 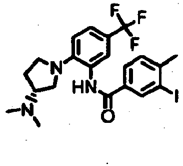 | 698 | 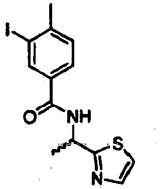 |
| 656 | 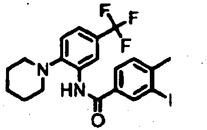 | 699 | 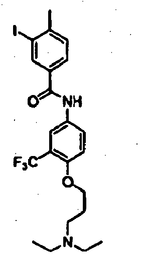 |
| 657 | 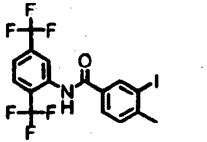 | 700 | 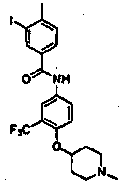 |
| 658 | 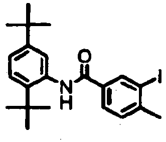 | 701 | 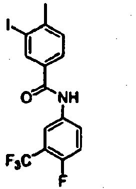 |
| 659 | 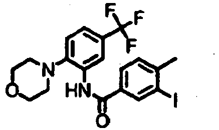 | 702 | 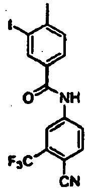 |
| 660 | 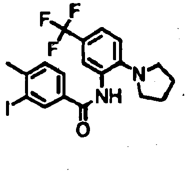 | 703 | 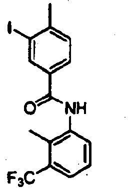 |
| 661 | 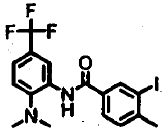 | 704 | 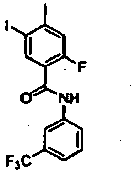 |
| 662 | 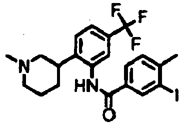 | 705 | 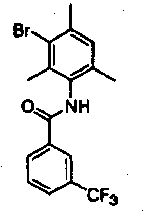 |
| 663 | 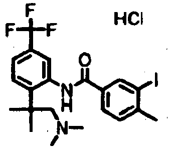 | 706 | 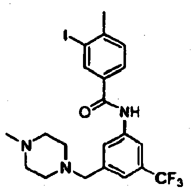 |
| 664 | 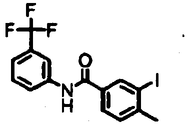 | 707 | 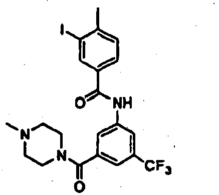 |
| 665 | 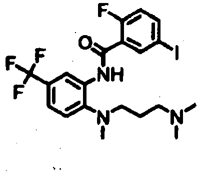 | 708 | 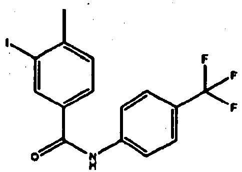 |
| 666 | 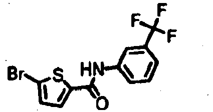 | 709 | 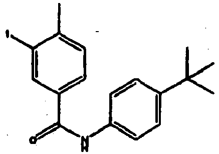 |
| 667 | 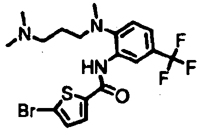 | 710 | 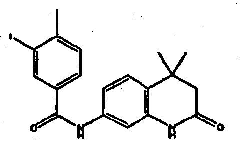 |
| 668 | 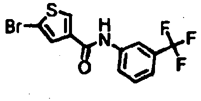 | 711 | 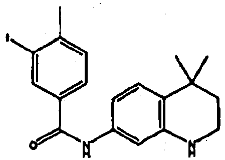 |
| 669 | 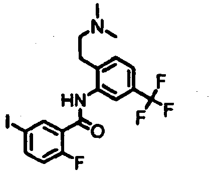 | 712 | 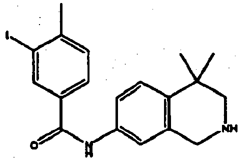 |
| 670 | 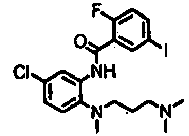 | 713 | 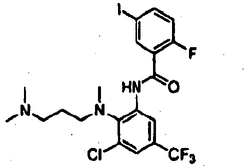 |
| 671 | 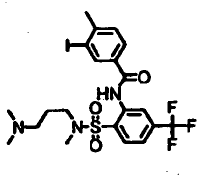 | 714 | 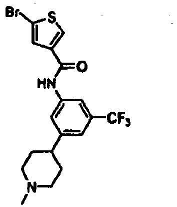 |
| 672 | 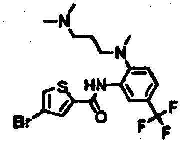 | 715 | 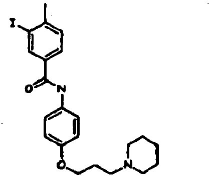 |
| 673 | 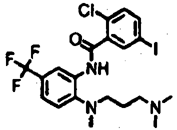 | 716 | 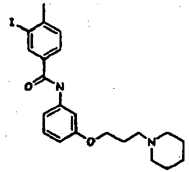 |
| 674 | 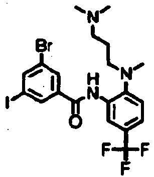 | 717 | 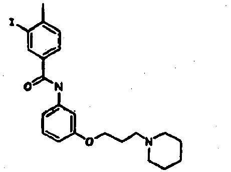 |
| 675 | 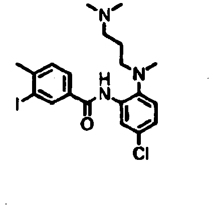 | 718 | 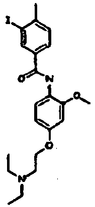 |
| 676 | 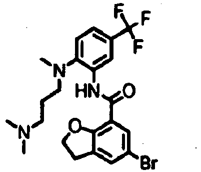 | 719 | 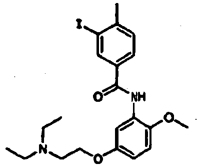 |
| 677 | 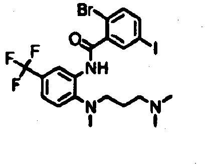 | 720 | 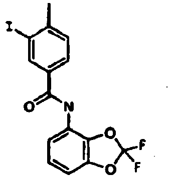 |
| 678 | 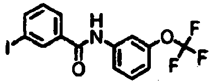 | 721 | 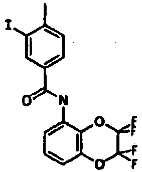 |
| 679 | 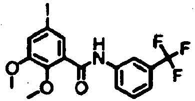 | | |
| 680 | 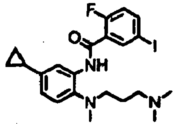 | | |
| 681 | 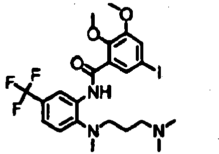 | | |
| 682 | 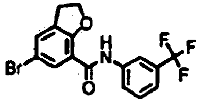 | | |
| 683 | 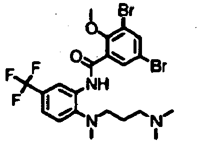 | | |

Various different CD rings (quinolines, quinazolines, aza-quinazolines and diaza-quinazolines), which are contemplated herein, may be made by various methods, as represented by Examples 722-740 and 742-744.

### Example 722

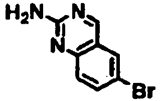

### Synthesis of 6-bromoquinazolin-2-amine

Quinazoline-2,6-diamine (5.0 g, 30.9 mmol, see Example 744) was dissolved in a solution of methanesulfonic acid (82 mL, 2 M solution in water) at 0 °C to give an orange solution. A solution of sodium nitrite (2.3 g, 34.0 mmol) in 10 ml water was added slowly dropwise via addition funnel over 15 minutes. The resulting orange/brown solution was allowed to stir for 30 min, and was poured slowly into a dark purple solution of CuBr (4.9 g, 34 mmol) in 82 ml of 48% HBr at 0 °C. The resulting purple mixture with a white precipitate was allowed to stir for 30 min, at which point a reflux condenser was added and the mixture heated to 70 °C for 1 h. The resulting homogeneous black-purple solution was cooled to room temperature and was transferred to a 1 L Erlenmeyer flask. With stirring at 0 °C, the solution was basified with conc. Ammonium hydroxide (approx 250 ml), resulting in the formation of a fine yellow precipitate. The mixture was filtered through a filter paper cone, and the resulting orange-yellow solids were rinsed into a separate 1 L Erlenmeyer flask with 300 ml 1N HCl to give an orange solution. The filtrate was basified to pH 9-10 with concentrated ammonium hydroxide, resulting in the formation of a yellow precipitate. The mixture was extracted with ethyl acetate (8 x 700 ml), and the combined organic extracts were dried over anhyd. Sodium sulfate, filtered, and concentrated in vacuo to give a light yellow powder. This material was further purified by the following procedure: the solid material and 500 ml ethanol were heated to 75°C. 400 ml water was added and the mixture reheated to 75°C. The resulting mixture was allowed to cool to room temperature and was held at 0°C overnight. The resulting mixture was filtered through a 0.45 micron membrane, and the solids washed with 50 ml each ethanol and diethyl ether, and dried under vacuum to give the title compound as a yellow powder.

2-Amino-6-bromoquinazoline was also made by an alternative method, as described below:
A stirred mixture of guanidine carbonate (281 g, 1.56 mol, 1.3 equiv), diisopropyl-ethylamine (DIPEA, 540 mL, 3.12 mol, 2.6 equiv) and 1-methylpyrrolidinone (NMP, 2 L) was heated to about 150-160°C with a heating mantle. A solution of 5-bromo-2-fluoro-benzaldehyde (250 g, 1.2 mol, 1.0 equiv) in NMP (100 ml) was added dropwise via addition funnel over 1 h while remaining at reflux. Upon complete addition, the mixture was maintained at 150-160°C for an additional 1-2 h until complete consumption of the aldehyde was determined by LC analysis. Upon completion, the mantle was turned off and the reaction was allowed to cool to below 100°C. At this point, the reaction was quenched by the addition of ice (2 kg) and water (4 L). The resulting bronze solid was stirred for an additional 30 min, and isolated by vacuum filtration. The solids were washed with water (1 L) then denatured EtOH (1 L). The solids were then transferred to a 5-L flask and stirred in denatured EtOH (2 L) for 2 h before refiltering. The solids were then washed with EtOH (0.5 L), a 1:1 mixture of toluene/EtOH (0.5 L), then toluene (0.5 L). The bright yellow powder was then dried to constant weight under vacuum to yield 2-amino-6-bromoquinazoline. MS m/z = 224, 226 [M+1]⁺; Calc' d for C₈H₇BrN₃: 223. 98, 225.98.

### Example 723

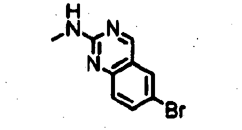

### Synthesis of 6-bromo-N-methylquinazolin-2-amine hydrochloride

### Step 1. Synthesis of N-(6-bromoquinazolin-2-yl)formamide

A mixture of 11 ml HOAc and 5.5 ml formic acid was heated to 55°C with a water-cooled reflux condenser for 2 h. The reaction was cooled to ambient temperature and 6-bromoquinazolin-2-amine (2.0 g, 8.9 mmol) was added to give an orange mixture. Over time the reaction became light yellow with a thick precipitate. After 4 days, the mixture was diluted with 30 ml diethyl ether and filtered. The solid was washed with diethyl ether, ethanol, and diethyl ether to give the title compound as a light yellow solid. MS (m/z): 251.9 (M+H⁺) ; Calc' d for C₉H₆BrN₃O- 252 . 00.

### Step 2. 6-bromo-N-methylquinazolin-2-amine hydrochloride

To a slurry of N-(6-bromoquinazolin-2-yl)formamide (1.9 g, 7.5 mmol) in DMF at 0 °C was added NaH (60% in mineral oil, 0.38 g, 8.3 mmol). After 1 h, MeI (0.93 ml, 15 mmol) was added and the reaction was allowed to warm to ambient temperature. After 2 h, an additional quantity of NaH (0.050 g, 1.3 mmol) was added. After 1 h, the homogeneous orange reaction was concentrated in vacuo to give an orange solid. This material was treated with 50 ml of 6N HCl and was heated to 100 °C with a water-cooled reflux condenser for 3 h. An additional 50 ml 6N HCl was added and heating was continued for 1 h. The reaction was cooled to ambient temperature, and a precipitate formed. After 12 h, the mixture was filtered and the precipitate was collected. The material was recrystallized from hot 6N HCl to give the desired product as a light yellow solid. MS (m/z): 237 . 9 (M+H⁺) ; Calc' d for C₉H₈BrN₃: 238.08.

Example 723 was also made by an alternative method: Diisopropylethylamine (1.42 ml, 8.13 mmol) was added to 6-bromo-2-iodoquinazoline (0.91g, 2.71 mmol) and methylamine (Aldrich) (13.6 ml of a 2.0 M solution in THF). The mixture was heated at 70°C in a sealed tube for 14 h. The solvent was removed by rotary evaporation and the title compound was isolated as a yellow solid by trituration from MeOH or by flash chromatography on silica, eluting with hexane/ethyl acetate.
MS m/z = 239 [M+H]⁺. Calc'd for C₉H₈BrN₃: 238.

### Example 724

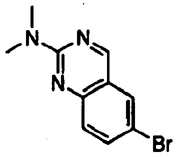

### Synthesis of 6-bromo-N,N-dimethylquinazolin-2-amine

Step 1 - 6-bromoquinazolin-2-amine (800 mg, 3.57 mmol) and NaH (150 mg of a 60% dispersion in mineral oil, 3.75 mmol)were taken up in DMF (15 ml) and heated at about 55°C under an atmosphere of N₂ for 1h. The mixture was allowed to cool to RT and then added to neat MeI (0.24 ml, 3.75 mmol) via cannula transfer. After 16h, the reaction was quenched with H₂O and extracted with CH₂Cl₂. The organic extracts were dried with Na₂SO₄, filtered (with the recovery of some 6-bromoquinazolin-2-amine) and concentrated. Purification by flash chromatography, eluting with hexane/EtOAc) afforded pure 6-bromo-N-methylquinazolin-2-amine (Example 714) as a yellow solid MS m/z = 239 [M+H]⁺. Calc'd for C₉H₈N₃: 238, and the title compound as a yellow solid, MS m/z = 253 [M+H]⁺; Calc'd for C₁₀H₁₀BrN₃: 252.

### Example 725

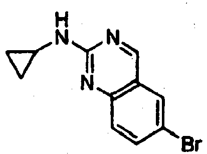

### Synthesis of 6-bromo-N-cyclopropylquinazolin-2-amine

6-bromo-N-cyclopropylquinazolin-2-amine was prepared according to the alternative method used for the preparation of Example 723, using cyclopropylamine in place of methylamine and isopropyl alcohol in place of THF. This method afforded the title compound as a pale yellow solid. MS *m*/*z* = 264 [M+H]⁺; Calc'd for C₁₁H₁₀BrN₃: 264.

### Example 726

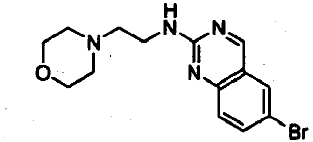

### Synthesis of 6-bromo-N-(2-morpholinoethyl)quinazolin-2-amine)

Method 1: Diisopropylethylamine (0.783 ml, 4.47 mmol) was added to 6-bromo-2-iodoquinazoline (1.0 g, 2.98 mmol) and 2-morpholinoethanamine (1.2 ml, 8.96 mmol) in IPA. The mixture was heated at 80°C in a sealed tube for 12 h. The solvent was removed by rotary evaporation and the title compound was isolated as a pale yellow solid by flash chromatography eluting with MeOH/CH₂Cl₂. MS m/z = 337 [M+H]⁺; Calc'd for C₁₄H₁₇BrN₄O: 337.

Example 726 was also made by an alternative method as described below:
Method 2: A three-necked 0.25L round-bottom flask equipped with magnetic stirrer, temperature probe, and a reflux condenser was charged with 2-amino-6-bromoquinazoline (11.2g, 50.0mmol) followed by 4-(2-aminoethyl)morpholine (0.10L, 0.76mol; available from Aldrich Chem Co.). p-Toluenesulfonic acid mono-hydrate (19.0g, 100mmol; available from Alfa-Aesar) was carefully added portionwise while stirring the reaction. The resulting heterogenous mixture was heated in an oil bath at about 165°C. The reaction mixture became a clear, dark-orange solution within 10-15min. The reaction progression was monitored by HPLC, and was found to be complete after about 5h. Excess 4-(2-aminoethyl) morpholine (about 77g, 0.59mol) was recovered by short-path distillation under reduced pressure (bath temp. about 160°C). The thick oily residue that solidified on standing was partitioned between DCM (about 0.30L), water (about 0.23L), and 3.3M aq. HC1. (about 70ml). Three layers formed: aqueous (top), oily organic (middle), and organic (bottom). Both organic layers (middle and bottom) were collected. The aqueous layer was extracted with DCM (about 0.20L) and set aside. Combined organic extracts, diluted with DCM (about 0.35L), were washed with 2.5M NaOH (about 0.10L) until two clear layers resulted. The organic layer was separated, dried over anh. Na₂SO₄, and the solvent was removed under reduced pressure to give the title compound crude as an orange semi-solid. Chromatographic purification afforded the title compound as a yellow amorphous solid.

The aqueous layer was basified using aq. NaOH and extracted using DCM (3x0.15L). The combined extracts were dried over Na₂SO₄ and concentrated under reduced pressure. Chromatographic purification of the crude afforded additional amounts of the title compound as a yellow solid.

### Example 727

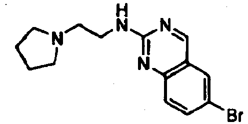

6-bromo-N-(2-(pyrrolidin-1-yl)ethyl)quinazolin-2-amine was synthesized in accordance with the method of Example 726, method 1 using 2-(pyrrolidin-1-yl)ethanamine in place of 2-morpholinoethanamine. The method afforded the title compound as a yellow solid. MS *m*/*z* = 321 [M+H]⁺. Calc'd for C₁₄H₁₇BrN: 321.

### Example 728

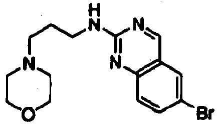

6-bromo-N-(3-morpholinopropyl)quinazolin-2-amine was synthesized in accordance with the method of Example 726, method 1, affording the title compound as a pale yellow solid. MS *m*/*z* = 352 [M+H]⁺. Calc'd for C₁₅H₁₉BrN₄O: 351.

### Example 729

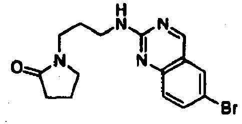

1-(3-(6-bromoquinazolin-2-ylamino)propyl)pyrrolidin-2-one was synthesized in accordance with the method of Example 726, method 1, affording the title compound as a pale yellow solid. MS*m*/*z* = 349 [M+H]⁺. Calc'd for C₁₅H₁₇BrN₄O: 349.

### Example 730

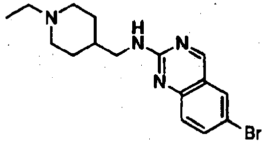

6-bromo-N-((1-ethylpiperidin-4-yl)methyl)quinazolin-2-amine was synthesized in accordance with the method of Example 726, method 1, affording the title compound as a pale yellow solid. MS *m*/*z* = 349 [M+H]⁺. Calc' d for C₁₆H₂₁BrN₄: 349.

### Example 731

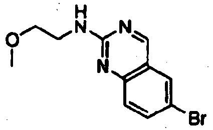

6-bromo-N-(2-methoxyethyl)quinazolin-2-amine was synthesized in accordance with the method of Example 726, method 1, affording the title compound as a pale yellow solid. MS *m*/*z* = 282 [M+H]⁺. Calc'd for C₁₁H₁₂BrN₃O: 282.

### Example 732

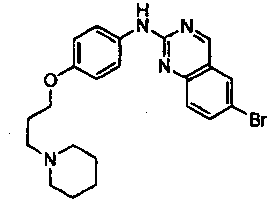

### Synthesis of (6-bromo-N-(4-(3-(piperidin-1-yl)propoxy)phenyl)quinazolin-2-amine)

Trifluoroacetic acid (0.089 ml) was added to 6-bromo-2-iodoquinazoline (155 mg, 0.462 mmol) and 4-(3-(piperidin-1-yl)propoxy)benzenamine (112 mg, 0.508 mmol; prepared according to methods E, F and G described in PCT patent Application No. WO 03/018021) in IPA. The mixture was heated at 80°C in a sealed tube for 14 h. The solvent was removed by rotary evaporation and the title compound was isolated as a yellow solid by flash chromatography eluting with MeOH/CH₂Cl₂. MS *m*/*z* = 441 [M+H]⁺, 442 [M+H]⁺; Calc'd for C₂₂H₂₅BrN₄O: 441.

### Example 733

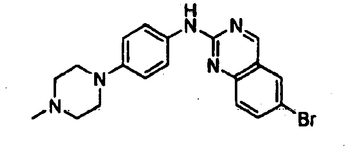

6-bromo-N-(4-(9-methylpiperazin-1-yl)phenyl)quinazolin-2-amine was prepared by a method similar to the procedure described above in Example 732, using 4-(4-methylpiperazin-1-yl)benzenamine in place of 4-(3-(piperidin-1-yl)propoxy)benzenamine, and affording the title compound as a yellow solid. MS *m*/*z* = 398 [M+H]⁺; Calc' d for C₁₉H₂₀BrN₅: 398.

### Example 734

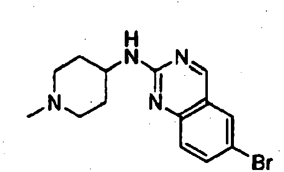

### Synthesis of 6-bromo-N-(1-methylpiperidin-4-yl)quinazolin-2-amine

To a solution of 1-methylpiperidin-4-amine (170 mg, 1.5 mmol) in THF (8 ml) was added NaH (60 mg of a 60% dispersion in mineral oil, 1.5 mmol). After 10 min at RT, 6-bromo-2-iodoquinazoline was added at once. After 2 h, the reaction mixture was quenched with 1N HCl. 0.4 mL Et₃N was added and the mixture was extracted with CH₂Cl₂. The organic extracts were washed twice with H₂O and dried over Na₂SO₄ then concentrated. The title compound was isolated as a pale yellow solid by flash chromatography eluting with MeOH/CH₂Cl₂. MS *m*/*z* = 321 [M]⁺, 322 [M+H]⁺; Calc'd for C₁₄H₁₇BrN₄: 321.

### Example 735

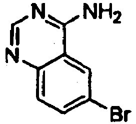

### Synthesis of 6-bromoquinazolin-4-amine

### Step 1: (E)-N'-(4-bromo-2-cyanophenyl)-N,N-dimethylformamidine

After 20 minutes of stirring p-toluenesulfonyl chloride (29 g, 152 mmol) in DMF (200 ml), 2-amino-5-bromobenzonitrile (20 g, 101 mmol) was added in portions. The mixture was stirred for 30 minutes at RT. The mixture was concentrated and the crude (E)-N'-(4-bromo-2-cyanophenyl)-N,N-dimethylformamidine was carried on to the next step without purification.

### Step 2: 6-bromoquinazolin-4-amine

To the crude (E)-N'-(4-bromo-2-cyanophenyl)-N,N-dimethylformamidine (25.4 g, 101 mmol) was added EtOH (150 mL) and NH₄OH (20 ml). The mixture was stirred for 1 hour at reflux. The resulting solid was filtered and washed with EtOH, and Et₂O and dried to yield 6-bromoquinazolin-4-amine. MS m/z = 224, 226 [M, M+2]⁺. Calc'd for C₈H₆BrN₃: 224.06.

### Example 736

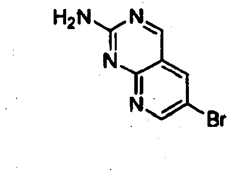

### Synthesis of 6-Bromopyrido[2,3-d]pyrimidin-2-amine

### Step 1: Pyrido[2,3-d]pyrimidin-2-amine

To 2-fluoronicotinaldehyde (1.00 g, 8.0 mmol), guanidine carbonate (2.02 g, 11.2 mmol), and K₂CO₃ (1.55 g, 11.2 mmol) was added CH₃CN (31 mL). The mixture was stirred for 21 hours at reflux and concentrated. The resulting solid was washed with water and Et₂O, and dried to yield pyrido[2,3-d]pyrimidin-2-amine as a light brown solid. MS *m*/*z* = 147 [M+H]⁺. Calc' d for C₇H₆N₄: 146.15.

### Step 2: 6-bromopyrido[2,3-d]pyrimidin-2-amine

To pyrido[2,3-d]pyrimidin-2-amine (560 mg, 3.83 mmol) in acetic acid (10 mL) was added bromine (0.39 mL, 7.66 mmol). The reaction was stirred for 18 hours at 110 °C, cooled, quenched with saturated NaHCO₃, and extracted into CH₂Cl₂. The organic layer was dried over anhydrous Na₂SO₄, filtered, concentrated, and purified by passing through a plug of silica gel (10% MeOH/CH₂Cl₂) to yield 6-bromopyrido[2,3-d]pyrimidin-2-amine as an orangish solid. MS *m*/*z* = 225 [M+H]⁺. Calc'd for C₇H₅BrN₄: 225.05.

### Example 737

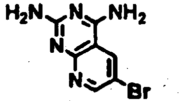

### Synthesis of 6-bromopyrido[2,3-d]pyrimidine-2,4-diamine

The title compound was synthesized by the method described in J. Med. Chem., 40, 470, 1997.

### Example 738

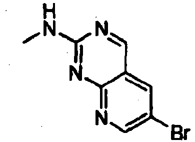

### Synthesis of 6-bromo-N-methylpyrido[2,3-d]pyrimidin-2-amine

### Step 1: 5-bromo-2-fluoronicotinaldehyde

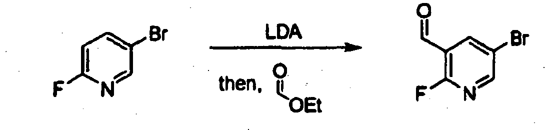

To a solution of diisopropylamine (9.6 ml, 68.6 mmol, 1.1 equiv) in THF (90 ml) at 0°C, was added n-BuLi (27.9 ml, 2.5M in hexanes). After 20 min, the solution was cooled to -78°C and diluted with THF (90 ml). A solution of 2-fluro-5-bromo-pyridine (11.1 g, 63.2 mmol, 1.0 equiv) in THF (90 ml) was added via addition funnel over ca. 15 min. After 1.5 h, ethyl formate (10.3 ml, 127mmol, 2.0 equiv) was added dropwise and the solution was stirred for 1 h before quenching with a 1:1 mixture of saturated aqueous ammonium chloride and acetic acid (18ml). The resulting slurry was warmed to 25°C and Na₂SO₄ (ca. 20g) added. After filtering and concentration *in vacuo,* the resulting solid was recrystallized from CH₂Cl₂ to afford 5-bromo-2-fluoronicotinaldehyde. MS (M+H⁺) 204; Calc'd for C₆H₃BrFNO: 204.0.

### Step 2: 6-bromo-N-methylpyrido[2,3-d]pyrimidin-2-amine

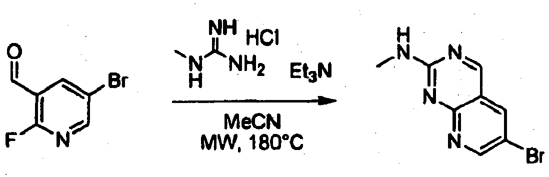

To a mixture of 5-bromo-2-fluoronicotinaldehyde (300 mg, 1.47 mmol, 1.0 equiv) and 1-methylguanidine hydrochloride (193 mg, 1.76 mmol, 1.2 equiv) in MeCN (18 mL) was added triethylamine (0.61 mL, 4.41 mmol, 3.0 equiv). The mixture was exposed to microwave radiation for 10 min at 180°C. After concentrating *in vacuo,* the resulting residue was taken up in CH₂Cl₂ (ca. 25 mL) and washed with water and brine. After drying the organic layer with Na₂SO₄, the solvent was removed *in vacuo* and residue purified by silica gel chromatography (1:3 hexanes:EtOAc to 100% EtOAc) to afford 6-bromo-N-methylpyrido[2,3-d]pyrimidin-2-amine. MS (M+H⁺) 239; Calculated for C₈H₇BrN₄: 239.1.

### Example 739

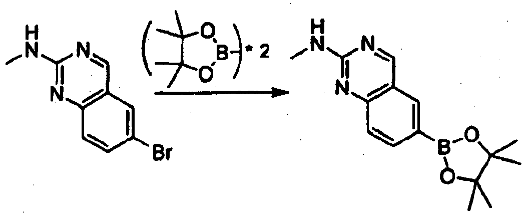

### Synthesis of N-methyl-6-(4,4,5,5-tetramethyl-1,3,2-dioxaborolan-2-yl)quinazolin-2-amine

A resealable tube was charged with Pd₂(dba)₃ (0.014 g, 0.016 mmol), (o-biphenyl)Pcy₂ (0.017 g, 0.047 mmol) and 4 ml of dioxane. The tube was flushed with argon and sealed. The reaction mixture was stirred at room temperature for 15 minutes. To this mixture was then added 6-bromo-N-methylnaphthalen-2-amine (0.18 g, 0.8 mmol), bispinacolatodiboron (0.24 g, 1.0 mmol) and potassium acetate (0.125 g, 1.3 mmol). The tube was again flushed with argon and the sealed reaction mixture was stirred at 80 °C for 18 hours. The brown mixture was cooled and filtered through a pad of celite. Purification was accomplished using silica chromatography, EtOAc: hexanes, 5-50% to afford N-methyl-6-(4,4,5,5-tetramethyl-1,3,2-dioxaborolan-2-yl)quinazolin-2-amine. MS m/z = 286.2; Calc'd for C₁₅H₂₀BN₃O₂: 285.

### Example 740

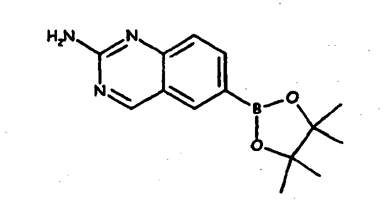

### Synthesis of 2-Amino-6-(4,4,5,5-tetramethyl-1,3,2-dioxaborolan-2-yl)quinazoline

Method 1: To 2-amino-6-bromoquinazoline (37.85 g, 0.169 mol, 1.0 equiv, Example 160) was added bis(pinacolato)diboron (45.1 g, 0.178 mol, 1.05 equiv), KOAc (33.2 g, 0.338 mol, 2.0 equiv), PdCl₂(dppf)·CH₂Cl₂ (1.38 g, 1.7 mmol, 0.01 equiv), and 1,4-dioxane (300 ml). The mixture was heated for 18 h at 85°C, cooled to RT, and treated with K₂CO₃ (23.4 g, 0.169 mol, 1.0 equiv). The mixture diluted with EtOAc (500 ml), then filtered through a plug of Celite to remove solids. The solids were washed colorless with EtOAc (500 mL), and concentrated to dryness. The residue was dissolved in CH₂Cl₂ (200 ml), diluted with hexane, and concentrated to remove CH₂Cl₂. The resulting reddish tan solid was collected by vacuum filtration and washed with hexane. The solids were dried to yield 2-amino-6-(4,4,5,5-tetramethyl-1,3,2-dioxaborolan-2-yl)quinazoline. MS *m*/*z* = 272 [M+H]⁺. Calc'd for C₁₄H₁₉BN₃O₂: 272.16.

Method 2: To 2-fluoro-5-(4,4,5,5-tetramethyl-1,3,2-dioxaborolan-2-yl)benzaldehyde (3.0 g, 12 mmol, 1.0 equiv.; See Example 741) was added guanidine carbonate (2.8 g, 15.6 mol, 1.3 equiv), K₂CO₃ (2.15 g, 15.6 mmol, 1.3 equiv), and acetonitrile (30 mL). The stirred mixture was heated to 175 °C for 20 min in a microwave reactor at 500 W power. Upon cooling, the mixture was stirred with acetone (150 ml), then filtered to remove solids. The orange filtrate was concentrated, and the residue was purified by silica gel flash chromatography to yield 2-amino-6-(4,4,5,5-tetramethyl-1,3,2-dioxaborolan-2-yl)quinazoline. MS *m*/*z* = 272 [M+H]⁺; Calc'd for C₁₄H₁₉BN₃O₂: 272.16.

### Example 741

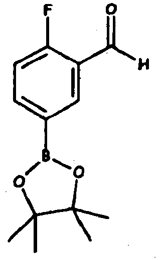

### Synthesis of 2-Fluoro-5-(4,4,5,5-tetramethyl-1,3,2-dioxaborolan-2-yl)benzaldehyde

To 5-bromo-2-fluorobenzaldehyde (26.8 g, 0.129 mol, 1.0 equiv) was added bis(pinacolato)diboron (34.5 g, 0.136 mol, 1.05 equiv), KOAc (38 g, 0.387 mol, 3.0 equiv), PdCl₂(dppf)·CH₂Cl₂ (3.17 g, 3.9 mmol, 0.03 equiv), and DMSO (200 ml). The mixture was heated for 5 h at 80°C, cooled to RT, and quenched by the addition of water (300 ml). The mixture was extracted with EtOAc (2x200 ml), and the combined extracts were washed with water (100 ml) and brine (100 ml). The organic layer was dried over anhydrous Na₂SO₄. The mixture was filtered to remove solids, concentrated to a black oil, then purified by silica gel flash chromatography (10% EtOAc in hexane). The desired fractions were combined and concentrated to yield a waxy solid, 2-fluoro-5-(4,4,5,5-tetramethyl-1,3,2-dioxaborolan-2-yl)benzaldehyde. MS *m*/*z* = 251 [M+H]⁺; Calc' d for C₁₃H₁₆BFO₃: 250.12.

### Example 742

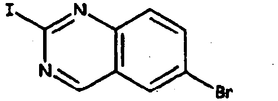

### Synthesis of 6-Bromo-2-iodoquinazoline

To a heated mixture (70°C) of 2-amino-6-bromoquinazoline (30.0 g, 0.134 mol, 1.0 equiv), diiodomethane (179.5 g, 0.67 mol, 5.0 equiv), Cu(I)I (26.0 g, 0.134 mol, 1.0 equiv), and THF (500 ml) was added isoamyl nitrite (49.0 g, 0.402 mol, 3.0 equiv) via addition funnel over 20 min. The mixture was heated at reflux (90°C bath temp) for 1.5 h, then cooled to RT. The mixture was diluted with EtOAc (500 ml), then filtered through a plug of Celite to remove solids. The solids were washed with EtOAc (500 mL), and concentrated to dryness. The residue was triturated with acetone (100 mL), diluted with hexane (300 ml), and the tan solid was collected by vacuum filtration and washed with hexane. The solids were dried to yield 6-Bromo-2-iodoquinazoline. MS *m*/*z* = 335, 337 [M+H]⁺; Calc'd for C₈H₅BrIN₂: 334.86, 336.86.

### Example 743

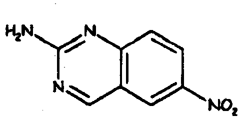

### Synthesis of 2-Amino-6-nitroquinazoline

A stirred mixture of guanidine carbonate (223 g, 1.25 mol, 1.4 equiv), 2-fluoro-5-nitrobenzaldehyde (151 g, 0.89 mol, 1.0 equiv), K₂CO₃ (171 g, 1.25 mol, 1.4 equiv), and acetonitrile (1.5 L) was heated to reflux with a heating mantle. The mixture was maintained at reflux for 8 h, at which point the reactor was set to distill off solvent. In this manner, the mixture was concentrated to about one-half volume (700 ml recovered). The mantle was removed and the reaction was allowed to cool to below 70°C. At this point, the reaction was quenched by the addition of 6N HCl (850 ml), which brought the pH of the mixture below pH 1. The mixture was then filtered to remove solids, which were then washed with 1N HCl (100 ml) and water (200 ml). The filtrate partitioned into a dark brown organic layer (about 200 ml) and a bright orange aqueous layer (about 2 L). The aqueous layer was separated, extracted with EtOAc (2x150 ml), and filtered through paper. The aqueous filtrate was neutralized by the portionwise addition of 6N NaOH (150 ml), which caused the precipitation of the product and brought the pH of the mixture to pH 9-10. The product was isolated by vacuum filtration, and washed with water (100 ml) and Et₂O (2x100 ml). The mustard yellow powder was then dried to constant weight under vacuum to yield 2-amino-6-nitroquinazoline. MS *m*/*z* = 191 (M+H]⁺; Calc'd for C₈H₆N₄O₂: 190.05.

### Example 744

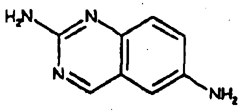

### Synthesis of 2,6-Diaminoquinazoline

A mixture of 2-amino-6-nitroquinazoline (69.5 g, 0.37 mol, 1.0 equiv), 10% Pd/C (50 wt% H₂O, 19.44 g, 0.025 equiv Pd) and 1:1 EtOAc/MeOH (800 ml) was stirred under a balloon of hydrogen gas for 7 h at ambient temperature. The balloon was refilled as necessary to maintain sufficient H₂ in the reaction. After 7 h, the greenish-black mixture was filtered through a large glass funnel containing a ½" plug of Celite. The solids were washed with portions of warm MeOH (12x500 ml portions) until the filtrate was colorless. The yellow-green filtrate was filtered and concentrated by rotary evaporation. During concentration, a green-brown precipitate was formed, and the solid was further precipitated by the addition of CH₂Cl₂ (300 ml). The title compound was isolated by vacuum filtration, and washed with CH₂Cl₂ (2x100 ml). The green-brown powder was then dried to constant weight under vacuum to yield 2,6-diaminoquinazoline. MS *m*/*z* = 161 [M+H]⁺; Calc'd for C₈H₈N₄: 160.07.

Various different B-CD linked ring intermediates (R³ substituted CD ring systems), which are contemplated herein, may be made by various methods, such as coupling ring B to ring CD by Suzuki-type coupling methods, as represented by Examples 735-748. Suitable halide and boronate intermediates to affect such a coupling are described herein.

### Example 745

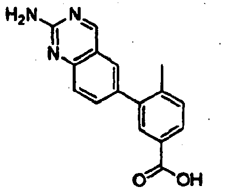

### Synthesis of 3-(2-amino-6-quinazolinyl)-4-methyl-benzoic acid

6-bromoquinazolin-2-amine (418 mg, 1.87 mmol), 4-methyl-3-(4,4,5,5-tetramethyl-1,3,2-dioxaborolan-2-yl)benzoic acid (444 mg, 1.70 mmol), tetrakis(triphenylphosphine)palladium (98.3 mg, 0.085 mmol), 2M Na₂CO₃ (2.55 ml), CH₃CN (6.23 ml), and water (6.23 ml) were combined in a screw-cap sealed tube and heated to 80°C overnight. The hot solution was filtered through filter paper, and the filter paper was rinsed with hot CH₃CN/water (1:1). The filtrate was concentrated *in vacuo* to one half volume. The resulting liquid was extracted once with ethyl acetate then acidified to pH 3 with 1N HCl. The precipitate was filtered, and the solid was rinsed successively with water, ethanol, and diethyl ether to provide 3-(2-amino-6-quinazolinyl)-4-methyl-benzoic acid as a yellow solid. MS *m*/*z* = 280 [M+H]⁺; Calc'd for C₁₆H₁₃N₃O₂: 279.

### Example 746

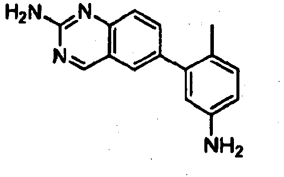

### Synthesis of 6-(5-Amino-2-methylphenyl)quinazolin-2-amine

A resealable tube was charged with 6-(4,4,5,5-tetramethyl-1,3,2-dioxaborolan-2-yl)quinazolin-2-amine (0.200 g, 0.738 mmol), 3-iodo-4-methylaniline (0.181 g, 0.775 mmol), potassium carbonate (0.163 g, 1.18 mmol), N,N-dimethylformamide (7 ml), and water (1.8 ml). Dichloro[1,1'-bis(diphenylphosphino)ferrocene]palladium (II) dichloromethane adduct (0.060 g, 0.074 mmol) was added and the system was purged with argon. The tube was sealed and the mixture stirred at room temperature for 16 h. The reaction mixture was partitioned between ethyl acetate and water. The aqueous phase was separated and extracted with ethyl acetate. The combined organic phases were washed with brine, dried over anhydrous sodium sulfate, filtered, and concentrated to afford a brown oil. This material was purified via column chromatography on silica gel (gradient elution with 0-50% (90:10:1 dichloromethane/methanol/ammonium hydroxide)-dichloromethane) to afford 6-(5-amino-2-methylphenyl)quinazolin-2-amine as a pale brown solid. MS (M+H⁺) 251.1; Calculated for C₁₅H₁₄N₄: 250.

### Example 747

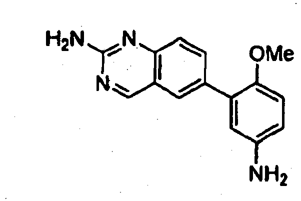

### Synthesis of 6-(5-Amino-2-methoxyphenyl)quinazolin-2-amine

A resealable tube was charged with 6-(4,4,5,5-tetramethyl-1,3,2-dioxaborolan-2-yl)quinazolin-2-amine (0.250 g, 0.922 mmol), 3-chloro-4-methoxyaniline (0.108 g, 0.615 mmol), potassium phosphate monohydrate (0.261 g, 1.23 mmol), palladium acetate (0.0055 g, 0.025 mmol), and 2-dicyclohexylphosphino-2',6'-dimethoxy-1,1'-biphenyl (0.020 g, 0.049 mmol). Toluene (2 ml) was added and the system was purged with argon. The tube was sealed and the mixture stirred at 100°C for 6 h. The reaction mixture was cooled to room temperature and filtered through a pad of Celite along with ethyl acetate. The filtrate was concentrated to afford a brown oil. This material was purified via preparative thin layer chromatography (eluting with 95:5:0.5 dichloromethane/ methanol/ammonium hydroxide)-dichloromethane) to afford 6-(5-amino-2-methoxyphenyl)quinazolin-2-amine as a light brown solid. MS (M+H⁺) 267.1; Calculated for C₁₅H₁₄N₄O: 266.

### Example 748

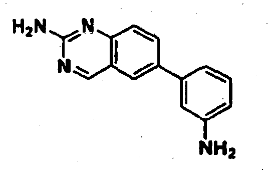

### Synthesis of 6-(3-Aminophenyl)quinazolin-2-amine

6-(3-Aminophenyl)quinazolin-2-amine was synthesized from 3-bromoaniline by a method similar to that described in Example 747. 6-(3-aminophenyl)quinazolin-2-amine was obtained as a yellow-brown solid. MS (M+H⁺) 237.1; Calculated for C₁₄H₁₂N₄: 236.

### Example 749

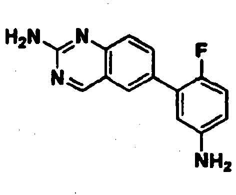

### Synthesis of 6-(5-Amino-2-fluorophenyl)quinazolin-2-amine

6-(5-Amino-2-fluorophenyl)quinazolin-2-amine was synthesized from 3-bromo-4-fluoroaniline by a method similar to that described in Example 747. 6-(5-Amino-2-fluorophenyl)quinazolin-2-amine was obtained as a light brown solid. MS (M+H⁺) 255.0; Calculated for C₁₄H₁₁FN₄: 254.

### Example 750

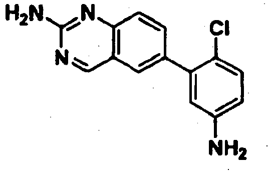

### Synthesis of 6-(5-Amino-2-chlorophenyl)quinazolin-2-amine

### Step 1: 3-bromo-4-chloroaniline

Raney nickel (6 g) was added to a solution of 3-bromo-4-chloronitrobenzene (2.00 g, 8.46 mmol) in ethanol (50 ml). The mixture stirred at room temperature in a capped flask for 4 days. The reaction mixture was filtered through a pad of Celite and the solution was concentrated to afford a yellow-brown oil. This oil was purified via column chromatography on silica gel (gradient elution with 0-50% ethyl acetate-hexane) to afford 3-bromo-4-chloroaniline as an off-white solid. MS (M+H⁺) 207.9; Calculated for C₆H₅BrClN: 206.

### Step 2: 6-(5-Amino-2-chlorophenyl)quinazolin-2-amine

6-(5-Amino-2-chlorophenyl)quinazolin-2-amine was synthesized from 3-bromo-4-chloroaniline by a method similar to that described in Example 738. 6-(5-Amino-2-chlorophenyl)quinazolin-2-amine was obtained as a brown solid. MS (M+H⁺) 271.0; Calculated for C₁₄H₁₁ClN₄: 270.

### Example 751

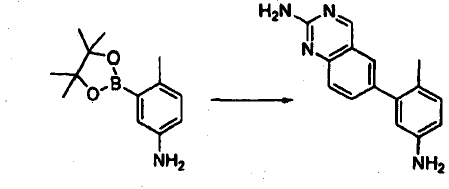

### Synthesis of 6-(5-amino-2-methylphenyl)quinazolin-2-amine

The title compound was also made by a route other than that described in Example 746.

### Step 1: 4-methyl-3-(4,4,5,5-tetramethyl-1,3,2-dioxaborolan-2-yl)benzenamine

3-iodo-4-methylbenzenamine (5.0 g, 21.45 mmol), 4,4,5,5-tetramethyl-2-(4,4,5,5-tetramethyl-1,3,2-dioxaborolan-2-yl)-1,3,2-dioxaborolane (6.0 g, 23.6 mmol), [1,1'-bis(diphenylphosphino)ferrocene]palladium (II) chloride [Pd(dppf)Cl₂] (0.785 g, 1.07 mmol) and KOAc (7.4 g, 75.1 mmol)were taken up in DMSO (60 ml) under at atmosphere of N₂. The mixture was heated for 14 h in a sealed tube at 80 °C. DMSO was removed by rotary evaporation. The crude residue was taken up in EtOAc and filtered to remove the insoluble precipitates. The organics were then washed three times with H₂O and dried over Na₂SO₄. The title compound was isolated as a sticky brown solid by flash chromatography on silica, eluting with a gradient, 0 to 10% MeOH/CH₂Cl₂.

### Step 2: 6-(5-amino-2-methylphenyl)quinazolin-2-amine

4-Methyl-3-(4,4,5,5-tetramethyl-1,3,2-dioxaborolan-2-yl)benzenamine (5.0g, 21.45 mmol), 6-bromoquinazolin-2-amine (4.0g, 17.87 mmol), and Pd(PPh₃)₄ (2.48g, 2.14 mmol) were taken up in toluene (175 ml), EtOH (35 mL) and 2M Na₂CO₃ (30 ml) under N₂. The mixture was heated at 80°C for 6 h. The solvents were removed by rotary evaporation and the crude residue was taken up in 1:1 CH₂Cl₂/H₂O. The aqueous layer was extracted 10 times with CH₂Cl₂ and the combined organics were dried over Na₂SO₄ then concentrated. The title compound was isolated as a pale yellow solid by flash chromatography eluting with a gradient 0 to 10% MeOH/CH₂Cl₂. MS *m*/*z* = 250 [M+H]⁺. Calc'd for C₁₅H₁₄N₄: 251.

### Example 752

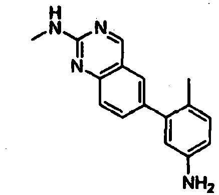

6-(5-amino-2-methylphenyl)-N-methylquinazolin-2-amine) was prepared by a method similar to that described in Example 751 above, using 6-bromo-N-methylquinazolin-2-amine in place of 6-bromoquinazolin-2-amine, affording the title compound as a tan solid. MS *m*/*z* = 265 [M+H]⁺; Calc'd for C₁₆H₁₆N₄ : 264.

### Example 753

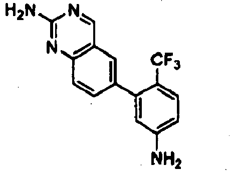

### Synthesis of 6-(5-amino-2-(trifluoromethyl)phenyl)quinazolin-2-amine

### Step 1: 3-(4,4,5,5-tetramethyl-1,3,2-dioxaborolan-2-yl)-4-(trifluoromethyl)benzenamine

Tricyclohexylphosphine (0.2 g, 0.74 mmol) and Pd₂(dba)₃ (0.28 g, 0.31 mmol) were taken up in dioxane (55 ml) under an atmosphere of N₂ and allowed to stir at RT for 30 min. 4,4,5,5-tetramethyl-2-(4,4,5,5-tetramethyl-1,3,2-dioxaborolan-2-yl)-1,3,2-dioxaborolane (3.38 g, 13.3 mmol), KOAc (1.61 g, 16.37 mmol)and 3-chloro-4-(trifluoromethyl)benzenamine (2.0 g, 10.23 mmol) were successively added, followed by dioxane (5 ml). The mixture was then heated at 80°C for 2.5 days. The mixture was then filtered to remove the insoluble precipitates, then concentrated. The residue was taken up in CH₂Cl₂ and washed twice with H₂O, dried over Na₂SO₄ and concentrated. 3-(4,4,5,5-Tetramethyl-1,3,2-dioxaborolan-2-yl)-4-(trifluoromethyl)benzenamine was isolated as a sticky pale yellow solid by flash chromatography on silica eluting with a gradient, 0 to 3% MeOH/CH₂Cl₂. MS *m*/*z* = 288 [M+H]⁺; Calc'd for C₁₃H₁₇BF₃NO₂: 287.

Step 2: 6-(5-amino-2-methylphenyl)-N-methylquinazolin-2-amine) was prepared by a method similar to that described in Example 752, using 3-(4,4,5,5-tetramethyl-1,3,2-dioxaborolan-2-yl)-4-(trifluoromethyl)benzenamine in place of 4-methyl-3-(4,4,5,5-tetramethyl-1,3,2-dioxaborolan-2-yl)benzenamine, affording the title compound as a pale yellow solid. MS *m*/*z* = 305 [M+H]⁺; Calc' d for C₁₅H₁₁F₃N₄: 304.

### Example 754

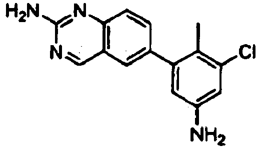

### Synthesis of 6-(5-amino-3-chloro-2-methylphenyl)quinazolin-2-amine

### Step 1: 6-(3-chloro-2-methyl-5-nitrophenyl)quinazolin-2-amine

Sodium carbonate (2M in water, 19.3 ml, 38.7 mmol) was added to a solution of 6-(4,4,5,5-tetramethyl-1,3,2-dioxaborolan-2-yl)quinazolin-2-amine (3.5 g, 12.9 mmol), 1,3-dichloro-2-methyl-5-nitrobenzene (5.3 g, 25.8 mmol), and tetrakis(triphenylphosphine)palladium (0) (0.745 g, 0.64 mmol) in toluene (200 ml) and ethanol (40 ml). The mixture was mixture was heated overnight at 80°C under a nitrogen atmosphere. The reaction mixture was concentrated and partitioned between ethyl acetate and water. The aqueous phase was separated and extracted with ethyl acetate. The combined organic phases were washed with brine, dried over anhydrous sodium sulfate, filtered, and concentrated to afford a dark red oil. This material was purified via column chromatography on silica gel (gradient elution with 5-100% (90:10:1 dichloromethane/methanol/ammonium hydroxide)-dichloromethane then 7:7:7:1:0.1 (methyl-tert-butylether/hexane/dichloromethane/methanol/ammonium hydroxide)-hexane) to afford 6-(3-chloro-2-methyl-5-nitrophenyl)quinazolin-2-amine as a tan solid. MS (M+H⁺) 315.1; Calculated for C₁₅H₁₁ClN₄O₂: 314.

### Step 2: 6-(5-amino-3-chloro-2-methylphenyl)quinazolin-2-amine

Stannous chloride (0.983 mg, 5.2 mmol) was added to a solution of 6-(3-chloro-2-methyl-5-nitrophenyl)quinazolin-2-amine (0.544 g, 1.7 mmol) in ethanol (50 ml). The mixture was heated at 75°C for 14 hours under a nitrogen atmosphere and then cooled to room temperature. Potassium carbonate (1M in water, 10 ml) was added and the mixture stirred vigorously for several hours. The mixture was filtered through a pad of Celite along with methanol, and the filtrate was concentrated under reduced pressure. This material was partitioned between ethyl acetate and saturated aqueous sodium bicarbonate solution. The aqueous phase was separated and extracted with ethyl acetate. The combined organic phases were washed with brine, dried over anhydrous sodium sulfate, filtered, and concentrated to afford 6-(5-amino-3-chloro-2-methylphenyl)quinazolin-2-amine as a yellow solid. MS (M+H⁺) 285.0; Calculated for C₁₅H₁₃ClN₄: 284.

### Example 755 (Reference Example)

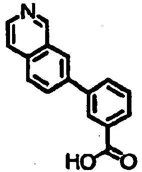

### Synthesis of 3-(isoquinolin-7-yl)benzoic acid

### Step 1. Synthesis of isoquinolin-7-yl trifluoromethanesulfonate

To a mixture of 7-hydroxyisoquinoline (1.2 g, 7.9 mmol) and DIPEA (1.4 ml, 7.9 mmol) in 15 ml MeOH at 0°C was added N-phenyl(bistrifluoromethanesulfonimide) (3.7 g, 10 mmol). The reaction was allowed to warm to ambient temperature. After 18 h, the volatile organics were removed in vacuo to give a brown oil. Purification by silica gel chromatography (EtOAc/hexanes) provided the title compound as a light yellow oil. MS (ES⁺): 278.0 (M+H)⁺. Calc'd for C₁₀H₆F₃NO₃S: 277.22.

### Step 2. 3-(isoquinolin-7-yl)benzoic acid

A mixture of isoquinolin-7-yl trifluoromethanesulfonate (0.17 g, 0.61 mmol), 3-boronobenzoic acid (0.10 g, 0.61 mmol), tetrakis(triphenylphosphine) Palladium (0) (0.035 g, 0.030 mmol), sodium carbonate (2.0 M solution in water, 0.61 ml, 1.2 mmol), 2.4 ml water and 3 ml acetonitrile was heated 90 °C under nitrogen with a water-cooled reflux condensor for 12 h. The reaction was cooled to ambient temperature and was filtered. The filtrate was concentrated under reduced pressure to ½ the original volume, and added to dichloromethane, water, and 1N NaOH. The aqueous layer was washed once with dichloromethane, and then was adjusted to pH 7 with 1N HCl. A precipitate formed which was isolated by filtration to give the desired product as a gray solid. MS (ES⁺) : 250.1 (M+H)⁺; Calc'd for C₁₆H₁₁NO₂: 249.26.

### Example 756

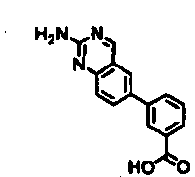

### Synthesis of 3-(2-aminoquinazolin-6-yl)benzoic acid

The title compound was synthesized by a procedure similar to that described in Example 755, affording a yellow solid. MS (ES⁺) : 266.0 (M+H)⁺. Calc'd for C₁₅H₁₁N₃O₂: 265.27.

### Example 757

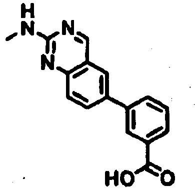

### Synthesis of 3-(2-(methylamino)quinazolin-6-yl)benzoic acid

The title compound was synthesized by a procedure similar to that described in Example 755 affording a yellow solid. MS (ES⁺): 280.1 (M+H)⁺. Calc' d for C₁₆H₁₃N₃O₂: 279.29.

### Example 758

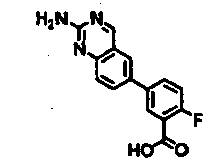

### Synthesis of 5-(2-aminoquinazolin-6-yl)-2-fluorobenzoic acid

### Step 1. Synthesis of 2-fluoro-5-(4,4,5,5-tetramethyl-1,3,2-dioxaborolan-2-yl)benzoic acid

A mixture of 2-fluoro-5-iodobenzoic acid (5.0 g, 19 mmol), 4,4,5,5-tetramethyl-2-(4,4,5,5-tetramethyl-1,3,2-dioxaborolan-2-yl)-1,3,2-dioxaborolane (5.3 g, 21 mmol), dichloro[1,1'-bis(diphenylphosphino)ferrocene]palladium (II) dichloromethane adduct (0.41 g, 0.56 mmol), KOAc (5.5 g, 56 mmol) in 60 ml DMSO was heated to 80°C under nitrogen with a water-cooled reflux condensor. After 20 h, the solvent was removed under reduced pressure, and the material was partitioned between 2N NaOH and dichloromethane. The aqueous layer was washed three times with dichloromethane, and twice with EtOAc. The aqueous layer was acidified to pH 2 at 0°C with 6N HCl, resulting in the formation of a fine, white precipitate. The mixture was extracted three times with diethyl ether. The combined organic layers were dried with Na₂SO₄, filtered, and concentrated to give the title compound as a white solid. MS (ES⁺): 267.1 (M+H)⁺. Calc'd for C₁₃H₁₆BFO₄: 266.07.

### Step 2. 5-(2-aminoquinazolin-6-yl)-2-fluorobenzoic acid

The title compound was synthesized by a procedure similar to that described in Example 745, affording a yellow solid. MS (ES⁺) : 284.1 (M+H)⁺. Calc'd for C₁₅H₁₀FN₃O₂: 283.26.

### Example 759

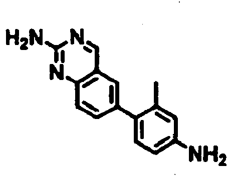

### Synthesis of 6-(4-amino-2-methylphenyl)quinazolin-2-amine

### Step 1. Synthesis of 3-methyl-4-(4,4,5,5-tetramethyl-1,3,2-dioxaborolan-2-yl)benzenamine

A mixture of 4-bromo-3-methylbenzenamine (4.0 g, 22 mmol), 4,4,5,5-tetramethyl-2-(4,4,5,5-tetramethyl-1,3,2-dioxaborolan-2-yl)-1,3,2-dioxaborolane (6.0 g, 24 mmol), dichloro[1,1'-bis(diphenylphosphino)ferrocene]palladium (II) dichloromethane adduct (0.47 g, 0.56 mmol), KOAc (6.3 g, 65 mmol) in 43 mL anhydrous dioxane was heated to 80°C under nitrogen with a water-cooled reflux condensor. After 12 h, the reaction was cooled to ambient temperature and filtered through celite, rinsing with dichloromethane. The solvent was removed under reduced pressure, and the material was filtered through silica gel with 40% EtOAc/hexanes. The solvent was removed to give the title compound as an orange oil which slowly solidified. MS (ES⁺): 234.1 (M+H)⁺. Calc' d for C₁₃H₂₀BNO₂: 233.11.

### Step 2. 6-(4-amino-2-methylphenyl)quinazolin-2-amine

A mixture of 3-methyl-4-(4,4,5,5-tetramethyl-1,3,2-dioxaborolan-2-yl)benzenamine (1.0 g, 4.27 mmol), 6-bromoquinazoline-2-amine (0.48 g, 2.1 mmol), tetrakis(triphenylphosphine) palladium (0) (0.12 g, 0.11 mmol), sodium carbonate (2.0 M solution in water, 4.3 ml, 8.5 mmol), and 20 ml dioxane was heated to 80°C under nitrogen with a water-cooled reflux condensor. After 8 h, the reaction was cooled to ambient temperature and concentrated under reduced pressure. The residue was partitioned between water and EtOAc. The aqueous layer was extracted twice with EtOAc, and the combined organic layers were dried with Na₂SO₄, filtered, and concentrated. The resulting solid was suspended in dichloromethane and filtered to give the title compound as a brown solid. MS (ES⁺) : 251.1 (M+H)⁺; Calc'd for C₁₅H₁₄N₄: 250.30.

### Example 760

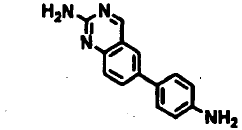

### Synthesis of 6-(4-aminophenyl) quinazolin-2-amine

The title compound was synthesized in a manner similar to the method described in Example 759, affording a yellow solid. MS (ES⁺) : 237.1 (M+H)⁺. Calc' d for C₁₉H₁₂N₄: 236.27.

### Example 761

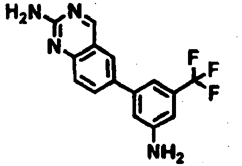

### Synthesis of 6-(3-amino-5-(trifluoromethyl)phenyl)quinazolin-2-amine

The title compound was synthesized in a manner similar to the method described in Example 759, except that a mixture of toluene and ethanol was used as solvent, affording an orange solid. MS (ES⁺) : 305.0 (M+H)⁺; Calc'd for C₁₅H₁₁F₃N₄: 304.27.

### Example 762 (Reference Example)

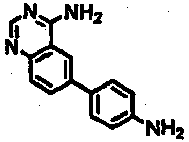

### Synthesis of 6-(4-aminophenyl)quinazolin-4-amine

A mixture of 4-(4,4,5,5-tetramethyl-1,3,2-dioxaborolan-2-yl)benzenamine (2.0 g, 9.1 mmol), 6-bromoquinazolin-4-amine (1.8 g, 8.2 mmol), bis(diphenylphosphino)ferrocene]palladium (II) dichloromethane adduct (0.33 g, 0.45 mmol), sodium carbonate (2.0 M solution in water, 9.1 ml, 18 mmol), and 23 ml dioxane was heated to 80°C under nitrogen in a sealed tube. After 4 h, the reaction was cooled to ambient temperature and allowed to stand overnight. The resulting precipitate was collected by filtration, rinsing with EtOAc to give the title compound as an off-white solid. MS (ES⁺): 237.0 (M+H)⁺. Calc'd for C₁₄H₁₂N₄: 236.27.

### Example 763

### General synthesis of acid chlorides

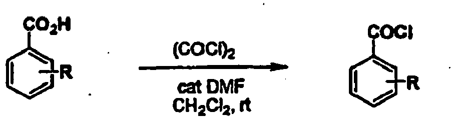

### Synthesis of 3,5-ditert-butyl benzoyl chloride

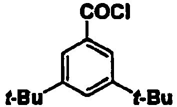

Oxalyl chloride (0.542 g, 0.37 ml, 4.27 mmol) was added dropwise to a solution of 3,5-di-tert-butylbenzoic acid (0.200 g, 0.853 mmol) and dichloromethane (4 ml). N,N-Dimethylformamide (1 drop) was added and the colorless solution stirred at RT for 3 h. The solution was concentrated to afford 3,5-di-tert-butylbenzoyl chloride as a yellow oil.

The following acid chlorides were prepared according to the methods described in Example 763 above. 1-methyl-1H-indole-2-carbonyl chloride, 2-chloro-3-(trifluoromethyl)benzoyl chloride, 4-chloro-3-(trifluoromethyl)benzoyl chloride, 2-chloro-3-methylbenzoyl chloride, and 2-chloro-3-fluorobenzoyl chloride.

The following compounds in Table 1 are additional representative examples of Formula II, as provided by the present invention. Compounds of Table 1 falling within claims 1 and 2 are according to the invention. All other compounds are reference compounds.

**Table 1**

| | | | | | |
|---|---|---|---|---|---|
| 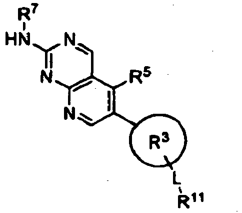 | | | | | |

| Ex. No. | R³ | R⁵ | R⁷ | L | R¹¹ |
|---|---|---|---|---|---|
| 764 | 2-CH₃-phenyl | H | H | m-C(O)NH- | 1-piperidinylpropylo xyphenyl |
| 765 | 4-CH₃-phenyl | H | H | m-C(O)NH- | cyclopropyl |
| 766 | 5-CH₃-phenyl | H | H | m-C(O)NH- | 2-(3-dimethylaminopropyl)methylamino-5-CF₃-phenyl |
| 767 | 6-CH₃-phenyl | H | H | m-C(O)NH- | 2-dimethylamino-5-CF₃-phenyl |
| 768 | 2-OCH₃-phenyl | H | H | m-C(O)NH- | 1-piperidinylpropyloxyphenyl |
| 769 | 4-OCH₃-phenyl | H | H | m-C(O)NH- | cyclopropyl |
| 770 | 5-OCH₃-phenyl | H | H | m-C(O)NH- | 2-(3-dimethylaminopropyl)methylamino-5-CF₃-phenyl |
| 771 | 6-OCH₃-phenyl | H | H | m-C(O)NH- | 2-dimethylamino-5-CF₃-phenyl |
| 772 | 2-F-phenyl | H | H | m-C(O)NH- | 1-piperidinylpropyloxyphenyl |
| 773 | 4-F-phenyl | H | H | m-C(O)NH- | cyclopropyl |
| 774 | 5-F-phenyl | H | H | m-C(O)NH- | 2-(3-dimethylaminopropyl)methylamino-5-CF₃-phenyl |
| 775 | 6-F-phenyl | H | H | m-C(O)NH- | 2-dimethylamino-5-CF₃-phenyl |
| 776 | 2-thiophene | H | H | m-C(O)NH- | 1-1-piperidinylpropylo xyphenyl |
| 777 | 3-thiophene | H | H | m-C(O)NH- | cyclopropyl |
| 778 | 2-pyridine | H | H | m-C(O)NH- | 2-(3-dimethylaminopropyl)methylamino-5-CF₃-phenyl |
| 779 | 3-pyridine | H | H | m-C(O)NH- | 2-dimethylamino-5-CF₃-phenyl |
| 780 | 2-CH₃-phenyl | CH₃ | H | m-C(O)NH- | 1-piperidinylpropyloxyphenyl |
| 781 | 4-CH₃-phenyl | CH₃ | H | m-C(O)NH- | cyclopropyl |
| 782 | 5-CH₃-phenyl | CH₃ | H | m-C(O)NH- | 2-(3-dimethylaminopropyl)methylamino-5-CF₃-phenyl |
| 783 | 6-CH₃-phenyl | CH₃ | H | m-C(O)NH- | 2-dimethylamino-5-CF₃-phenyl |
| 784 | 2-OCH₃-phenyl | CH₃ | H | m-C(O)NH- | 1-piperidinylpropyloxyphenyl |
| 785 | 4-OCH₃-phenyl | CH₃ | H | m-C(O)NH- | cyclopropyl |
| 786 | 5-OCH₃-phenyl | CH₃ | H | m-C(O)NH- | 2-(3-dimethylaminopropyl)methylamino-5-CF₃-phenyl |
| 787 | 6-OCH₃-phenyl | CH₃ | H | m-C(O)NH- | 2-dimethylamino-5-CF₃-phenyl |
| 788 | 2-F-phenyl | CH₃ | H | m-C(O)NH- | 1-piperidinylpropyloxyphenyl |
| 789 | 4-F-phenyl | CH₃ | H | m-C(O)NH- | cyclopropyl |
| 790 | 5-F-phenyl | CH₃ | H | m-C(O)NH- | 2-(3-dimethylaminopropyl)methylamino-5-CF₃-phenyl |
| 791 | 6-F-phenyl | CH₃ | H | m-C(O)NH- | 2-dimethylamino-5-CF₃-phenyl |
| 792 | 2-thiophene | CH₃ | H | m-C(O)NH- | 1-piperidinylpropyloxyphenyl |
| 793 | 3-thiophene | CH₃ | H | m-C(O)NH- | cyclopropyl |
| 794 | 2-pyridine | CH₃ | H | m-C(O)NH- | 2-(3-dimethylaminopropyl)methylamino-5-CF₃-phenyl |
| 795 | 3-pyridine | CH₃ | H | m-C(O)NH- | 2-dimethylamino-5-CF₃-phenyl |
| 796 | 2-CH₃-phenyl | H | -(CH₂)₃-morpholine | m-C(O)NH- | 1-piperidinylpropyloxyphenyl |
| 797 | 4-CH₃-phenyl | H | -(CH₂)₃ morpholine | m-C(O)NH- | cyclopropyl |
| 798 | 5-CH₃-phenyl | H | -(CH₂)₃ morpholine | m-C(O)NH- | 2-(3-dimethylaminopropyl)methylamino-5-CF₃-phenyl |
| 799 | 6-CH₃-phenyl | H | -(CH₂)₃ morpholine | m-C(O)NH- | 2-dimethylamino-5-CF₃-phenyl |
| 800 | 2-OCH₃-phenyl | H | -(CH₂)₃ morpholine | m-C(O)NH- | 1-piperidinylpropyloxyphenyl |
| 801 | 4-OCH₃-phenyl | H | -(CH₂)₃ morpholine | m-C(O)NH- | cyclopropyl |
| 802 | 5-OCH₃-phenyl | H | -(CH₂)₃ morpholine | m-C(O)NH- | 2-(3-dimethylaminopropyl)methylamino-5-CF₃-phenyl |
| 803 | 6-OCH₃-phenyl | H | (CH₂)₃-morpholine | m-C(O)NH- | 2-dimethylamino-5-CF₃-phenyl |
| 804 | 2-F-phenyl | H | -(CH₂)₃ morpholine | m-C(O)NH- | 1-piperidinylpropyloxyphenyl |
| 805 | 4-F-phenyl | H | -(CH₂)₃ morpholine | m-C(O)NH- | cyclopropyl |
| 806 | 5-F-phenyl | H | -(CH₂)₃ morpholine | m-C(O)NH- | 2-(3-dimethylaminopropyl)methylamino-5-CF₃-phenyl |
| 807 | 6-F-phenyl | H | -(CH₂)₃-morpholine | m-C(O)NH- | 2-dimethylamino-5-CF₃-phenyl |
| 808 | 2-thiophene | H | -(CH₂)₃ morpholine | m-C(O)NH- | 1-piperidinylpropyloxyphenyl |
| 809 | 3-thiophene | H | -(CH₂)₃ morpholine | m-C(O)NH- | cyclopropyl |
| 810 | 2-pyridine | H | -(CH₂)₃-morpholine | m-C(O)NH- | 2-(3-dimethylaminopropy1l)methylamino-5-CF₃-phenyl |
| 811 | 3-pyridine | H | -(CH₂)₃ morpholine | m-C(O)NH- | 2-dimethylamino-5-CF₃-phenyl |
| 812 | 2-CH₃-phenyl | H | CH₃ | m-C(O)NH- | 1-piperidinylpropyloxyphenyl |
| 813 | 4-CH₃-phenyl | H | CH₃ | m-C(O)NH- | cyclopropyl |
| 814 | 5-CH₃-phenyl | H | CH₃ | m-C(O)NH- | 2-(3-dimethylaminopropyl)methylamino-5-CF₃-phenyl |
| 815 | 6-CH₃-phenyl | H | CH₃ | m-C(O)NH- | 2-dimethylamino-5-CF₃-phenyl |
| 816 | 2-OCH₃-phenyl | H | CH₃ | m-C(O)NH- | 1-piperidinylpropyloxyphenyl |
| 817 | 4-OCH₃-phenyl | H | CH₃ | m-C(O)NH- | cyclopropyl |
| 818 | 5-OCH₃-phenyl | H | CH₃ | m-C(O)NH- | 2-(3-dimethylaminopropyl)methylamino-5-CF₃-phenyl |
| 819 | 6-OCH₃-phenyl | H | CH₃ | m-C(O)NH- | 2-dimethylamino-5-CF₃-phenyl |
| 820 | 2-F-phenyl | H | CH₃ | m-C(O)NH- | 1-piperidinylpropyloxyphenyl |
| 821 | 4-F-phenyl | H | CH₃ | m-C(O)NH- | cyclopropyl |
| 822 | 5-F-phenyl | H | CH₃ | m-C(O)NH- | 2-(3-dimethylaminopropyl)methylamino-5-CF₃-phenyl |
| 823 | 6-F-phenyl | H | CH₃ | m-C(O)NH- | 2-dimethylamino-5-CF₃-phenyl |
| 824 | 2-thiophene | H | CH₃ | m-C(O)NH- | 1-piperidinylpropyloxyphenyl |
| 825 | 3-thiophene | H | CH₃ | m-C(O)NH- | cyclopropyl |
| 826 | 2-pyridine | H | CH₃ | m-C(O)NH- | 2-(3-dimethylaminopropyl)methylamino-5-CF₃-phenyl |
| 827 | 3-pyridine | H | CH₃ | m-C(O)NH- | 2-dimethylamino-5-CF₃-phenyl |
| 828 | 2-CH₃-phenyl | H | -O(CH₂)₂-piperidine | m-C(O)NH- | 1-piperidinylpropyloxyphenyl |
| 829 | 4-CH₃-phenyl | H | -O(CH₂)₂-piperidine | m-C(O)NH- | cyclopropyl |
| 830 | 5-CH₃-phenyl | H | -O(CH₂)₂-piperidine | m-C(O)NH- | 2-(3-dimethylaminopropyl)methylamino-5-CF₃-phenyl |
| 831 | 6-CH₃-phenyl | H | -O(CH₂)₂-piperidine | m-C(O)NH- | 2-dimethylamino-5-CF₃-phenyl |
| 832 | 2-OCH₃-phenyl | H | -O(CH₂)₂-piperidine | m-C(O)NH- | 1-piperidinylpropyloxyphenyl |
| 833 | 4-OCH₃-phenyl | H | -O(CH₂)₂-piperidine | m-C(O)NH- | cyclopropyl |
| 834 | 5-OCH₃-phenyl | H | -O(CH₂)₂-piperidine | m-C(O)NH- | 2-(3-dimethylaminopropyl)methylamino-5-CF₃-phenyl |
| 835 | 6-OCH₃-phenyl | H | -O(CH₂)₂-piperidine | m-C(O)NH- | 2-dimethylamino-5-CF₃-phenyl |
| 836 | 2-F-phenyl | H | -O(CH₂)₂-piperidine | m-C(O)NH- | 1-piperidinylpropyloxyphenyl |
| 837 | 4-F-phenyl | H | -O(CH₂)₂-piperidine | m-C(O)NH- | cyclopropyl |
| 838 | 5-F-phenyl | H | -O(CH₂)₂-piperidine | m-C(O)NH- | 2-(3-dimethylaminopropyl)methylamino-5-CF₃-phenyl |
| 839 | 6-F-phenyl | H | -O(CH₂)₂-piperidine | m-C(O)NH- | 2-dimethylamino-5-CF₃-phenyl |
| 840 | 2-thiophene | H | -O(CH₂)₂-piperidine | m-C(O)NH- | 1-piperidinylpropyloxyphenyl |
| 841 | 3-thiophene | H | -O(CH₂)₂-piperidine | m-C(O)NH- | cyclopropyl |
| 842 | 2-pyridine | H | -O(CH₂)₂-piperidine | m-C(O)NH- | 2-(3-dimethylaminopropyl)methylamino-5-CF₃-phenyl |
| 843 | 3-pyridine | H | -O(CH₂)₂-piperidine | m-C(O)NH- | 2-dimethylamino-5-CF₃-phenyl |
| 844 | 2-CH₃-phenyl | H | -O(CH₂)₂-piperzine | m-C(O)NH- | 1-piperidinylpropyloxyphenyl |
| 845 | 4-CH₃-phenyl | H | -O(CH₂)₂-piperzine | m-C(O)NH- | cyclopropyl |
| 846 | 5-CH₃-phenyl | H | -O(CH₂)₂-piperzine | m-C(O)NH- | 2-(3-dimethylaminopropyl)methylamino-5-CF₃-phenyl |
| 847 | 6-CH₃-phenyl | H | -O(CH₂)₂-piperzine | m-C(O)NH- | 2-dimethylamino-5-CF₃-phenyl |
| 848 | 2-OCH₃-phenyl | H | -O(CH₂)₂-piperzine | m-C(O)NH- | 1-piperidinylpropyloxyphenyl |
| 849 | 4-OCH₃-phenyl | H | -O(CH₂)₂-piperzine | m-C(O)NH- | cyclopropyl |
| 850 | 5-OCH₃-phenyl | H | -O(CH₂)₂-piperzine | m-C(O)NH- | 2-(3-dimethylaminopropyl)methylamino-5-CF₃-phenyl |
| 851 | 6-OCH₃-phenyl | H | -O(CH₂)₂-piperzine | m-C(O)NH- | 2-dimethylamino-5-CF₃-phenyl |
| 852 | 2-F-phenyl | H | -O(CH₂)₂-piperzine | m-C(O)NH- | 1-piperidinylpropyloxyphenyl |
| 853 | 4-F-phenyl | H | -O(CH₂)₂-piperzine | m-C(O)NH- | cyclopropyl |
| 854 | 5-F-phenyl | H | O(CH₂)₂-piperzine | m-C(O)NH- | 2-(3-dimethylaminopropyl)methylamino-5-CF₃-phenyl |
| 855 | 6-F-phenyl | H | -O(CH₂)₂-piperzine | m-C(O)NH- | 2-dimethylamino-5-CF₃-phenyl |
| 856 | 2-thiophene | H | -O(CH₂)₂-piperzine | m-C(O)NH- | 1-piperidinylpropyloxyphenyl |
| 857 | 3-thiophene | H | -O(CH₂)₂-piperzine | m-C(O)NH- | cyclopropyl |
| 858 | 2-pyridine | H | -O(CH₂)₂-piperzine | m-C(O)NH- | 2-(3-dimethylaminopropyl)methylamino-5-CF₃-phenyl |
| 859 | 3-pyridine | H | -O(CH₂)₂-piperzine | m-C(O)NH- | 2-dimethylamino-5-CF₃-phenyl |
| 860 | 2-CH₃-phenyl | H | 1-pyrrolidinylethyl | m-C(O)NH- | 1-piperidinylpropyloxyphenyl |
| 861 | 4-CH₃-phenyl | H | 1-pyrrolidinylethyl | m-C(O)NH- | cyclopropyl |
| 862 | 5-CH₃-phenyl | H | 1-pyrrolidinylethyl | m-C(O)NH- | 2-(3-dimethylaminopropyl)methylamino-5-CF₃-phenyl |
| 863 | 6-CH₃-phenyl | H | 1-pyrrolidinylethyl | m-C(O)NH- | 2-dimethylamino-5-CF₃-phenyl |
| 864 | 2-OCH₃-phenyl | H | 1-pyrrolidinylethyl | m-C(O)NH- | 1-piperidinylpropyloxyphenyl |
| 865 | 4-OCH₃-phenyl | H | 1-pyrrolidinylethyl | m-C(O)NH- | cyclopropyl |
| 866 | 5-OCH₃-phenyl | H | 1-pyrrolidinylethyl | m-C(O)NH- | 2-(3-dimethylaminopropyl)methylamino-5-CF₃-phenyl |
| 867 | 6-OCH₃-phenyl | H | 1-pyrrolidinylethyl | m-C(O)NH- | 2-dimethylamino-5-CF₃-phenyl |
| 868 | 2-F-phenyl | H | 1-pyrrolidinylethyl | m-C(O)NH- | 1-piperidinylpropyloxyphenyl |
| 869 | 4-F-phenyl | H | 1-pyrrolidinylethyl | m-C(O)NH- | cyclopropyl |
| 870 | 5-F-phenyl | H | 1-pyrrolidinylethyl | m-C(O)NH- | 2-(3-dimethylaminopropyl)methylamino-5-CF₃-phenyl |
| 871 | 6-F-phenyl | H | 1-pyrrolidinylethyl | m-C(O)NH- | 2-dimethylamino-5-CF₃-phenyl |
| 872 | 2-thiophene | H | 1-pyrrolidinylethyl | m-C(O)NH- | 1-piperidinylpropyloxyphenyl |
| 873 | 3-thiophene | H | 1-pyrrolidinylethyl | m-C(O)NH- | cyclopropyl |
| 874 | 2-pyridine | H | 1-pyrrolidinylethyl | m-C(O)NH- | 2-(3-dimethylaminopropyl)methylamino-5-CF₃-phenyl |
| 875 | 3-pyridine | H | 1-pyrrolidinylethyl | m-C(O)NH- | 2-dimethylamino-5-CF₃-phenyl |
| 876 | 2-CH₃-phenyl | H | H | p-NHC(O)NH- | 1-piperidinylpropyloxyphenyl |
| 877 | 4-CH₃-phenyl | H | H | p-NHC(O)NH- | cyclopropyl |
| 878 | 5-CH₃-phenyl | H | H | p-NHC(O)NH- | 2-(3-dimethylaminopropyl)methylamino-5-CF₃-phenyl |
| 879 | 6-CH₃-phenyl | H | H | p-NHC(O)NH- | 2-dimethylamino-5-CF₃-phenyl |
| 880 | 2-OCH₃-phenyl | H | H | p-NHC(O)NH- | 1-piperidinylpropyloxyphenyl |
| 881 | 4-OCH₃-phenyl | H | H | p-NHC(O)NH- | cyclopropyl |
| 882 | 5-OCH₃-phenyl | H | H | p-NHC(O)NH- | 2-(3-dimethylaminopropyl)methylamino-5 CF₃-phenyl |
| 883 | 6-OCH₃-phenyl | H | H | p-NHC(O)NH- | 2-dimethylamino-5-CF₃-phenyl |
| 884 | 2-F-phenyl | H | H | p-NHC(O)NH- | 1-piperidinylpropyloxyphenyl |
| 885 | 4-F-phenyl | H | H | p-NHC(O)NH- | cyclopropyl |
| 886 | 5-F-phenyl | H | H | p-NHC(O)NH- | 2-(3-dimethylaminopropyl)methylamino-5-CF₃-phenyl |
| 887 | 6-F-phenyl | H | H | p-NHC(O)NH- | 2-dimethylamino-5-CF₃-phenyl |
| 888 | 2-thiophene | H | H | p-NHC(O)NH- | 1-piperidinylpropyloxyphenyl |
| 889 | 3-thiophene | H | H | p-NHC(O)NH- | cyclopropyl |
| 890 | 2-pyridine | H | H | p-NHC(O)NH- | 2-(3-dimethylaminopropyl)methylamino-5 CF₃-phenyl |
| 891 | 3-pyridine | H | H | p-NHC(O)NH- | 2-dimethylamino-5-CF₃-phenyl |
| 892 | 2-CH₃-phenyl | CH₃ | H | p-NHC(O)NH- | 1-piperidinylpropyloxyphenyl |
| 893 | 4-CH₃-phenyl | CH₃ | H | p-NHC(O)NH- | cyclopropyl |
| 894 | 5-CH₃-phenyl | CH₃ | H | p-NHC(O)NH- | 2-(3-dimethylaminopropyl)methylamino-5-CF₃-phenyl |
| 895 | 6-CH₃-phenyl | CH₃ | H | p-NHC(O)NH- | 2-dimethylamino-5-CF₃-phenyl |
| 896 | 2-OCH₃-phenyl | CH₃ | H | p-NHC(O)NH- | 1-piperidinylpropyloxyphenyl |
| 897 | 4-OCH₃-phenyl | CH₃ | H | p-NHC(O)NH- | cyclopropyl |
| 898 | 5-OCH₃-phenyl | CH₃ | H | p-NHC(O)NH- | 2-(3-dimethylaminopropyl)methylamino-5-CF₃-phenyl |
| 899 | 6-OCH₃-phenyl | CH₃ | H | p-NHC(O)NH- | 2-dimethylamino-5-CF₃-phenyl |
| 900 | 2-F-phenyl | CH₃ | H | p-NHC(0)NH- | 1-piperidinylpropyloxyphenyl |
| 901 | 4-F-phenyl | CH₃ | H | p-NHC(O)NH- | cyclopropyl |
| 902 | 5-F-phenyl | CH₃ | H | p-NHC(0)NH- | 2-(3-dimethylaminopropyl)methylamino-5-CF₃-phenyl |
| 903 | 6-F-phenyl | CH₃ | H | p-NHC(O)NH- | 2-dimethylamino-5-CF₃-phenyl |
| 904 | 2-thiophene | CH₃ | H | p-NHC(O)NH- | 1-piperidinylpropyloxyphenyl |
| 905 | 3-thiophene | CH₃ | H | p-NHC(O)NH- | cyclopropyl |
| 906 | 2-pyridine | CH₃ | H | p-NHC(O)NH- | 2-(3-dimethylaminopropyl)methylamino-5-CF₃-phenyl |
| 907 | 3-pyridine | CH₃ | H | p-NHC(O)NH- | 2-dimethylamino-5-CF₃-phenyl |
| 908 | 2-CH₃-phenyl | H | -(CH₂)₃-morpholine | p-NHC(O)NH- | 1-piperidinylpropyloxyphenyl |
| 909 | 4-CH₃-phenyl | H | -(CH₂)₃ morpholine | p-NHC(O)NH- | cyclopropyl |
| 910 | 5-CH₃-phenyl | H | -(CH₂)₃ morpholine | p-NHC(O)NH- | 2-(3-dimethylaminopropyl)methylamino-5-CF₃-phenyl |
| 911 | 6-CH₃-phenyl | H | -(CH₂)₃-morpholine | p-NHC(O)NH- | 2-dimethylamino-5-CF₃-phenyl |
| 912 | 2-OCH₃-phenyl | H | -(CH₂)₃-morpholine | p-NHC(O)NH- | 1-piperidinylpropyloxyphenyl |
| 913 | 4-OCH₃-phenyl | H | -(CH₂)₃ morpholine | p-NHC(O)NH- | cyclopropyl |
| 914 | 5-OCH₃-phenyl | H | -(CH₂)₃ morpholine | p-NHC(O)NH- | 2-(3-dimethylaminopropyl)methylamino-5-CF₃-phenyl |
| 915 | 6-OCH₃-phenyl | H | -(CH₂)₃-morpholine | p-NHC(O)NH- | 2-dimethylamino-5-CF₃-phenyl |
| 916 | 2-F-phenyl | H | -(CH₂)₃ morpholine | p-NHC(O)NH- | 1-piperidinylpropyloxyphenyl |
| 917 | 4-F-phenyl | H | -(CH₂)₃ morpholine | p-NHC(O)NH- | cyclopropyl |
| 918 | 5-F-phenyl | H | -(CH₂)₃ morpholine | p-NHC(O)NH- | 2-(3-dimethylaminopropyl)methylamino-5 CF₃-phenyl |
| 919 | 6-F-phenyl | H | -(CH₂)₃-morpholine | p-NHC(O)NH- | 2-dimethylamino-5-CF₃-phenyl |
| 920 | 2-thiophene | H | -(CH₂)₃ morpholine | p-NHC(O)NH- | 1-piperidinylpropyloxyphenyl |
| 921 | 3-thiophene | H | -(CH₂)₃-morpholine | p-NHC(O)NH- | cyclopropyl |
| 922 | 2-pyridine | H | -(CH₂)₃-morpholine | p-NHC(O)NH- | 2-(3-dimethylaminopropyl)methylamino-5-CF₃-phenyl |
| 923 | 3-pyridine | H | -(CH₂)₃-morpholine | p-NHC(O)NH- | 2-dimethylamino-5-CF₃-phenyl |
| 924 | 2-CH₃-phenyl | H | CH₃ | p-NHC(O)NH- | 1-piperidinylpropyloxyphenyl |
| 925 | 4-CH₃-phenyl | H | CH₃ | p-NHC(O)NH- | cyclopropyl |
| 926 | 5-CH₃-phenyl | H | CH₃ | p-NHC(O)NH- | 2-(3-dimethylaminopropyl)methylamino-5-CF₃-phenyl |
| 927 | 6-CH₃-phenyl | H | CH₃ | p-NHC(O)NH- | 2-dimethylamino-5-CF₃-phenyl |
| 928 | 2-OCH₃-phenyl | H | CH₃ | p-NHC(O)NH- | 1-piperidinylpropyloxyphenyl |
| 909 | 4-OCH₃-phenyl | H | CH₃ | p-NHC(O)NH- | cyclopropyl |
| 910 | 5-OCH₃-phenyl | H | CH₃ | p-NHC(O)NH- | 2-(3-dimethylaminopropyl)methylamino-5-CF₃-phenyl |
| 911 | 6-OCH₃-phenyl | H | CH₃ | p-NHC(O)NH- | 2-dimethylamino-5-CF₃-phenyl |
| 912 | 2-F-phenyl | H | CH₃ | p-NHC(O)NH- | 1-piperidinylpropyloxyphenyl |
| 913 | 4-F-phenyl | H | CH₃ | p-NHC(O)NH- | cyclopropyl |
| 914 | 5-F-phenyl | H | CH₃ | p-NHC(O)NH- | 2-(3-dimethylaminopropyl)methylamino-5-CF₃-phenyl |
| 915 | 6-F-phenyl | H | CH₃ | p-NHC(O)NH- | 2-dimethylamino-5-CF₃-phenyl |
| 916 | 2-thiophene | H | CH₃ | p-NHC(O)NH- | 1-piperidinylpropyloxyphenyl |
| 917 | 3-thiophene | H | CH₃ | p-NHC(O)NH- | cyclopropyl |
| 918 | 2-pyridine | H | CH₃ | p-NHC(O)NH- | 2-(3-dimethylaminopropyl)methylamino-5-CF₃-phenyl |
| 919 | 3-pyridine | H | CH₃ | p-NHC(O)NH- | 2-dimethylamino-5-CF₃-phenyl |
| 920 | 2-CH₃-phenyl | H | -O(CH₂)₂-piperidine | p-NHC(O)NH- | 1-piperidinylpropyloxyphenyl |
| 921 | 4-CH₃-phenyl | H | -O(CH₂)₂-piperidine | p-NHC(O)NH- | cyclopropyl |
| 922 | 5-CH₃-phenyl | H | -O(CH₂)₂-piperidine | p-NHC(O)NH- | 2-(3-dimethylaminopropyl)methylamino-5 CF₃-phenyl |
| 923 | 6-CH₃-phenyl | H | -O(CH₂)₂-piperidine | p-NHC(O)NH- | 2-dimethylamino-5-CF₃-phenyl |
| 924 | 2-OCH₃-phenyl | H | -O(CH₂)₂-piperidine | p-NHC(O)NH- | 1-piperidinylpropyloxyphenyl |
| 925 | 4-OCH₃-phenyl | H | -O(CH₂)₂-piperidine | p-NHC(O)NH- | cyclopropyl |
| 926 | 5-OCH₃-phenyl | H | -O(CH₂)₂-piperidine | p-NHC(O)NH- | 2-(3-dimethylaminopropyl)methylamino-5-CF₃-phenyl |
| 927 | 6-OCH₃-phenyl | H | -O(CH₂)₂-piperidine | p-NHC(O)NH- | 2-dimethylamino-5-CF₃-phenyl |
| 928 | 2-F-phenyl | H | -O(CH₂)₂-piperidine | p-NHC(O)NH- | 1-piperidinylpropyloxyphenyl |
| 929 | 4-F-phenyl | H | -O(CH₂)₂-piperidine | p-NHC(O)NH- | cyclopropyl |
| 930 | 5-F-phenyl | H | -O(CH₂)₂-piperidine | p-NHC(O)NH- | 2-(3-dimethylaminopropyl)methylamino-5-CF₃-phenyl |
| 931 | 6-F-phenyl | H | -O(CH₂)₂-piperidine | p-NHC(O)NH- | 2-dimethylamino-5-CF₃-phenyl |
| 932 | 2-thiophene | H | -O(CH₂)₂-piperidine | p-NHC(O)NH- | 1-piperidinylpropyloxyphenyl |
| 933 | 3-thiophene | H | -O(CH₂)₂-piperidine | p-NHC(O)NH- | cyclopropyl |
| 934 | 2-pyridine | H | -O(CH₂)₂-piperidine | p-NHC(O)NH- | 2-(3-dimethylaminopropyl)methylamino-5-CF₃-phenyl |
| 935 | 3-pyridine | H | -O(CH₂)₂-piperidine | p-NHC(O)NH- | 2-dimethylamino-5-CF₃-phenyl |
| 936 | 2-CH₃-phenyl | H | -O(CH₂)₂-piperzine | p-NHC(O)NH- | 1-piperidinylpropyloxyphenyl |
| 937 | 4-CH₃-phenyl | H | -O(CH₂)₂-piperzine | p-NHC(O)NH- | cyclopropyl |
| 938 | 5-CH₃-phenyl | H | -O(CH₂)₂-piperzine | p-NHC(O)NH- | 2-(3-dimethylaminopropyl)methylamino-5-CF₃-phenyl |
| 939 | 6-CH₃-phenyl | H | -O(CH₂)₂-piperzine | p-NHC(O)NH- | 2-dimethylamino-5-CF₃-phenyl |
| 940 | 2-OCH₃-phenyl | H | -O(CH₂)₂-piperzine | p-NHC(O)NH- | 1-piperidinylpropyloxyphenyl |
| 941 | 4-OCH₃-phenyl | H | -O(CH₂)₂-piperzine | p-NHC(O)NH- | cyclopropyl |
| 942 | 5-OCH₃-phenyl | H | -O(CH₂)₂-piperzine | p-NHC(O)NH- | 2-(3-dimethylaminopropyl)methylamino-5-CF₃-phenyl |
| 943 | 6-OCH₃-phenyl | H | -O(CH₂)₂-piperzine | p-NHC(O)NH- | 2-dimethylamino-5-CF₃-phenyl |
| 944 | 2-F-phenyl | H | -O(CH₂)₂-piperzine | p-NHC(O)NH- | 1-piperidinylpropyloxyphenyl |
| 945 | 4-F-phenyl | H | -O(CH₂)₂-piperzine | p-NHC(O)NH- | cyclopropyl |
| 946 | 5-F-phenyl | H | -O(CH₂)₂-piperzine | p-NHC(O)NH- | 2-(3-dimethylaminopropyl)methylamino-5 CF₃-phenyl |
| 947 | 6-F-phenyl | H | -O(CH₂)₂-piperzine | p-NHC(O)NH- | 2-dimethylamino-5-CF₃-phenyl |
| 948 | 2-thiophene | H | -O(CH₂)₂-piperzine | p-NHC(O)NH- | 1-piperidinylpropyloxyphenyl |
| 949 | 3-thiophene | H | -O(CH₂)₂-piperzine | p-NHC(O)NH- | cyclopropyl |
| 950 | 2-pyridine | H | -O(CH₂)₂-piperzine | p-NHC(O)NH- | 2-(3-dimethylaminopropyl)methylamino-5-CF₃-phenyl |
| 951 | 3-pyridine | H | -O(CH₂)₂-piperzine | p-NHC(O)NH- | 2-dimethylamino-5-CF₃-phenyl |
| 952 | | | | | |
| 953 | 2-CH₃-phenyl | H | H | m-NHC(O)NH- | 1-piperidinylpropyloxyphenyl |
| 954 | 4-CH₃-phenyl | H | H | m-NHC(O)NH- | cyclopropyl |
| 955 | 5-CH₃-phenyl | H | H | m-NHC(O)NH- | 2-(3-dimethylaminopropyl)methylamino-5 CF₃-phenyl |
| 956 | 6-CH₃-phenyl | H | H | m-NHC(O)NH- | 2-dimethylamino-5-CF₃-phenyl |
| 957 | 2-OCH₃-phenyl | H | H | m-NHC(O)NH- | 1-piperidinylpropyloxyphenyl |
| 958 | 4-OCH₃-phenyl | H | H | m-NHC(O)NH- | cyclopropyl |
| 959 | 5-OCH₃-phenyl | H | H | m-NHC(O)NH- | 2-(3-dimethylaminopropyl)methylamino-5-CF₃-phenyl |
| 960 | 6-OCH₃-phenyl | H | H | m-NHC(O)NH- | 2-dimethylamino-5-CF₃-phenyl |
| 961 | 2-F-phenyl | H | H | m-NHC(O)NH- | 1-piperidinylpropyloxyphenyl |
| 962 | 4-F-phenyl | H | H | m-NHC(O)NH- | cyclopropyl |
| 963 | 5-F-phenyl | H | H | m-NHC(O)NH- | 2-(3-dimethylaminopropyl)methylamino-5-CF₃-phenyl |
| 964 | 6-F-phenyl | H | H | m-NHC(O)NH- | 2-dimethylamino-5-CF₃-phenyl |
| 965 | 2-thiophene | H | H | m-NHC(O)NH- | 1-piperidinylpropyloxyphenyl |
| 966 | 3-thiophene | H | H | m-NHC(O)NH- | cyclopropyl |
| 967 | 2-pyridine | H | H | m-NHC(O)NH- | 2-(3-dimethylaminopropyl)methylamino-5-CF₃-phenyl |
| 968 | 3-pyridine | H | H | m-NHC(O)NH- | 2-dimethylamino-5-CF₃-phenyl |
| 969 | 2-CH₃-phenyl | CH₃ | H | m-NHC(O)NH- | 1-piperidinylpropyloxyphenyl |
| 970 | 4-CH₃-phenyl | CH₃ | H | m-NHC(O)NH- | cyclopropyl |
| 971 | 5-CH₃-phenyl | CH₃ | H | m-NHC(O)NH- | 2-(3-dimethylaminopropyl)methylamino-5-CF₃-phenyl |
| 972 | 6-CH₃-phenyl | CH₃ | H | m-NHC(O)NH- | 2-dimethylamino-5-CF₃-phenyl |
| 973 | 2-OCH₃-phenyl | CH₃ | H | m-NHC(O)NH- | 1-piperidinylpropyloxyphenyl |
| 974 | 4-OCH₃-phenyl | CH₃ | H | m-NHC(O)NH- | cyclopropyl |
| 975 | 5-OCH₃-phenyl | CH₃ | H | m-NHC(O)NH- | 2-(3-dimethylaminopropyl)methylamino-5-CF₃-phenyl |
| 976 | 6-OCH₃-phenyl | CH₃ | H | m-NHC(O)NH- | 2-dimethylamino-5-CF₃-phenyl |
| 977 | 2-F-phenyl | CH₃ | H | m-NHC(O)NH- | 1-piperidinylpropyloxyphenyl |
| 978 | 4-F-phenyl | CH₃ | H | m-NHC(O)NH- | cyclopropyl |
| 979 | 5-F-phenyl | CH₃ | H | m-NHC(O)NH- | 2-(3-dimethylaminopropyl)methylamino-5-CF₃-phenyl |
| 980 | 6-F-phenyl | CH₃ | H | m-NHC(O)NH- | 2-dimethylamino-5-CF₃-phenyl |
| 981 | 2-thiophene | CH₃ | H | m-NHC(O)NH- | 1-piperidinylpropyloxyphenyl |
| 982 | 3-thiophene | CH₃ | H | m-NHC(O)NH- | cyclopropyl |
| 983 | 2-pyridine | CH₃ | H | m-NHC(O)NH- | 2-(3-dimethylaminopropyl)methylamino-5-CF₃-phenyl |
| 984 | 3-pyridine | CH₃ | H | m-NHC(O)NH- | 2-dimethylamino-5-CF₃-phenyl |
| 985 | 2-CH₃-phenyl | H | -(CH₂)₃ morpholine | m-NHC(O)NH- | 1-piperidinylpropyloxyphenyl |
| 986 | 4-CH₃-phenyl | H | -(CH₂)₃-morpholine | m-NHC(O)NH- | cyclopropyl |
| 987 | 5-CH₃-phenyl | H | -(CH₂)₃ morpholine | m-NHC(O)NH- | 2-(3-dimethylaminopropyl)methylamino-5-CF₃-phenyl |
| 988 | 6-CH₃-phenyl | H | -(CH₂)₃ morpholine | m-NHC(O)NH- | 2-dimethylamino-5-CF₃-phenyl |
| 989 | 2-OCH₃-phenyl | H | -(CH₂)₃-morpholine | m-NHC(O)NH- | 1-piperidinylpropyloxyphenyl |
| 990 | 4-OCH₃-phenyl | H | -(CH₂)₃ morpholine | m-NHC(O)NH- | cyclopropyl |
| 991 | 5-OCH₃-phenyl | H | -(CH₂)₃ morpholine | m-NHC(O)NH- | 2-(3-dimethylaminopropyl)methylamino-5-CF₃-phenyl |
| 992 | 6-OCH₃-phenyl | H | -(CH₂)₃ morpholine | m-NHC(O)NH- | 2-dimethylamino-5-CF₃-phenyl |
| 993 | 2-F-phenyl | H | -(CH₂)₃ morpholine | m-NHC(O)NH- | 1-piperidinylpropyloxyphenyl |
| 994 | 4-F-phenyl | H | -(CH₂)₃-morpholine | m-NHC(O)NH- | cyclopropyl |
| 995 | 5-F-phenyl | H | -(CH₂)₃ morpholine | m-NHC(O)NH- | 2-(3-dimethylaminopropyl)methylamino-5-CF₃-phenyl |
| 996 | 6-F-phenyl | H | -(CH₂)₃-morpholine | m-NHC(O)NH- | 2-dimethylamino-5-CF₃-phenyl |
| 997 | 2-thiophene | H | -(CH₂)₃ morpholine | m-NHC(O)NH- | 1-piperidinylpropyloxyphenyl |
| 998 | 3-thiophene | H | -(CH₂)₃ morpholine | m-NHC(O)NH- | cyclopropyl |
| 999 | 2-pyridine | H | -(CH₂)₃ morpholine | m-NHC(O)NH- | 2-(3-dimethylaminopropyl)methylamino-5-CF₃-phenyl |
| 1000 | 3-pyridine | H | -(CH₂)₃-morpholine | m-NHC(O)NH- | 2-dimethylamino-5-CF₃-phenyl |
| 1001 | 2-CH₃-phenyl | H | CH₃ | m-NHC(O)NH- | 1-piperidinylpropyloxyphenyl |
| 1002 | 4-CH₃-phenyl | H | CH₃ | m-NHC(O)NH- | cyclopropyl |
| 1003 | 5-CH₃-phenyl | H | CH₃ | m-NHC(O)NH- | 2-(3-dimethylaminopropyl)methylamino-5-CF₃-phenyl |
| 1004 | 6-CH₃-phenyl | H | CH₃ | m-NHC(O)NH- | 2-dimethylamino-5-CF₃-phenyl |
| 1005 | 2-OCH₃-phenyl | H | CH₃ | m-NHC(O)NH- | 1-piperidinylpropyloxyphenyl |
| 1006 | 4-OCH₃-phenyl | H | CH₃ | m-NHC(O)NH- | cyclopropyl |
| 1007 | 5-OCH₃-phenyl | H | CH₃ | m-NHC(O)NH- | 2-(3-dimethylaminopropyl)methylamino-5-CF₃-phenyl |
| 1008 | 6-OCH₃-phenyl | H | CH₃ | m-NHC(O)NH- | 2-dimethylamino-5-CF₃-phenyl |
| 1009 | 2-F-phenyl | H | CH₃ | m-NHC(O)NH- | 1-piperidinylpropyloxyphenyl |
| 1010 | 4-F-phenyl | H | CH₃ | m-NHC(O)NH- | cyclopropyl |
| 1011 | 5-F-phenyl | H | CH₃ | m-NHC(O)NH- | 2-(3-dimethylaminopropyl)methylamino-5-CF₃-phenyl |
| 1012 | 6-F-phenyl | H | CH₃ | m-NHC(O)NH- | 2-dimethylamino-5-CF₃-phenyl |
| 1013 | 2-thiophene | H | CH₃ | m-NHC(O)NH- | 1-piperidinylpropyloxyphenyl |
| 1014 | 3-thiophene | H | CH₃ | m-NHC(O)NH- | cyclopropyl |
| 1015 | 2-pyridine | H | CH₃ | m-NHC(O)NH- | 2-(3-dimethylaminopropyl)methylamino-5-CF₃-phenyl |
| 1016 | 3-pyridine | H | CH₃ | m-NHC(O)NH- | 2-dimethylamino-5-CF₃-phenyl |
| 1017 | 2-CH₃-phenyl | H | -O(CH₂)₂-piperidine | m-NHC(O)NH- | 1-piperidinylpropyloxyphenyl |
| 1018 | 4-CH₃-phenyl | H | -O(CH₂)₂-piperidine | m-NHC(O)NH- | cyclopropyl |
| 1019 | 5-CH₃-phenyl | H | -O(CH₂)₂-piperidine | m-NHC(O)NH- | 2-(3-dimethylaminopropyl)methylamino-5-CF₃-phenyl |
| 1020 | 6-CH₃-phenyl | H | -O(CH₂)₂-piperidine | m-NHC(O)NH- | 2-dimethylamino-5-CF₃-phenyl |
| 1021 | 2-OCH₃-phenyl | H | -O(CH₂)₂-piperidine | m-NHC(O)NH- | 1-piperidinylpropyloxyphenyl |
| 1022 | 4-OCH₃-phenyl | H | -O(CH₂)₂-piperidine | m-NHC(O)NH- | cyclopropyl |
| 1023 | 5-OCH₃-phenyl | H | -O(CH₂)₂-piperidine | m-NHC(O)NH- | 2-(3-dimethylaminopropy1l)methylamino-5-CF₃-phenyl |
| 1024 | 6-OCH₃-phenyl | H | -O(CH₂)₂-piperidine | m-NHC(O)NH- | 2-dimethylamino-5-CF₃-phenyl |
| 1025 | 2-F-phenyl | H | -O(CH₂)₂-piperidine | m-NHC(O)NH- | 1-piperidinylpropyloxyphenyl |
| 1026 | 4-F-phenyl | H | -O(CH₂)₂-piperidine | m-NHC(O)NH- | cyclopropyl |
| 1027 | 5-F-phenyl | H | -O(CH₂)₂-piperidine | m-NHC(O)NH- | 2-(3-dimethylaminopropyl)methylamino-5-CF₃-phenyl |
| 1028 | 6-F-phenyl | H | -O(CH₂)₂-piperidine | m-NHC(O)NH- | 2-dimethylamino-5-CF₃-phenyl |
| 1029 | 2-thiophene | H | -O(CH₂)₂-piperidine | m-NHC(O)NH- | 1-piperidinylpropyloxyphenyl |
| 1030 | 3-thiophene | H | -O(CH₂)₂-piperidine | m-NHC(O)NH- | cyclopropyl |
| 1031 | 2-pyridine | H | -O(CH₂)₂-piperidine | m-NHC(O)NH- | 2-(3-dimethylaminopropy1l)methylamino-5-CF₃-phenyl |
| 1032 | 3-pyridine | H | -O(CH₂)₂-piperidine | m-NHC(O)NH- | 2-dimethylamino-5-CF₃-phenyl |
| 1033 | 2-CH₃-phenyl | H | -O(CH₂)₂-piperzine | m-NHC(O)NH- | 1-piperidinylpropyloxyphenyl |
| 1034 | 4-CH₃-phenyl | H | -O(CH₂)₂-piperzine | m-NHC(O)NH- | cyclopropyl |
| 1035 | 5-CH₃-phenyl | H | -O(CH₂)₂-piperzine | m-NHC(O)NH- | 2-(3-dimethylaminopropyl)methylamino-5-CF₃-phenyl |
| 1036 | 6-CH₃-phenyl | H | -O(CH₂)₂-piperzine | m-NHC(O)NH- | 2-dimethylamino-5-CF₃-phenyl |
| 1037 | 2-OCH₃-phenyl | H | -O(CH₂)₂-piperzine | m-NHC(O)NH- | 1-piperidinylpropyloxyphenyl |
| 1038 | 4-OCH₃-phenyl | H | -O(CH₂)₂-piperzine | m-NHC(O)NH- | cyclopropyl |
| 1039 | 5-OCH₃-phenyl | H | -O(CH₂)₂-piperzine | m-NHC(O)NH- | 2-(3-dimethylaminopropyl)methylamino-5-CF₃-phenyl |
| 1040 | 6-OCH₃-phenyl | H | -O(CH₂)₂-piperzine | m-NHC(O)NH- | 2-dimethylamino-5-CF₃-phenyl |
| 1041 | 2-F-phenyl | H | -O(CH₂)₂-piperzine | m-NHC(O)NH- | 1-piperidinylpropyloxyphenyl |
| 1042 | 4-F-phenyl | H | -O(CH₂)₂-piperzine | m-NHC(O)NH- | cyclopropyl |
| 1043 | 5-F-phenyl | H | -O(CH₂)₂-piperzine | m-NHC(O)NH- | 2-(3-dimethylaminopropyl)methylamino-5-CF₃-phenyl |
| 1044 | 6-F-phenyl | H | -O(CH₂)₂-piperzine | m-NHC(O)NH- | 2-dimethylamino-5-CF₃-phenyl |
| 1045 | 2-thiophene | H | -O(CH₂)₂-piperzine | m-NHC(O)NH- | 1-piperidinylpropyloxyphenyl |
| 1046 | 3-thiophene | H | -O(CH₂)₂-piperzine | m-NHC(O)NH- | cyclopropyl |
| 1047 | 2-pyridine | H | -O(CH₂)₂-piperzine | m-NHC(O)NH- | 2-(3-dimethylaminopropyl)methylamino-5-CF₃-phenyl |
| 1048 | 3-pyridine | H | O(CH₂)₂-piperzine | m-NHC(O)NH- | 2-dimethylamino-5-CF₃-phenyl |
| 1049 | 2-CH₃-phenyl | H | H | p-C(O)NH- | 1-piperidinylpropyloxyphenyl |
| 1050 | 4-CH₃-phenyl | H | H | p-C(O)NH- | cyclopropyl |
| 1051 | 5-CH₃-phenyl | H | H | p-C(O)NH- | 2-(3-dimethylaminopropyl)methylamino-5-CF₃-phenyl |
| 1052 | 6-CH₃-phenyl | H | H | p-C(O)NH- | 2-dimethylamino-5-CF₃-phenyl |
| 1053 | 2-OCH₃-phenyl | H | H | p-C(O)NH- | 1-piperidinylpropyloxyphenyl |
| 1054 | 4-OCH₃-phenyl | H | H | p-C(O)NH- | cyclopropyl |
| 1055 | 5-OCH₃-phenyl | H | H | p-C(O)NH- | 2-(3-dimethylaminopropyl)methylamino-5-CF₃-phenyl |
| 1056 | 6-OCH₃-phenyl | H | H | p-C(O)NH- | 2-dimethylamino-5-CF₃-phenyl |
| 1057 | 2-F-phenyl | H | H | p-C(O)NH- | 1-piperidinylpropyloxyphenyl |
| 1058 | 4-F-phenyl | H | H | p-C(O)NH- | cyclopropyl |
| 1059 | 5-F-phenyl | H | H | p-C(O)NH- | 2-(3-dimethylaminopropyl)methylamino-5-CF₃-phenyl |
| 1060 | 6-F-phenyl | H | H | p-C(O)NH- | 2-dimethylamino-5-CF₃-phenyl |
| 1061 | 2-thiophene | H | H | p-C(O)NH- | 1-piperidinylpropyloxyphenyl |
| 1062 | 3-thiophene | H | H | p-C(O)NH- | cyclopropyl |
| 1063 | 2-pyridine | H | H | p-C(O)NH- | 2-(3-dimethylaminopropyl)methylamino-5-CF₃-phenyl |
| 1064 | 3-pyridine | H | H | p-C(O)NH- | 2-dimethylamino-5-CF₃-phenyl |
| 1065 | 2-CH₃-phenyl | CH₃ | H | p-C(O)NH- | 1-piperidinylpropyloxyphenyl |
| 1066 | 4-CH₃-phenyl | CH₃ | H | p-C(O)NH- | cyclopropyl |
| 1067 | 5-CH₃-phenyl | CH₃ | H | p-C(O)NH- | 2-(3-dimethylaminopropyl)methylamino-5-CF₃-phenyl |
| 1068 | 6-CH₃-phenyl | CH₃ | H | p-C(O)NH- | 2-dimethylamino-5-CF₃-phenyl |
| 1069 | 2-OCH₃-phenyl | CH₃ | H | p-C(O)NH- | 1-piperidinylpropyloxyphenyl |
| 1070 | 4-OCH₃-phenyl | CH₃ | H | p-C(O)NH- | cyclopropyl |
| 1071 | 5-OCH₃-phenyl | CH₃ | H | p-C(O)NH- | 2-(3-dimethylaminopropyl)methylamino-5-CF₃-phenyl |
| 1072 | 6-OCH₃-phenyl | CH₃ | H | p-C(O)NH- | 2-dimethylamino-5-CF₃-phenyl |
| 1073 | 2-F-phenyl | CH₃ | H | p-C(O)NH- | 1-piperidinylpropyloxyphenyl |
| 1074 | 4-F-phenyl | CH₃ | H | p-C(O)NH- | cyclopropyl |
| 1075 | 5-F-phenyl | CH₃ | H | p-C(O)NH- | 2-(3-dimethylaminopropyl)methylamino-5-CF₃-phenyl |
| 1076 | 6-F-phenyl | CH₃ | H | p-C(O)NH- | 2-dimethylamino-5-CF₃-phenyl |
| 1077 | 2-thiophene | CH₃ | H | p-C(O)NH- | 1-piperidinylpropyloxyphenyl |
| 1078 | 3-thiophene | CH₃ | H | p-C(O)NH- | cyclopropyl |
| 1079 | 2-pyridine | CH₃ | H | p-C(O)NH- | 2-(3-dimethylaminopropyl)methylamino-5-CF₃-phenyl |
| 1080 | 3-pyridine | CH₃ | H | p-C(O)NH- | 2-dimethylamino-5-CF₃-phenyl |
| 1081 | 2-CH₃-phenyl | H | -(CH₂)₃ morpholine | p-C(O)NH- | 1-piperidinylpropylo xyphenyl |
| 1082 | 4-CH₃-phenyl | H | -(CH₂)₃ morpholine | p-C(O)NH- | cyclopropyl |
| 1083 | 5-CH₃-phenyl | H | -(CH₂)₃-morpholine | p-C(O)NH- | 2-(3-dimethylaminopropyl)methylamino-5-CF₃-phenyl |
| 1084 | 6-CH₃-phenyl | H | -(CH₂)₃ morpholine | p-C(O)NH- | 2-dimethylamino-5-CF₃-phenyl |
| 1085 | 2-OCH₃-phenyl | H | -(CH₂)₃ morpholine | p-C(O)NH- | 1-piperidinylpropyloxyphenyl |
| 1086 | 4-OCH₃-phenyl | H | -(CH₂)₃ morpholine | p-C(O)NH- | cyclopropyl |
| 1087 | 5-OCH₃-phenyl | H | -(CH₂)₃ morpholine | p-C(O)NH- | 2-(3-dimethylaminopropyl)methylamino-5-CF₃-phenyl |
| 1088 | 6-OCH₃-phenyl | H | -(CH₂)₃ morpholine | p-C(O)NH- | 2-dimethylamino-5-CF₃-phenyl |
| 1089 | 2-F-phenyl | H | -(CH₂)₃ morpholine | p-C(O)NH- | 1-piperidinylpropyloxyphenyl |
| 1090 | 4-F-phenyl | H | -(CH₂)₃morpholine | p-C(O)NH- | cyclopropyl |
| 1091 | 5-F-phenyl | H | -(CH₂)₃-morpholine | p-C(O)NH- | 2-(3-dimethylaminopropyl)methylamino-5-CF₃-phenyl |
| 1092 | 6-F-phenyl | H | -(CH₂)₃ morpholine | p-C(O)NH- | 2-dimethylamino-5-CF₃-phenyl |
| 1093 | 2-thiophene | H | -(CH₂)₃ morpholine | p-C(O)NH- | 1-piperidinylpropyloxyphenyl |
| 1094 | 3-thiophene | H | -(CH₂)₃ morpholine | p-C(O)NH- | cyclopropyl |
| 1095 | 2-pyridine | H | -(CH₂)₃-morpholine | p-C(O)NH- | 2-(3-dimethylaminopropyl)methylamino-5-CF₃-phenyl |
| 1096 | 3-pyridine | H | -(CH₂)₃-morpholine | p-C(O)NH- | 2-dimethylamino-5-CF₃-phenyl |
| 1097 | 2-CH₃-phenyl | H | CH₃ | p-C(O)NH- | 1-piperidinylpropyloxyphenyl |
| 1098 | 4-CH₃-phenyl | H | CH₃ | p-C(O)NH- | cyclopropyl |
| 1099 | 5-CH₃-phenyl | H | CH₃ | p-C(O)NH- | 2-(3-dimethylaminopropyl)methylamino-5-CF₃-phenyl |
| 1100 | 6-CH₃-phenyl | H | CH₃ | p-C(O)NH- | 2-dimethylamino-5-CF₃-phenyl |
| 1101 | 2-OCH₃-phenyl | H | CH₃ | p-C(O)NH- | 1-piperidinylpropyloxyphenyl |
| 1102 | 4-OCH₃-phenyl | H | CH₃ | p-C(O)NH- | cyclopropyl |
| 1103 | 5-OCH₃-phenyl | H | CH₃ | p-C(O)NH- | 2-(3-dimethylaminopropyl)methylamino-5-CF₃-phenyl |
| 1104 | 6-OCH₃-phenyl | H | CH₃ | p-C(O)NH- | 2-dimethylamino-5-CF₃-phenyl |
| 1105 | 2-F-phenyl | H | CH₃ | p-C(O)NH- | 1-piperidinylpropyloxyphenyl |
| 1106 | 4-F-phenyl | H | CH₃ | p-C(O)NH- | cyclopropyl |
| 1107 | 5-F-phenyl | H | CH₃ | p-C(O)NH- | 2-(3-dimethylaminopropyl)methylamino-5-CF₃-phenyl |
| 1108 | 6-F-phenyl | H | CH₃ | p-C(O)NH- | 2-dimethylamino-5-CF₃-phenyl |
| 1109 | 2-thiophene | H | CH₃ | p-C(O)NH- | 1-piperidinylpropyloxyphenyl |
| 1110 | 3-thiophene | H | CH₃ | p-C(O)NH- | cyclopropyl |
| 1111 | 2-pyridine | H | CH₃ | p-C(O)NH- | 2-(3-dimethylaminopropyl)methylamino-5-CF₃-phenyl |
| 1112 | 3-pyridine | H | CH₃ | p-C(O)NH- | 2-dimethylamino-5-CF₃-phenyl |
| 1113 | 2-CH₃-phenyl | H | -O(CH₂)₂-piperidine | p-C(O)NH- | 1-piperidinylpropyloxyphenyl |
| 1114 | 4-CH₃-phenyl | H | -O(CH₂)₂-piperidine | p-C(O)NH- | cyclopropyl |
| 1115 | 5-CH₃-phenyl | H | -O(CH₂)₂-piperidine | p-C(O)NH- | 2-(3-dimethylaminopropyl)methylamino-5-CF₃-phenyl |
| 1116 | 6-CH₃-phenyl | H | -O(CH₂)₂-piperidine | p-C(O)NH- | 2-dimethylamino-5-CF₃-phenyl |
| 1117 | 2-OCH₃-phenyl | H | -O(CH₂)₂-piperidine | p-C(O)NH- | 1-piperidinylpropyloxyphenyl |
| 1118 | 4-OCH₃-phenyl | H | -O(CH₂)₂-piperidine | p-C(O)NH- | cyclopropyl |
| 1119 | 5-OCH₃-phenyl | H | -O(CH₂)₂-piperidine | p-C(O)NH- | 2-(3-dimethylaminopropyl)methylamino-5-CF₃-phenyl |
| 1120 | 6-OCH₃-phenyl | H | -O(CH₂)₂-piperidine | p-C(O)NH- | 2-dimethylamino-5-CF₃-phenyl |
| 1121 | 2-F-phenyl | H | -O(CH₂)₂-piperidine | p-C(O)NH- | 1-piperidinylpropyloxyphenyl |
| 1122 | 4-F-phenyl | H | -O(CH₂)₂-piperidine | p-C(O)NH- | cyclopropyl |
| 1123 | 5-F-phenyl | H | -O(CH₂)₂-piperidine | p-C(O)NH- | 2-(3-dimethylaminopropyl)methylamino-5-CF₃-phenyl |
| 1124 | 6-F-phenyl | H | -O(CH₂)₂-piperidine | p-C(O)NH- | 2-dimethylamino-5-CF₃-phenyl |
| 1125 | 2-thiophene | H | O(CH₂)₂-piperidine | p-C(O)NH- | 1-piperidinylpropyloxyphenyl |
| 1126 | 3-thiophene | H | -O(CH₂)₂-piperidine | p-C(O)NH- | cyclopropyl |
| 1127 | 2-pyridine | H | -O(CH₂)₂-piperidine | p-C(O)NH- | 2-(3-dimethylaminopropyl)methylamino-5-CF₃-phenyl |
| 1128 | 3-pyridine | H | -O(CH₂)₂-piperidine | p-C(O)NH- | 2-dimethylamino-5-CF₃-phenyl |
| 1129 | 2-CH₃-phenyl | H | -O(CH₂)₂-piperzine | p-C(O)NH- | 1-piperidinylpropyloxyphenyl |
| 1130 | 4-CH₃-phenyl | H | -O(CH₂)₂-piperzine | p-C(O)NH- | cyclopropyl |
| 1131 | 5-CH₃-phenyl | H | -O(CH₂)₂-piperzine | p-C(O)NH- | 2-(3-dimethylaminopropyl)methylamino-5-CF₃-phenyl |
| 1132 | 6-CH₃-phenyl | H | -O(CH₂)₂-piperzine | p-C(O)NH- | 2-dimethylamino-5-CF₃-phenyl |
| 1133 | 2-OCH₃-phenyl | H | -O(CH₂)₂-piperzine | p-C(O)NH- | 1-piperidinylpropyloxyphenyl |
| 1134 | 4-OCH₃-phenyl | H | -O(CH₂)₂-piperzine | p-C(O)NH- | cyclopropyl |
| 1135 | 5-OCH₃-phenyl | H | -O(CH₂)₂-piperzine | p-C(O)NH- | 2-(3-dimethylaminopropyl)methylamino-5-CF₃-phenyl |
| 1136 | 6-OCH₃-phenyl | H | -O(CH₂)₂-piperzine | p-C(O)NH- | 2-dimethylamino-5-CF₃-phenyl |
| 1137 | 2-F-phenyl | H | -O(CH₂)₂-piperzine | p-C(O)NH- | 1-piperidinylpropyloxyphenyl |
| 1138 | 4-F-phenyl | H | -O(CH₂)₂-piperzine | p-C(O)NH- | cyclopropyl |
| 1139 | 5-F-phenyl | H | -O(CH₂)₂-piperzine | p-C(O)NH- | 2-(3-dimethylaminopropyl)methylamino-5-CF₃-phenyl |
| 1140 | 6-F-phenyl | H | -O(CH₂)₂-piperzine | p-C(O)NH- | 2-dimethylamino-5-CF₃-phenyl |
| 1141 | 2-thiophene | H | -O(CH₂)₂-piperzine | p-C(O)NH- | 1-piperidinylpropyloxyphenyl |
| 1142 | 3-thiophene | H | -O(CH₂)₂-piperzine | p-C(O)NH- | cyclopropyl |
| 1143 | 2-pyridine | H | -O(CH₂)₂-piperzine | p-C(O)NH- | 2-(3-dimethylaminopropyl)methylamino-5-CF₃-phenyl |
| 1144 | 3-pyridine | H | -O(CH₂)₂-piperzine | p-C(O)NH- | 2-dimethylamino-5-CF₃-phenyl |
| 1145 | 2-CH₃-phenyl | H | 1-pyrrolidinylethyl | p-C(O)NH- | 1-piperidinylpropyloxyphenyl |
| 1146 | 4-CH₃-phenyl | H | 1-pyrrolidinylethyl | p-C(O)NH- | cyclopropyl |
| 1147 | 5-CH₃-phenyl | H | 1-pyrrolidinylethyl | p-C(O)NH- | 2-(3-dimethylaminopropyl)methylamino-5-CF₃-phenyl |
| 1148 | 6-CH₃-phenyl | H | 1-pyrrolidinylethyl | p-C(O)NH- | 2-dimethylamino-5-CF₃-phenyl |
| 1149 | 2-OCH₃-phenyl | H | 1-pyrrolidinylethyl | p-C(O)NH- | 1-piperidinylpropyloxyphenyl |
| 1150 | 4-OCH₃-phenyl | H | 1-pyrrolidinylethyl | p-C(O)NH- | cyclopropyl |
| 1151 | 5-OCH₃-phenyl | H | 1-pyrrolidinylethyl | p-C(O)NH- | 2-(3-dimethylaminopropyl)methylamino-5-CF₃-phenyl |
| 1152 | 6-OCH₃-phenyl | H | 1-pyrrolidinylethyl | p-C(O)NH- | 2-dimethylamino-5-CF₃-phenyl |
| 1153 | 2-F-phenyl | H | 1-pyrrolidinylethyl | p-C(O)NH- | 1-piperidinylpropyloxyphenyl |
| 1154 | 4-F-phenyl | H | 1-pyrrolidinylethyl | p-C(O)NH- | cyclopropyl |
| 1155 | 5-F-phenyl | H | 1-pyrrolidinylethyl | p-C(O)NH- | 2-(3-dimethylaminopropyl)methylamino-5-CF₃-phenyl |
| 1156 | 6-F-phenyl | H | 1-pyrrolidinylethyl | p-C(O)NH- | 2-dimethylamino-5-CF₃-phenyl |
| 1157 | 2-thiophene | H | 1-pyrrolidinylethyl | p-C(O)NH- | 1-piperidinylpropyloxyphenyl |
| 1158 | 3-thiophene | H | 1-pyrrolidinylethyl | p-C(O)NH- | cyclopropyl |
| 1159 | 2-pyridine | H | 1-pyrrolidinylethyl | p-C(O)NH- | 2-(3-dimethylaminopropyl)methylamino-5-CF₃-phenyl |
| 1160 | 3-pyridine | H | 1-pyrrolidinylethyl | p-C(O)NH- | 2-dimethylamino-5-CF₃-phenyl |
| 1161 | 2-CH₃-phenyl | H | H | m-NH-C(O)- | 1-piperidinylpropyloxyphenyl |
| 1162 | 4-CH₃-phenyl | H | H | m-NH-C(O)- | cyclopropyl |
| 1163 | 5-CH₃-phenyl | H | H | m-NH-C(O)- | 2-(3-dimethylaminopropyl)methylamino-5-CF₃-phenyl |
| 1164 | 6-CH₃-phenyl | H | H | m-NH-C(O)- | 2-dimethylamino-5-CF₃-phenyl |
| 1165 | 2-OCH₃-phenyl | H | H | m-NH-C(O)- | 1-piperidinylpropyloxyphenyl |
| 1166 | 4-OCH₃-phenyl | H | H | m-NH-C(O)- | cyclopropyl |
| 1167 | 5-OCH₃-phenyl | H | H | m-NH-C(O)- | 2-(3-dimethylaminopropyl)methylamino-5-CF₃-phenyl |
| 1168 | 6-OCH₃-phenyl | H | H | m-NH-C(O)- | 2-dimethylamino-5-CF₃-phenyl |
| 1169 | 2-F-phenyl | H | H | m-NH-C(O)- | 1-piperidinylpropyloxyphenyl |
| 1170 | 4-F-phenyl | H | H | m-NH-C(O)- | cyclopropyl |
| 1171 | 5-F-phenyl | H | H | m-NH-C(O)- | 2-(3-dimethylaminopropyl)methylamino-5-CF₃-phenyl |
| 1172 | 6-F-phenyl | H | H | m-NH-C(O)- | 2-dimethylamino-5-CF₃-phenyl |
| 1173 | 2-thiophene | H | H | m-NH-C(O)- | 1-piperidinylpropyloxyphenyl |
| 1174 | 3-thiophene | H | H | m-NH-C(O)- | cyclopropyl |
| 1175 | 2-pyridine | H | H | m-NH-C(O)- | 2-(3-dimethylaminopropyl)methylamino-5-CF₃-phenyl |
| 1176 | 3-pyridine | H | H | m-NH-C(O)- | 2-dimethylamino-5-CF₃-phenyl |
| 1177 | 2-CH₃-phenyl | CH₃ | H | m-NH-C(O)- | 1-piperidinylpropyloxyphenyl |
| 1178 | 4-CH₃-phenyl | CH₃ | H | m-NH-C(O)- | cyclopropyl |
| 1179 | 5-CH₃-phenyl | CH₃ | H | m-NH-C(O)- | 2-(3-dimethylaminopropyl)methylamino-5-CF₃-phenyl |
| 1180 | 6-CH₃-phenyl | CH₃ | H | m-NH-C(O)- | 2-dimethylamino-5-CF₃-phenyl |
| 1181 | 2-OCH₃-phenyl | CH₃ | H | m-NH-C(O)- | 1-piperidinylpropyloxyphenyl |
| 1182 | 4-OCH₃-phenyl | CH₃ | H | m-NH-C(O)- | cyclopropyl |
| 1183 | 5-OCH₃-phenyl | CH₃ | H | m-NH-C(O)- | 2-(3-dimethylaminopropyl)methylamino-5-CF₃-phenyl |
| 1184 | 6-OCH₃-phenyl | CH₃ | H | m-NH-C(O)- | 2-dimethylamino-5-CF₃-phenyl |
| 1185 | 2-F-phenyl | CH₃ | H | m-NH-C(O)- | 1-piperidinylpropyloxyphenyl |
| 1186 | 4-F-phenyl | CH₃ | H | m-NH-C(O)- | cyclopropyl |
| 1187 | 5-F-phenyl | CH₃ | H | m-NH-C(O)- | 2-(3-dimethylaminopropyl)methylamino-5-CF₃-phenyl |
| 1188 | 6-F-phenyl | CH₃ | H | m-NH-C(O)- | 2-dimethylamino-5-CF₃-phenyl |
| 1189 | 2-thiophene | CH₃ | H | m-NH-C(O)- | 1-piperidinylpropyloxyphenyl |
| 1190 | 3-thiophene | CH₃ | H | m-NH-C(O)- | cyclopropyl |
| 1191 | 2-pyridine | CH₃ | H | m-NH-C(O)- | 2-(3-dimethylaminopropyl)methylamino-5-CF₃-phenyl |
| 1192 | 3-pyridine | CH₃ | H | m-NH-C(O)- | 2-dimethylamino-5-CF₃-phenyl |
| 1193 | 2-CH₃-phenyl | H | -(CH₂)₃ morpholine | m-NH-C(O)- | 1-piperidinylpropyloxyphenyl |
| 1194 | 4-CH₃-phenyl | H | -(CH₂)₃-morpholine | m-NH-C(O)- | cyclopropyl |
| 1195 | 5-CH₃-phenyl | H | -(CH₂)₃ morpholine | m-NH-C(O)- | 2-(3-dimethylaminopropyl)methylamino-5-CF₃-phenyl |
| 1196 | 6-CH₃-phenyl | H | -(CH₂)₃-morpholine | m-NH-C(O)- | 2-dimethylamino-5-CF₃-phenyl |
| 1197 | 2-OCH₃-phenyl | H | -(CH₂)₃-morpholine | m-NH-C(O)- | 1-piperidinylpropyloxyphenyl |
| 1198 | 4-OCH₃-phenyl | H | -(CH₂)₃ morpholine | m-NH-C(O)- | cyclopropyl |
| 1199 | 5-OCH₃-phenyl | H | -(CH₂)₃ morpholine | m-NH-C(O)- | 2-(3-dimethylaminopropyl)methylamino-5-CF₃-phenyl |
| 1200 | 6-OCH₃-phenyl | H | -(CH₂)₃-morpholine | m-NH-C(O)- | 2-dimethylamino-5-CF₃-phenyl |
| 1201 | 2-F-phenyl | H | -(CH₂)₃-morpholine | m-NH-C(O)- | 1-piperidinylpropyloxyphenyl |
| 1202 | 4-F-phenyl | H | -(CH₂)₃-morpholine | m-NH-C(O)- | cyclopropyl |
| 1203 | 5-F-phenyl | H | -(CH₂)₃ morpholine | m-NH-C(O)- | 2-(3-dimethylaminopropyl)methylamino-5-CF₃-phenyl |
| 1204 | 6-F-phenyl | H | -(CH₂)₃-morpholine | m-NH-C(O)- | 2-dimethylamino-5-CF₃-phenyl |
| 1205 | 2-thiophene | H | -(CH₂)₃-morpholine | m-NH-C(O)- | 1-piperidinylpropyloxyphenyl |
| 1206 | 3-thiophene | H | -(CH₂)₃ morpholine | m-NH-C(O)- | cyclopropyl |
| 1207 | 2-pyridine | H | -(CH₂)₃ morpholine | m-NH-C(O)- | 2-(3-dimethylaminopropyl)methylamino-5-CF₃-phenyl |
| 1208 | 3-pyridine | H | -(CH₂)₃ morpholine | m-NH-C(O)- | 2-dimethylamino-5-CF₃-phenyl |
| 1209 | 2-CH₃-phenyl | H | CH₃ | m-NH-C(O)- | 1-piperidinylpropyloxyphenyl |
| 1210 | 4-CH₃-phenyl | H | CH₃ | m-NH-C(O)- | cyclopropyl |
| 1211 | 5-CH₃-phenyl | H | CH₃ | m-NH-C(O)- | 2-(3-dimethylaminopropyl)methylamino-5-CF₃-phenyl |
| 1212 | 6-CH₃-phenyl | H | CH₃ | m-NH-C(O)- | 2-dimethylamino-5-CF₃-phenyl |
| 1213 | 2-OCH₃-phenyl | H | CH₃ | m-NH-C(O)- | 1-piperidinylpropyloxyphenyl |
| 1214 | 4-OCH₃-phenyl | H | CH₃ | m-NH-C(O)- | cyclopropyl |
| 1215 | 5-OCH₃-phenyl | H | CH₃ | m-NH-C(O)- | 2-(3-dimethylaminopropyl)methylamino-5-CF₃-phenyl |
| 1216 | 6-OCH₃-phenyl | H | CH₃ | m-NH-C(O)- | 2-dimethylamino-5-CF₃-phenyl |
| 1217 | 2-F-phenyl | H | CH₃ | m-NH-C(O)- | 1-piperidinylpropyloxyphenyl |
| 1218 | 4-F-phenyl | H | CH₃ | m-NH-C(O)- | cyclopropyl |
| 1219 | 5-F-phenyl | H | CH₃ | m-NH-C(O)- | 2-(3-dimethylaminopropyl)methylamino-5-CF₃-phenyl |
| 1220 | 6-F-phenyl | H | CH₃ | m-NH-C(O)- | 2-dimethylamino-5-CF₃-phenyl |
| 1221 | 2-thiophene | H | CH₃ | m-NH-C(O)- | 1-piperidinylpropyloxyphenyl |
| 1222 | 3-thiophene | H | CH₃ | m-NH-C(O)- | cyclopropyl |
| 1223 | 2-pyridine | H | CH₃ | m-NH-C(O)- | 2-(3-dimethylaminopropyl)methylamino-5-CF₃-phenyl |
| 1224 | 3-pyridine | H | CH₃ | m-NH-C(O)- | 2-dimethylamino-5-CF₃-phenyl |
| 1225 | 2-CH₃-phenyl | H | -O(CH₂)₂-piperidine | m-NH-C(O)- | 1-piperidinylpropyloxyphenyl |
| 1226 | 4-CH₃-phenyl | H | -O(CH₂)₂-piperidine | m-NH-C(O)- | cyclopropyl |
| 1227 | 5-CH₃-phenyl | H | -O(CH₂)₂-piperidine | m-NH-C(O)- | 2-(3-dimethylaminopropyl)methylamino-5-CF₃-phenyl |
| 1228 | 6-CH₃-phenyl | H | -O(CH₂)₂-piperidine | m-NH-C(O)- | 2-dimethylamino-5-CF₃-phenyl |
| 1229 | 2-OCH₃-phenyl | H | -O(CH₂)₂-piperidine | m-NH-C(O)- | 1-piperidinylpropyloxyphenyl |
| 1230 | 4-OCH₃-phenyl | H | -O(CH₂)₂-piperidine | m-NH-C(O)- | cyclopropyl |
| 1231 | 5-OCH₃-phenyl | H | -O(CH₂)₂-piperidine | m-NH-C(O)- | 2-(3-dimethylaminopropyl)methylamino-5-CF₃-phenyl |
| 1232 | 6-OCH₃-phenyl | H | -O(CH₂)₂-piperidine | m-NH-C(O)- | 2-dimethylamino-5-CF₃-phenyl |
| 1233 | 2-F-phenyl | H | -O(CH₂)₂-piperidine | m-NH-C(O)- | 1-piperidinylpropyloxyphenyl |
| 1234 | 4-F-phenyl | H | -O(CH₂)₂-piperidine | m-NH-C(O)- | cyclopropyl |
| 1235 | 5-F-phenyl | H | -O(CH₂)₂-piperidine | m-NH-C(O)- | 2-(3-dimethylaminopropyl)methylamino-5-CF₃-phenyl |
| 1236 | 6-F-phenyl | H | -O(CH₂)₂-piperidine | m-NH-C(O)- | 2-dimethylamino-5-CF₃-phenyl |
| 1237 | 2-thiophene | H | -O(CH₂)₂-piperidine | m-NH-C(O)- | 1-piperidinylpropyloxyphenyl |
| 1238 | 3-thiophene | H | -O(CH₂)₂-piperidine | m-NH-C(O)- | cyclopropyl |
| 1239 | 2-pyridine | H | -O(CH₂)₂-piperidine | m-NH-C(O)- | 2-(3-dimethylaminopropyl)methylamino-5-CF₃-phenyl |
| 1240 | 3-pyridine | H | -O(CH₂)₂-piperidine | m-NH-C(O)- | 2-dimethylamino-5-CF₃-phenyl |
| 1241 | 2-CH₃-phenyl | H | -O(CH₂)₂-piperzine | m-NH-C(O)- | 1-piperidinylpropyloxyphenyl |
| 1242 | 4-CH₃-phenyl | H | -O(CH₂)₂-piperzine | m-NH-C(O)- | cyclopropyl |
| 1243 | 5-CH₃-phenyl | H | -O(CH₂)₂-piperzine | m-NH-C(O)- | 2-(3-dimethylaminopropyl)methylamino-5-CF₃-phenyl |
| 1244 | 6-CH₃-phenyl | H | -O(CH₂)₂-piperzine | m-NH-C(O)- | 2-dimethylamino-5-CF₃-phenyl |
| 1245 | 2-OCH₃-phenyl | H | -O(CH₂)₂-piperzine | m-NH-C(O)- | 1-piperidinylpropyloxyphenyl |
| 1246 | 4-OCH₃-phenyl | H | -O(CH₂)₂-piperzine | m-NH-C(O)- | cyclopropyl |
| 1247 | 5-OCH₃-phenyl | H | -O(CH₂)₂-piperzine | m-NH-C(O)- | 2-(3-dimethylaminopropyl)methylamino-5-CF₃-phenyl |
| 1248 | 6-OCH₃-phenyl | H | -O(CH₂)₂-piperzine | m-NH-C(O)- | 2-dimethylamino-5-CF₃-phenyl |
| 1249 | 2-F-phenyl | H | -O(CH₂)₂-piperzine | m-NH-C(O)- | 1-piperidinylpropyloxyphenyl |
| 1250 | 4-F-phenyl | H | -O(CH₂)₂-piperzine | m-NH-C(O)- | cyclopropyl |
| 1251 | 5-F-phenyl | H | -O(CH₂)₂-piperzine | m-NH-C(O)- | 2-(3-dimethylaminopropyl)methylamino-5-CF₃-phenyl |
| 1252 | 6-F-phenyl | H | -O(CH₂)₂-piperzine | m-NH-C(O)- | 2-dimethylamino-5-CF₃-phenyl |
| 1253 | 2-thiophene | H | -O(CH₂)₂-piperzine | m-NH-C(O)- | 1-piperidinylpropyloxyphenyl |
| 1254 | 3-thiophene | H | -O(CH₂)₂-piperzine | m-NH-C(O)- | cyclopropyl |
| 1255 | 2-pyridine | H | -O(CH₂)₂-piperzine | m-NH-C(O)- | 2-(3-dimethylaminopropyl)methylamino-5-CF₃-phenyl |
| 1256 | 3-pyridine | H | -O(CH₂)₂-piperzine | m-NH-C(O)- | 2-dimethylamino-5-CF₃-phenyl |
| 1257 | 2-CH₃-phenyl | H | 1-pyrrolidinylethyl | m-NH-C(O)- | 1-piperidinylpropyloxyphenyl |
| 1258 | 4-CH₃-phenyl | H | 1-pyrrolidinylethyl | m-NH-C(O)- | cyclopropyl |
| 1259 | 5-CH₃-phenyl | H | 1-pyrrolidinylethyl | m-NH-C(O)- | 2-(3-dimethylaminopropyl)methylamino-5-CF₃-phenyl |
| 1260 | 6-CH₃-phenyl | H | 1-pyrrolidinylethyl | m-NH-C(O)- | 2-dimethylamino-5-CF₃-phenyl |
| 1261 | 2-OCH₃-phenyl | H | 1-pyrrolidinylethyl | m-NH-C(O)- | 1-piperidinylpropyloxyphenyl |
| 1262 | 4-OCH₃-phenyl | H | 1-pyrrolidinylethyl | m-NH-C(O)- | cyclopropyl |
| 1263 | 5-OCH₃-phenyl | H | 1-pyrrolidinylethyl | m-NH-C(O)- | 2-(3-dimethylaminopropyl)methylamino-5-CF₃-phenyl |
| 1264 | 6-OCH₃-phenyl | H | 1-pyrrolidinylethyl | m-NH-C(O)- | 2-dimethylamino-5-CF₃-phenyl |
| 1265 | 2-F-phenyl | H | 1-pyrrolidinylethyl | m-NH-C(O)- | 1-piperidinylpropyloxyphenyl |
| 1266 | 4-F-phenyl | H | 1-pyrrolidinylethyl | m-NH-C(O)- | cyclopropyl |
| 1267 | 5-F-phenyl | H | 1-pyrrolidinylethyl | m-NH-C(O)- | 2-(3-dimethylaminopropyl)methylamino-5-CF₃-phenyl |
| 1268 | 6-F-phenyl | H | 1-pyrrolidinylethyl | m-NH-C(O)- | 2-dimethylamino-5-CF₃-phenyl |
| 1269 | 2-thiophene | H | 1-pyrrolidinylethyl | m-NH-C(O)- | 1-piperidinylpropyloxyphenyl |
| 1270 | 3-thiophene | H | 1-pyrrolidinylethyl | m-NH-C(O)- | cyclopropyl |
| 1271 | 2-pyridine | H | 1-pyrrolidinylethyl | m-NH-C(O)- | 2-(3-dimethylaminopropyl)methylamino-5-CF₃-phenyl |
| 1272 | 3-pyridine | H | 1-pyrrolidinylethyl | m-NH-C(O)- | 2-dimethylamino-5-CF₃-phenyl |
| 1273 | 2-CH₃-phenyl | H | H | m-C(O)NH- | 1,1-dimethylethyl-3-oxazole |
| 1274 | 4-CH₃-phenyl | H | H | m-C(O)NH- | 4-OCF₃-phenyl |
| 1275 | 5-CH₃-phenyl | H | H | m-C(O)NH- | 1,1-dimethylethyl-1-methyl-1H-pyrazole |
| 1276 | 6-CH₃-phenyl | H | H | m-C(O)NH- | 3,4-dimethyl-5-isoxazole |
| 1277 | 2-OCH₃-phenyl | H | H | m-C(O)NH- | 1,1-dimethylethyl-3-oxazole |
| 1278 | 4-OCH₃-phenyl | H | CH | m-C(O)NH- | 4-OCF₃-phenyl |
| 1279 | 5-OCH₃-phenyl | H | H | m-C(O)NH- | 1,1-dimethylethyl-1-methyl-1H-pyrazole |
| 1280 | 6-OCH₃-phenyl | H | H | m-C(O)NH- | 3,4-dimethyl-5-isoxazole |
| 1281 | 2-F-phenyl | H | H | m-C(O)NH- | 1,1-dimethylethyl-3-oxazole |
| 1282 | 4-F-phenyl | H | H | m-C(O)NH- | 4-OCF₃-phenyl |
| 1283 | 5-F-phenyl | H | H | m-C(O)NH- | 1,1-dimethylethyl-1-methyl-1H-pyrazole |
| 1284 | 6-F-phenyl | H | H | m-C(O)NH- | 3,4-dimethyl-5-isoxazole |
| 1285 | 2-thiophene | H | H | m-C(O)NH- | 1,1-dimethylethyl-3-oxazole |
| 1286 | 3-thiophene | H | H | m-C(O)NH- | 4-OCF₃-phenyl |
| 1287 | 2-pyridine | H | H | m-C(O)NH- | 1,1-dimethylethyl-1-methyl-1H-pyrazole |
| 1288 | 3-pyridine | H | H | m-C(O)NH- | 3,4-dimethyl-5-isoxazole |
| 1289 | 2-CH₃-phenyl | CH₃ | H | m-C(O)NH- | 1,1-dimethylethyl-3-oxazole |
| 1290 | 4-CH₃-phenyl | CH₃ | H | m-C(O)NH- | 4-OCF₃-phenyl |
| 1291 | 5-CH₃-phenyl | CH₃ | H | m-C(O)NH- | 1,1-dimethylethyl-1-methyl-1H-pyrazole |
| 1292 | 6-CH₃-phenyl | CH₃ | H | m-C(O)NH- | 3,4-dimethyl-5-isoxazole |
| 1293 | 2-OCH₃-phenyl | CH₃ | H | m-C(O)NH- | 1,1-dimethylethyl-3-oxazole |
| 1294 | 4-OCH₃-phenyl | CH₃ | H | m-C(O)NH- | 4-OCF₃-phenyl |
| 1295 | 5-OCH₃-phenyl | CH₃ | H | m-C(O)NH- | 1,1-dimethylethyl-1-methyl-1H-pyrazole |
| 1296 | 6-OCH₃-phenyl | CH₃ | H | m-C(O)NH- | 3,4-dimethyl-5-isoxazole |
| 1297 | 2-F-phenyl | CH₃ | H | m-C(O)NH- | 1,1-dimethylethyl-3-oxazole |
| 1298 | 4-F-phenyl | CH₃ | H | m-C(O)NH- | 4-OCF₃-phenyl |
| 1299 | 5-F-phenyl | CH₃ | H | m-C(O)NH- | 1,1-dimethylethyl-1-methyl-1H-pyrazole |
| 1300 | 6-F-phenyl | CH₃ | H | m-C(O)NH- | 3,4-dimethyl-5-isoxazole |
| 1301 | 2-thiophene | CH₃ | H | m-C(O)NH- | 1,1-dimethylethyl-3-oxazole |
| 1302 | 3-thiophene | CH₃ | H | m-C(O)NH- | 4-OCF₃-phenyl |
| 1303 | 2-pyridine | CH₃ | H | m-C(O)NH- | 1,1-dimethylethyl-1-methyl-1H-pyrazole |
| 1304 | 3-pyridine | CH₃ | H | m-C(O)NH- | 3,4-dimethyl-5-isoxazole |
| 1305 | 2-CH₃-phenyl | H | -(CH₂)₃-morpholine | m-C(O)NH- | 1,1-dimethylethyl-3-oxazole |
| 1306 | 4-CH₃-phenyl | H | -(CH₂)₃-morpholine | m-C(O)NH- | 4-OCF₃-phenyl |
| 1307 | 5-CH₃-phenyl | H | -(CH₂)₃-morpholine | m-C(O)NH- | 1,1-dimethylethyl-1-methyl-1H-pyrazole |
| 1308 | 6-CH₃-phenyl | H | -(CH₂)₃-morpholine | m-C(O)NH- | 3,4-dimethyl-5-isoxazole |
| 1309 | 2-OCH₃-phenyl | H | -(CH2)₃--morpholine | m-C(O)NH- | 1,1-dimethylethyl-3-oxazole |
| 1310 | 4-OCH₃-phenyl | H | -(CH₂)₃-morpholine | m-C(O)NH- | 4-OCF₃-phenyl |
| 1311 | 5-OCH₃-phenyl | H | -(CH₂)₃-morpholine | m-C(O)NH- | 1,1-dimethylethyl-1-methyl-1H-pyrazole |
| 1312 | 6-OCH₃-phenyl | H | -(CH₂)₃-morpholine | m-C(O)NH- | 3,4-dimethyl-5-isoxazole |
| 1313 | 2-F-phenyl | H | -(CH₂)₃-morpholine | m-C(O)NH- | 1,1-dimethylethyl-3-oxazole |
| 1314 | 4-F-phenyl | H | -(CH₂)₃-morpholine | m-C(O)NH- | 4-OCF₃-phenyl |
| 1315 | 5-F-phenyl | H | -(CH₂)₃-morpholine | m-C(O)NH- | 1,1-dimethylethyl-1-methyl-1H-pyrazole |
| 1316 | 6-F-phenyl | H | -(CH₂)₃-morpholine | m-C(O)NH- | 3,4-dimethyl-5-isoxazole |
| 1317 | 2-thiophene | H | -(CH₂)₃-morpholine | m-C(O)NH- | 1,1-dimethylethyl-3-oxazole |
| 1318 | 3-thiophene | H | -(CH₂)₃-morpholine | m-C(O)NH- | 4-OCF₃-phenyl |
| 1319 | 2-pyridine | H | -(CH₂)₃-morpholine | m-C(O)NH- | 1,1-dimethylethyl-1-methyl-1H-pyrazole |
| 1320 | 3-pyridine | H | -(CH₂)₃-morpholine | m-C(O)NH- | 3,4-dimethyl-5-isoxazole |
| 1321 | 2-CH₃-phenyl | H | CH₃ | m-C(O)NH- | 1,1-dimethylethyl-3-oxazole |
| 1322 | 4-CH₃-phenyl | H | CH₃ | m-C(O)NH- | 4-OCF₃-phenyl |
| 1323 | 5-CH₃-phenyl | H | CH₃ | m-C(O)NH- | 1,1-dimethylethyl-1-methyl-1H-pyrazole |
| 1324 | 6-CH₃-phenyl | H | CH₃ | m-C(O)NH- | 3,4-dimethyl-5-isoxazole |
| 1325 | 2-OCH₃-phenyl | H | CH₃ | m-C(O)NH- | 1,1-dimethylethyl-3-oxazole |
| 1326 | 4-OCH₃-phenyl | H | CH₃ | m-C(O)NH- | 4-OCF₃-phenyl |
| 1327 | 5-OCH₃-phenyl | H | CH₃ | m-C(O)NH- | 1,1-dimethylethyl-1-methyl-1H-pyrazole |
| 1328 | 6-OCH₃-phenyl | H | CH₃ | m-C(O)NH- | 3,4-dimethyl-5-isoxazole |
| 1329 | 2-F-phenyl | H | CH₃ | m-C(O)NH- | 1,1-dimethylethyl-3-oxazole |
| 1330 | 4-F-phenyl | H | CH₃ | m-C(O)NH- | 4-OCF₃-phenyl |
| 1331 | 5-F-phenyl | H | CH₃ | m-C(O)NH- | 1,1-dimethylethyl-1-methyl-1H-pyrazole |
| 1332 | 6-F-phenyl | H | CH₃ | m-C(O)NH- | 3,4-dimethyl-5-isoxazole |
| 1333 | 2-thiophene | H | CH₃ | m-C(O)NH- | 1,1-dimethylethyl-3-oxazole |
| 1334 | 3-thiophene | H | CH₃ | m-C(O)NH- | 4-OCF₃-phenyl |
| 1335 | 2-pyridine | H | CH₃ | m-C(O)NH- | 1,1-dimethylethyl-1-methyl-1H-pyrazole |
| 1336 | 3-pyridine | H | CH₃ | m-C(O)NH- | 3,4-dimethyl-5-isoxazole |
| 1337 | 2-CH₃-phenyl | H | -O(CH₂)₂-piperidine | m-C(O)NH- | 1,1-dimethylethyl-3-oxazole |
| 1338 | 4-CH₃-phenyl | H | -O(CH₂)₂-piperidine | m-C(O)NH- | 4-OCF₃-phenyl |
| 1339 | 5-CH₃-phenyl | H | -O(CH₂)₂-piperidine | m-C(O)NH- | 1,1-dimethylethyl-1-methyl-1H-pyrazole |
| 1340 | 6-CH₃-phenyl | H | -O(CH₂)₂-piperidine | m-C(O)NH- | 3,4-dimethyl-5-isoxazole |
| 1341 | 2-OCH₃-phenyl | H | -O(CH₂)₂-piperidine | m-C(O)NH- | 1,1-dimethylethyl-3-oxazole |
| 1342 | 4-OCH₃-phenyl | H | -O(CH₂)₂-piperidine | m-C(O)NH- | 4-OCF₃-phenyl |
| 1343 | 5-OCH₃-phenyl | H | -O(CH₂)₂-piperidine | m-C(O)NH- | 1,1-dimethylethyl-1-methyl-1H-pyrazole |
| 1344 | 6-OCH₃-phenyl | H | -O(CH₂)₂-piperidine | m-C(O)NH- | 3,4-dimethyl-5-isoxazole |
| 1345 | 2-F-phenyl | H | -O(CH₂)₂-piperidine | m-C(O)NH- | 1,1-dimethylethyl-3-oxazole |
| 1346 | 4-F-phenyl | H | -O(CH₂)₂-piperidine | m-C(O)NH- | 4-OCF₃-phenyl |
| 1347 | 5-F-phenyl | H | -O(CH₂)₂-piperidine | m-C(O)NH- | 1,1-dimethylethyl-1-methyl-1H-pyrazole |
| 1348 | 6-F-phenyl | H | -O(CH₂)₂-piperidine | m-C(O)NH- | 3,4-dimethyl-5-isoxazole |
| 1349 | 2-thiophene | H | -O(CH₂)₂-piperidine | m-C(O)NH- | 1,1-dimethylethyl-3-oxazole |
| 1350 | 3-thiophene | H | -O(CH₂)₂-piperidine | m-C(O)NH- | 4-OCF₃-phenyl |
| 1351 | 2-pyridine | H | -O(CH₂)₂-piperidine | m-C(O)NH- | 1,1-dimethylethyl-1-methyl-1H-pyrazole |
| 1352 | 3-pyridine | H | -O(CH₂)₂-piperidine | m-C(O)NH- | 3,4-dimethyl-5-isoxazole |
| 1353 | 2-CH₃-phenyl | H | -O(CH₂)₂-piperzine | m-C(O)NH- | 1,1-dimethylethyl-3-oxazole |
| 1354 | 4-CH₃-phenyl | H | -O(CH₂)₂-piperzine | m-C(O)NH- | 4-OCF₃-phenyl |
| 1355 | 5-CH₃-phenyl | H | -O(CH₂)₂-piperzine | m-C(O)NH- | 1,1-dimethylethyl-1-methyl-1H-pyrazole |
| 1356 | 6-CH₃-phenyl | H | -O(CH₂)₂-piperzine | m-C(O)NH- | 3,4-dimethyl-5-isoxazole |
| 1357 | 2-OCH₃-phenyl | H | -O(CH₂)₂-piperzine | m-C(O)NH- | 1,1-dimethylethyl-3-oxazole |
| 1358 | 4-OCH₃-phenyl | H | -O(CH₂)₂-piperzine | m-C(O)NH- | 4-OCF₃-phenyl |
| 1359 | 5-OCH₃-phenyl | H | -O(CH₂)₂-piperzine | m-C(O)NH- | 1,1-dimethylethyl-1-methyl-1H-pyrazole |
| 1360 | 6-OCH₃-phenyl | H | -O(CH₂)₂-piperzine | m-C(O)NH- | 3,4-dimethyl-5-isoxazole |
| 1361 | 2-F-phenyl | H | -O(CH₂)₂-piperzine | m-C(O)NH- | 1,1-dimethylethyl-3-oxazole |
| 1362 | 4-F-phenyl | H | -O(CH₂)₂-piperzine | m-C(O)NH- | 4-OCF₃-phenyl |
| 1363 | 5-F-phenyl | H | -O(CH₂)₂-piperzi ne | m-C(O)NH- | 1,1-dimethylethyl-1-methyl-1H-pyrazole |
| 1364 | 6-F-phenyl | H | -O(CH₂)₂-piperzine | m-C(O)NH- | 3,4-dimethyl-5-isoxazole |
| 1365 | 2-thiophene | H | -O(CH₂)₂-piperzine | m-C(O)NH- | 1,1-dimethylethyl-3-oxazole |
| 1366 | 3-thiophene | H | -O(CH₂)₂-piperzine | m-C(O)NH- | 4-OCF₃-phenyl |
| 1367 | 2-pyridine | H | -O(CH₂)₂-piperzine | m-C(O)NH- | 1,1-dimethylethyl-1-methyl-1H-pyrazole |
| 1368 | 3-pyridine | H | -O(CH₂)₂-piperzine | m-C(O)NH- | 3,4-dimethyl-5-isoxazole |
| 1369 | 2-CH₃-phenyl | H | 1-pyrrolidinylethyl | m-C(O)NH- | 1,1-dimethylethyl-3-oxazole |
| 1370 | 4-CH₃-phenyl | H | 1-pyrrolidinylethyl | m-C(O)NH- | 4-OCF₃-phenyl |
| 1371 | 5-CH₃-phenyl | H | 1-pyrrolidinylethyl | m-C(O)NH- | 1,1-dimethylethyl-1-methyl-1H-pyrazole |
| 1372 | 6-CH₃-phenyl | H | 1-pyrrolidinylethyl | m-C(O)NH- | 3,4-dimethyl-5-isoxazole |
| 1373 | 2-OCH₃-phenyl | H | 1-pyrrolidinylethyl | m-C(O)NH- | 1,1-dimethylethyl-3-oxazole |
| 1374 | 4-OCH₃-phenyl | H | 1-pyrrolidinylethyl | m-C(O)NH- | 4-OCF₃-phenyl |
| 1375 | 5-OCH₃-phenyl | H | 1-pyrrolidinylethyl | m-C(O)NH- | 1,1-dimethylethyl-1-methyl-1H-pyrazole |
| 1376 | 6-OCH₃-phenyl | H | 1-pyrrolidinylethyl | m-C(O)NH- | 3,4-dimethyl-5-isoxazole |
| 1377 | 2-F-phenyl | H | 1-pyrrolidinylethyl | m-C(O)NH- | 1,1-dimethylethyl-3-oxazole |
| 1378 | 4-F-phenyl | H | 1-pyrrolidinylethyl | m-C(O)NH- | 4-OCF₃-phenyl |
| 1379 | 5-F-phenyl | H | 1-pyrrolidinylethyl | m-C(O)NH- | 1,1-dimethylethyl-1-methyl-1H-pyrazole |
| 1380 | 6-F-phenyl | H | 1-pyrrolidinylethyl | m-C(O)NH- | 3,4-dimethyl-5-isoxazole |
| 1381 | 2-thiophene | H | 1-pyrrolidinylethyl | m-C(O)NH- | 1,1-dimethylethyl-3-oxazole |
| 1382 | 3-thiophene | H | 1-pyrrolidinylethyl | m-C(O)NH- | 4-OCF₃-phenyl |
| 1383 | 2-pyridine | H | 1-pyrrolidinylethyl | m-C(O)NH- | 1,1-dimethylethyl-1-methyl-1H-pyrazole |
| 1384 | 3-pyridine | H | 1-pyrrolidinylethyl | m-C(O)NH- | 3,4-dimethyl-5-isoxazole |
| 1385 | 2-CH₃-phenyl | H | H | p-NHC(O)NH- | 1,1-dimethylethyl-3-oxazole |
| 1386 | 4-CH₃-phenyl | H | H | p-NHC(O)NH- | 4-OCF₃-phenyl |
| 1387 | 5-CH₃-phenyl | H | H | p-NHC(O)NH- | 1,1-dimethylethyl-1-methyl-1H-pyrazole |
| 1388 | 6-CH₃-phenyl | H | H | p-NHC(O)NH- | 3,4-dimethyl-5-isoxazole |
| 1389 | 2-OCH₃-phenyl | H | H | p-NHC(O)NH- | 1,1-dimethylethyl-3-oxazole |
| 1390 | 4-OCH₃-phenyl | H | H | p-NHC(O)NH- | 4-OCF₃-phenyl |
| 1391 | 5-OCH₃-phenyl | H | H | p-NHC(O)NH- | 1,1-dimethylethyl-1-methyl-1H-pyrazole |
| 1392 | 6-OCH₃-phenyl | H | H | p-NHC(O)NH- | 3,4-dimethyl-5-isoxazole |
| 1393 | 2-F-phenyl | H | H | p-NHC(O)NH- | 1,1-dimethylethyl-3-oxazole |
| 1394 | 4-F-phenyl | H | H | p-NHC(O)NH- | 4-OCF₃-phenyl |
| 1395 | 5-F-phenyl | H | H | p-NHC(O)NH- | 1,1-dimethylethyl-1-methyl-1H-pyrazole |
| 1396 | 6-F-phenyl | H | H | p-NHC(O)NH- | 3,4-dimethyl-5-isoxazole |
| 1397 | 2-thiophene | H | H | p-NHC(O)NH- | 1,1-dimethylethyl-3-oxazole |
| 1398 | 3-thiophene | H | H | p-NHC(O)NH- | 4-OCF₃-phenyl |
| 1399 | 2-pyridine | H | H | p-NHC(O)NH- | 1,1-dimethylethyl-1-methyl-1H-pyrazole |
| 1400 | 3-pyridine | H | H | p-NHC(O)NH- | 3,4-dimethyl-5-isoxazole |
| 1401 | 2-CH₃-phenyl | CH₃ | H | p-NHC(O)NH- | 1,1-dimethylethyl-3-oxazole |
| 1402 | 4-CH₃-phenyl | CH₃ | H | p-NHC(O)NH- | 4-OCF₃-phenyl |
| 1403 | 5-CH₃-phenyl | CH₃ | H | p-NHC(O)NH- | 1,1-dimethylethyl-1-methyl-1H-pyrazole |
| 1404 | 6-CH₃-phenyl | CH₃ | H | p-NHC(O)NH- | 3,4-dimethyl-5-isoxazole |
| 1405 | 2-OCH₃-phenyl | CH₃ | H | p-NHC(O)NH- | 1,1-dimethylethyl-3-oxazole |
| 1406 | 4-OCH₃-phenyl | CH₃ | H | p-NHC(O)NH- | 4-OCF₃-phenyl |
| 1407 | 5-OCH₃-phenyl | CH₃ | H | p-NHC(O)NH- | 1,1-dimethylethyl-1-methyl-1H-pyrazole |
| 1408 | 6-OCH₃-phenyl | CH₃ | H | p-NHC(O)NH- | 3,4-dimethyl-5-isoxazole |
| 1409 | 2-F-phenyl | CH₃ | H | p-NHC(O)NH- | 1,1-dimethylethyl-3-oxazole |
| 1410 | 4-F-phenyl | CH₃ | H | p-NHC(O)NH- | 4-OCF₃-phenyl |
| 1411 | 5-F-phenyl | CH₃ | H | p-NHC(O)NH- | 1,1-dimethylethyl-1-methyl-1H-pyrazole |
| 1412 | 6-F-phenyl | CH₃ | H | p-NHC(O)NH- | 3,9-dimethyl-5-isoxazole |
| 1413 | 2-thiophene | CH₃ | H | p-NHC(O)NH- | 1,1-dimethylethyl-3-oxazole |
| 1414 | 3-thiophene | CH₃ | H | p-NHC(O)NH- | 4-OCF₃-phenyl |
| 1415 | 2-pyridine | CH₃ | H | p-NHC(O)NH- | 1,1-dimethylethyl-1-methyl-1H-pyrazole |
| 1416 | 3-pyridine | CH₃ | H | p-NHC(O)NH- | 3,4-dimethyl-5-isoxazole |
| 1417 | 2-CH₃-phenyl | H | -(CH₂)₃ morpholine | p-NHC(O)NH- | 1,1-dimethylethyl-3-oxazole |
| 1418 | 4-CH₃-phenyl | H | -(CH₂)₃ morpholine | p-NHC(O)NH- | 4-OCF₃-phenyl |
| 1419 | 5-CH₃-phenyl | H | -(CH₂)₃ morpholine | p-NHC(O)NH- | 1,1-dimethylethyl-1-methyl-1H-pyrazole |
| 1420 | 6-CH₃-phenyl | H | -(CH₂)₃ morpholine | p-NHC(O)NH- | 3,4-dimethyl-5-isoxazole |
| 1421 | 2-OCH₃-phenyl | H | -(CH₂)₃ morpholine | p-NHC(O)NH- | 1,1-dimethylethyl-3-oxazole |
| 1422 | 4-OCH₃-phenyl | H | -(CH₂)₃ morpholine | p-NHC(O)NH- | 4-OCF₃-phenyl |
| 1423 | 5-OCH₃-phenyl | H | -(CH₂)₃-morpholine | p-NHC(O)NH- | 1,1-dimethylethyl-1-methyl-1H-pyrazole |
| 1424 | 6-OCH₃-phenyl | H | -(CH₂)₃-morpholine | p-NHC(O)NH- | 3,4-dimethyl-5-isoxazole |
| 1425 | 2-F-phenyl | H | -(CH₂)₃ morpholine | p-NHC(O)NH- | 1,1-dimethylethyl-3-oxazole |
| 1426 | 4-F-phenyl | H | -(CH₂)₃ morpholine | p-NHC(O)NH- | 4-OCF₃-phenyl |
| 1427 | 5-F-phenyl | H | -(CH₂)₃ morpholine | p-NHC(O)NH- | 1,1-dimethylethyl-1-methyl-1H-pyrazole |
| 1428 | 6-F-phenyl | H | -(CH₂)₃ morpholine | p-NHC(O)NH- | 3,4-dimethyl-5-isoxazole |
| 1429 | 2-thiophene | H | -(CH₂)₃ morpholine | p-NHC(O)NH- | 1,1-dimethylethyl-3-oxazole |
| 1430 | 3-thiophene | H | -(CH₂)₃ morpholine | p-NHC(O)NH- | 4-OCF₃-phenyl |
| 1431 | 2-pyridine | H | -(CH₂)₃-morpholine | p-NHC(O)NH- | 1,1-dimethylethyl-1-methyl-1H-pyrazole |
| 1432 | 3-pyridine | H | -(CH₂)₃-morpholine | p-NHC(O)NH- | 3,4-dimethyl-5-isoxazole |
| 1433 | 2-CH₃-phenyl | H | CH₃ | p-NHC(O)NH- | 1,1-dimethylethyl-3-oxazole |
| 1434 | 4-CH₃-phenyl | H | CH₃ | p-NHC(O)NH- | 4-OCF₃-phenyl |
| 1435 | 5-CH₃-phenyl | H | CH₃ | p-NHC(O)NH- | 1,1-dimethylethyl-1-methyl-1H-pyrazole |
| 1436 | 6-CH₃-phenyl | H | CH₃ | p-NHC(O)NH- | 3,4-dimethyl-5-isoxazole |
| 1437 | 2-OCH₃-phenyl | H | CH₃ | p-NHC(O)NH- | 1,1-dimethylethyl-3-oxazole |
| 1438 | 4-OCH₃-phenyl | H | CH₃ | p-NHC(O)NH- | 4-OCF₃-phenyl |
| 1439 | 5-OCH₃-phenyl | H | CH₃ | p-NHC(O)NH- | 1,1-dimethylethyl-1-methyl-1H-pyrazole |
| 1440 | 6-OCH₃-phenyl | H | CH₃ | p-NHC(O)NH- | 3,4-dimethyl-5-isoxazole |
| 1441 | 2-F-phenyl | H | CH₃ | p-NHC(O)NH- | 1,1-dimethylethyl-3-oxazole |
| 1442 | 4-F-phenyl | H | CH₃ | p-NHC(O)NH- | 4-OCF₃-phenyl |
| 1443 | 5-F-phenyl | H | CH₃ | p-NHC(O)NH- | 1,1-dimethylethyl-1-methyl-1H-pyrazole |
| 1444 | 6-F-phenyl | H | CH₃ | p-NHC(O)NH- | 3,4-dimethyl-5-isoxazole |
| 1445 | 2-thiophene | H | CH₃ | p-NHC(O)NH- | 1,1-dimethylethyl-3-oxazole |
| 1446 | 3-thiophene | H | CH₃ | p-NHC(O)NH- | 4-OCF₃-phenyl |
| 1447 | 2-pyridine | H | CH₃ | p-NHC(O)NH- | 1,1-dimethylethyl-1-methyl-1H-pyrazole |
| 1448 | 3-pyridine | H | CH₃ | p-NHC(O)NH- | 3,4-dimethyl-5-isoxazole |
| 1449 | 2-CH₃-phenyl | H | -O(CH₂)₂-piperidine | p-NHC(O)NH- | 1,1-dimethylethyl-3-oxazole |
| 1450 | 4-CH₃-phenyl | H | -O(CH₂)₂-piperidine | p-NHC(O)NH- | 4-OCF₃-phenyl |
| 1451 | 5-CH₃-phenyl | H | -O(CH₂)₂-piperidine | p-NHC(O)NH- | 1,1-dimethylethyl-1-methyl-1H-pyrazole |
| 1452 | 6-CH₃-phenyl | H | -O(CH₂)₂-piperidine | p-NHC(O)NH- | 3,4-dimethyl-5-isoxazole |
| 1453 | 2-OCH₃-phenyl | H | -O(CH₂)₂-piperidine | p-NHC(O)NH- | 1,1-dimethylethyl-3-oxazole |
| 1454 | 4-OCH₃-phenyl | H | -O(CH₂)₂-piperidine | p-NHC(O)NH- | 4-OCF₃-phenyl |
| 1455 | 5-OCH₃-phenyl | H | -O(CH₂)₂-piperidine | p-NHC(O)NH- | 1,1-dimethylethyl-1-methyl-1H-pyrazole |
| 1456 | 6-OCH₃-phenyl | H | -O(CH₂)₂-piperidine | p-NHC(O)NH- | 3,4-dimethyl-5-isoxazole |
| 1457 | 2-F-phenyl | H | -O(CH₂)₂-piperidine | p-NHC(O)NH- | 1,1-dimethylethyl-3-oxazole |
| 1458 | 4-F-phenyl | H | -O(CH₂)₂-piperidine | p-NHC(O)NH- | 4-OCF₃-phenyl |
| 1459 | 5-F-phenyl | H | -O(CH₂)₂-piperidine | p-NHC(O)NH- | 1,1-dimethylethyl-1-methyl-1H-pyrazole |
| 1460 | 6-F-phenyl | H | -O(CH₂)₂-piperidine | p-NHC(O)NH- | 3,4-dimethyl-5-isoxazole |
| 1461 | 2-thiophene | H | -O(CH₂)₂-piperidine | p-NHC(O)NH- | 1,1-dimethylethyl-3-oxazole |
| 1462 | 3-thiophene | H | -O(CH₂)₂-piperidine | p-NHC(O)NH- | 4-OCF₃-phenyl |
| 1463 | 2-pyridine | H | -O(CH₂)₂-piperidine | p-NHC(O)NH- | 1,1-dimethylethyl-1-methyl-1H-pyrazole |
| 1464 | 3-pyridine | H | -O(CH₂)₂-piperidine | p-NHC(O)NH- | 3,4-dimethyl-5-isoxazole |
| 1465 | 2-CH₃-phenyl | H | -O(CH₂)₂-piperzine | p-NHC(O)NH- | 1,1-dimethylethyl-3-oxazole |
| 1466 | 4-CH₃-phenyl | H | -O(CH₂)₂-piperzine | p-NHC(O)NH- | 9-OCF₃-phenyl |
| 1467 | 5-CH₃-phenyl | H | -O(CH₂)₂-piperzine | p-NHC(O)NH- | 1,1-dimethylethyl-1-methyl-1H-pyrazole |
| 1468 | 6-CH₃-phenyl | H | -O(CH₂)₂-piperzine | p-NHC(O)NH- | 3,4-dimethyl-5-isoxazole |
| 1469 | 2-OCH₃-phenyl | H | -O(CH₂)₂-piperzine | p-NHC(O)NH- | 1,1-dimethylethyl-3-oxazole |
| 1470 | 4-OCH₃-phenyl | H | -O(CH₂)₂-piperzine | p-NHC(O)NH- | 4-OCF₃-phenyl |
| 1471 | 5-OCH₃-phenyl | H | -O(CH₂)₂-piperzine | p-NHC(O)NH- | 1,1-dimethylethyl-1-methyl-1H-pyrazole |
| 1472 | 6-OCH₃-phenyl | H | -O(CH₂)₂-piperzine | p-NHC(O)NH- | 3,4-dimethyl-5-isoxazole |
| 1473 | 2-F-phenyl | H | -O(CH₂)₂-piperzine | p-NHC(O)NH- | 1,1-dimethylethyl3-oxazole |
| 1474 | 4-F-phenyl | H | -O(CH₂)₂-piperzine | p-NHC(O)NH- | 4-OCF₃-phenyl |
| 1475 | 5-F-phenyl | H | -O(CH₂)₂-piperzine | p-NHC(O)NH- | 1,1-dimethylethyl-1-methyl-1H-pyrazole |
| 1476 | 6-F-phenyl | H | -O(CH₂)₂-piperzine | p-NHC(O)NH- | 3,4-dimethyl-5-isoxazole |
| 1477 | 2-thiophene | H | -O(CH₂)₂-piperzine | p-NHC(O)NH- | 1,1-dimethylethyl-3-oxazole |
| 1478 | 3-thiophene | H | -O(CH₂)₂-piperzine | p-NHC(O)NH- | 4-OCF₃-phenyl |
| 1479 | 2-pyridine | H | -O(CH₂)₂-piperzine | p-NHC(O)NH- | 1,1-dimethylethyl-1-methyl-1H-pyrazole |
| 1480 | 3-pyridine | H | -O(CH₂)₂-piperzine | p-NHC(O)NH- | 3,4-dimethyl-5-isoxazole |
| 1481 | 2-CH₃-phenyl | H | H | m-NHC(O)NH- | 1,1-dimethylethyl-3-oxazole |
| 1482 | 4-CH₃-phenyl | H | H | m-NHC(O)NH- | 4-OCF₃-phenyl |
| 1483 | 5-CH₃-phenyl | H | H | m-NHC(O)NH- | 1,1-dimethylethyl-1-methyl-1H-pyrazole |
| 1484 | 6-CH₃-phenyl | H | H | m-NHC(O)NH- | 3,4-dimethyl-5-isoxazole |
| 1485 | 2-OCH₃-phenyl | H | H | m-NHC(O)NH- | 1,1-dimethylethyl-3-oxazole |
| 1486 | 4-OCH₃-phenyl | H | H | m-NHC(O)NH- | 4-OCF₃-phenyl |
| 1487 | 5-OCH₃-phenyl | H | H | m-NHC(O)NH- | 1,1-dimethylethyl-1-methyl-1H-pyrazole |
| 1488 | 6-OCH₃-phenyl | H | H | m-NHC(O)NH- | 3,9-dimethyl-5-isoxazole |
| 1489 | 2-F-phenyl | H | H | m-NHC(O)NH- | 1,1-dimethylethyl-3-oxazole |
| 1490 | 4-F-phenyl | H | H | m-NHC(O)NH- | 4-OCF₃-phenyl |
| 1491 | 5-F-phenyl | H | H | m-NHC(O)NH- | 1,1-dimethylethyl-1-methyl-1H-pyrazole |
| 1492 | 6-F-phenyl | H | H | m-NHC(O)NH- | 3,4-dimethyl-5-isoxazole |
| 1493 | 2-thiophene | H | H | m-NHC(O)NH- | 1,1-dimethylethyl-3-oxazole |
| 1494 | 3-thiophene | H | H | m-NHC(O)NH- | 4-OCF₃-phenyl |
| 1495 | 2-pyridine | H | H | m-NHC(O)NH- | 1,1-dimethylethyl-1-methyl-1H-pyrazole |
| 1496 | 3-pyridine | H | H | m-NHC(O)NH- | 3,4-dimethyl-5-isoxazole |
| 1497 | 2-CH₃-phenyl | CH₃ | H | m-NHC(O)NH- | 1,1-dimethylethyl-3-oxazole |
| 1498 | 4-CH₃-phenyl | CH₃ | H | m-NHC(O)NH- | 4-OCF₃-phenyl |
| 1499 | 5-CH₃-phenyl | CH₃ | H | m-NHC(O)NH- | 1,1-dimethylethyl-1-methyl-1H-pyrazole |
| 1500 | 6-CH₃-phenyl | CH₃ | H | m-NHC(O)NH- | 3,4-dimethyl-5-isoxazole |
| 1501 | 2-OCH₃-phenyl | CH₃ | H | m-NHC(O)NH- | 1,1-dimethylethyl-3-oxazole |
| 1502 | 4-OCH₃-phenyl | CH₃ | H | m-NHC(O)NH- | 4-OCF₃-phenyl |
| 1503 | 5-OCH₃-phenyl | CH₃ | H | m-NHC(O)NH- | 1,1-dimethylethyl-1-methyl-1H-pyrazole |
| 1504 | 6-OCH₃-phenyl | CH₃ | H | m-NHC(O)NH- | 3,4-dimethyl-5-isoxazole |
| 1505 | 2-F-phenyl | CH₃ | H | m-NHC(O)NH- | 1,1-dimethylethyl-3-oxazole |
| 1506 | 4-F-phenyl | CH₃ | H | m-NHC(O)NH- | 4-OCF₃-phenyl |
| 1507 | 5-F-phenyl | CH₃ | H | m-NHC(O)NH- | 1,1-dimethylethyl-1-methyl-1H-pyrazole |
| 1508 | 6-F-phenyl | CH₃ | H | m-NHC(O)NH- | 3,4-dimethyl-5-isoxazole |
| 1509 | 2-thiophene | CH₃ | H | m-NHC(O)NH- | 1,1-dimethylethyl-3-oxazole |
| 1510 | 3-thiophene | CH₃ | H | m-NHC(O)NH- | 4-OCF₃-phenyl |
| 1511 | 2-pyridine | CH₃ | H | m-NHC(O)NH- | 1,1-dimethylethyl-1-methyl-1H-pyrazole |
| 1512 | 3-pyridine | CH₃ | H | m-NHC(O)NH- | 3,4-dimethyl-5-isoxazole |
| 1513 | 2-CH₃-phenyl | H | -(CH₂)₃-morpholine | m-NHC(O)NH- | 1,1-dimethylethyl-3-oxazole |
| 1514 | 4-CH₃-phenyl | H | -(CH₂)₃morpholine | m-NHC(O)NH- | 4-OCF₃-phenyl |
| 1515 | 5-CH₃-phenyl | H | -(CH₂)₃-morpholine | m-NHC(O)NH- | 1,1-dimethylethyl-1-methyl-1H-pyrazole |
| 1516 | 6-CH₃-phenyl | H | -(CH₂)₃-morpholine | m-NHC(O)NH- | 3,4-dimethyl-5-isoxazole |
| 1517 | 2-OCH₃-phenyl | H | -(CH₂)₃-morpholine | m-NHC(O)NH- | 1,1-dimethylethyl-3-oxazole |
| 1518 | 4-OCH₃-phenyl | H | -(C-H₂)₃-morpholine | m-NHC(O)NH- | 4-OCF₃-phenyl |
| 1519 | 5-OCH₃-phenyl | H | -(CH₂)₃-morpholine | m-NHC(O)NH- | 1,1-dimethylethyl-1-methyl-1H-pyrazole |
| 1520 | 6-OCH₃-phenyl | H | -(CH₂)₃-morpholine | m-NHC(O)NH- | 3,4-dimethyl-5-isoxazole |
| 1521 | 2-F-phenyl | H | -(CH₂)₃-morpholine | m-NHC(O)NH- | 1,1-dimethylethyl-3-oxazole |
| 1522 | 4-F-phenyl | H | -(CH₂)₃ morpholine | m-NHC(O)NH- | 4-OCF₃-phenyl |
| 1523 | 5-F-phenyl | H | -(CH₂)₃ morpholine | m-NHC(O)NH- | 1,1-dimethylethyl-1-methyl-1H-pyrazole |
| 1524 | 6-F-phenyl | H | -(CH₂)₃-morpholine | m-NHC(O)NH- | 3,4-dimethyl-5-isoxazole |
| 1525 | 2-thiophene | H | -(CH₂)₃-morpholine | m-NHC(O)NH- | 1,1-dimethylethyl-3-oxazole |
| 1526 | 3-thiophene | H | -(CH₂)₃-morpholine | m-NHC(O)NH- | 4-OCF₃-phenyl |
| 1527 | 2-pyridine | H | -(CH₂)₃-morpholine | m-NHC(O)NH- | 1,1-dimethylethyl-1-methyl-1H-pyrazole |
| 1528 | 3-pyridine | H | -(CH₂)₃ morpholine | m-NHC(O)NH- | 3,4-dimethyl-5-isoxazole |
| 1529 | 2-CH₃-phenyl | H | CH₃ | m-NHC(O)NH- | 1,1-dimethylethyl-3-oxazole |
| 1530 | 4-CH₃-phenyl | H | CH₃ | m-NHC(O)NH- | 4-OCF₃-phenyl |
| 1531 | 5-CH₃-phenyl | H | CH₃ | m-NHC(O)NH- | 1,1-dimethylethyl-1-methyl-1H-pyrazole |
| 1532 | 6-CH₃-phenyl | H | CH₃ | m-NHC(O)NH- | 3,4-dimethyl-5-isoxazole |
| 1533 | 2-OCH₃-phenyl | H | CH₃ | m-NHC(O)NH- | 1,1-dimethylethyl-3-oxazole |
| 1534 | 4-OCH₃-phenyl | H | CH₃ | m-NHC(O)NH- | 4-OCF₃-phenyl |
| 1535 | 5-OCH₃-phenyl | H | CH₃ | m-NHC(O)NH- | 1,1-dimethylethyl-1-methyl-1H-pyrazole |
| 1536 | 6-OCH₃-phenyl | H | CH₃ | m-NHC(O)NH- | 3,4-dimethyl-5-isoxazole |
| 1537 | 2-F-phenyl | H | CH₃ | m-NHC(O)NH- | 1,1-dimethylethyl-3-oxazole |
| 1538 | 4-F-phenyl | H | CH₃ | m-NHC(O)NH- | 4-OCF₃-phenyl |
| 1539 | 5-F-phenyl | H | CH₃ | m-NHC(O)NH- | 1,1-dimethylethyl-1-methyl-1H-pyrazole |
| 1540 | 6-F-phenyl | H | CH₃ | m-NHC(O)NH- | 3,4-dimethyl-5-isoxazole |
| 1541 | 2-thiophene | H | CH₃ | m-NHC(O)NH- | -1,1-dimethylethyl-3-oxazole |
| 1542 | 3-thiophene | H | CH₃ | m-NHC(O)NH- | 4-OCF₃-phenyl |
| 1543 | 2-pyridine | H | CH₃ | m-NHC(O)NH- | 1,1-dimethylethyl-1-methyl-1H-pyrazole |
| 1544 | 3-pyridine | H | CH₃ | m-NHC(O)NH- | 3,4-dimethyl-5-isoxazole |
| 1545 | 2-CH₃-phenyl | H | -O(CH₂)₂-piperidine | m-NHC(O)NH- | 1,1-dimethylethyl-3-oxazole |
| 1546 | 4-CH₃-phenyl | H | -O(CH₂)₂-piperidine | m-NHC(O)NH- | 4-OCF₃-phenyl |
| 1547 | 5-CH₃-phenyl | H | -O(CH₂)₂-piperidine | m-NHC(O)NH- | 1,1-dimethylethyl-1-methyl-1H-pyrazole |
| 1548 | 6-CH₃-phenyl | H | -O(CH₂)₂-piperidine | m-NHC(O)NH- | 3,4-dimethyl-5-isoxazole |
| 1549 | 2-OCH₃-phenyl | H | -O(CH₂)₂-piperidine | m-NHC(O)NH- | 1,1-dimethylethyl-3-oxazole |
| 1550 | 4-OCH₃-phenyl | H | O(CH₂)₂-piperidine | m-NHC(O)NH- | 4-OCF₃-phenyl |
| 1551 | 5-OCH₃-phenyl | H | -O(CH₂)₂-piperidine | m-NHC(O)NH- | 1,1-dimethylethyl-1-methyl-1H-pyrazole |
| 1552 | 6-OCH₃-phenyl | H | -O(CH₂)₂-piperidine | m-NHC(O)NH- | 3,4-dimethyl-5-isoxazole |
| 1553 | 2-F-phenyl | H | -O(CH₂)₂-piperidine | m-NHC(O)NH- | 1,1-dimethylethyl-3-oxazole |
| 1554 | 4-F-phenyl | H | -O(CH₂)₂-piperidine | m-NHC(O)NH- | 4-OCF₃-phenyl |
| 1555 | 5-F-phenyl | H | -O(CH₂)₂-piperidine | m-NHC(O)NH- | 1,1-dimethylethyl-1-methyl-1H-pyrazole |
| 1556 | 6-F-phenyl | H | -O(CH₂)₂-piperidine | m-NHC(O)NH- | 3,4-dimethyl-5-isoxazole |
| 1557 | 2-thiophene | H | -O(CH₂)₂-piperidine | m-NHC(O)NH- | 1,1-dimethylethyl-3-oxazole |
| 1558 | 3-thiophene | H | -O(CH₂)₂-piperidine | m-NHC(O)NH- | 4-OCF₃-phenyl |
| 1559 | 2-pyridine | H | -O(CH₂)₂-piperidine | m-NHC(O)NH- | 1,1-dimethylethyl-1-methyl-1H-pyrazole |
| 1560 | 3-pyridine | H | -O(CH₂)₂-piperidine | m-NHC(O)NH- | 3,4-dimethyl-5-isoxazole |
| 1561 | 2-CH₃-phenyl | H | -O(CH₂)₂-piperzine | m-NHC(O)NH- | 1,1-dimethylethyl-3-oxazole |
| 1562 | 4-CH₃-phenyl | H | -O(CH₂)₂-piperzine | m-NHC(O)NH- | 4-OCF₃-phenyl |
| 1563 | 5-CH₃-phenyl | H | -O(CH₂)₂ piperzine | m-NHC(O)NH- | 1,1-dimethylethyl-1-methyl-1H-pyrazole |
| 1564 | 6-CH₃-phenyl | H | -O(CH₂)₂-piperzine | m-NHC(O)NH- | 3,4-dimethyl-5-isoxazole |
| 1565 | 2-OCH₃-phenyl | H | -O(CH₂)₂ piperzine | m-NHC(O)NH- | 1,1-dimethylethyl-3-oxazole |
| 1566 | 4-OCH₃-phenyl | H | -O(CH₂)₂-piperzine | m-NHC(O)NH- | 4-OCF₃-phenyl |
| 1567 | 5-OCH₃-phenyl | H | -O(CH₂)₂-piperzine | m-NHC(O)NH- | 1,1-dimethylethyl-1-methyl-1H-pyrazole |
| 1568 | 6-OCH₃-phenyl | H | -O(CH₂)₂-piperzine | m-NHC(O)NH- | 3,4-dimethyl-5isoxazole |
| 1569 | 2-F-phenyl | H | -O(CH₂)₂-piperzine | m-NHC(O)NH- | 1,1-dimethylethyl-3-oxazole |
| 1570 | 4-F-phenyl | H | -O(CH₂)₂-piperzine | m-NHC(O)NH- | 4-OCF₃-phenyl |
| 1571 | 5-F-phenyl | H | -O(CH₂)₂-piperzine | m-NHC(O)NH- | 1,1-dimethylethyl-1-methyl-1H-pyrazole |
| 1572 | 6-F-phenyl | H | -O(CH₂)₂-piperzine | mNHC(O)NH- | 3,4-dimethyl-5-isoxazole |
| 1573 | 2-thiophene | H | -O(CH₂)₂-piperzine | m-NHC(O)NH- | 1,1-dimethylethyl-3-oxazole |
| 1574 | 3-thiophene | H | -O(CH₂)₂-piperzine | m-NHC(O)NH- | 4-OCF₃-phenyl |
| 1575 | 2-pyridine | H | -O(CH₂)₂-piperzine | m-NHC(O)NH- | 1,1-dimethylethyl-1-methyl-1H-pyrazole |
| 1576 | 3-pyridine | H | -O(CH₂)₂-piperzine | m-NHC(O)NH- | 3,4-dimethyl-5-isoxazole |
| 1577 | 2-CH₃-phenyl | H | H | p-C(O)NH- | 1,1-dimethylethyl-3-oxazole |
| 1578 | 4-CH₃-phenyl | H | H | p-C(O)NH- | 4-OCF₃-phenyl |
| 1579 | 5-CH₃-phenyl | H | H | p-C(O)NH- | 1,1-dimethylethyl-1-methyl-1H-pyrazole |
| 1580 | 6-CH₃-phenyl | H | H | p-C(O)NH- | 3,4-dimethyl-5-isoxazole |
| 1581 | 2-OCH₃-phenyl | H | H | p-C(O)NH- | 1,1-dimethylethyl-3-oxazole |
| 1582 | 4 -OCH₃-phenyl | H | H | p-C(O)NH- | 4-OCF₃-phenyl |
| 1583 | 5-OCH₃-phenyl | H | H | p-C(O)NH- | 1,1-dimethylethyl-1-methyl-1H-pyrazole |
| 1584 | 6-OCH₃-phenyl | H | H | p-C(O)NH- | 3,4-dimethyl-5-isoxazole |
| 1585 | 2-F-phenyl | H | H | p-C(O)NH- | 1,1-dimethylethyl-3-oxazole |
| 1586 | 4-F-phenyl | H | H | p-C(O)NH- | 4-OCF₃-phenyl |
| 1587 | 5-F-phenyl | H | H | p-C(O)NH- | 1,1-dimethylethyl-1-methyl-1H-pyrazole |
| 1588 | 6-F-phenyl | H | H | p-C(O)NH- | 3,4-dimethyl-5-isoxazole |
| 1589 | 2-thiophene | H | H | p-C(O)NH- | 1,1-dimethylethyl-3-oxazole |
| 1590 | 3-thiophene | H | H | p-C(O)NH- | 4-OCF₃-phenyl |
| 1591 | 2-pyridine | H | H | p-C(O)NH- | 1,1-dimethylethyl-1-methyl-1H-pyrazole |
| 1592 | 3-pyridine | H | H | p-C(O)NH- | 3,4-dimethyl-5-isoxazole |
| 1593 | 2-CH₃-phenyl | CH₃ | H | p-C(O)NH- | 1,1-dimethylethyl-3-oxazole |
| 1594 | 4-CH₃-phenyl | CH₃ | H | p-C(O)NH- | 4-OCF₃-phenyl |
| 1595 | 5-CH₃-phenyl | CH₃ | H | p-C(O)NH- | 1,1-dimethylethyl-1-methyl-1H-pyrazole |
| 1596 | 6-CH₃-phenyl | CH₃ | H | p-C(O)NH- | 3,4-dimethyl-5-isoxazole |
| 1597 | 2-OCH₃-phenyl | CH₃ | H | p-C(O)NH- | 1,1-dimethylethyl-3-oxazole |
| 1598 | 4-OCH₃-phenyl | CH₃ | H | p-C(O)NH- | 4-OCF₃-phenyl |
| 1599 | 5-OCH₃-phenyl | CH₃ | H | p-C(O)NH- | 1,1-dimethylethyl-1-methyl-1H-pyrazole |
| 1600 | 6-OCH₃-phenyl | CH₃ | H | p-C(O)NH- | 3,4-dimethyl-5-isoxazole |
| 1601 | 2-F-phenyl | CH₃ | H | p-C(O)NH- | 1,1-dimethylethyl-3-oxazole |
| 1602 | 4-F-phenyl | CH₃ | H | p-C(O)NH- | 4-OCF₃-phenyl |
| 1603 | 5-F-phenyl | CH₃ | H | p-C(O)NH- | 1,1-dimethylethyl-1-methyl-1H-pyrazole |
| 1604 | 6-F-phenyl | CH₃ | H | p-C(O)NH- | 3,4-dimethyl-5-isoxazole |
| 1605 | 2-thiophene | CH₃ | H | p-C(O)NH- | 1,1-dimethylethyl-3-oxazole |
| 1606 | 3-thiophene | CH₃ | H | p-C(O)NH- | 4-OCF₃-phenyl |
| 1607 | 2-pyridine | CH₃ | H | p-C(O)NH- | 1,1-dimethylethyl-1-methyl-1H-pyrazole |
| 1608 | 3-pyridine | CH₃ | H | p-C(O)NH- | 3,4-dimethyl-5-isoxazole |
| 1609 | 2-CH₃-phenyl | H | -(CH₂)₃-morpholine | p-C(O)NH- | 1,1-dimethylethyl-3-oxazole |
| 1610 | 4-CH₃-phenyl | H | -(CH₂)₃ morpholine | p-C(O)NH- | 4-OCF₃-phenyl |
| 1611 | 5-CH₃-phenyl | H | -(CH₂)₃-morpholine | p-C(O)NH- | 1,1-dimethylethyl-1-methyl-1H-pyrazole |
| 1612 | 6-CH₃-phenyl | H | -(CH₂)₃-morpholine | p-C(O)NH- | 3,4-dimethyl-5-isoxazole |
| 1613 | 2-OCH₃-phenyl | H | -(CH₂)₃-morpholine | p-C(O)NH- | 1,1-dimethylethyl-3-oxazole |
| 1614 | 4-OCH₃-phenyl | H | -(CH₂)₃ morpholine | p-C(O)NH- | 4-OCF₃-phenyl |
| 1615 | 5-OCH₃-phenyl | H | -(CH₂)₃-morpholine | p-C(O)NH- | 1,1-dimethylethyl-1-methyl-1H-pyrazole |
| 1616 | 6-OCH₃-phenyl | H | -(CH₂)₃ morpholine | p-C(O)NH- | 3,4-dimethyl-5-isoxazole |
| 1617 | 2-F-phenyl | H | -(CH₂)₃ morpholine | p-C(O)NH- | 1,1-dimethylethyl-3-oxazole |
| 1618 | 4-F-phenyl | H | -(CH₂)₃ morpholine | p-C(O)NH- | 4-OCF₃-phenyl |
| 1619 | 5-F-phenyl | H | -(CH₂)₃ morpholine | p-C(O)NH- | 1,1-dimethylethyl-1-methyl-1H-pyrazole |
| 1620 | 6-F-phenyl | H | -(CH₂)₃ morpholine | p-C(O)NH- | 3,4-dimethyl-5-isoxazole |
| 1621 | 2-thiophene | H | -(CH₂)₃ morpholine | p-C(O)NH- | 1,1-dimethylethyl-3-oxazole |
| 1622 | 3-thiophene | H | -(CH₂)₃ morpholine | p-C(O)NH- | 4-OCF₃-phenyl |
| 1623 | 2-pyridine | H | -(CH₂)₃ morpholine | p-C(O)NH- | 1,1-dimethylethyl-1-methyl-1H-pyrazole |
| 1624 | 3-pyridine | H | -(CH₂)₃-morpholine | p-C(O)NH- | 3,4-dimethyl-5-isoxazole |
| 1625 | 2-CH₃-phenyl | H | CH₃ | p-C(O)NH- | 1,1-dimethylethyl-3-oxazole |
| 1626 | 4-CH₃-phenyl | H | CH₃ | p-C(O)NH- | 4-OCF₃-phenyl |
| 1627 | 5-CH₃-phenyl | H | CH₃ | p-C(O)NH- | 1,1-dimethylethyl-1-methyl-1H-pyrazole |
| 1628 | 6-CH₃-phenyl | H | CH₃ | p-C(O)NH- | 3,4-dimethyl-5-isoxazole |
| 1629 | 2-OCH₃-phenyl | H | CH₃ | p-C(O)NH- | 1,1-dimethylethyl-3-oxazole |
| 1630 | 4-OCH₃-phenyl | H | CH₃ | p-C(O)NH- | 4-OCF₃-phenyl |
| 1631 | 5-OCH₃-phenyl | H | CH₃ | p-C(O)NH- | 1,1-dimethylethyl-1-methyl-1H-pyrazole |
| 1632 | 6-OCH₃-phenyl | H | CH₃ | p-C(O)NH- | 3,4-dimethyl-5isoxazole |
| 1633 | 2-F-phenyl | H | CH₃ | p-C(O)NH- | 1,1-dimethylethyl-3-oxazole |
| 1634 | 4-F-phenyl | H | CH₃ | p-C(O)NH- | 4-OCF₃-phenyl |
| 1635 | 5-F-phenyl | H | CH₃ | p-C(O)NH- | 1,1-dimethylethyl-1-methyl-1H-pyrazole |
| 1636 | 6-F-phenyl | H | CH₃ | p-C(O)NH- | 3,4-dimethyl-5-isoxazole |
| 1637 | 2-thiophene | H | CH₃ | p-C(O)NH- | 1,1-dimethylethyl-3-oxazole |
| 1638 | 3-thiophene | H | CH₃ | p-C(O)NH- | 4-OCF₃-phenyl |
| 1639 | 2-pyridine | H | CH₃ | p-C(O)NH- | 1,1-dimethylethyl-1-methyl-1H-pyrazole |
| 1640 | 3-pyridine | H | CH₃ | p-C(O)NH- | 3,4-dimethyl-5-isoxazole |
| 1641 | 2-CH₃-phenyl | H | -O(CH₂)₂-piperidine | p-C(O)NH- | 1,1-dimethylethyl-3-oxazole |
| 1642 | 4-CH₃-phenyl | H | -O(CH₂)₂-piperidine | p-C(O)NH- | 4-OCF₃-phenyl |
| 1643 | 5-CH₃-phenyl | H | -O(CH₂)₂-piperidine | p-C(O)NH- | 1,1-dimethylethyl-1-methyl-1H-pyrazole |
| 1644 | 6-CH₃-phenyl | H | -O(CH₂)₂-piperidine | p-C(O)NH- | 3,4-dimethyl-5-isoxazole |
| 1645 | 2-OCH₃-phenyl | H | -O(CH₂)₂-piperidine | p-C(O)NH- | 1,1-dimethylethyl-3-oxazole |
| 1646 | 4-OCH₃-phenyl | H | -O(CH₂)₂-piperidine | p-C(O)NH- | 4-OCF₃-phenyl |
| 1647 | 5-OCH₃-phenyl | H | -O(CH₂)₂-piperidine | p-C(O)NH- | 1,1-dimethylethyl-1-methyl-1H-pyrazole |
| 1648 | 6-OCH₃-phenyl | H | -O(CH₂)₂-piperidine | p-C(O)NH- | 3,4-dimethyl-5-isoxazole |
| 1649 | 2-F-phenyl | H | -O(CH₂)₂-piperidine | p-C(O)NH- | 1,1-dimethylethyl3-oxazole |
| 1650 | 4-F-phenyl | H | -O(CH₂)₂-piperidine | p-C(O)NH- | 4-OCF₃-phenyl |
| 1651 | 5-F-phenyl | H | -O(CH₂)₂-piperidine | p-C(O)NH- | 1,1-dimethylethyl-1-methyl-1H-pyrazole |
| 1652 | 6-F-phenyl | H | -O(CH₂)₂-piperidine | p-C(O)NH- | 3,4-dimethyl-5-isoxazole |
| 1653 | 2-thiophene | H | -O(CH₂)₂-piperidine | p-C(O)NH- | 1,1-dimethylethyl-3-oxazole |
| 1654 | 3-thiophene | H | -O(CH₂)₂-piperidine | p-C(O)NH- | 4-OCF₃-phenyl |
| 1655 | 2-pyridine | H | -O(CH₂)₂-piperidine | p-C(O)NH- | 1,1-dimethylethyl-1-methyl-1H-pyrazole |
| 1656 | 3-pyridine | H | -O(CH₂)₂-piperidine | p-C(O)NH- | 3,4-dimethyl-5-isoxazole |
| 1657 | 2-CH₃-phenyl | H | -O(CH₂)₂-piperzi ne | p-C(O)NH- | 1,1-dimethylethyl-3-oxazole |
| 1658 | 4-CH₃-phenyl | H | O(CH₂)₂-piperzine | p-C(O)NH- | 4-OCF₃-phenyl |
| 1659 | 5-CH₃-phenyl | H | -O(CH₂)₂-piperzine | p-C(O)NH- | 1,1-dimethylethyl-1-methyl-1H-pyrazole |
| 1660 | 6-CH₃-phenyl | H | -O(CH₂)₂-piperzine | p-C(O)NH- | 3,4-dimethyl-5-isoxazole |
| 1661 | 2-OCH₃-phenyl | H | -O(CH₂)₂-piperzi ne | p-C(O)NH- | 1,1-dimethylethyl-3-oxazole |
| 1662 | 4-OCH₃-phenyl | H | -O(CH₂)₂-piperzine | p-C(O)NH- | 4-OCF₃-phenyl |
| 1663 | 5-OCH₃-phenyl | H | -O(CH₂)₂-piperzine | p-C(O)NH- | 1,1-dimethylethyl-1-methyl-1H-pyrazole |
| 1664 | 6-OCH₃-phenyl | H | O(CH₂)₂-piperzine | p-C(O)NH- | 3,4-dimethyl-5-isoxazole |
| 1665 | 2-F-phenyl | H | -O(CH₂)₂-piperzine | p-C(O)NH- | 1,1-dimethylethyl-3-oxazole |
| 1666 | 4-F-phenyl | H | O(CH₂)₂-piperzine | p-C(O)NH- | 4-OCF₃-phenyl |
| 1667 | 5-F-phenyl | H | -O(CH₂)₂-piperzine | p-C(O)NH- | 1,1-dimethylethyl-1-methyl-1Hpyrazole |
| 1668 | 6-F-phenyl | H | -O(CH₂)₂-piperzine | p-C(O)NH- | 3,4-dimethyl-5-isoxazole |
| 1669 | 2-thiophene | H | -O(CH₂)₂-piperzine | p-C(O)NH- | 1,1-dimethylethyl-3-oxazole |
| 1670 | 3-thiophene | H | -O(CH₂)₂-piperzine | p-C(O)NH- | 4-OCF₃-phenyl |
| 1671 | 2-pyridine | H | -O(CH₂)₂-piperzine | p-C(O)NH- | 1,1-dimethylethyl-1-methyl-1H-pyrazole |
| 1672 | 3-pyridine | H | -O(CH₂)₂-piperzine | p-C(O)NH- | 3,4-dimethyl-5-isoxazole |
| 1673 | 2-CH₃-phenyl | H | 1-pyrrolidinylethyl | p-C(O)NH- | 1,1-dimethylethyl-3-oxazole |
| 1674 | 4-CH₃-phenyl | H | 1-pyrrolidinylethyl | p-C(O)NH- | 4-OCF₃-phenyl |
| 1675 | 5-CH₃-phenyl | H | 1-pyrrolidinylethyl | p-C(O)NH- | 1,1-dimethylethyl-1-methyl-1H-pyrazole |
| 1676 | 6-CH₃-phenyl | H | 1-pyrrolidinylethyl | p-C(O)NH- | 3,4-dimethyl-5-isoxazole |
| 1677 | 2-OCH₃-phenyl | H | 1-pyrrolidinylethyl | p-C(O)NH- | 1,1-dimethylethyl-3-oxazole |
| 1678 | 4-OCH₃-phenyl | H | 1-pyrrolidinylethyl | p-C(O)NH- | 4-OCF₃-phenyl |
| 1679 | 5-OCH₃-phenyl | H | 1-pyrrolidinylethyl | p-C(O)NH- | 1,1-dimethylethyl-1-methyl-1H-pyrazole |
| 1680 | 6-OCH₃-phenyl | H | 1-pyrrolidinylethyl | p-C(O)NH- | 3,4-dimethyl-5-isoxazole |
| 1681 | 2-F-phenyl | H | 1-pyrrolidinylethyl | p-C(O)NH- | 1,1-dimethylethyl-3-oxazole |
| 1682 | 4-F-phenyl | H | 1-pyrrolidinylethyl | p-C(O)NH- | 4-OCF₃-phenyl |
| 1683 | 5-F-phenyl | H | 1-pyrrolidinylethyl | p-C(O)NH- | 1,1-dimethylethyl-1-methyl-1H-pyrazole |
| 1684 | 6-F-phenyl | H | 1-pyrrolidinylethyl | p-C(O)NH- | 3,4-dimethyl-5-isoxazole |
| 1685 | 2-thiophene | H | 1-pyrrolidinylethyl | p-C(O)NH- | 1,1-dimethylethyl-3-oxazole |
| 1686 | 3-thiophene | H | 1-pyrrolidinylethyl | p-C(O)NH- | 4-OCF₃-phenyl |
| 1687 | 2-pyridine | H | 1-pyrrolidinylethyl | p-C(O)NH- | 1,1-dimethylethyl-1-methyl-1H-pyrazole |
| 1688 | 3-pyridine | H | 1-pyrrolidinylethyl | p-C(O)NH- | 3,4-dimethyl-5-isoxazole |
| 1689 | 2-CH₃-phenyl | H | H | m-NH-C(O)- | 1,1-dimethylethyl-3-oxazole |
| 1690 | 4-CH₃-phenyl | H | H | m-NH-C(O)- | 4-OCF₃-phenyl |
| 1691 | 5-CH₃-phenyl | H | H | m-NH-C(O)- | 1,1-dimethylethyl-1-methyl-1H-pyrazole |
| 1692 | 6-CH₃-phenyl | H | H | m-NH-C(O)- | 3,4-dimethyl-5-isoxazole |
| 1693 | 2-OCH₃-phenyl | H | H | m-NH-C(O)- | 1,1-dimethylethyl-3-oxazole |
| 1694 | 4-OCH₃-phenyl | H | H | m-NH-C(O)- | 4-OCF₃-phenyl |
| 1695 | 5-OCH₃-phenyl | H | H | m-NH-C(O)- | 1,1-dimethylethyl-1-methyl-1H-pyrazole |
| 1696 | 6-OCH₃-phenyl | H | H | m-NH-C(O)- | 3,4-dimethyl-5-isoxazole |
| 1697 | 2-F-phenyl | H | H | m-NH-C(O)- | 1,1-dimethylethyl-3-oxazole |
| 1698 | 4-F-phenyl | H | H | m-NH-C(O)- | 4-OCF₃-phenyl |
| 1699 | 5-F-phenyl | H | H | m-NH-C(O)- | 1,1-dimethylethyl-1-methyl-1H-pyrazole |
| 1700 | 6-F-phenyl | H | H | m-NH-C(O)- | 3,4-dimethyl-5-isoxazole |
| 1701 | 2-thiophene | H | H | m-NH-C(O)- | 1,1-dimethylethyl-3-oxazole |
| 1702 | 3-thiophene | H | H | m-NH-C(O)- | 4-OCF₃-phenyl |
| 1703 | 2-pyridine | H | H | m-NH-C(O)- | 1,1-dimethylethyl-1-methyl-1H-pyrazole |
| 1704 | 3-pyridine | H | H | m-NH-C(O)- | 3,4-dimethyl-5-isoxazole |
| 1705 | 2-CH₃-phenyl | CH₃ | H | m-NH-C(O)- | 1,1-dimethylethyl-3-oxazole |
| 1706 | 4-CH₃-phenyl | CH₃ | H | m-NH-C(O)- | 4-OCF₃-phenyl |
| 1707 | 5-CH₃-phenyl | CH₃ | H | m-NH-C(O)- | 1,1-dimethylethyl-1-methyl-1H-pyrazole |
| 1708 | 6-CH₃-phenyl | CH₃ | H | m-NH-C(O)- | 3,4-dimethyl-5-isoxazole |
| 1709 | 2-OCH₃-phenyl | CH₃ | H | m-NH-C(O)- | 1,1-dimethylethyl-3-oxazole |
| 1710 | 4-OCH₃-phenyl | CH₃ | H | m-NH-C(O)- | 4-OCF₃-phenyl |
| 1711 | 5-OCH₃phenyl | CH₃ | H | m-NH-C(O)- | 1,1-dimethylethyl-1-methyl-1H-pyrazole |
| 1712 | 6-OCH₃-phenyl | CH₃ | H | m-NH-C(O)- | 3,4-dimethyl-5-isoxazole |
| 1713 | 2-F-phenyl | CH₃ | H | m-NH-C(O)- | 1,1-dimethylethyl-3-oxazole |
| 1714 | 4-F-phenyl | CH₃ | H | m-NH-C(O)- | 4-OCF₃-phenyl |
| 1715 | 5-F-phenyl | CH₃ | H | m-NHC(O)- | 1,1-dimethylethyl-1-methyl-1H-pyrazole |
| 1716 | 6-F-phenyl | CH₃ | H | m-NH-C(O)- | 3,4-dimethyl-5-isoxazole |
| 1717 | 2-thiophene | CH₃ | H | m-NH-C(O)- | 1,1-dimethylethyl-3-oxazole |
| 1718 | 3-thiophene | CH₃ | H | m-NHC(O)- | 4-OCF₃-phenyl |
| 1719 | 2-pyridine | CH₃ | H | m-NH-C(O)- | 1,1-dimethylethyl-1-methyl-1H-pyrazole |
| 1720 | 3-pyridine | CH₃ | H | m-NH-C(O)- | 3,4-dimethyl-5-isoxazole |
| 1721 | 2-CH₃-phenyl | H | -(CH₂)₃ morpholine | m-NH-C(O)- | 1,1-dimethylethyl3-oxazole |
| 1722 | 4-CH₃-phenyl | H | -(CH₂)₃ morpholine | m-NH-C(O)- | 4-OCF₃-phenyl |
| 1723 | 5-CH₃-phenyl | H | -(CH₂)₃ morpholine | m-NH-C(O)- | 1,1-dimethylethyl-1-methyl-1H-pyrazole |
| 1724 | 6-CH₃-phenyl | H | -(CH₂)₃-morpholine | m-NH-C(O)- | 3,4-dimethyl-5-isoxazole |
| 1725 | 2-OCH₃-phenyl | H | -(CH₂)₃ morpholine | m-NH-C(O)- | 1,1-dimethylethyl-3-oxazole |
| 1726 | 4-OCH₃-phenyl | H | -(CH₂)₃ morpholine | m-NH-C(O)- | 4-OCF₃-phenyl |
| 1727 | 5-OCH₃-phenyl | H | -(CH₂)₃ morpholine | m-NH-C(O)- | 1,1-dimethylethyl-1-methyl-1H-pyrazole |
| 1728 | 6-OCH₃-phenyl | H | -(CH₂)₃-morpholine | m-NH-C(O)- | 3,4-dimethyl-5-isoxazole |
| 1729 | 2-F-phenyl | H | -(CH₂)₃ morpholine | m-NH-C(O)-- | 1,1-dimethylethyl-3-oxazole |
| 1730 | 4-F-phenyl | H | -(CH₂)₃ morpholine | m-NH-C(O)- | 4-OCF₃-phenyl |
| 1731 | 5-F-phenyl | H | -(CH₂)₃ morpholine | m-NHC(O)- | 1,1-dimethylethyl-1-methyl-1H-pyrazole |
| 1732 | 6-F-phenyl | H | -(CH₂)₃-morpholine | m-NH-C(O)- | 3,4-dimethyl-5isoxazole |
| 1733 | 2-thiophene | H | -(CH₂)₃ morpholine | m-NH-C(O)- | 1,1-dimethylethyl-3-oxazole |
| 1734 | 3-thiophene | H | -(CH₂)₃ morpholine | m-NH-C(O)- | 4-OCF₃-phenyl |
| 1735 | 2-pyridine | H | -(CH₂)₃-morpholine | m-NH-C(O)- | 1,1-dimethylethyl-1-methyl-1H-pyrazole |
| 1736 | 3-pyridine | H | -(CH₂)₃ morpholine | m-NH-C(O)- | 3,4-dimethyl-5-isoxazole |
| 1737 | 2-CH₃-phenyl | H | CH₃ | m-NH-C(O)- | 1,1-dimethylethyl-3-oxazole |
| 1738 | 4-CH₃-phenyl | H | CH₃ | m-NH-C(O)- | 4-OCF₃-phenyl |
| 1739 | 5-CH₃-phenyl | H | CH₃ | m-NH-C(O)- | 1,1-dimethylethyl1-methyl-1H-pyrazole |
| 1740 | 6-CH₃-phenyl | H | CH₃ | m-NH-C(O)- | 3,4-dimethyl-5-isoxazole |
| 1741 | 2-OCH₃phenyl | H | CH₃ | m-NH-C(O)- | 1,1-dimethylethyl-3-oxazole |
| 1742 | 4-OCH₃-phenyl | H | CH₃ | m-NHC(O)- | 4-OCF₃-phenyl |
| 1743 | 5-OCH₃-phenyl | H | CH₃ | m-NH-C(O)- | 1,1-dimethylethyl-1-methyl-1H-pyrazole |
| 1744 | 6-OCH₃phenyl | H | CH₃ | m-NH-C(O)- | 3,4-dimethyl-5-isoxazole |
| 1745 | 2-F-phenyl | H | CH₃ | m-NH-C(O)- | 1,1-dimethylethyl-3-oxazole |
| 1746 | 4-F-phenyl | H | CH₃ | m-NHC(O)- | 4-OCF₃-phenyl |
| 1747 | 5-F-phenyl | H | CH₃ | m-NH-C(O)- | 1,1-dimethylethyl-1-methyl-1H-pyrazole |
| 1748 | 6-F-phenyl | H | CH₃ | m-NHC(O)- | 3,4-dimethyl-5-isoxazole |
| 1749 | 2-thiophene | H | CH₃ | m-NH-C(O)- | 1,1-dimethylethyl-3-oxazole |
| 1750 | 3-thiophene | H | CH₃ | m-NH-C(O)- | 4-OCF₃-phenyl |
| 1751 | 2-pyridine | H | CH₃ | m-NH-C(O)- | 1,1-dimethylethyl-1-methyl-1H-pyrazole |
| 1752 | 3-pyridine | H | CH₃ | m-NH-C(O)- | 3,4-dimethyl-5-isoxazole |
| 1753 | 2-CH₃-phenyl | H | -O(CH₂)₂-piperidine | m-NH-C(O)- | 1,1-dimethylethyl-3-oxazole |
| 1754 | 4-CH₃-phenyl | H | -O(CH₂)₂-piperidine | m-NH-C(O)- | 4-OCF₃-phenyl |
| 1755 | 5-CH₃-phenyl | H | -O(CH₂)₂-piperidine | m-NH-C(O)- | 1,1-dimethylethyl-1-methyl-1H-pyrazole |
| 1756 | 6-CH₃-phenyl | H | -O(CH₂)₂-piperidine | m-NH-C(O)- | 3,4-dimethyl-5-isoxazole |
| 1757 | 2-OCH₃-phenyl | H | -O(CH₂)₂-piperidine | m-NH-C(O)- | 1,1-dimethylethyl-3-oxazole |
| 1758 | 4-OCH₃-phenyl | H | O(CH₂)₂-piperidine | m-NH-C(O)- | 4-OCF₃-phenyl |
| 1759 | 5-OCH₃-phenyl | H | -O(CH₂)₂-piperidine | m-NH-C(O)- | 1,1-dimethylethyl-1-methyl-1H-pyrazole |
| 1760 | 6-OCH₃-phenyl | H | -O(CH₂)₂-piperidine | m-NH-C(O)- | 3,4-dimethyl-5-isoxazole |
| 1761 | 2-F-phenyl | H | -O(CH₂)₂-piperidine | m-NH-C(O)- | 1,1-dimethylethyl-3-oxazole |
| 1762 | 4-F-phenyl | H | -O(CH₂)₂-piperidine | m-NH-C(O)- | 4-OCF₃-phenyl |
| 1763 | 5-F-phenyl | H | -O(CH₂)₂-piperidine | m-NH-C(O)- | 1,1-dimethylethy-1-methyl-1H-pyrazole |
| 1764 | 6-F-phenyl | H | -O(CH₂)₂-piperidine | m-NH-C(O)- | 3,4-dimethyl-5-isoxazole |
| 1765 | 2-thiophene | H | -O(CH₂)₂-piperidine | m-NH-C(O)- | 1,1-dimethylethyl-3-oxazole |
| 1766 | 3-thiophene | H | -O(CH₂)₂-piperidine | m-NH-C(O)- | 4-OCF₃-phenyl |
| 1767 | 2-pyridine | H | -O(CH₂)₂-piperidine | m-NH-C(O)- | 1,1-dimethylethyl-1-methyl-1H-pyrazole |
| 1768 | 3-pyridine | H | -O(CH₂)₂-piperidine | m-NH-C(O)- | 3,4-dimethyl-5-isoxazole |
| 1769 | 2-CH₃-phenyl | H | -O(CH₂)₂-piperzine | m-NH-C(O)- | 1,1-dimethylethyl-3-oxazole |
| 1770 | 4-CH₃-phenyl | H | O(CH₂)₂-piperzine | m-NH-C(O)- | 4-OCF₃-phenyl |
| 1771 | 5-CH₃-phenyl | H | -O(CH₂)₂-piperzine | m-NH-C(O)- | 1,1-dimethylethyl-1-methyl-1H-pyrazole |
| 1772 | 6-CH₃-phenyl | H | -O(CH₂)₂-piperzine | m-NHC(O)- | 3,4-dimethyl-5-isoxazole |
| 1773 | 2-OCH₃-phenyl | H | -O(CH₂)₂-piperzine | m-NH-C(O)- | 1,1-dimethylethyl-3-oxazole |
| 1774 | 4-OCH₃-phenyl | H | -O(CH₂)₂-piperzine | m-NH-C(O)- | 4-OCF₃-phenyl |
| 1775 | 5-OCH₃-phenyl | H | -O(CH₂)₂-piperzine | m-NH-C(O)- | 1,1-dimethylethyl-1-methyl-1H-pyrazole |
| 1776 | 6-OCH₃phenyl | H | -O(CH₂)₂-piperzine | m-NH-C(O)- | 3,4-dimethyl-5-isoxazole |
| 1777 | 2-F-phenyl | H | -O(CH₂)₂-piperzine | m-NH-C(O)- | 1,1-dimethylethyl-3-oxazole |
| 1778 | 4-F-phenyl | H | -O(CH₂)₂-piperzine | m-NH-C(O)- | 4-OCF₃-phenyl |
| 1779 | 5-F-phenyl | H | -O(CH₂)₂-piperzine | m-NH-C(O)- | 1,1-dimethylethyl-1-methyl-1H-pyrazole |
| 1780 | 6-F-phenyl | H | -O(CH₂)₂-piperzine | m-NH-C(O)- | 3,4-dimethyl-5isoxazole |
| 1781 | 2-thiophene | H | -O(CH₂)₂-piperzine | m-NH-C(O)- | 1,1-dimethylethyl-3-oxazole |
| 1782 | 3-thiophene | H | O(CH₂)₂-piperzine | m-NH-C(O)- | 4-OCF₃-phenyl |
| 1783 | 2-pyridine | H | -O(CH₂)₂-piperzine | m-NH-C(O)- | 1,1-dimethylethyl-1-methyl-1Hpyrazole |
| 1784 | 3-pyridine | H | -O(CH₂)₂-piperzine | m-NH-C(O)- | 3,4-dimethyl-5-isoxazole |
| 1785 | 2-CH₃-phenyl | H | 1-pyrrolidinylethyl | m-NH-C(O)- | 1,1-dimethylethyl-3-oxazole |
| 1786 | 4-CH₃-phenyl | H | 1-pyrrolidinylethyl | m-NH-C(O)- | 4-OCF₃-phenyl |
| 1787 | 5-CH₃-phenyl | H | 1-pyrrolidinylethyl | m-NH-C(O)- | 1,1-dimethylethyl-1-methyl-1Hpyrazole |
| 1788 | 6-CH₃-phenyl | H | 1-pyrrolidinylet hyl | m-NH-C(O)- | 3,4-dimethyl-5-isoxazole |
| 1789 | 2-OCH₃-phenyl | H | 1-pyrrolidinylethyl | m-NH-C(O)- | 1,1-dimethylethyl3-oxazole |
| 1790 | 4-OCH₃-phenyl | H | 1-pyrrolidinylethyl | m-NH-C(O)- | 4-OCF₃-phenyl |
| 1791 | 5-OCH₃-phenyl | H | 1-pyrrolidinylethyl | m-NH-C(O)- | 1,1-dimethylethyl-1-methyl-1H-pyrazole |
| 1792 | 6-OCH₃-phenyl | H | 1-pyrrolidinylethyl | m-NH-C(O)- | 3,4-dimethyl-5-isoxazole |
| 1793 | 2-F-phenyl | H | 1-pyrrolidinylethyl | m-NH-C(O)- | 1,1-dimethylethyl-3-oxazole |
| 1794 | 4-F-phenyl | H | 1-pyrrolidinylethyl | m-NH-C(O)- | 4-OCF₃-phenyl |
| 1795 | 5-F-phenyl | H | 1-pyrrolidinylethyl | m-NH-C(O)- | 1,1-dimethylethyl-1-methyl-1H-pyrazole |
| 1796 | 6-F-phenyl | H | 1-pyrrolidinylethyl | m-NH-C(O)- | 3,4-dimethyl-5-isoxazole |
| 1797 | 2-thiophene | H | 1-pyrrolidinylethyl | m-NH-C(O)- | 1,1-dimethylethyl3-oxazole |
| 1798 | 3-thiophene | H | 1-pyrrolidinylethyl | m-NH-C(O)- | 4-OCF₃-phenyl |
| 1799 | 2-pyridine | H | 1-pyrrolidinylethyl | m-NH-C(O)- | 1,1-dimethylethyl-1-methyl-1H-pyrazole |
| 1800 | 3-pyridine | H | 1pyrrolidinylethyl | m-NH-C(O)- | 3,4-dimethyl-5-isoxazole |

**Table 2**

| | | | | |
|---|---|---|---|---|
| 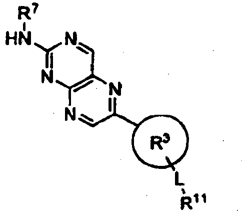 | | | | |

| Example No. | R³ | R⁷ | L | R¹¹ |
|---|---|---|---|---|
| 1801 | 2-CH₃-phenyl | H | m-C(O)NH- | 1-piperidinylpropyloxyphenyl |
| 1802 | 4-(CH₃-phenyl | H | m-C(O)NH- | cyclopropyl |
| 1803 | 5-CH₃-phenyl | H | m-C(O)NH- | 2-(3-dimethylaminopropyl)methylamino-5-CF₃-phenyl |
| 1804 | 6-CH₃-phenyl | H | m-C(O)NH- | 2-dimethylamino-5-CF₃-phenyl |
| 1805 | 2-OCH₃-phenyl | H | m-C(O)NH- | 1-piperidinylpropyloxyphenyl |
| 1806 | 4-OCH₃-phenyl | H | m-C(O)NH- | cyclopropyl |
| 1807 | 5-OCH₃-phenyl | H | m-C(O)NH- | 2-(3-dimethylaminopropyl)methylamino-5-CF₃-phenyl |
| 1808 | 6-OCH₃-phenyl | H | m-C(O)NH- | 2-dimethylamino-5-CF₃-phenyl |
| 1809 | 2-F-phenyl | H | mC(O)NH- | 1-piperidinylpropyloxyphenyl |
| 1810 | 4-F-phenyl | H | m-C(O)NH- | cyclopropyl |
| 1811 | 5-F-phenyl | H | m-C(O)NH- | 2-(3-dimethylaminopropyl)methylamino-5-CF₃-phenyl |
| 1812 | 6-F-phenyl | H | m-C(O)NH- | 2-dimethylamino-5-CF₃-phenyl |
| 1813 | 2-thiophene | H | m-C(O)NH- | 1-piperidinylpropyloxyphenyl |
| 1814 | 3-thiophene | H | m-C(O)NH- | cyclopropyl |
| 1815 | 2-pyridine | H | m-C(O)NH- | 2-(3-dimethylaminopropyl)methylamino-5-CF₃-phenyl |
| 1816 | 3-pyridine | H | m-C(O)NH- | 2-dimethylamino-5-CF₃-phenyl |
| 1817 | 2-CH₃-phenyl | H | m-C(O)NH- | 1-piperidinylpropyloxyphenyl |
| 1818 | 4-CH₃-phenyl | H | m-C(O)NH- | cyclopropyl |
| 1819 | 5-CH₃-phenyl | H | m-C(O)NH- | 2-(3-dimethylaminopropyl)methylamino-5-CF₃-phenyl |
| 1820 | 6-CH₃-phenyl | H | m-C(O)NH- | 2-dimethylamino-5CF₃-phenyl |
| 1821 | 2-OCH₃-phenyl | H | m-C(O)NH- | 1-piperidinylpropyloxyphenyl |
| 1822 | 4-OCH₃-phenyl | H | mC(O)NH- | cyclopropyl |
| 1823 | 5-OCH₃-phenyl | H | m-C(O)NH- | 2-(3dimethylaminopropyl)methylamino-5-CF₃-phenyl |
| 1824 | 6-OCH₃-phenyl | H | m-C(O)NH- | 2-dimethylamino-5-CF₃-phenyl |
| 1825 | 2-Fphenyl | H | m-C(O)NH- | 1-piperidinylpropyloxyphenyl |
| 1826 | 4-F-phenyl | H | m-C(O)NH- | cyclopropyl |
| 1827 | 5-F-phenyl | H | m-C(O)NH- | 2-(3-dimethylaminopropyl)methylamino-5-CF₃-phenyl |
| 1828 | 6-F-phenyl | H | m-C(O)NH- | 2-dimethylamino-5-CF₃-phenyl |
| 1829 | 2-thiophene | H | m-C(O)NH- | 1-piperidinylpropyloxyphenyl |
| 1830 | 3-thiophene | H | m-C(O)NH- | cyclopropyl |
| 1831 | 2pyridine | H | m-C(O)NH- | 2-(3-dimethylaminopropyl)methylamino-5-CF₃-phenyl |
| 1832 | 3-pyridine | H | m-C(O)NH- | 2-dimethylamino-5CF₃-phenyl |
| 1833 | 2-CH₃-phenyl | -(CH₂)₃-morpholine | m-C(O)NH- | 1piperidinylpropyloxyphenyl |
| 1834 | 4-CH₃-phenyl | -(CH₂)₃-morpholine | m-C(O)NH- | cyclopropyl |
| 1835 | 5-CH₃-phenyl | -(CH₂)₃-morpholine | m-C(O)NH- | 2-(3-dimethylaminopropyl)methylamino-5-CF₃-phenyl |
| 1836 | 6-CH₃-phenyl | -(CH₂)₃-morpholine | m-C(O)NH- | 2-dimethylamino-5-CF₃-phenyl |
| 1837 | 2-OCH₃-phenyl | -(CH₂)₃-morpholine | m-C(O)NH- | 1-piperidinylpropyloxyphenyl |
| 1838 | 4-OCH₃-phenyl | -(CH₂)₃-morpholine | m-C(O)NH- | cyclopropyl |
| 1839 | 5-OCH₃-phenyl | -(CH₂)₃-morpholine | m-C(O)NH- | 2-(3-dimethylaminopropyl)methylamino-5-CF₃-phenyl |
| 1840 | 6-OCH₃-phenyl | -(CH₂)₃-morpholine | m-C(O)NH- | 2-dimethylamino-5-CF₃-phenyl |
| 1841 | 2-F-phenyl | -(CH₂)₃-morpholine | m-C(O)NH- | 1-piperidinylpropyloxyphenyl |
| 1842 | 4-F-phenyl | -(CH₂)₃-morpholine | m-C(O)NH- | cyclopropyl |
| 1843 | 5-F-phenyl | -(CH₂)₃-morpholine | m-C(O)NH- | 2-(3-dimethylaminopropyl)methylamino-5-CF₃-phenyl |
| 1844 | 6-F-phenyl | -(CH₂)₃-morpholine | m-C(O)NH- | 2-dimethylamino-5-CF₃-phenyl |
| 1845 | 2-thiophene | -(CH₂)₃-morpholine | m-C(O)NH- | 1-piperidinylpropyloxyphenyl |
| 1846 | 3-thiophene | -(CH₂)₃-morpholine | m-C(O)NH- | cyclopropyl |
| 1847 | 2-pyridine | -(CH₂)₃-morpholine | m-C(O)NH- | 2-(3-dimethylaminopropyl)methylamino-5-CF3-phenyl |
| 1848 | 3-pyridine | -(CH₂)₃-morpholine | m-C(O)NH- | 2-dimethylamino-5-CF₃-phenyl |
| 1849 | 2-CH₃-phenyl | CH₃ | m-C(O)NH- | 1-piperidinylpropyloxyphenyl |
| 1850 | 4-CH₃-phenyl | CH₃ | m-C(O)NH- | cyclopropyl |
| 1851 | 5-CH₃-phenyl | CH₃ | m-C(O)NH- | 2-(3-dimethylaminopropyl)methylamino-5-CF₃-phenyl |
| 1852 | 6-CH₃-phenyl | CH₃ | m-C(O)NH- | 2-dimethylamino-5-CF₃-phenyl |
| 1853 | 2-OCH₃-phenyl | CH₃ | m-C(O)NH- | 1-piperidinylpropyloxyphenyl |
| 1854 | 4-OCH₃-phenyl | CH₃ | m-C(O)NH- | cyclopropyl |
| 1855 | 5-OCH₃-phenyl | CH₃ | m-C(O)NH- | 2-(3-dimethylaminopropyl)methylamino-5-CF₃-phenyl |
| 1856 | 6-OCH₃phenyl | CH₃ | m-C(O)NH- | 2-dimethylamino-5-CF₃-phenyl |
| 1857 | 2-F-phenyl | CH₃ | mC(O)NH- | 1-piperidinylpropyloxyphenyl |
| 1858 | 4-F-phenyl | CH₃ | m-C(O)NH- | cyclopropyl |
| 1859 | 5-F-phenyl | CH₃ | mC(O)NH- | 2-(3-dimethylaminopropyl)methylamino-5-CF₃-phenyl |
| 1860 | 6-F-phenyl | CH₃ | m-C(O)NH- | 2-dimethylamino-5-CF₃-phenyl |
| 1861 | 2-thiophene | CH₃ | m-C(O)NH- | 1-piperidinylpropyloxyphenyl |
| 1862 | 3-thiophene | CH₃ | m-C(O)NH- | cyclopropyl |
| 1863 | 2pyridine | CH₃ | m-C(O)NH- | 2-(3-dimethylaminopropyl)methylamino-5-CF₃-phenyl |
| 1864 | 3-pyridine | CH₃ | m-C(O)NH- | 2-dimethylamino-5-CF₃-phenyl |
| 1865 | 2-CH₃phenyl | -O(CH₂)₂-piperidine | m-C(O)NH- | 1-piperidinylpropyloxyphenyl |
| 1866 | 4-CH₃-phenyl | -O(CH₂)₂-piperidine | m-C(O)NH- | cyclopropyl |
| 1867 | 5-CH₃-phenyl | -O(CH₂)₂-piperidine | m-C(O)NH- | 2-(3-dimethylaminopropyl)methylamino-5-CF₃-phenyl |
| 1868 | 6-CH₃phenyl | -O(CH₂)₂-piperidine | m-C(O)NH- | 2-dimethylamino-5-CF₃-phenyl |
| 1869 | 2-OCH₃-phenyl | -O(CH₂)₂-piperidine | mC(O)NH- | 1-piperidinylpropyloxyphenyl |
| 1870 | 4-OCH₃-phenyl | -O(CH₂)₂-piperidine | m-C(O)NH- | cyclopropyl |
| 1871 | 5-OCH₃-phenyl | -O(CH₂)₂-piperidine | m-C(O)NH- | 2-(3-dimethylaminopropyl)methylamino-S-CF₃-phenyl |
| 1872 | 6-OCH₃phenyl | -O(CH₂)₂-piperidine | m-C(O)NH- | 2-dimethylamino-5CF₃-phenyl |
| 1873 | 2-F-phenyl | -O(CH₂)₂-piperidine | m-C(O)NH- | 1-piperidinylpropyloxyphenyl |
| 1874 | 4-F-phenyl | -O(CH₂)₂-piperidine | m-C(O)NH- | cyclopropyl |
| 1875 | 5-F-phenyl | -O(CH₂)₂-piperidine | m-C(O)NH- | 2-(3-dimethylaminopropyl)methylamino-5-CF₃-phenyl |
| 1876 | 6-F-phenyl | -O(CH₂)₂-piperidine | m-C(O)NH- | 2-dimethylamino-5-CF₃-phenyl |
| 1877 | 2-thiophene | -O(CH₂)₂-piperidine | m-C(O)NH- | 1-piperidinylpropyloxyphenyl |
| 1878 | 3-thiophene | -O(CH₂)₂-piperidine | m-C(O)NH- | cyclopropyl |
| 1879 | 2-pyridine | -O(CH₂)₂-piperidine | m-C(O)NH- | 2-(3-dimethylaminopropyl)methylamino-5-CF₃-phenyl |
| 1880 | 3-pyridine | -O(CH₂)₂-piperidine | m-C(O)NH- | |
| 1881 | 2-CH₃-phenyl | -O(CH₂)₂piperzine | m-C(O)NH- | 1-piperidinylpropyloxyphenyl |
| 1882 | 4-CH₃-phenyl | -O(CH₂)₂-piperzine | mC(O)NH- | cyclopropyl |
| 1883 | 5-CH₃-phenyl | -O(CH₂)₂-piperzine | m-C(O)NH- | 2-(3-dimethylaminopropyl)methylamino-5-CF₃-phenyl |
| 1884 | 6-CH₃-phenyl | -O(CH₂)₂-piperzine | m-C(O)NH- | 2-dimethylamino-5-CF₃-phenyl |
| 1885 | 2-OCH₃phenyl | -O(CH₂)₂-piperzine | m-C(O)NH- | 1-piperidinylpropyloxyphenyl |
| 1886 | 4-OCH₃-phenyl | -O(CH₂)₂-piperzine | mC(O)NH- | cyclopropyl |
| 1887 | 5-OCH₃-phenyl | -O(CH₂)₂-piperzine | m-C(O)NH- | 2-(3-dimethylaminopropyl)methylamino-5-CF3-phenyl |
| 1888 | 6-OCH₃-phenyl | -O(CH₂)₂piperzine | m-C(O)NH- | 2-dimethylamino-5-CF₃-phenyl |
| 1889 | 2-Fphenyl | -O(CH₂)₂-piperzine | m-C(O)NH- | 1-piperidinylpropyloxyphenyl |
| 1890 | 4-Fphenyl | -O(CH₂)₂-piperzine | m-C(O)NH- | cyclopropyl |
| 1891 | 5-F-phenyl | -O(CH₂)₂piperzine | m-C(O)NH- | 2-(3-dimethylaminopropyl)methylamino-5-CF₃-phenyl |
| 1892 | 6-Fphenyl | -O(CH₂)₂-piperzine | m-C(O)NH- | 2-dimethylamino-5-CF₃-phenyl |
| 1893 | 2-thiophene | -O(CH₂)₂-piperzine | m-C(O)NH- | 1-piperidinylpropyloxyphenyl |
| 1894 | 3-thiophene | -O(CH₂)₂-piperzine | m-C(O)NH- | cyclopropyl |
| 1895 | 2-pyridine | -O(CH₂)₂-piperzine | mC(O)NH- | 2-(3-dimethylaminopropyl)methylamino-5-CF₃-phenyl |
| 1896 | 3-pyridine | -O(CH₂)₂-piperzine | m-C(O)NH- | 2-dimethylamino-5-CF₃-phenyl |
| 1897 | 2-CH₃-phenyl | 1-pyrrolidinylethyl | m-C(O)NH- | 1piperidinylpropyloxyphenyl |
| 1898 | 4-CH₃-phenyl | 1-pyrrolidinylethyl | m-C(O)NH- | cyclopropyl |
| 1899 | 5-CH₃-phenyl | 1-pyrrolidinylethyl | m-C(O)NH- | 2-(3dimethylaminopropyl)methylamino-5-CF₃-phenyl |
| 1900 | 6-CH₃-phenyl | 1-pyrrolidinylethyl | m-C(O)NH- | 2-dimethylamino-5CF₃-phenyl |
| 1901 | 2-OCH₃-phenyl | 1-pyrrolidinylethyl | m-C(O)NH- | 1-piperidinylpropyloxyphenyl |
| 1902 | 4-OCH₃phenyl | 1-pyrrolidiny lethyl | m-C(O)NH- | cyclopropyl |
| 1903 | 5-OCH₃-phenyl | 1-pyrrolidinylethyl | m-C(O)NH- | 2-(3-dimethylaminopropyl)methylamino-5-CF₃-phenyl |
| 1904 | 6-OCH₃-phenyl | 1-pyrrolidinylethyl | m-C(O)NH- | 2-dimethylamino-5-CF₃-phenyl |
| 1905 | 2-F-phenyl | 1-pyrrolidinylethyl | m-C(O)NH- | 1-piperidinylpropyloxyphenyl |
| 1906 | 4-F-phenyl | 1-pyrrolidinylethyl | m-C(O)NH- | cyclopropyl |
| 1907 | 5-F-phenyl | 1-pyrrolidinylethyl | m-C(O)NH- | 2-(3-dimethylaminopropyl)methylamino-5-CF₃-phenyl |
| 1908 | 6-F-phenyl | 1-pyrrolidinylethyl | m-C(O)NH- | 2-dimethylamino-5-CF₃-phenyl |
| 1909 | 2-thiophene | 1-pyrrolidinylethyl | m-C(O)NH- | 1piperidinylpropyloxyphenyl |
| 1910 | 3-thiophene | 1-pyrrolidinylethyl | m-C(O)NH- | cyclopropyl |
| 1911 | 2-pyridine | 1-pyrrolidinylethyl | mC(O)NH- | 2-(3-dimethylaminopropyl)methylamino-5-CF₃-phenyl |
| 1912 | 3-pyridine | 1-pyrrolidinylethyl | m-C(O)NH- | 2-dimethylamino-5-CF₃-phenyl |
| 1913 | 2-CH₃-phenyl | H | p-NHC(O)NH- | 1-piperidinylpropyloxyphenyl |
| 1914 | 4-CH₃phenyl | H | p-NHC(O)NH- | cyclopropyl |
| 1915 | 5-CH₃-phenyl | H | pNHC(O)NH- | 2-(3-dimethylaminopropyl)methylamino-5-CF₃-phenyl |
| 1916 | 6-CH₃-phenyl | H | p-NHC(O)NH- | 2-dimethylamino-5-CF₃-phenyl |
| 1917 | 2-OCH₃-phenyl | H | p-NHC(O)NH | 1-piperidinylpropyloxyphenyl |
| 1918 | 4-OCH₃-phenyl | H | p-NHC(O)NH- | cyclopropyl |
| 1919 | 5-(CH₃-phenyl | H | p-NHC(O)NH- | 2-(3-dimethylaminopropyl)methylamino-5-CF₃-phenyl |
| 1920 | 6-OCH₃-phenyl | H | pNHC(O)NH- | 2-dimethylamino-5-CF₃-phenyl |
| 1921 | 2-F-phenyl | H | p-NHC(O)NH- | 1-piperidinylpropyloxyphenyl |
| 1922 | 4-F-phenyl | H | pNHC(O)NH- | cyclopropyl |
| 1923 | 5-F-phenyl | H | p-NHC(O)NH- | 2-(3-dimethylaminopropyl)methylamino-5-CF₃-phenyl |
| 1924 | 6-F-phenyl | H | p-NHC(O)NH- | 2-dimethylamino-5-CF₃-phenyl |
| 1925 | 2-thiophene | H | pNHC(O)NH- | 1-piperidinylpropyloxyphenyl |
| 1926 | 3-thiophene | H | p-NHC(O)NH- | cyclopropyl |
| 1927 | 2-pyridine | H | p-NHC(O)NH- | 2-(3-dimethylaminopropyl)methylamino-5-CF₃-phenyl |
| 1928 | 3-pyridine | H | p-NHC(O)NH- | 2-dimethylamino-5-CF₃-phenyl |
| 1929 | 2-CH₃-phenyl | H | p-NHC(O)NH- | 1-piperidinylpropyloxyphenyl |
| 1930 | 4-CH₃-phenyl | H | p-NHC(O)NH- | cyclopropyl |
| 1931 | 5-CH₃-phenyl | H | p-NHC(O)NH- | 2-(3-dimethylaminopropyl)methylamino-5-CF₃-phenyl |
| 1932 | 6-CH₃-phenyl | H | p-NHC(O)NH- | 2-dimethylamino-5-CF₃-phenyl |
| 1933 | 2-OCH₃-phenyl | H | p-NHC(O)NH- | 1-piperidinylpropyloxyphenyl |
| 1934 | 4-OCH₃-phenyl | H | p-NHC(O)NH- | cyclopropyl |
| 1935 | 5-OCH₃-phenyl | H | p-NHC(O)NH- | 2-(3-dimethylaminopropyl)methylamino-5-CF₃-phenyl |
| 1936 | 6-OCH₃-phenyl | H | p-NHC(O)NH- | 2-dimethylamino-5-CF₃-phenyl |
| 1937 | 2-F-phenyl | H | p-NHC(O)NH- | 1-piperidinylpropyloxyphenyl |
| 1938 | 4-F-phenyl | H | p-NHC(O)NH- | cyclopropyl |
| 1939 | 5-F-phenyl | H | p-NHC(O)NH- | 2-(3-dimethylaminopropyl)methylamino-5-CF₃-phenyl |
| 1940 | 6-F-phenyl | H | p-NHC(O)NH- | 2-dimethylamino-5-CF₃-phenyl |
| 1941 | 2-thiophene | H | p-NHC(O)NH- | 1-piperidinylpropyloxyphenyl |
| 1942 | 3-thiophene | H | p-NHC(O)NH- | cyclopropyl |
| 1943 | 2-pyridine | H | p-NHC(O)NH- | 2-(3-dimethylaminopropyl)methylamino-5-CF₃-phenyl |
| 1944 | 3-pyridine | H | p-NHC(O)NH- | 2-dimethylamino-5-CF₃-phenyl |
| 1945 | 2-CH₃-phenyl | -(CH₂)₃-morpholine | p-NHC(O)NH- | 1-piperidinylpropyloxyphenyl |
| 1946 | 4-CH₃-phenyl | -(CH₂)₃-morpholine | p-NHC(O)NH- | cyclopropyl |
| 1947 | 5-CH₃-phenyl | -(CH₂)₃-morpholine | p-NHC(O)NH- | 2-(3-dimethylaminopropyl)methylamino-5-CF₃-phenyl |
| 1948 | 6-CH₃-phenyl | -(CH₂)₃-morpholine | pNHC(O)NH- | 2-dimethylamino-5-CF₃-phenyl |
| 1949 | 2-OCH₃-phenyl | -(CH₂)₃-morpholine | p-NHC(O)NH- | 1-piperidinylpropyloxyphenyl |
| 1950 | 4-OCH₃phenyl | -(CH₂)₃-morpholine | p-NHC(O)NH- | cyclopropyl |
| 1951 | 5-OCH₃-phenyl | -(CH₂)₃-morpholine | P-NHC(O)NH- | 2-(3-dimethylaminopropyl)methylamino-5-CF₃-phenyl |
| 1952 | 6-OCH₃-phenyl | -(CH₂)₃-morpholine | p-NHC(O)NH- | 2-dimethylamino-5-CF₃-phenyl |
| 1953 | 2-F-phenyl | -(CH₂)₃-morpholine | p-NHC(O)NH- | 1-piperidinylpropyloxyphenyl |
| 1954 | 4-F-phenyl | -(CH₂)₃-morpholine | p-NHC(O)NH- | cyclopropyl |
| 1955 | 5-F-phenyl | -(CH₂)₃-morpholine | p-NHC(O)NH- | 2-(3-dimethylaminopropyl)methylamino-5-CF₃-phenyl |
| 1956 | 6-F-phenyl | -(CH₂)₃-morpholine | p-NHC(O)NH- | 2-dimethylamino-5-CF₃-phenyl |
| 1957 | 2-thiophene | -(CH₂)₃-morpholine | P-NHC(O)NH- | 1-piperidinylpropyloxyphenyl |
| 1958 | 3-thiophene | -(CH₂)₃-morpholine | p-NHC(O)NH- | cyclopropyl |
| 1959 | 2-pyridine | -(CH₂)₃-morpholine | P-NHC(O)NH- | 2-(3-dimethylaminopropyl)methylamino-5-CF₃-phenyl |
| 1960 | 3-pyridine | -(CH₂)₃-morpholine | p-NHC(O)NH- | 2-dimethylamino-5-CF₃-phenyl |
| 1961 | 2-CH₃-phenyl | CH₃ | p-NHC(O)NH- | 1-piperidinylpropyloxyphenyl |
| 1962 | 4-CH₃-phenyl | CH₃ | p-NHC(O)NH- | cyclopropyl |
| 1963 | 5-CH₃-phenyl | CH₃ | p-NHC(O)NH- | 2-(3-dimethylaminopropyl)methylamino-5-CF₃-phenyl |
| 1964 | 6-CH₃-phenyl | CH₃ | p-NHC(O)NH- | 2-dimethylamino-5-CF₃-phenyl |
| 1965 | 2-OCH₃-phenyl | CH₃ | p-NHC(O)NH- | 1-piperidinylpropyloxyphenyl |
| 1966 | 4-OCH₃-phenyl | CH₃ | p-NHC(O)NH- | cyclopropyl |
| 1967 | 5-OCH₃-phenyl | CH₃ | p-NHC(O)NH- | 2-(3-dimethylaminopropyl)methylamino-5-CF3-phenyl |
| 1968 | 6-OCH₃-phenyl | CH₃ | p-NHC(O)NH- | 2-dimethylamino-5-CF₃-phenyl |
| 1969 | 2-F-phenyl | CH₃ | p-NHC(O)NH- | 1-piperidinylpropyloxyphenyl |
| 1970 | 4-F-phenyl | CH₃ | p-NHC(O)NH- | cyclopropyl |
| 1971 | 5-F-phenyl | CH₃ | p-NHC(O)NH- | 2-(3-dimethylaminopropyl)methylamino-5-CF₃-phenyl |
| 1972 | 6-F-phenyl | CH₃ | p-NHC(O)NH- | 2-dimethylamino-5-CF₃-phenyl |
| 1973 | 2-thiophene | CH₃ | p-NHC(O)NH- | 1-piperidinylpropyloxyphenyl |
| 1974 | 3-thiophene | CH₃ | p-NHC(O)NH- | cyclopropyl |
| 1975 | 2-pyridine | CH₃ | p-NHC(O)NH- | 2-(3-dimethylaminopropyl)methylamino-5-CF₃-phenyl |
| 1976 | 3-pyridine | CH₃ | p-NHC(O)NH- | 2-dimethylamino-5-CF₃-phenyl |
| 1977 | 2-CH₃-phenyl | -O(CH₂)₂-piperidine | p-NHC(O)NH- | 1-piperidinylpropyloxyphenyl |
| 1978 | 4-(CH₃-phenyl | -O(CH₂)₂-piperidine | p-NHC(O)NH- | cyclopropyl |
| 1979 | 5-CH₃-phenyl | -O(CH₂)₂-piperidine | p-NHC(O)NH- | 2-(3-dimethylaminopropyl)methylamino-5-CF₃-phenyl |
| 1980 | 6-CH₃-phenyl | -O(CH₂)₂-piperidine | p-NHC(O)NH- | 2-dimethylamino-5-CF₃-phenyl |
| 1981 | 2-OCH₃-phenyl | -O(CH₂)₂-piperidine | p-NHC(O)NH- | 1-piperidinylpropyloxyphenyl |
| 1982 | 4-(CH₃-phenyl | -O(CH₂)₂-piperidine | p-NHC(O)NH- | cyclopropyl |
| 1983 | 5-OCH₃-phenyl | -O(CH₂)₂-piperidine | p-NHC(O)NH- | 2-(3-dimethylaminopropyl)methylamino-5-CF₃-phenyl |
| 1984 | 6-OCH₃-phenyl | -O(CH₂)₂-piperidine | p-NHC(O)NH- | 2-dimethylamino-5-CF₃-phenyl |
| 1985 | 2-F-phenyl | -O(CH₂)₂-piperidine | p-NHC(O)NH- | 1-piperidinylpropyloxyphenyl |
| 1986 | 4-F-phenyl | -O(CH₂)₂-piperidine | p-NHC(O)NH- | cyclopropyl |
| 1987 | 5-F-phenyl | -O(CH₂)₂-piperidine | p-NHC(O)NH- | 2-(3-dimethylaminopropyl)methylamino-5-CF₃-phenyl |
| 1988 | 6-F-phenyl | -O(CH₂)₂-piperidine | p-NHC(O)NH- | 2-dimethylamino-5-CF₃-phenyl |
| 1989 | 2-thiophene | -O(CH₂)₂-piperidine | p-NHC(O)NH- | 1-piperidinylpropyloxyphenyl |
| 1990 | 3-thiophene | -O(CH₂)₂-piperidine | p-NHC(O)NH- | cyclopropyl |
| 1991 | 2-pyridine | -O(CH₂)₂-piperidine | p-NHC(O)NH- | 2-(3-dimethylaminopropyl)methylamino-5-CF₃-phenyl |
| 1992 | 3-pyridine | -O(CH₂)₂-piperidine | p-NHC(O)NH- | 2-dimethylamino-5-CF₃-phenyl |
| 1993 | 2-CH₃-phenyl | -O(CH₂)₂piperzine | p-NHC(O)NH- | 1-piperidinylpropyloxyphenyl |
| 1994 | 4-CH₃-phenyl | -O(CH₂)₂-piperzine | pNHC(O)NH- | cyclopropyl |
| 1995 | 5-CH₃-phenyl | -O(CH₂)₂-piperzine | pNHC(O)NH- | 2-(3-dimethylaminopropyl)methylamino-5-CF₃-phenyl |
| 1996 | 6-CH₃-phenyl | -O(CH₂)₂-piperzine | pNHC(O)NH- | 2-dimethylamino-5-CF₃-phenyl |
| 1997 | 2-OCH₃-phenyl | -O(CH₂)₂-piperzine | p-NHC(O)NH- | 1-piperidinylpropyloxyphenyl |
| 1998 | 4-OCH₃-phenyl | -O(CH₂)₂-piperzine | p-NHC(O)NH- | cyclopropyl |
| 1999 | 5-OCH₃phenyl | -O(CH₂)₂-piperzine | p-NHC(O)NH- | 2-(3dimethylaminopropyl)methylamino-5-CF₃-phenyl |
| 2000 | 6-OCH₃-phenyl | -O(CH₂)₂-piperzine | pNHC(O)NH- | 2-dimethylamino-5-CF₃-phenyl |
| 2001 | 2-Fphenyl | -O(CH₂)₂-piperzine | p-NHC(O)NH- | 1-piperidinylpropyloxyphenyl |
| 2002 | 4-Fphenyl | -O(CH₂)₂-piperzine | p-NHC(O)NH- | cyclopropyl |
| 2003 | 5-Fphenyl | -O(CH₂)₂-piperzine | p-NHC(O)NH- | 2-(3-dimethylaminopropyl)methylamino-5-CF₃-phenyl |
| 2004 | 6-F-phenyl | -O(CH₂)₂-piperzine | p-NHC(O)NH- | 2-dimethylamino-5-CF₃-phenyl |
| 2005 | 2-thiophene | -O(CH₂)₂-piperzine | p-NHC(O)NH- | 1-piperidinylpropyloxyphenyl |
| 2006 | 3-thiophene | -O(CH₂)₂-piperzine | p-NHC(O)NH- | cyclopropyl |
| 2007 | 2-pyridine | -O(CH₂)₂-piperzine | p-NHC(O)NH- | 2-(3-dimethylaminopropyl)methylamino-5-CF₃-phenyl |
| 2008 | 3-pyridine | -O(CH₂)₂-piperzine | p-NHC(O)NH- | 2-dimethylamino-5-CF₃-phenyl |
| 2009 | 2-CH₃-phenyl | H | m-NHC(O)NH- | 1-piperidinylpropyloxyphenyl |
| 2010 | 4-CH₃-phenyl | H | m-NHC(O)NH- | cyclopropyl |
| 2011 | 5-CH₃-phenyl | H | m-NHC(O)NH- | 2-(3-dimethylaminopropyl)methylamino-5-CF₃-phenyl |
| 2012 | 6-CH₃-phenyl | H | m-NHC(O)NH- | 2-dimethylamino-5-CF₃-phenyl |
| 2013 | 2-OCH₃-phenyl | H | m-NHC(O)NH- | 1-piperidinylpropyloxyphenyl |
| 2014 | 4-OCH₃-phenyl | H | m-NHC(O)NH- | cyclopropyl |
| 2015 | 5-OCH₃-phenyl | H | m-NHC(O)NH- | 2-(3-dimethylaminopropyl)methylamino-5-CF3-phenyl |
| 2016 | 6-OCH₃-phenyl | H | mNHC(O)NH- | 2-dimethylamino-5-CF₃-phenyl |
| 2017 | 2-F-phenyl | H | m-NHC(O)NH- | 1-piperidinylpropyloxyphenyl |
| 2018 | 4-F-phenyl | H | m-NHC(O)NH- | cyclopropyl |
| 2019 | 5-F-phenyl | H | m-NHC(O)NH- | 2-(3-dimethylaminopropyl)methylamino-5-CF₃-phenyl |
| 2020 | 6-F-phenyl | H | m-NHC(O)NH- | 2-dimethylamino-5-CF₃-phenyl |
| 2021 | 2-thiophene | H | m-NHC(O)NH- | 1-piperidinylpropyloxyphenyl |
| 2022 | 3-thiophene | H | m-NHC(O)NH- | cyclopropyl |
| 2023 | 2-pyridine | H | m-NHC(O)NH- | 2-(3-dimethylaminopropyl)methylamino-5-CF₃-phenyl |
| 2024 | 3-pyridine | H | m-NHC(O)NH- | 2-dimethylamino-5-CF₃-phenyl |
| 2025 | 2-CH₃-phenyl | H | m-NHC(O)NH- | 1-piperidinylpropyloxyphenyl |
| 2026 | 4-(CH₃-phenyl | H | m-NHC(O)NH- | cyclopropyl |
| 2027 | 5-CH₃-phenyl | H | m-NHC(O)NH- | 2-(3-dimethylaminopropyl)methylamino-5-CF₃-phenyl |
| 2028 | 6-CH₃-phenyl | H | m-NHC(O)NH- | 2-dimethylamino-5-CF₃-phenyl |
| 2029 | 2-OCH₃-phenyl | H | m-NHC(O)NH- | 1-piperidinylpropyloxyphenyl |
| 2030 | 4-OCH₃-phenyl | H | m-NHC(O)NH- | cyclopropyl |
| 2031 | 5-OCH₃-phenyl | H | m-NHC(O)NH- | 2-(3-dimethylaminopropyl)methylamino-5-CF₃-phenyl |
| 2032 | 6-OCH₃-phenyl | H | m-NHC(O)NH- | 2-dimethylamino-5-CF₃-phenyl |
| 2033 | 2-F-phenyl | H | m-NHC(O)NH- | 1-piperidinylpropyloxyphenyl |
| 2034 | 4-F-phenyl | H | m-NHC(O)NH- | cyclopropyl |
| 2035 | 5-F-phenyl | H | m-NHC(O)NH- | 2-(3-dimethylaminopropyl)methylamino-5-CF₃-phenyl |
| 2036 | 6-F-phenyl | H | m-NHC(O)NH- | 2-dimethylamino-5-CF₃-phenyl |
| 2037 | 2-thiophene | H | m-NHC(O)NH- | 1-piperidinylpropyloxyphenyl |
| 2038 | 3-thiophene | H | m-NHC(O)NH- | cyclopropyl |
| 2039 | 2-pyridine | H | m-NHC(O)NH- | 2-(3-dimethylaminopropyl)methylamino-5-CF₃-phenyl |
| 2040 | 3-pyridine | H | m-NHC(O)NH- | 2-dimethylamino-5-CF₃-phenyl |
| 2041 | 2-CH₃-phenyl | -(CH₂)₃-morpholine | m-NHC(O)NH- | 1-piperidinylpropyloxyphenyl |
| 2042 | 4-CH₃-phenyl | -(CH₂)₃-morpholine | mNHC(O)NH- | cyclopropyl |
| 2043 | 5-CH₃-phenyl | -(CH₂)₃-morpholine | m-NHC(O)NH- | 2-(3-dimethylaminopropyl)methylamino-5-CF₃-phenyl |
| 2044 | 6-CH₃-phenyl | -(CH₂)₃-morpholine | m-NHC(O)NH- | 2-dimethylamino-5-CF₃-phenyl |
| 2045 | 2-OCH₃-phenyl | -(CH₂)₃-morpholine | m-NHC(O)NH- | 1-piperidinylpropyloxyphenyl |
| 2046 | 4-OCH₃-phenyl | -(CH₂)₃-morpholine | m-NHC(O)NH- | cyclopropyl |
| 2047 | 5-OCH₃phenyl | -(CH₂)₃-morpholine | m-NHC(O)NH- | 2-(3-dimethylaminopropyl)methylamino-5-CF₃-phenyl |
| 2048 | 6-OCH₃-phenyl | -(CH₂)₃morpholine | m-NHC(O)NH- | 2-dimethylamino-5-CF₃-phenyl |
| 2049 | 2-F-phenyl | -(CH₂)₃-morpholine | m-NHC(O)NH- | 1-piperidinylpropyloxyphenyl |
| 2050 | 4-F-phenyl | -(CH₂)₃-morpholine | m-NHC(O)NH- | cyclopropyl |
| 2051 | 5-F-phenyl | -(CH₂)₃-morpholine | m-NHC(O)NH- | 2-(3-dimethylaminopropyl)methylamino-5-CF₃-phenyl |
| 2052 | 6-F-phenyl | -(CH₂)₃-morpholine | m-NHC(O)NH- | 2-dimethylamino-5-CF₃-phenyl |
| 2053 | 2-thiophene | -(CH₂)₃-morpholine | m-NHC(O)NH- | 1-piperidinylpropyloxyphenyl |
| 2054 | 3-thiophene | -(CH₂)₃-morpholine | mNHC(O)NH- | cyclopropyl |
| 2055 | 2-pyridine | -(CH₂)₃-morpholine | m-NHC(O)NH- | 2-(3-dimethylaminopropyl)methylamino-5-CF3-phenyl |
| 2056 | 3-pyridine | -(CH₂)₃ morpholine | mNHC(O)NH- | 2-dimethylamino-5-CF₃-phenyl |
| 2057 | 2-CH₃-phenyl | CH₃ | m-NHC(O)NH- | 1-piperidinylpropyloxyphenyl |
| 2058 | 4-CH₃-phenyl | CH₃ | m-NHC(O)NH- | cyclopropyl |
| 2059 | 5-CH₃-phenyl | CH₃ | m-NHC(O)NH- | 2-(3-dimethylaminopropyl)methylamino-5-CF₃-phenyl |
| 2060 | 6-CH₃-phenyl | CH₃ | m-NHC(O)NH- | 2-dimethylamino-5-CF₃-phenyl |
| 2061 | 2-OCH₃-phenyl | CH₃ | m-NHC(O)NH- | 1-piperidinylpropyloxyphenyl |
| 2062 | 4-OCH₃-phenyl | CH₃ | m-NHC(O)NH- | cyclopropyl |
| 2063 | 5-OCH₃phenyl | CH₃ | m-NHC(O)NH- | 2-(3-dimethylaminopropyl)methylamino-5-CF₃-phenyl |
| 2064 | 6-OCH₃-phenyl | CH₃ | m-NHC(O)NH- | 2-dimethylamino-5CF₃-phenyl |
| 2065 | 2-F-phenyl | CH₃ | m-NHC(O)NH- | 1-piperidinylpropyloxyphenyl |
| 2066 | 4-Fphenyl | CH₃ | m-NHC(O)NH- | cyclopropyl |
| 2067 | 5-F-phenyl | CH₃ | m-NHC(O)NH- | 2-(3-dimethylaminopropyl)methylamino-5-CF3-phenyl |
| 2068 | 6-F-phenyl | CH₃ | m-NHC(O)NH- | 2-dimethylamino-5-CF₃-phenyl |
| 2069 | 2-thiophene | CH₃ | m-NHC(O)NH- | 1-piperidinylpropyloxyphenyl |
| 2070 | 3-thiophene | CH₃ | m-NHC(O)NH- | cyclopropyl |
| 2071 | 2-pyridine | CH₃ | m-NHC(O)NH- | 2-(3-dimethylaminopropyl)methylamino-5-CF₃-phenyl |
| 2072 | 3-pyridine | CH₃ | m-NHC(O)NH- | 2-dimethylamino-5-CF₃-phenyl |
| 2073 | 2-CH₃-phenyl | -O(CH₂)₂-piperidine | m-NHC(O)NH- | 1-piperidinylpropyloxyphenyl |
| 2074 | 4-(CH₃-phenyl | -O(CH₂)₂-piperidine | m-NHC(O)NH- | cyclopropyl |
| 2075 | 5-CH₃-phenyl | -O(CH₂)₂-piperidine | m-NHC(O)NH- | 2-(3dimethylaminopropyl)methylamino-5-CF₃-phenyl |
| 2076 | 6-CH₃-phenyl | -O(CH₂)₂-piperidine | m-NHC(O)NH- | 2-dimethylamino-5-CF₃-phenyl |
| 2077 | 2-OCH₃-phenyl | -O(CH₂)₂-piperidine | m-NHC(O)NH- | 1-piperidinylpropyloxyphenyl |
| 2078 | 4-OCH₃-phenyl | -O(CH₂)₂-piperidine | mNHC(O)NH- | cyclopropyl |
| 2079 | 5-OCH₃-phenyl | -O(CH₂)₂-piperidine | m-NHC(O)NH- | 2-(3-dimethylaminopropyl)methylamino-5-CF₃-phenyl |
| 2080 | 6-OCH₃-phenyl | -O(CH₂)₂piperidine | m-NHC(O)NH- | 2-dimethylamino-5-CF₃-phenyl |
| 2081 | 2-F-phenyl | -O(CH₂)₂-piperidine | m-NHC(O)NH- | 1-piperidinylpropyloxyphenyl |
| 2082 | 4-F-phenyl | -O(CH₂)₂-piperidine | m-NHC(O)NH- | cyclopropyl |
| 2083 | 5-F-phenyl | -O(CH₂)₂-piperidine | m-NHC(O)NH- | 2-(3-dimethylaminopropyl)methylamino-5-CF₃-phenyl |
| 2084 | 6-F-phenyl | -O(CH₂)₂-piperidine | m-NHC(O)NH- | 2-dimethylamino-5-CF₃-phenyl |
| 2085 | 2-thiophene | -O(CH₂)₂-piperidine | m-NHC(O)NH- | 1-piperidinylpropyloxyphenyl |
| 2086 | 3-thiophene | -O(CH₂)₂-piperidine | m-NHC(O)NH- | cyclopropyl |
| 2087 | 2-pyridine | -O(CH₂)₂-piperidine | m-NHC(O)NH- | 2-(3-dimethylaminopropyl)methylamino-5-CF₃-phenyl |
| 2088 | 3-pyridine | -O(CH₂)₂piperidine | m-NHC(O)NH- | 2-dimethylamino-5-CF₃-phenyl |
| 2089 | 2-CH₃-phenyl | -O(CH₂)₂-piperzine | m-NHC(O)NH- | 1-piperidinylpropyloxyphenyl |
| 2090 | 4-CH₃-phenyl | -O(CH₂)₂piperzine | m-NHC(O)NH- | cyclopropyl |
| 2091 | 5-CH₃-phenyl | -O(CH₂)₂-piperzine | mNHC(O)NH- | 2-(3-dimethylaminopropyl)methylamino-5-CF₃-phenyl |
| 2092 | 6-CH₃-phenyl | -O(CH₂)₂-piperzine | m-NHC(O)NH- | 2-dimethylamino-5-CF₃-phenyl |
| 2093 | 2-OCH₃-phenyl | -O(CH₂)₂-piperzine | m-NHC(O)NH- | 1-piperidinylpropyloxyphenyl |
| 2094 | 4-OCH₃-phenyl | -O(CH₂)₂piperzine | m-NHC(O)NH- | cyclopropyl |
| 2095 | 5-OCH₃-phenyl | -O(CH₂)₂-piperzine | m-NHC(O)NH- | 2-(3-dimethylaminopropyl)methylamino-5-CF₃-phenyl |
| 2096 | 6-OCH₃-phenyl | -O(CH₂)₂-piperzine | m-NHC(O)NH- | 2-dimethylamino-5-CF₃-phenyl |
| 2097 | 2-phenyl | -O(CH₂)₂-piperzine | m-NHC(O)NH- | 1-piperidinylpropyloxyphenyl |
| 2098 | 4-F-phenyl | -O(CH₂)₂-piperzine | m-NHC(O)NH- | cyclopropyl |
| 2099 | 5-F-phenyl | -O(CH₂)₂-piperzine | m-NHC(O)NH- | 2-(3-dimethylaminopropyl)methylamino-5-CF₃-phenyl |
| 2100 | 6-F-phenyl | -O(CH₂)₂-piperzine | m-NHC(O)NH- | 2-dimethylamino-5-CF₃-phenyl |
| 2101 | 2-thiophene | -O(CH₂)₂-piperzine | m-NHC(O)NH- | 1-piperidinylpropyloxyphenyl |
| 2102 | 3-thiophene | -O(CH₂)₂-piperzine | m-NHC(O)NH- | cyclopropyl |
| 2103 | 2-pyridine | -O(CH₂)₂-piperzine | m-NHC(O)NH- | 2-(3-dimethylaminopropyl)methylamino-5-CF₃-phenyl |
| 2104 | 3-pyridine | -O(CH₂)₂-piperzine | m-NHC(O)NH- | 2-dimethylamino-5-CF₃-phenyl |
| 2105 | 2-CH₃-phenyl | H | pC(O)NH- | 1-piperidinylpropyloxyphenyl |
| 2106 | 4-CH₃-phenyl | H | p-C(O)NH- | cyclopropyl |
| 2107 | 5-CH₃-phenyl | H | p-C(O)NH- | 2-(3-dimethylaminopropyl)methylamino-5-CF₃-phenyl |
| 2108 | 6-CH₃phenyl | H | p-C(O)NH- | 2-dimethylamino-5-CF₃-phenyl |
| 2109 | 2-OCH₃-phenyl | H | p-C(O)NH- | 1-piperidinylpropyloxyphenyl |
| 2110 | 4-OCH₃-phenyl | H | p-C(O)NH- | cyclopropyl |
| 2111 | 5-OCH₃-phenyl | H | p-C(O)NH- | 2-(3-dimethylaminopropyl)methylamino-5-CF₃-phenyl |
| 2112 | 6-OCH₃-phenyl | H | p-C(O)NH- | 2-dimethylamino-5-CF₃-phenyl |
| 2113 | 2-F-phenyl | H | p-C(O)NH- | 1-piperidinylpropyloxyphenyl |
| 2114 | 4-F-phenyl | H | p-C(O)NH- | cyclopropyl |
| 2115 | 5-F-phenyl | H | p-C(O)NH- | 2-(3-dimethylaminopropyl)methylamino-5-CF₃-phenyl |
| 2116 | 6-F-phenyl | H | p-C(O)NH- | 2-dimethylamino-5-CF₃-phenyl |
| 2117 | 2-thiophene | H | p-C(O)NH- | 1-piperidinylpropyloxyphenyl |
| 2118 | 3-thiophene | H | p-C(O)NH- | cyclopropyl |
| 2119 | 2-pyridine | H | p-C(O)NH- | 2-(3-dimethylaminopropyl)methylamino-5-CF₃-phenyl |
| 2120 | 3-pyridine | H | p-C(O)NH- | 2-dimethylamino-5-CF₃-phenyl |
| 2121 | 2-CH₃-phenyl | H | p-C(O)NH- | 1-piperidinylpropyloxyphenyl |
| 2122 | 4-CH₃-phenyl | H | p-C(O)NH- | cyclopropyl |
| 2123 | 5-CH₃-phenyl | H | p-C(O)NH- | 2-(3-dimethylaminopropyl)methylamino-5-CF₃-phenyl |
| 2124 | 6-CH₃-phenyl | H | p-C(O)NH- | 2-dimethylamino-5-CF₃-phenyl |
| 2125 | 2-OCH₃-phenyl | H | p-C(O)NH- | 1-piperidinylpropyloxyphenyl |
| 2126 | 4-OCH₃-phenyl | H | p-C(O)NH- | cyclopropyl |
| 2127 | 5-OCH₃-phenyl | H | p-C(O)NH- | 2-(3-dimethylaminopropyl)methylamino-5-CF₃-phenyl |
| 2128 | 6-OCH₃-phenyl | H | p-C(O)NH- | 2-dimethylamino-5-CF₃-phenyl |
| 2129 | 2-Fphenyl | H | p-C(O)NH- | 1-piperidinylpropyloxyphenyl |
| 2130 | 4-F-phenyl | H | p-C(O)NH- | cyclopropyl |
| 2131 | 5-F-phenyl | H | p-C(O)NH- | 2-(3dimethylaminopropyl)methylamino-5-CF₃-phenyl |
| 2132 | 6-F-phenyl | H | p-C(O)NH- | 2-dimethylamino-5-CF₃-phenyl |
| 2133 | 2-thiophene | H | p-C(O)NH- | 1-piperidinylpropyloxyphenyl |
| 2134 | 3-thiophene | H | p-C(O)NH- | cyclopropyl |
| 2135 | 2-pyridine | H | p-C(O)NH- | 2-(3-dimethylaminopropyl)methylamino-5-CF₃-phenyl |
| 2136 | 3-pyridine | H | p-C(O)NH- | 2-dimethylamino-5-CF₃-phenyl |
| 2137 | 2-CH₃-phenyl | -(CH₂)₃-morpholine | p-C(O)NH- | 1-piperidinylpropyloxyphenyl |
| 2138 | 4-CH₃-phenyl | -(CH₂)₃-morpholine | p-C(O)NH- | cyclopropyl |
| 2139 | 5-CH₃-phenyl | -(CH₂)₃-morpholine | p-C(O)NH- | 2-(3-dimethylaminopropyl)methylamino-5-CF₃-phenyl |
| 2140 | 6-CH₃-phenyl | -(CH₂)₃-morpholine | p-C(O)NH- | 2-dimethylamino-5-CF₃-phenyl |
| 2141 | 2-OCH₃-phenyl | -(CH₂)₃-morpholine | p-C(O)NH- | 1-piperidinylpropyloxyphenyl |
| 2142 | 4-OCH₃-phenyl | -(CH₂)₃-morpholine | p-C(O)NH- | cyclopropyl |
| 2143 | 5-OCH₃-phenyl | -(CH₂)₃-morpholine | p-C(O)NH- | 2-(3-dimethylaminopropyl)methylamino-5-CF₃-phenyl |
| 2144 | 6-OCH₃-phenyl | -(CH₂)₃-morpholine | p-C(O)NH- | 2-dimethylamino-5-CF₃-phenyl |
| 2145 | 2-F-phenyl | -(CH₂)₃-morpholine | p-C(O)NH- | 1-piperidinylpropyloxyphenyl |
| 2146 | 4-F-phenyl | -(CH₂)₃-morpholine | p-C(O)NH- | cyclopropyl |
| 2147 | 5-F-phenyl | -(CH₂)₃-morpholine | p-C(O)NH- | 2-(3-dimethylaminopropyl)methylamino-5-CF₃-phenyl |
| 2148 | 6-F-phenyl | -(CH₂)₃-morpholine | p-C(O)NH- | 2-dimethylamino-5-CF₃-phenyl |
| 2149 | 2-thiophene | -(CH₂)₃-morpholine | p-C(O)NH- | 1-piperidinylpropyloxyphenyl |
| 2150 | 3-thiophene | -(CH₂)₃-morpholine | P-C(O)NH- | cyclopropyl |
| 2151 | 2-pyridine | -(CH₂)₃-morpholine | pC(O)NH- | 2-(3-dimethylaminopropyl)methylamino-5-CF₃-phenyl |
| 2152 | 3-pyridine | -(CH₂)₃-morpholine | p-C(O)NH- | 2-dimethylamino-5CF₃-phenyl |
| 2153 | 2-CH₃-phenyl | CH₃ | p-C(O)NH- | 1-piperidinylpropyloxyphenyl |
| 2154 | 4-CH₃-phenyl | CH₃ | p-C(O)NH- | cyclopropyl |
| 2155 | 5-CH₃-phenyl | CH₃ | p-C(O)NH- | 2-(3-dimethylaminopropyl)methylamino-5-CF₃-phenyl |
| 2156 | 6-CH₃-phenyl | CH₃ | p-C(O)NH- | 2-dimethylamino-5-CF₃-phenyl |
| 2157 | 2-OCH₃-phenyl | CH₃ | p-C(O)NH- | 1-piperidinylpropyloxyphenyl |
| 2158 | 4-OCH₃-phenyl | CH₃ | p-C(O)NH- | cyclopropyl |
| 2159 | 5-OCH₃-phenyl | CH₃ | p-C(O)NH- | 2-(3-dimethylaminopropyl)methylamino-5-CF₃-phenyl |
| 2160 | 6-OCH₃-phenyl | CH₃ | p-C(O)NH- | 2-dimethylamino-5-CF₃-phenyl |
| 2161 | 2-Fphenyl | CH₃ | p-C(O)NH- | piperidinylpropyloxyphenyl |
| 2162 | 4-F-phenyl | CH₃ | p-C(O)NH- | cyclopropyl |
| 2163 | 5-F-phenyl | CH₃ | p-C(O)NH- | 2-(3-dimethylaminopropyl)methylamino-5-CF₃-phenyl |
| 2164 | 6-F-phenyl | CH₃ | p-C(O)NH- | 2-dimethylamino-5-CF₃-phenyl |
| 2165 | 2-thiophene | CH₃ | p-C(O)NH- | 1-piperidinylpropyloxyphenyl |
| 2166 | 3-thiophene | CH₃ | p-C(O)NH- | cyclopropyl |
| 2167 | 2-pyridine | CH₃ | p-C(O)NH- | 2-(3-dimethylaminopropyl)methylamino-5-CF₃-phenyl |
| 2168 | 3-pyridine | CH₃ | p-C(O)NH- | 2-dimethylamino-5-CF₃-phenyl |
| 2169 | 2-CH₃phenyl | -O(CH₂)₂-piperidine | p-C(O)NH- | 1-piperidinylpropyloxyphenyl |
| 2170 | 4-CH₃-phenyl | -O(CH₂)₂-piperidine | pC(O)NH- | cyclopropyl |
| 2171 | 5-CH₃-phenyl | -O(CH₂)₂-piperidine | pC(O)NH- | 2-(3-dimethylaminopropyl)methylamino-5-CF₃-phenyl |
| 2172 | 6-CH₃-phenyl | -O(CH₂)₂-piperidine | p-C(O)NH- | 2-dimethylamino-5-CF₃-phenyl |
| 2173 | 2-OCH₃-phenyl | -O(CH₂)₂-piperidine | p-C(O)NH- | 1-piperidinylpropyloxyphenyl |
| 2174 | 4-OCH₃-phenyl | -O(CH₂)₂-piperidine | p-C(O)NH- | cyclopropyl |
| 2175 | 5-OCH₃-phenyl | -O(CH₂)₂-piperidine | p-C(O)NH- | 2-(3-dimethylaminopropyl)methylamino-5-CF₃-phenyl |
| 2176 | 6-OCH₃-phenyl | -O(CH₂)₂-piperidine | p-C(O)NH- | 2-dimethylamino-5-CF₃-phenyl |
| 2177 | 2-F-phenyl | -O(CH₂)₂-piperidine | p-C(O)NH- | 1-piperidinylpropyloxyphenyl |
| 2178 | 4-F-phenyl | -O(CH₂)₂-piperidine | p-C(O)NH- | cyclopropyl |
| 2179 | 5-F-phenyl | -O(CH₂)₂-piperidine | p-C(O)NH- | 2-(3-dimethylaminopropyl)methylamino-5-CF₃-phenyl |
| 2180 | 6-F-phenyl | -O(CH₂)₂-piperidine | p-C(O)NH- | 2-dimethylamino-5-CF₃-phenyl |
| 2181 | 2-thiophene | -O(CH₂)₂-piperidine | p-C(O)NH- | 1-piperidinylpropyloxyphenyl |
| 2182 | 3-thiophene | -O(CH₂)₂-piperidine | p-C(O)NH- | cyclopropyl |
| 2183 | 2-pyridine | -O(CH₂)₂-piperidine | p-C(O)NH- | 2-(3-dimethylaminopropyl)methylamino-5-CF₃-phenyl |
| 2184 | 3-pyridine | -O(CH₂)₂-piperidine | p-C(O)NH- | 2-dimethylamino-5-CF₃-phenyl |
| 2185 | 2-CH₃-phenyl | -O(CH₂)₂-piperzine | p-C(O)NH- | 1-piperidinylpropyloxyphenyl |
| 2186 | 4-CH₃-phenyl | -O(CH₂)₂-piperzine | p-C(O)NH- | cyclopropyl |
| 2187 | 5-CH₃-phenyl | -O(CH₂)₂-piperzine | p-C(O)NH- | 2-(3-dimethylaminopropyl)methylamino-5-CF₃-phenyl |
| 2188 | 6-CH₃-phenyl | -O(CH₂)₂-piperzine | p-C(O)NH- | 2-dimethylamino-5-CF₃-phenyl |
| 2189 | 2-OCH₃-phenyl | -O(CH₂)₂-piperzine | p-C(O)NH- | 1-piperidinylpropyloxyphenyl |
| 2190 | 4-OCH₃-phenyl | -O(CH₂)₂-piperzine | p-C(O)NH- | cyclopropyl |
| 2191 | 5-OCH₃-phenyl | -O(CH₂)₂-piperzine | p-C(O)NH- | 2-(3-dimethylaminopropyl)methylamino-5-CF₃-phenyl |
| 2192 | 6-OCH₃-phenyl | -O(CH₂)₂-piperzine | p-C(O)NH- | 2-dimethylamino-5-CF₃-phenyl |
| 2193 | 2-F-phenyl | -O(CH₂)₂-piperzine | p-C(O)NH- | 1-piperidinylpropyloxyphenyl |
| 2194 | 4-F-phenyl | -O(CH₂)₂-piperzine | p-C(O)NH- | cyclopropyl |
| 2195 | 5-F-phenyl | -O(CH₂)₂-piperzine | p-C(O)NH- | 2-(3-dimethylaminopropyl)methylamino-5-CF₃-phenyl |
| 2196 | 6-F-phenyl | -O(CH₂)₂-piperzine | p-C(O)NH- | 2-dimethylamino-5-CF₃-phenyl |
| 2197 | 2-thiophene | -O(CH₂)₂-piperzine | p-C(O)NH- | 1-piperidinylpropyloxyphenyl |
| 2198 | 3-thiophene | -O(CH₂)₂-piperzine | p-C(O)NH- | cyclopropyl |
| 2199 | 2-pyridine | -O(CH₂)₂-piperzine | p-C(O)NH- | 2-(3-dimethylaminopropyl)methylamino-5-CF₃-phenyl |
| 2200 | 3-pyridine | -O(CH₂)₂-piperzine | p-C(O)NH- | 2-dimethylamino-5-CF₃-phenyl |
| 2201 | 2-CH₃-phenyl | 1-pyrrolidinylethyl | p-C(O)NH- | 1-piperidinylpropyloxyphenyl |
| 2202 | 4-CH₃-phenyl | 1-pyrrolidinylethyl | p-C(O)NH- | cyclopropyl |
| 2203 | 5-CH₃-phenyl | 1-pyrrolidinylethyl | p-C(O)NH- | 2-(3-dimethylaminopropyl)methylamino-5-CF₃-phenyl |
| 2204 | 6-CH₃-phenyl | 1-pyrrolidinylethyl | p-C(O)NH- | 2-dimethylamino-5CF₃-phenyl |
| 2205 | 2-OCH₃-phenyl | 1-pyrrolidinylethyl | p-C(O)NH- | 1-piperidinylpropyloxyphenyl |
| 2206 | 4-OCH₃-phenyl | 1-pyrrolidinylethyl | p-C(O)NH- | cyclopropyl |
| 2207 | 5-OCH₃-phenyl | 1-pyrrolidinylethyl | p-C(O)NH- | 2-(3-dimethylaminopropyl)methylamino-5-CF₃-phenyl |
| 2208 | 6-OCH₃-phenyl | 1-pyrrolidinylethyl | p-C(O)NH- | 2-dimethylamino-5-CF₃-phenyl |
| 2209 | 2-F-phenyl | 1-pyrrolidinylethyl | p-C (O) NH- | 1-piperidinylpropyloxyphenyl |
| 2210 | 4-F-phenyl | 1-pyrrolidinylethyl | p-C(O)NH- | cyclopropyl |
| 2211 | 5-F-phenyl | 1-pyrrolidinylethyl | p-C(O)NH- | 2-(3-dimethylaminopropyl)methylamino-5-CF₃-phenyl |
| 2212 | 6-F-phenyl | 1-pyrrolidinylethyl | p-C(O)NH- | 2-dimethylamino-5-CF₃-phenyl |
| 2213 | 2-thiophene | 1-pyrrolidinylethyl | p-C(O)NH- | 1-piperidinylpropyloxyphenyl |
| 2214 | 3-thiophene | 1-pyrrolidinylethyl | p-C(O)NH- | cyclopropyl |
| 2215 | 2pyridine | 1-pyrrolidinylethyl | p-C(O)NH- | 2-(3-dimethylaminopropyl)methylamino-5-CF₃-phenyl |
| 2216 | 3-pyridine | 1pyrrolidinylethyl | p-C(O)NH- | 2-dimethylamino-5-CF₃-phenyl |
| 2217 | 2-CH₃-phenyl | H | m-NH-C(O)- | 1-piperidinylpropyloxyphenyl |
| 2218 | 4-CH₃-phenyl | H | m-NH-C(O)- | cyclopropyl |
| 2219 | 5-CH₃-phenyl | H | m-NH-C(O)- | 2-(3-dimethylaminopropyl)methylamino-5-CF₃-phenyl |
| 2220 | 6-CH₃-phenyl | H | m-NH-C(O)- | 2-dimethylamino-5-CF₃-phenyl |
| 2221 | 2-OCH₃-phenyl | H | m-NH-C(O)- | 1-piperidinylpropyloxyphenyl |
| 2222 | 4-OCH₃-phenyl | H | m-NH-C(O)- | cyclopropyl |
| 2223 | 5-OCH₃-phenyl | H | m-NH-C(O)- | 2-(3-dimethylaminopropyl)methylamino-5-CF₃-phenyl |
| 2224 | 6-OCH₃-phenyl | H | m-NH-C(O)- | 2-dimethylamino-5CF₃-phenyl |
| 2225 | 2-F-phenyl | H | m-NH-C(O)- | 1-piperidinylpropyloxyphenyl |
| 2226 | 4-F-phenyl | H | m-NH-C(O)- | cyclopropyl |
| 2227 | 5-F-phenyl | H | m-NH-C(O)- | 2-(3-dimethylaminopropyl)methylamino-5-CF₃-phenyl |
| 2228 | 6-F-phenyl | H | m-NH-C(O)- | 2-dimethylamino-5-CF₃-phenyl |
| 2229 | 2-thiophene | H | m-NH-C(O)- | 1-piperidinylpropyloxyphenyl |
| 2230 | 3-thiophene | H | m-NH-C(O)- | cyclopropyl |
| 2231 | 2-pyridine | H | m-NH-C(O)- | 2-(3-dimethylaminopropyl)methylamino-5-CF₃-phenyl |
| 2232 | 3-pyridine | H | m-NH-C(O)- | 2-dimethylamino-5-CF₃-phenyl |
| 2233 | 2-CH₃-phenyl | H | m-NH-C(O)- | 1-piperidinylpropyloxyphenyl |
| 2234 | 4-CH₃-phenyl | H | m-NH-C(O)- | cyclopropyl |
| 2235 | 5-CH₃-phenyl | H | m-NH-C(O)- | 2-(3-dimethylaminopropyl)methylamino-5-CF₃-phenyl |
| 2236 | 6-CH₃-phenyl | H | m-NH-C(O)- | 2-dimethylamino-5-CF₃-phenyl |
| 2237 | 2-OCH₃-phenyl | H | m-NH-C(O)- | 1-piperidinylpropyloxyphenyl |
| 2238 | 4-OCH₃-phenyl | H | m-NH-C(O)- | cyclopropyl |
| 2239 | 5-OCH₃phenyl | CH | m-NH-C(O)- | 2-(3-dimethylaminopropyl)methylamino-5-CF₃-phenyl |
| 2240 | 6-OCH₃-phenyl | H | m-NH-C(O)- | 2-dimethylamino-5-CF₃-phenyl |
| 2241 | 2-F-phenyl | H | m-NH-C(O)- | 1-piperidinylpropyloxyphenyl |
| 2242 | 4-F-phenyl | H | m-NH-C(O)- | cyclopropyl |
| 2243 | 5-F-phenyl | H | m-NH-C(O)- | 2-(3-dimethylaminopropyl)methylamino-5-CF₃-phenyl |
| 2244 | 6-F-phenyl | H | m-NH-C(O)- | 2-dimethylamino-5-CF₃-phenyl |
| 2245 | 2-thiophene | H | m-NH-C(O)- | 1-piperidinylpropyloxyphenyl |
| 2246 | 3-thiophene | H | m-NH-C(O)- | cyclopropyl |
| 2247 | 2-pyridine | H | m-NH-C(O)- | 2-(3-dimethylaminopropyl)methylamino-5-CF₃-phenyl |
| 2248 | 3-pyridine | H | m-NH-C(O)- | 2-dimethylamino-5-CF₃-phenyl |
| 2249 | 2-CH₃-phenyl | -(CH₂)₃-morpholine | m-NH-C(O)- | 1-piperidinylpropyloxyphenyl |
| 2250 | 4-CH₃-phenyl | -(CH₂)₃-morpholine | m-NH-C(O)- | cyclopropyl |
| 2251 | 5-CH₃-phenyl | -(CH₂)₃-morpholine | m-NH-C(O)- | 2-(3-dimethylaminopropyl)methylamino-5-CF₃-phenyl |
| 2252 | 6-CH₃-phenyl | -(CH₂)₃-morpholine | m-NH-C(O)- | 2-dimethylamino-5-CF₃-phenyl |
| 2253 | 2-OCH₃-phenyl | -(CH₂)₃-morpholine | m-NH-C(O)- | 1-piperidinylpropyloxyphenyl |
| 2254 | 4-OCH₃-phenyl | -(CH₂)₃-morpholine | m-NH-C(O)- | cyclopropyl |
| 2255 | 5-OCH₃phenyl | -(CH₂)₃-morpholine | m-NH-C(O)- | 2-(3-dimethylaminopropyl)methylamino-5-CF₃-phenyl |
| 2256 | 6-OCH₃-phenyl | -(CH₂)₃-morpholine | m-NH-C(O)- | 2-dimethylamino-5-CF₃-phenyl |
| 2257 | 2-F-phenyl | -(CH₂)₃-morpholine | m-NHC(O)- | 1-piperidinylpropyloxyphenyl |
| 2258 | 4-F-phenyl | -(CH₂)₃-morpholine | m-NH-C(O)- | cyclopropyl |
| 2259 | 5-F-phenyl | -(CH₂)₃morpholine | m-NH-C(O)- | 2-(3dimethylaminopropyl)methylamino-5-CF₃-phenyl |
| 2260 | 6-F-phenyl | -(CH₂)₃-morpholine | m-NH-C(O)- | 2-dimethylamino-5-CF₃-phenyl |
| 2261 | 2-thiophene | -(CH₂)₃morpholine | m-NH-C(O)- | 1-piperidinylpropyloxyphenyl |
| 2262 | 3-thiophene | -(CH₂)₃-morpholine | m-NH-C(O)- | cyclopropyl |
| 2263 | 2-pyridine | -(CH₂)₃-morpholine | m-NHC(O)- | 2-(3-dimethylaminopropyl)methylamino-5-CF₃-phenyl |
| 2264 | 3-pyridine | -(CH₂)₃-morpholine | m-NH-C(O)- | 2-dimethylamino-5-CF₃-phenyl |
| 2265 | 2-CH₃-phenyl | CH₃ | m-NH-C(O)- | 1-piperidinylpropyloxyphenyl |
| 2266 | 4-(CH₃-phenyl | CH₃ | m-NH-C(O)- | cyclopropyl |
| 2267 | 5-CH₃-phenyl | CH₃ | m-NH-C(O)- | 2-(3-dimethylaminopropyl)methylamino-5-CF₃-phenyl |
| 2268 | 6-CH₃-phenyl | CH₃ | m-NH-C(O)- | 2-dimethylamino-5-CF₃-phenyl |
| 2269 | 2-OCH₃-phenyl | CH₃ | m-NH-C(O)- | 1-piperidinylpropyloxyphenyl |
| 2270 | 4-OCH₃-phenyl | CH₃ | m-NH-C(O)- | cyclopropyl |
| 2271 | 5-OCH₃-phenyl | CH₃ | m-NH-C(O)- | 2-(3-dimethylaminopropyl)methylamino-5-CF₃-phenyl |
| 2272 | 6-OCH₃-phenyl | CH₃ | m-NH-C(O)- | 2-dimethylamino-5-CF₃-phenyl |
| 2273 | 2-F-phenyl | CH₃ | m-NH-C(O)- | 1-piperidinylpropyloxyphenyl |
| 2274 | 4-F-phenyl | CH₃ | m-NH-C(O)- | cyclopropyl |
| 2275 | 5-F-phenyl | CH₃ | m-NH-C(O)- | 2-(3-dimethylaminopropyl)methylamino-5-CF₃-phenyl |
| 2276 | 6-F-phenyl | CH₃ | m-NH-C(O)- | 2-dimethylamino-5-CF₃-phenyl |
| 2277 | 2-thiophene | CH₃ | m-NH-C(O)- | 1-piperidinylpropyloxyphenyl |
| 2278 | 3-thiophene | CH₃ | m-NH-C(O)- | cyclopropyl |
| 2279 | 2-pyridine | CH₃ | m-NH-C(O)- | 2-(3-dimethylaminopropyl)methylamino-5-CF₃-phenyl |
| 2280 | 3-pyridine | CH₃ | m-NH-C(O)- | 2-dimethylamino-5-CF₃-phenyl |
| 2281 | 2-CH₃-phenyl | -O(CH₂)₂-piperidine | m-NH-C(O)- | 1-piperidinylpropyloxyphenyl |
| 2282 | 4-CH₃-phenyl | -O(CH₂)₂-piperidine | m-NH-C(O)- | cyclopropyl |
| 2283 | 5-CH₃-phenyl | -O(CH₂)₂-piperidine | m-NH-C(O)- | 2-(3-dimethylaminopropyl)methylamino-5-CF₃-phenyl |
| 2284 | 6-CH₃-phenyl | -O(CH₂)₂-piperidine | m-NH-C(O)- | 2-dimethylamino-5-CF₃-phenyl |
| 2285 | 2-OCH₃-phenyl | -O(CH₂)₂-piperidine | m-NH-C(O)- | 1-piperidinylpropyloxyphenyl |
| 2286 | 4-OCH₃-phenyl | -O(CH₂)₂-piperidine | m-NH-C(O)- | cyclopropyl |
| 2287 | 5-OCH₃-phenyl | -O(CH₂)₂-piperidine | m-NH-C(O)- | 2-(3-dimethylaminopropyl)methylamino-5-CF₃-phenyl |
| 2288 | 6-OCH₃-phenyl | -O(CH₂)₂-piperidine | m-NH-C(O)- | 2-dimethylamino-5-CF₃-phenyl |
| 2289 | 2-F-phenyl | -O(CH₂)₂-piperidine | m-NH-C(O)- | 1-piperidinylpropyloxyphenyl |
| 2290 | 4-F-phenyl | -O(CH₂)₂-piperidine | m-NH-C(O)- | cyclopropyl |
| 2291 | 5-F-phenyl | -O(CH₂)₂-piperidine | m-NH-C(O)- | 2-(3-dimethylaminopropyl)methylamino-5-CF₃-phenyl |
| 2292 | 6-F-phenyl | -O(CH₂)₂-piperidine | m-NH-C(O)- | 2-dimethylamino-5-CF₃-phenyl |
| 2293 | 2-thiophene | -O((CH₂)₂-piperidine | m-NH-C(O)- | 1-piperidinylpropyloxyphenyl |
| 2294 | 3-thiophene | -O(CH₂)₂-piperidine | m-NH-C(O)- | cyclopropyl |
| 2295 | 2-pyridine | -O(CH₂)₂-piperidine | m-NH-C(O)- | 2-(3-dimethylaminopropyl)methylamino-5-CF₃-phenyl |
| 2296 | 3-pyridine | -O(CH₂)₂-piperidine | m-NH-C(O)- | 2-dimethylamino-5-CF₃-phenyl |
| 2297 | 2-CH₃-phenyl | -O(CH₂)₂-piperzine | m-NH-C(O)- | 1-piperidinylpropyloxyphenyl |
| 2298 | 4-CH₃-phenyl | -O(CH₂)₂-piperzine | m-NH-C(O)- | cyclopropyl |
| 2299 | 5-CH₃-phenyl | -O(CH₂)₂piperzine | m-NH-C(O)- | 2-(3-dimethylaminopropyl)methylamino-5-CF₃-phenyl |
| 2300 | 6-CH₃phenyl | -O(CH₂)₂-piperzine | m-NH-C(O)- | 2-dimethylamino-5-CF₃-phenyl |
| 2301 | 2-OCH-₃-phenyl | -O(CH₂)₂-piperzine | m-NH-C(O)- | 1-piperidinylpropyloxyphenyl |
| 2302 | 4-OCH₃phenyl | -O(CH₂)₂-piperzine | m-NH-C(O)- | cyclopropyl |
| 2303 | 5-OCH₃-phenyl | -O(CH₂)₂-piperzine | m-NH-C(O)- | 2-(3-dimethylaminopropyl)methylamino-5-CF₃-phenyl |
| 2304 | 6-OCH₃-phenyl | -O(CH₂)₂-piperzine | m-NH-C(O)- | 2-dimethylamino-5-CF₃-phenyl |
| 2305 | 2-Fphenyl | -O(CH₂)₂-piperzine | m-NH-C(O)- | 1-piperidinylpropyloxyphenyl |
| 2306 | 4-F-phenyl | -O(CH₂)₂-piperzine | m-NH-C(O)- | cyclopropyl |
| 2307 | 5-F-phenyl | -O(CH₂)₂-piperzine | m-NH-C(O)- | 2-(3-dimethylaminopropyl)methylamino-5-CF₃-phenyl |
| 2308 | 6-F-phenyl | -O(CH₂)₂-piperzine | m-NH-C(O)- | 2-dimethylamino-5-CF₃-phenyl |
| 2309 | 2-thiophene | -O(CH₂)₂-piperzine | m-NH-C(O)- | 1-piperidinylpropyloxyphenyl |
| 2310 | 3-thiophene | -O(CH₂)₂-piperzine | m-NH-C(O)- | cyclopropyl |
| 2311 | 2-pyridine | -O(CH₂)₂-piperzine | m-NH-C(O)- | 2-(3-dimethylaminopropyl)methylamino-5-CF₃-phenyl |
| 2312 | 3-pyridine | -O(CH₂)₂-piperzine | m-NH-C(O)- | 2-dimethylamino-5-CF₃-phenyl |
| 2313 | 2-CH₃-phenyl | 1-pyrrolidinylethyl | m-NH-C(O)- | 1-piperidinylpropyloxyphenyl |
| 2314 | 4-CH₃-phenyl | 1-pyrrolidinylethyl | m-NH-C(O)- | cyclopropyl |
| 2315 | 5-CH₃-phenyl | 1-pyrrolidinylethyl | m-NH-C(O)- | 2-(3-dimethylaminopropyl)methylamino-5-CF₃-phenyl |
| 2316 | 6-CH₃-phenyl | 1-pyrrolidinylethyl | m-NH-C(O)- | 2-dimethylamino-5-CF₃-phenyl |
| 2317 | 2-OCH₃-phenyl | 1-pyrrolidinylethyl | m-NH-C(O)- | 1-piperidinylpropyloxyphenyl |
| 2318 | 4-OCH₃-phenyl | 1-pyrrolidinylethyl | m-NH-C(O)- | cyclopropyl |
| 2319 | 5-OCH₃-phenyl | 1-pyrrolidinylethyl | m-NH-C(O)- | 2-(3-dimethylaminopropyl)methylamino-5-CF₃-phenyl |
| 2320 | 6-OCH₃-phenyl | 1-pyrrolidinylethyl | m-NH-C(O)- | 2-dimethylamino-5-CF₃-phenyl |
| 2321 | 2-F-phenyl | 1-pyrrolidinylethyl | m-NH-C(O)- | 1-piperidinylpropyloxyphenyl |
| 2322 | 4-F-phenyl | 1-pyrrolidinylethyl | m-NH-C(O)- | cyclopropyl |
| 2323 | 5-F-phenyl | 1-pyrrolidinylethyl | m-NH-C(O)- | 2-(3-dimethylaminopropyl)methylamino-5-CF₃-phenyl |
| 2324 | 6-F-phenyl | 1-pyrrolidinylethyl | m-NH-C(O)- | 2-dimethylamino-5-CF₃-phenyl |
| 2325 | 2-thiophene | 1-pyrrolidinylethyl | m-NH-C(O)- | 1-piperidinylpropyloxyphenyl |
| 2326 | 3-thiophene | 1-pyrrolidinylethyl | m-NH-C(O)- | cyclopropyl |
| 2327 | 2-pyridine | 1-pyrrolidinylethyl | m-NH-C(O)- | 2-(3-dimethylaminopropyl)methylamino-5-CF₃-phenyl |
| 2328 | 3-pyridine | 1-pyrrolidinylethyl | m-NH-C(O)- | 2-dimethylamino-5-CF₃-phenyl |
| 2329 | 2-CH₃-phenyl | H | m-C(O)NH- | 1,1-dimethylethyl-3-oxazole |
| 2330 | 4-CH₃-phenyl | H | m-C(O)NH- | 4-OCF₃-phenyl |
| 2331 | 5-CH₃-phenyl | H | m-C(O)NH- | 1,1-dimethylethyl-1-methyl-1H-pyrazole |
| 2332 | 6-CH₃-phenyl | H | m-C(O)NH- | 3,4-dimethyl-5-isoxazole |
| 2333 | 2-OCH₃-phenyl | H | m-C(O)NH- | 1,1-dimethylethyl-3-oxazole |
| 2334 | 4-OCH₃-phenyl | H | m-C(O)NH- | 4-OCF₃-phenyl |
| 2335 | 5-OCH₃-phenyl | H | m-C(O)NH- | 1,1-dimethylethyl-1-methyl-1H-pyrazole |
| 2336 | 6-OCH₃-phenyl | H | m-C(O)NH- | 3,4-dimethyl-5-isoxazole |
| 2337 | 2-Fphenyl | H | m-C(O)NH- | 1,1-dimethylethyl-3-oxazole |
| 2338 | 4-F-phenyl | H | m-C(O)NH- | 4-OCF₃-phenyl |
| 2339 | 5-F-phenyl | H | m-C(O)NH- | 1,1-dimethylethyl-1-methyl-1H-pyrazole |
| 2340 | 6-F-phenyl | H | m-C(O)NH- | 3,4-dimethyl-5-isoxazole |
| 2341 | 2-thiophene | H | m-C(O)NH- | 1,1-dimethylethyl-3-oxazole |
| 2342 | 3-thiophene | H | m-C(O)NH- | 4-OCF₃-phenyl |
| 2343 | 2-pyridine | H | m-C(O)NH- | 1,1-dimethylethyl-1-methyl-1H-pyrazole |
| 2344 | 3-pyridine | H | m-C(O)NH- | 3,4-dimethyl-5-isoxazole |
| 2345 | 2-CH₃-phenyl | H | m-C(O)NH- | 1,1-dimethylethyl-3-oxazole |
| 2346 | 4-CH₃-phenyl | H | m-C(O)NH- | 4-OCF₃-phenyl |
| 2347 | 5-CH₃-phenyl | H | m-C(O)NH- | 1,1-dimethylethyl-1-methyl-1H-pyrazole |
| 2348 | 6-CH₃-phenyl | H | m-C(O)NH- | 3,4-dimethyl-5-isoxazole |
| 2349 | 2-OCH₃-phenyl | H | m-C(O)NH- | 1,1-dimethylethyl-3-oxazole |
| 2350 | 4-OCH₃-phenyl | H | m-C(O)NH- | 4-OCF₃-phenyl |
| 2351 | 5-OCH₃-phenyl | H | m-C(O)NH- | 1,1-dimethylethyl-1-methyl-1H-pyrazole |
| 2352 | 6-OCH₃-phenyl | H | m-C(O)NH- | 3,4-dimethyl-5-isoxazole |
| 2353 | 2-Fphenyl | H | m-C(O)NH- | 1,1-dimethylethyl-3-oxazole |
| 2354 | 4-F-phenyl | H | m-C(O)NH- | 4-OCF₃-phenyl |
| 2355 | 5-F-phenyl | H | m-C(O)NH- | 1,1-dimethylethyl-1-methyl-1H-pyrazole |
| 2356 | 6-F-phenyl | H | m-C(O)NH- | 3,4-dimethyl-5-isoxazole |
| 2357 | 2-thiophene | H | m-C(O)NH- | 1,1-dimethylethyl-3-oxazole |
| 2358 | 3-thiophene | H | m-C(O)NH- | 4-OCF₃-phenyl |
| 2359 | 2-pyridine | H | m-C(O)NH- | 1,1-dimethylethyl-1-methyl-1H-pyrazole |
| 2360 | 3-pyridine | H | m-C(O)NH- | 3,4-dimethyl-5-isoxazole |
| 2361 | 2-CH₃-phenyl | -(CH₂)₃-morpholine | m-C(O)NH- | 1,1-dimethylethyl-3-oxazole |
| 2362 | 4-CH₃-phenyl | -(CH₂)₃-morpholine | m-C(O)NH- | 4-OCF₃-phenyl |
| 2363 | 5-CH₃-phenyl | -(CH₂)₃-morpholine | m-C(O)NH- | 1,1-dimethylethyl-1-methyl-1H-pyrazole |
| 2364 | 6-CH₃-phenyl | -(CH₂)₃-morpholine | m-C(O)NH- | 3,4-dimethyl-5-isoxazole |
| 2365 | 2-OCH₃-phenyl | -(CH₂)₃-morpholine | m-C(O)NH- | 1,1-dimethylethyl-3-oxazole |
| 2366 | 4-OCH₃-phenyl | -(CH₂)₃-morpholine | m-C(O)NH- | 4-OCF₃-phenyl |
| 2367 | 5-OCH₃-phenyl | -(CH₂)₃-morpholine | m-C(O)NH- | 1,1-dimethylethyl-1-methyl-1H-pyrazole |
| 2368 | 6-OCH₃-phenyl | -(CH₂)₃-morpholine | m-C(O)NH- | 3,4-dimethyl-5-isoxazole |
| 2369 | 2-F-phenyl | -(CH₂)₃-morpholine | m-C(O)NH- | 1,1-dimethylethyl-3-oxazole |
| 2370 | 4-F-phenyl | -(CH₂)₃-morpholine | m-C(O)NH- | 4-OCF₃-phenyl |
| 2371 | 5-F-phenyl | -(CH₂)₃-morpholine | m-C(O)NH- | 1,1-dimethylethyl-1-methyl-1H-pyrazole |
| 2372 | 6-F-phenyl | -(CH₂)₃-morpholine | m-C(O)NH- | 3,4-dimethyl-5-isoxazole |
| 2373 | 2-thiophene | -(CH₂)₃-morpholine | m-C(O)NH- | 1,1-dimethylethyl-3-oxazole |
| 2374 | 3-thiophene | -(CH₂)₃-morpholine | m-C(O)NH- | 4-OCF₃-phenyl |
| 2375 | 2-pyridine | -(CH₂)₃-morpholine | m-C(O)NH- | 1,1-dimethylethyl-1-methyl-1H-pyrazole |
| 2376 | 3-pyridine | -(CH₂)₃-morpholine | m-C(O)NH- | 3,4-dimethyl-5-isoxazole |
| 2377 | 2-CH₃-phenyl | CH₃ | m-C(O)NH- | 1,1-dimethylethyl-3-oxazole |
| 2378 | 4-CH₃-phenyl | CH₃ | m-C(O)NH- | 4-OCF₃-phenyl |
| 2379 | 5-CH₃-phenyl | CH₃ | m-C(O)NH- | 1,1-dimethylethyl-1-methyl-1H-pyrazole |
| 2380 | 6-CH₃-phenyl | CH₃ | m-C(O)NH- | 3,4-dimethyl-5-isoxazole |
| 2381 | 2-OCH₃-phenyl | CH₃ | m-C(O)NH- | 1,1-dimethylethyl-3-oxazole |
| 2382 | 4-OCH₃-phenyl | CH₃ | m-C(O)NH- | 4-OCF₃-phenyl |
| 2383 | 5-OCH₃-phenyl | CH₃ | m-C(O)NH- | 1,1-dimethylethyl-1-methyl-1H-pyrazole |
| 2384 | 6-OCH₃-phenyl | CH₃ | m-C(O)NH- | 3,4-dimethyl-5-isoxazole |
| 2385 | 2-F-phenyl | CH₃ | m-C(O)NH- | 1,1-dimethylethyl-3-oxazole |
| 2386 | 4-F-phenyl | CH₃ | m-C(O)NH- | 4-OCF₃-phenyl |
| 2387 | 5-F-phenyl | CH₃ | m-C(O)NH- | 1,1-dimethylethyl-1-methyl-1H-pyrazole |
| 2388 | 6-F-phenyl | CH₃ | m-C(O)NH- | 3,4-dimethyl-5-isoxazole |
| 2389 | 2-thiophene | CH₃ | m-C(O)NH- | 1,1-dimethylethyl-3-oxazole |
| 2390 | 3-thiophene | CH₃ | m-C(O)NH- | 4-OCF₃-phenyl |
| 2391 | 2-pyridine | CH₃ | m-C(O)NH- | 1,1-dimethylethyl-1-methyl-1H-pyrazole |
| 2392 | 3-pyridine | CH₃ | m-C(O)NH- | 3,4-dimethyl-5-isoxazole |
| 2393 | 2-CH₃-phenyl | -O(CH₂)₂-piperidine | m-C(O)NH- | 1,1-dimethylethyl-3-oxazole |
| 2394 | 4-CH₃-phenyl | -O(CH₂)₂-piperidine | m-C(O)NH- | 4-OCF₃-phenyl |
| 2395 | 5-CH₃-phenyl | -O(CH₂)₂-piperidine | m-C(O)NH- | 1,1-dimethylethyl-1-methyl-1H-pyrazole |
| 2396 | 6-CH₃-phenyl | -O(CH₂)₂-piperidine | m-C(O)NH- | 3,4-dimethyl-5-isoxazole |
| 2397 | 2-OCH₃-phenyl | -O(CH₂)₂-piperidine | m-C(O)NH- | 1,1-dimethylethyl-3-oxazole |
| 2398 | 4-OCH₃-phenyl | -O(CH₂)₂-piperidine | m-C(O)NH- | 4-OCF₃-phenyl |
| 2399 | 5-OCH₃-phenyl | -O(CH₂)₂-piperidine | m-C(O)NH- | 1,1-dimethylethyl-1-methyl-1H-pyrazole |
| 2400 | 6-OCH₃-phenyl | -O(CH₂)₂-piperidine | m-C(O)NH- | 3,4-dimethyl-5-isoxazole |
| 2401 | 2-F-phenyl | -O(CH₂)₂-piperidine | m-C(O)NH- | 1,1-dimethylethyl-3-oxazole |
| 2402 | 4-F-phenyl | -O(CH₂)₂-piperidine | m-C(O)NH- | 4-OCF₃-phenyl |
| 2403 | 5-F-phenyl | -O(CH₂)₂-piperidine | m-C(O)NH- | 1,1-dimethylethyl-1-methyl-1H-pyrazole |
| 2404 | 6-F-phenyl | -O(CH₂)₂-piperidine | m-C(O)NH- | 3,4-dimethyl-5-isoxazole |
| 2405 | 2-thiophene | -O(CH₂)₂-piperidine | m-C(O)NH- | 1,1-dimethylethyl-3-oxazole |
| 2406 | 3-thiophene | -O(CH₂)₂-piperidine | m-C(O)NH- | 4-OCF₃-phenyl |
| 2407 | 2-pyridine | -O(CH₂)₂-piperidine | m-C(O)NH- | 1,1-dimethylethyl-1-methyl-1H-pyrazole |
| 2408 | 3-pyridine | -O(CH₂)₂-piperidine | m-C(O)NH- | 3,4-dimethyl-5-isoxazole |
| 2409 | 2-CH₃-phenyl | -O(CH₂)₂-piperzine | m-C(O)NH- | 1,1-dimethylethyl-3-oxazole |
| 2410 | 4-CH₃-phenyl | -O(CH₂)₂-piperzine | m-C(O)NH- | 4-OCF₃-phenyl |
| 2411 | 5-CH₃-phenyl | -O(CH₂)₂-piperzine | m-C(O)NH- | 1,1-dimethylethyl-1-methyl-1H-pyrazole |
| 2412 | 6-CH₃phenyl | -O(CH₂)₂-piperzine | m-C(O)NH- | 3,4-dimethyl-5-isoxazole |
| 2413 | 2-OCH₃-phenyl | -O(CH₂)₂-piperzine | m-C(O)NH- | 1,1-dimethylethyl-3-oxazole |
| 2414 | 4-OCH₃-phenyl | -O(CH₂)₂-piperzine | mC(O)NH- | 4-OCF₃-phenyl |
| 2415 | 5-OCH₃-phenyl | -O(CH₂)₂-piperzine | m-C(O)NH- | 1,1-dimethylethyl-1-methyl-1H-pyrazole |
| 2416 | 6-OCH₃-phenyl | -O(CH₂)₂-piperzine | m-C(O)NH- | 3,4-dimethyl-5-isoxazole |
| 2417 | 2-F-phenyl | -O(CH₂)₂-piperzine | m-C(O)NH- | 1,1-dimethylethyl-3-oxazole |
| 2418 | 4-F-phenyl | -O(CH₂)₂-piperzine | m-C(O)NH- | 4-OCF₃-phenyl |
| 2419 | 5-F-phenyl | -O(CH₂)₂-piperzine | m-C(O)NH- | 1,1-dimethylethyl-1-methyl-1H-pyrazole |
| 2420 | 6-F-phenyl | -O(CH₂)₂-piperzine | m-C(O)NH- | 3,4-dimethyl-5-isoxazole |
| 2421 | 2-thiophene | -O(CH₂)₂-piperzine | m-C(O)NH- | 1,1-dimethylethyl-3-oxazole |
| 2422 | 3-thiophene | -O(CH₂)₂-piperzine | m-C(O)NH- | 4-OCF₃-phenyl |
| 2423 | 2-pyridine | -O(CH₂)₂-piperzine | m-C(O)NH- | 1,1-dimethylethyl-1-methyl-1H-pyrazole |
| 2424 | 3-pyridine | -O(CH₂)₂-piperzine | m-C(O)NH- | 3,4-dimethyl-5-isoxazole |
| 2425 | 2-CH₃-phenyl | 1-pyrrolidinylethyl | m-C(O)NH- | 1,1-dimethylethyl-3-oxazole |
| 2426 | 4-CH₃-phenyl | 1-pyrrolidinylethyl | m-C(O)NH- | 4-OCF₃-phenyl |
| 2427 | 5-CH₃-phenyl | 1-pyrrolidinylethyl | m-C(O)NH- | 1,1-dimethylethyl-1-methyl-1H-pyrazole |
| 2428 | 6-CH₃-phenyl | 1-pyrrolidinylethyl | m-C(O)NH- | 3,4-dimethyl-5-isoxazole |
| 2429 | 2-OCH₃-phenyl | 1-pyrrolidinylethyl | m-C(O)NH- | 1,1-dimethylethyl-3-oxazole |
| 2430 | 4-OCH₃-phenyl | 1-pyrrolidinylethyl | m-C(O)NH- | 4-OCF₃-phenyl |
| 2431 | 5-OCH₃-phenyl | 1-pyrrolidinylethyl | m-C(O)NH- | 1,1-dimethylethyl-1-methyl-1H-pyrazole |
| 2432 | 6-OCH₃-phenyl | 1-pyrrolidinylethyl | m-C(O)NH- | 3,4-dimethyl-5-isoxazole |
| 2433 | 2-F-phenyl | 1-pyrrolidinylethyl | m-C(O)NH- | 1,1-dimethylethyl-3-oxazole |
| 2434 | 4-F-phenyl | 1-pyrrolidinylethyl | m-C(O)NH- | 4-OCF₃-phenyl |
| 2435 | 5-F-phenyl | 1-pyrrolidinylethyl | m-C(O)NH- | 1,1-dimethylethyl-1-methyl-1H-pyrazole |
| 2436 | 6-F-phenyl | 1-pyrrolidinylethyl | m-C(O)NH- | 3,4-dimethyl-5-isoxazole |
| 2437 | 2-thiophene | 1-pyrrolidinylethyl | m-C(O)NH- | 1,1-dimethylethyl-3-oxazole |
| 2438 | 3-thiophene | 1-pyrrolidinylethyl | m-C(O)NH- | 4-OCF₃-phenyl |
| 2439 | 2-pyridine | 1pyrrolidinylethyl | m-C(O)NH- | 1,1-dimethylethyl-1-methyl-1H-pyrazole |
| 2440 | 3-pyridine | 1-pyrrolidinylethyl | m-C(O)NH- | 3,4-dimethyl-5-isoxazole |
| 2441 | 2-CH₃-phenyl | H | p-NHC(O)NH- | 1,1-dimethylethyl-3-oxazole |
| 2442 | 4-CH₃-phenyl | H | p-NHC(O)NH- | 4-OCF₃-phenyl |
| 2443 | 5-CH₃-phenyl | H | p-NHC(O)NH- | 1,1-dimethylethyl-1-methyl-1H-pyrazole |
| 2444 | 6-CH₃-phenyl | H | p-NHC(O)NH- | 3,4-dimethyl-5-isoxazole |
| 2445 | 2-OCH₃-phenyl | H | p-NHC(O)NH- | 1,1-dimethylethyl-3-oxazole |
| 2446 | 4-OCH₃-phenyl | H | p-NHC(O)NH- | 4-OCF₃-phenyl |
| 2447 | 5-OCH₃-phenyl | H | p-NHC(O)NH- | 1,1-dimethylethyl-1-methyl-1H-pyrazole |
| 2448 | 6-OCH₃-phenyl | H | p-NHC(O)NH- | 3,4-dimethyl-5-isoxazole |
| 2449 | 2-F-phenyl | H | p-NHC(O)NH- | 1,1-dimethylethyl-3-oxazole |
| 2450 | 4-F-phenyl | H | p-NHC(O)NH- | 4-OCF₃-phenyl |
| 2451 | 5-F-phenyl | H | p-NHC(O)NH- | 1,1-dimethylethyl-1-methyl-1H-pyrazole |
| 2452 | 6-F-phenyl | H | p-NHC(O)NH- | 3,4-dimethyl-5-isoxazole |
| 2453 | 2-thiophene | H | p-NHC(O)NH- | 1,1-dimethylethyl-3-oxazole |
| 2454 | 3-thiophene | H | p-NHC(O)NH- | 4-OCF₃-phenyl |
| 2455 | 2-pyridine | H | p-NHC(O)NH- | 1,1-dimethylethyl-1-methyl-1H-pyrazole |
| 2456 | 3-pyridine | H | p-NHC(O)NH- | 3,4-dimethyl-5-isoxazole |
| 2457 | 2-CH₃-phenyl | H | p-NHC(O)NH- | 1,1-dimethylethyl-3-oxazole |
| 2458 | 4-CH₃-phenyl | H | p-NHC(O)NH- | 4-OCF₃-phenyl |
| 2459 | 5-CH₃-phenyl | H | p-NHC(O)NH- | 1,1-dimethylethyl-1-methyl-1H-pyrazole |
| 2460 | 6-CH₃-phenyl | H | p-NHC(O)NH- | 3,4-dimethyl-5-isoxazole |
| 2461 | 2-OCH₃-phenyl | H | p-NHC(O)NH- | 1,1-dimethylethyl-3-oxazole |
| 2462 | 4-OCH₃-phenyl | H | p-NHC(O)NH- | 4-OCF₃-phenyl |
| 2463 | 5-OCH₃-phenyl | H | p-NHC(O)NH- | 1,1-dimethylethyl-1-methyl-1H-pyrazole |
| 2464 | 6-OCH₃-phenyl | H | p-NHC(O)NH- | 3,4-dimethyl-5-isoxazole |
| 2465 | 2-F-phenyl | H | p-NHC(O)NH- | 1,1-dimethylethyl-3-oxazole |
| 2466 | 4-F-phenyl | H | p-NHC(O)NH- | 4-OCF₃-phenyl |
| 2467 | 5-F-phenyl | H | p-NHC(O)NH- | 1,1-dimethylethyl-1-methyl-1H-pyrazole |
| 2468 | 6-F-phenyl | H | p-NHC(O)NH- | 3,4-dimethyl-5-isoxazole |
| 2469 | 2-thiophene | H | p-NHC(O)NH- | 1,1-dimethylethyl-3-oxazole |
| 2470 | 3-thiophene | H | p-NHC(O)NH- | 4-OCF₃-phenyl |
| 2471 | 2-pyridine | H | p-NHC(O)NH- | 1,1-dimethylethyl-1-methyl-1H-pyrazole |
| 2472 | 3-pyridine | H | p-NHC(O)NH- | 3,4-dimethyl-5-isoxazole |
| 2473 | 2-CH₃-phenyl | -(CH₂)₃-morpholine | p-NHC(O)NH- | 1,1-dimethylethyl-3-oxazole |
| 2474 | 4-CH₃-phenyl | -(CH₂)₃-morpholine | p-NHC(O)NH- | 4-OCF₃-phenyl |
| 2475 | 5-CH₃-phenyl | -(CH₂)₃-morpholine | p-NHC(O)NH- | 1,1-dimethylethyl-1-methyl-1H-pyrazole |
| 2476 | 6-CH₃-phenyl | -(CH₂)₃-morpholine | p-NHC(O)NH- | 3,4-dimethyl-5-isoxazole |
| 2477 | 2-OCH₃-phenyl | -(CH₂)₃-morpholine | p-NHC(O)NH- | 1,1-dimethylethyl-3-oxazole |
| 2478 | 4-OCH₃-phenyl | -(CH₂)₃-morpholine | p-NHC(O)NH- | 4-OCF₃-phenyl |
| 2479 | 5-OCH₃-phenyl | -(CH₂)₃-morpholine | p-NHC(O)NH- | 1,1-dimethylethyl-1-methyl-1H-pyrazole |
| 2480 | 6-OCH₃-phenyl | -(CH₂)₃-morpholine | p-NHC(O)NH- | 3,4-dimethyl-5-isoxazole |
| 2481 | 2-F-phenyl | -(CH₂)₃-morpholine | p-NHC(O)NH- | 1,1-dimethylethyl-3-oxazole |
| 2482 | 4-F-phenyl | -(CH₂)₃-morpholine | p-NHC(O)NH- | 4-OCF₃-phenyl |
| 2483 | 5-F-phenyl | -(CH₂)₃-morpholine | p-NHC(O)NH- | 1,1-dimethylethyl-1-methyl-1H-pyrazole |
| 2484 | 6-F-phenyl | -(CH₂)₃-morpholine | p-NHC(O)NH- | 3,4-dimethyl-5-isoxazole |
| 2485 | 2-thiophene | -(CH₂)₃-morpholine | p-NHC(O)NH- | 1,1-dimethylethyl-3-oxazole |
| 2486 | 3-thiophene | -(CH₂)₃-morpholine | p-NHC(O)NH- | 4-OCF₃-phenyl |
| 2487 | 2-pyridine | -(CH₂)₃-morpholine | p-NHC(O)NH- | 1,1-dimethylethyl-1-methyl-1H-pyrazole |
| 2488 | 3-pyridine | -(CH₂)₃-morpholine | p-NHC(O)NH- | 3,4-dimethyl-5-isoxazole |
| 2489 | 2-CH₃-phenyl | CH₃ | p-NHC(O)NH- | 1,1-dimethylethyl-3-oxazole |
| 2490 | 4-CH₃-phenyl | CH₃ | p-NHC(O)NH- | 4-OCF₃-phenyl |
| 2491 | 5-CH₃-phenyl | CH₃ | p-NHC(O)NH- | 1,1-dimethylethyl-1-methyl-1H-pyrazole |
| 2492 | 6-CH₃-phenyl | CH₃ | p-NHC(O)NH- | 3,4-dimethyl-5-isoxazole |
| 2493 | 2-OCH₃-phenyl | CH₃ | p-NHC(O)NH- | 1,1-dimethylethyl-3-oxazole |
| 2494 | 4-OCH₃-phenyl | CH₃ | p-NHC(O)NH- | 4-OCF₃-phenyl |
| 2495 | 5-OCH₃-phenyl | CH₃ | p-NHC(O)NH- | 1,1-dimethylethyl-1-methyl-1H-pyrazole |
| 2496 | 6-OCH₃-phenyl | CH₃ | p-NHC(O)NH- | 3,4-dimethyl-5-isoxazole |
| 2497 | 2-F-phenyl | CH₃ | p-NHC(O)NH- | 1,1-dimethylethyl-3-oxazole |
| 2498 | 4-F-phenyl | CH₃ | p-NHC(O)NH- | 4-OCF₃-phenyl |
| 2499 | 5-F-phenyl | CH₃ | p-NHC(O)NH- | 1,1-dimethylethyl-1-methyl-1H-pyrazole |
| 2500 | 6-F-phenyl | CH₃ | p-NHC(O)NH- | 3,4-dimethyl-5-isoxazole |
| 2501 | 2-thiophene | CH₃ | p-NHC(O)NH- | 1,1-dimethylethyl-3-oxazole |
| 2502 | 3-thiophene | CH₃ | p-NHC(O)NH- | 4-OCF₃-phenyl |
| 2503 | 2-pyridine | CH₃ | p-NHC(O)NH- | 1,1-dimethylethyl-1-methyl-1H-pyrazole |
| 2504 | 3-pyridine | CH₃ | p-NHC(O)NH- | 3,4-dimethyl-5-isoxazole |
| 2505 | 2-CH₃-phenyl | -O(CH₂)₂-piperidine | p-NHC(O)NH- | 1,1-dimethylethyl-3-oxazole |
| 2506 | 4-CH₃-phenyl | -O(CH₂)₂-piperidine | p-NHC(O)NH- | 4-OCF₃-phenyl |
| 2507 | 5-CH₃-phenyl | -O(CH₂)₂-piperidine | p-NHC(O)NH- | 1,1-dimethylethyl-1-methyl-1H-pyrazole |
| 2508 | 6-CH₃-phenyl | -O(CH₂)₂-piperidine | p-NHC(O)NH- | 3,4-dimethyl-5-isoxazole |
| 2509 | 2-OCH₃-phenyl | -O(CH₂)₂-piperidine | p-NHC(O)NH- | 1,1-dimethylethyl-3-oxazole |
| 2510 | 4-OCH₃-phenyl | -O(CH₂)₂-piperidine | p-NHC(O)NH- | 4-OCF₃-phenyl |
| 2511 | 5-OCH₃-phenyl | -O(CH₂)₂-piperidine | p-NHC(O)NH- | 1,1-dimethylethyl-1-methyl-1H-pyrazole |
| 2512 | 6-OCH₃-phenyl | -O(CH₂)₂-piperidine | p-NHC(O)NH- | 3,4-dimethyl-5-isoxazole |
| 2513 | 2-F-phenyl | -O(CH₂)₂-piperidine | p-NHC(O)NH- | 1,1-dimethylethyl-3-oxazole |
| 2514 | 4-F-phenyl | -O(CH₂)₂-piperidine | p-NHC(O)NH- | 4-OCF₃-phenyl |
| 2515 | 5-F-phenyl | -O(CH₂)₂-piperidine | p-NHC(O)NH- | 1,1-dimethylethyl-1-methyl-1H-pyrazole |
| 2516 | 6-F-phenyl | -O(CH₂)₂-piperidine | p-NHC(O)NH- | 3,4-dimethyl-5-isoxazole |
| 2517 | 2-thiophene | -O(CH₂)₂-piperidine | p-NHC(O)NH- | 1,1-dimethylethyl-3-oxazole |
| 2518 | 3-thiophene | -O(CH₂)₂-piperidine | p-NHC(O)NH- | 4-OCF₃-phenyl |
| 2519 | 2-pyridine | -O(CH₂)₂-piperidine | p-NHC(O)NH- | 1,1-dimethylethyl-1-methyl-1H-pyrazole |
| 2520 | 3-pyridine | -O(CH₂)₂-piperidine | p-NHC(O)NH- | 3,4-dimethyl-5-isoxazole |
| 2521 | 2-CH₃-phenyl | -O(CH₂)₂-piperzine | p-NHC(O)NH- | 1,1-dimethylethyl-3-oxazole |
| 2522 | 4-CH₃-phenyl | -O(CH₂)₂-piperzine | p-NHC(O)NH- | 4-OCF₃-phenyl |
| 2523 | 5-CH₃-phenyl | -O(CH₂)₂-piperzine | p-NHC(O)NH- | 1,1-dimethylethyl-1methyl-1H-pyrazole |
| 2524 | 6-CH₃-phenyl | -O(CH₂)₂-piperzine | p-NHC(O)NH- | 3,4-dimethyl-5-isoxazole |
| 2525 | 2-OCH₃-phenyl | -O(CH₂)₂-piperzine | p-NHC(O)NH- | 1,1-dimethylethyl-3-oxazole |
| 2526 | 4-OCH₃-phenyl | -O(CH₂)₂-piperzine | p-NHC(O)NH- | 4-OCF₃-phenyl |
| 2527 | 5-OCH₃-phenyl | -O(CH₂)₂-piperzine | p-NHC(O)NH- | 1,1-dimethylethyl-1-methyl-1H-pyrazole |
| 2528 | 6-OCH₃-phenyl | -O(CH₂)₂-piperzine | p-NHC(O)NH- | 3,4-dimethyl-5-isoxazole |
| 2529 | 2-F-phenyl | -O(CH₂)₂-piperzine | p-NHC(O)NH- | 1,1-dimethylethyl-3-oxazole |
| 2530 | 4-F-phenyl | -O(CH₂)₂-piperzine | p-NHC(O)NH- | 4-OCF₃-phenyl |
| 2531 | 5-F-phenyl | -O(CH₂)₂-piperzine | p-NHC(O)NH- | 1,1-dimethylethyl-1-methyl-1H-pyrazole |
| 2532 | 6-F-phenyl | -O(CH₂)₂-piperzine | p-NHC(O)NH- | 3,4-dimethyl-5-isoxazole |
| 2533 | 2-thiophene | -O(CH₂)₂-piperzine | p-NHC(O)NH- | 1,1-dimethylethyl-3-oxazole |
| 2534 | 3-thiophene | -O(CH₂)₂-piperzine | p-NHC(O)NH- | 4-OCF₃-phenyl |
| 2535 | 2-pyridine | -O(CH₂)₂-piperzine | p-NHC(O)NH- | 1,1-dimethylethyl-1-methyl-1H-pyrazole |
| 2536 | 3-pyridine | -O(CH₂)₂-piperzine | p-NHC(O)NH- | 3,4-dimethyl-5-isoxazole |
| 2537 | | | 1,1-dimethylethyl-3- | 1,1-dimethylethyl-3-oxazole |
| 2538 | 2-CH₃-phenyl | H | m-NHC(O)NH- | 4-OCF₃-phenyl |
| 2539 | 4-CH₃-phenyl | H | m-NHC(O)NH | 1,1-dimethylethyl-1-methyl-1H-pyrazole |
| 2540 | 5-CH₃-phenyl | H | m-NHC(O)NH- | 3,4-dimethyl-5-isoxazole |
| 2541 | 6-CH₃-phenyl | H | m-NHC(O)NH- | 1,1-dimethylethyl-3-oxazole |
| 2542 | 2-OCH₃-phenyl | H | m-NHC(O)NH- | 4-OCF₃-phenyl |
| 2543 | 4-OCH₃-phenyl | H | m-NHC(O)NH- | 1,1-dimethylethyl-1-methyl-1H-pyrazole |
| 2544 | 5-OCH₃-phenyl | H | m-NHC(O)NH- | 3,4-dimethyl-5-isoxazole |
| 2545 | 6-OCH₃-phenyl | H | m-NHC(O)NH- | 1,1-dimethylethyl-3-oxazole |
| 2546 | 2-F-phenyl | H | m-NHC(O)NH- | 4-OCF₃-phenyl |
| 2547 | 4-F-phenyl | H | m-NHC(O)NH- | 1,1-dimethylethyl-1-methyl-1H-pyrazole |
| 2548 | 5-F-phenyl | H | m-NHC(O)NH- | 3,4-dimethyl-5-isoxazole |
| 2549 | 6-F-phenyl | H | m-NHC(O)NH- | 1,1-dimethylethyl-3-oxazole |
| 2550 | 2-thiophene | H | m-NHC(O)NH- | 4-OCF₃-phenyl |
| 2551 | 3-thiophene | H | m-NHC(O)NH- | 1,1-dimethylethyl-1-methyl-1H-pyrazole |
| 2552 | 2-pyridine | H | m-NHC(O)NH- | 3,4-dimethyl-5-isoxazole |
| 2553 | 3-pyridine | H | m-NHC(O)NH- | 1,1-dimethylethyl-3-oxazole |
| 2554 | 2-CH₃-phenyl | H | m-NHC(O)NH- | 4-OCF₃-phenyl |
| 2555 | 4-CH₃-phenyl | H | m-NHC(O)NH | 1,1-dimethylethyl-1-methyl-1H-pyrazole |
| 2556 | 5-CH₃-phenyl | H | m-NHC(O)NH- | 3,4-dimethyl-5-isoxazole |
| 2557 | 6-CH₃-phenyl | H | m-NHC(O)NH- | 1,1-dimethylethyl-3-oxazole |
| 2558 | 2-OCH₃-phenyl | H | m-NHC(O)NH- | 4-OCF₃-phenyl |
| 2559 | 4-OCH₃-phenyl | H | m-NHC(O)NH- | 1,1-dimethylethyl-1-methyl-1H-pyrazole |
| 2560 | 5-OCH₃-phenyl | H | m-NHC(O)NH- | 3,4-dimethyl-5-isoxazole |
| 2561 | 6-OCH₃-phenyl | H | m-NHC(O)NH- | 1,1-dimethylethyl-3-oxazole |
| 2562 | 2-F-phenyl | H | m-NHC(O)NH- | 4-OCF₃-phenyl |
| 2563 | 4-F-phenyl | H | m-NHC(O)NH- | 1,1-dimethylethyl-1-methyl-1H-pyrazole |
| 2564 | 5-F-phenyl | H | m-NHC(O)NH- | 3,4-dimethyl-5-isoxazole |
| 2565 | 6-F-phenyl | H | m-NHC(O)NH- | 1,1-dimethylethyl-3-oxazole |
| 2566 | 2-thiophene | H | m-NHC(O)NH- | 4-OCF₃-phenyl |
| 2567 | 3-thiophene | H | m-NHC(O)NH- | 1,1-dimethylethyl-1-methyl-1H-pyrazole |
| 2568 | 2-pyridine | H | m-NHC(O)NH- | 3,4-diethyl-5-isoxazole |
| 2569 | 3-pyridine | H | m-NHC(O)NH- | 1,1-dimethylethyl-3-oxazole |
| 2570 | 2-CH₃-phenyl | -(CH₂)₃-morpholine | m-NHC(O)NH- | 4-OCF₃-phenyl |
| 2571 | 4-CH₃-phenyl | -(CH₂)₃-morpholine | m-NHC(O)NH | 1,1-dimethylethyl-1-methyl-1H-pyrazole |
| 2572 | 5-CH₃-phenyl | -(CH₂)₃-morpholine | m-NHC(O)NH- | 3,4-dimethyl-5-isoxazole |
| 2573 | 6-CH₃-phenyl | -(CH₂)₃-morpholine | m-NHC(O)NH- | 1,1-dimethylethyl-3-oxazole |
| 2574 | 2-OCH₃-phenyl | -(CH₂)₃-morpholine | m-NHC(O)NH- | 4-OCF₃-phenyl |
| 2575 | 4-OCH₃-phenyl | -(CH₂)₃-morpholine | m-NHC(O)NH- | 1,1-dimethylethyl-1-methyl-1H-pyrazole |
| 2576 | 5-OCH₃-phenyl | -(CH₂)₃-morpholine | m-NHC(O)NH- | 3,4-dimethyl-5-isoxazole |
| 2577 | 6-OCH₃-phenyl | -(CH₂)₃-morpholine | m-NHC(O)NH- | 1,1-dimethylethyl-3-oxazole |
| 2578 | 2-F-phenyl | -(CH₂)₃-morpholine | m-NHC(O)NH- | 4-OCF₃-phenyl |
| 2579 | 4-F-phenyl | -(CH₂)₃-morpholine | m-NHC(O)NH- | 1,1-dimethylethyl-1-methyl-1H-pyrazole |
| 2580 | 5-F-phenyl | -(CH₂)₃-morpholine | m-NHC(O)NH- | 3,4-dimethyl-5-isoxazole |
| 2581 | 6-F-phenyl | -(CH₂)₃-morpholine | m-NHC(O)NH- | 1,1-dimethylethyl-3-oxazole |
| 2582 | 2-thiophene | -(CH₂)₃-morpholine | m-NHC(O)NH- | 4-OCF₃-phenyl |
| 2583 | 3-thiophene | -(CH₂)₃-morpholine | m-NHC(O)NH- | 1,1-dimethylethyl-1-methyl-1H-pyrazole |
| 2584 | 2-pyridine | -(CH₂)₃-morpholine | m-NHC(O)NH- | 3,4-dimethyl-5-isoxazole |
| 2585 | 3-pyridine | -(CH₂)₃-morpholine | m-NHC(O)NH- | 1,1-dimethylethyl-3-oxazole |
| 2586 | 2-CH₃-phenyl | CH₃ | m-NHC(O)NH- | 4-OCF₃-phenyl |
| 2587 | 4-CH₃-phenyl | CH₃ | m-NHC(O)NH | 1,1-dimethylethyl-1-methyl-1H-pyrazole |
| 2588 | 5-CH₃-phenyl | CH₃ | m-NHC(O)NH- | 3,4-dimethyl-5-isoxazole |
| 2589 | 6-CH₃-phenyl | CH₃ | m-NHC(O)NH- | 1,1-dimethylethyl-3-oxazole |
| 2590 | 2-OCH₃-phenyl | CH₃ | m-NHC(O)NH- | 4-OCF₃-phenyl |
| 2591 | 4-OCH₃-phenyl | CH₃ | m-NHC(O)NH- | 1,1-dimethylethyl-1-methyl-1H-pyrazole |
| 2592 | 5-OCH₃-phenyl | CH₃ | m-NHC(O)NH- | 3,4-dimethyl-5-isoxazole |
| 2593 | 6-OCH₃-phenyl | CH₃ | m-NHC(O)NH- | 1,1-dimethylethyl-3-oxazole |
| 2594 | 2-F-phenyl | CH₃ | m-NHC(O)NH- | 4-OCF₃-phenyl |
| 2595 | 4-F-phenyl | CH₃ | m-NHC(O)NH- | 1,1-dimethylethyl-1-methyl-1H-pyrazole |
| 2596 | 5-F-phenyl | CH₃ | m-NHC(O)NH- | 3,4-dimethyl-5-isoxazole |
| 2597 | 6-F-phenyl | CH₃ | m-NHC(O)NH- | 1,1-dimethylethyl-3-oxazole |
| 2598 | 2-thiophene | CH₃ | m-NHC(O)NH- | 4-OCF₃-phenyl |
| 2599 | 3-thiophene | CH₃ | m-NHC(O)NH | 1,1-dimethylethyl-1-methyl-1H-pyrazole |
| 2600 | 2-pyridine | CH₃ | m-NHC(O)NH- | 3,4-dimethyl-5-isoxazole |
| 2601 | 3-pyridine | CH₃ | m-NHC(O)NH- | 1,1-dimethylethyl-3-oxazole |
| 2602 | 2-CH₃-phenyl | -O(CH₂)₂-piperidine | m-NHC(O)NH- | 4-OCF₃-phenyl |
| 2603 | 4-CH₃-phenyl | -O(CH₂)₂-piperidine | m-NHC(O)NH | 1,1-dimethylethyl-1-methyl-1H-pyrazole |
| 2604 | 5-CH₃-phenyl | -O(CH₂)₂-piperidine | m-NHC(O)NH- | 3,4-dimethyl-5-isoxazole |
| 2605 | 6-CH₃-phenyl | -O(CH₂)₂-piperidine | m-NHC(O)NH- | 1,1-dimethylethyl-3-oxazole |
| 2606 | 2-OC_{H}3-phenyl | -O(CH₂)₂-piperidine | m-NHC(O)NH- | 4-OCF₃-phenyl |
| 2607 | 4-OCH₃-phenyl | -O(CH₂)₂-piperidine | m-NHC(O)NH- | 1,1-dimethylethyl-1-methyl-1H-pyrazole |
| 2608 | 5-OCH₃-phenyl | -O(CH₂)₂-piperidine | m-NHC(O)NH- | 3,4-dimethyl-5-isoxazole |
| 2609 | 6-OCH₃-phenyl | -O(CH₂)₂-piperidine | m-NHC(O)NH- | 1,1-dimethylethyl-3-oxazole |
| 2610 | 2-F-phenyl | -O(CH₂)₂-piperidine | m-NHC(O)NH- | 4-OCF₃-phenyl |
| 2611 | 4-F-phenyl | -O(CH₂)₂-piperidine | m-NHC(O)NH- | 1,1-dimethylethyl-1-methyl-1H-pyrazole |
| 2612 | 5-F-phenyl | -O(CH₂)₂-piperidine | m-NHC(O)NH- | 3,4-dimethyl-5-isoxazole |
| 2613 | 6-F-phenyl | -O(CH₂)₂-piperidine | m-NHC(O)NH- | 1,1-dimethylethyl-3-oxazole |
| 2614 | 2-thiophene | -O(CH₂)₂-piperidine | m-NHC(O)NH- | 4-OCF₃-phenyl |
| 2615 | 3-thiophene | -O(CH₂)₂-piperidine | m-NHC(O)NH- | 1,1-dimethylethyl-1-methyl-1H-pyrazole |
| 2616 | 2-pyridine | -O(CH₂)₂-piperidine | m-NHC(O)NH- | 3,4-dimethyl-5-isoxazole |
| 2617 | 3-pyridine | -O(CH₂)₂-piperidine | m-NHC(O)NH- | 1,1-dimethylethyl-3-oxazole |
| 2618 | 2-CH₃-phenyl | -O(CH₂)₂-piperzine | m-NHC(O)NH- | 4-OCF₃-phenyl |
| 2619 | 4-CH₃-phenyl | -O(CH₂)₂-piperzine | m-NHC(O)NH | 1,1-dimethylethyl-1-methyl-1H-pyrazole |
| 2620 | 5-CH₃-phenyl | -O(CH₂)₂-piperzine | m-NHC(O)NH- | 3,4-dimethyl-5-isoxazole |
| 2621 | 6-CH₃-phenyl | -O(CH₂)₂-piperzine | m-NHC(O)NH- | 1,1-dimethylethyl-3-oxazole |
| 2622 | 2-OCH₃-phenyl | -O(CH₂)₂-piperzine | m-NHC(O)NH- | 4-OCF₃-phenyl |
| 2623 | 4-OCH₃-phenyl | -O(CH₂)₂-piperzine | m-NHC(O)NH- | 1,1-dimethylethyl-1-methyl-1H-pyrazole |
| 2624 | 5-OCH₃-phenyl | -O(CH₂)₂-piperzine | m-NHC(O)NH- | 3,4-dimethyl-5-isoxazole |
| 2625 | 6-OCH₃-phenyl | -O(CH₂)₂-piperzine | m-NHC(O)NH- | 1,1-dimethylethyl-3-oxazole |
| 2626 | 2-F-phenyl | -O(CH₂)₂-piperzine | m-NHC(O)NH- | 4-OCF₃-phenyl |
| 2627 | 4-F-phenyl | -O(CH₂)₂-piperzine | m-NHC(O)NH- | 1,1-dimethylethyl-1-methyl-1H-pyrazole |
| 2628 | 5-F-phenyl | -O(CH₂)₂-piperzine | m-NHC(O)NH- | 3,4-dimethyl-5-isoxazole |
| 2629 | 6-F-phenyl | -O(CH₂)₂-piperzine | m-NHC(O)NH- | 1,1-dimethylethyl-3-oxazole |
| 2630 | 2-thiophene | -O(CH₂)₂-piperzine | m-NHC(O)NH- | 4-OCF₃-phenyl |
| 2631 | 3-thiophene | -O(CH₂)₂-piperzine | m-NHC(O)NH- | 1,1-dimethylethyl-1-methyl-1H-pyrazole |
| 2632 | 2-pyridine | -O(CH₂)₂-piperzine | m-NHC(O)NH- | 3,4-dimethyl-5-isoxazole |
| 2633 | 3-pyridine | -O(CH₂)₂-piperzine | m-NHC(O)NH- | 1,1-dimethylethyl-3-oxazole |
| 2634 | 2-CH₃-phenyl | H | p-C(O)NH- | 4-OCF₃-phenyl |
| 2635 | 4-CH₃-phenyl | H | p-C(O)NH- | 1,1-dimethylethyl-1-methyl-1H-pyrazole |
| 2636 | 5-CH₃-phenyl | H | p-C(O)NH- | 3,4-dimethyl-5-isoxazole |
| 2637 | 6-CH₃-phenyl | H | p-C(O)NH- | 1,1-dimethylethyl-3-oxazole |
| 2638 | 2-OCH₃-phenyl | H | p-C(O)NH- | 4-OCF₃-phenyl |
| 2639 | 4-OCH₃-phenyl | H | p-C(O)NH- | 1,1-dimethylethyl-1-methyl-1H-pyrazole |
| 2640 | 5-OCH₃-phenyl | H | p-C(O)NH- | 3,4-dimethyl-5-isoxazole |
| 2641 | 6-OCH₃-phenyl | H | p-C(O)NH- | 1,1-dimethylethyl-3-oxazole |
| 2642 | 2-F-phenyl | H | p-C(O)NH- | 4-OCF₃-phenyl |
| 2643 | 4-F-phenyl | H | p-C(O)NH- | 1,1-dimethylethyl-1-methyl-1H-pyrazole |
| 2644 | 5-F-phenyl | H | p-C(O)NH- | 3,4-dimethyl-5-isoxazole |
| 2645 | 6-F-phenyl | H | p-C(O)NH- | 1,1-dimethylethyl-3-oxazole |
| 2646 | 2-thiophene | H | p-C(O)NH- | 4-OCF₃-phenyl |
| 2647 | 3-thiophene | H | p-C(O)NH- | 1,1-dimethylethyl-1-methyl-1H-pyrazole |
| 2648 | 2-pyridine | H | p-C(O)NH- | 3,4-dimethyl-5-isoxazole |
| 2649 | 3-pyridine | H | p-C(O)NH- | 1,1-dimethylethyl-3-oxazole |
| 2650 | 2-CH₃-phenyl | H | p-C(O)NH- | 4-OCF₃-phenyl |
| 2651 | 4-CH₃-phenyl | H | p-C(O)NH- | 1,1-dimethylethyl-1-methyl-1H-pyrazole |
| 2652 | 5-CH₃-phenyl | H | p-C(O)NH- | 3,4-dimethyl-5-isoxazole |
| 2653 | 6-CH₃-phenyl | H | p-C(O)NH- | 1,1-dimethylethyl-3-oxazole |
| 2654 | 2-OCH₃-phenyl | H | p-C(O)NH- | 4-OCF₃-phenyl |
| 2655 | 4-OCH₃-phenyl | H | p-C(O)NH- | 1,1-dimethylethyl-1-methyl-1H-pyrazole |
| 2656 | 5-OCH₃-phenyl | H | p-C(O)NH- | 3,4-dimethyl-5-isoxazole |
| 2657 | 6-OCH₃-phenyl | H | p-C(O)NH- | 1,1-dimethylethyl-3-oxazole |
| 2360 | 2-F-phenyl | H | p-C(O)NH- | 4-OCF₃-phenyl |
| 2361 | 4-F-phenyl | H | p-C(O)NH- | 1,1-dimethylethyl-1-methyl-1H-pyrazole |
| 2362 | 5-F-phenyl | ¹H | p-C(O)NH- | 3,4-dimethyl-5-isoxazole |
| 2363 | 6-F-phenyl | H | p-C(O)NH- | 1,1-dimethylethyl-3-oxazole |
| 2364 | 2-thiophene | H | p-C(O)NH- | 4-OCF₃-phenyl |
| 2365 | 3-thiophene | H | p-C(O)NH- | 1,1-dimethylethyl-1-methyl-1H-pyrazole |
| 2366 | 2-pyridine | H | p-C(O)NH- | 3,4-dimethyl-5-isoxazole |
| 2367 | 3-pyridine | H | p-C(O)NH- | 1,1-dimethylethyl-3-oxazole |
| 2368 | 2-CH₃-phenyl | -(CH₂)₃-morpholine | p-C(O)NH- | 4-OCF₃-phenyl |
| 2369 | 4-CH₃-phenyl | -(CH₂)₃-morpholine | p-C(O)NH- | 1,1-dimethylethyl-1-methyl-1H-pyrazole |
| 2370 | 5-CH₃-phenyl | -(CH₂)₃-morpholine | p-C(O)NH- | 3,4-dimethyl-5-isoxazole |
| 2371 | 6-CH₃-phenyl | -(CH₂)₃-morpholine | p-C(O)NH- | 1,1-dimethylethyl-3-oxazole |
| 2372 | 2-OCH₃-phenyl | -(CH₂)₃-morpholine | p-C(O)NH- | 4-OCF₃-phenyl |
| 2373 | 4-OCH₃-phenyl | -(CH₂)₃-morpholine | p-C(O)NH- | 1,1-dimethylethyl-1-methyl-1H-pyrazole |
| 2374 | 5-OCH₃-phenyl | -(CH₂)₃-morpholine | p-C(O)NH- | 3,4-dimethyl-5-isoxazole |
| 2375 | 6-OCH₃-phenyl | -(CH₂)₃-morpholine | p-C(O)NH- | 1,1-dimethylethyl-3-oxazole |
| 2376 | 2-F-phenyl | -(CH₂)₃-morpholine | p-C(O)NH- | 4-OCF₃-phenyl |
| 2377 | 4-F-phenyl | -(CH₂)₃-morpholine | p-C(O)NH- | 1,1-dimethylethyl-1-methyl-1H-pyrazole |
| 2378 | 5-F-phenyl | -(CH₂)₃-morpholine | p-C(O)NH- | 3,4-dimethyl-5-isoxazole |
| 2379 | 6-F-phenyl | -(CH₂)₃-morpholine | p-C(O)NH- | 1,1-dimethylethyl-3-oxazole |
| 2380 | 2-thiophene | -(CH₂)₃-morpholine | p-C(O)NH- | 4-OCF₃-phenyl |
| 2381 | 3-thiophene | -(CH₂)₃-morpholine | p-C(O)NH- | 1,1-dimethylethyl-1-methyl-1H-pyrazole |
| 2382 | 2-pyridine | -(CH₂)₃-morpholine | p-C(O)NH- | 3,4-dimethyl-5-isoxazole |
| 2383 | 3-pyridine | -(CH₂)₃-morpholine | p-C(O)NH- | 1,1-dimethylethyl-3-oxazole |
| 2384 | 2-CH₃-phenyl | CH₃ | p-C (O)NH- | 4-OCF₃-phenyl |
| 2385 | 4-CH₃-phenyl | CH₃ | p-C(O)NH- | 1,1-dimethylethyl-1-methyl-1H-pyrazole |
| 2386 | 5-CH₃-5-CH₃-phenyl | CH₃ | p-C(O)NH- | 3,4-dimethyl-5-isoxazole |
| 2387 | 6-CH₃-phenyl | CH₃ | p-C(O)NH- | 1,1-dimethylethyl-3-oxazole |
| 2388 | 2-OCH₃-phenyl | CH₃ | p-C(O)NH- | 4 -OCF₃-phenyl |
| 2389 | 4-(CH₃-phenyl | CH₃ | p-C(O)NH- | 1,1-dimethylethyl-1-methyl-1H-pyrazole |
| 2390 | 5-OCH₃-phenyl | CH₃ | p-C(O)NH- | 3,4-dimethyl-5-isoxazole |
| 2391 | 6-OCH₃-phenyl | CH₃ | p-C (O) NH- | 1,1-dimethylethyl-3-oxazole |
| 2392 | 2-F-phenyl | CH₃ | p-C(O)NH- | 4-OCF₃-phenyl |
| 2393 | 4-F-phenyl | CH₃ | p-C(O)NH- | 1,1-dimethylethyl-1-methyl-1H-pyrazole |
| 2394 | 5-F-phenyl | CH₃ | p-C(O)NH- | 3,4-dimethyl-5-isoxazole |
| 2395 | 6-F-phenyl | CH₃ | p-C(O)NH- | 1,1-dimethylethyl-3-oxazole |
| 2396 | 2-thiophene | CH₃ | p-C(O)NH- | 4-OCF₃-phenyl |
| 2397 | 3-thiophene | CH₃ | p-C(O)NH- | 1,1-dimethylethyl-1-methyl-1H-pyrazole |
| 2398 | 2-pyridine | CH₃ | p-C(O)NH- | 3,4-dimethyl-5-isoxazole |
| 2399 | 3-pyridine | CH₃ | p-(O)NH- | 1,1-dimethylethyl-3-oxazole |
| 2400 | 2-CH₃-phenyl | -O(CH₂)₂-piperidine | p-C(O)NH- | 4-OCF₃-phenyl |
| 2401 | 4-CH₃-phenyl | -O(CH₂)₂-piperidine | p-C(O)NH- | 1,1-dimethylethyl-1-methyl-1H-pyrazole |
| 2402 | 5-CH₃-phenyl | -O(CH₂)₂-piperidine | p-C(O)NH- | 3,4-dimethyl-5-isoxazole |
| 2403 | 6-CH₃-phenyl | -O(CH₂)₂-piperidine | p-C(O)NH- | 1,1-dimethylethyl-3-oxazole |
| 2404 | 2-OCH₃-phenyl | -O(CH₂)₂-piperidine | p-C(O)NH- | 4-OCF₃-phenyl |
| 2405 | 4-OCH₃-phenyl | -O(CH₂)₂-piperidine | p-C(O)NH- | 1,1-dimethylethyl-1-methyl-1H-pyrazole |
| 2406 | 5-OCH₃-phenyl | -O(CH₂)₂-piperidine | p-C(O)NH- | 3,4-dimethyl-5-isoxazole |
| 2407 | 6-OCH₃-phenyl | -O(CH₂)₂-piperidine | p-C(O)NH- | 1,1-dimethylethyl-3-oxazole |
| 2408 | 2-F-phenyl | -O(CH₂)₂-piperidine | p-C(O)NH- | 4-OCF₃-phenyl |
| 2409 | 4-F-phenyl | -O(CH₂)₂-piperidine | p-C(O)NH- | 1,1-dimethylethyl-1-methyl-1H-pyrazole |
| 2410 | 5-F-phenyl | -O(CH₂)₂-piperidine | p-C(O)NH- | 3,4-dimethyl-5-isoxazole |
| 2411 | 6-F-phenyl | -O(CH₂)₂-piperidine | p-C(O)NH- | 1,1-dimethylethyl-3-oxazole |
| 2412 | 2-thiophene | -O(CH₂)₂-piperidine | p-C(O)NH- | 4-OCF₃-phenyl |
| 2413 | 3-thiophene | -O(CH₂)₂-piperidine | p-C(O)NH- | 1,1-dimethylethyl-1-methyl-1H-pyrazole |
| 2414 | 2-pyridine | -O(CH₂)₂-piperidine | p-C(O)NH- | 3,4-dimethyl-5-isoxazole |
| 2415 | 3-pyridine | -O(CH₂)₂-piperidine | p-C(O)NH- | 1,1-dimethylethyl-3-oxazole |
| 2416 | 2-(CH₃-phenyl | -O(CH₂)₂-piperzine | p-C(O)NH- | 4-OCF₃-phenyl |
| 2417 | 4-CH₃-phenyl | -O(CH₂)₂-piperzine | p-C(O)NH- | 1,1-dimethylethyl-1-methyl-1H-pyrazole |
| 2418 | 5-CH₃-phenyl | -O(CH₂)₂-piperzine | p-C(O)NH- | 3,4-dimethyl-5-isoxazole |
| 2419 | 6-CH₃-phenyl | -O(CH₂)₂-piperzine | p-C(O)NH- | 1,1-dimethylethyl-3-oxazole |
| 2420 | 2-OCH₃-phenyl | -O(CH₂)₂-piperzine | p-C(O)NH- | 4-OCF₃-phenyl |
| 2421 | 4-OCH₃-phenyl | -O(CH₂)₂-piperzine | p-C(O)NH- | 1,1-dimethylethyl-1-methyl-1H-pyrazole |
| 2422 | 5-OCH₃-phenyl | -O(CH₂)₂-piperzine | p-C(O)NH- | 3,4-dimethyl-5-isoxazole |
| 2423 | 6-OCH₃-phenyl | -O(CH₂)₂-piperzine | p-C(O)NH- | 1,1-dimethylethyl-3-oxazole |
| 2424 | 2-F-phenyl | -O(CH₂)₂-piperzine | p-C(O)NH- | 4-OCF₃-phenyl |
| 2425 | 4-F-phenyl | -O(CH₂)₂-piperzine | p-C(O)NH- | 1,1-dimethylethyl-1-methyl-1H-pyrazole |
| 2426 | 5-F-phenyl | -O(CH₂)₂-piperzine | p-C(O)NH- | 3,4-dimethyl-5-isoxazole |
| 2427 | 6-F-phenyl | -O(CH₂)₂-piperzine | p-C(O)NH- | 1,1-dimethylethyl-3-oxazole |
| 2428 | 2-thiophene | -O(CH₂)₂-piperzine | p-C(O)NH- | 4-OCF₃-phenyl |
| 2429 | 3-thiophene | -O(CH₂)₂-piperzine | p-C(O)NH- | 1,1-dimethylethyl-1-methyl-1H-pyrazole |
| 2430 | 2-pyridine | -O(CH₂)₂-piperzine | p-C(O)NH- | 3,4-dimethyl-5-isoxazole |
| 2431 | 3-pyridine | -O(CH₂)₂-piperzine | p-C(O)NH- | 1,1-dimethylethyl-3-oxazole |
| 2432 | 2-CH₃-phenyl | 1-pyrrolidinylethyl | p-C(O)NH- | 4-OC F₃-phenyl |
| 2433 | 4-CH₃-phenyl | 1-pyrrolidinylethyl | p-C(O)NH- | 1,1-dimethylethyl-1-methyl-1H-pyrazole |
| 2434 | 5-CH₃-phenyl | 1-pyrrolidinylethyl | p-C(O)NH- | 3,4-dimethyl-5-isoxazole |
| 2435 | 6-CH₃-phenyl | 1-pyrrolidinylethyl | p-C(O)NH- | 1,1-dimethylethyl-3-oxazole |
| 2436 | 2-OCH₃-phenyl | 1-pyrrolidinylethyl | p-C(O)NH- | 4-OCF₃-phenyl |
| 2437 | 4-OCH₃-phenyl | 1-pyrrolidinylethyl | p-C(O)NH- | 1,1-dimethylethyl-1-methyl-1H-pyrazole |
| 2438 | 5-OCH₃-phenyl | 1-pyrrolidinylethyl | p-C(O)NH- | 3,4-dimethyl-5isoxazole |
| 2439 | 6-OCH₃-phenyl | 1-pyrrolidinylethyl | p-C(O)NH- | 1,1-dimethylethyl-3-oxazole |
| 2440 | 2-F-phenyl | 1-pyrrolidinylethyl | p-C(O)NH- | 4-OCF₃-phenyl |
| 2441 | 4-Fphenyl | 1-pyrrolidinylethyl | p-C(O)NH- | 1,1-dimethylethyl-1-methyl-1H-pyrazole |
| 2442 | 5-F-phenyl | 1-pyrrolidinylethyl | p-C(O)NH- | 3,4-dimethyl-5-isoxazole |
| 2443 | 6-F-phenyl | 1-pyrrolidinylethyl | p-C(O)NH- | 1,1-dimethylethyl-3-oxazole |
| 2444 | 2-thiophene | 1-pyrrolidinylethyl | p-C(O)NH- | 4-OCF₃-phenyl |
| 2445 | 3-thiophene | 1-pyrrolidinylethyl | p-C(O)NH- | 1,1-dimethylethyl-1-methyl-1H-pyrazole |
| 2446 | 2-pyridine | 1-pyrrolidinylethyl | p-C(O)NH- | 3,4-dimethyl-5-isoxazole |
| 2447 | 3-pyridine | 1-pyrrolidinylethyl | p-C(O)NH- | 1,1-dimethylethyl-3-oxazole |
| 2448 | 2-CH₃-phenyl | H | m-NH-C(O)- | 4-OCF₃-phenyl |
| 2449 | 4-CH₃-phenyl | H | m-NH-C(O)- | 1,1-dimethylethyl-1-methyl-1H-pyrazole |
| 2450 | 5-CH₃-phenyl | H | m-NH-C(O)- | 3,4-dimethyl-5-isoxazole |
| 2451 | 6-CH₃-phenyl | H | m-NH-C(O)- | 1,1-dimethylethyl-3-oxazole |
| 2452 | 2-OCH₃-phenyl | H | m-NH-C(O)- | 4 -OCF₃-phenyl |
| 2453 | 4-OCH₃-phenyl | H | m-NH-C(O)- | 1,1-dimethylethyl-1-methyl-1H-pyrazole |
| 2454 | 5-OCH₃-phenyl | H | m-NH-C(O)- | 3,4-dimethyl-5-isoxazole |
| 2455 | 6-OCH₃-phenyl | H | m-NH-C(O)- | 1,1-dimethylethyl-3-oxazole |
| 2456 | 2-F-phenyl | H | m-NH-C(O)- | 4-OCF₃-phenyl |
| 2457 | 4-F-phenyl | H | m-NH-C(O)- | 1,1-dimethylethyl-1-methyl-1H-pyrazole |
| 2458 | 5-F-phenyl | H | m-NH-C(O)- | 3,4-dimethyl-5-isoxazole |
| 2459 | 6-F-phenyl | H | m-NH-C(O)- | 1,1-dimethylethyl-3-oxazole |
| 2460 | 2-thiophene | H | m-NH-C(O)- | 4-OCF₃-phenyl |
| 2461 | 3-thiophene | H | m-NH-C(O)- | 1,1-dimethylethyl-1-methyl-1H-pyrazole |
| 2462 | 2-pyridine | H | m-NH-C(O)- | 3,4-dimethyl-5-isoxazole |
| 2463 | 3-pyridine | H | m-NH-C(O)- | 1,1-dimethylethyl-3-oxazole |
| 2464 | 2-CH₃-phenyl | H | m-NH-C(O)- | 4-OC F₃-phenyl |
| 2465 | 4-CH₃-phenyl | H | m-NH-C(O)- | 1,1-dimethylethyl-1-methyl-1H-pyrazole |
| 2466 | 5-CH₃-phenyl | H | m-NH-C(O)- | 3,4-dimethyl-5-isoxazole |
| 2467 | 6-CH₃-phenyl | H | m-NH-C(O)- | 1,1-dimethylethyl-3-oxazole |
| 2468 | 2-OCH₃-phenyl | H | m-NH-C(O)- | 4-OCF₃-phenyl |
| 2469 | 4-OCH₃-phenyl | H | m-NH-C(O)- | 1,1-dimethylethyl-1-methyl-1H-pyrazole |
| 2470 | 5-OCH₃-phenyl | H | m-NH-C(O)- | 3,4-dimethyl-5-isoxazole |
| 2471 | 6-OCH₃-phenyl | H | m-NH-C(O)- | 1,1-dimethylethyl-3oxazole |
| 2472 | 2-F-phenyl | H | m-NH-C(O)- | 4-OCF₃-phenyl |
| 2473 | 4-F-phenyl | H | m-NH-C(O)- | 1,1-dimethylethyl-1-methyl-1H-pyrazole |
| 2474 | 5-F-phenyl | H | m-NH-C(O)- | 3,4-dimethyl-5-isoxazole |
| 2475 | 6-F-phenyl | H | m-NH-C(O)- | 1,1-dimethylethyl-3-oxazole |
| 2476 | 2-thiophene | H | m-NHC(O)- | 4 -OCF₃-phenyl |
| 2477 | 3-thiophene | H | m-NH-C(O)- | 1,1-dimethylethyl-1-methyl-1H-pyrazole |
| 2478 | 2-pyridine | H | m-NH-C(O)- | 3,4-dimethyl-5-isoxazole |
| 2479 | 3pyridine | H | m-NH-C(O)- | 1,1-dimethylethyl-3-oxazole |
| 2480 | 2-CH₃-phenyl | -(CH₂)₃-morpholine | m-NH-C(O)- | 4-OCF₃-phenyl |
| 2481 | 4-CH₃-phenyl | -(CH₂)₃-morpholine | m-NH-C(O)- | 1,1-dimethylethyl-1-methyl-1H-pyrazole |
| 2482 | 5-CH₃-phenyl | -(CH₂)₃-morpholine | m-NH-C(O)- | 3,4-dimethyl-5-isoxazole |
| 2483 | 6-CH₃-phenyl | -(CH₂)₃-morpholine | m-NH-C(O)- | 1,1-dimethylethyl-3-oxazole |
| 2484 | 2-OCH₃-phenyl | -(CH₂)₃-morpholine | m-NH-C(O)- | 4 -OCF₃-phenyl |
| 2485 | 4-OCH₃-phenyl | -(CH₂)₃-morpholine | m-NH-C(O)- | 1,1-dimethylethyl-1-methyl-1H-pyrazole |
| 2486 | 5-OCH₃-phenyl | -(CH₂)₃-morpholine | m-NH-C(O)- | 3,4-dimethyl-5-isoxazole |
| 2487 | 6-OCH₃-phenyl | -(CH₂)₃-morpholine | m-NH-C(O)- | 1,1-dimethylethyl-3-oxazole |
| 2488 | 2-F-phenyl | -(CH₂)₃-morpholine | m-NH-C(O)- | 4-OCF₃-phenyl |
| 2489 | 4-F-phenyl | -(CH₂)₃-morpholine | m-NH-C(O)- | 1,1-dimethylethyl-1-methyl-1H-pyrazole |
| 2490 | 5-F-phenyl | -(CH₂)₃-morpholine | m-NH-C(O)- | 3,4-dimethyl-5-isoxazole |
| 2491 | 6-F-phenyl | -(CH₂)₃-morpholine | m-NH-C(O)- | 1,1-dimethylethyl-3-oxazole |
| 2492 | 2-thiophene | -(CH₂)₃-morpholine | m-NH-C(O)- | 4-OCF₃-phenyl |
| 2493 | 3-thiophene | -(CH₂)₃-morpholine | m-NH-C(O)- | 1,1-dimethylethyl-1-methyl-1H-pyrazole |
| 2494 | 2-pyridine | -(CH₂)₃-morpholine | m-NH-C(O)- | 3,4-dimethyl-5-isoxazole |
| 2495 | 3-3-pyridine | -(CH₂)₃-morpholine | m-NH-C(O)- | 1,1-dimethylethyl-3-oxazole |
| 2496 | 2-(CH₃-phenyl | CH₃ | m-NH-C(O)- | 4-OCF₃-phenyl |
| 2497 | 4-CH₃-phenyl | CH₃ | m-NH-C(O)- | 1,1-dimethylethyl-1-methyl-1H-pyrazole |
| 2498 | 5-CH₃-phenyl | CH₃ | m-NH-C(O)- | 3,4-dimethyl-5-isoxazole |
| 2499 | 6-CH₃-phenyl | CH₃ | m-NH-C(O)- | 1,1-dimethylethyl-3-oxazole |
| 2500 | 2-OCH₃-phenyl | CH₃ | m-NH-C(O)- | 4-OCF₃-phenyl |
| 2501 | 4-OCH₃-phenyl | CH₃ | m-NH-C(O)- | 1,1-dimethylethyl-1-methyl-1H-pyrazole |
| 2502 | 5-OCH₃-phenyl | CH₃ | m-NH-C(O)- | 3,4-dimethyl-5-isoxazole |
| 2503 | 6-OCH₃-phenyl | CH₃ | m-NH-C(O)- | 1,1-dimethylethyl-3-oxazole |
| 2504 | 2-F-phenyl | CH₃ | m-NH-C(O)- | 4-OCF₃-phenyl |
| 2505 | 4-F-phenyl | CH₃ | m-NH-C(O)- | 1,1-dimethylethyl-1-methyl-1H-pyrazole |
| 2506 | 5-F-phenyl | CH₃ | m-NH-C(O)- | 3,4-dimethyl-5-isoxazole |
| 2507 | 6-F-phenyl | CH₃ | m-NH-C(O)- | 1,1-dimethylethyl-3-oxazole |
| 2508 | 2-thiophene | CH₃ | m-NH-C(O)- | 4-OCF₃-phenyl |
| 2509 | 3-thiophene | CH₃ | m-NH-C(O)- | 1,1-dimethylethyl-1-methyl-1H-pyrazole |
| 2510 | 2-pyridine | CH₃ | m-NH-C(O)- | 3,4-dimethyl-5-isoxazole |
| 2511 | 3-pyridine | CH₃ | m-NH-C(O)- | 1,1-dimethylethyl-3-oxazole |
| 2512 | 2-CH₃-phenyl | -O(CH₂)₂-piperidine | m-NH-C(O)- | 4-OCF₃-phenyl |
| 2513 | 4-CH₃-phenyl | -O(CH₂)₂-piperidine | m-NH-C(O)- | 1,1-dimethylethyl-1-methyl-1H-pyrazole |
| 2514 | 5-CH₃-phenyl | -O(CH₂)₂-piperidine | m-NH-C(O)- | 3,4-dimethyl-5-isoxazole |
| 2515 | 6-CH₃-phenyl | -O(CH₂)₂-piperidine | m-NH-C(O)- | 1,1-dimethylethyl-3-oxazole |
| 2516 | 2-OCH₃-phenyl | -O(CH₂)₂-piperidine | m-NH-C(O)- | 4-OCF₃-phenyl |
| 2517 | 4-OCH₃-phenyl | -O(CH₂)₂-piperidine | m-NH-C(O)- | 1,1-dimethylethyl-1-methyl-1H-pyrazole |
| 2518 | 5-OCH₃-phenyl | -O(CH₂)₂-piperidine | m-NH-C(O)- | 3,4-dimethyl-5-isoxazole |
| 2519 | 6-OCH₃-phenyl | -O(CH₂)₂-piperidine | m-NH-C(O)- | 1,1-dimethylethyl-3-oxazole |
| 2520 | 2-F-phenyl | -O(CH₂)₂-piperidine | m-NH-C(O)- | 4-OCF₃-phenyl |
| 2521 | 4-F-phenyl | -O(CH₂)₂-piperidine | m-NH-C(O)- | 1,1-dimethylethyl-1-methyl-1H-pyrazole |
| 2522 | 5-F-phenyl | -O(CH₂)₂-piperidine | m-NH-C(O)- | 3,4-dimethyl-5-isoxazole |
| 2523 | 6-F-phenyl | -O(CH₂)₂-piperidine | m-NH-C(O)- | 1,1-dimethylethyl-3-oxazole |
| 2524 | 2-thiophene | -O(CH₂)₂-piperidine | m-NH-C(O)- | 4-OCF₃-phenyl |
| 2525 | 3-thiophene | -O(CH₂)₂-piperidine | m-NH-C(O)- | 1,1-dimethylethyl-1-methyl-1H-pyrazole |
| 2526 | 2-pyridine | -O(CH₂)₂-piperidine | m-NH-C(O)- | 3,4-dimethyl-5-isoxazole |
| 2527 | 3-pyridine | -O(CH₂)₂-piperidine | m-NH-C(O)- | 1,1-dimethylethyl-3-oxazole |
| 2528 | 2-CH₃-phenyl | -O(CH₂)₂-piperzine | m-NH-C(O)- | 4-OCF₃-phenyl |
| 2529 | 4-CH₃-phenyl | -O(CH₂)₂-piperzine | m-NH-C(O)- | 1,1-dimethylethyl-1-methyl-1H-pyrazole |
| 2530 | 5-CH₃-phenyl | -O(CH₂)₂-piperzine | m-NH-C(O)- | 3,4-dimethyl-5-isoxazole |
| 2531 | 6-CH₃-phenyl | -O(CH₂)₂-piperzine | m-NH-C(O)- | 1,1-dimethylethyl-3-oxazole |
| 2532 | 2-OCH₃-phenyl | -O(CH₂)₂-piperzine | m-NH-C(O)- | 4-OCF₃-phenyl |
| 2533 | 4-OCH₃-phenyl | -O(CH₂)₂-piperzine | m-NH-C(O)- | 1,1-dimethylethyl-1-methyl-1H-pyrazole |
| 2534 | 5-OCH₃-phenyl | -O(CH₂)₂-piperzine | m-NH-C(O)- | 3,4-dimethyl-5-isoxazole |
| 2535 | 6-OCH₃-phenyl | -O(CH₂)₂-piperzine | m-NH-C(O)- | 1,1-dimethylethyl-3-oxazole |
| 2536 | 2-F-phenyl | -O(CH₂)₂-piperzine | m-NH-C(O)- | 4-OCF₃-phenyl |
| 2537 | 4-F-phenyl | -O(CH₂)₂-piperzine | m-NH-C(O)- | 1,1-dimethylethyl-1-methyl-1H-pyrazole |
| 2538 | 5-F-phenyl | -O(CH₂)₂-piperzine | m-NH-C(O)- | 3,4-dimethyl-5-isoxazole |
| 2539 | 6-F-phenyl | -O(CH₂)₂-piperzine | m-NH-C(O)- | 1,1-dimethylethyl-3-oxazole |
| 2540 | 2-thiophene | -O(CH₂)₂-piperzine | m-NH-C(O)- | 4-OCF₃-phenyl |
| 2541 | 3-thiophene | -O(CH₂)₂-piperzine | m-NH-C(O)- | 1,1-dimethylethyl-1-methyl-1H-pyrazole |
| 2542 | 2-pyridine | -O(CH₂)₂-piperzine | m-NH-C(O)- | 3,4-dimethyl-5-isoxazole |
| 2543 | 3-pyridine | -O(CH₂)₂-piperzine | m-NH-C(O)- | 1,1-dimethylethyl-3-oxazole |
| 2544 | 2-CH₃-phenyl | 1-pyrrolidinylethyl | m-NH-C(O)- | 4-OCF₃-phenyl |
| 2545 | 4-(CH₃-phenyl | 1-pyrrolidinylethyl | m-NH-C(O)- | 1,1-dimethylethyl-1-methyl-1H-pyrazole |
| 2546 | 5-CH₃-phenyl | 1-pyrrolidinylethyl | m-NH-C(O)- | 3,4-dimethyl-5-isoxazole |
| 2547 | 6-CH₃-phenyl | 1-pyrrolidinylethyl | m-NH-C(O)- | 1,1-dimethylethyl-3-oxazole |
| 2548 | 2-OCH₃-phenyl | 1-pyrrolidinylethyl | m-NH-C(O)- | 4-OCF₃-phenyl |
| 2549 | 4-OCH₃-phenyl | 1-pyrrolidinylethyl | m-NH-C(O)- | 1,1-dimethylethyl-1-methyl-1H-pyrazole |
| 2550 | 5-OCH₃-phenyl | 1-pyrrolidinylethyl | m-NH-C(O)- | 3,4-dimethyl-5-isoxazole |
| 2551 | 6-OCH₃-phenyl | 1-pyrrolidinylethyl | m-NH-C(O)- | 1,1-dimethylethyl-3-oxazole |
| 2552 | 2-F-phenyl | 1-pyrrolidinylethyl | m-NH-C(O)- | 4-OCF₃-phenyl |
| 2553 | 4-F-phenyl | 1-pyrrolidinylethyl | m-NH-C(O)- | 1,1-dimethylethyl-1-methyl-1H-pyrazole |
| 2554 | 5-F-phenyl | 1-pyrrolidinylethyl | m-NH-C(O)- | 3,4-dimethyl-5-isoxazole |
| 2555 | 6-F-phenyl | 1-pyrrolidinylethyl | m-NH-C(O)- | 1,1-dimethylethyl-3-oxazole |
| 2556 | 2-thiophene | 1-pyrrolidinylethyl | m-NH-C(O)- | 4-OCF₃-phenyl |
| 2557 | 3-thiophene | 1pyrrolidinylethyl | m-NH-C(O)- | 1,1-dimethylethyl-1-methyl-1H-pyrazole |
| 2558 | 2-pyridine | 1-pyrrolidinylethyl | m-NH-C(O)- | 3,4-dimethyl-5-isoxazole |
| 2559 | 3-pyridine | 1-pyrrolidinylethyl | m-NH-C(O)- | 1,1-dimethylethyl-3-oxazole |

While the examples described above and hereinbelow (Examples 2560-3400) provide processes for synthesizing compounds of Formula II, other methods may be utilized to prepare such compounds. Methods involving the use of protecting groups may be used. Particularly, if one or more functional groups, for example carboxy, hydroxy, amino, or mercapto groups, are or need to be protected in preparing the compounds of the invention, because they are not intended to take part in a specific reaction or chemical transformation, various known conventional protecting groups may be used. For example, protecting groups typically utilized in the synthesis of natural and synthetic compounds, including peptides, nucleic acids, derivatives thereof and sugars, having multiple reactive centers, chiral centers and other sites potentially susceptible to the reaction reagents and/or conditions, may be used.

The protecting groups may already be present in precursors and should protect the functional groups concerned against unwanted secondary reactions, such as acylations, etherifications, esterifications, oxidations, solvolysis, and similar reactions. It is a characteristic of protecting groups that they readily lend themselves, i.e. without undesired secondary reactions, to removal, typically accomplished by solvolysis, reduction, photolysis or other methods of removal such as by enzyme activity, under conditions analogous to physiological conditions. It should also be appreciated that the protecting groups should not be present in the end-products. The specialist knows, or can easily establish, which protecting groups are suitable with the reactions described herein.

The protection of functional groups by protecting groups, the protecting groups themselves, and their removal reactions (commonly referred to as "deprotection") are described, for example, in standard reference works, such as J.F.W. McOmie, Protective Groups in Organic Chemistry, Plenum Press, London and New York (1973), in T.W. Greene, Protective Groups in Organic Synthesis, Wiley, New York (1981), in The Peptides, Volume 3, E. Gross and J. Meienhofer editors, Academic Press, London and New York (1981), in Methoden der Organischen Chemie (Methods of Organic Chemistry), Houben Weyl, 4th edition, Volume 15/1, Georg Thieme Verlag, Stuttgart (1974), in H.-D. Jakubke and H. Jescheit, Aminosäuren, Peptide, Proteine (Amino Acids, Peptides, Proteins), Verlag Chemie, Weinheim, Deerfield Beach, and Basel (1982), and in Jochen Lehmann, Chemie der Kohlenhydrate: Monosaccharide und Derivate (Chemistry of Carbohydrates: Monosaccharides and Derivatives), Georg Thieme Verlag, Stuttgart (1974).

Synthetic procedures may also be carried out where functional groups of starting compounds, which are not intended to take part in the reaction, may be present in unprotected form without the added step of protecting that group by, for example, one or more of the protecting groups mentioned above or taught in the references above.

Salts of a compound of the invention having a salt-forming group may be prepared in a conventional manner or manner known to persons skilled in the art. For example, acid addition salts of compounds of the invention may be obtained by treatment with an acid or with a suitable anion exchange reagent. A salt with two acid molecules (for example a dihalogenide) may also be converted into a salt with one acid molecule per compound (for example a monohalogenide); this may be done by heating to a melt, or for example by heating as a solid under a high vacuum at elevated temperature, for example from 50 °C to 170 °C, one molecule of the acid being expelled per molecule of the compound.

Acid salts can usually be converted to free-base compounds, e.g. by treating the salt with suitable basic agents, for example with alkali metal carbonates, alkali metal hydrogen carbonates, or alkali metal hydroxides, typically potassium carbonate or sodium hydroxide. Exemplary salt forms and their preparation are described herein in the Definition section of the application.

All synthetic procedures described herein can be carried out under known reaction conditions, advantageously under those described herein, either in the absence or in the presence (usually) of solvents or diluents. As appreciated by those of ordinary skill in the art, the solvents should be inert with respect to, and should be able to dissolve, the starting materials and other reagents used. Solvents should be able to partially or wholly solubilize the reactants in the absence or presence of catalysts, condensing agents or neutralizing agents, for example ion exchangers, typically cation exchangers for example in the H⁺ form. The ability of the solvent to allow and/or influence the progress or rate of the reaction is generally dependant on the type and properties of the solvent(s), the reaction conditions including temperature, pressure, atmospheric conditions such as in an inert atmosphere under argon or nitrogen, and concentration, and of the reactants themselves.

Suitable solvents for conducting reactions to synthesize compounds of the invention include, without limitation, water; esters, including lower alkyl-lower alkanoates, e.g., EtOAc; ethers including aliphatic ethers, e.g., Et₂O and ethylene glycol dimethylether or cyclic ethers, e.g., THF; liquid aromatic hydrocarbons, including benzene, toluene and xylene; alcohols, including MeOH, EtOH, 1-propanol, IPOH, n- and t-butanol; nitriles including CH₃CN; halogenated hydrocarbons, including CH₂Cl₂, CHCl₃ and CCl₄; acid amides including DMF; sulfoxides, including DMSO; bases, including heterocyclic nitrogen bases, e.g. pyridine; carboxylic acids, including lower alkanecarboxylic acids, e.g.., AcOH; inorganic acids including HCl, HBr, HF, H₂SO₄ and the like; carboxylic acid anhydrides, including lower alkane acid anhydrides, e.g., acetic anhydride; cyclic, linear, or branched hydrocarbons, including cyclohexane, hexane, pentane, isopentane and the like, and mixtures of these solvents, such as purely organic solvent combinations, or water-containing solvent combinations e.g., aqueous solutions. These solvents and solvent mixtures may also be used in "working-up" the reaction as well as in processing the reaction and/or isolating the reaction product(s), such as in chromatography.

Starting materials of the invention, are either known, commercially available, or can be synthesized in analogy to or according to methods that are known in the art. Many starting materials may be prepared according to known processes and, in particular, can be prepared using processes described in the examples. In synthesizing starting materials, functional groups may be protected with suitable protecting groups when necessary. Protecting groups, their introduction and removal are described above.

Compounds of the present invention can possess, in general, one or more asymmetric carbon atoms and are thus capable of existing in the form of optical isomers as well as in the form of racemic or non-racemic mixtures thereof. The optical isomers can be obtained by resolution of the racemic mixtures according to conventional processes, e.g., by formation of diastereoisomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereoisomers by crystallization followed by liberation of the optically active bases from these salts. A different process for separation of optical isomers involves the use of a chiral chromatography column optimally chosen to maximize the separation of the enantiomers. Still another available method involves synthesis of covalent diastereoisomeric molecules by reacting compounds of the invention with an optically pure acid in an activated form or an optically pure isocyanate. The synthesized diastereoisomers can be separated by conventional means such as chromatography, distillation, crystallization or sublimation, and then hydrolyzed to deliver the enantiomerically pure compound. The optically active compounds of the invention can likewise be obtained by using optically active starting materials. These isomers may be in the form of a free acid, a free base, an ester or a salt.

The compounds of the invention may contain one or more asymmetric centers and thus occur as racemates and racemic mixtures, scalemic mixtures, single enantiomers, individual diastereomers and diastereomeric mixtures. All such isomeric forms of these compounds are expressly included in the present invention.

The compounds of this invention may also be represented in multiple tautomeric forms. The invention expressly includes all tautomeric forms of the compounds described herein.

The compounds may also occur in cis- or trans- or E-or Z- double bond isomeric forms. All such isomeric forms of such compounds are expressly included in the present invention. All crystal forms of the compounds described herein are expressly included in the present invention.

Substituents on ring moieties (e.g., phenyl, thienyl, etc.) may be attached to specific atoms, whereby they are intended to be fixed to that atom, or they may be drawn unattached to a specific atom, whereby they are intended to be attached at any available atom that is not already substituted by an atom other than H (hydrogen).

The compounds of this invention may contain heterocyclic ring systems attached to another ring system. Such heterocyclic ring systems may be attached through a carbon atom or a heteroatom in the ring system.

Alternatively, a compound of any of the formulas described herein may be synthesized according to any of the procedures described herein. In the procedures described herein, the steps may be performed in an alternate order and may be preceded, or followed, by additional protection/deprotection steps as necessary. The procedures may further use appropriate reaction conditions, including inert solvents, additional reagents, such as bases (e.g., LDA, DIEA, pyridine, K₂CO₃, and the like), catalysts, and salt forms of the above. The intermediates may be isolated or carried on in situ, with or without purification. Purification methods are known in the art and include, for example, crystallization, chromatography (liquid and gas phase, and the like), extraction, distillation, trituration, reverse phase HPLC and the like. Reactions conditions such as temperature, duration, pressure, and atmosphere (inert gas, ambient) are known in the art and may be adjusted as appropriate for the reaction.

As can be appreciated by the skilled artisan, the above synthetic schemes are not intended to comprise a comprehensive list of all means by which the compounds described and claimed in this application may be synthesized. Further methods will be evident to those of ordinary skill in the art. Additionally, the various synthetic steps described above may be performed in an alternate sequence or order to give the desired compounds. Synthetic chemistry transformations and protecting group methodologies (protection and deprotection) useful in synthesizing the inhibitor compounds described herein are known in the art and include, for example, those such as described in R. Larock, Comprehensive Organic Transformations, VCH Publishers (1989); T.W. Greene and P.G.M. Wuts, Protective Groups in Organic Synthesis, 3rd edition, John Wiley and Sons (1999); L. Fieser and M. Fieser, Fieser and Fieser's Reagents for Organic Synthesis, John Wiley and Sons (1994); A. Katritzky and A. Pozharski, Handbook of Heterocyclic Chemistry, 2nd edition (2001); M. Bodanszky, A. Bodanszky, The Practice of Peptide Synthesis, Springer-Verlag, Berlin Heidelberg (1984); J. Seyden-Penne, Reductions by the Alumino- and Borohydrides in Organic Synthesis, 2nd edition, Wiley-VCH, (1997); and L. Paquette, editor, Encyclopedia of Reagents for Organic Synthesis, John Wiley and Sons (1995).

The compounds of the invention may be modified by appending appropriate functionalities to enhance selective biological properties. Such modifications are known in the art and include those which increase biological penetration into a given biological compartment (*e.g.*, blood, lymphatic system, central nervous system), increase oral availability, increase solubility to allow administration by injection, alter metabolism and alter rate of excretion. By way of example, a compound of the invention may be modified to incorporate a hydrophobic group or "greasy" moiety in an attempt to enhance the passage of the compound through a hydrophobic membrane, such as a cell wall.

These detailed descriptions fall within the scope, and serve to exemplify, the above-described General Synthetic Procedures which form part of the invention. These detailed descriptions are presented for illustrative purposes only and are not intended as a restriction on the scope of the invention.

Although the pharmacological properties of the compounds of the invention (Formula II) vary with structural change, in general, activity possessed by compounds of Formula II may be demonstrated both in vitro as well as in vivo. Particularly, the pharmacological properties of the compounds of this invention may be confirmed by a number of pharmacological in vitro assays. Exemplified pharmacological assays, described herein after Examples 2560-3400 below, have been carried out with the compounds according to the invention. Compounds of the invention were found to inhibit the activity of various kinase enzymes, including, without limitation, Tie-2, Lck, p38 and VEGF receptor kinases at doses less than 25 µM.

### Example 2560

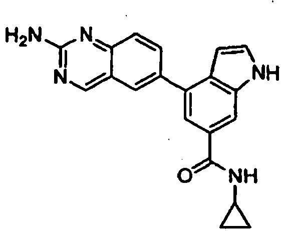

### 4-(2-Aminoquinazolin-6-yl)-N-cyclopropyl-1H-indole-6-carboxamide

Step 1: A mixture of methyl 4-bromo-1H-indole-6-carboxylate (1 g, 3.95 mmol), cyclopropanamine (1.0 ml, 11.8 mmol) in 50 ml THF stirred at 0 °C was treated with sodium bis(trimethylsilyl)amide (9 ml, 9 mmol). The mixture was stirred at 0 °C, allowing to warm to room temperature for 2 h, (MS: M+1 found to be 301/303). The mixture was quenched with 20 ml sat. NH₄Cl, extracted with ethyl acetate 3 x 50 ml. The combined organics were dried over anhydrous Na₂SO₄, concentrated via vacuo and purified by column chromatography eluting with 10-30% ethyl acetate / hexane to give the 4-bromo-N-cyclopropyl-1H-indole-6-carboxamide 0.34 g as pale yellow solid. MS (ES+): 279/301 (M+H); MW (calculated): 279.1.

Step 2: A mixture of 4-bromo-N-cyclopropyl-1H-indole-6-carboxamide (0.1 g, 0.358 mmol), 6-(4,4,5,5-tetramethyl-1,3,2-dioxaborolan-2-yl)quinazolin-2-amine (97 mg, 0.358 mmol), and Tetrakis(triphenylphosphine) palladium(0) (2.1 mg, 0.0018 mmol) in 5 ml DME/EtOH (4:1) was treated with the 2M aqueous solution of potassium carbonate (0.55 ml, 1.1 mmol). The mixture was warmed up to 120 °C in a microwave and stirred for 20 min. The mixture was cooled down to room temperature, diluted with 100 ml DCM, washed with H₂O 3 x 20 ml, brine 20 ml, dried over anhydrous Na₂SO₄, concentrated via vacuo. The crude product was purified by column chromatography eluting with 50% ethyl acetate / hexane to give the title compound 30 mg as pale yellow solid. MS (ES+): 344 (M+H); MW (calculated): 343.4.

### Example 2561

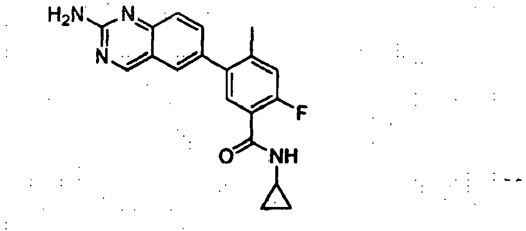

### 5-(2-aminoquinazolin-6-yl)-N-cyclopropyl-2-fluoro-4-methylbenzamide

Step 1: A mixture of 5 ml bromine and iron (0.6 g, 10 mmol) stirred at room temperature was treated with 2-fluoro-4-methylbenzoic acid (2.44 g, 15.8 mmol) in 5 portions. The mixture was stirred at room temperature in a sealed tube for 30 min. The mixture was poured into 150 ml 1M Na₂S₂O₃ and ice. The mixture was stirred for 30min and extracted with ethyl acetate 3 x 50 ml. The combined organics were washed with brine 50 ml, dried over anhydrous Na₂SO₄ and concentrated via vacuo. The crude product was purified via flash chromatography (silica gel) eluting with 1/1 hexanes/ethyl acetate to give 5-bromo-2-fluoro-4-methylbenzoic acid 3.2 g as white solid. MS (ES+): 233/235 (M+H); MW (calculated): 233.0.

Step 2: A mixture of 5-bromo-2-fluoro-4-methylbenzoic acid (3.2 g, 13.7 mmol) in sulfur chloride oxide (Cl₂SO) (11 ml, 153 mmol) was refluxed under nitrogen for 2 h. The clear resulting solution was concentrated via vacuo to remove the thionyl chloride.

A mixture of the residue in 50 ml DCM stirred at 0 °C was treated with cyclopropanamine (1.45 ml, 20 mmol) and triethylamine (3.8 ml, 17.4 mmol) drop wise. The mixture was stirred in a temperature rising from 0 °C to room temperature, for 2 h. The reaction was quenched with 50 ml H₂O, extracted with ethyl acetate 3 x 50 ml. The combined organics were washed with brine 50 ml, dried over anhydrous Na₂SO₄ and concentrated via vacuo. The crude product was purified via flash chromatography (silica gel) eluting with 1/1 hexanes/ ethyl acetate to give 5-bromo-N-cyclopropyl-2-fluoro-4-methylbenzamide 3.5 g as white solid. MS (ES+): 272/272 (M+H); MW (calculated): 272.1.

Step 3: A mixture of 5-bromo-N-cyclopropyl-2-fluoro-4-methylbenzamide (0.27 g, 1 mmol), 6-(4,9,5,5-tetramethyl-1,3,2-dioxaborolan-2-yl)quinazolin-2-amine (0.27 g, 1 mmol), and Tetrakis(triphenylphosphine) palladium(0) (0.11 g, 0.1 mmol) in 5 ml DME/EtOH (4:1) was treated with the 2M aqueous solution of potassium carbonate (1.5 ml, 3.0 mmol). The mixture was warmed up to 130 °C in a microwave and stirred for 20 min. The mixture was cooled down to room temperature, diluted with 100 ml DCM, washed with H₂O (3 x 20 ml), brine (1 x 20 ml), dried over anhydrous Na₂SO₄ and concentrated in vacuo. The crude product was purified by column chromatography eluting with 5% 2M ammonia methanol /DCM to give the title compound as pale yellow solid. MS (ES+): 337 (M+H); MW (calculated): 336.4.

### Example 2562

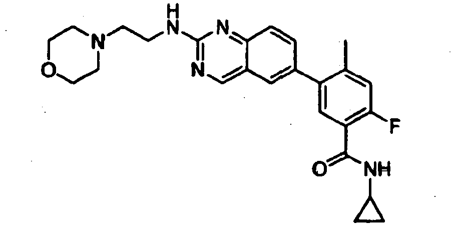

### N-cyclopropyl-2-fluoro-4-methyl-5-(2-(2-morpholinoethylamino)quinazolin-6-yl)benzamide

The title compound was prepared by a neat (solvent free) reaction between 5-(2-aminoquinazolin-6-yl)-N-cyclopropyl-2-fluoro-4-methylbenzamide and 2-morpholinoethanamine, at 140 °C. MS (ES+): 450 (M+H); MW (calculated): 449.5.

### Example 2563

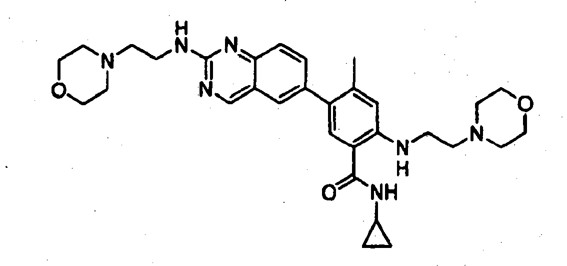

### N-cyclopropyl-4-methyl-2-(2-morpholinoethylamino)-5-(2-(2-morpholinoethylamino)quinazolin-6-yl)benzamide

A neat reaction between 5-(2-aminoquinazolin-6-yl)-N-cyclopropyl-2-fluoro-4-methylbenzamide and 2-morpholinoethanamine at 140 °C gave the title compound. MS (ES+): 560 (M+H); MW (calculated): 559.7.

### Example 2564

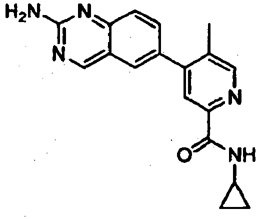

### 4-(2-aminoquinazolin-6-yl)-N-cyclopropyl-5-methylpicolinamide

Step 1: A mixture of 2,5-dimethylpyridine (34.5 ml, 0.3 mol) in 120 ml AcOH stirred at 57 °C was treated with 21 ml 30% H₂O₂, and stirred at 57 °C for 6 h. The mixture was treated with another 21 ml 30% H₂O₂ and stirred at 60 °C for 15h. The mixture was concentrated in vacuo to remove the AcOH, then diluted with 100 ml H₂O. The mixture was neutralized with solid Na₂CO₃ carefully to pH of about 7. The mixture was extracted with DCM 3 x 100ml. The combined organics were washed with brine 50 ml, dried over anhydrous Na₂SO₄ and concentrated in vacuo to give a crude 2,5-dimethylpyridine n-oxide in 35 g as pale yellow solid. MS (ES+): 124 (M+H); MW (calculated): 123.1.

Step 2: The above 2,5-dimethylpyridine n-oxide was added to the mixture of 29 ml fuming HNO₃ and 120 ml conc. H₂SO₄ at room temperature. The mixture was warmed up to 90 °C and stirred for 4 h. The mixture was poured into 300 ml crashed ice, neutralized with solid Na₂CO₃ to pH of about 7. The yellow precipitate was collected by filtration and washed with H₂O 2 x 100ml. Recrystalization of the precipitate from ethanol gave 2,5-dimethyl-4-nitropyridine n-oxide as yellow needle crystal solid. MS (ES+): 169 (M+H); MW (calculated): 168.1.

Step 3: A solution of 2,5-dimethyl-4-nitropyridine n-oxide (13.3 g, 79.2 mmol) stirred at 0 °C was slowly treated with 100 ml AcOCl. The mixture was stirred at 55 °C for 30 min. The mixture was poured into 150 ml crashed ice, neutralized with solid Na₂CO₃ to pH of about 7. The mixture was extracted with DCM 3 x 100ml. The combined organics were washed with brine 50 ml, dried over anhydrous Na₂SO₄ and concentrated in vacuo to give crude 2,5-dimethyl-4-chloropyridine n-oxide as yellow solid. MS (ES+): 159 (M+H); MW (calculated): 157.6.

Step 4: The crude 2,5-dimethyl-4-chloropyridine n-oxide obtained in step 3 above in 100 ml acetic anhydride was stirred at 120 °C for 3 h. The mixture was concentrated in vacuo to remove the acetic anhydride. The residue was dissolved in 300 ml DCM, washed with sat. NaHCO₃ carefully to pH of about 7. The organic layer was dried over anhydrous Na₂SO₄, concentrated in vacuo and purified on silica gel eluting with 5% 2M ammonia methanol / DCM to give (4-chloro-5-methylpyridin-2-yl)methyl acetate as pale yellow solid. MS (ES+): 200 (M+H); MW (calculated): 199.6.

Step 5: A mixture of (4-chloro-5-methylpyridin-2-yl)methyl acetate (9.8 g, 49 mmol) in 50 ml THF /H₂O (4:1) stirred at 0 °C was treated with lithium hydroxide monohydrate (4.55 g, 108 mmol). The mixture was stirred at 0 °C and allowed to warm to room temperature over 15 min. The mixture was quenched with 20 ml sat. NH₄Cl, extracted with ethyl acetate (3 x 50 ml). The combined organics were dried over anhydrous Na₂SO₄, concentrated in vacuo and purified on silica gel, eluting with 10-50% ethyl acetate / hexane to give (4-chloro-5-methylpyridin-2-yl)methanol as a pale yellow solid. MS (ES+): 158 (M+H); MW (calculated): 157.6.

Step 6: A mixture of (4-chloro-5-methylpyridin-2-yl)methanol (5.7 g, 36 mmol) in 25 ml dioxane stirred at room temperature was treated with aqueous solution of 5.8 g Na₂CO₃ in 50 ml H₂O and a solution of 6.9 g KMnO₄ in 100 ml H₂O. The mixture was stirred at room temperature for 2 h. The mixture was filtered and the filtrate was washed with 20 ml 1 N NaOH. The water phase was acidified with conc. HCl to pH of about 4, upon which a white solid precipitated out. The white solid was collected by filtration and dried in a vacuum oven affording 4-chloro-5-methylpicolinic acid as a white solid. MS (ES⁻): 170 (M-1); MW (calculated): 171.5.

Step 7: Using the procedure of Example 2561, step 2, starting from 4-chloro-5-methylpicolinic acid gave 4-chloro-N-cyclopropyl-5-methylpicolinamide. MS (ES+): 211 (M+H); MW (calculated): 210.6.

Step 8: Using the procedure of Example 2560, step 2, starting from 4-chloro-N-cyclopropyl-5-methylpicolinamide and 6-(4,4,5,5-tetramethyl-1,3,2-dioxaborolan-2-yl)quinazolin-2-amine gave the title compound. MS (ES+): 320 (M+H); MW (calculated): 319.3.

### Example 2565

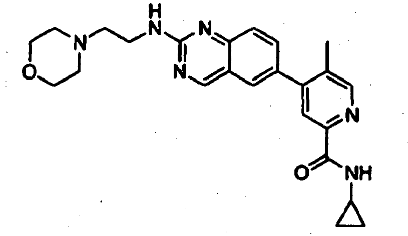

### N-cyclopropyl-5-methyl-4-(2-(2-morpholinoethylamino)quinazolin-6-yl)picolinamide

A neat reaction between 4-(2-aminoquinazolin-6-yl)-N-cyclopropyl-5-methylpicolinamide and 2-morpholinoethanamine at 140 °C gave the title compound. MS (ES+): 433 (M+H); MW (calculated): 432.5.

### Example 2566

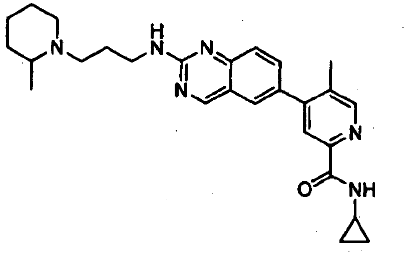

### N-cyclopropyl-5-methyl-4-(2-(3-(2-methylpiperidin-1-yl)propylamino)quinazolin-6-yl)picolinamide

A neat reaction between 4-(2-aminoquinazolin-6-yl)-N-cyclopropyl-5-methylpicolinamide and 3-(2-methylpiperidin-1-yl)propan-1-amine at 140 °C gave the title compound. MS (ES+): 459 (M+H); MW (calculated): 458.6.

### Example 2567

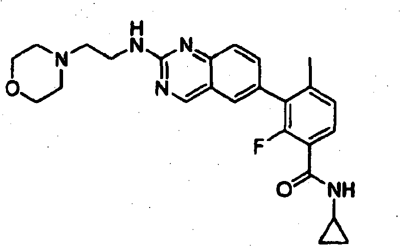

### N-cyclopropyl-2-fluoro-4-methyl-3-(2-(2-morpholinoethylamino)quinazolin-6-yl)benzamide

Step 1: The solid of N-(2-bromo-3-methylphenyl)-2-(hydroxyimino)acetamide (15 g, 58 mmol) (which was prepared by a method similar to that describe in 2 step of J. Med. Chem., 1991, 34 (1), 217) was added in 5 portions to 60 ml CH₃SO₃H stirred at 0 °C. The mixture was warmed up to 80 °C and stirred for 30 min. The mixture was cooled down to room temperature, poured onto 100 g crushed ice and diluted with 300 ml H₂O. The purple precipitate was collected, and dissolved in 300 ml 1N NaOH. The mixture was neutralized carefully with AcOH to pH of about 5, and the precipitate was filtered and washed with 20 ml H₂O. The clear water solution was further acidified by conc. HCl to pH of about 1, the orange precipitate was collected and washed with 50 ml H2O. The combined solids were dried in vacuum oven for 15 h to give the 7-bromo-6-methylindoline-2,3-dione 5.2 g as yellow solid. MS (ES+): 240/242 (M+H); MW (calculated): 240.0.

Step 2: A mixture of 7-bromo-6-methylindoline-2,3-dione (5.1 g, 21 mmol), KOH (1.4 g, 25 mmol) and KCl (3.2 g, 42 mmol) in 40 ml H₂O stirred at 0 °C was treated with 3.6 ml 30% H2O2 dropwise in 20 min. The mixture was stirred at 0 and allowed to warm to room temperature while stirring over 4 h. The mixture was neutralized with AcOH to pH of about 5, a yellow solid was precipitated out. The solid was collected by filtration, washed with H2O 2 x 50 ml and dried in vacuum oven for 15 h to give the 2-amino-3-bromo-4-methylbenzoic acid as a pale yellow solid. MS (ES+): 230/232 (M+H); MW (calculated): 230.1.

Step 3: A mixture of 2-amino-3-bromo-4-methylbenzoic acid (2.3 g, 10 mmol) in 100 ml DCM stirred at 0 °C was treated with NOBF₄ (1.3 g, 11mmol). The mixture was stirred at 0 °C to room temperature for 1h, concentrated in vacuo. The residue was heated up to 140 °C and maintained at that tepmerature for 1h. The mixture was woked up using a standard base-acid work-up to give 2-(3-bromo-2-fluoro-4-methylphenyl)-2-oxoacetic acid as a dark solid.

Step 4: A mixture of 2-(3-bromo-2-fluoro-4-methylphenyl)-2-oxoacetic acid (2.2 g, 9.36 mmol) in sulfur chloride oxide (Cl₂SO) (11 ml, 153 mmol) was refluxed under nitrogen for 2 h. The clear solution was concentrated in vacuo to remove the thionyl chloride.

The residue above in 50 ml DCM was stirred at 0 °C and treated with cyclopropanamine (1.45 ml, 20 mmol) and triethylamine (3.8 ml, 17.4 mmol) drop wise. The mixture was stirred from 0 °C to room temperature for 2 h. The reaction was quenched with 50 ml H₂O, extracted with ethyl acetate 3 x 50 ml. The combined organics were washed with brine 50 ml, dried over anhydrous Na₂SO₄ and concentrated in vacuo. The crude residue was purified via flash chromatography (silica gel) eluting with 1/1 hexanes/ ethyl acetate to afford 3-bromo-N-cyclopropyl-2-fluoro-4-methylbenzamide as a white solid. MS (ES+): 272/274 (M+H); MW (calculated): 272.1.

Step 5: Using the procedure of Example 2560, step 2, starting from 3-bromo-N-cyclopropyl-2-fluoro-4-methylbenzamide and N-(2-morpholinoethyl)-6-(4,4,5,5-tetramethyl-1,3,2-dioxaborolan-2-yl)quinazolin-2-amine gave the title compound. MS (ES+): 450 (M+H); MW (calculated): 449.5.

### Example 2568

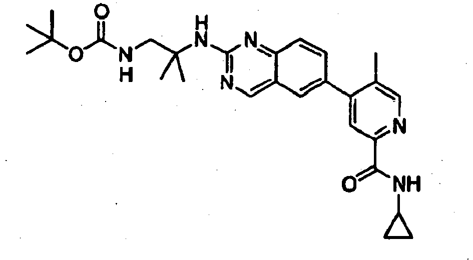

### Tert-butyl 2-(6-(2-(cyclopropylcarbamoyl)-5-methylpyridin-4-yl)quinazolin-2-ylamino)-2-methylpropylcarbamate

A mixture of tert-butyl 2-(6-bromoquinazolin-2-ylamino)-2-methylpropylcarbamate (0.395 g, 1 mmol), bis(pinacolato)diboron (0.254 g, 1 mmol), potassium acetate (0.35 g, 3 mmol) and dichloro[1,1'-bis(diphenylphosphino)ferrocene]palladium (ii) dichloromethane adduct (41 mg, 0.05 mol) in 4 ml DMF was stirred at 90 °C for 1 h. The mixture turned to dark red. The mixture was cooled to room temperature, and treated with 4-chloro-N-cyclopropyl-5-methylpicolinamide (0.21g, 1 mmol), Tetrakis(triphenylphosphine) palladium(0) (58 mg, 0.05 mmol), 5 ml DME/EtOH (4:1) and 2M aqueous solution of Potassium carbonate (1.5 ml, 3 mmol). The mixture was warmed up to 90 °C and stirred for 1.5h. The mixture was cooled down to room temperature, diluted with 100 ml DCM and washed with H₂O (3 x 20 ml), brine (20 ml), dried over anhydrous Na₂SO₄ and concentrated in vacuo. The crude was purified by column chromatography eluting with 10 - 35% ethyl acetate / hexane to give tert-butyl 2-(6-(2-(cyclopropylcarbamoyl)-5-methylpyridin-4-yl)quinazolin-2-ylamino)-2-methylpropylcarbamate as a pale yellow solid. MS (ES+): 491 (M+K); MW (calculated): 490.6.

### Example 2569

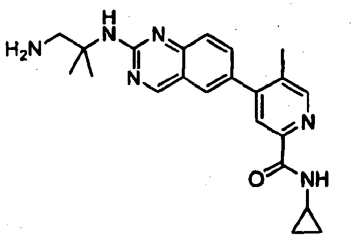

### 4-(2-(1-amino-2-methylpropan-2-ylamino)quinazolin-6-yl)-N-cyclopropyl-5-methylpicolinamide

Tert-butyl 2-(6-(2-(cyclopropylcarbamoyl)-5-methylpyridin-4-yl)quinazolin-2-ylamino)-2-methylpropylcarbamate in methanol was treated with 4 N HCl in dioxane at room temperature to give 4-(2-(1-amino-2-methylpropan-2-ylamino)quinazolin-6-yl)-N-cyclopropyl-5-methylpicolinamide. MS (ES+): 391 (M+H); MW (calculated): 390.5.

### Example 2570

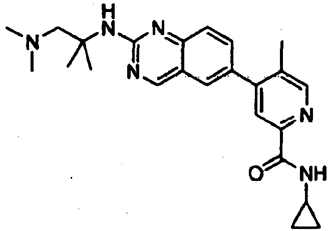

### N-cyclopropyl-4-(2-(1-(dimethylamino)-2-methylpropan-2-ylamino)quinazolin-6-yl)-5-methylpicolinamide

A one pot reaction procedure using the method described in Example 2568 and starting from 6-bromo-N-(1-(dimethylamino)-2-methylpropan-2-yl)quinazolin-2-amine and 4-chloro-N-cyclopropyl-5-methylpicolinamide gave the title compound. MS (ES+): 419 (M+H); MW (calculated): 418.5.

### Example 2571

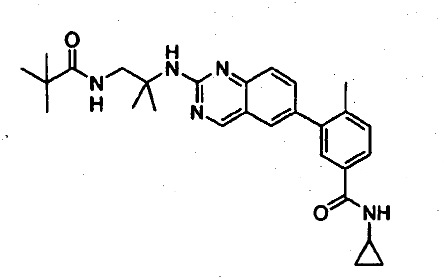

### N-cyclopropyl-4-methyl-3-(2-(2-methyl-1-pivalamidopropan-2-ylamino)quinazolin-6-yl)benzamide

Using the method described in step 2 of Example 2560, reacting N-(2-(6-bromoquinazolin-2-ylamino)-2-methylpropyl)pivalamide and N-cyclopropyl-4-methyl-3-(4,9,5,5-tetramethyl-1,3,2-dioxaborolan-2-yl)benzamide gave the title compound. MS (ES+): 474 (M+H); MW (calculated): 473.6.

### Example 2572

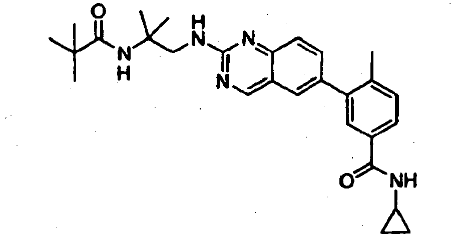

### N-cyclopropyl-4-methyl-3-(2-(2-methyl-2-pivalamidopropylamino)quinazolin-6-yl)benzamide

Using the method described in step 2 of Example 2560, reacting N-(1-(6-bromoquinazolin-2-ylamino)-2-methylpropan-2-yl)pivalamide and N-cyclopropyl-4-methyl-3-(4,4,5,5-tetramethyl-1,3,2-dioxaborolan-2-yl)benzamide gave the title compound. MS (ES+): 474 (M+H); MW (calculated): 473.6.

### Example 2573

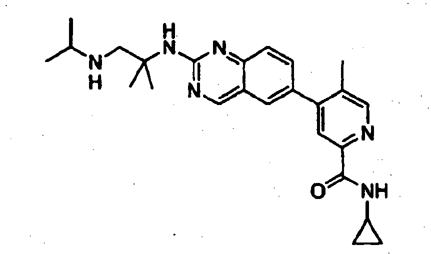

### N-cyclopropyl-4-(2-(1-(isopropylamino)-2-methylpropan-2-ylamino)quinazolin-6-yl)-5-methylpicolinamide

Using the method described in step 2 of Example 2568, reacting 6-bromo-N-(1-(isopropylamino)-2-methylpropan-2-yl)quinazolin-2-amine and 4-chloro-N-cyclopropyl-5-methylpicolinamide gave the title compound. MS (ES+): 433 (M+H); MW (calculated): 432.5.

### Example 2574

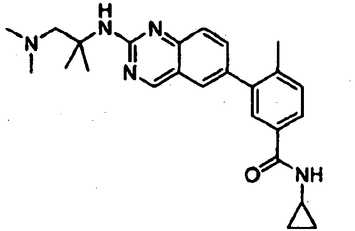

### N-cyclopropyl-3-(2-(1-(dimethylamino)-2-methylpropan-2-ylamino)quinazolin-6-yl)-4-methylbenzamide

Using the method described in step 2 of Example 2560, reacting 6-bromo-N-(1-(dimethylamino)-2-methylpropan-2-yl)quinazolin-2-amine and N-cyclopropyl-4-methyl-3-(4,4,5,5-tetramethyl-1,3,2-dioxaborolan-2-yl)benzamide gave the title compound. MS (ES+): 418 (M+H); MW (calculated): 417.5.

### Example 2575

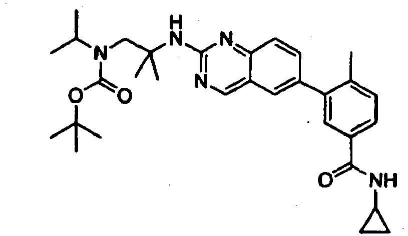

### Tert-butyl 2-(6-(5-(cyclopropylcarbamoyl)-2-methylphenyl)quinazolin-2-ylamino)-2-methylpropyl(isopropyl)carbamate

Using the method described in step 2 of Example 2560, reacting tert-butyl 2-(6-bromoquinazolin-2-ylamino)-2-methylpropyl(isopropyl)carbamate and N-cyclopropyl-4-methyl-3-(4,4,5,5-tetramethyl-1,3,2-dioxaborolan-2-yl)benzamide gave the title compound. MS (ES+): 532 (M+H); MW (calculated): 531.7.

### Example 2576

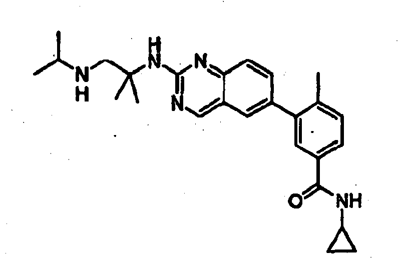

### N-cyclopropyl-3-(2-(1-(isopropylamino)-2-methylpropan-2-ylamino)quinazolin-6-yl)-4-methylbenzamide

Tert-butyl 2-(6-(5-(cyclopropylcarbamoyl)-2-methylphenyl)quinazolin-2-ylamino)-2-methylpropyl(isopropyl)carbamate in methanol was treated with 4N HCl in dioxane at room temperature to give the title compound. MS (ES+): 432 (M+H); MW (calculated): 4321.5.

### Example 2577 (Reference Example)

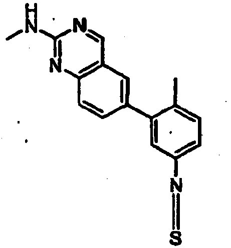

### 6-(5-isothiocyanato-2-methylphenyl)-N-methylquinazolin-2-amine

6-(5-amino-2-methylphenyl)-N-methylquinazolin-2-amine (0.500 g, 1.9 mmol) was taken up in CH₂Cl₂ (∼ 12 mL). To the solution was added O,O-dipyridin-2-yl carbonothioate (0.44 g, 1.9 mmol). The light brown solution was stirred at RT for 3 h. The crude reaction mixture was filtered through a Buchner apparatus with micromembrane filter, and the filtrate was washed with CH₂Cl₂ and dried to afford 6-(5-isothiocyanato-2-methylphenyl)-N-methylquinazolin-2-amine as a pale yellow powder (crop 1) MS (ESI, pos. ion) m/z: 307. The mother liquors were washed with water then dried over Na₂SO₄, filtered and concentrated to afford 6-(5-isothiocyanato-2-methylphenyl)-N-methylquinazolin-2-amine as a yellow/tan solid (crop 2). Crops 1 and 2 were used without further purification.

### Example 2578 (Reference Example)

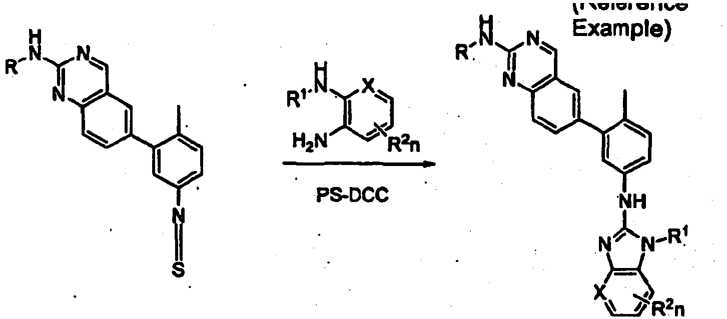

### Example 2578a (Reference Example)

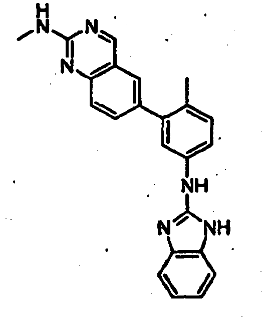

### 6-(5-(1H-benzo[d]imidazol-2-ylamino)-2-methylphenyl)-N-methylquinazolin-2-amine

In a 16 x 120 mm resealable pyrex tube 6-(5-isothiocyanato-2-methylphenyl)-N-methylquinazolin-2-amine (0.080 g, 0.26 mmol) [prepared according to Example 2576], Polymer supported Carbodiimide from Argonaut Technologies, 1.6 mmol/g (PS-DCC) (0.488 g, 0.78 mmol) and benzene-1,2-diamine (0.042 g, 0.39 mmol) were taken up in THF (6 mL). The tube was sealed and the mixture was stirred at 70 °C overnight. After cooling, the crude reaction mixture was filtered through a medium glass frit, and PS-DCC was washed with CH₂Cl₂. The solution was concentrated to dryness. The crude reaction mixture was taken up in minimal MeOH/DMSO and purified by preparative HPLC on an acidic Shimadzu chromatography column {15-85% (0.1% TFA in CH₃CN) in H2O over 15 min}. Pure product fractions were combined and neutralized with saturated NaHCO₃ then extracted with CH₂Cl₂, dried over Na₂SO₄, filtered and concentrated to dryness to afford 6-(5-(1H-benzo[d]imidazol-2-ylamino)-2-methylphenyl)-N-methylquinazolin-2-amine as a pale yellow solid. MS (ESI, pos. ion) m/z: 381.1 [M+1].

### Example 2579 (Reference Example)

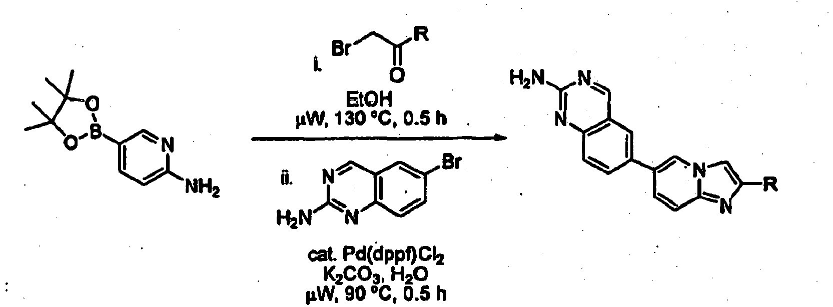

### Example 2579a (Reference Example)

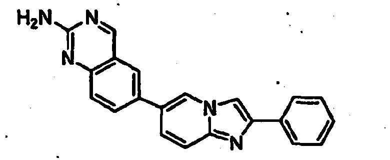

### 6-(2-phenylH-imidazo[1,2-a]pyridin-6-yl)quinazolin-2-amine

To a 10-20 L microwave reaction vessel was added 2-amino-5-(4,4,5,5-tetramethyl-1,3,2-dioxaborolan-2-yl)pyridine (0.200 g, 0.909 mmol), followed by 2-bromo-1-phenylethanone (0.199 g, 1.000 mmol). EtOH (2.0 mL) was added, the vessel was sealed, and the system was purged with N₂. The mixture was heated in the microwave for 30 min at 130 °C then cooled to RT and unsealed. To the reaction vessel was added 6-bromoquinolin-2-amine (0.170 g, 0.758 mmol), Pd(dppf)Cl₂ (0.093 g, 0.114 mmol), and K₂CO₃ (0.356 g, 2.576 mmol). EtOH (2.0 mL) and water (1.3 mL) was added, the vessel was sealed, and the system was purged with N₂. The mixture was heated in the microwave for 30 min at 90°C then allowed to cool to RT, transferred to a round bottom flask with EtOH and concentrated. The initial crude product was first purified by a MeOH wash. The solid was then further purified by ISCO column chromatography (SiO₂, gradient eluent: 20→40% 90:10:1 (CH₂Cl₂:CH₃OH:NH₄OH) in CH₂Cl₂) to afford 6-(2-PhenylH-imidazo[1,2-a]pyridin-6-yl)quinazolin-2-amine. MS (ESI, pos. ion), *m*/*z* 338.1 [M+H].

### Example 2580

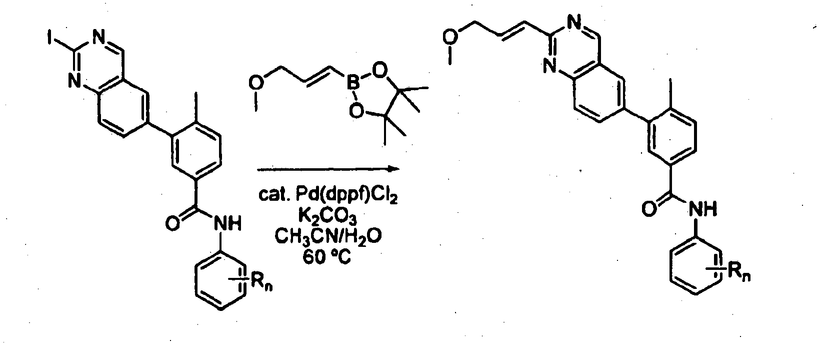

### Example 2580a

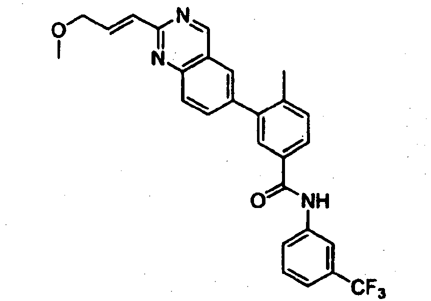

### (E)-3-(2-(3-Methoxyprop-1-enyl)quinazolin-6-yl)-4-methyl-N-(3-(trifluoromethyl)phenyl)benzamide

In a 16 x 120 mm resealable pyrex tube (E)-3-(2-(3-methoxyprop-1-enyl)quinazolin-6-yl)-4-methyl-N-(3-(trifluoromethyl)phenyl)benzamide (0.037 g, 0.069 mmol), Pd(dppf)Cl₂ (0.005 g, 0.007 mmol), and K₂CO₃ (0.015 g, 0.110 mmol) were taken up in CH₃CN (1.0 mL). (E)-2-(3-methoxyprop-1-enyl)-4,4,5,5-tetramethyl-1,3,2-dioxaborolane (0.023 mL, 0.110 mmol) and H₂O (0.20 mL) were added and the tube was sealed and heated at 60°C for 1.25 h. After cooling, the crude reaction mixture was taken up in CH₂Cl₂ and washed with water then dried over Na₂SO₄ and concentrated. The residue was purified by preparative MPLC on an Isco instrument eluting with 5-50% EtOAc in Hexanes over 30 min to afford (E)-3-(2-(3-methoxyprop-1-enyl)quinazolin-6-yl)-9-methyl-N-(3-(trifluoromethyl)phenyl)benzamide as a pale orange solid. MS (ESI, pos. ion) m/z: 478 [M+1].

### Example 2581

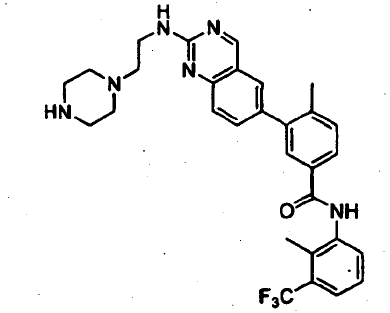

### 4-methyl-N-(2-methyl-3-(trifluoromethyl)phenyl)-3-(2-(2-(piperazin-1-yl)ethylamino)quinazolin-6-yl)benzamide

*Tert*-butyl 4-(2-(6-(2-methyl-5-((2-methyl-3-(trifluoromethyl)phenyl)carbamoyl)phenyl)quinazolin-2-ylamino)ethyl)piperazine-1-carboxylate (0.076 g, 0.117 mmol) [prepared according to Method A1] was dissolved in CH₂Cl₂ (2 mL) and TFA (0.400 mL, 5.19 mmol) was added. The mixture was allowed to stir at RT for 3 h then concentrated. The residue was purified by preparative MPLC on the Isco eluting with 10-100% 90:10:1 CH₂Cl₂:MeOH:NH₃ in CH₂Cl₂ over 10 min to afford the product as an off-white solid. MS (ESI, pos. ion) m/z: 549.2 [M+1].

### Example 2582 (Reference Example)

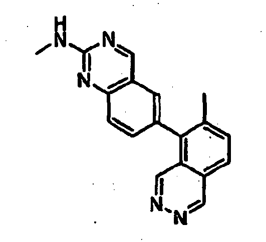

### N-Methyl-6-(6-methylphthalazin-5-yl)quinazolin-2-amine

In a 16 x 120 mm resealable pyrex tube, N-methyl-6-(4,4,5,5-tetramethyl-1,3,2-dioxaborolan-2-yl)quinazolin-2-amine (0.18 g, 0.62 mmol) [Example 739], Potassium carbonate (0.11 g, 0.79 mmol), Pd(dppf)Cl₂ (0.036 g, 0.049 mmol), and 5-bromo-6-methylphthalazine (0.110 g, 0.49 mmol) were taken up in DMF (3 mL) and H₂O (1 mL). The mixture was purged with N₂ and the sealed tube was heated at 60 °C for 2 h. The mixture was cooled, then taken up in CH₂Cl₂ and washed with water then dried over Na₂SO₄ and concentrated. The crude material was purified by MPLC: Isco {Redi-Sep® pre-packed silica gel column (40 g); eluent gradient: 5-80% EtOAc in hexanes over 20 min to afford the product as a tan solid. MS (ESI, pos. ion) m/z: 302.0 [M+1].

### Example 2583 (Reference Example)

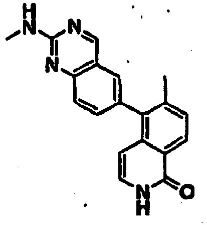

### 6-methyl-5-(2-(methylamino)quinazolin-6-yl)isoquinolin-1(2H)-one

A clear 80 ml microwave vessel was charged with N-methyl-6-(4,4,5,5-tetramethyl-1,3,2-dioxaborolan-2-yl)quinazolin-2-amine (19 g, 67 mmol), 5-iodo-6-methylisoquinolin-1(2H)-one (16 g, 56 mmol), Tetrakis(triphenylphosphine) palladium(0) (8 g, 7 mmol), 2 M sodium carbonate (19 ml, 112 mmol) and 40 ml of dioxane. The mixture was capped and heated in the CEM microwave for 15 mins at 150°C with the PowerMax set at 150 W. The reaction was then partitioned between water and ethyl acetate. The precipitate that formed was collected by suction filtration. The organic layer was washed with brine and dried with sodium sulfate. The collected solid and the organic layer were mixed together with a small amount of methanol to aid in dissolution. The solution was loaded unto silica and purified by column chromatography on silica gel using a gradient of 2 to 10% MeOH in dichloromethane to give 6-methyl-5-(2-(methylamino)quinazolin-6-yl)isoquinolin-1(2H)-one as a light yellow solid. MS (ESI, pos. ion) m/z: 317.1 [M+1].

### Example 2584 (Reference Example)

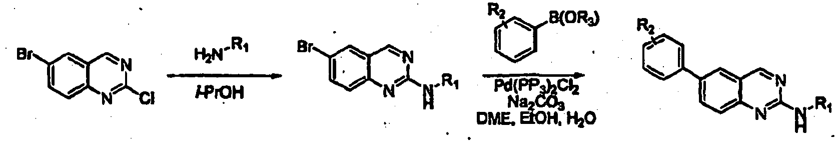

### Example 2584a (Reference Example)

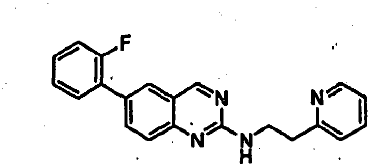

### 6-(2-fluorophenyl)-N-(2-(pyridin-2-yl)ethyl)quinazolin-2-amine

### Step 1: 6-bromo-N-(2-(pyridin-2-yl)ethyl)quinazolin-2-amine.

Into three separate microwave tubes were placed 6-bromo-2-chloroquinazoline (1.0 g, 4.1 mmol), 2-(2-ethylamino)-pyridine (590 ul, 4.9 mmol, Aldrich) and 2-propanol (10 ml). Each tube was heated to 140°C in the Emry's Optimizer microwave for 1 h. The mixtures were combined and cooled to -20°C overnight. The solids were collected by filtration, washed with cold 2-propanol and air-dried. Yield: 2.17 g (53%). MS (ESI, pos. ion) *m*/*z*: 329, 331 (M+1).

Step 2: A mixture of 6-bromo-N-(2-(pyridin-2-yl)ethyl)quinazolin-2-amine (165 mg, 0.50 mmol), 2-fluorobenzeneboronic acid (84 mg, 0.60 mmol, Lancaster), sodium carbonate (159 mg, 1.5 mmol, JT Baker) and trans-dichlorobis(triphenylphosphine)palladium (II) (35 mg, 0.050 mmol, Strem) in a mixture of ethylene glycol dimethyl ether, ethanol, and water (7:2:3, 2.0 ml) was heated to 150°C for 15 min in the Emry's Optimizer microwave. The mixture was diluted with MeOH and concentrated over Si02. Purification by flash chromatography (MeOH/CH2Cl2 = 0 → 2%) afforded the title compound. MS (ESI, pos. ion) *m*/*z*: 345.1 (M+1).

### Example 2584b (Reference Example)

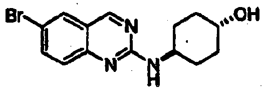

(1r,4r)-4-(6-bromoquinazolin-2-ylamino)cyclohexanol was prepared analogous to the method described in Example 2584a.

### Example 2585 (Reference Example)

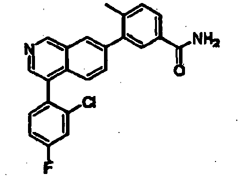

### 3-(4-(2-chloro-4-fluorophenyl)isoquinolin-7-yl)-4-methylbenzamide

### Step 1: 3-(4-bromoisoquinolin-7-yl)-4-methylbenzamide

Sodium carbonate (1 M, 1.65 ml, 1.65 mmol) and 4 mL of DME were added to 4,7-dibromoisoquinoline (0.237 g, 0.826 mmol), 4-methyl-3-(4,4,5,5-tetramethyl-1,3,2-dioxaborolan-2-yl)benzamide (0.216 g, 0.826 mmol), and tetrakis(triphenylphosphine)palladium (0.0477 g, 0.0413 mmol). The mixture was heated at reflux for 15 h and then cooled to 23°C. Water was added and the mixture was extracted with dichloromethane (3X). The combined organic layers were dried over anhydrous sodium sulfate, filtered and concentrated to give 354 mg of crude material. Crude material was purified by column chromatography (1% MeOH in dichloromethane to 5% MeOH in dichloromethane) to give 162 mg of 3-(4-bromoisoquinolin-7-yl)-4-methylbenzamide as a light yellow solid. MS(ES+): 341.1 (M+H).

Step 2: A microwave tube was charged with 3-(4-bromoisoquinolin-7-yl)-4-methylbenzamide (0.144 g, 0.42 mmol), 2-chloro-4-fluorophenylboronic acid (0.074 g, 0.42 mmol), tetrakis(triphenylphosphine)palladium (0.049 g, 0.042 mmol) and 2M potassium carbonate (0.63 ml, 1.3 mmol) in 2 mL of 4/1 DME/EtOH. The mixture was heated in the microwave at 140°C for 20 minutes. Water was added and the mixture was extracted with dichloromethane (3X). The combined organic layers were dried over anhydrous sodium sulfate, filtered and concentrated to give 200 mg of crude material which was purified by HPLC to give 3-(4-(2-chloro-4-fluorophenyl)isoquinolin-7-yl)-4-methylbenzamide as a white solid. MS(ES+): 391.1 (M+H).

### Example 2586 (Reference Example)

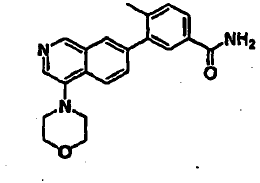

### 4-methyl-3-(4-morpholinoisoquinolin-7-yl)benzamide

Morpholine (0.03 ml, 0.3 mmol) was added dropwise to a solution of 3-(4-bromoisoquinolin-7-yl)-4-methylbenzamide (0.050 g, 0.1 mmol), tris(dibenzylideneacetone)dipalladium(0) chloroform adduct (0.02 g, 0.01 mmol), 2-(Dicyclohexylphosphino)-2',4',6'-tri-i-propyl-1,1'-biphenyl (0.03 g, 0.06 mmol) and cesium carbonate (0.10 g, 0.3 mmol) in 1 mL of dioxane. The mixture was heated to reflux and stirred overnight. The reaction was cooled to room temperature, filtered through celite and concentrated. The crude material was purified by HPLC to 4-methyl-3-(4-morpholinoisoquinolin-7-yl)benzamide as a white solid. MS(ES+): 348.2 (M+H).

### Example 2587

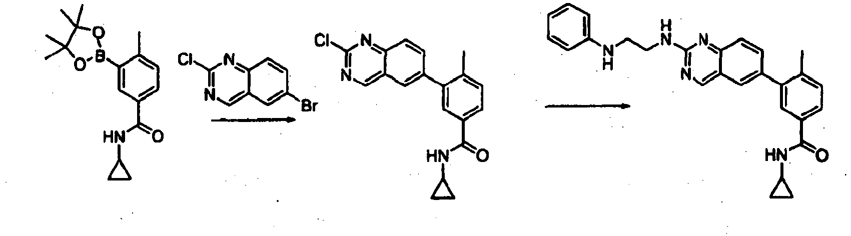

### N-cyclopropyl-4-methyl-3-(2-(2-(phenylamino)ethylamino)quinazolin-6-yl)benzamide

### Step 1: 3-(2-chloroquinazolin-6-yl)-N-cyclopropyl-4-methylbenzamide

DME (80 mL) and water (20 mL) were added to 6-bromo-2-chloroquinazoline (5.000 g, 20.5 mmol) [building block examples], N-cyclopropyl-4-methyl-3-(4,4,5,5-tetramethyl-1,3,2-dioxaborolan-2-yl)benzamide (7.42 g, 24.6 mmol), palladium acetate (0.0461 g, 0.205 mmol), triphenylphosphine (0.135 g, 0.513 mmol) and potassium acetate (10.1 g, 103 mmol) under nitrogen. The mixture was heated to 80°C and stirred overnight. The reaction was then cooled to 23°C. The organic layer was separated, dried over sodium sulfate, filtered and concentrated to give 13.05 g of a yellowish brown solid. The crude material was purified by silica gel chromatography (1% MeOH in dichloromethane to 2% MeOH in dichloromethane) to give 1.61 g of 3-(2-chloroquinazolin-6-yl)-N-cyclopropyl-4-methylbenzamide as a light yellow foam. MS(ES+): 338.1 (M+H).

### Step 2: N-cyclopropyl-4-methyl-3-(2-(2-(phenylamino)ethylamino)quinazolin-6-yl)benzamide

A microwave tube was charged with 3-(2-chloroquinazolin-6-yl)-N-cyclopropyl-4-methylbenzamide (0.170 g, 0.503 mmol), N-(2-aminoethyl)benzenamine (0.198 ml, 1.51 mmol) and potassium carbonate (0.0696 g, 0.503 mmol) in 5 mL of acetonitrile. The mixture was heated to 150°C for 20 minutes in the microwave. The reaction was diluted with ethyl acetate (30 mL) and washed with 1M NaOH. The organic layer was dried over sodium sulfate, filtered and concentrated to give 335 mg of a dark yellow oil. The crude material was purified by silica gel chromatography (1% MeOH in dichloromethane to 3% MeOH in dichloromethane) to give N-cyclopropyl-4-methyl-3-(2-(2-(phenylamino)ethylamino)quinazolin-6-yl)benzamide as a light yellow foam. MS(ES+): 438.2 (M+H).

### Example 2588

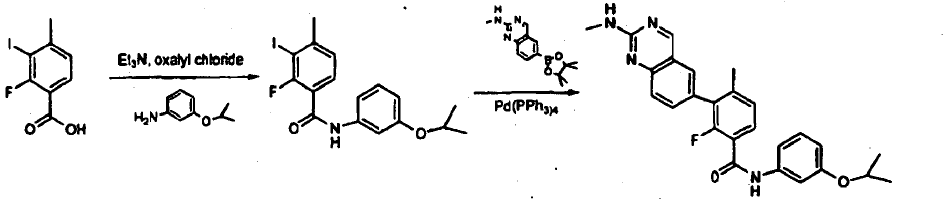

### 2-fluoro-N-(3-isopropoxyphenyl)-4-methyl-3-(2-(methylamino)quinazolin-6-yl)benzamide

### Step 1: 2-fluoro-3-iodo-N-(3-isopropoxyphenyl)-4-methylbenzamide:

To a suspension of 2-fluoro-3-iodo-4-methylbenzoic acid (684 mg, 2.44 mmol) in DCM (20 ml) was added 2 M solution of oxalyl dichloride in DCM (1.83 ml, 3.66 mmol) at 0°C and then 1 drop of DMF (cat.) was added. The resulting mixture.was stirred at RT for 3 hr and then solvent was removed under vacuum. DCM (5 ml) was added to the residue and the mixture was added slowly to a mixture of 3-isopropoxybenzenamine (0.54 ml, 3.66 mmol) and triethylamine (0.68 ml, 4.89 mmol) in DCM (10 ml) at 0°C. The resulting mixture was stirred at RT overnight and then water (50 ml) was added. The mixture was extracted with DCM (3x50 ml). The combined organic layers were washed with brine (100 ml) and then dried over Na₂SO₄. The solvent was removed in vacuo and residue was purified by TLC plate eluting with EtOAc/hexane (1:10) to provide the title compound as a tan solid. MS: found 414(M+H)⁺.

### Step 2: 2-fluoro-N-(3-isopropoxyphenyl)-4-methyl-3-(2-(methylamino)quinazolin-6-yl)benzamide:

A mixture of 2-fluoro-3-iodo-N-(3-isopropoxyphenyl)-4-methylbenzamide (630 mg, 1.53 mmol), N-methyl-6-(4,4,5,5-tetramethyl-1,3,2-dioxaborolan-2-yl)quinazolin-2-amine (522 mg, 1.83 mmol) and sodium carbonate (2M) (1.53 ml, 3.05 mmol) in DME (25 ml) was flushed with N₂ and then tetrakis(triphenylphosphine)palladium (176 mg, 0.15 mmol) was added. The mixture was refluxed for 24 hr and the cooled to RT. Solvent was removed in vacuo and the crude was purified by flash column chromatography, eluting with EtOAc/hexane (1:1) to obtain the title compound. MS: found 445 (M+H)⁺.

### Example 2589

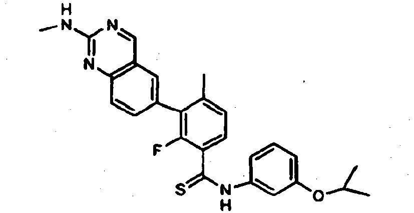

### 2-fluoro-N-(3-isopropoxyphenyl)-4-methyl-3-(2-(methylamino)quinazolin-6-yl)benzothioamide

A mixture of lawesson's reagent (47 mg, 115 µmol) and 2-fluoro-N-(3-isopropoxyphenyl)-4-methyl-3-(2-(methylamino)quinazolin-6-yl)benzamide (93 mg, 209 µmol) in toluene (8 ml) was refluxed for 16 hr. After cooling to RT, solvent was removed in vacuo and the crude was purified by flash column chromatography, eluting with EtOAc/hexane (1:1) to obtain the title compound. MS: (M+H)⁺ 461

### Example 2590

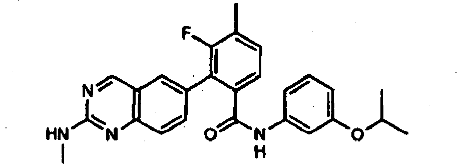

### 3-fluoro-N-(3-isopropoxyphenyl)-4-methyl-2-(2-(methylamino)quinazolin-6-yl)benzamide

The title compound was prepared in a manner analogous to that described in Example 2588. MS: Found m/z = 414(M+H)⁺

### Example 2591

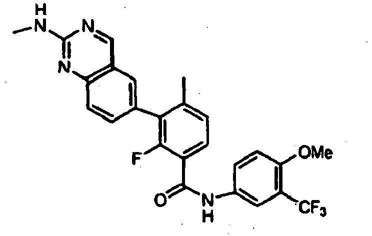

### 2-fluoro-N-(4-methoxy-3-(trifluoromethyl)phenyl)-4-methyl-3-(2-(methylamino)quinazolin-6-yl)benzamide

The title compound was prepared in a manner analogous to that described in Example 2588. MS: Found m/z = 485(M+H)⁺

### Example 2591 (General Method)

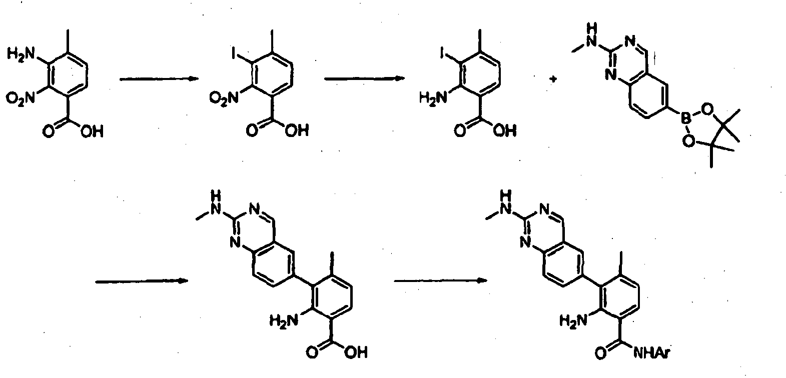

### 2-amino-N-(4-(aryl)phenyl)-4-methyl-3-(2-(methylamino)-6-quinazolinyl)benzamide

### Step 1: 3-Iodo-4-methyl-2-nitrobenzoic acid.

A solution of 3-amino-4-methyl-2-nitrobenzoic acid (1.00 g, 5.10 mmol, prepared according to procedures from U.S. Pat. Appl. Publ., 2004167194, 26 Aug 2004) in a mixture of DMSO (25.0 ml) and aqueous sulfuric acid (30%) (25.0 ml) was cooled to 0°C, and a solution of sodium nitrite (0.243 ml, 7.65 mmol) in water (3 mL) was added. The reaction mixture was stirred at 0°C for 1 h, after which a solution potassium iodide (2.54 g, 15.3 mmol) in water (5 mL) was added. After 1 h of stirring at that temperature EtOAc (200 mL) was added, and the mixture was washed sequentially with brine, 10% NaHSO₃, and water. The organic phase was dried over Na₂SO₄, filtered, and concentrated to afford an orange solid (1.27 g, 4.12 mmol, 81% yield). MS (ESI, neg.ion) m/z: 306.1 (M-1).

### Step 2: 2-amino-3-iodo-4-methylbenzoic acid

To a 100-mL round-bottomed flask equipped with a stir-bar and a reflux condenser was added 3-iodo-4-methyl-2-nitrobenzoic acid (3.1 g, 10.00 mmol), ethanol (25.0 ml, 543 mmol), and acetic acid (4.0 ml, 70 mmol). The mixture was heated to 60°C at which point the suspension became a clear orange solution. Iron powder (2.0 g, 36 mmol) was added in one portion and the mixture heated to reflux. After 30 minutes at reflux, the solution became opaque and took on a mustard color. Heating was continued for another 4 hours before the hot reaction mixture was poured into 200 mL 2N aqueous HCl. The suspension was extracted with 2x 150 mL EtOAc and the combined organics were washed with 1x 2N HCl and 2x brine. The organic layer was then dried over Na₂SO₄, and concentrated to afford a tan solid, 2-amino-3-iodo-4-methylbenzoic acid (2.56 g, 8.4 mmol, 84% yield). MS (ESI, positive ion) m/z: 277.9 (M+1).

### Step 3: 2-amino-4-methyl-3-(2-(methylamino)quinazolin-6-yl)benzoic acid

A microwave reactor vessel was charged with N-methyl-6-(4,4,5,5-tetramethyl-1,3,2-dioxaborolan-2-yl)quinazolin-2-amine (1.34 g, 4.69 mmol), 2-amino-3-iodo-4-methylbenzoic acid (1.00 g, 3.61 mmol), sodium carbonate hydrate (1.34 g, 10.8 mmol), dichloropalladiumbistriphenylphosphine (0.127 g, 0.180 mmol) and 15.0 mL of 10:1 DMF:H₂O. Sealed vessel and heated to 150°C for 900s in the microwave. Reaction mixture diluted with water, neutralized with TFA and extracted with EtOAc. The organics were washed with water and brine, then dried over Nₐ2SO₄. The solution was concentrated in vacuo and the resulting residue purified by column chromatography (2% AcOH in EtOAc/hexanes.) The cleanest fractions were combined and concentrated to afford 2-amino-4-methyl-3-(2-(methylamino)quinazolin-6-yl)benzoic acid (0.420 g, 1.36 mmol, 37.7% yield). MS (ESI, positive ion) m/z: 309.1 (M+1).

### Step 4: General procedure

TBTU (0.109 g, 0.341 mmol) was added as a solid in one portion to a stirring a solution of 2-amino-4-methyl-3-(2-(methylamino)quinazolin-6-yl)benzoic acid (0.0700 g, 0.227 mmol), amine (0.681 mmol), and hunig's base (0.119 ml, 0.681 mmol) in DMF (1.01 ml, 12.9 mmol) at RT. The reaction was stirred overnight. Reaction mixture was neutralized with HCl in dioxane and purified by reverse phase HPLC. The pure product fractions were combined and lyophilized. The residue was redissolved in dichloromethane, washed with aqueous sodium bicarbonate, water and brine, dried over sodium sulfate and concentrated, to yield the title compound, which was used without any further purification.

### Example 2591a

### 2-amino-4-methyl-3-(2-(methylamino)-6-quinazolinyl)-N-(3-((1-methylethyl)oxy)phenyl)benzamide

The title compound was prepared with 3-isopropoxybenzeneamine (0.100 mL), using the general procedure described in Example 2591. MS: Found m/z = 442.1(M+1).

### Example 2591b

### 2-amino-4-methyl-3-(2-(methylamino)-6-quinazolinyl)-N-(3-(1-methylethyl)phenyl)benzamide

The title compound was prepared with 3-iso-propylbenzeneamine (0.096 mL), using the general procedure described in Example 2591. MS: Found m/z = 426.3(M+1).

### Example 2591c

### 2-amino-N-(4-(1,1-dimethylethyl)phenyl)-4-methyl-3-(2-(methylamino)-6-quinazolinyl)benzamide

The title compound was prepared with 4-tert-butylbenzeneamine (0.102 mL), using the general procedure described in Example 2591. MS: Found m/z = 440.1(M+1).

### Example 2592

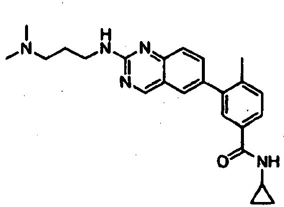

### N-cyclopropyl-3-(2-(3-(dimethylamino)propylamino)quinazolin-6-yl)-4-methylbenzamide

### Step 1: 6-bromo-N-(3-(dimethylamino)propyl)quinazolin-2-amine

A mixture of 6-bromoquinazolin-2-amine (100 mg, 0.45 mmol), N1,N1-dimethylpropane-1,3-diamine (684 mg, 6.7 mmol) and p-Toluenesulfonic acid monohydrate (169 mg, 0.90 mmol) in a sealed glass tube was heated in a oil bath at 165 °C for 16 hours. The reaction mixture was dissolved in methanol (2 mL), filtered and then purified using Gilson purification system. Stationary phase: Phenomenex C-18 column, Synergi 4µ.Max-RP, 150 x 21.20 mm. Gradient used: 10% to 90% Acetonitrile in water (both solvents containing 0.1% TFA) at 20 mL/min over 15 mins. The fractions were evaporated under reduced pressure to remove ACN and the remainder was separated between Satd. NaHCO₃ (30 mL) and EtOAc (15 mL). The organic layer was dried over MgSO₄ and evaporated to provide title compound as yellow solid. MS Found m/z: 310(M+1). Step 2: A mixture of 6-bromo-N-(3-(dimethylamino)propyl)quinazolin-2-amine (100 mg, 0.32 mmol), N-cyclopropyl-4-methyl-3-(4,4,5,5-tetramethyl-1,3,2-dioxaborolan-2-yl)benzamide (97 mg, 0.30 mmol), tetrakis(triphenylphosphine)palladium (26 mg, 0.02 mmol) and Cesium fluoride (147 mg, 0.96 mmol) in dioxane (1 mL)/water (1 mL) in a sealed glass tube in Smith synthesizer microwave at 150 °C for 10 minutes. The reaction mixture was poured in EtOAc (30 mL) and washed with water (2 x 50 mL). The organic layer was dried over MgSO₄ and evaporated to provide a crude product, which was purified by Gilson purification system. Stationary phase: Phenomenex C-18 column, Synergi 4µ.Max-RP, 150 x 21.20 mm. Gradient used: 10% to 90% Acetonitrile in water (both solvents containing 0.1% TFA) at 20 mL/min over 15 mins. The fractions were evaporated under reduced pressure to remove ACN and the remainder was separated between Satd. NaHCO₃ (30 mL) and EtOAc (15 mL). The organic layer was dried over MgSO₄ and evaporated to provide the title compound. MS Found m/z = 404(M+1).

### Example 2593 (General Scheme)

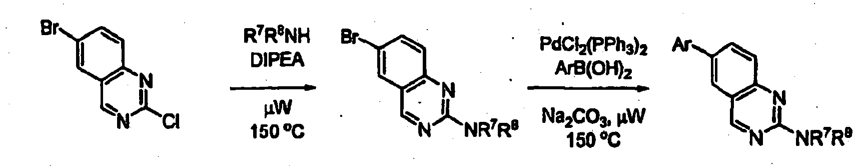

### Example 2593a (Reference Example)

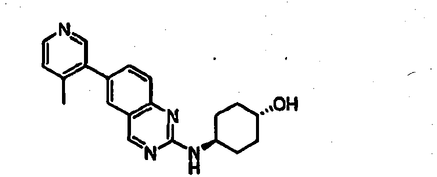

### (1R,4R)-4-(6-(4-methylpyridin-3-yl)quinazolin-2-ylamino)cyclohexanol

### Step 1: (1R,4R)-4-(6-bromoquinazolin-2-ylamino)cyclohexanol

A mixture of 6-bromo-2-chloroquinazoline (1.0 g, 4.1 mmol), (1r,4r)-4-aminocyclohexanol (0.47 g, 4.1 mmol) and N,N-diisopropylethylamine (0.8g, 6.2 mmol) dissolved in anhydrous DMF (1 mL) was heated in Smith synthesizer microwave at 165°C for 20 min. The reaction mixture was dissolved in EtOAc (25 mL) and washed with water (2 x 50 mL). The organic layer was dried over MgSO₄ and evaporated to provide crude product, which was purified by flash chromatography on SiO₂ (50-100% EtOAc in Hexanes), to provide the title compound. MS Found m/z = 323 (M+1).

Step 2: A mixture of (1r, 4r)-4-(6-bromoquinazolin-2-ylamino)cyclohexanol (100 mg, 0.31 mmol), 4-methylpyridin-3-ylboronic acid (43 mg, 0.31 mmol), dichlorobis(triphenylphosphine)palladium(II) (15 mg, 0.02 mmol) and Sodium Carbonate (154 mg, 1.2 mmol) in DME/water/EtOH mixture (2 mL, 7:3:2 mixture) was heated in a sealed glass tube in Smith synthesizer microwave at 150 °C for 15 minutes. The reaction mixture was dissolved in methanol (3 mL), filtered and then purified by Gilson purification system. Stationary phase: Phenomenex C-18 column, Synergi 4µ.Max-RP, 150 x 21.20 mm. Gradient used: 5% to 50% Acetonitrile in water (both solvents containing 0.1% TFA) at 20 mL/min over 10 mins. The fractions were evaporated under reduced pressure to remove ACN and the remainder was poured into Satd. NaHCO₃ (30 mL) and extracted with EtOAc (3 x 30 mL). The organic layers were combined and dried over Na₂SO₄ and evaporated to provide title compound as a pale yellow solid. MS Found m/z = 335(M+1).

### Example 2594 (Reference Example)

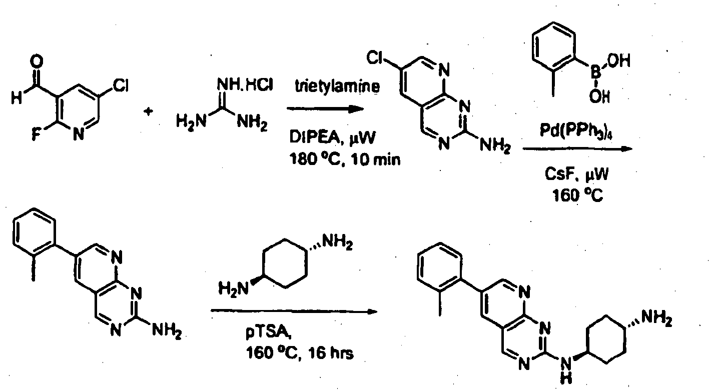

### (1R,4R)-N1-(6-o-tolylpyrido[2,3-d]pyrimidin-2-yl)cyclohexane-1,4-diamine

### Step 1: 6-chloropyrido[2,3-d]pyrimidin-2-amine

A mixture of 5-chloro-2-fluoronicotinaldehyde (1g, 6.3 mmol), triethylamine (2.6 mL, 18.9 mmol) and guanidine hydrochloride (716 mg, 7.5 mmol) in MeOH (2 mL) was heated in a sealed glass tube in Smith synthesizer microwave at 180°C for 10 minutes. The crude product was adsorbed on silica gel and purified by flash chromatography on SiO₂ (20-100% EtOAc in Hexanes) to provide the title compound. MS: Found m/z: 181(M+1).

### Step 2: 6-o-tolylpyrido[2,3-d]pyrimidin-2-amine

A mixture of 6-chloropyrido[2,3-d]pyrimidin-2-amine (140 mg, 0.77 mmol), o-tolylboronic acid (116 mg, 0.85 mmol), tetrakis(triphenylphosphine)palladium (63 mg, 0.05 mmol) and Cesium fluoride (353 mg, 2.3 mmol) in dioxane (1 mL)/water (1 mL) in a sealed glass tube was heated in Smith synthesizer microwave at 160°C for 10 minutes. The crude product was adsorbed on silica gel and purification was effected by flash chromatography on SiO₂ (40-100% EtOAc in Hexanes) to provide the title compound. MS: (ESI) m/z: 237.1(M+1).

### Step 3: (1R, 4R)-N1-(6-o-tolylpyrido[2,3-d]pyrimidin-2-yl)cyclohexane-1,4-diamine

A mixture of 6-o-tolylpyrido[2,3-d]pyrimidin-2-amine (60 mg, 0.25 mmol), (1r, 4r)-cyclohexane-1,4-diamine (434 mg, 3.8mmol) and p-toluenesulfonic acid monohydrate (97 mg, 0.50mmol) in a sealed glass tube was heated in a oil bath at 165°C for 16 hours. The reaction mixture was dissolved in DMSO to make 2mL, filtered and then purified by Gilson purification system. Stationary phase: Phenomenex C-18 column, Synergi 4µ.Max-RP, 150 x 21.20 mm. Gradient used: 5% to 35% Acetonitrile in water (both solvents containing 0.1% TFA) at 20mL/min over 12mins. The fractions were evaporated under reduced pressure to remove ACN and the remainder was separated between Satd. NaHCO₃ (30mL) and EtOAc (15mL). The organic layer was dried over MgSO₄ and evaporated to provide title compound as a yellow solid. MS: (ESI) m/z: 334 (M+1).

### Example 2595 (Reference Example)

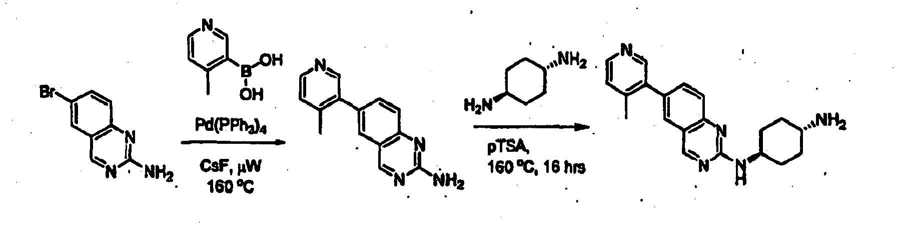

### (1r,4r)-N1-(6-(4-methylpyridin-3-yl)quinazolin-2-yl)cyclohexane-1,4-diamine

The title compound was prepared in a manner analogous t that described in step 2 and 3 of Example 2594. MS: (ESI) m/z: 334 (M+1).

### Example 2596 (Reference Example)

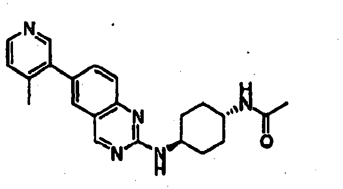

### N-((1r,4r)-4-(6-(4-methylpyridin-3-yl)quinazoling-2-ylamino)cyclohexyl) acetamide

To a solution of (1r,4r)-N1-(6-(4-methylpyridin-3-yl)quinazolin-2-yl)cyclohexane-1,4-diamine (85 mg, 255 µmol) in 5mL DCM was added potassium carbonate (53 mg) followed by drop-wise addition of acetic anhydride (14 µl, 153 µmol). The reaction mixture was allowed to stir at 25°C for 2 hours. The reaction mixture was poured in a separating funnel containing DCM (20mL) and water (50 mL). The organic layer was dried over MgSO4 and evaporated to yield the title compound. MS: Found(ESI) m/z: 376(M+1).

### Example 2597 (Reference Example)

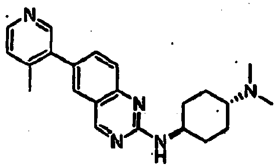

### (1r,4r)-N1,N1-dimethyl-N4-(6-(4-methylpyridin-3-yl)quinazolin-2-yl) cyclohexane-1,4-diamine

To a solution of (1r,4r)-N1-(6-(4-methylpyridin-3-yl)quinazolin-2-yl)cyclohexane-1,4-diamine (85 mg, 255 µmol) in MeOH (2mL) was added formaldehyde solution (95 µl, 1275 µmol) and acetic acid (0.2 mg, 3 µmol). After 30 minutes, sodium triacetoxyborohydride (108 mg, 510 µmol) was added and the mixture was allowed to stir at RT for 2 hours followed by heating under reflux condenser at 60°C for 3 hours. The reaction mixture was evaporated under reduced pressure and the residue re-dissolved in 10mL EtOAc and washed with satd. NaHCO₃ (2 x 25mL). The organic layer was dried over MgSO₄ and the crude product obtained was dissolved in MeOH and purified by Gilson purification system. Stationary phase: Phenomenex C-18 column, Synergi 4µ.Max-RP, 150 x 21.20 mm. Gradient used: 10% to 50% Acetonitrile in water (both solvents containing 0.1% TFA) at 20 mL/min over 10mins. The fractions were evaporated under reduced pressure to remove ACN and the remainder was separated between Satd. NaHCO₃ (30mL) and EtOAc (20 mL). The organic layer was dried over MgSO₄ and evaporated to yield the title compound. MS: Found (ESI) m/z: 362(M+1).

### Example 2598 (Reference Example)

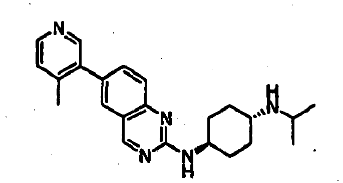

### (1r,4r)-Nl-isopropyl-N4-(6-(4-methylpyridin-3-yl)quinazolin-2-yl)cyclohexane-1,4-diamine

To a solution of (1r,4r)-N1-(6-(4-methylpyridin-3-yl)quinazolin-2-yl)cyclohexane-1,4-diamine (85 mg, 255 µmol) in MeOH (2mL) was added acetone (94 µl, 1275 µmol) and acetic acid (0.15 mg, 2.5 µmol) and the mixture was allowed to stir for 16 hours at room temperature followed by heating for 3 hours under reflux condenser at 60°C. The reaction mixture was evaporated under reduced pressure and re-dissolved in EtOAc (10mL) and washed with satd. NaHCO₃ (2 x 25mL). The organic layer was dried over MgSO₄ to yield crude which was dissolved in MeOH and purified by Gilson purification system. Stationary phase: Phenomenex C-18 column, Synergi 4µ.Max-RP, 150 x 21.20 mm. Gradient used: 10% to 50% Acetonitrile in water (both solvents containing 0.1% TFA) at 20mL/min over 10mins. The fractions were evaporated under reduced pressure to remove ACN and the remainder was separated between Satd. NaHCO₃ (30mL) and EtOAc (20mL). The organic layer was dried over MgSO₄ and evaporated to yield pure MS: Found (ESI) m/z: 376 (M+1).

### Example 2599 (Reference Example)

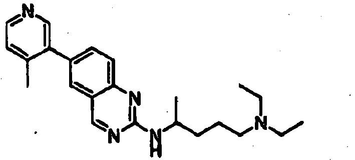

### N-(5-(diethylamino)pentan-2-yl)-6-(4-methylpyridin-3-yl)quinazolin-2-amine

### Step 1: 6-Bromo-N-(5-(diethylamino)pentan-2-yl)quinazolin-2-amine:

A mixture of 6-bromo-2-chloroquinazoline (2.43 g, 10.0mmol) and 2-amino-5-diethylaminopentane (2.6 ml, 13mmol) in NMP (5.0 ml) and dioxanes (5.0 ml) in a sealed glass tube was heated in a Personal Chemistry microwave at 150°C for 10 min. The mixture was treated with 30 ml of 1N NaOH and extracted with EtOAc (3 X 80 ml). The combined organic layers were washed with brine (10 ml), dried over magnesium sulfate and concentrated under reduced pressure. Purification was effected by flash chromatography on SiO₂ (2-15% of [2 M NH₃ in MeOH] in DCM) to provide the title compound (3.1 g, 85% yield) as a amorphous yellow solid. MS: (ESI) m/z: 366.2(M+1).

Step 2: A mixture of 6-bromo-N-(5-(diethylamino)pentan-2-yl)quinazolin-2-amine (160 mg, 0.43mmol), 4-methylpyridin-3-ylboronic acid (66mg, 0.48mmol), tetrakis(triphenylphosphine)palladium (26 mg, 0.02mmol) and 2N Na₂CO₃ (0.5 ml) in dioxane (2ml) in a sealed glass tube was heated in a Personal Chemistry microwave at 130°C for 20 min. The mixture was treated with 3 ml of 1N NaOH and extracted with EtOAc (3 X 5ml). The combined organic layers were dried over sodium sulfate and concentrated under reduced pressure. Purification was effected by flash chromatography on SiO₂ (2-15% of [2 M NH₃ in MeOH] in DCM) to provide the title compound as a amorphous yellow solid. MS: (ESI) m/z: 378.4 (M+1).

### Example 2600 (Reference Example)

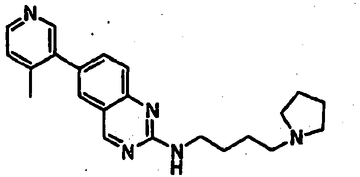

### 6-(4-methylpyridin-3-yl)-N-(4-(pyrrolidin-1-yl)butyl)quinazolin-2-amine

A mixture of 6-(4-methylpyridin-3-yl)quinazolin-2-amine (83mg, 0.35mmol), 4-(pyrrolidin-1-yl)butan-1-amine (497mg, 3.5mmol) and p-Toluenesulfonic acid monohydrate (133 mg, 0.7ml) in a sealed glass tube was heated in an oil bath at 165°C for 5 h. The mixture was treated with 3 ml of 1 N NaOH and extracted with EtOAc (3 X 10ml). The combined organic layers were dried over sodium sulfate and concentrated under reduced pressure. Purification was effected by flash chromatography on SiO₂ (2-12% of [2 M NH₃ in MeOH] in DCM) to provide the title compound as a amorphous yellow solid. MS: (ESI) m/z: 362(M+1).

### Example 2601

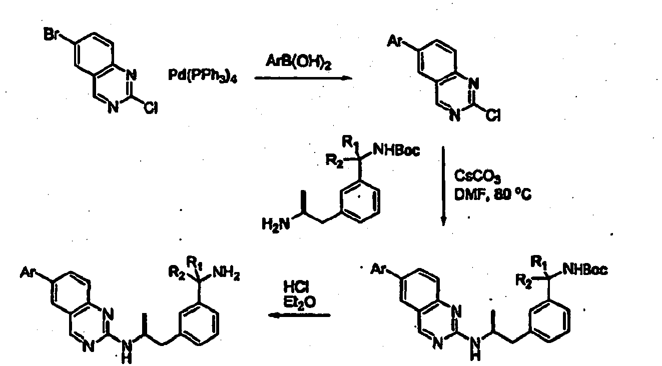

### Example 2601a (Reference Example)

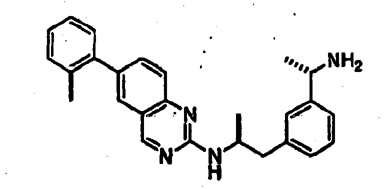

### N-((S)-1-(3-((S)-1-Aminoethyl)phenyl)propan-2-yl)-6-o-tolylquinazolin-2-amine

### Step 1: 2-Chloro-6-o-tolylquinazoline

A mixture of 6-bromo-2-chloroquinazoline (500 mg, 2.05mmol), o-tolylboronic acid (293 mg, 2.15mmol), tetrakis(triphenylphosphine)palladium (127 mg, 0.11mmol) and 2N Na₂CO₃ (2.2 ml) in DME (4 ml)/EtOH (4 ml)/water (1 ml) in a sealed glass tube was heated in a Personal Chemistry microwave at 120°C for 20 min. The mixture was treated with 5 ml of 1N NaOH and extracted with EtOAc (3 X 20ml). The combined organic layers were dried over sodium sulfate and concentrated under reduced pressure. Purification was effected by flash chromatography on SiO₂ (15-55% EtOAc in Hexanes) to provide the title compound (370, 71% yield) as a yellow amorphous solid. MS (ESI) m/z: 255 (M+1).

### Step 2: Tert-butyl-(S)-1-(3-((S)-2-(6-o-tolylquinazolin-2-ylamino)propyl)phenyl)ethylcarbamate

A mixture of 2-chloro-6-o-tolylquinazoline (282 mg, 1.11mmol), tert-butyl (S)-1-(3-((S)-2-aminopropyl)phenyl)ethylcarbamate (370 mg, 1.33mmol) and cesium carbonate (321mg, 1.66mmol) in 3ml of DMF was heated in an oil bath at 80°C for 3 h. The mixture was cool to RT, filtered through a fritted funnel, rinsed with EtOAc. The filtrate was concentrated and the residue was purified by flash chromatography on SiO₂ (25-70% EtOAc in Hexanes) to provide the title compound (385 mg, 70% yield) as a yellow amorphous solid. MS: (ESI) m/z: 497 (M+1).

### Step 3: N-((S)-1-(3-((S)-1-Aminoethyl)phenyl)propan-2-yl)-6-o-tolylquinazolin-2-amine

To a solution of tert-butyl-(S)-1-(3-((S)-2-(6-o-tolylquinazolin-2-ylamino)propyl)phenyl)ethylcarbamate (347 mg, 0.70mmol) in 3 ml ether at RT was added 1N HCl in ether (3.5 ml, 3.5mmol). The mixture was stirred for 4 h, and the volatiles were removed under reduced pressure. The yellow residue was dissolved in 2 ml MeOH and 1 ml DMSO, and loaded on a reversed phased HPLC (10-90% [0.1% TFA in ACN] in [0.1% TFA in water]). The desired fractions were collected, concentrated, and partitioned between 1N NaOH (5ml) and EtOAc (50ml). The EtOAc layer was dried and concentrated to provide the title compound as a yellow amorphous solid. MS: (ESI) m/z: 397 (M+1).

### Example 2602

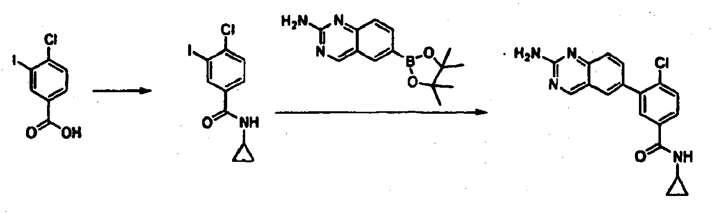

### 3-(2-Aminoquinazolin-6-yl)-4-chloro-N-cyclopropylbenzamide

### Step 1: 4-Chloro-N-cyclopropyl-3-iodobenzamide

4-Chloro-3-iodobenzoic acid (9.00 g, 31.9 mmol) was suspended in thionyl chloride (15mL) before it was heated to 65°C for 4 h. The reaction mixture was concentrated before it was dissolved in dioxane (10 mL), cyclopropylamine (2.45 mL, 35 mmol) and Hünigs base (11 mL, 64 mmol) were added simultaneously at ambient temperature. The reaction mixture was stirred for 16 h at ambient temperature when it was diluted with dichloromethane (100· mL), washed with saturated, aqueous sodium bicarbonate, separated, dried over anhydrous sodium sulfate and concentrated. The crude solid was dissolved in dichloromethane (15mL) and placed at -5°C for 16 h before the solid which had formed was filtered and washed with hexanes to give the title compound. MS (ES+): 322 (M+H)⁺.

### Step 2: 3-(2-Aminoquinazolin-6-yl)-4-chloro-N-cyclopropylbenzamide

To 4-chloro-*N*-cyclopropyl-3-iodobenzamide (52 mg, 0.16mmol), 6-(4,4,5,5-tetramethyl-1,3,2-dioxaborolan-2-yl)quinazolin-2-amine (40 mg, 0.15mmol), and tetrakis(triphenylphosphine)palladium (17 mg, 0.015mmol) was added DME (3.0mL), 2 M sodium carbonate in water (0.15mL, 0.030mmol), and potassium bromide (36mg, 0.30 mmol) before it was placed in the microwave at 150°C for 10 min. The reaction mixture was diluted with 50mL DCM, washed with 20mL saturated, aqueous NaHCO₃, and dried over anhydrous Na₂SO₄. After purification by chromatography, the title compound was obtained. MS: (ES+): 339(M+H)⁺.

### Example 2603

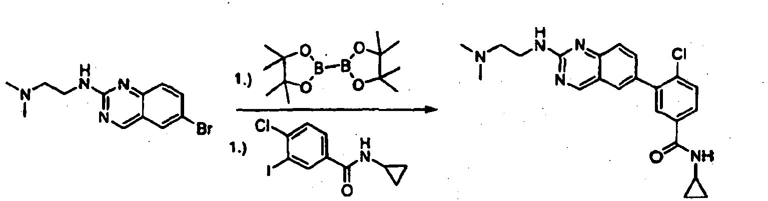

### 4-Chloro-N-cyclopropyl-3-(2-(2-(dimethylamino)ethylamino)quinazolin-6-yl)benzamide

To 6-bromo-*N*-(2-(dimethylamino)ethyl)quinazolin-2-amine (143mg, 0.48mmol), bis(pinacolato)diboron (185mg, 0.73mmol), and potassium acetate (93mg, 0.97mmol) was added DMF (5mL) before PdCl₂(dppf) (35mg, 0.048mmol) was added; the reaction mixture was heated to 80°C for 3 h when the boronic ester was observed by MS. The above reaction mixture was added to a vial containing 4-chloro-*N*-cyclopropyl-3-iodobenzamide (156mg, 0.48mmol), tetrakis(triphenylphosphine)palladium (56mg, 0.048mmol), 2M sodium carbonate in water (0.5mL, 1.0mmol), and ethanol (5mL) and was placed in the microwave for 10 min. at 130°C. The reaction mixture was diluted with 50mL DCM, washed with 20mL saturated, aqueous NaHCO₃, and dried over anhydrous Na₂SO₄. After purification by HPLC, the title compound was obtained. MS (ES+): 410 (M+H)⁺.

### Example 2604

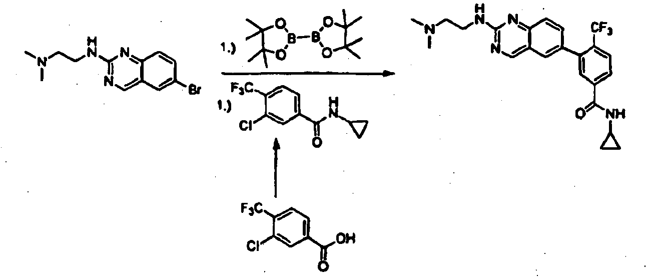

### N-cyclopropyl-3-(2-(2-(dimethylamino)ethylamino)quinazolin-6-yl)-4-(trifluoromethyl)benzamide

### Step A: 3-Chloro-N-cyclopropyl-4-(trifluoromethyl)benzamide

3-Chloro-4-(trifluoromethyl)benzoic acid (3.0g, 13.49mmol) was suspended in thionyl chloride (20mL) before it was heated to 80 °C for 1h. The reaction mixture was concentrated before it was dissolved in dioxane (20mL) and cyclopropylamine (2.1mL, 30mmol) simultaneously at ambient temperature. The reaction mixture was stirred for 16 h at ambient temperature when it was diluted with EtOAc (50mL), washed with 3N HCl and saturated, aqueous sodium bicarbonate separated, dried over anhydrous sodium sulfate, and concentrated to give the title compound. MS (ES+): 264(M+H)⁺.

### Step B: N-cyclopropyl-3-(2-(2-(dimethylamino)ethylamino)quinazolin-6-yl)-4-(trifluoromethyl)benzamide

To 3-chloro-*N*-cyclopropyl-4-(trifluoromethyl)benzamide (27 mg, 0.10 mmol), bis(pinacolato)diboron (39 mg, 0.15 mmol), potassium acetate (20 mg, 0.21 mmol), and potassium bromide (18 mg, 0.15 mmol) was added dioxane (1.5 mL) before it was sparged with nitrogen gas for 10 min. Pd(dba)₂ (9 mg, 0.015 mmol) and PCy₃ (4 mg, 0.015 mmol) were added to the reaction mixture before it was placed in the microwave for 10 min. at 160°C when the boronic ester was observed by MS. The above reaction mixture was added to a vial containing 6-bromo-*N*-(2-(dimethylamino)ethyl)quinazolin-2-amine (30 mg, 0.10 mmol), tetrakis(triphenylphosphine)palladium (18 mg, 0.15 mmol), 2 M sodium carbonate in water (0.10mL, 0.21 mmol), and ethanol (3mL) and was placed in the microwave for 10 min. at 165°C. The reaction mixture was diluted with 50mL EtOAc, washed with 20mL saturated, aqueous NaHCO₃, and dried over anhydrous Na₂SO₄. After purification by prep TLC, then HPLC, the title compound was obtained. MS: (ES+): 994(M+H)⁺,

### Example 2605

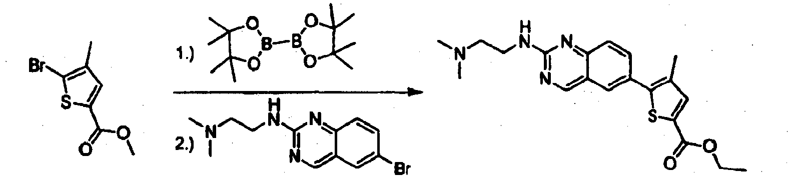

### Ethyl 5-(2-(2-(dimethylamino)ethylamino)quinazolin-6-yl)-4-methylthiophene-2-carboxylate

To 6-bromo-*N*-(2-(dimethylamino)ethyl)quinazolin-2-amine (150 mg, 508 µmol), bis(pinacolato)diboron (194 mg, 762 µmol), potassium acetate (199 mg, 2033 µmol), and 1,1'-bis(diphenylphosphino)ferrocene-palladium dichloride (37 mg, 51 µmol) was added DMF (2.5 ml) and was placed in the microwave for 10 minutes at 160°C when the boronic ester was observed by MS. The reaction mixture was placed in another microwave vial containing methyl 5-bromo-4-methylthiophene-2-carboxylate (143 mg, 610 µmol), tetrakis(triphenylphosphine)palladium (59 mg, 51 µmol), potassium carbonate- 2 M in water (1016 µl, 2033 µmol), and ethanol (2.5 ml) and placed back in the microwave for 10 minutes at 160°C giving crude product as the acid and ethyl ester by MS. The reaction mixture was diluted with 75mL of EtOAc, added to an separatory funnel, partitioned with sodium bicarbonate (saturated, aqueous), washed 3 times with 50mL of sodium bicarbonate (saturated, aqueous), and separated; the EtOAc layer was dried over sodium sulfate and concentrated to give crude ester. After purification by HPLC, the title compound was obtained. MS(ES+): 385(M+H)⁺.

### Example 2606 (General Method)

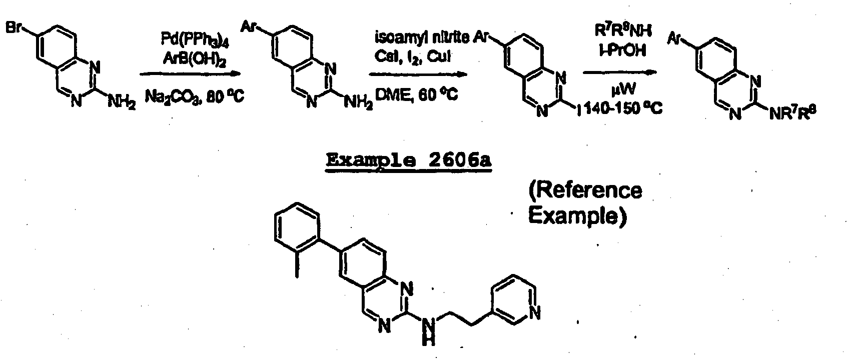

### 6-(2-Methylphenyl)-N-(2-(3-pyridinyl)ethyl)-2-quinazolinamine

### Step 1: 6-o-Tolylquinazolin-2-amine

A mixture of 6-bromoquinazolin-2-amine (3.02 g, 13.5 mmol), o-tolylboronic acid (2.23 g, 16.4 mmol), Na₂CO₃ (6.02 g, 56.8 mmol) and PdCl₂ (PPh)₂ (550 mg, 0.8 mmol) in 70 mL DME/30 mL H₂O/20 mL EtOH was heated to 80°C for 3 h. The mixture was cooled to room temperature and partitioned between EtOAc/H₂O. The aqueous layer was extracted with EtOAc (3X) and the combined organics were washed with brine and dried over Na₂SO₄. The solution was filtered, evaporated onto SiO₂ and purified by flash column chromatography eluting with EtOAc:hexane (0:1 → 1:0) to give 1.85 g (58 %) of a yellow amorphous solid. MS (ESI) m/z: 236.1(M+1).

### Step 2: 2-Iodo-6-o-tolylquinazoline

To a room temperature solution of 6-o-tolylquinazolin-2-amine (1.85 g, 8.0 mmol) in 40 mL DME was added CsI (2.01, 7.8 mmol), iodine (1.00 g, 3.9 mmol), CuI (454 mg, 2.4 mmol) and isoamyl nitrite (4.2 mL, 31.3 mmol). The mixture was heated to 60°C for 3 h. The reaction mixture was filtered through a pad of Celite and the pad was washed with toluene until the filtrate became clear. The filtrate was concentrated in vacuo, redissolved in EtOAc, evaporated onto SiO₂ and purified by flash column chromatography eluting with EtOAc:hexane (0:1 → 1:4) to give 703 mg (26 %) of an orange amorphous solid. MS (ESI) m/z: 347.0 (M+1).

### Step 3: 6-(2-Methylphenyl)-N-(2-(3-pyridinyl)ethyl)-2-quinazolinamine

A mixture of 2-iodo-6-o-tolylquinazoline (100 mg, 0.3mmol) and 3-(2-aminoethyl)pyridine (0.050mL, 0.4mmol) in 2mL isopropanol was heated to 140-150°C for 15-25 min in the Smith Optimizer microwave by Personal Chemistry. The reaction mixture was evaporated onto SiO₂ and purified by flash column chromatography eluting with 2M NH₃ in MeOH/CH₂Cl₂ (0:1 → 3:97) to the title compound as a light yellow amorphous solid. MS (ESI) m/z: 341.1 (M+1).

### Example 2607 (Reference Example)

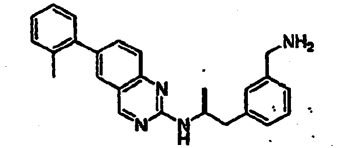

### N-((1S)-2-(3-(Aminomethyl)phenyl)-1-methylethyl)-6-(2-methylphenyl)-2-quinazolinamine

### Step 1: tert-Butyl (3-((S)-2-(6-o-tolylquinazolin-2-ylamino)propyl)phenyl)methylcarbamate

A mixture of 2-iodo-6-o-tolylquinazoline (153 mg, 0.4 mmol), (S)-tert-butyl 3-(2-aminopropyl)benzylcarbamate (142 mg, 0.5 mmol) and Cs₂CO₃ in 3 mL DMF was heated to 80°C for 4 h. The reaction mixture was cooled to room temperature and diluted with H₂O. The solution was extracted with EtOAc (3X) and the combined organics were evaporated onto SiO₂ and purified by flash column chromatography eluting with EtOAc/hexane (0:1 → 3:7) to give the title compound as a yellow oil. MS (ESI) m/z: 483.2 (M+1).

### Step 2: N-((1S)-2-(3-(Aminomethyl)phenyl)-1-methylethyl)-6-(2-methylphenyl)-2-quinazolinamine

To a cooled (0°C) solution of tert-butyl (3-((S)-2-(6-o-tolylquinazolin-2-ylamino)propyl)phenyl)methylcarbamate (150 mg, 0.3 mmol) in 4 mL Et₂O was added 1M HCl in Et₂O (0.6 mL). The reaction was warmed to room temperature and monitored by TLC. After 6 h, the reaction was diluted with MeOH, evaporated onto SiO₂ and purified by flash column chromatography eluting with 2M NH₃ in MeOH/CH₂Cl₂ (0:1 → 4:96) to give the title compound as a yellow tar. MS (ESI) m/z: 383.2 (M+1).

### Example 2608 (Reference Example)

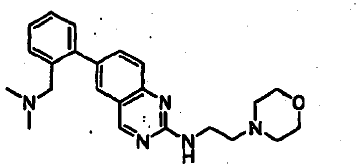

### 6-(2-((Dimethylamino)methyl)phenyl)-N-(2-(4-morpholinyl)ethyl)-2-quinazolinamine

### Step 1: 6-Bromo-N-(2-morpholinoethyl)quinazolin-2-amine

A mixture of 6-bromoquinazolin-2-amine (900 mg, 4.0 mmol), 4-(2-aminoethyl) morpholine (8.0 mL, 61 mmol) and p-toluensulfonic acid (1.55 g, 8.1 mmol) was heated to 160°C. After 15 h, excess amine was removed in vacuo and the residue was dissolved in 30 mL CH₂Cl₂. The solution was partitioned against 23 mL H₂O/7 mL 2M HCl and the aqueous layer was extracted with CH₂Cl₂ (3X). The combined organics were diluted with 35 mL CH₂Cl₂, washed with 2.5M NaOH (2X) and dried over Na₂SO₄. The acidic aqueous layer was basified with 5M NaOH, extracted with CH₂Cl₂ (3X) and dried over Na₂SO₄. All organic extracts were combined, evaporated onto SiO₂ and purified by flash column chromatography eluting with 2M NH₃ in MeOH/CH₂Cl₂ (0:1 → 1:19) to give 576 mg (43 %) of a yellow solid. MS (ESI) m/z: 339.0, 337.0 (M+1).

### Step 2: 6-(2-((Dimethylamino)methyl)phenyl)-N-(2-(4-morpholinyl)ethyl)-2-quinazolinamine

A mixture of 6-bromo-N-(2-morpholinoethyl)quinazolin-2-amine (120 mg, 0.4 mmol), 2-(N,N-dimethylaminomethyl)phenyl boronic acid (86 mg, 0.5 mmol), Na₂CO₃ (187 mg, 1.8 mmol) and PdCl₂(PPh)₂ (20 mg, 0.03 mmol) in 3.5 mL DME/1.5 mL H₂O/1.0 mL EtOH was heated to 80 °C. Additional boronic acid (80 mg) and PdCl₂(PPh)₂ (24 mg) were added. After 25 h, the mixture was cooled to room temperature and partitioned between EtOAc/H₂O. The aqueous layer was extracted with EtOAc (3X) and the combined organics were evaporated onto SiO₂ and purified by flash column chromatography eluting with 2M NH₃ in MeOH/CH₂Cl₂ (0:1 → 4:96) to give the title compound as a yellow tar. MS (ESI) m/z: 392.2(M+1).

### Example 2609

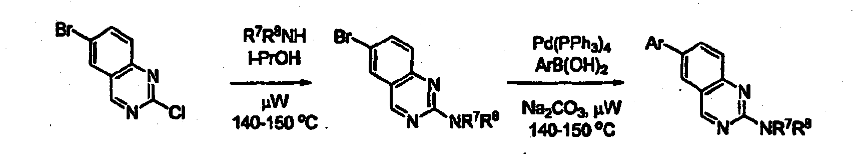

### Example 2609a (Reference Example)

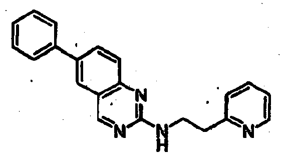

### Step 1: 6-Bromo-N-(2-(pyridin-2-yl)ethyl)quinazolin-2-amine

Three reaction vessels were charged with 6-bromo-2-chloroquinazoline (1.00 g, 4.1 mmol) and 2-(2-aminoethyl)pyridine (0.75 mL, 6.3 mmol) and 8 mL of 2-propanol. The reaction vessels were heated to 140°C for 20 min in the Smith Optimizer microwave by Personal Chemistry. The reaction mixtures were combined and the solid was filtered, washed with a minimal amount of 2-propanol and dried in vacuo to give 2.36 g (58 %) of a light yellow amorphous solid. MS (ESI, pos.ion) m/z: 329.0 (M+1).

### Step 2: 6-Phenyl-N-(2-(2-pyridinyl)ethyl)-2-quinazolinamine

A mixture of 6-bromo-N-(2-(pyridin-2-yl)ethyl)quinazolin-2-amine (163 mg, 0.5 mmol), phenyl boronic acid (88 mg, 0.7 mmol), Na₂CO₃ (272 mg, 2.6 mmol) and PdCl₂ (PPh)₂ (22 mg, 0.03 mmol) in 2.1 mL DME/0.9 mL H₂O/0.6 mL EtOH was heated to 150°C for 25 min in the Smith Optimizer microwave by Personal Chemistry. The reaction mixture was partitioned between EtOAc/brine and the aqueous layer was extracted with EtOAc (3X). The combined organics were evaporated onto SiO₂ and purified by flash column chromatography eluting with 2M NH₃ in MeOH/CH₂Cl₂ (0:1 → 3:97) to give 86 mg (53 %) of a yellow amorphous solid. MS (ESI, pos.ion) m/z: 327.1 (M+1).

### Example 2610 (Reference Example)

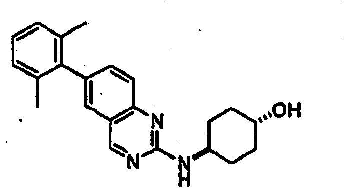

### 4-((6-(2,6-Dimethylphenyl)-2-quinazolinyl)amino)cyclohexanol

The title compound, a yellow amorphous solid, was prepared in a manner analogous to that described in Example 2609a. MS (ESI) m/z: 348.2 (M+1).

### Example 2611

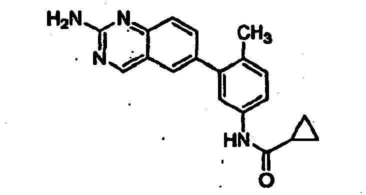

### N-(3-(2-aminoquinazolin-6-yl)-4 methylphenyl)cyclopropanecarboxamide

The title compound, a tan solid, was prepared in a manner analogous to that described in the Examples, which represent method F1. MS: (ES+): 319.1(M+H)⁺

### Example 2612

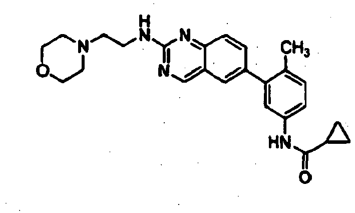

### N-(4-Methyl-3-(2-(2-morpholinoethylamino)quinazolin-6-yl)phenyl)cyclopropanecarboxamide

### Step 1. N-(4-Methyl-3-(4,4,5,5-tetramethyl-1,3,2-dioxaborolan-2-yl)phenyl)cyclopropanecarboxamide

A solution of *N*-(3-Bromo-4-methylphenyl)cyclopropanecarboxamide (456 mg, 1.8mmol) in DMF (5 ml) under nitrogen were added Pd(dppf)Cl₂ (132 mg, 0.18mmol), and bis(pinacolato)diboron (686 mg, 2.7mmol), followed by potassium acetate (883 mg, 9mmol). After stirring at 80°C for 20 h, the cooled reaction was filtered via a pad of Celite® and the filtrate was partitioned between EtOAc and water. The organic layer was dried (MgSO₄), filtered, and concentrated in vacuo. Flash chromatography eluting with a solvent gradient of 5:10:85, 10:10:80, and 15:10:75 EtOAc-CH₂Cl₂-Hexane afforded the title compound as an off-white solid. MS (ES+): 302.2 (M+H)⁺.

### Step 2. N-(4-Methyl-3-(2-(2-morpholinoethylamino)quinazolin-6-yl)phenyl)cyclopropanecarboxamide

To 6-bromo-N-(2-morpholinoethyl)quinazolin-2-amine (200 mg, 0.59 mmol) under argon were added N-(4-methyl-3-(4,4,5,5-tetramethyl-1,3,2-dioxaborolan-2-yl)phenyl)cyclopropanecarboxamide (210 mg, 0.71 mmol), Pd(OAc)₂ (6.6 mg, 0.03 mmol), tri-o-tolylphosphine (22 mg, 0.071 mmol), and DME (3 ml), followed by 2M aqueous sodium carbonate solution (0.9 ml, 1.8 mmol). The reaction was stirred at 80°C for 20 h. The cooled reaction was diluted with CH₂Cl₂ and washed with saturated aqueous NaHCO₃ and brine; dried (MgSO₄). Flash chromatography of the crude product mixture with 1%, 3%, and 5% MeOH/CH₂Cl₂ afforded the title compound as a yellow glassy foam. MS (ES+): 432.2 (M+H)⁺.

### Example 2613

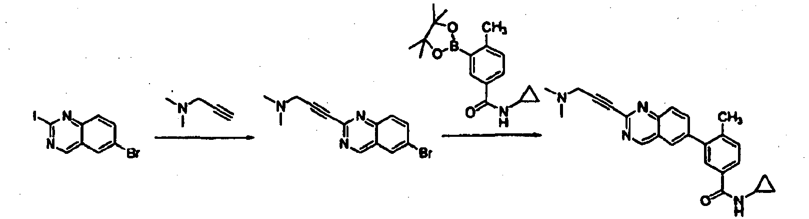

### Example 2613a

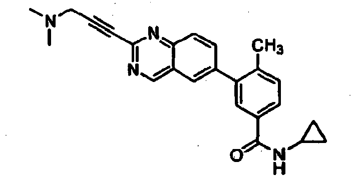

### N-Cyclopropyl-3-(2-(3-(dimethylamino)prop-1-ynyl)quinazolin-6-yl)-4-methylbenzamide

### Step 1. 3-(6-Bromoquinazolin-2-yl)-N,N-dimethylprop-2-yn-1-amine

Into a 15-ml round bottom flask under argon were charged with 6-bromo-2-iodoquinazoline (100 mg, 0.3 mmol), 1-dimethylamino-2-propyne (30 ml, 0.33 mmol), Cl₂Pd(PPh₃)₂ (21 mg, 0.03 mmol), and CuI (3 mg, 0.015 mmol), followed by Et₃N (2 ml). The resulting mixture was stirred at RT for 1 h and 60°C for 2 h. The cooled reaction was diluted with CH₂Cl₂ and washed with saturated aqueous NaHCO₃ and brine; dried (MgSO₄). Flash chromatography with a gradient of 1%, 3%, and 5% MeOH/CH₂Cl₂ afforded the title compound as a tan glass. MS (ES+): 291.0 (M+H)⁺.

### Step 2. N-Cyclopropyl-3-(2-(3-(dimethylamino)prop-1-ynyl)quinazolin-6-yl)-4-methylbenzamide

The title compound was synthesized from 3-(6-bromoquinazolin-2-yl)-N,N-dimethylprop-2-yn-1-amine by a method analogous to that described in Step 2 of Example 2612. MS (ES+): 385.2 (M+H)⁺.

### Example 2614

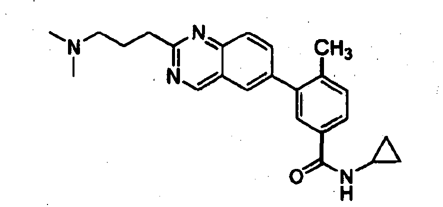

### N-Cyclopropyl-3-(2-(3-(dimethylamino)propyl)quinazolin-6-yl)-4-methylbenzamide

Into a 15-ml round bottom flask were charged with N-cyclopropyl-3-(2-(3-(dimethylamino)prop-1-ynyl)quinazolin-6-yl)-4-methylbenzamide (20.8 mg, 0.054 mmol; Example 4), 10% Pd/C (2.1 mg), and MeOH (1 ml). The mixture was hydrogenated (double-walled balloon pressure) at RT for 3 h. The mixture was filtered via a pad of Celite® and the filtrate was concentrated in vacuo and chromatographed over silica eluting with a gradient of 1%, 3%, 5%, and 10% 2M NH₃ in MeOH/CH₂Cl₂ to afford the title compound as a light yellow solid. MS (ES+): 389.2 (M+H)⁺.

### Example 2615

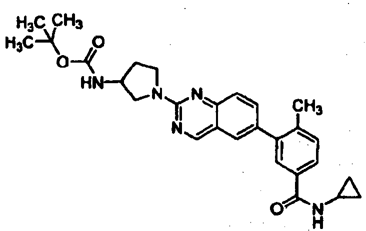

### Tert-Butyl 1-(6-(5-(cyclopropylcarbamoyl)-2-methylphenyl)quinazolin-2-yl)pyrrolidin-3-ylcarbamate

### Step 1. 3-(2-Chloroquinazolin-6-yl)-N-cyclopropyl-4-methylbenzamide

The title compound was synthesized from 6-bromo-2-chloroquinazoline and N-cyclopropyl-4-methyl-3-(4,4,5,5-tetramethyl-1,3,2-dioxaborolan-2-yl)benzamide, by a method analogous to that described in Step 2 of Example 2612. MS (ES+): 338.1 (M+H)⁺

### Step 2. tert-Butyl 1-(6-(5-(cyclopropylcarbamoyl)-2-methylphenyl)quinazolin-2-yl)pyrrolidin-3-ylcarbamate

To a solution of 3-(2-chloroquinazolin-6-yl)-N-cyclopropyl-4-methylbenzamide (50 mg, 0.15 mmol) in dichloroethane (2 ml) was added 3-(tert-butoxylcarbonylamino)pyrrolidine (42 mg, 0.23 mmol) and diisopropylethylamine (52 ml, 0.3 mmol). The resulting mixture was stirred at 80°C for 20 h. The cooled reaction was diluted with CH₂Cl₂ and washed with saturated aqueous NaHCO₃ and brine; dried (MgSO₄). Flash chromatography of the crude product mixture eluting with a gradient of 25:10:65, 40:10:50, 50:10:40, 60:10:30, and 70:10:20 EtOAc-CH₂Cl₂-Hexane afforded the title compound as a light yellow glass. MS (ES+): 488.2(M+H)⁺.

### Example 2616

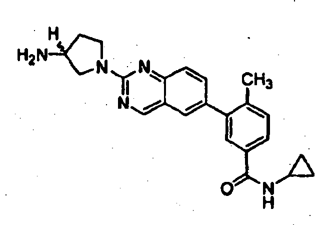

### (+/-) 3-(2-(3-aminopyrrolidin-1-yl)quinazolin-6-yl)-N-cyclopropyl-4-methylbenzamide

A solution of tert-Butyl 1-(6-(5-(cyclopropylcarbamoyl)-2-methylphenyl)quinazolin-2-yl)pyrrolidin-3-ylcarbamate (44 mg, 0.09 mmol) in 4N HCl in dioxane (2ml) was stirred at RT for 1 h. The mixture was concentrated in vacuo and the residue was partitioned between CH₂Cl₂ and saturated NaHCO₃. The aqueous layer was back-extracted with CH₂Cl₂ (2x). The organic extracts were combined, dried (MgSO₄), and chromatographed over silica gel eluting with a gradient 1%, 3%, and 5% 2M NH₃ in MeOH/CH₂Cl₂ to afford the title compound as a yellow solid. MS (ES+): 388.2 (M+H)⁺.

### Example 2617

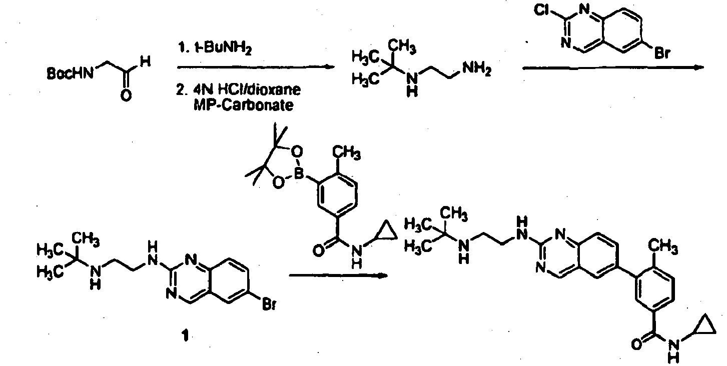

### Example 2617a

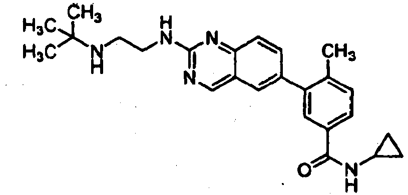

### 3-(2-(2-(tert-Butylamino)ethylamino)quinazolin-6-yl)-N-cyclopropyl-4-methylbenzamide

### Step 1. N¹-tert-butylethane-1,2-diamine

To a solution of tert-butyl N-(2-oxoethyl)carbamate (500 mg, 3.1 mmol) in CHCl₃ (15 ml) was added tert-butyl amine (0.5 ml, 4.7 mmol) and sodium triacetoxyborohydride (1.9 g, 9 mmol). The mixture was stirred at 70°C for 3h. The cooled reaction was diluted with CH₂Cl₂ and washed with saturated aqueous NaHCO₃ and brine; dried (MgSO₄) and concentrated in vacuo to give a colorless foam. To the Boc-protected intermediate was added 4N HCl in dioxane (5 ml) and a few drops of MeOH. After stirring the mixture at RT for 2 h, the reaction was concentrated in vacuo to give the title compound as a dihydrochloride salt. A solution of the dihydrochloride salt in MeOH (5 ml) was added MP-Carbonate (3.2 g, 9.3 mmol, 2.9 mmol/g) and gently stirred at RT for 20 h. The resin was filtered off and the filtrate was concentrated in vacuo and dried under high vacuum to afford the title compound as a free base. MS (ES+): 117.4 (M+H)⁺.

### Step 2. 6-Bromo-N-(2-(tert-butylamino)ethyl)quinazolin-2-amine

The title compound was synthesized from 6-bromo-2-iodoquinazoline and N¹-tert-butylethane-1,2-diamine by a method analogous to that described in step 2 of Example 2615. MS (ES+): 324.1(M+H)⁺

### Step 3. 3-(2-(2-(tert-Butylamino)ethylamino)quinazolin-6-yl)-N-cyclopropyl-4-methylbenzamide

The title compound was synthesized from 6-bromo-N-(2-(tert-butylamino)ethyl)quinazolin-2-amine and N-cyclopropyl-4-methyl-3-(4,4,5,5-tetramethyl-1,3,2-dioxaborolan-2-yl)benzamide by a method analogous to that described in by using the method described in Step 2 of Example 2612. MS (ES+): 418.3 (M+H)⁺.

### Example 2618

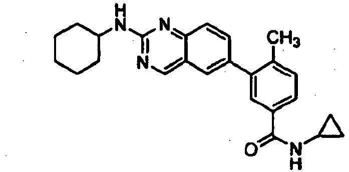

### 3-(2-(Cyclohexylamino)quinazolin-6-yl)-N-cyclopropyl-4-methylbenzamide

3-(2-Chloroquinazolin-6-yl)-N-cyclopropyl-4-methylbenzamide and cyclohexylamine (1 ml) were combined and heated at 100°C for 1 h. A tan solid precipitated out from the reaction mixture upon cooling. The tan solid was washed with water and triturated with toluene to afford the title compound as a light yellow solid. MS (ES+): 401.2 (M+H)⁺.

### Example 2619

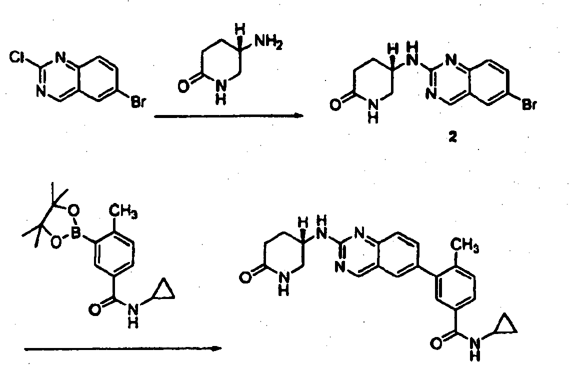

### Example 2619a

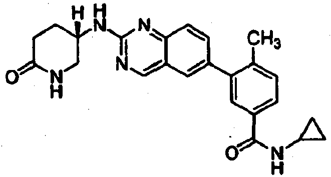

### N-Cyclopropyl-4-methyl-3-(2-((S)-6-oxopiperidin-3-ylamino)quinazolin-6-yl)benzamide

### Step 1:(S)-5-(6-Bromoquinazolin-2-ylamino)piperidin-2-one

A solution of 4-(S)-amino-□-valerolactam hydrochloride salt (1 g, 6.6mmol) in MeOH (5 ml) was added MP-Carbonate (4.6 g, 13.2mmol) and gently stirred at RT for 18h. The resin was filtered off and the filtrate was concentrated in vacuo to give the free base of 4-(S)-amino-□-valerolactam as a light brown gum. To the crude free base in NMP (10 ml) were added 6-bromo-2-chloroquinazoline (730 mg, 3mmol) and K₂CO₃ (910 mg, 6.6mmol)). The mixture was stirred at 80 °C for 3 h. The cooled reaction was diluted with water and stirred for 1 h. The maize solid formed was filtered, washed with a copious amount of water, and triturated with MeOH to afford the title compound. MS (ES+): 322.1 (M+H)⁺.

### Step 2. N-Cyclopropyl-4-methyl-3-(2-((S)-6-oxopiperidin-3-ylamino)quinazolin-6-yl)benzamide

The title compound was synthesized from (S)-5-(6-bromoquinazolin-2-ylamino)piperidin-2-one and N-cyclopropyl-4-methyl-3-(4,4,5,5-tetramethyl-1,3,2-dioxaborolan-2-yl)benzamide by a method analogous to that described in Step 2 of Example 2612. MS (ES+): 416.2 (M+H)⁺.

### Example 2620

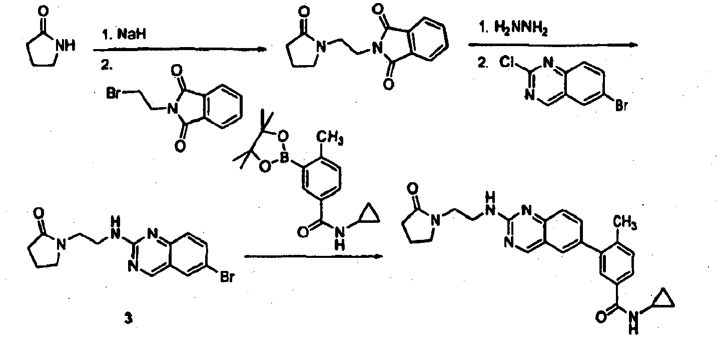

### Example 2620a

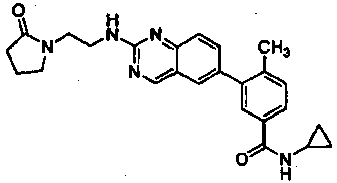

### N-Cyclopropyl-4-methyl-3-(2-(2-(2-oxopyrrolidin-1-yl)ethylamino)quinazolin-6-yl)benzamide

### Step 1: 2-(2-(2-Oxopyrrolidin-1-yl)ethyl)isoindoline-1,3-dione

A solution of 2-pyrrolidinone (5g, 4.5ml) in DMF (100ml) under argon was added NaH (2.7 g, 70.44mmol) and stirred at 60°C for 1 h. A solution of N-(2-bromoethyl)phthalimide (30 g, 117.4 mmol) in DMF (20 ml) was added to the mixture, and the reaction was stirred at 100°C for 20 h. The cooled reaction was diluted with EtOAc and washed with water and dried over (MgSO₄). Flash chromatography of the resulting crude oil with a solvent gradient of 40:10:50, 50:10:40, 60:10:30, and 70:10:20 EtOAc-CH₂Cl₂-Hexane afforded the title compound as a golden yellow oil. MS (ES+) : 259.1 (M+H)⁺.

### Step 2. 1-(2-(6-Bromoquinazolin-2-ylamino)ethyl)pyrrolidin-2-one

A solution of 2-(2-(2-oxopyrrolidin-1-yl)ethyl)isoindoline-1,3-dione (347mg, 1.3 mmol) in EtOH (7ml) was added hydrazine (82 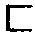 l, 2.6 mmol) and heated at 80°C for 2 h. The white precipitate was filtered off and discarded. The filtrate was concentrated in vacuo to afford a crude crop of the amine intermediate. A solution of the crude amine in NMP (2 ml) was next treated with 6-bromo-2-chloroquinazoline (101 mg, 0.42 mmol) and K₂CO₃ (120 mg, 0.83 mmol) and heated at 80°C for 3 h. The cooled reaction was diluted with EtOAc and washed with saturated aqueous NaHCO₃ and brine; dried (MgSO₄). Flash chromatography eluting with a solvent gradient of 1%, 3%, and 5% MeOH/CH₂Cl₂ afforded the title compound as a maize solid. MS (ES+): 336.1(M+H)⁺.

### Step 3. N-Cyclopropyl-4-methyl-3-(2-(2-(2-oxopyrrolidin-1-yl)ethylamino)quinazolin-6-yl)benzamide

The title compound was synthesized from 1-(2-(6-bromoquinazolin-2-ylamino)ethyl)pyrrolidin-2-one and N-cyclopropyl-4-methyl-3-(4,4,5,5-tetramethyl-1,3,2-dioxaborolan-2-yl)benzamide by a method analogous to that described in Step 2 of Example 2612. MS (ES+): 430.2 (M+H)⁺.

### Example 2621

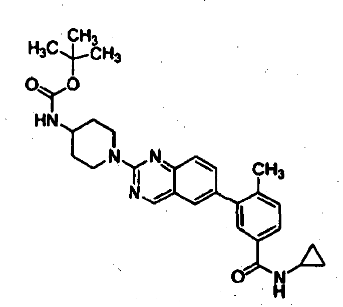

### Tert-Butyl 1-(6-(5-(cyclopropylcarbamoyl)-2-methylphenyl)quinazolin-2-yl)piperidin-4-ylcarbamate

The title compound, a light yellow solid, was synthesized by a method analogous to that described in Example 2615. MS (ES+): 502.3 (M+H)⁺

### Example 2622

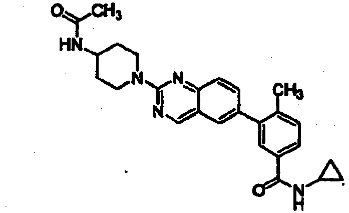

### 3-(2-(4-Acetamidopiperidin-1-yl)quinazolin-6-yl)-N-cyclopropyl-4-methylbenzamide

To a solution of 3-(2-(4-aminopiperidin-1-yl)quinazolin-6-yl)-N-cyclopropyl-4-methylbenzamide (55.00 mg, 137 µmol) in CH₂Cl₂ (1 ml) was added acetyl anhydride (65 µl, 0.68 mmol) and 4-(dimethylamino)pyridine (17 mg, 137 µmol). The resulting golden yellow mixture was stirred at RT for 3 h. The reaction was diluted with CH₂Cl₂ and washed with NaHCO₃ and dried over MgSO₄. The resulting crude oil was purified by prep HPLC to afford the title compound as a yellow glassy solid. MS (ES+): 444.2(M+H)⁺.

### Example 2623 (Reference Example)

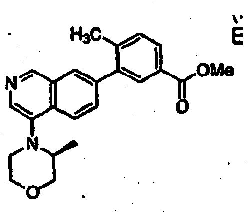

### Methyl 4-methyl-3-(4-((S)-3-methylmorpholino)isoquinolin-7-yl)benzoate

### Step 1: Methyl 4-methyl-3-(4,4,5,5-tetramethyl-1,3,2-dioxaborolan-2-yl)benzoate

To a solution of 4-methyl-3-(4,4,5,5-tetramethyl-1,3,2-dioxaborolan-2-yl)benzoic acid (400 mg, 1.5 mmol) in MeOH (1.23 ml, 3.05 mmol) was added three drops of sulfuric acid (12.9 µl, 0.15 mmol) and the mixture was stirred at 60°C for 20 h. The cooled reaction was diluted with CH₂Cl₂ and washed with saturated aqueous NaHCO₃ and water and dried over MgSO₄. Concentration and trituration of the resulting crude solid with MeOH afforded the title compound as an off-white solid. MS (ES+): 277.1(M+H)⁺.

### Step 2. Methyl 3-(4-bromoisoquinolin-7-yl)-4-methylbenzoate

Into a 100-ml round bottom flask under argon was added 4,7-dibromoisoquinoline (320 mg, 1.11 mmol), methyl 4-methyl-3-(4,4,5,5-tetramethyl-1,3,2-dioxaborolan-2-yl)benzoate (308 mg, 1.11 mmol), Pd(PPh₃)₄ (64 mg, 0.055 mmol), EtOH (2 ml), and 1,2-dimethoxyethane (8 ml), followed by a 2M K₂CO₃ aqueous solution (1.67 ml, 3.34 mmol). The reaction was heated at 90°C for 2 h. The cooled reaction was diluted with CH₂Cl₂ and washed with saturated aqueous NaHCO₃ and brine, then dried over MgSO₄ and concentrated in vacuo. CombiFlash purification of the crude mmaterial (10% to 25% EtOAc/Hexane) afforded the title compound as a yellow solid. MS (ES+): 357.0(M+H)⁺.

### Step 3. Methyl 4-methyl-3-(4-((S)-3-methylmorpholino)isoquinolin-7-yl)benzoate

A 50-ml round bottom flask under argon was charged with methyl 3-(4-bromoisoquinolin-7-yl)-4-methylbenzoate (75 mg, 0.21 mmol), (S)-3-methylmorpholine (43 mg, 0.42 mmol), palladium acetate (4.7 mg, 0.021 mmol), X-PHOS (40 mg, 0.084 mmol), and toluene (1 ml), followed by cesium carbonate (206 mg, 0.63 mmol). The reaction was stirred at 100°C for 20 h. The cooled reaction was diluted with CH₂Cl₂ and washed with saturated aqueous NaHCO₃, brine and dried over MgSO₄. The solution was filtered and concentrated in vacuo. CombiFlash purification (20% to 80% EtOAc/Hexane) afforded the title compound as a tan solid. MS (ES+): 377.2(M+H)⁺.

### Example 2624

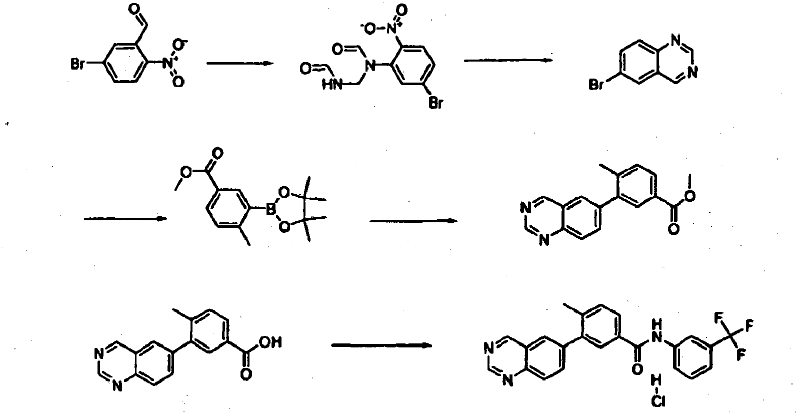

### 4-Methyl-3-(quinazolin-6-yl)-N-(3-(trifluoromethyl)phenyl)benzamide hydrochloride

### Step 1: 5-bromo-2-nitrobenzaldehyde

3-Bromobenzaldehyde (100 g, 540 mmol) was added dropwise over 20 min to a vigorously stirred mixture of nitric acid (54 mL, 540 mmol) and sulfuric acid (650 mL, 12194 mmol) cooled in an ice bath. When addition was complete, the cooling bath was removed and the orange solution was stirred at ambient temperature for 3 h. The solution was poured onto 3L of crushed ice, and the resulting pale precipitate was collected by filtration, washing with water. The solid was dissolved in EtOAc (500mL), residual water was separated off, and the solution was dried (Na₂SO₄). The solution was concentrated to give a pale yellow solid, which was recrystallized from hexane (350mL) to give 5-bromo-2-nitrobenzaldehyde as pale yellow needles.

### Step 2: N-[(5-Bromo-2-nitro-phenyl)-formylamino-methyl]-formamide

5-Bromo-2-nitrobenzaldehyde (62.54 g, 272 mmol) was suspended in formamide (100mL, 2518mmol) and heated at 80°C. Most of the starting material dissolved upon heating. Anhydrous HCl (generated by dropwise addition of conc. sulfuric acid to NaCl) was gently passed over the stirred reaction mixture. After 1 h, the reaction mixture had solidified. The reaction mixture was cooled in an ice bath and ethanol (100 mL) was added. The solid was broken up and allowed to stand for 30 min before filtering off and washing with a small amount of ice-cold ethanol to give 94g of crude product as a pale yellow solid. This was suspended in acetonitrile (300mL) and heated to boiling for 5 min before cooling in an ice bath. The product was filtered off as a fine white crystalline solid.

### Step 3: 6-Bromoquinazoline

N-[(5-Bromo-2-nitro-phenyl)-formylamino-methyl]-formamide (49.17 g, 163mmol) and zinc dust (140 g, 2141mmol) were intimately mixed and added to crushed ice (540 g) in a 2L beaker (effervescence from reaction will nearly fill beaker). The mixture was stirred with a mechanical stirrer, and acetic acid (210mL, 3668mmol) was added slowly over 10 min. The mixture was stirred for 3h, after which time effervescence had subsided, and the mixture was filtered. The filtrate was treated with 10N NaOH (60mL) and extracted with Et₂O (3 x 400mL). The combined ether extracts were filtered (considerable ppt rendered partition difficult), and remaining water separated off. The ether extracts were dried (MgSO₄) and filtered. Concentration in vacuo gave a yellow solid (18.3g) which was recrystallized from acetonitrile (100mL) to give 6-bromoquinazoline as a tan crystalline solid. MS: Found (M)⁺, (M+2)⁺ of 209, 211, respectively.

### Step 4: Methyl 4-methyl-3-(4,4,5,5-tetramethyl-1,3,2-dioxaborolan-2-yl)benzoate

4,4,5,5-Tetramethyl-2-(4,4,5,5-tetramethyl-1,3,2-dioxaborolan-2-yl)-1,3,2-dioxaborolane (1.62 g, 6.37 mmol), potassium acetate (1.88 g, 19.1 mmol) and PdCl₂ (dppf) (0.260 g, 0.319 mmol) were added to a 50mL flask and flushed with N₂. Dioxane (20mL) was added and then methyl 3-bromo-4-methylbenzoate (1.00 ml, 6.37 mmol) was added to the stirred slurry. The resulting orange suspension was heated at 90°C. After 1 h, LC-MS indicated -50% conversion. The mixture was heated for a total of 16 h, after which time LC-MS indicated almost complete conversion. The mixture was cooled, filtered washing with THF, and concentrated to give a dark residue. This was dissolved in DCM and quickly passed through a plug of silica gel, washing with 33% EtOAc/hexane to give a pale green solution. Concentration and recrystallization from hexane gave methyl 4-methyl-3-(4,4,5,5-tetramethyl-1,3,2-dioxaborolan-2-yl)benzoate as green-tinged crystals. MS: Found 277 (M+H)⁺

### Step 5: Methyl 4-methyl-3-(quinazolin-6-yl)benzoate

6-Bromoquinazoline (0.100 g, 0.478 mmol), methyl 4-methyl-3-(4,4,5,5-tetramethyl-1,3,2-dioxaborolan-2-yl)benzoate (0.159 g, 0.574 mmol), sodium carbonate monohydrate (0.356 g, 2.87 mmol) and PdCl₂(PPh₃)₂ (0.0168 g, 0.0239 mmol) were added to a 5 mL microwave tube flushed with N2, capped, and DMF (2.0 mL) and water (0.2 mL) were added. The vial was heated at 150°C in a microwave for about 16-18 min and cooled. The resulting mixture contained a fine black suspension and some solid material at the bottom of the tube. The mixture was filtered, washing with a small amount of DMF, diluted with EtOAC and washed with water. The organic layer was concentrated and purified by flash chromatography (50% EtOAc/ hexane) to give methyl 4-methyl-3-(quinazolin-6-yl)benzoate as a white solid. MS: Found 279 (M+H)⁺

### Step 6: 4-Methyl-3-(quinazolin-6-yl)benzoic acid

Methyl 4-methyl-3-(quinazolin-6-yl)benzoate (0.1265 g, 0.4545 mmol) was dissolved in THF (3 mL) and treated with lithium hydroxide hydrate (0.05722 g, 1.364 mmol). Water (1 mL) was added and the mixture stirred at RT. After 16 h, LC-MS indicated -90% conversion in a very clean reaction. The mixture was heated at 55 °C for a further 4 h, after which time LC-MS indicated complete conversion. The solution was neutralized (0.68 mL of 2 N HCl). Concentration to dryness then gave a white paste, which was used without purification. MS: Found 265 (M+H)⁺

### Step 7: 4-Methyl-3-(quinazolin-6-yl)-N-(3-(trifluoromethyl)phenyl)benzamide hydrochloride

4-Methyl-3-(quinazolin-6-yl)benzoic acid (120 mg, 0.4545 mmol) and 3-(trifluoromethyl)aniline (62.4 µl, 0.500 mmol) were dissolved in THF (3 mL) and the stirred soltuion was treated with HATU (207 mg, 0.545 mmol) resulting in a suspension. DMF (3 mL) was added to aid solubility, although there was still a suspension. The mixture was stirred for 1 h, after which time LC-MS indicated incomplete reaction. Additional trifluoromethylaniline (100 uL), triethylamine (100 uL) and HATU (200 mg) were added, and the mixture heated at 50 °C for 2 h, after which time LC-MS indicated completion. The mixture was diluted with ethyl acetate and washed with 2 N NaOH. The organic layer was dried (Na2SO4) and purified by flash chromatography (50% EtOAc / hexane) to give the product as a pale yellow oil. The sample failed to crystallize, and so was dissolved in 2 M HCl (4 mL) and lyophilized yielding 4-methyl-3-(quinazolin-6-yl)-N-(3-(trifluoromethyl)phenyl)benzamide hydrochloride as a tan solid. MS: Found 408 (M+H)⁺

### Example 2625

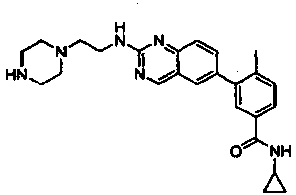

### N-cyclopropyl-4-methyl-3-(2-(2-(piperazin-1-yl)ethylamino)quinazolin-6-yl)benzamide

Into a 10 ml round bottom flask was added tert-butyl 4-(2-(6-(5-(cyclopropylcarbamoyl)-2-methylphenyl)quinazolin-2-ylamino)ethyl)piperazine-1-carboxylate (80mg, 0.15mmol) [prepared according to Method B] and MeOH (1mL), and 4.0M HCl in 1,4-dioxane (0.375mL, 1.5mmol) was added at 0°C. The mixture was brought to room temperature for 2h. Sodium bicarbonate (sat.) was added and the mixture was extracted with dichloromethane. The organic layers were combined, dried over sodium sulfate, filtered and concentrated. The crude residue was purified on ISCO using 1-2% (2M NH₃ in MeOH) in dichloromethane to give N-cyclopropyl-4-methyl-3-(2-(2-(piperazin-1-yl)ethylamino)quinazolin-6-yl)benzamide as a solid. MS(ES+): 431.2(M+H)

### Example 2626

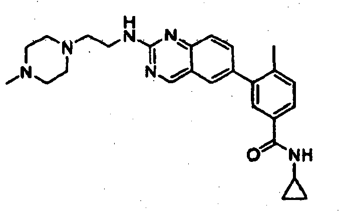

### N-cyclopropyl-4-methyl-3-(2-(2-(4-methylpiperazin-1-yl)ethylamino)quinazolin-6-yl)benzamide

Into a 10ml round-bottom flask equipped with stir bar was added N-cyclopropyl-4-methyl-3-(2-(2-(piperazin-1-yl)ethylamino)quinazolin-6-yl)benzamide, (200mg, 0.46mmol), 1:1 CHCl₃:MeOH (2ml), sodium triacetoxyborohydride (276mg. 1.2mmol), and formaldehyde 37% in H₂O (0.138g, 4.6mmol). Reaction stirred at RT for 16 hr. Sodium bicarbonate (sat.) was added and the mixture was extracted with dichloromethane. The organic layers were combined, dried over sodium sulfate, filtered and concentrated. Crude residue was purified on ISCO using 0-5% (2M NH₃ in MeOH) in dichloromethane to give N-cyclopropyl-4-methyl-3-(2-(2-(4-methylpiperazin-1-yl)ethylamino)quinazolin-6-yl)benzamide as a light yellow solid. MS(ES+): 445.2(M+H)

### Example 2627

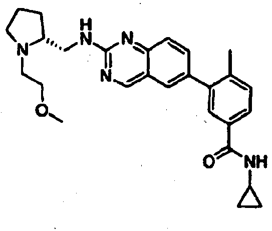

### N-cyclopropyl-3-(2-(((R)-1-(2-methoxyethyl)pyrrolidin-2-yl)methylamino)quinazolin-6-yl)-4-methylbenzamide

Into a 10 ml round bottom flask equipped with stir bar and condenser with N₂ inlet was added N-cyclopropyl-4-methyl-3-(2-((R)-pyrrolidin-2-ylmethylamino)quinazolin-6-yl)benzamide (50mg, 0.125mmol), chloroform (1.2ml 0.4M), Diisopropylamine (32uL, 0.187mmol), and 1-bromo-2-methoxyethane (18uL, 0.187mmol). Reaction was run at 60°C under N₂ for 16hr. The reaction was cooled to room temperature and sodium bicarbonate (sat.) was added and the mixture was extracted with dichloromethane. The organic layers were combined, dried over sodium sulfate, filtered and concentrated. The crude residue was purified on ISCO using 1-4% (2M NH₃ in Methanol) in dichloromethane to give N-cyclopropyl-3-(2-(((R)-1-(2-methoxyethyl)pyrrolidin-2-yl)methylamino)quinazolin-6-yl)-4-methylbenzamide as a solid. MS(ES+): 460.2 (M+H).

### Example 2628

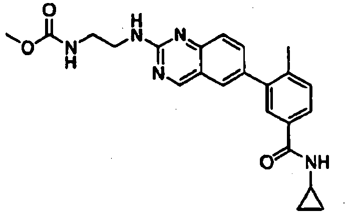

### Methyl 2-(6-(5-(cyclopropylcarbamoyl)-2-methylphenyl)quinazolin-2-ylamino)ethylcarbamate

Into a 25 ml round bottom flask equipped with stir bar was added 3-(2-((1S,2S)-2-aminocyclohexylamino)quinazolin-6-yl)-N-cyclopropyl-4-methylbenzamide (50 mg, 120 µmol), [prepared according to the method of Example 2625], N-ethyl-N-isopropylpropan-2-amine (42 µl, 241 µmol) and Dichloromethane. Reaction was cooled to -78°C in dry ice acetone bath and methylcarbonochloridate (23 µl, 241 µmol) was added one drop/sec. Reaction was complete in about 10sec. The reaction was poured into sodium bicarbonate (sat.) and extracted with dichloromethane 3X. The organic layers were combined, dried over sodium sulfate, filtered and concentrated. The product was purified by reverse phase HPLC, along with ISCO using gradient of 100% dichloromethane to 10% 4 molar ammonium chloride in Methanol. Methyl 2-(6-(5-(cyclopropylcarbamoyl)-2-methylphenyl)quinazolin-2-ylamino)ethylcarbamate was obtained as a solid. MS(ES+): 420.2 (M+H).

### Example 2629 (Reference Example)

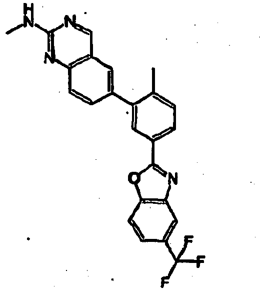

### N-methyl-6-(2-methyl-5-(5-(trifluoromethyl)-1,3-benzoxazol-2-yl)phenyl)-2-quinazolinamine

N-(2-fluoro-5-(trifluoromethyl)phenyl)-4-methyl-3-(2-(methylamino)quinazolin-6-yl)benzamide (100mg, 0.22mmol) was added to a suspension of NaH (10mg, 0.33mmol) in DMF (2mL). The reaction mixture was heated to 100°C and stirred at that temperature for 4 days. HPLC purification afforded the title compound as a white solid. MS (ESI) m/z: 435.1 [M+1].

### Example 2630

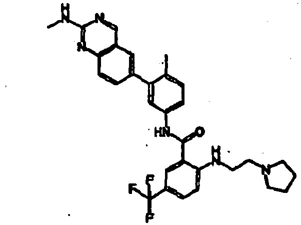

### N-(4-methyl-3-(2-(methylamino)-6-quinazolinyl)phenyl)-2-((2-(1-pyrrolidinyl)ethyl)amino)-5-(trifluoromethyl)benzamide

2-(pyrrolidin-1-yl)ethanamine (0.02 mL, 198 µmol) and K₂CO₃ (46 mg, 330 µmol) were added to a solution of 2-fluoro-N-(4-methyl-3-(2-(methylamino)quinazolin-6-yl)phenyl)-5-(trifluoromethyl)benzamide (75.00mg, 165 µmol) in DMF (3mL). The reaction mixture was heated to 50°C for 1 hour, at which time it was cooled to RT, diluted with water and extracted with CH₂Cl₂. The combined organic fractions were washed with water and brine, dried over sodium sulfate and concentrated to give crude material. The crude material was purified by MPLC: Isco, Redi-Sep® pre-packed silica gel column (40 g) using eluent gradient: 3-80% 90/10/1 CH2Cl2 in CH2Cl2 over 20 min to afford the title compound as a yellow solid. MS (ESI) m/z: 549.3 [M+1].

### Example 2631 (Reference Example)

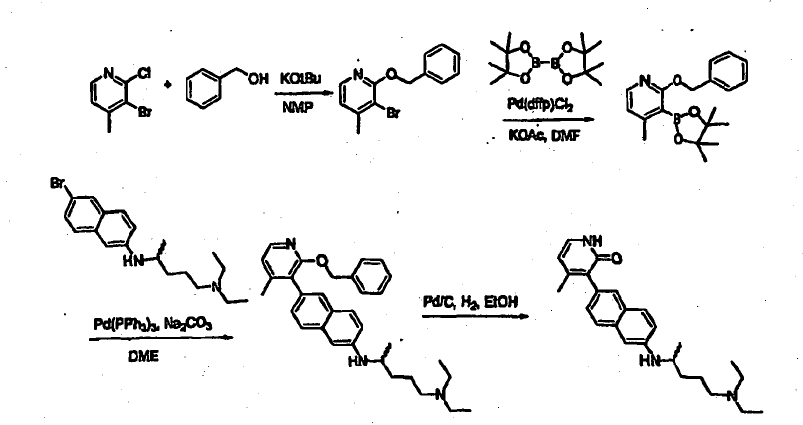

### 3-(2-(5-(diethylamino)pentan-2-ylamino)quinazolin-6-yl)-4-methylpyridin-2(1H)-one

### Step 1: 2-(benzyloxy)-3-bromo-4-methylpyridine

A mixture of 3-bromo-2-chloro-4-picoline (0.500 g, 2.42 mmol, Asymchem Laboratories, Inc.), benzyl alcohol (1.26 ml, 12.1 mmol, Aldrich) and potassium tert-butoxide (1.49 g, 13.3 mmol, Aldrich) in 4.0 ml of NMP in a microwave tube was heated in a Personal Chemistry microwave at 100°C for 7 min. The mixture was diluted with EtOAc (50 mL) and washed with saturated aqueous solution of sodium bicarbonate, and brine. The resulting organic solution was then dried over magnesium sulfate and concentrated under reduced pressure. Purification of the crude material by flash chromatography on SiO₂ (EtOAc:hexane=20:80) gave 2-(benzyloxy)-3-bromo-4-methylpyridine (0.665 g, 2.39 mmol, 98.7% yield) as a colorless oil. MS (ESI, pos.ion) m/z: 279.6

### Step 2: 2-(benzyloxy)-4-methyl-3-(4,4,5,5-tetramethyl-1,3,2-dioxaborolan-2-yl)pyridine

To a mixture of 2-(benzyloxy)-3-bromo-4-methylpyridine (0.556 g, 2.0 mmol), 4,4,5,5-tetramethyl-2-(4,4,5,5-tetramethyl-1,3,2-dioxaborolan-2-yl)-1,3,2-dioxaborolane (0.76 g, 3.0 mmol, Aldrich) and potassium acetate (0.98 g, 10.0 mmol, J.T. Baker-A Division of Mallinckrodt Baker, Inc.) in DMF (15ml) was bubbled with N₂ gas for 20 minutes. It was then treated with 1,1'-bis(diphenylphosphino)ferrocene-palladium dichloride (0.15 g, 0.20 mmol, Strem Chemicals, Inc.). The reaction mixture was stirred at 85°C under N₂ for 24 h. The DMF was then removed in vacuo. The residue was diluted with 50 ml of EtOAc, 40 ml of 1N aqueous HCl and filtered through Celite pad. The organic phase was separated and aqueous phase was extracted with EtOAc (2 X 30 ml). The combined organic phases were washed with saturated aqueous solution of sodium bicarbonate, and brine. The resulting organic solution was then dried over magnesium sulfate and concentrated under reduced pressure to give 2-(benzyloxy)-4-methyl-3-(4,4,5,5-tetramethyl-1,3,2-dioxaborolan-2-yl)pyridine (0.422 g, 0.84 mmol) as a green solid. MS: (ESI) m/z: 326

### Step 3: 6-(2-(benzyloxy)-4-methylpyridin-3-yl)-N-(5-(diethylamino)pentan-2-yl)quinazolin-2-amine

A mixture of 2-(benzyloxy)-4-methyl-3-(4,4,5,5-tetramethyl-1,3,2-dioxaborolan-2-yl)pyridine (0.38 g, 0.66 mmol), 6-bromo-N-(5-(diethylamino)pentan-2-yl)quinazolin-2-amine (0.200 g, 0.55 mmol), tetrakis(triphenylphosphine) palladium(0) (0.063 g, 0.055 mmol, Strem Chemicals, Inc.) in 2.0 ml of DME and Sodium carbonate (2.0 ml, 2.0 mmol) in a microwave tube was heated in a Personal Chemistry microwave at 130°C for 20 min. The mixture was diluted with EtOAc (50mL) and washed with saturated aqueous solution of sodium bicarbonate and brine. The resulting organic solution was then dried over magnesium sulfate and concentrated under reduced pressure. Purification of the crude material by flash chromatography on SiO₂ (2 M NH₃ in MeOH:EtOAc= 20:80) gave 6-(2-(benzyloxy)-4-methylpyridin-3-yl)-N-(5-(diethylamino)pentan-2-yl)quinazolin-2-amine (0.277 g, 0.46 mmol) as an yellow solid. MS:(ESI) m/z: 484

### Step 4: 3-(2-(5-(diethylamino)pentan-2-ylamino)quinazolin-6-yl)-4-methylpyridin-2(1H)-one

To a stirred solution of 6-(2-(benzyloxy)-4-methylpyridin-3-yl)-N-(5-(diethylamino)pentan-2-yl)quinazolin-2-amine (0.212 g, 0.44 mmol) in ethanol (10ml) was added palladium, 10wt.% (dry basis) on activated carbon (0.047 g, 0.044 mmol, Aldrich) and the reaction mixture was stirred under an H₂ atmosphere using a balloon. After stirring for 16 h, the reaction mixture was filtered through a pad of Celite, which was then rinsed with 50 mL of EtOH. The filtrate was concentrated in vacuo. Purification was effected by Prep-HPLC to give 3-(2-(5-(diethylamino)pentan-2-ylamino)quinazolin-6-yl)-4-methylpyridin-2(1H)-one (0.134 g, 0.34 mmol) as an yellow solid. MS (ESI) m/z: 394.2

### Example 2632 (Reference Example)

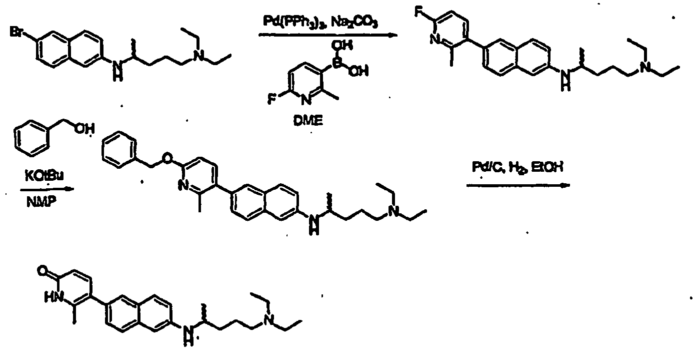

### 6-(6-(benzyloxy)-2-methylpyridin-3-yl)-N-(5-(diethplamino)pentan-2-yl)quinazolin-2-amine

### Step 1: N-(5-(diethylamino)pentan-2-yl)-6-(6-fluoro-2-methylpyridin-3-yl)quinazolin-2-amine

A mixture of 6-fluoro-2-methylpyridin-3-ylboronic acid (0.1 g, 0.7 mmol, Asymchem Laboratories, Inc), 6-bromo-N-(5-(diethylamino)pentan-2-yl)quinazolin-2-amine (0.200 g, 0.5 mmol, Pettus example 1) and Tetrakis(triphenylphosphine) Palladium (0) (0.06 g, 0.05 mmol, Strem) in 2.0 ml of DME and sodium carbonate (2 ml, 2 mmol) in a microwave tube was heated in a Personal Chemistry microwave at 130°C for 20 min. The mixture was diluted with EtOAc (50 mL) and washed with saturated aqueous solution of sodium bicarbonate, and brine. The resulting organic solution was then dried over magnesium sulfate and concentrated under reduced pressure. Purification was effected by flash chromatography on SiO₂ (2 M NH₃ in MeOH:EtOAc=10:90) to give N-(5-(diethylamino)pentan-2-yl)-6-(6-fluoro-2-methylpyridin-3-yl)quinazolin-2-amine (0.115 g, 0.3 mmol) as an yellow solid. MS: (ESI) m/z: 396.4.

### Step 2: 6-(6-(benzyloxy)-2-methylpyridin-3-yl)-N-(5-(diethylamino)pentan-2-yl)quinazolin-2-amine

A mixture of N-(5-(diethylamino)pentan-2-yl)-6-(6-fluoro-2-methylpyridin-3-yl)quinazolin-2-amine (0.400 g, 1.01 mmol), phenylmethanol (0.419 ml, 4.05 mmol, Aldrich) and potassium tert-butoxide (0.499 g, 4.45 mmol, Aldrich) in 4.0 ml NMP in a microwave tube was heated in a Personal Chemistry microwave at 100°C for 2 min. The mixture was diluted with EtOAc (50 mL) and washed with saturated aqueous solution of sodium bicarbonate, and brine. The resulting organic solution was then dried over magnesium sulfate and concentrated under reduced pressure. Purification was effected by flash chromatography on SiO₂ (2 M NH₃ in MeOH:EtOAc=20:80) to give 6-(6-(benzyloxy)-2-methylpyridin-3-yl)-N-(5-(diethylamino)pentan-2-yl)quinazolin-2-amine (0.387 g, 0.800 mmol) as an yellow solid. MS: (ESI) m/z: 485.

### Step 3: 6-(6-(benzyloxy)-2-methylpyridin-3-yl)-N-(5-(diethylamino)pentan-2-yl)quinazolin-2-amine

To a stirred solution of 6-(6-(benzyloxy)-2-methylpyridin-3-yl)-N-(5-(diethylamino)pentan-2-yl)quinazolin-2-amine (0.387 g, 0.80 mmol) in ethanol (20 ml) was added Palladium 10% on carbon (0.085 g, 0.080 mmol, Strem) and the reaction mixture was stirred under an H₂ atmosphere using a balloon. After stirring for 16 h, the reaction mixture was filtered through a pad of Celite, which was then rinsed with 50 mL of EtOH. The filtrate was concentrated in vacuo. Purification was effected Prep-HPLC to give 5-(2-(5-(diethylamino)pentan-2-ylamino)quinazolin-6-yl)-6-methylpyridin-2 (1H)-one (0.223 g, 0.57 mmol) as a light yellow solid. MS (ESI) m/z: 394.6

The following Examples 2633-2713 represent various intermediate building blocks, which were synthesized in order to prepare compounds of Formula II of the present invention. Such intermediates are merely exemplary, and as such, should not be construed to limit the scope of the present invention in any way.

### Examples 2633-2644

The following compounds were prepared in a manner analogous to one or more method steps described in Example 587.

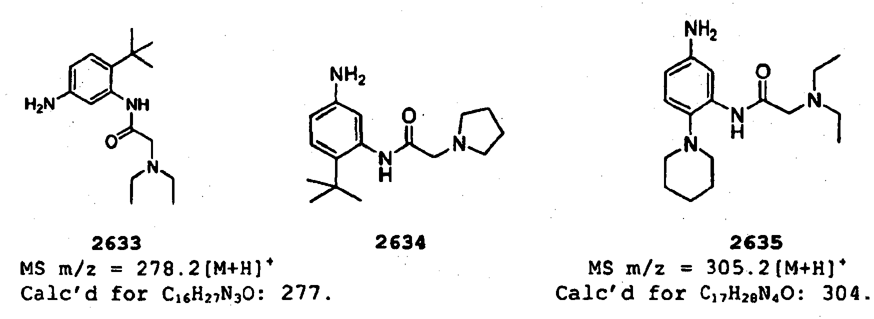

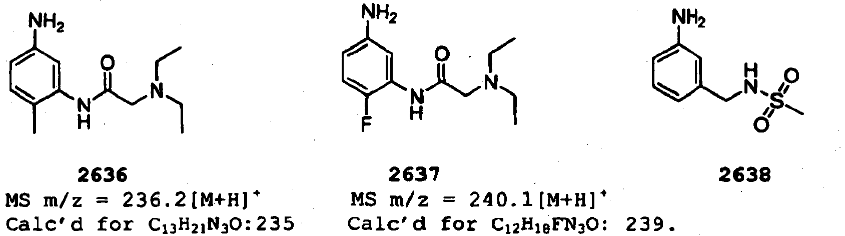

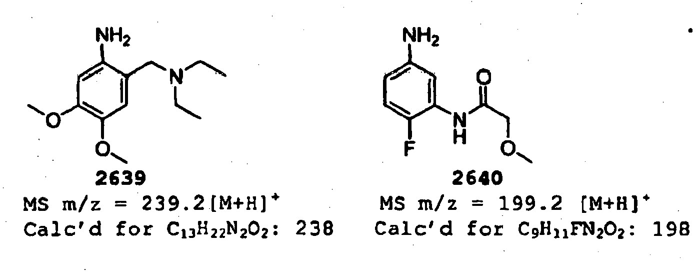

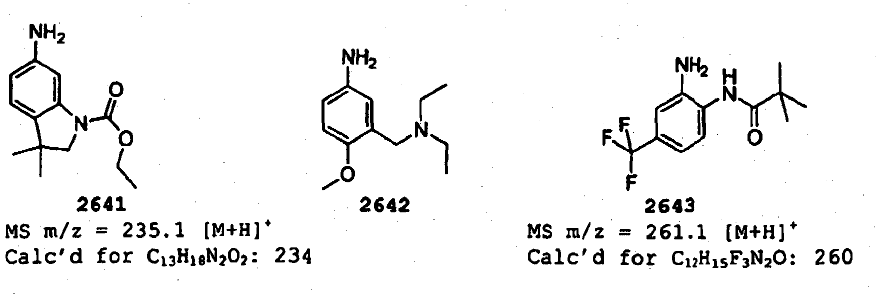

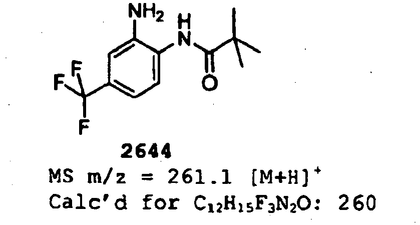

The following compound intermediates, **Examples 2645** and **2646**, were prepared by a method analogous to that described in PCT Patent Publication No. WO 2005/009978.

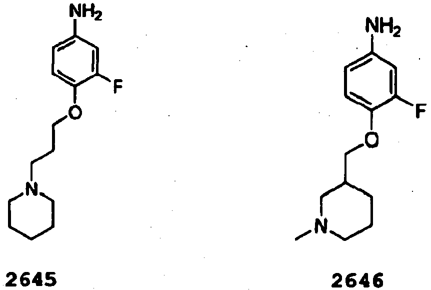

### Example 2647

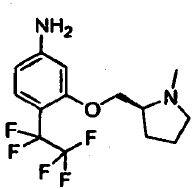

### (S)-3-((1-methylpyrrolidin-2-yl)methoxy)-4-(perfluoroethyl)benzenamine

The title compound was prepared according to the method described in WO 04/0854258.

### Example 2648

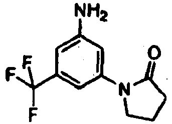

### 1-(3-amino-5-(trifluoromethyl)phenyl)pyrrolidin-2-one

The title compound was prepared according to the method described in WO 05/070891.

### Example 2649

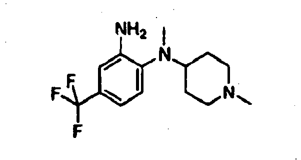

### N1-methyl-N1-(1-methylpiperidin-4-yl)-4-(trifluoromethyl)benzene-1,2-diamine

The title compound was prepared according to the method described in pending U.S. Patent Application No. 60/569,193. MS m/z = 288.1 [M+H]⁺; Calc'd for C₁₄H₂₀F₃N₃: 287.

### Example 2650

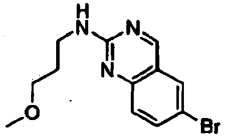

6-bromo-N-(3-methoxypropyl)quinazolin-2-amine was synthesized in accordance with the method of Example 726, Method 1, and obtained as a pale yellow solid. MS *m*/*z* = 296, 298 [M]⁺, [M+2]⁺; Calc'd for C₁₂H₁₄BrN₃O: 296.

### Example 2651

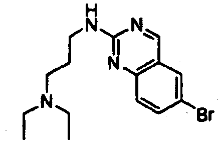

6-bromo-N-(3-(diethylamino)propyl)quinazolin-2-amine, was synthesized in accordance with the method of Example 726, Method 1, and obtained as a pale yellow solid. MS *m*/*z* = 337, 339 [M]⁺, [M+2]⁺; Calc'd for C₁₅H₂₁BrN₄: 337.

### Example 2652

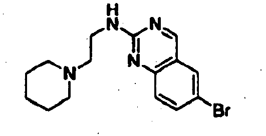

6-bromo-N-(2-(piperidin-1-yl)ethyl)quinazolin-2-amine was synthesized in accordance with the method of Example 726, Method 1, and obtained as a yellow solid. MS *m*/*z* = 335, 337 [M]⁺, [M+2]⁺; Calc'd for C₁₅H₁₉BrN₄ : 335.

### Example 2653

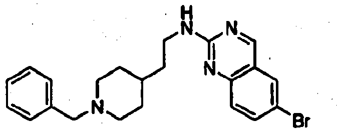

N-(2-(1-benzylpiperidin-4-yl)ethyl)-6-bromoquinazolin-2-amine was synthesized in accordance with the method of Example 726, Method 1, and obtained as a pale yellow solid. MS *m*/*z* = 425, 427 [M]⁺, [M+2]⁺; Calc'd for C₂₂H₂₅BrN₄: 425.

### Example 2654

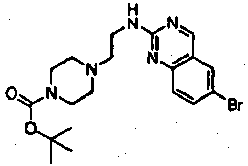

Tert-butyl 4-(2-(6-bromoquinazolin-2-ylamino)ethyl)piperazine-1-carboxylate was synthesized in accordance with the method of Example 726, Method 1, and obtained as a yellow solid. MS *m*/*z* = 436, 438 [M]⁺, [M+2]⁺; Calc'd for C₁₉H₂₆BrN₅O₂: 436.

### Example 2655

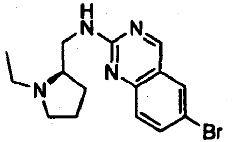

(R)-6-bromo-N-((1-ethylpyrrolidin-2-yl)methyl)quinazolin-2-amine was synthesized in accordance with the method of Example 726, Method 1, and obtained as a yellow solid. MS *m*/*z* = 335, 337 [M]⁺, [M+2]⁺; Calc'd for C₁₉H₂₆BrN₅O₂: 335.

### Example 2656

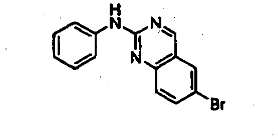

6-bromo-N-phenylquinazolin-2-amine was synthesized in accordance with the method of Example 732, Method 1, and obtained as a pale yellow solid. MS *m*/*z* = 300, 302 [M]⁺, [M+2]⁺; Calc'd for C₁₄H₁₀BrN₃: 300.

### Example 2657

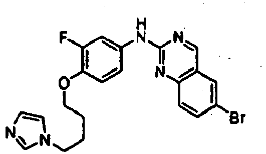

N-(4-(4-(1H-imidazol-1-yl)butoxy)-3-fluorophenyl)-6-bromoquinazolin-2-amine was synthesized in accordance with the method of Example 732, Method 1, and obtained as an orange solid. MS *m*/*z* = 456, 458 [M]⁺, [M+2]⁺; Calc'd for C₂₁H₁₉BrFN₅O: 456.

### Example 2658

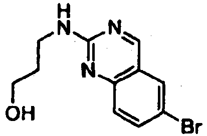

3-(6-bromoquinazolin-2-ylamino)propan-1-ol was synthesized in accordance with the method of Example 732, Method 1, and obtained as a yellow solid. MS *m*/*z* = 280, 282 [M]⁺, [M+2]⁺; Calc'd for C₁₁H₁₂BrN₃O: 282.

### Example 2659

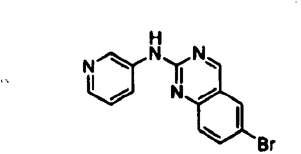

6-bromo-N-(pyridin-2-yl)quinazolin-2-amine was synthesized in accordance with the method of Example 732, Method 1, and obtained as a brown solid. MS *m*/*z* = 302, 303 [M]⁺, [M+2]⁺; Calc'd for C₁₃H₉BrN₄ : 301.

### Example 2660

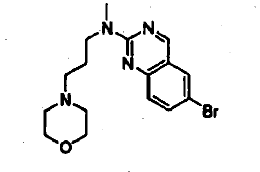

6-bromo-N-(3-morpholinopropyl)quinazolin-2-amine (0.225 g, 0.64 mmol) [Example 728] was taken up in THF (3.5 mL) and DMF (3 mL). NaH (0.077 g, 1.92 mmol, 60% dispersion in mineral oil) was added and the mixture was allowed to stir at RT for 10 min. MeI (0.27 g, 1.92 mmol) was added and the mixture was allowed to stir at RT for 3 h. After quenching with water, the mixture was extracted with CH₂Cl₂, washed with brine, then dried over Na₂SO₄ and concentrated to an orange solid. This material was used without further purification. MS *m*/*z* = 366 [M+1]⁺; Calc'd for C₁₆H₂₁BrN₄O: 365.

### Example 2661

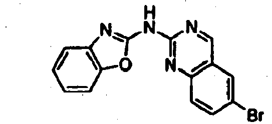

2-amino-6-bromoquinazoline [Example 722] (0.300 g, 1.14 mmol) was taken up in DMF (5 mL), and the suspension was purged with N2. NaH (0.064 g, 1.61 mmol, 60% dispersion on mineral oil) was added and the mixture was allowed to stir for 10 min at rt. 2-chlorobenzo[d]oxazole (0.226 mg, 1.47 mmol) was added and the mixture was allowed to stir overnight at RT. The suspension was filtered through a Buchner apparatus with micromembrane and the filtrate was washed with MeOH (crop 1, ∼50% pure). The mother liquors were concentrated and triturated with MeOH. Filtration and washing with MeOH afforded crop 2 of the compound above as a pale yellow solid (∼85% pure). Crop 2 was used without further purification. MS *m*/*z* = 341, 343 [M]⁺, [M+2]⁺; Calc'd for C₁₅H₉BrN₄O: 341.

### Example 2662

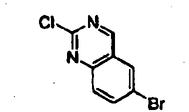

A three-necked 1L round-bottom flask equipped with magnetic stirrer, temperature probe and a reflux condenser was charged with 2-amino-6-bromoquinazoline [Example 722] (22.4 g, 0.100 mol) and suspended in a mixture of 1,2-dichloroethane (0.45 L) and DMF (45 mL). To the resulting heterogenous mixture was added tetrabutylammonium chloride (84 g, 0.30 mol). The reaction vessel was placed in a 52°C oil-bath with efficient agitation and the trimethylchlorosilane (38mL, 0.30 mol) was added slowly upon which slight exotherm was observed (44°C to 46°C) and the reaction mixture gradually became a clear orange solution. Once the internal temperature reached 50°C tert-butylnitrite (18mL, 0.15 mol) was added dropwise via syringe pump over 2h. Once all the nitrite was added, heating was continued for 45 min. At this point the clear dark-orange solution was concentrated under reduced pressure to a thick oil. Water (0.60L) was slowly added with stirring and the resulting fine yellow precipitate collected by vacuum filtration. The solid was rinsed on a filter with water and dried to afford 6-bromo-2-chloroquinazoline as a yellow powder. MS m/z = 243, 245 [M]⁺, [M+2]⁺; Calc'd for C₈H₄BrClN₂: 243.

### Example 2663

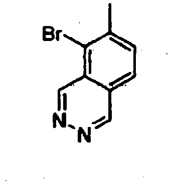

### 5-bromo-6-methylphthalazine

A 20 x150 mm pyrex tube was charged with 6-methylphthalazine (1.0 g, 6.9 mmol), Fe powder (0.81 g, 15 mmol) and Br₂ (4.3 ml, 83 mmol) in CH₂Cl₂ (5 mL). The mixture was allowed to stir at rt for 0.5 h then heated to 50 °C for 2 d. The crude reaction mixture was poured into aqueous Na₂SO₄ and extracted 3 times with CH₂Cl₂. The organics were washed with brine and dried over Na₂SO₄. After concentration, the residue was taken up in minimal CH₂Cl₂ and purified by MPLC (Isco: Redi-Sep® pre-packed silica gel column (120 g); eluent gradient: 3-10% MeOH in CH₂Cl₂) to afford a tan solid (∼85% 5-bromo-6-methylphthalazine, 15% 6-methylphthalazine by 1H NMR). This material was used without further purification.

### Example 2664

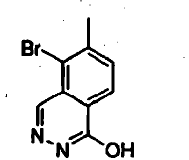

### Step 1: 6-methylphthalazin-1(2H)-one

To a slurry of 6-bromophthalazin-1-ol(6.0 g, 27 mmol) [Bakthavatchatam, R.; Blum, C.; Brielmann, H. L.; Caldwell, T. M.; De Lombaert, S.; Hodgetts, K. J. WO 03/062209 A2, 2003] and Pd(dppf)₂Cl₂ (2.0 g, 3mmol) in DMF (150 mL) in a 350 mL sealed tube reactor at RT was added tetramethylstannane (6.0 mL, 47 mmol). The reaction vessel was sealed and then heated to 65 °C for 24 h, after which time the LCMS showed complete conversion of starting material. The reaction mixture was concentrated to dryness and the crude dark brown residue was taken up in water and extracted with ethyl acetate (4 x 100 mL). The combined organic layers were washed with saturated KF, dried over Na₂SO₄ and concentrated to give a light orange solid. This material was taken up in CH₂Cl₂/MeOH and purified by MPLC (Isco; 120 g SiO₂ column, eluting with 0 to 60 % EtOAc in hexanes) to afford 1.97 g of an orange solid. This material was triturated with CH₂Cl₂ and filtered to afford 6-methylphthalazin-1(2H)-one as a white solid. LCMS (ESI) m/z 161 [M+1]; Calcd for C₉H₈N₂O: 161.

### Step 2: 5-bromo-6-methylphthalazin-1(2H)-one

In a 16x100 mm pyrex tube, 6-methylphthalazin-1(2H)-one (0.100 g, 0.62 mmol) and Fe powder (0.073 g, 1.3 mmol) were taken up in Br₂ (0.39 ml, 7.5 mmol) and CH₂Cl₂ (2 mL). The mixture was allowed to stir at rt for 0.5 h then heated to 50 °C for 2 d. After cooling, the mixture was poured into aqueous Na₂SO₄ and extracted with CH₂Cl₂. The extracts were washed with brine and dried over Na₂SO₄ then concentrated to afford an off-white solid (5-bromo-6-methylphthalazin-1(2H)-one, and 6-methylphthalazin-1(2H)-one by 1H NMR). This material was used without further purification. LCMS (ESI) *m*/*z* 139, 241 [M], [M+2]; Calcd for C₉H₇BrN₂O: 239.

### Example 2665

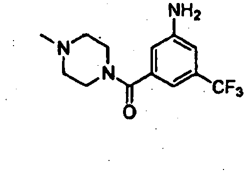

### (3-amino-5-(trifluoromethyl)phenyl)(4-methylpiperazin-1-yl)methanone

### Step 1. Preparation of (4-methylpiperazin-1-yl)(3-nitro-5-trifluoromethyl)phenyl)-methanone

A solution of thionyl chloride (30 ml) and 3-nitro-5 (trifluoromethyl)benzoic acid (10 g) was heated to reflux for 2 h. The reaction mixture was concentrated under reduced pressure and treated with toluene (10 ml) which was then removed under reduced pressure to afford 3-nitro-5-(trifluoromethyl)benzoyl chloride. To a solution of 3-nitro-5-(trifluoromethyl)benzoyl chloride (2.35 g, 9.3 mmol) in CH₂Cl₂ (40 ml) at room temperature was added *N*-methylpiperazine (1.26 ml, 9.3 mmol) and the mixture was allowed to stir for 30 min. The reaction was concentrated under reduced pressure, taken up in 1 M HCl (50 ml) and the aqueous layer was washed with Et₂O (2 x 20 ml). The aqueous layer was basified to a pH of about 9 with 6 N NaOH, and the aqueous layer was extracted with Et₂O (3 x 50 ml). The organic extracts were combined and washed with water (1 x 20 ml) followed by brine (1 x 20 ml), and dried over anhydrous sodium sulfate, filtered and concentrated under reduced pressure to afford (4-methylpiperazin-1-yl)(3-nitro-5-trifluoromethyl)phenyl)-methanone as a tan oil, which was used without further purification.

### Step 2. Preparation of (3-amino-5-(trifluoromethyl)phenyl)(4-methylpiperazin-1-yl)methanone

To an argon purged solution of (4-methylpiperazin-1-yl(3-nitro-5-trifluoromethyl)phenyl)-methanone (1.03 g, 3.25 mmol) was added Pd/C (344 mg, 0.32 mmol, 10%). The mixture was placed under an atmosphere of H₂ at RT for 5 h. The reaction was purged with argon and filtered through Celite. The filtrate was concentrated under reduced pressure to afford (3-amino-5-(trifluoromethyl)phenyl)(4-methylpiperazin-1-yl)methanone as an off-white solid. MS *m*/*z* = 288 [M+H]⁺; Calc'd for C₁₃H₁₆F₃N₃O: 287.3.

### Example 2666

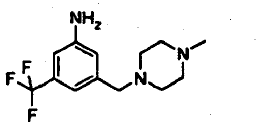

### 3-((4-methylpiperazin-1-yl)methyl)-5-(trifluoromethyl)-benzenamine

To LAH (1.84 g, 48.5 mmol) in THF (50 ml) at RT was added (4-methylpiperazin-1-yl)(3-nitro-5-trifluoromethyl)phenyl)-methanone (1.54 g, 4.85 mmol) in THF (10 ml). The resulting mixture was refluxed for 5 h. The reaction mixture was cooled to 0°C at which point water (1.84 ml), 15% aq. NaOH (1.84 ml and water (3.68 ml) were successively added. The resulting mixture was allowed to stir at room temperature for 1 h. The mixture was filtered through Celite, concentrated under reduced pressure and purified via flash chromatography (silica gel, 0 to 25% MeOH in CH₂Cl₂, gradient elution) to afford 3-((4-methylpiperazin-1-yl)methyl)-5-(trifluoromethyl)benzenamine as a colorless oil. MS: *m*/*z* = 274 [M+H]⁺; Calc'd for C₁₃H₁₈F₃N₃: 273.30.

### Example 2667

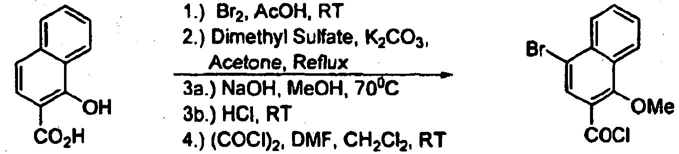

The compound was synthesized in accordance with the method described in J. Med. Chem. 2001, 44, 1815.

### Example 2668

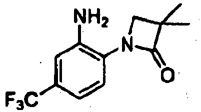

### 1-(2-amino-4-(trifluoromethyl)phenyl)-3,3-dimethylazetidin-2-one

### Step 1: 3-chloro-2,2-dimethyl-N-(2-nitro-4-(trifluoromethyl)phenyl)propanamide

To a solution of 2-nitro-4-(trifluoromethyl)benzenamine (3.00 g, 14.9 mmol, 1.0 equiv) in CH₂Cl₂ (90 mL) at 25°C, was added 3-chloro-2,2-dimethylpropanoyl chloride (3.9 mL, 29.8 mmol, 2.0 equiv) followed by triethylamine (4.2 mL, 29.8 mmol, 2.0 equiv). The solution was heated to 40°C. After 48 h, the solution was washed with water (50 mL), and brine (50 mL). After concentration *in vacuo,* the residue was purified by silica gel chromatography (1:10 hexanes:EtOAc to 1:5 hexanes:EtOAc) to afford 3-chloro-2,2-dimethyl-N-(2-nitro-4-(trifluoromethyl)phenyl)propanamide.

### Step 2: 3,3-dimethyl-1-(2-nitro-4-(trifluoromethyl)phenyl)azetidin-2-one

To a mixture of 3-chloro-2,2-dimethyl-N-(2-nitro-4-(trifluoromethyl)phenyl)propanamide (3.24 g, 10.0 mmol, 1.0 equiv) in acetone (100 mL) was added K₂CO₃ (3.5 g, 25.0 mmol, 2.5 equiv). The mixture was heated to 50°C for 48 h. After filtering, the solvent was removed *in vacuo* and the resulting residue purified by silica gel chromatography (1:10 hexanes:EtOAc to 1:5 hexanes:EtOAc) to afford 3,3-dimethyl-1-(2-nitro-4-(trifluoromethyl)phenyl)azetidin-2-one. MS: (MH⁺) 289.1; Calculated 288.1 for C₁₂H₁₁F₃N₂O₃.

### Step 3: 1-(2-amino-4-(trifluoromethyl)phenyl)-3,3-dimethylazetidin-2-one

A mixture of 3,3-dimethyl-1-(2-nitro-4-(trifluoromethyl)phenyl)azetidin-2-one (1.67 g, 5.8 mmol, 1.0 equiv) and 10% Pd/C (300 mg) in MeOH (30 mL) was exposed to an atmosphere of H₂. After consumption of the starting material, the mixture was filtered and concentrated to afford 1-(2-amino-4-(trifluoromethyl)phenyl)-3,3-dimethylazetidin-2-one, which was advanced without further purification. MS: (MH⁺) 259.1; Calculated 258.1 for C₁₂H₁₃F₃N₂O.

### Example 2669

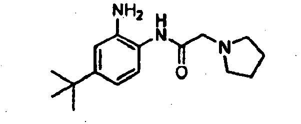

### N-(2-amino-4-(trifluoromethyl)phenyl)-2-(pyrrolidin-1-yl)acetamide

### Step 1: N-(4-tert-butyl-2-nitrophenyl)-2-chloroacetamide

To a solution of 4-tert-butyl-2-nitrobenzenamine (3.04 g, 15.67 mmol, 1.0 equiv) in CH₂Cl₂ (90 mL) at 0°C was added chloroacetyl chloride (1.63 mL, 20.4 mmol, 1.3 equiv) followed by triethylamine (5.40 mL, 23.5 mmol, 2.5 equiv). After 1h, the solution was warmed to 25°C and allowed to stir until the reaction was complete. The solution was washed with water and dried with Na₂SO₄. The solvent was removed in vacuo and the residue purified by silica gel chromatography (9:1 hexanes:EtOAc) to afford N-(4-tert-butyl-2-nitrophenyl)-2-chloroacetamide. MS (MH⁺) 271; Calculated 270.1 for C₁₂H₁₅ClN₂O₃.

### Step 2: N-(4-tert-butyl-2-nitrophenyl)-2-(pyrrolidin-1-yl)acetamide

To a resealable tube charged with N-(4-tert-butyl-2-nitrophenyl)-2-chloroacetamide (0.250 g, 0.93 mmol, 1.0 equiv) and THF (3 mL) was added pyrrolidine (0.092 mL, 1.11 mmol, 1.2 equiv) and triethylamine (0.390 mL, 2.77 mmol, 3.0 equiv). The solution was heated to 80°C. After 6h, the mixture was cooled to room temperature, diluted with EtOAc and washed with water and brine. The organic fraction was dried with Na₂SO₄ and concentrated in vacuo to afford N-(4-tert-butyl-2-nitrophenyl)-2-(pyrrolidin-1-yl)acetamide that was advanced without further purification. MS (MH⁺) 306; Calculated 305.1 for C₁₆H₂₃N₃O₃.

### Step 3: N-(2-amino-4-tert-butylphenyl)-2-(pyrrolidin-1-yl)acetamide

A mixture of N-(4-tert-butyl-2-nitrophenyl)-2-(pyrrolidin-1-yl)acetamide (281 mg, 0.92 mmol, 1.0 equiv) and Adam's catalyst (5 mg) in EtOAc (15 mL) was exposed to an atmosphere of H₂ (balloon). Upon completion of the reduction, the reaction mixture was filtered through celite and concentrated *in* vacuo to afford N-(2-amino-4-tert-butylphenyl)-2-(pyrrolidin-1-yl)acetamide, which was advanced without further purification. MS (MH⁺) 276; Calculated 275.2 for C₁₆H₂₅N₃O.

The following aniline intermediates, Examples 2670-2676, were prepared in a manner similar to that described in Example 2669.

| **Example** | **Structure** | **Name** | **Cal'd MS** | **M+H⁺** |
|---|---|---|---|---|
| 2670 | 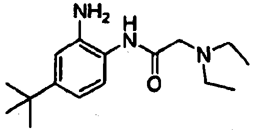 | N-(2-amino-4-tert-butylphenyl)-2-(diethylamino)acetamide | 277.2 | 278 |
| 2671 | 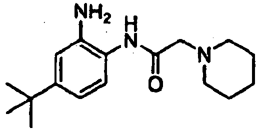 | N-(2-amino-4-tert-butylphenyl)-2-(piperidin-1-yl)acetamide | 289.2 | 290 |
| 2672 | 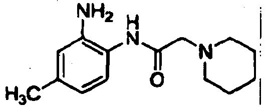 | N-(2-amino-4-methylphenyl)-2-(piperidin-1-yl)acetamide | 247.2 | 248 |
| 2673 | 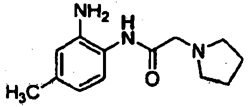 | N-(2-amino-4-methylphenyl)-2-(pyrrolidin-1-yl)acetamide | 233.2 | 234 |
| 2674 | 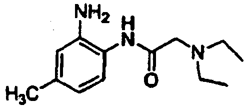 | N-(2-amino-4-methylphenyl)-2-(diethylamino)acetamide | 235.2 | 236 |
| 2675 | 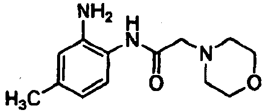 | N-(2-amino-4-methylphenyl)-2-morpholinoacetamide | 249.2 | 250 |
| 2676 | 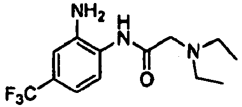 | N-(2-amino-4-(trifluoromethyl)phenyl)-2-(diethylamino)acetamide | 289.1 | 290 |

### Example 2677

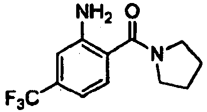

(2-amino-4-(trifluoromethyl)phenyl)(pyrrolidin-1-yl)methanone was prepared in a manner similar to that described in Example 568. MS (MH⁺) 259; Calculated 258.1 for C₁₂H₁₃F₃N₂O.

### Example 2678

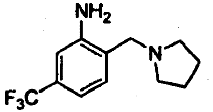

2-(pyrrolidin-1-ylmethyl)-5-(trifluoromethyl)benzenamine was prepared in a manner similar to that described in Example 570. MS (MH⁺) 245; Calculated 244.1 for C₁₂H₁₅F₃N₂.

### Example 2679

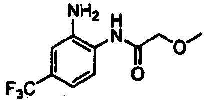

### N-(2-amino-4-(trifluoromethyl)phenyl)-2-methoxyacetamide

### Step 1: 2-methoxy-N-(2-nitro-4-(trifluoromethyl)phenyl)acetamide

To a solution of 2-nitro-4-(trifluoromethyl)benzenamine (0.537 g, 2.61 mmol, 1.0 equiv) in CH2Cl2 at room temperature was added methoxy acetyl chloride (0.368 g, 3.39mmol, 1.3 equiv) followed by triethylamine (0.728 mL, 5.22 mmol, 2.0 equiv). After 24 hrs, water was added and the organic fraction was washed with brine and dried with Na2S04. Concentration *in vacuo* and purification of the resulting residue by silica gel chromatography (10-30% Hexanes:EtOAc) afforded 2-methoxy-N-(2-nitro-4-(trifluoromethyl)phenyl)acetamide. MS (MH⁺) 279; Calc'd 278.1 for C₁₀H₉F₃N₂O₄.

### Step 2: N-(2-amino-4-(trifluoromethyl)phenyl)-2-methoxyacetamide

A mixture of 2-methoxy-N-(2-nitro-4-(trifluoromethyl)phenyl)acetamide (273 mg, 0.98 mmol, 1.0 equiv) and Pd/C (50 mg) in EtOAc (5 mL) and MeOH (15 mL) was exposed to an atmosphere of H₂ (balloon). Upon completion of the reduction, the reaction mixture was filtered through celite and concentrated *in vacuo* to afford N-(2-amino-4-(trifluoromethyl)phenyl)-2-methoxyacetamide, which carried forward without further purification. MS: (MH⁺) 249; Calc'd 248.1 for C₁₀H₁₁F₃N₂O₂.

### Example 2680

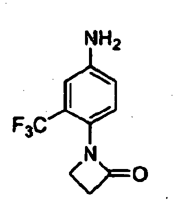

### 1-(4-amino-2-(trifluoromethyl)phenyl)azetidin-2-one

To a mixture of 4-bromo-3-(trifluoromethyl)benzenamine (1.00 g, 4.16 mmol, 1.0 equiv), N1,N2-dimethylethane-1,2-diamine (0.089 mL, 0.83 mmol, 0.2 equiv), potassium carbonate (1.14 g, 8.32 mmol, 2.0 equiv), copper(I) iodide (0.055 g, 0.29 mmol, 0.07 equiv), and 2-azetidinone (444 mg, 6.25 mmol, 1.5 equiv) in a resealable tube was added Toluene (2.5 mL). The mixture was heated at 110°C for 32 hrs before filtering and washing the solids with EtOAc. The filtrate was concentrated *in vacuo* and purified by silical gel chromatography (20-50% Hexanes:EtOAc) to afford 1-(4-amino-2-(trifluoromethyl)phenyl)azetidin-2-one. MS (MH⁺) 231; Calc'd 230.1 for C₁₀H₉F₃N₂O.

The following aniline intermediates, Examples 2681-2686, were prepared in a manner similar to that described in Example 2680.

| **Example** | **Structure** | **Name** | **Cal'd MS** | **M+H⁺** |
|---|---|---|---|---|
| 2681 | 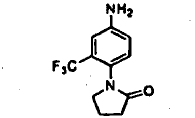 | 1-(4-amino-2-(trifluoromethyl)phenyl)pyrrolidin-2-one | 244.1 | 245 |
| 2682 | 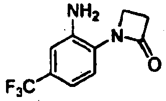 | 1-(2-amino-4-(trifluoromethyl)phenyl) azetidin-2-one | 230.1 | 231 |
| 2683 | 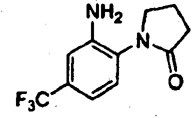 | 1-(2-amino-4-(trifluoromethyl)phenyl) pyrrolidin-2-one | 244.1 | 245 |
| 2684 | 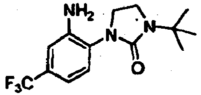 | 1-(2-amino-4-(trifluoromethyl)phenyl)-3-tert-butylimidazolidin-2-one | 301.1 | 302 |
| 2685 | 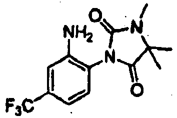 | 3-(2-amino-4-(trifluoromethyl)phenyl)-1,5,5-trimethylimidazolidine-2,4-dione | 301.1 | 302 |
| 2686 | 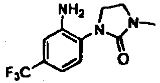 | 1-(2-amino-4-(trifluoromethyl)phenyl)-3-methylimidazolidin-2-one | 259.1 | 260 |

### Example 2687

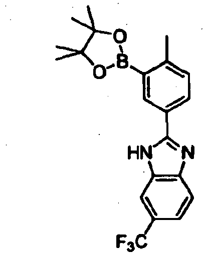

### 2-(4-methyl-3-(4,4,5,5-tetramethyl-1,3,2-dioxaborolan-2-yl)phenyl)-6-(trifluoromethyl)-1H-benzo[d]imidazole

A mixture of 4-methyl-3-(4,4,5,5-tetramethyl-1,3,2-dioxaborolan-2-yl)benzoic acid (124 mg, 0.473 mmol, 1.0 equiv) and thionyl chloride (3 mL) was heated at 75°C for 1 hr. The resulting solution was cooled and the excess thionyl chloride removed *in vacuo.* To the residue was added CH₂Cl₂ (5 mL) followed by 4-(trifluoromethyl)benzene-1,2-diamine (124 mg, 0.473 mmol, 1.0 equiv) and triethylamine (0.165 mL, 1.18 mmol, 2.5 equiv). After 4 hrs, the solvent was removed *in vacuo* and the residue taken up in EtOH (5 mL). Concentrated HCl (0.50 mL) was added and the solution was heated at 60°C for 24 hrs. After cooling to room temperature, the solvent was removed *in vacuo.* The residue was taken up in EtOAc and neutralized with saturated NaHCO₃. The organic fraction was washed with brine, dried with Na₂SO₄, and concentrated in vacuo to afford crude 2-(4-methyl-3-(4,4,5,5-tetramethyl-1,3,2-dioxaborolan-2-yl)phenyl)-6-(trifluoromethyl)-1H-benzo[d]imidazole, that was advanced without further purification. MS: (MH⁺) 403; Calc'd 402.2 for C₂₁H₂₂BF₃N₂O₂.

The following aniline intermediates, Examples 2688-2690, were prepared in a manner similar to that described in Example 2687.

| **Example** | **Structure** | **Name** | **Cal'd MS** | **M+H⁺** |
|---|---|---|---|---|
| 2688 | 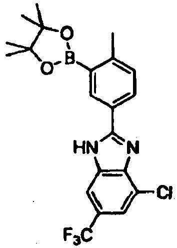 | 4-chloro-2-(4-methyl-3-(4,4,5,5-tetramethyl-1,3,2-dioxaborolan-2-yl)phenyl)-6-(trifluoromethyl)-1H-benzo[d]imidazole | 436.1 | 437 |
| 2689 | 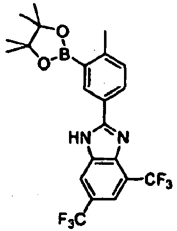 | 2-(4-methyl-3-(4,4,5,5-tetramethyl-1,3,2-dioxaborolan-2-yl)phenyl)-4,6-bis(trifluoromethyl)-1H-benzo[d]imidazole | 470.2 | 471 |
| 2690 | 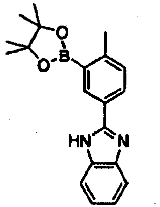 | 2-(4-methyl-3-(4,4,5,5-tetramethyl-1,3,2-dioxaborolan-2-yl)phenyl)-1H-benzo[d]imidazole | 334.2 | 335 |

### Example 2691

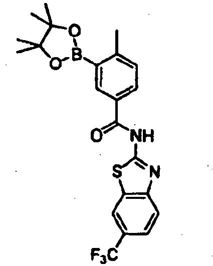

### 4-methyl-3-(4,4,5,5-tetramethyl-1,3,2-dioxaborolan-2-yl)-N-(6-(trifluoromethyl)benzo[d]thiazol-2-yl)benzamide

A mixture of 4-methyl-3-(4,4,5,5-tetramethyl-1,3,2-dioxaborolan-2-yl)benzoic acid (178 mg, 0.677 mmol, 1.1 equiv) and thionyl chloride (3 mL) was heated at 75°C for 1 hr. The resulting solution was cooled and the excess thionyl chloride removed in vacuo. To the residue was added CH₂Cl₂ (15 mL) followed by 6-(trifluoromethyl)benzo[d]thiazol-2-amine (0.148 g, 0.677 mmol) and triethylamine (0.283 ml, 2.03 mmol, 3.0 equiv). After 24 hrs at RT, the solution was diluted with dichloromethane and washed with water and brine. After concentration *in vacuo,* the crude 4-methyl-3-(4,4,5,5-tetramethyl-1,3,2-dioxaborolan-2-yl)-N-(6-(trifluoromethyl)benzo[d]thiazol-2-yl)benzamide was advanced without further purification. MS: (MH⁺) 463; Calc'd 462.1 for C₂₂H₂₂BF₃N₂O₃S.

The following boronic ester intermediates, Examples 2692-2693, were prepared in a manner similar to that described in Example 2691.

| **Example** | **Structure** | **Name** | **Cal'd MS** | **M+H⁺** |
|---|---|---|---|---|
| 2692 | 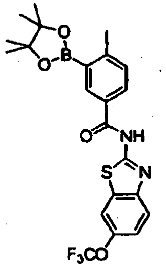 | 4-methyl-3-(4,4,5,5-tetramethyl-1,3,2-dioxaborolan-2-yl)-N-(6-(trifluoromethoxy)benzo[ d]thiazol-2-yl)benzamide | 478.1 | 479 |
| 2693 | 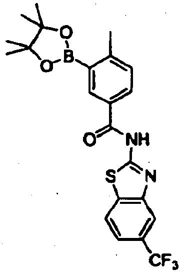 | 4-methyl-3-(4,4,5,5-tetramethyl-1,3,2-dioxaborolan-2-yl)-N-(5-(trifluoromethyl)benzo[d]thiazol-2-yl)benzamide | 462.1 | 463 |

### Example 2694

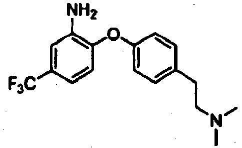

2-(4-(2-(dimethylamino)ethyl)phenoxy)-5-(trifluoromethyl)benzenamine was prepared in a manner analogous to that described in Example 588. MS: (MH⁺) 325; Calc'd 324.1 for C₁₇H₁₉F₃N₂O.

### Example 2695

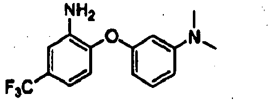

2-(3-(dimethylamino)phenoxy)-5-(trifluoromethyl)benzenamine was prepared in a manner analogous to that described in Example 588. MS (MH⁺) 297; Calculated 296.1 for C₁₅H₁₅F₃N₂O.

The following examples, 2696-2708 were prepared in a manner analogous to that described in Example 588 herein.

| **Example** | **Structure** | **Name** | **Cal'd MS** | **M+H⁺** |
|---|---|---|---|---|
| 2696 | 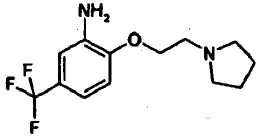 | 2-(2-(pyrrolidin-1-yl)ethoxy)-5-(trifluoromethyl)benzenamine | 274.13 | **275.1** |
| 2697 | 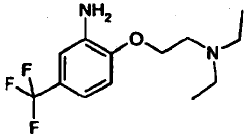 | 2-(2-(diethylamino)ethoxy)-5-(trifluoromethyl)benzenamine | 276.14 | 277.1 |
| 2698 | 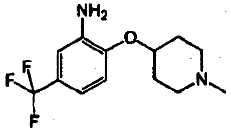 | 2-(1-methylpiperidin-4-yloxy)-5-(trifluoromethyl)benzenamine | 274.13 | 275.0 |
| 2699 | 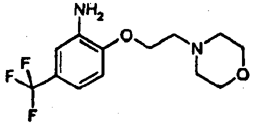 | 2-(2-morpholinoethoxy)-5-(trifluoromethyl)benzenamine | 290.12 | 291.1 |
| 2700 | 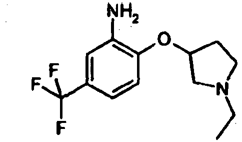 | 2-(1-ethylpyrrolidin-3-yloxy)-5-(trifluoromethyl)benzenamine | 274.13 | 275.1 |
| 2701 | 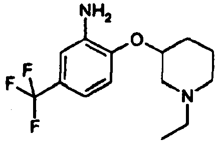 | 2-(1-ethylpiperidin-3-yloxy)-5-(trifluoromethyl)benzenamine | 288.14 | 289.1 |
| 2702 | 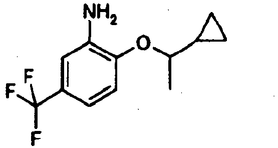 | 2-(1-cyclopropylethoxy)-5-(trifluoromethyl)benzenamine | 245.1 | 246.0 |
| 2703 | 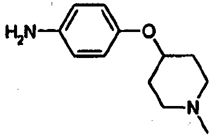 | 4-(1-methylpiperidin-4-yloxy)benzenamine | 206.14 | 207.1 |
| 2704 | 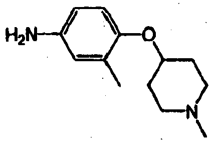 | 3-methyl-4-(1-methylpiperidin-4-yloxy)benzenamine | 220.16 | 221.1 |
| 2705 | 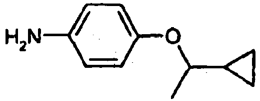 | 4-(1-cyclopropylethoxy)benzenamine | 177.12 | 178.1 |
| 2706 | 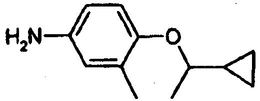 | 4-(1-cyclopropylethoxy)-3-methylbenzenamine | 191.13 | 192.1 |
| 2707 | 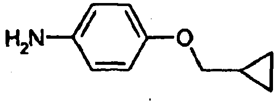 | 4-(cyclopropylmethoxy)benzenamine | 163.10 | 164.1 |
| 2708 | 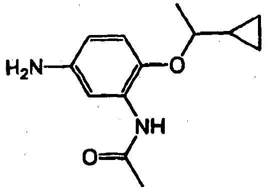 | N-(5-amino-2-(1-cyclopropyl-ethoxy)phenyl)acetamide | 234.14 | 235.1 |

### Example 2709

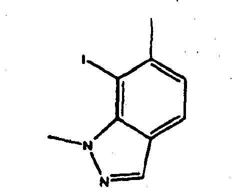

### 7-iodo-1,6-dimethyl-1H-indazole

Into a 16 x 100mm vial was placed 1,6-dimethyl-1H-indazol-7-amine (0.100 g, 0.620 mmol)(synthesis described in patent application A-1018-1046. MS (ESI, pos. ion) m/z: 162.1 [M+1].), THF (3.00 ml, 37.0 mmol), diiodomethane (0.255 ml, 3.16 mmol), isopentyl nitrite (0.250 ml, 1.86 mmol), and copper(I) iodide (0.0210 ml, 0.620 mmol). Vial sparged with argon, capped and heated to 100°C for 4 hours. Solvents were removed under reduced pressure and the residue triturated with dichloromethane. The solid was filtered to afford the title compound. MS: (ESI) m/z: 273.0 [M+1].

### Example 2710

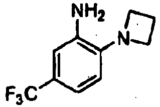

2-(azetidin-1-yl)-5-(trifluoromethyl)benzenamine was prepared in a manner analogous to that described in Example 55 of pending U.S. Patent Application No. 60/569,193. MS (MH⁺) 217; Calculated 216.1 for C₁₀H₁₁F₃N₂.

### Example 2711

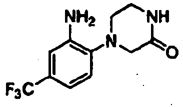

4-(2-amino-4-(trifluoromethyl)phenyl)piperazin-2-one was prepared in a manner analogous to that described in Example 55 of pending U.S. Patent Application No. 60/569,193. MS (MH⁺) 260; Calculated 259.1 for C₁₁H₁₂F₃N₃O.

### Example 2712

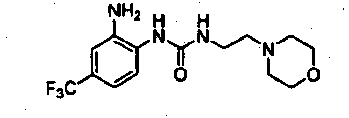

### 1-(2-amino-4-(trifluoromethyl)phenyl)-3-(2-morpholinoethyl)urea

### Step 1: 1-(2-morpholinoethyl)-3-(2-nitro-4-(trifluoromethyl)phenyl)urea

To a solution of 1-isocyanato-2-nitro-4-(trifluoromethyl)benzene (0.735 g, 3.17 mmol, 1.0 equiv) in benzene (10 mL) at 25°C, was added 2-morpholinoethanamine (433 mg, 3.33 mmol, 1.05 equiv). The solution was heated to 40°C. After 24 h, the mixture was cooled to room temperature and filtered. The filtrate was washed with benzene and the crude 1-(2-morpholinoethyl)-3-(2-nitro-4-(trifluoromethyl)phenyl)urea was used without further purification. MS (MH⁺) 363; Calculated 362.1 for C₁₄H₁₇F₃N₄O₄.

### Step 2: 1-(2-amino-4-(trifluoromethyl)phenyl)-3-(2-morpholinoethyl)urea

A mixture of 1-(2-morpholinoethyl)-3-(2-nitro-4-(trifluoromethyl)phenyl)urea (0.970 g, 2.68 mmol, 1.0 equiv) and Pd/C (300 mg) in EtOAc (10 mL) and MeOH (20 mL) was exposed to an atmosphere of H₂ (balloon). Upon completion of the reduction, the reaction mixture was filtered through celite and concentrated *in vacuo.* The residue was purified by silica gel chromatography using 10% MeOH in CH₂Cl₂ to afford 1-(2-amino-4-(trifluoromethyl)phenyl)-3-(2-morpholinoethyl)urea. MS: (MH⁺) 333; Calc'd 332.2 for C₁₄H₁₉F₃N₄O₂.

### Example 2713

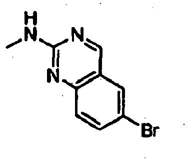

### 6-bromo-N-methylquinazolin-2-amine

### Step 1: 6-bromo-N-methylquinazolin-2-amine

A 1-L high-pressure reactor was charged with 6-bromoquinazolin-2-amine (20.0 g, 89.3 mmol, 1.0 equiv), p-Toluenesulfonic acid monohydrate (33.9 g, 179 mmol, 2.0 equiv), and methylamine (ca. 150 g). The reactor was slowly heated to 150°C (820 psi internal pressure). After 24 hrs, the reactor was cooled to room temperature and excess methylamine was slowly vented. The crude residue was taken up in CH₂Cl₂ (1.0 L) and washed with saturated NaHCO₃ and brine. The organic fraction was concentrated *in vacuo* and purified by silica gel chromatography (1-3% MeOH in CH₂Cl₂) to afford 6-bromo-N-methylquinazolin-2-amine (14.6g, 61.3 mmol, 68%) as a yellow solid. MS (MH⁺) 238.0; Calculated 238.0 for C₉H₉BrN₃.

The following Examples 3000-3400 should assist in better understanding of the scope of the compounds of the present invention.

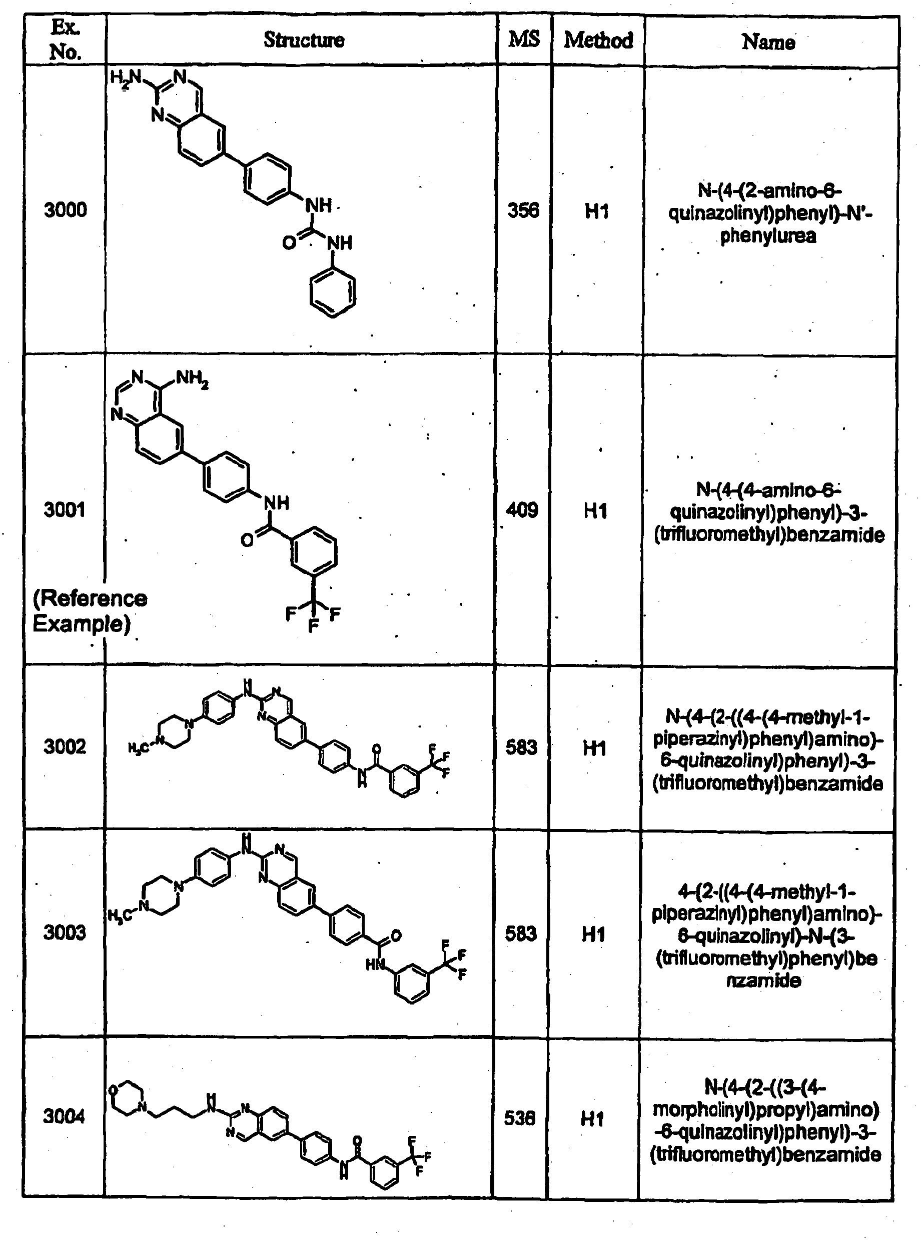

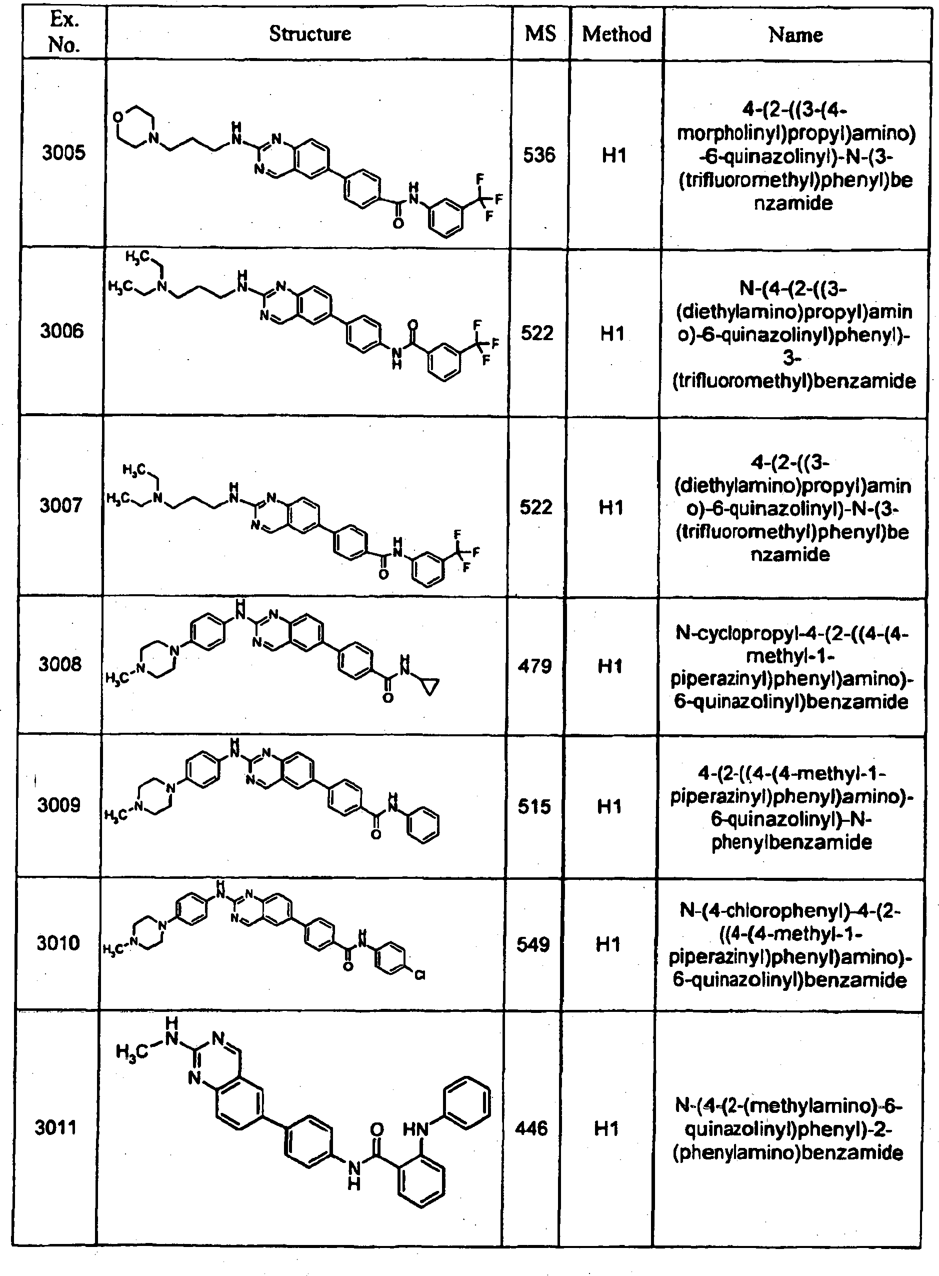

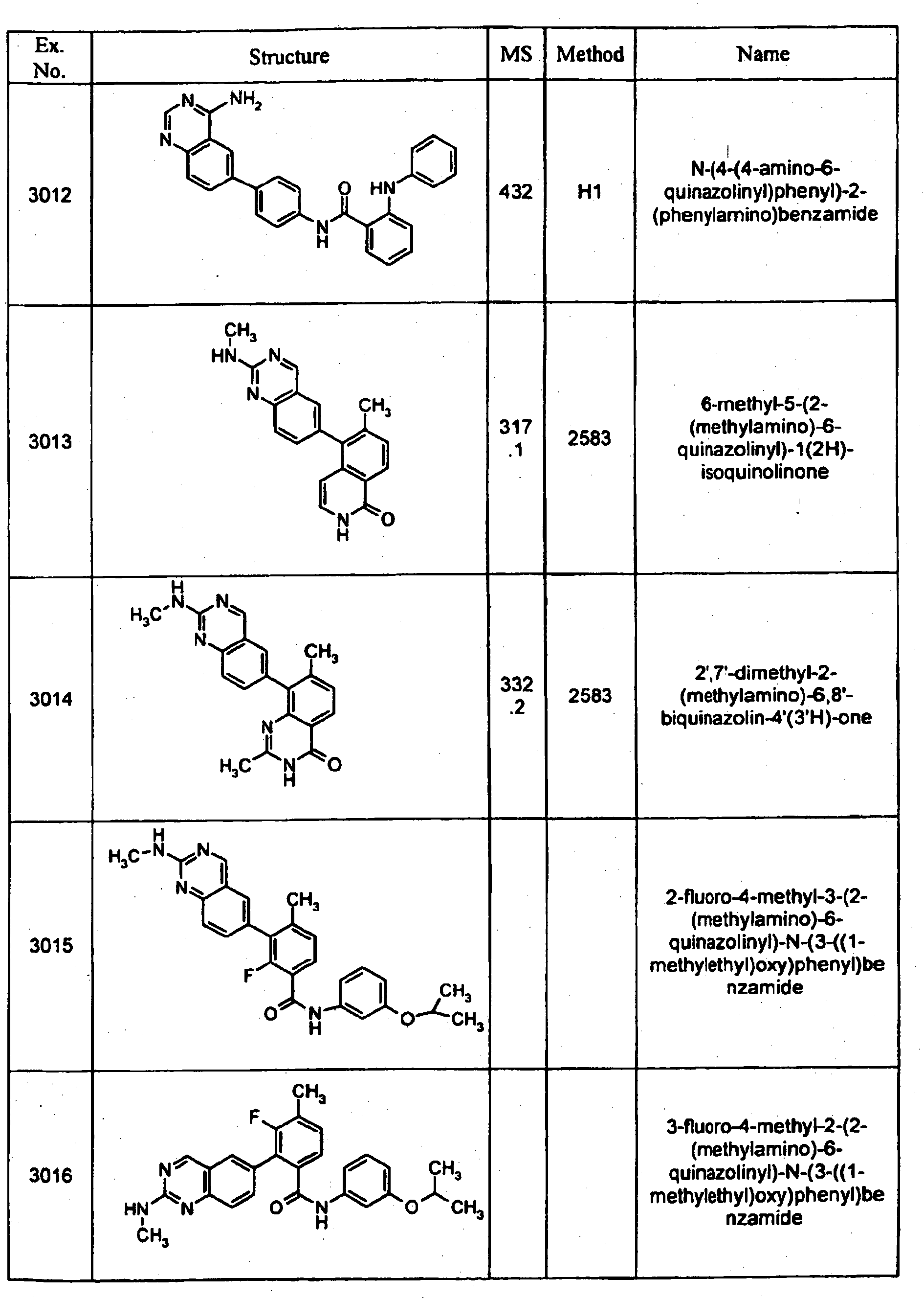

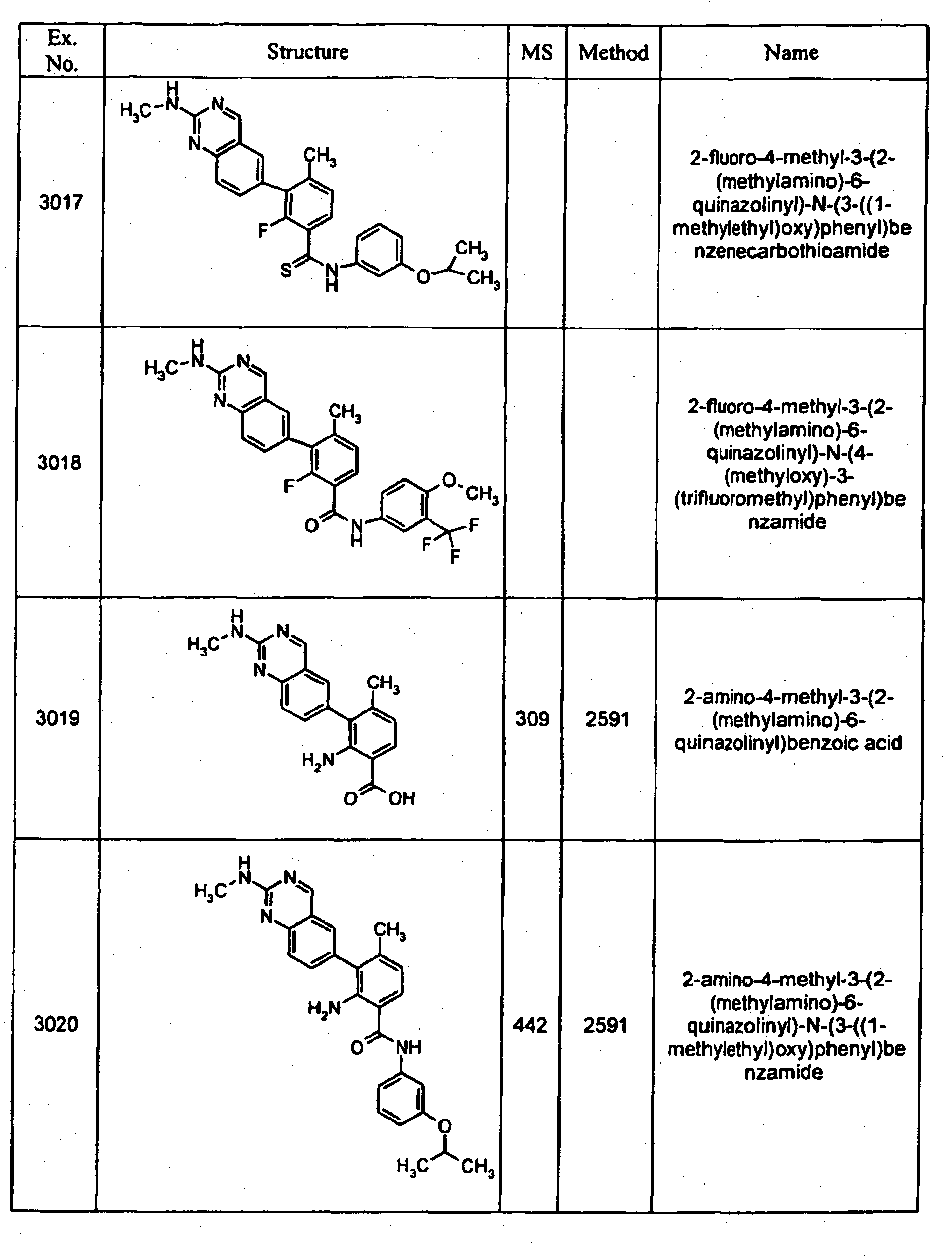

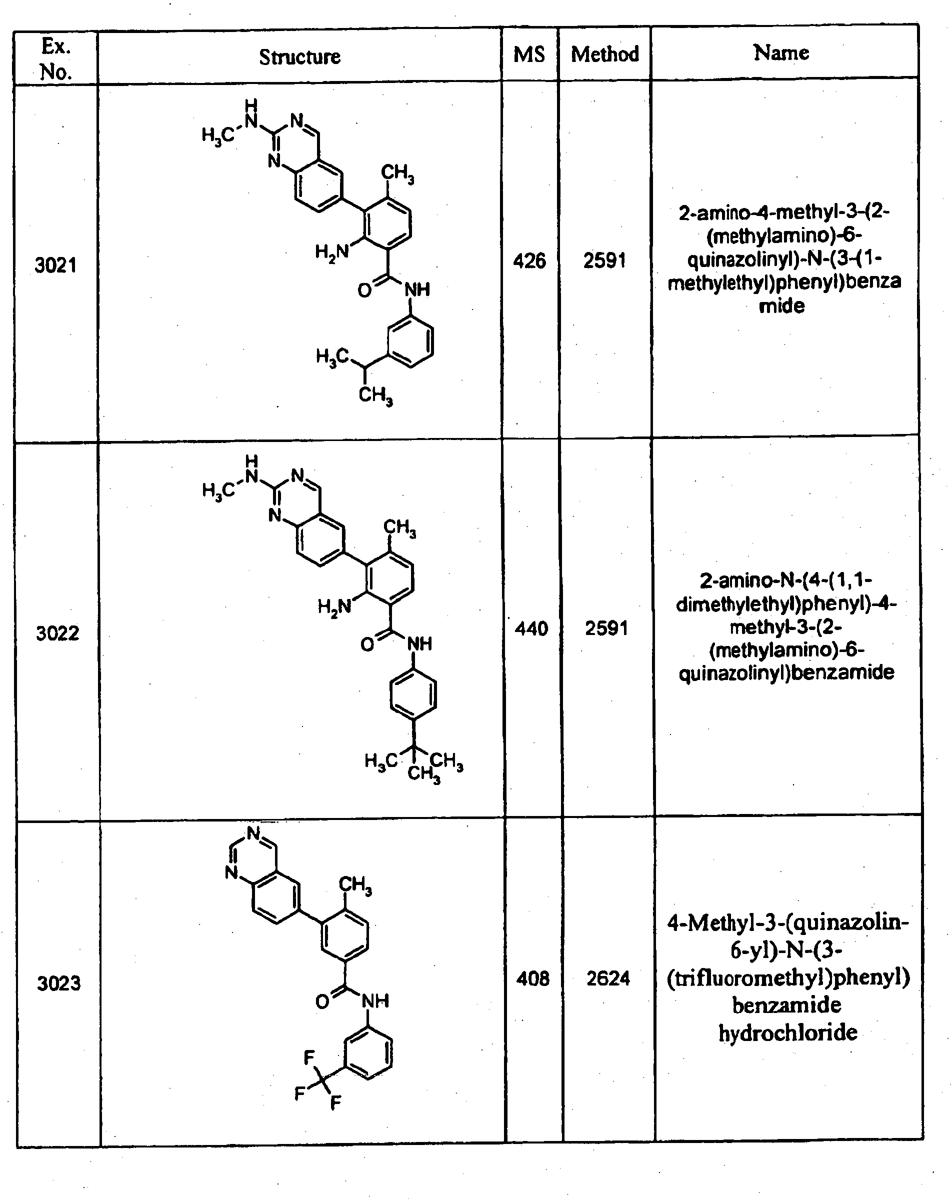

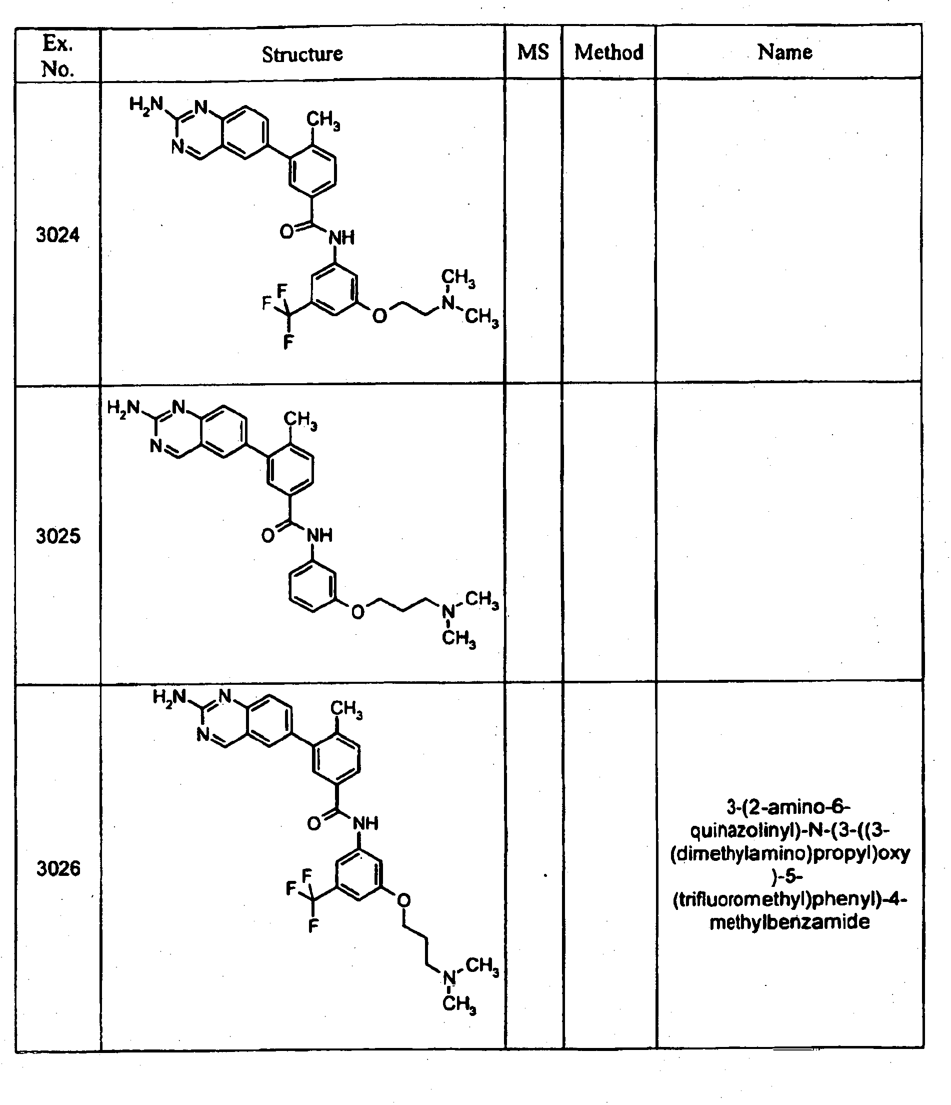

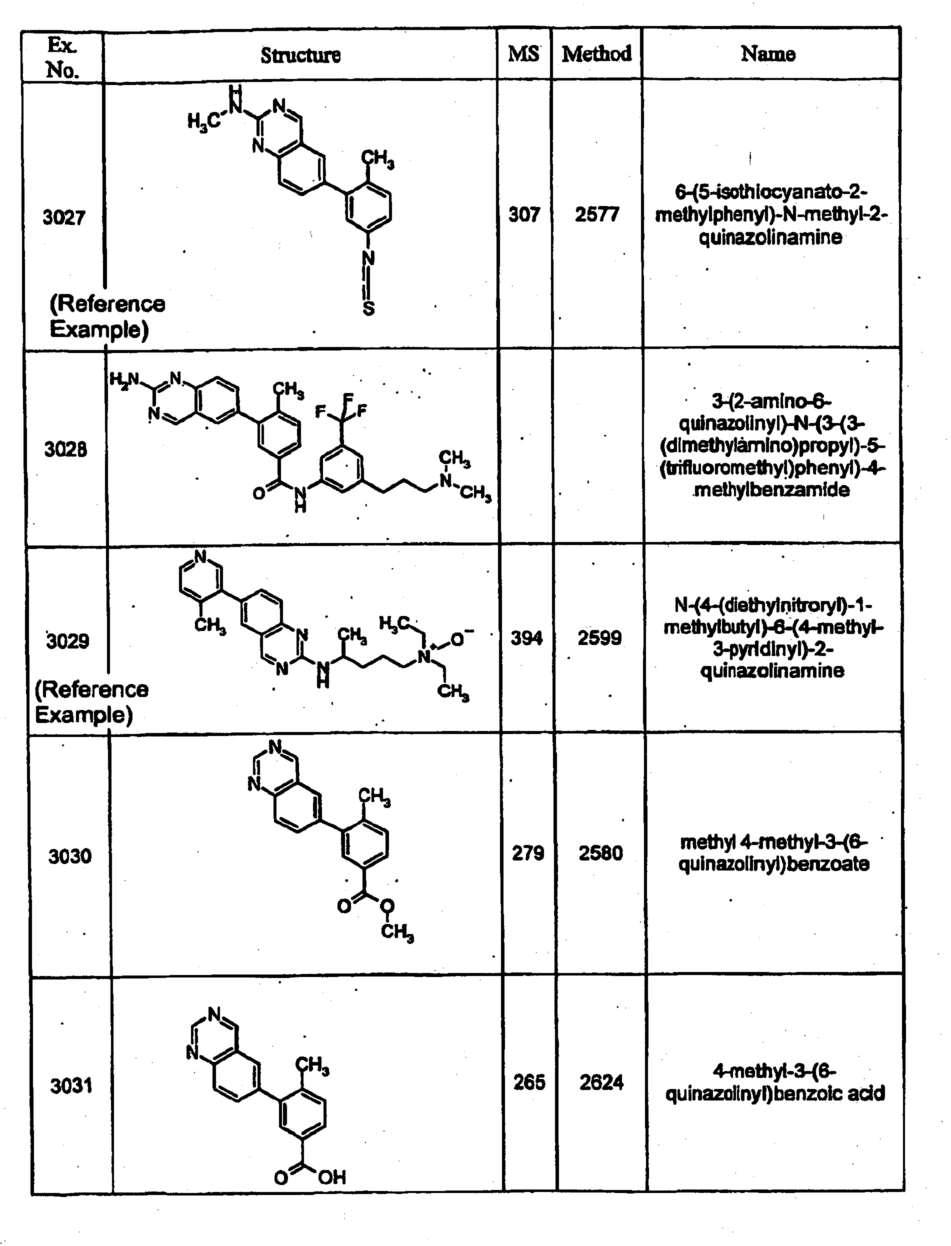

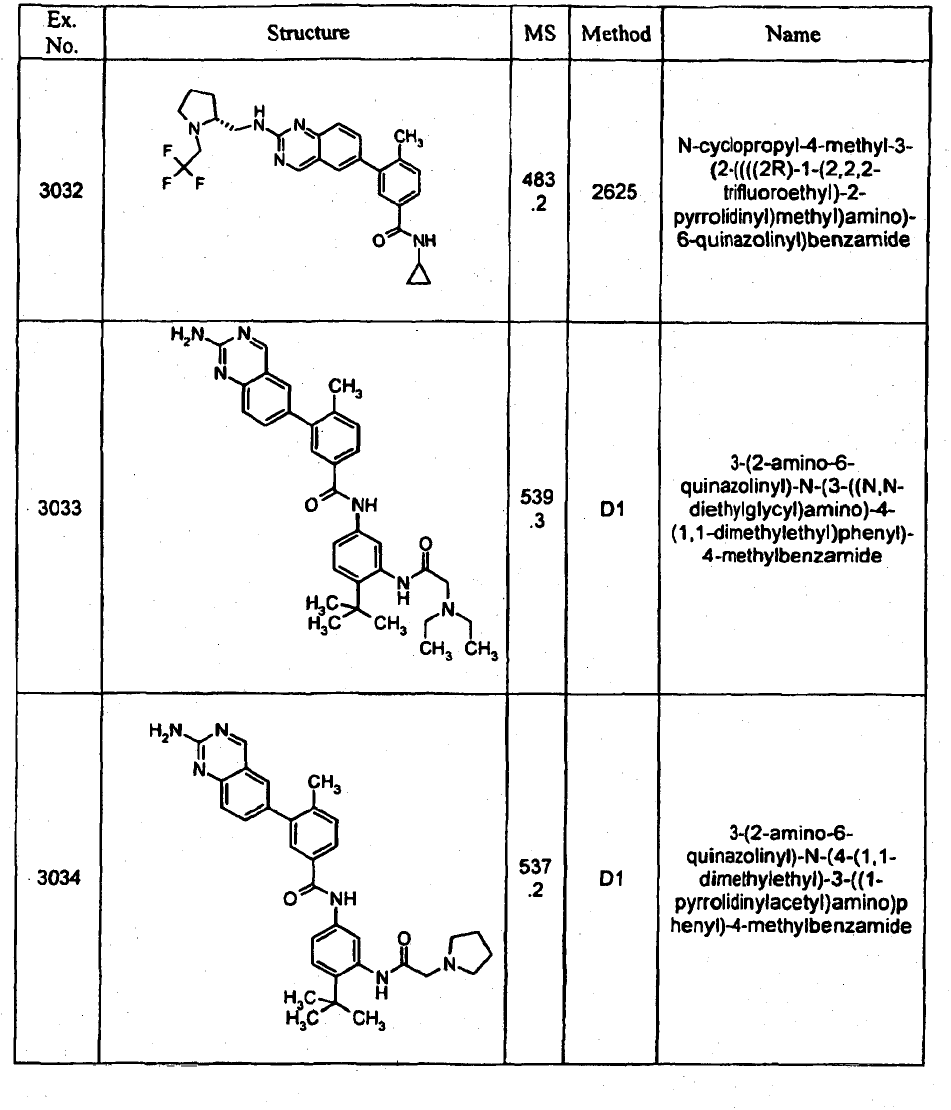

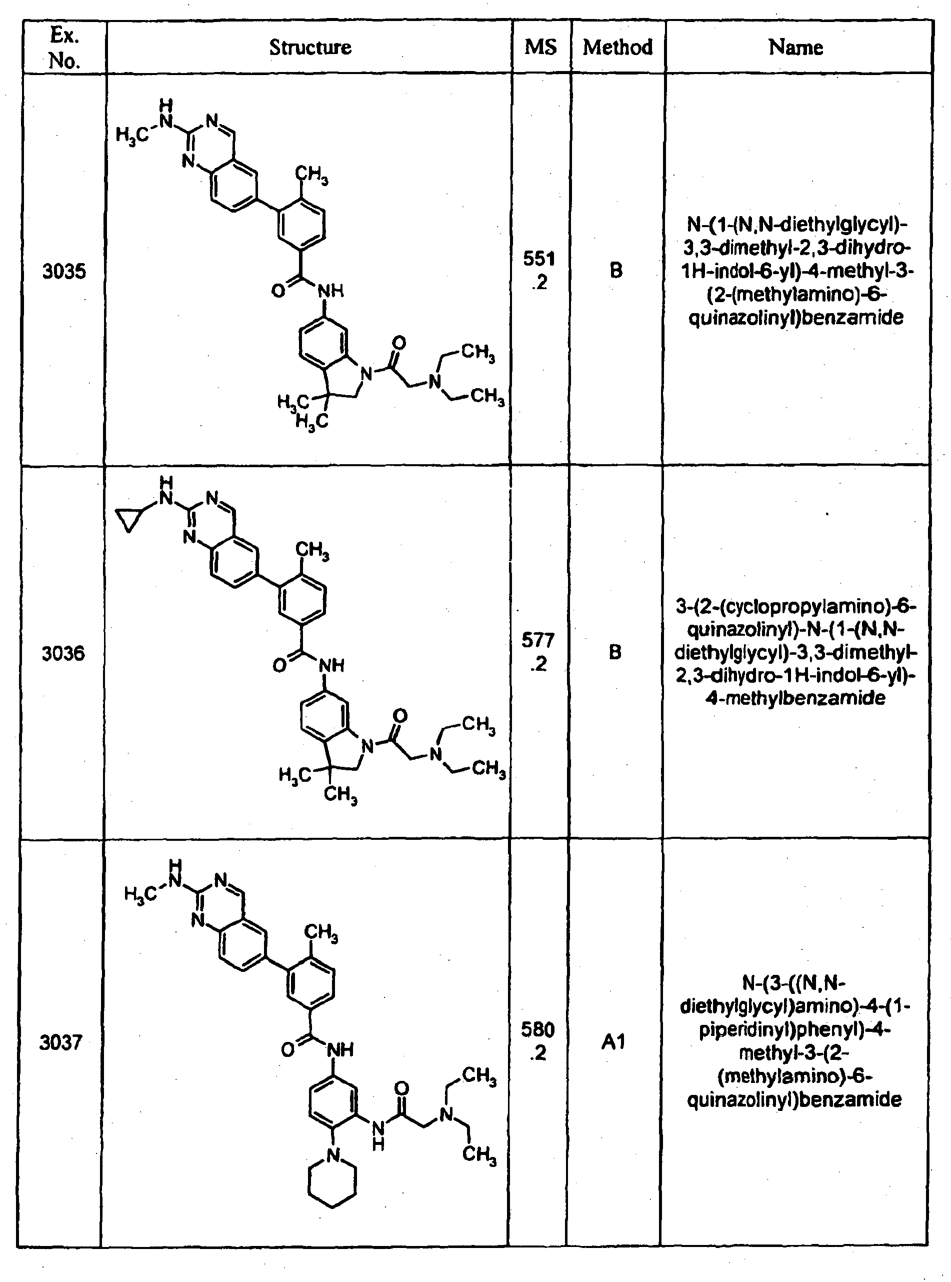

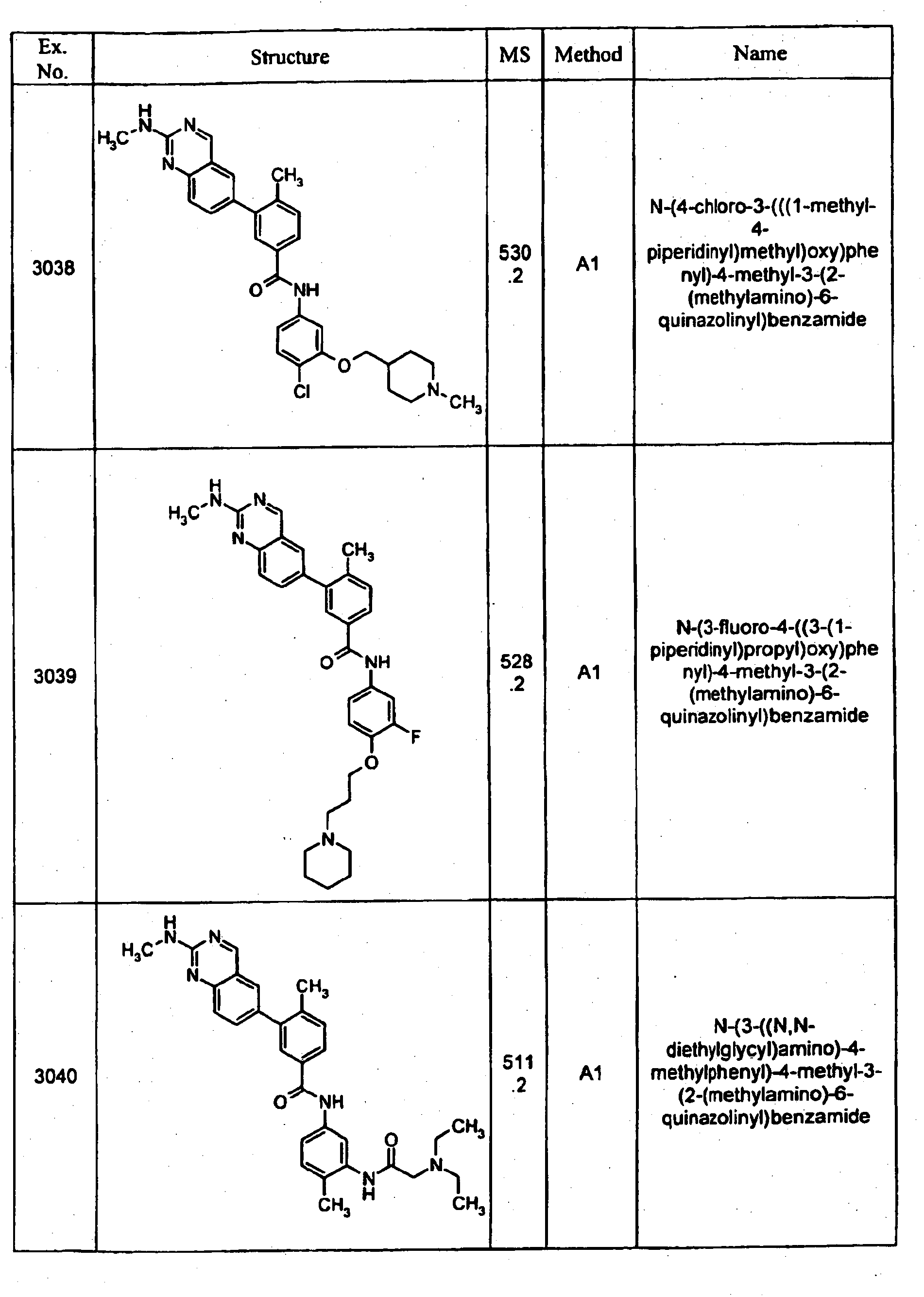

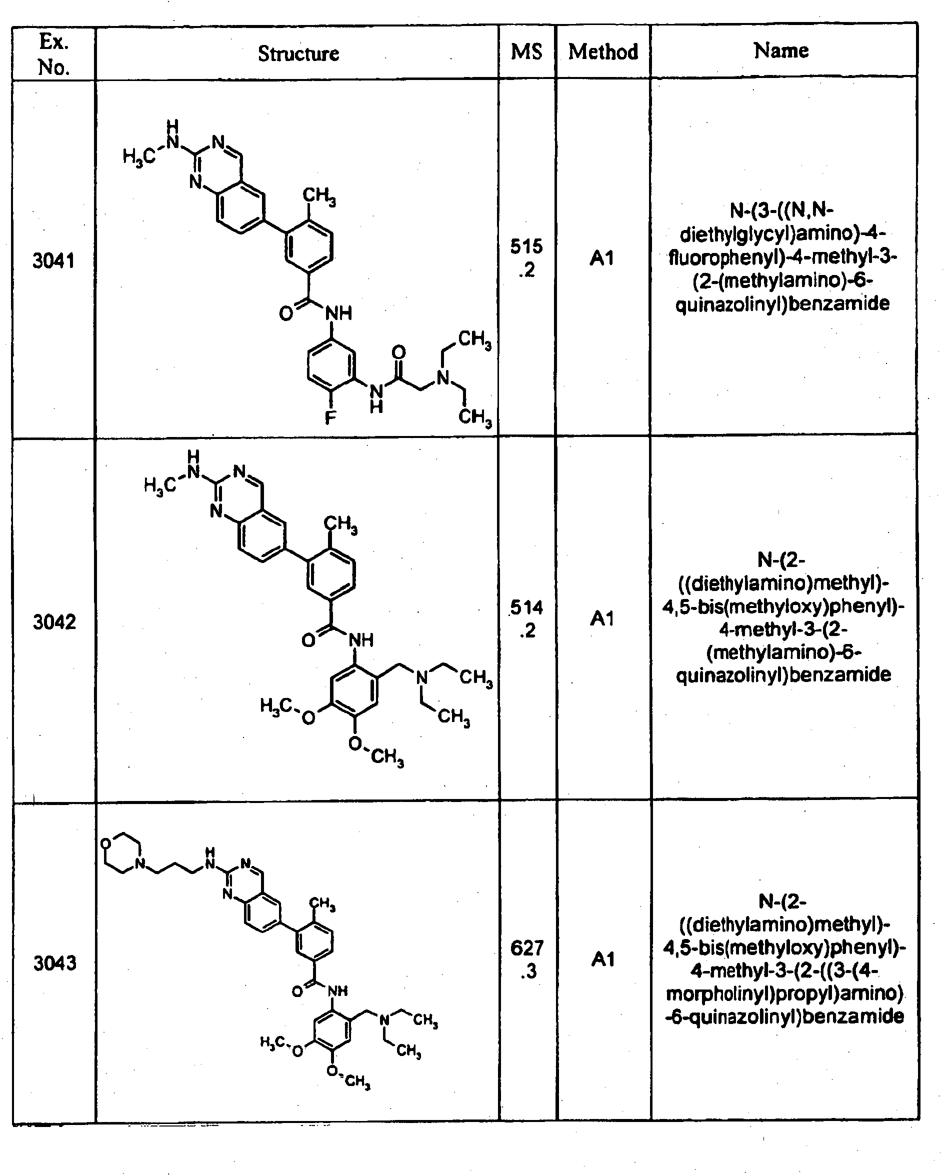

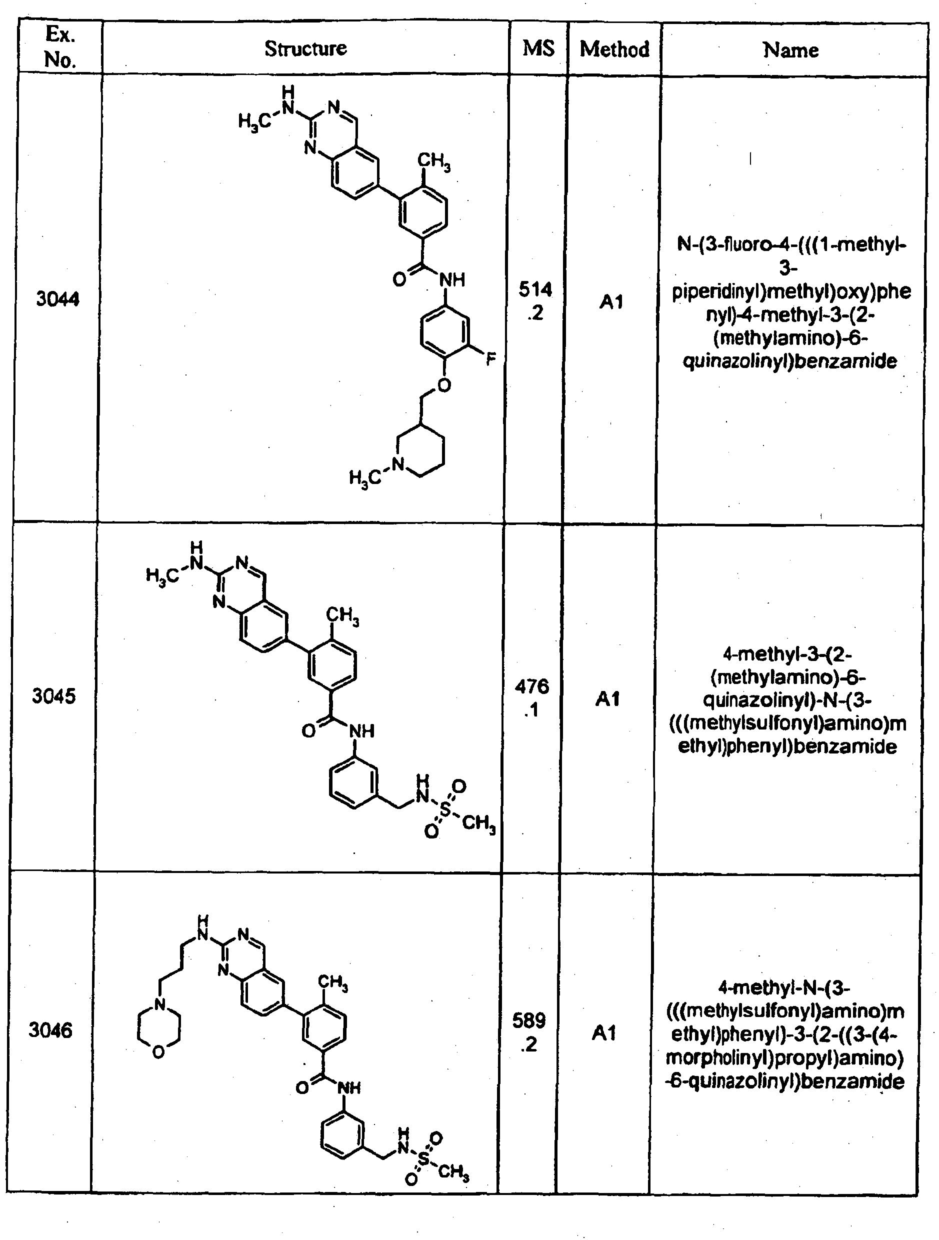

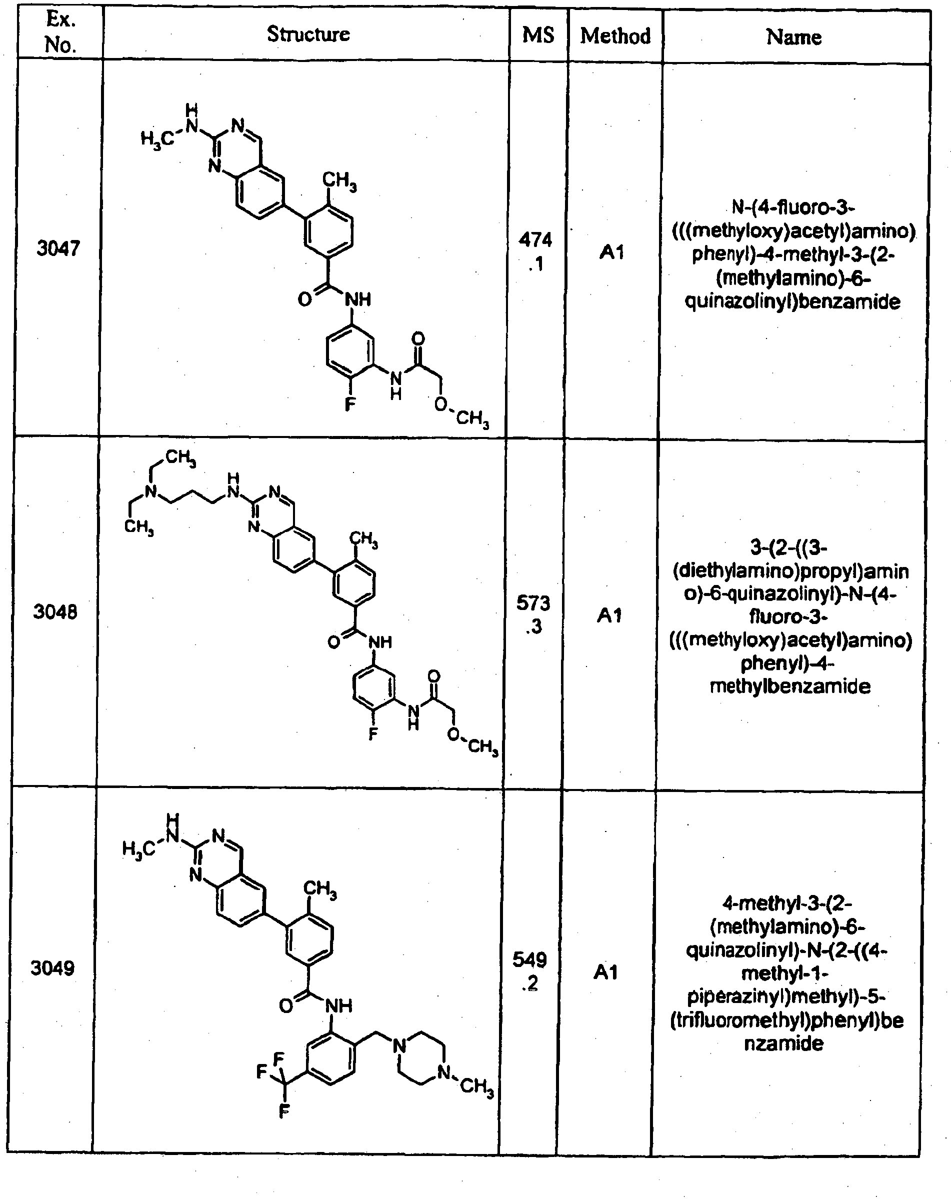

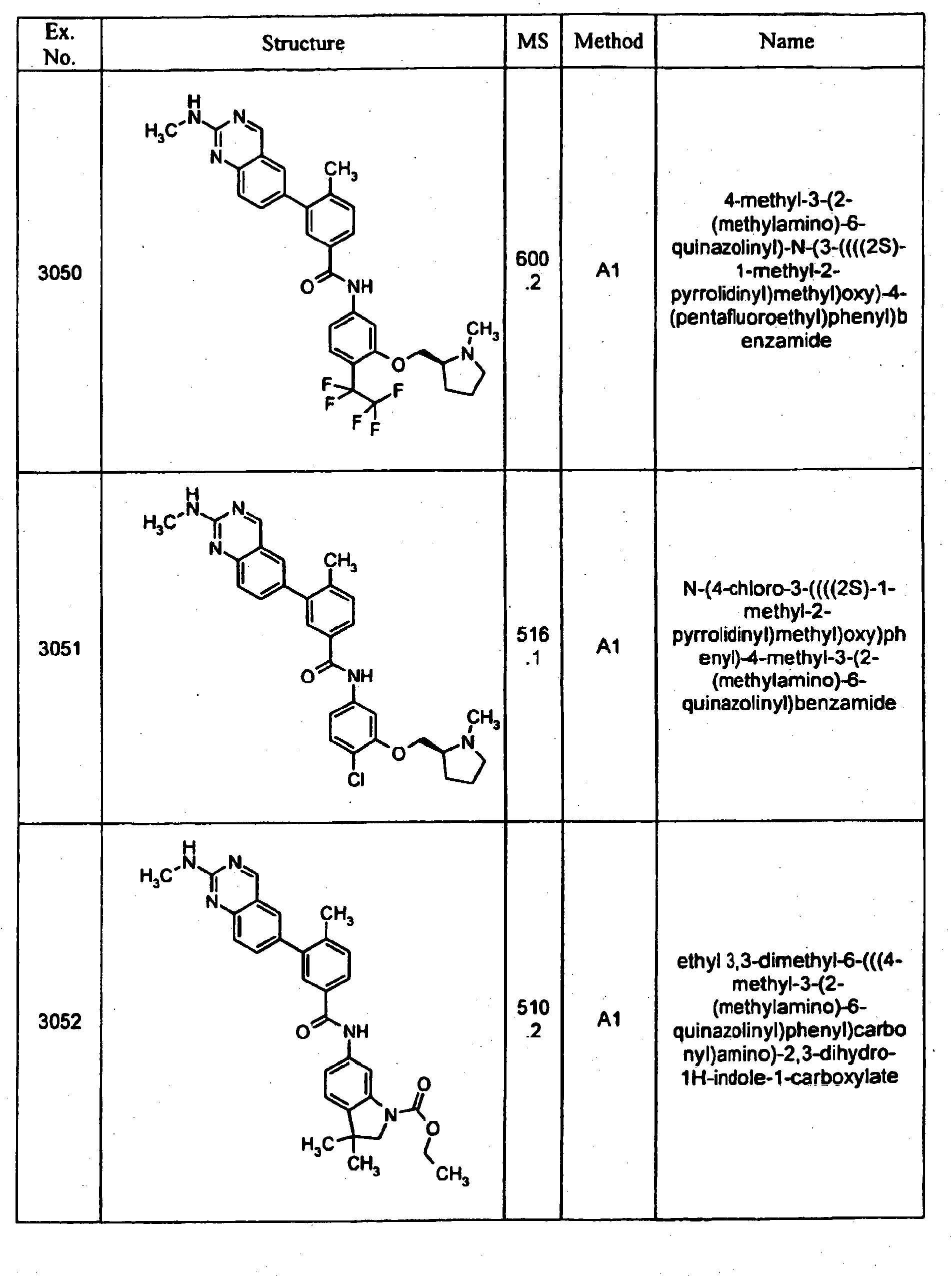

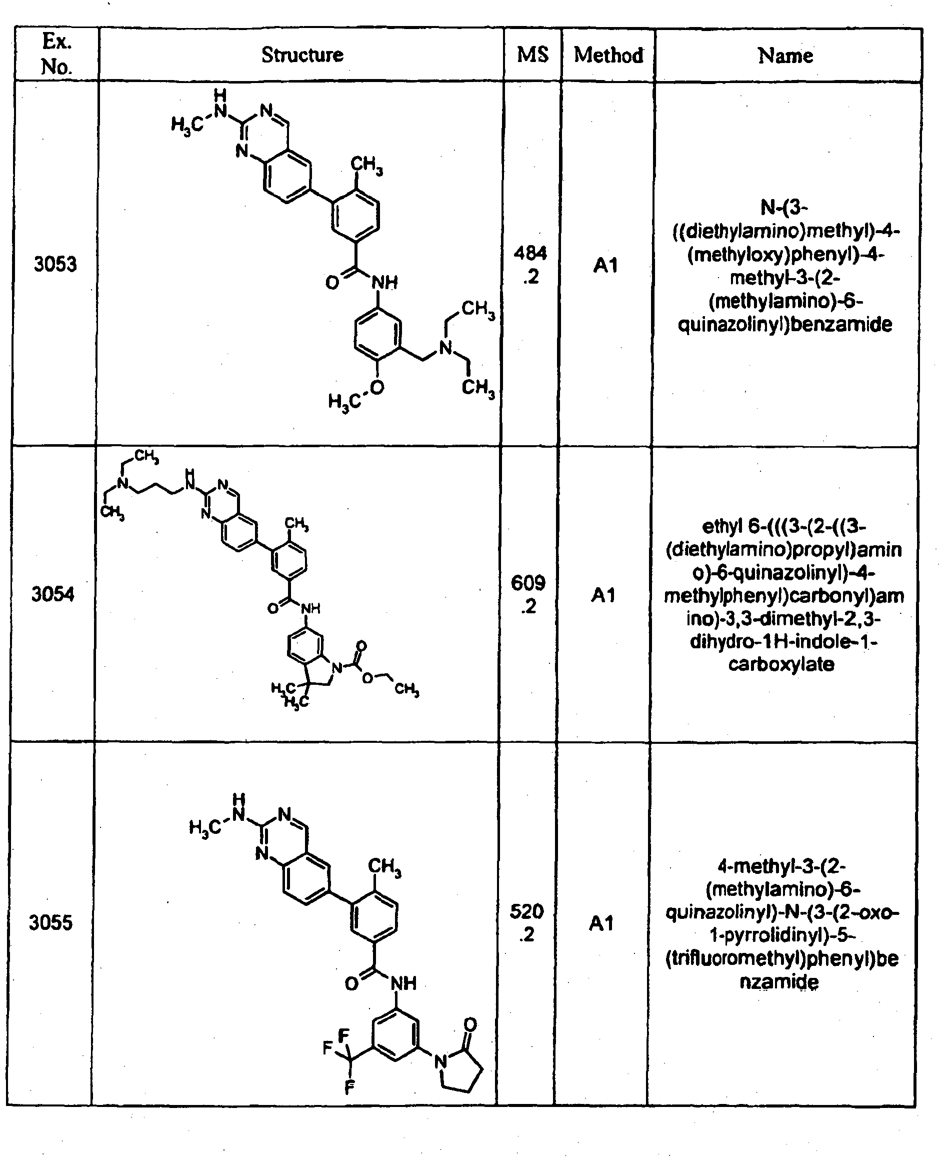

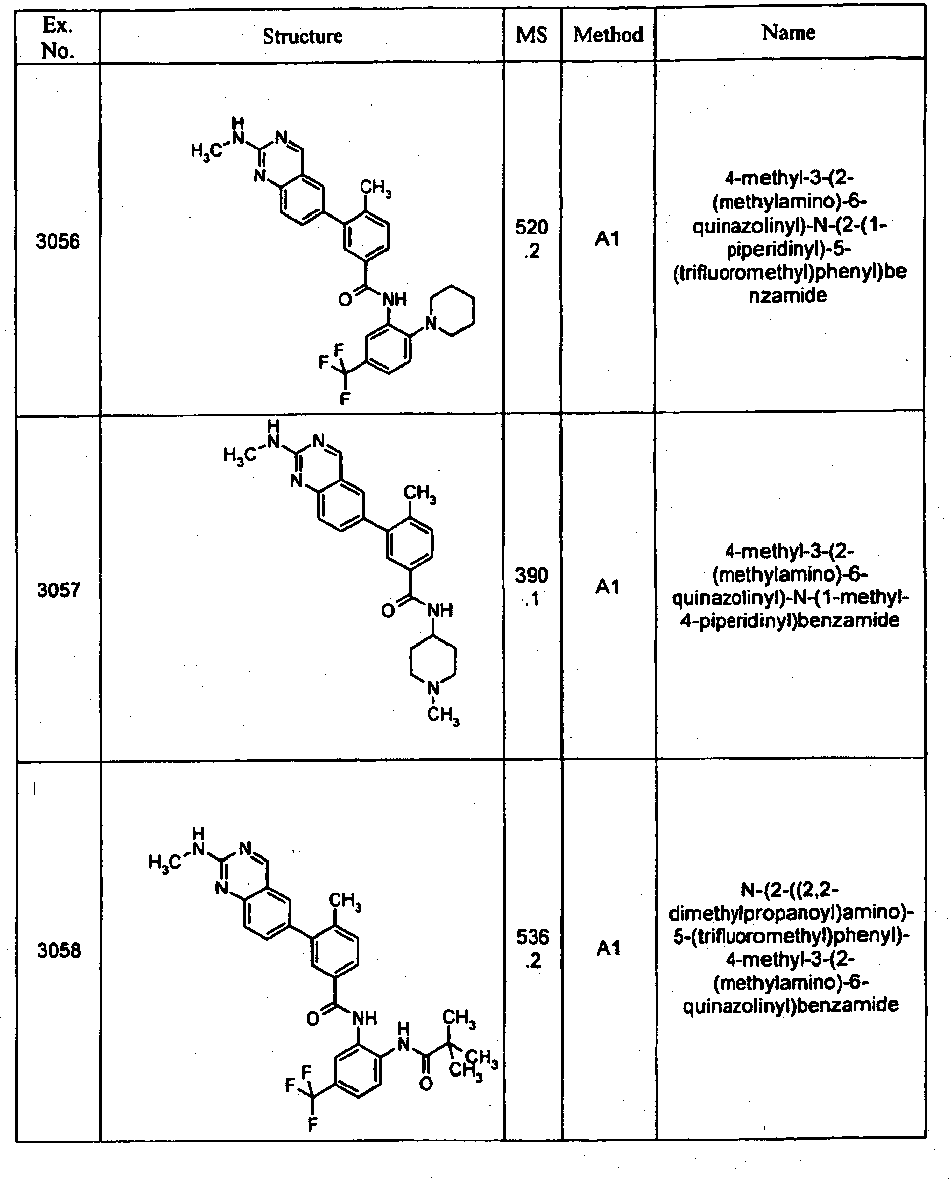

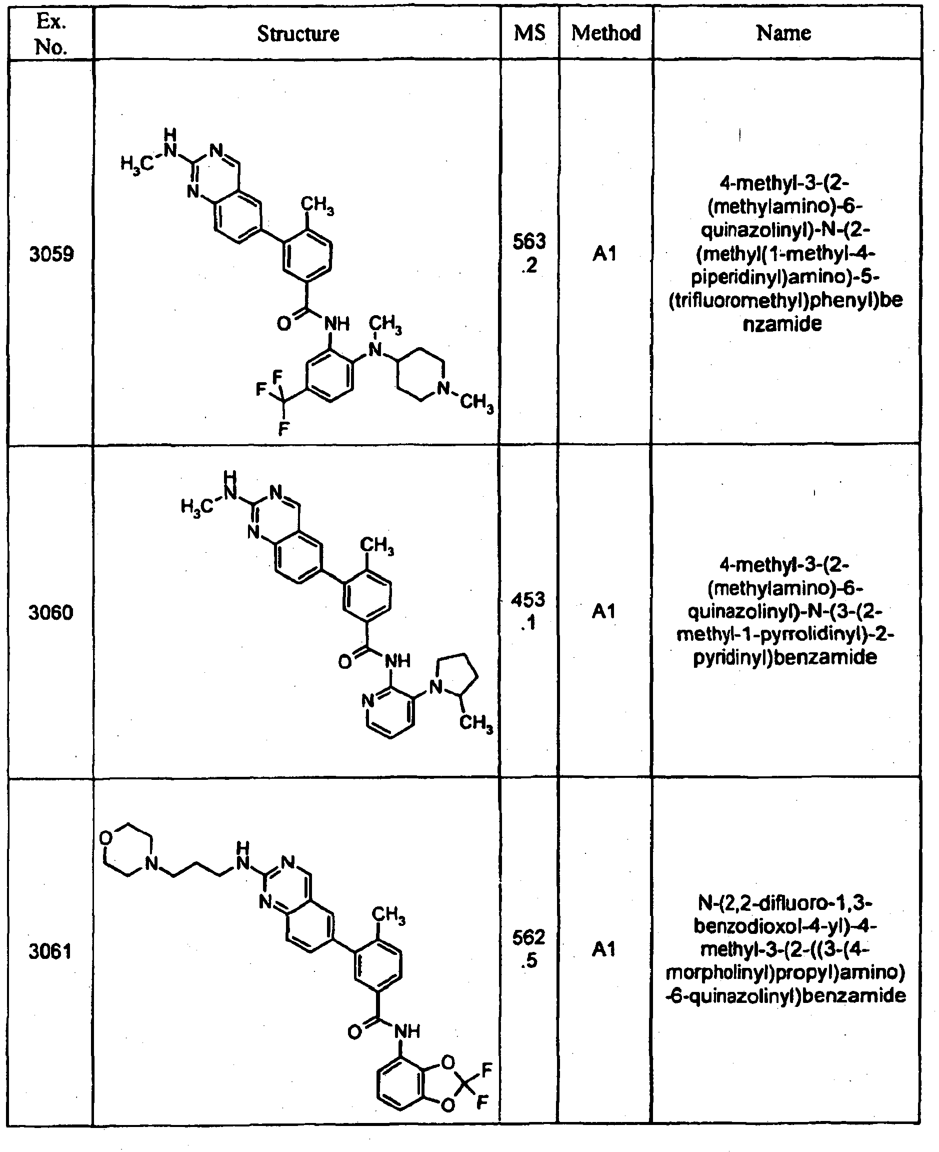

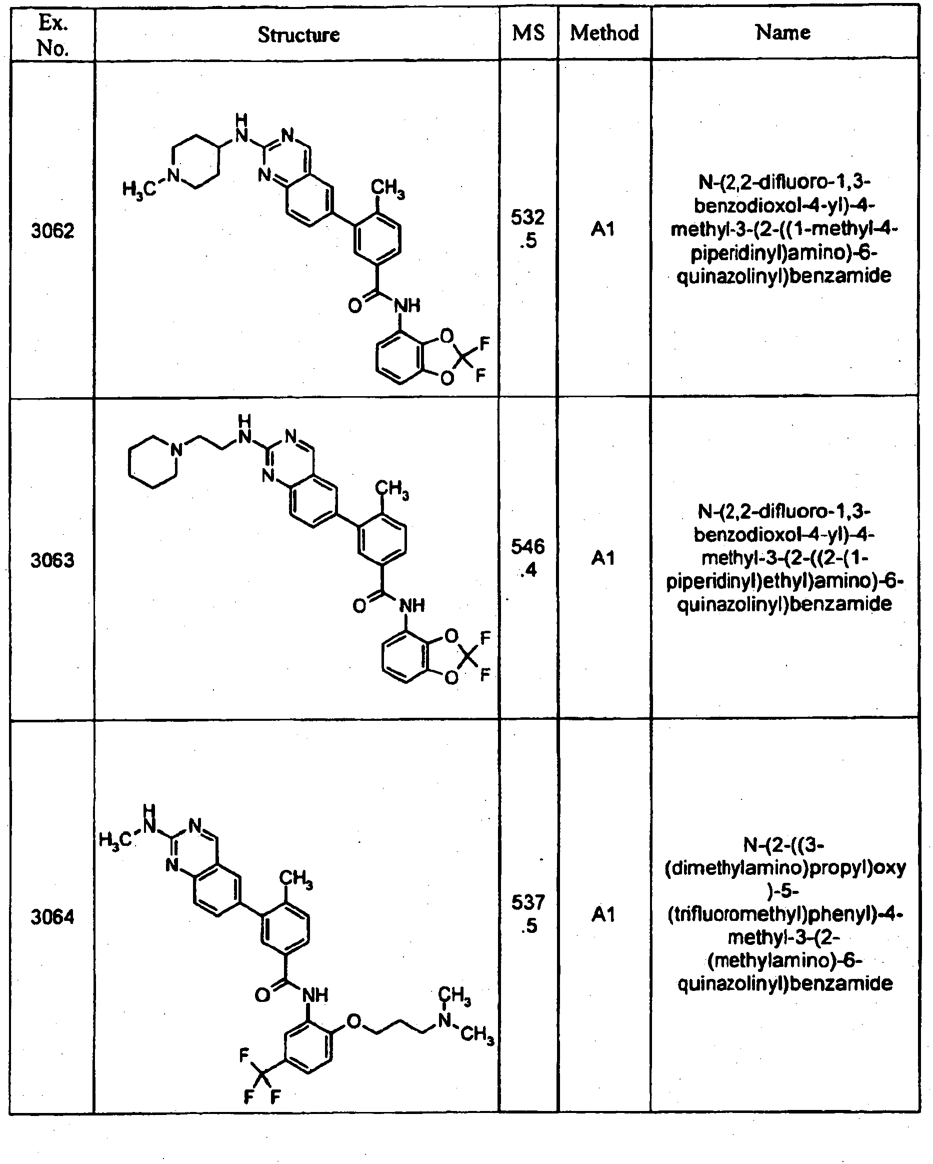

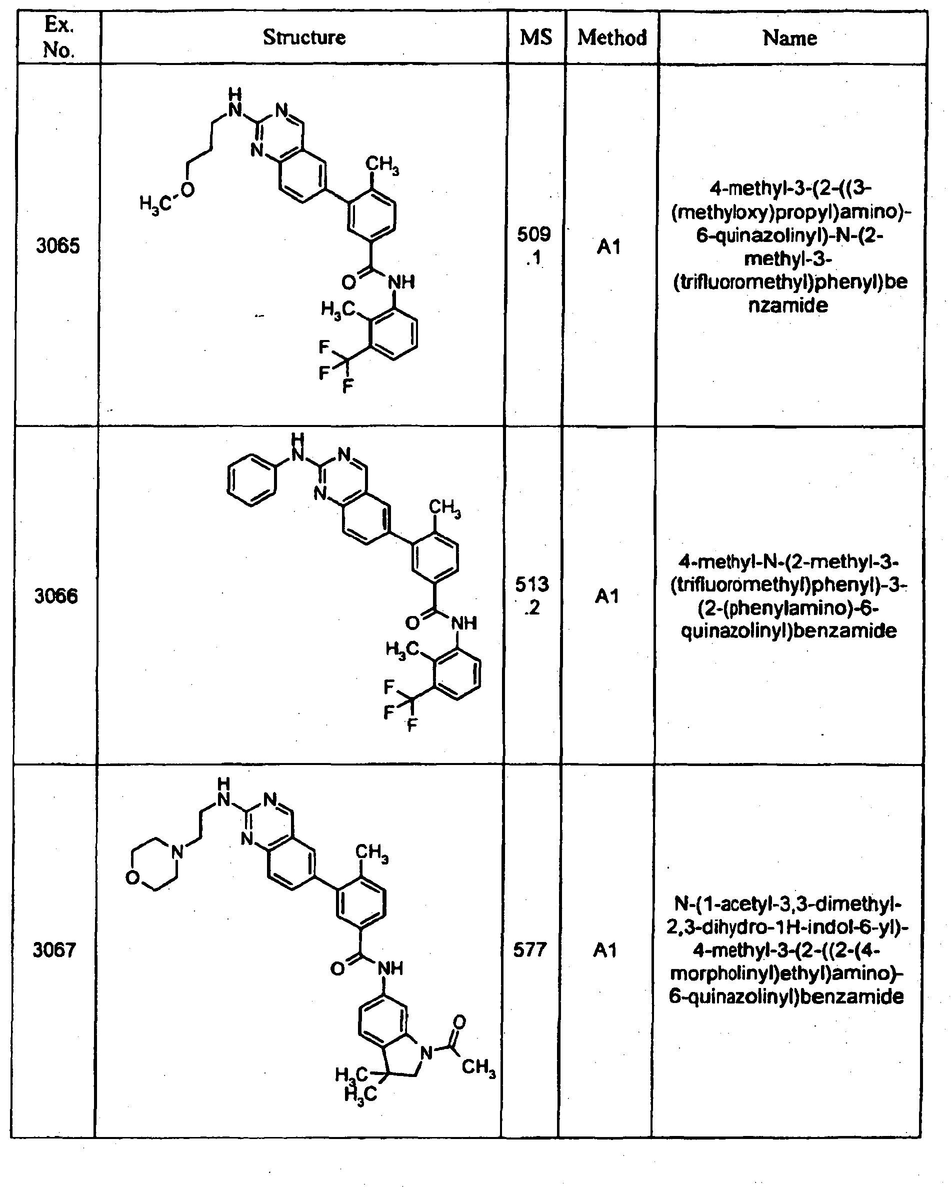

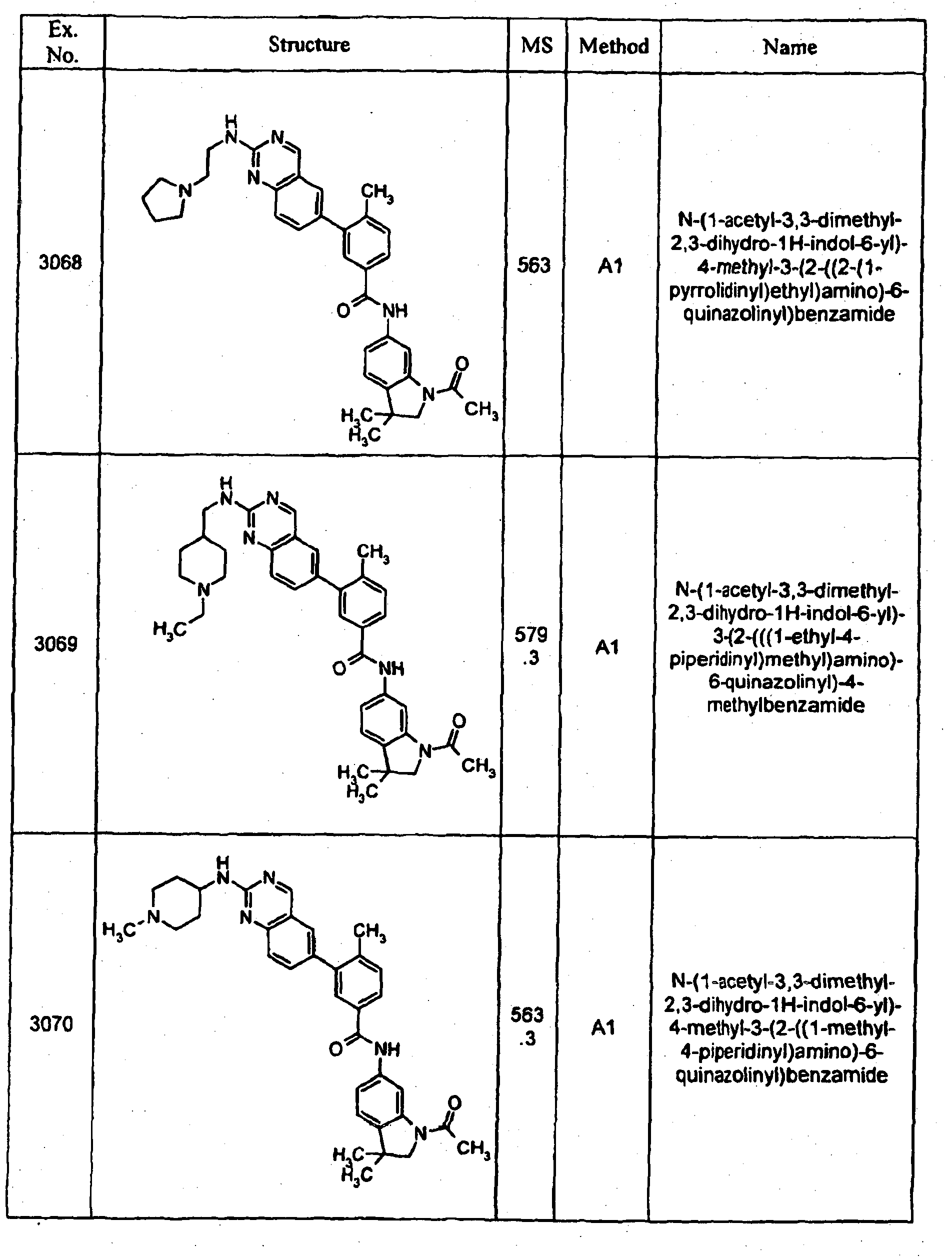

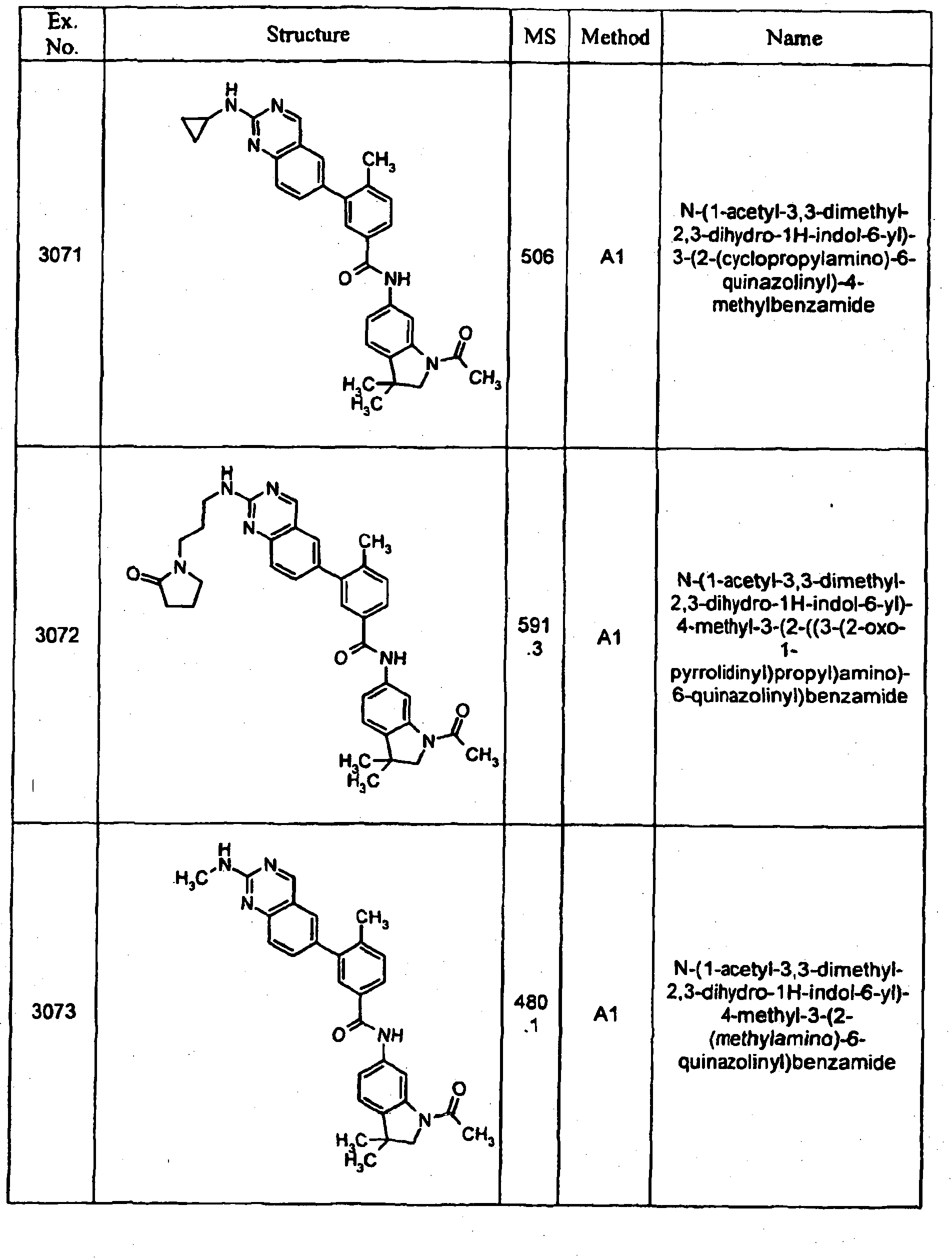

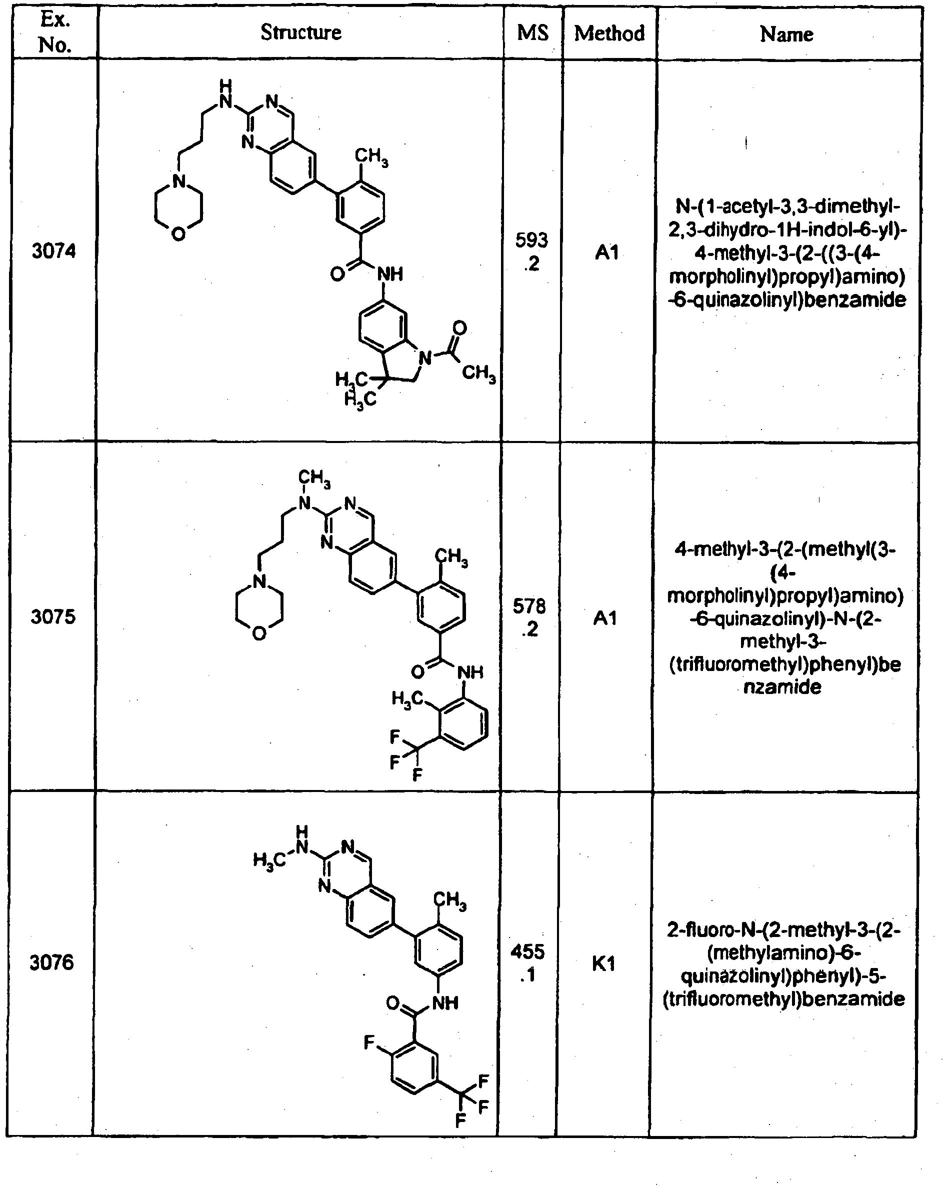

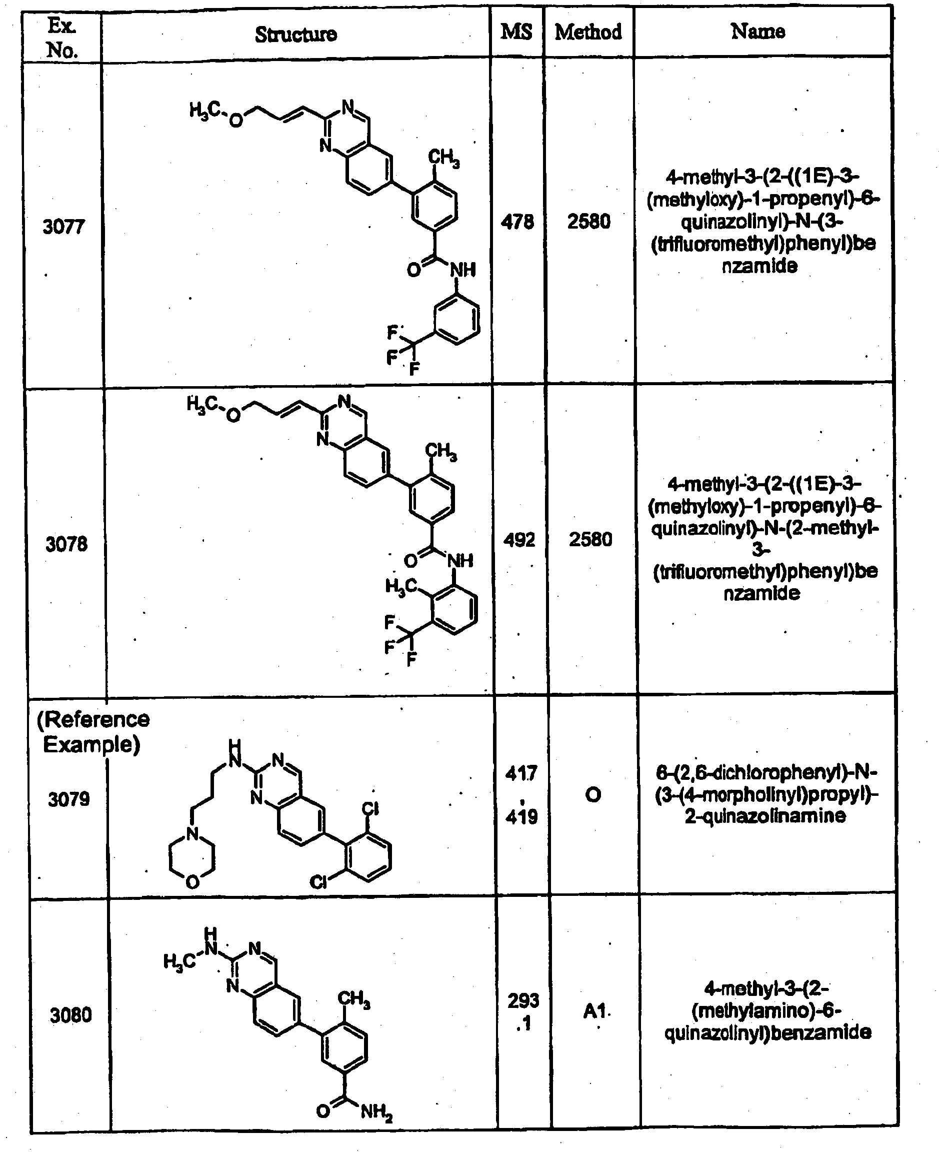

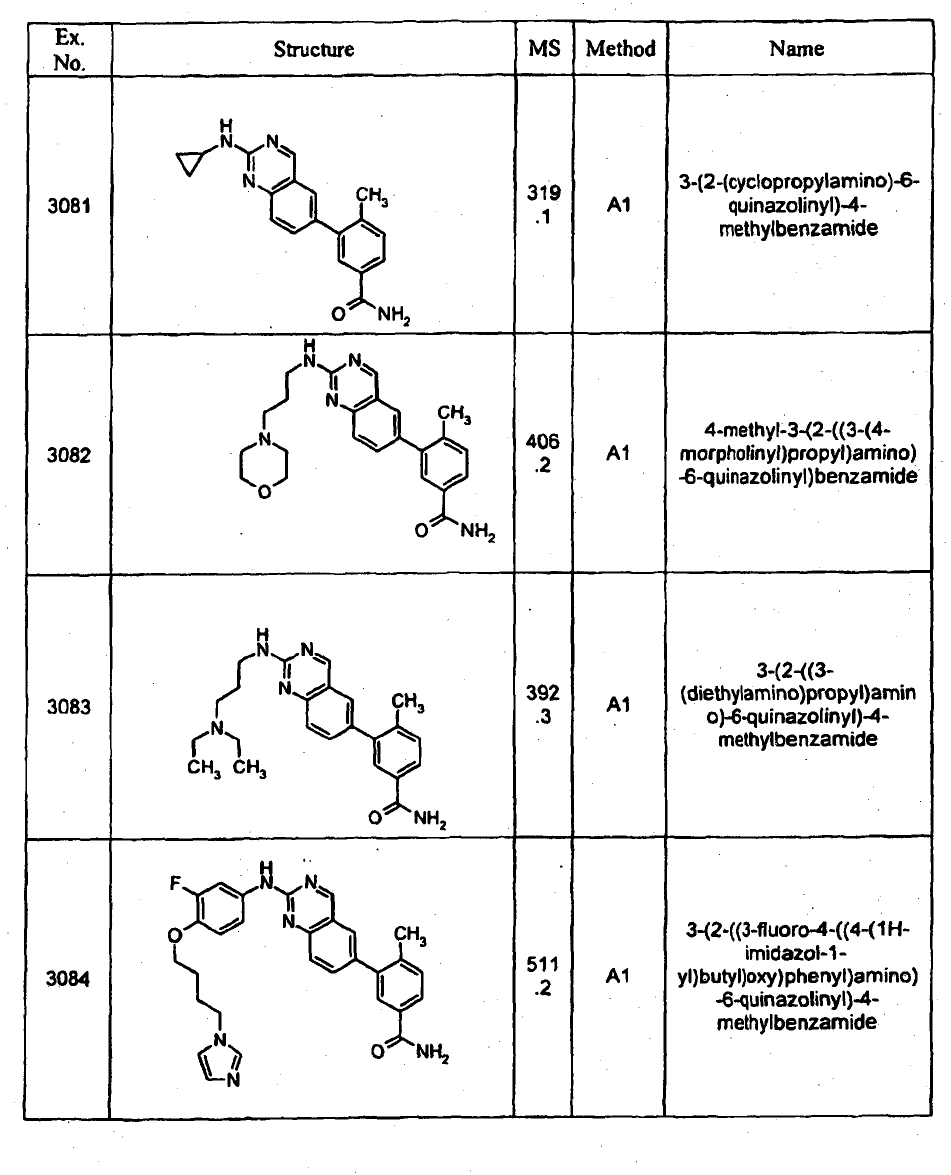

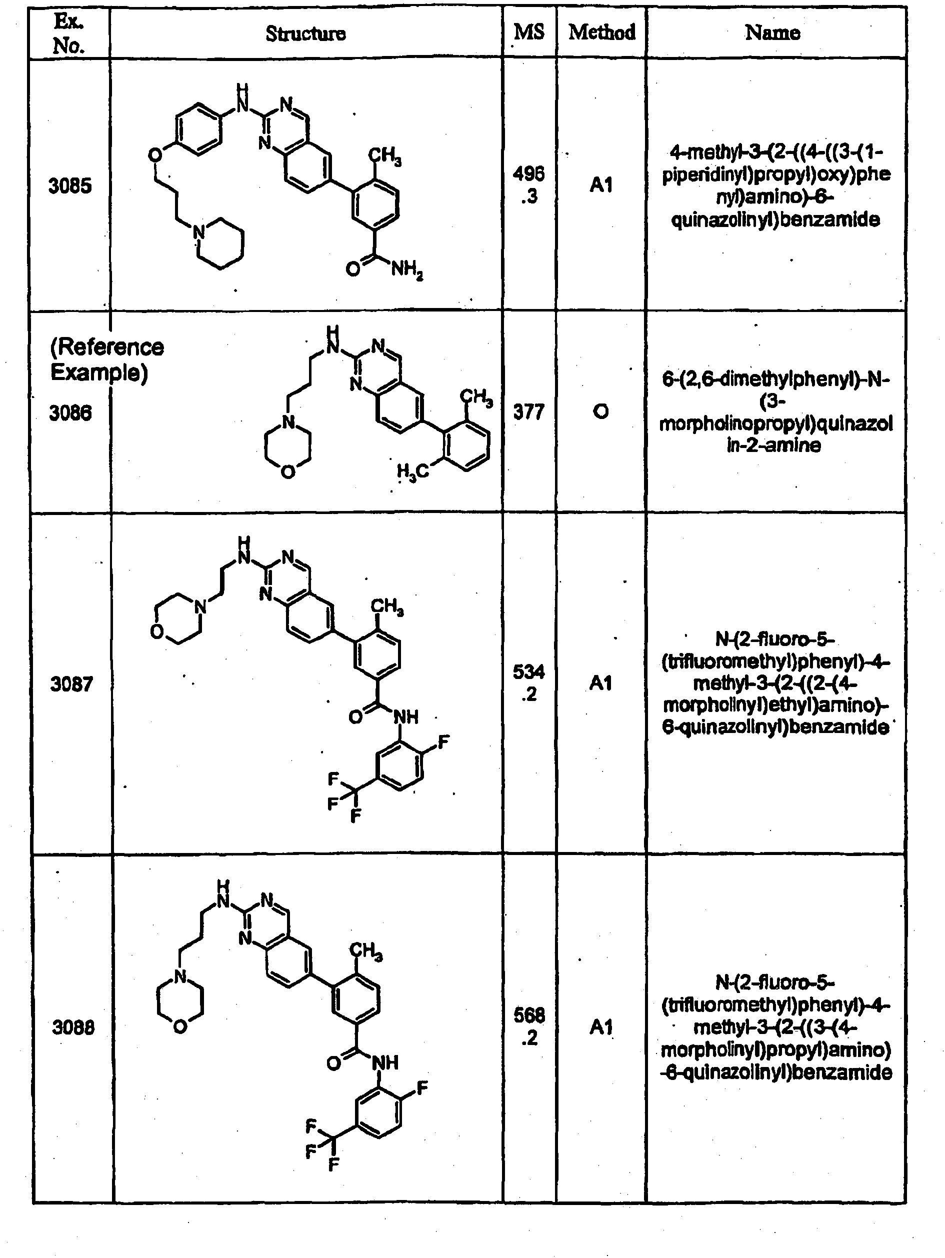

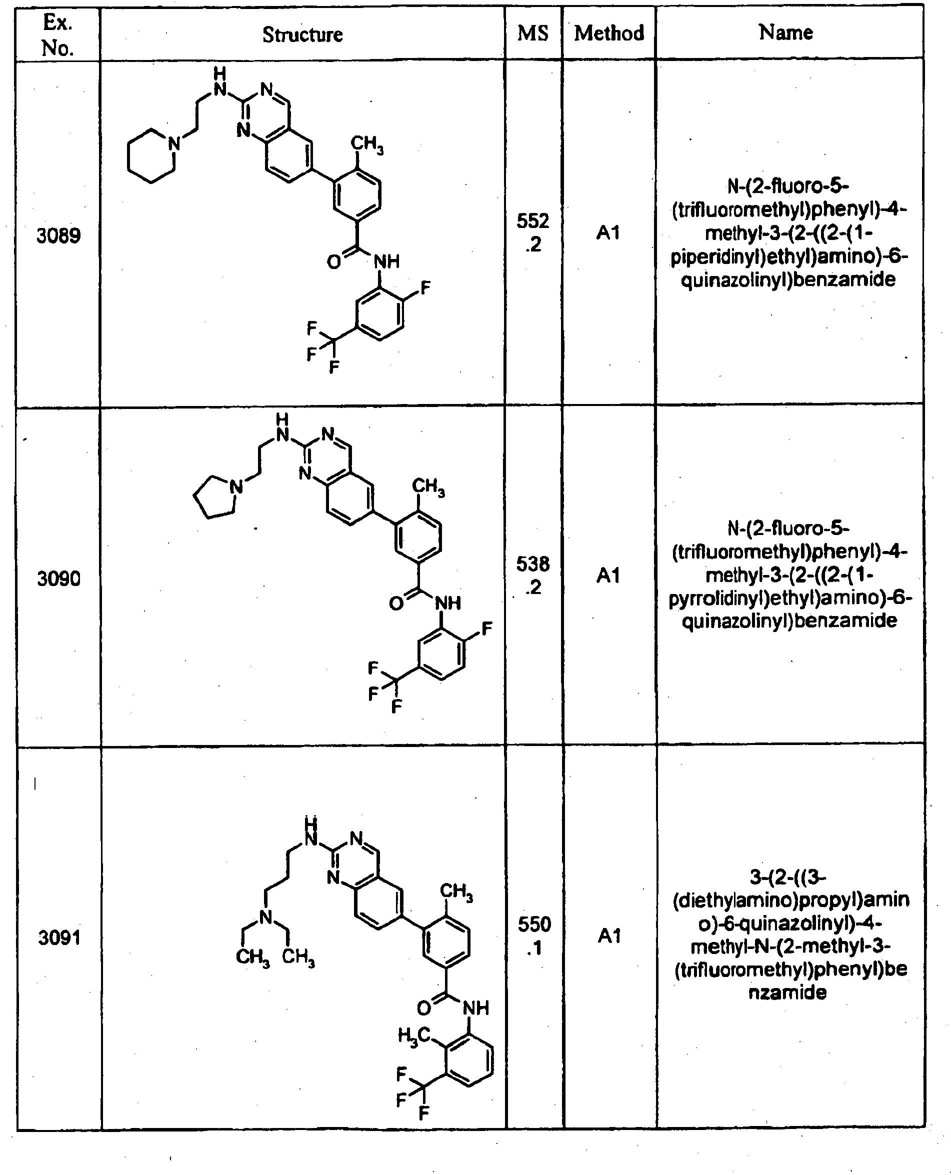

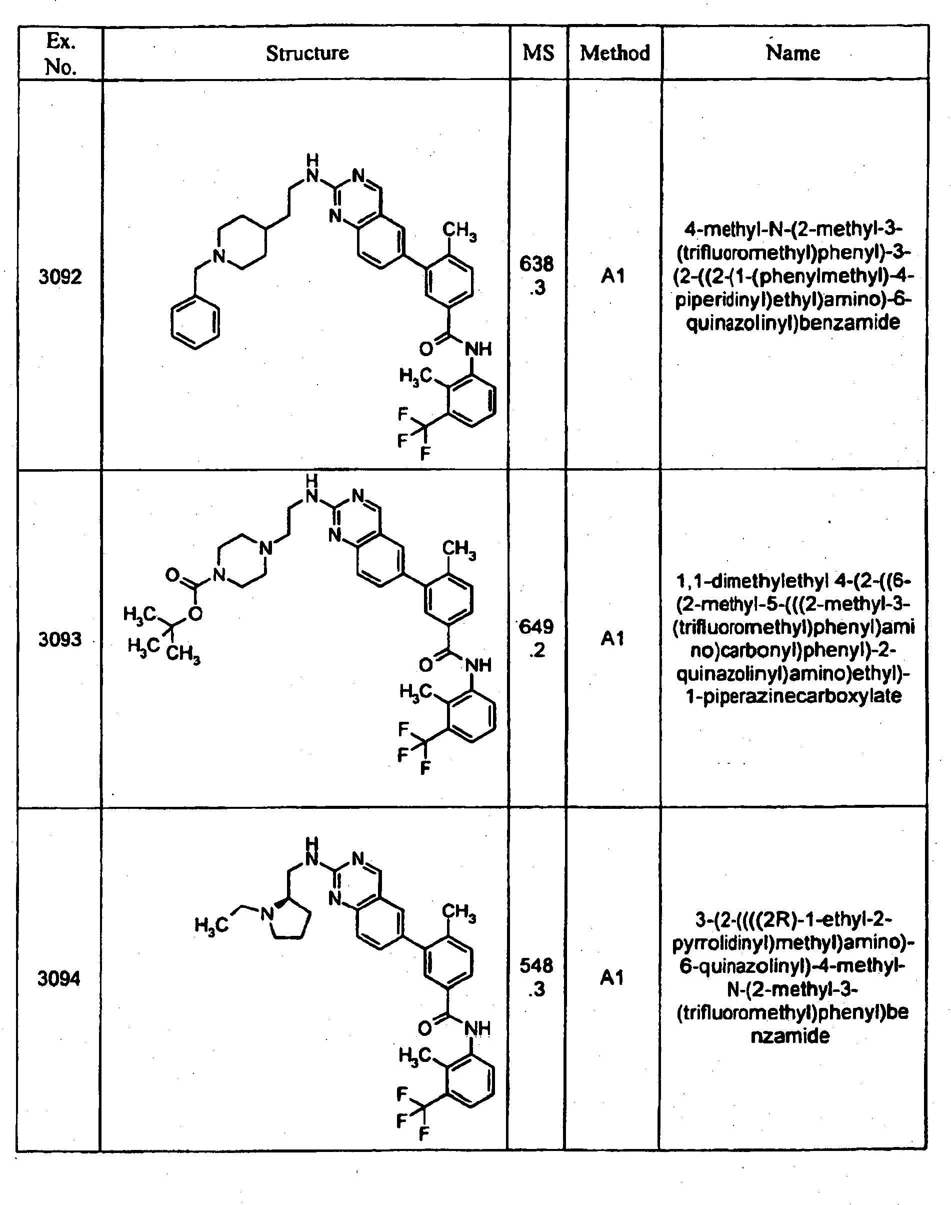

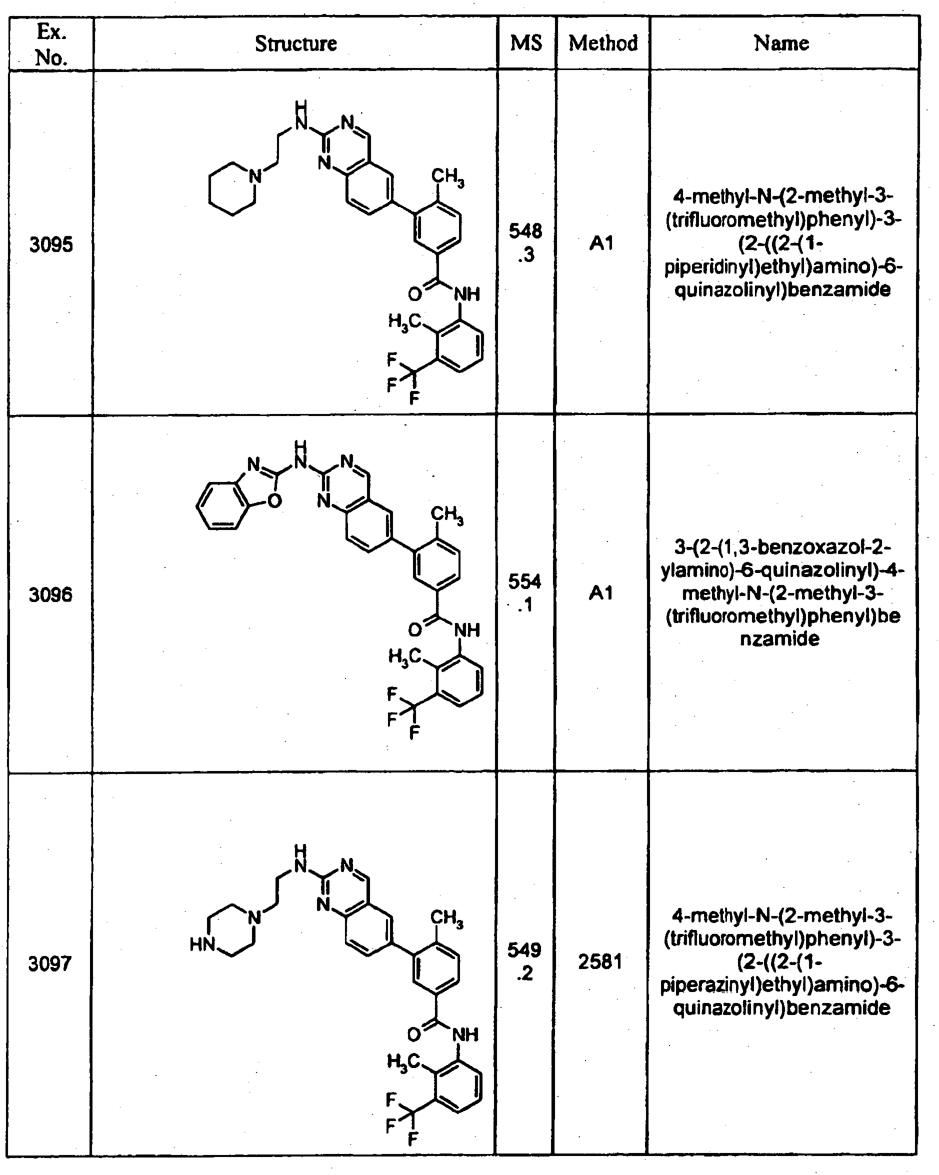

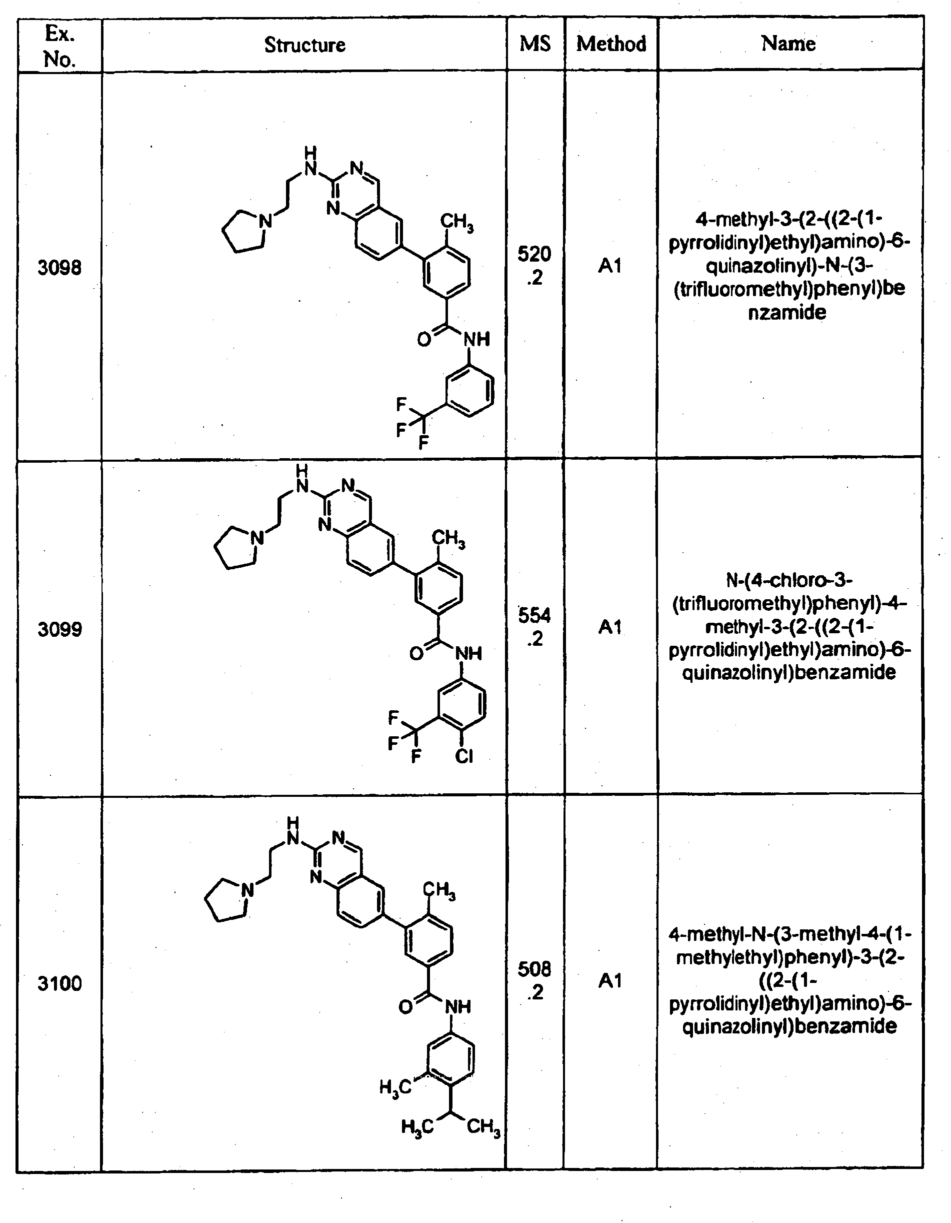

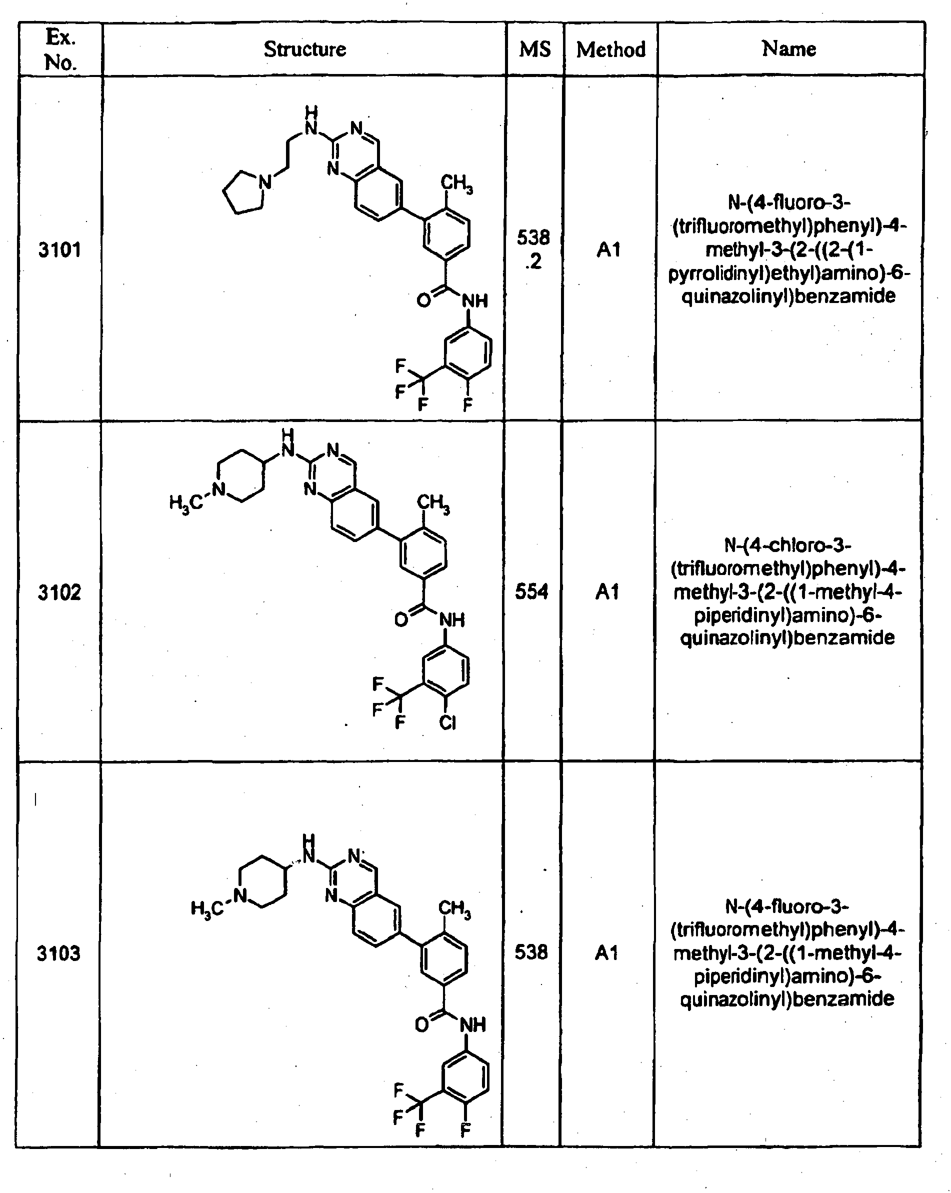

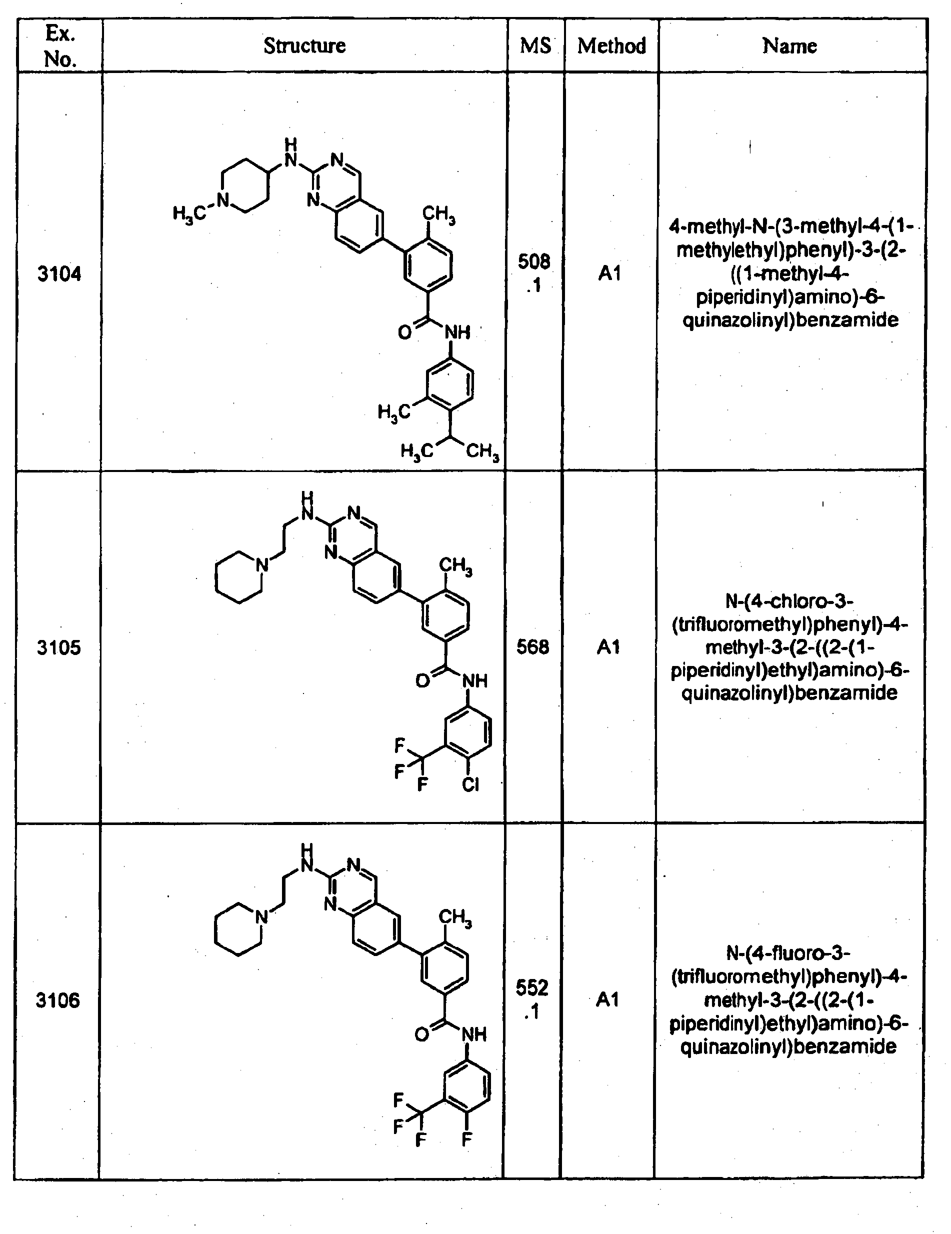

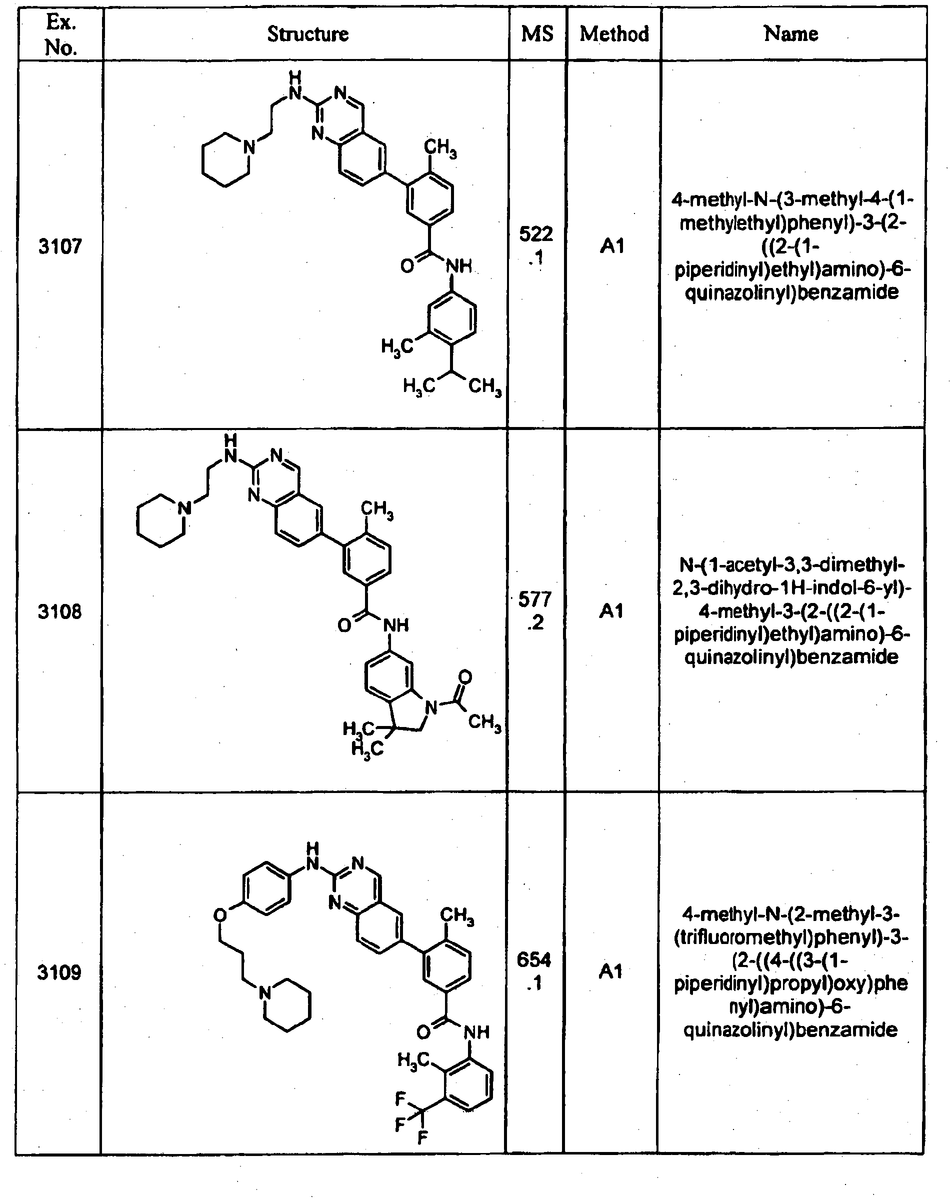

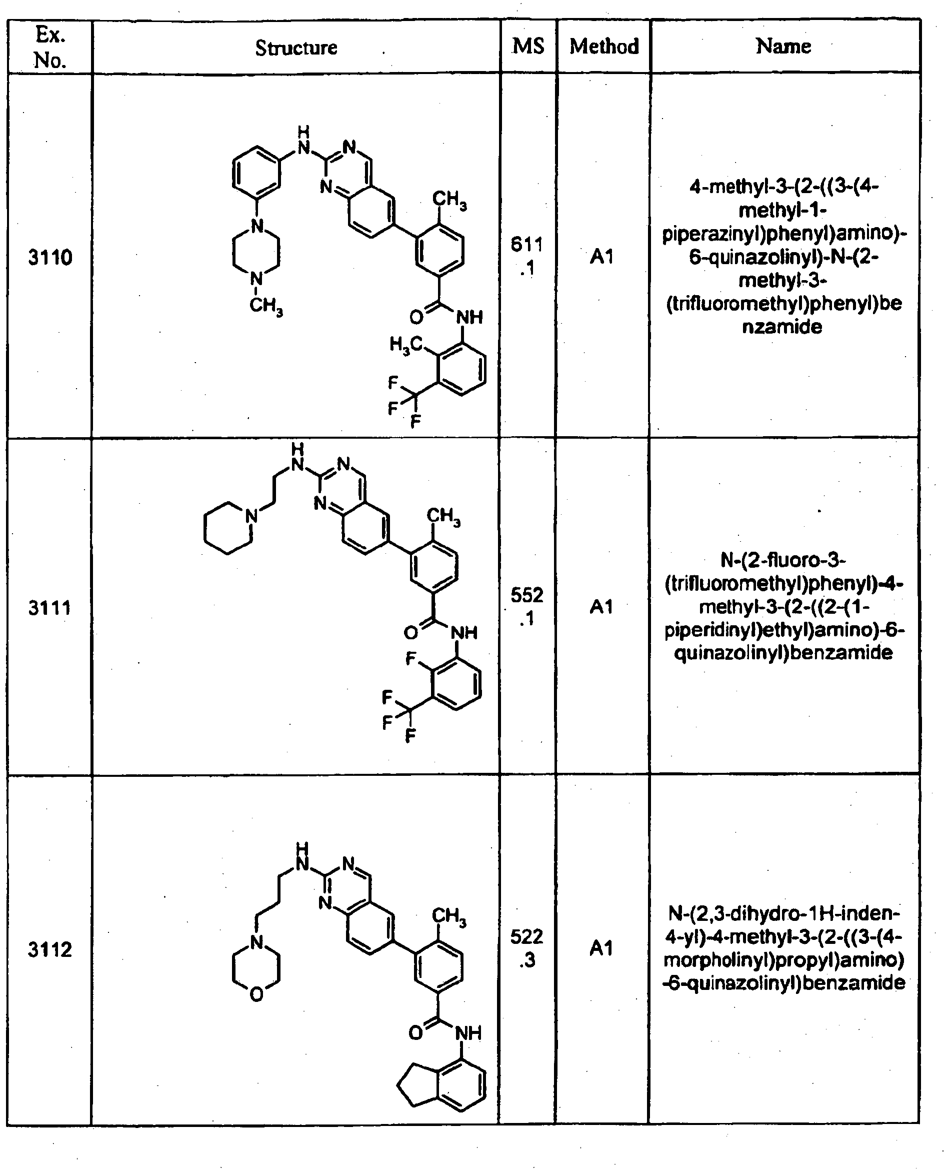

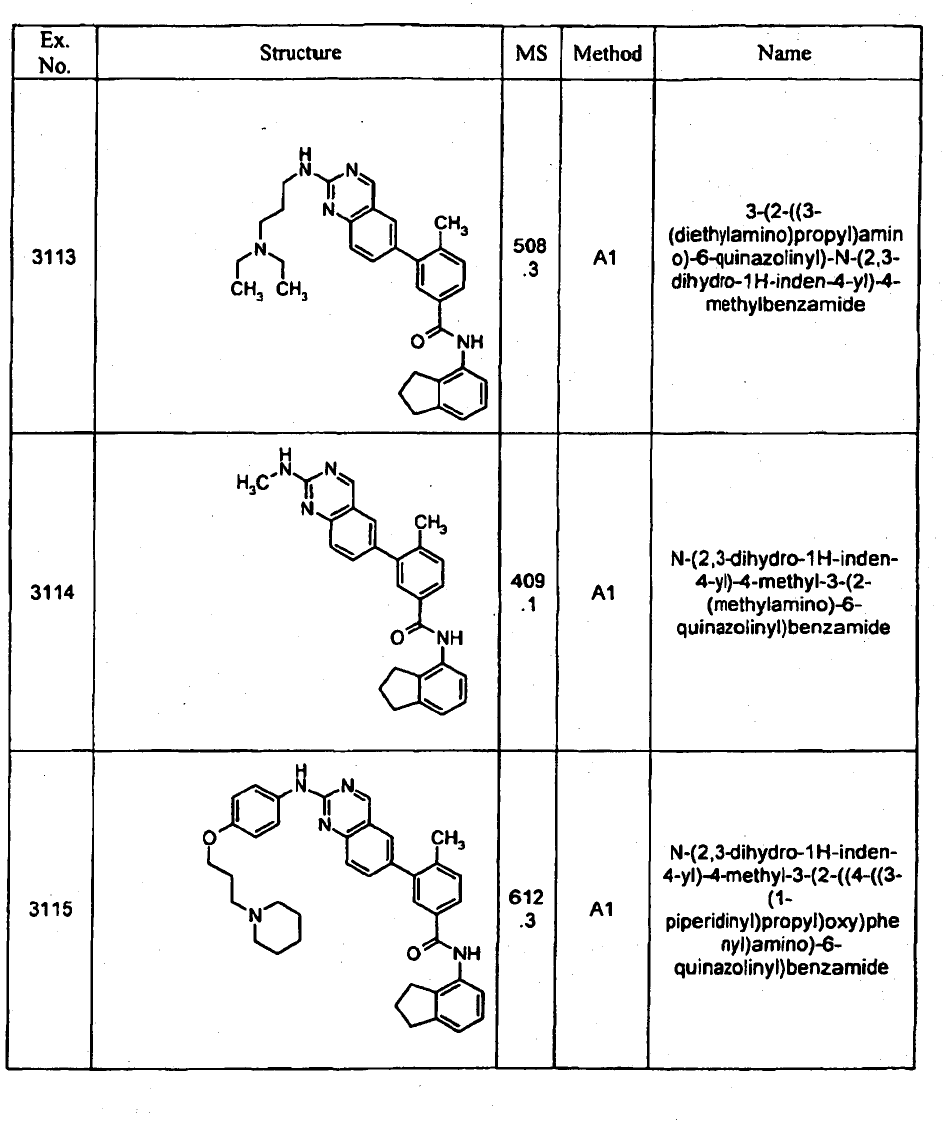

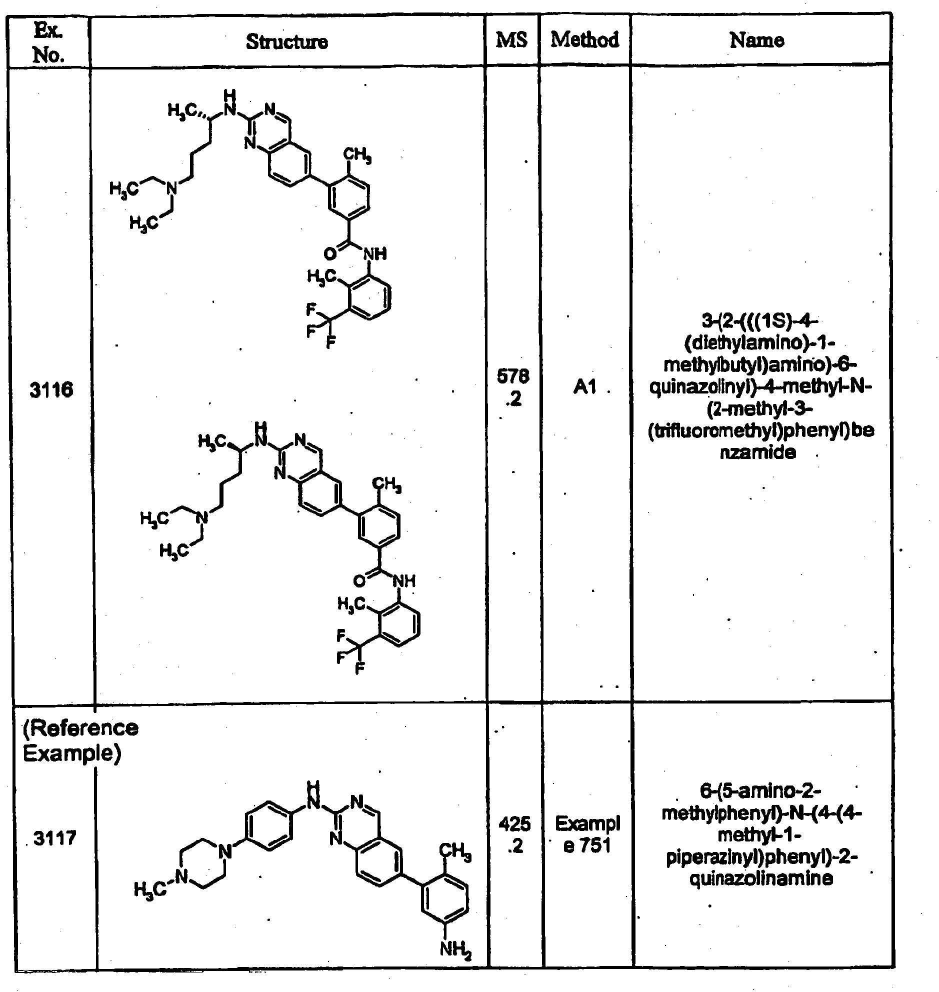

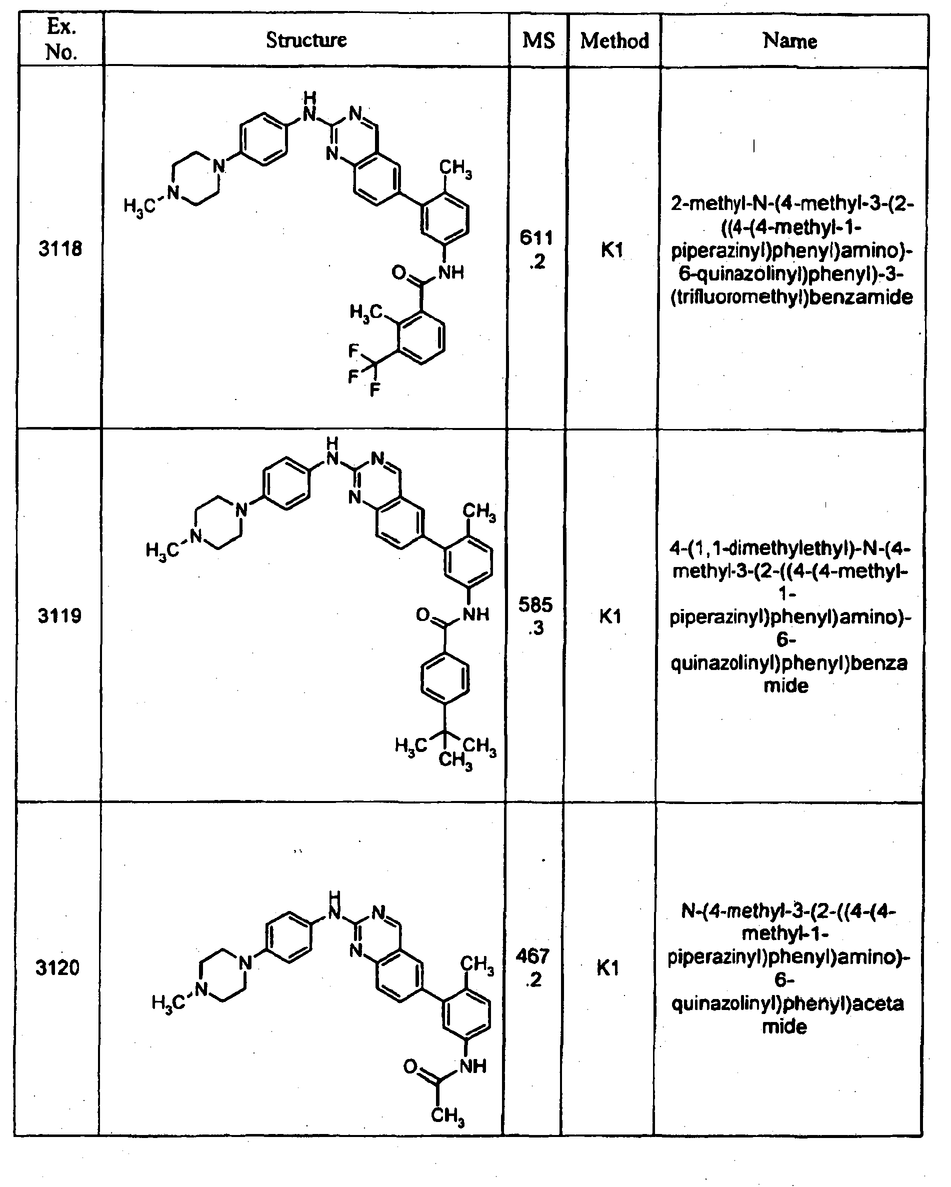

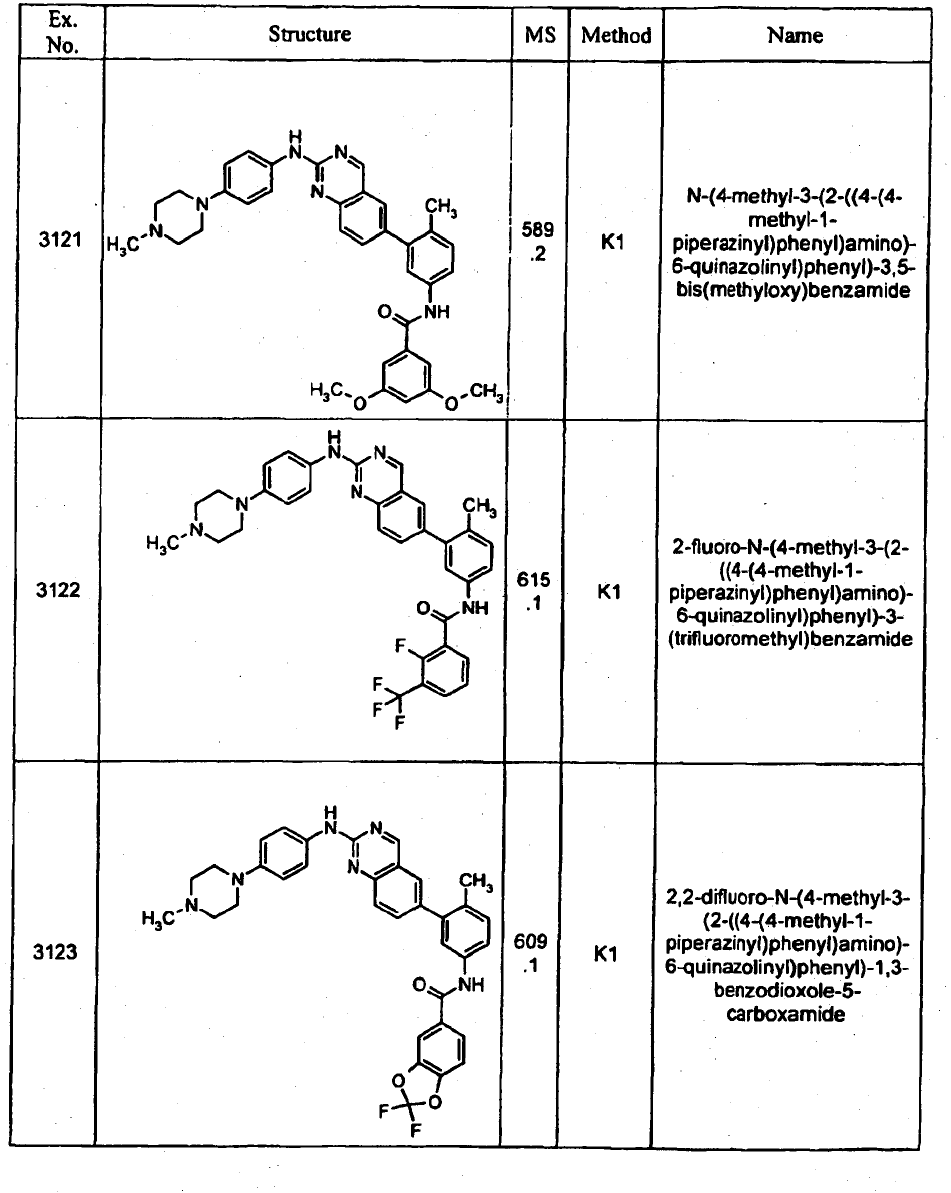

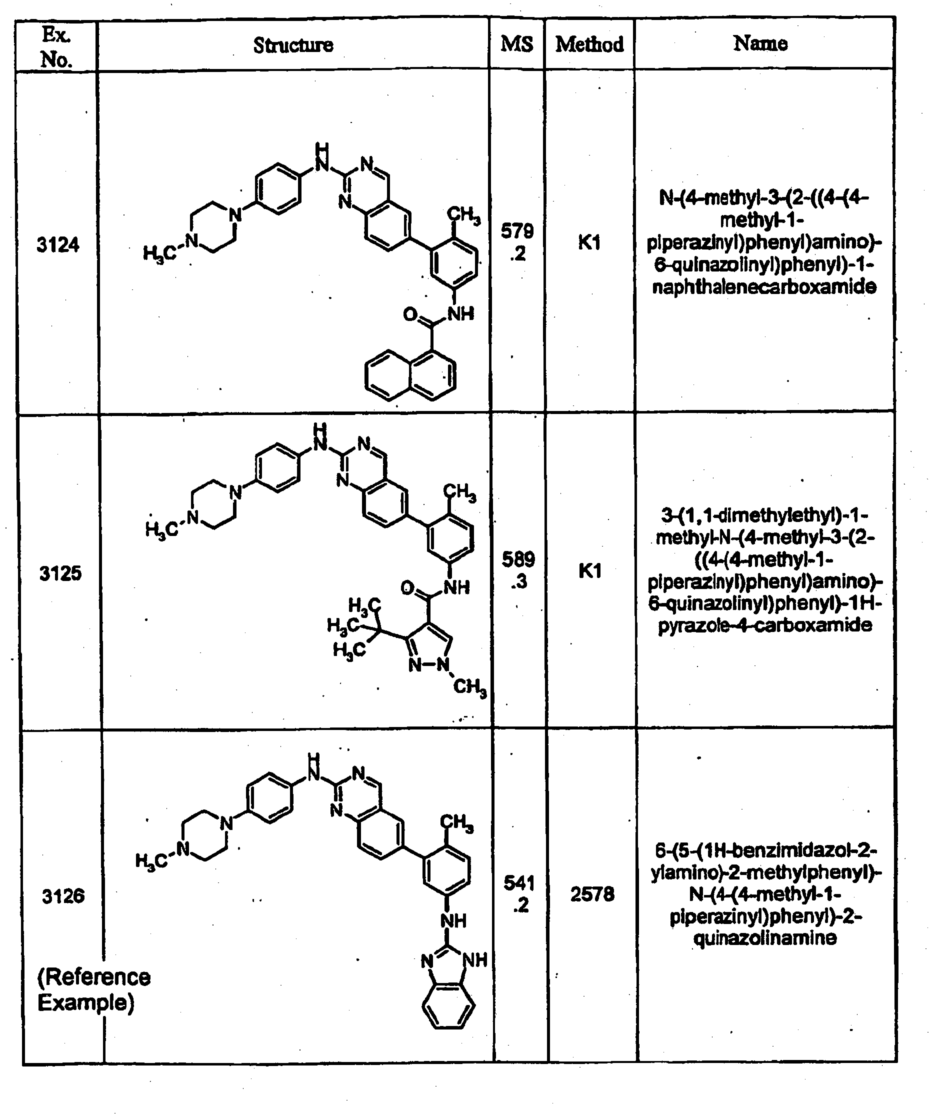

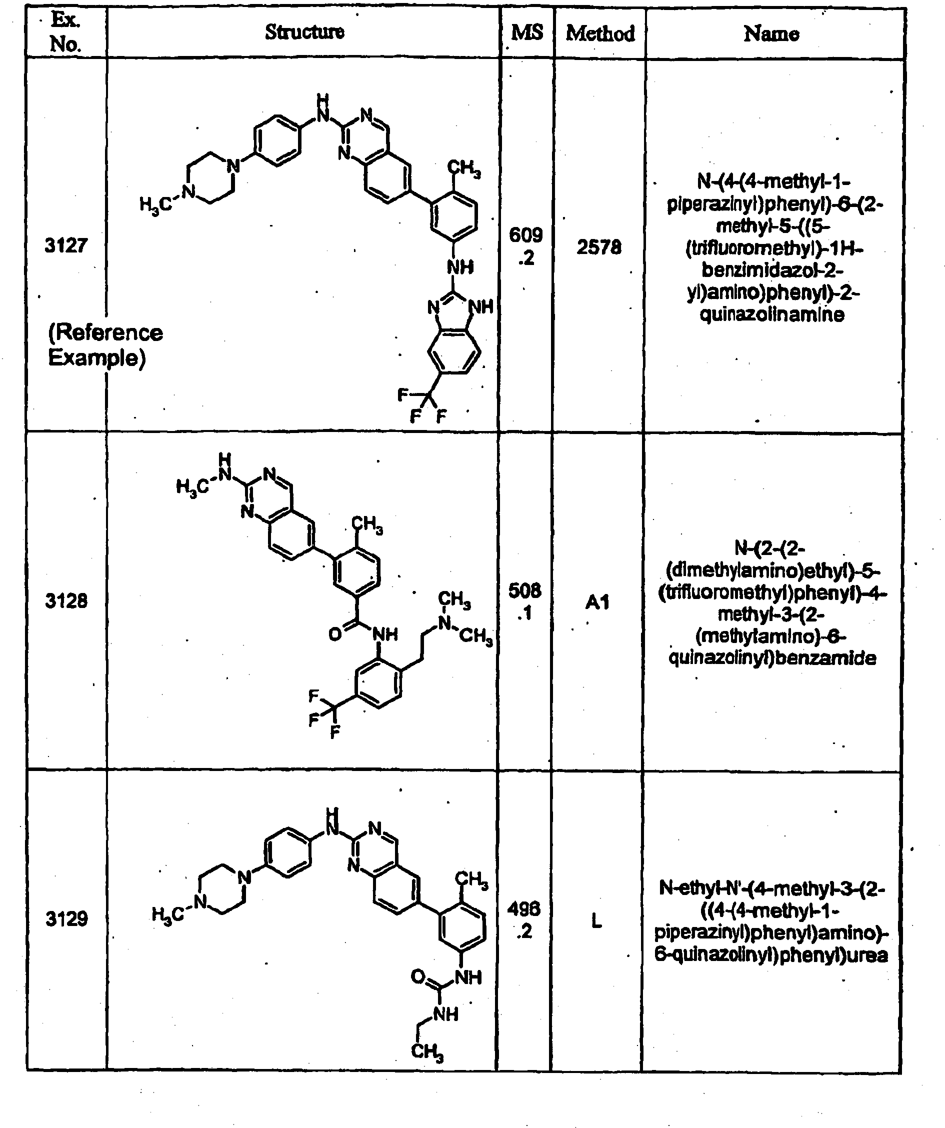

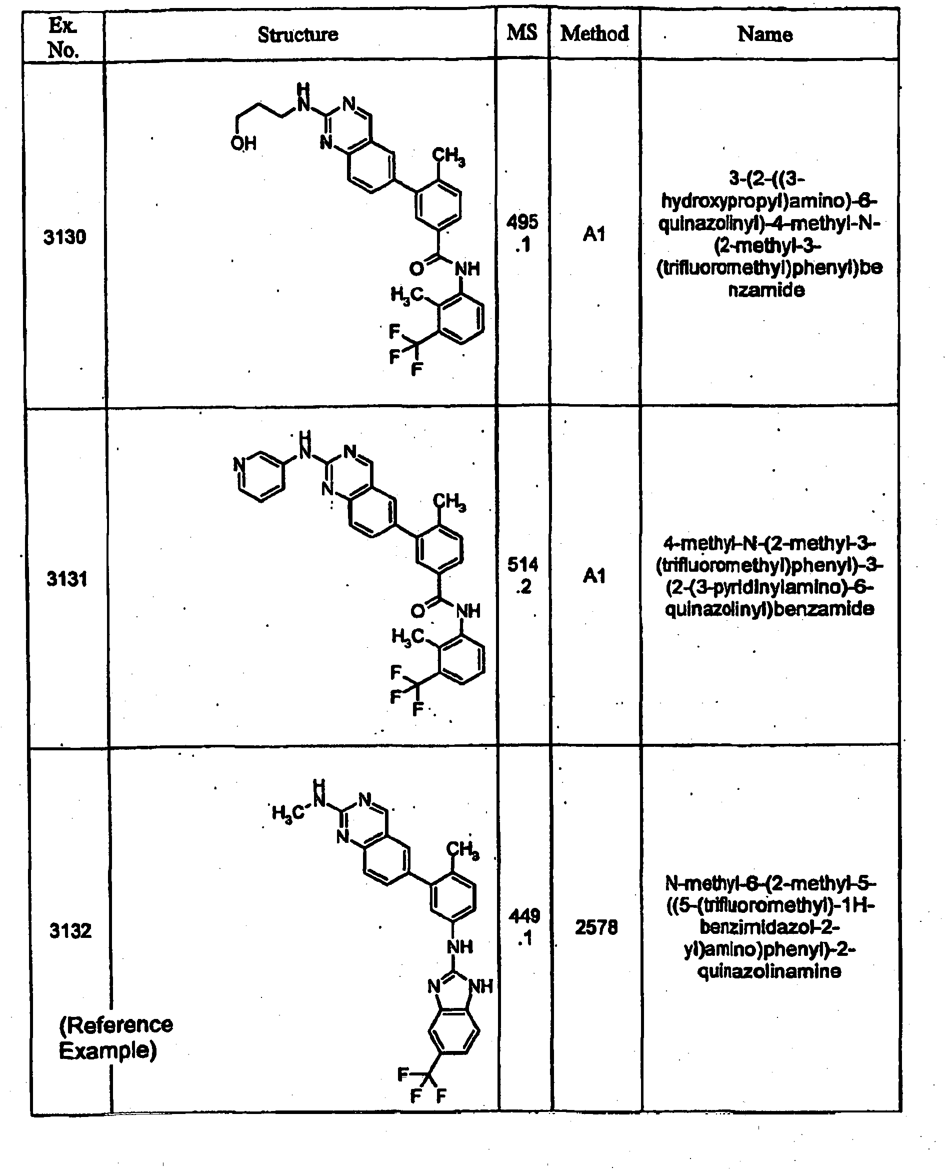

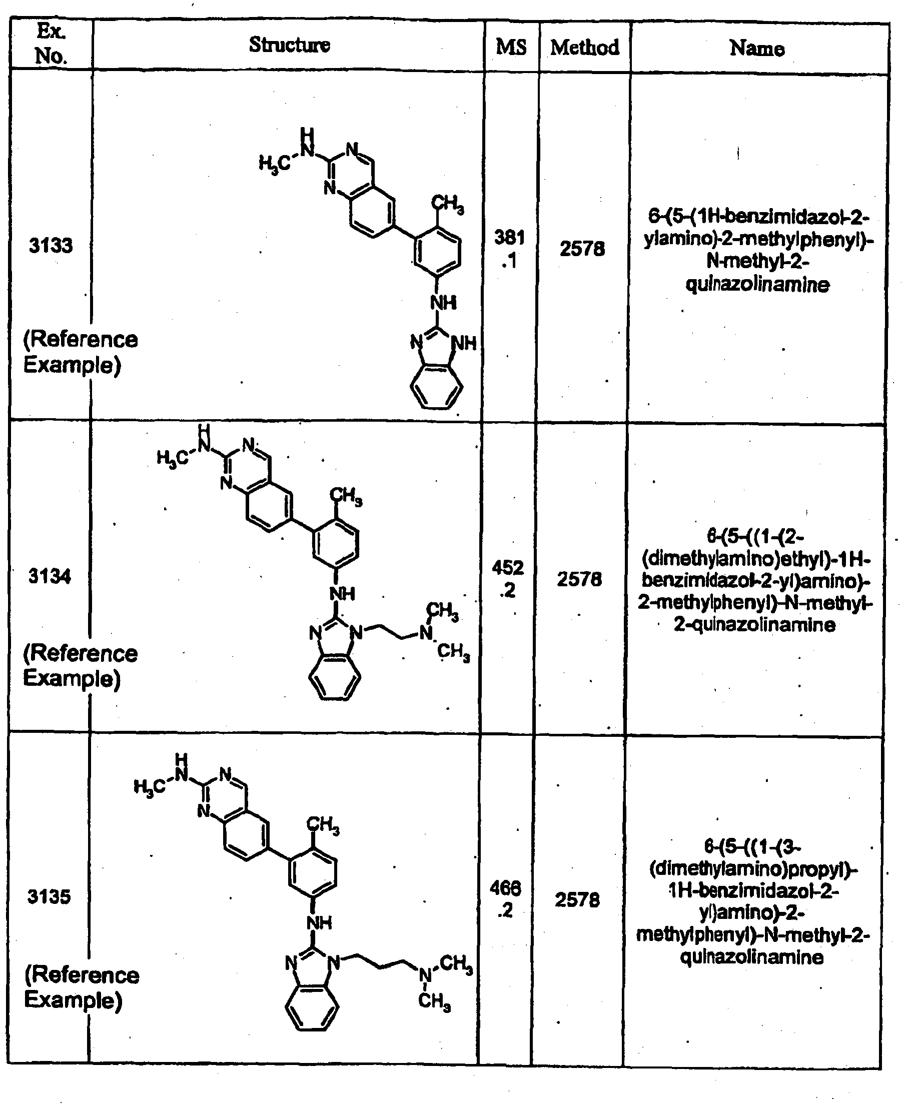

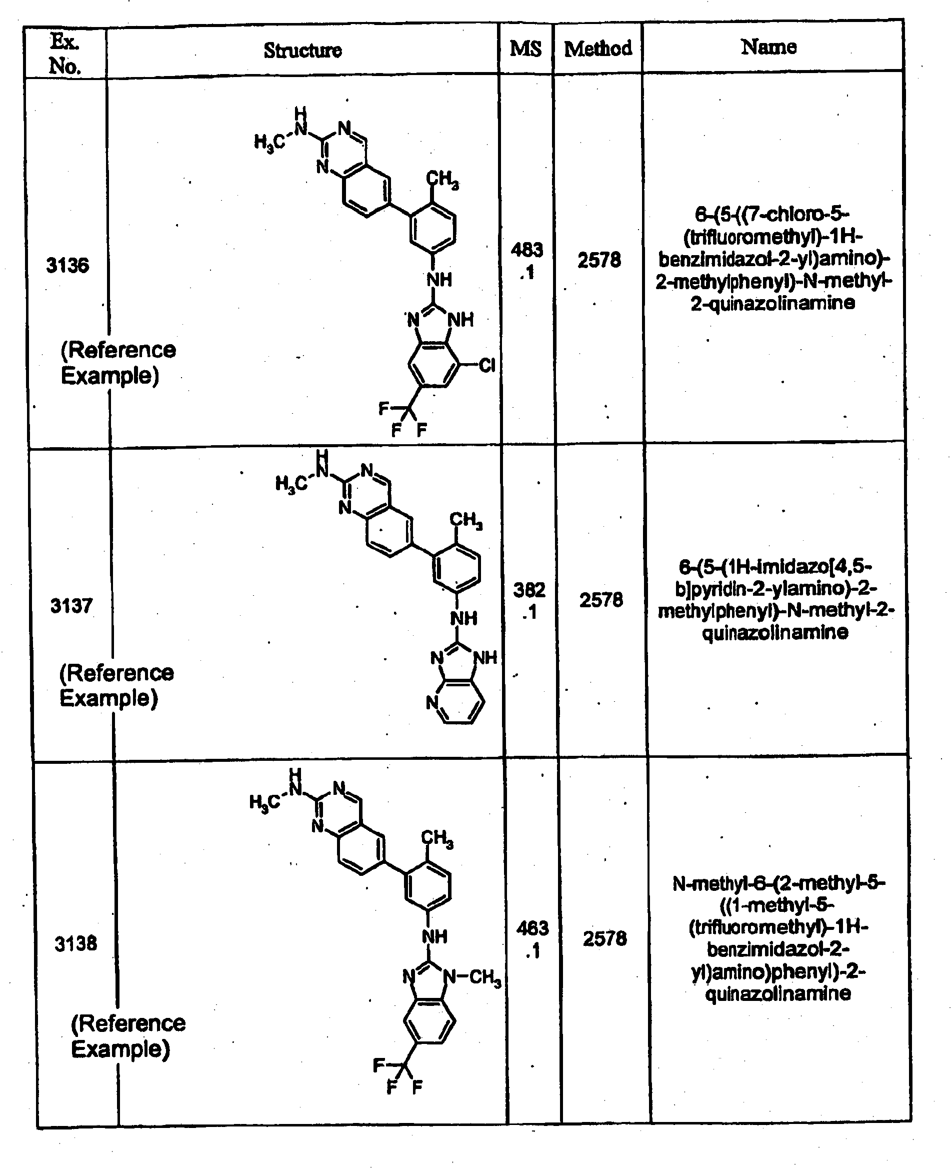

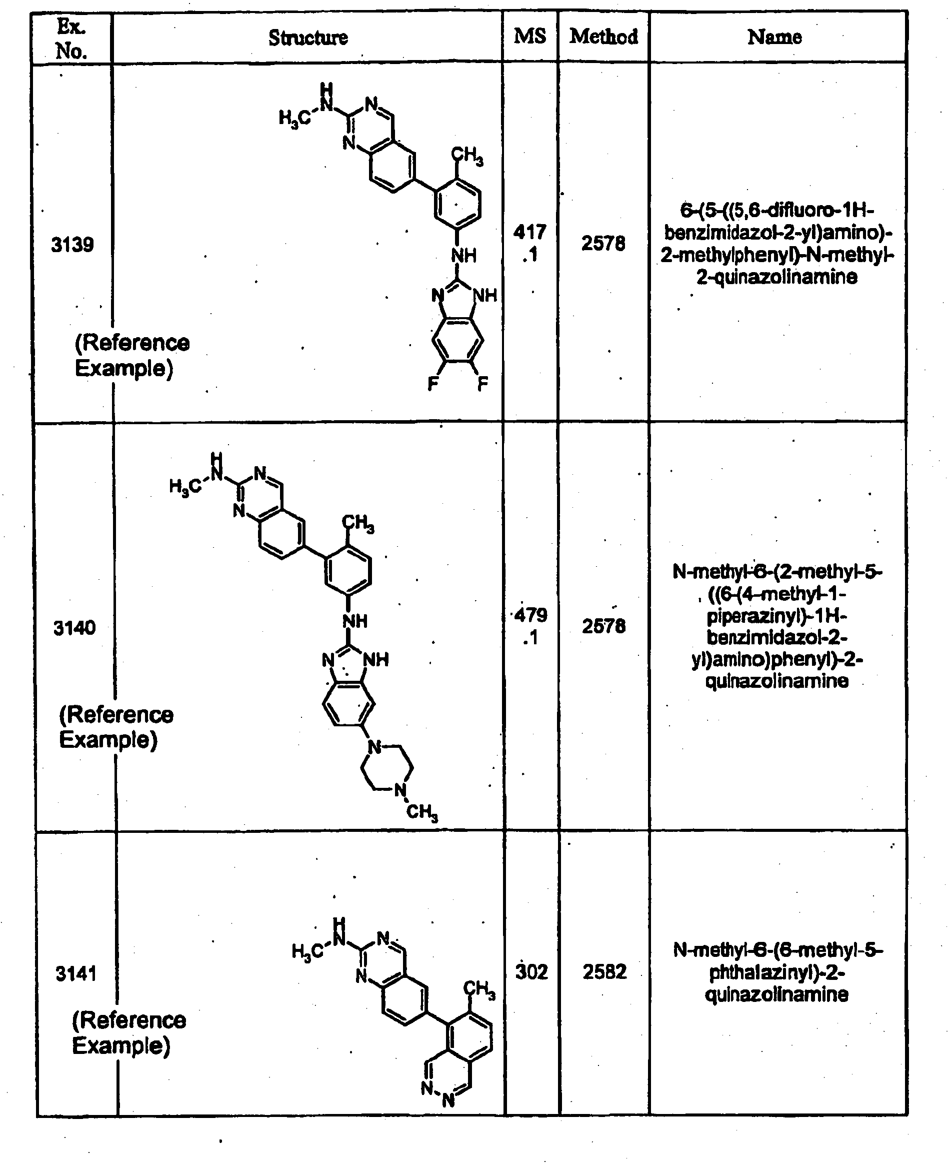

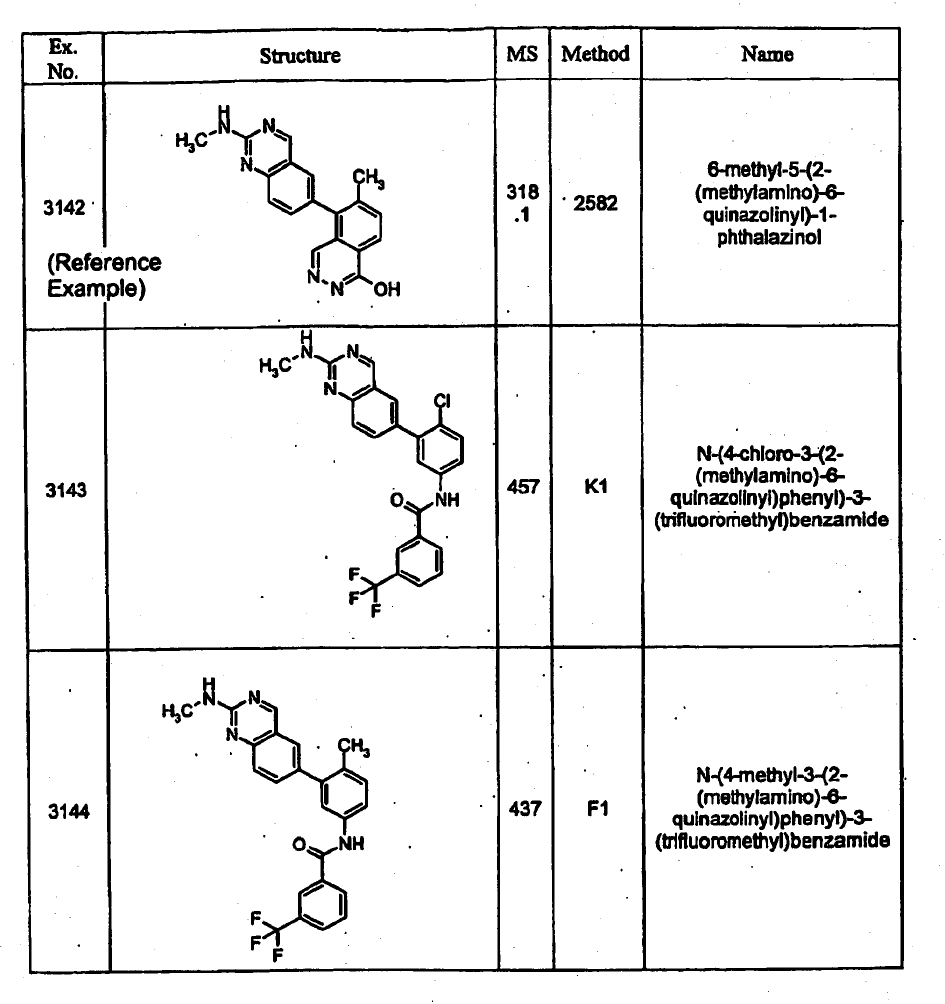

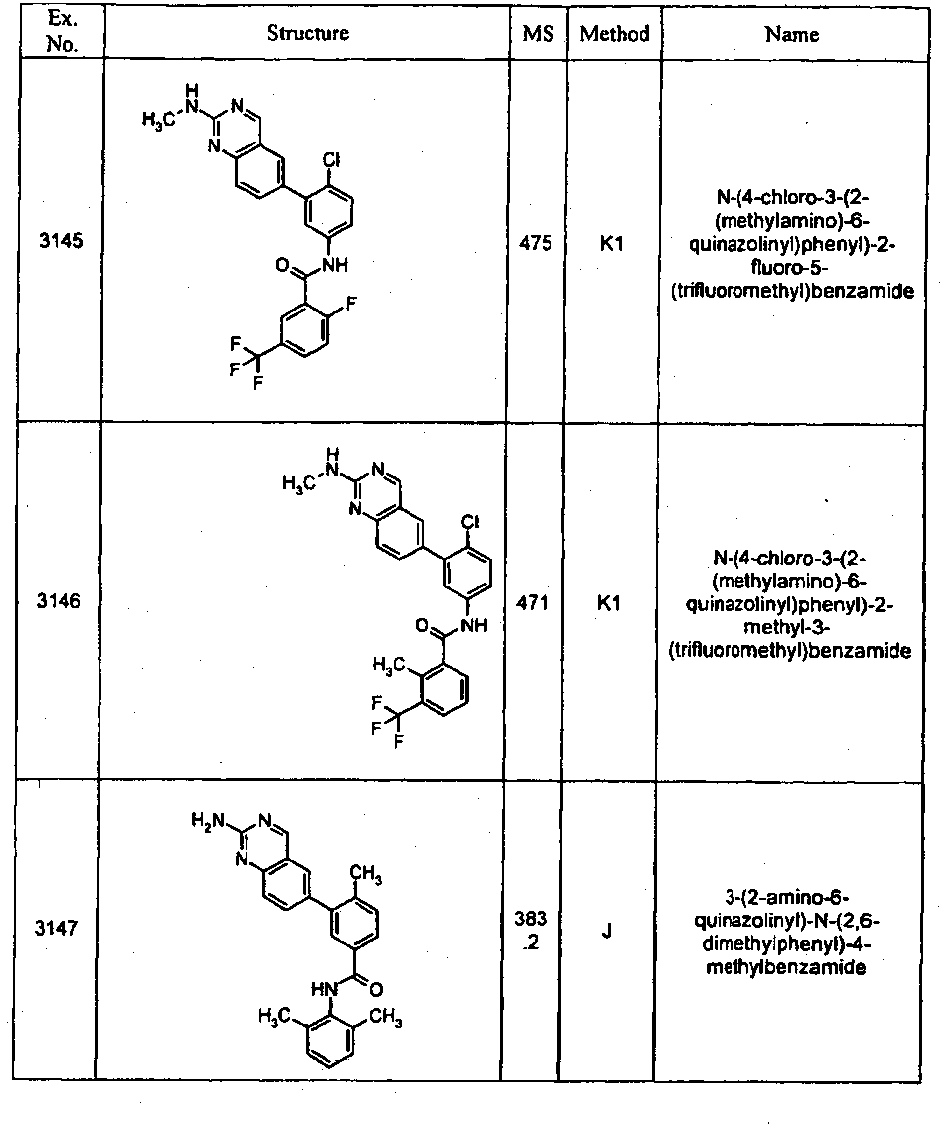

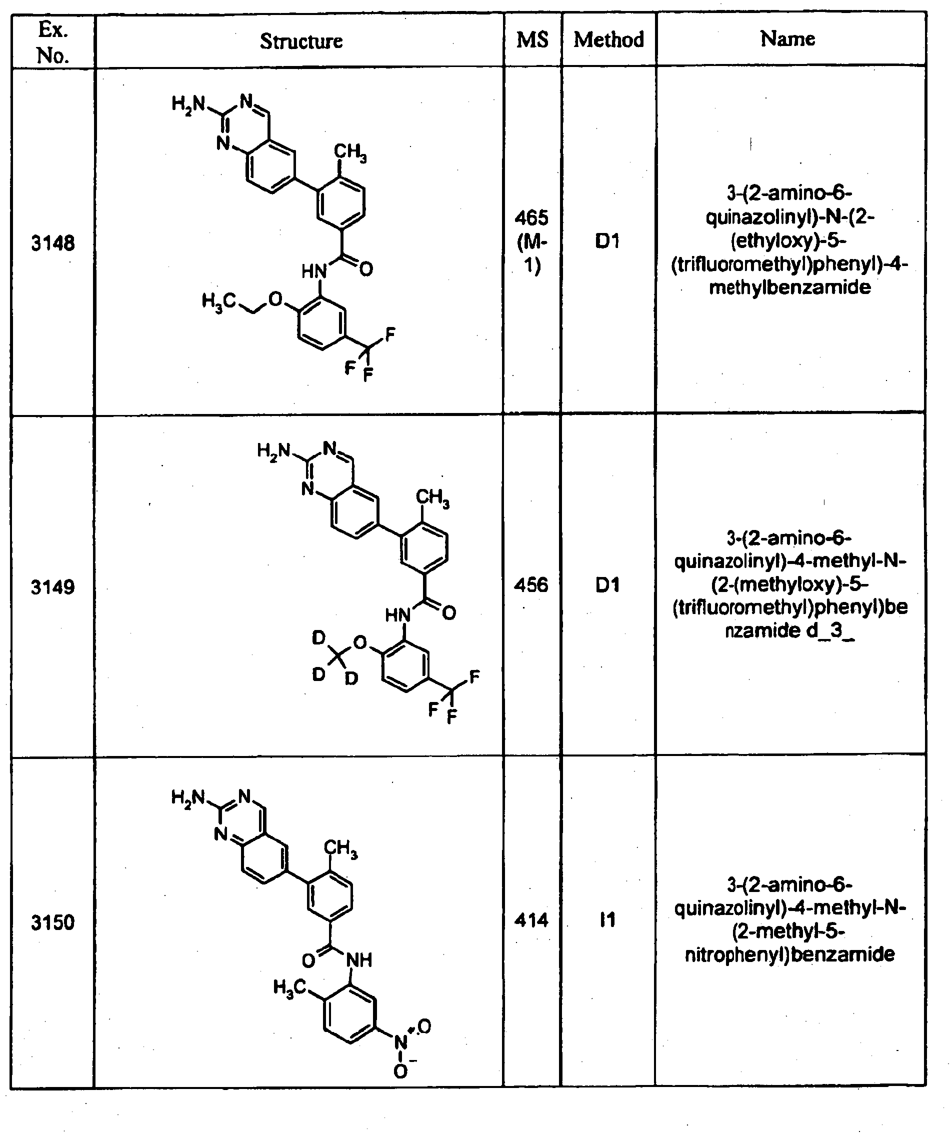

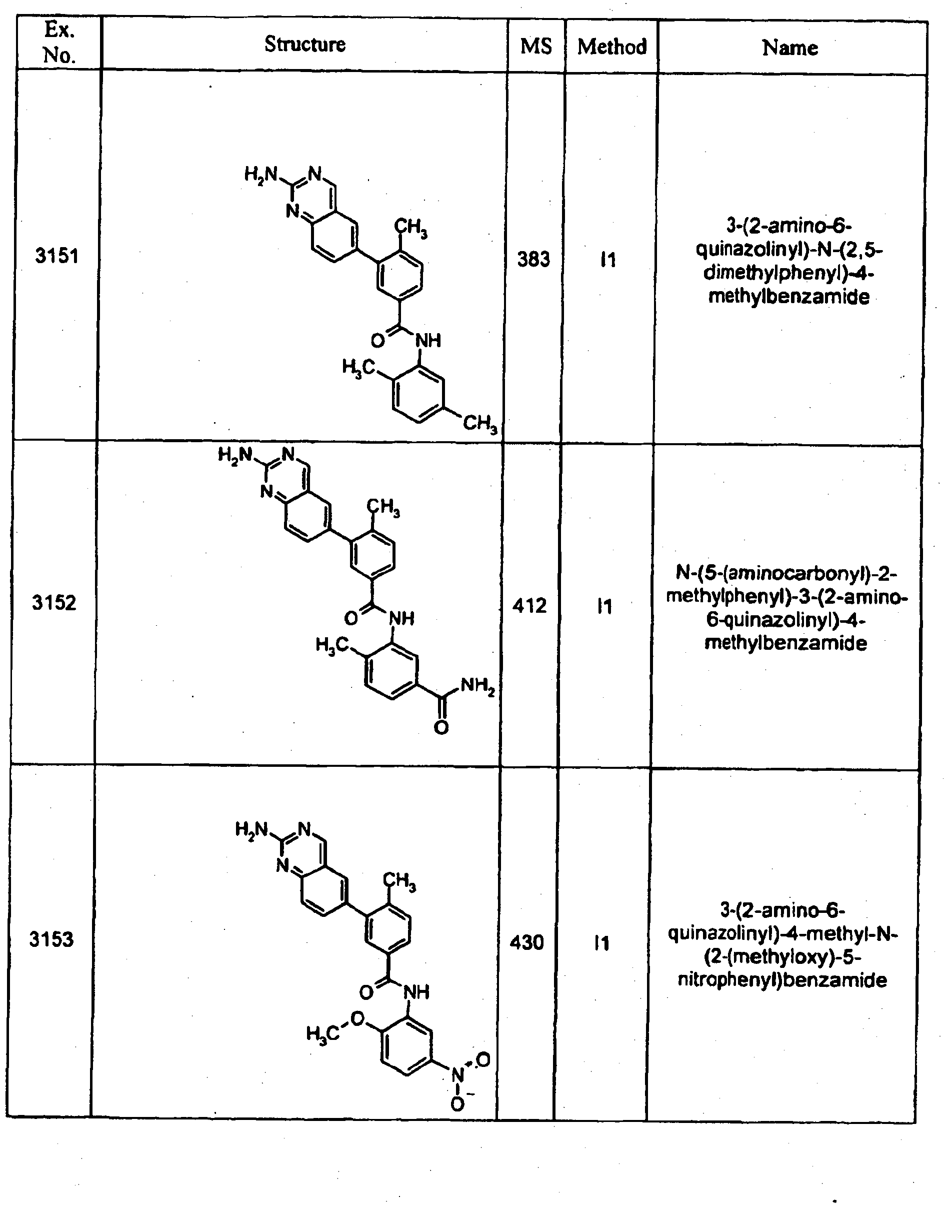

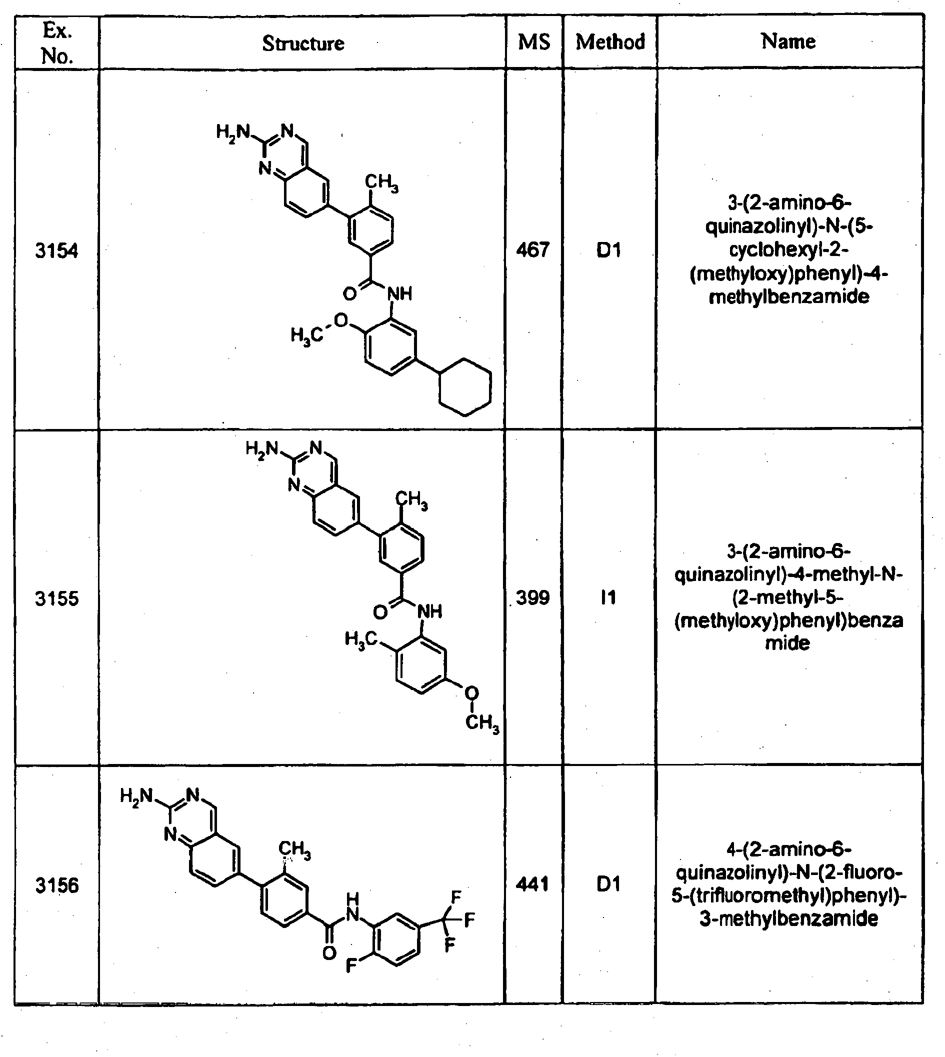

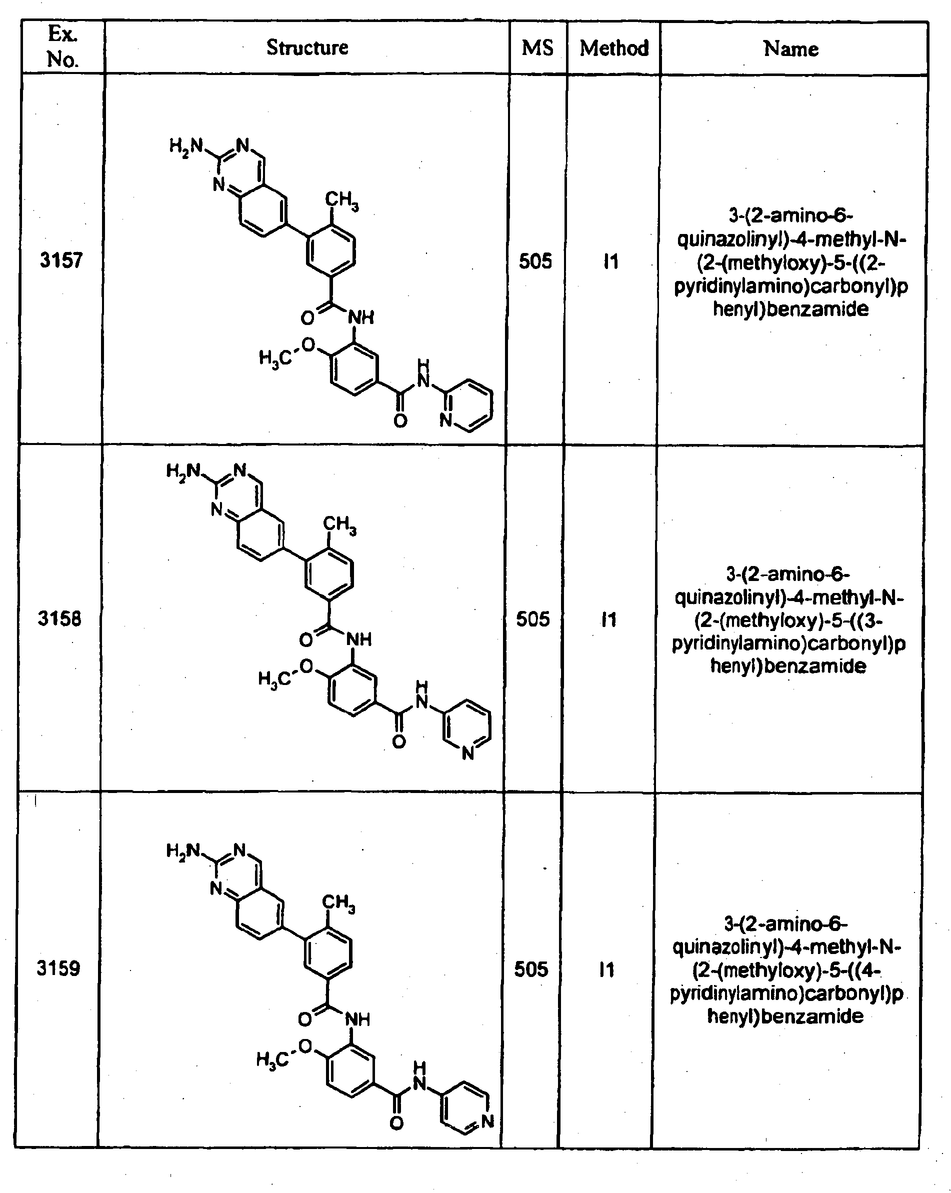

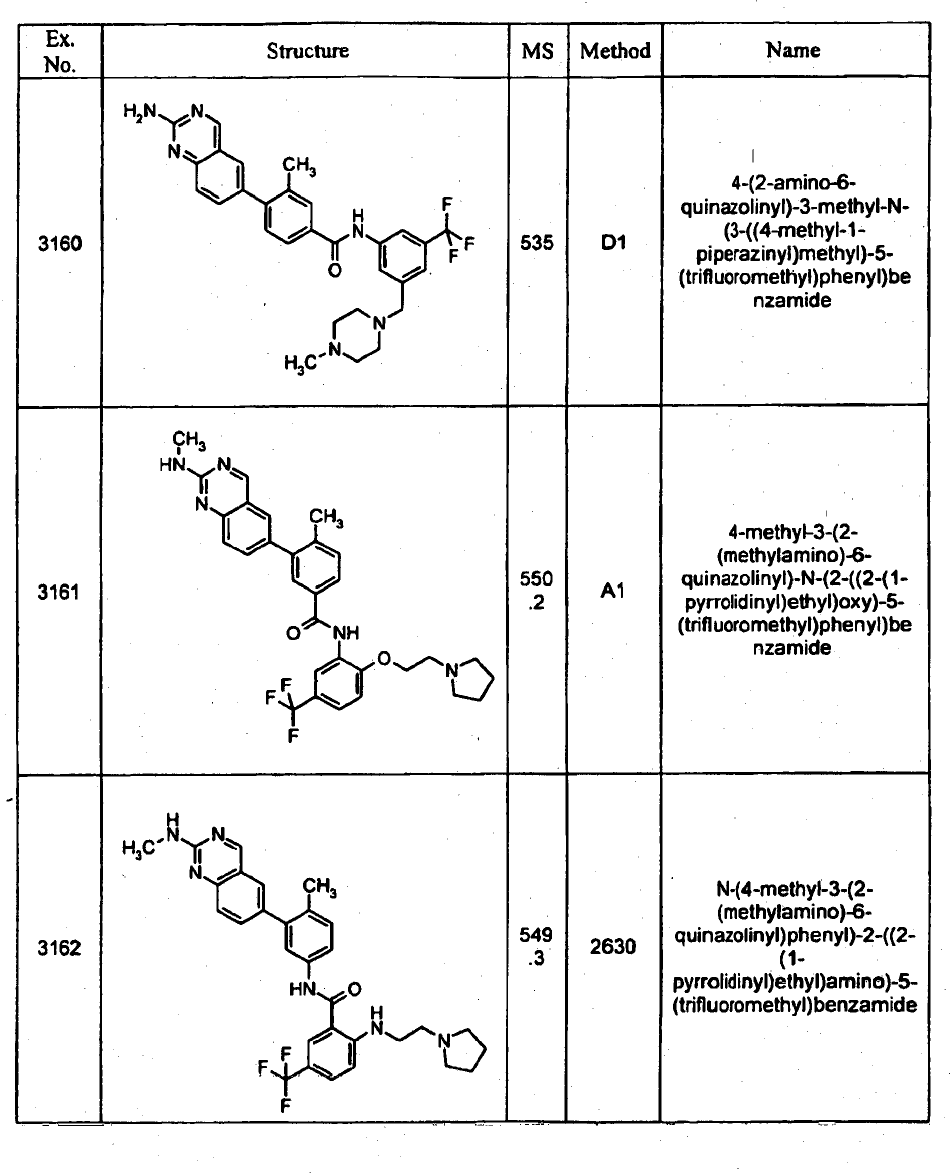

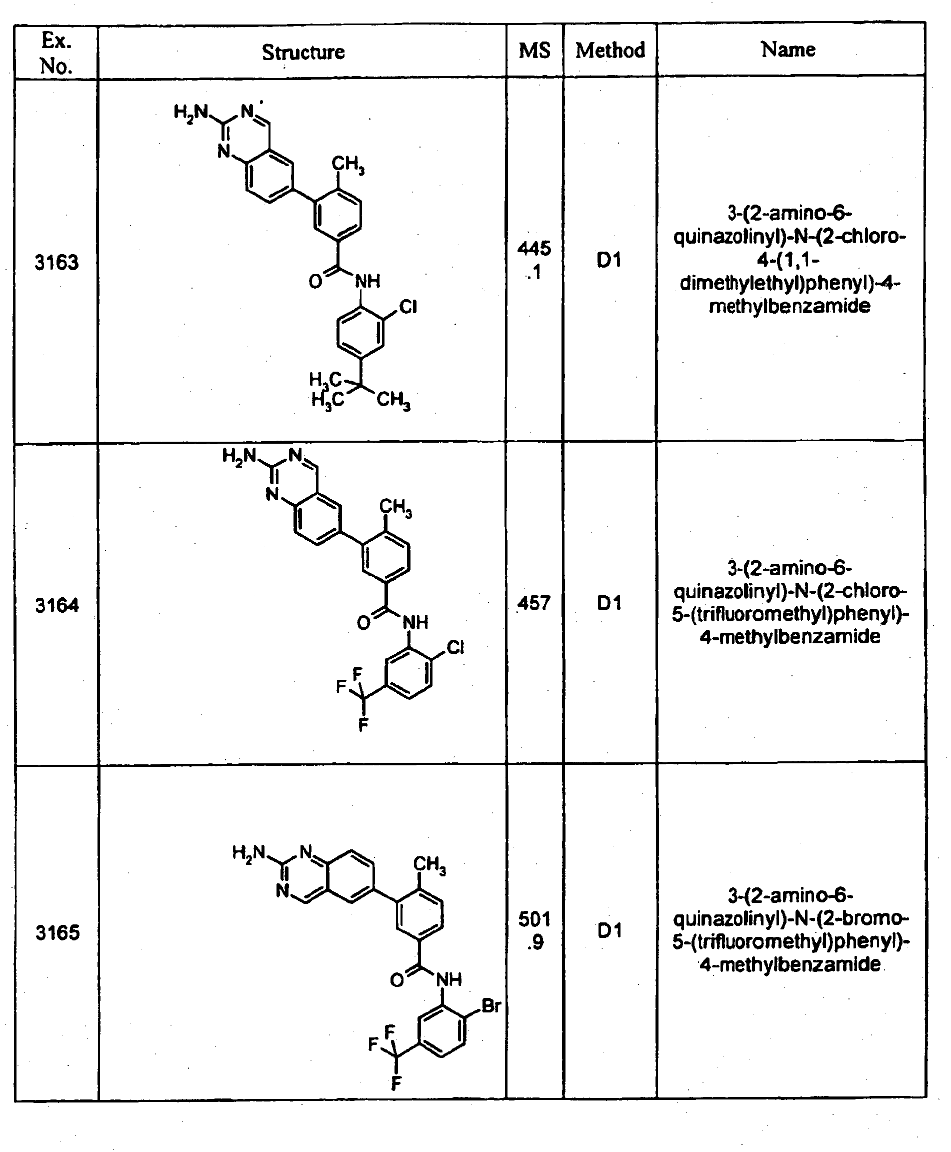

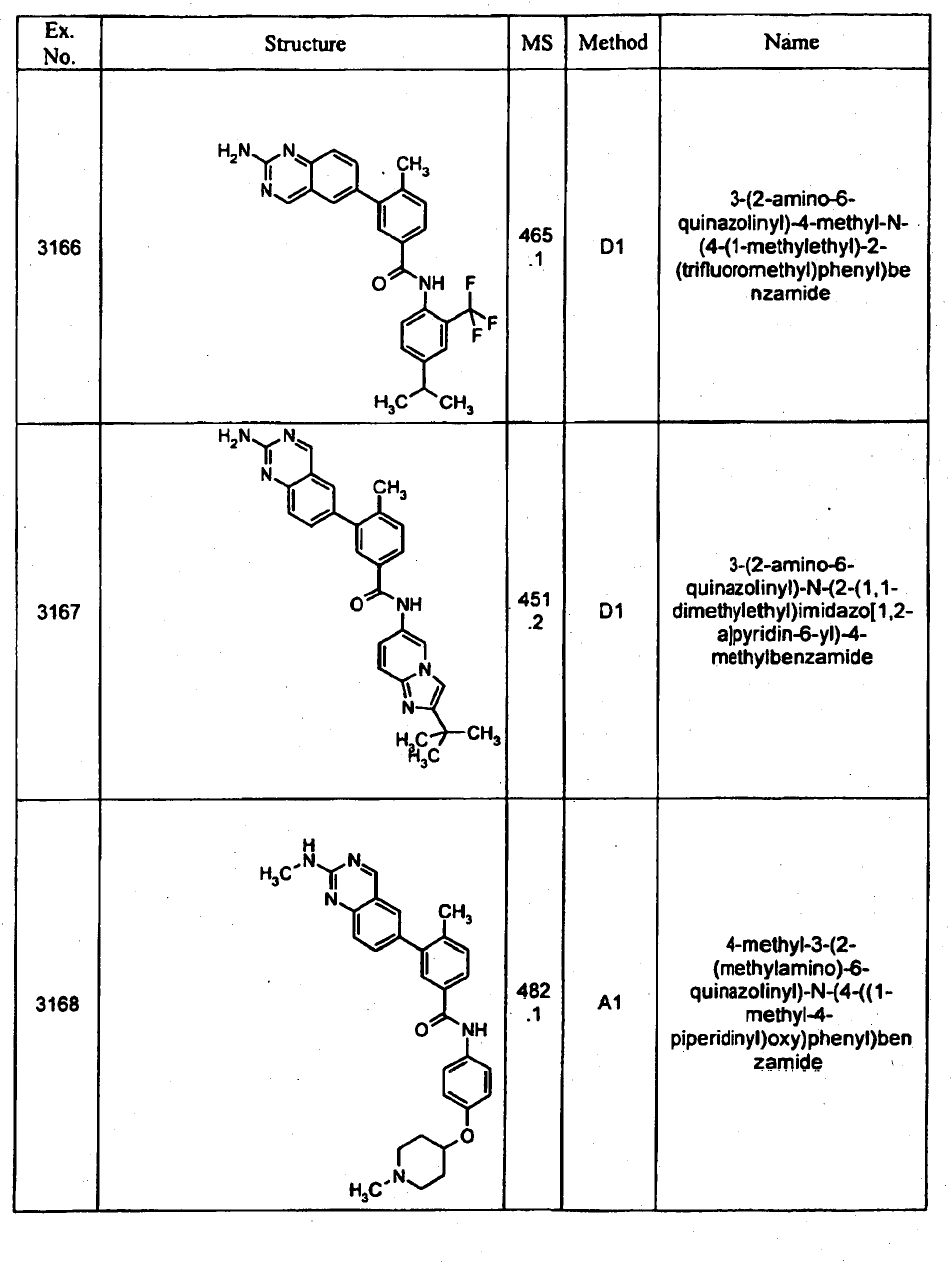

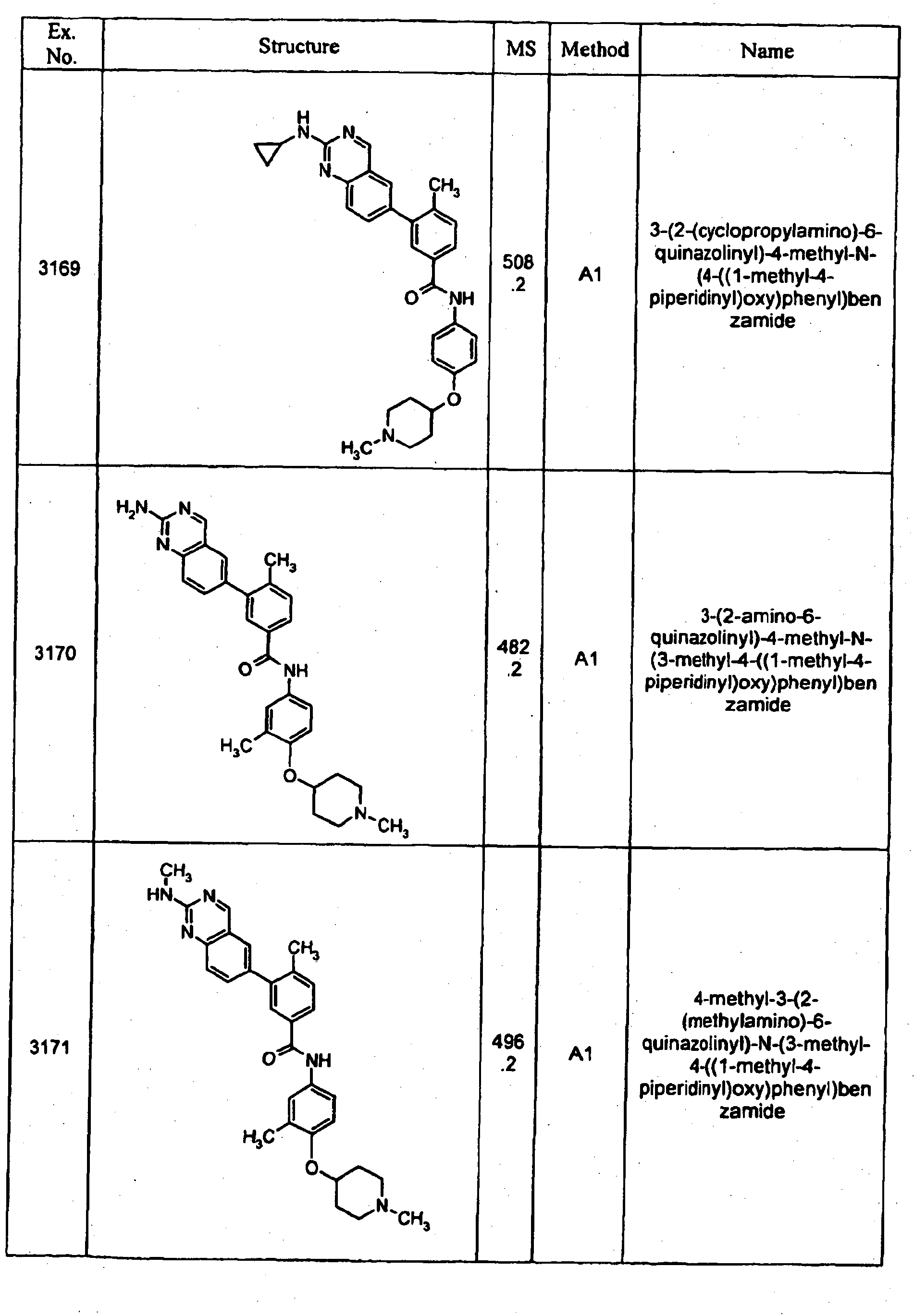

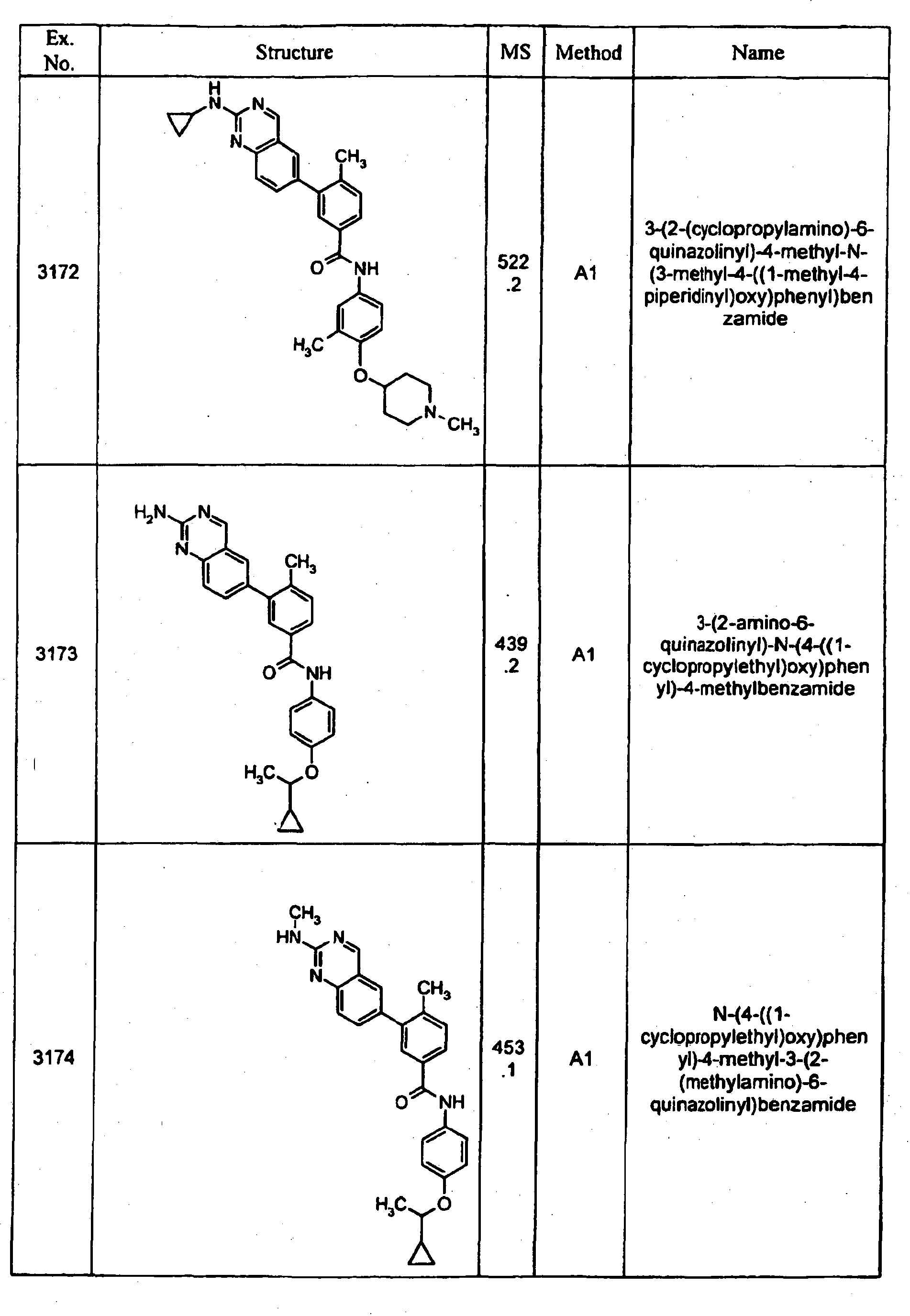

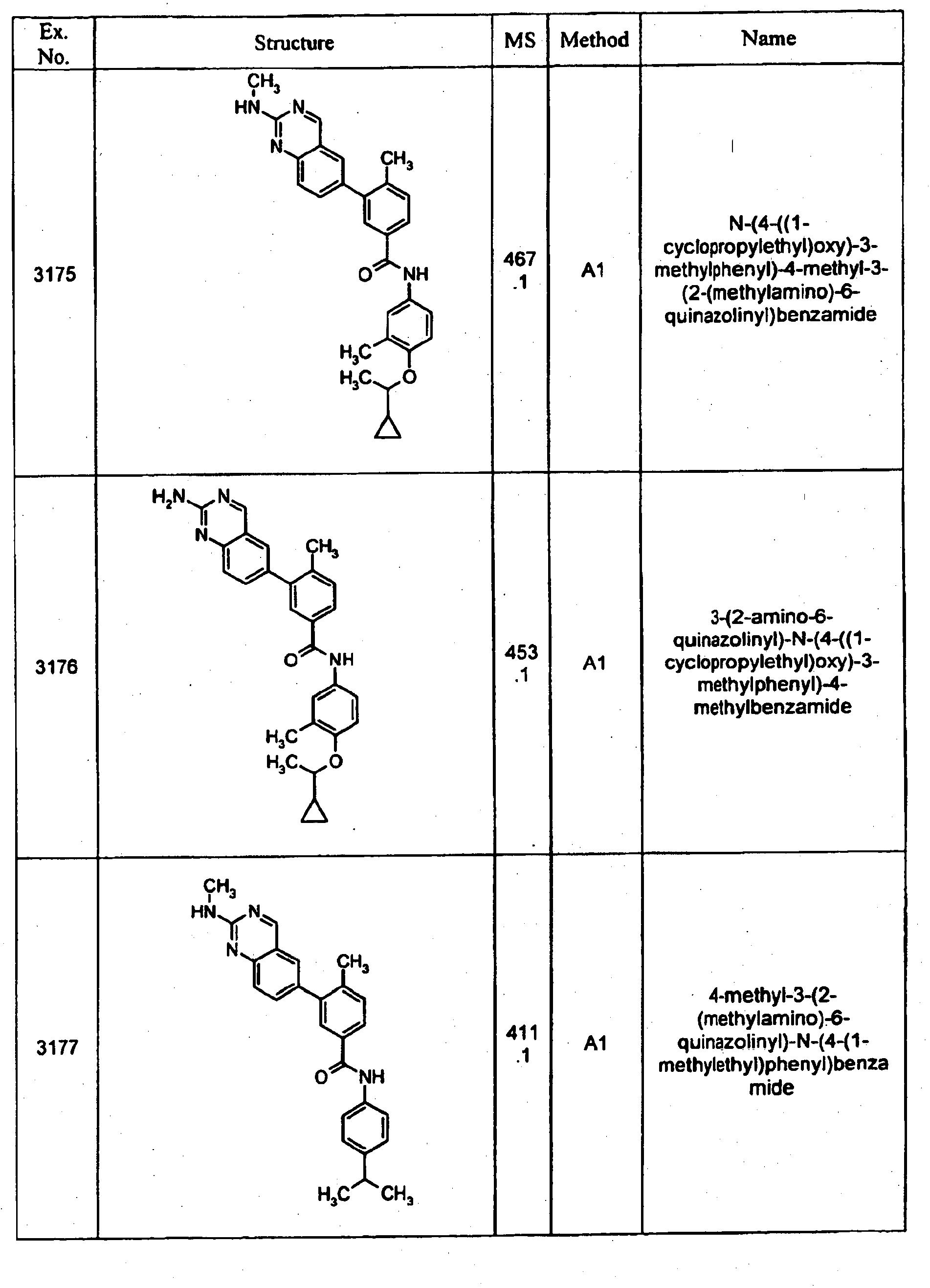

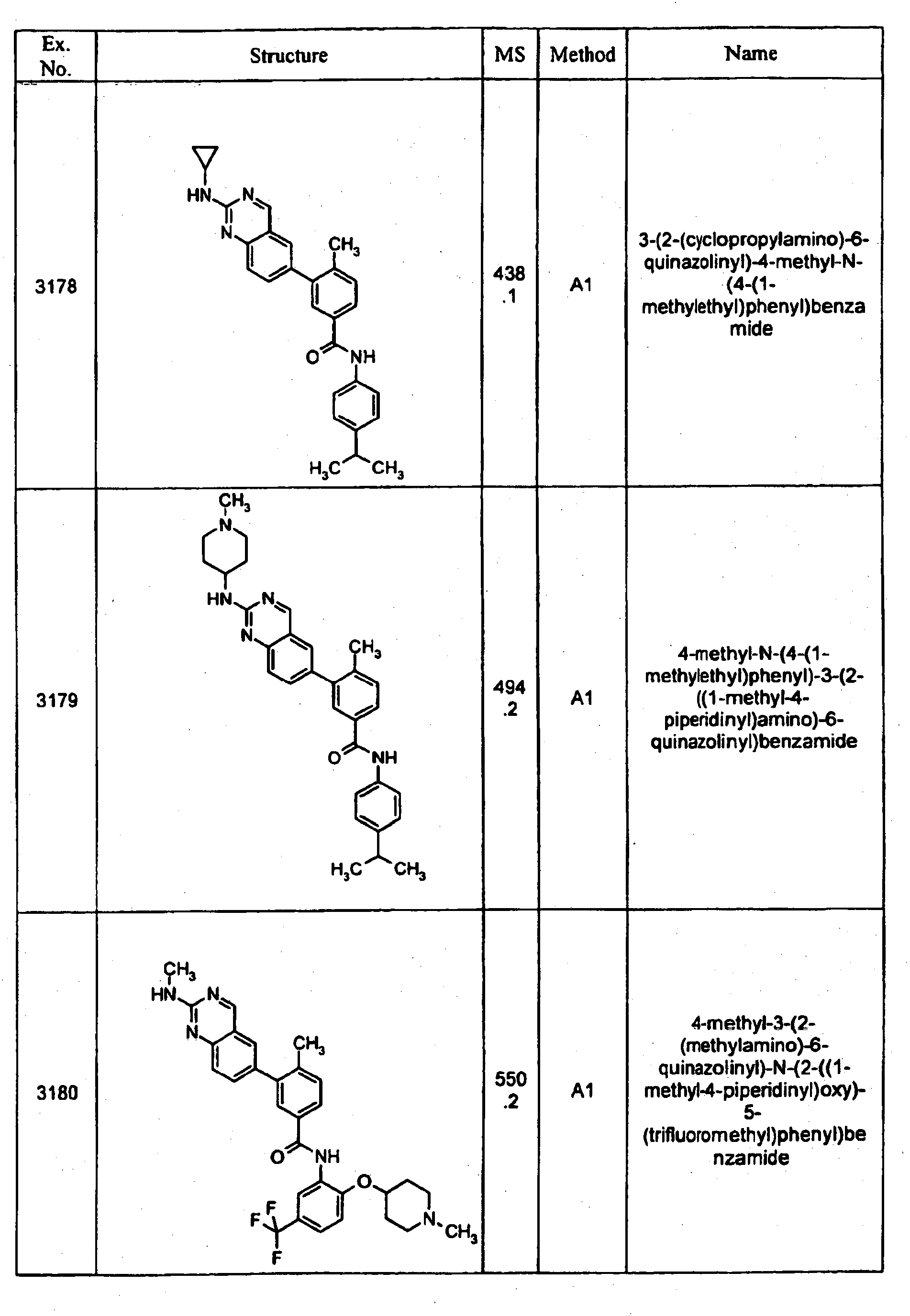

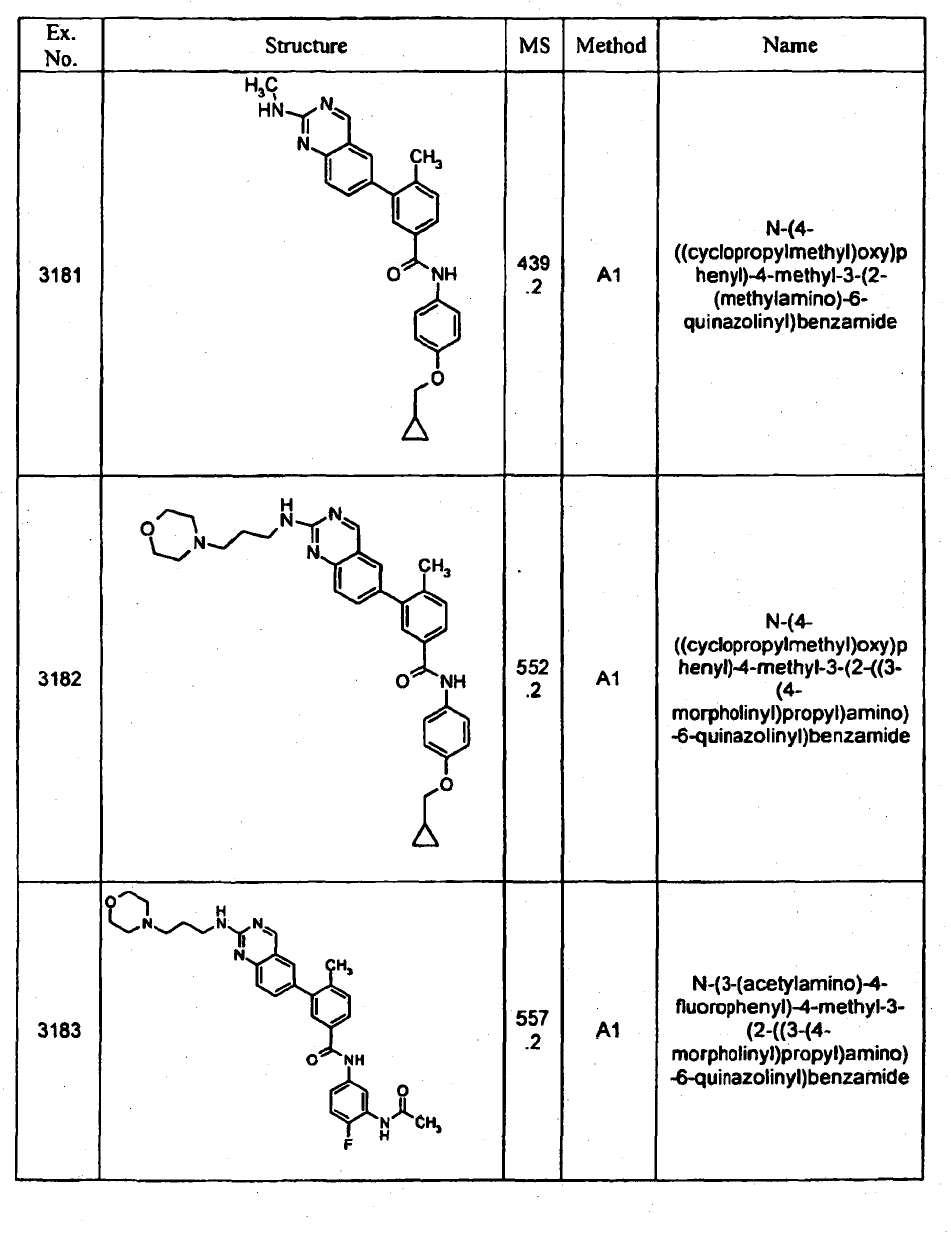

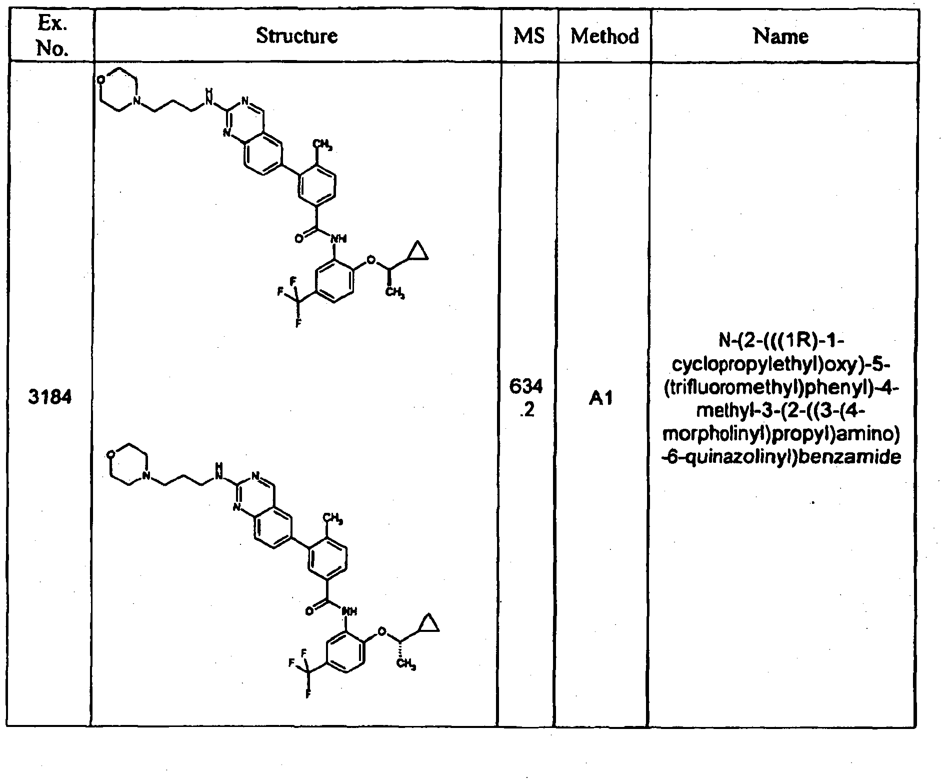

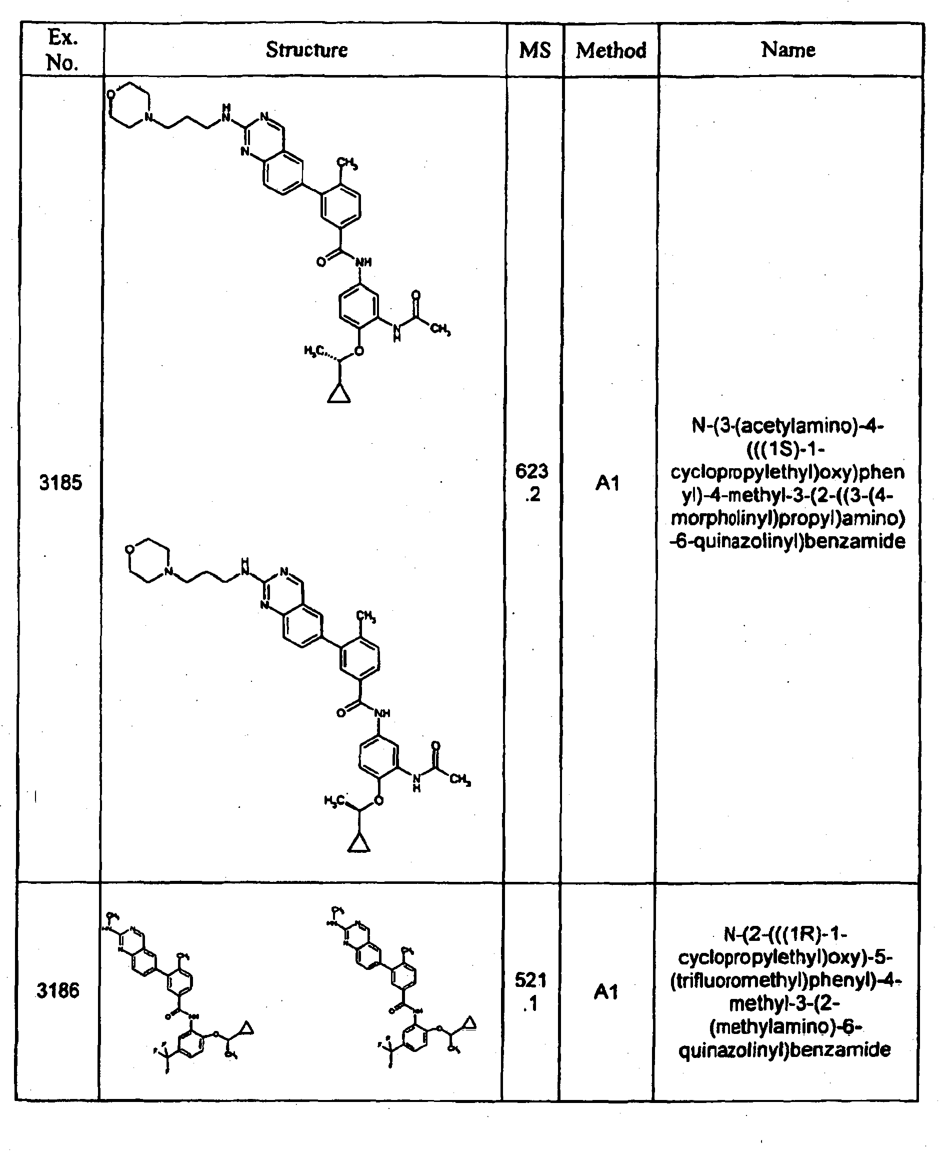

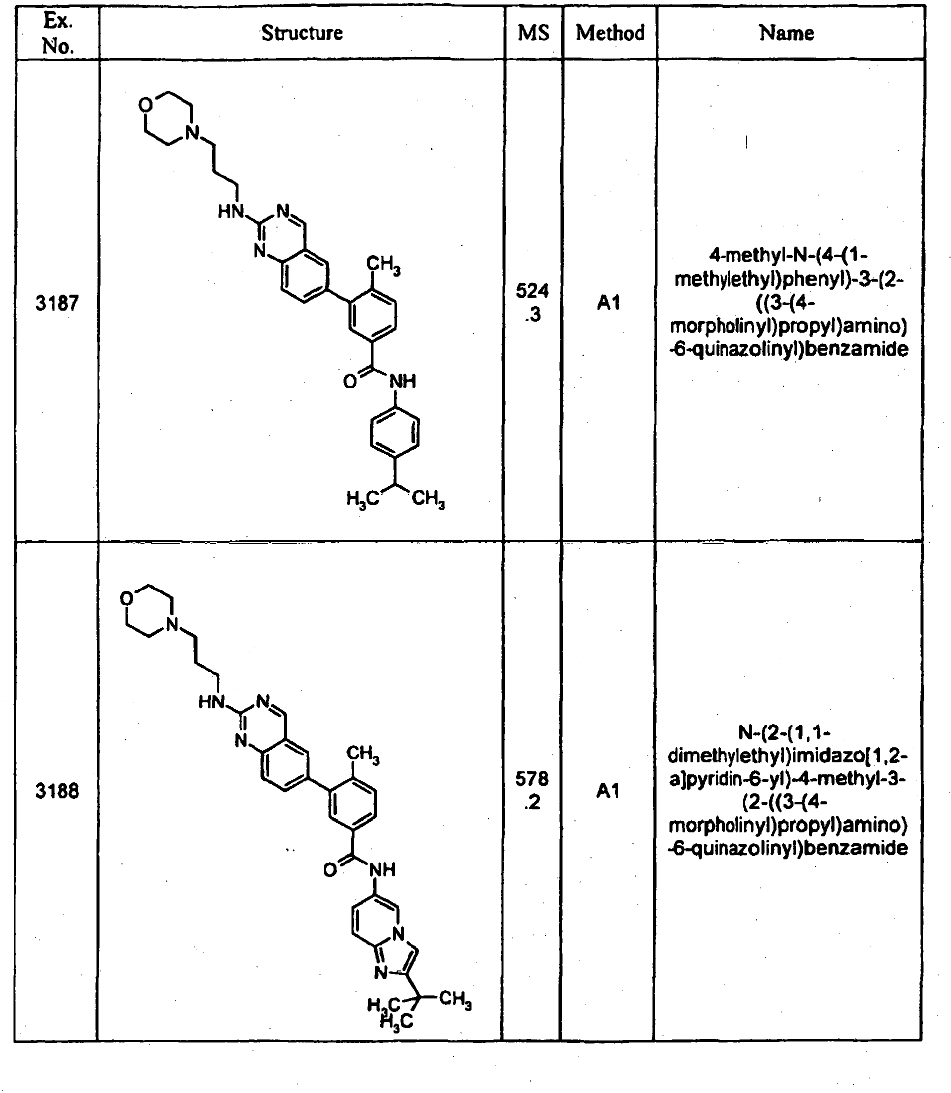

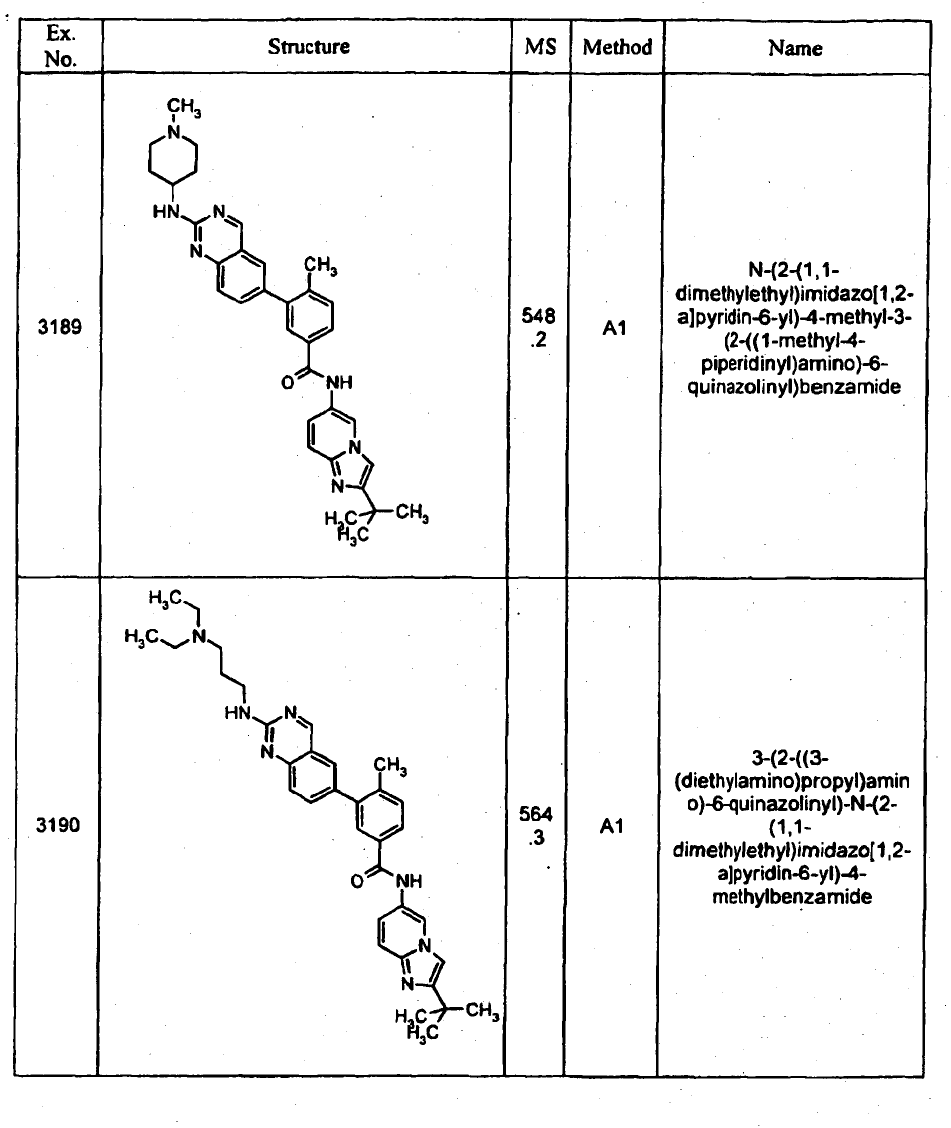

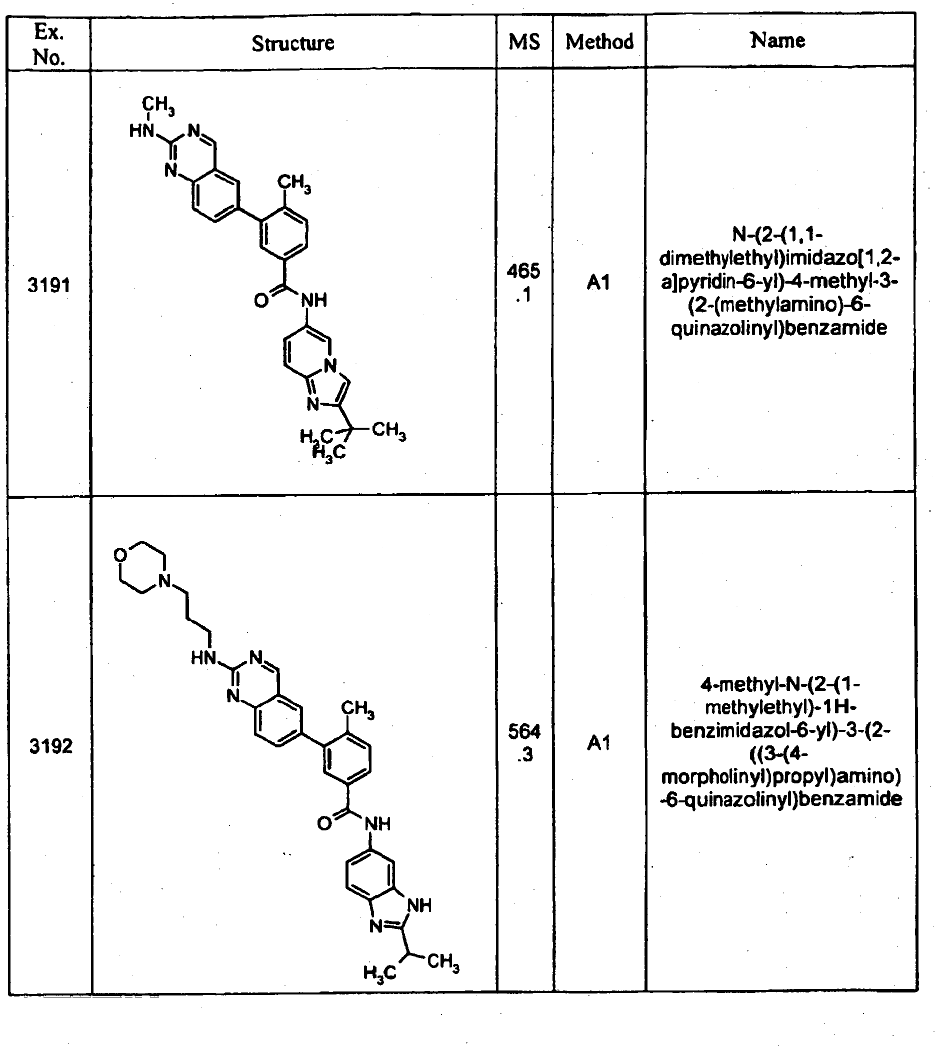

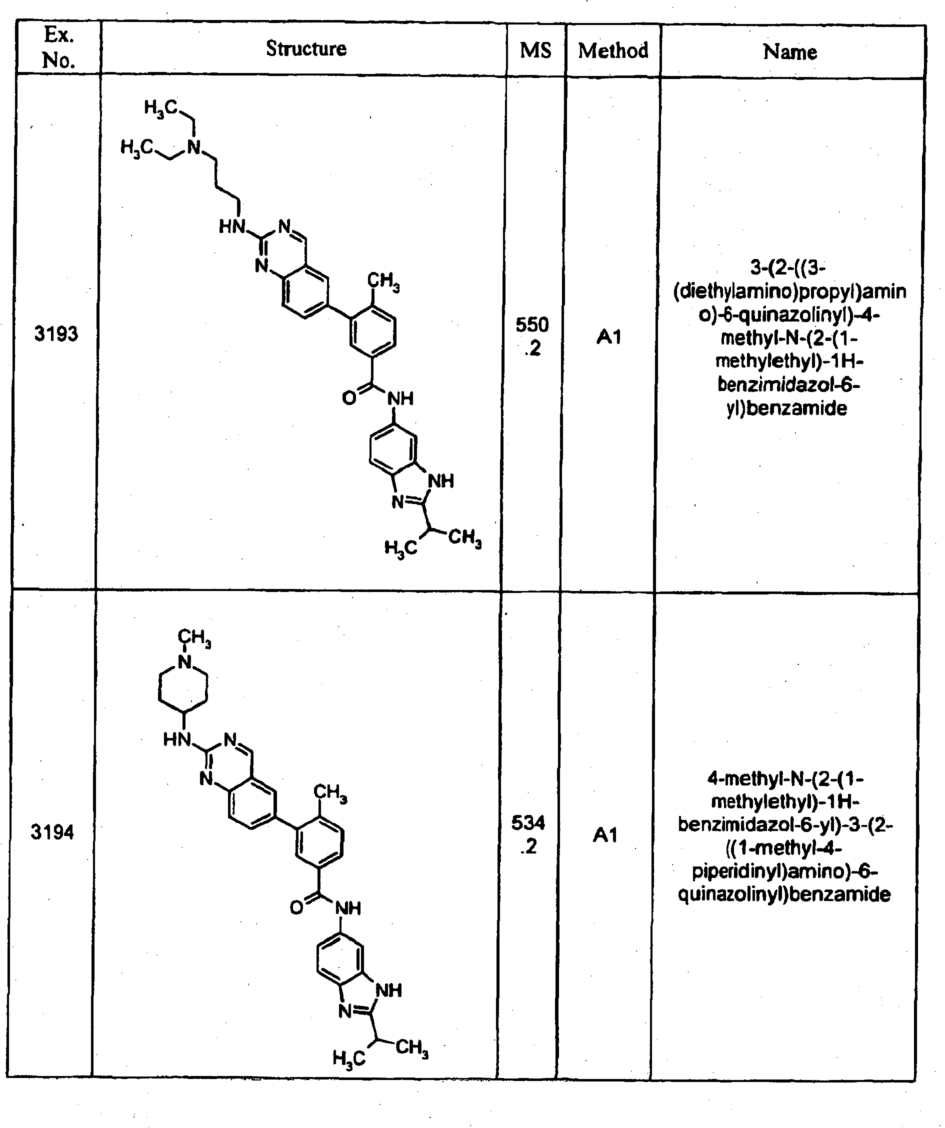

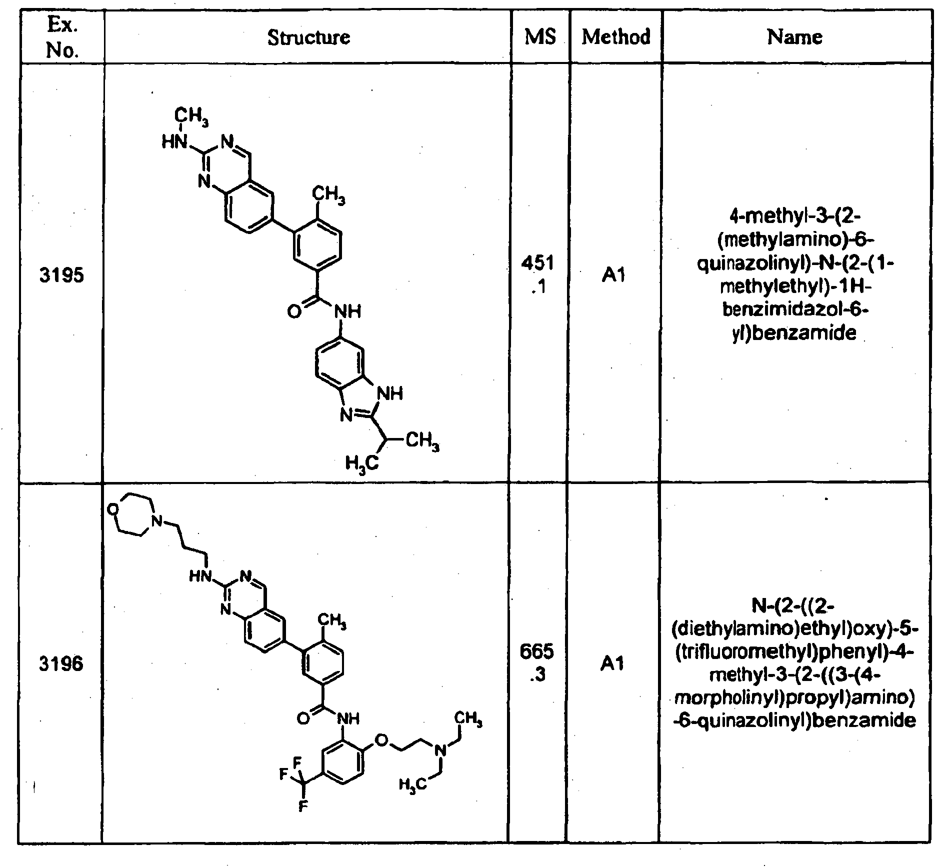

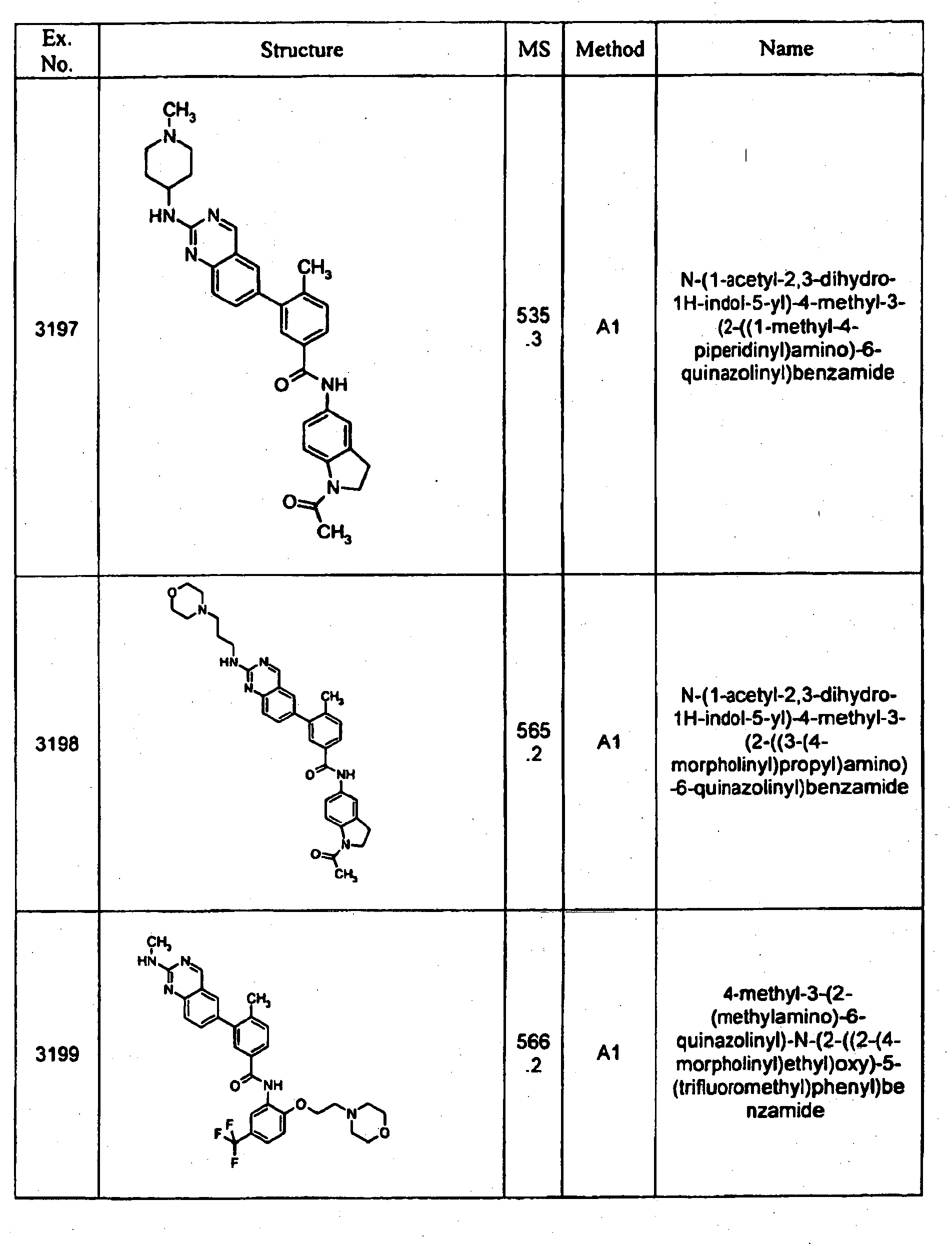

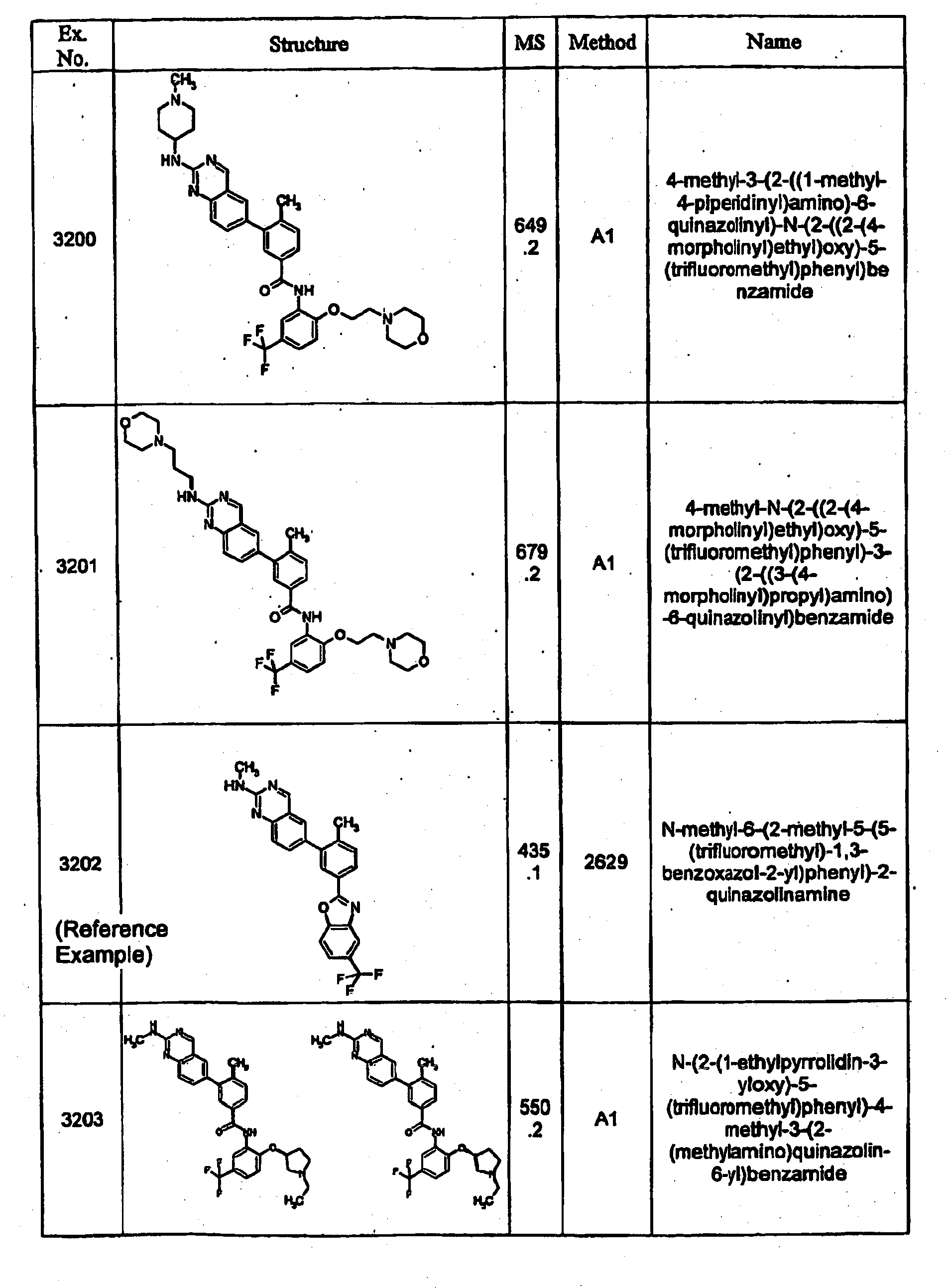

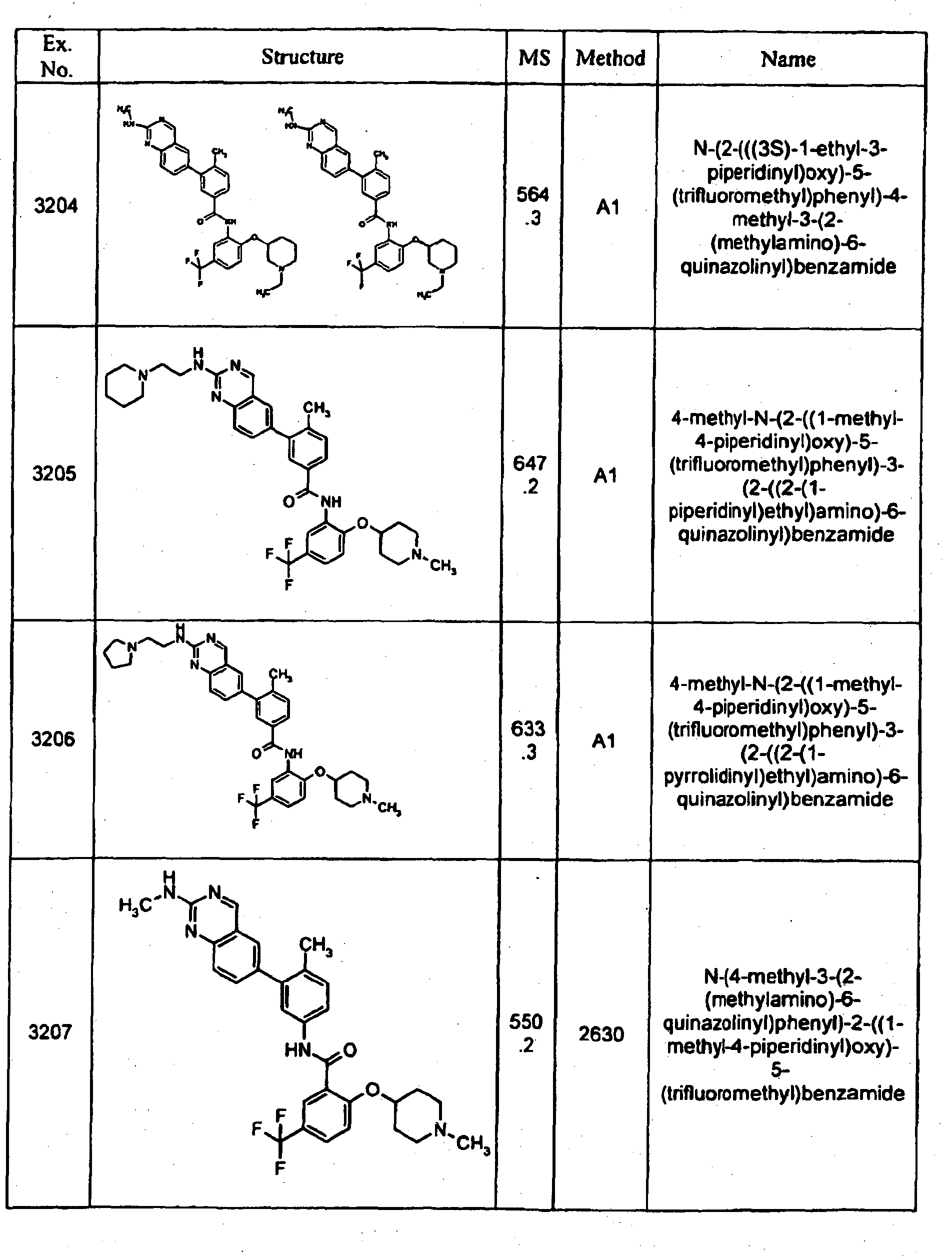

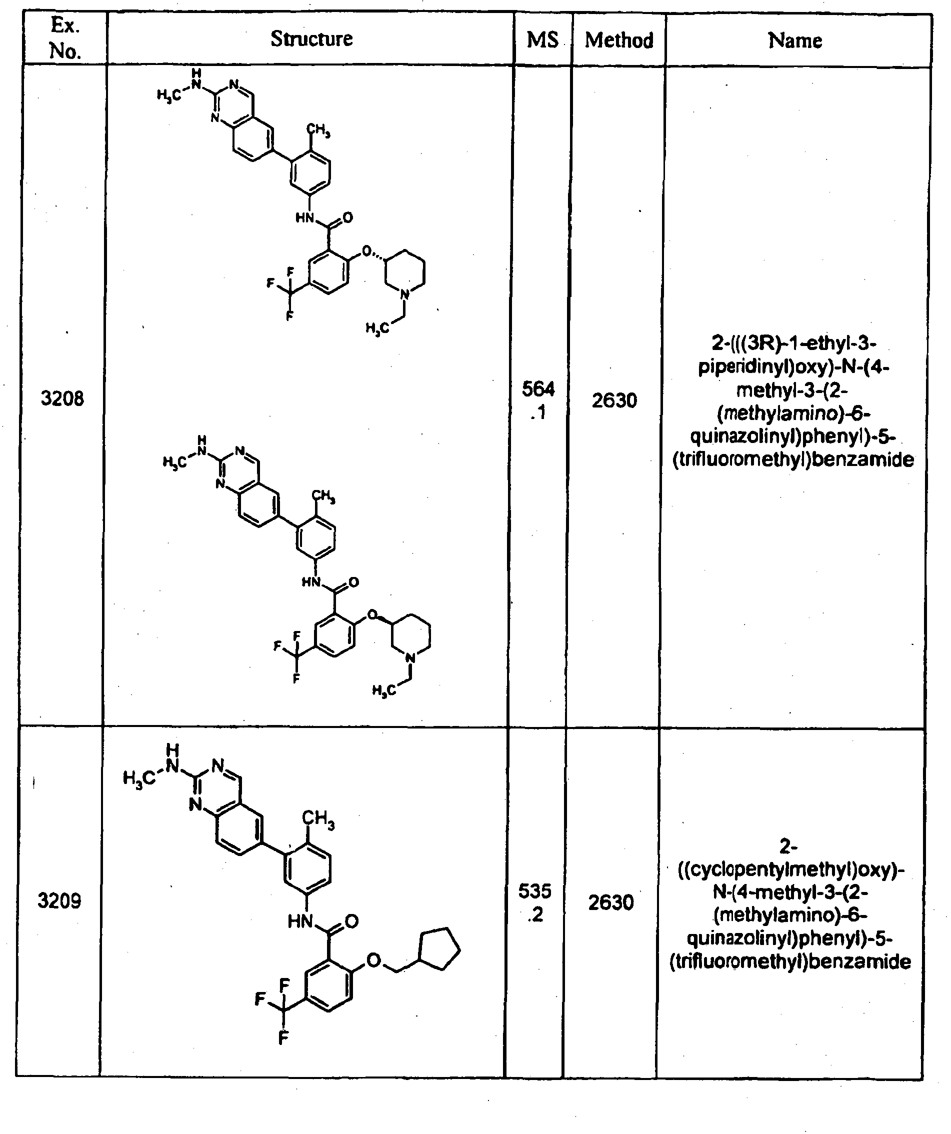

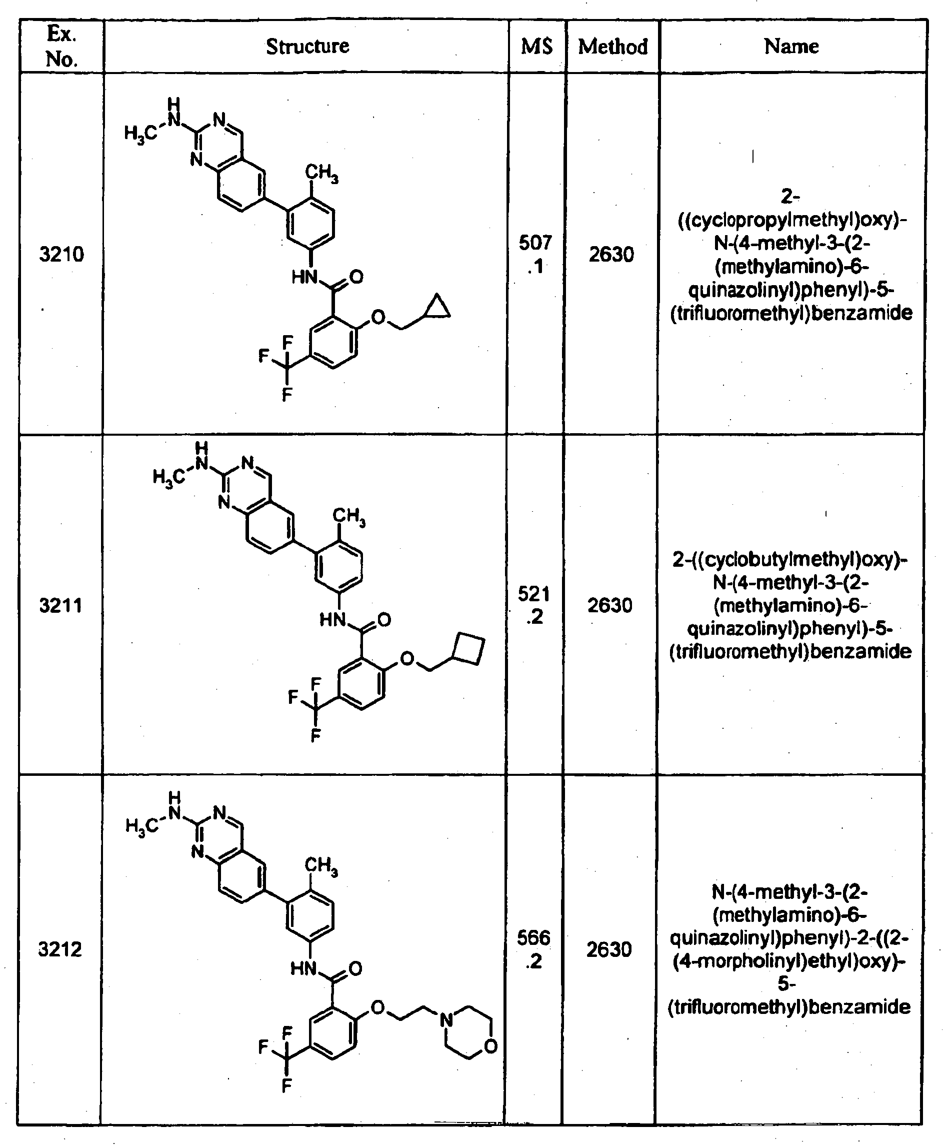

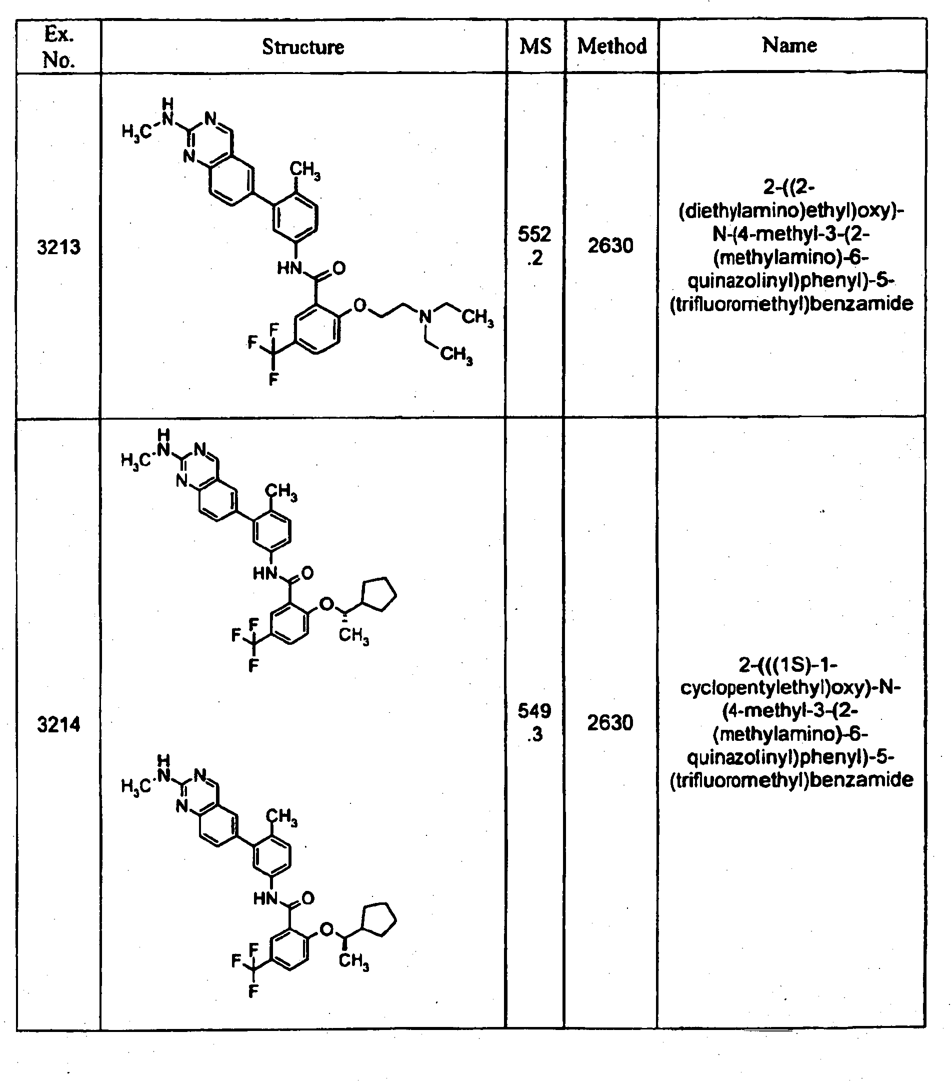

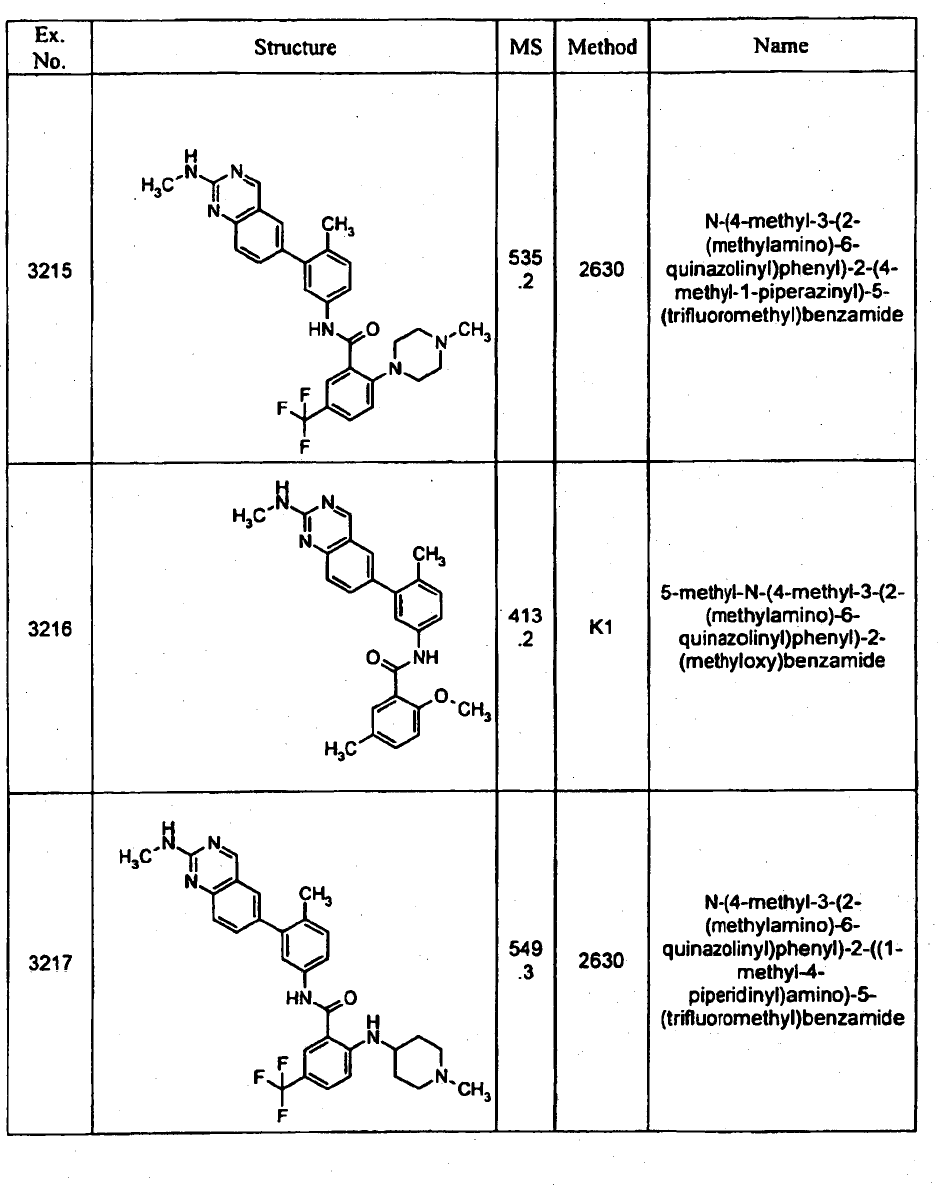

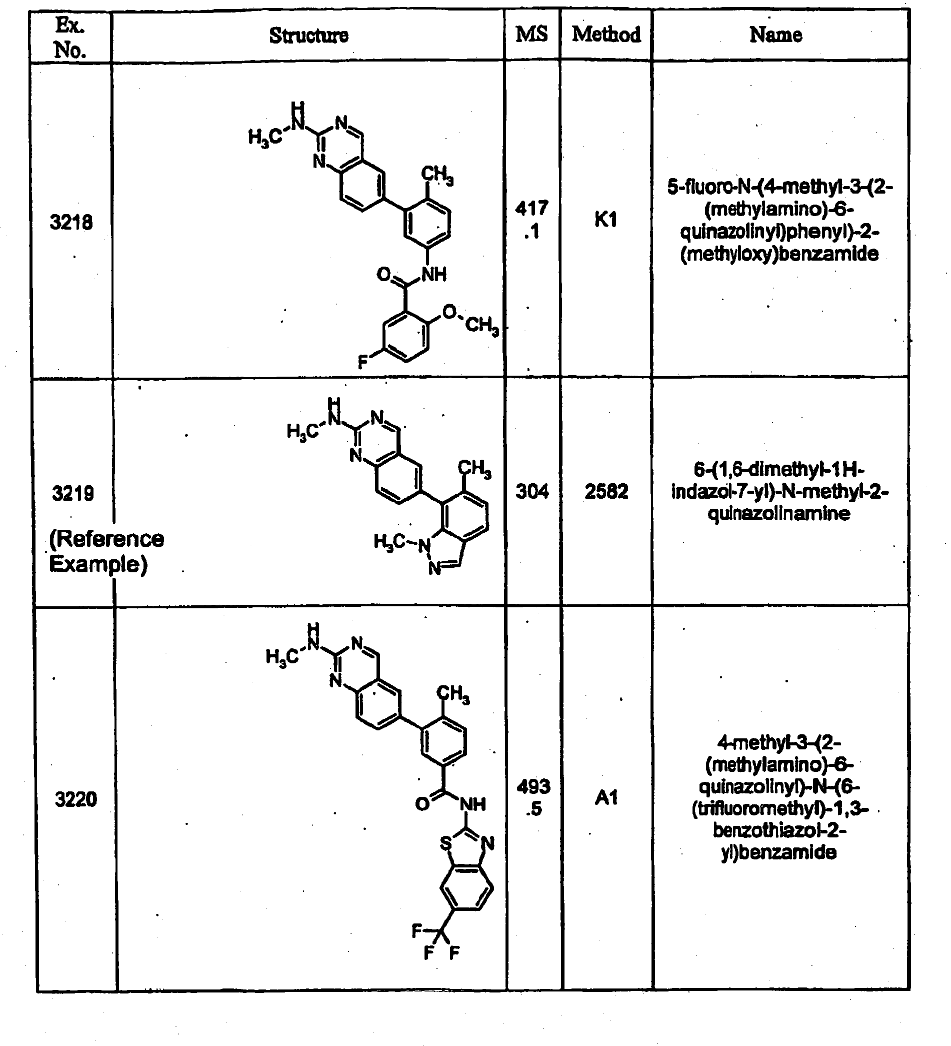

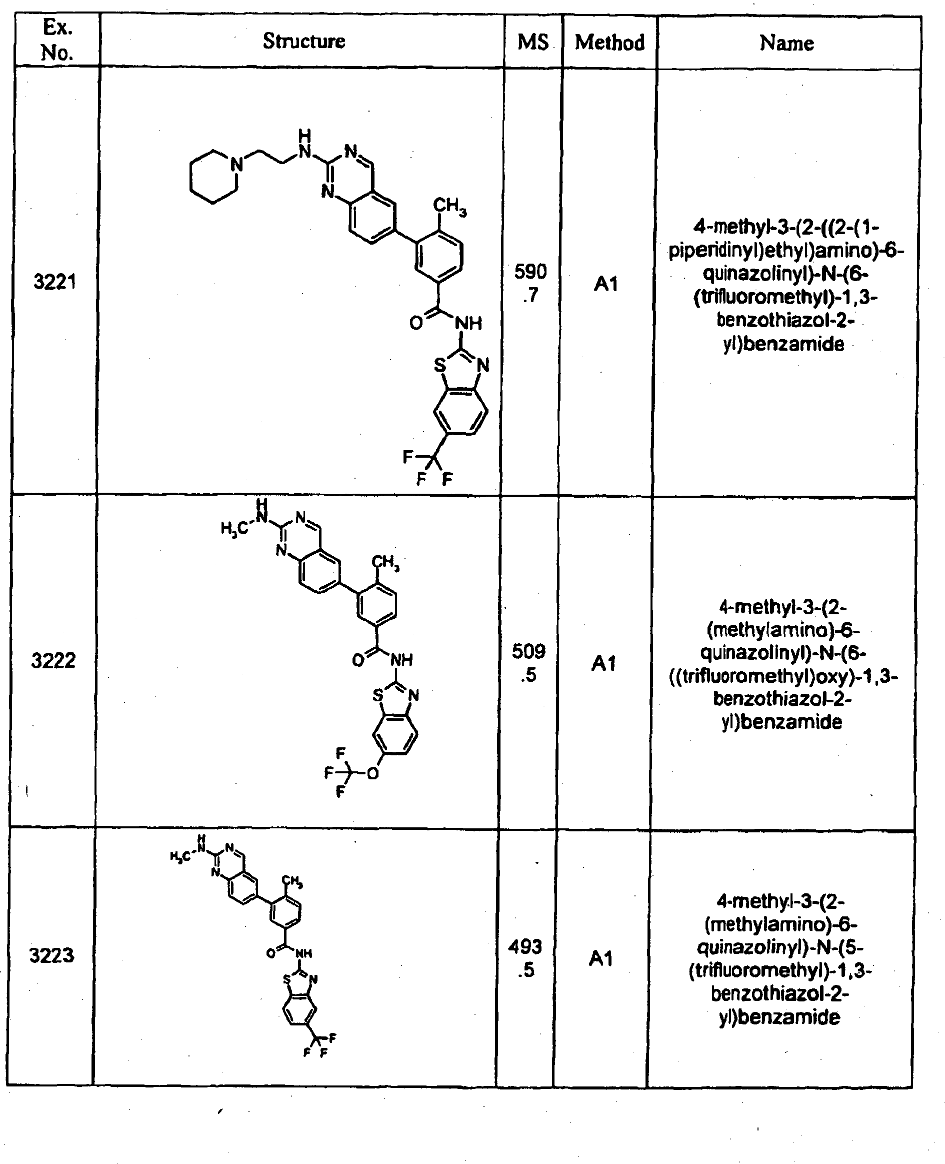

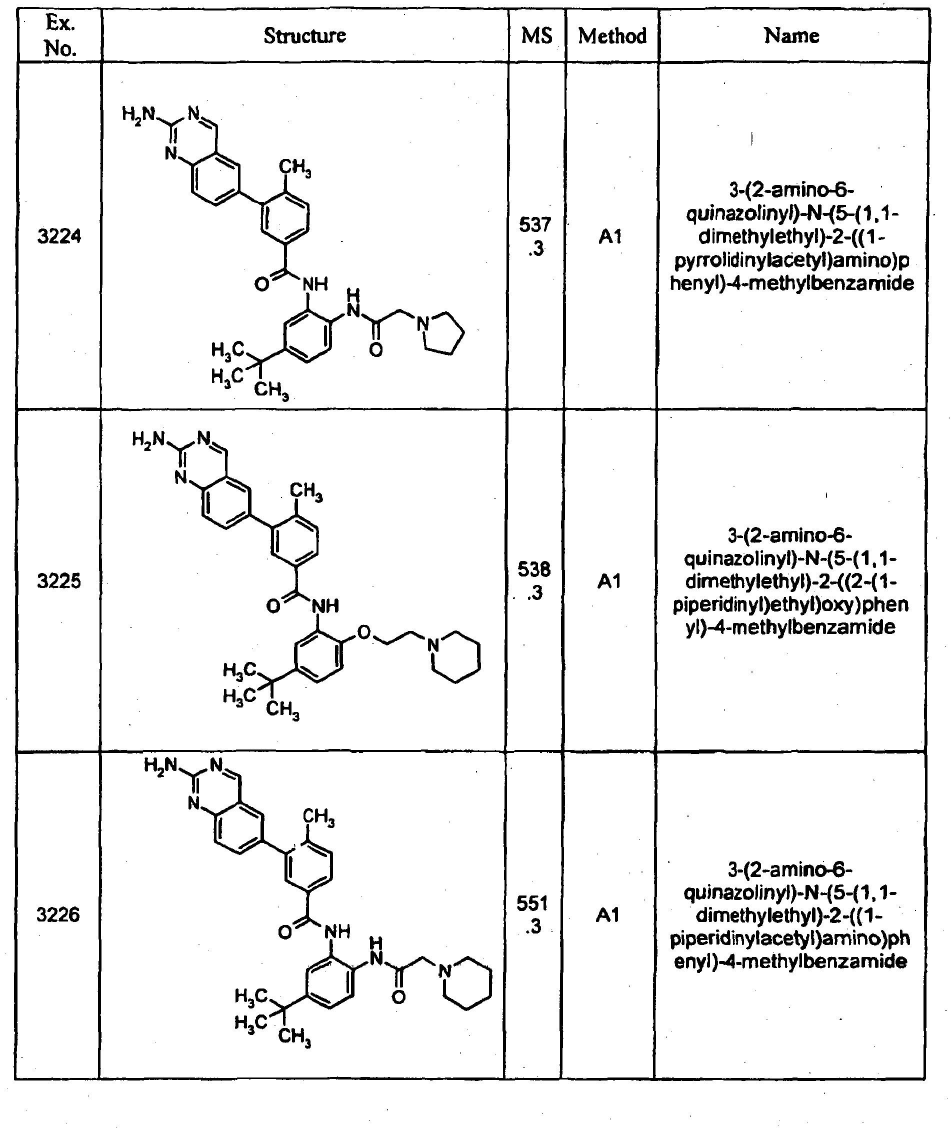

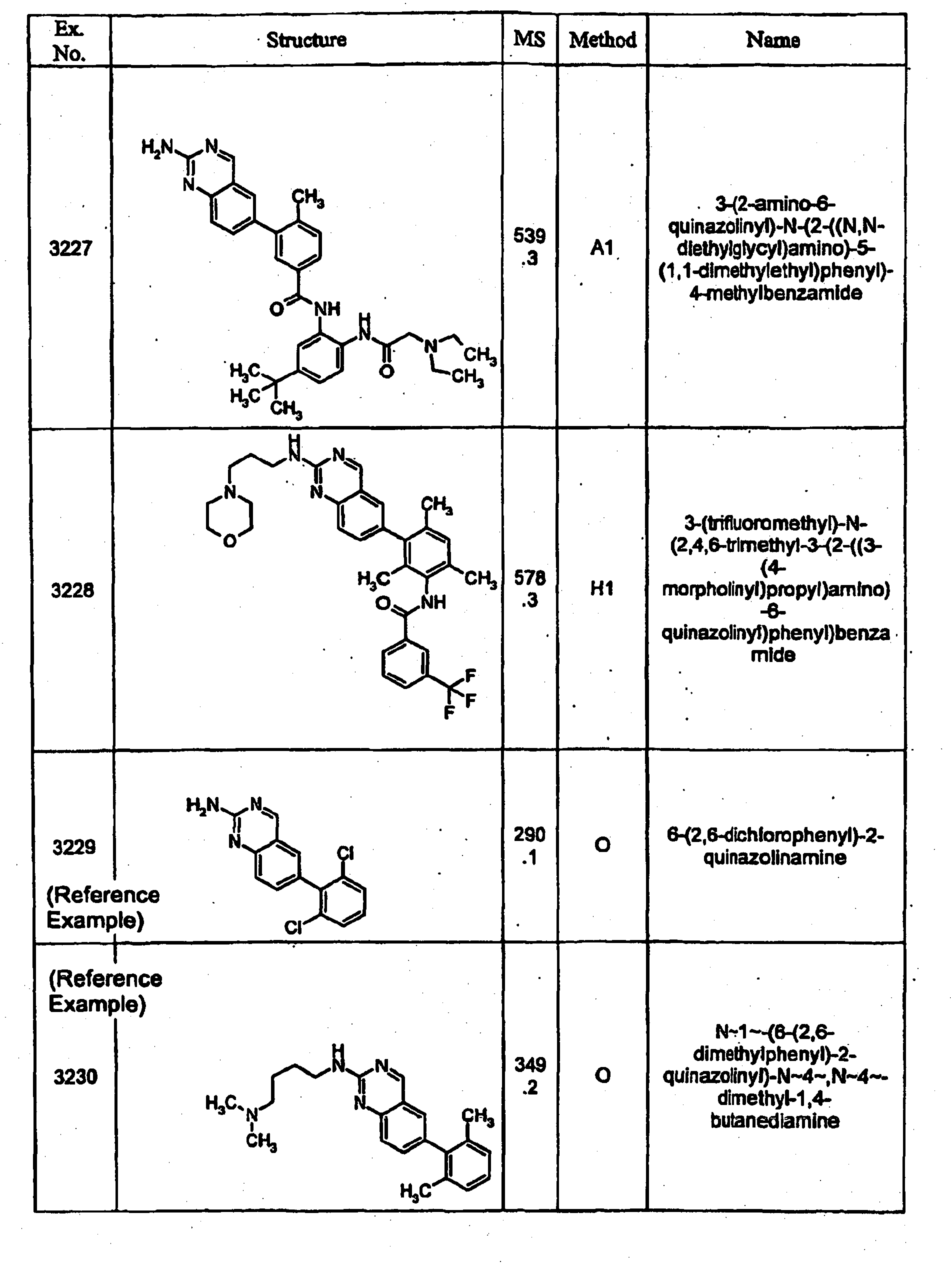

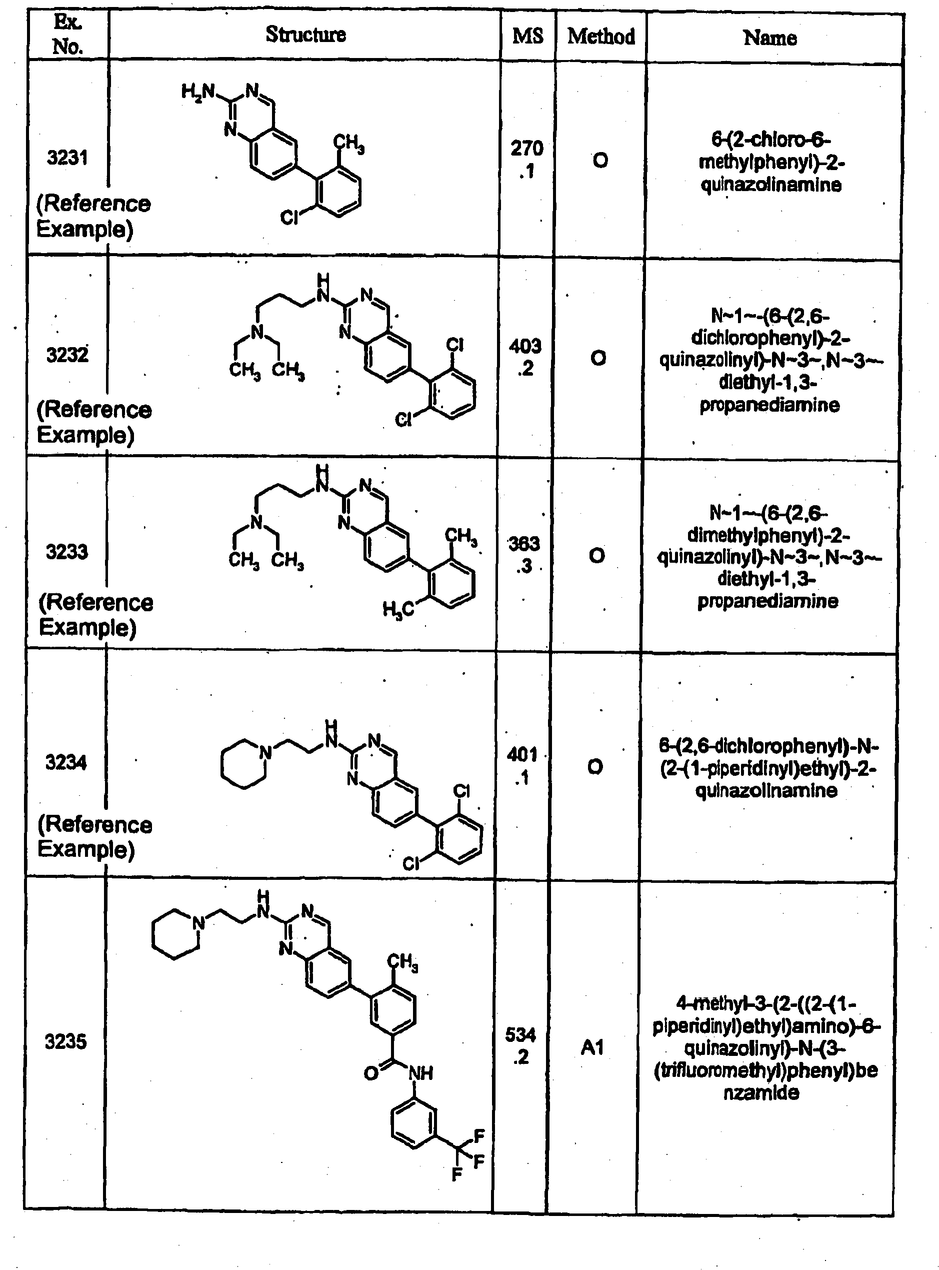

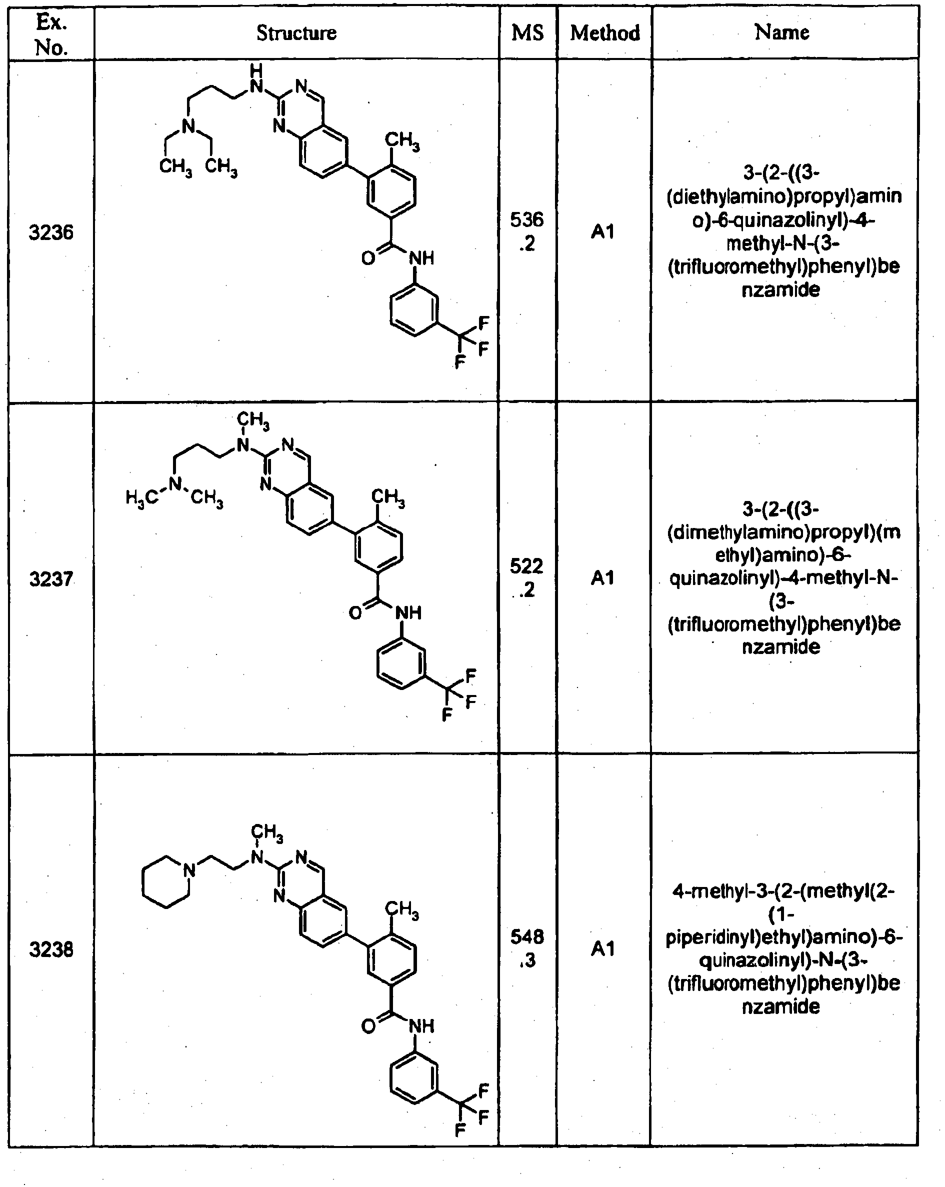

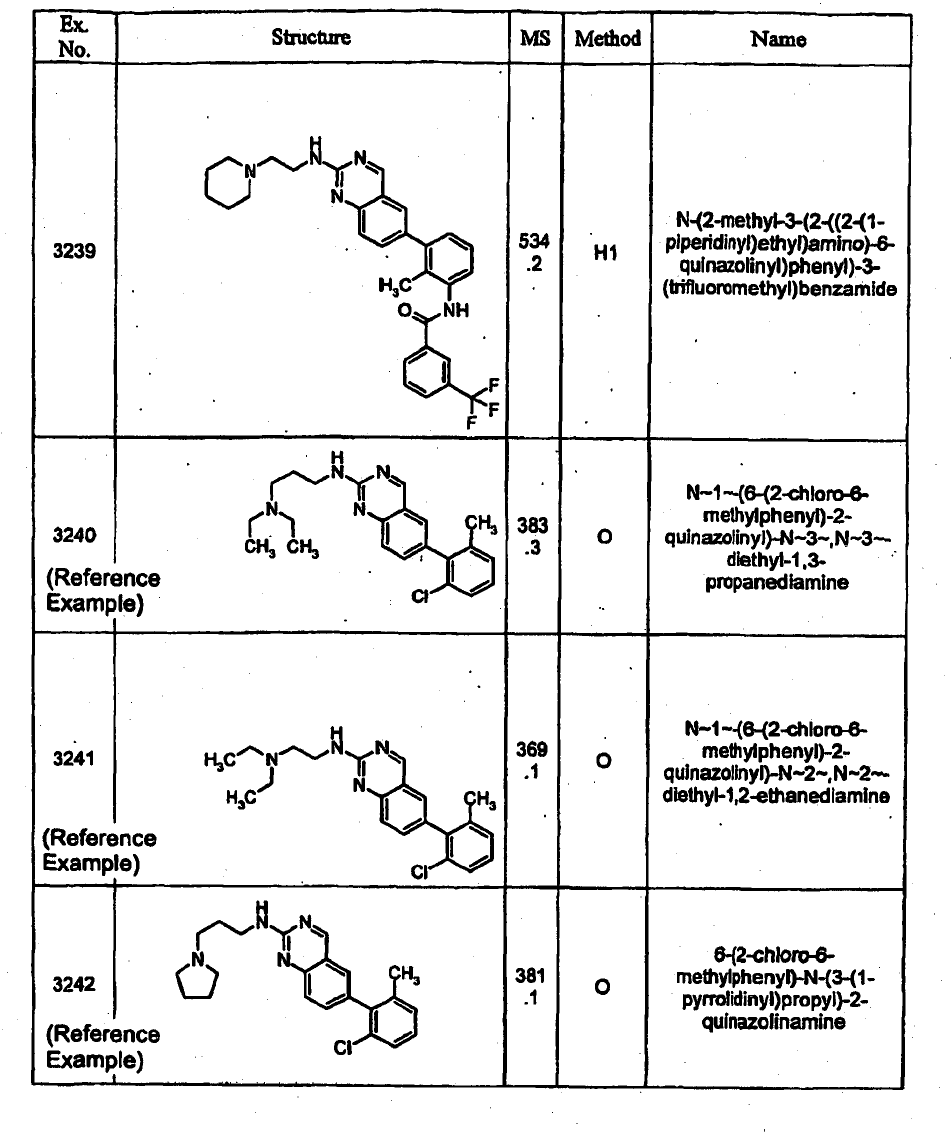

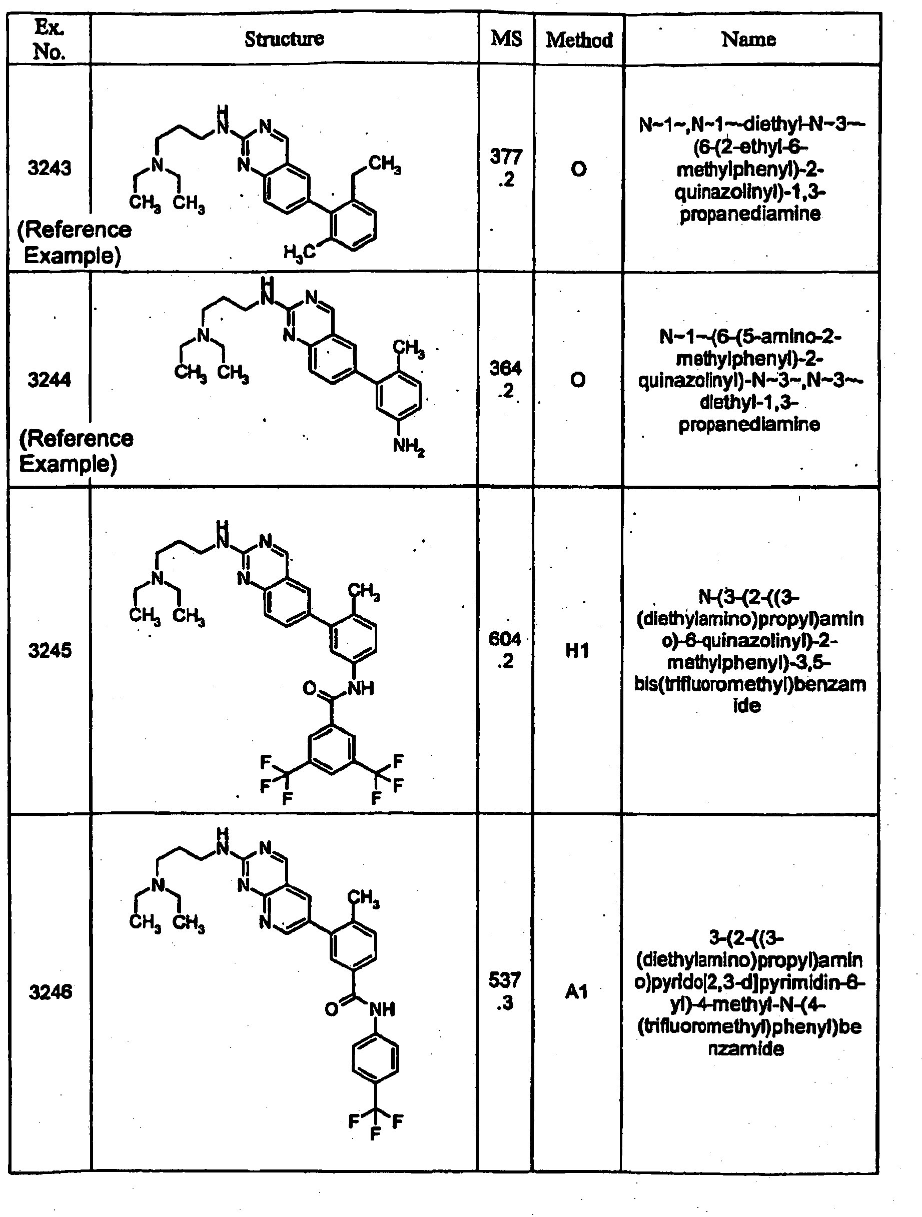

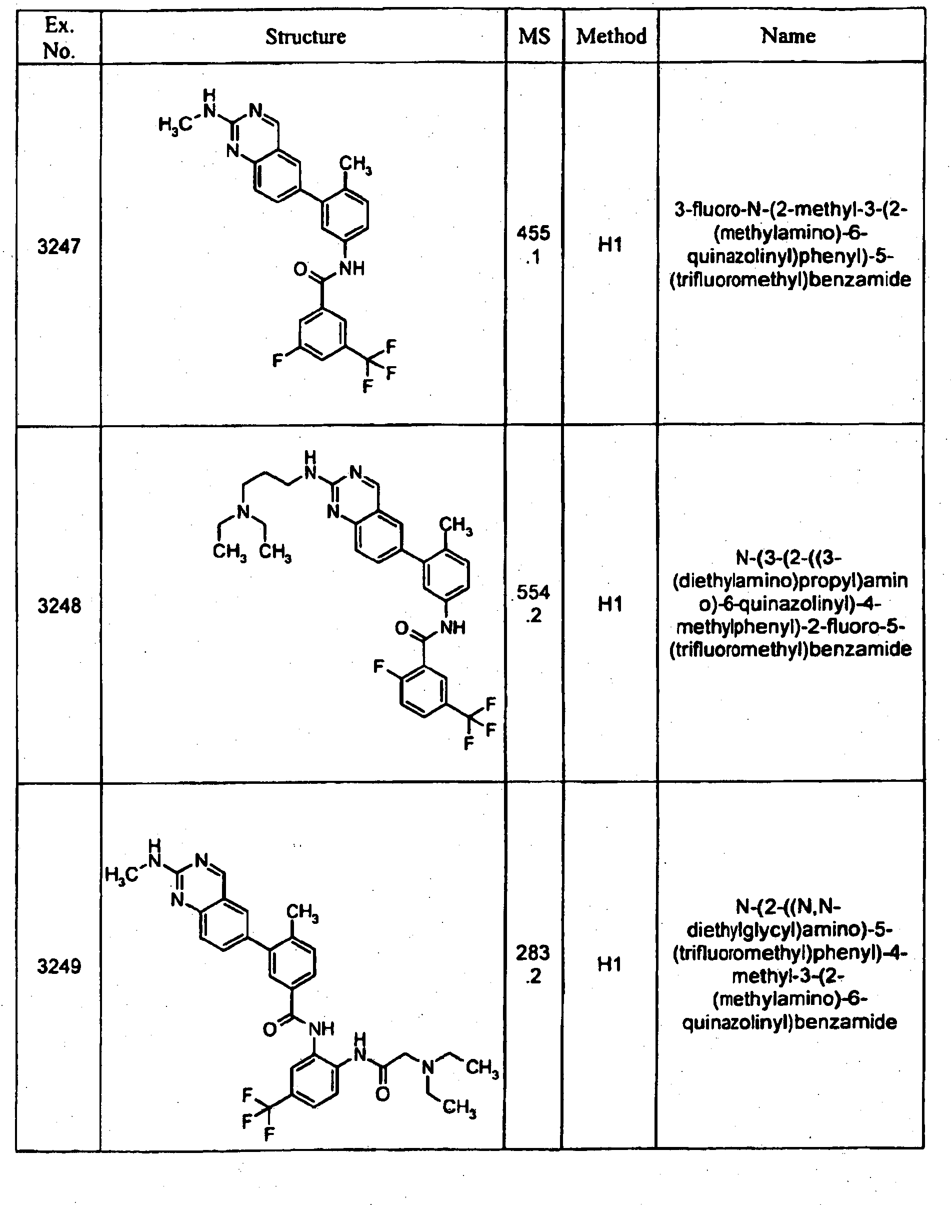

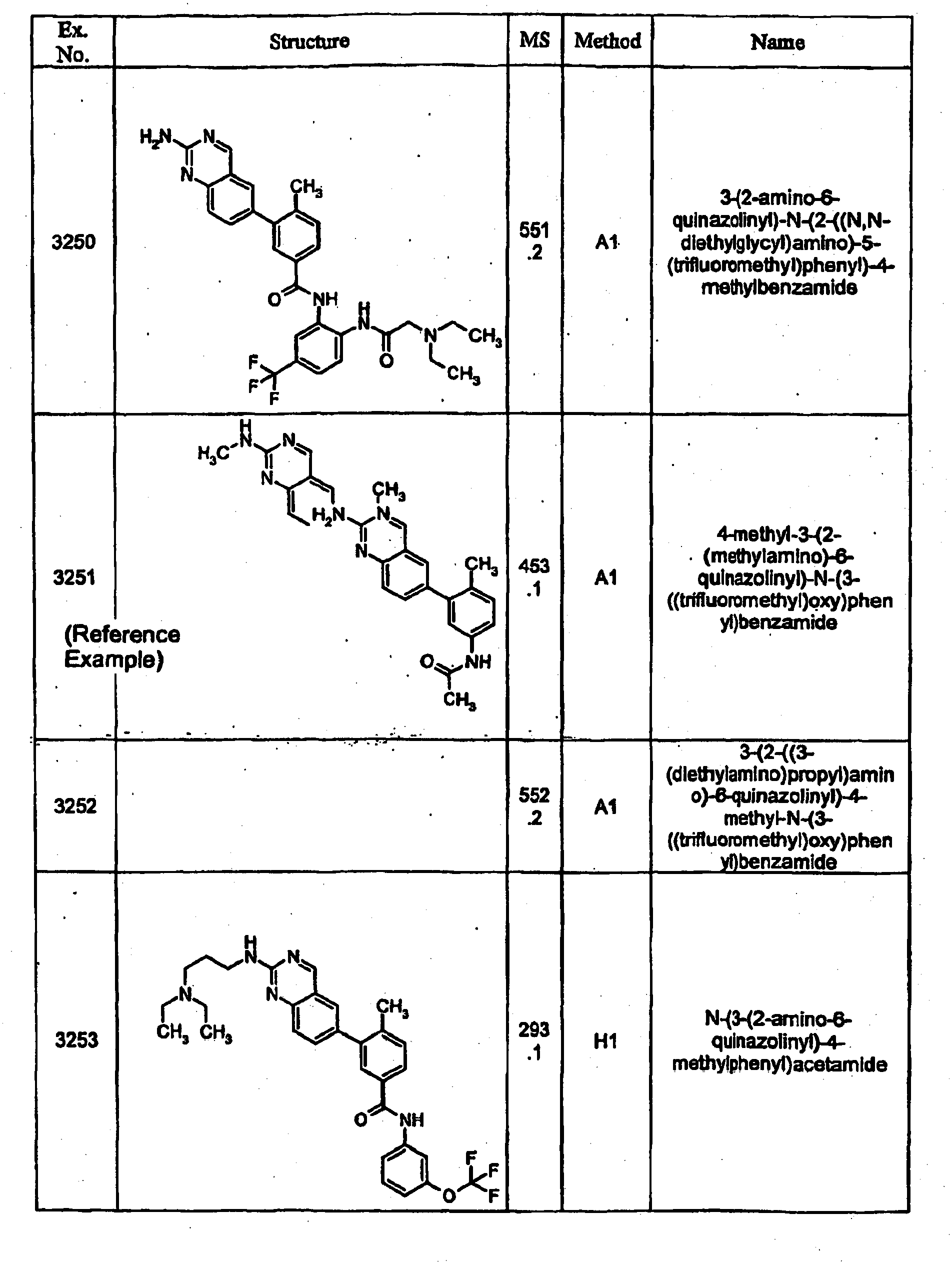

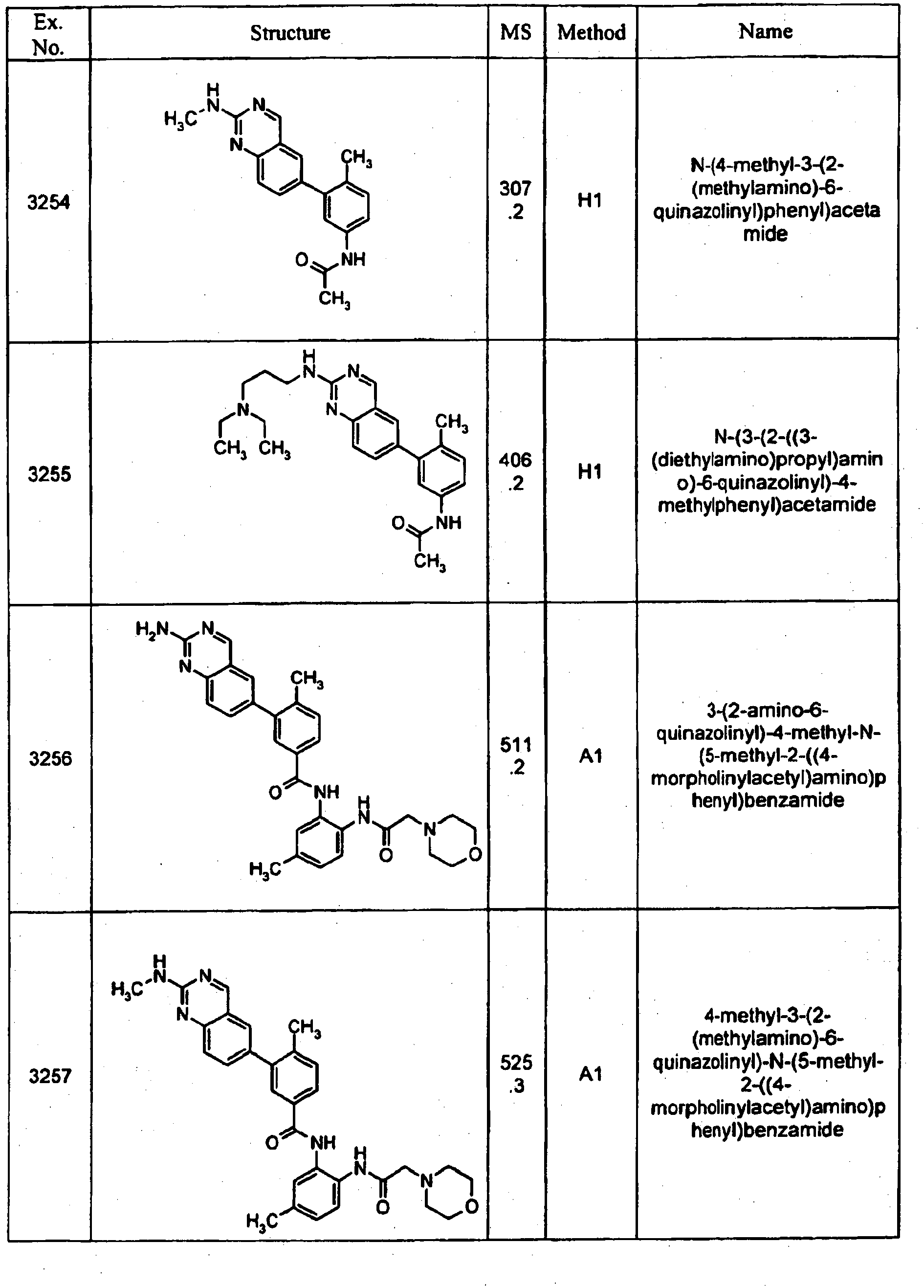

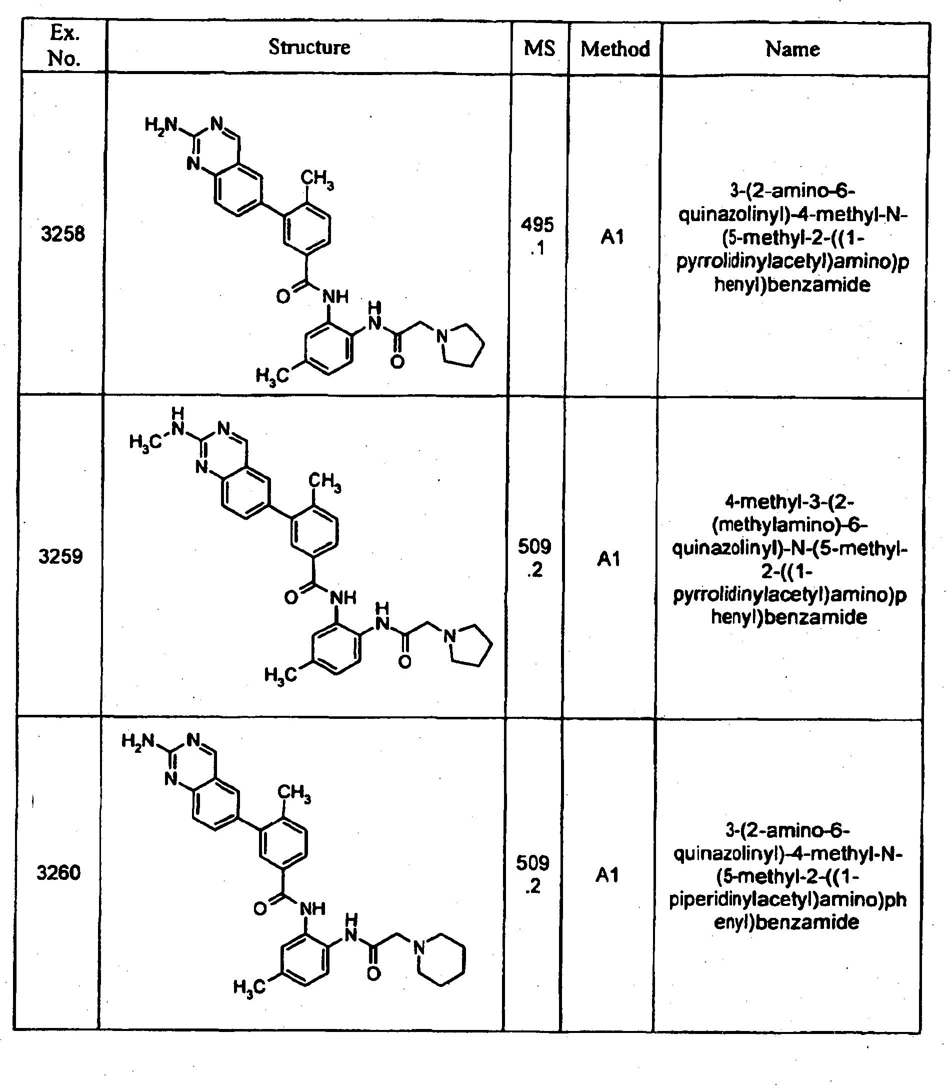

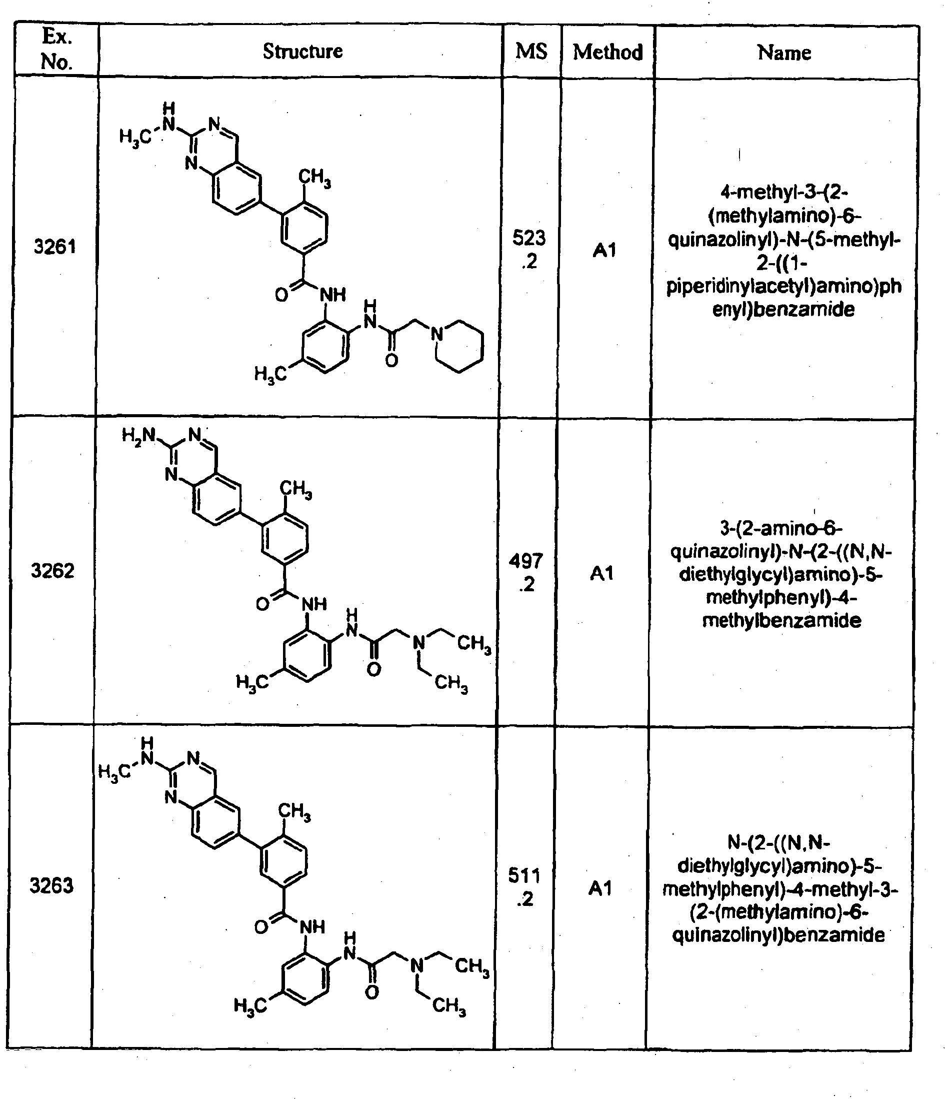

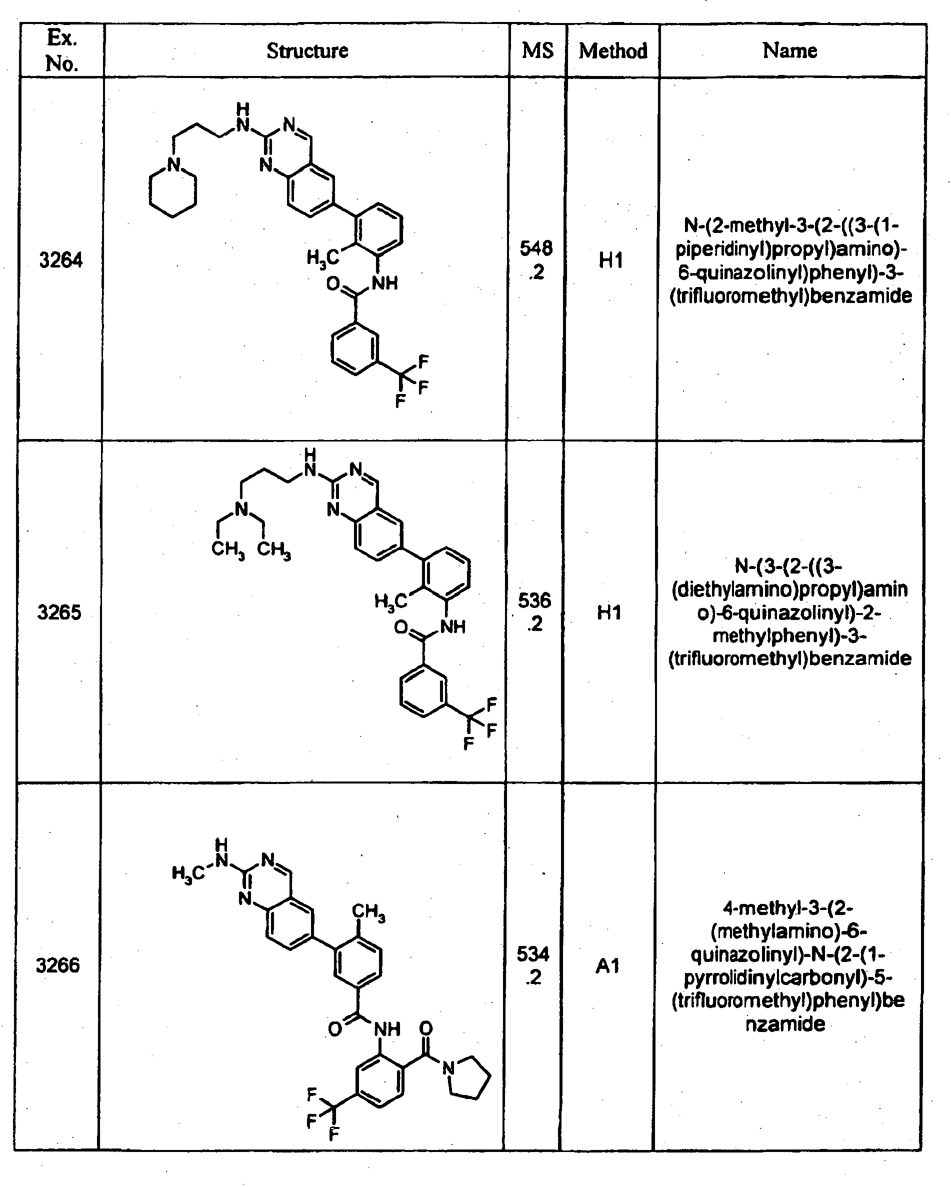

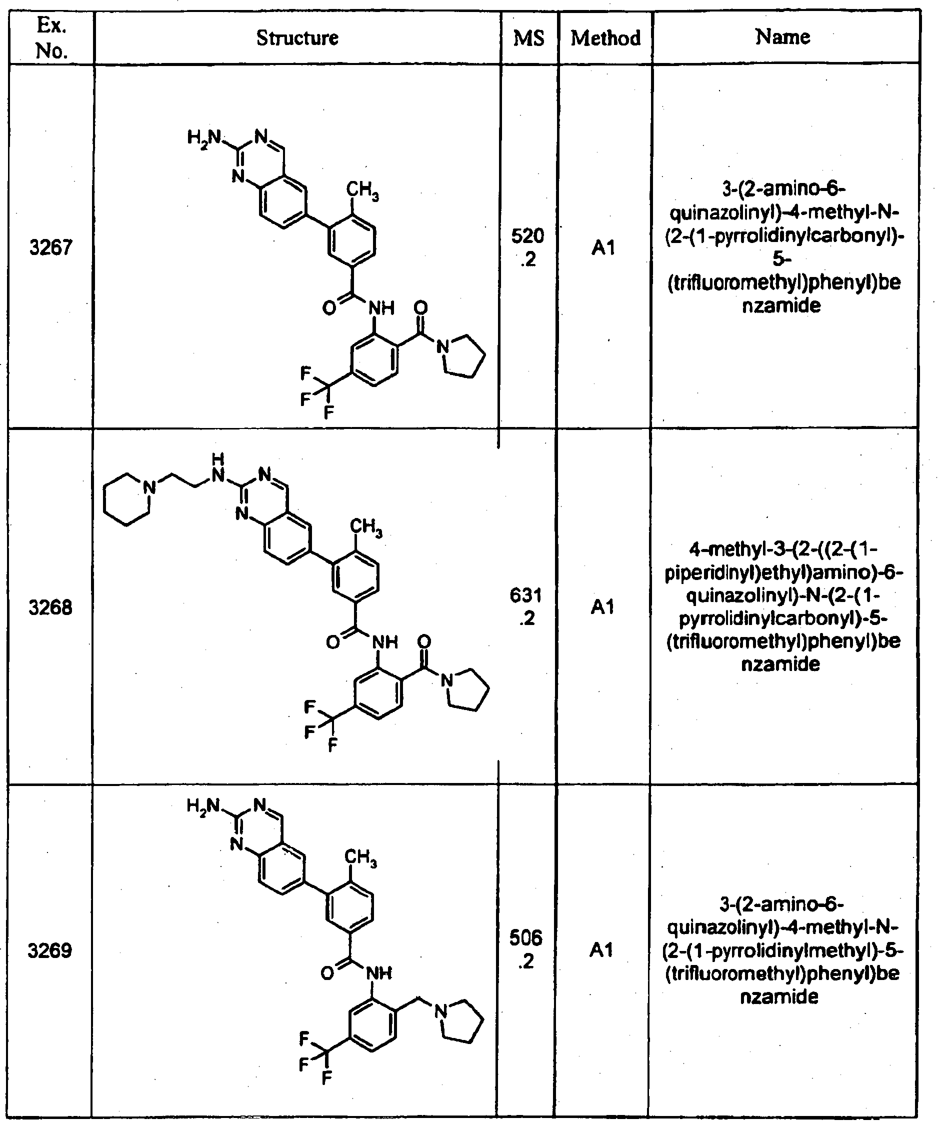

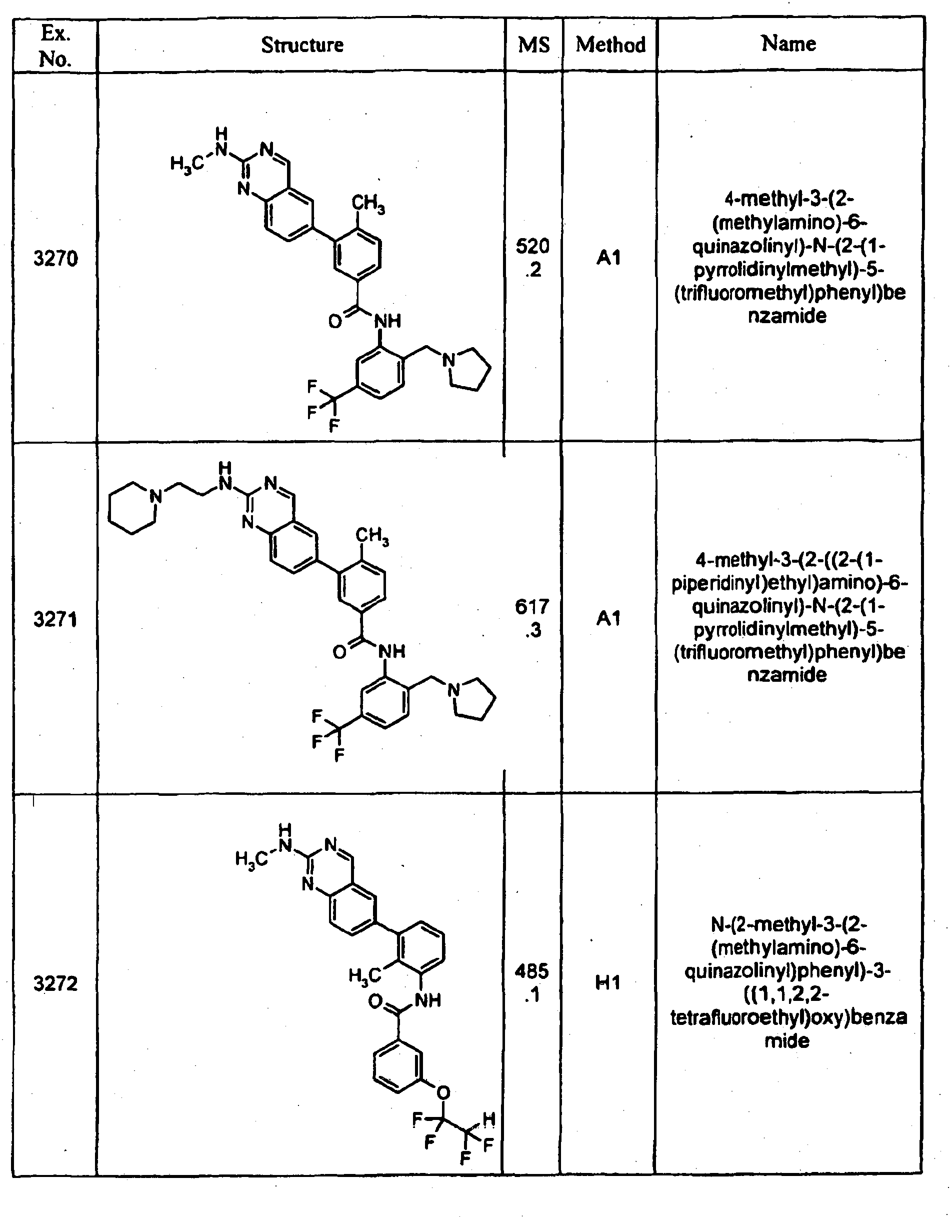

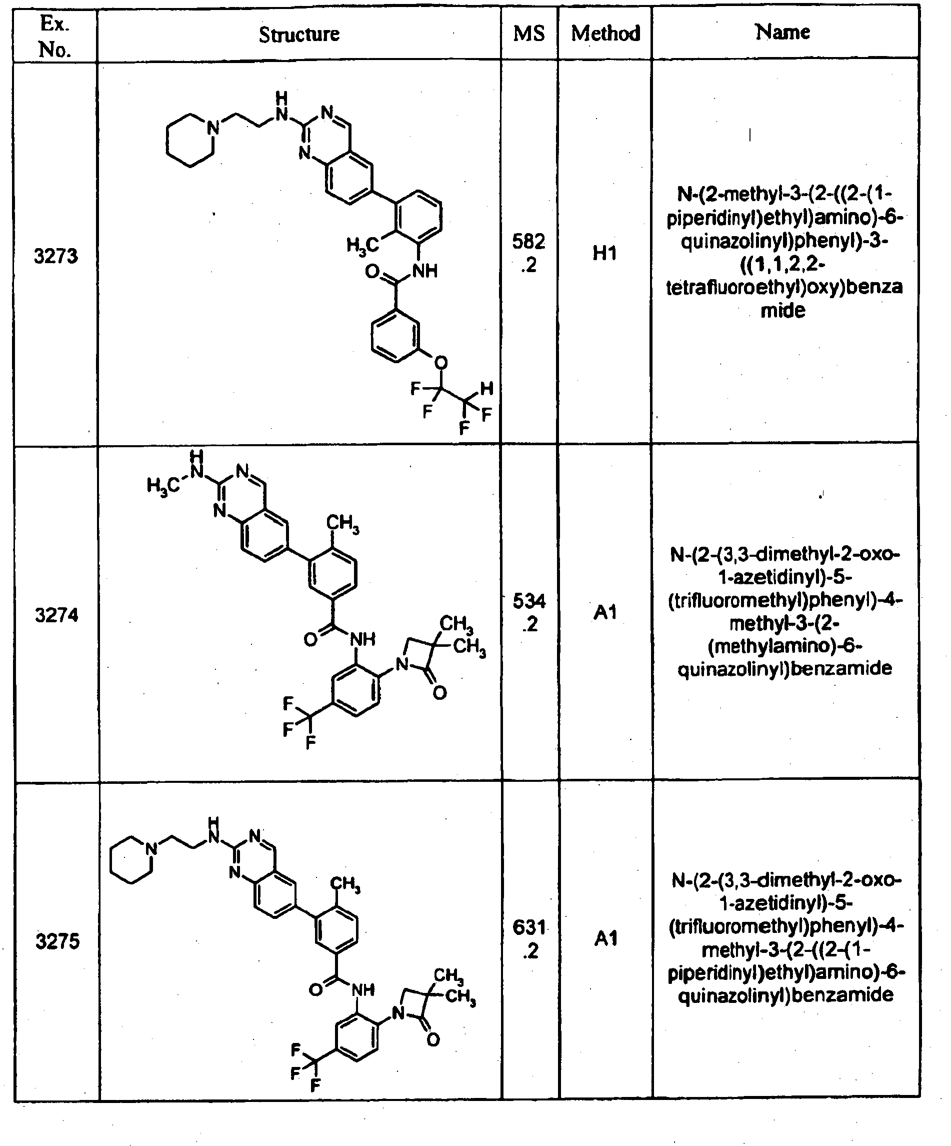

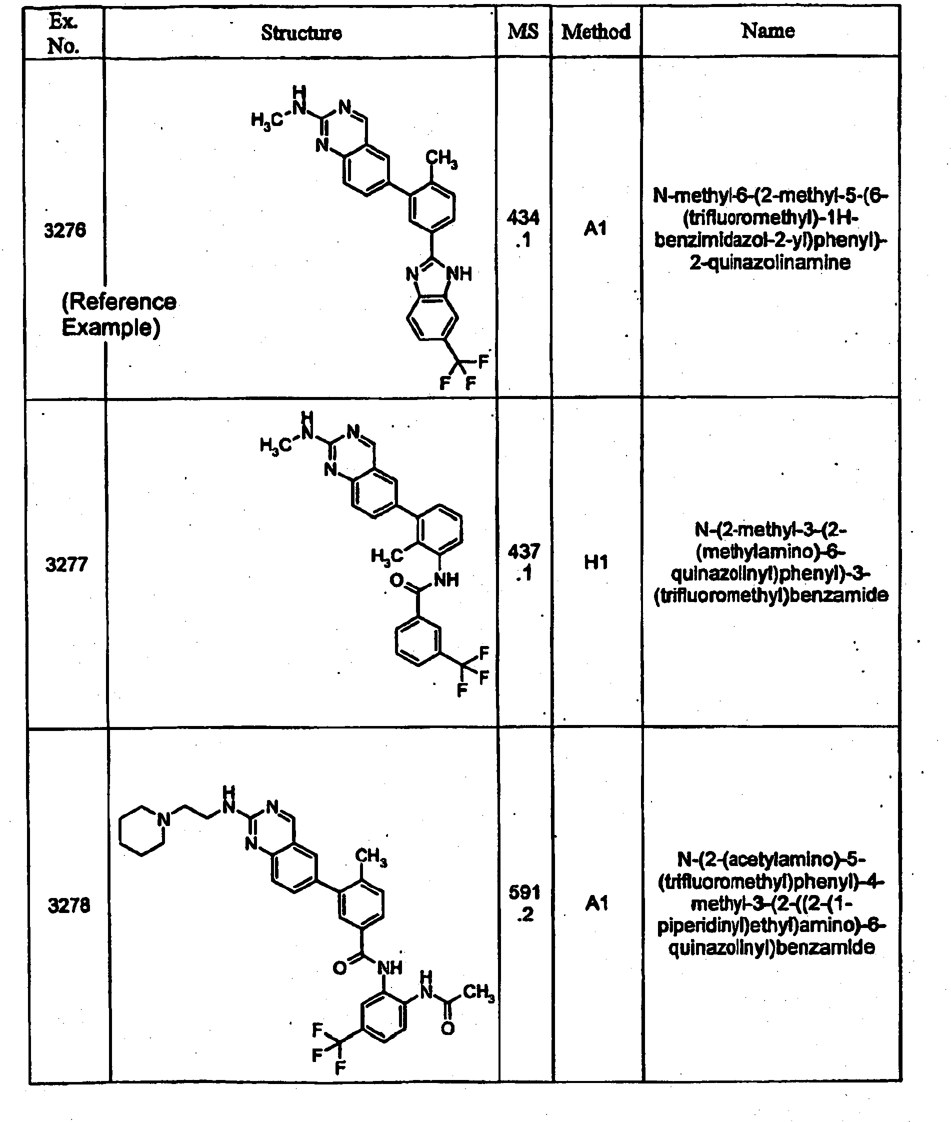

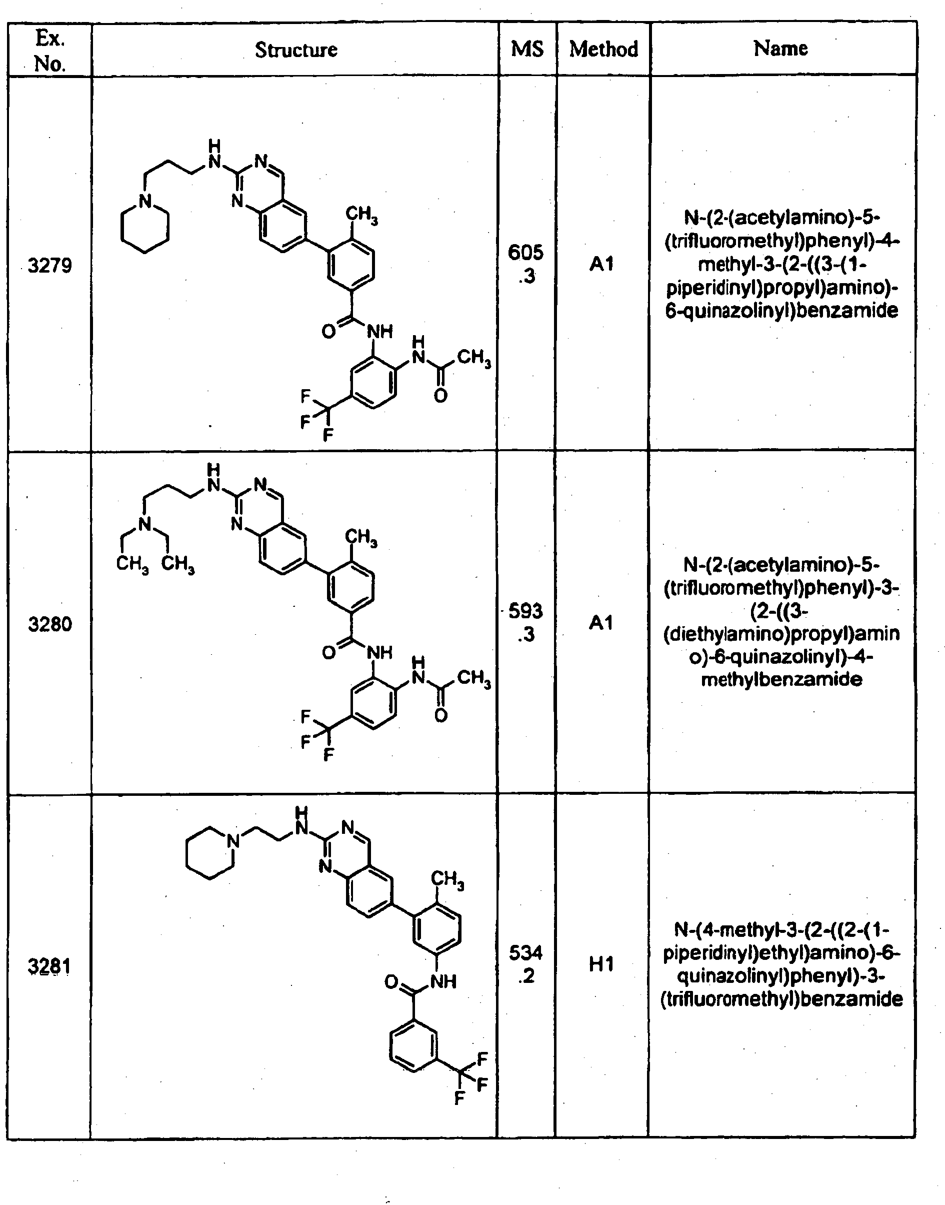

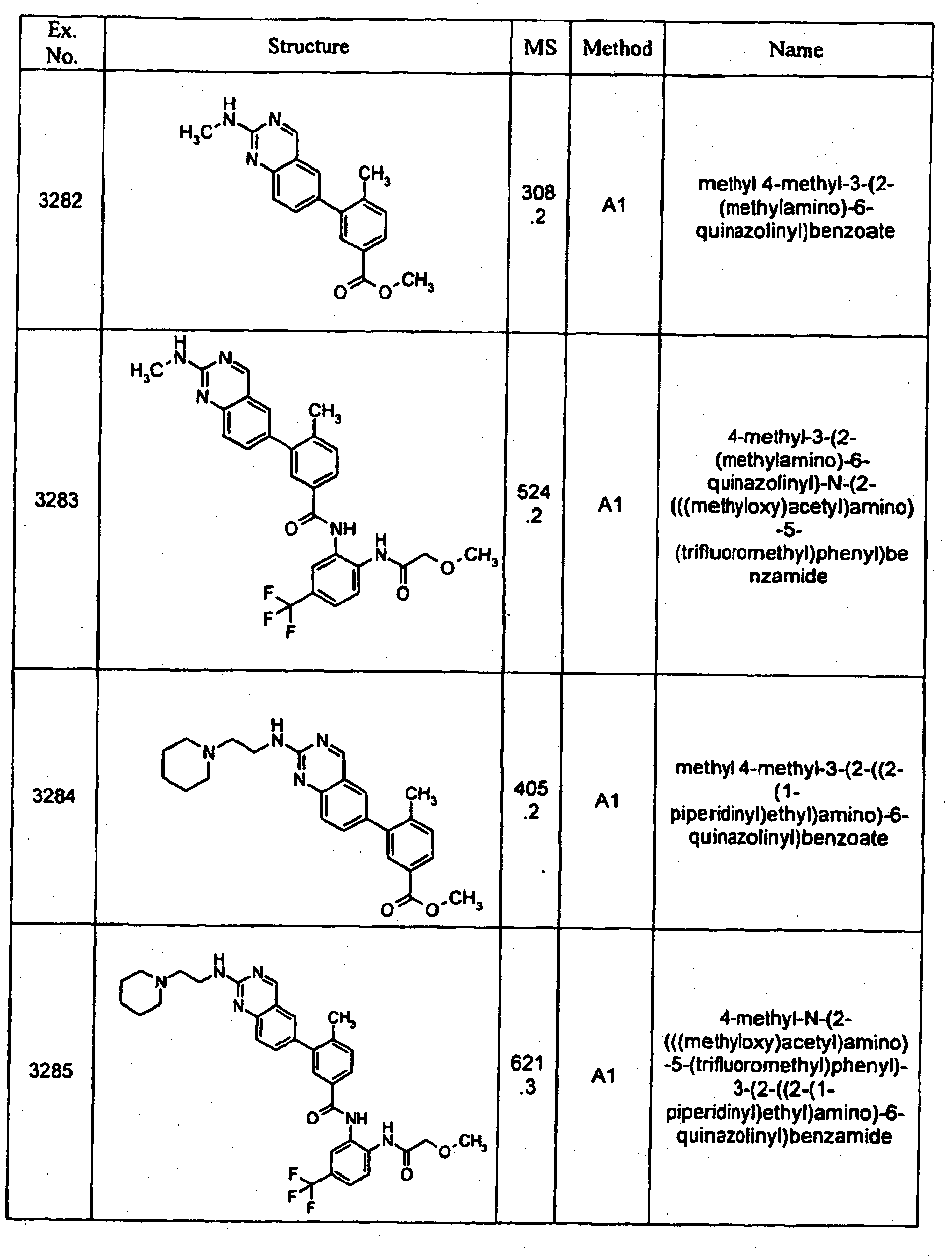

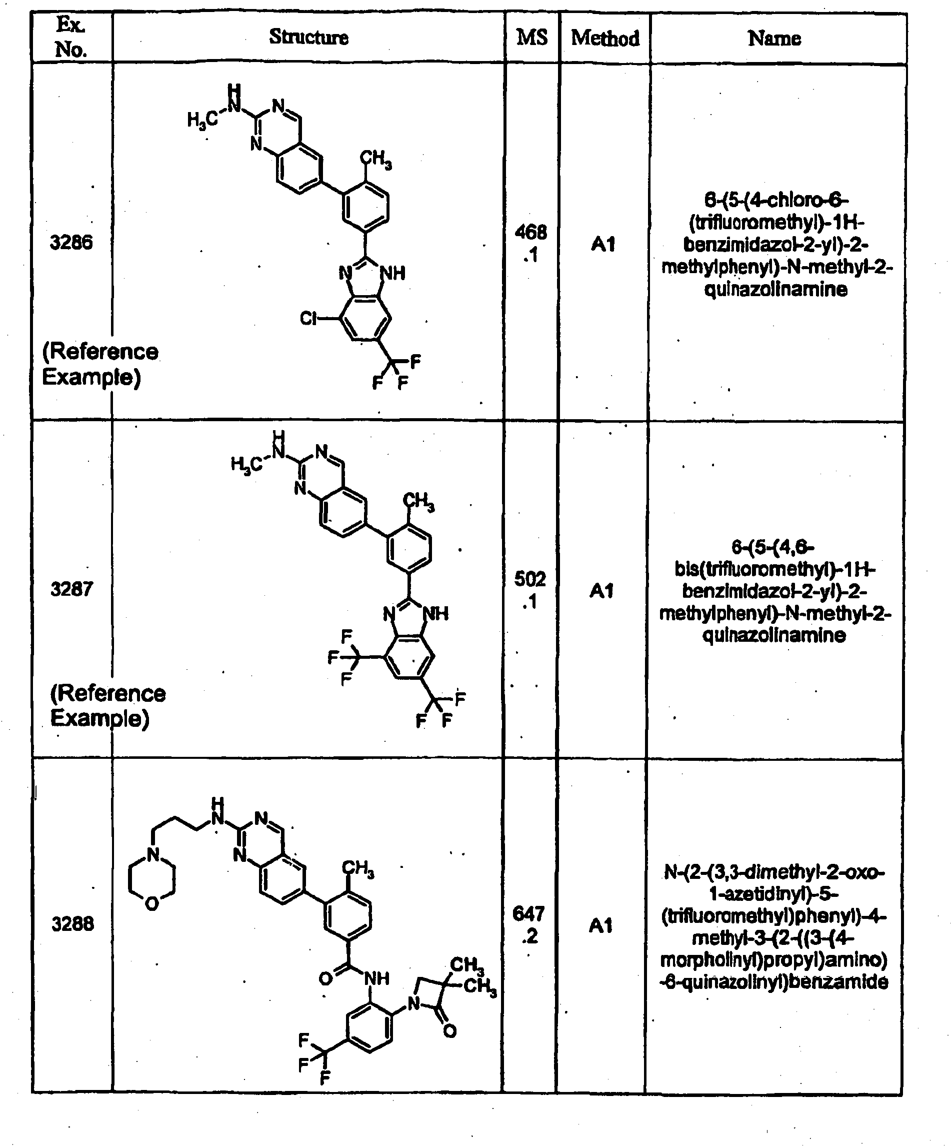

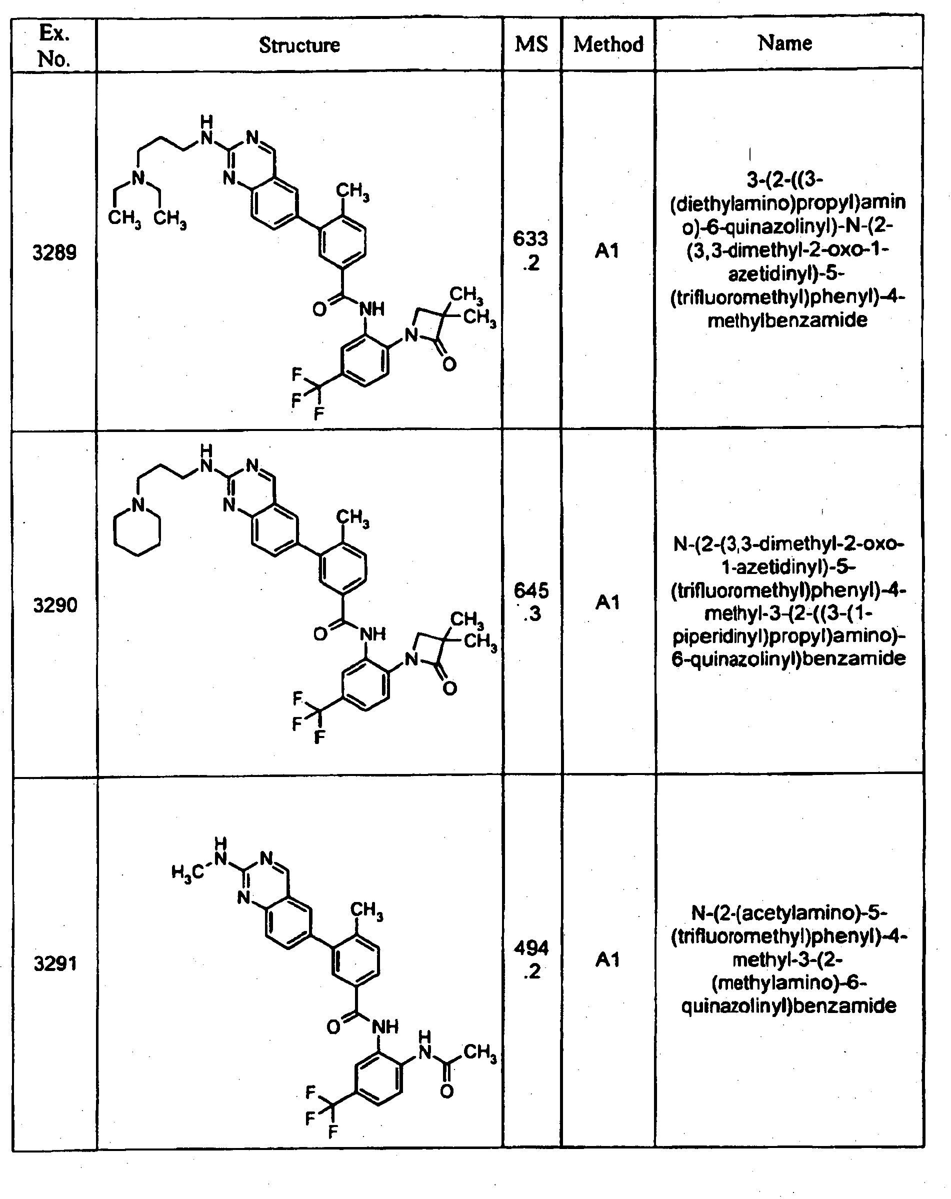

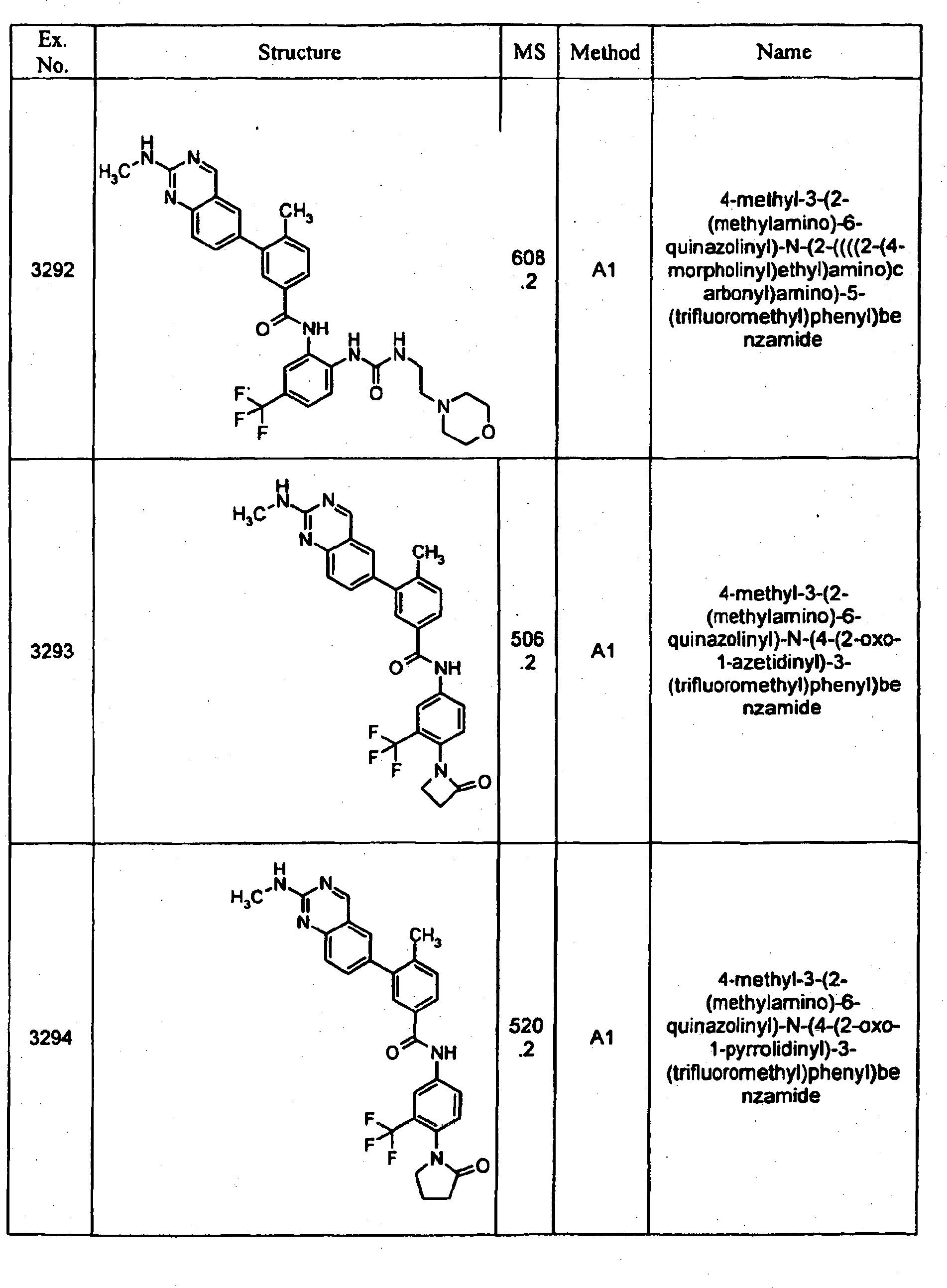

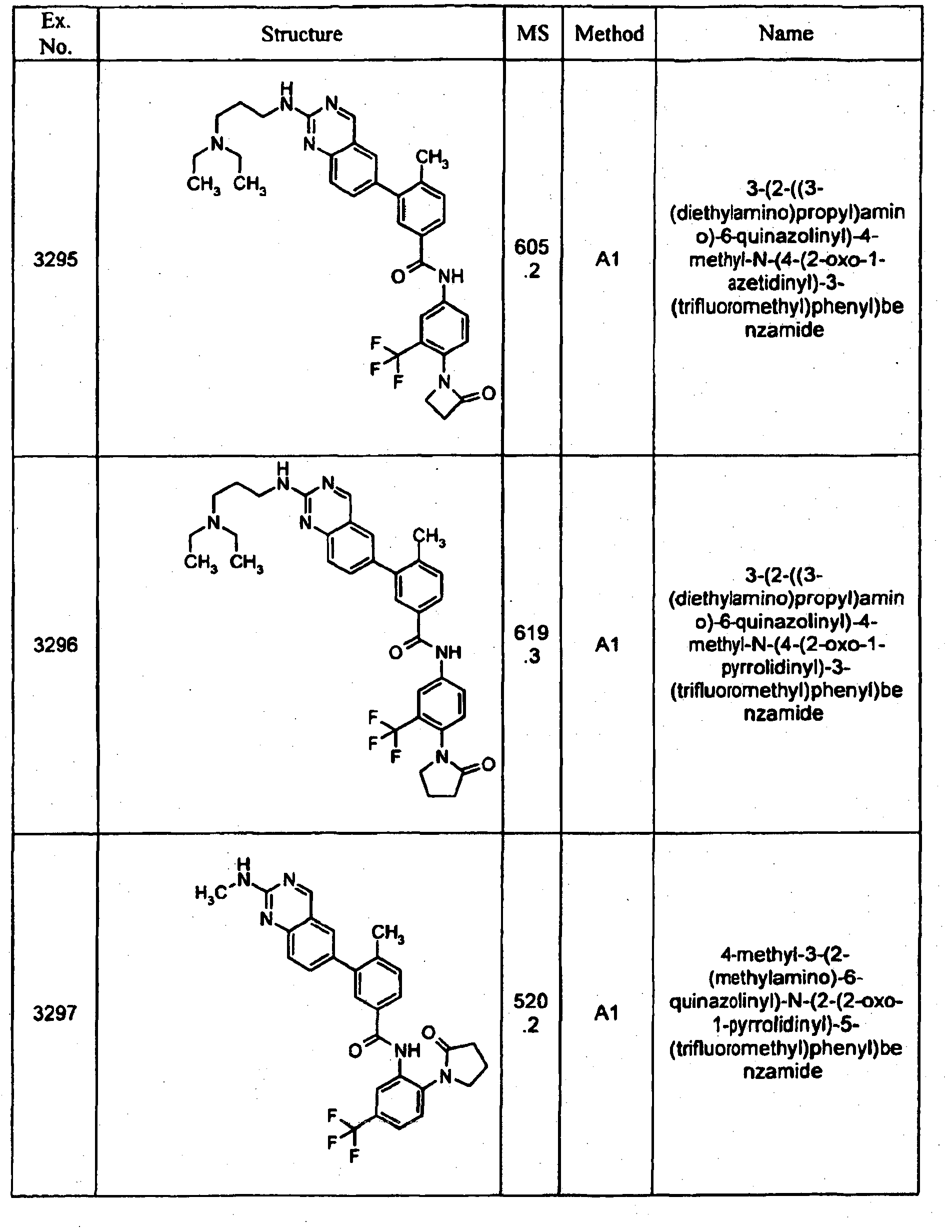

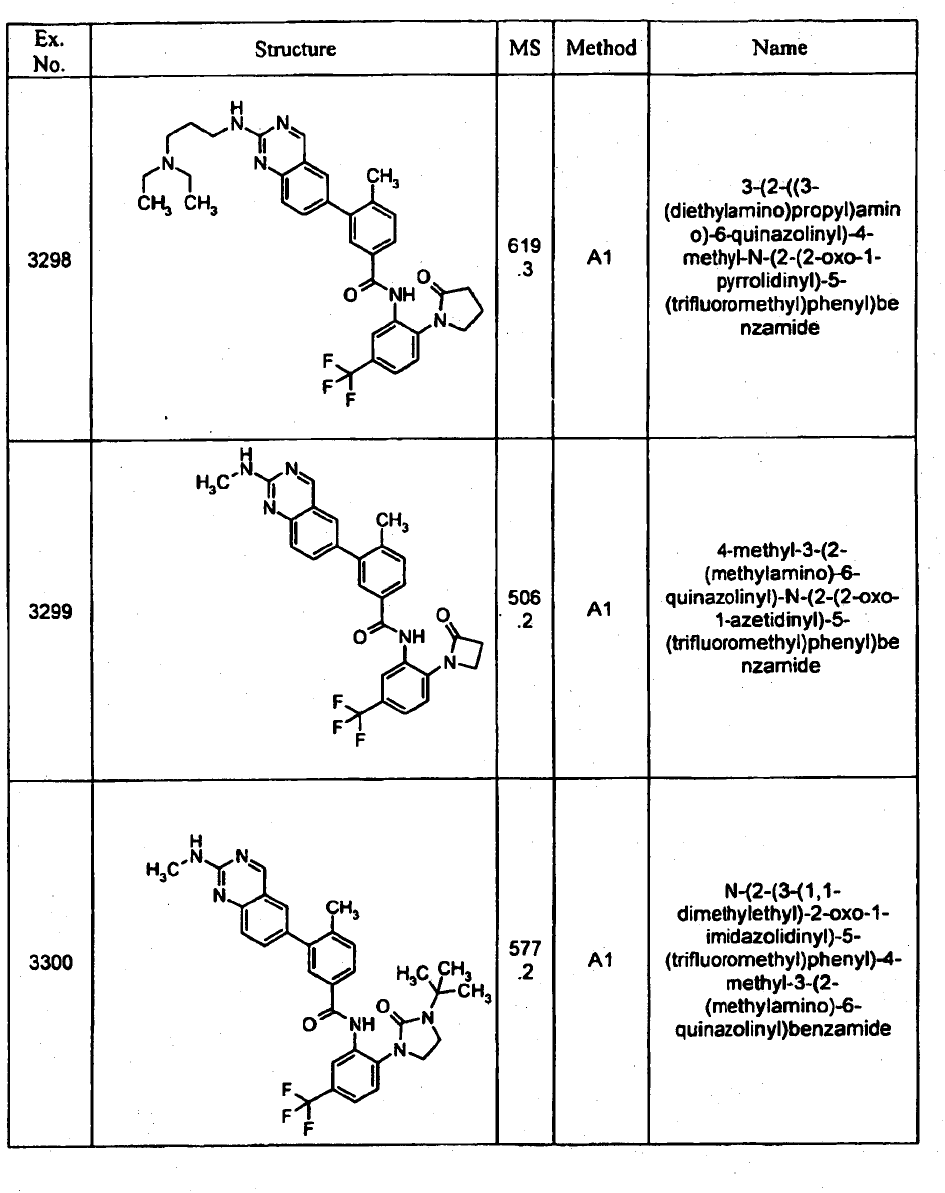

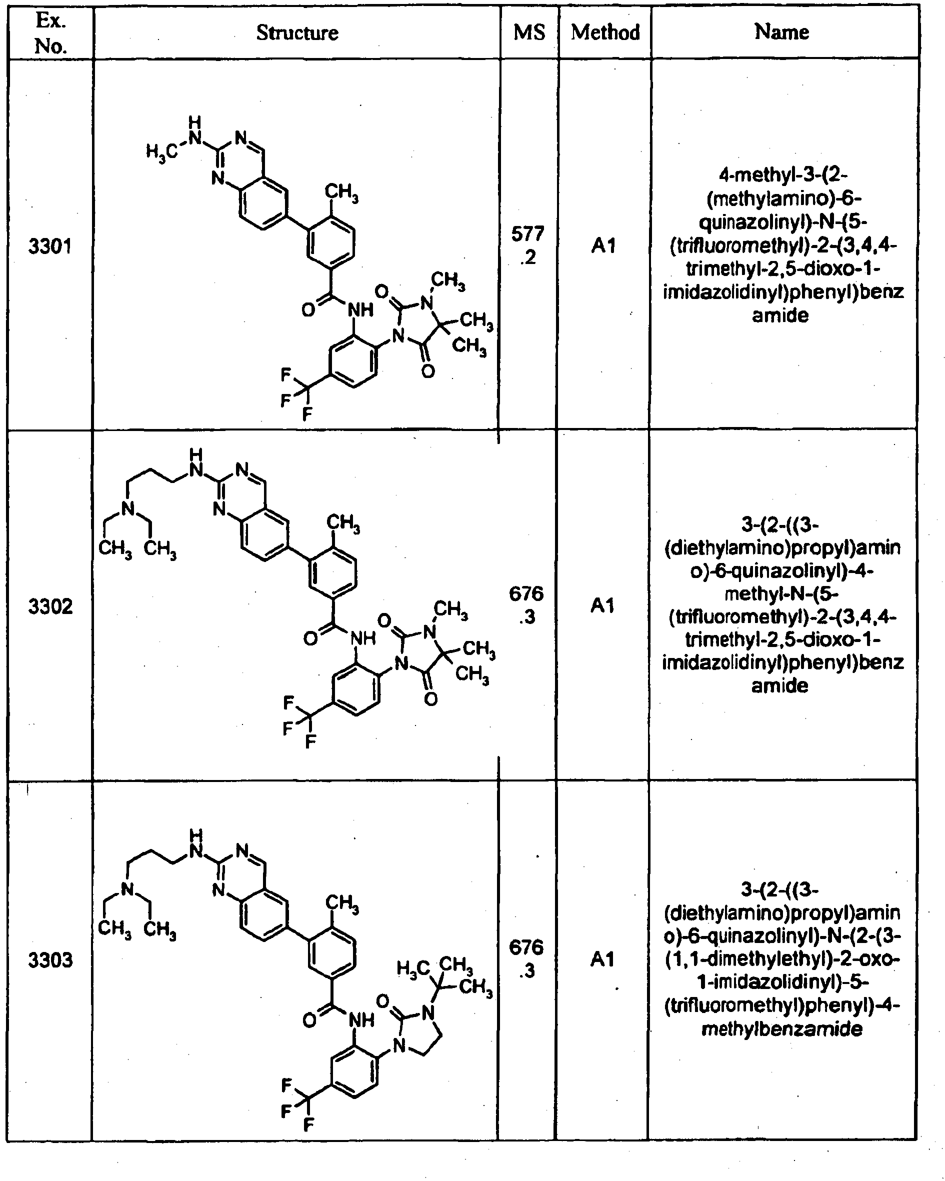

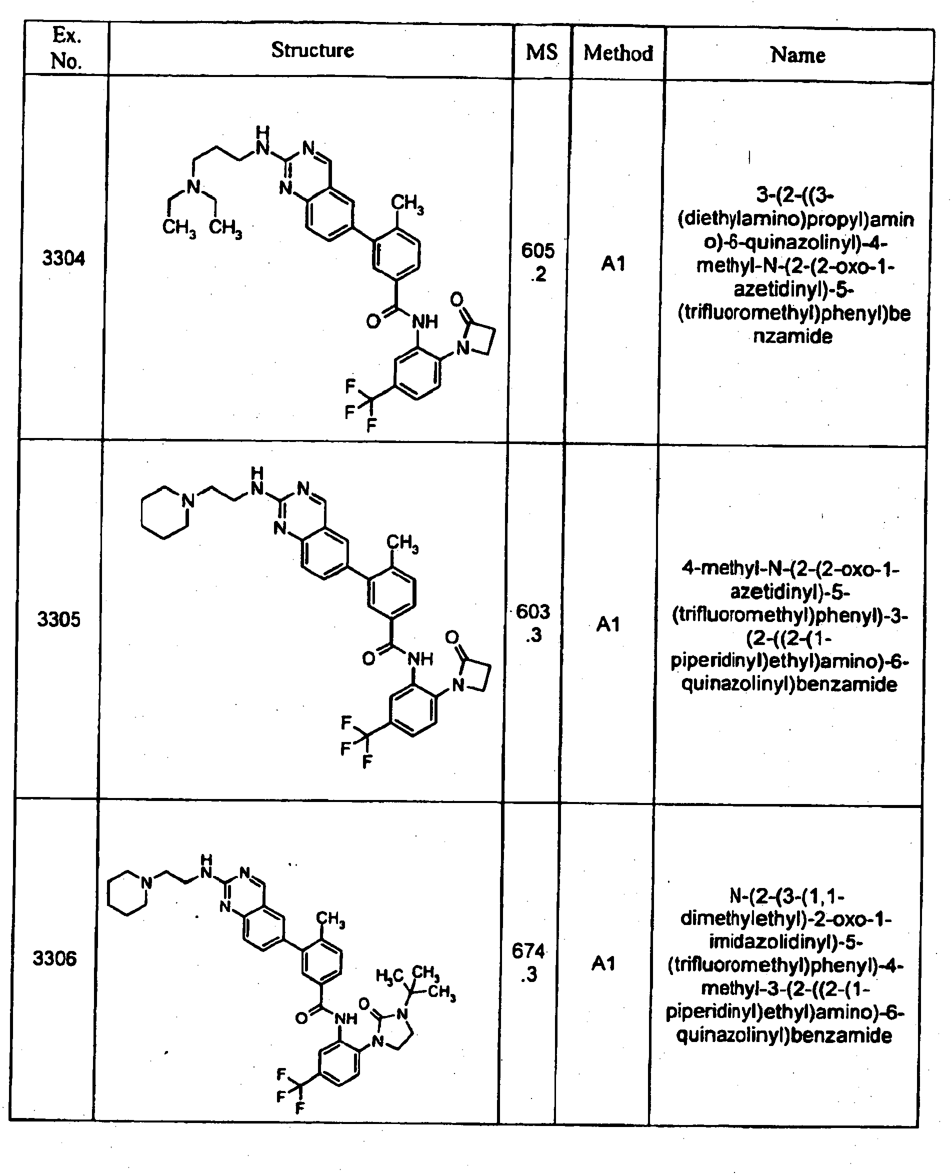

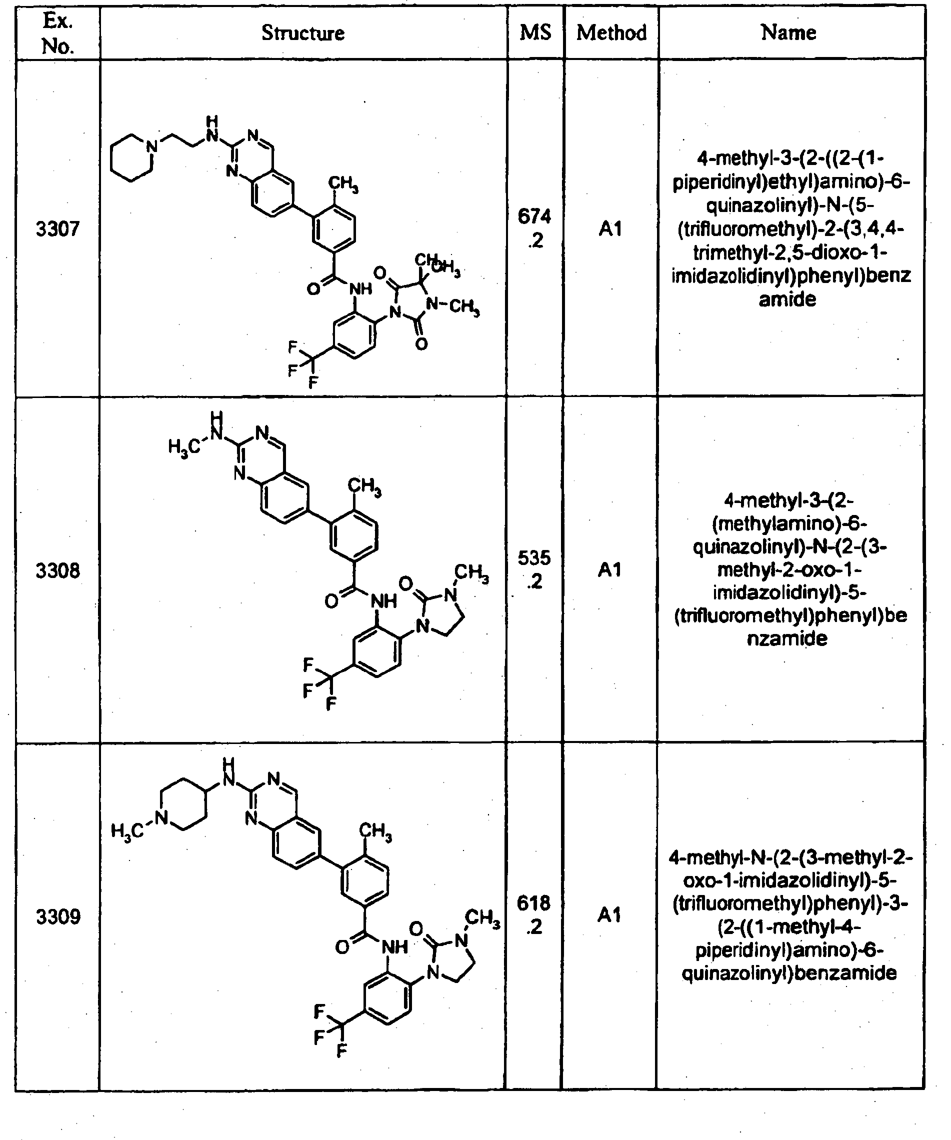

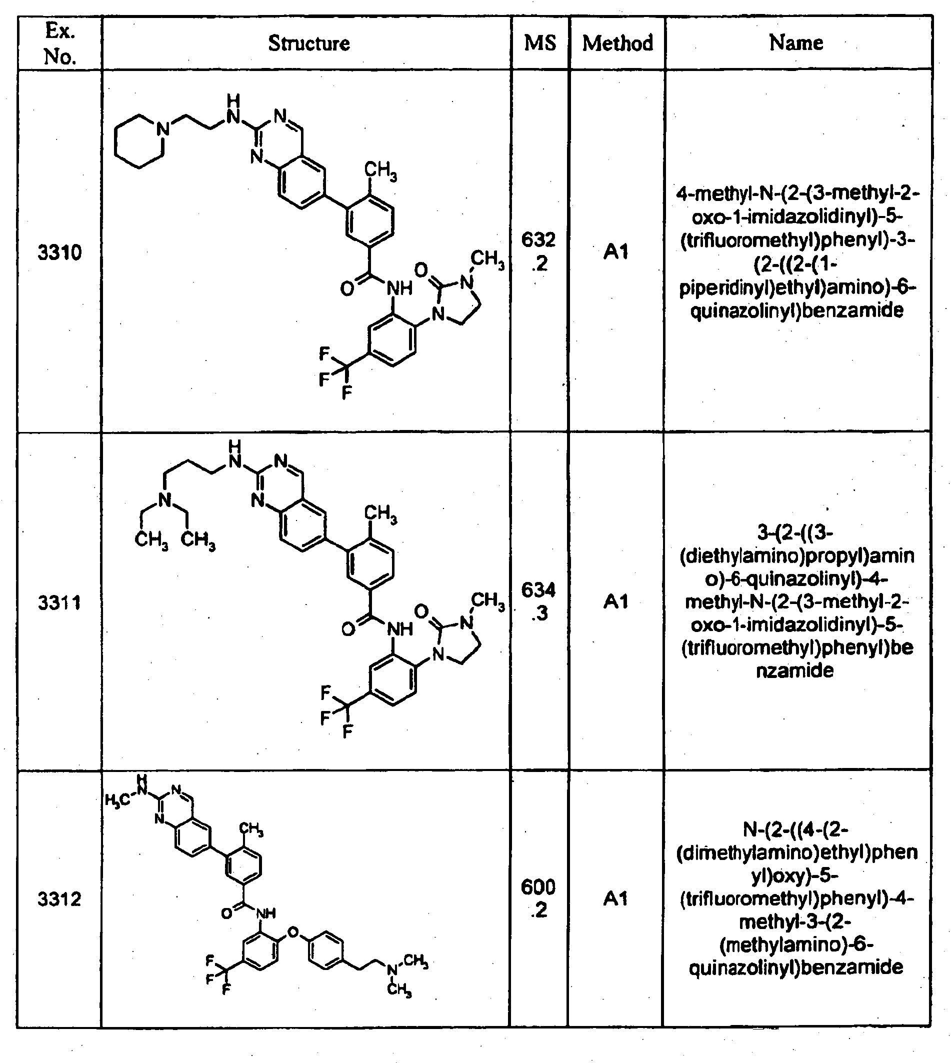

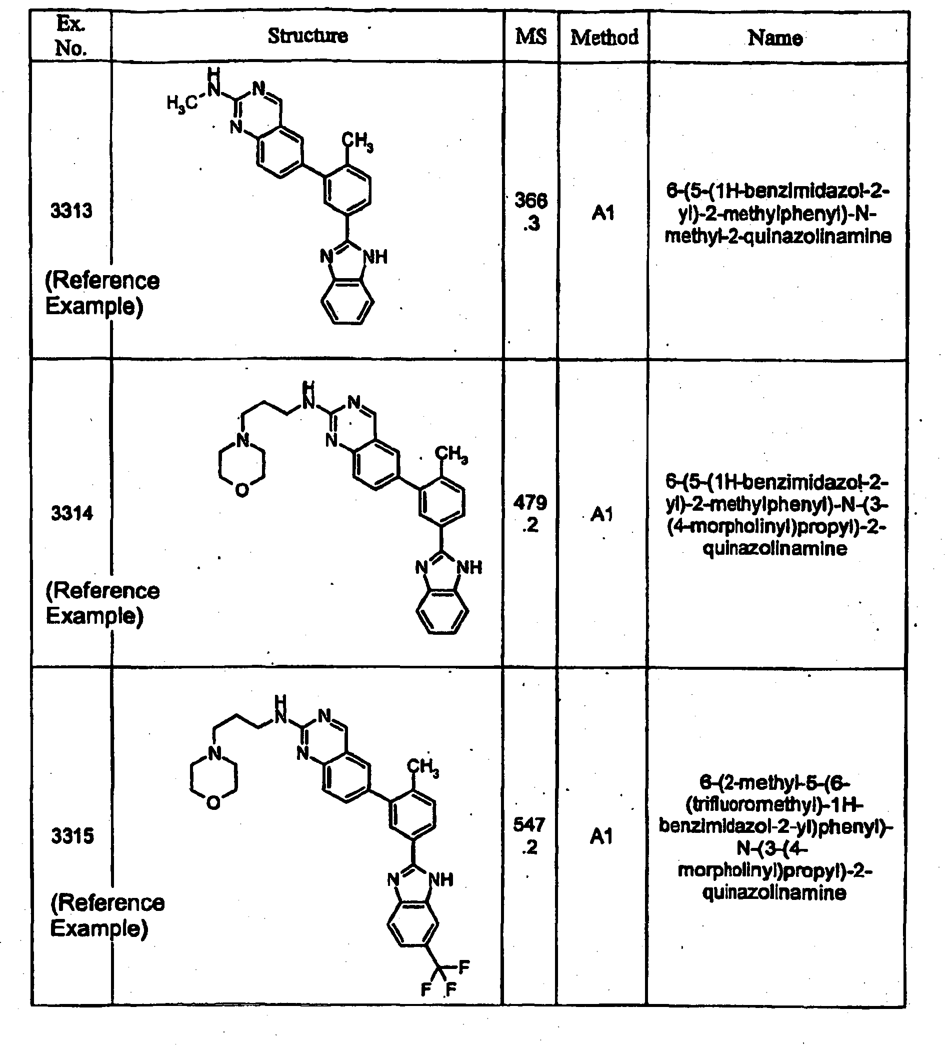

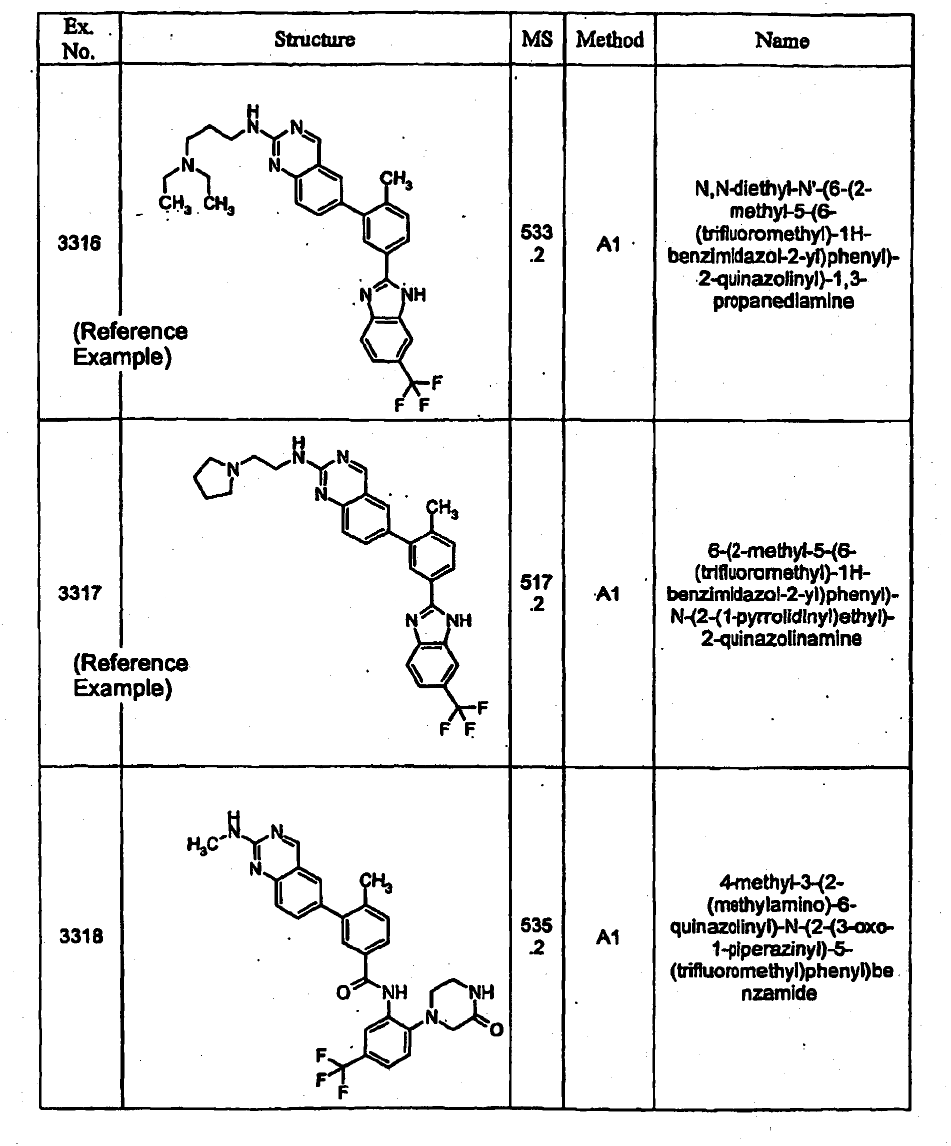

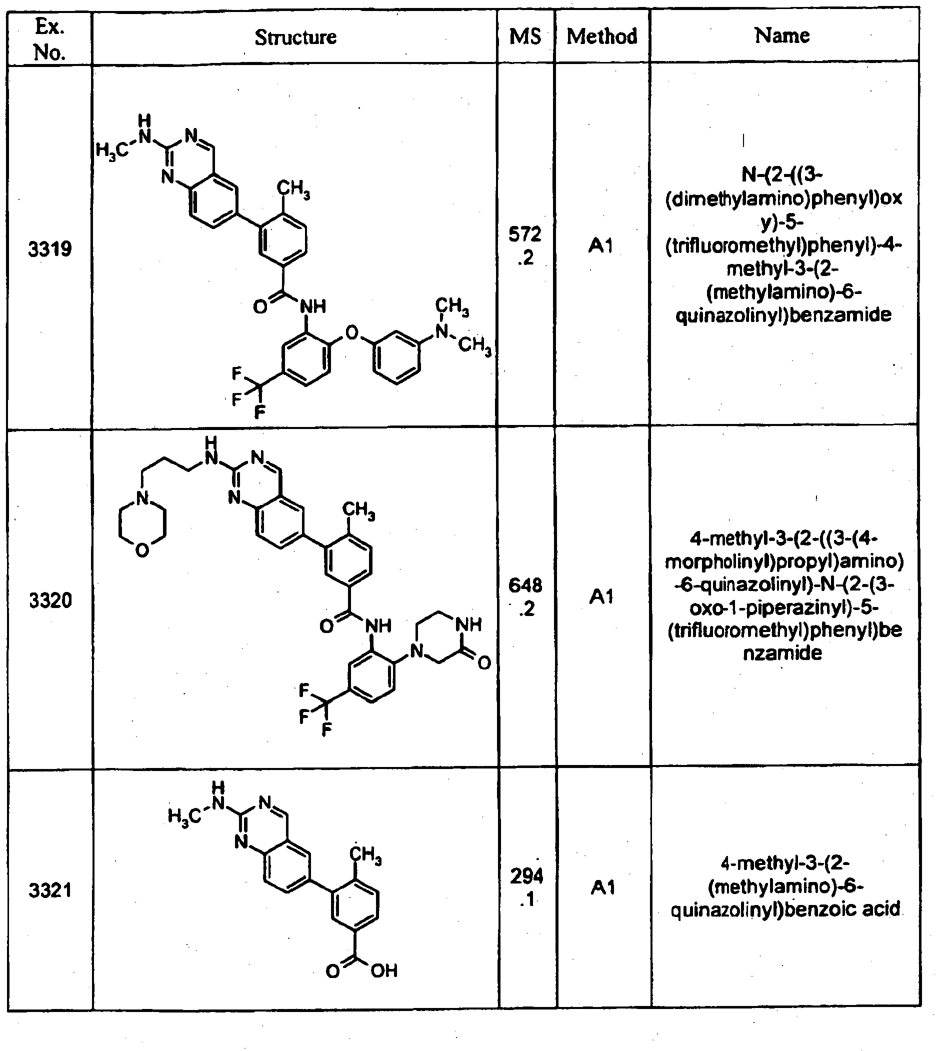

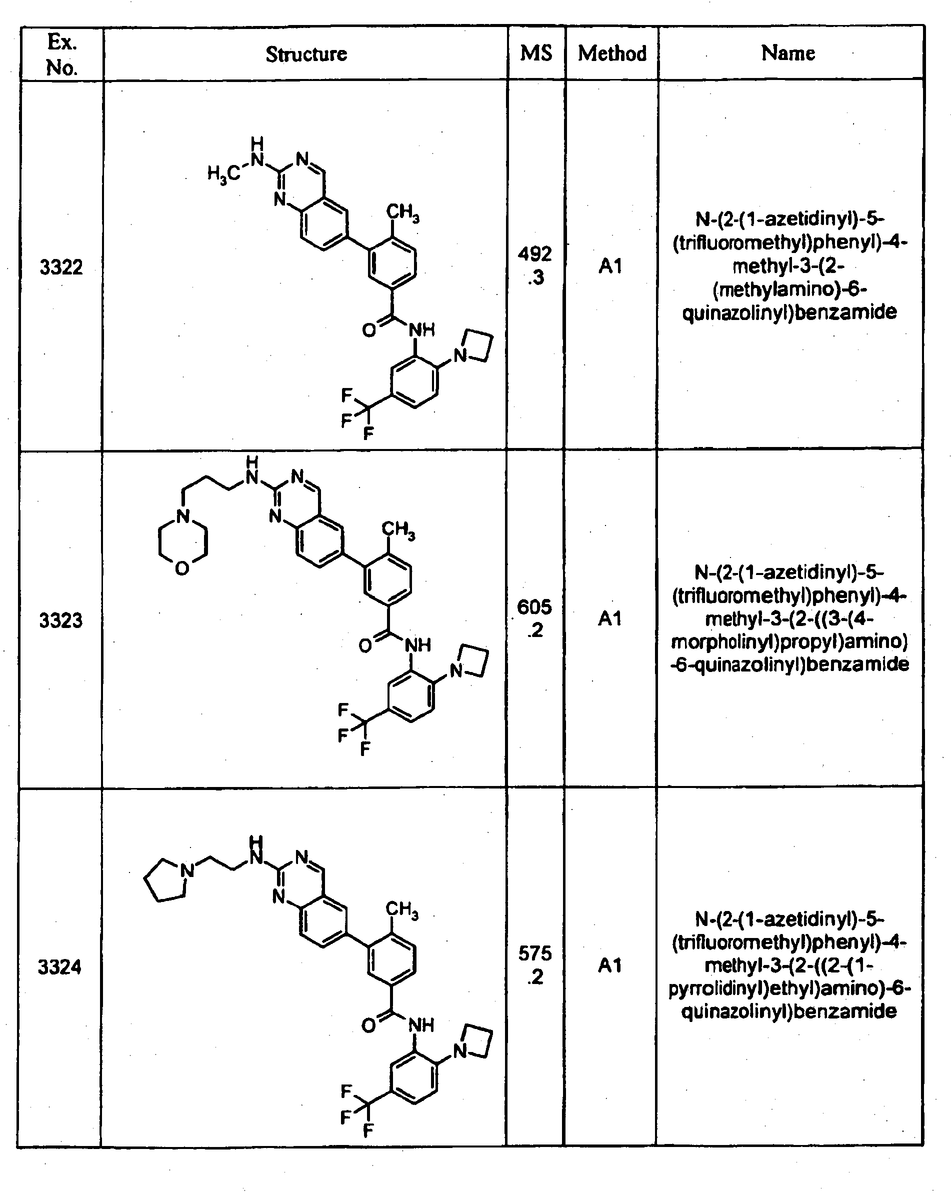

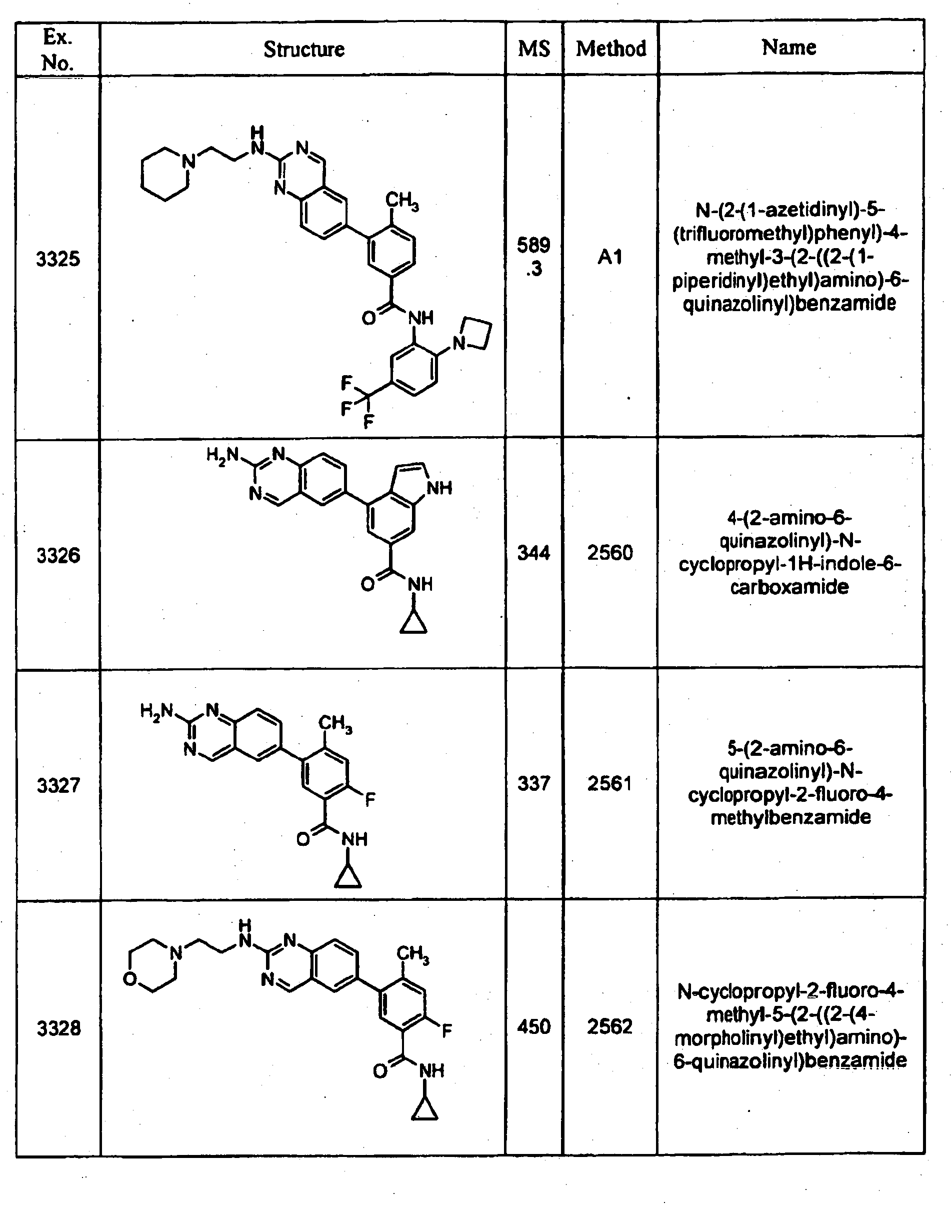

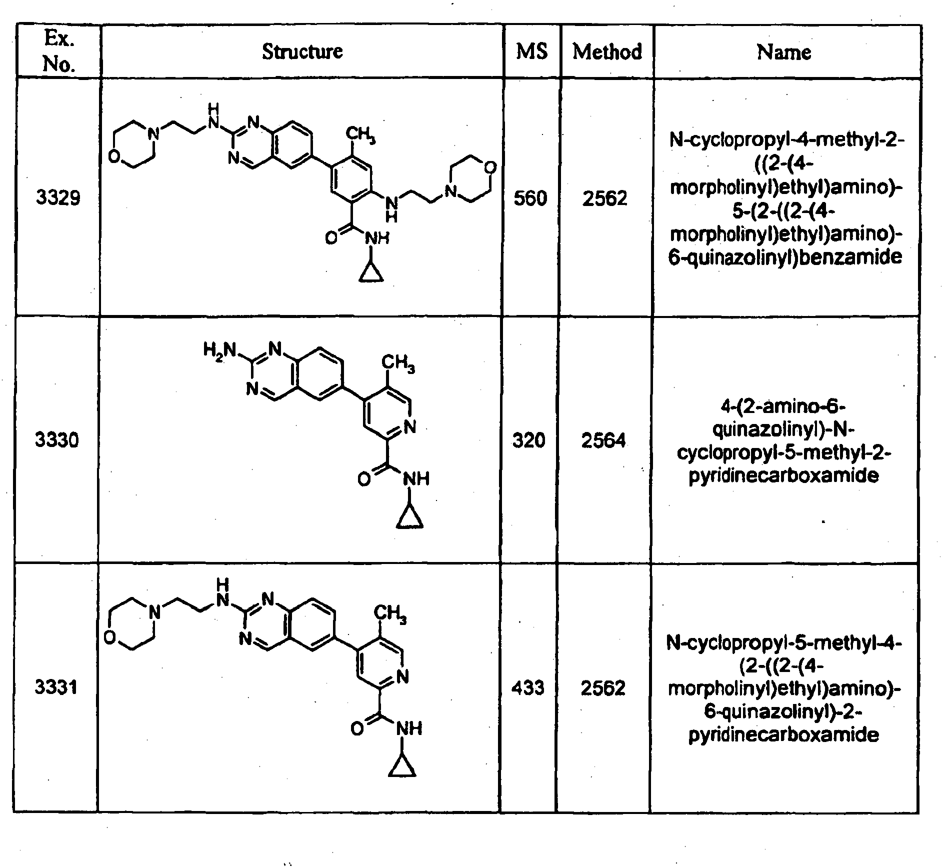

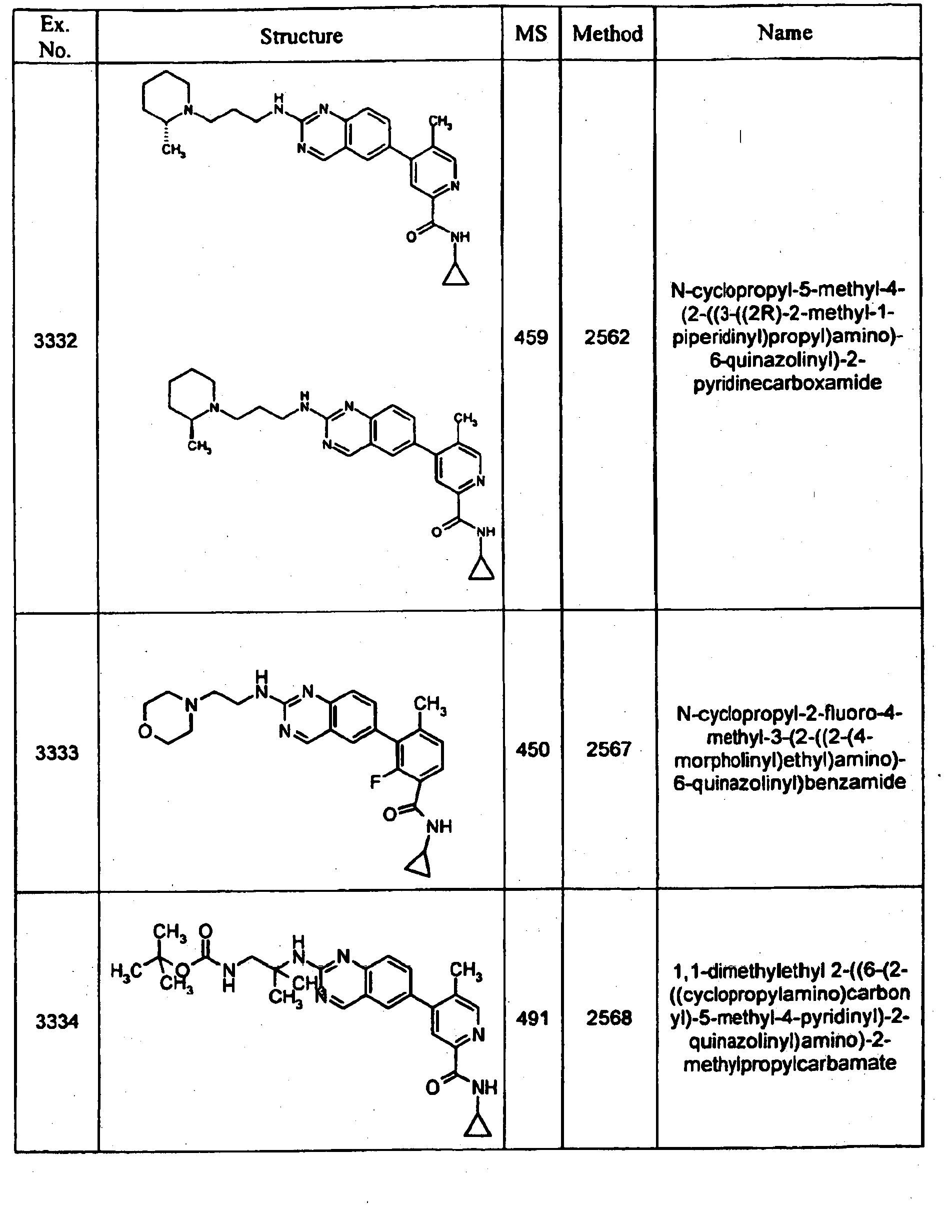

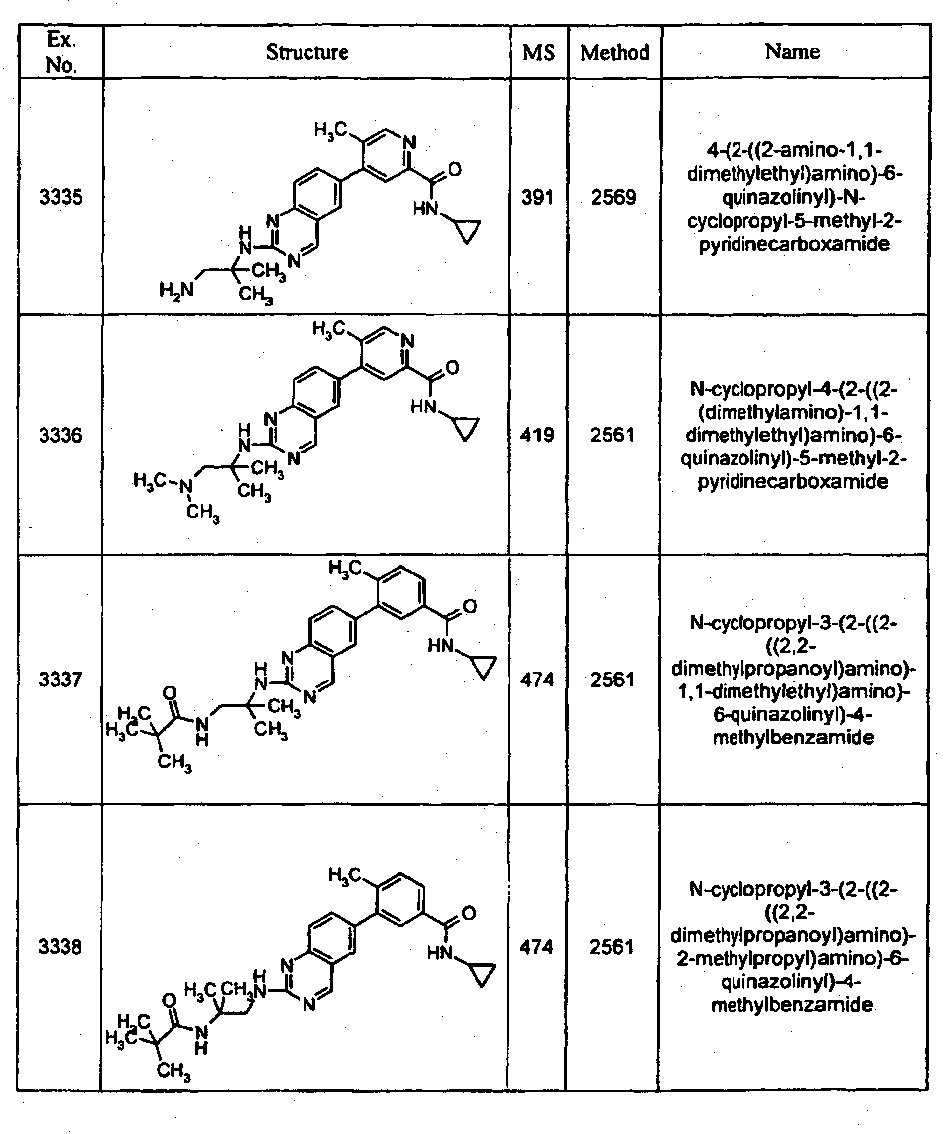

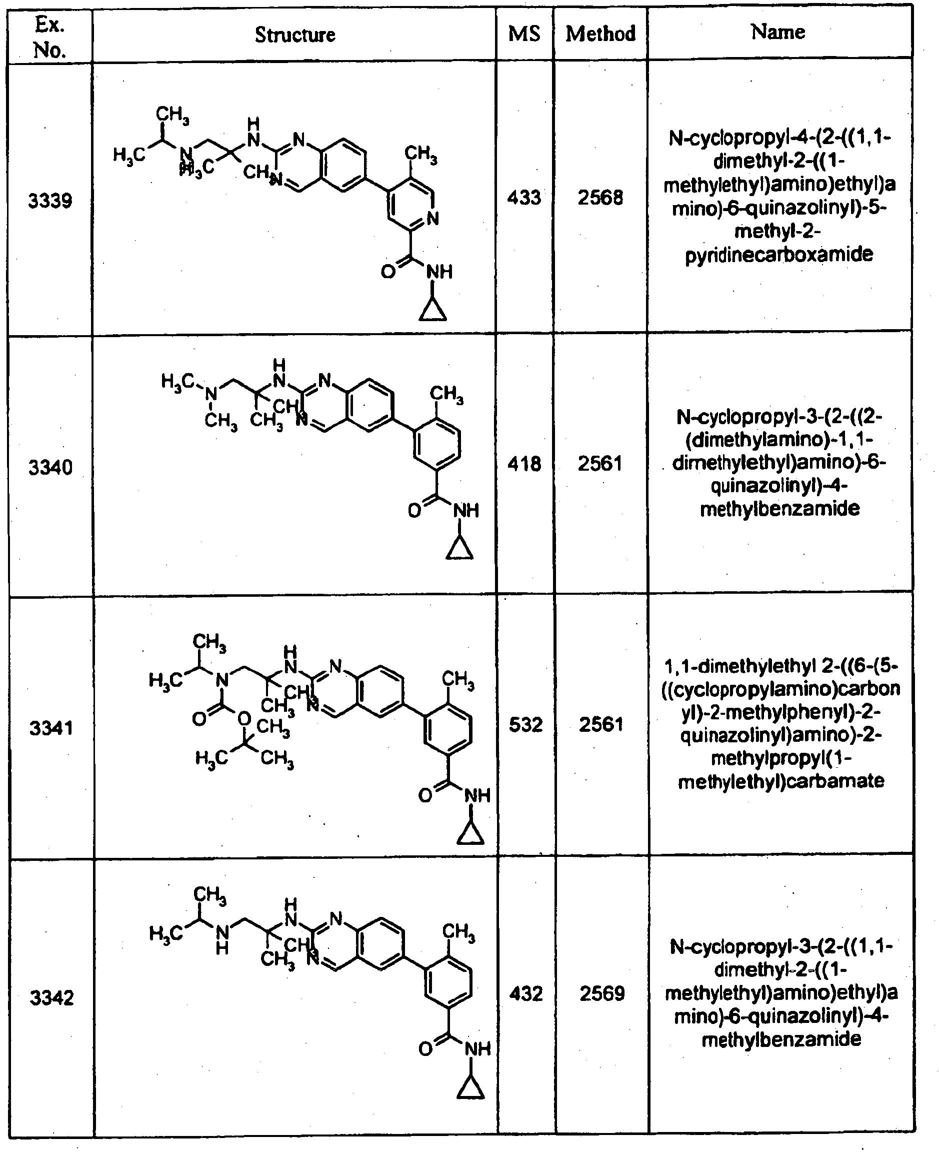

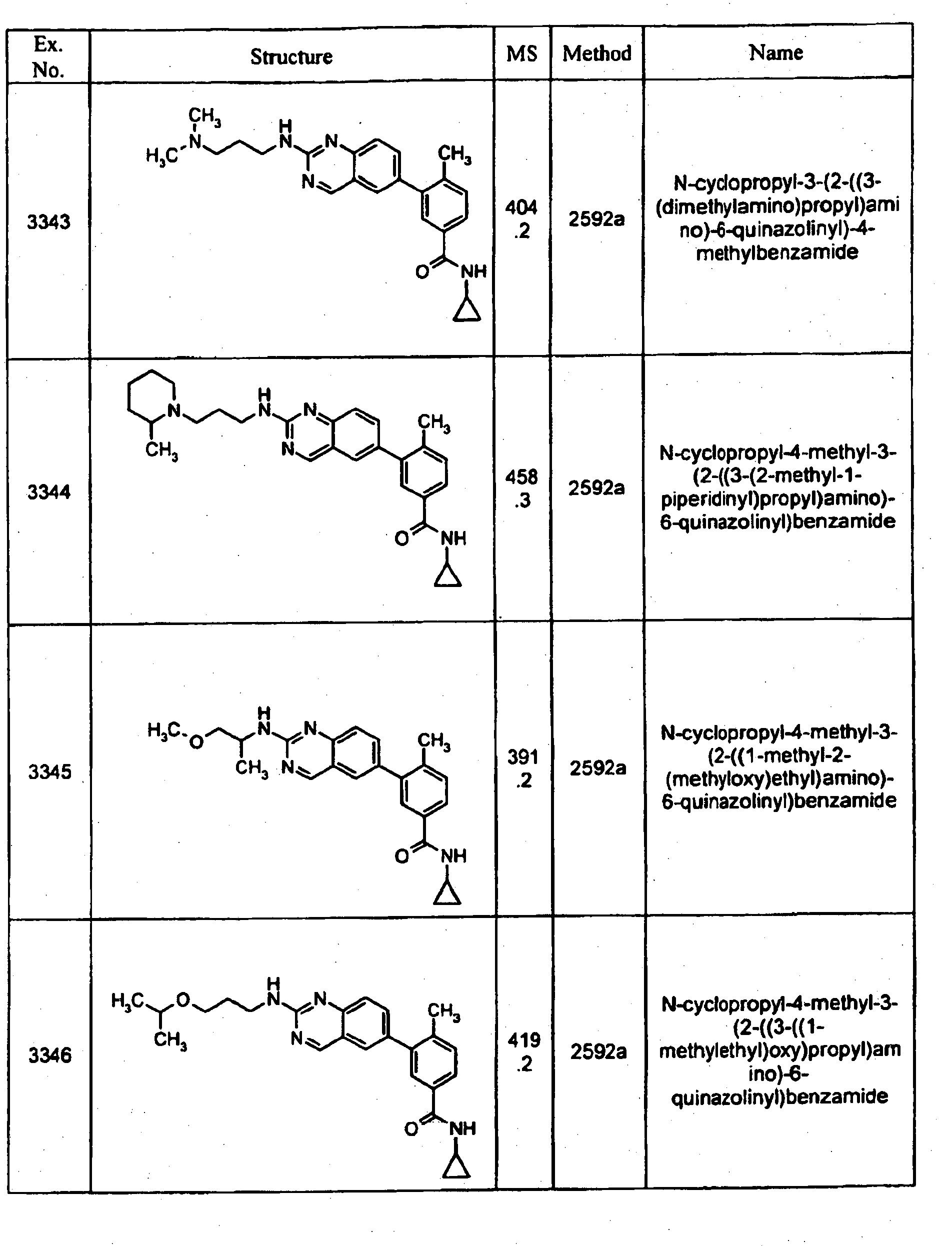

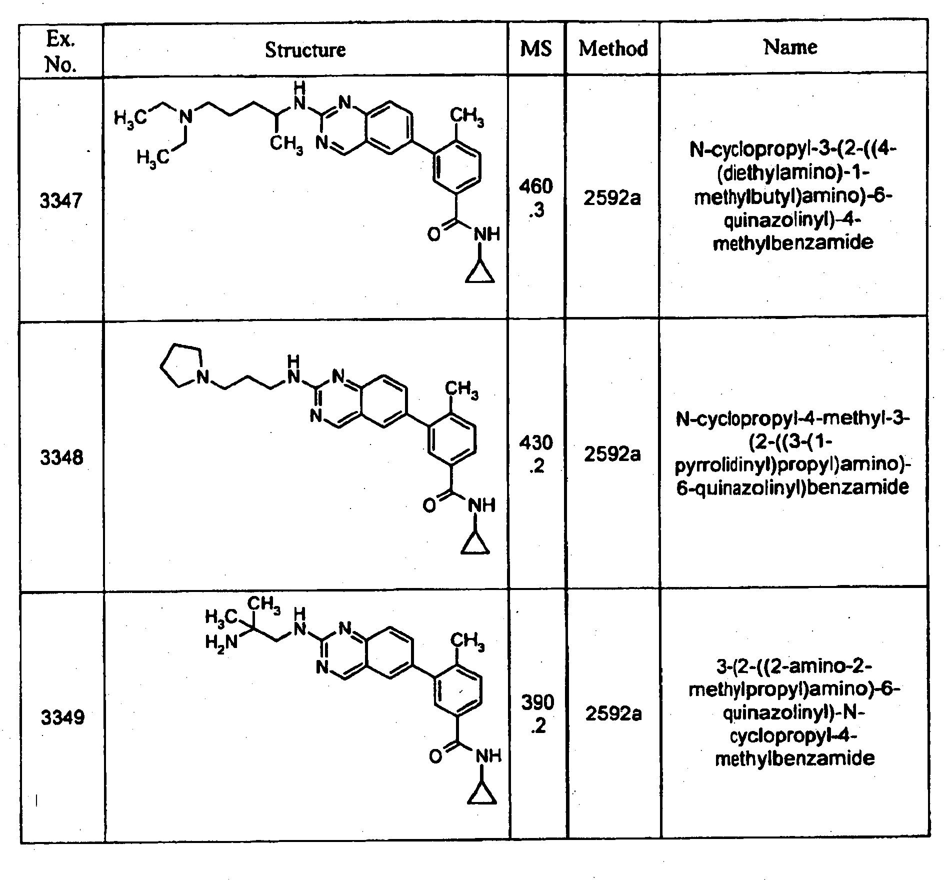

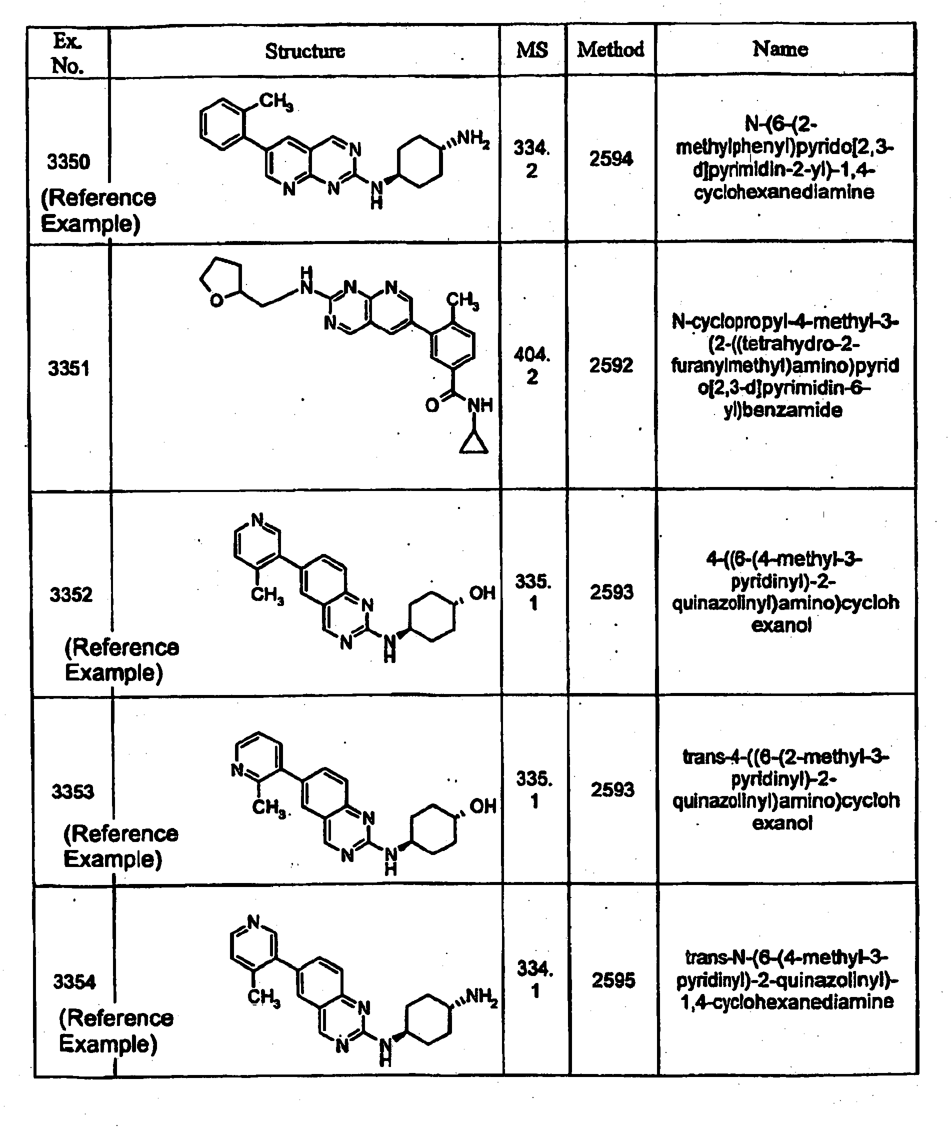

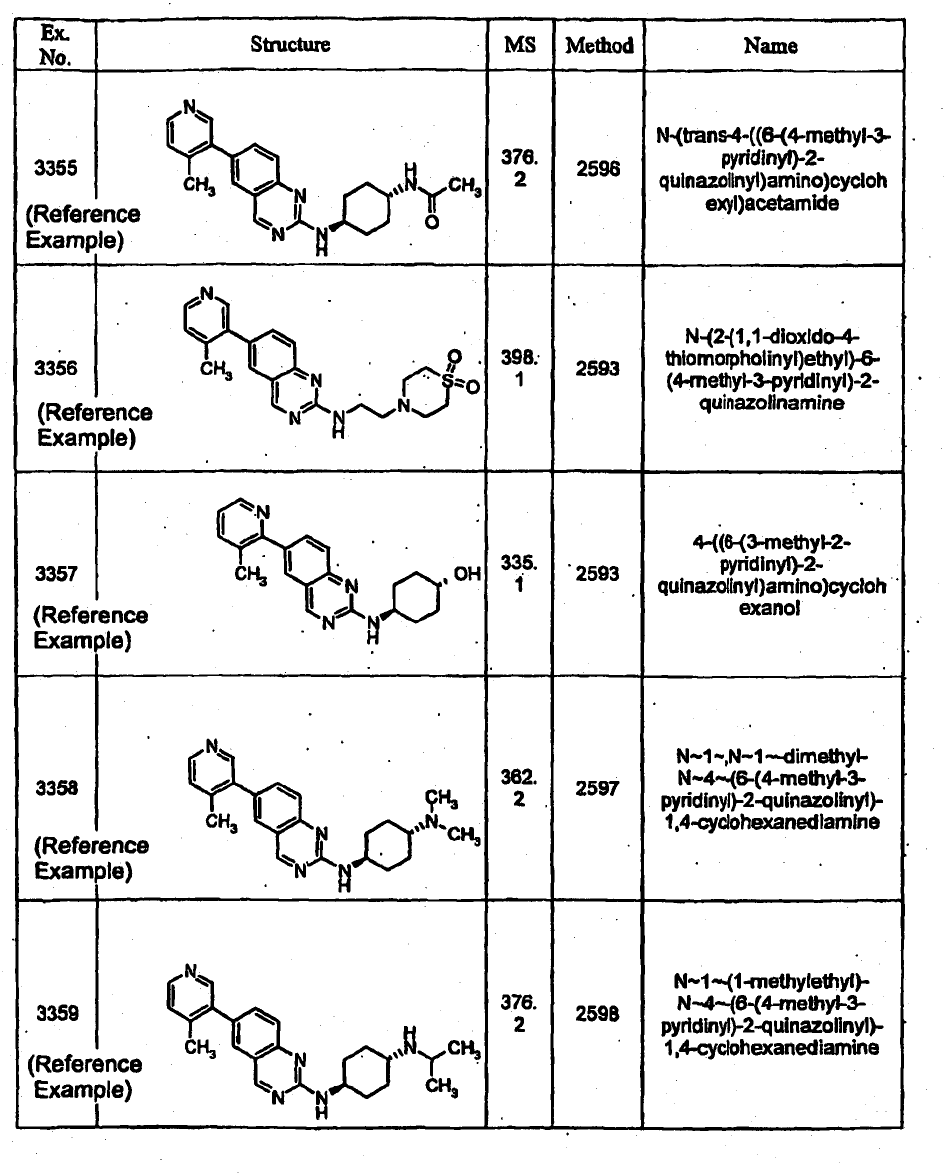

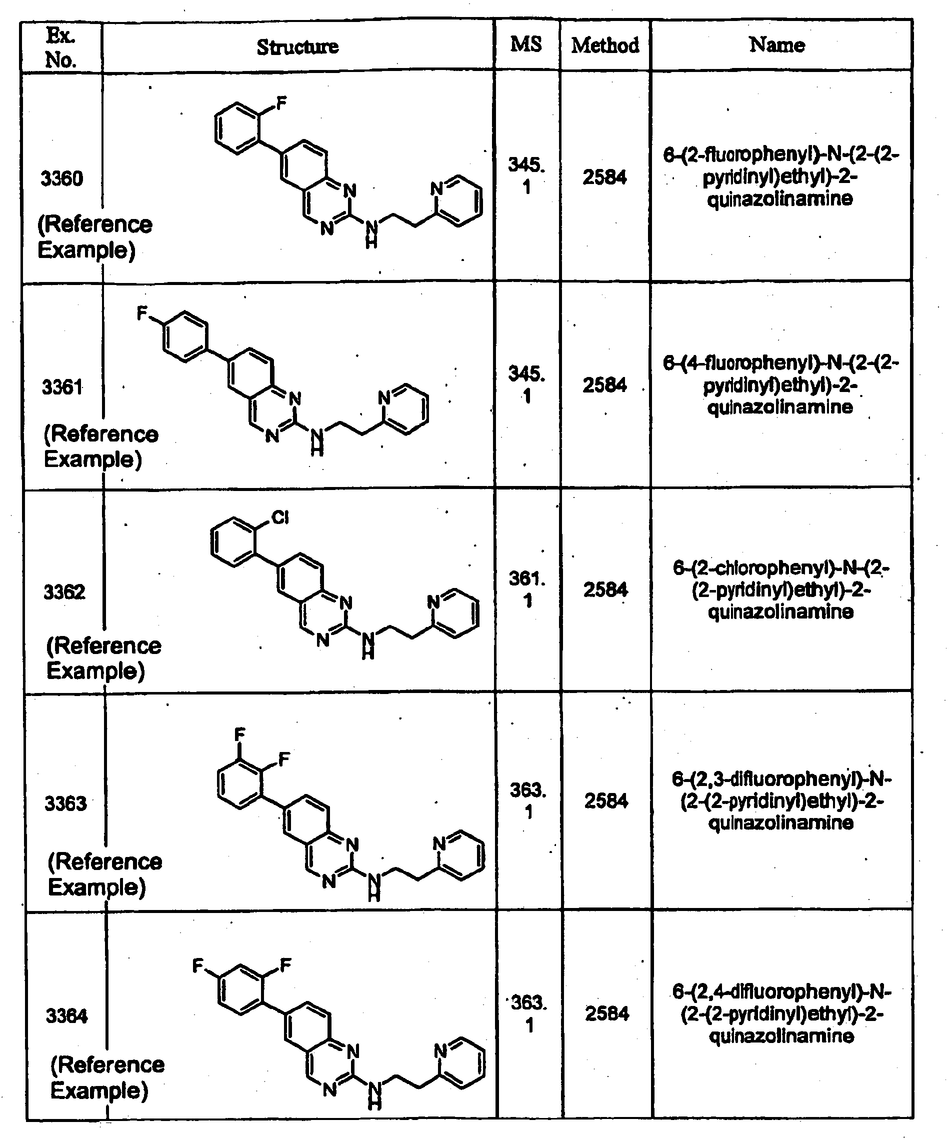

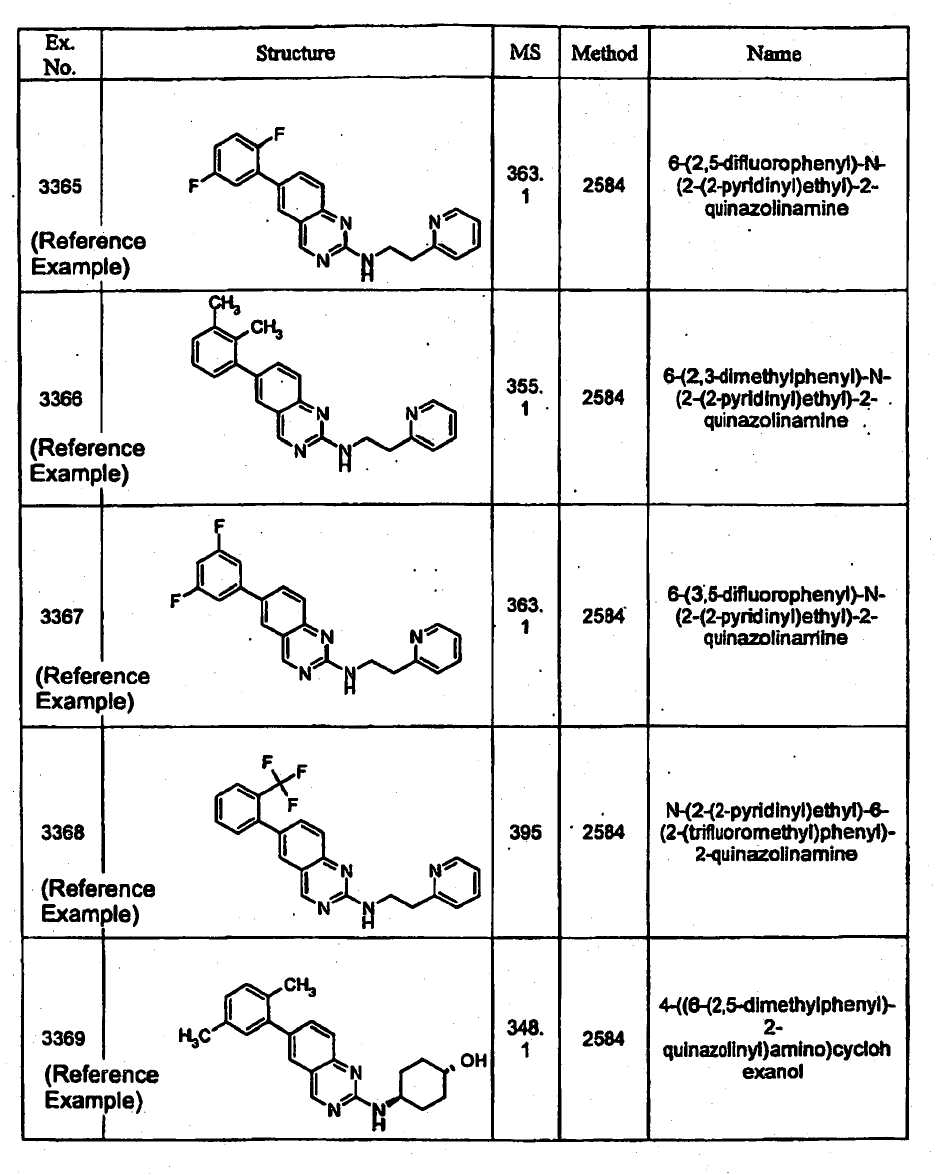

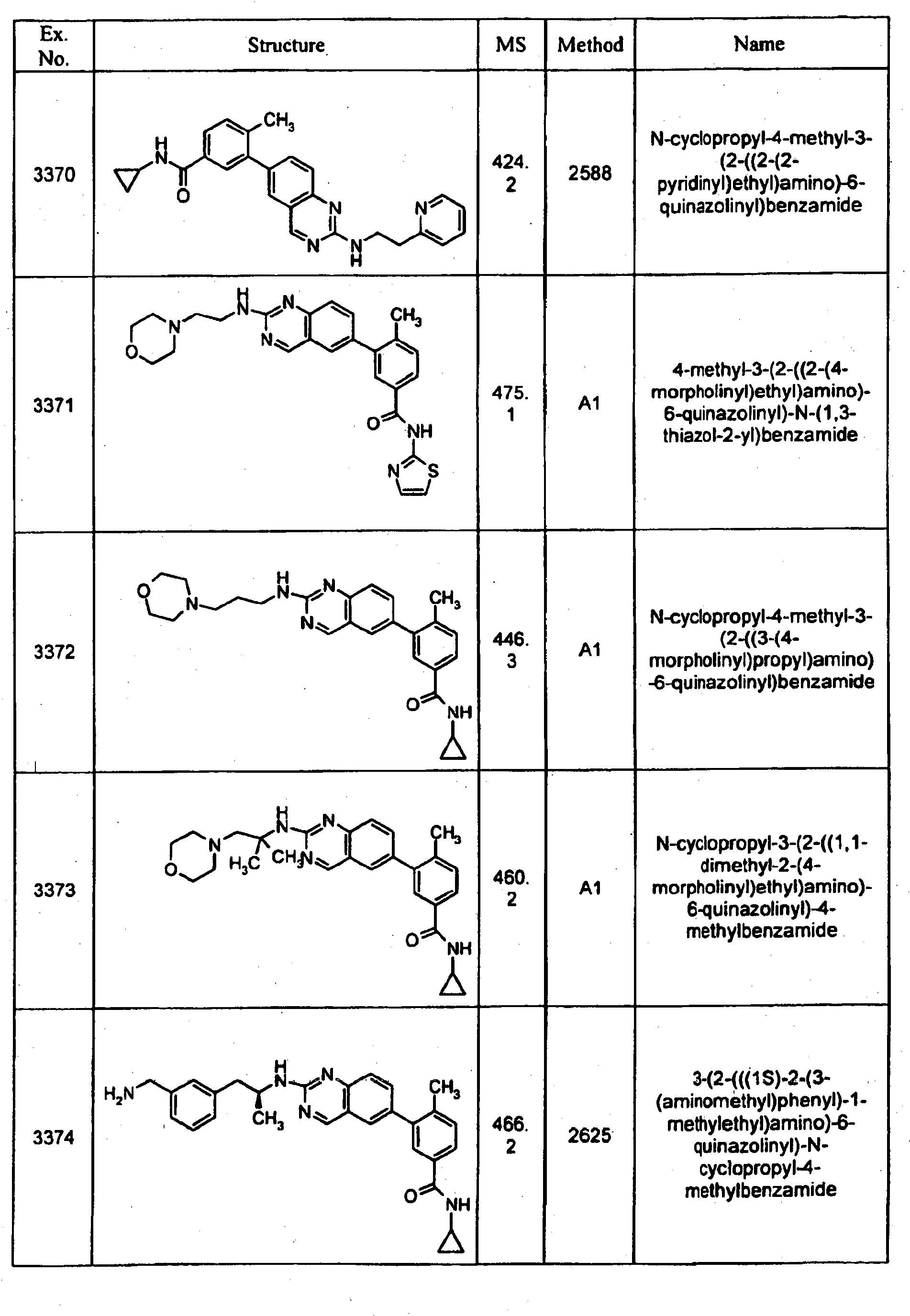

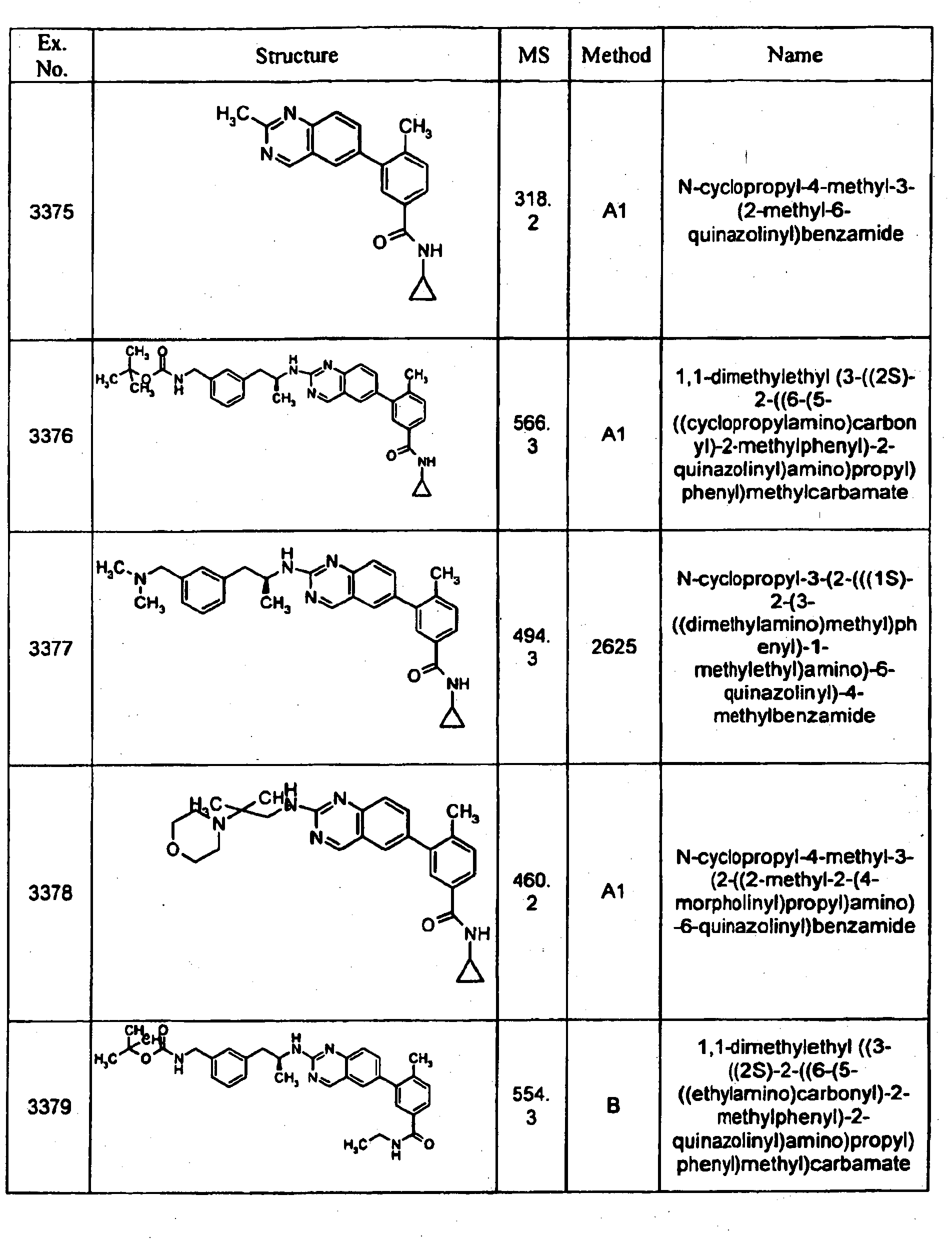

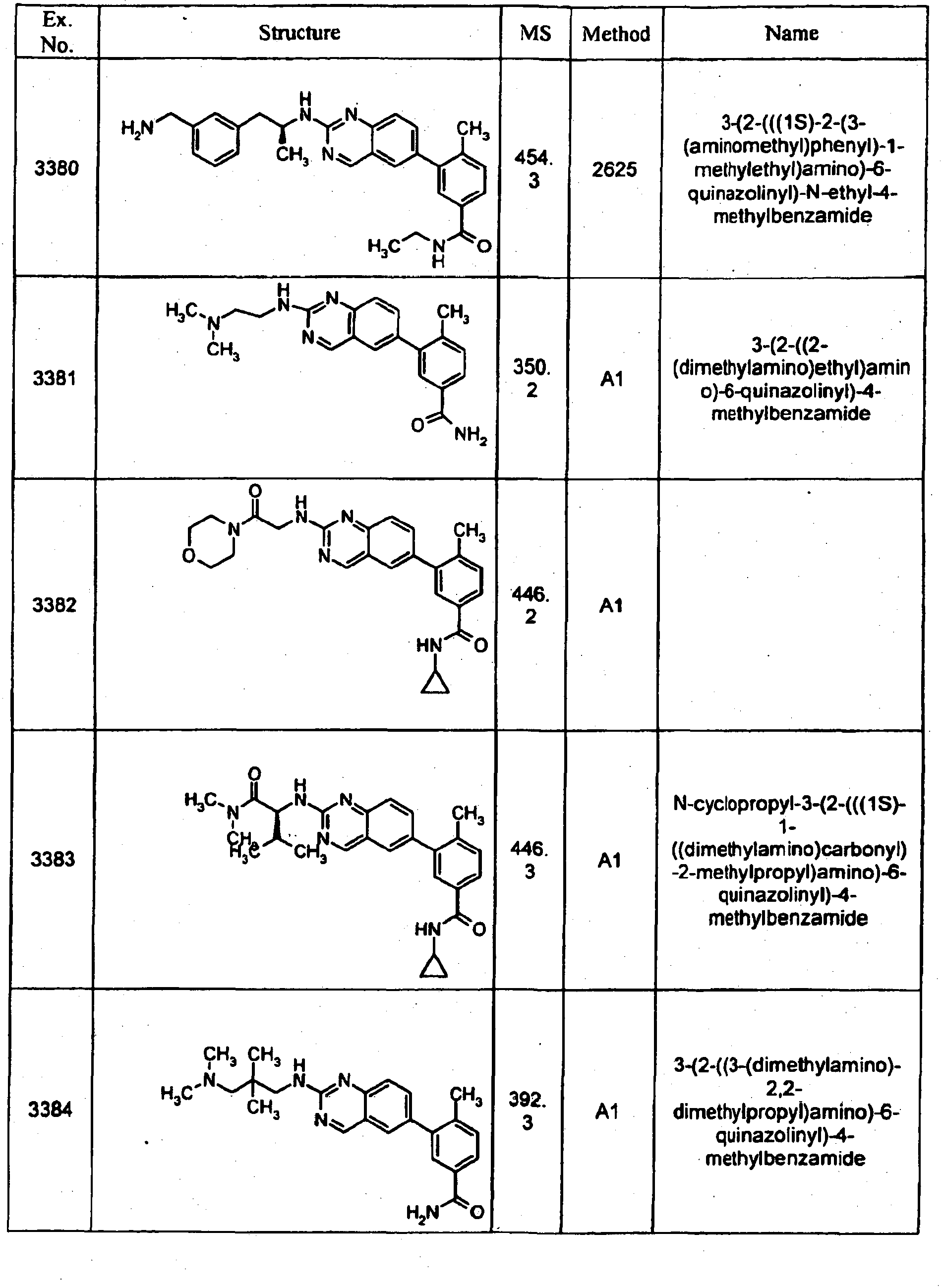

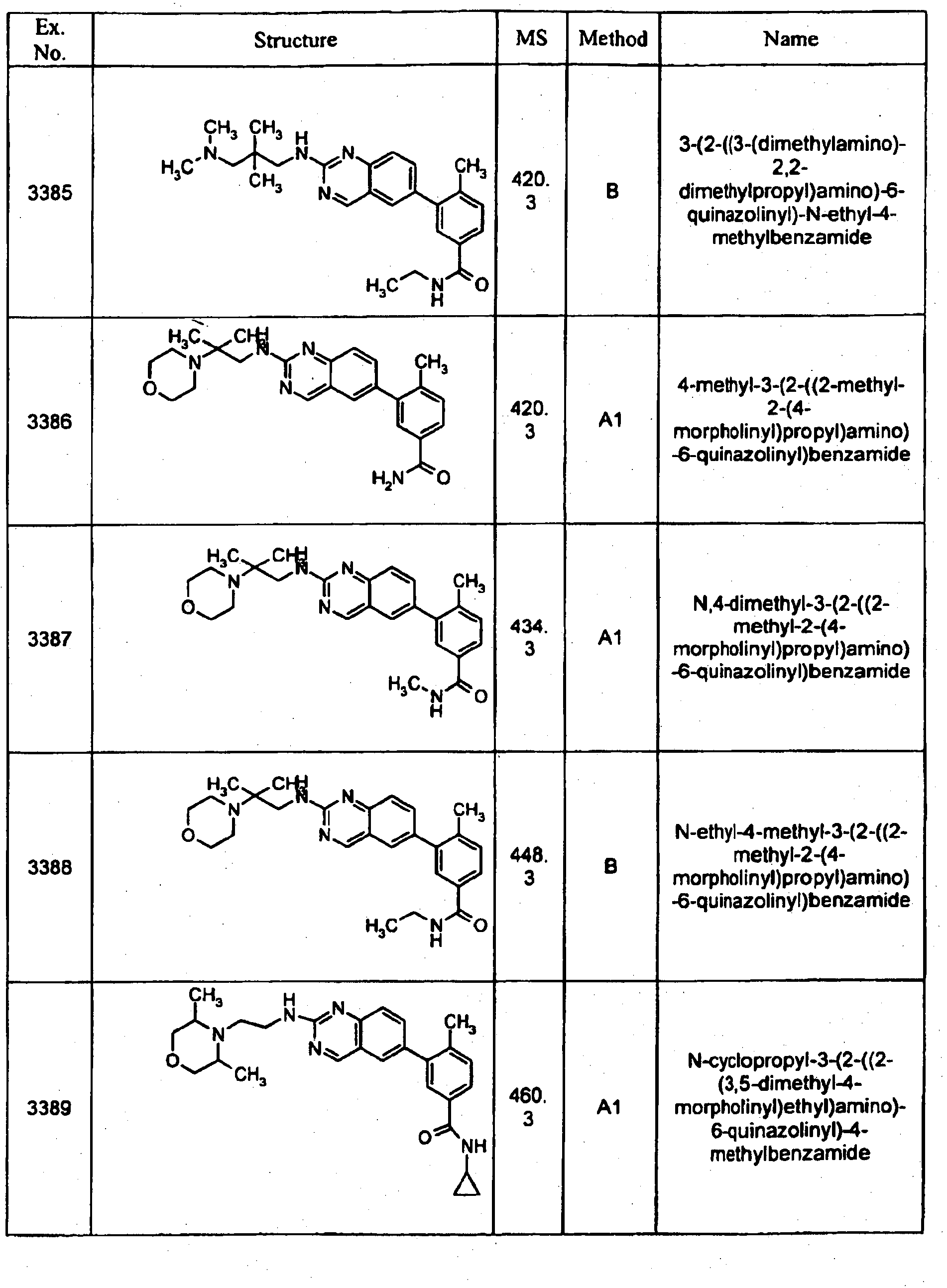

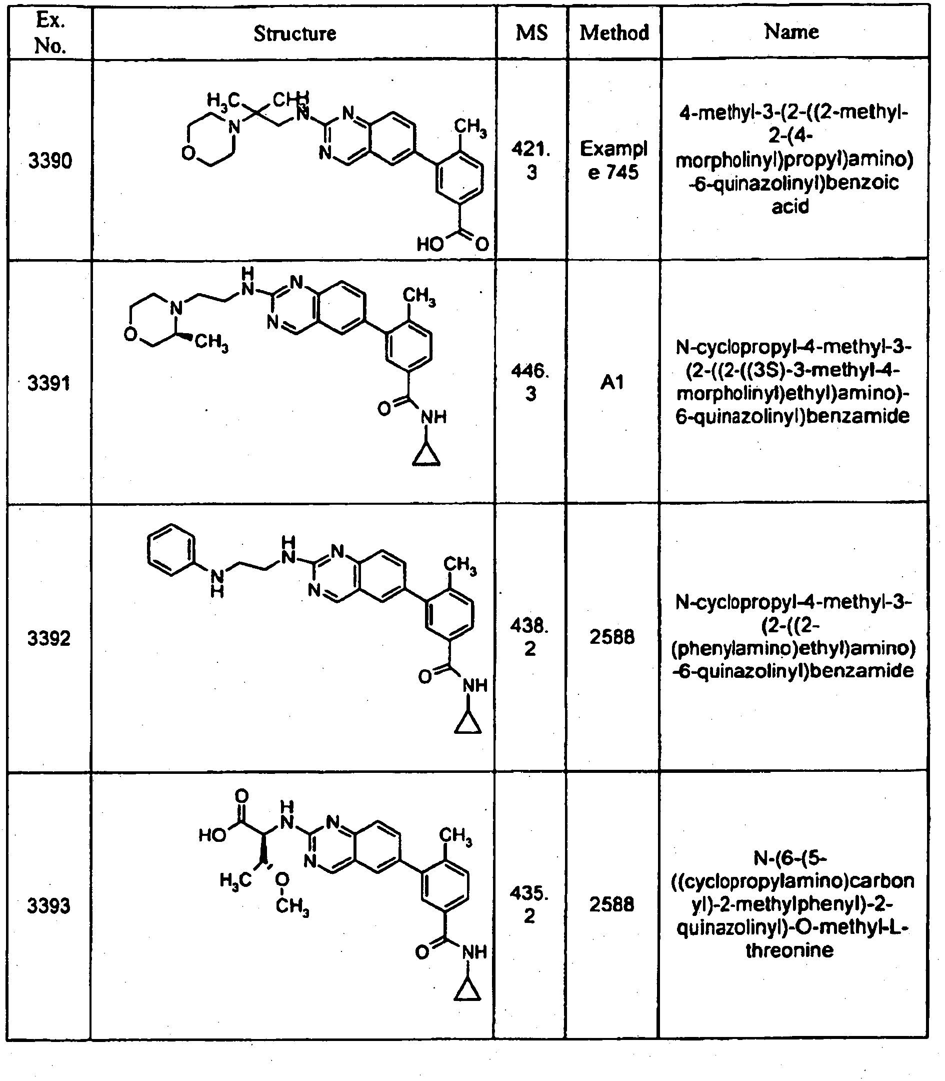

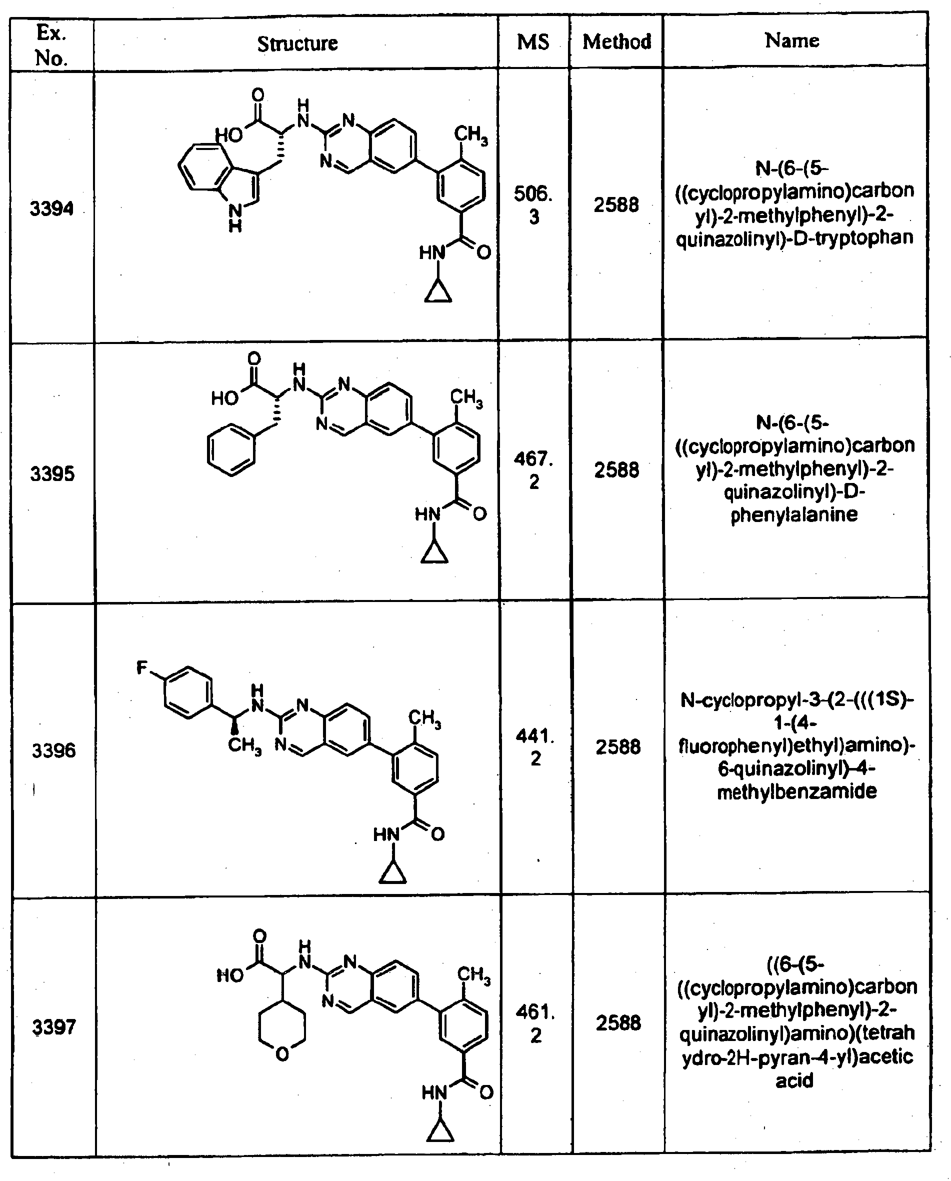

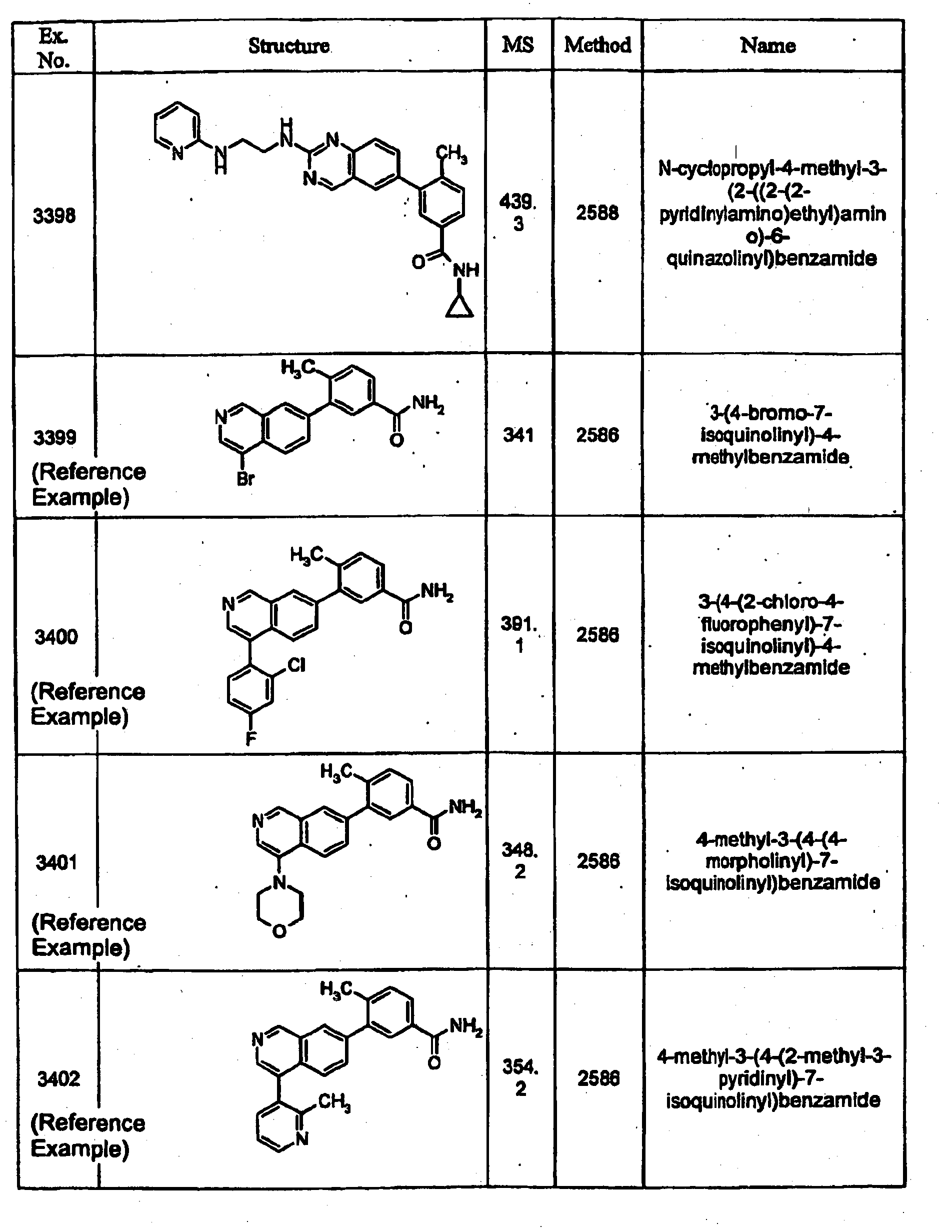

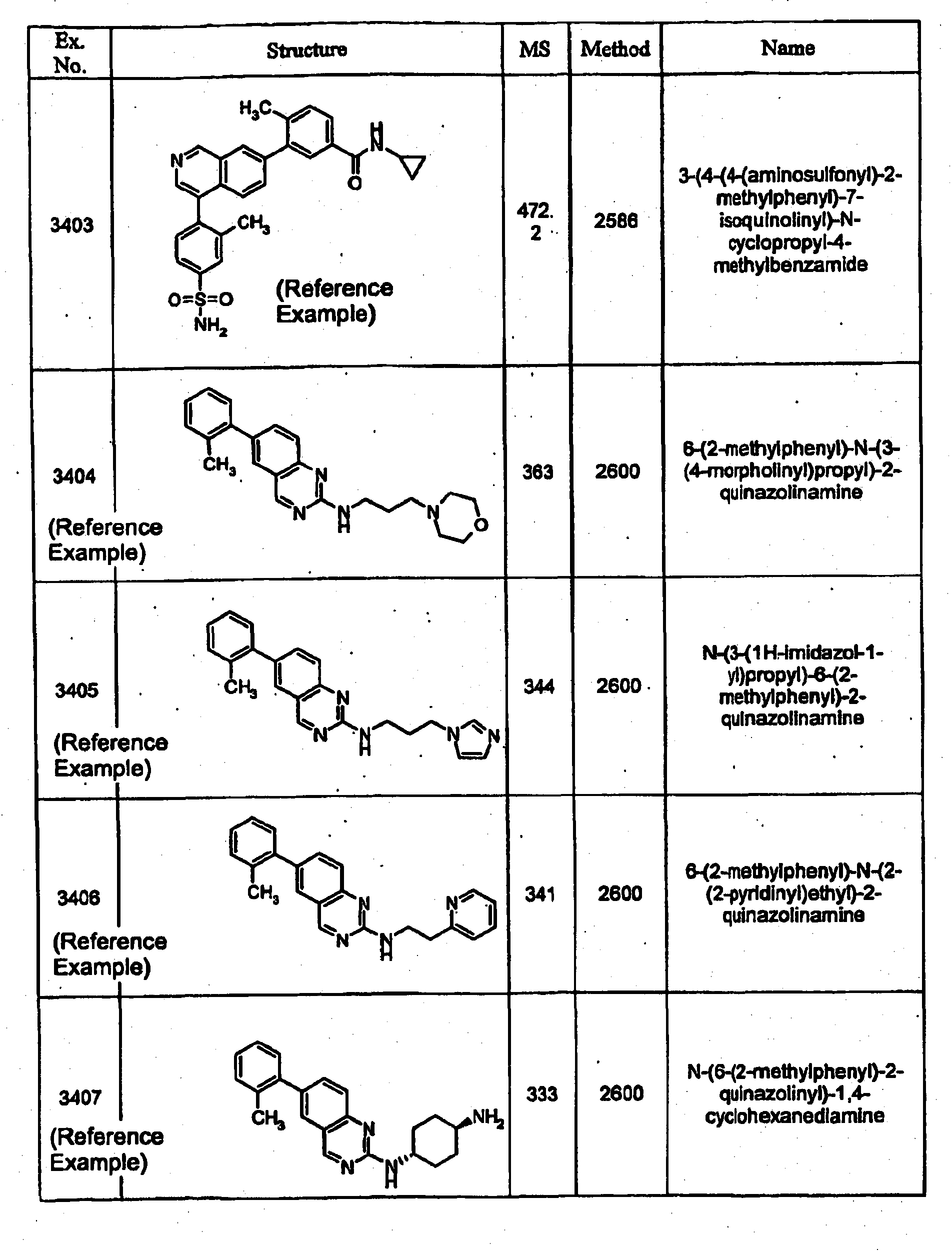

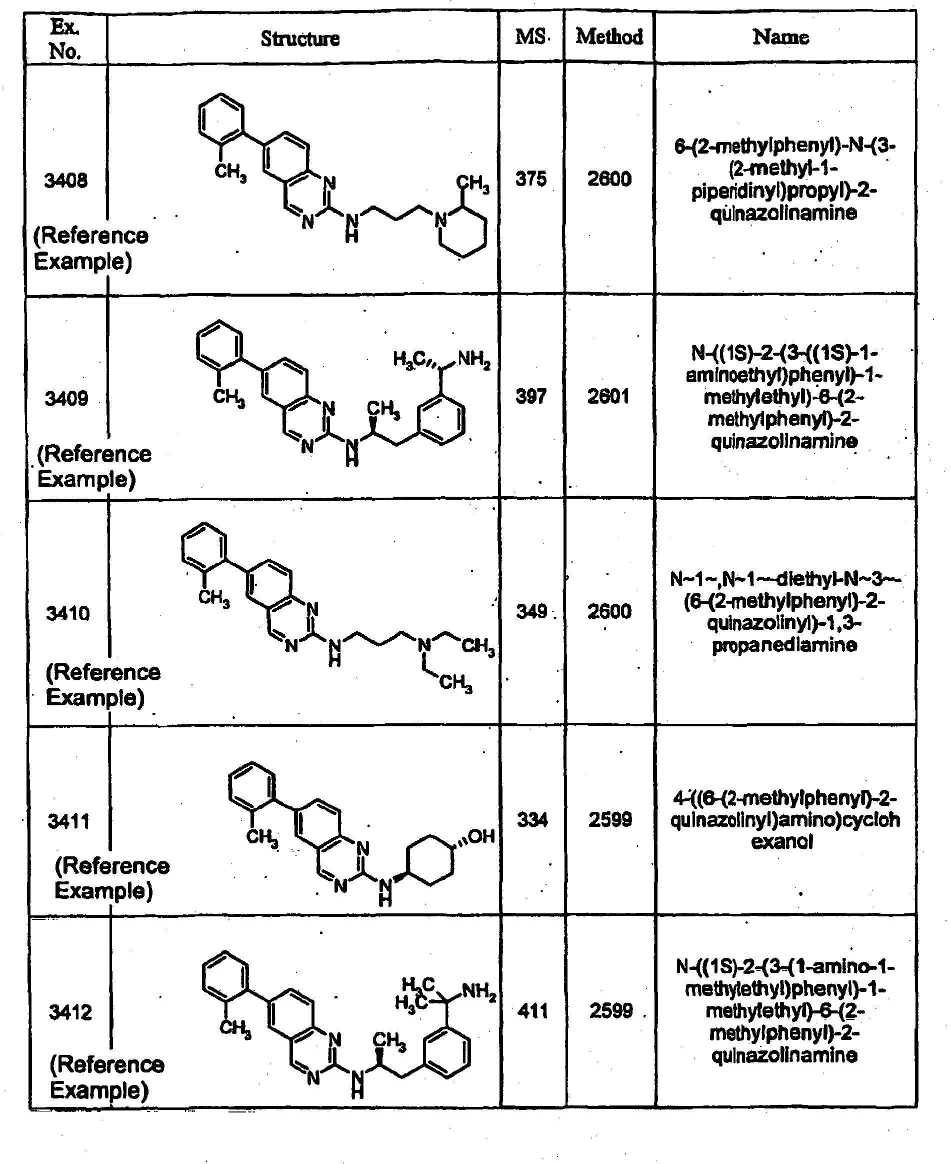

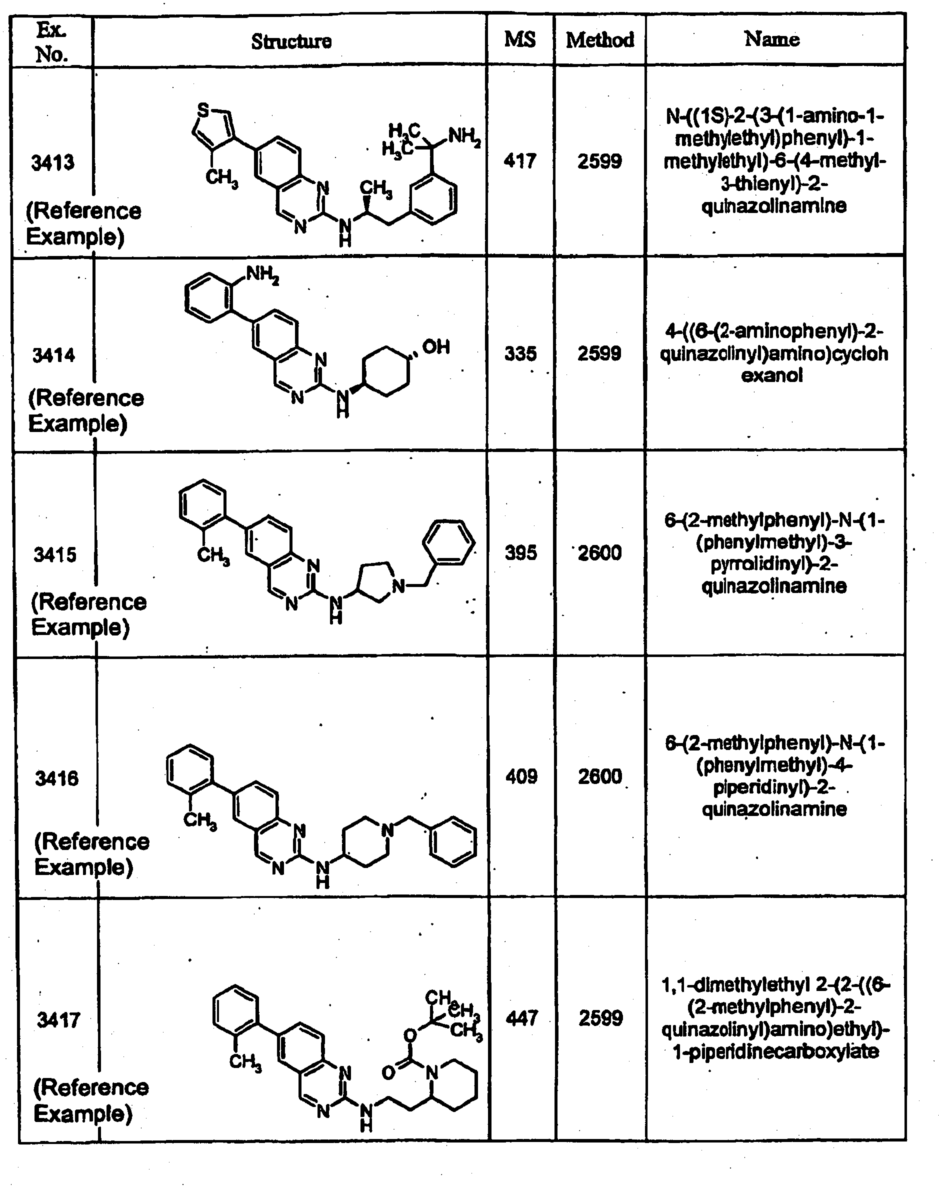

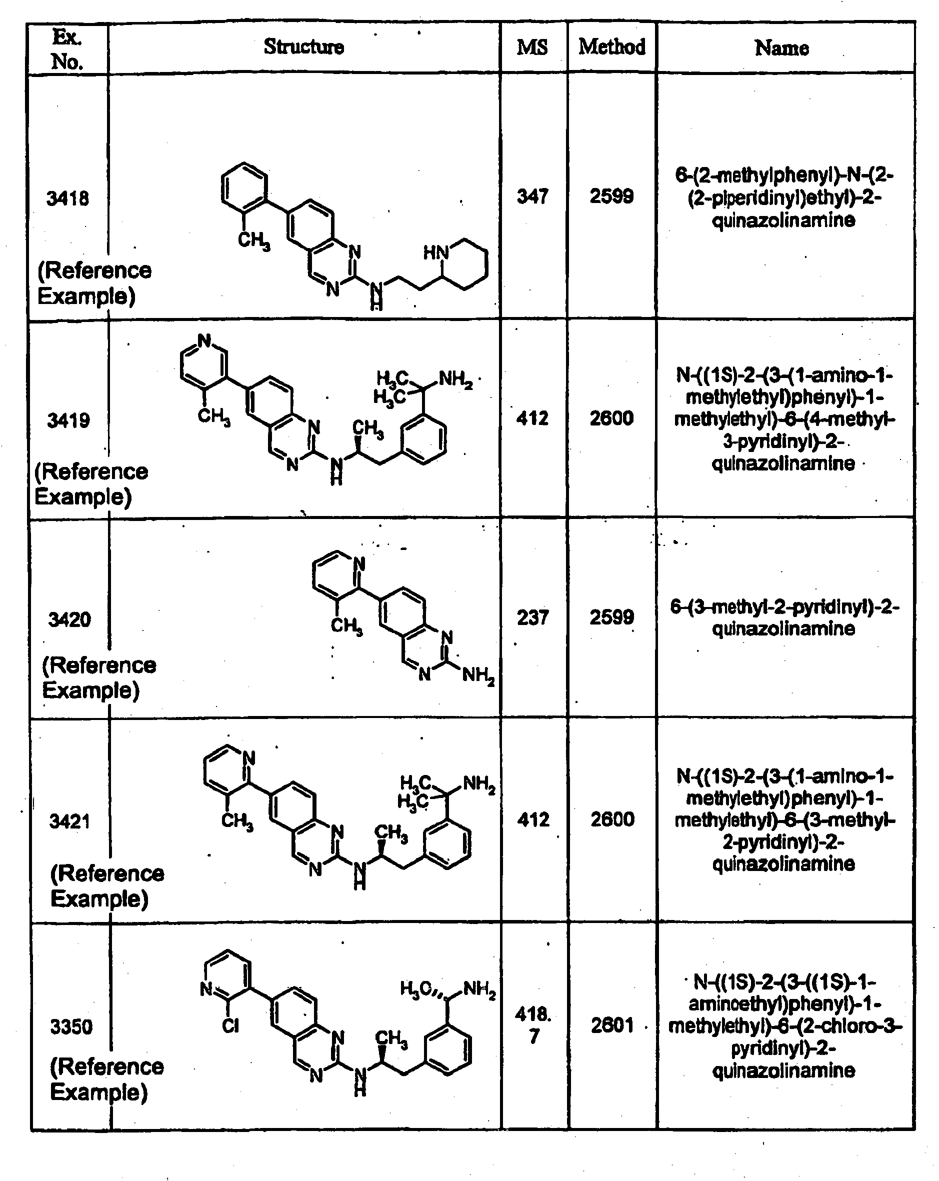

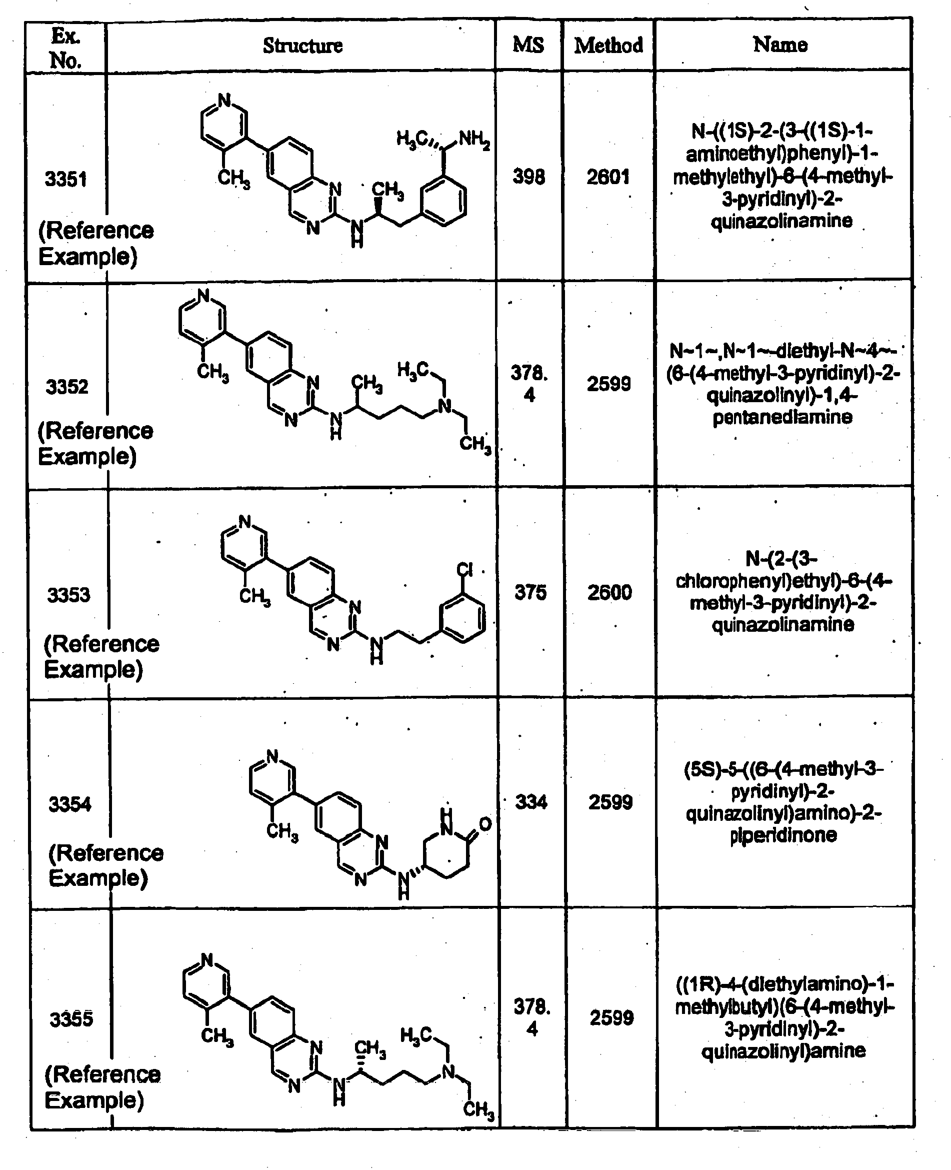

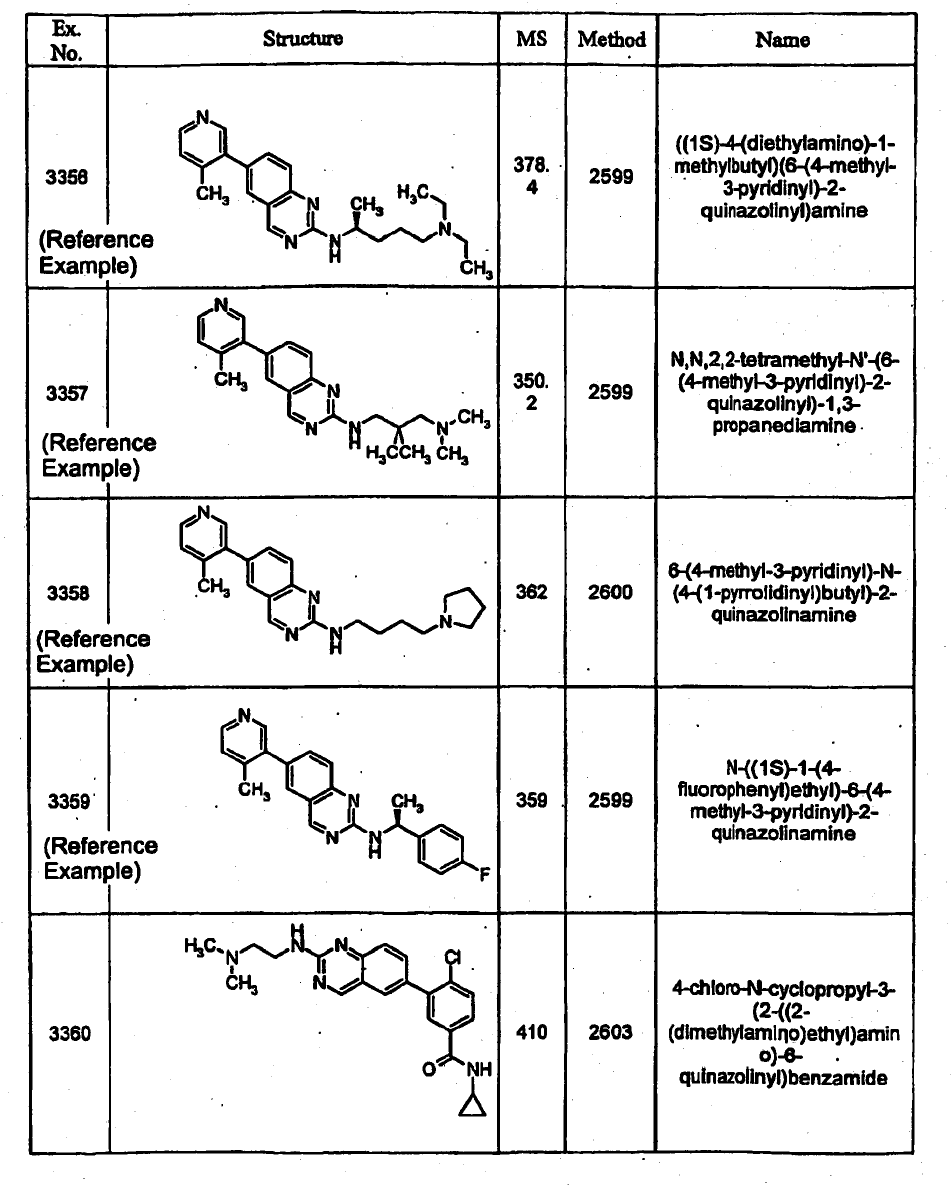

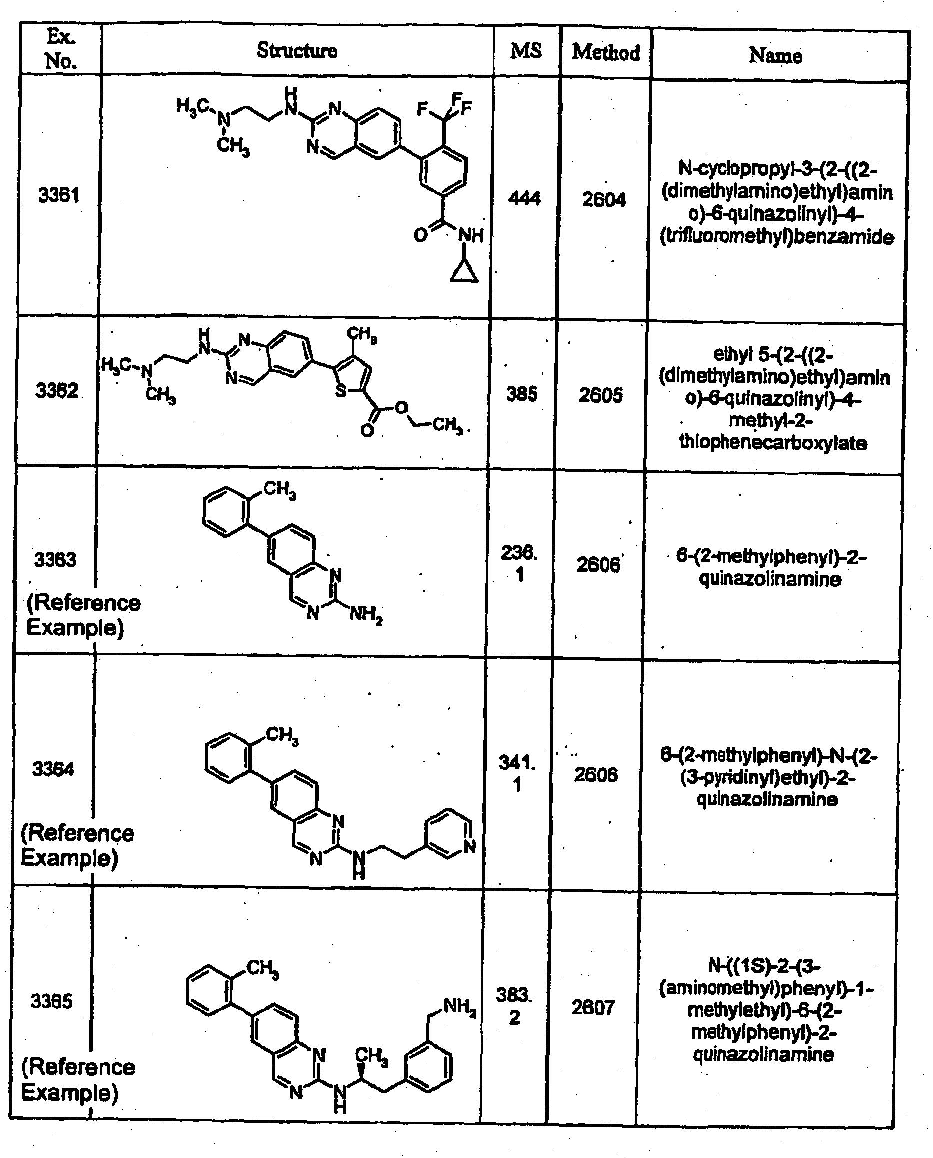

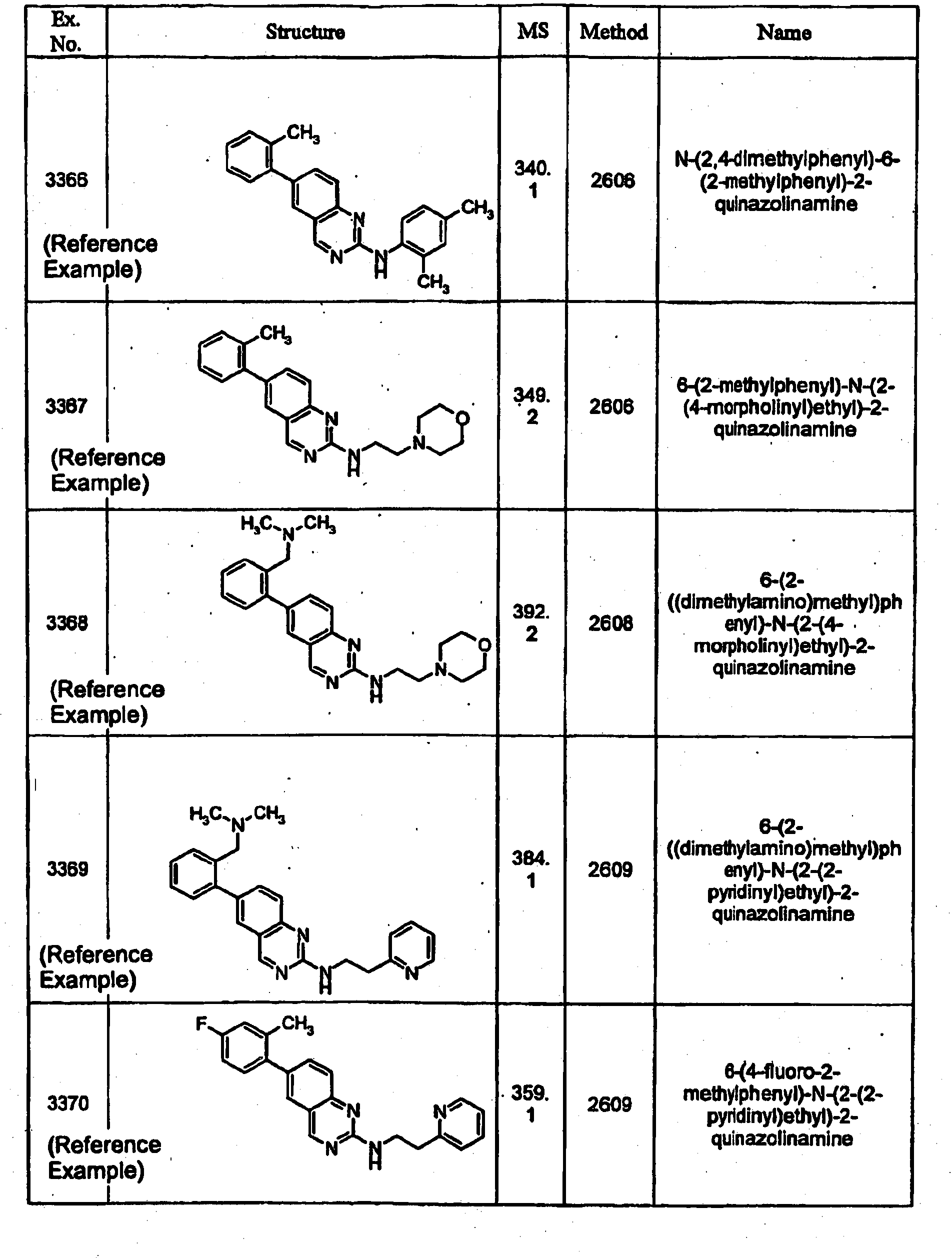

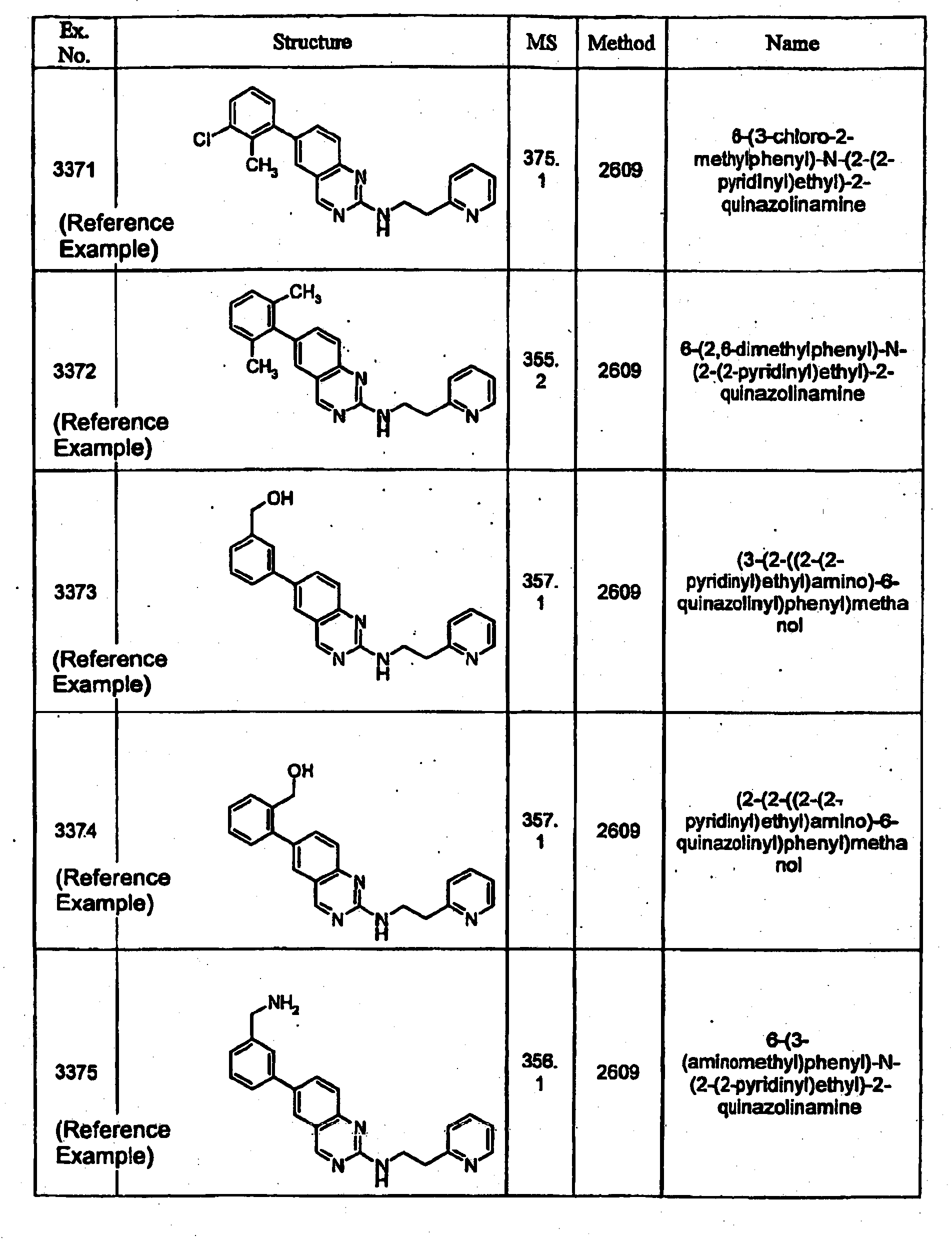

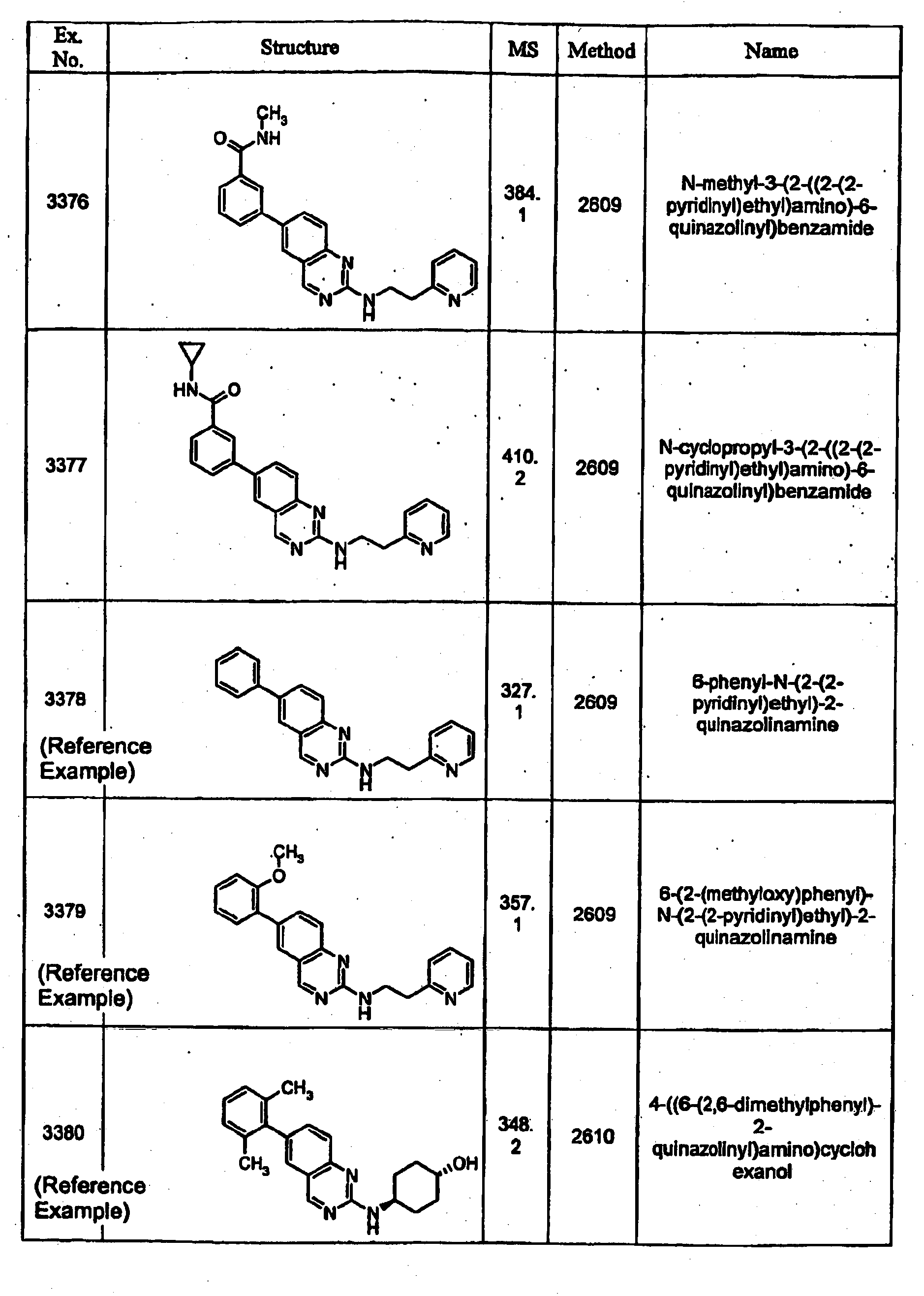

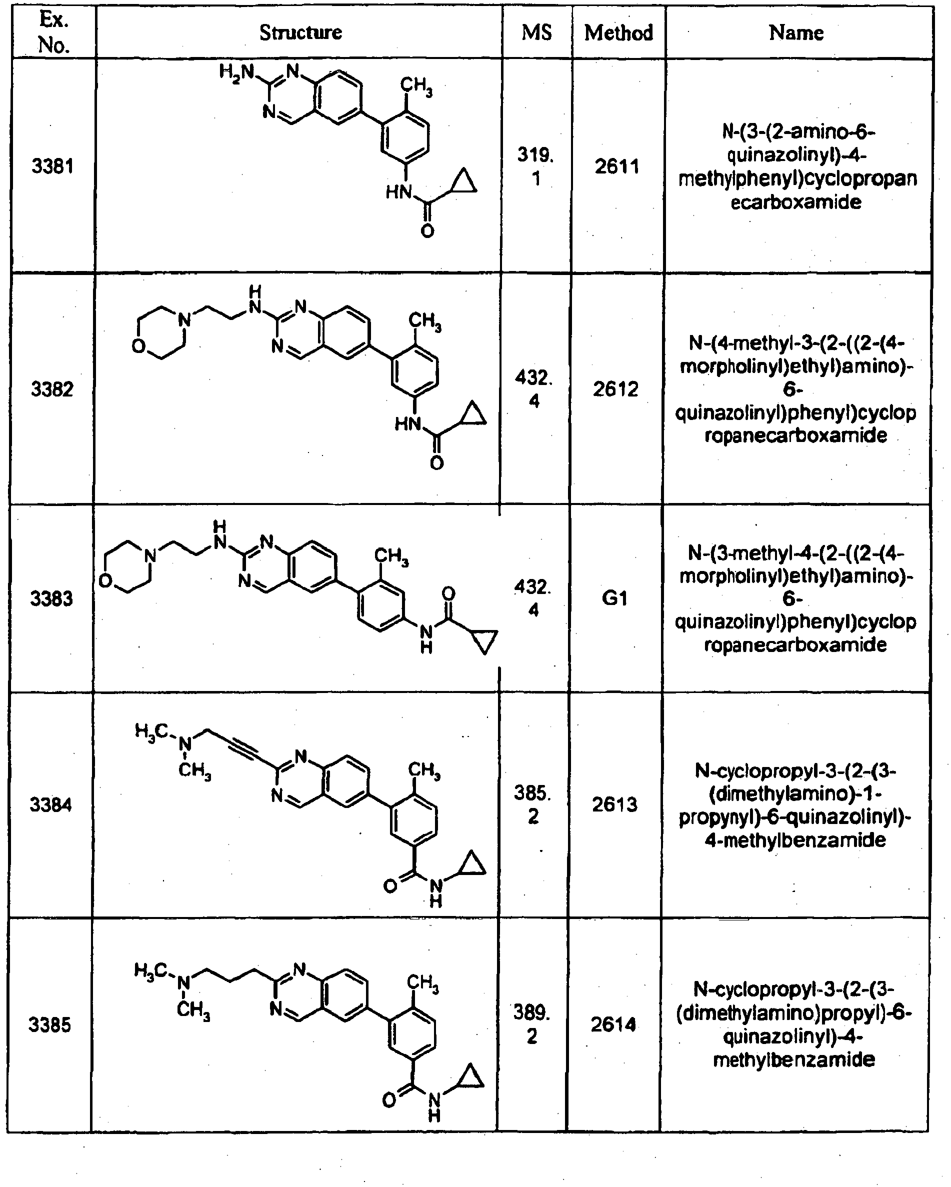

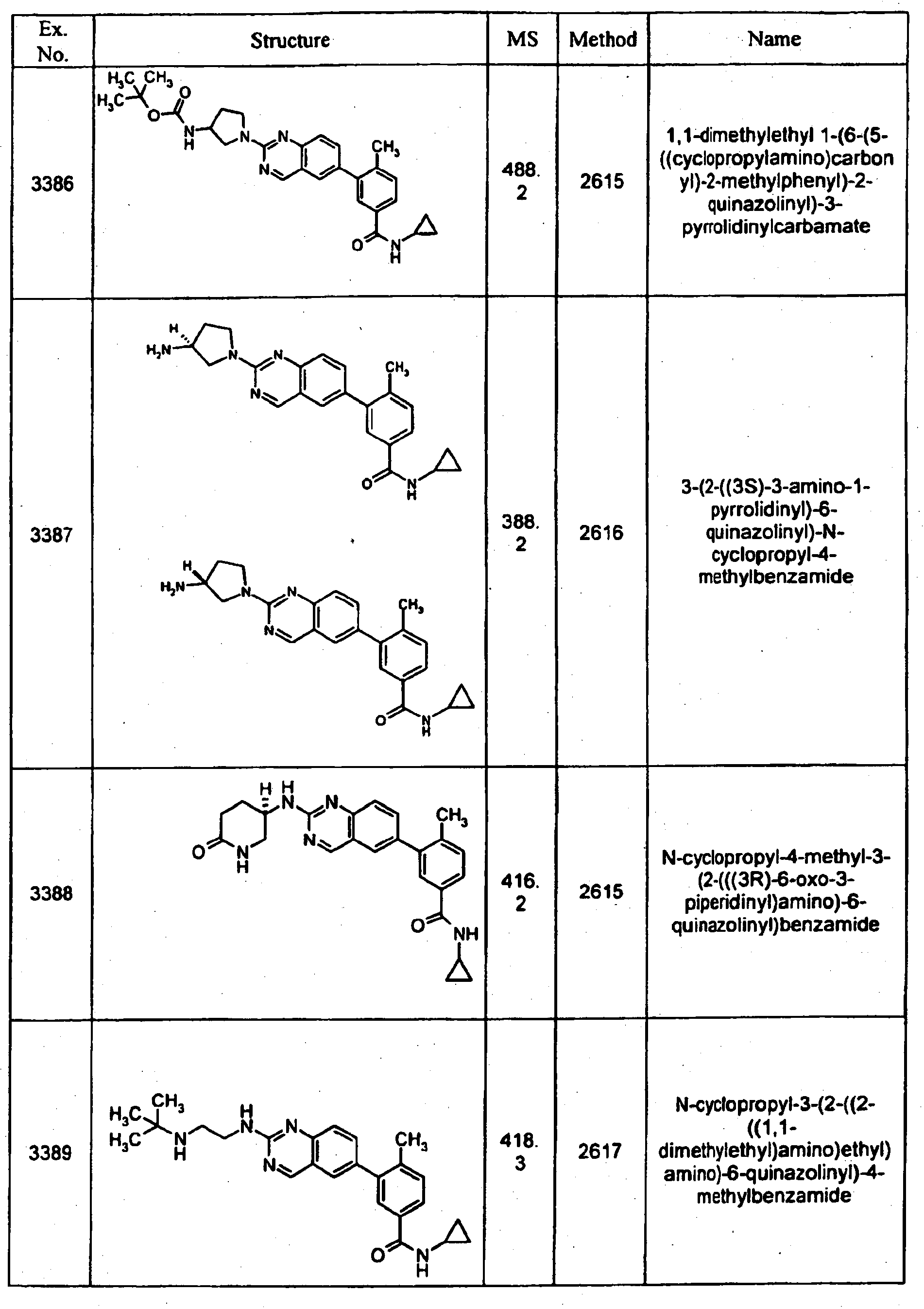

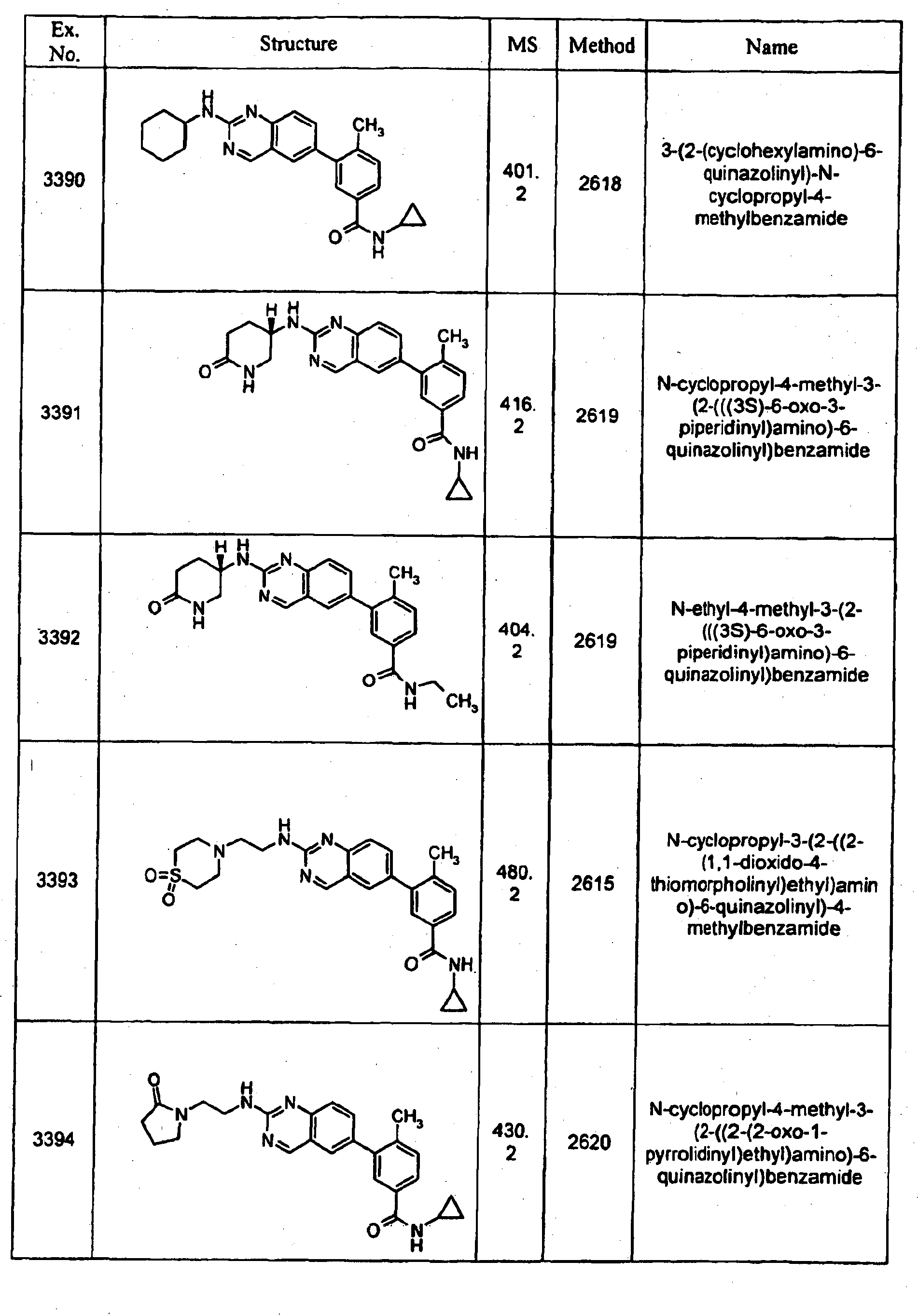

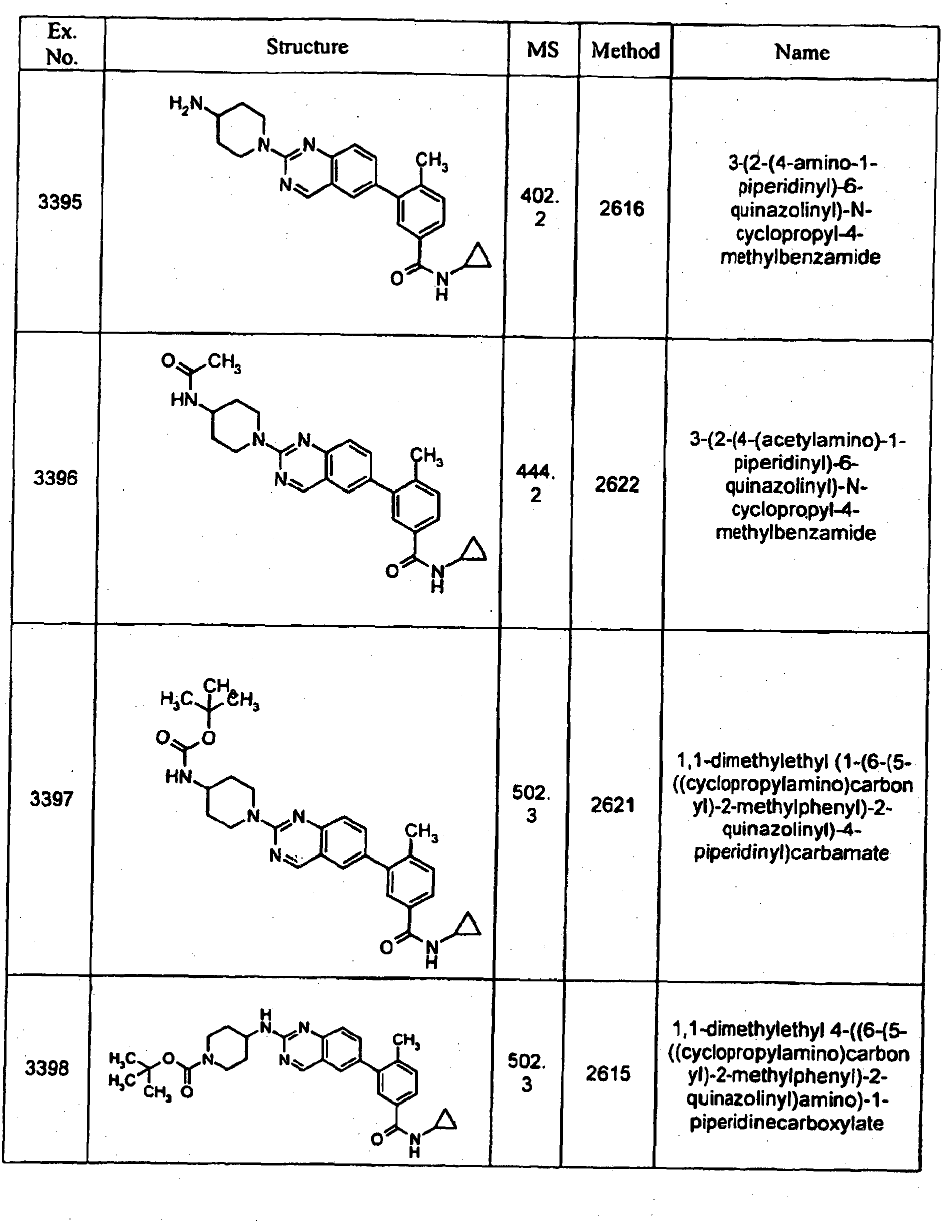

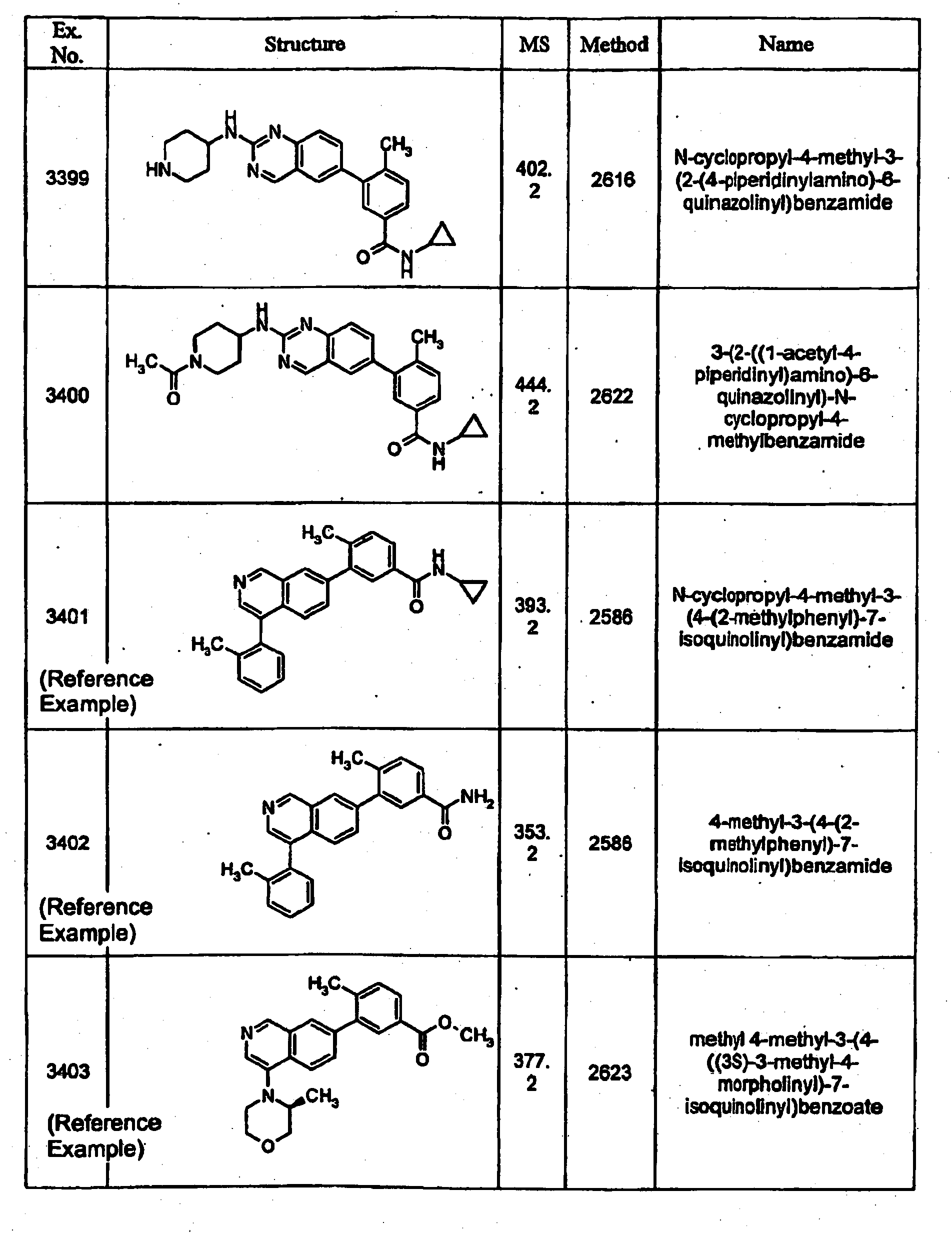

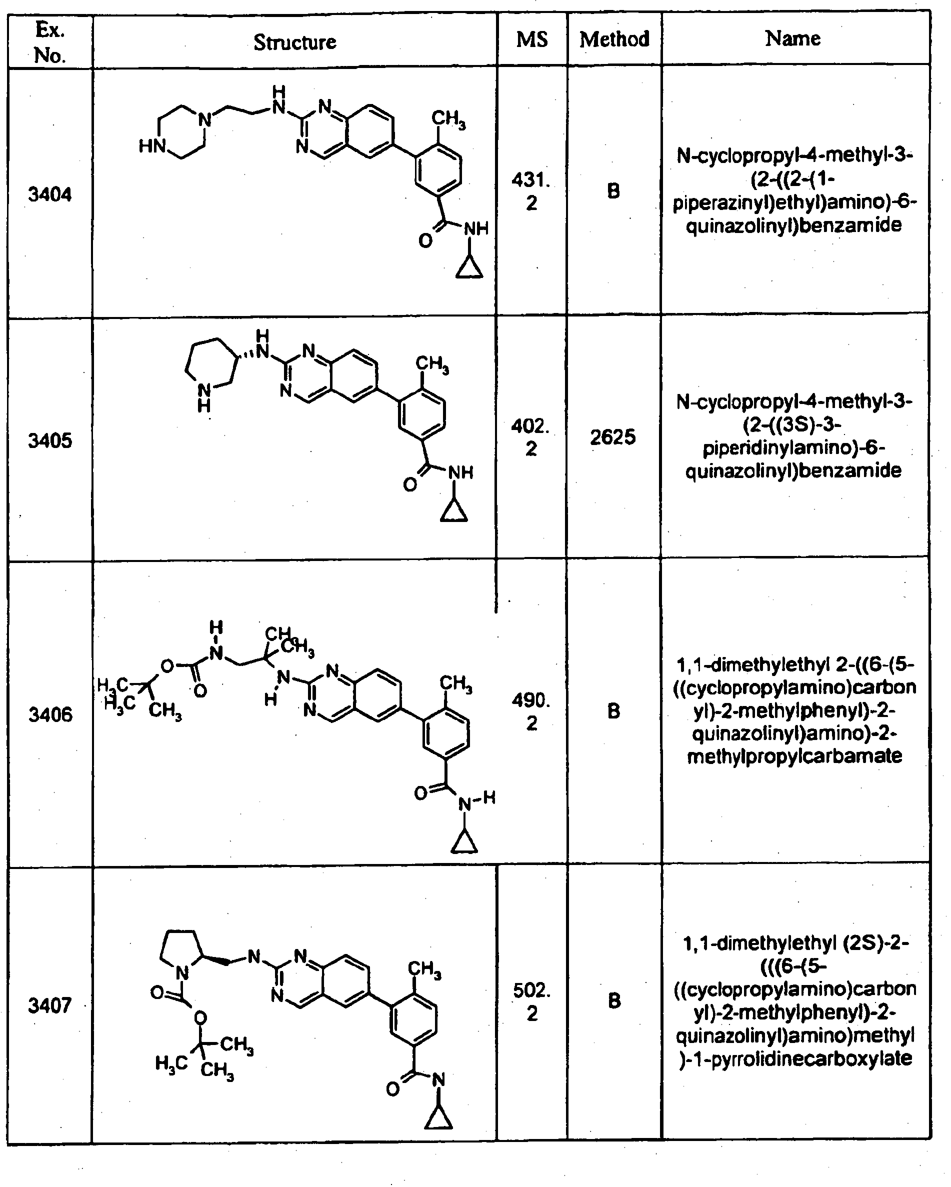

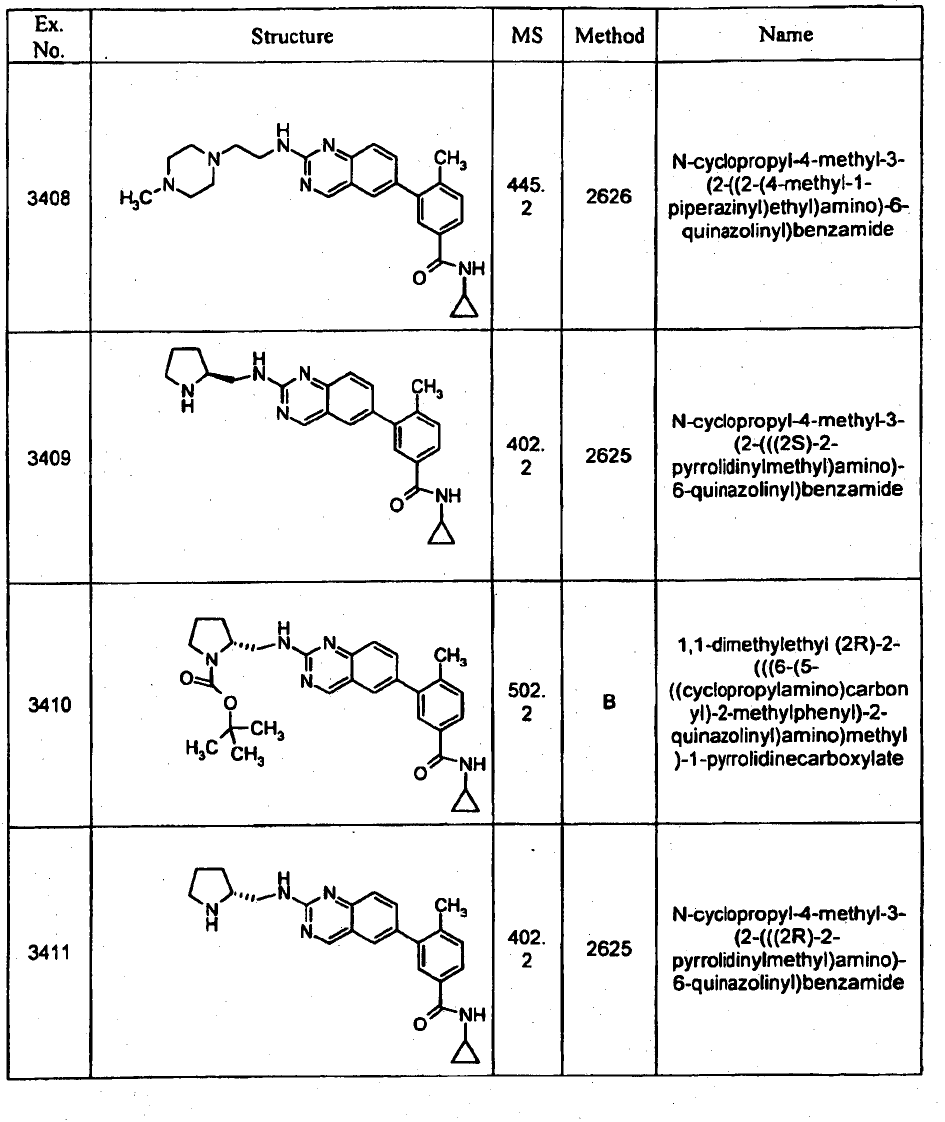

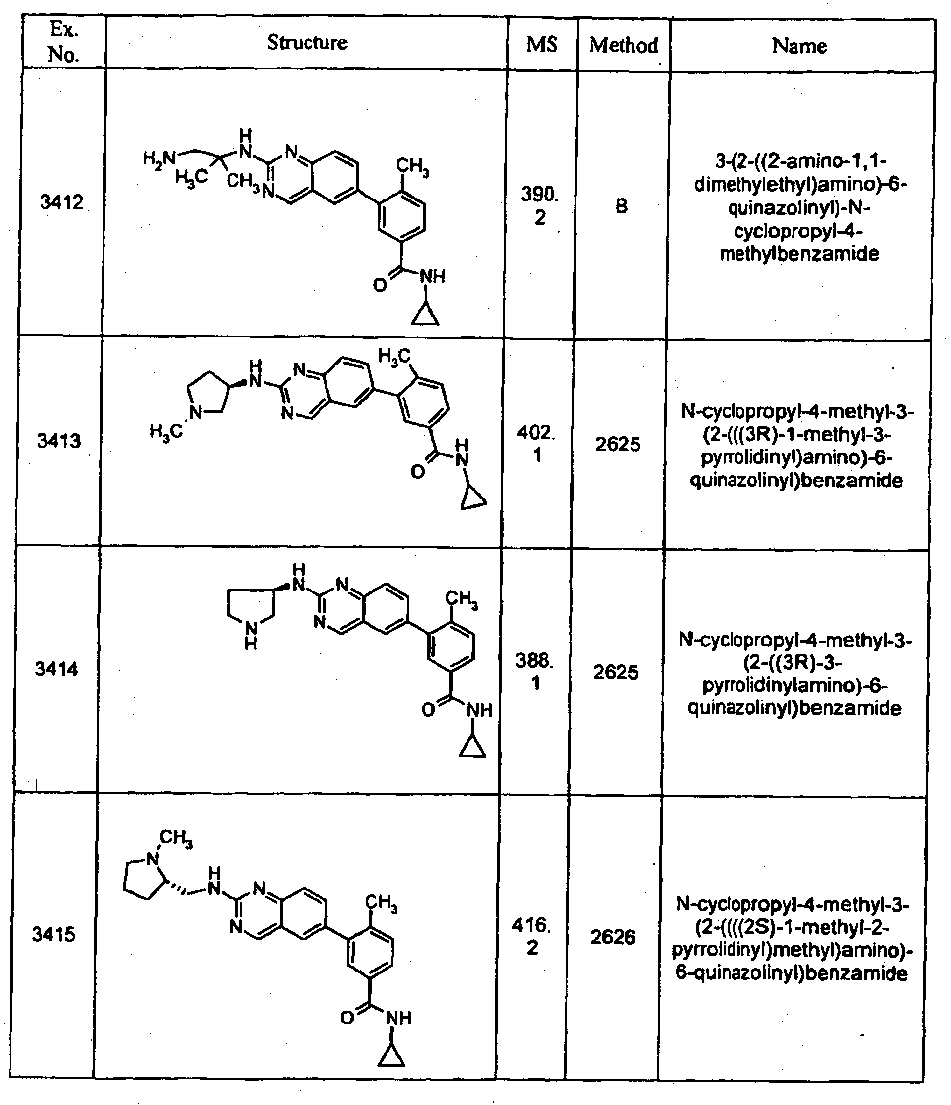

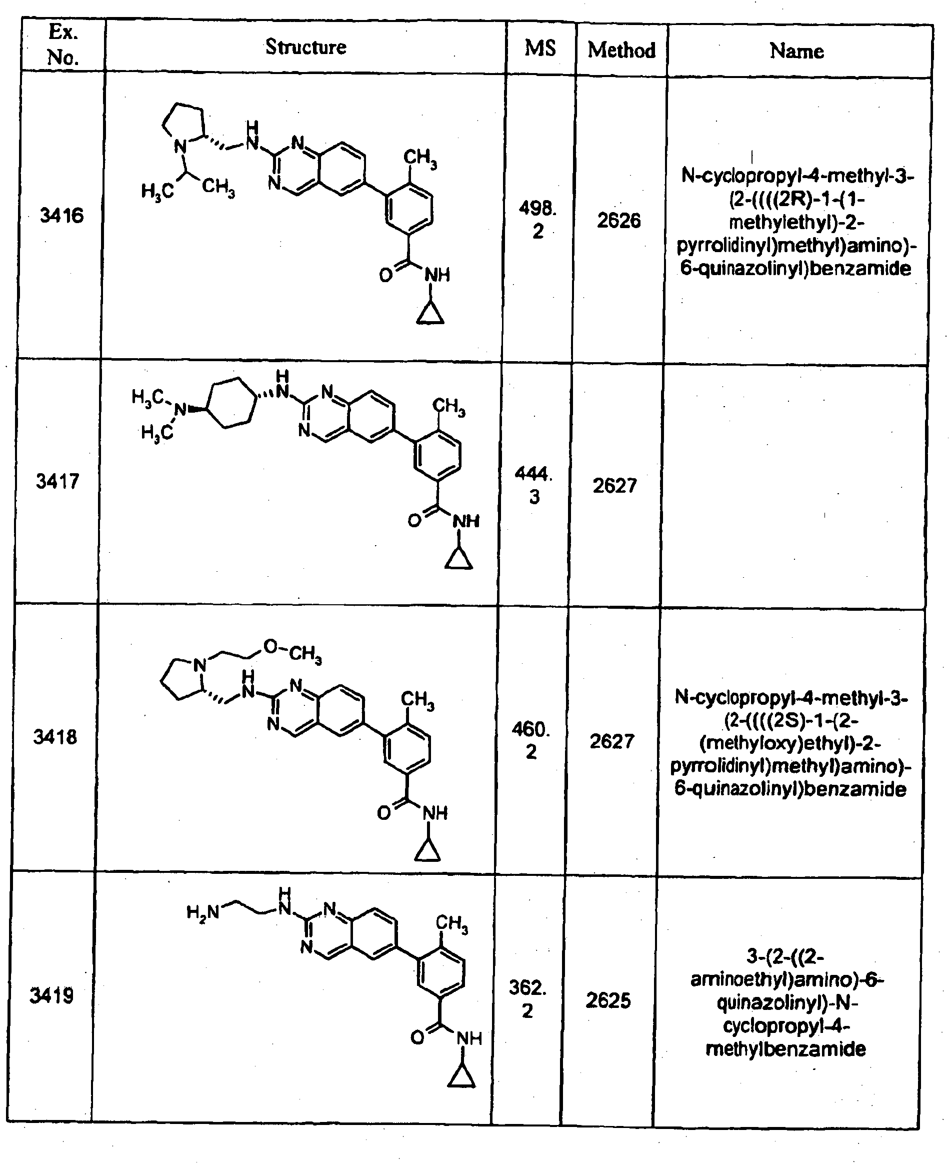

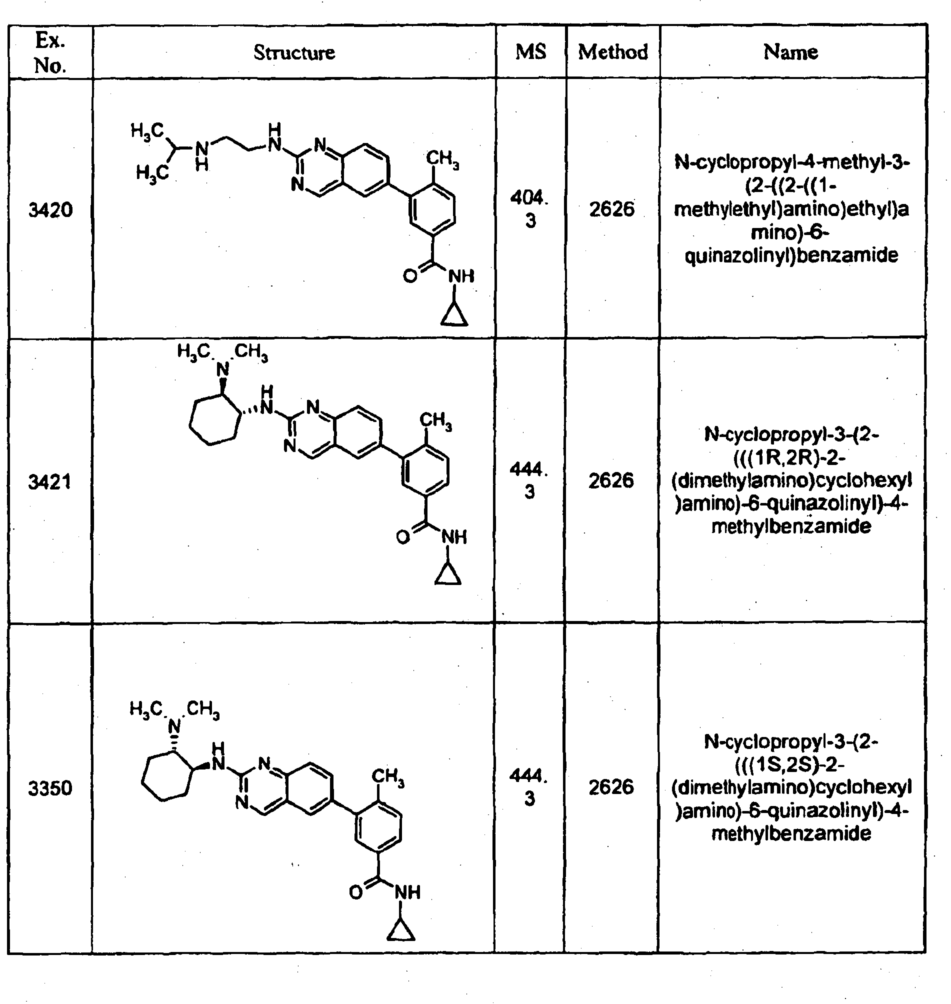

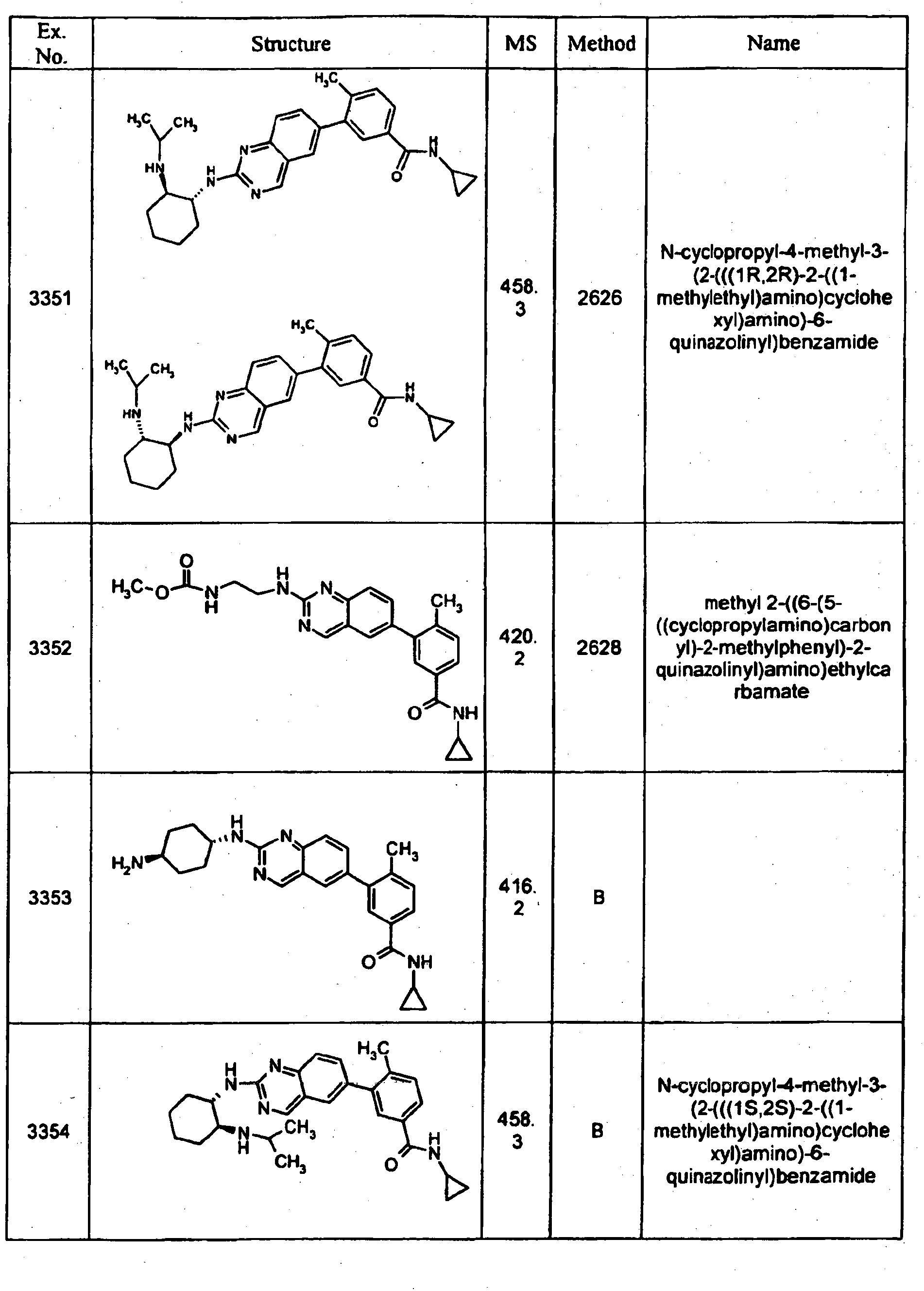

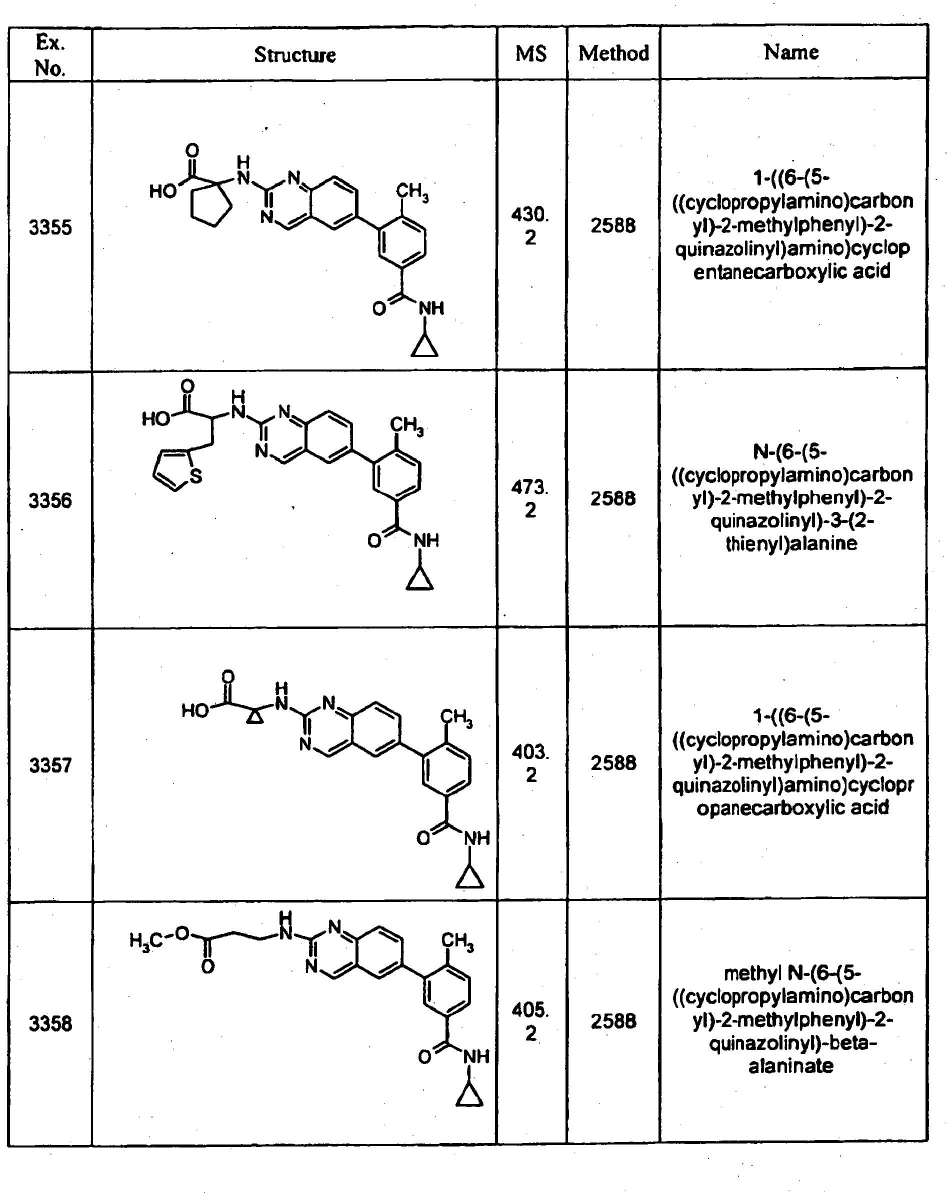

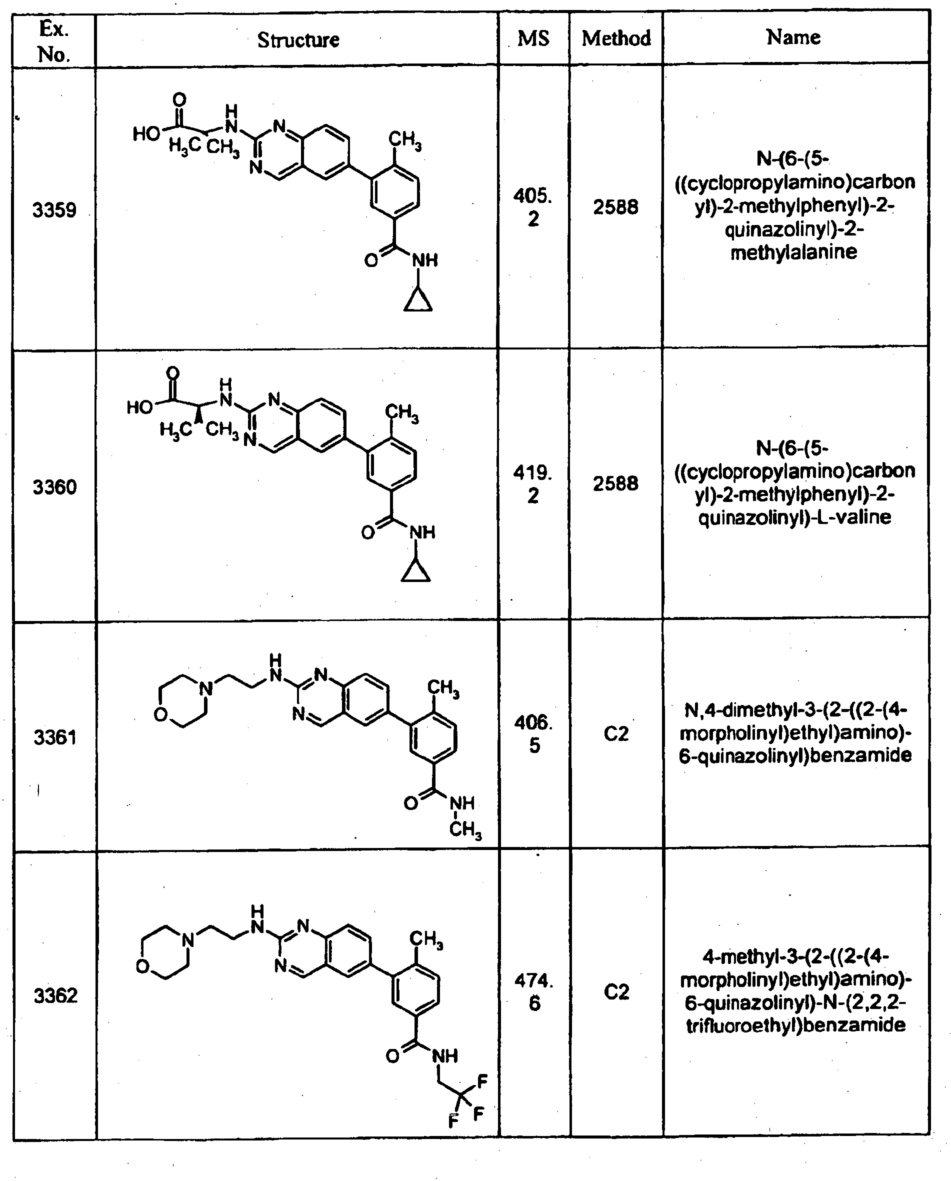

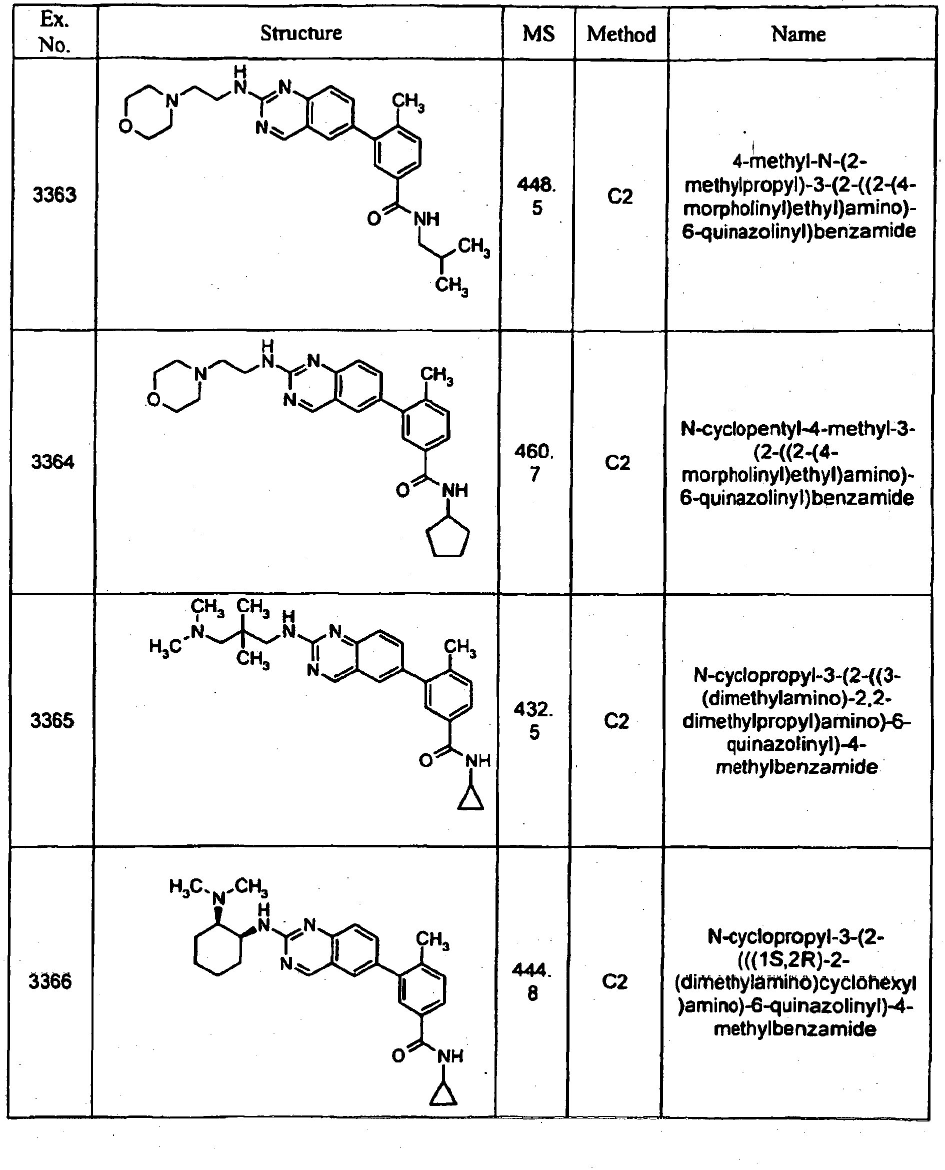

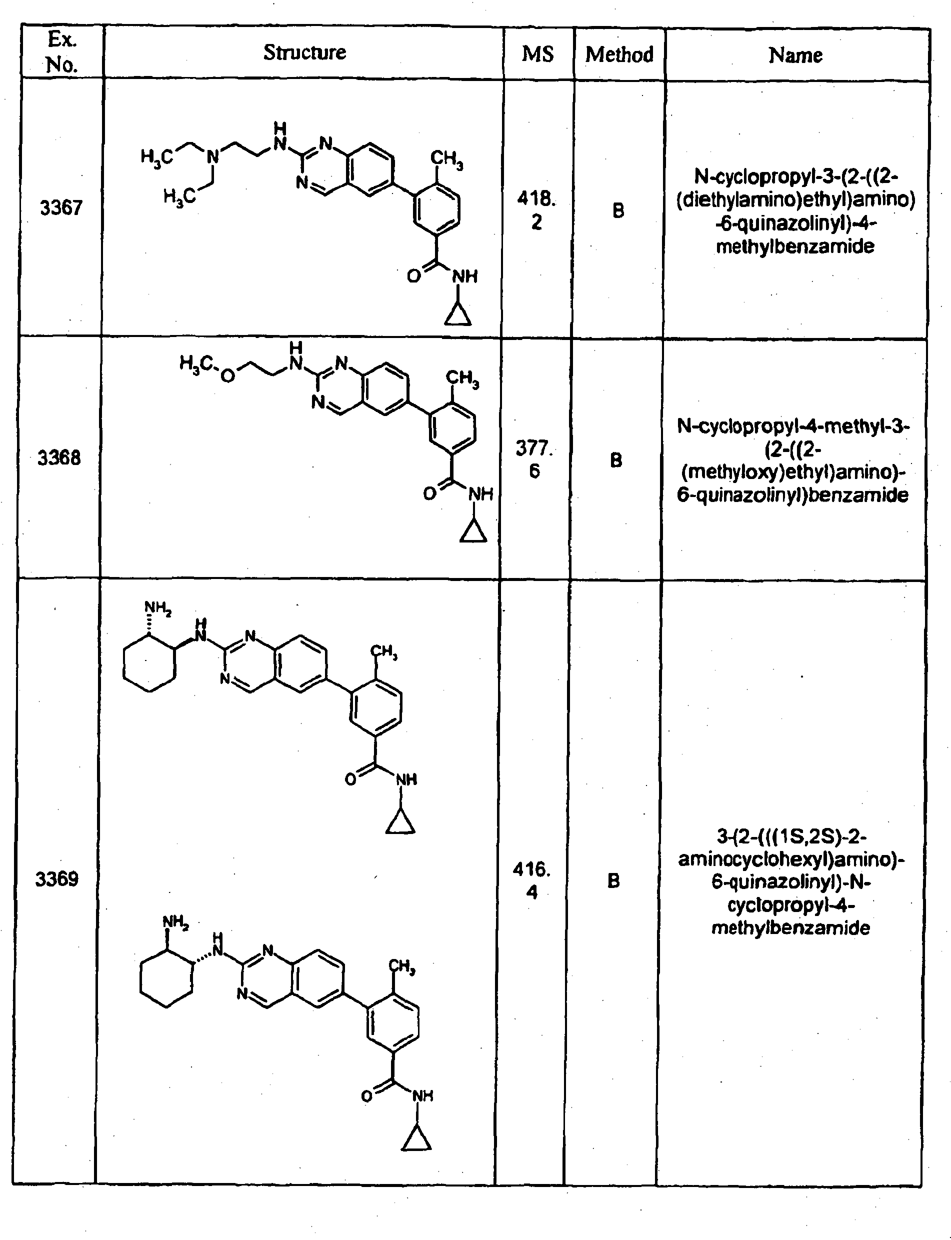

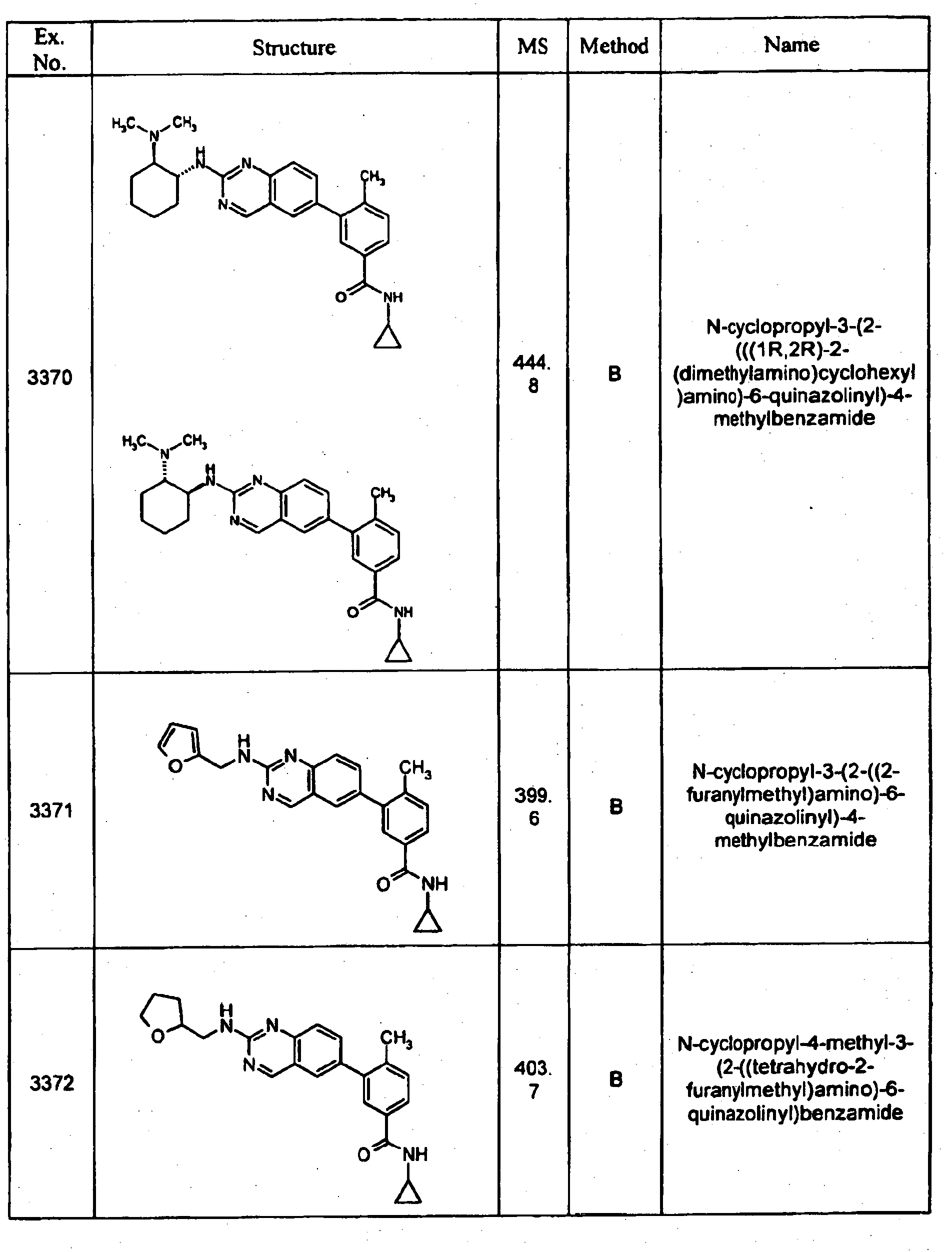

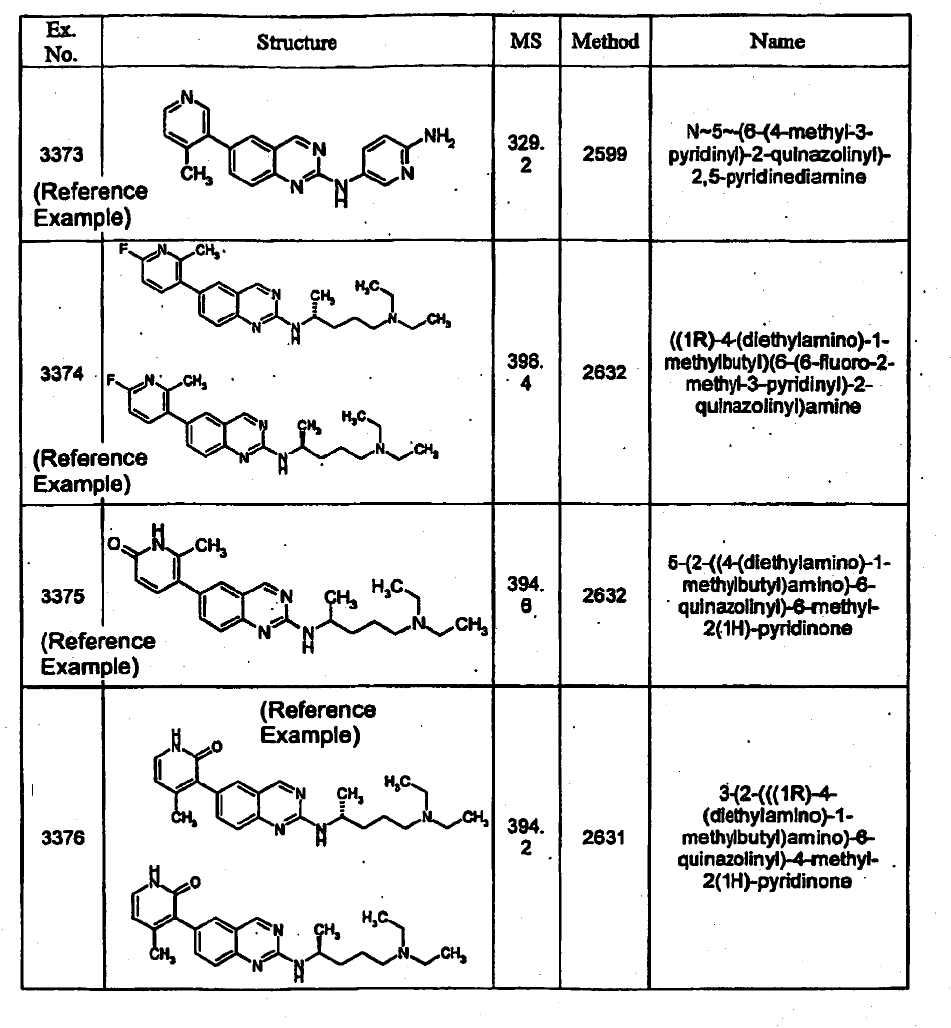

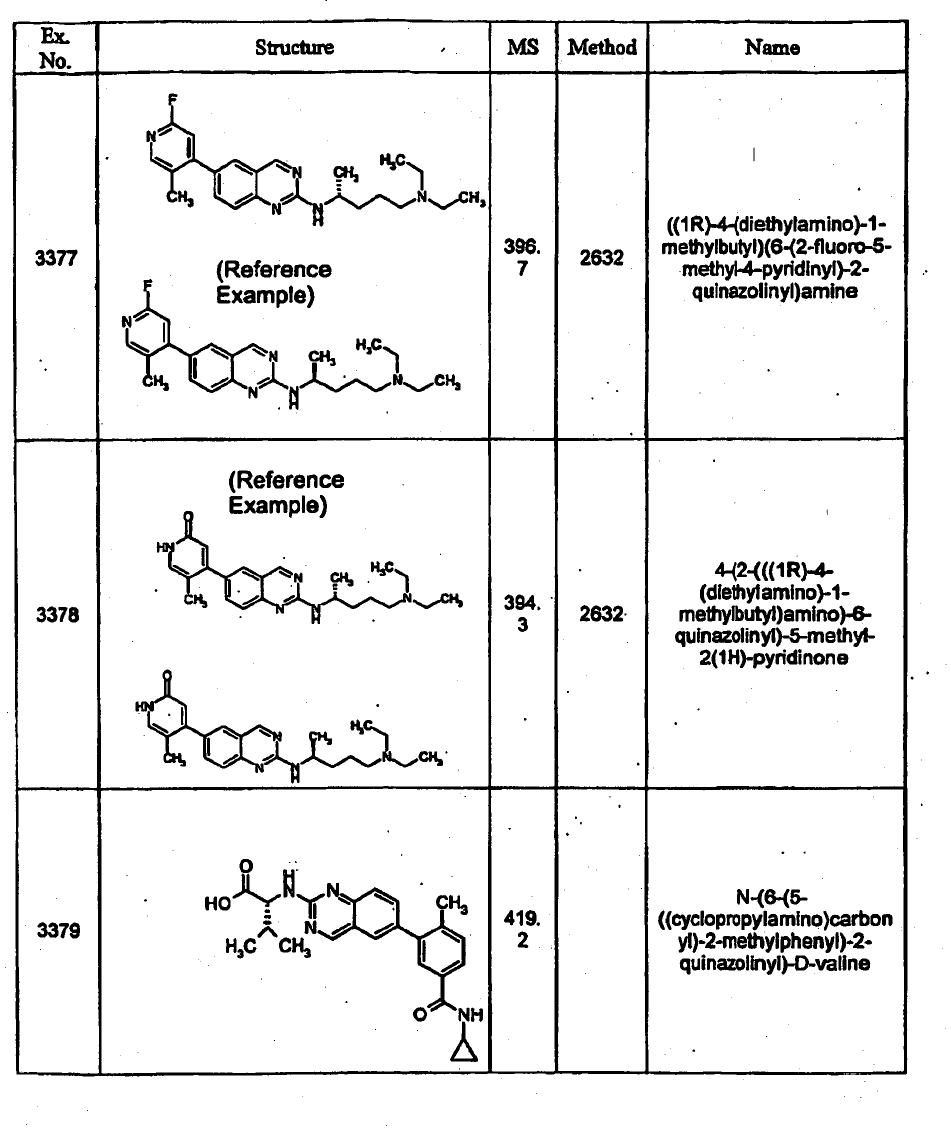

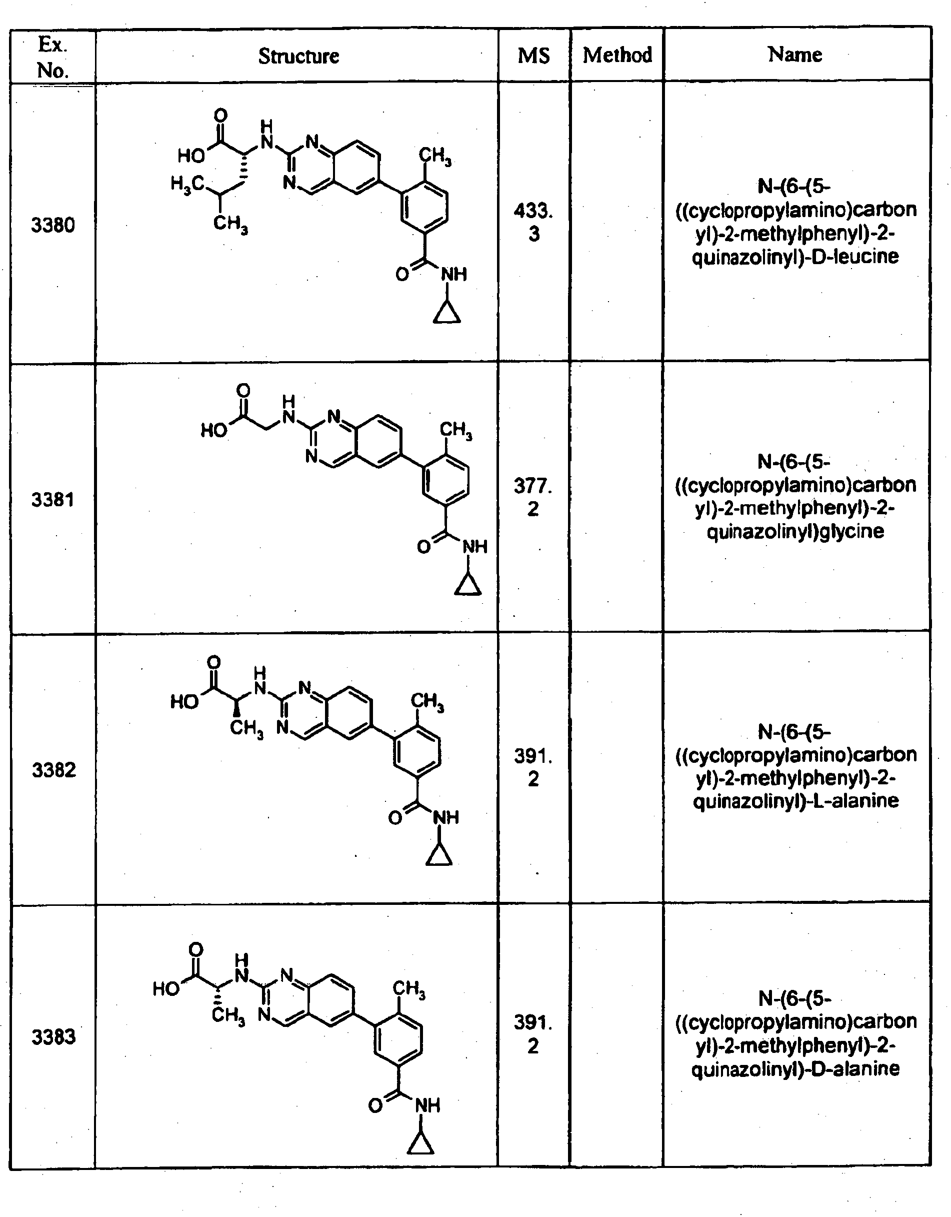

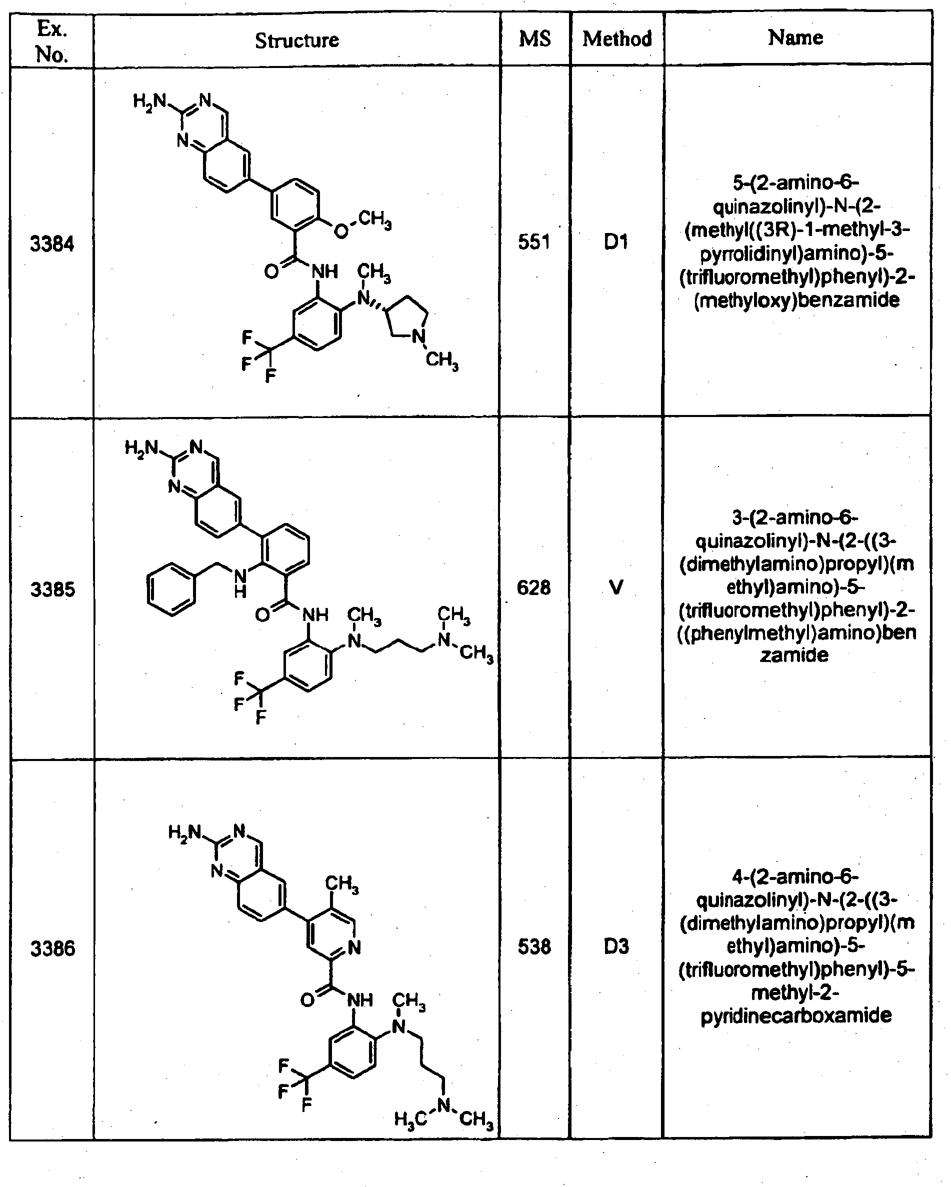

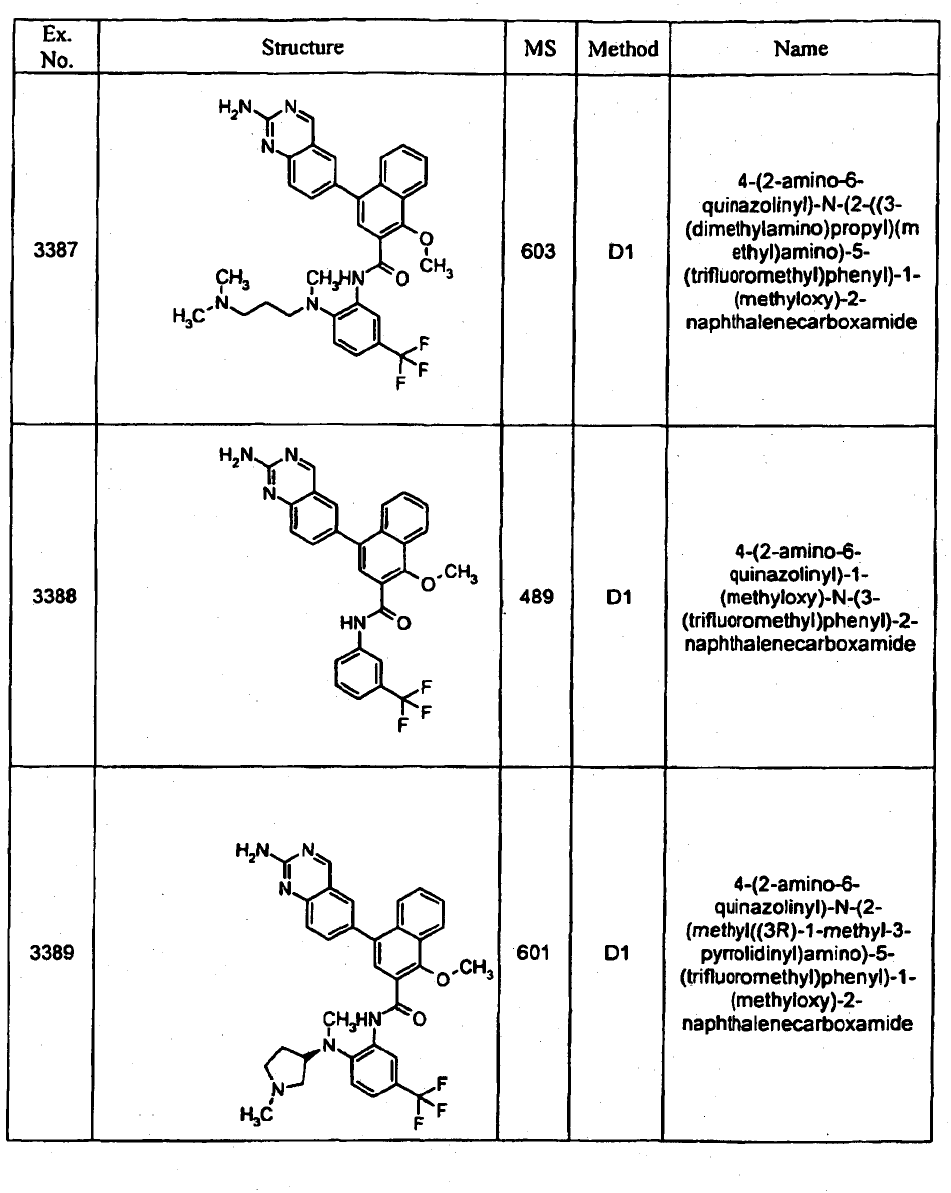

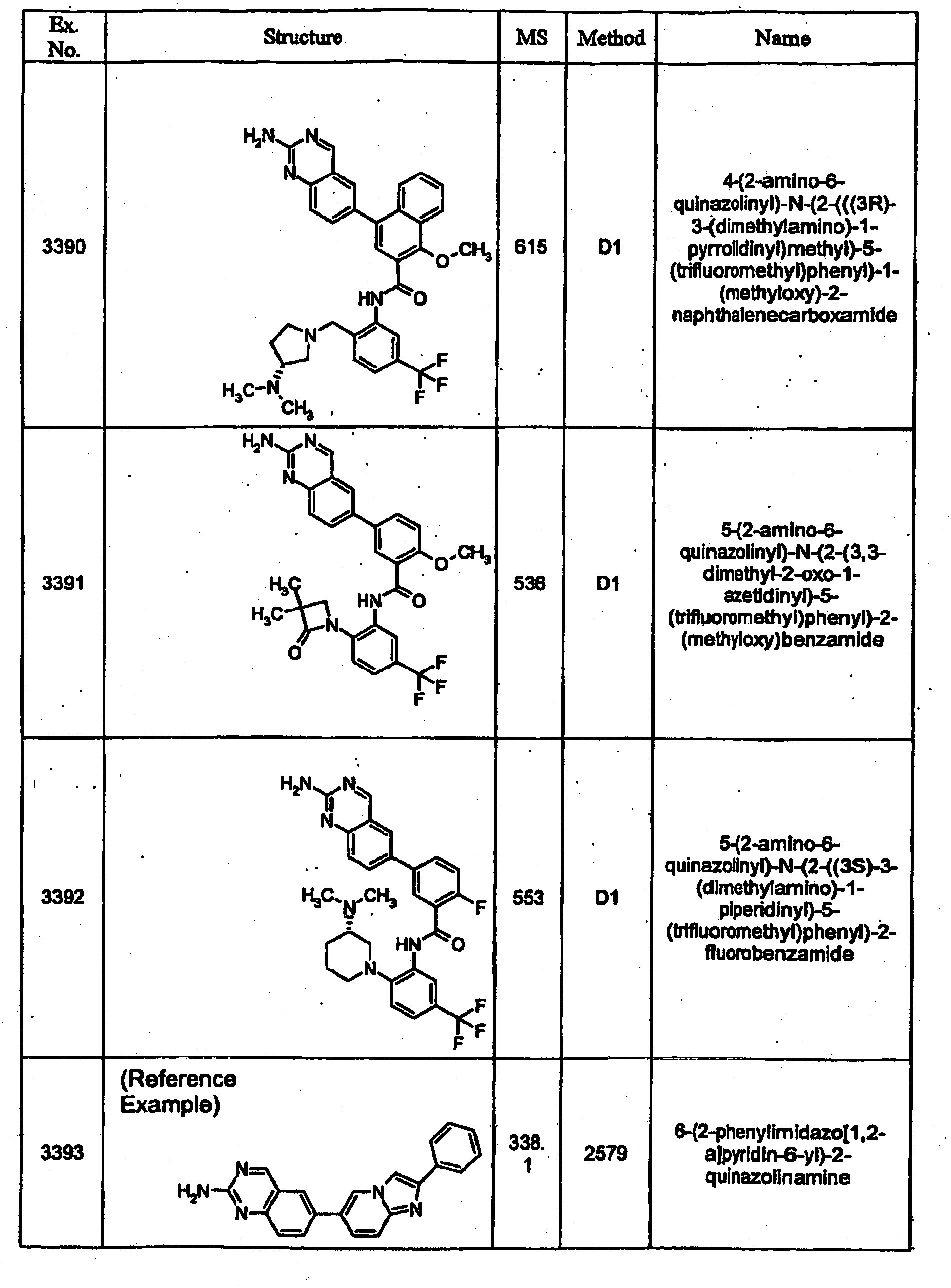

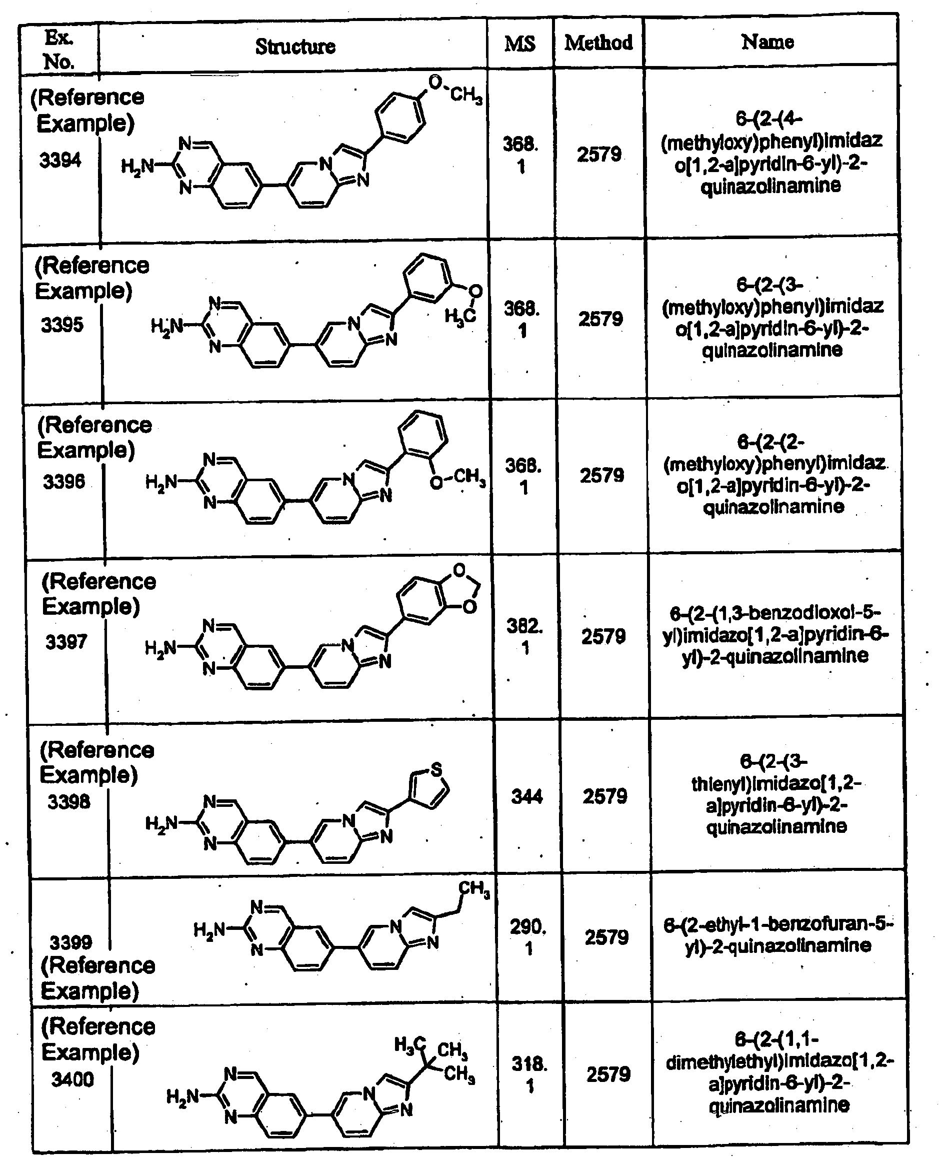

### BIOLOGICAL EVALUATION

The following assays can be employed to determine the degree of activity of a compound as a protein kinase inhibitor. Compounds described herein have been tested in one or more of these assays, and have shown activity. Representative compounds of the invention were tested and found to exhibit IC₅₀ values of at least < 25 µM in any one of the described assays, thereby demonstrating and confirming the utility of the compounds of the invention as protein kinase inhibitors and in the prophylaxis and treatment of immune diseases, proliferative disorders, angiogenic diseases, etc.

### TIE-2- HOMOGENOUS TIME RESOLVED FLOURESCENT (HTRF) KINASE

### ASSAY

IC₅₀'s for the inhibition of the Tie-2 kinase enzyme for individual compounds were measured using an HTRF assay, utilizing the following procedure:
In a 96 well plate (available from Costar Co.) was placed 1 uL of each test and standard compound per well in 100% DMSO having a 25 uM final compound concentration (3-fold, 10 point dilution). To each well was added 20 uL of a reaction mix formed from Tie-2 (4.0 uL; of a 10 mM stock solution available from Gibco), 0.05% BSA (0.1 uL; from a 10% stock solution available from Sigma-Aldrich Co.), 0.002 mM of BLC HER-2 KKK (Biotinylated Long chain peptide; 0.04 uL; from a 0.002 mM stock solution), 0.01 mM concentration of ATP (0.02 uL; commercially available from Sigma-Aldrich Co.) and the remaining solution was water (15.84 uL) to make to a total volume of 20 uL/well.

The reaction was initiated in each well by adding 20 uL per well of an enzyme preparation consisting of a 50 mM concentration of Hepes (1.0 uL; from a 1000 mM stock solution commercially available from Gibco Co.), 0.05% concentration of BSA (0.1 uL), 4 mM of DTT (0.08 uL; from a 1000 mM stock solution available from Sigma-Aldrich Co.), a 2.4 x 10⁻⁷ concentration of Tie-2 (0.02 uL, from a 4 mM concentration stock), with the remaining volume being water (18.8 uL) to dilute the enzyme preparation to a total volume of 20 uL. The plate was incubated for about 90 minutes at RT. After incubation, a 160 uL of a filtered detection mixture, prepared from 0.001 mg/ml of SA-APC (0.0765 uL; available as a 2.09 mg/ml stock solution from Gibco), 0.03125 nM concentration of Eu-Ab (0.1597 uL; available in a 31.3 nM stock solution from Gibco), with the remaining volume being Detection buffer (159.73 uL), was added to each well to stop the reaction therein. The plate was then allowed to equilibrate for about 3 hr and read on a Ruby Star fluorescent reader (available from BMG Technologies, Inc.) using a 4 parameter fit using activity base to calculate the corresponding IC₅₀'s for the test and standard compounds in each well. The following exemplary compounds were found to have IC₅₀'s for the inhibition of Tie-2 as measured by the HTRF assay of less than or equal to 1 uM: Examples 1-12, 16-32, 34-41, 58-61, 64-67, 74-84, 87-110, 13-120, 122-131, 133, 136-144, 146-148, 150, 152-167, 169-171, 173-179, 184, 186, 189-194, 196-200, 202-203, 214-222, 224-225, 227-232, 234, 236-269, 271, 273, 276, 279, 280, 282, 284, 287, 290, 292-296, 298, 300-302, 304, 315-320, 322, 324-326, 328, 332-336, 339-342, 345, 347-349, 351, 355-356, 359-361, 363-374, 377, 396, 401-403, 405-407, 415-423, 429-435, 438, 441, 442, 444-453, 455, 457, 464-466, 469, 471, 473, 479, 481, 484, 489-995, 503, 512, 513, 519-522, 524, 525, 528-531, 534 and 537-542.

### TIE-2 CELL-BASED DELFIA ASSAY

### Day 1 - Plate Preparation

Three 175ml flasks of EAHY926 cells were obtained from the University of N. Carolina. All cells were trypsinized (i.e., washed with 20 mL of PBS followed by 3 mL of trypsin-EDTA obtained from Gibco Co., cat. no. 25300-054, for 5 min at RT), then cultured in a growth medium solution containing DMEM (High glucose, Gibco Co., cat. no. 1965-092), 10% FBS serum (Gibco Co., cat. no. 10099-141) and P/S (Penicillin-Streptomycin-Glutamine; Gibco Co., cat. no. 10378-016) culture media. The cells were counted using a Z2® coulter® counter. The cells were plated in four 24-well tissue culture plates (Costar Co., cat. no. 353047) to initially contain 4x10⁵ cells/ml per well, and then loaded to 500 uL volume having a final cell density of 2 x 10⁵ cells/well. The cells were incubated for 5 or more hours at 37 °C under 5% CO₂. The DMEM + 10% serum + P/S culture media was removed and the cells washed twice with 500 uL of PBS (without Ca+ and Mg⁺⁺; Gibco Co., cat. no. 14190-136) at RT. 500 uL of 0.5% FBS + F12 (F12 nutrient mixture; Gibco Co., cat. no. 11765-054) was added to each well and the cells were incubated at 37 °C overnight (about 15 hr).

100ug of anti-hTie2 antibody (R & D Systems, Inc., Cat. No. AF313) was diluted with 10mL of ice-cold PBS to prepare a 10ug/mL antibody concentration stock. A 96-well microplate (Perkin-Elmer Wallac, cat. no. AAAND-0001) was coated with 100uL of the anti-Tie2 antibody stock and the coated plate was stored at 4°C overnight.

### Day 2 - Compound Plate Preparation

The media in the microplate was replaced with a preparation of 500uL DMEM + 1% BSA (Bovine Serum Albumin; ICN Biomedicals, Inc., cat. no. 160069). 20 uL of a selected Tie2 reference compound was placed in a selected well of the 96-well plate, and diluted 1:4 with 100% DMSO from an initial concentration of about 10 mM to a final concentration of about 2.5mM, then diluted 1:3 with 100% DMSO for a 10 point dilution to a final concentration of about 0.128 uM.

Test compounds (10 uL of a 10 mM concentration) were similarly diluted 1:4 with 100% DMSO to obtain a sample concentration of about 2.5mM, then diluted 1:3 for a 10 point dilution to finally obtain a concentration of about 0.128 uM for each test compound. 20 uL of 100% DMSO served as positive controls, while and 10 uL of the 2.5mM concentration of the reference compound served as the negative control.

A 2 uL aliquot from each well (test compounds, positive and negative controls) in the 96-well plate was added to designated wells in the 24-well cell culture plate (1:250). The culture plate was incubated for 2.5 at 37 °C in an atmosphere of about 5% CO₂.

The Tie-2 ligand was stimulated with the following series of preparations: (1) about 0.5 mL of a protease inhibitor cocktail (Sigma-Aldrich Co., cat. no. P8340) was thawed; (2) to prepare the phosphatase inhibitor, a 300 mM NaVO₄ (Sigma-Aldrich Chem. Co., cat. no. S6508-10G) stock solution in PBS was made and stored at RT. Two 1 mL aliquots of the NaVO₄ solution was prepared in separate two vials by adding 100 uL of the NaVO₄ stock solution to 900 uL RT PBS and each solution was activated by adding 6 uL of H₂O₂ to each vial. Both NaVO₄ solutions were mixed, wrapped in aluminum foil and stored at RT for 15 min.

The Delfia plates, containing 200 uL of PBS + 0.1%TWEEN20, were washed three times and blocked by adding 200 uL of a diluted solution of 5% BSA (16 mL of stock 7.5% BSA solution, available from Perkin-Elmer Wallac, Cat. No. CR84-100, was diluted with 8 mL of room temperature PBS). The plates were then stored at room temperature for about one hour.

100 uL of 35% BSA solution was diluted with 3.4 mL of ice cold PBS to make a 1% BSA/PBS solution. 100 uL of this 1% BSA/ PBS solution was diluted with 900 uL of ice cold PBS. hang1 was reconstituted with 250 uL of ice cold PBS + 0.1% BSA to make a 100 ug/mL concentration in solution. The solution was separated into 70 uL aliquots and stored at -80 °C.

1mL of the 30 mM solution of NaVO₄/PBS was diluted with 99 mL of ice cold PBS to form a 300 uM concentration. The solution was kept cold on ice. 210 uL of the activated NaVO₄ and 280 uL of the protease inhibitor preparation was added to 21 mL of RIPA buffer and kept cold on ice. Dilute hAng1 and stimulate cells:

70uL of the 100ug/mL stock solution was added to 700uL in 1% BSA/DMEM (1:10) to 10ug/mL concentration, and it was stored on ice. 5uL of this 10ug/mL hAng1 preparation was added to each well of the 24-well plate. The plate was shaken at 700 rpm at 37 °C for about 2.5 minutes.

After shaking, the wells were incubated for 7.5 min at 37 °C. The media was removed and 400uL of ice cold PBS + 300 uM NaVO₄ was added. The wells were kept on ice for at least 5 min and washed 1 X with ice cold PBS + 300 uM NaVO₄. The wells were tapped against a dry paper towel.
The cells were lysed with 150 uL of RIPA, 300 uM of NaVO₄, and 100 uL/1*10⁷ cells protease inhibitor cocktail (purchased from Sigma-Aldrich, Cat. No. P8340). The solution was incubated, then shaken on ice for 30 min.

The BSA blocking solution was removed from the 96-well plates, which were then tapped dry. 140 uL of cell lysate was added to the antibody-coated plate and the plate was incubated at 4 °C for 2 hours.

Delfia 25X Wash Buffer Concentrate (purchased from Perkin-Elmer Wallac, Cat. No. 1244-114) was diluted with 24 parts DDI water to obtain a washing solution. The lysate was removed and the plate was washed three times each with 400 uL of Delfia washing solution. The plate was tap dried with a paper towel.

The Anti-Phosphotyrosine clone 4G10 (purchased from Upstatebiotech Co., Cat. No. 05-321) was diluted with Delfia Assay Buffer (purchased from Perkin-Elmer Wallac, cat. no. 1244-1111) to make a solution of about 1 ug/mL in concentration. 100 uL of antibody was added to the plate and the plate was incubated at room temperature for one hour. The plate was again washed three times with 400 uL pre-time of the Delfia Washing solution.

The Eu-N1 labeled anti-mouse antibody (purchased from Perkin-Elmer Wallac, cat. no. AD0124) was diluted with Delfia Assay Buffer to make a solution of about 0.1 ug/mL in concentration.

100 uL of antibody was added to the plate and the plate was incubated at room temperature for one hour. The plate was again washed with Delfia Wash Buffer three times as described above. 100 uL of Delfia Enhancement Solution (purchased from Perkin-Elmer Wallac, Cat. No. 1244-105) was added to each well and the plate was incubated at room temperature for 5 min in the dark.

The Europium signal was measured with a Victor multilabel counter (Wallac Model 1420) while shaking (shake fast, linear, .10mm for 1s) using a Europium protocol.

Raw data was analyzed using a fit equation in XLFit. IC₅₀ values were then determined using Grafit software. Each of the examples described herein exhibited activity in the HTRF assay and the delfia cell-based assay with IC₅₀ values less than 10.0 µM. A majority of the compounds of examples 1-562 were found to have an IC₅₀ of less than 25 µM in the Tie-2 cell-based Delfia assay.

The compounds of the invention also were found to have inhibitory activity with respect to other kinase enzymes as well. For example, the compounds were found to be inhibitors of Lck, p38 and/or VEGF. The exemplary assays described as follows were used to make such determination.

### LCK-HOMOGENOUS TIME RESOLVED FLOURESCENT (HTRF) KINASE ASSAY

The LCK HTRF assay begins with LCK in the presence of ATP phosphorylating the biotinylated peptide Gastrin. The reaction incubates for 90 min. To quench the assay detection reagents are added which both stop the reaction by diluting out the enzyme and chelating the metals due to the presence of EDTA. Once the detection reagents are added the assay incubates for 30 min to allow for equilibration of the detection reagents.

The LCK HTRF assay is comprised of 10 µL of compound in 100% DMSO, 15 µL of ATP and biotinylated Gastrin, and 15 µL of LCK KD GST (225-509) for a final volume of 40 µL. The final concentration of gastrin is 1.2µM. The final concentration of ATP is 0.5µM (Km app= 0.6µM+/-0.1) and the final concentration of LCK is 250pM. Buffer conditions are as follows: 50mM HEPES pH 7.5, 50mM NaCl, 20mM MgCl, 5mM MnCl, 2mM DTT, 0.05% BSA.

The assay is quenched and stopped with 160 µL of detection reagent. Detection reagents are as follows: Buffer made of 50mM Tris, pH 7.5, 100mM NaCl, 3mM EDTA, 0.05% BSA, 0.1% Tween20. Added to this buffer prior to reading is Steptavidin allophycocyanin (SA-APC) at a final conc in the assay of 0.0004 mg/mL, and europilated anti-phosphotyrosine Ab (Eu-anti-PY) at a final conc of 0.025nM.

The assay plate is read in either a Discovery or a RubyStar. The eu-anti-PY is excited at 320 nm and emits at 615 nm to excite the SA-APC which in turn emits at 655 nm. The ratio of SA-APC at 655 nm (excited due to close proximity to the Eu-anti-PY because of phosphorylation of the peptide) to free Eu-anti-PY at 615 nm will give substrate phosphorylation.

Assays for other kinases are done in a similar way as described above, varying the concentrations of enzyme, peptide substrate, and ATP added to the reaction, depending on the specific activity of the kinase and measured Km's for the substrates.

### Human mixed lymphocyte reaction (huMLR):

The purpose of this assay is to test the potency of T cell activation inhibitors in an *in vitro* model of allogeneic T cell stimulation. Human peripheral blood lymphocytes (hPBL; 2x10⁵/well) are incubated with mitomycin C-treated B lymphoblastoid cells (JY cell line; 1x10⁵/well) as allogeneic stimulators in the presence or absence of dilutions of potential inhibitor compound in 96-well round-bottom tissue culture plates. These cultures are incubated at 37 °C in 5% CO₂ for 6 days total. The proliferative response of the hPBL is measured by ³H-thymidine incorporation overnight between days 5 and 6 after initiation of culture. Cells are harvested onto glass fiber filters and ³H-thymidine incorporation into DNA is analyzed by liquid scintillation counter.

### Jurkat proliferation/survival assay:

The purpose of this assay is to test the general antiproliferative/cytotoxic effect of compounds on the Jurkat human T cell line. Jurkat cells (1x10⁵/well) are plated in 96-well flat-bottom tissue culture plates with or without compound dilutions and cultured for 72 h at 37 °C in 5% CO₂. Viable cell number is determined during the last 4 h of culture by adding 10 µL/well WST-1 dye. WST-1 dye conversion relies on active mitochondrial electron transport for reduction of the tetrazolium dye. The dye conversion is read by OD at 450-600 nm.

### Anti-CD3/CD28-induced T cell IL-2 secretion and proliferation assay:

The purpose of this assay is to test the potency of T cell receptor (TCR; CD3) and CD28 signaling pathway inhibitors in human T cells. T cells are purified from human peripheral blood lymphocytes (hPBL) and pre-incubated with or without compound prior to stimulation with a combination of an anti-CD3 and an anti-CD28 antibody in 96-well tissue culture plates (1x10⁵ T cells/well). Cells are cultured for -20 h at 37 °C in 5% CO₂, then secreted IL-2 in the supernatants is quantified by cytokine ELISA (Pierce/Endogen). The cells remaining in the wells are then pulsed with ³H-thymidine overnight to assess the T cell proliferative response. Cells are harvested onto glass fiber filters and ³H-thymidine incorporation into DNA is analyzed by liquid scintillation counter. For comparison purposes, phorbol myristic acid (PMA) and calcium ionophore can be used in combination to induce IL-2 secretion from purified T cells. Potential inhibitor compounds can be tested for inhibition of this response as described above for anti-CD3 and -CD28 antibodies.

Assays for other kinases are done in a similar way as described above, varying the concentrations of enzyme, peptide substrate, and ATP added to the reaction, depending on the specific activity of the kinase and measured Km's for the substrates.

Exemplary compounds 1-12, 15-41, 44, 45, 47, 51-53, 55, 56, 58-71, 74-84, 86-93, 94-101, 103, 105-107, 109-111, 113-131, 133, 134, 136-149, 153-160, 162-238, 240-367, 369-383, 385, 388-410, 412, 414-417, 419-484, 487-495, 501-511, 512, 513 and 515-535 exhibited an average IC₅₀ value of 25uM or less in the human HTRF assay for the inhibition of the Lck kinase enzyme. Exemplary compounds 1-12, 15, 16, 18-41, 47, 51-53, 55, 56, 58-71, 74-84, 89-93, 95, 96, 98, 100-101, 103, 105-107, 109, 110, 113-131, 133, 136-148, 153-160, 162-163, 165-179, 183, 184, 186-236, 238, 243-258, 260-277, 279-366, 369-374, 376-381, 383, 385, 393-396, 400-410, 412, 414-417, 419-421, 423-484, 487-495, 501-503, 507-510, 512, 513 and 517-535 exhibited an average IC₅₀ value of 1 uM or less in the human HTFR assay for the inhibition of the Lck kinase enzyme.

The compounds were also found to be active inhibitors of the VEGF kinase receptor, as measured by the following described assays.

### HUVEC Proliferation Assay

Human Umbilical Vein Endothelial cells are purchased from Clonetics, Inc., as cryopreserved cells harvested from a pool of donors. These cells, at passage 1, are thawed and expanded in EBM-2 complete medium, until passage 2 or 3. The cells are trypsinized, washed in DMEM + 10% FBS + antibiotics, and spun at 1000 rpm for 10 min. Prior to centrifugation of the cells, a small amount is collected for a cell count. After centrifugation, the medium is discarded, and the cells are resuspended in the appropriate volume of DMEM + 10% FBS + antibiotics to achieve a concentration of 3x10⁵ cells/mL. Another cell count is performed to confirm the cell concentration. The cells are diluted to 3x10⁴ cells/mL in DMEM + 10% FBS + antibiotics, and 100 µL of cells are added to a 96-well plate. The cells are incubated at 37 °C for 22 h.

Prior to the completion of the incubation period, compound dilutions are prepared. Five-point, five-fold serial dilutions are prepared in DMSO, at concentrations 400-fold greater than the final concentrations desired. 2.5 µL of each compound dilution are diluted further in a total of 1 mL DMEM + 10% FBS + antibiotics (400x dilution). Medium containing 0.25% DMSO is also prepared for the 0 µM compound sample. At the 22 h timepoint, the medium is removed from the cells, and 100 µL of each compound dilution is added. The cells are incubated at 37 °C for 2-3 h.

During the compound pre-incubation period, the growth factors are diluted to the appropriate concentrations. Solutions of DMEM + 10% FBS + antibiotics, containing either VEGF or bFGF at the following concentrations: 50, 10, 2, 0.4, 0.08, and 0 ng/mL are prepared. For the compound-treated cells, solutions of VEGF at 550 ng/mL or bFGF at 220 ng/mL for 50 ng/mL or 20 ng/mL final concentrations, respectively, are prepared since 10 µL of each will be added to the cells (110 µL final volume). At the appropriate time after adding the compounds, the growth factors are added. VEGF is added to one set of plates, while bFGF is added to another set of plates. For the growth factor control curves, the media on wells B4-G6 of plates 1 and 2 are replaced with media containing VEGF or bFGF at the varying concentrations (50 - 0 ng/mL). The cells are incubated at 37 °C for an additional 72 h.

At the completion of the 72 h incubation period, the medium is removed, and the cells are washed twice with PBS. After the second wash with PBS, the plates are tapped gently to remove excess PBS, and the cells are placed at -70 °C for at least 30 min. The cells are thawed and analyzed using the CyQuant fluorescent dye (Molecular Probes C-7026), following the manufacturer's recommendations. The plates are read on a Victor/Wallac 1420 workstation at 485 nm/530 nm (excitation/emission). Raw data is collected and analyzed using a 4-parameter fit equation in XLFit. IC₅₀ values are then determined.

Many of the compounds of examples 1-562 were found to have an IC₅₀ of less than 25 µM in the VEGF Huvec assay.

The following assays were used to characterize the ability of compounds of Formula II to inhibit the production of TNF-α and IL-1-β. The second assay measured the inhibition of TNF-α and/or IL-1-β in mice after oral administration of the test compounds.

### Lipopolysaccharide-activated monocyte TNF production assay

### Isolation of monocytes

Test compounds were evaluated *in vitro* for the ability to inhibit the production of TNF by monocytes activated with bacterial lipopolysaccharide (LPS). Fresh residual source leukocytes (a byproduct of plateletpheresis) were obtained from a local blood bank, and peripheral blood mononuclear cells (PBMCs) were isolated by density gradient centrifugation on Ficol-Paque Plus (Pharmacia). PBMCs were suspended at 2 x 10⁶/ml in DMEM supplemented to contain 2% FCS, 10 mM, 0.3 mg/ml glutamate, 100 U/ml penicillin G and 100 mg/ml streptomycin sulfate (complete media). Cells were plated into Falcon flat bottom, 96 well culture plates (200 µl/well) and cultured overnight at 37°C and 6% CO₂. Non-adherent cells were removed by washing with 200 µl/well of fresh medium. Wells containing adherent cells (-70% monocytes) were replenished with 100 µl of fresh medium.

### Preparation of test compound stock solutions

Test compounds were dissolved in DMZ. Compound stock solutions were prepared to an initial concentration of 10 - 50 µM. Stocks were diluted initially to 20 - 200 µM in complete media. Nine two-fold serial dilutions of each compound were then prepared in complete medium.

### Treatment of cells with test compounds and activation of TNF production with lipopolysaccharide

One hundred microliters of each test compound dilution were added to microliter wells containing adherent monocytes and 100 µl complete medium. Monocytes were cultured with test compounds for 60 min at which time 25 µl of complete medium containing 30 ng/ml lipopolysaccharide from *E. coli* K532 were added to each well. Cells were cultured an additional 4 hrs. Culture supernatants were then removed and TNF presence in the supernatants was quantified using an ELISA.

### TNF ELISA

Flat bottom, 96 well Corning High Binding ELISA plates were coated overnight (4°C) with 150 µL/well of 3 µg/ml murine anti-human TNF-α MAb (R&D Systems #MAB210). Wells were then blocked for 1 hr at room temperature with 200 µL/well of CaCl₂-free ELISA buffer supplemented to contain 20 mg/ml BSA (standard ELISA buffer: 20 mM, 150 mM NaCl, 2 mM CaCl₂, 0.15 mM thimerosal, pH 7.4). Plates were washed and replenished with 100 µl of test supernatants (diluted 1:3) or standards. Standards consisted of eleven 1.5-fold serial dilutions from a stock of 1 ng/ml recombinant human TNF (R&D Systems). Plates were incubated at room temperature for 1 hr on orbital shaker (300 rpm), washed and replenished with 100 µl/well of 0.5 µg/ml goat anti-human TNF-α (R&D systems #AB-210-NA) biotinylated at a 4:1 ratio. Plates were incubated for 40 min, washed and replenished with 100 µl/well of alkaline phosphatase-conjugated streptavidin (Jackson ImmunoResearch #016-050-084) at 0.02 µg/ml. Plates were incubated 30 min, washed and replenished with 200 µl/well of 1 mg/ml of p-nitrophenyl phosphate. After 30 min, plates were read at 405 nm on a Vₘₐₓ plate reader.

### Data analysis

Standard curve data were fit to a second order polynomial and unknown TNF-α concentrations determined from their OD by solving this equation for concentration. TNF concentrations were then plotted vs. test compound concentration using a second order polynomial. This equation was then used to calculate the concentration of test compounds causing a 50% reduction in TNF production.

### Inhibition of LPS-Induced TNF-α production in mice

Male DBA/1LACJ mice were dosed with vehicle or test compounds in a vehicle (the vehicle consisting of 0.5% tragacanth in 0.03 N HCl) 30 minutes prior to lipopolysaccharide (2 mg/kg, I.V.) injection. Ninety minutes after LPS injection, blood was collected and the serum was analyzed by ELISA for TNF levels.

The following compounds exhibit activities in the monocyte assay (LPS induced TNF release) with IC₅₀ values of 25 µM or less: Examples 1-9, 11, 12, 16, 18-20, 23-41, 51-59, 61-65, 69-71, 74-81, 83, 84, 109, 110, 114, 116-126, 128, 136-148, 155, 156, 158-160, 164, 167, 183-193, 196-202, 214-225, 227-228, 231-235, 244, 270, 280-281, 283-284, 303-304, 319, 323, 339-346, 352, 353, 355-363, 365, 369, 419-434, 481-491, 520, 524, 534 and 535.

### INDICATIONS

Accordingly, compounds of the invention are useful or the prevention or treatment of inflammation, cancer and related diseases. The compounds of the invention have kinase modulatory activity in general, and kinase inhibitory activity in particular. In one embodiment of the invention, there is disclosed a method of modulating a protein kinase enzyme in a subject, the method comprising administering to the subject an effective dosage amount of a compound of a compound of Formulae I and II. In another embodiment, the kinase enzyme is ab1, Akt, bcr-ab1, Blk, Brk, Btk, c-kit, c-Met, c-src, c-fms, CDK1, CDK2, CDK3, CDK4, CDK5, CDK6, CDK7, CDK8, CDK9, CDK10, cRaf1, CSF1R, CSK, EGFR, ErbB2, ErbB3, ErbB4, Erk, Fak, fes, FGFR1, FGFR2, FGFR3, FGFR4, FGFR5, Fgr, flt-1, Fps, Frk, Fyn, Hck, IGF-1R, INS-R, Jak, KDR, Lck, Lyn, MEK, p38, PDGFR, PIK, PKC, PYK2, ros, tie, tie2, TRK, Yes or Zap70.

Various of the compounds of the invention have selective inhibitory activity for specific kinase receptor enzymes, including Tie-2, Lck, p38 and VEGFR/KDR. Accordingly, the compounds of the invention would be useful in therapy as antineoplasia agents, anti-inflammatory agents or to minimize deleterious effects of Tie-2, Lck, VEGF and/or p38.

Compounds of the invention would be useful for the treatment of neoplasia including cancer and metastasis, including : carcinoma such as cancer of the bladder, breast, colon, kidney, liver, lung (including small cell lung cancer), esophagus, gall-bladder, ovary, pancreas, stomach, cervix, thyroid, prostate, and skin (including squamous cell carcinoma); hematopoietic tumors of lymphoid linkage (including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias, myelodysplastic syndrome and promyelocytic leukemia); tumors of mesenchymal origin (including fibrosarcoma and rhabdomyosarcoma, and other sarcomas, e.g. soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma). The compounds are useful for the treatment of neoplasia selected from lung cancer, colon cancer and breast cancer.

The compounds would also be useful for treatment of ophthalmological conditions such as corneal graft rejection, ocular neovascularization, retinal neovascularization including neovascularization following injury or infection, diabetic retinopathy, retrolental fibroplasia and neovascular glaucoma; retinal ischemia; vitreous hemorrhage; ulcerative diseases such as gastric ulcer; pathological, but non-malignant, conditions such as hemangiomas, including infantile hemaginomas, angiofibroma of the nasopharynx and avascular necrosis of bone; and disorders of the female reproductive system such as endometriosis. The compounds are also useful for the treatment of edema, and conditions of vascular hyperpermeability.

Based on the ability to modulate kinases impacting angiogenesis, the compounds of the invention are also useful in treatment and therapy of proliferative diseases. Particularly, these compounds can be used for the treatment of an inflammatory rheumatoid or rheumatic disease, especially of manifestations at the locomotor apparatus, such as various inflammatory rheumatoid diseases, especially chronic polyarthritis including rheumatoid arthritis, juvenile arthritis or psoriasis arthropathy; paraneoplastic syndrome or tumor-induced inflammatory diseases, turbid effusions, collagenosis, such as systemic Lupus erythematosus, poly-myositis, dermato-myositis, systemic sclerodermia or mixed collagenosis; postinfectious arthritis (where no living pathogenic organism can be found at or in the affected part of the body), seronegative spondylarthritis, such as spondylitis ankylosans; vasculitis, sarcoidosis, or arthrosis; or further any combinations thereof. An example of an inflammation related disorder is (a) synovial inflammation, for example, synovitis, including any of the particular forms of synovitis, in particular bursal synovitis and purulent synovitis, as far as it is not crystal-induced. Such synovial inflammation may for example, be consequential to or associated with disease, e.g. arthritis, e.g. osteoarthritis, rheumatoid arthritis or arthritis deformans. The present invention is further applicable to the systemic treatment of inflammation, e.g. inflammatory diseases or conditions, of the joints or locomotor apparatus in the region of the tendon insertions and tendon sheaths. Such inflammation may be, for example, consequential to or associated with disease or further (in a broader sense of the invention) with surgical intervention, including, in particular conditions such as insertion endopathy, myofasciale syndrome and tendomyosis. The present invention is further applicable to the treatment of inflammation, e.g. inflammatory disease or condition, of connective tissues including dermatomyositis and myositis.

The compounds of the invention can also be used as active agents against such disease states as arthritis, atherosclerosis, psoriasis, hemangiomas, myocardial angiogenesis, coronary and cerebral collaterals, ischemic limb angiogenesis, wound healing, peptic ulcer Helicobacter related diseases, fractures, cat scratch fever, rubeosis, neovascular glaucoma and retinopathies such as those associated with diabetic retinopathy or macular degeneration. In addition, some of these compounds can be used as active agents against solid tumors, malignant ascites, hematopoietic cancers and hyperproliferative disorders such as thyroid hyperplasia (especially Grave's disease), and cysts (such as hypervascularity of ovarian stroma, characteristic of polycystic ovarian syndrome (Stein- Leventhal syndrome)) since such diseases require a proliferation of blood vessel cells for growth and/or metastasis.

The compounds of the invention can also be used as active agents against burns, chronic lung disease, stroke, polyps, anaphylaxis, chronic and allergic inflammation, ovarian hyperstimulation syndrome, brain tumor-associated cerebral edema, high-altitude, trauma or hypoxia induced cerebral or pulmonary edema, ocular and macular edema, ascites, and other diseases where vascular hyperpermeability, effusions, exudates, protein extravasation, or edema is a manifestation of the disease. The compounds will also be useful in treating disorders in which protein extravasation leads to the deposition of fibrin and extracellular matrix, promoting stromal proliferation (e.g. fibrosis, cirrhosis and carpal tunnel syndrome).

The compounds of the invention are also useful in the treatment of ulcers including bacterial, fungal, Mooren ulcers and ulcerative colitis.

The compounds of the invention are also useful in the treatment of conditions wherein undesired angiogenesis, edema, or stromal deposition occurs in viral infections such as Herpes simplex, Herpes Zoster, AIDS, Kaposi's sarcoma, protozoan infections and toxoplasmosis, following trauma, radiation, stroke, endometriosis, ovarian hyperstimulation syndrome, systemic lupus, sarcoidosis, synovitis, Crohn's disease, sickle cell anemia, Lyme disease, pemphigoid, Paget's disease, hyperviscosity syndrome, Osler-Weber-Rendu disease, chronic inflammation, chronic occlusive pulmonary disease, asthma, and inflammatory rheumatoid or rheumatic disease. The compounds are also useful in the reduction of sub-cutaneous fat and for the treatment of obesity. The compounds of the invention are also useful in the treatment of ocular conditions such as ocular and macular edema, ocular neovascular disease, scleritis, radial keratotomy, uveitis, vitritis, myopia, optic pits, chronic retinal detachment, post-laser complications, glaucoma, conjunctivitis, Stargardt's disease and Eales disease in addition to retinopathy and macular degeneration. The compounds of the invention are also useful in the treatment of cardiovascular conditions such as atherosclerosis, restenosis, arteriosclerosis, vascular occlusion and carotid obstructive disease.

The compounds of the invention are also useful in the treatment of cancer related indications such as solid tumors, sarcomas (especially Ewing's sarcoma and osteosarcoma), retinoblastoma, rhabdomyosarcomas, neuroblastoma, hematopoietic malignancies, including leukemia and lymphoma, tumor- induced pleural or pericardial effusions, and malignant ascites.

The compounds of the invention are also useful in the treatment of diabetic conditions such as diabetic retinopathy and microangiopathy.

The compounds of the present invention are also capable of inhibiting other protein kinases, including for example, Src, fgf, c-Met, ron, ckit and ret, and thus may be effective in the treatment of diseases associated with other protein kinases. More specifically, the compounds of the present invention inhibit the Src-family of protein tyrosine kinases such as Lck, Fyn(B), Fyn(T), Lyn, Src, Yes, Hck, Fgr and Blk, and are thus useful in the treatment, including prevention and therapy, of protein tyrosine kinase-associated disorders such as immunologic disorders. "Protein tyrosine kinase-associated disorders" are those disorders which result from aberrant tyrosine kinase activity, and/or which are alleviated by the inhibition of one or more of these enzymes. For example, Lck inhibitors are of value in the treatment of a number of such disorders (for example, the treatment of autoimmune diseases), as Lck inhibition blocks T cell activation. The treatment of T cell mediated diseases, including inhibition of T cell activation and proliferation, is a preferred embodiment of the present invention. Compounds of the present invention which selectively block T cell activation and proliferation are preferred. Also, compounds of the present invention which may block the activation of endothelial cell protein tyrosine kinase by oxidative stress, thereby limiting surface expression of adhesion molecules that induce neutrophil binding, and which can inhibit protein tyrosine kinase necessary for neutrophil activation would be useful, for example, in the treatment of ischemia and reperfusion injury.

The present invention also discloses methods for the treatment of protein tyrosine kinase-associated disorders, comprising the step of administering to a subject in need thereof at least one compound of the Formula II in an amount effective therefor. Other therapeutic agents such as those described below may be employed with the inventive compounds in the present methods. In the methods of the present invention, such other therapeutic agent(s) may be administered prior to, simultaneously with or following the administration of the compound(s) of the present invention.

Use of the compound(s) of the present invention in treating protein tyrosine kinase-associated disorders is exemplified by treating a range of disorders such as: arthritis (such as rheumatoid arthritis, psoriatic arthritis or osteoarthritis); transplant (such as organ transplant, acute transplant or heterograft or homograft (such as is employed in burn treatment)) rejection; protection from ischemic or reperfusion injury such as ischemic or reperfusion injury incurred during organ transplantation, myocardial infarction, stroke or other causes; transplantation tolerance induction; multiple sclerosis; inflammatory bowel disease, including ulcerative colitis and Crohn's disease; lupus (systemic lupus erythematosis); graft vs. host diseases; T -cell mediated hypersensitivity diseases, including contact hypersensitivity, delayed-type hypersensitivity, and gluten-sensitive enteropathy (Celiac disease); Type 1 diabetes; psoriasis; contact dermatitis (including that due to poison ivy); Hashimoto's thyroiditis; Sjogren's syndrome; Autoimmune Hyperthyroidism, such as Graves Disease; Addison's disease (autoimmune disease of the adrenal glands); Autoimmune polyglandular disease (also known as autoimmune polyglandular syndrome); autoimmune alopecia; pernicious anemia; vitiligo; autoimmune hypopituatarism; Guillain-Barre syndrome; other autoimmune diseases; cancers where Lck or other Src-family kinases such as Src are activated or overexpressed, such as colon carcinoma and thymoma, or cancers where Src-family kinase activity facilitates tumor growth or survival; glomerulonephritis, serum sickness; uticaria; allergic diseases such as respiratory allergies (asthma, hayfever, allergic rhinitis) or skin allergies; scleracielma; mycosis fungoides; acute inflammatory responses (such as acute respiratory distress syndrome and ishchemia/reperfusion injury); dermatomyositis; alopecia areata; chronic actinic dermatitis; eczema; Behcet's disease; Pustulosis palmoplanteris; Pyoderma gangrenum; Sezary's syndrome; atopic dermatitis; systemic schlerosis; and morphea. The present invention also discloses a method for treating the aforementioned disorders such as atopic dermatitis by administration of a therapeutically effective amount of a compound of the present invention, which is an inhibitor of protein tyrosine kinase, to a patient in need of such treatment.

Src-family kinases other than Lck, such as Hck and Fgr , are important in the Fcγ receptor induced respiratory burst of neutrophils as well as the Fcγ receptor responses of monocytes and macrophages. The compounds of the present invention may inhibit the Fcγ induced respiratory burst response in neutrophils, and may also inhibit the Fcγ dependent production of TNFα. The ability to inhibit Fcγ receptor dependent neutrophil, monocyte and macrophage responses would result in additionalanti-inflammatory activity for the present compounds in addition to their effects on T cells. This activity would be especially of value, for example, in the treatment of inflammatory diseases, such as arthritis or inflammatory bowel disease.

The present compounds may also be of value for the treatment of autoimmune glomerulonephritis and other instances of glomerulonephritis induced by deposition of immune complexes in the kidney that trigger Fcγ receptor responses and which can lead to kidney damage.

In addition, certain Src family kinases, such as Lyn and Fyn(B), may be important in the Fcε receptor induced degranulation of mast cells and basophils that plays an important role in asthma, allergic rhinitis, and other allergic disease. Fcε receptors are stimulated by IgE-antigen complexes. The compounds of the present invention may inhibit the Fcε induced degranulation responses. The ability to inhibit Fcε receptor dependent mast cell and basophil responses may result in additional anti-inflammatory activity for the present compounds beyond their effect on T cells.

The combined activity of the present compounds towards monocytes, macrophages, T cells, etc. may prove to be a valuable tool in the treatment of any of the aforementioned disorders.

In a particular embodiment, the compounds of the present invention are useful for the treatment of the aforementioned exemplary disorders irrespective of their etiology, for example, for the treatment of rheumatoid arthritis, transplant rejection, multiple sclerosis, inflammatory bowel disease, lupus, graft v. host disease, T cell mediated hypersensitivity disease, psoriasis, Hashimoto's thyroiditis, Guillain-Barre syndrome, cancer, contact dermatitis, allergic disease such as allergic rhinitis, asthma, ischemic or reperfusion injury, or atopic dermatitis whether or not associated with PTK.

In another embodiment, the compounds are useful for the treatment of rheumatoid spondylitis, gouty arthritis, adult respiratory distress syndrome (ARDS), anaphylaxis, muscle degeneration, cachexia, Reiter's syndrome, type II diabetes, bone resorption diseases, graft vs. host reaction, Alzheimer's disease, atherosclerosis, brain trauma, multiple sclerosis, cerebral malaria, sepsis, septic shock, toxic shock syndrome, fever, and myalgias due to infection, or which subject is infected by HIV-1, HIV-2, HIV-3, cytomegalovirus (CMV), influenza, adenovirus, the herpes viruses (including HSV-1, HSV-2), or herpes zoster in a subject, the method comprising administering to the subject a pharmaceutical composition comprising an effective dosage amount of a compound according to any of Claims 1-18.

In yet another embodiment, the compounds are useful for decreasing the level of one or more of TNF-α, IL-1β, IL-6 and IL-8 in a subject, which is typically a human.

Besides being useful for human treatment, these compounds are useful for veterinary treatment of companion animals, exotic animals and farm animals, including mammals, rodents, and the like. For example, animals including horses, dogs, and cats may be treated with compounds provided by the invention.

### FORMULATION AND METHOD OF USE

Treatment of diseases and disorders herein is intended to also include therapeutic administration of a compound of the invention, or a pharmaceutical salt thereof, or a pharmaceutical composition of either to a subject (*i.e.*, an animal, preferably a mammal, most preferably a human) which may be in need of preventative treatment, such as, for example, for pain, inflammation, cancer and the like. Treatment also encompasses prophylactic administration of a compound of the invention, or a pharmaceutical salt thereof, or a pharmaceutical composition of either to a subject (*i.e*., an animal, preferably a mammal, most preferably a human). Generally, the subject is initially diagnosed by a licensed physician and/or authorized medical practitioner, and a regimen for prophylactic and/or therapeutic treatment via administration of the compound(s) or compositions of the invention is suggested, recommended or prescribed.

While it may be possible to administer a compound of the invention alone, in the methods described, the compound administered normally will be present as an active ingredient in a pharmaceutical composition. Thus, in another embodiment of the invention, there is provided a pharmaceutical composition comprising a compound of this invention in combination with a pharmaceutically acceptable carrier, which includes diluents, excipients, adjuvants and the like (collectively referred to herein as "carrier" materials) as described herein, and, if desired, other active ingredients. A pharmaceutical composition of the invention may comprise an effective amount of a compound of the invention or an effective dosage amount of a compound of the invention. An effective dosage amount of a compound of the invention includes an amount less than, equal to or greater than an effective amount of the compound; for example, a pharmaceutical composition in which two or more unit dosages, such as in tablets, capsules and the like, are required to administer an effective amount of the compound, or alternatively, a multi-dose pharmaceutical composition, such as powders, liquids and the like, in which an effective amount of the compound is administered by administering a portion of the composition.

The compound(s) of the present invention may be administered by any suitable route, preferably in the form of a pharmaceutical composition adapted to such a route, and in a dose effective for the treatment intended. The compounds and compositions of the present invention may, for example, be administered orally, mucosally, topically, rectally, pulmonarily such as by inhalation spray, or parentally including intravascularly, intravenously, intraperitoneally, subcutaneously, intramuscularly intrasternally and infusion techniques, in dosage unit formulations containing conventional pharmaceutically acceptable carriers, adjuvants, and vehicles.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. For example, these may contain an amount of active ingredient from about 1 to 2000 mg, and typically from about 1 to 500 mg. A suitable daily dose for a human or other mammal may vary widely depending on the condition of the patient and other factors, but, once again, can be determined using routine methods and practices.

The amount of compounds which are administered and the dosage regimen for treating a disease condition with the compounds and/or compositions of this invention depends on a variety of factors, including the age, weight, sex and medical condition of the subject, the type of disease, the severity of the disease, the route and frequency of administration, and the particular compound employed. Thus, the dosage regimen may vary widely, but can be determined routinely using standard methods. A daily dose of about 0.01 to 500 mg/kg, advantageously between about 0.01 and about 50 mg/kg, and more advantageously about 0.01 and about 30 mg/kg body weight may be appropriate. The daily dose can be administered in one to four doses per day.

For therapeutic purposes, the active compounds of this invention are ordinarily combined with one or more adjuvants or "excipients" appropriate to the indicated route of administration. If administered on a per dose basis, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, to form the final formulation. For example, the active compound(s) and excipient(s) may be tableted or encapsulated by known and accepted methods for convenient administration. Examples of suitable formulations include, without limitation, pills, tablets, soft and hard-shell gel capsules, troches, orally-dissolvable forms and delayed or controlled-release formulations thereof. Particularly, capsule or tablet formulations may contain one or more controlled-release agents, such as hydroxypropylmethyl cellulose, as a dispersion with the active compound(s).

In the case of psoriasis and other skin conditions, it may be preferable to apply a topical preparation of compounds of this invention to the affected area two to four times a day.

Formulations suitable for topical administration include liquid or semi-liquid preparations suitable for penetration through the skin (*e.g.*, liniments, lotions, ointments, creams, pastes, suspensions and the like) and drops suitable for.administration to the eye, ear, or nose. A suitable topical dose of active ingredient of a compound of the invention is 0.1 mg to 150 mg administered one to four, preferably one or two times daily. For topical administration, the active ingredient may comprise from 0.001% to 10% w/w, *e.g.*, from 1% to 2% by weight of the formulation, although it may comprise as much as 10% w/w, but preferably not more than 5% w/w, and more preferably from 0.1% to 1% of the formulation.

When formulated in an ointment, the active ingredients may be employed with either paraffinic or a water-miscible ointment base. Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound, which enhances absorption or penetration of the active ingredient through the skin or other affected areas. Examples of such dermal penetration enhancers include DMSO and related analogs.

The compounds of this invention can also be administered by transdermal device. Preferably transdermal administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base, which forms the oily dispersed phase of the cream formulations. Emulsifiers and emulsion stabilizers suitable for use in the formulation of the present invention include, for example, Tween 60, Span 80, cetostearyl alcohol, myristyl alcohol, glyceryl monostearate, sodium lauryl sulfate, glyceryl distearate alone or with a wax, or other materials well known in the art.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The active ingredients are preferably present in such formulations in a concentration of 0.5 to 20%, advantageously 0.5 to 10% and particularly about 1.5% w/w.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (ie. Captisol), cosolvent solubilization (ie. propylene glycol) or micellar solubilization (ie. Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

The active ingredient may also be administered by injection as a composition with suitable carriers including saline, dextrose, or water. The daily parenteral dosage regimen will be from about 0.1 to about 30 mg/kg of total body weight, preferably from about 0.1 to about 10 mg/kg, and more preferably from about 0.25 mg to 1 mg/kg.

For pulmonary administration, the pharmaceutical composition may be administered in the form of an aerosol or with an inhaler including dry powder aerosol.

Suppositories for rectal administration of the drug can be prepared by mixing the drug with a suitable nonirritating excipient such as cocoa butter and polyethylene glycols that are solid at ordinary temperatures but liquid at the rectal temperature and will therefore melt in the rectum and release the drug.

The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

### COMBINATIONS

While the compounds of the invention can be dosed or administered as the sole active pharmaceutical agent, they can also be used in combination with one or more compounds of the invention or in conjunction with other agents. When administered as a combination, the therapeutic agents can be formulated as separate compositions that are administered simultaneously or sequentially at different times, or the therapeutic agents can be given as a single composition.

The phrase "co-therapy" (or "combination-therapy"), in defining use of a compound of the present invention and another pharmaceutical agent, is intended to embrace administration of each agent in a sequential manner in a regimen that will provide beneficial effects of the drug combination, and is intended as well to embrace coadministration of these agents in a substantially simultaneous manner, such as in a single capsule having a fixed ratio of these active agents or in multiple, separate capsules for each agent.

Specifically, the administration of compounds of the present invention may be in conjunction with additional therapies known to those skilled in the art in the prevention or treatment of neoplasia, such as with radiation therapy or with cytostatic or cytotoxic agents.

If formulated as a fixed dose, such combination products employ the compounds of this invention within the accepted dosage ranges. Compounds of Formulae I and II may also be administered sequentially with known anticancer or cytotoxic agents when a combination formulation is inappropriate. The invention is not limited in the sequence of administration; compounds of the invention may be administered either prior to, simultaneous with or after administration of the known anticancer or cytotoxic agent.

Currently, standard treatment of primary tumors consists of surgical excision followed by either radiation or IV administered chemotherapy. The typical chemotherapy regime consists of either DNA alkylating agents, DNA intercalating agents, CDK inhibitors, or microtubule poisons. The chemotherapy doses used are just below the maximal tolerated dose and therefore dose limiting toxicities typically include, nausea, vomiting, diarrhea, hair loss, neutropenia and the like.

There are large numbers of antineoplastic agents available in commercial use, in clinical evaluation and in pre-clinical development, which would be selected for treatment of neoplasia by combination drug chemotherapy. Such antineoplastic agents fall into several major categories, namely, antibiotic-type agents, alkylating agents, antimetabolite agents, hormonal agents, immunological agents, interferon-type agents and a category of miscellaneous agents.

A first family of antineoplastic agents, which may be used in combination with compounds of the invention consists of antimetabolite-type/thymidilate synthase inhibitor antineoplastic agents. Suitable antimetabolite antineoplastic agents may be selected from but not limited to the group consisting of 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, Taiho UFT and uricytin.

A second family of antineoplastic agents, which may be used in combination with compounds of the invention consists of alkylating-type antineoplastic agents. Suitable alkylating-type antineoplastic agents may be selected from but not limited to the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, lomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromus-tine, Tanabe Seiyaku TA-077, tauromustine, temozolomide, teroxirone, tetraplatin and trimelamol.

A third family of antineoplastic agents which may be used in combination with compounds of the invention consists of antibiotic-type antineoplastic agents. Suitable antibiotic-type antineoplastic agents may be selected from but not limited to the group consisting of Taiho 4181-A, aclarubicin, actinomycin D, actinoplanone, Erbamont ADR-456, aeroplysinin derivative, Ajinomoto AN-201-II, Ajinomoto AN-3, Nippon Soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb, Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindanycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-25024 and zorubicin.

A fourth family of antineoplastic agents which may be used in combination with compounds of the invention consists of a miscellaneous family of antineoplastic agents, including tubulin interacting agents, topoisomerase II inhibitors, topoisomerase I inhibitors and hormonal agents, selected from but not limited to the group consisting of α-carotene, α-difluoromethyl-arginine, acitretin, Biotec AD-5, Kyorin AHC-52, alstonine, amonafide, amphethinile, amsacrine, Angiostat, ankinomycin, anti-neoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristol-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, docetaxel elliprabin, elliptinium acetate, Tsumura EPMTC, the epothilones, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyurea, BTG ICRF-187, ilmofosine, isoglutamine, isotretinoin, Otsuka JI-36, Ramot K-477, Otsuak K-76COONa, Kureha Chemical K-AM, MECT Corp KI-8110, American Cyanamid L-623, leukoregulin, lonidamine, Lundbeck LU-23-112, Lilly LY-186641, NCI (US) MAP, marycin, Merrel Dow MDL-27048, Medco MEDR-340, merbarone, merocyanlne derivatives, methylanilinoacridine, Molecular Genetics MGI-136, minactivin, mitonafide, mitoquidone mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, ocreotide, Ono ONO-112, oquizanocine, Akzo Org-10172, paclitaxel, pancratistatin, pazelliptine, Warner-Lambert PD-111707, Warner-Lambert PD-115934, Warner-Lambert PD-131141, Pierre Fabre PE-1001, ICRT peptide D, piroxantrone, polyhaematoporphyrin, polypreic acid, Efamol porphyrin, probimane, procarbazine, proglumide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, topotecan, Topostin, Teijin TT-82, Kyowa Hakko UCN-01, Kyowa Hakko UCN-1028, ukrain, Eastman Kodak USB-006, vinblastine sulfate, vincristine, vindesine, vinestramide, vinorelbine, vintriptol, vinzolidine, withanolides and Yamanouchi YM-534.

Alternatively, the compounds of the invention may also be used in co-therapies with other anti-neoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-N1, interferon alfa-n3, interferon alfacon-1, interferon alpha, natural, interferon beta, interferon beta-1a, interferon beta-1b, interferon gamma, natural interferon gamma-1a, interferon gamma-1b, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburicase, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, VIRULIZIN, zinostatin stimalamer, or zoledronic acid; abarelix; AE 941 (Aeterna), ambamustine, antisense oligonucleotide, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA·125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techniclone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

Alternatively, the compounds of the invention may also be used in co-therapies with other anti-neoplastic agents, such as other kinase inhibitors including p38 inhibitors and CDK inhibitors, TNF inhibitors, metallomatrix proteases inhibitors (MMP), COX-2 inhibitors including celecoxib, rofecoxib, parecoxib, valdecoxib, and etoricoxib, NSAID's, SOD mimics or α_{ν}β₃ inhibitors.

## Claims

1. A compound having a formula II 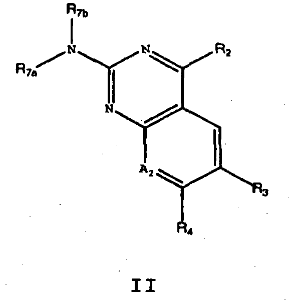 or stereoisomer, tautomer, solvate or pharmaceutically acceptable salt thereof, wherein
A² is CR⁵ or N;
R² is H, NO₂, CN, OR^{7b}, SR^{7b}, C₁₋₁₀-alkyl or C₃₋₁₀-cycloalkyl;
R³ is 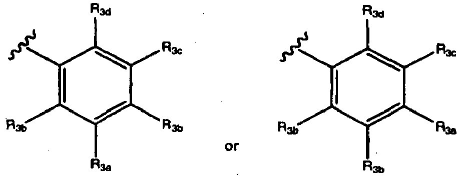 wherein
R^{3a} is C(O)R¹⁰, COOR¹⁰, C(O)R¹¹, COOR¹¹, C(O)SR¹⁰, C(O)SR¹¹, C(O)NR¹⁰R¹⁰, C(S)NR¹⁰R¹⁰, C(O)NR¹⁰R¹¹, C(S)NR¹⁰R¹¹, NR¹⁰C(O)R¹⁰, NR¹⁰C(S)R¹⁰, NR¹⁰C(O)R¹¹, NR¹⁰C(S)R¹¹, NR¹⁰C(O)NR¹⁰R¹⁰, NR¹⁰C(O)NR¹⁰R¹¹, NR¹⁰C(S)NR¹⁰R¹⁰, NR¹⁰C(S)NR¹⁰R¹¹, NR¹⁰(COOR¹⁰), NR¹⁰(COOR¹¹), S(O)₂R¹¹, S(O)₂NR¹⁰R¹⁰, S(O)₂NR¹⁰R¹¹, NR¹⁰S(O)₂NR¹⁰R¹¹, NR¹⁰S(O)₂R¹⁰ or NR¹⁰S(O)₂R¹¹;
R^{3b} is H, F, Cl, Br, I, CF₃, CF₂CF₃, OCF₃, OCF₂CF₃, CN, NO₂, NH₂, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl;
R^{3c} is H, F, Cl, Br, I, CF₃, CF₂CF₃, OCF₃, OCF₂CF₃, CN, NO₂, NH₂, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl;
R^{3d} is H, F, Cl, Br, I, CF₃, CF₂CF₃, OCF₃, OCF₂CF₃, CN, NO₂, NH₂, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl;
R⁴ is H, F, Cl, Br, I, CF₃, CF₂CF₃, OCF₃, CN, methyl, ethyl, propyl, isopropyl, n-butyl, sec-butyl, tert-butyl, OH, methoxyl, ethoxyl, propoxyl;
R⁵ is H, F, Cl, Br, I, CF₃, CF₂CF₃, OCF₃, CN, methyl, ethyl, propyl, isopropyl, n-butyl, sec-butyl, tert-butyl, OH, methoxyl, ethoxyl, propoxyl;
R⁶ is H, CN, methyl, ethyl, propyl, isopropyl or n-butyl;
R^{7a} is H, C(O)R⁸, COOR⁸, C(O)R⁹, COOR⁹, C(O)NR⁸R⁹, C(O)NR⁹R⁹, S(O)₂R⁸, S(O)₂NR⁸R⁹, S(O)₂R⁹, S(O)₂NR⁹R⁹, C₁₋₁₀-alkyl, C₂₋₁₀-alkenyl, C₂₋₁₀-alkynyl or C₃₋₁₀-cycloalkyl, each of the C₁₋₁₀-alkyl, C₂₋₁₀-alkenyl, C₂₋₁₀-alkynyl and C₃₋₁₀-cycloalkyl optionally comprising 1-4 heteroatoms selected from N, O and S and optionally substituted with one or more substituents of NR⁸R⁹, NR⁹R⁹, OR⁸, SR⁸, OR⁹, SR⁹, C(O)R⁸, C(O)R⁹, C(O)NR⁸R⁹, C(O)NR⁹R⁹, NR⁹C(O)R⁸, NR⁹C(O)R⁹, NR⁹C(O)NR⁸R⁹, NR⁹C(O)NR⁹R⁹, NR⁹(COOR⁸), NR⁹(COOR⁹), S(O)₂R⁸, S(O)₂NR⁸R⁹, S(O)₂R⁹, S(O)₂NR⁹R⁹, NR⁹S(O)₂NR⁸R⁹, NR⁹S(O)₂NR⁹R⁹, NR⁹S(O)₂R⁸, NR⁹S(O)₂R⁹, R⁸ or R⁹;
R^{7b} is H;
R⁸ is a ring system selected from phenyl, naphthyl, pyridyl, piperazinyl, triazinyl, quinolinyl, isoquinolinyl, quinazolinyl, isoquinazolinyl, thiophenyl, furyl, pyrrolyl, imidazolyl, triazolyl, thiazolyl, oxazolyl, isoxazolyl, isothiazolyl, indolyl, isoindolyl, benzofuranyl, benzothiophenyl, benzimidazolyl, tetrahydrofuranyl, pyrrolidinyl, oxazolinyl, isoxazolinyl, thiazolinyl, pyrazolinyl, morpholinyl, piperidinyl, piperazinyl, pyranyl, dioxozinyl, cyclopropyl, cyclobutyl, cyclopentyl, cyclohexyl and cycloheptyl, wherein the ring system is optionally substituted independently with 1-3 substituents of R⁹, oxo, NR⁹R⁹, OR⁹; SR⁹, C(O)R⁹, phenyl, pyridyl, piperidyl, piperazinyl or morpholinyl;
R⁹ is H, halo, haloalkyl, CN, OH, NO₂, NH₂, acetyl, C₁₋₁₀-alkyl, C₂₋₁₀-alkenyl, C₂₋₁₀-alkynyl, C₃₋₁₀-cycloalkyl, C₁₋₁₀-alkylamino-, C₁₋₁₀-dialkylamino-, C₁₋₁₀-alkoxyl, C₁₋₁₀-thioalkoxyl or a ring system selected from phenyl, naphthyl, pyridyl, piperazinyl, triazinyl, quinolinyl, isoquinolinyl, quinazolinyl, isoquinazolinyl, thiophenyl, furyl, pyrrolyl, imidazolyl, triazolyl, thiazolyl, oxazolyl, isoxazolyl, isothiazolyl, indolyl, isoindolyl, benzofuranyl, benzothiophenyl, benzimidazolyl, tetrahydrofuranyl, pyrrolidinyl, oxazolinyl, isoxazolinyl, thiazolinyl, pyrazolinyl, morpholinyl, piperidinyl, piperazinyl, pyranyl and dioxozinyl, each of the C₁₋₁₀-alkyl, C₂₋₁₀-alkenyl, C₂₋₁₀-alkynyl, C₃₋₁₀-cycloalkyl, C₁₋₁₀-alkylamino-, C₁₋₁₀-dialkylamino-, C₁₋₁₀-alkoxyl, C₁₋₁₀-thioalkoxyl and ring system optionally substituted independently with 1-3 substituents of halo, haloalkyl, CN, NO₂, NH₂, OH, oxo, methyl, methoxyl, ethyl, ethoxyl, propyl, propoxyl, isopropyl, cyclopropyl, butyl, isobutyl, tert-butyl, methylamino, dimethylamino, ethylamino, diethylamino, propylamine, isopropylamine, dipropylamine, diisopropylamine, benzyl or phenyl;
R¹⁰ is H, methyl, ethyl, propyl, isopropyl, n-butyl, sec-butyl, tert-butyl, acetylenyl, cyclopropyl, cyclobutyl, cyclopentyl, cyclohexyl, OH, methoxyl, ethoxyl, propoxyl, SH, thiomethyl or thioethyl; each of which is optionally substituted with one or more substituents of R¹¹, R¹² or R¹⁶;
R¹¹ is phenyl, naphthyl, pyridyl, quinolinyl, tetrahydroquinolinyl, oxo-tetrahydroquinolinyl, isoquinolinyl, oxo-tetrahydroisoquinolinyl, tetrahydroisoquinolinyl, thiophenyl, tetrahydrofuranyl, thieno-pyrazolyl, tetrahydropentapyrazolyl, imidazolyl, thiazolyl, thiadiazolyl, benzothiazolyl, oxazolyl, oxadiazolyl, benzoxazolyl, benzoxadiazolyl, isoxazolyl, isothiazolyl, indolyl, 2,3-dihydroindolyl, benzofuranyl, benzothiophenyl, benzimidazolyl, 2,3-dihydro-1,4-benzoxazinyl, 1,3-benzodioxolyl, cyclopropyl, cyclobutyl, cyclopentyl and cyclohexyl, said ring system optionally substituted independently with 1-3 substituents of R¹², R¹³, R¹⁴ or R¹⁶;
R¹² is H, C₁₋₁₀-alkyl, C₂₋₁₀-alkenyl, C₂₋₁₀-alkynyl, C₃₋₁₀-cycloalkyl, C₄₋₁₀-cycloalkenyl, C₁₋₁₀-alkylamino-, C₁₋₁₀-dialkylamino- or C₁₋₁₀-alkoxyl, each of which is optionally substituted independently with 1-3 substituents of R¹³, R¹⁴ R¹⁵ or R¹⁶;
R¹³ is NR¹⁴R¹⁵, NR¹⁵R¹⁵, OR¹⁴; SR¹⁴, OR¹⁵; SR¹⁵, C(O)R¹⁴, OC(O)R¹⁴, COOR¹⁴, C(O)R¹⁵, OC(O)R¹⁵, COOR¹⁵, C(O)NR¹⁴R¹⁵, C(O)NR¹⁵R¹⁵, NR¹⁴C(O)R¹⁴, NR¹⁵C(O)R¹⁴, NR¹⁴C(O)R¹⁵, NR¹⁵C(O)R¹⁵, NR¹⁵C(O)NR¹⁴R¹⁵, NR¹⁵C(O)NR¹⁵R¹⁵, NR¹⁵(COOR¹⁴), NR¹⁵(COOR¹⁵), OC(O)NR¹⁴R¹⁵, OC(O)NR¹⁵R¹⁵, S(O)₂R¹⁴, S(O)₂R¹⁵, S(O)₂NR¹⁴R¹⁵, S(O)₂NR¹⁵R¹⁵, NR¹⁴S(O)₂NR¹⁴R¹⁵, NR¹⁵S(O)₂NR¹⁵R¹⁵, NR¹⁴S(O)₂R¹⁴ or NR¹⁵S(O)₂R¹⁵;
R¹⁴ is phenyl, pyridyl, pyrimidinyl, thiophenyl, furyl, tetrahydrofuryl, pyrrolyl, pyrazolyl, thieno-pyrazolyl, imidazolyl, triazolyl, thiazolyl, thiadiazolyl, benzothiazolyl, oxazolyl, oxadiazolyl, benzoxazolyl, benzoxadiazolyl, isoxazolyl, isothiazolyl, indolyl, azaindolyl, isoindolyl, indazolyl, benzofuranyl, benzothiophenyl, benzimidazolyl, pyrrolidinyl, pyrazolinyl, morpholinyl, piperidinyl, piperazinyl, 2,3-dihydro-1,4-benzoxazinyl, 1,3-benzodioxolyl, cyclopropyl, cyclobutyl, azetidinyl, cyclopentyl and cyclohexyl, each of which is optionally substituted independently with 1-3 substituents of R¹⁵ or R¹⁶;
R¹⁵ is H or C₁₋₁₀-alkyl, C₂₋₁₀-alkenyl, C₂₋₁₀-alkynyl, C₃₋₁₀-cycloalkyl, C₄₋₁₀-cycloalkenyl, C₁₋₁₀-alkylamino-, C₁₋₁₀-dialkylamino-, C₁₋₁₀-alkoxyl or C₁₋₁₀-thioalkoxyl, each of which is optionally substituted independently with 1-3 substituents of R¹⁶; and
R¹⁶ is H, halo, haloalkyl, CN, OH, NO₂, NH₂, OH, methyl, methoxyl, ethyl, ethoxyl, propyl, propoxyl, isopropyl, cyclopropyl, butyl, isobutyl, tert-butyl, methylamino, dimethylamino, ethylamino, diethylamino, isopropylamino, oxo, acetyl, benzyl or a ring system selected from phenyl, pyridyl, thiophenyl, furyl, tetrahydrofuryl, pyrrolyl, pyrazolyl, thieno-pyrazolyl, imidazolyl, triazolyl, thiazolyl, thiadiazolyl, benzothiazolyl, oxazolyl, oxadiazolyl, benzoxazolyl, benzoxadiazolyl, isoxazolyl, isothiazolyl, indolyl, azaindolyl, isoindolyl, indazolyl, benzofuranyl, benzothiophenyl, benzimidazolyl, pyrrolidinyl, pyrazolinyl, morpholinyl, piperidinyl, piperazinyl, cyclopropyl, cyclobutyl, azetidinyl, cyclopentyl and cyclohexyl, said ring system optionally substituted independently with 1-3 substituents of halo, haloalkyl, CN, NO₂, NH₂, OH, methyl, methoxyl, ethyl, ethoxyl, propyl, propoxyl, isopropyl, cyclopropyl, butyl, isobutyl, tert-butyl, methylamino, dimethylamino, ethylamino, diethylamino, isopropylamino, benzyl or phenyl;
provided that
(1) when A² is N, then R⁴ is not halo or hydroxyl.

2. A compound and pharmaceutically acceptable salts thereof, selected from:
N-(3-(2-amino-6-quinazolinyl)-4-methylphenyl)-2-((3-(dimethylamino)propyl)(methyl)amino)-5-(trifluoromethyl)benzamide;
5-(2-amino-6-quinazolinyl)-N-(3-chloro-2-((3-(dimethylamino)propyl)(methyl)amino)-5-(trifluoromethyl)phenyl)-2-fluorobenzamide;
5-(2-amino-6-quinazolinyl)-N-(3-(1-methyl-4-piperidinyl)-5-(trifluoromethyl)phenyl)-3-thiophenecarboxamide;
3-(2-amino-6-quinazolinyl)-4-methyl-N-(2-methyl-3-((4-methyl-1-piperazinyl)methyl)phenyl)benzamide;
3-(2-amino-6-quinazolinyl)-N-(4-(1,1-dimethylethyl)-3-((N,N-dimethylglycyl)amino)phenyl)-4-methylbenzamide;
3-(((3-(2-amino-6-quinazolinyl)-4-methylphenyl)carbonyl)amino)-2,6-difluoro-N-methylbenzamide;
3-(2-amino-6-quinazolinyl)-N-(2-fluoro-4-(1-pyrrolidinyl)-3-(1-pyrrolidinylcarbonyl)phenyl)-4-methylbenzamide;
3-(2-amino-6-quinazolinyl)-4-methyl-N-(4-methyl-3-((4-morpholinylacetyl)amino)phenyl)benzamide;
3-(2-amino-6-quinazolinyl)-4-methyl-N-(2-methyl-3-((4-morpholinylacetyl)amino)phenyl)benzamide;
3-(2-amino-6-quinazolinyl)-N-(3-((N,N-diethylglycyl)amino)-2-methylphenyl)-4-methylbenzamide;
3-(2-amino-6-quinazolinyl)-N-(1-(N,N-diethylglycyl)-3,3-dimethyl-2,3-dihydro-1H-indol-6-yl)-4-methylbenzamide;
3-(2-amino-6-quinazolinyl)-N-(3,3-dimethyl-1-(4-morpholinylacetyl)-2,3-dihydro-1H-indol-6-yl)-4-methylbenzamide;
3-(2-amino-6-quinazolinyl)-4-methyl-N-(2-methyl-3-(trifluoromethyl)phenyl)benzamide;
3-(2-amino-6-quinazolinyl)-4-methyl-N-(3-((trifluoromethyl)oxy)phenyl)benzamide; phenylmethyl 3-(2-amino-6-quinazolinyl)-4-methylbenzoate;
3-(2-amino-6-quinazolinyl)-N-(6-chloro-3-pyridinyl)-4-methylbenzamide;
3-(2-amino-6-quinazolinyl)-N-(4-(cyclopropylethynyl)-3-(trifluoromethyl)phenyl)-4-methylbenzamide;
3-(2-amino-6-quinazolinyl)-N-(4-bromo-3-(trifluoromethyl)phenyl)-4-methylbenzamide;
3-(2-amino-6-quinazolinyl)-4-methyl-N-(4-(phenylethynyl)-3-(trifluoromethyl)phenyl)benzamide;
3-(2-amino-6-quinazolinyl)-4-chloro-N-(3-(trifluoromethyl)phenyl)benzamide;
3-(2-amino-6-quinazolinyl)-4-methyl-N-(2-((2,2,2-trifluoroethyl)oxy)-5-(trifluoromethyl)phenyl)benzamide;
3-(2-amino-6-quinazolinyl)-4-methyl-N-(4-((3-(1-piperidinyl)propyl)oxy)phenyl)benzamide;
3-(2-amino-6-quinazolinyl)-4-methyl-N-(3-((3-(1-piperidinyl)propyl)oxy)phenyl)benzamide;
3-(2-amino-6-quinazolinyl)-N-(4-((3-(dimethylamino)propyl)oxy)phenyl)-4-methylbenzamide;
3-(2-amino-6-quinazolinyl)-N-(4-((2-(diethylamino)ethyl)oxy)-2-(methyloxy)phenyl)-4-methylbenzamide;
3-(2-amino-6-quinazolinyl)-N-(5-((2-(diethylamino)ethyl)oxy)-2-(methyloxy)phenyl)-4-methylbenzamide;
3-(2-amino-6-quinazolinyl)-N-(2,4-bis(methyloxy)-5-(trifluoromethyl)phenyl)-4-methylbenzamide;
3-(2-amino-6-quinazolinyl)-N-(5-((diethylamino)sulfonyl)-2-(methyloxy)phenyl)-4-methylbenzamide;
3-(2-amino-6-quinazolinyl)-N-(2,3-dichlorophenyl)-4-methylbenzamide;
3-(2-amino-6-quinazolinyl)-N-(2,6-bis(methyloxy)-3-pyridinyl)-4-methylbenzamide;
N-(2-(acetylamino)-5-(trifluoromethyl)phenyl)-3-(2-amino-6-quinazolinyl)-4-methylbenzamide;
3-(2-amino-6-quinazolinyl)-4-methyl-N-(2-(methyloxy)-5-((3-(4-methyl-1-piperazinyl)propyl)oxy)phenyl)benzamide;
3-(2-amino-6-quinazolinyl)-N-(2,2-difluoro-1,3-benzodioxol-4-yl)-4-methylbenzamide;
3-(2-amino-6-quinazolinyl)-4-methyl-N-(2,2,3,3-tetrafluoro-2,3-dihydro-1,4-benzodioxin-5-yl)benzamide;
3-(7-isoquinolinyl)-N-(3-(trifluoromethyl)phenyl)benzamide;
3-(1-hydroxy-7-isoquinolinyl)-N-(3-(trifluoromethyl)phenyl)benzamide;
3-(2-amino-6-quinazolinyl)-N-(3-(trifluoromethyl)phenyl)benzamide;
3-(7-isoquinolinyl)-N-(2-(1-piperidinyl)-5-(trifluoromethyl)phenyl)benzamide;
3-(2-amino-6-quinazolinyl)-N-(2-(1-piperidinyl)-5-(trifluoromethyl)phenyl)benzamide;
3-(2-(methylamino)-6-quinazolinyl)-N-(3-(trifluoromethyl)phenyl)benzamide;
4-(2-amino-6-quinazolinyl)-N-(3-(trifluoromethyl)phenyl)benzamide;
3-(2-amino-6-quinazolinyl)-4-methyl-N-(2-(4-methyl-1-piperazinyl)-5-(trifluoromethyl)phenyl)benzamide;
3-(2-amino-6-quinazolinyl)-N-(2-((3R)-3-(dimethylamino)-1-pyrrolidinyl)-5-(trifluoromethyl)phenyl)-4-methylbenzamide;
3-(2-amino-6-quinazolinyl)-4-methyl-N-(2-(1-piperidinyl)-5-(trifluoromethyl)phenyl)benzamide;
3-(2-amino-6-quinazolinyl)-N-(2,5-bis(trifluoromethyl)phenyl)-4-methylbenzamide;
3-(2-amino-6-quinazolinyl)-N-(2,5-bis(1,1-dimethylethyl)phenyl)-4-methylbenzamide;
3-(2-amino-6-quinazolinyl)-4-methyl-N-(2-(4-morpholinyl)-5-(trifluoromethyl)phenyl)benzamide;
3-(2-amino-6-quinazolinyl)-4-methyl-N-(2-(1-pyrrolidinyl)-5-(trifluoromethyl)phenyl)benzamide;
3-(2-amino-6-quinazolinyl)-N-(2-(dimethylamino)-5-(trifluoromethyl)phenyl)-4-methylbenzamide;
N-(3-(2-amino-6-quinazolinyl)-5-(trifluoromethyl)phenyl)-3-(trifluoromethyl)benzamide;
N-(3-(2-amino-6-quinazolinyl)-5-(trifluoromethyl)phenyl)-3-(1-methylethyl)benzamide;
3-(2-amino-6-quinazolinyl)-4-methyl-N-(2-((3R)-1-methyl-3-piperidinyl)-5-(trifluoromethyl)phenyl)benzamide;
3-(2-amino-6-quinazolinyl)-N-(3-(1-methylethyl)phenyl)benzamide;
3-(2-amino-6-quinazolinyl)-N-(2-(2-(dimethylamino)-1,1-dimethylethyl)-5-(trifluoromethyl)phenyl)benzamide;
3-(2-amino-6-quinazolinyl)-N-(2-(2-(dimethylamino)-1,1-dimethylethyl)-5-(trifluoromethyl)phenyl)-4-methylbenzamide;
3-(2-amino-6-quinazolinyl)-4-methyl-N-(2-(methyl((3S)-1-methyl-3-pyrrolidinyl)amino)-5-(trifluoromethyl)phenyl)benzamide;
N-(2-(2-(dimethylamino)-1,1-dimethylethyl)-5-(trifluoromethyl)phenyl)-3-(2-(methylamino)-6-quinazolinyl)benzamide;
3-(2-amino-6-quinazolinyl)-N-(2-(methyl((3R)-1-methyl-3-pyrrolidinyl)amino)-5-(trifluoromethyl)phenyl)benzamide;
3-(2-amino-6-quinazolinyl)-N-(2-(4-methyl-1-piperazinyl)-5-(trifluoromethyl)phenyl)benzamide;
3-(2-amino-6-quinazolinyl)-N-(2-((3-(dimethylamino)propyl)(methyl)amino)-5-(trifluoromethyl)phenyl)benzamide;
3-(2-(methylamino)-6-quinazolinyl)-N-(2-((4-methyl-1-piperazinyl)methyl)-5-(trifluoromethyl)phenyl)benzamide;
3-(2-amino-6-quinazolinyl)-4-methyl-N-(2-((4-methyl-1-piperazinyl)methyl)-5-(trifluoromethyl)phenyl)benzamide;
3-(2-amino-6-quinazolinyl)-N-(2-((3-(dimethylamino)propyl)(methyl)amino)-5-(1,1-dimethylethyl)phenyl)-4-methylbenzamide;
3-(2,4-diaminopyrido[2,3-d]pyrimidin-6-yl)-4-methyl-N-(3-(trifluoromethyl)phenyl)benzamide;
5-(2-amino-6-quinazolinyl)-2-fluoro-N-(2-(1-piperidinyl)-5-(trifluoromethyl)phenyl)benzamide;
5-(2-amino-6-quinazolinyl)-N-(2-((3-(dimethylamino)propyl)(methyl)amino)-5-(trifluoromethyl)phenyl)-2-fluorobenzamide;
3-(2-amino-6-quinazolinyl)-N-(2-(2-(dimethylamino)ethyl)-5-(trifluoromethyl)phenyl)-4-methylbenzamide;
5-(2-amino-6-quinazolinyl)-2-fluoro-N-(2-(methyl((3R)-1-methyl-3-pyrrolidinyl)amino)-5-(trifluoromethyl)phenyl)benzamide;
3-(2-amino-6-quinazolinyl)-4-methyl-N-(2-((2-(1-pyrrolidinyl)ethyl)oxy)-5-(trifluoromethyl)phenyl)benzamide;
3-(2-amino-6-quinazolinyl)-N-(2-((3-(dimethylamino)propyl)(methyl)amino)-5-(1-methylethyl)phenyl)benzamide;
5-(2-amino-6-quinazolinyl)-2-fluoro-N-(2-(4-methyl-1-piperazinyl)-5-(trifluoromethyl)phenyl)benzamide;
3-(2-amino-6-quinazolinyl)-N-(2-((3S)-3-(dimethylamino)-1-piperidinyl)-5-(trifluoromethyl)phenyl)-4-methylbenzamide;
5-(2-amino-6-quinazolinyl)-N-(2-((3R)-3-(dimethylamino)-1-pyrrolidinyl)-5-(trifluoromethyl)phenyl)-2-fluorobenzamide;
3-(2-amino-6-quinazolinyl)-N-(2-(4-(dimethylamino)-1-piperidinyl)-5-(trifluoromethyl)phenyl)-4-methylbenzamide;
3-(2-amino-6-quinazolinyl)-4-methyl-N-(4-((trifluoromethyl)oxy)phenyl)benzamide;
3-(2-amino-6-quinazolinyl)-N-(2-((3-(dimethylamino)propyl)(methyl)amino)-5-(1,1-dimethylethyl)phenyl)benzamide;
5-(2-amino-6-quinazolinyl)-N-(2-((3-(dimethylamino)propyl)(methyl)amino)-5-(1,1-dimethylethyl)phenyl)-2-fluorobenzamide;
5-(2-amino-6-quinazolinyl)-N-(2-((3-(dimethylamino)propyl)(methyl)amino)-5-ethynylphenyl)-2-fluorobenzamide;
5-(2-amino-6-quinazolinyl)-N-(2-((2-(dimethylamino)ethyl)(methyl)amino)-5-(trifluoromethyl)phenyl)-2-fluorobenzamide;
5-(2-amino-6-quinazolinyl)-N-(2-(dimethylamino)-5-(trifluoromethyl)phenyl)-2-fluorobenzamide;
N-(2-((3-(dimethylamino)propyl)(methyl)amino)-5-(trifluoromethyl)phenyl)-2-fluoro-5-(2-(methylamino)-6-quinazolinyl)benzamide;
5-(2-amino-6-quinazolinyl)-N-(2-((3-(dimethylamino)propyl)(methyl)amino)-5-(pentafluoroethyl)phenyl)-2-fluorobenzamide;
6-(2,6-dimethylphenyl)-2-quinazolinamine;
3-(2-amino-6-quinazolinyl)-N-(2-(dimethylamino)-5-(trifluoromethyl)phenyl)benzamide;
3-(2-amino-6-quinazolinyl)-N-(3-(1,1-dimethylethyl)phenyl)benzamide;
6-(2,6-bis(methyloxy)phenyl)-2-quinazolinamine;
N-(3-(1,1-dimethylethyl)phenyl)-3-(2-(methylamino)-6-quinazolinyl)benzamide;
3-(2-amino-6-quinazolinyl)-N-(3,5-bis(methyloxy)phenyl)benzamide;
3-(2-amino-6-quinazolinyl)-N-(5-(1,1-dimethylethyl)-3-isoxazolyl)benzamide;
3-(2-amino-6-quinazolinyl)-N-(3-((1,1,2,2-tetrafluoroethyl)oxy)phenyl)benzamide;
3-(2-amino-6-quinazolinyl)-N-(5-(1,1-dimethylethyl)-1H-pyrazol-3-yl)benzamide;
3-(2-amino-6-quinazolinyl)-N-(3-chlorophenyl)benzamide;
5-(2-amino-6-quinazolinyl)-N-(5-bromo-2-((3-(dimethylamino)propyl)(methyl)amino)phenyl)-2-fluorobenzamide;
5-(2-amino-6-quinazolinyl)-N-(2-((3-(dimethylamino)propyl)oxy)-5-(trifluoromethyl)phenyl)-2-fluorobenzamide;
5-(2-amino-6-quinazolinyl)-N-(3-(trifluoromethyl)phenyl)-2-thiophenecarboxamide;
3-(2-amino-6-quinazolinyl)-4-methyl-N-(3-(1-methyl-4-piperidinyl)-5-(trifluoromethyl)phenyl)benzamide;
5-(2-amino-6-quinazolinyl)-N-(2-((3-(dimethylamino)propyl)(methyl)amino)-5-(trifluoromethyl)phenyl)-2-thiophenecarboxamide;
5-(2-amino-6-quinazolinyl)-N-(3-(trifluoromethyl)phenyl)-3-thiophenecarboxamide;
5-(2-amino-6-quinazolinyl)-N-(2-(2-(dimethylamino)ethyl)-5-(trifluoromethyl)phenyl)-2-fluorobenzamide;
N-(4-(2-amino-6-quinazolinyl)-3-methylphenyl)-N'-(3-fluoro-5-(trifluoromethyl)phenyl)urea;
5-(2-amino-6-quinazolinyl)-N-(5-chloro-2-((3-(dimethylamino)propyl)(methyl)amino)phenyl)-2-fluorobenzamide;
4-methyl-3-(2-(4-methyl-1-piperazinyl)-6-quinazolinyl)-N-(3-(trifluoromethyl)phenyl)benzamide;
N-(4-(2-amino-6-quinazolinyl)-3-methylphenyl)-N'-(5-chloro-2-(methyloxy)phenyl)urea;
4-(2-amino-6-quinazolinyl)-N-(3-(trifluoromethyl)phenyl)-2-pyridinecarboxamide;
3-(2-amino-6-quinazolinyl)-N-(2-(((3-(dimethylamino)propyl)(methyl)amino)sulfonyl)-5-(trifluoromethyl)phenyl)-4-methylbenzamide;
4-(2-amino-6-quinazolinyl)-N-(2-((3-(dimethylamino)propyl)(methyl)amino)-5-(trifluoromethyl)phenyl)-2-pyridinecarboxamide;
4-(2-amino-6-quinazolinyl)-N-(2-((3-(dimethylamino)propyl)(methyl)amino)-5-(trifluoromethyl)phenyl)-2-thiophenecarboxamide;
5-(2-amino-6-quinazolinyl)-N-(2-((3-(dimethylamino)propyl)(methyl)amino)-5-(trifluoromethyl)phenyl)-3-thiophenecarboxamide;
5-(2-amino-6-quinazolinyl)-N-(2-((3-(dimethylamino)propyl)(methyl)amino)-5-(trifluoromethyl)phenyl)-2-(methyloxy)benzamide;
5-(2-amino-6-quinazolinyl)-2-chloro-N-(2-((3-(dimethylamino)propyl)(methyl)amino)-5-(trifluoromethyl)phenyl)benzamide;
3-(2-amino-6-quinazolinyl)-5-bromo-N-(2-((3-(dimethylamino)propyl)(methyl)amino)-5-(trifluoromethyl)phenyl)benzamide;
3-(2-amino-6-quinazolinyl)-N-(5-chloro-2-((3-(dimethylamino)propyl)(methyl)amino)phenyl)-4-methylbenzamide;
5-(2-amino-6-quinazolinyl)-N-(2-((3-(dimethylamino)propyl)(methyl)amino)-5-(trifluoromethyl)phenyl)-2,3-dihydro-1-benzofuran-7-carboxamide;
5-(2-amino-6-quinazolinyl)-2-bromo-N-(2-((3-(dimethylamino)propyl)(methyl)amino)-5-(trifluoromethyl)phenyl)benzamide;
3-(2-amino-6-quinazolinyl)-N-(3-((trifluoromethyl)oxy)phenyl)benzamide;
5-(2-amino-6-quinazolinyl)-2,3-bis(methyloxy)-N-(3-(trifluoromethyl)phenyl)benzamide;
5-(2-amino-6-quinazolinyl)-2-(methyloxy)-N-(3-(trifluoromethyl)phenyl)benzamide;
5-(2-amino-6-quinazolinyl)-N-(5-cyclopropyl-2-((3-(dimethylamino)propyl)(methyl)amino)phenyl)-2-fluorobenzamide;
5-(2-amino-6-quinazolinyl)-N-(2-((3-(dimethylamino)propyl)(methyl)amino)-5-(trifluoromethyl)phenyl)-2,3-bis(methyloxy)benzamide;
5-(2-amino-6-quinazolinyl)-N-(3-(trifluoromethyl)phenyl)-2,3-dihydro-1-benzofuran-7-carboxamide;
5-(2-amino-6-quinazolinyl)-3-bromo-N-(2-((3-(dimethylamino)propyl)(methyl)amino)-5-(trifluoromethyl)phenyl)-2-(methyloxy)benzamide;
3-(2-amino-6-quinazolinyl)-5-bromo-N-(2-((3-(dimethylamino)propyl)(methyl)amino)-5-(trifluoromethyl)phenyl)-2-(methyloxy)benzamide;
5-(2-amino-6-quinazolinyl)-N-(2-((3-(dimethylamino)propyl)(methyl)amino)-5-(trifluoromethyl)phenyl)-1-methyl-2-oxo-1,2-dihydro-3-pyridinecarboxamide;
5-(2-amino-6-quinazolinyl)-2-fluoro-N-(3-((4-methyl-1-piperazinyl)methyl)-5-(trifluoromethyl)phenyl)benzamide;
5-(2-amino-6-quinazolinyl)-2,3-bis(methyloxy)-N-(3-((4-methyl-1-piperazinyl)methyl)-5-(trifluoromethyl)phenyl)benzamide;
1-(4-(2-amino-6-quinazolinyl)-3-methylphenyl)-3-(3-(trifluoromethyl)phenyl)-2-imidazolidinone;
5-(2-amino-6-quinazolinyl)-2-(methyloxy)-N-(3-((4-methyl-1-piperazinyl)methyl)-5-(trifluoromethyl)phenyl)benzamide;
N-(4-(2-amino-6-quinazolinyl)phenyl)-N'-(3-fluoro-5-(trifluoromethyl)phenyl)urea;
N-(4-(2-amino-6-quinazolinyl)phenyl)-N'-(3-fluorophenyl)urea;
5-(2-amino-6-quinazolinyl)-2-((2-(dimethylamino)ethyl)oxy)-N-(3-(trifluoromethyl)phenyl)benzamide;
5-(2-amino-6-quinazolinyl)-2-(methyloxy)-N-(3-((trifluoromethyl)oxy)phenyl)benzamide;
5-(2-amino-6-quinazolinyl)-2,3,4-tris(methyloxy)-N-(3-(trifluoromethyl)phenyl)benzamide;
5-(2-amino-6-quinazolinyl)-N-(2-((3-(dimethylamino)propyl)(methyl)amino)-5-(trifluoromethyl)phenyl)-2,3,4-tris(methyloxy)benzamide;
5-(2-amino-6-quinazolinyl)-N-(2-((3-(dimethylamino)propyl)(methyl)amino)-5-(trifluoromethyl)phenyl)-2-(ethyloxy)benzamide;
3-(2-amino-6-quinazolinyl)-2,6-bis(methyloxy)-N-(3-(trifluoromethyl)phenyl)benzamide;
5-(2-amino-6-quinazolinyl)-2-(dimethylamino)-N-(2-((3-(dimethylamino)propyl)(methyl)amino)-5-(trifluoromethyl)phenyl)benzamide;
N-(4-(2-amino-6-quinazolinyl)-3-methylphenyl)-3-(trifluoromethyl)benzamide;
N-(4-(2-amino-6-quinazolinyl)phenyl)-3-(trifluoromethyl)benzamide;
N-(4-(4-amino-6-quinazolinyl)phenyl)-N'-(3-fluorophenyl)urea;
3-(2-amino-6-quinazolinyl)-N-(2-((3-(dimethylamino)propyl)(methyl)amino)-5-(trifluoromethyl)phenyl)-2,6-bis(methyloxy)benzamide;
5-(2-amino-6-quinazolinyl)-2-fluoro-N-(3-(trifluoromethyl)phenyl)benzamide;
N-(3-(2-amino-6-quinazolinyl)-4-methylphenyl)-3-(1-methylethyl)benzamide;
N-(5-(2-amino-6-quinazolinyl)-2-fluorophenyl)benzamide;
N-(5-(2-amino-6-quinazolinyl)-2-fluorophenyl)cyclopropanecarboxamide;
N-(5-(2-amino-6-quinazolinyl)-2-fluorophenyl)-2,3-dichlorobenzamide;
N-(5-(2-amino-6-quinazolinyl)-2-fluorophenyl)-4-(1,1-dimethylethyl)benzamide;
N-(5-(2-amino-6-quinazolinyl)-2-fluorophenyl)-3-fluoro-5-(trifluoromethyl)benzamide;
N-(5-(2-amino-6-quinazolinyl)-2-fluorophenyl)-3,5-bis(methyloxy)benzamide;
N-(5-(2-amino-6-quinazolinyl)-2-fluorophenyl)-3-(1-methylethyl)benzamide;
N-(5-(2-amino-6-quinazolinyl)-2-fluorophenyl)-2-fluoro-5-(trifluoromethyl)benzamide;
N-(5-(2-amino-6-quinazolinyl)-2-fluorophenyl)-2-(4-methyl-1-piperazinyl)-5-(trifluoromethyl)benzamide;
N-(5-(2-amino-6-quinazolinyl)-2-fluorophenyl)-2-((3-(dimethylamino)propyl)(methyl)amino)-5-(trifluoromethyl)benzamide;
N-(5-(2-amino-6-quinazolinyl)-2-fluorophenyl)-2-((3R)-3-(dimethylamino)-1-pyrrolidinyl)-5-(trifluoromethyl)benzamide;
3-(2-amino-6-quinazolinyl)-N-(2-(1,1-dioxido-4-thiomorpholinyl)-5-(trifluoromethyl)phenyl)-4-methylbenzamide;
3-(2-amino-6-quinazolinyl)-N-(2-((3-(dimethylamino)propyl)(methyl)amino)-5-(trifluoromethyl)phenyl)-4-methylbenzamide;
3-(2-amino-6-quinazolinyl)-4-methyl-N-phenylbenzamide;
3-(2-amino-6-quinazolinyl)-4-methyl-N-(4-(phenyloxy)phenyl)benzamide;
3-(2-amino-6-quinazolinyl)-4-methyl-N-(2-(methyloxy)phenyl)benzamide;
3-(2-amino-6-quinazolinyl)-N-(2,4-bis(methyloxy)phenyl)-4-methylbenzamide;
3-(2-amino-6-quinazolinyl)-N-cyclohexyl-4-methylbenzamide;
3-(2-amino-6-quinazolinyl)-4-methyl-N-(2-(1-piperidinyl)phenyl)benzamide;
3-(2-amino-6-quinazolinyl)-N-(4-chloro-3-(trifluoromethyl)phenyl)-4-methylbenzamide;
3-(2-amino-6-quinazolinyl)-N-cyclopentyl-4-methylbenzamide;
N-(2-((3-(dimethylamino)propyl)(methyl)amino)-5-(trifluoromethyl)phenyl)-4-methyl-3-(2-(methylamino)-6-quinazolinyl)benzamide;
3-(2-amino-6-quinazolinyl)-N-(2-((3-(dimethylamino)propyl)(methyl)amino)phenyl)-4-methylbenzamide;
3-(2-amino-6-quinazolinyl)-N-(2-((3-(dimethylamino)propyl)(methyl)amino)-4-methylphenyl)-4-methylbenzamide;
3-(2-amino-6-quinazolinyl)-4-methyl-N-(4-(1-methyl-4-piperidinyl)phenyl)benzamide;
3-(2-amino-6-quinazolinyl)-4-methyl-N-(2-(4-methyl-1-piperazinyl)phenyl)benzamide;
3-(2-amino-6-quinazolinyl)-4-methyl-N-(2-methyl-6-(methyloxy)-3-(trifluoromethyl)phenyl)benzamide;
3-(2-amino-6-quinazolinyl)-N-(3-chloro-4-((2-(1-piperidinyl)ethyl)oxy)phenyl)-4-methylbenzamide;
3-(2-amino-6-quinazolinyl)-N-(2-fluoro-5-(trifluoromethyl)phenyl)-4-methylbenzamide;
3-(2-amino-6-quinazolinyl)-4-methyl-N-(5-quinolinyl)benzamide;
3-(2-amino-6-quinazolinyl)-4-methyl-N-(8-quinolinyl)benzamide;
3-(2-amino-6-quinazolinyl)-N-(5,6-bis(methyloxy)-1,3-cyclohexadien-1-yl)-4-methylbenzamide;
3-(2-amino-6-quinazolinyl)-N-(5-chloro-6-(methyloxy)-1,3-cyclohexadien-1-yl)-4-methylbenzamide;
3-(2-amino-6-quinazolinyl)-N-(2-((3-(dimethylamino)propyl)(methyl)amino)-4,5-dimethylphenyl)-4-methylbenzamide;
3-(2-amino-6-quinazolinyl)-N-(6-((3-(dimethylamino)propyl)(methyl)amino)-2-methyl-3-(trifluoromethyl)phenyl)-4-methylbenzamide;
3-(2-amino-6-quinazolinyl)-N-(2-((3-(dimethylamino)propyl)(methyl)amino)-4-(trifluoromethyl)phenyl)-4-methylbenzamide;
3-(2-amino-6-quinazolinyl)-4-methyl-N-(4-(methyloxy)-3-(trifluoromethyl)phenyl)benzamide;
4-methyl-3-(2-(methylamino)-6-quinazolinyl)-N-(2-methyl-3-(trifluoromethyl)phenyl)benzamide;
N-(4-chloro-3-(trifluoromethyl)phenyl)-4-methyl-3-(2-(methylamino)-6-quinazolinyl)benzamide;
3-(2-amino-6-quinazolinyl)-N-(4-chloro-2-methyl-3-(trifluoromethyl)phenyl)-4-methylbenzamide;
N-(4-chloro-2-methyl-3-(trifluoromethyl)phenyl)-4-methyl-3-(2-(methylamino)-6-quinazolinyl)benzamide;
N-(2-fluoro-5-(trifluoromethyl)phenyl)-4-methyl-3-(2-(methylamino)-6-quinazolinyl)benzamide;
3-(2-amino-6-quinazolinyl)-4-methyl-N-(3-(trifluoromethyl)phenyl)benzamide;
N-(3-(2-amino-6-quinazolinyl)-4-methylphenyl)-N'-(3-(trifluoromethyl)phenyl)urea;
3-(2-amino-6-quinazolinyl)-4-methyl-N-(4-(4-methyl-1-piperazinyl)phenyl)benzamide;
3-(2-amino-6-quinazolinyl)-4-methyl-N-(phenylmethyl)benzamide;
N-(1-acetyl-3,3-dimethyl-2,3-dihydro-1H-indol-6-yl)-3-(2-amino-6-quinazolinyl)-4-methylbenzamide;
N-(1-acetyl-2,3-dihydro-1H-indol-6-yl)-3-(2-amino-6-quinazolinyl)-4-methylbenzamide;
N-(3-(2-amino-6-quinazolinyl)-4-methylphenyl)-N'-phenylurea;
N-(3-(2-amino-6-quinazolinyl)-4-methylphenyl)-N'-(4-(phenyloxy)phenyl)urea;
N-(3-(2-amino-6-quinazolinyl)-4-methylphenyl)-N'-(3,5-bis(methyloxy)phenyl)urea;
3-(2-amino-6-quinazolinyl)-4-methyl-N-(3-pyridinylmethyl)benzamide;
3-(2-amino-6-quinazolinyl)-4-methyl-N-(2-pyridinylmethyl)benzamide;
3-(2-amino-6-quinazolinyl)-4-methyl-N-((1R)-1-phenylethyl)benzamide;
3-(2-amino-6-quinazolinyl)-4-methyl-N-((3-(trifluoromethyl)phenyl)methyl)benzamide;
3-(2-amino-6-quinazolinyl)-N-(3,5-bis(trifluoromethyl)phenyl)-4-methylbenzamide;
3-(2-amino-6-quinazolinyl)-N-(2,3-dihydro-1H-inden-4-yl)-4-methylbenzamide;
3-(2-amino-6-quinazolinyl)-4-methyl-N-(2-(methylsulfanyl)-3-(trifluoromethyl)phenyl)benzamide;
N-(3-(2-amino-6-quinazolinyl)-4-methylphenyl)-N'-(phenylmethyl)urea;
N-(3-(2-amino-6-quinazolinyl)-4-methylphenyl)-N'-(3-(trifluoromethyl)phenyl)thiourea;
4-methyl-3-(2-(methylamino)-6-quinazolinyl)-N-(3-(trifluoromethyl)phenyl)benzamide;
N-(3-(2-amino-6-quinazolinyl)-4-methylphenyl)-N'-phenylthiourea;
3-(2-amino-6-quinazolinyl)-4-methyl-N-(4-(pentafluoroethyl)phenyl)benzamide;
3-(trifluoromethyl)phenyl 3-(2-amino-6-quinazolinyl)-4-methylphenylcarbamate;
N-(6-(2-methyl-5-((phenylcarbonyl)amino)phenyl)-2-quinazolinyl)benzamide;
N-(3-(2-amino-6-quinazolinyl)-4-methylphenyl)benzamide;
N-(3-(2-amino-6-quinazolinyl)-4-methylphenyl)-3-(trifluoromethyl)benzamide;
3-(2-amino-6-quinazolinyl)-N-(2-fluoro-3-(trifluoromethyl)phenyl)-4-methylbenzamide;
3-(2-amino-6-quinazolinyl)-4-methyl-N-((1S)-1-phenylethyl)benzamide;
3-(2-amino-6-quinazolinyl)-N-((1R)-2-(dimethylamino)-1-phenylethyl)-4-methylbenzamide;
3-(2-amino-6-quinazolinyl)-4-methyl-N-((1R)-2-(4-methyl-1-piperazinyl)-1-phenylethyl)benzamide;
N-(3-(2-amino-6-quinazolinyl)-4-methylphenyl)-4-(1,1-dimethylethyl)benzamide;
N-(3-(2-amino-6-quinazolinyl)-4-methylphenyl)-2,3-dichlorobenzamide;
N-(3-(2-amino-6-quinazolinyl)-4-methylphenyl)-3,5-bis(trifluoromethyl)benzamide;
N-(3-(2-amino-6-quinazolinyl)-4-methylphenyl)-2-fluoro-3-(trifluoromethyl)benzamide;
N-(3-(2-amino-6-quinazolinyl)-4-methylphenyl)-4-((trifluoromethyl)oxy)benzamide;
N-(3-(2-amino-6-quinazolinyl)-4-methylphenyl)-3,5-dichlorobenzamide;
N-(3-(2-amino-6-quinazolinyl)-4-methylphenyl)-3-fluoro-5-(trifluoromethyl)benzamide;
N-(3-(2-amino-6-quinazolinyl)-4-methylphenyl)-4-(methyloxy)-3-(trifluoromethyl)benzamide;
N-(3-(2-amino-6-quinazolinyl)-4-methylphenyl)-2,3-dichlorobenzamide;
N-(3-(2-amino-6-quinazolinyl)-4-methylphenyl)-2-methyl-3-(trifluoromethyl)benzamide;
N-(3-(2-amino-6-quinazolinyl)-4-methylphenyl)-2-naphthalenecarboxamide;
N-(3-(2-amino-6-quinazolinyl)-4-methylphenyl)-1-naphthalenecarboxamide;
N-(3-(2-amino-6-quinazolinyl)-4-methylphenyl)-2,2-difluoro-1,3-benzodioxole-4-carboxamide;
N-(3-(2-amino-6-quinazolinyl)-4-methylphenyl)-5,5,8,8-tetramethyl-5,6,7,8-tetrahydro-2-naphthalenecarboxamide;
N-(3-(2-amino-6-quinazolinyl)-4-methylphenyl)-3-(1,1-dimethylethyl)-1-methyl-1H-pyrazole-5-carboxamide;
4-methyl-3-(2-((2-(4-morpholinyl)ethyl)amino)-6-quinazolinyl)-N-(3-(trifluoromethyl)phenyl)benzamide;
2,3-dichloro-N-(4-methyl-3-(2-(methylamino)-6-quinazolinyl)phenyl)benzamide;
N-(4-methyl-3-(2-(methylamino)-6-quinazolinyl)phenyl)-N'-(3-(trifluoromethyl)phenyl)urea;
N-(3-(2-amino-6-quinazolinyl)-4-methylphenyl)-3-chloro-2-fluorobenzamide;
N-(3-(2-amino-6-quinazolinyl)-4-methylphenyl)-3-fluoro-2-(trifluoromethyl)benzamide;
N-(3-(2-amino-6-quinazolinyl)-4-methylphenyl)-1-benzofuran-2-carboxamide;
N-(3-(2-amino-6-quinazolinyl)-4-methylphenyl)-1,3-dimethyl-1H-thieno[2,3-c]pyrazole-5-carboxamide;
N-(3-(2-amino-6-quinazolinyl)-4-(trifluoromethyl)phenyl)-3-(trifluoromethyl)benzamide;
N-(3-(2-amino-6-quinazolinyl)-4-(trifluoromethyl)phenyl)-N'-(3-(trifluoromethyl)phenyl)urea;
2,3-dichloro-N-(4-methyl-3-(2-((2-(4-morpholinyl)ethyl)amino)-6-quinazolinyl)phenyl)benzamide;
N-(2,3-dihydro-1H-inden-4-yl)-4-methyl-3-(2-((2-(4-morpholinyl)ethyl)amino)-6-quinazolinyl)benzamide;
N-(3-(2-amino-6-quinazolinyl)-4-methylphenyl)-3-(trifluoromethyl)benzenesulfonamide;
3-(2-amino-6-quinazolinyl)-4-methyl-N-((1S)-1-(1,3-thiazol-2-yl)ethyl)benzamide;
3-(2-amino-6-quinazolinyl)-N-(2,3-dimethyl-4-(methyloxy)phenyl)-4-methylbenzamide;
N-(1-acetyl-2,3-dihydro-1H-indol-7-yl)-3-(2-amino-6-quinazolinyl)-4-methylbenzamide;
3-(2-amino-6-quinazolinyl)-4-methyl-N-(1-methyl-1H-indol-4-yl)benzamide;
N,N'-bis(3-(2-amino-6-quinazolinyl)-4-methylphenyl)urea;
N-(2-fluoro-3-(trifluoromethyl)phenyl)-4-methyl-3-(2-((2-(4-morpholinyl)ethyl)amino)-6-quinazolinyl)benzamide;
N-(2-fluoro-3-(trifluoromethyl)phenyl)-4-methyl-3-(2-((3-(4-morpholinyl)propyl)amino)-6-quinazolinyl)benzamide;
N-(2-fluoro-3-(trifluoromethyl)phenyl)-4-methyl-3-(2-((2-(methyloxy)ethyl)amino)-6-quinazolinyl)benzamide;
N-(2-fluoro-3-(trifluoromethyl)phenyl)-4-methyl-3-(2-((1-methyl-4-piperidinyl)amino)-6-quinazolinyl)benzamide;
N-(2-fluoro-3-(trifluoromethyl)phenyl)-4-methyl-3-(2-((2-(1-pyrrolidinyl)ethyl)amino)-6-quinazolinyl)benzamide;
N-(2-fluoro-3-(trifluoromethyl)phenyl)-4-methyl-3-(2-(methylamino)-6-quinazolinyl)benzamide;
3-(2-amino-6-quinazolinyl)-N-(2,1,3-benzoxadiazol-4-yl)-4-methylbenzamide;
3-(2-amino-6-quinazolinyl)-N-(2-(2-cyanoethyl)-2,4,5,6-tetrahydrocyclopenta[c]pyrazol-3-yl)-4-methylbenzamide;
3-(2-amino-6-quinazolinyl)-N-(4-((3-(diethylamino)propyl)oxy)-3-(trifluoromethyl)phenyl)-4-methylbenzamide;
3-(2-amino-6-quinazolinyl)-4-methyl-N-(4-((1-methyl-4-piperidinyl)oxy)-3-(trifluoromethyl)phenyl)benzamide;
3-(2-amino-6-quinazolinyl)-N-(4-fluoro-3-(trifluoromethyl)phenyl)-4-methylbenzamide;
N-(4-fluoro-3-(trifluoromethyl)phenyl)-4-methyl-3-(2-(methylamino)-6-quinazolinyl)benzamide;
3-(2-amino-6-quinazolinyl)-N-(4-cyano-3-(trifluoromethyl)phenyl)-4-methylbenzamide;
4-methyl-N-(2-methyl-3-(trifluoromethyl)phenyl)-3-(2-((2-(4-morpholinyl)ethyl)amino)-6-quinazolinyl)benzamide;
3-(2-(cyclopropylamino)-6-quinazolinyl)-4-methyl-N-(3-(trifluoromethyl)phenyl)benzamide;
N-(2-fluoro-3-(trifluoromethyl)phenyl)-4-methyl-3-(2-((4-(4-methyl-1-piperazinyl)phenyl)amino)-6-quinazolinyl)benzamide;
N-(2-fluoro-3-(trifluoromethyl)phenyl)-4-methyl-3-(2-((4-((3-(1-piperidinyl)propyl)oxy)phenyl)amino)-6-quinazolinyl)benzamide;
4-methyl-N-(2-methyl-3-(trifluoromethyl)phenyl)-3-(2-((3-(4-morpholinyl)propyl)amino)-6-quinazolinyl)benzamide;
4-methyl-N-(2-methyl-3-(trifluoromethyl)phenyl)-3-(2-((2-(1-pyrrolidinyl)ethyl)amino)-6-quinazolinyl)benzamide;
4-methyl-N-(2-methyl-3-(trifluoromethyl)phenyl)-3-(2-((3-(2-oxo-1-pyrrolidinyl)propyl)amino)-6-quinazolinyl)benzamide;
3-(2-(((1-ethyl-4-piperidinyl)methyl)amino)-6-quinazolinyl)-4-methyl-N-(2-methyl-3-(trifluoromethyl)phenyl)benzamide;
4-methyl-3-(2-((1-methyl-4-piperidinyl)amino)-6-quinazolinyl)-N-(2-methyl-3-(trifluoromethyl)phenyl)benzamide;
3-(2-(cyclopropylamino)-6-quinazolinyl)-4-methyl-N-(2-methyl-3-(trifluoromethyl)phenyl)benzamide;
6-(2,6-dimethylphenyl)-N-(2-(4-morpholinyl)ethyl)-2-quinazolinamine;
6-(2,6-dimethylphenyl)-N-(4-(4-methyl-1-piperazinyl)phenyl)-2-quinazolinamine;
6-(2,6-dimethylphenyl)-N-(4-((3-(1-piperidinyl)propyl)oxy)phenyl)-2-quinazolinamine;
5-(2-amino-6-quinazolinyl)-2-fluoro-4-methyl-N-(3-(trifluoromethyl)phenyl)benzamide;
2-fluoro-4-methyl-5-(2-(methylamino)-6-quinazolinyl)-N-(3-(trifluoromethyl)phenyl)benzamide;
5-(2-(cyclopropylamino)-6-quinazolinyl)-2-fluoro-4-methyl-N-(3-(trifluoromethyl)phenyl)benzamide;
3-(2-(cyclopropylamino)-6-quinazolinyl)-4-methyl-N-(4-((1-methyl-4-piperidinyl)oxy)-3-(trifluoromethyl)phenyl)benzamide;
3-(2-amino-6-quinazolinyl)-4-methyl-N-(4-(trifluoromethyl)phenyl)benzamide;
3-(2-amino-6-quinazolinyl)-N-(4-(1,1-dimethylethyl)phenyl)-4-methylbenzamide;
3-(2-amino-6-quinazolinyl)-N-(4,4-dimethyl-2-oxo-1,2,3,4-tetrahydro-7-quinolinyl)-4-methylbenzamide;
3-(2-amino-6-quinazolinyl)-N-(4,4-dimethyl-1,2,3,4-tetrahydro-7-quinolinyl)-4-methylbenzamide;
3-(2-amino-6-quinazolinyl)-N-(4,4-dimethyl-1,2,3,4-tetrahydro-7-isoquinolinyl)-4-methylbenzamide;
5-(2-amino-6-quinazolinyl)-2-fluoro-N-(4-(trifluoromethyl)phenyl)benzamide;
5-(2-amino-6-quinazolinyl)-N-(4,4-dimethyl-2-oxo-1,2,3,4-tetrahydro-7-quinolinyl)-2-fluorobenzamide;
5-(2-amino-6-quinazolinyl)-N-(4-cyclohexylphenyl)-2-fluorobenzamide;
5-(2-amino-6-quinazolinyl)-2-fluoro-N-(4-(4-morpholinyl)phenyl)benzamide;
5-(2-amino-6-quinazolinyl)-N-(4-(1,1-dimethylethyl)phenyl)-2-fluorobenzamide;
3-(2-amino-6-quinazolinyl)-5-chloro-N-(4-(trifluoromethyl)phenyl)benzamide;
3-(2-amino-6-quinazolinyl)-5-chloro-N-cyclopropylbenzamide;
3-(2-amino-6-quinazolinyl)-5-chloro-N-(1-methylethyl)benzamide;
3-(2-amino-6-quinazolinyl)-5-bromo-N-(4-(trifluoromethyl)phenyl)benzamide;
3-(2-amino-6-quinazolinyl)-5-bromo-N-(4-chloro-3-(trifluoromethyl)phenyl)benzamide;
3-(2-amino-6-quinazolinyl)-5-bromo-N-(3-methyl-4-(1-methylethyl)phenyl)benzamide;
3-(2-amino-6-quinazolinyl)-5-bromo-N-(4-((trifluoromethyl)oxy)phenyl)benzamide;
3-(2-amino-6-quinazolinyl)-5-bromo-N-(3-chloro-4-methylphenyl)benzamide;
3-(2-amino-6-quinazolinyl)-5-bromo-N-cyclopropylbenzamide;
N-(3-(2-amino-6-quinazolinyl)phenyl)-5-(dimethylamino)-1-naphthalenesulfonamide;
N-(5-(2-amino-6-quinazolinyl)-2-methylphenyl)-3-(trifluoromethyl)benzamide;
N-(5-(2-amino-6-quinazolinyl)-2-methylphenyl)-4-(1,1-dimethylethyl)benzamide;
N-(5-(2-amino-6-quinazolinyl)-2-methylphenyl)-5-methyl-3-isoxazolecarboxamide;
N-(5-(2-amino-6-quinazolinyl)-2-methylphenyl)-3,4-difluorobenzamide;
N-(5-(2-amino-6-quinazolinyl)-2-methylphenyl)cyclopropanecarboxamide;
N-(3-(2-amino-6-quinazolinyl)-5-chlorophenyl)-3-(trifluoromethyl)benzamide;
N'-(3-(2-amino-6-quinazolinyl)-5-chlorophenyl)-N,N-dimethylurea;
N-(3-(2-amino-6-quinazolinyl)-5-chlorophenyl)-4-(1,1-dimethylethyl)benzamide;
N-(3-(2-amino-6-quinazolinyl)-5-chlorophenyl)-4-(dimethylamino)benzamide;
N-(3-(2-amino-6-quinazolinyl)-5-chlorophenyl)-3-((trifluoromethyl)oxy)benzamide;
N-(3-(2-amino-6-quinazolinyl)-5-chloro-4-methylphenyl)-4-(trifluoromethyl)benzamide;
N-(3-(2-amino-6-quinazolinyl)-5-chloro-4-methylphenyl)-3-(trifluoromethyl)benzamide;
N-(3-(2-amino-6-quinazolinyl)-5-chloro-4-methylphenyl)-3-chlorobenzamide;
N-(3-(2-amino-6-quinazolinyl)-5-chloro-4-methylphenyl)-4-chlorobenzamide;
N-(3-(2-amino-6-quinazolinyl)-5-chloro-4-methylphenyl)-4-(methyloxy)-3-(trifluoromethyl)benzamide;
3-(2-amino-6-quinazolinyl)-N-cyclopropyl-4-(methyloxy)benzamide;
3-(2-amino-6-quinazolinyl)-2-methyl-N-(3-(trifluoromethyl)phenyl)benzamide;
3-(2-amino-6-quinazolinyl)-4-methyl-N-(1-methylethyl)benzamide;
ethyl 7-(2-methyl-5-(((3-(trifluoromethyl)phenyl)amino)carbonyl)phenyl)-3-isoquinolinecarboxylate;
3-(2-amino-6-quinazolinyl)-N-(2-(dimethylamino)ethyl)-4-methylbenzamide;
7-(2-methyl-5-(((3-(trifluoromethyl)phenyl)amino)carbonyl)phenyl)-3-isoquinolinecarboxylic acid;
N-(2-(dimethylamino)ethyl)-7-(2-methyl-5-(((3-(trifluoromethyl)phenyl)amino)carbonyl)phenyl)-3-isoquinolinecarboxamide;
3-(2-amino-6-quinazolinyl)-4-methyl-N-(1-methylpropyl)benzamide;
ethyl 7-(5-((cyclopropylamino)carbonyl)-2-methylphenyl)-3-isoquinolinecarboxylate;
7-(5-((cyclopropylamino)carbonyl)-2-methylphenyl)-3-isoquinolinecarboxylic acid;
N-cyclobutyl-4-methyl-3-(2-((2-(4-morpholinyl)ethyl)amino)-6-quinazolinyl)benzamide;
7-(5-((cyclopropylamino)carbonyl)-2-methylphenyl)-N-(2-(dimethylamino)ethyl)-3-isoquinolinecarboxamide;
3-(2-aminopyrido[2,3-d]pyrimidin-6-yl)-N-(3-(trifluoromethyl)phenyl)benzamide;
4-methyl-3-(2-(methylamino)pyrido[2,3-d]pyrimidin-6-yl)-N-phenylbenzamide;
3-(2-amino-6-quinazolinyl)-N-(2-((3-(dimethylamino)propyl)(methyl)amino)-5-ethynylphenyl)-4-methylbenzamide;
N-(2-((3-(dimethylamino)propyl)(methyl)amino)-5-(trifluoromethyl)phenyl)-4-methyl-3-(2-(methylamino)pyrido[2,3-d]pyrimidin-6-yl)benzamide;
3-(2-amino-6-quinazolinyl)-4-methyl-N-(3-((((2S)-1-methyl-2-pyrrolidinyl)methyl)oxy)-5-(trifluoromethyl)phenyl)benzamide;
N-(2-((3-(dimethylamino)propyl)(methyl)amino)-5-(trifluoromethyl)phenyl)-3-(2-(((ethylamino)carbonyl)amino)-6-quinazolinyl)-4-methylbenzamide;
3-(2-(((ethylamino)carbonyl)amino)-6-quinazolinyl)-4-methyl-N-(3-(trifluoromethyl)phenyl)benzamide;
3-(2-amino-6-quinazolinyl)-4-methyl-N-(3-((1-methylethyl)oxy)phenyl)benzamide;
3-(2-amino-6-quinazolinyl)-N-(3-(ethyloxy)phenyl)-4-methylbenzamide;
3-(2-(dimethylamino)-6-quinazolinyl)-4-methyl-N-(3-(trifluoromethyl)phenyl)benzamide;
3-(2-(dimethylamino)-6-quinazolinyl)-4-methyl-N-(4-(trifluoromethyl)phenyl)benzamide;
2-fluoro-5-(2-(methylamino)pyrido[2,3-d]pyrimidin-6-yl)-N-(3-(trifluoromethyl)phenyl)benzamide;
N-(2-((3-(dimethylamino)propyl)(methyl)amino)-5-(trifluoromethyl)phenyl)-2-fluoro-5-(2-(methylamino)pyrido[2,3-d]pyrimidin-6-yl)benzamide;
3-(2-amino-6-quinazolinyl)-N-(5-(1,1-dimethylethyl)-2-(methyloxy)phenyl)-4-methylbenzamide;
N-(3-(2-amino-6-quinazolinyl)-4-methylphenyl)-2,5-bis(trifluoromethyl)benzamide;
N-(3-(2-amino-6-quinazolinyl)-4-methylphenyl)-2-(trifluoromethyl)benzamide;
N-(3-(2-amino-6-quinazolinyl)-4-methylphenyl)-2-fluoro-4-(trifluoromethyl)benzamide;
N-(3-(2-amino-6-quinazolinyl)-4-methylphenyl)-1-methyl-1H-indole-2-carboxamide;
N-(3-(2-amino-6-quinazolinyl)-4-methylphenyl)-2,4-bis(trifluoromethyl)benzamide;
N-(3-(2-amino-6-quinazolinyl)-4-methylphenyl)-2,3-difluorobenzamide;
N-(3-(2-amino-6-quinazolinyl)-4-methylphenyl)-3,5-bis(1,1-dimethylethyl)benzamide;
N-(3-(2-amino-6-quinazolinyl)-4-methylphenyl)-3,5-bis(methyloxy)benzamide;
N-(3-(2-amino-6-quinazolinyl)-4-methylphenyl)-3,4-dimethylbenzamide;
N-(3-(2-amino-6-quinazolinyl)-4-methylphenyl)-2-chloro-3-(trifluoromethyl)benzamide;
N-(3-(2-amino-6-quinazolinyl)-4-methylphenyl)-2,3-dimethylbenzamide;
N-(3-(2-amino-6-quinazolinyl)-4-methylphenyl)-4-chloro-2-(trifluoromethyl)benzamide;
N-(3-(2-amino-6-quinazolinyl)-4-(methyloxy)phenyl)-1-methyl-1H-indole-2-carboxamide;
N-(3-(2-amino-6-quinazolinyl)-4-methylphenyl)-2-chloro-3-methylbenzamide;
N-(3-(2-amino-6-quinazolinyl)-4-methylphenyl)-2-chloro-3-fluorobenzamide;
N-(3-(2-amino-6-quinazolinyl)-4-(methyloxy)phenyl)-2-fluoro-3-(trifluoromethyl)benzamide;
N-(3-(2-amino-6-quinazolinyl)-4-methylphenyl)-3-chloro-2,4-difluorobenzamide;
N-(3-(2-amino-6-quinazolinyl)-4-methylphenyl)-2,3-difluoro-4-(trifluoromethyl)benzamide;
N-(3-(2-amino-6-quinazolinyl)-4-methylphenyl)-2,3-difluoro-4-methylbenzamide;
N-(3-(2-amino-6-quinazolinyl)phenyl)-2-fluoro-3-(trifluoromethyl)benzamide;
N-(3-(2-amino-6-quinazolinyl)phenyl)-2,3-dichlorobenzamide;
N-(3-(2-amino-6-quinazolinyl)-4-fluorophenyl)-2-fluoro-3-(trifluoromethyl)benzamide;
N-(3-(2-amino-6-quinazolinyl)-4-fluorophenyl)-2,3-dichlorobenzamide;
N-(3-(2-amino-6-quinazolinyl)-4-chlorophenyl)-2-fluoro-3-(trifluoromethyl)benzamide;
N-(3-(2-amino-6-quinazolinyl)-4-chlorophenyl)-2,3-dichlorobenzamide;
3-(2-amino-6-quinazolinyl)-4-fluoro-N-(2-fluoro-3-(trifluoromethyl)phenyl)benzamide;
3-(2-amino-6-quinazolinyl)-4-fluoro-N-(3-(trifluoromethyl)phenyl)benzamide;
3-(2-amino-6-quinazolinyl)-N-(5-(1,1-dimethylethyl)-2-(methyloxy)phenyl)-4-fluorobenzamide;
3-(2-amino-6-quinazolinyl)-4-chloro-N-(5-(1,1-dimethylethyl)-2-(methyloxy)phenyl)benzamide;
3-(2-amino-6-quinazolinyl)-4-chloro-N-(2-fluoro-3-(trifluoromethyl)phenyl)benzamide;
3-(2-amino-6-quinazolinyl)-4-chloro-N-(2-methyl-3-(trifluoromethyl)phenyl)benzamide;
3-(2-amino-6-quinazolinyl)-4-chloro-N-(3-((trifluoromethyl)oxy)phenyl)benzamide;
3-(4-amino-6-quinazolinyl)-4-methyl-N-(3-(trifluoromethyl)phenyl)benzamide;
3-(4-amino-6-quinazolinyl)-4-methyl-N-(4-(phenyloxy)phenyl)benzamide;
3-(4-(((1E)-(dimethylamino)methylidene)amino)-6-quinazolinyl)-4-methyl-N-(4-(phenyloxy)phenyl)benzamide;
3-(4-amino-6-quinazolinyl)-4-methyl-N-phenylbenzamide;
3-(4-amino-6-quinazolinyl)-N-(3-bromophenyl)-4-methylbenzamide;
3-(4-amino-6-quinazolinyl)-N-(4-(1,1-dimethylethyl)phenyl)-4-methylbenzamide;
3-(4-amino-6-quinazolinyl)-4-methyl-N-(4-(4-morpholinyl)phenyl)benzamide;
3-(4-amino-6-quinazolinyl)-4-methyl-N-(2-((4-methyl-1-piperazinyl)methyl)-5-(trifluoromethyl)phenyl)benzamide;
3-(4-amino-6-quinazolinyl)-4-methyl-N-(2-(4-morpholinyl)-5-(trifluoromethyl)phenyl)benzamide;
3-(4-amino-6-quinazolinyl)-N-(2-(dimethylamino)-5-(trifluoromethyl)phenyl)-4-methylbenzamide;
3-(4-amino-6-quinazolinyl)-4-methyl-N-(2-(1-pyrrolidinyl)-5-(trifluoromethyl)phenyl)benzamide;
3-(4-amino-6-quinazolinyl)-N-(2-fluoro-5-(trifluoromethyl)phenyl)-4-methylbenzamide;
3-(4-amino-6-quinazolinyl)-4-methyl-N-(2-(methyloxy)-5-(trifluoromethyl)phenyl)benzamide;
3-(4-amino-6-quinazolinyl)-N-cyclopropyl-4-methylbenzamide;
3-(4-amino-6-quinazolinyl)-N-cyclopentyl-4-methylbenzamide;
3-(4-amino-6-quinazolinyl)-N-(2,3-dimethylphenyl)-4-methylbenzamide;
3-(4-amino-6-quinazolinyl)-N-(2,5-bis(methyloxy)phenyl)-4-methylbenzamide;
3-(4-amino-6-quinazolinyl)-N-(cyclopropylmethyl)-4-methylbenzamide;
3-(4-amino-6-quinazolinyl)-4-methyl-N-(3-((1-methylethyl)oxy)phenyl)benzamide;
3-(2-amino-6-quinazolinyl)-N-(3,4-dimethyl-5-isoxazolyl)-4-methylbenzamide;
3-(4-amino-6-quinazolinyl)-N-(3-(1,1-dimethylethyl)phenyl)-4-methylbenzamide;
3-(2-amino-6-quinazolinyl)-N-(5-(1,1-dimethylethyl)-3-isoxazolyl)-4-methylbenzamide;
3-(4-amino-6-quinazolinyl)-N-(3,4-bis(methyloxy)-5-((3-(4-methyl-1-piperazinyl)propyl)oxy)phenyl)-4-methylbenzamide;
3-(4-amino-6-quinazolinyl)-4-methyl-N-(3-((trifluoromethyl)oxy)phenyl)benzamide;
3-(4-amino-6-quinazolinyl)-N-(4,5-dimethyl-3-isoxazolyl)-4-methylbenzamide;
3-(4-amino-6-quinazolinyl)-4-methyl-N-(phenylmethyl)benzamide;
3-(4-amino-6-quinazolinyl)-4-methyl-N-(2-methyl-5-(1-methylethyl)phenyl)benzamide;
3-(4-amino-6-quinazolinyl)-4-methyl-N-(3-((phenylmethyl)oxy)phenyl)benzamide;
3-(2-amino-6-quinazolinyl)-4-methyl-N-(4-pyridinylmethyl)benzamide;
3-(2-amino-6-quinazolinyl)-4-methyl-N-(2-pyridinylmethyl)benzamide;
3-(4-amino-6-quinazolinyl)-N-(1,1'-biphenyl-3-yl)-4-methylbenzamide;
3-(4-amino-6-quinazolinyl)-N-(5-(1,1-dimethylethyl)-2-(methyloxy)phenyl)-4-methylbenzamide;
3-(4-amino-6-quinazolinyl)-N-(5-(1,1-dimethylethyl)-3-isoxazolyl)-4-methylbenzamide;
3-(4-amino-6-quinazolinyl)-4-methyl-N-(3-methyl-5-isoxazolyl)benzamide;
3-(4-amino-6-quinazolinyl)-4-methyl-N-(3-((1,1,2,2-tetrafluoroethyl)oxy)phenyl)benzamide;
3-(4-amino-6-quinazolinyl)-N-(5-cyclohexyl-2-(methyloxy)phenyl)-4-methylbenzamide;
3-(4-amino-6-quinazolinyl)-N-(3,5-bis(1,1-dimethylethyl)phenyl)-4-methylbenzamide;
3-(2-amino-6-quinazolinyl)-4-methyl-N-(3-methyl-5-isoxazolyl)benzamide;
3-(4-amino-6-quinazolinyl)-4-methyl-N-((2S)-tetrahydro-2-furanylmethyl)benzamide;
3-(2-amino-6-quinazolinyl)-N-(3,5-bis(1,1-dimethylethyl)phenyl)-4-methylbenzamide;
3-(2-amino-6-quinazolinyl)-4-methyl-N-(4-pyridinyl)benzamide;
3-(2-amino-6-quinazolinyl)-4-methyl-N-(2-pyridinyl)benzamide;
3-(2-amino-6-quinazolinyl)-N-(2-chloro-5-(trifluoromethyl)-3-pyridinyl)-4-methylbenzamide;
3-(2-amino-6-quinazolinyl)-4-methyl-N-(1,3-thiazol-2-yl)benzamide;
3-(2-amino-6-quinazolinyl)-4-methyl-N-(3-pyridinyl)benzamide;
3-(2-amino-6-quinazolinyl)-N-(5-bromo-2-pyridinyl)-4-methylbenzamide;
3-(2-amino-6-quinazolinyl)-4-methyl-N-(6-(4-morpholinyl)-3-pyridinyl)benzamide;
3-(2-amino-6-quinazolinyl)-4-methyl-N-(3-quinolinyl)benzamide;
3-(2-amino-6-quinazolinyl)-4-methyl-N-(6-quinolinyl)benzamide;
3-(2-amino-6-quinazolinyl)-N-(2-chloro-4-pyridinyl)-4-methylbenzamide;
3-(2-amino-6-quinazolinyl)-4-methyl-N-(2-naphthalenyl)benzamide;
3-(2-amino-6-quinazolinyl)-N-(4,5-dihydro-1,3-thiazol-2-yl)-4-methylbenzamide;
S-(4,5-dihydro-1,3-thiazol-2-yl)3-(2-amino-6-quinazolinyl)-4-methylbenzenecarbothioate;
3-(2-amino-6-quinazolinyl)-N-(5-ethyl-1,3,4-thiadiazol-2-yl)-4-methylbenzamide;
3-(2-amino-6-quinazolinyl)-4-methyl-N-(4-methyl-2-pyridinyl)benzamide;
3-(2-amino-6-quinazolinyl)-N-(4-ethyl-2-pyridinyl)-4-methylbenzamide;
3-(2-amino-6-quinazolinyl)-4-methyl-N-(4-methyl-1,3-thiazol-2-yl)benzamide;
3-(2-amino-6-quinazolinyl)-N-(5-chloro-1,3-benzoxazol-2-yl)-4-methylbenzamide;
3-(2-amino-6-quinazolinyl)-4-methyl-N-(5-(trifluoromethyl)-1,3,4-thiadiazol-2-yl)benzamide;
3-(2-amino-6-quinazolinyl)-4-methyl-N-(2-(3-pyridinyl)ethyl)benzamide;
3-(2-amino-6-quinazolinyl)-4-methyl-N-(5-methyl-3-isothiazolyl)benzamide;
3-(2-amino-6-quinazolinyl)-4-methyl-N-(4-(trifluoromethyl)-2-pyridinyl)benzamide;
3-(2-amino-6-quinazolinyl)-N-(3,5-dichloro-2-pyridinyl)-4-methylbenzamide;
3-(2-amino-6-quinazolinyl)-4-methyl-N-(2-(methyloxy)-3-pyridinyl)benzamide;
3-(2-amino-6-quinazolinyl)-N-(4,5-dimethyl-1,3-thiazol-2-yl)-4-methylbenzamide;
3-(2-amino-6-quinazolinyl)-4-methyl-N-(4-methyl-1,3-benzothiazol-2-yl)benzamide;
3-(2-amino-6-quinazolinyl)-N-(6-(ethyloxy)-1,3-benzothiazol-2-yl)-4-methylbenzamide;
3-(2-amino-6-quinazolinyl)-4-methyl-N-(5-nitro-1,3-thiazol-2-yl)benzamide;
3-(2-amino-6-quinazolinyl)-N-(6-fluoro-1,3-benzothiazol-2-yl)-4-methylbenzamide;
3-(2-amino-6-quinazolinyl)-N-(4-(1,1-dimethylethyl)-1,3-thiazol-2-yl)-4-methylbenzamide;
3-(2-amino-6-quinazolinyl)-N-(5-iodo-2-pyridinyl)-4-methylbenzamide;
3-(2-amino-6-quinazolinyl)-4-methyl-N-(1-methyl-1H-benzimidazol-2-yl)benzamide;
N-(2-acetyl-3-thienyl)-3-(2-amino-6-quinazolinyl)-4-methylbenzamide;
3-(2-amino-6-quinazolinyl)-N-(5-bromo-1,3-thiazol-2-yl)-4-methylbenzamide;
3-(2-amino-6-quinazolinyl)-4-methyl-N-((6-(trifluoromethyl)-3-pyridinyl)methyl)benzamide;
3-(2-amino-6-quinazolinyl)-4-methyl-N-(3-((phenylmethyl)oxy)-2-pyridinyl)benzamide;
3-(2-amino-6-quinazolinyl)-N-(2-fluoro-4-(1-pyrrolidinyl)-3-(1-pyrrolidinylcarbonyl)phenyl)-4-methylbenzamide;
3-(2-amino-6-quinazolinyl)-4-methyl-N-(6-(trifluoromethyl)-2-pyridinyl)benzamide;
3-(2-amino-6-quinazolinyl)-4-methyl-N-(5-(trifluoromethyl)-2-pyridinyl)benzamide;
3-(2-amino-6-quinazolinyl)-N-(3-hydroxy-2-pyridinyl)-4-methylbenzamide;
3-(2-amino-6-quinazolinyl)-N-(2,4-difluorophenyl)-4-methylbenzamide;
3-(2-amino-6-quinazolinyl)-4-methyl-N-(3-((3-(1-piperidinyl)propyl)oxy)-2-pyridinyl)benzamide;
3-(2-amino-6-quinazolinyl)-N-(3-cyanophenyl)-4-methylbenzamide;
3-(2-amino-6-quinazolinyl)-N-(3-((2-(diethylamino)ethyl)oxy)-2-pyridinyl)-4-methylbenzamide;
3-(2-amino-6-quinazolinyl)-4-methyl-N-(4-(methyloxy)-1,1'-biphenyl-3-yl)benzamide;
3-(2-amino-6-quinazolinyl)-4-methyl-N-(2-methyl-5-(trifluoromethyl)phenyl)benzamide;
3-(2-amino-6-quinazolinyl)-N-(5-chloro-2-(methyloxy)phenyl)-4-methylbenzamide;
3-(2-amino-6-quinazolinyl)-N-(2-((3-(dimethylamino)propyl)oxy)-5-(trifluoromethyl)phenyl)-4-methylbenzamide;
3-(2-amino-6-quinazolinyl)-4-methyl-N-(2-(methyloxy)-5-((phenylamino)carbonyl)phenyl)benzamide;
3-(2-amino-6-quinazolinyl)-4-methyl-N-(2-(methylsulfanyl)-5-(trifluoromethyl)phenyl)benzamide;
3-(2-amino-6-quinazolinyl)-N-(2-fluoro-5-methylphenyl)-4-methylbenzamide;
3-(2-amino-6-quinazolinyl)-N-(2,5-bis(methyloxy)phenyl)-4-methylbenzamide;
5-(2-amino-6-quinazolinyl)-2-chloro-N-(3-(trifluoromethyl)phenyl)benzamide;
3-(2-amino-6-quinazolinyl)-5-fluoro-N-(3-(trifluoromethyl)phenyl)benzamide;
3-(2-amino-6-quinazolinyl)-5-bromo-N-(4-(1,1-dimethylethyl)phenyl)benzamide;
3-(2-amino-6-quinazolinyl)-N-(3,5-bis(methyloxy)phenyl)-5-bromobenzamide;
3-(2-amino-6-quinazolinyl)-5-bromo-N-(3-(trifluoromethyl)phenyl)benzamide;
3-(2-amino-6-quinazolinyl)-5-chloro-N-(3-(trifluoromethyl)phenyl)benzamide;
3-(2-amino-6-quinazolinyl)-4-chloro-N-(2-(1-pyrrolidinyl)-5-(trifluoromethyl)phenyl)benzamide;
3-(2-amino-6-quinazolinyl)-4-chloro-N-(2-(1-piperidinyl)-5-(trifluoromethyl)phenyl)benzamide;
3-(2-amino-6-quinazolinyl)-4-chloro-N-(2-(4-morpholinyl)-5-(trifluoromethyl)phenyl)benzamide;
3-(4-amino-6-quinazolinyl)-4-methyl-N-(3-(1-methylethyl)phenyl)benzamide;
3-(4-amino-6-quinazolinyl)-N-(3-(1-methylethyl)phenyl)benzamide;
3-(4-amino-6-quinazolinyl)-4-methyl-N-(2-(methyl((3S)-1-methyl-3-pyrrolidinyl)amino)-5-(trifluoromethyl)phenyl)benzamide;
3-(4-amino-6-quinazolinyl)-N-(2-((3-(dimethylamino)propyl)(methyl)amino)-5-(trifluoromethyl)phenyl)-4-methylbenzamide;
3-(4-amino-6-quinazolinyl)-4-methyl-N-(2-(1-piperidinyl)-5-(trifluoromethyl)phenyl)benzamide;
N-(3-(4-amino-6-quinazolinyl)phenyl)-3-(trifluoromethyl)benzamide;
N-(3-(4-amino-6-quinazolinyl)phenyl)benzamide;
N-(3-(4-amino-6-quinazolinyl)phenyl)-3-(methyloxy)benzamide;
N-(3-(4-amino-6-quinazolinyl)phenyl)-4-(trifluoromethyl)benzamide;
3-(2-amino-6-quinazolinyl)-N-cyclopropyl-4-methylbenzamide;
3-(2-amino-6-quinazolinyl)-4-methyl-N-(2-(methyloxy)-5-(trifluoromethyl)phenyl)benzamide;
N-cyclopropyl-4-methyl-3-(2-((2-(4-morpholinyl)ethyl)amino)-6-quinazolinyl)benzamide;
4-methyl-N-(2-(methyloxy)-5-(trifluoromethyl)phenyl)-3-(2-((2-(4-morpholinyl)ethyl)amino)-6-quinazolinyl)benzamide;
N-cyclopropyl-3-(2-((2-(dimethylamino)ethyl)amino)-6-quinazolinyl)-4-methylbenzamide;
N-cyclopropyl-4-methyl-3-(2-((1-methyl-4-piperidinyl)amino)-6-quinazolinyl)benzamide;
1,1-dimethylethyl 2-(((6-(5-((cyclopropylamino)carbonyl)-2-methylphenyl)-2-quinazolinyl)amino)methyl)-1-piperidinecarboxylate;
N-cyclopropyl-4-methyl-3-(2-((2-(1-piperidinyl)ethyl)amino)-6-quinazolinyl)benzamide;
N-cyclopropyl-4-methyl-3-(2-(4-methyl-1-piperazinyl)-6-quinazolinyl)benzamide;
1,1-dimethylethyl 4-(2-((6-(5-((cyclopropylamino)carbonyl)-2-methylphenyl)-2-quinazolinyl)amino)ethyl)-1-piperazinecarboxylate;
4-methyl-3-(2-(methylamino)pyrido[2,3-d]pyrimidin-6-yl)-N-(3-(trifluoromethyl)phenyl)benzamide;
N-(2,3-dihydro-1H-inden-4-yl)-4-methyl-3-(2-(methylamino)pyrido[2,3-d]pyrimidin-6-yl)benzamide;
N-(4-methyl-3-(2-(methylamino)pyrido[2,3-d]pyrimidin-6-yl)phenyl)-3-(trifluoromethyl)benzamide;
N-(4-chloro-3-(trifluoromethyl)phenyl)-4-methyl-3-(2-(methylamino)pyrido[2,3-d]pyrimidin-6-yl)benzamide;
4-methyl-3-(2-(methylamino)pyrido[2,3-d]pyrimidin-6-yl)-N-(3-methyl-4-(1-methylethyl)phenyl)benzamide;
N-(4-methyl-3-(2-(methylamino)pyrido[2,3-d]pyrimidin-6-yl)phenyl)-3,5-bis(trifluoromethyl)benzamide;
2,3-dichloro-N-(4-methyl-3-(2-(methylamino)pyrido[2,3-d]pyrimidin-6-yl)phenyl)benzamide;
2-fluoro-N-(4-methyl-3-(2-(methylamino)pyrido[2,3-d]pyrimidin-6-yl)phenyl)-3-(trifluoromethyl)benzamide;
4-methyl-3-(2-(methylamino)pyrido[2,3-d]pyrimidin-6-yl)-N-(2-methyl-3-(trifluoromethyl)phenyl)benzamide;
2-fluoro-N-(4-methyl-3-(2-(methylamino)pyrido[2,3-d]pyrimidin-6-yl)phenyl)-5-(trifluoromethyl)benzamide;
4-(1,1-dimethylethyl)-N-(4-methyl-3-(2-(methylamino)pyrido[2,3-d]pyrimidin-6-yl)phenyl)benzamide;
4-methyl-3-(2-(methylamino)pyrido[2,3-d]pyrimidin-6-yl)-N-(4-(trifluoromethyl)phenyl)benzamide;
N-(4-(1,1-dimethylethyl)phenyl)-4-methyl-3-(2-(methylamino)pyrido[2,3-d]pyrimidin-6-yl)benzamide;
N-(3-(1,1-dimethylethyl)phenyl)-4-methyl-3-(2-(methylamino)pyrido[2,3-d]pyrimidin-6-yl)benzamide;
N-(4-methyl-3-(2-(methylamino)pyrido[2,3-d]pyrimidin-6-yl)phenyl)-2,4-bis(trifluoromethyl)benzamide;
N-(4-methyl-3-(2-(methylamino)pyrido[2,3-d]pyrimidin-6-yl)phenyl)-2,3-dihydro-1-benzofuran-7-carboxamide;
3-chloro-2-fluoro-N-(4-methyl-3-(2-(methylamino)pyrido[2,3-d]pyrimidin-6-yl)phenyl)benzamide;
3-fluoro-N-(4-methyl-3-(2-(methylamino)pyrido[2,3-d]pyrimidin-6-yl)phenyl)-2-(trifluoromethyl)benzamide;
2-chloro-3-fluoro-N-(4-methyl-3-(2-(methylamino)pyrido[2,3-d]pyrimidin-6-yl)phenyl)benzamide;
2-chloro-3-methyl-N-(4-methyl-3-(2-(methylamino)pyrido[2,3-d]pyrimidin-6-yl)phenyl)benzamide;
2,3-difluoro-N-(4-methyl-3-(2-(methylamino)pyrido[2,3-d]pyrimidin-6-yl)phenyl)-4-(trifluoromethyl)benzamide;
2,3-difluoro-4-methyl-N-(4-methyl-3-(2-(methylamino)pyrido[2,3-d]pyrimidin-6-yl)phenyl)benzamide;
4-methyl-3-(2-(methylamino)pyrido[2,3-d]pyrimidin-6-yl)-N-(3-((trifluoromethyl)oxy)phenyl)benzamide;
2-methyl-3-(2-(methylamino)pyrido[2,3-d]pyrimidin-6-yl)-N-(3-(trifluoromethyl)phenyl)benzamide;
2-methyl-3-(2-(methylamino)pyrido[2,3-d]pyrimidin-6-yl)-N-(2-methyl-3-(trifluoromethyl)phenyl)benzamide;
N-(2,3-dichlorophenyl)-2-methyl-3-(2-(methylamino)pyrido[2,3-d]pyrimidin-6-yl)benzamide;
2-methyl-3-(2-(methylamino)pyrido[2,3-d]pyrimidin-6-yl)-N-(4-(methyloxy)-3-(trifluoromethyl)phenyl)benzamide;
2-methyl-3-(2-(methylamino)pyrido[2,3-d]pyrimidin-6-yl)-N-(3-((trifluoromethyl)oxy)phenyl)benzamide;
N-(4-methyl-3-(2-(methylamino)pyrido[2,3-d]pyrimidin-6-yl)phenyl)-4-(trifluoromethyl)benzamide;
4-methyl-3-(2-(methylamino)pyrido[2,3-d]pyrimidin-6-yl)-N-(4-(methyloxy)-3-(trifluoromethyl)phenyl)benzamide;
4-methyl-3-(2-(methylamino)pyrido[2,3-d]pyrimidin-6-yl)-N-(2-pyridinylmethyl)benzamide;
N-(2-methyl-3-(2-(methylamino)pyrido[2,3-d]pyrimidin-6-yl)phenyl)-3-(trifluoromethyl)benzamide;
2,2-difluoro-N-(4-methyl-3-(2-(methylamino)pyrido[2,3-d]pyrimidin-6-yl)phenyl)-1,3-benzodioxole-4-carboxamide;
N-(5-(1,1-dimethylethyl)-2-(methyloxy)phenyl)-4-methyl-3-(2-(methylamino)pyrido[2,3-d]pyrimidin-6-yl)benzamide;
3-(2-amino-6-quinazolinyl)-4-methyl-N-(4-((((2-(4-morpholinyl)ethyl)amino)carbonyl)amino)-3-(trifluoromethyl)phenyl)benzamide;
3-(2-amino-6-quinazolinyl)-N-(4-((((2-(diethylamino)ethyl)amino)carbonyl)amino)-3-(trifluoromethyl)phenyl)-4-methylbenzamide;
4-methyl-3-(2-(methylamino)pyrido[2,3-d]pyrimidin-6-yl)-N-(4-((((2-(4-morpholinyl)ethyl)amino)carbonyl)amino)-3-(trifluoromethyl)phenyl)benzamide;
N-(4-((((2-(diethylamino)ethyl)amino)carbonyl)amino)-3-(trifluoromethyl)phenyl)-4-methyl-3-(2-(methylamino)pyrido[2,3-d]pyrimidin-6-yl)benzamide;
3-(2-amino-6-quinazolinyl)-4-methyl-N-(3-(((2-(4-morpholinyl)ethyl)amino)carbonyl)-5-(trifluoromethyl)phenyl)benzamide;
4-methyl-3-(2-(methylamino)pyrido[2,3-d]pyrimidin-6-yl)-N-(3-(((2-(4-morpholinyl)ethyl)amino)carbonyl)-5-(trifluoromethyl)phenyl)benzamide;
3-(2-amino-6-quinazolinyl)-N-(3-(((2-(diethylamino)ethyl)amino)carbonyl)-5-(trifluoromethyl)phenyl)-4-methylbenzamide;
N-(3-(((2-(diethylamino)ethyl)amino)carbonyl)-5-(trifluoromethyl)phenyl)-4-methyl-3-(2-(methylamino)pyrido[2,3-d]pyrimidin-6-yl)benzamide;
N-(5-((diethylamino)sulfonyl)-2-(methyloxy)phenyl)-4-methyl-3-(2-(methylamino)pyrido[2,3-d]pyrimidin-6-yl)benzamide;
N-(3-(2-amino-6-quinazolinyl)-4-methylphenyl)-3,4,5-tris(methyloxy)benzamide;
N-(4-methyl-3-(2-(methylamino)pyrido[2,3-d]pyrimidin-6-yl)phenyl)-3,4,5-tris(methyloxy)benzamide;
3-fluoro-N-(4-methyl-3-(2-(methylamino)pyrido[2,3-d]pyrimidin-6-yl)phenyl)-5-(trifluoromethyl)benzamide;
3-(2-amino-6-quinazolinyl)-4-methyl-N-(3-((4-methyl-1-piperazinyl)carbonyl)-5-(trifluoromethyl)phenyl)benzamide;
3-(2-amino-6-quinazolinyl)-4-methyl-N-(3-((4-methyl-1-piperazinyl)methyl)-5-(trifluoromethyl)phenyl)benzamide;
N-(3-(2-amino-6-quinazolinyl)-2-methylphenyl)-3-(trifluoromethyl)benzamide;
N-(5-(1,1-dimethylethyl)-2-((4-morpholinylacetyl)amino)phenyl)-4-methyl-3-(2-(methylamino)pyrido[2,3-d]pyrimidin-6-yl)benzamide;
3-(2-amino-6-quinazolinyl)-N-(5-(1,1-dimethylethyl)-2-((4-morpholinylacetyl)amino)phenyl)-4-methylbenzamide;
N-(3-(2-amino-6-quinazolinyl)-2-methylphenyl)-3-fluoro-5-(trifluoromethyl)benzamide;
3-fluoro-N-(2-methyl-3-(2-(methylamino)pyrido[2,3-d]pyrimidin-6-yl)phenyl)-5-(trifluoromethyl)benzamide;
N-(3-(2-amino-6-quinazolinyl)-2-methylphenyl)-3,5-bis(trifluoromethyl)benzamide;
N-(2-methyl-3-(2-(methylamino)pyrido[2,3-d]pyrimidin-6-yl)phenyl)-3,5-bis(trifluoromethyl)benzamide;
N-(3-(2-amino-6-quinazolinyl)-2-methylphenyl)-2-fluoro-5-(trifluoromethyl)benzamide;
2-fluoro-N-(2-methyl-3-(2-(methylamino)pyrido[2,3-d]pyrimidin-6-yl)phenyl)-5-(trifluoromethyl)benzamide;
N-(3-(2-amino-6-quinazolinyl)-2,4,6-trimethylphenyl)-3-(trifluoromethyl)benzamide;
4-((3-(2-amino-6-quinazolinyl)-4-methylphenyl)carbonyl)-6-(1,1-dimethylethyl)-3,4-dihydro-2(1H)-quinoxalinone;
N-(4-(2-amino-6-quinazolinyl)phenyl)-N'-phenylurea;
N-(4-(4-amino-6-quinazolinyl)phenyl)-3-(trifluoromethyl)benzamide;
N-(4-(2-((4-(4-methyl-1-piperazinyl)phenyl)amino)-6-quinazolinyl)phenyl)-3-(trifluoromethyl)benzamide;
4-(2-((4-(4-methyl-1-piperazinyl)phenyl)amino)-6-quinazolinyl)-N-(3-(trifluoromethyl)phenyl)benzamide;
N-(4-(2-((3-(4-morpholinyl)propyl)amino)-6-quinazolinyl)phenyl)-3-(trifluoromethyl)benzamide;
4-(2-((3-(4-morpholinyl)propyl)amino)-6-quinazolinyl)-N-(3-(trifluoromethyl)phenyl)benzamide;
N-(4-(2-((3-(diethylamino)propyl)amino)-6-quinazolinyl)phenyl)-3-(trifluoromethyl)benzamide;
4-(2-((3-(diethylamino)propyl)amino)-6-quinazolinyl)-N-(3-(trifluoromethyl)phenyl)benzamide;
N-cyclopropyl-4-(2-((4-(4-methyl-1-piperazinyl)phenyl)amino)-6-quinazolinyl)benzamide;
4-(2-((4-(4-methyl-1-piperazinyl)phenyl)amino)-6-quinazolinyl)-N-phenylbenzamide;
N-(4-chlorophenyl)-4-(2-((4-(4-methyl-1-piperazinyl)phenyl)amino)-6-quinazolinyl)benzamide;
N-(4-(2-(methylamino)-6-quinazolinyl)phenyl)-2-(phenylamino)benzamide;
N-(4-(4-amino-6-quinazolinyl)phenyl)-2-(phenylamino)benzamide;
6-methyl-5-(2-(methylamino)-6-quinazolinyl)-1(2H)-isoquinolinone;
2',7'-dimethyl-2-(methylamino)-6,8'-biquinazolin-4'(3'H)-one;
2-fluoro-4-methyl-3-(2-(methylamino)-6-quinazolinyl)-N-(3-((1-methylethyl)oxy)phenyl)benzamide;
3-fluoro-4-methyl-2-(2-(methylamino)-6-quinazolinyl)-N-(3-((1-methylethyl)oxy)phenyl)benzamide;
2-fluoro-4-methyl-3-(2-(methylamino)-6-quinazolinyl)-N-(3-((1-methylethyl)oxy)phenyl)benzenecarbothioamide;
2-fluoro-4-methyl-3-(2-(methylamino)-6-quinazolinyl)-N-(4-(methyloxy)-3-(trifluoromethyl)phenyl)benzamide;
2-amino-4-methyl-3-(2-(methylamino)-6-quinazolinyl)benzoic acid;
2-amino-4-methyl-3-(2-(methylamino)-6-quinazolinyl)-N-(3-((1-methylethyl)oxy)phenyl)benzamide;
2-amino-4-methyl-3-(2-(methylamino)-6-quinazolinyl)-N-(3-(1-methylethyl)phenyl)benzamide;
2-amino-N-(4-(1,1-dimethylethyl)phenyl)-4-methyl-3-(2-(methylamino)-6-quinazolinyl)benzamide;
4-Methyl-3-(quinazolin-6-yl)-N-(3-(trifluoromethyl)phenyl)benzamide hydrochloride;
3-(2-amino-6-quinazolinyl)-N-(3-((3-(dimethylamino)propyl)oxy)-5-(trifluoromethyl)phenyl)-4-methylbenzamide;
6-(5-isothiocyanato-2-methylphenyl)-N-methyl-2-quinazolinamine;
6-(2-phenylimidazo[1,2-a]pyridin-6-yl)-2-quinazolinamine;
6-(2-(4-(methyloxy)phenyl)imidazo[1,2-a]pyridin-6-yl)-2-quinazolinamine;
6-(2-(3-(methyloxy)phenyl)imidazo[1,2-a]pyridin-6-yl)-2-quinazolinamine;
6-(2-(2-(methyloxy)phenyl)imidazo[1,2-a]pyridin-6-yl)-2-quinazolinamine;
6-(2-(1,3-benzodioxol-5-yl)imidazo[1,2-a]pyridin-6-yl)-2-quinazolinamine;
6-(2-(3-thienyl)imidazo[1,2-a]pyridin-6-yl)-2-quinazolinamine;
6-(2-ethyl-1-benzofuran-5-yl)-2-quinazolinamine;
6-(2-(1,1-dimethylethyl)imidazo[1,2-a]pyridin-6-yl)-2-quinazolinamine;
3-(2-amino-6-quinazolinyl)-N-(3-(3-(dimethylamino)propyl)-5-(trifluoromethyl)phenyl)-4-methylbenzamide;
N-(4-(diethylnitroryl)-1-methylbutyl)-6-(4-methyl-3-pyridinyl)-2-quinazolinamine;
methyl 4-methyl-3-(6-quinazolinyl)benzoate;
4-methyl-3-(6-quinazolinyl)benzoic acid;
N-cyclopropyl-4-methyl-3-(2-((((2R)-1-(2,2,2-trifluoroethyl)-2-pyrrolidinyl)methyl)amino)-6-quinazolinyl)benzamide;
3-(2-amino-6-quinazolinyl)-N-(3-((N,N-diethylglycyl)amino)-4-(1,1-dimethylethyl)phenyl)-4-methylbenzamide;
3-(2-amino-6-quinazolinyl)-N-(4-(1,1-dimethylethyl)-3-((1-pyrrolidinylacetyl)amino)phenyl)-4-methylbenzamide;
N-(1-(N,N-diethylglycyl)-3,3-dimethyl-2,3-dihydro-1H-indol-6-yl)-4-methyl-3-(2-(methylamino)-6-quinazolinyl)benzamide;
3-(2-(cyclopropylamino)-6-quinazolinyl)-N-(1-(N,N-diethylglycyl)-3,3-dimethyl-2,3-dihydro-1H-indol-6-yl)-4-methylbenzamide;
N-(3-((N,N-diethylglycyl)amino)-4-(1-piperidinyl)phenyl)-4-methyl-3-(2-(methylamino)-6-quinazolinyl)benzamide;
N-(4-chloro-3-(((1-methyl-4-piperidinyl)methyl)oxy)phenyl)-4-methyl-3-(2-(methylamino)-6-quinazolinyl)benzamide;
N-(3-fluoro-4-((3-(1-piperidinyl)propyl)oxy)phenyl)-4-methyl-3-(2-(methylamino)-6-quinazolinyl)benzamide;
N-(3-((N,N-diethylglycyl)amino)-4-methylphenyl)-4-methyl-3-(2-(methylamino)-6-quinazolinyl)benzamide;
N-(3-((N,N-diethylglycyl)amino)-4-fluorophenyl)-4-methyl-3-(2-(methylamino)-6-quinazolinyl)benzamide;
N-(2-((diethylamino)methyl)-4,5-bis(methyloxy)phenyl)-4-methyl-3-(2-(methylamino)-6-quinazolinyl)benzamide;
N-(2-((diethylamino)methyl)-4,5-bis(methyloxy)phenyl)-4-methyl-3-(2-((3-(4-morpholinyl)propyl)amino)-6-quinazolinyl)benzamide;
N-(3-fluoro-4-(((1-methyl-3-piperidinyl)methyl)oxy)phenyl)-4-methyl-3-(2-(methylamino)-6-quinazolinyl)benzamide;
4-methyl-3-(2-(methylamino)-6-quinazolinyl)-N-(3-(((methylsulfonyl)amino)methyl)phenyl)benzamide;
4-methyl-N-(3-(((methylsulfonyl)amino)methyl)phenyl)-3-(2-((3-(4-morpholinyl)propyl)amino)-6-quinazolinyl)benzamide;
N-(4-fluoro-3-(((methyloxy)acetyl)amino)phenyl)-4-methyl-3-(2-(methylamino)-6-quinazolinyl)benzamide;
3-(2-((3-(diethylamino)propyl)amino)-6-quinazolinyl)-N-(4-fluoro-3-(((methyloxy)acetyl)amino)phenyl)-4-methylbenzamide;
4-methyl-3-(2-(methylamino)-6-quinazolinyl)-N-(2-((4-methyl-1-piperazinyl)methyl)-5-(trifluoromethyl)phenyl)benzamide;
4-methyl-3-(2-(methylamino)-6-quinazolinyl)-N-(3-((((2S)-1-methyl-2-pyrrolidinyl)methyl)oxy)-4-(pentafluoroethyl)phenyl)benzamide;
N-(4-chloro-3-((((2S)-1-methyl-2-pyrrolidinyl)methyl)oxy)phenyl)-4-methyl-3-(2-(methylamino)-6-quinazolinyl)benzamide;
ethyl 3,3-dimethyl-6-(((4-methyl-3-(2-(methylamino)-6-quinazolinyl)phenyl)carbonyl)amino)-2,3-dihydro-1H-indole-1-carboxylate;
N-(3-((diethylamino)methyl)-4-(methyloxy)phenyl)-4-methyl-3-(2-(methylamino)-6-quinazolinyl)benzamide;
ethyl 6-(((3-(2-((3-(diethylamino)propyl)amino)-6-quinazolinyl)-4-methylphenyl)carbonyl)amino)-3,3-dimethyl-2,3-dihydro-1H-indole-1-carboxylate;
4-methyl-3-(2-(methylamino)-6-quinazolinyl)-N-(3-(2-oxo-1-pyrrolidinyl)-5-(trifluoromethyl)phenyl)benzamide;
4-methyl-3-(2-(methylamino)-6-quinazolinyl)-N-(2-(1-piperidinyl)-5-(trifluoromethyl)phenyl)benzamide;
4-methyl-3-(2-(methylamino)-6-quinazolinyl)-N-(1-methyl-4-piperidinyl)benzamide;
N-(2-((2,2-dimethylpropanoyl)amino)-5-(trifluoromethyl)phenyl)-4-methyl-3-(2-(methylamino)-6-quinazolinyl)benzamide;
4-methyl-3-(2-(methylamino)-6-quinazolinyl)-N-(2-(methyl(1-methyl-4-piperidinyl)amino)-5-(trifluoromethyl)phenyl)benzamide;
4-methyl-3-(2-(methylamino)-6-quinazolinyl)-N-(3-(2-methyl-1-pyrrolidinyl)-2-pyridinyl)benzamide;
N-(2,2-difluoro-1,3-benzodioxol-4-yl)-4-methyl-3-(2-((3-(4-morpholinyl)propyl)amino)-6-quinazolinyl)benzamide;
N-(2,2-difluoro-1,3-benzodioxol-4-yl)-4-methyl-3-(2-((1-methyl-4-piperidinyl)amino)-6-quinazolinyl)benzamide;
N-(2,2-difluoro-1,3-benzodioxol-4-yl)-4-methyl-3-(2-((2-(1-piperidinyl)ethyl)amino)-6-quinazolinyl)benzamide;
N-(2-((3-(dimethylamino)propyl)oxy)-5-(trifluoromethyl)phenyl)-4-methyl-3-(2-(methylamino)-6-quinazolinyl)benzamide;
4-methyl-3-(2-((3-(methyloxy)propyl)amino)-6-quinazolinyl)-N-(2-methyl-3-(trifluoromethyl)phenyl)benzamide;
4-methyl-N-(2-methyl-3-(trifluoromethyl)phenyl)-3-(2-(phenylamino)-6-quinazolinyl)benzamide;
N-(1-acetyl-3,3-dimethyl-2,3-dihydro-1H-indol-6-yl)-4-methyl-3-(2-((2-(4-morpholinyl)ethyl)amino)-6-quinazolinyl)benzamide;
N-(1-acetyl-3,3-dimethyl-2,3-dihydro-1H-indol-6-yl)-4-methyl-3-(2-((2-(1-pyrrolidinyl)ethyl)amino)-6-quinazolinyl)benzamide;
N-(1-acetyl-3,3-dimethyl-2,3-dihydro-1H-indol-6-yl)-3-(2-(((1-ethyl-4-piperidinyl)methyl)amino)-6-quinazolinyl)-4-methylbenzamide;
N-(1-acetyl-3,3-dimethyl-2,3-dihydro-1H-indol-6-yl)-4-methyl-3-(2-((1-methyl-4-piperidinyl)amino)-6-quinazolinyl)benzamide;
N-(1-acetyl-3,3-dimethyl-2,3-dihydro-1H-indol-6-yl)-3-(2-(cyclopropylamino)-6-quinazolinyl)-4-methylbenzamide;
N-(1-acetyl-3,3-dimethyl-2,3-dihydro-1H-indol-6-yl)-4-methyl-3-(2-((3-(2-oxo-1-pyrrolidinyl)propyl)amino)-6-quinazolinyl)benzamide;
N-(1-acetyl-3,3-dimethyl-2,3-dihydro-1H-indol-6-yl)-4-methyl-3-(2-(methylamino)-6-quinazolinyl)benzamide;
N-(1-acetyl-3,3-dimethyl-2,3-dihydro-1H-indol-6-yl)-4-methyl-3-(2-((3-(4-morpholinyl)propyl)amino)-6-quinazolinyl)benzamide;
4-methyl-3-(2-(methyl(3-(4-morpholinyl)propyl)amino)-6-quinazolinyl)-N-(2-methyl-3-(trifluoromethyl)phenyl)benzamide;
2-fluoro-N-(2-methyl-3-(2-(methylamino)-6-quinazolinyl)phenyl)-5-(trifluoromethyl)benzamide;
4-methyl-3-(2-((1E)-3-(methyloxy)-1-propenyl)-6-quinazolinyl)-N-(3-(trifluoromethyl)phenyl)benzamide;
4-methyl-3-(2-((1E)-3-(methyloxy)-1-propenyl)-6-quinazolinyl)-N-(2-methyl-3-(trifluoromethyl)phenyl)benzamide;
6-(2,6-dichlorophenyl)-N-(3-(4-morpholinyl)propyl)-2-quinazolinamine;
4-methyl-3-(2-(methylamino)-6-quinazolinyl)benzamide;
3-(2-(cyclopropylamino)-6-quinazolinyl)-4-methylbenzamide;
4-methyl-3-(2-((3-(4-morpholinyl)propyl)amino)-6-quinazolinyl)benzamide;
3-(2-((3-(diethylamino)propyl)amino)-6-quinazolinyl)-4-methylbenzamide;
3-(2-((3-fluoro-4-((4-(1H-imidazol-1-yl)butyl)oxy)phenyl)amino)-6-quinazolinyl)-4-methylbenzamide;
4-methyl-3-(2-((4-((3-(1-piperidinyl)propyl)oxy)phenyl)amino)-6-quinazolinyl)benzamide;
6-(2,6-dimethylphenyl)-N-(3-morpholinopropyl)quinazolin-2-amine;
N-(2-fluoro-5-(trifluoromethyl)phenyl)-4-methyl-3-(2-((2-(4-morpholinyl)ethyl)amino)-6-quinazolinyl)benzamide;
N-(2-fluoro-5-(trifluoromethyl)phenyl)-4-methyl-3-(2-((3-(4-morpholinyl)propyl)amino)-6-quinazolinyl)benzamide;
N-(2-fluoro-5-(trifluoromethyl)phenyl)-4-methyl-3-(2-((2-(1-piperidinyl)ethyl)amino)-6-quinazolinyl)benzamide;
N-(2-fluoro-5-(trifluoromethyl)phenyl)-4-methyl-3-(2-((2-(1-pyrrolidinyl)ethyl)amino)-6-quinazolinyl)benzamide;
3-(2-((3-(diethylamino)propyl)amino)-6-quinazolinyl)-4-methyl-N-(2-methyl-3-(trifluoromethyl)phenyl)benzamide;
4-methyl-N-(2-methyl-3-(trifluoromethyl)phenyl)-3-(2-((2-(1-(phenylmethyl)-4-piperidinyl)ethyl)amino)-6-quinazolinyl)benzamide;
1,1-dimethylethyl 4-(2-((6-(2-methyl-5-(((2-methyl-3-(trifluoromethyl)phenyl)amino)carbonyl)phenyl)-2-quinazolinyl)amino)ethyl)-1-piperazinecarboxylate;
3-(2-((((2R)-1-ethyl-2-pyrrolidinyl)methyl)amino)-6-quinazolinyl)-4-methyl-N-(2-methyl-3-(trifluoromethyl)phenyl)benzamide;
4-methyl-N-(2-methyl-3-(trifluoromethyl)phenyl)-3-(2-((2-(1-piperidinyl)ethyl)amino)-6-quinazolinyl)benzamide;
3-(2-(1,3-benzoxazol-2-ylamino)-6-quinazolinyl)-4-methyl-N-(2-methyl-3-(trifluoromethyl)phenyl)benzamide;
4-methyl-N-(2-methyl-3-(trifluoromethyl)phenyl)-3-(2-((2-(1-piperazinyl)ethyl)amino)-6-quinazolinyl)benzamide;
4-methyl-3-(2-((2-(1-pyrrolidinyl)ethyl)amino)-6-quinazolinyl)-N-(3-(trifluoromethyl)phenyl)benzamide;
N-(4-chloro-3-(trifluoromethyl)phenyl)-4-methyl-3-(2-((2-(1-pyrrolidinyl)ethyl)amino)-6-quinazolinyl)benzamide;
4-methyl-N-(3-methyl-4-(1-methylethyl)phenyl)-3-(2-((2-(1-pyrrolidinyl)ethyl)amino)-6-quinazolinyl)benzamide;
N-(4-fluoro-3-(trifluoromethyl)phenyl)-4-methyl-3-(2-((2-(1-pyrrolidinyl)ethyl)amino)-6-quinazolinyl)benzamide;
N-(4-chloro-3-(trifluoromethyl)phenyl)-4-methyl-3-(2-((1-methyl-4-piperidinyl)amino)-6-quinazolinyl)benzamide;
N-(4-fluoro-3-(trifluoromethyl)phenyl)-4-methyl-3-(2-((1-methyl-4-piperidinyl)amino)-6-quinazolinyl)benzamide;
4-methyl-N-(3-methyl-4-(1-methylethyl)phenyl)-3-(2-((1-methyl-4-piperidinyl)amino)-6-quinazolinyl)benzamide;
N-(4-chloro-3-(trifluoromethyl)phenyl)-4-methyl-3-(2-((2-(1-piperidinyl)ethyl)amino)-6-quinazolinyl)benzamide;
N-(4-fluoro-3-(trifluoromethyl)phenyl)-4-methyl-3-(2-((2-(1-piperidinyl)ethyl)amino)-6-quinazolinyl)benzamide;
4-methyl-N-(3-methyl-4-(1-methylethyl)phenyl)-3-(2-((2-(1-piperidinyl)ethyl)amino)-6-quinazolinyl)benzamide;
N-(1-acetyl-3,3-dimethyl-2,3-dihydro-1H-indol-6-yl)-4-methyl-3-(2-((2-(1-piperidinyl)ethyl)amino)-6-quinazolinyl)benzamide;
4-methyl-N-(2-methyl-3-(trifluoromethyl)phenyl)-3-(2-((4-((3-(1-piperidinyl)propyl)oxy)phenyl)amino)-6-quinazolinyl)benzamide;
4-methyl-3-(2-((3-(4-methyl-1-piperazinyl)phenyl)amino)-6-quinazolinyl)-N-(2-methyl-3-(trifluoromethyl)phenyl)benzamide;
N-(2-fluoro-3-(trifluoromethyl)phenyl)-4-methyl-3-(2-((2-(1-piperidinyl)ethyl)amino)-6-quinazolinyl)benzamide;
N-(2,3-dihydro-1H-inden-4-yl)-4-methyl-3-(2-((3-(4-morpholinyl)propyl)amino)-6-quinazolinyl)benzamide;
3-(2-((3-(diethylamino)propyl)amino)-6-quinazolinyl)-N-(2,3-dihydro-1H-inden-4-yl)-4-methylbenzamide;
N-(2,3-dihydro-1H-inden-4-yl)-4-methyl-3-(2-(methylamino)-6-quinazolinyl)benzamide;
N-(2,3-dihydro-1H-inden-4-yl)-4-methyl-3-(2-((4-((3-(1-piperidinyl)propyl)oxy)phenyl)amino)-6-quinazolinyl)benzamide;
3-(2-(((1S)-4-(diethylamino)-1-methylbutyl)amino)-6-quinazolinyl)-4-methyl-N-(2-methyl-3-(trifluoromethyl)phenyl)benzamide;
6-(5-amino-2-methylphenyl)-N-(4-(4-methyl-1-piperazinyl)phenyl)-2-quinazolinamine;
2-methyl-N-(4-methyl-3-(2-((4-(4-methyl-1-piperazinyl)phenyl)amino)-6-quinazolinyl)phenyl)-3-(trifluoromethyl)benzamide;
4-(1,1-dimethylethyl)-N-(4-methyl-3-(2-((4-(4-methyl-1-piperazinyl)phenyl)amino)-6-quinazolinyl)phenyl)benzamide;
N-(4-methyl-3-(2-((4-(4-methyl-1-piperazinyl)phenyl)amino)-6-quinazolinyl)phenyl)acetamide;
N-(4-methyl-3-(2-((4-(4-methyl-1-piperazinyl)phenyl)amino)-6-quinazolinyl)phenyl)-3,5-bis(methyloxy)benzamide;
2-fluoro-N-(4-methyl-3-(2-((4-(4-methyl-1-piperazinyl)phenyl)amino)-6-quinazolinyl)phenyl)-3-(trifluoromethyl)benzamide;
2,2-difluoro-N-(4-methyl-3-(2-((4-(4-methyl-1-piperazinyl)phenyl)amino)-6-quinazolinyl)phenyl)-1,3-benzodioxole-5-carboxamide;
N-(4-methyl-3-(2-((4-(4-methyl-1-piperazinyl)phenyl)amino)-6-quinazolinyl)phenyl)-1-naphthalenecarboxamide;
3-(1,1-dimethylethyl)-1-methyl-N-(4-methyl-3-(2-((4-(4-methyl-1-piperazinyl)phenyl)amino)-6-quinazolinyl)phenyl)-1H-pyrazole-4-carboxamide;
6-(5-(1H-benzimidazol-2-ylamino)-2-methylphenyl)-N-(4-(4-methyl-1-piperazinyl)phenyl)-2-quinazolinamine;
N-(4-(4-methyl-1-piperazinyl)phenyl)-6-(2-methyl-5-((5-(trifluoromethyl)-1H-benzimidazol-2-yl)amino)phenyl)-2-quinazolinamine;
N-(2-(2-(dimethylamino)ethyl)-5-(trifluoromethyl)phenyl)-4-methyl-3-(2-(methylamino)-6-quinazolinyl)benzamide;
N-ethyl-N'-(4-methyl-3-(2-((4-(4-methyl-1-piperazinyl)phenyl)amino)-6-quinazolinyl)phenyl)urea;
3-(2-((3-hydroxypropyl)amino)-6-quinazolinyl)-4-methyl-N-(2-methyl-3-(trifluoromethyl)phenyl)benzamide;
4-methyl-N-(2-methyl-3-(trifluoromethyl)phenyl)-3-(2-(3-pyridinylamino)-6-quinazolinyl)benzamide;
N-methyl-6-(2-methyl-5-((5-(trifluoromethyl)-1H-benzimidazol-2-yl)amino)phenyl)-2-quinazolinamine;
6-(5-(1H-benzimidazol-2-ylamino)-2-methylphenyl)-N-methyl-2-quinazolinamine;
6-(5-((1-(2-(dimethylamino)ethyl)-1H-benzimidazol-2-yl)amino)-2-methylphenyl)-N-methyl-2-quinazolinamine;
6-(5-((1-(3-(dimethylamino)propyl)-1H-benzimidazol-2-yl)amino)-2-methylphenyl)-N-methyl-2-quinazolinamine;
6-(5-((7-chloro-5-(trifluoromethyl)-1H-benzimidazol-2-yl)amino)-2-methylphenyl)-N-methyl-2-quinazolinamine;
6-(5-(1H-imidazo[4,5-b]pyridin-2-ylamino)-2-methylphenyl)-N-methyl-2-quinazolinamine;
N-methyl-6-(2-methyl-5-((1-methyl-5-(trifluoromethyl)-1H-benzimidazol-2-yl)amino)phenyl)-2-quinazolinamine;
6-(5-((5,6-difluoro-1H-benzimidazol-2-yl)amino)-2-methylphenyl)-N-methyl-2-quinazolinamine;
N-methyl-6-(2-methyl-5-((6-(4-methyl-1-piperazinyl)-1H-benzimidazol-2-yl)amino)phenyl)-2-quinazolinamine;
N-methyl-6-(6-methyl-5-phthalazinyl)-2-quinazolinamine;
6-methyl-5-(2-(methylamino)-6-quinazolinyl)-1-phthalazinol;
N-(4-chloro-3-(2-(methylamino)-6-quinazolinyl)phenyl)-3-(trifluoromethyl)benzamide;
N-(4-methyl-3-(2-(methylamino)-6-quinazolinyl)phenyl)-3-(trifluoromethyl)benzamide;
N-(4-chloro-3-(2-(methylamino)-6-quinazolinyl)phenyl)-2-fluoro-5-(trifluoromethyl)benzamide;
N-(4-chloro-3-(2-(methylamino)-6-quinazolinyl)phenyl)-2-methyl-3-(trifluoromethyl)benzamide;
3-(2-amino-6-quinazolinyl)-N-(2,6-dimethylphenyl)-4-methylbenzamide;
3-(2-amino-6-quinazolinyl)-N-(2-(ethyloxy)-5-(trifluoromethyl)phenyl)-4-methylbenzamide;
3-(2-amino-6-quinazolinyl)-4-methyl-N-(2-(methyloxy)-5-(trifluoromethyl)phenyl)benzamide d_3_;
3-(2-amino-6-quinazolinyl)-4-methyl-N-(2-methyl-5-nitrophenyl)benzamide;
3-(2-amino-6-quinazolinyl)-N-(2,5-dimethylphenyl)-4-methylbenzamide;
N-(5-(aminocarbonyl)-2-methylphenyl)-3-(2-amino-6-quinazolinyl)-4-methylbenzamide;
3-(2-amino-6-quinazolinyl)-4-methyl-N-(2-(methyloxy)-5-nitrophenyl)benzamide;
3-(2-amino-6-quinazolinyl)-N-(5-cyclohexyl-2-(methyloxy)phenyl)-4-methylbenzamide;
3-(2-amino-6-quinazolinyl)-4-methyl-N-(2-methyl-5-(methyloxy)phenyl)benzamide;
4-(2-amino-6-quinazolinyl)-N-(2-fluoro-5-(trifluoromethyl)phenyl)-3-methylbenzamide;
3-(2-amino-6-quinazolinyl)-4-methyl-N-(2-(methyloxy)-5-((2-pyridinylamino)carbonyl)phenyl)benzamide;
3-(2-amino-6-quinazolinyl)-4-methyl-N-(2-(methyloxy)-5-((3-pyridinylamino)carbonyl)phenyl)benzamide;
3-(2-amino-6-quinazolinyl)-4-methyl-N-(2-(methyloxy)-5-((4-pyridinylamino)carbonyl)phenyl)benzamide;
4-(2-amino-6-quinazolinyl)-3-methyl-N-(3-((4-methyl-1-piperazinyl)methyl)-5-(trifluoromethyl)phenyl)benzamide;
4-methyl-3-(2-(methylamino)-6-quinazolinyl)-N-(2-((2-(1-pyrrolidinyl)ethyl)oxy)-5-(trifluoromethyl)phenyl)benzamide;
N-(4-methyl-3-(2-(methylamino)-6-quinazolinyl)phenyl)-2-((2-(1-pyrrolidinyl)ethyl)amino)-5-(trifluoromethyl)benzamide;
3-(2-amino-6-quinazolinyl)-N-(2-chloro-4-(1,1-dimethylethyl)phenyl)-4-methylbenzamide;
3-(2-amino-6-quinazolinyl)-N-(2-chloro-5-(trifluoromethyl)phenyl)-4-methylbenzamide;
3-(2-amino-6-quinazolinyl)-N-(2-bromo-5-(trifluoromethyl)phenyl)-4-methylbenzamide;
3-(2-amino-6-quinazolinyl)-4-methyl-N-(4-(1-methylethyl)-2-(trifluoromethyl)phenyl)benzamide;
3-(2-amino-6-quinazolinyl)-N-(2-(1,1-dimethylethyl)imidazo[1,2-a]pyridin-6-yl)-4-methylbenzamide;
4-methyl-3-(2-(methylamino)-6-quinazolinyl)-N-(4-((1-methyl-4-piperidinyl)oxy)phenyl)benzamide;
3-(2-(cyclopropylamino)-6-quinazolinyl)-4-methyl-N-(4-((1-methyl-4-piperidinyl)oxy)phenyl)benzamide;
3-(2-amino-6-quinazolinyl)-4-methyl-N-(3-methyl-4-((1-methyl-4-piperidinyl)oxy)phenyl)benzamide;
4-methyl-3-(2-(methylamino)-6-quinazolinyl)-N-(3-methyl-4-((1-methyl-4-piperidinyl)oxy)phenyl)benzamide;
3-(2-(cyclopropylamino)-6-quinazolinyl)-4-methyl-N-(3-methyl-4-((1-methyl-4-piperidinyl)oxy)phenyl)benzamide;
3-(2-amino-6-quinazolinyl)-N-(4-((1-cyclopropylethyl)oxy)phenyl)-4-methylbenzamide;
N-(4-((1-cyclopropylethyl)oxy)phenyl)-4-methyl-3-(2-(methylamino)-6-quinazolinyl)benzamide;
N-(4-((1-cyclopropylethyl)oxy)-3-methylphenyl)-4-methyl-3-(2-(methylamino)-6-quinazolinyl)benzamide;
3-(2-amino-6-quinazolinyl)-N-(4-((1-cyclopropylethyl)oxy)-3-methylphenyl)-4-methylbenzamide;
4-methyl-3-(2-(methylamino)-6-quinazolinyl)-N-(4-(1-methylethyl)phenyl)benzamide;
3-(2-(cyclopropylamino)-6-quinazolinyl)-4-methyl-N-(4-(1-methylethyl)phenyl)benzamide;
4-methyl-N-(4-(1-methylethyl)phenyl)-3-(2-((1-methyl-4-piperidinyl)amino)-6-quinazolinyl)benzamide;
4-methyl-3-(2-(methylamino)-6-quinazolinyl)-N-(2-((1-methyl-4-piperidinyl)oxy)-5-(trifluoromethyl)phenyl)benzamide;
N-(4-((cyclopropylmethyl)oxy)phenyl)-4-methyl-3-(2-(methylamino)-6-quinazolinyl)benzamide;
N-(4-((cyclopropylmethyl)oxy)phenyl)-4-methyl-3-(2-((3-(4-morpholinyl)propyl)amino)-6-quinazolinyl)benzamide;
N-(3-(acetylamino)-4-fluorophenyl)-4-methyl-3-(2-((3-(4-morpholinyl)propyl)amino)-6-quinazolinyl)benzamide;
N-(2-(((1R)-1-cyclopropylethyl)oxy)-5-(trifluoromethyl)phenyl)-4-methyl-3-(2-((3-(4-morpholinyl)propyl)amino)-6-quinazolinyl)benzamide;
N-(3-(acetylamino)-4-(((1S)-1-cyclopropylethyl)oxy)phenyl)-4-methyl-3-(2-((3-(4-morpholinyl)propyl)amino)-6-quinazolinyl)benzamide;
N-(2-(((1R)-1-cyclopropylethyl)oxy)-5-(trifluoromethyl)phenyl)-4-methyl-3-(2-(methylamino)-6-quinazolinyl)benzamide;
4-methyl-N-(4-(1-methylethyl)phenyl)-3-(2-((3-(4-morpholinyl)propyl)amino)-6-quinazolinyl)benzamide;
N-(2-(1,1-dimethylethyl)imidazo[1,2-a]pyridin-6-yl)-4-methyl-3-(2-((3-(4-morpholinyl)propyl)amino)-6-quinazolinyl)benzamide;
N-(2-(1,1-dimethylethyl)imidazo[1,2-a]pyridin-6-yl)-4-methyl-3-(2-((1-methyl-4-piperidinyl)amino)-6-quinazolinyl)benzamide;
3-(2-((3-(diethylamino)propyl)amino)-6-quinazolinyl)-N-(2-(1,1-dimethylethyl)imidazo[1,2-a]pyridin-6-yl)-4-methylbenzamide;
N-(2-(1,1-dimethylethyl)imidazo[1,2-a]pyridin-6-yl)-4-methyl-3-(2-(methylamino)-6-quinazolinyl)benzamide;
4-methyl-N-(2-(1-methylethyl)-1H-benzimidazol-6-yl)-3-(2-((3-(4-morpholinyl)propyl)amino)-6-quinazolinyl)benzamide;
3-(2-((3-(diethylamino)propyl)amino)-6-quinazolinyl)-4-methyl-N-(2-(1-methylethyl)-1H-benzimidazol-6-yl)benzamide;
4-methyl-N-(2-(1-methylethyl)-1H-benzimidazol-6-yl)-3-(2-((1-methyl-4-piperidinyl)amino)-6-quinazolinyl)benzamide;
4-methyl-3-(2-(methylamino)-6-quinazolinyl)-N-(2-(1-methylethyl)-1H-benzimidazol-6-yl)benzamide;
N-(2-((2-(diethylamino)ethyl)oxy)-5-(trifluoromethyl)phenyl)-4-methyl-3-(2-((3-(4-morpholinyl)propyl)amino)-6-quinazolinyl)benzamide;
N-(1-acetyl-2,3-dihydro-1H-indol-5-yl)-4-methyl-3-(2-((1-methyl-4-piperidinyl)amino)-6-quinazolinyl)benzamide;
N-(1-acetyl-2,3-dihydro-1H-indol-5-yl)-4-methyl-3-(2-((3-(4-morpholinyl)propyl)amino)-6-quinazolinyl)benzamide;
4-methyl-3-(2-(methylamino)-6-quinazolinyl)-N-(2-((2-(4-morpholinyl)ethyl)oxy)-5-(trifluoromethyl)phenyl)benzamide;
4-methyl-3-(2-((1-methyl-4-piperidinyl)amino)-6-quinazolinyl)-N-(2-((2-(4-morpholinyl)ethyl)oxy)-5-(trifluoromethyl)phenyl)benzamide;
4-methyl-N-(2-((2-(4-morpholinyl)ethyl)oxy)-5-(trifluoromethyl)phenyl)-3-(2-((3-(4-morpholinyl)propyl)amino)-6-quinazolinyl)benzamide;
N-methyl-6-(2-methyl-5-(5-(trifluoromethyl)-1,3-benzoxazol-2-yl)phenyl)-2-quinazolinamine;
N-(2-(1-ethylpyrrolidin-3-yloxy)-5-(trifluoromethyl)phenyl)-4-methyl-3-(2-(methylamino)quinazolin-6-yl)benzamide;
N-(2-(((3S)-1-ethyl-3-piperidinyl)oxy)-5-(trifluoromethyl)phenyl)-4-methyl-3-(2-(methylamino)-6-quinazolinyl)benzamide;
4-methyl-N-(2-((1-methyl-4-piperidinyl)oxy)-5-(trifluoromethyl)phenyl)-3-(2-((2-(1-piperidinyl)ethyl)amino)-6-quinazolinyl)benzamide;
4-methyl-N-(2-((1-methyl-4-piperidinyl)oxy)-5-(trifluoromethyl)phenyl)-3-(2-((2-(1-pyrrolidinyl)ethyl)amino)-6-quinazolinyl)benzamide;
N-(4-methyl-3-(2-(methylamino)-6-quinazolinyl)phenyl)-2-((1-methyl-4-piperidinyl)oxy)-5-(trifluoromethyl)benzamide;
2-(((3R)-1-ethyl-3-piperidinyl)oxy)-N-(4-methyl-3-(2-(methylamino)-6-quinazolinyl)phenyl)-5-(trifluoromethyl)benzamide;
2-((cyclopentylmethyl)oxy)-N-(4-methyl-3-(2-(methylamino)-6-quinazolinyl)phenyl)-5-(trifluoromethyl)benzamide;
2-((cyclopropylmethyl)oxy)-N-(4-methyl-3-(2-(methylamino)-6-quinazolinyl)phenyl)-5-(trifluoromethyl)benzamide;
2-((cyclobutylmethyl)oxy)-N-(4-methyl-3-(2-(methylamino)-6-quinazolinyl)phenyl)-5-(trifluoromethyl)benzamide;
N-(4-methyl-3-(2-(methylamino)-6-quinazolinyl)phenyl)-2-((2-(4-morpholinyl)ethyl)oxy)-5-(trifluoromethyl)benzamide;
2-((2-(diethylamino)ethyl)oxy)-N-(4-methyl-3-(2-(methylamino)-6-quinazolinyl)phenyl)-5-(trifluoromethyl)benzamide;
2-(((1S)-1-cyclopentylethyl)oxy)-N-(4-methyl-3-(2-(methylamino)-6-quinazolinyl)phenyl)-5-(trifluoromethyl)benzamide;
N-(4-methyl-3-(2-(methylamino)-6-quinazolinyl)phenyl)-2-(4-methyl-1-piperazinyl)-5-(trifluoromethyl)benzamide;
5-methyl-N-(4-methyl-3-(2-(methylamino)-6-quinazolinyl)phenyl)-2-(methyloxy)benzamide;
N-(4-methyl-3-(2-(methylamino)-6-quinazolinyl)phenyl)-2-((1-methyl-4-piperidinyl)amino)-5-(trifluoromethyl)benzamide;
5-fluoro-N-(4-methyl-3-(2-(methylamino)-6-quinazolinyl)phenyl)-2-(methyloxy)benzamide;
6-(1,6-dimethyl-1H-indazol-7-yl)-N-methyl-2-quinazolinamine;
4-methyl-3-(2-(methylamino)-6-quinazolinyl)-N-(6-(trifluoromethyl)-1,3-benzothiazol-2-yl)benzamide;
4-methyl-3-(2-((2-(1-piperidinyl)ethyl)amino)-6-quinazolinyl)-N-(6-(trifluoromethyl)-1,3-benzothiazol-2-yl)benzamide;
4-methyl-3-(2-(methylamino)-6-quinazolinyl)-N-(6-((trifluoromethyl)oxy)-1,3-benzothiazol-2-yl)benzamide;
4-methyl-3-(2-(methylamino)-6-quinazolinyl)-N-(5-(trifluoromethyl)-1,3-benzothiazol-2-yl)benzamide;
3-(2-amino-6-quinazolinyl)-N-(5-(1,1-dimethylethyl)-2-((1-pyrrolidinylacetyl)amino)phenyl)-4-methylbenzamide;
3-(2-amino-6-quinazolinyl)-N-(5-(1,1-dimethylethyl)-2-((2-(1-piperidinyl)ethyl)oxy)phenyl)-4-methylbenzamide;
3-(2-amino-6-quinazolinyl)-N-(5-(1,1-dimethylethyl)-2-((1-piperidinylacetyl)amino)phenyl)-4-methylbenzamide;
3-(2-amino-6-quinazolinyl)-N-(2-((N,N-diethylglycyl)amino)-5-(1,1-dimethylethyl)phenyl)-4-methylbenzamide;
3-(trifluoromethyl)-N-(2,4,6-trimethyl-3-(2-((3-(4-morpholinyl)propyl)amino)-6-quinazolinyl)phenyl)benzamide;
6-(2,6-dichlorophenyl)-2-quinazolinamine;
N∼1∼-(6-(2,6-dimethylphenyl)-2-quinazolinyl)-N∼4∼,N∼4∼-dimethyl-1,4-butanediamine;
6-(2-chloro-6-methylphenyl)-2-quinazolinamine;
N∼1∼-(6-(2,6-dichlorophenyl)-2-quinazolinyl)-N∼3∼,N∼3∼-diethyl-1,3-propanediamine;
N∼1∼-(6-(2,6-dimethylphenyl)-2-quinazolinyl)-N∼3∼,N∼3∼-diethyl-1,3-propanediamine;
6-(2,6-dichlorophenyl)-N-(2-(1-piperidinyl)ethyl)-2-quinazolinamine;
4-methyl-3-(2-((2-(1-piperidinyl)ethyl)amino)-6-quinazolinyl)-N-(3-(trifluoromethyl)phenyl)benzamide;
3-(2-((3-(diethylamino)propyl)amino)-6-quinazolinyl)-4-methyl-N-(3-(trifluoromethyl)phenyl)benzamide;
3-(2-((3-(dimethylamino)propyl)(methyl)amino)-6-quinazolinyl)-4-methyl-N-(3-(trifluoromethyl)phenyl)benzamide;
4-methyl-3-(2-(methyl(2-(1-piperidinyl)ethyl)amino)-6-quinazolinyl)-N-(3-(trifluoromethyl)phenyl)benzamide;
N-(2-methyl-3-(2-((2-(1-piperidinyl)ethyl)amino)-6-quinazolinyl)phenyl)-3-(trifluoromethyl)benzamide;
N∼1∼-(6-(2-chloro-6-methylphenyl)-2-quinazolinyl)-N∼3∼,N∼3∼-diethyl-1,3-propanediamine;
N∼1∼-(6-(2-chloro-6-methylphenyl)-2-quinazolinyl)-N∼2∼,N∼2∼-diethyl-1,2-ethanediamine;
6-(2-chloro-6-methylphenyl)-N-(3-(1-pyrrolidinyl)propyl)-2-quinazolinamine;
N∼1∼,N∼1∼-diethyl-N∼3∼-(6-(2-ethyl-6-methylphenyl)-2-quinazolinyl)-1,3-propanediamine;
N∼1∼-(6-(5-amino-2-methylphenyl)-2-quinazolinyl)-N∼3∼,N∼3∼-diethyl-1,3-propanediamine;
N-(3-(2-((3-(diethylamino)propyl)amino)-6-quinazolinyl)-2-methylphenyl)-3,5-bis(trifluoromethyl)benzamide;
3-(2-((3-(diethylamino)propyl)amino)pyrido[2,3-d]pyrimidin-6-yl)-4-methyl-N-(4-(trifluoromethyl)phenyl)benzamide;
3-fluoro-N-(2-methyl-3-(2-(methylamino)-6-quinazolinyl)phenyl)-5-(trifluoromethyl)benzamide;
N-(3-(2-((3-(diethylamino)propyl)amino)-6-quinazolinyl)-4-methylphenyl)-2-fluoro-5-(trifluoromethyl)benzamide;
N-(3-(2-((3-(diethylamino)propyl)amino)-6-quinazolinyl)-4-methylphenyl)-3-fluoro-5-(trifluoromethyl)benzamide;
N-(2-((N,N-diethylglycyl)amino)-5-(trifluoromethyl)phenyl)-4-methyl-3-(2-(methylamino)-6-quinazolinyl)benzamide;
3-(2-amino-6-quinazolinyl)-N-(2-((N,N-diethylglycyl)amino)-5-(trifluoromethyl)phenyl)-4-methylbenzamide;
4-methyl-3-(2-(methylamino)-6-quinazolinyl)-N-(3-((trifluoromethyl)oxy)phenyl)benzamide;
3-(2-((3-(diethylamino)propyl)amino)-6-quinazolinyl)-4-methyl-N-(3-((trifluoromethyl)oxy)phenyl)benzamide;
N-(3-(2-amino-6-quinazolinyl)-4-methylphenyl)acetamide;
N-(4-methyl-3-(2-(methylamino)-6-quinazolinyl)phenyl)acetamide;
N-(3-(2-((3-(diethylamino)propyl)amino)-6-quinazolinyl)-4-methylphenyl)acetamide;
3-(2-amino-6-quinazolinyl)-4-methyl-N-(5-methyl-2-((4-morpholinylacetyl)amino)phenyl)benzamide;
4-methyl-3-(2-(methylamino)-6-quinazolinyl)-N-(5-methyl-2-((4-morpholinylacetyl)amino)phenyl)benzamide;
3-(2-amino-6-quinazolinyl)-4-methyl-N-(5-methyl-2-((1-pyrrolidinylacetyl)amino)phenyl)benzamide;
4-methyl-3-(2-(methylamino)-6-quinazolinyl)-N-(5-methyl-2-((1-pyrrolidinylacetyl)amino)phenyl)benzamide;
3-(2-amino-6-quinazolinyl)-4-methyl-N-(5-methyl-2-((1-piperidinylacetyl)amino)phenyl)benzamide;
4-methyl-3-(2-(methylamino)-6-quinazolinyl)-N-(5-methyl-2-((1-piperidinylacetyl)amino)phenyl)benzamide;
3-(2-amino-6-quinazolinyl)-N-(2-((N,N-diethylglycyl)amino)-5-methylphenyl)-4-methylbenzamide;
N-(2-((N,N-diethylglycyl)amino)-5-methylphenyl)-4-methyl-3-(2-(methylamino)-6-quinazolinyl)benzamide;
N-(2-methyl-3-(2-((3-(1-piperidinyl)propyl)amino)-6-quinazolinyl)phenyl)-3-(trifluoromethyl)benzamide;
N-(3-(2-((3-(diethylamino)propyl)amino)-6-quinazolinyl)-2-methylphenyl)-3-(trifluoromethyl)benzamide;
4-methyl-3-(2-(methylamino)-6-quinazolinyl)-N-(2-(1-pyrrolidinylcarbonyl)-5-(trifluoromethyl)phenyl)benzamide;
3-(2-amino-6-quinazolinyl)-4-methyl-N-(2-(1-pyrrolidinylcarbonyl)-5-(trifluoromethyl)phenyl)benzamide;
4-methyl-3-(2-((2-(1-piperidinyl)ethyl)amino)-6-quinazolinyl)-N-(2-(1-pyrrolidinylcarbonyl)-5-(trifluoromethyl)phenyl)benzamide;
3-(2-amino-6-quinazolinyl)-4-methyl-N-(2-(1-pyrrolidinylmethyl)-5-(trifluoromethyl)phenyl)benzamide;
4-methyl-3-(2-(methylamino)-6-quinazolinyl)-N-(2-(1-pyrrolidinylmethyl)-5-(trifluoromethyl)phenyl)benzamide;
4-methyl-3-(2-((2-(1-piperidinyl)ethyl)amino)-6-quinazolinyl)-N-(2-(1-pyrrolidinylmethyl)-5-(trifluoromethyl)phenyl)benzamide;
N-(2-methyl-3-(2-(methylamino)-6-quinazolinyl)phenyl)-3-((1,1,2,2-tetrafluoroethyl)oxy)benzamide;
N-(2-methyl-3-(2-((2-(1-piperidinyl)ethyl)amino)-6-quinazolinyl)phenyl)-3-((1,1,2,2-tetrafluoroethyl)oxy)benzamide;
N-(2-(3,3-dimethyl-2-oxo-1-azetidinyl)-5-(trifluoromethyl)phenyl)-4-methyl-3-(2-(methylamino)-6-quinazolinyl)benzamide;
N-(2-(3,3-dimethyl-2-oxo-1-azetidinyl)-5-(trifluoromethyl)phenyl)-4-methyl-3-(2-((2-(1-piperidinyl)ethyl)amino)-6-quinazolinyl)benzamide;
N-methyl-6-(2-methyl-5-(6-(trifluoromethyl)-1H-benzimidazol-2-yl)phenyl)-2-quinazolinamine;
N-(2-methyl-3-(2-(methylamino)-6-quinazolinyl)phenyl)-3-(trifluoromethyl)benzamide;
N-(2-(acetylamino)-5-(trifluoromethyl)phenyl)-4-methyl-3-(2-((2-(1-piperidinyl)ethyl)amino)-6-quinazolinyl)benzamide;
N-(2-(acetylamino)-5-(trifluoromethyl)phenyl)-4-methyl-3-(2-((3-(1-piperidinyl)propyl)amino)-6-quinazolinyl)benzamide;
N-(2-(acetylamino)-5-(trifluoromethyl)phenyl)-3-(2-((3-(diethylamino)propyl)amino)-6-quinazolinyl)-4-methylbenzamide;
N-(4-methyl-3-(2-((2-(1-piperidinyl)ethyl)amino)-6-quinazolinyl)phenyl)-3-(trifluoromethyl)benzamide;
methyl 4-methyl-3-(2-(methylamino)-6-quinazolinyl)benzoate;
4-methyl-3-(2-(methylamino)-6-quinazolinyl)-N-(2-(((methyloxy)acetyl)amino)-5-(trifluoromethyl)phenyl)benzamide;
methyl 4-methyl-3-(2-((2-(1-piperidinyl)ethyl)amino)-6-quinazolinyl)benzoate;
4-methyl-N-(2-(((methyloxy)acetyl)amino)-5-(trifluoromethyl)phenyl)-3-(2-((2-(1-piperidinyl)ethyl)amino)-6-quinazolinyl)benzamide;
6-(5-(4-chloro-6-(trifluoromethyl)-1H-benzimidazol-2-yl)-2-methylphenyl)-N-methyl-2-quinazolinamine;
6-(5-(4,6-bis(trifluoromethyl)-1H-benzimidazol-2-yl)-2-methylphenyl)-N-methyl-2-quinazolinamine;
N-(2-(3,3-dimethyl-2-oxo-1-azetidinyl)-5-(trifluoromethyl)phenyl)-4-methyl-3-(2-((3-(4-morpholinyl)propyl)amino)-6-quinazolinyl)benzamide;
3-(2-((3-(diethylamino)propyl)amino)-6-quinazolinyl)-N-(2-(3,3-dimethyl-2-oxo-1-azetidinyl)-5-(trifluoromethyl)phenyl)-4-methylbenzamide;
N-(2-(3,3-dimethyl-2-oxo-1-azetidinyl)-5-(trifluoromethyl)phenyl)-4-methyl-3-(2-((3-(1-piperidinyl)propyl)amino)-6-quinazolinyl)benzamide;
N-(2-(acetylamino)-5-(trifluoromethyl)phenyl)-4-methyl-3-(2-(methylamino)-6-quinazolinyl)benzamide;
4-methyl-3-(2-(methylamino)-6-quinazolinyl)-N-(2-((((2-(4-morpholinyl)ethyl)amino)carbonyl)amino)-5-(trifluoromethyl)phenyl)benzamide;
4-methyl-3-(2-(methylamino)-6-quinazolinyl)-N-(4-(2-oxo-1-azetidinyl)-3-(trifluoromethyl)phenyl)benzamide;
4-methyl-3-(2-(methylamino)-6-quinazolinyl)-N-(4-(2-oxo-1-pyrrolidinyl)-3-(trifluoromethyl)phenyl)benzamide;
3-(2-((3-(diethylamino)propyl)amino)-6-quinazolinyl)-4-methyl-N-(4-(2-oxo-1-azetidinyl)-3-(trifluoromethyl)phenyl)benzamide;
3-(2-((3-(diethylamino)propyl)amino)-6-quinazolinyl)-4-methyl-N-(4-(2-oxo-1-pyrrolidinyl)-3-(trifluoromethyl)phenyl)benzamide;
4-methyl-3-(2-(methylamino)-6-quinazolinyl)-N-(2-(2-oxo-1-pyrrolidinyl)-5-(trifluoromethyl)phenyl)benzamide;
3-(2-((3-(diethylamino)propyl)amino)-6-quinazolinyl)-4-methyl-N-(2-(2-oxo-1-pyrrolidinyl)-5-(trifluoromethyl)phenyl)benzamide;
4-methyl-3-(2-(methylamino)-6-quinazolinyl)-N-(2-(2-oxo-1-azetidinyl)-5-(trifluoromethyl)phenyl)benzamide;
N-(2-(3-(1,1-dimethylethyl)-2-oxo-1-imidazolidinyl)-5-(trifluoromethyl)phenyl)-4-methyl-3-(2-(methylamino)-6-quinazolinyl)benzamide;
4-methyl-3-(2-(methylamino)-6-quinazolinyl)-N-(5-(trifluoromethyl)-2-(3,4,4-trimethyl-2,5-dioxo-1-imidazolidinyl)phenyl)benzamide;
3-(2-((3-(diethylamino)propyl)amino)-6-quinazolinyl)-4-methyl-N-(5-(trifluoromethyl)-2-(3,4,4-trimethyl-2,5-dioxo-1-imidazolidinyl)phenyl)benzamide;
3-(2-((3-(diethylamino)propyl)amino)-6-quinazolinyl)-N-(2-(3-(1,1-dimethylethyl)-2-oxo-1-imidazolidinyl)-5-(trifluoromethyl)phenyl)-4-methylbenzamide;
3-(2-((3-(diethylamino)propyl)amino)-6-quinazolinyl)-4-methyl-N-(2-(2-oxo-1-azetidinyl)-5-(trifluoromethyl)phenyl)benzamide;
4-methyl-N-(2-(2-oxo-1-azetidinyl)-5-(trifluoromethyl)phenyl)-3-(2-((2-(1-piperidinyl)ethyl)amino)-6-quinazolinyl)benzamide;
N-(2-(3-(1,1-dimethylethyl)-2-oxo-1-imidazolidinyl)-5-(trifluoromethyl)phenyl)-4-methyl-3-(2-((2-(1-piperidinyl)ethyl)amino)-6-quinazolinyl)benzamide;
4-methyl-3-(2-((2-(1-piperidinyl)ethyl)amino)-6-quinazolinyl)-N-(5-(trifluoromethyl)-2-(3,4,4-trimethyl-2,5-dioxo-1-imidazolidinyl)phenyl)benzamide;
4-methyl-3-(2-(methylamino)-6-quinazolinyl)-N-(2-(3-methyl-2-oxo-1-imidazolidinyl)-5-(trifluoromethyl)phenyl)benzamide;
4-methyl-N-(2-(3-methyl-2-oxo-1-imidazolidinyl)-5-(trifluoromethyl)phenyl)-3-(2-((1-methyl-4-piperidinyl)amino)-6-quinazolinyl)benzamide;
4-methyl-N-(2-(3-methyl-2-oxo-1-imidazolidinyl)-5-(trifluoromethyl)phenyl)-3-(2-((2-(1-piperidinyl)ethyl)amino)-6-quinazolinyl)benzamide;
3-(2-((3-(diethylamino)propyl)amino)-6-quinazolinyl)-4-methyl-N-(2-(3-methyl-2-oxo-1-imidazolidinyl)-5-(trifluoromethyl)phenyl)benzamide;
N-(2-((4-(2-(dimethylamino)ethyl)phenyl)oxy)-5-(trifluoromethyl)phenyl)-4-methyl-3-(2-(methylamino)-6-quinazolinyl)benzamide;
6-(5-(1H-benzimidazol-2-yl)-2-methylphenyl)-N-methyl-2-quinazolinamine;
6-(5-(1H-benzimidazol-2-yl)-2-methylphenyl)-N-(3-(4-morpholinyl)propyl)-2-quinazolinamine;
6-(2-methyl-5-(6-(trifluoromethyl)-1H-benzimidazol-2-yl)phenyl)-N-(3-(4-morpholinyl)propyl)-2-quinazolinamine;
N,N-diethyl-N'-(6-(2-methyl-5-(6-(trifluoromethyl)-1H-benzimidazol-2-yl)phenyl)-2-quinazolinyl)-1,3-propanediamine;
6-(2-methyl-5-(6-(trifluoromethyl)-1H-benzimidazol-2-yl)phenyl)-N-(2-(1-pyrrolidinyl)ethyl)-2-quinazolinamine;
4-methyl-3-(2-(methylamino)-6-quinazolinyl)-N-(2-(3-oxo-1-piperazinyl)-5-(trifluoromethyl)phenyl)benzamide;
N-(2-((3-(dimethylamino)phenyl)oxy)-5-(trifluoromethyl)phenyl)-4-methyl-3-(2-(methylamino)-6-quinazolinyl)benzamide;
4-methyl-3-(2-((3-(4-morpholinyl)propyl)amino)-6-quinazolinyl)-N-(2-(3-oxo-1-piperazinyl)-5-(trifluoromethyl)phenyl)benzamide;
4-methyl-3-(2-(methylamino)-6-quinazolinyl)benzoic acid;
N-(2-(1-azetidinyl)-5-(trifluoromethyl)phenyl)-4-methyl-3-(2-(methylamino)-6-quinazolinyl)benzamide;
N-(2-(1-azetidinyl)-5-(trifluoromethyl)phenyl)-4-methyl-3-(2-((3-(4-morpholinyl)propyl)amino)-6-quinazolinyl)benzamide;
N-(2-(1-azetidinyl)-5-(trifluoromethyl)phenyl)-4-methyl-3-(2-((2-(1-pyrrolidinyl)ethyl)amino)-6-quinazolinyl)benzamide;
N-(2-(1-azetidinyl)-5-(trifluoromethyl)phenyl)-4-methyl-3-(2-((2-(1-piperidinyl)ethyl)amino)-6-quinazolinyl)benzamide;
4-(2-amino-6-quinazolinyl)-N-cyclopropyl-1H-indole-6-carboxamide;
5-(2-amino-6-quinazolinyl)-N-cyclopropyl-2-fluoro-4-methylbenzamide;
N-cyclopropyl-2-fluoro-4-methyl-5-(2-((2-(4-morpholinyl)ethyl)amino)-6-quinazolinyl)benzamide;
N-cyclopropyl-4-methyl-2-((2-(4-morpholinyl)ethyl)amino)-5-(2-((2-(4-morpholinyl)ethyl)amino)-6-quinazolinyl)benzamide;
4-(2-amino-6-quinazolinyl)-N-cyclopropyl-5-methyl-2-pyridinecarboxamide;
N-cyclopropyl-5-methyl-4-(2-((2-(4-morpholinyl)ethyl)amino)-6-quinazolinyl)-2-pyridinecarboxamide;
N-cyclopropyl-5-methyl-4-(2-((3-((2R)-2-methyl-1-piperidinyl)propyl)amino)-6-quinazolinyl)-2-pyridinecarboxamide;
N-cyclopropyl-2-fluoro-4-methyl-3-(2-((2-(4-morpholinyl)ethyl)amino)-6-quinazolinyl)benzamide;
1,1-dimethylethyl 2-((6-(2-((cyclopropylamino)carbonyl)-5-methyl-4-pyridinyl)-2-quinazolinyl)amino)-2-methylpropylcarbamate;
4-(2-((2-amino-1,1-dimethylethyl)amino)-6-quinazolinyl)-N-cyclopropyl-5-methyl-2-pyridinecarboxamide;
N-cyclopropyl-4-(2-((2-(dimethylamino)-1,1-dimethylethyl)amino)-6-quinazolinyl)-5-methyl-2-pyridinecarboxamide;
N-cyclopropyl-3-(2-((2-((2,2-dimethylpropanoyl)amino)-1,1-dimethylethyl)amino)-6-quinazolinyl)-4-methylbenzamide;
N-cyclopropyl-3-(2-((2-((2,2-dimethylpropanoyl)amino)-2-methylpropyl)amino)-6-quinazolinyl)-4-methylbenzamide;
N-cyclopropyl-4-(2-((1,1-dimethyl-2-((1-methylethyl)amino)ethyl)amino)-6-quinazolinyl)-5-methyl-2-pyridinecarboxamide;
N-cyclopropyl-3-(2-((2-(dimethylamino)-1,1-dimethylethyl)amino)-6-quinazolinyl)-4-methylbenzamide;
1,1-dimethylethyl 2-((6-(5-((cyclopropylamino)carbonyl)-2-methylphenyl)-2-quinazolinyl)amino)-2-methylpropyl(1-methylethyl)carbamate;
N-cyclopropyl-3-(2-((1,1-dimethyl-2-((1-methylethyl)amino)ethyl)amino)-6-quinazolinyl)-4-methylbenzamide;
N-cyclopropyl-3-(2-((3-(dimethylamino)propyl)amino)-6-quinazolinyl)-4-methylbenzamide;
N-cyclopropyl-4-methyl-3-(2-((3-(2-methyl-1-piperidinyl)propyl)amino)-6-quinazolinyl)benzamide;
N-cyclopropyl-4-methyl-3-(2-((1-methyl-2-(methyloxy)ethyl)amino)-6-quinazolinyl)benzamide;
N-cyclopropyl-4-methyl-3-(2-((3-((1-methylethyl)oxy)propyl)amino)-6-quinazolinyl)benzamide;
N-cyclopropyl-3-(2-((4-(diethylamino)-1-methylbutyl)amino)-6-quinazolinyl)-4-methylbenzamide;
N-cyclopropyl-4-methyl-3-(2-((3-(1-pyrrolidinyl)propyl)amino)-6-quinazolinyl)benzamide;
3-(2-((2-amino-2-methylpropyl)amino)-6-quinazolinyl)-N-cyclopropyl-4-methylbenzamide;
N-(6-(2-methylphenyl)pyrido[2,3-d]pyrimidin-2-yl)-1,4-cyclohexanediamine;
N-cyclopropyl-4-methyl-3-(2-((tetrahydro-2-furanylmethyl)amino)pyrido[2,3-d]pyrimidin-6-yl)benzamide;
4-((6-(4-methyl-3-pyridinyl)-2-quinazolinyl)amino)cyclohexanol;
trans-4-((6-(2-methyl-3-pyridinyl)-2-quinazolinyl)amino)cyclohexanol;
trans-N-(6-(4-methyl-3-pyridinyl)-2-quinazolinyl)-1,4-cyclohexanediamine;
N-(trans-4-((6-(4-methyl-3-pyridinyl)-2-quinazolinyl)amino)cyclohexyl)acetamide;
N-(2-(1,1-dioxido-4-thiomorpholinyl)ethyl)-6-(4-methyl-3-pyridinyl)-2-quinazolinamine;
4-((6-(3-methyl-2-pyridinyl)-2-quinazolinyl)amino)cyclohexanol;
N∼1∼,N∼1∼-dimethyl-N∼4∼-(6-(4-methyl-3-pyridinyl)-2-quinazolinyl)-1,4-cyclohexanediamine;
N∼1∼-(1-methylethyl)-N∼4∼-(6-(4-methyl-3-pyridinyl)-2-quinazolinyl)-1,4-cyclohexanediamine;
6-(2-fluorophenyl)-N-(2-(2-pyridinyl)ethyl)-2-quinazolinamine;
6-(4-fluorophenyl)-N-(2-(2-pyridinyl)ethyl)-2-quinazolinamine;
6-(2-chlorophenyl)-N-(2-(2-pyridinyl)ethyl)-2-quinazolinamine;
6-(2,3-difluorophenyl)-N-(2-(2-pyridinyl)ethyl)-2-quinazolinamine;
6-(2,4-difluorophenyl)-N-(2-(2-pyridinyl)ethyl)-2-quinazolinamine;
6-(2,5-difluorophenyl)-N-(2-(2-pyridinyl)ethyl)-2-quinazolinamine;
6-(2,3-dimethylphenyl)-N-(2-(2-pyridinyl)ethyl)-2-quinazolinamine;
6-(3,5-difluorophenyl)-N-(2-(2-pyridinyl)ethyl)-2-quinazolinamine;
N-(2-(2-pyridinyl)ethyl)-6-(2-(trifluoromethyl)phenyl)-2-quinazolinamine;
4-((6-(2,5-dimethylphenyl)-2-quinazolinyl)amino)cyclohexanol;
N-cyclopropyl-4-methyl-3-(2-((2-(2-pyridinyl)ethyl)amino)-6-quinazolinyl)benzamide;
4-methyl-3-(2-((2-(4-morpholinyl)ethyl)amino)-6-quinazolinyl)-N-(1,3-thiazol-2-yl)benzamide;
N-cyclopropyl-4-methyl-3-(2-((3-(4-morpholinyl)propyl)amino)-6-quinazolinyl)benzamide;
N-cyclopropyl-3-(2-((1,1-dimethyl-2-(4-morpholinyl)ethyl)amino)-6-quinazolinyl)-4-methylbenzamide;
3-(2-(((1S)-2-(3-(aminomethyl)phenyl)-1-methylethyl)amino)-6-quinazolinyl)-N-cyclopropyl-4-methylbenzamide;
N-cyclopropyl-4-methyl-3-(2-methyl-6-quinazolinyl)benzamide;
1,1-dimethylethyl (3-((2S)-2-((6-(5-((cyclopropylamino)carbonyl)-2-methylphenyl)-2-quinazolinyl)amino)propyl)phenyl)methylcarbamate;
N-cyclopropyl-3-(2-(((1S)-2-(3-((dimethylamino)methyl)phenyl)-1-methylethyl)amino)-6-quinazolinyl)-4-methylbenzamide;
N-cyclopropyl-4-methyl-3-(2-((2-methyl-2-(4-morpholinyl)propyl)amino)-6-quinazolinyl)benzamide;
1,1-dimethylethyl ((3-((2S)-2-((6-(5-((ethylamino)carbonyl)-2-methylphenyl)-2-quinazolinyl)amino)propyl)phenyl)methyl)carbamate;
3-(2-(((1S)-2-(3-(aminomethyl)phenyl)-1-methylethyl)amino)-6-quinazolinyl)-N-ethyl-4-methylbenzamide;
3-(2-((2-(dimethylamino)ethyl)amino)-6-quinazolinyl)-4-methylbenzamide;
N-cyclopropyl-3-(2-(((1S)-1-((dimethylamino)carbonyl)-2-methylpropyl)amino)-6-quinazolinyl)-4-methylbenzamide;
3-(2-((3-(dimethylamino)-2,2-dimethylpropyl)amino)-6-quinazolinyl)-4-methylbenzamide;
3-(2-((3-(dimethylamino)-2,2-dimethylpropyl)amino)-6-quinazolinyl)-N-ethyl-4-methylbenzamide;
4-methyl-3-(2-((2-methyl-2-(4-morpholinyl)propyl)amino)-6-quinazolinyl)benzamide;
N,4-dimethyl-3-(2-((2-methyl-2-(4-morpholinyl)propyl)amino)-6-quinazolinyl)benzamide;
N-ethyl-4-methyl-3-(2-((2-methyl-2-(4-morpholinyl)propyl)amino)-6-quinazolinyl)benzamide;
N-cyclopropyl-3-(2-((2-(3,5-dimethyl-4-morpholinyl)ethyl)amino)-6-quinazolinyl)-4-methylbenzamide;
4-methyl-3-(2-((2-methyl-2-(4-morpholinyl)propyl)amino)-6-quinazolinyl)benzoic acid;
N-cyclopropyl-4-methyl-3-(2-((2-((3S)-3-methyl-4-morpholinyl)ethyl)amino)-6-quinazolinyl)benzamide;
N-cyclopropyl-4-methyl-3-(2-((2-(phenylamino)ethyl)amino)-6-quinazolinyl)benzamide;
N-(6-(5-((cyclopropylamino)carbonyl)-2-methylphenyl)-2-quinazolinyl)-O-methyl-L-threonine;
N-(6-(5-((cyclopropylamino)carbonyl)-2-methylphenyl)-2-quinazolinyl)-D-tryptophan;
N-(6-(5-((cyclopropylamino)carbonyl)-2-methylphenyl)-2-quinazolinyl)-D-phenylalanine;
N-cyclopropyl-3-(2-(((1S)-1-(4-fluorophenyl)ethyl)amino)-6-quinazolinyl)-4-methylbenzamide;
((6-(5-((cyclopropylamino)carbonyl)-2-methylphenyl)-2-quinazolinyl)amino)(tetrahydro-2H-pyran-4-yl)acetic acid;
N-cyclopropyl-4-methyl-3-(2-((2-(2-pyridinylamino)ethyl)amino)-6-quinazolinyl)benzamide;
3-(4-bromo-7-isoquinolinyl)-4-methylbenzamide;
3-(4-(2-chloro-4-fluorophenyl)-7-isoquinolinyl)-4-methylbenzamide;
4-methyl-3-(4-(4-morpholinyl)-7-isoquinolinyl)benzamide;
4-methyl-3-(4-(2-methyl-3-pyridinyl)-7-isoquinolinyl)benzamide;
3-(4-(4-(aminosulfonyl)-2-methylphenyl)-7-isoquinolinyl)-N-cyclopropyl-4-methylbenzamide;
6-(2-methylphenyl)-N-(3-(4-morpholinyl)propyl)-2-quinazolinamine;
N-(3-(1H-imidazol-1-yl)propyl)-6-(2-methylphenyl)-2-quinazolinamine;
6-(2-methylphenyl)-N-(2-(2-pyridinyl)ethyl)-2-quinazolinamine;
N-(6-(2-methylphenyl)-2-quinazolinyl)-1,4-cyclohexanediamine;
6-(2-methylphenyl)-N-(3-(2-methyl-1-piperidinyl)propyl)-2-quinazolinamine;
N-((1S)-2-(3-((1S)-1-aminoethyl)phenyl)-1-methylethyl)-6-(2-methylphenyl)-2-quinazolinamine;
N∼1∼,N∼1∼-diethyl-N∼3∼-(6-(2-methylphenyl)-2-quinazolinyl)-1,3-propanediamine;
4-((6-(2-methylphenyl)-2-quinazolinyl)amino)cyclohexanol;
N-((1S)-2-(3-(1-amino-1-methylethyl)phenyl)-1-methylethyl)-6-(2-methylphenyl)-2-quinazolinamine;
N-((1S)-2-(3-(1-amino-1-methylethyl)phenyl)-1-methylethyl)-6-(4-methyl-3-thienyl)-2-quinazolinamine;
4-((6-(2-aminophenyl)-2-quinazolinyl)amino)cyclohexanol;
6-(2-methylphenyl)-N-(1-(phenylmethyl)-3-pyrrolidinyl)-2-quinazolinamine;
6-(2-methylphenyl)-N-(1-(phenylmethyl)-4-piperidinyl)-2-quinazolinamine;
1,1-dimethylethyl 2-(2-((6-(2-methylphenyl)-2-quinazolinyl)amino)ethyl)-1-piperidinecarboxylate;
6-(2-methylphenyl)-N-(2-(2-piperidinyl)ethyl)-2-quinazolinamine;
N-((1S)-2-(3-(1-amino-1-methylethyl)phenyl)-1-methylethyl)-6-(4-methyl-3-pyridinyl)-2-quinazolinamine;
6-(3-methyl-2-pyridinyl)-2-quinazolinamine;
N-((1S)-2-(3-(1-amino-1-methylethyl)phenyl)-1-methylethyl)-6-(3-methyl-2-pyridinyl)-2-quinazolinamine;
N-((1S)-2-(3-((1S)-1-aminoethyl)phenyl)-1-methylethyl)-6-(2-chloro-3-pyridinyl)-2-quinazolinamine;
N-((1S)-2-(3-((1S)-1-aminoethyl)phenyl)-1-methylethyl)-6-(4-methyl-3-pyridinyl)-2-quinazolinamine;
N∼1∼,N∼1∼-diethyl-N∼4∼-(6-(4-methyl-3-pyridinyl)-2-quinazolinyl)-1,4-pentanediamine;
N-(2-(3-chlorophenyl)ethyl)-6-(4-methyl-3-pyridinyl)-2-quinazolinamine;
(5S)-5-((6-(4-methyl-3-pyridinyl)-2-quinazolinyl)amino)-2-piperidinone;
((1R)-4-(diethylamino)-1-methylbutyl)(6-(4-methyl-3-pyridinyl)-2-quinazolinyl)amine;
((1S)-4-(diethylamino)-1-methylbutyl)(6-(4-methyl-3-pyridinyl)-2-quinazolinyl)amine;
N,N,2,2-tetramethyl-N'-(6-(4-methyl-3-pyridinyl)-2-quinazolinyl)-1,3-propanediamine;
6-(4-methyl-3-pyridinyl)-N-(4-(1-pyrrolidinyl)butyl)-2-quinazolinamine;
N-((1S)-1-(4-fluorophenyl)ethyl)-6-(4-methyl-3-pyridinyl)-2-quinazolinamine;
4-chloro-N-cyclopropyl-3-(2-((2-(dimethylamino)ethyl)amino)-6-quinazolinyl)benzamide;
N-cyclopropyl-3-(2-((2-(dimethylamino)ethyl)amino)-6-quinazolinyl)-4-(trifluoromethyl)benzamide;
ethyl 5-(2-((2-(dimethylamino)ethyl)amino)-6-quinazolinyl)-4-methyl-2-thiophenecarboxylate;
6-(2-methylphenyl)-2-quinazolinamine;
6-(2-methylphenyl)-N-(2-(3-pyridinyl)ethyl)-2-quinazolinamine;
N-((1S)-2-(3-(aminomethyl)phenyl)-1-methylethyl)-6-(2-methylphenyl)-2-quinazolinamine;
N-(2,4-dimethylphenyl)-6-(2-methylphenyl)-2-quinazolinamine;
6-(2-methylphenyl)-N-(2-(4-morpholinyl)ethyl)-2-quinazolinamine;
6-(2-((dimethylamino)methyl)phenyl)-N-(2-(4-morpholinyl)ethyl)-2-quinazolinamine;
6-(2-((dimethylamino)methyl)phenyl)-N-(2-(2-pyridinyl)ethyl)-2-quinazolinamine;
6-(4-fluoro-2-methylphenyl)-N-(2-(2-pyridinyl)ethyl)-2-quinazolinamine;
6-(3-chloro-2-methylphenyl)-N-(2-(2-pyridinyl)ethyl)-2-quinazolinamine;
6-(2,6-dimethylphenyl)-N-(2-(2-pyridinyl)ethyl)-2-quinazolinamine;
(3-(2-((2-(2-pyridinyl)ethyl)amino)-6-quinazolinyl)phenyl)methanol;
(2-(2-((2-(2-pyridinyl)ethyl)amino)-6-quinazolinyl)phenyl)methanol;
6-(3-(aminomethyl)phenyl)-N-(2-(2-pyridinyl)ethyl)-2-quinazolinamine;
N-methyl-3-(2-((2-(2-pyridinyl)ethyl)amino)-6-quinazolinyl)benzamide;
N-cyclopropyl-3-(2-((2-(2-pyridinyl)ethyl)amino)-6-quinazolinyl)benzamide;
6-phenyl-N-(2-(2-pyridinyl)ethyl)-2-quinazolinamine;
6-(2-(methyloxy)phenyl)-N-(2-(2-pyridinyl)ethyl)-2-quinazolinamine;
4-((6-(2,6-dimethylphenyl)-2-quinazolinyl)amino)cyclohexanol;
N-(3-(2-amino-6-quinazolinyl)-4-methylphenyl)cyclopropanecarboxamide;
N-(4-methyl-3-(2-((2-(4-morpholinyl)ethyl)amino)-6-quinazolinyl)phenyl)cyclopropanecarboxamide;
N-(3-methyl-4-(2-((2-(4-morpholinyl)ethyl)amino)-6-quinazolinyl)phenyl)cyclopropanecarboxamide;
N-cyclopropyl-3-(2-(3-(dimethylamino)-1-propynyl)-6-quinazolinyl)-4-methylbenzamide;
N-cyclopropyl-3-(2-(3-(dimethylamino)propyl)-6-quinazolinyl)-4-methylbenzamide;
1,1-dimethylethyl 1-(6-(5-((cyclopropylamino)carbonyl)-2-methylphenyl)-2-quinazolinyl)-3-pyrrolidinylcarbamate;
3-(2-((3S)-3-amino-1-pyrrolidinyl)-6-quinazolinyl)-N-cyclopropyl-4-methylbenzamide;
N-cyclopropyl-4-methyl-3-(2-(((3R)-6-oxo-3-piperidinyl)amino)-6-quinazolinyl)benzamide;
N-cyclopropyl-3-(2-((2-((1,1-dimethylethyl)amino)ethyl)amino)-6-quinazolinyl)-4-methylbenzamide;
3-(2-(cyclohexylamino)-6-quinazolinyl)-N-cyclopropyl-4-methylbenzamide;
N-cyclopropyl-4-methyl-3-(2-(((3S)-6-oxo-3-piperidinyl)amino)-6-quinazolinyl)benzamide;
N-ethyl-4-methyl-3-(2-(((3S)-6-oxo-3-piperidinyl)amino)-6-quinazolinyl)benzamide;
N-cyclopropyl-3-(2-((2-(1,1-dioxido-4-thiomorpholinyl)ethyl)amino)-6-quinazolinyl)-4-methylbenzamide;
N-cyclopropyl-4-methyl-3-(2-((2-(2-oxo-1-pyrrolidinyl)ethyl)amino)-6-quinazolinyl)benzamide;
3-(2-(4-amino-1-piperidinyl)-6-quinazolinyl)-N-cyclopropyl-4-methylbenzamide;
3-(2-(4-(acetylamino)-1-piperidinyl)-6-quinazolinyl)-N-cyclopropyl-4-methylbenzamide;
1,1-dimethylethyl (1-(6-(5-((cyclopropylamino)carbonyl)-2-methylphenyl)-2-quinazolinyl)-4-piperidinyl)carbamate;
1,1-dimethylethyl 4-((6-(5-((cyclopropylamino)carbonyl)-2-methylphenyl)-2-quinazolinyl)amino)-1-piperidinecarboxylate;
N-cyclopropyl-4-methyl-3-(2-(4-piperidinylamino)-6-quinazolinyl)benzamide;
3-(2-((1-acetyl-4-piperidinyl)amino)-6-quinazolinyl)-N-cyclopropyl-4-methylbenzamide;
N-cyclopropyl-4-methyl-3-(4-(2-methylphenyl)-7-isoquinolinyl)benzamide;
4-methyl-3-(4-(2-methylphenyl)-7-isoquinolinyl)benzamide;
methyl 4-methyl-3-(4-((3S)-3-methyl-4-morpholinyl)-7-isoquinolinyl)benzoate;
N-cyclopropyl-4-methyl-3-(2-((2-(1-piperazinyl)ethyl)amino)-6-quinazolinyl)benzamide;
N-cyclopropyl-4-methyl-3-(2-((3S)-3-piperidinylamino)-6-quinazolinyl)benzamide;
1,1-dimethylethyl 2-((6-(5-((cyclopropylamino)carbonyl)-2-methylphenyl)-2-quinazolinyl)amino)-2-methylpropylcarbamate;
1,1-dimethylethyl (2S)-2-(((6-(5-((cyclopropylamino)carbonyl)-2-methylphenyl)-2-quinazolinyl)amino)methyl)-1-pyrrolidinecarboxylate;
N-cyclopropyl-4-methyl-3-(2-((2-(4-methyl-1-piperazinyl)ethyl)amino)-6-quinazolinyl)benzamide;
N-cyclopropyl-4-methyl-3-(2-(((2S)-2-pyrrolidinylmethyl)amino)-6-quinazolinyl)benzamide;
1,1-dimethylethyl (2R)-2-(((6-(5-((cyclopropylamino)carbonyl)-2-methylphenyl)-2-quinazolinyl)amino)methyl)-1-pyrrolidinecarboxylate;
N-cyclopropyl-4-methyl-3-(2-(((2R)-2-pyrrolidinylmethyl)amino)-6-quinazolinyl)benzamide;
3-(2-((2-amino-1,1-dimethylethyl)amino)-6-quinazolinyl)-N-cyclopropyl-4-methylbenzamide;
N-cyclopropyl-4-methyl-3-(2-(((3R)-1-methyl-3-pyrrolidinyl)amino)-6-quinazolinyl)benzamide;
N-cyclopropyl-4-methyl-3-(2-((3R)-3-pyrrolidinylamino)-6-quinazolinyl)benzamide;
N-cyclopropyl-4-methyl-3-(2-((((2S)-1-methyl-2-pyrrolidinyl)methyl)amino)-6-quinazolinyl)benzamide;
N-cyclopropyl-4-methyl-3-(2-((((2R)-1-(1-methylethyl)-2-pyrrolidinyl)methyl)amino)-6-quinazolinyl)benzamide;
N-cyclopropyl-4-methyl-3-(2-((((2S)-1-(2-(methyloxy)ethyl)-2-pyrrolidinyl)methyl)amino)-6-quinazolinyl)benzamide;
3-(2-((2-aminoethyl)amino)-6-quinazolinyl)-N-cyclopropyl-4-methylbenzamide;
N-cyclopropyl-4-methyl-3-(2-((2-((1-methylethyl)amino)ethyl)amino)-6-quinazolinyl)benzamide
N-cyclopropyl-3-(2-(((1R,2R)-2-(dimethylamino)cyclohexyl)amino)-6-quinazolinyl)-4-methylbenzamide
N-cyclopropyl-3-(2-(((1S,2S)-2-(dimethylamino)cyclohexyl)amino)-6-quinazolinyl)-4-methylbenzamide
N-cyclopropyl-4-methyl-3-(2-(((1R,2R)-2-((1-methylethyl)amino)cyclohexyl)amino)-6-quinazolinyl)benzamide methyl 2-((6-(5-((cyclopropylamino)carbonyl)-2-methylphenyl)-2-quinazolinyl)amino)ethylcarbamate
N-cyclopropyl-4-methyl-3-(2-(((1S,2S)-2-((1-methylethyl)amino)cyclohexyl)amino)-6-quinazolinyl)benzamide;
1-((6-(5-((cyclopropylamino)carbonyl)-2-methylphenyl)-2-quinazolinyl)amino)cyclopentanecarboxylic acid;
N-(6-(5-((cyclopropylamino)carbonyl)-2-methylphenyl)-2-quinazolinyl)-3-(2-thienyl)alanine;
1-((6-(5-((cyclopropylamino)carbonyl)-2-methylphenyl)-2-quinazolinyl)amino)cyclopropanecarboxylic acid;
methyl N-(6-(5-((cyclopropylamino)carbonyl)-2-methylphenyl)-2-quinazolinyl)-beta-alaninate;
N-(6-(5-((cyclopropylamino)carbonyl)-2-methylphenyl)-2-quinazolinyl)-2-methylalanine;
N-(6-(5-((cyclopropylamino)carbonyl)-2-methylphenyl)-2-quinazolinyl)-L-valine;
N,4-dimethyl-3-(2-((2-(4-morpholinyl)ethyl)amino)-6-quinazolinyl)benzamide;
4-methyl-3-(2-((2-(4-morpholinyl)ethyl)amino)-6-quinazolinyl)-N-(2,2,2-trifluoroethyl)benzamide;
4-methyl-N-(2-methylpropyl)-3-(2-((2-(4-morpholinyl)ethyl)amino)-6-quinazolinyl)benzamide;
N-cyclopentyl-4-methyl-3-(2-((2-(4-morpholinyl)ethyl)amino)-6-quinazolinyl)benzamide;
N-cyclopropyl-3-(2-((3-(dimethylamino)-2,2-dimethylpropyl)amino)-6-quinazolinyl)-4-methylbenzamide;
N-cyclopropyl-3-(2-(((1S,2R)-2-(dimethylamino)cyclohexyl)amino)-6-quinazolinyl)-4-methylbenzamide;
N-cyclopropyl-3-(2-((2-(diethylamino)ethyl)amino)-6-quinazolinyl)-4-methylbenzamide;
N-cyclopropyl-4-methyl-3-(2-((2-(methyloxy)ethyl)amino)-6-quinazolinyl)benzamide;
3-(2-(((1S,2S)-2-aminocyclohexyl)amino)-6-quinazolinyl)-N-cyclopropyl-4-methylbenzamide;
N-cyclopropyl-3-(2-(((1R,2R)-2-(dimethylamino)cyclohexyl)amino)-6-quinazolinyl)-4-methylbenzamide;
N-cyclopropyl-3-(2-((2-furanylmethyl)amino)-6-quinazolinyl)-4-methylbenzamide;
N-cyclopropyl-4-methyl-3-(2-((tetrahydro-2-furanylmethyl)amino)-6-quinazolinyl)benzamide;
N∼5∼-(6-(4-methyl-3-pyridinyl)-2-quinazolinyl)-2,5-pyridinediamine;
((1R)-4-(diethylamino)-1-methylbutyl)(6-(6-fluoro-2-methyl-3-pyridinyl)-2-quinazolinyl)amine;
5-(2-((4-(diethylamino)-1-methylbutyl)amino)-6-quinazolinyl)-6-methyl-2(1H)-pyridinone;
3-(2-(((1R)-4-(diethylamino)-1-methylbutyl)amino)-6-quinazolinyl)-4-methyl-2(1H)-pyridinone;
((1R)-4-(diethylamino)-1-methylbutyl)(6-(2-fluoro-5-methyl-4-pyridinyl)-2-quinazolinyl)amine;
4-(2-(((1R)-4-(diethylamino)-1-methylbutyl)amino)-6-quinazolinyl)-5-methyl-2(1H)-pyridinone;
N-(6-(5-((cyclopropylamino)carbonyl)-2-methylphenyl)-2-quinazolinyl)-D-valine;
N-(6-(5-((cyclopropylamino)carbonyl)-2-methylphenyl)-2-quinazolinyl)-D-leucine;
N-(6-(5-((cyclopropylamino)carbonyl)-2-methylphenyl)-2-quinazolinyl)glycine;
N-(6-(5-((cyclopropylamino)carbonyl)-2-methylphenyl)-2-quinazolinyl)-L-alanine;
N-(6-(5-((cyclopropylamino)carbonyl)-2-methylphenyl)-2-quinazolinyl)-D-alanine;
5-(2-amino-6-quinazolinyl)-N-(2-(methyl((3R)-1-methyl-3-pyrrolidinyl)amino)-5-(trifluoromethyl)phenyl)-2-(methyloxy)benzamide;
3-(2-amino-6-quinazolinyl)-N-(2-((3-(dimethylamino)propyl) (methyl)amino)-5-(trifluoromethyl)phenyl)-2-((phenylmethyl)amino)benzamide;
4-(2-amino-6-quinazolinyl)-N-(2-((3-(dimethylamino)propyl)(methyl)amino)-5-(trifluoromethyl)phenyl)-5-methyl-2-pyridinecarboxamide;
4-(2-amino-6-quinazolinyl)-N-(2-((3-(dimethylamino)propyl)(methyl)amino)-5-(trifluoromethyl)phenyl)-1-(methyloxy)-2-naphthalenecarboxamide;
4-(2-amino-6-quinazolinyl)-1-(methyloxy)-N-(3-(trifluoromethyl)phenyl)-2-naphthalenecarboxamide;
4-(2-amino-6-quinazolinyl)-N-(2-(methyl((3R)-1-methyl-3-pyrrolidinyl)amino)-5-(trifluoromethyl)phenyl)-1-(methyloxy)-2-naphthalenecarboxamide;
4-(2-amino-6-quinazolinyl)-N-(2-(((3R)-3-(dimethylamino)-1-pyrrolidinyl)methyl)-5-(trifluoromethyl)phenyl)-1-(methyloxy)-2-naphthalenecarboxamide;
5-(2-amino-6-quinazolinyl)-N-(2-(3,3-dimethyl-2-oxo-1-azetidinyl)-5-(trifluoromethyl)phenyl)-2-(methyloxy)benzamide; and
5-(2-amino-6-quinazolinyl)-N-(2-((3S)-3-(dimethylamino)-1-piperidinyl)-5-(trifluoromethyl)phenyl)-2-fluorobenzamide;
6-(2-phenylimidazo[1,2-a]pyridin-6-yl)-2-quinazolinamine 6-(2-(4-(methyloxy)phenyl)imidazo[1,2-a]pyridin-6-yl)-2-quinazolinamine;
6-(2-(3-(methyloxy)phenyl)imidazo[1,2-a]pyridin-6-yl)-2-quinazolinamine;
6-(2-(2-(methyloxy)phenyl)imidazo[1,2-a]pyridin-6-yl)-2-quinazolinamine;
6-(2-(1,3-benzodioxol-5-yl)imidazo[1,2-a]pyridin-6-yl)-2-quinazolinamine;
6-(2-(3-thienyl)imidazo[1,2-a]pyridin-6-yl)-2-quinazolinamine;
6-(2-ethyl-1-benzofuran-5-yl)-2-quinazolinamine; and
6-(2-(1,1-dimethylethyl)imidazo[1,2-a]pyridin-6-yl)-2-quinazolinamine.

3. A pharmaceutical composition comprising a pharmaceutically acceptable carrier and a compound according to Claim 1 or 2.

4. Compound according to Claim 1 or 2 for use in modulating in a subject a protein kinase enzyme selected from the group consisting of KDR, Lck, p38, tie2 and a combination thereof.

5. Compound according to Claim 1 or 2 for use in treating a disorder related to at least one of Tie-2, Lck, KDR and P38 in a subject.

6. Compound according to Claim 1 or 2 for use in treating a proliferation-related disorder in a subject.

7. The compound of Claim 6, wherein the disorder is selected from the group consisting of myocardial infarction, coronary artery disease, peripheral vascular disease, stroke, ocular neovascularization, retinopathy, diabetic retinopathy, age-related macular degeneration, psoriasis, hemangioblastoma, hemangioma, arteriosclerosis, inflammatory disease rheumatoid arthritis, asthma, arterial or post-transplantational atherosclerosis, endometriosis, leukemia and combinations thereof.

8. Compound according to Claim 1 or 2 for use in treating cancer in a subject.

9. The compound of Claim 8, wherein administering the effective amount of the compound to the subject comprises administering the compound in combination with one or more compounds selected from antineoplastic agents, anti-angiogenic agents, chemotherapeutic agents and peptidal cancer therapy agents.

10. The compound of Claim 9, wherein the antineoplastic agents are selected from antibiotic-type agents, alkylating agents, antimetabolite agents, hormonal agents, immunological agents, interferon-type agents, kinase inhibitors, miscellaneous agents and combinations thereof.

11. Pharmaceutical composition of Claim 3 for use in treating cancer in a subject.

12. Compound according to Claim 1 or 2 for use in treating angiogenesis in a subject.

13. Compound according to Claim 1 or 2 for use in reducing blood flow in a tumor in a subject.

14. Compound according to Claim 1 or 2 for use in reducing tumor size in a subject.

15. Compound according to Claim 1 or 2 for use in treating inflammation in a subject.

16. Compound according to Claim 1 or 2 for use in inhibiting T cell activation and proliferation in a subject.

17. Compound according to Claim 1 or 2 for use in treating arthritis, rheumatoid arthritis, psoriatic arthritis, or osteoarthritis in a subject.

18. Compound according to Claim 1 or 2 for use in treating organ transplant, acute transplant or heterograft or homograft rejection, or transplantation tolerance induction in a subject.

19. Compound according to Claim 1 or 2 for use in treating ischemic or reperfusion injury, myocardial infarction, or stroke in a subject.

20. Compound according to Claim 1 or 2 for use in treating multiple sclerosis, inflammatory bowel disease, including ulcerative colitis, Crohn's disease, lupus, contact hypersensitivity, delayed-type hypersensitivity, and gluten-sensitive enteropathy, type 1 diabetes, psoriasis, contact dermatitis, Hashimoto's thyroiditis, Sjogren's syndrome, autoimmune hyperthyroidism, Addison's disease, autoimmune polyglandular disease, autoimmune alopecia, pernicious anemia, vitiligo, autoimmune hypopituatarism, Guillain-Barre syndrome, glomerulonephritis, serum sickness, uticaria, allergic diseases, asthma, hayfever, allergic rhinitis, scleracielma, mycosis fungoides, dermatomyositis, alopecia areata, chronic actinic dermatitis, eczema, Behcet's disease, Pustulosis palmoplanteris, Pyoderma gangrenum, Sezary's syndrome, atopic dermatitis, systemic schlerosis, morphea or atopic dermatitis in a subject.

21. Compound according to Claim 1 or 2 for use in treating colon carcinoma or thymoma in a subject.

22. Compound according to Claim 1 or 2 for use in decreasing the level of one or more of TNF-α, IL-1β, IL-6 and IL-8 in a subject.

23. Compound according to Claim 1 or 2 for use in treating rheumatoid spondylitis, gouty arthritis, adult respiratory distress syndrome (ARDS), anaphylaxis, muscle degeneration, cachexia, Reiter's syndrome, type II diabetes, bone resorption diseases, graft vs. host reaction, Alzheimer's disease, atherosclerosis, brain trauma, multiple sclerosis, cerebral malaria, sepsis, septic shock, toxic shock syndrome, fever, and myalgias due to infection, or which subject is infected by HIV-1, HIV-2, HIV-3, cytomegalovirus (CMV), influenza, adenovirus, the herpes viruses (including HSV-1, HSV-2), or herpes zoster in a subject.

24. Use of a compound according to Claim 1 or 2 for the preparation of a medicament for the treatment of inflammation or cancer in a subject.

25. Use of a compound according to Claim 1 or 2 for the preparation of a medicament for the inhibition of T cell activation and proliferation in a subject.

26. Use of a compound according to Claim 1 or 2 for the preparation of a medicament for the treatment of arthritis, rheumatoid arthritis, psoriatic arthritis, or osteoarthritis in a subject.

27. Use of a compound according to Claim 1 or 2 for the preparation of a medicament for the treatment of organ transplant, acute transplant or heterograft or homograft rejection, or transplantation tolerance induction in a subject.

28. Use of a compound according to Claim 1 or 2 for the preparation of a medicament for the treatment of ischemic or reperfusion injury, myocardial infarction, or stroke in a subject.

29. Use of a compound according to Claim 1 or 2 for the preparation of a medicament for the treatment of multiple sclerosis, inflammatory bowel disease, including ulcerative colitis, Crohn's disease, lupus, contact dermatitis, Hashimoto's thyroiditis, Sjogren's syndrome, autoimmune hyperthyroidism, Addison's disease, autoimmune polyglandular disease, autoimmune alopecia, pernicious anemia, vitiligo, autoimmune hypopituatarism, Guillain-Barre syndrome, glomerulonephritis, serum sickness, uticaria, allergic diseases, asthma, hayfever, allergic rhinitis, scleracielma, mycosis fungoides, dermatomyositis, alopecia areata, chronic actinic dermatitis, eczema, Behcet's disease, Pustulosis palmoplanteris, Pyoderma gangrenum, Sezary's syndrome, atopic dermatitis, systemic schlerosis, morphea, atopic dermatitis, rheumatoid spondylitis, gouty arthritis, adult respiratory distress syndrome (ARDS), anaphylaxis, muscle degeneration, cachexia, Reiter's syndrome, type II diabetes, bone resorption diseases, graft vs. host reaction, Alzheimer's disease, atherosclerosis, brain trauma, multiple sclerosis, cerebral malaria, sepsis, septic shock, toxic shock syndrome, fever, and myalgias due to infection, or which subject is infected by HIV-1, HIV-2, HIV-3, cytomegalovirus (CMV), influenza, adenovirus, the herpes viruses (including HSV-1, HSV-2), or herpes zoster in a subject.

30. Use of a compound according to Claim 1 or 2 for the preparation of a medicament for the treatment of colon carcinoma or thymoma in a subject.

## Patentansprüche

1. Verbindung mit einer Formel II 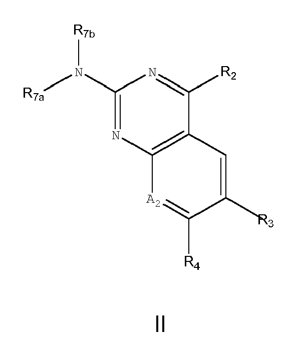 oder ein Stereoisomer, Tautomer, Solvat oder pharmazeutisch verträgliches Salz davon, wobei
A² ist CR⁵ oder N;
R² ist H, NO₂, CN, OR^{7b}, SR^{7b}, C₁₋₁₀-Alkyl oder C₃₋₁₀-Cycloalkyl;
R³ ist 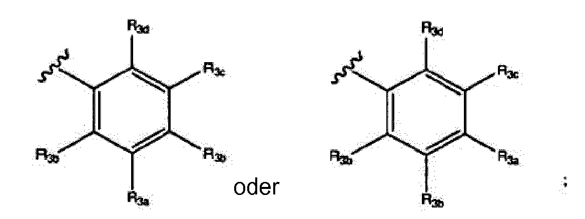 worin
R^{3a} ist C(O)R¹⁰, COOR¹⁰, C(O)R¹¹, COOR¹¹, C(O)SR¹⁰, C(O)SR¹¹, C(O)NR¹⁰R¹⁰, C(S)NR¹⁰R¹⁰, C(O)NR¹⁰R¹¹, C(S)NR¹⁰R¹¹, NR¹⁰C(O)R¹⁰, NR¹⁰C(S)R¹⁰, NR¹⁰C(O)R¹¹, NR¹⁰C(S)R¹¹, NR¹⁰C(O)NR¹⁰R¹⁰, NR¹⁰C(O)NR¹⁰R¹¹, NR¹⁰C(S)NR¹⁰R¹⁰, NR¹⁰C(S)NR¹⁰R¹¹, NR¹⁰(COOR¹⁰), NR¹⁰(COOR¹¹), S(O)₂R¹¹, S(O)₂NR¹⁰R¹⁰, S(O)₂NR¹⁰R¹¹, NR¹⁰S(O)₂NR¹⁰R¹¹, NR¹⁰S(O)₂R¹⁰ oder NR¹⁰S(O)₂R¹¹;
R^{3b} ist H, F, Cl, Br, I, CF₃, CF₂CF₃, OCF₃, OCF₂CF₃, CN, NO₂, NH₂, Methyl, Ethyl, Propyl, Isopropyl, n-Butyl, sec-Butyl, tert-Butyl, Acetylenyl, Cyclopropyl, Cyclobutyl, Cyclopentyl, Cyclohexyl, OH, Methoxyl, Ethoxyl, Propoxyl, SH, Thiomethyl oder Thioethyl;
R^{3c} ist H, F, Cl, Br, I, CF₃, CF₂CF₃, OCF₃, OCF₂CF₃, CN, NO₂, NH₂, Methyl, Ethyl, Propyl, Isopropyl, n-Butyl, sec-Butyl, tert-Butyl, Acetylenyl, Cyclopropyl, Cyclobutyl, Cyclopentyl, Cyclohexyl, OH, Methoxyl, Ethoxyl, Propoxyl, SH, Thiomethyl oder Thioethyl;
R^{3d} ist H, F, Cl, Br, I, CF₃, CF₂CF₃, OCF₃, OCF₂CF₃, CN, NO₂, NH₂, Methyl, Ethyl, Propyl, Isopropyl, n-Butyl, sec-Butyl, tert-Butyl, Acetylenyl, Cyclopropyl, Cyclobutyl, Cyclopentyl, Cyclohexyl, OH, Methoxyl, Ethoxyl, Propoxyl, SH, Thiomethyl oder Thioethyl;
R⁴ ist H, F, Cl, Br, I, CF₃, CF₂CF₃, OCF₃, CN, Methyl, Ethyl, Propyl, Isopropyl, n-Butyl, sec-Butyl, tert-Butyl, OH, Methoxyl, Ethoxyl, Propoxyl;
R⁵ ist H, F, Cl, Br, I, CF₃, CF₂CF₃, OCF₃, CN, Methyl, Ethyl, Propyl, Isopropyl, n-Butyl, sec-Butyl, tert-Butyl, OH, Methoxyl, Ethoxyl, Propoxyl;
R⁶ ist H, CN, Methyl, Ethyl, Propyl, Isopropyl oder n-Butyl;
R^{7a} ist H, C(O)R⁸, COOR⁸, C(O)R⁹, COOR⁹, C(O)NR⁸R⁹, C(O)NR⁹R⁹, S(O)₂R⁸, S(O)₂NR⁸R⁹, S(O)₂R⁹, S(O)₂NR⁹R⁹, C₁₋₁₀-Alkyl, C₂₋₁₀-Alkenyl, C₂₋₁₀-Alkinyl oder C₃₋₁₀-Cycloalkyl, wobei jedes von dem C₁₋₁₀-Alkyl, C₂₋₁₀-Alkenyl, C₂₋₁₀-Alkinyl und C₃₋₁₀-Cycloalkyl gegebenenfalls 1-4 Heteroatome, ausgewählt aus N, O und S, umfasst und gegebenenfalls mit einem oder mehreren Substituenten von NR⁸R⁹, NR⁹R⁹, OR⁸, SR⁸, OR⁹, SR⁹, C(O)R⁸, C(O)R⁹, C(O)NR⁸R⁹, C(O)NR⁹R⁹, NR⁹C(O)R⁸, NR⁹C(O)R⁹, NR⁹C(O)NR⁸R⁹, NR⁹C(O)NR⁹R⁹, NR⁹(COOR⁸), NR⁹(COOR⁹), S(O)₂R⁸, S(O)₂NR⁸R⁹, S(O)₂R⁹, S(O)₂NR⁹R⁹, NR⁹S(O)₂NR⁸R⁹, NR⁹S(O)₂NR⁹R⁹, NR⁹S(O)₂R⁸, NR⁹S(O)₂R⁹, R⁸ oder R⁹ substituiert ist;
R^{7b} ist H;
R⁸ ist ein Ringsystem, ausgewählt aus Phenyl, Naphthyl, Pyridyl, Piperazinyl, Triazinyl, Chinolinyl, Isochinolinyl, Chinazolinyl, Isochinazolinyl, Thiophenyl, Furyl, Pyrrolyl, Imidazolyl, Triazolyl, Thiazolyl, Oxazolyl, Isoxazolyl, Isothiazolyl, Indolyl, Isoindolyl, Benzofuranyl, Benzothiophenyl, Benzimidazolyl, Tetrahydrofuranyl, Pyrrolidinyl, Oxazolinyl, Isoxazolinyl, Thiazolinyl, Pyrazolinyl, Morpholinyl, Piperidinyl, Piperazinyl, Pyranyl, Dioxozinyl, Cyclopropyl, Cyclobutyl, Cyclopentyl, Cyclohexyl und Cycloheptyl, wobei das Ringsystem gegebenenfalls unabhängig substituiert ist mit 1-3 Substituenten von R⁹, Oxo, NR⁹R⁹, OR⁹; SR⁹, C(O)R⁹, Phenyl, Pyridyl, Piperidyl, Piperazinyl oder Morpholinyl;
R⁹ ist H, Halogen, Halogenalkyl, CN, OH, NO₂, NH₂, Acetyl, C₁₋₁₀-Alkyl, C₂₋₁₀-Alkenyl, C₂₋₁₀-Alkinyl, C₃₋₁₀-Cycloalkyl, C₁₋₁₀-Alkylamino-, C₁₋₁₀-Dialkylamino-, C₁₋₁₀-Alkoxyl, C₁₋₁₀-Thioalkoxyl oder ein Ringsystem, ausgewählt aus Phenyl, Naphthyl, Pyridyl, Piperazinyl, Triazinyl, Chinolinyl, Isochinolinyl, Chinazolinyl, Isochinazolinyl, Thiophenyl, Furyl, Pyrrolyl, Imidazolyl, Triazolyl, Thiazolyl, Oxazolyl, Isoxazolyl, Isothiazolyl, Indolyl, Isoindolyl, Benzofuranyl, Benzothiophenyl, Benzimidazolyl, Tetrahydrofuranyl, Pyrrolidinyl, Oxazolinyl, Isoxazolinyl, Thiazolinyl, Pyrazolinyl, Morpholinyl, Piperidinyl, Piperazinyl, Pyranyl und Dioxozinyl, wobei jedes von dem C₁₋₁₀-Alkyl, C₂₋₁₀-Alkenyl, C₂₋₁₀-Alkinyl, C₃₋₁₀-Cycloalkyl, C₁₋₁₀-Alkylamino-, C₁₋₁₀-Dialkylamino-, C₁₋₁₀-Alkoxyl, C₁₋₁₀-Thioalkoxyl und das Ringsystem gegebenenfalls unabhängig mit 1-3 Substituenten von Halogen, Halogenalkyl, CN, NO₂, NH₂, OH, Oxo, Methyl, Methoxyl, Ethyl, Ethoxyl, Propyl, Propoxyl, Isopropyl, Cyclopropyl, Butyl, Isobutyl, tert-Butyl, Methylamino, Dimethylamino, Ethylamino, Diethylamino, Propylamin, Isopropylamin, Dipropylamin, Diisopropylamin, Benzyl oder Phenyl substituiert ist;
R¹⁰ ist H, Methyl, Ethyl, Propyl, Isopropyl, n-Butyl, sec-Butyl, tert-Butyl, Acetylenyl, Cyclopropyl, Cyclobutyl, Cyclopentyl, Cyclohexyl, OH, Methoxyl, Ethoxyl, Propoxyl, SH, Thiomethyl oder Thioethyl; von denen jedes gegebenenfalls mit einem oder mehreren Substituenten von R¹¹, R¹² oder R¹⁶ substituiert ist;
R¹¹ ist Phenyl, Naphthyl, Pyridyl, Chinolinyl, Tetrahydrochinolinyl, Oxo-tetrahydrochinolinyl, Isochinolinyl, Oxo-tetrahydroisochinolinyl, Tetrahydroisochinolinyl, Thiophenyl, Tetrahydrofuranyl, Thieno-pyrazolyl, Tetrahydropentapyrazolyl, Imidazolyl, Thiazolyl, Thiadiazolyl, Benzothiazolyl, Oxazolyl, Oxadiazolyl, Benzoxazolyl, Benzoxadiazolyl, Isoxazolyl, Isothiazolyl, Indolyl, 2,3-Dihydroindolyl, Benzofuranyl, Benzothiophenyl, Benzimidazolyl, 2,3-Dihydro-1,4-benzoxazinyl, 1,3-Benzodioxolyl, Cyclopropyl, Cyclobutyl, Cyclopentyl und Cyclohexyl, wobei das Ringsystem gegebenenfalls unabhängig mit 1-3 Substituenten von R¹², R¹³, R¹⁴ oder R¹⁶ substituiert ist;
R¹² ist H, C₁₋₁₀-Alkyl, C₂₋₁₀-Alkenyl, C₂₋₁₀-Alkinyl, C₃₋₁₀-Cycloalkyl, C₄₋₁₀-Cycloalkenyl, C₁₋₁₀-Alkylamino-, C₁₋₁₀-Dialkylamino- oder C₁₋₁₀-Alkoxyl, von denen jedes gegebenenfalls unabhängig mit 1-3 Substituenten von R¹³, R¹⁴ R¹⁵ oder R¹⁶ substituiert ist;
R¹³ ist NR¹⁴R¹⁵, NR¹⁵R¹⁵, OR¹⁴; SR¹⁴, OR¹⁵; SR¹⁵, C(O)R¹⁴, OC(O)R¹⁴, COOR¹⁴, C(O)R¹⁵, OC(O)R¹⁵, COOR¹⁵, C(O)NR¹⁴R¹⁵, C(O)NR¹⁵R¹⁵, NR¹⁴C(O)R¹⁴, NR¹⁵C(O)R¹⁴, NR¹⁴C(O)R¹⁵, NR¹⁵C(O)R¹⁵, NR¹⁵C(O)NR¹⁴R¹⁵, NR¹⁵C(O)NR¹⁵R¹⁵, NR¹⁵(COOR¹⁴), NR¹⁵(COOR¹⁵), OC(O)NR¹⁴R¹⁵, OC(O)NR¹⁵R¹⁵, S(O)₂R¹⁴, S(O)₂R¹⁵, S(O)₂NR¹⁴R¹⁵, S(O)₂NR¹⁵R¹⁵, NR¹⁴S(O)₂NR¹⁴R¹⁵, NR¹⁵S(O)₂NR¹⁵R¹⁵, NR¹⁴S(O)₂R¹⁴ oder NR¹⁵S(O)₂R¹⁵;
R¹⁴ ist Phenyl, Pyridyl, Pyrimidinyl, Thiophenyl, Furyl, Tetrahydrofuryl, Pyrrolyl, Pyrazolyl, Thieno-pyrazolyl, Imidazolyl, Triazolyl, Thiazolyl, Thiadiazolyl, Benzothiazolyl, Oxazolyl, Oxadiazolyl, Benzoxazolyl, Benzoxadiazolyl, Isoxazolyl, Isothiazolyl, Indolyl, Azaindolyl, Isoindolyl, Indazolyl, Benzofuranyl, Benzothiophenyl, Benzimidazolyl, Pyrrolidinyl, Pyrazolinyl, Morpholinyl, Piperidinyl, Piperazinyl, 2,3-Dihydro-1,4-benzoxazinyl, 1,3-Benzodioxolyl, Cyclopropyl, Cyclobutyl, Azetidinyl, Cyclopentyl und Cyclohexyl, von denen jedes gegebenenfalls unabhängig mit 1-3 Substituenten von R¹⁵ oder R¹⁶ substituiert ist;
R¹⁵ ist H oder C₁₋₁₀-Alkyl, C₂₋₁₀-Alkenyl, C₂₋₁₀-Alkinyl, C₃₋₁₀-Cycloalkyl, C₄₋₁₀-Cycloalkenyl, C₁₋₁₀-Alkylamino-, C₁₋₁₀-Dialkylamino-, C₁₋₁₀-Alkoxyl oder C₁₋₁₀-Thioalkoxyl, von denen jedes gegebenenfalls unabhängig mit 1-3 Substituenten von R¹⁶ substituiert ist; und
R¹⁶ ist H, Halogen, Halogenalkyl, CN, OH, NO₂, NH₂, OH, Methyl, Methoxyl, Ethyl, Ethoxyl, Propyl, Propoxyl, Isopropyl, Cyclopropyl, Butyl, Isobutyl, tert-Butyl, Methylamino, Dimethylamino, Ethylamino, Diethylamino, Isopropylamino, Oxo, Acetyl, Benzyl oder ein Ringsystem, ausgewählt aus Phenyl, Pyridyl, Thiophenyl, Furyl, Tetrahydrofuryl, Pyrrolyl, Pyrazolyl, Thieno-pyrazolyl, Imidazolyl, Triazolyl, Thiazolyl, Thiadiazolyl, Benzothiazolyl, Oxazolyl, Oxadiazolyl, Benzoxazolyl, Benzoxadiazolyl, Isoxazolyl, Isothiazolyl, Indolyl, Azaindolyl, Isoindolyl, Indazolyl, Benzofuranyl, Benzothiophenyl, Benzimidazolyl, Pyrrolidinyl, Pyrazolinyl, Morpholinyl, Piperidinyl, Piperazinyl, Cyclopropyl, Cyclobutyl, Azetidinyl, Cyclopentyl und Cyclohexyl, wobei das Ringsystem gegebenenfalls unabhängig mit 1-3 Substituenten von Halogen, Halogenalkyl, CN, NO₂, NH₂, OH, Methyl, Methoxyl, Ethyl, Ethoxyl, Propyl, Propoxyl, Isopropyl, Cyclopropyl, Butyl, Isobutyl, tert-Butyl, Methylamino, Dimethylamino, Ethylamino, Diethylamino, Isopropylamino, Benzyl oder Phenyl substituiert ist,
vorausgesetzt, dass
(1) wenn A² N ist, dann R⁴ nicht Halogen oder Hydroxyl ist.

2. Verbindung und pharmazeutisch verträgliche Salze davon, ausgewählt aus:
N-(3-(2-Amino-6-chinazolinyl)-4-methylphenyl)-2-((3-(dimethylamino)propyl)(methyl)amino)-5-(trifluormethyl)benzamid;
5-(2-Amino-6-chinazolinyl)-N-(3-chlor-2-((3-(dimethylamino)propyl)(methyl)amino)-5-(trifluormethyl)phenyl)-2-fluorbenzamid;
5-(2-Amino-6-chinazolinyl)-N-(3-(1-methyl-4-piperidinyl)-5-(trifluormethyl)phenyl)-3-thiophencarboxamid;
3-(2-Amino-6-chinazolinyl)-4-methyl-N-(2-methyl-3-((4-methyl-1-piperazinyl)methyl)phenyl)benzamid;
3-(2-Amino-6-chinazolinyl)-N-(4-(1,1-dimethylethyl)-3-((N,N-dimethylglycyl)amino)phenyl)-4-methylbenzamid;
3-(((3-(2-Amino-6-chinazolinyl)-4-methylphenyl)carbonyl)amino)-2,6-difluor-N-methylbenzamid;
3-(2-Amino-6-chinazolinyl)-N-(2-fluor-4-(1-pyrrolidinyl)-3-(1-pyrrolidinylcarbonyl)phenyl)-4-methylbenzamid;
3-(2-Amino-6-chinazolinyl)-4-methyl-N-(4-methyl-3-((4-morpholinylacetyl)amino)phenyl)benzamid;
3-(2-Amino-6-chinazolinyl)-4-methyl-N-(2-methyl-3-((4-morpholinylacetyl)amino)phenyl)benzamid;
3-(2-Amino-6-chinazolinyl)-N-(3-((N,N-diethylglycyl)amino)-2-methylphenyl)-4-methylbenzamid;
3-(2-Amino-6-chinazolinyl)-N-(1-(N,N-diethylglycyl)-3,3-dimethyl-2,3-dihydro-1H-indol-6-yl)-4-methylbenzamid;
3-(2-Amino-6-chinazolinyl)-N-(3,3-dimethyl-1-(4-morpholinylacetyl)-2,3-dihydro-1H-indol-6-yl)-4-methylbenzamid;
3-(2-Amino-6-chinazolinyl)-4-methyl-N-(2-methyl-3-(trifluormethyl)phenyl)benzamid;
3-(2-Amino-6-chinazolinyl)-4-methyl-N-(3-((trifluormethyl)oxy)phenyl)benzamid;
Phenylmethyl-3-(2-amino-6-chinazolinyl)-4-methylbenzoat;
3-(2-Amino-6-chinazolinyl)-N-(6-chlor-3-pyridinyl)-4-methylbenzamid;
3-(2-Amino-6-chinazolinyl)-N-(4-(cyclopropylethinyl)-3-(trifluormethyl)phenyl)-4-methylbenzamid;
3-(2-Amino-6-chinazolinyl)-N-(4-brom-3-(trifluormethyl)phenyl)-4-methylbenzamid;
3-(2-Amino-6-chinazolinyl)-4-methyl-N-(4-(phenylethinyl)-3-(trifluormethyl)phenyl)benzamid;
3-(2-Amino-6-chinazolinyl)-4-chlor-N-(3-(trifluormethyl)phenyl)benzamid;
3-(2-Amino-6-chinazolinyl)-4-methyl-N-(2-((2,2,2-trifluorethyl)oxy)-5-(trifluormethyl)phenyl)benzamid;
3-(2-Amino-6-chinazolinyl)-4-methyl-N-(4-((3-(1-piperidinyl)propyl)oxy)phenyl)benzamid;
3-(2-Amino-6-chinazolinyl)-4-methyl-N-(3-((3-(1-piperidinyl)propyl)oxy)phenyl)benzamid;
3-(2-Amino-6-chinazolinyl)-N-(4-((3-(dimethylamino)propyl)oxy)phenyl)-4-methylbenzamid;
3-(2-Amino-6-chinazolinyl)-N-(4-((2-(diethylamino)ethyl)oxy)-2-(methyloxy)phenyl)-4-methylbenzamid;
3-(2-Amino-6-chinazolinyl)-N-(5-((2-(diethylamino)ethyl)oxy)-2-(methyloxy)phenyl)-4-methylbenzamid;
3-(2-Amino-6-chinazolinyl)-N-(2,4-bis(methyloxy)-5-(trifluormethyl)phenyl)-4-methylbenzamid;
3-(2-Amino-6-chinazolinyl)-N-(5-((diethylamino)sulfonyl)-2-(methyloxy)phenyl)-4-methylbenzamid;
3-(2-Amino-6-chinazolinyl)-N-(2,3-dichlorphenyl)-4-methylbenzamid;
3-(2-Amino-6-chinazolinyl)-N-(2,6-bis(methyloxy)-3-pyridinyl)-4-methylbenzamid;
N-(2-(Acetylamino)-5-(trifluormethyl)phenyl)-3-(2-amino-6-chinazolinyl)-4-methylbenzamid;
3-(2-Amino-6-chinazolinyl)-4-methyl-N-(2-(methyloxy)-5-((3-(4-methyl-1-piperazinyl)propyl)oxy)phenyl)benzamid;
3-(2-Amino-6-chinazolinyl)-N-(2,2-difluor-1,3-benzodioxol-4-yl)-4-methylbenzamid;
3-(2-Amino-6-chinazolinyl)-4-methyl-N-(2,2,3,3-tetrafluor-2,3-dihydro-1,4-benzodioxin-5-yl)benzamid;
3-(7-Isochinolinyl)-N-(3-(trifluormethyl)phenyl)benzamid;
3-(1-Hydroxy-7-isochinolinyl)-N-(3-(trifluormethyl)phenyl)benzamid;
3-(2-Amino-6-chinazolinyl)-N-(3-(trifluormethyl)phenyl)benzamid;
3-(7-Isochinolinyl)-N-(2-(1-piperidinyl)-5-(trifluormethyl)phenyl)benzamid;
3-(2-Amino-6-chinazolinyl)-N-(2-(1-piperidinyl)-5-(trifluormethyl)phenyl)benzamid;
3-(2-(Methylamino)-6-chinazolinyl)-N-(3-(trifluormethyl)phenyl)benzamid;
4-(2-Amino-6-chinazolinyl)-N-(3-(trifluormethyl)phenyl)benzamid;
3-(2-Amino-6-chinazolinyl)-4-methyl-N-(2-(4-methyl-1-piperazinyl)-5-(trifluormethyl)phenyl)benzamid;
3-(2-Amino-6-chinazolinyl)-N-(2-((3R)-3-(dimethylamino)-1-pyrrolidinyl)-5-(trifluormethyl)phenyl)-4-methylbenzamid;
3-(2-Amino-6-chinazolinyl)-4-methyl-N-(2-(1-piperidinyl)-5-(trifluormethyl)phenyl)benzamid;
3-(2-Amino-6-chinazolinyl)-N-(2,5-bis(trifluormethyl)phenyl)-4-methylbenzamid;
3-(2-Amino-6-chinazolinyl)-N-(2,5-bis(1,1-dimethylethyl)phenyl)-4-methylbenzamid;
3-(2-Amino-6-chinazolinyl)-4-methyl-N-(2-(4-morpholinyl)-5-(trifluormethyl)phenyl)benzamid;
3-(2-Amino-6-chinazolinyl)-4-methyl-N-(2-(1-pyrrolidinyl)-5-(trifluormethyl)phenyl)benzamid;
3-(2-Amino-6-chinazolinyl)-N-(2-(dimethylamino)-5-(trifluormethyl)phenyl)-4-methylbenzamid;
N-(3-(2-Amino-6-chinazolinyl)-5-(trifluormethyl)phenyl)-3-(trifluormethyl)benzamid;
N-(3-(2-Amino-6-chinazolinyl)-5-(trifluormethyl)phenyl)-3-(1-methylethyl)benzamid;
3-(2-Amino-6-chinazolinyl)-4-methyl-N-(2-((3R)-1-methyl-3-piperidinyl)-5-(trifluormethyl)phenyl)benzamid;
3-(2-Amino-6-chinazolinyl)-N-(3-(1-methylethyl)phenyl)benzamid;
3-(2-Amino-6-chinazolinyl)-N-(2-(2-(dimethylamino)-1,1-dimethylethyl)-5-(trifluormethyl)phenyl)benzamid;
3-(2-Amino-6-chinazolinyl)-N-(2-(2-(dimethylamino)-1,1-dimethylethyl)-5-(trifluormethyl)phenyl)-4-methylbenzamid;
3-(2-Amino-6-chinazolinyl)-4-methyl-N-(2-(methyl((3S)-1-methyl-3-pyrrolidinyl)amino)-5-(trifluormethyl)phenyl)benzamid;
N-(2-(2-(Dimethylamino)-1,1-dimethylethyl)-5-(trifluormethyl)phenyl)-3-(2-(methylamino)-6-chinazolinyl)benzamid;
3-(2-Amino-6-chinazolinyl)-N-(2-(methyl((3R)-1-methyl-3-pyrrolidinyl)amino)-5-(trifluormethyl)phenyl)benzamid;
3-(2-Amino-6-chinazolinyl)-N-(2-(4-methyl-1-piperazinyl)-5-(trifluormethyl)phenyl)benzamid;
3-(2-Amino-6-chinazolinyl)-N-(2-((3-(dimethylamino)propyl)(methyl)amino)-5-(trifluormethyl)phenyl)benzamid;
3-(2-(Methylamino)-6-chinazolinyl)-N-(2-((4-methyl-1-piperazinyl)methyl)-5-(trifluormethyl)phenyl)benzamid;
3-(2-Amino-6-chinazolinyl)-4-methyl-N-(2-((4-methyl-1-piperazinyl)methyl)-5-(trifluormethyl)phenyl)benzamid;
3-(2-Amino-6-chinazolinyl)-N-(2-((3-(dimethylamino)propyl)(methyl)amino)-5-(1,1-dimethylethyl)phenyl)-4-methylbenzamid;
3-(2,4-Diaminopyrido[2,3-d]pyrimidin-6-yl)-4-methyl-N-(3-(trifluormethyl)phenyl)benzamid;
5-(2-Amino-6-chinazolinyl)-2-fluor-N-(2-(1-piperidinyl)-5-(trifluormethyl)phenyl)benzamid;
5-(2-Amino-6-chinazolinyl)-N-(2-((3-(dimethylamino)propyl)(methyl)amino)-5-(trifluormethyl)phenyl)-2-fluorbenzamid;
3-(2-Amino-6-chinazolinyl)-N-(2-(2-(dimethylamino)ethyl)-5-(trifluormethyl)phenyl)-4-methylbenzamid;
5-(2-Amino-6-chinazolinyl)-2-fluor-N-(2-(methyl((3R)-1-methyl-3-pyrrolidinyl)amino)-5-(trifluormethyl)phenyl)benzamid;
3-(2-Amino-6-chinazolinyl)-4-methyl-N-(2-((2-(1-pyrrolidinyl)ethyl)oxy)-5-(trifluormethyl)phenyl)benzamid;
3-(2-Amino-6-chinazolinyl)-N-(2-((3-(dimethylamino)propyl)(methyl)amino)-5-(1-methylethyl)phenyl)benzamid;
5-(2-Amino-6-chinazolinyl)-2-fluor-N-(2-(4-methyl-1-piperazinyl)-5-(trifluormethyl)phenyl)benzamid;
3-(2-Amino-6-chinazolinyl)-N-(2-((3S)-3-(dimethylamino)-1-piperidinyl)-5-(trifluormethyl)phenyl)-4-methylbenzamid;
5-(2-Amino-6-chinazolinyl)-N-(2-((3R)-3-(dimethylamino)-1-pyrrolidinyl)-5-(trifluormethyl)phenyl)-2-fluorbenzamid;
3-(2-Amino-6-chinazolinyl)-N-(2-(4-(dimethylamino)-1-piperidinyl)-5-(trifluormethyl)phenyl)-4-methylbenzamid;
3-(2-Amino-6-chinazolinyl)-4-methyl-N-(4-((trifluormethyl)oxy)phenyl)benzamid;
3-(2-Amino-6-chinazolinyl)-N-(2-((3-(dimethylamino)propyl)(methyl)amino)-5-(1,1-dimethylethyl)phenyl)benzamid;
5-(2-Amino-6-chinazolinyl)-N-(2-((3-(dimethylamino)propyl)(methyl)amino)-5-(1,1-dimethylethyl)phenyl)-2-fluorbenzamid;
5-(2-Amino-6-chinazolinyl)-N-(2-((3-(dimethylamino)propyl)(methyl)amino)-5-ethinylphenyl)-2-fluorbenzamid;
5-(2-Amino-6-chinazolinyl)-N-(2-((2-(dimethylamino)ethyl)(methyl)amino)-5-(trifluormethyl)phenyl)-2-fluorbenzamid;
5-(2-Amino-6-chinazolinyl)-N-(2-(dimethylamino)-5-(trifluormethyl)phenyl)-2-fluorbenzamid;
N-(2-((3-(Dimethylamino)propyl)(methyl)amino)-5-(trifluormethyl)phenyl)-2-fluor-5-(2-(methylamino)-6-chinazolinyl)benzamid;
5-(2-Amino-6-chinazolinyl)-N-(2-((3-(dimethylamino)propyl)(methyl)amino)-5-(pentafluorethyl)phenyl)-2-fluorbenzamid;
6-(2,6-Dimethylphenyl)-2-chinazolinamin;
3-(2-Amino-6-chinazolinyl)-N-(2-(dimethylamino)-5-(trifluormethyl)phenyl)benzamid;
3-(2-Amino-6-chinazolinyl)-N-(3-(1,1-dimethylethyl)phenyl)benzamid;
6-(2,6-Bis(methyloxy)phenyl)-2-chinazolinamin;
N-(3-(1,1-Dimethylethyl)phenyl)-3-(2-(methylamino)-6-chinazolinyl)benzamid;
3-(2-Amino-6-chinazolinyl)-N-(3,5-bis(methyloxy)phenyl)benzamid;
3-(2-Amino-6-chinazolinyl)-N-(5-(1,1-dimethylethyl)-3-isoxazolyl)benzamid;
3-(2-Amino-6-chinazolinyl)-N-(3-((1,1,2,2-tetrafluorethyl)oxy)phenyl)benzamid;
3-(2-Amino-6-chinazolinyl)-N-(5-(1,1-dimethylethyl)-1H-pyrazol-3-yl)benzamid;
3-(2-Amino-6-chinazolinyl)-N-(3-chlorphenyl)benzamid;
5-(2-Amino-6-chinazolinyl)-N-(5-brom-2-((3-(dimethylamino)propyl)(methyl)amino)phenyl)-2-fluorbenzamid;
5-(2-Amino-6-chinazolinyl)-N-(2-((3-(dimethylamino)propyl)oxy)-5-(trifluormethyl)phenyl)-2-fluorbenzamid;
5-(2-Amino-6-chinazolinyl)-N-(3-(trifluormethyl)phenyl)-2-thiophencarboxamid;
3-(2-Amino-6-chinazolinyl)-4-methyl-N-(3-(1-methyl-4-piperidinyl)-5-(trifluormethyl)phenyl)benzamid;
5-(2-Amino-6-chinazolinyl)-N-(2-((3-(dimethylamino)propyl)(methyl)amino)-5-(trifluormethyl)phenyl)-2-thiophencarboxamid;
5-(2-Amino-6-chinazolinyl)-N-(3-(trifluormethyl)phenyl)-3-thiophencarboxamid;
5-(2-Amino-6-chinazolinyl)-N-(2-(2-(dimethylamino)ethyl)-5-(trifluormethyl)phenyl)-2-fluorbenzamid;
N-(4-(2-Amino-6-chinazolinyl)-3-methylphenyl)-N'-(3-fluor-5-(trifluormethyl)phenyl)harnstoff;
5-(2-Amino-6-chinazolinyl)-N-(5-chlor-2-((3-(dimethylamino)propyl)(methyl)amino)phenyl)-2-fluorbenzamid;
4-Methyl-3-(2-(4-methyl-1-piperazinyl)-6-chinazolinyl)-N-(3-(trifluormethyl)phenyl)benzamid;
N-(4-(2-Amino-6-chinazolinyl)-3-methylphenyl)-N'-(5-chlor-2-(methyloxy)phenyl)harnstoff;
4-(2-Amino-6-chinazolinyl)-N-(3-(trifluormethyl)phenyl)-2-pyridincarboxamid;
3-(2-Amino-6-chinazolinyl)-N-(2-(((3-(dimethylamino)propyl)(methyl)amino)sulfonyl)-5-(trifluormethyl)phenyl)-4-methylbenzamid;
4-(2-Amino-6-chinazolinyl)-N-(2-((3-(dimethylamino)propyl)(methyl)amino)-5-(trifluormethyl)phenyl)-2-pyridincarboxamid;
4-(2-Amino-6-chinazolinyl)-N-(2-((3-(dimethylamino)propyl)(methyl)amino)-5-(trifluormethyl)phenyl)-2-thiophencarboxamid;
5-(2-Amino-6-chinazolinyl)-N-(2-((3-(dimethylamino)propyl)(methyl)amino)-5-(trifluormethyl)phenyl)-3-thiophencarboxamid;
5-(2-Amino-6-chinazolinyl)-N-(2-((3-(dimethylamino)propyl)(methyl)amino)-5-(trifluormethyl)phenyl)-2-(methyloxy)benzamid;
5-(2-Amino-6-chinazolinyl)-2-chlor-N-(2-((3-(dimethylamino)propyl)(methyl)amino)-5-(trifluormethyl)phenyl)benzamid;
3-(2-Amino-6-chinazolinyl)-5-brom-N-(2-((3-(dimethylamino)propyl)(methyl)amino)-5-(trifluormethyl)phenyl)benzamid;
3-(2-Amino-6-chinazolinyl)-N-(5-chlor-2-((3-(dimethylamino)propyl)(methyl)amino)phenyl)-4-methylbenzamid;
5-(2-Amino-6-chinazolinyl)-N-(2-((3-(dimethylamino)propyl)(methyl)amino)-5-(trifluormethyl)phenyl)-2,3-dihydro-1-benzofuran-7-carboxamid;
5-(2-Amino-6-chinazolinyl)-2-brom-N-(2-((3-(dimethylamino)propyl)(methyl)amino)-5-(trifluormethyl)phenyl)benzamid;
3-(2-Amino-6-chinazolinyl)-N-(3-((trifluormethyl)oxy)phenyl)benzamid;
5-(2-Amino-6-chinazolinyl)-2,3-bis(methyloxy)-N-(3-(trifluormethyl)phenyl)benzamid;
5-(2-Amino-6-chinazolinyl)-2-(methyloxy)-N-(3-(trifluormethyl)phenyl)benzamid;
5-(2-Amino-6-chinazolinyl)-N-(5-cyclopropyl-2-((3-(dimethylamino)propyl)(methyl)amino)phenyl)-2-fluorbenzamid;
5-(2-Amino-6-chinazolinyl)-N-(2-((3-(dimethylamino)propyl)(methyl)amino)-5-(trifluormethyl)phenyl)-2,3-bis(methyloxy)benzamid;
5-(2-Amino-6-chinazolinyl)-N-(3-(trifluormethyl)phenyl)-2,3-dihydro-1-benzofuran-7-carboxamid;
5-(2-Amino-6-chinazolinyl)-3-brom-N-(2-((3-(dimethylamino)propyl)(methyl)amino)-5-(trifluormethyl)phenyl)-2-(methyloxy)benzamid;
3-(2-Amino-6-chinazolinyl)-5-brom-N-(2-((3-(dimethylamino)propyl)(methyl)amino)-5-(trifluormethyl)phenyl)-2-(methyloxy)benzamid;
5-(2-Amino-6-chinazolinyl)-N-(2-((3-(dimethylamino)propyl)(methyl)amino)-5-(trifluormethyl)phenyl)-1-methyl-2-oxo-1,2-dihydro-3-pyridincarboxamid;
5-(2-Amino-6-chinazolinyl)-2-fluor-N-(3-((4-methyl-1-piperazinyl)methyl)-5-(trifluormethyl)phenyl)benzamid;
5-(2-Amino-6-chinazolinyl)-2,3-bis(methyloxy)-N-(3-((4-methyl-1-piperazinyl)methyl)-5-(trifluormethyl)phenyl)benzamid;
1-(4-(2-Amino-6-chinazolinyl)-3-methylphenyl)-3-(3-(trifluormethyl)phenyl)-2-imidazolidinon;
5-(2-Amino-6-chinazolinyl)-2-(methyloxy)-N-(3-((4-methyl-1-piperazinyl)methyl)-5-(trifluormethyl)phenyl)benzamid;
N-(4-(2-Amino-6-chinazolinyl)phenyl)-N'-(3-fluor-5-(trifluormethyl)phenyl)harnstoff;
N-(4-(2-Amino-6-chinazolinyl)phenyl)-N'-(3-fluorphenyl)harnstoff;
5-(2-Amino-6-chinazolinyl)-2-((2-(dimethylamino)ethyl)oxy)-N-(3-(trifluormethyl)phenyl)benzamid;
5-(2-Amino-6-chinazolinyl)-2-(methyloxy)-N-(3-((trifluormethyl)oxy)phenyl)benzamid;
5-(2-Amino-6-chinazolinyl)-2,3,4-tris(methyloxy)-N-(3-(trifluormethyl)phenyl)benzamid;
5-(2-Amino-6-chinazolinyl)-N-(2-((3-(dimethylamino)propyl)(methyl)amino)-5-(trifluormethyl)phenyl)-2,3,4-tris(methyloxy)benzamid;
5-(2-Amino-6-chinazolinyl)-N-(2-((3-(dimethylamino)propyl)(methyl)amino)-5-(trifluormethyl)phenyl)-2-(ethyloxy)benzamid;
3-(2-Amino-6-chinazolinyl)-2,6-bis(methyloxy)-N-(3-(trifluormethyl)phenyl)benzamid;
5-(2-Amino-6-chinazolinyl)-2-(dimethylamino)-N-(2-((3-(dimethylamino)propyl)(methyl)amino)-5-(trifluormethyl)phenyl)benzamid;
N-(4-(2-Amino-6-chinazolinyl)-3-methylphenyl)-3-(trifluormethyl)benzamid;
N-(4-(2-Amino-6-chinazolinyl)phenyl)-3-(trifluormethyl)benzamid;
N-(4-(4-Amino-6-chinazolinyl)phenyl)-N'-(3-fluorphenyl)harnstoff;
3-(2-Amino-6-chinazolinyl)-N-(2-((3-(dimethylamino)propyl)(methyl)amino)-5-(trifluormethyl)phenyl)-2,6-bis(methyloxy)benzamid;
5-(2-Amino-6-chinazolinyl)-2-fluor-N-(3-(trifluormethyl)phenyl)benzamid;
N-(3-(2-Amino-6-chinazolinyl)-4-methylphenyl)-3-(1-methylethyl)benzamid;
N-(5-(2-Amino-6-chinazolinyl)-2-fluorphenyl)benzamid;
N-(5-(2-Amino-6-chinazolinyl)-2-fluorphenyl)cyclopropancarboxamid;
N-(5-(2-Amino-6-chinazolinyl)-2-fluorphenyl)-2,3-dichlorbenzamid;
N-(5-(2-Amino-6-chinazolinyl)-2-fluorphenyl)-4-(1,1-dimethylethyl)benzamid;
N-(5-(2-Amino-6-chinazolinyl)-2-fluorphenyl)-3-fluor-5-(trifluormethyl)benzamid;
N-(5-(2-Amino-6-chinazolinyl)-2-fluorphenyl)-3,5-bis(methyloxy)benzamid;
N-(5-(2-Amino-6-chinazolinyl)-2-fluorphenyl)-3-(1-methylethyl)benzamid;
N-(5-(2-Amino-6-chinazolinyl)-2-fluorphenyl)-2-fluor-5-(trifluormethyl)benzamid;
N-(5-(2-Amino-6-chinazolinyl)-2-fluorphenyl)-2-(4-methyl-1-piperazinyl)-5-(trifluormethyl)benzamid;
N-(5-(2-Amino-6-chinazolinyl)-2-fluorphenyl)-2-((3-(dimethylamino)propyl)(methyl)amino)-5-(trifluormethyl)benzamid;
N-(5-(2-Amino-6-chinazolinyl)-2-fluorphenyl)-2-((3R)-3-(dimethylamino)-1-pyrrolidinyl)-5-(trifluormethyl)benzamid;
3-(2-Amino-6-chinazolinyl)-N-(2-(1,1-dioxido-4-thiomorpholinyl)-5-(trifluormethyl)phenyl)-4-methylbenzamid;
3-(2-Amino-6-chinazolinyl)-N-(2-((3-(dimethylamino)propyl)(methyl)amino)-5-(trifluormethyl)phenyl)-4-methylbenzamid;
3-(2-Amino-6-chinazolinyl)-4-methyl-N-phenylbenzamid;
3-(2-Amino-6-chinazolinyl)-4-methyl-N-(4-(phenyloxy)phenyl)benzamid;
3-(2-Amino-6-chinazolinyl)-4-methyl-N-(2-(methyloxy)phenyl)benzamid;
3-(2-Amino-6-chinazolinyl)-N-(2,4-bis(methyloxy)phenyl)-4-methylbenzamid;
3-(2-Amino-6-chinazolinyl)-N-cyclohexyl-4-methylbenzamid;
3-(2-Amino-6-chinazolinyl)-4-methyl-N-(2-(1-piperidinyl)phenyl)benzamid;
3-(2-Amino-6-chinazolinyl)-N-(4-chlor-3-(trifluormethyl)phenyl)-4-methylbenzamid;
3-(2-Amino-6-chinazolinyl)-N-cyclopentyl-4-methylbenzamid;
N-(2-((3-(Dimethylamino)propyl)(methyl)amino)-5-(trifluormethyl)phenyl)-4-methyl-3-(2-(methylamino)-6-chinazolinyl)benzamid;
3-(2-Amino-6-chinazolinyl)-N-(2-((3-(dimethylamino)propyl)(methyl)amino)phenyl)-4-methylbenzamid;
3-(2-Amino-6-chinazolinyl)-N-(2-((3-(dimethylamino)propyl)(methyl)amino)-4-methylphenyl)-4-methylbenzamid;
3-(2-Amino-6-chinazolinyl)-4-methyl-N-(4-(1-methyl-4-piperidinyl)phenyl)benzamid;
3-(2-Amino-6-chinazolinyl)-4-methyl-N-(2-(4-methyl-1-piperazinyl)phenyl)benzamid;
3-(2-Amino-6-chinazolinyl)-4-methyl-N-(2-methyl-6-(methyloxy)-3-(trifluormethyl)phenyl)benzamid;
3-(2-Amino-6-chinazolinyl)-N-(3-chlor-4-((2-(1-piperidinyl)ethyl)oxy)phenyl)-4-methylbenzamid;
3-(2-Amino-6-chinazolinyl)-N-(2-fluor-5-(trifluormethyl)phenyl)-4-methylbenzamid;
3-(2-Amino-6-chinazolinyl)-4-methyl-N-(5-chinolinyl)benzamid;
3-(2-Amino-6-chinazolinyl)-4-methyl-N-(8-chinolinyl)benzamid;
3-(2-Amino-6-chinazolinyl)-N-(5,6-bis(methyloxy)-1,3-cyclohexadien-1-yl)-4-methylbenzamid;
3-(2-Amino-6-chinazolinyl)-N-(5-chlor-6-(methyloxy)-1,3-cyclohexadien-1-yl)-4-methylbenzamid;
3-(2-Amino-6-chinazolinyl)-N-(2-((3-(dimethylamino)propyl)(methyl)amino)-4,5-dimethylphenyl)-4-methylbenzamid;
3-(2-Amino-6-chinazolinyl)-N-(6-((3-(dimethylamino)propyl)(methyl)amino)-2-methyl-3-(trifluormethyl)phenyl)-4-methylbenzamid;
3-(2-Amino-6-chinazolinyl)-N-(2-((3-(dimethylamino)propyl)(methyl)amino)-4-(trifluormethyl)phenyl)-4-methylbenzamid;
3-(2-Amino-6-chinazolinyl)-4-methyl-N-(4-(methyloxy)-3-(trifluormethyl)phenyl)benzamid;
4-Methyl-3-(2-(methylamino)-6-chinazolinyl)-N-(2-methyl-3-(trifluormethyl)phenyl)benzamid;
N-(4-Chlor-3-(trifluormethyl)phenyl)-4-methyl-3-(2-(methylamino)-6-chinazolinyl)benzamid;
3-(2-Amino-6-chinazolinyl)-N-(4-chlor-2-methyl-3-(trifluormethyl)phenyl)-4-methylbenzamid;
N-(4-Chlor-2-methyl-3-(trifluormethyl)phenyl)-4-methyl-3-(2-(methylamino)-6-chinazolinyl)benzamid;
N-(2-Fluor-5-(trifluormethyl)phenyl)-4-methyl-3-(2-(methylamino)-6-chinazolinyl)benzamid;
3-(2-Amino-6-chinazolinyl)-4-methyl-N-(3-(trifluormethyl)phenyl)benzamid;
N-(3-(2-Amino-6-chinazolinyl)-4-methylphenyl)-N'-(3-(trifluormethyl)phenyl)harnstoff;
3-(2-Amino-6-chinazolinyl)-4-methyl-N-(4-(4-methyl-1-piperazinyl)phenyl)benzamid;
3-(2-Amino-6-chinazolinyl)-4-methyl-N-(phenylmethyl)benzamid;
N-(1-Acetyl-3,3-dimethyl-2,3-dihydro-1H-indol-6-yl)-3-(2-amino-6-chinazolinyl)-4-methylbenzamid;
N-(1-Acetyl-2,3-dihydro-1H-indol-6-yl)-3-(2-amino-6-chinazolinyl)-4-methylbenzamid;
N-(3-(2-Amino-6-chinazolinyl)-4-methylphenyl)-N'-phenylharnstoff;
N-(3-(2-Amino-6-chinazolinyl)-4-methylphenyl)-N'-(4-(phenyloxy)phenyl)harnstoff;
N-(3-(2-Amino-6-chinazolinyl)-4-methylphenyl)-N'-(3,5-bis(methyloxy)phenyl)harnstoff;
3-(2-Amino-6-chinazolinyl)-4-methyl-N-(3-pyridinylmethyl)benzamid;
3-(2-Amino-6-chinazolinyl)-4-methyl-N-(2-pyridinylmethyl)benzamid;
3-(2-Amino-6-chinazolinyl)-4-methyl-N-((1R)-1-phenylethyl)benzamid;
3-(2-Amino-6-chinazolinyl)-4-methyl-N-((3-(trifluormethyl)phenyl)methyl)benzamid;
3-(2-Amino-6-chinazolinyl)-N-(3,5-bis(trifluormethyl)phenyl)-4-methylbenzamid;
3-(2-Amino-6-chinazolinyl)-N-(2,3-dihydro-1H-inden-4-yl)-4-methylbenzamid;
3-(2-Amino-6-chinazolinyl)-4-methyl-N-(2-(methylsulfanyl)-3-(trifluormethyl)phenyl)benzamid;
N-(3-(2-Amino-6-chinazolinyl)-4-methylphenyl)-N'-(phenylmethyl)harnstoff;
N-(3-(2-Amino-6-chinazolinyl)-4-methylphenyl)-N'-(3-(trifluormethyl)phenyl)thioharnstoff;
4-Methyl-3-(2-(methylamino)-6-chinazolinyl)-N-(3-(trifluormethyl)phenyl)benzamid;
N-(3-(2-Amino-6-chinazolinyl)-4-methylphenyl)-N'-phenylthioharnstoff;
3-(2-Amino-6-chinazolinyl)-4-methyl-N-(4-(pentafluorethyl)phenyl)benzamid;
3-(Trifluormethyl)phenyl-3-(2-amino-6-chinazolinyl)-4-methylphenylcarbamat;
N-(6-(2-Methyl-5-((phenylcarbonyl)amino)phenyl)-2-chinazolinyl)benzamid;
N-(3-(2-Amino-6-chinazolinyl)-4-methylphenyl)benzamid;
N-(3-(2-Amino-6-chinazolinyl)-4-methylphenyl)-3-(trifluormethyl)benzamid;
3-(2-Amino-6-chinazolinyl)-N-(2-fluor-3-(trifluormethyl)phenyl)-4-methylbenzamid;
3-(2-Amino-6-chinazolinyl)-4-methyl-N-((1S)-1-phenylethyl)benzamid;
3-(2-Amino-6-chinazolinyl)-N-((1R)-2-(dimethylamino)-1-phenylethyl)-4-methylbenzamid;
3-(2-Amino-6-chinazolinyl)-4-methyl-N-((1R)-2-(4-methyl-1-piperazinyl)-1-phenylethyl)benzamid;
N-(3-(2-Amino-6-chinazolinyl)-4-methylphenyl)-4-(1,1-dimethylethyl)benzamid;
N-(3-(2-Amino-6-chinazolinyl)-4-methylphenyl)-2,3-dichlorbenzamid;
N-(3-(2-Amino-6-chinazolinyl)-4-methylphenyl)-3,5-bis(trifluormethyl)benzamid;
N-(3-(2-Amino-6-chinazolinyl)-4-methylphenyl)-2-fluor-3-(trifluormethyl)benzamid;
N-(3-(2-Amino-6-chinazolinyl)-4-methylphenyl)-4-((trifluormethyl)oxy)benzamid;
N-(3-(2-Amino-6-chinazolinyl)-4-methylphenyl)-3,5-dichlorbenzamid;
N-(3-(2-Amino-6-chinazolinyl)-4-methylphenyl)-3-fluor-5-(trifluormethyl)benzamid;
N-(3-(2-Amino-6-chinazolinyl)-4-methylphenyl)-4-(methyloxy)-3-(trifluormethyl)benzamid;
N-(3-(2-Amino-6-chinazolinyl)-4-methylphenyl)-2,3-dichlorbenzamid;
N-(3-(2-Amino-6-chinazolinyl)-4-methylphenyl)-2-methyl-3-(trifluormethyl)benzamid;
N-(3-(2-Amino-6-chinazolinyl)-4-methylphenyl)-2-naphthalincarboxamid;
N-(3-(2-Amino-6-chinazolinyl)-4-methylphenyl)-1-naphthalincarboxamid;
N-(3-(2-Amino-6-chinazolinyl)-4-methylphenyl)-2,2-difluor-1,3-benzodioxol-4-carboxamid;
N-(3-(2-Amino-6-chinazolinyl)-4-methylphenyl)-5,5,8,8-tetramethyl-5,6,7,8-tetrahydro-2-naphthalincarboxamid;
N-(3-(2-Amino-6-chinazolinyl)-4-methylphenyl)-3-(1,1-dimethylethyl)-1-methyl-1H-pyrazol-5-carboxamid;
4-Methyl-3-(2-((2-(4-morpholinyl)ethyl)amino)-6-chinazolinyl)-N-(3-(trifluormethyl)phenyl)benzamid;
2,3-Dichlor-N-(4-methyl-3-(2-(methylamino)-6-chinazolinyl)phenyl)benzamid;
N-(4-Methyl-3-(2-(methylamino)-6-chinazolinyl)phenyl)-N'-(3-(trifluormethyl)phenyl)harnstoff;
N-(3-(2-Amino-6-chinazolinyl)-4-methylphenyl)-3-chlor-2-fluorbenzamid;
N-(3-(2-Amino-6-chinazolinyl)-4-methylphenyl)-3-fluor-2-(trifluormethyl)benzamid;
N-(3-(2-Amino-6-chinazolinyl)-4-methylphenyl)-1-benzofuran-2-carboxamid;
N-(3-(2-Amino-6-chinazolinyl)-4-methylphenyl)-1,3-dimethyl-1H-thieno[2,3-c]pyrazol-5-carboxamid;
N-(3-(2-Amino-6-chinazolinyl)-4-(trifluormethyl)phenyl)-3-(trifluormethyl)benzamid;
N-(3-(2-Amino-6-chinazolinyl)-4-(trifluormethyl)phenyl)-N'-(3-(trifluormethyl)phenyl)harnstoff;
2,3-Dichlor-N-(4-methyl-3-(2-((2-(4-morpholinyl)ethyl)amino)-6-chinazolinyl)phenyl)benzamid;
N-(2,3-Dihydro-1H-inden-4-yl)-4-methyl-3-(2-((2-(4-morpholinyl)ethyl)amino)-6-chinazolinyl)benzamid;
N-(3-(2-Amino-6-chinazolinyl)-4-methylphenyl)-3-(trifluormethyl)benzolsulfonamid;
3-(2-Amino-6-chinazolinyl)-4-methyl-N-((1S)-1-(1,3-thiazol-2-yl)ethyl)benzamid;
3-(2-Amino-6-chinazolinyl)-N-(2,3-dimethyl-4-(methyloxy)phenyl)-4-methylbenzamid;
N-(1-Acetyl-2,3-dihydro-1H-indol-7-yl)-3-(2-amino-6-chinazolinyl)-4-methylbenzamid;
3-(2-Amino-6-chinazolinyl)-4-methyl-N-(1-methyl-1H-indol-4-yl)benzamid;
N,N'-Bis(3-(2-amino-6-chinazolinyl)-4-methylphenyl)harnstoff;
N-(2-Fluor-3-(trifluormethyl)phenyl)-4-methyl-3-(2-((2-(4-morpholinyl)ethyl)amino)-6-chinazolinyl)benzamid;
N-(2-Fluor-3-(trifluormethyl)phenyl)-4-methyl-3-(2-((3-(4-morpholinyl)propyl)amino)-6-chinazolinyl)benzamid;
N-(2-Fluor-3-(trifluormethyl)phenyl)-4-methyl-3-(2-((2-(methyloxy)ethyl)amino)-6-chinazolinyl)benzamid;
N-(2-Fluor-3-(trifluormethyl)phenyl)-4-methyl-3-(2-((1-methyl-4-piperidinyl)amino)-6-chinazolinyl)benzamid;
N-(2-Fluor-3-(trifluormethyl)phenyl)-4-methyl-3-(2-((2-(1-pyrrolidinyl)ethyl)amino)-6-chinazolinyl)benzamid;
N-(2-Fluor-3-(trifluormethyl)phenyl)-4-methyl-3-(2-(methylamino)-6-chinazolinyl)benzamid;
3-(2-Amino-6-chinazolinyl)-N-(2,1,3-benzoxadiazol-4-yl)-4-methylbenzamid;
3-(2-Amino-6-chinazolinyl)-N-(2-(2-cyanoethyl)-2,4,5,6-tetrahydrocyclopenta[c]pyrazol-3-yl)-4-methylbenzamid;
3-(2-Amino-6-chinazolinyl)-N-(4-((3-(diethylamino)propyl)oxy)-3-(trifluormethyl)phenyl)-4-methylbenzamid;
3-(2-Amino-6-chinazolinyl)-4-methyl-N-(4-((1-methyl-4-piperidinyl)oxy)-3-(trifluormethyl)phenyl)benzamid;
3-(2-Amino-6-chinazolinyl)-N-(4-fluor-3-(trifluormethyl)phenyl)-4-methylbenzamid;
N-(4-Fluor-3-(trifluormethyl)phenyl)-4-methyl-3-(2-(methylamino)-6-chinazolinyl)benzamid;
3-(2-Amino-6-chinazolinyl)-N-(4-cyano-3-(trifluormethyl)phenyl)-4-methylbenzamid;
4-Methyl-N-(2-methyl-3-(trifluormethyl)phenyl)-3-(2-((2-(4-morpholinyl)ethyl)amino)-6-chinazolinyl)benzamid;
3-(2-(Cyclopropylamino)-6-chinazolinyl)-4-methyl-N-(3-(trifluormethyl)phenyl)benzamid;
N-(2-Fluor-3-(trifluormethyl)phenyl)-4-methyl-3-(2-((4-(4-methyl-1-piperazinyl)phenyl)amino)-6-chinazolinyl)benzamid;
N-(2-Fluor-3-(trifluormethyl)phenyl)-4-methyl-3-(2-((4-((3-(1-piperidinyl)propyl)oxy)phenyl)amino)-6-chinazolinyl)benzamid;
4-Methyl-N-(2-methyl-3-(trifluormethyl)phenyl)-3-(2-((3-(4-morpholinyl)propyl)amino)-6-chinazolinyl)benzamid;
4-Methyl-N-(2-methyl-3-(trifluormethyl)phenyl)-3-(2-((2-(1-pyrrolidinyl)ethyl)amino)-6-chinazolinyl)benzamid;
4-Methyl-N-(2-methyl-3-(trifluormethyl)phenyl)-3-(2-((3-(2-oxo-1-pyrrolidinyl)propyl)amino)-6-chinazolinyl)benzamid;
3-(2-(((1-Ethyl-4-piperidinyl)methyl)amino)-6-chinazolinyl)-4-methyl-N-(2-methyl-3-(trifluormethyl)phenyl)benzamid;
4-Methyl-3-(2-((1-methyl-4-piperidinyl)amino)-6-chinazolinyl)-N-(2-methyl-3-(trifluormethyl)phenyl)benzamid;
3-(2-(Cyclopropylamino)-6-chinazolinyl)-4-methyl-N-(2-methyl-3-(trifluormethyl)phenyl)benzamid;
6-(2,6-Dimethylphenyl)-N-(2-(4-morpholinyl)ethyl)-2-chinazolinamin;
6-(2,6-Dimethylphenyl)-N-(4-(4-methyl-1-piperazinyl)phenyl)-2-chinazolinamin;
6-(2,6-Dimethylphenyl)-N-(4-((3-(1-piperidinyl)propyl)oxy)phenyl)-2-chinazolinamin;
5-(2-Amino-6-chinazolinyl)-2-fluor-4-methyl-N-(3-(trifluormethyl)phenyl)benzamid;
2-Fluor-4-methyl-5-(2-(methylamino)-6-chinazolinyl)-N-(3-(trifluormethyl)phenyl)benzamid;
5-(2-(Cyclopropylamino)-6-chinazolinyl)-2-fluor-4-methyl-N-(3-(trifluormethyl)phenyl)benzamid;
3-(2-(Cyclopropylamino)-6-chinazolinyl)-4-methyl-N-(4-((1-methyl-4-piperidinyl)oxy)-3-(trifluormethyl)phenyl)benzamid;
3-(2-Amino-6-chinazolinyl)-4-methyl-N-(4-(trifluormethyl)phenyl)benzamid;
3-(2-Amino-6-chinazolinyl)-N-(4-(1,1-dimethylethyl)phenyl)-4-methylbenzamid;
3-(2-Amino-6-chinazolinyl)-N-(4,4-dimethyl-2-oxo-1,2,3,4-tetrahydro-7-chinolinyl)-4-methylbenzamid;
3-(2-Amino-6-chinazolinyl)-N-(4,4-dimethyl-1,2,3,4-tetrahydro-7-chinolinyl)-4-methylbenzamid;
3-(2-Amino-6-chinazolinyl)-N-(4,4-dimethyl-1,2,3,4-tetrahydro-7-isochinolinyl)-4-methylbenzamid;
5-(2-Amino-6-chinazolinyl)-2-fluor-N-(4-(trifluormethyl)phenyl)benzamid;
5-(2-Amino-6-chinazolinyl)-N-(4,4-dimethyl-2-oxo-1,2,3,4-tetrahydro-7-chinolinyl)-2-fluorbenzamid;
5-(2-Amino-6-chinazolinyl)-N-(4-cyclohexylphenyl)-2-fluorbenzamid;
5-(2-Amino-6-chinazolinyl)-2-fluor-N-(4-(4-morpholinyl)phenyl)benzamid;
5-(2-Amino-6-chinazolinyl)-N-(4-(1,1-dimethylethyl)phenyl)-2-fluorbenzamid;
3-(2-Amino-6-chinazolinyl)-5-chlor-N-(4-(trifluormethyl)phenyl)benzamid;
3-(2-Amino-6-chinazolinyl)-5-chlor-N-cyclopropylbenzamid;
3-(2-Amino-6-chinazolinyl)-5-chlor-N-(1-methylethyl)benzamid;
3-(2-Amino-6-chinazolinyl)-5-brom-N-(4-(trifluormethyl)phenyl)benzamid;
3-(2-Amino-6-chinazolinyl)-5-brom-N-(4-chlor-3-(trifluormethyl)phenyl)benzamid;
3-(2-Amino-6-chinazolinyl)-5-brom-N-(3-methyl-4-(1-methylethyl)phenyl)benzamid;
3-(2-Amino-6-chinazolinyl)-5-brom-N-(4-((trifluormethyl)oxy)phenyl)benzamid;
3-(2-Amino-6-chinazolinyl)-5-brom-N-(3-chlor-4-methylphenyl)benzamid;
3-(2-Amino-6-chinazolinyl)-5-brom-N-cyclopropylbenzamid;
N-(3-(2-Amino-6-chinazolinyl)phenyl)-5-(dimethylamino)-1-naphthalinsulfonamid;
N-(5-(2-Amino-6-chinazolinyl)-2-methylphenyl)-3-(trifluormethyl)benzamid;
N-(5-(2-Amino-6-chinazolinyl)-2-methylphenyl)-4-(1,1-dimethylethyl)benzamid;
N-(5-(2-Amino-6-chinazolinyl)-2-methylphenyl)-5-methyl-3-isoxazolcarboxamid;
N-(5-(2-Amino-6-chinazolinyl)-2-methylphenyl)-3,4-difluorbenzamid;
N-(5-(2-Amino-6-chinazolinyl)-2-methylphenyl)cyclopropancarboxamid;
N-(3-(2-Amino-6-chinazolinyl)-5-chlorphenyl)-3-(trifluormethyl)benzamid;
N'-(3-(2-Amino-6-chinazolinyl)-5-chlorphenyl)-N,N-dimethylharnstoff;
N-(3-(2-Amino-6-chinazolinyl)-5-chlorphenyl)-4-(1,1-dimethylethyl)benzamid;
N-(3-(2-Amino-6-chinazolinyl)-5-chlorphenyl)-4-(dimethylamino)benzamid;
N-(3-(2-Amino-6-chinazolinyl)-5-chlorphenyl)-3-((trifluormethyl)oxy)benzamid;
N-(3-(2-Amino-6-chinazolinyl)-5-chlor-4-methylphenyl)-4-(trifluormethyl)benzamid;
N-(3-(2-Amino-6-chinazolinyl)-5-chlor-4-methylphenyl)-3-(trifluormethyl)benzamid;
N-(3-(2-Amino-6-chinazolinyl)-5-chlor-4-methylphenyl)-3-chlorbenzamid;
N-(3-(2-Amino-6-chinazolinyl)-5-chlor-4-methylphenyl)-4-chlorbenzamid;
N-(3-(2-Amino-6-chinazolinyl)-5-chlor-4-methylphenyl)-4-(methyloxy)-3-(trifluormethyl)benzamid;
3-(2-Amino-6-chinazolinyl)-N-cyclopropyl-4-(methyloxy)benzamid;
3-(2-Amino-6-chinazolinyl)-2-methyl-N-(3-(trifluormethyl)phenyl)benzamid;
3-(2-Amino-6-chinazolinyl)-4-methyl-N-(1-methylethyl)benzamid;
Ethyl-7-(2-methyl-5-(((3-(trifluormethyl)phenyl)amino)carbonyl)phenyl)-3-isochinolincarboxylat;
3-(2-Amino-6-chinazolinyl)-N-(2-(dimethylamino)ethyl)-4-methylbenzamid;
7-(2-Methyl-5-(((3-(trifluormethyl)phenyl)amino)carbonyl)phenyl)-3-isochinolincarbonsäure;
N-(2-(Dimethylamino)ethyl)-7-(2-methyl-5-(((3-(trifluormethyl)phenyl)amino)carbonyl)phenyl)-3-isochinolincarboxamid;
3-(2-Amino-6-chinazolinyl)-4-methyl-N-(1-methylpropyl)benzamid;
Ethyl-7-(5-((cyclopropylamino)carbonyl)-2-methylphenyl)-3-isochinolincarboxylat;
7-(5-((Cyclopropylamino)carbonyl)-2-methylphenyl)-3-isochinolincarbonsäure;
N-Cyclobutyl-4-methyl-3-(2-((2-(4-morpholinyl)ethyl)amino)-6-chinazolinyl)benzamid;
7-(5-((Cyclopropylamino)carbonyl)-2-methylphenyl)-N-(2-(dimethylamino)ethyl)-3-isochinolincarboxamid;
3-(2-Aminopyrido[2,3-d]pyrimidin-6-yl)-N-(3-(trifluormethyl)phenyl)benzamid;
4-Methyl-3-(2-(methylamino)pyrido[2,3-d]pyrimidin-6-yl)-N-phenylbenzamid;
3-(2-Amino-6-chinazolinyl)-N-(2-((3-(dimethylamino)propyl)(methyl)amino)-5-ethinylphenyl)-4-methylbenzamid;
N-(2-((3-(Dimethylamino)propyl)(methyl)amino)-5-(trifluormethyl)phenyl)-4-methyl-3-(2-(methylamino)pyrido[2,3-d]pyrimidin-6-yl)benzamid;
3-(2-Amino-6-chinazolinyl)-4-methyl-N-(3-((((2S)-1-methyl-2-pyrrolidinyl)methyl)oxy)-5-(trifluormethyl)phenyl)benzamid;
N-(2-((3-(Dimethylamino)propyl)(methyl)amino)-5-(trifluormethyl)phenyl)-3-(2-(((ethylamino)carbonyl)amino)-6-chinazolinyl)-4-methylbenzamid;
3-(2-(((Ethylamino)carbonyl)amino)-6-chinazolinyl)-4-methyl-N-(3-(trifluormethyl)phenyl)benzamid;
3-(2-Amino-6-chinazolinyl)-4-methyl-N-(3-((1-methylethyl)oxy)phenyl)benzamid;
3-(2-Amino-6-chinazolinyl)-N-(3-(ethyloxy)phenyl)-4-methylbenzamid;
3-(2-(Dimethylamino)-6-chinazolinyl)-4-methyl-N-(3-(trifluormethyl)phenyl)benzamid;
3-(2-(Dimethylamino)-6-chinazolinyl)-4-methyl-N-(4-(trifluormethyl)phenyl)benzamid;
2-Fluor-5-(2-(methylamino)pyrido[2,3-d]pyrimidin-6-yl)-N-(3-(trifluormethyl)phenyl)benzamid;
N-(2-((3-(Dimethylamino)propyl)(methyl)amino)-5-(trifluormethyl)phenyl)-2-fluor-5-(2-(methylamino)pyrido[2,3-d]pyrimidin-6-yl)benzamid;
3-(2-Amino-6-chinazolinyl)-N-(5-(1,1-dimethylethyl)-2-(methyloxy)phenyl)-4-methylbenzamid;
N-(3-(2-Amino-6-chinazolinyl)-4-methylphenyl)-2,5-bis(trifluormethyl)benzamid;
N-(3-(2-Amino-6-chinazolinyl)-4-methylphenyl)-2-(trifluormethyl)benzamid;
N-(3-(2-Amino-6-chinazolinyl)-4-methylphenyl)-2-fluor-4-(trifluormethyl)benzamid;
N-(3-(2-Amino-6-chinazolinyl)-4-methylphenyl)-1-methyl-1H-indol-2-carboxamid;
N-(3-(2-Amino-6-chinazolinyl)-4-methylphenyl)-2,4-bis(trifluormethyl)benzamid;
N-(3-(2-Amino-6-chinazolinyl)-4-methylphenyl)-2,3-difluorbenzamid;
N-(3-(2-Amino-6-chinazolinyl)-4-methylphenyl)-3,5-bis(1,1-dimethylethyl)benzamid;
N-(3-(2-Amino-6-chinazolinyl)-4-methylphenyl)-3,5-bis(methyloxy)benzamid;
N-(3-(2-Amino-6-chinazolinyl)-4-methylphenyl)-3,4-dimethylbenzamid;
N-(3-(2-Amino-6-chinazolinyl)-4-methylphenyl)-2-chlor-3-(trifluormethyl)benzamid;
N-(3-(2-Amino-6-chinazolinyl)-4-methylphenyl)-2,3-dimethylbenzamid;
N-(3-(2-Amino-6-chinazolinyl)-4-methylphenyl)-4-chlor-2-(trifluormethyl)benzamid;
N-(3-(2-Amino-6-chinazolinyl)-4-(methyloxy)phenyl)-1-methyl-1H-indol-2-carboxamid;
N-(3-(2-Amino-6-chinazolinyl)-4-methylphenyl)-2-chlor-3-methylbenzamid;
N-(3-(2-Amino-6-chinazolinyl)-4-methylphenyl)-2-chlor-3-fluorbenzamid;
N-(3-(2-Amino-6-chinazolinyl)-4-(methyloxy)phenyl)-2-fluor-3-(trifluormethyl)benzamid;
N-(3-(2-Amino-6-chinazolinyl)-4-methylphenyl)-3-chlor-2,4-difluorbenzamid;
N-(3-(2-Amino-6-chinazolinyl)-4-methylphenyl)-2,3-difluor-4-(trifluormethyl)benzamid;
N-(3-(2-Amino-6-chinazolinyl)-4-methylphenyl)-2,3-difluor-4-methylbenzamid;
N-(3-(2-Amino-6-chinazolinyl)phenyl)-2-fluor-3-(trifluormethyl)benzamid;
N-(3-(2-Amino-6-chinazolinyl)phenyl)-2,3-dichlorbenzamid;
N-(3-(2-Amino-6-chinazolinyl)-4-fluorphenyl)-2-fluor-3-(trifluormethyl)benzamid;
N-(3-(2-Amino-6-chinazolinyl)-4-fluorphenyl)-2,3-dichlorbenzamid;
N-(3-(2-Amino-6-chinazolinyl)-4-chlorphenyl)-2-fluor-3-(trifluormethyl)benzamid;
N-(3-(2-Amino-6-chinazolinyl)-4-chlorphenyl)-2,3-dichlorbenzamid;
3-(2-Amino-6-chinazolinyl)-4-fluor-N-(2-fluor-3-(trifluormethyl)phenyl)benzamid;
3-(2-Amino-6-chinazolinyl)-4-fluor-N-(3-(trifluormethyl)phenyl)benzamid;
3-(2-Amino-6-chinazolinyl)-N-(5-(1,1-dimethylethyl)-2-(methyloxy)phenyl)-4-fluorbenzamid;
3-(2-Amino-6-chinazolinyl)-4-chlor-N-(5-(1,1-dimethylethyl)-2-(methyloxy)phenyl)benzamid;
3-(2-Amino-6-chinazolinyl)-4-chlor-N-(2-fluor-3-(trifluormethyl)phenyl)benzamid;
3-(2-Amino-6-chinazolinyl)-4-chlor-N-(2-methyl-3-(trifluormethyl)phenyl)benzamid;
3-(2-Amino-6-chinazolinyl)-4-chlor-N-(3-((trifluormethyl)oxy)phenyl)benzamid;
3-(4-Amino-6-chinazolinyl)-4-methyl-N-(3-(trifluormethyl)phenyl)benzamid;
3-(4-Amino-6-chinazolinyl)-4-methyl-N-(4-(phenyloxy)phenyl)benzamid;
3-(4-(((1E)-(Dimethylamino)methyliden)amino)-6-chinazolinyl)-4-methyl-N-(4-(phenyloxy)phenyl)benzamid;
3-(4-Amino-6-chinazolinyl)-4-methyl-N-phenylbenzamid;
3-(4-Amino-6-chinazolinyl)-N-(3-bromphenyl)-4-methylbenzamid;
3-(4-Amino-6-chinazolinyl)-N-(4-(1,1-dimethylethyl)phenyl)-4-methylbenzamid;
3-(4-Amino-6-chinazolinyl)-4-methyl-N-(4-(4-morpholinyl)phenyl)benzamid;
3-(4-Amino-6-chinazolinyl)-4-methyl-N-(2-((4-methyl-1-piperazinyl)methyl)-5-(trifluormethyl)phenyl)benzamid;
3-(4-Amino-6-chinazolinyl)-4-methyl-N-(2-(4-morpholinyl)-5-(trifluormethyl)phenyl)benzamid;
3-(4-Amino-6-chinazolinyl)-N-(2-(dimethylamino)-5-(trifluormethyl)phenyl)-4-methylbenzamid;
3-(4-Amino-6-chinazolinyl)-4-methyl-N-(2-(1-pyrrolidinyl)-5-(trifluormethyl)phenyl)benzamid;
3-(4-Amino-6-chinazolinyl)-N-(2-fluor-5-(trifluormethyl)phenyl)-4-methylbenzamid;
3-(4-Amino-6-chinazolinyl)-4-methyl-N-(2-(methyloxy)-5-(trifluormethyl)phenyl)benzamid;
3-(4-Amino-6-chinazolinyl)-N-cyclopropyl-4-methylbenzamid;
3-(4-Amino-6-chinazolinyl)-N-cyclopentyl-4-methylbenzamid;
3-(4-Amino-6-chinazolinyl)-N-(2,3-dimethylphenyl)-4-methylbenzamid;
3-(4-Amino-6-chinazolinyl)-N-(2,5-bis(methyloxy)phenyl)-4-methylbenzamid;
3-(4-Amino-6-chinazolinyl)-N-(cyclopropylmethyl)-4-methylbenzamid;
3-(4-Amino-6-chinazolinyl)-4-methyl-N-(3-((1-methylethyl)oxy)phenyl)benzamid;
3-(2-Amino-6-chinazolinyl)-N-(3,4-dimethyl-5-isoxazolyl)-4-methylbenzamid;
3-(4-Amino-6-chinazolinyl)-N-(3-(1,1-dimethylethyl)phenyl)-4-methylbenzamid;
3-(2-Amino-6-chinazolinyl)-N-(5-(1,1-dimethylethyl)-3-isoxazolyl)-4-methylbenzamid;
3-(4-Amino-6-chinazolinyl)-N-(3,4-bis(methyloxy)-5-((3-(4-methyl-1-piperazinyl)propyl)oxy)phenyl)-4-methylbenzamid;
3-(4-Amino-6-chinazolinyl)-4-methyl-N-(3-((trifluormethyl)oxy)phenyl)benzamid;
3-(4-Amino-6-chinazolinyl)-N-(4,5-dimethyl-3-isoxazolyl)-4-methylbenzamid;
3-(4-Amino-6-chinazolinyl)-4-methyl-N-(phenylmethyl)benzamid;
3-(4-Amino-6-chinazolinyl)-4-methyl-N-(2-methyl-5-(1-methylethyl)phenyl)benzamid;
3-(4-Amino-6-chinazolinyl)-4-methyl-N-(3-((phenylmethyl)oxy)phenyl)benzamid;
3-(2-Amino-6-chinazolinyl)-4-methyl-N-(4-pyridinylmethyl)benzamid;
3-(2-Amino-6-chinazolinyl)-4-methyl-N-(2-pyridinylmethyl)benzamid;
3-(4-Amino-6-chinazolinyl)-N-(1,1'-biphenyl-3-yl)-4-methylbenzamid;
3-(4-Amino-6-chinazolinyl)-N-(5-(1,1-dimethylethyl)-2-(methyloxy)phenyl)-4-methylbenzamid;
3-(4-Amino-6-chinazolinyl)-N-(5-(1,1-dimethylethyl)-3-isoxazolyl)-4-methylbenzamid;
3-(4-Amino-6-chinazolinyl)-4-methyl-N-(3-methyl-5-isoxazolyl)benzamid;
3-(4-Amino-6-chinazolinyl)-4-methyl-N-(3-((1,1,2,2-tetrafluorethyl)oxy)phenyl)benzamid;
3-(4-Amino-6-chinazolinyl)-N-(5-cyclohexyl-2-(methyloxy)phenyl)-4-methylbenzamid;
3-(4-Amino-6-chinazolinyl)-N-(3,5-bis(1,1-dimethylethyl)phenyl)-4-methylbenzamid;
3-(2-Amino-6-chinazolinyl)-4-methyl-N-(3-methyl-5-isoxazolyl)benzamid;
3-(4-Amino-6-chinazolinyl)-4-methyl-N-((2S)-tetrahydro-2-furanylmethyl)benzamid;
3-(2-Amino-6-chinazolinyl)-N-(3,5-bis(1,1-dimethylethyl)phenyl)-4-methylbenzamid;
3-(2-Amino-6-chinazolinyl)-4-methyl-N-(4-pyridinyl)benzamid;
3-(2-Amino-6-chinazolinyl)-4-methyl-N-(2-pyridinyl)benzamid;
3-(2-Amino-6-chinazolinyl)-N-(2-chlor-5-(trifluormethyl)-3-pyridinyl)-4-methylbenzamid;
3-(2-Amino-6-chinazolinyl)-4-methyl-N-(1,3-thiazol-2-yl)benzamid;
3-(2-Amino-6-chinazolinyl)-4-methyl-N-(3-pyridinyl)benzamid;
3-(2-Amino-6-chinazolinyl)-N-(5-brom-2-pyridinyl)-4-methylbenzamid;
3-(2-Amino-6-chinazolinyl)-4-methyl-N-(6-(4-morpholinyl)-3-pyridinyl)benzamid;
3-(2-Amino-6-chinazolinyl)-4-methyl-N-(3-chinolinyl)benzamid;
3-(2-Amino-6-chinazolinyl)-4-methyl-N-(6-chinolinyl)benzamid;
3-(2-Amino-6-chinazolinyl)-N-(2-chlor-4-pyridinyl)-4-methylbenzamid;
3-(2-Amino-6-chinazolinyl)-4-methyl-N-(2-naphthalinyl)benzamid;
3-(2-Amino-6-chinazolinyl)-N-(4,5-dihydro-1,3-thiazol-2-yl)-4-methylbenzamid;
S-(4,5-Dihydro-1,3-thiazol-2-yl) 3-(2-amino-6-chinazolinyl)-4-methylbenzolcarbothioat;
3-(2-Amino-6-chinazolinyl)-N-(5-ethyl-1,3,4-thiadiazol-2-yl)-4-methylbenzamid;
3-(2-Amino-6-chinazolinyl)-4-methyl-N-(4-methyl-2-pyridinyl)benzamid;
3-(2-Amino-6-chinazolinyl)-N-(4-ethyl-2-pyridinyl)-4-methylbenzamid;
3-(2-Amino-6-chinazolinyl)-4-methyl-N-(4-methyl-1,3-thiazol-2-yl)benzamid;
3-(2-Amino-6-chinazolinyl)-N-(5-chlor-1,3-benzoxazol-2-yl)-4-methylbenzamid;
3-(2-Amino-6-chinazolinyl)-4-methyl-N-(5-(trifluormethyl)-1,3,4-thiadiazol-2-yl)benzamid;
3-(2-Amino-6-chinazolinyl)-4-methyl-N-(2-(3-pyridinyl)ethyl)benzamid;
3-(2-Amino-6-chinazolinyl)-4-methyl-N-(5-methyl-3-isothiazolyl)benzamid;
3-(2-Amino-6-chinazolinyl)-4-methyl-N-(4-(trifluormethyl)-2-pyridinyl)benzamid;
3-(2-Amino-6-chinazolinyl)-N-(3,5-dichlor-2-pyridinyl)-4-methylbenzamid;
3-(2-Amino-6-chinazolinyl)-4-methyl-N-(2-(methyloxy)-3-pyridinyl)benzamid;
3-(2-Amino-6-chinazolinyl)-N-(4,5-dimethyl-1,3-thiazol-2-yl)-4-methylbenzamid;
3-(2-Amino-6-chinazolinyl)-4-methyl-N-(4-methyl-1,3-benzothiazol-2-yl)benzamid;
3-(2-Amino-6-chinazolinyl)-N-(6-(ethyloxy)-1,3-benzothiazol-2-yl)-4-methylbenzamid;
3-(2-Amino-6-chinazolinyl)-4-methyl-N-(5-nitro-1,3-thiazol-2-yl)benzamid;
3-(2-Amino-6-chinazolinyl)-N-(6-fluor-1,3-benzothiazol-2-yl)-4-methylbenzamid;
3-(2-Amino-6-chinazolinyl)-N-(4-(1,1-dimethylethyl)-1,3-thiazol-2-yl)-4-methylbenzamid;
3-(2-Amino-6-chinazolinyl)-N-(5-iod-2-pyridinyl)-4-methylbenzamid;
3-(2-Amino-6-chinazolinyl)-4-methyl-N-(1-methyl-1H-benzimidazol-2-yl)benzamid;
N-(2-Acetyl-3-thienyl)-3-(2-amino-6-chinazolinyl)-4-methylbenzamid;
3-(2-Amino-6-chinazolinyl)-N-(5-brom-1,3-thiazol-2-yl)-4-methylbenzamid;
3-(2-Amino-6-chinazolinyl)-4-methyl-N-((6-(trifluormethyl)-3-pyridinyl)methyl)benzamid;
3-(2-Amino-6-chinazolinyl)-4-methyl-N-(3-((phenylmethyl)oxy)-2-pyridinyl)benzamid;
3-(2-Amino-6-chinazolinyl)-N-(2-fluor-4-(1-pyrrolidinyl)-3-(1-pyrrolidinylcarbonyl)phenyl)-4-methylbenzamid;
3-(2-Amino-6-chinazolinyl)-4-methyl-N-(6-(trifluormethyl)-2-pyridinyl)benzamid;
3-(2-Amino-6-chinazolinyl)-4-methyl-N-(5-(trifluormethyl)-2-pyridinyl)benzamid;
3-(2-Amino-6-chinazolinyl)-N-(3-hydroxy-2-pyridinyl)-4-methylbenzamid;
3-(2-Amino-6-chinazolinyl)-N-(2,4-difluorphenyl)-4-methylbenzamid;
3-(2-Amino-6-chinazolinyl)-4-methyl-N-(3-((3-(1-piperidinyl)propyl)oxy)-2-pyridinyl)benzamid;
3-(2-Amino-6-chinazolinyl)-N-(3-cyanophenyl)-4-methylbenzamid;
3-(2-Amino-6-chinazolinyl)-N-(3-((2-(diethylamino)ethyl)oxy)-2-pyridinyl)-4-methylbenzamid;
3-(2-Amino-6-chinazolinyl)-4-methyl-N-(4-(methyloxy)-1,1'-biphenyl-3-yl)benzamid;
3-(2-Amino-6-chinazolinyl)-4-methyl-N-(2-methyl-5-(trifluormethyl)phenyl)benzamid;
3-(2-Amino-6-chinazolinyl)-N-(5-chlor-2-(methyloxy)phenyl)-4-methylbenzamid;
3-(2-Amino-6-chinazolinyl)-N-(2-((3-(dimethylamino)propyl)oxy)-5-(trifluormethyl)phenyl)-4-methylbenzamid;
3-(2-Amino-6-chinazolinyl)-4-methyl-N-(2-(methyloxy)-5-((phenylamino)carbonyl)phenyl)benzamid;
3-(2-Amino-6-chinazolinyl)-4-methyl-N-(2-(methylsulfanyl)-5-(trifluormethyl)phenyl)benzamid;
3-(2-Amino-6-chinazolinyl)-N-(2-fluor-5-methylphenyl)-4-methylbenzamid;
3-(2-Amino-6-chinazolinyl)-N-(2,5-bis(methyloxy)phenyl)-4-methylbenzamid;
5-(2-Amino-6-chinazolinyl)-2-chlor-N-(3-(trifluormethyl)phenyl)benzamid;
3-(2-Amino-6-chinazolinyl)-5-fluor-N-(3-(trifluormethyl)phenyl)benzamid;
3-(2-Amino-6-chinazolinyl)-5-brom-N-(4-(1,1-dimethylethyl)phenyl)benzamid;
3-(2-Amino-6-chinazolinyl)-N-(3,5-bis(methyloxy)phenyl)-5-brombenzamid;
3-(2-Amino-6-chinazolinyl)-5-brom-N-(3-(trifluormethyl)phenyl)benzamid;
3-(2-Amino-6-chinazolinyl)-5-chlor-N-(3-(trifluormethyl)phenyl)benzamid;
3-(2-Amino-6-chinazolinyl)-4-chlor-N-(2-(1-pyrrolidinyl)-5-(trifluormethyl)phenyl)benzamid;
3-(2-Amino-6-chinazolinyl)-4-chlor-N-(2-(1-piperidinyl)-5-(trifluormethyl)phenyl)benzamid;
3-(2-Amino-6-chinazolinyl)-4-chlor-N-(2-(4-morpholinyl)-5-(trifluormethyl)phenyl)benzamid;
3-(4-Amino-6-chinazolinyl)-4-methyl-N-(3-(1-methylethyl)phenyl)benzamid;
3-(4-Amino-6-chinazolinyl)-N-(3-(1-methylethyl)phenyl)benzamid;
3-(4-Amino-6-chinazolinyl)-4-methyl-N-(2-(methyl((3S)-1-methyl-3-pyrrolidinyl)amino)-5-(trifluormethyl)phenyl)benzamid;
3-(4-Amino-6-chinazolinyl)-N-(2-((3-(dimethylamino)propyl)(methyl)amino)-5-(trifluormethyl)phenyl)-4-methylbenzamid;
3-(4-Amino-6-chinazolinyl)-4-methyl-N-(2-(1-piperidinyl)-5-(trifluormethyl)phenyl)benzamid;
N-(3-(4-Amino-6-chinazolinyl)phenyl)-3-(trifluormethyl)benzamid;
N-(3-(4-Amino-6-chinazolinyl)phenyl)benzamid;
N-(3-(4-Amino-6-chinazolinyl)phenyl)-3-(methyloxy)benzamid;
N-(3-(4-Amino-6-chinazolinyl)phenyl)-4-(trifluormethyl)benzamid;
3-(2-Amino-6-chinazolinyl)-N-cyclopropyl-4-methylbenzamid;
3-(2-Amino-6-chinazolinyl)-4-methyl-N-(2-(methyloxy)-5-(trifluormethyl)phenyl)benzamid;
N-Cyclopropyl-4-methyl-3-(2-((2-(4-morpholinyl)ethyl)amino)-6-chinazolinyl)benzamid;
4-Methyl-N-(2-(methyloxy)-5-(trifluormethyl)phenyl)-3-(2-((2-(4-morpholinyl)ethyl)amino)-6-chinazolinyl)benzamid;
N-Cyclopropyl-3-(2-((2-(dimethylamino)ethyl)amino)-6-chinazolinyl)-4-methylbenzamid;
N-Cyclopropyl-4-methyl-3-(2-((1-methyl-4-piperidinyl)amino)-6-chinazolinyl)benzamid;
1,1-Dimethylethyl-2-(((6-(5-((cyclopropylamino)carbonyl)-2-methylphenyl)-2-chinazolinyl)amino)methyl)-1-piperidincarboxylat;
N-Cyclopropyl-4-methyl-3-(2-((2-(1-piperidinyl)ethyl)amino)-6-chinazolinyl)benzamid;
N-Cyclopropyl-4-methyl-3-(2-(4-methyl-1-piperazinyl)-6-chinazolinyl)benzamid;
1,1-Dimethylethyl-4-(2-((6-(5-((cyclopropylamino)carbonyl)-2-methylphenyl)-2-chinazolinyl)amino)ethyl)-1-piperazincarboxylat;
4-Methyl-3-(2-(methylamino)pyrido[2,3-d]pyrimidin-6-yl)-N-(3-(trifluormethyl)phenyl)benzamid;
N-(2,3-Dihydro-1H-inden-4-yl)-4-methyl-3-(2-(methylamino)pyrido[2,3-d]pyrimidin-6-yl)benzamid;
N-(4-Methyl-3-(2-(methylamino)pyrido[2,3-d]pyrimidin-6-yl)phenyl)-3-(trifluormethyl)benzamid;
N-(4-Chlor-3-(trifluormethyl)phenyl)-4-methyl-3-(2-(methylamino)pyrido[2,3-d]pyrimidin-6-yl)benzamid;
4-Methyl-3-(2-(methylamino)pyrido[2,3-d]pyrimidin-6-yl)-N-(3-methyl-4-(1-methylethyl)phenyl)benzamid;
N-(4-Methyl-3-(2-(methylamino)pyrido[2,3-d]pyrimidin-6-yl)phenyl)-3,5-bis(trifluormethyl)benzamid;
2,3-Dichlor-N-(4-methyl-3-(2-(methylamino)pyrido[2,3-d]pyrimidin-6-yl)phenyl)benzamid;
2-Fluor-N-(4-methyl-3-(2-(methylamino)pyrido[2,3-d]pyrimidin-6-yl)phenyl)-3-(trifluormethyl)benzamid;
4-Methyl-3-(2-(methylamino)pyrido[2,3-d]pyrimidin-6-yl)-N-(2-methyl-3-(trifluormethyl)phenyl)benzamid;
2-Fluor-N-(4-methyl-3-(2-(methylamino)pyrido[2,3-d]pyrimidin-6-yl)phenyl)-5-(trifluormethyl)benzamid;
4-(1,1-Dimethylethyl)-N-(4-methyl-3-(2-(methylamino)pyrido[2,3-d]pyrimidin-6-yl)phenyl)benzamid;
4-Methyl-3-(2-(methylamino)pyrido[2,3-d]pyrimidin-6-yl)-N-(4-(trifluormethyl)phenyl)benzamid;
N-(4-(1,1-Dimethylethyl)phenyl)-4-methyl-3-(2-(methylamino)pyrido[2,3-d]pyrimidin-6-yl)benzamid;
N-(3-(1,1-Dimethylethyl)phenyl)-4-methyl-3-(2-(methylamino)pyrido[2,3-d]pyrimidin-6-yl)benzamid;
N-(4-Methyl-3-(2-(methylamino)pyrido[2,3-d]pyrimidin-6-yl)phenyl)-2,4-bis(trifluormethyl)benzamid;
N-(4-Methyl-3-(2-(methylamino)pyrido[2,3-d]pyrimidin-6-yl)phenyl)-2,3-dihydro-1-benzofuran-7-carboxamid;
3-Chlor-2-fluor-N-(4-methyl-3-(2-(methylamino)pyrido[2,3-d]pyrimidin-6-yl)phenyl)benzamid;
3-Fluor-N-(4-methyl-3-(2-(methylamino)pyrido[2,3-d]pyrimidin-6-yl)phenyl)-2-(trifluormethyl)benzamid;
2-Chlor-3-fluor-N-(4-methyl-3-(2-(methylamino)pyrido[2,3-d]pyrimidin-6-yl)phenyl)benzamid;
2-Chlor-3-methyl-N-(4-methyl-3-(2-(methylamino)pyrido[2,3-d]pyrimidin-6-yl)phenyl)benzamid;
2,3-Difluor-N-(4-methyl-3-(2-(methylamino)pyrido[2,3-d]pyrimidin-6-yl)phenyl)-4-(trifluormethyl)benzamid;
2,3-Difluor-4-methyl-N-(4-methyl-3-(2-(methylamino)pyrido[2,3-d]pyrimidin-6-yl)phenyl)benzamid;
4-Methyl-3-(2-(methylamino)pyrido[2,3-d]pyrimidin-6-yl)-N-(3-((trifluormethyl)oxy)phenyl)benzamid;
2-Methyl-3-(2-(methylamino)pyrido[2,3-d]pyrimidin-6-yl)-N-(3-(trifluormethyl)phenyl)benzamid;
2-Methyl-3-(2-(methylamino)pyrido[2,3-d]pyrimidin-6-yl)-N-(2-methyl-3-(trifluormethyl)phenyl)benzamid;
N-(2,3-Dichlorphenyl)-2-methyl-3-(2-(methylamino)pyrido[2,3-d]pyrimidin-6-yl)benzamid;
2-Methyl-3-(2-(methylamino)pyrido[2,3-d]pyrimidin-6-yl)-N-(4-(methyloxy)-3-(trifluormethyl)phenyl)benzamid;
2-Methyl-3-(2-(methylamino)pyrido[2,3-d]pyrimidin-6-yl)-N-(3-((trifluormethyl)oxy)phenyl)benzamid;
N-(4-Methyl-3-(2-(methylamino)pyrido[2,3-d]pyrimidin-6-yl)phenyl)-4-(trifluormethyl)benzamid;
4-Methyl-3-(2-(methylamino)pyrido[2,3-d]pyrimidin-6-yl)-N-(4-(methyloxy)-3-(trifluormethyl)phenyl)benzamid;
4-Methyl-3-(2-(methylamino)pyrido[2,3-d]pyrimidin-6-yl)-N-(2-pyridinylmethyl)benzamid;
N-(2-Methyl-3-(2-(methylamino)pyrido[2,3-d]pyrimidin-6-yl)phenyl)-3-(trifluormethyl)benzamid;
2,2-Difluor-N-(4-methyl-3-(2-(methylamino)pyrido[2,3-d]pyrimidin-6-yl)phenyl)-1,3-benzodioxol-4-carboxamid;
N-(5-(1,1-Dimethylethyl)-2-(methyloxy)phenyl)-4-methyl-3-(2-(methylamino)pyrido[2,3-d]pyrimidin-6-yl)benzamid;
3-(2-Amino-6-chinazolinyl)-4-methyl-N-(4-((((2-(4-morpholinyl)ethyl)amino)carbonyl)amino)-3-(trifluormethyl)phenyl)benzamid;
3-(2-Amino-6-chinazolinyl)-N-(4-((((2-(diethylamino)ethyl)amino)carbonyl)amino)-3-(trifluormethyl)phenyl)-4-methylbenzamid;
4-Methyl-3-(2-(methylamino)pyrido[2,3-d]pyrimidin-6-yl)-N-(4-((((2-(4-morpholinyl)ethyl)amino)carbonyl)amino)-3-(trifluormethyl)phenyl)benzamid;
N-(4-((((2-(Diethylamino)ethyl)amino)carbonyl)amino)-3-(trifluormethyl)phenyl)-4-methyl-3-(2-(methylamino)pyrido[2,3-d]pyrimidin-6-yl)benzamid;
3-(2-Amino-6-chinazolinyl)-4-methyl-N-(3-(((2-(4-morpholinyl)ethyl)amino)carbonyl)-5-(trifluormethyl)phenyl)benzamid;
4-Methyl-3-(2-(methylamino)pyrido[2,3-d]pyrimidin-6-yl)-N-(3-(((2-(4-morpholinyl)ethyl)amino)carbonyl)-5-(trifluormethyl)phenyl)benzamid;
3-(2-Amino-6-chinazolinyl)-N-(3-(((2-(diethylamino)ethyl)amino)carbonyl)-5-(trifluormethyl)phenyl)-4-methylbenzamid;
N-(3-(((2-(Diethylamino)ethyl)amino)carbonyl)-5-(trifluormethyl)phenyl)-4-methyl-3-(2-(methylamino)pyrido[2,3-d]pyrimidin-6-yl)benzamid;
N-(5-((Diethylamino)sulfonyl)-2-(methyloxy)phenyl)-4-methyl-3-(2-(methylamino)pyrido[2,3-d]pyrimidin-6-yl)benzamid;
N-(3-(2-Amino-6-chinazolinyl)-4-methylphenyl)-3,4,5-tris(methyloxy)benzamid;
N-(4-Methyl-3-(2-(methylamino)pyrido[2,3-d]pyrimidin-6-yl)phenyl)-3,4,5-tris(methyloxy)benzamid;
3-Fluor-N-(4-methyl-3-(2-(methylamino)pyrido[2,3-d]pyrimidin-6-yl)phenyl)-5-(trifluormethyl)benzamid;
3-(2-Amino-6-chinazolinyl)-4-methyl-N-(3-((4-methyl-1-piperazinyl)carbonyl)-5-(trifluormethyl)phenyl)benzamid;
3-(2-Amino-6-chinazolinyl)-4-methyl-N-(3-((4-methyl-1-piperazinyl)methyl)-5-(trifluormethyl)phenyl)benzamid;
N-(3-(2-Amino-6-chinazolinyl)-2-methylphenyl)-3-(trifluormethyl)benzamid;
N-(5-(1,1-Dimethylethyl)-2-((4-morpholinylacetyl)amino)phenyl)-4-methyl-3-(2-(methylamino)pyrido[2,3-d]pyrimidin-6-yl)benzamid;
3-(2-Amino-6-chinazolinyl)-N-(5-(1,1-dimethylethyl)-2-((4-morpholinylacetyl)amino)phenyl)-4-methylbenzamid;
N-(3-(2-Amino-6-chinazolinyl)-2-methylphenyl)-3-fluor-5-(trifluormethyl)benzamid;
3-Fluor-N-(2-methyl-3-(2-(methylamino)pyrido[2,3-d]pyrimidin-6-yl)phenyl)-5-(trifluormethyl)benzamid;
N-(3-(2-Amino-6-chinazolinyl)-2-methylphenyl)-3,5-bis(trifluormethyl)benzamid;
N-(2-Methyl-3-(2-(methylamino)pyrido[2,3-d]pyrimidin-6-yl)phenyl)-3,5-bis(trifluormethyl)benzamid;
N-(3-(2-Amino-6-chinazolinyl)-2-methylphenyl)-2-fluor-5-(trifluormethyl)benzamid;
2-Fluor-N-(2-methyl-3-(2-(methylamino)pyrido[2,3-d]pyrimidin-6-yl)phenyl)-5-(trifluormethyl)benzamid;
N-(3-(2-Amino-6-chinazolinyl)-2,4,6-trimethylphenyl)-3-(trifluormethyl)benzamid;
4-((3-(2-Amino-6-chinazolinyl)-4-methylphenyl)carbonyl)-6-(1,1-dimethylethyl)-3,4-dihydro-2(1H)-chinoxalinon;
N-(4-(2-Amino-6-chinazolinyl)phenyl)-N'-phenylharnstoff;
N-(4-(4-Amino-6-chinazolinyl)phenyl)-3-(trifluormethyl)benzamid;
N-(4-(2-((4-(4-Methyl-1-piperazinyl)phenyl)amino)-6-chinazolinyl)phenyl)-3-(trifluormethyl)benzamid;
4-(2-((4-(4-Methyl-1-piperazinyl)phenyl)amino)-6-chinazolinyl)-N-(3-(trifluormethyl)phenyl)benzamid;
N-(4-(2-((3-(4-Morpholinyl)propyl)amino)-6-chinazolinyl)phenyl)-3-(trifluormethyl)benzamid;
4-(2-((3-(4-Morpholinyl)propyl)amino)-6-chinazolinyl)-N-(3-(trifluormethyl)phenyl)benzamid;
N-(4-(2-((3-(Diethylamino)propyl)amino)-6-chinazolinyl)phenyl)-3-(trifluormethyl)benzamid;
4-(2-((3-(Diethylamino)propyl)amino)-6-chinazolinyl)-N-(3-(trifluormethyl)phenyl)benzamid;
N-Cyclopropyl-4-(2-((4-(4-methyl-1-piperazinyl)phenyl)amino)-6-chinazolinyl)benzamid;
4-(2-((4-(4-Methyl-1-piperazinyl)phenyl)amino)-6-chinazolinyl)-N-phenylbenzamid;
N-(4-Chlorphenyl)-4-(2-((4-(4-methyl-1-piperazinyl)phenyl)amino)-6-chinazolinyl)benzamid;
N-(4-(2-(Methylamino)-6-chinazolinyl)phenyl)-2-(phenylamino)benzamid;
N-(4-(4-Amino-6-chinazolinyl)phenyl)-2-(phenylamino)benzamid;
6-Methyl-5-(2-(methylamino)-6-chinazolinyl)-1(2H)-isochinolinon;
2',7'-Dimethyl-2-(methylamino)-6,8'-bichinazolin-4'(3'H)-on;
2-Fluor-4-methyl-3-(2-(methylamino)-6-chinazolinyl)-N-(3-((1-methylethyl)oxy)phenyl)benzamid;
3-Fluor-4-methyl-2-(2-(methylamino)-6-chinazolinyl)-N-(3-((1-methylethyl)oxy)phenyl)benzamid;
2-Fluor-4-methyl-3-(2-(methylamino)-6-chinazolinyl)-N-(3-((1-methylethyl)oxy)phenyl)benzolcarbothioamid;
2-Fluor-4-methyl-3-(2-(methylamino)-6-chinazolinyl)-N-(4-(methyloxy)-3-(trifluormethyl)phenyl)benzamid;
2-Amino-4-methyl-3-(2-(methylamino)-6-chinazolinyl)benzoesäure;
2-Amino-4-methyl-3-(2-(methylamino)-6-chinazolinyl)-N-(3-((1-methylethyl)oxy)phenyl)benzamid;
2-Amino-4-methyl-3-(2-(methylamino)-6-chinazolinyl)-N-(3-(1-methylethyl)phenyl)benzamid;
2-Amino-N-(4-(1,1-dimethylethyl)phenyl)-4-methyl-3-(2-(methylamino)-6-chinazolinyl)benzamid;
4-Methyl-3-(chinazolin-6-yl)-N-(3-(trifluormethyl)phenyl)benzamid-hydrochlorid;
3-(2-Amino-6-chinazolinyl)-N-(3-((3-(dimethylamino)propyl)oxy)-5-(trifluormethyl)phenyl)-4-methylbenzamid;
6-(5-Isothiocyanato-2-methylphenyl)-N-methyl-2-chinazolinamin;
6-(2-Phenylimidazo[1,2-a]pyridin-6-yl)-2-chinazolinamin;
6-(2-(4-(Methyloxy)phenyl)imidazo[1,2-a]pyridin-6-yl)-2-chinazolinamin;
6-(2-(3-(Methyloxy)phenyl)imidazo[1,2-a]pyridin-6-yl)-2-chinazolinamin;
6-(2-(2-(Methyloxy)phenyl)imidazo[1,2-a]pyridin-6-yl)-2-chinazolinamin;
6-(2-(1,3-Benzodioxol-5-yl)imidazo[1,2-a]pyridin-6-yl)-2-chinazolinamin;
6-(2-(3-Thienyl)imidazo[1,2-a]pyridin-6-yl)-2-chinazolinamin;
6-(2-Ethyl-1-benzofuran-5-yl)-2-chinazolinamin;
6-(2-(1,1-Dimethylethyl)imidazo[1,2-a]pyridin-6-yl)-2-chinazolinamin;
3-(2-Amino-6-chinazolinyl)-N-(3-(3-(dimethylamino)propyl)-5-(trifluormethyl)phenyl)-4-methylbenzamid;
N-(4-(Diethylnitroryl)-1-methylbutyl)-6-(4-methyl-3-pyridinyl)-2-chinazolinamin;
Methyl-4-methyl-3-(6-chinazolinyl)benzoat;
4-Methyl-3-(6-chinazolinyl)benzoesäure;
N-Cyclopropyl-4-methyl-3-(2-((((2R)-1-(2,2,2-trifluorethyl)-2-pyrrolidinyl)methyl)amino)-6-chinazolinyl)benzamid;
3-(2-Amino-6-chinazolinyl)-N-(3-((N,N-diethylglycyl)amino)-4-(1,1-dimethylethyl)phenyl)-4-methylbenzamid;
3-(2-Amino-6-chinazolinyl)-N-(4-(1,1-dimethylethyl)-3-((1-pyrrolidinylacetyl)amino)phenyl)-4-methylbenzamid;
N-(1-(N,N-Diethylglycyl)-3,3-dimethyl-2,3-dihydro-1H-indol-6-yl)-4-methyl-3-(2-(methylamino)-6-chinazolinyl)benzamid;
3-(2-(Cyclopropylamino)-6-chinazolinyl)-N-(1-(N,N-diethylglycyl)-3,3-dimethyl-2,3-dihydro-1H-indol-6-yl)-4-methylbenzamid;
N-(3-((N,N-Diethylglycyl)amino)-4-(1-piperidinyl)phenyl)-4-methyl-3-(2-(methylamino)-6-chinazolinyl)benzamid;
N-(4-Chlor-3-(((1-methyl-4-piperidinyl)methyl)oxy)phenyl)-4-methyl-3-(2-(methylamino)-6-chinazolinyl)benzamid;
N-(3-Fluor-4-((3-(1-piperidinyl)propyl)oxy)phenyl)-4-methyl-3-(2-(methylamino)-6-chinazolinyl)benzamid;
N-(3-((N,N-Diethylglycyl)amino)-4-methylphenyl)-4-methyl-3-(2-(methylamino)-6-chinazolinyl)benzamid;
N-(3-((N,N-Diethylglycyl)amino)-4-fluorphenyl)-4-methyl-3-(2-(methylamino)-6-chinazolinyl)benzamid;
N-(2-((Diethylamino)methyl)-4,5-bis(methyloxy)phenyl)-4-methyl-3-(2-(methylamino)-6-chinazolinyl)benzamid;
N-(2-((Diethylamino)methyl)-4,5-bis(methyloxy)phenyl)-4-methyl-3-(2-((3-(4-morpholinyl)propyl)amino)-6-chinazolinyl)benzamid;
N-(3-Fluor-4-(((1-methyl-3-piperidinyl)methyl)oxy)phenyl)-4-methyl-3-(2-(methylamino)-6-chinazolinyl)benzamid;
4-Methyl-3-(2-(methylamino)-6-chinazolinyl)-N-(3-(((methylsulfonyl)amino)methyl)phenyl)benzamid;
4-Methyl-N-(3-(((methylsulfonyl)amino)methyl)phenyl)-3-(2-((3-(4-morpholinyl)propyl)amino)-6-chinazolinyl)benzamid;
N-(4-Fluor-3-(((methyloxy)acetyl)amino)phenyl)-4-methyl-3-(2-(methylamino)-6-chinazolinyl)benzamid;
3-(2-((3-(Diethylamino)propyl)amino)-6-chinazolinyl)-N-(4-fluor-3-(((methyloxy)acetyl)amino)phenyl)-4-methylbenzamid;
4-Methyl-3-(2-(methylamino)-6-chinazolinyl)-N-(2-((4-methyl-1-piperazinyl)methyl)-5-(trifluormethyl)phenyl)benzamid;
4-Methyl-3-(2-(methylamino)-6-chinazolinyl)-N-(3-((((2S)-1-methyl-2-pyrrolidinyl)methyl)oxy)-4-(pentafluorethyl)phenyl)benzamid;
N-(4-Chlor-3-((((2S)-1-methyl-2-pyrrolidinyl)methyl)oxy)phenyl)-4-methyl-3-(2-(methylamino)-6-chinazolinyl)benzamid;
Ethyl-3,3-dimethyl-6-(((4-methyl-3-(2-(methylamino)-6-chinazolinyl)phenyl)carbonyl)amino)-2,3-dihydro-1H-indol-1-carboxylat;
N-(3-((Diethylamino)methyl)-4-(methyloxy)phenyl)-4-methyl-3-(2-(methylamino)-6-chinazolinyl)benzamid;
Ethyl-6-(((3-(2-((3-(diethylamino)propyl)amino)-6-chinazolinyl)-4-methylphenyl)carbonyl)amino)-3,3-dimethyl-2,3-dihydro-1H-indol-1-carboxylat;
4-Methyl-3-(2-(methylamino)-6-chinazolinyl)-N-(3-(2-oxo-1-pyrrolidinyl)-5-(trifluormethyl)phenyl)benzamid;
4-Methyl-3-(2-(methylamino)-6-chinazolinyl)-N-(2-(1-piperidinyl)-5-(trifluormethyl)phenyl)benzamid;
4-Methyl-3-(2-(methylamino)-6-chinazolinyl)-N-(1-methyl-4-piperidinyl)benzamid;
N-(2-((2,2-Dimethylpropanoyl)amino)-5-(trifluormethyl)phenyl)-4-methyl-3-(2-(methylamino)-6-chinazolinyl)benzamid;
4-Methyl-3-(2-(methylamino)-6-chinazolinyl)-N-(2-(methyl(1-methyl-4-piperidinyl)amino)-5-(trifluormethyl)phenyl)benzamid;
4-Methyl-3-(2-(methylamino)-6-chinazolinyl)-N-(3-(2-methyl-1-pyrrolidinyl)-2-pyridinyl)benzamid;
N-(2,2-Difluor-1,3-benzodioxol-4-yl)-4-methyl-3-(2-((3-(4-morpholinyl)propyl)amino)-6-chinazolinyl)benzamid;
N-(2,2-Difluor-1,3-benzodioxol-4-yl)-4-methyl-3-(2-((1-methyl-4-piperidinyl)amino)-6-chinazolinyl)benzamid;
N-(2,2-Difluor-1,3-benzodioxol-4-yl)-4-methyl-3-(2-((2-(1-piperidinyl)ethyl)amino)-6-chinazolinyl)benzamid;
N-(2-((3-(Dimethylamino)propyl)oxy)-5-(trifluormethyl)phenyl)-4-methyl-3-(2-(methylamino)-6-chinazolinyl)benzamid;
4-Methyl-3-(2-((3-(methyloxy)propyl)amino)-6-chinazolinyl)-N-(2-methyl-3-(trifluormethyl)phenyl)benzamid;
4-Methyl-N-(2-methyl-3-(trifluormethyl)phenyl)-3-(2-(phenylamino)-6-chinazolinyl)benzamid;
N-(1-Acetyl-3,3-dimethyl-2,3-dihydro-1H-indol-6-yl)-4-methyl-3-(2-((2-(4-morpholinyl)ethyl)amino)-6-chinazolinyl)benzamid;
N-(1-Acetyl-3,3-dimethyl-2,3-dihydro-1H-indol-6-yl)-4-methyl-3-(2-((2-(1-pyrrolidinyl)ethyl)amino)-6-chinazolinyl)benzamid;
N-(1-Acetyl-3,3-dimethyl-2,3-dihydro-1H-indol-6-yl)-3-(2-(((1-ethyl-4-piperidinyl)methyl)amino)-6-chinazolinyl)-4-methylbenzamid;
N-(1-Acetyl-3,3-dimethyl-2,3-dihydro-1H-indol-6-yl)-4-methyl-3-(2-((1-methyl-4-piperidinyl)amino)-6-chinazolinyl)benzamid;
N-(1-Acetyl-3,3-dimethyl-2,3-dihydro-1H-indol-6-yl)-3-(2-(cyclopropylamino)-6-chinazolinyl)-4-methylbenzamid;
N-(1-Acetyl-3,3-dimethyl-2,3-dihydro-1H-indol-6-yl)-4-methyl-3-(2-((3-(2-oxo-1-pyrrolidinyl)propyl)amino)-6-chinazolinyl)benzamid;
N-(1-Acetyl-3,3-dimethyl-2,3-dihydro-1H-indol-6-yl)-4-methyl-3-(2-(methylamino)-6-chinazolinyl)benzamid;
N-(1-Acetyl-3,3-dimethyl-2,3-dihydro-1H-indol-6-yl)-4-methyl-3-(2-((3-(4-morpholinyl)propyl)amino)-6-chinazolinyl)benzamid;
4-Methyl-3-(2-(methyl(3-(4-morpholinyl)propyl)amino)-6-chinazolinyl)-N-(2-methyl-3-(trifluormethyl)phenyl)benzamid;
2-Fluor-N-(2-methyl-3-(2-(methylamino)-6-chinazolinyl)phenyl)-5-(trifluormethyl)benzamid;
4-Methyl-3-(2-((1E)-3-(methyloxy)-1-propenyl)-6-chinazolinyl)-N-(3-(trifluormethyl)phenyl)benzamid;
4-Methyl-3-(2-((1E)-3-(methyloxy)-1-propenyl)-6-chinazolinyl)-N-(2-methyl-3-(trifluormethyl)phenyl)benzamid;
6-(2,6-Dichlorphenyl)-N-(3-(4-morpholinyl)propyl)-2-chinazolinamin;
4-Methyl-3-(2-(methylamino)-6-chinazolinyl)benzamid;
3-(2-(Cyclopropylamino)-6-chinazolinyl)-4-methylbenzamid;
4-Methyl-3-(2-((3-(4-morpholinyl)propyl)amino)-6-chinazolinyl)benzamid;
3-(2-((3-(Diethylamino)propyl)amino)-6-chinazolinyl)-4-methylbenzamid;
3-(2-((3-Fluor-4-((4-(1H-imidazol-1-yl)butyl)oxy)phenyl)amino)-6-chinazolinyl)-4-methylbenzamid;
4-Methyl-3-(2-((4-((3-(1-piperidinyl)propyl)oxy)phenyl)amino)-6-chinazolinyl)benzamid;
6-(2,6-Dimethylphenyl)-N-(3-morpholinopropyl)chinazolin-2-amin;
N-(2-Fluor-5-(trifluormethyl)phenyl)-4-methyl-3-(2-((2-(4-morpholinyl)ethyl)amino)-6-chinazolinyl)benzamid;
N-(2-Fluor-5-(trifluormethyl)phenyl)-4-methyl-3-(2-((3-(4-morpholinyl)propyl)amino)-6-chinazolinyl)benzamid;
N-(2-Fluor-5-(trifluormethyl)phenyl)-4-methyl-3-(2-((2-(1-piperidinyl)ethyl)amino)-6-chinazolinyl)benzamid;
N-(2-Fluor-5-(trifluormethyl)phenyl)-4-methyl-3-(2-((2-(1-pyrrolidinyl)ethyl)amino)-6-chinazolinyl)benzamid;
3-(2-((3-(Diethylamino)propyl)amino)-6-chinazolinyl)-4-methyl-N-(2-methyl-3-(trifluormethyl)phenyl)benzamid;
4-Methyl-N-(2-methyl-3-(trifluormethyl)phenyl)-3-(2-((2-(1-(phenylmethyl)-4-piperidinyl)ethyl)amino)-6-chinazolinyl)benzamid;
1,1-Dimethylethyl-4-(2-((6-(2-methyl-5-(((2-methyl-3-(trifluormethyl)phenyl)amino)carbonyl)phenyl)-2-chinazolinyl)amino)ethyl)-1-piperazincarboxylat;
3-(2-((((2R)-1-Ethyl-2-pyrrolidinyl)methyl)amino)-6-chinazolinyl)-4-methyl-N-(2-methyl-3-(trifluormethyl)phenyl)benzamid;
4-Methyl-N-(2-methyl-3-(trifluormethyl)phenyl)-3-(2-((2-(1-piperidinyl)ethyl)amino)-6-chinazolinyl)benzamid;
3-(2-(1,3-Benzoxazol-2-ylamino)-6-chinazolinyl)-4-methyl-N-(2-methyl-3-(trifluormethyl)phenyl)benzamid;
4-Methyl-N-(2-methyl-3-(trifluormethyl)phenyl)-3-(2-((2-(1-piperazinyl)ethyl)amino)-6-chinazolinyl)benzamid;
4-Methyl-3-(2-((2-(1-pyrrolidinyl)ethyl)amino)-6-chinazolinyl)-N-(3-(trifluormethyl)phenyl)benzamid;
N-(4-Chlor-3-(trifluormethyl)phenyl)-4-methyl-3-(2-((2-(1-pyrrolidinyl)ethyl)amino)-6-chinazolinyl)benzamid;
4-Methyl-N-(3-methyl-4-(1-methylethyl)phenyl)-3-(2-((2-(1-pyrrolidinyl)ethyl)amino)-6-chinazolinyl)benzamid;
N-(4-Fluor-3-(trifluormethyl)phenyl)-4-methyl-3-(2-((2-(1-pyrrolidinyl)ethyl)amino)-6-chinazolinyl)benzamid;
N-(4-Chlor-3-(trifluormethyl)phenyl)-4-methyl-3-(2-((1-methyl-4-piperidinyl)amino)-6-chinazolinyl)benzamid;
N-(4-Fluor-3-(trifluormethyl)phenyl)-4-methyl-3-(2-((1-methyl-4-piperidinyl)amino)-6-chinazolinyl)benzamid;
4-Methyl-N-(3-methyl-4-(1-methylethyl)phenyl)-3-(2-((1-methyl-4-piperidinyl)amino)-6-chinazolinyl)benzamid;
N-(4-Chlor-3-(trifluormethyl)phenyl)-4-methyl-3-(2-((2-(1-piperidinyl)ethyl)amino)-6-chinazolinyl)benzamid;
N-(4-Fluor-3-(trifluormethyl)phenyl)-4-methyl-3-(2-((2-(1-piperidinyl)ethyl)amino)-6-chinazolinyl)benzamid;
4-Methyl-N-(3-methyl-4-(1-methylethyl)phenyl)-3-(2-((2-(1-piperidinyl)ethyl)amino)-6-chinazolinyl)benzamid;
N-(1-Acetyl-3,3-dimethyl-2,3-dihydro-1H-indol-6-yl)-4-methyl-3-(2-((2-(1-piperidinyl)ethyl)amino)-6-chinazolinyl)benzamid;
4-Methyl-N-(2-methyl-3-(trifluormethyl)phenyl)-3-(2-((4-((3-(1-piperidinyl)propyl)oxy)phenyl)amino)-6-chinazolinyl)benzamid;
4-Methyl-3-(2-((3-(4-methyl-1-piperazinyl)phenyl)amino)-6-chinazolinyl)-N-(2-methyl-3-(trifluormethyl)phenyl)benzamid;
N-(2-Fluor-3-(trifluormethyl)phenyl)-4-methyl-3-(2-((2-(1-piperidinyl)ethyl)amino)-6-chinazolinyl)benzamid;
N-(2,3-Dihydro-1H-inden-4-yl)-4-methyl-3-(2-((3-(4-morpholinyl)propyl)amino)-6-chinazolinyl)benzamid;
3-(2-((3-(Diethylamino)propyl)amino)-6-chinazolinyl)-N-(2,3-dihydro-1H-inden-4-yl)-4-methylbenzamid;
N-(2,3-Dihydro-1H-inden-4-yl)-4-methyl-3-(2-(methylamino)-6-chinazolinyl)benzamid;
N-(2,3-Dihydro-1H-inden-4-yl)-4-methyl-3-(2-((4-((3-(1-piperidinyl)propyl)oxy)phenyl)amino)-6-chinazolinyl)benzamid;
3-(2-(((1S)-4-(Diethylamino)-1-methylbutyl)amino)-6-chinazolinyl)-4-methyl-N-(2-methyl-3-(trifluormethyl)phenyl)benzamid;
6-(5-Amino-2-methylphenyl)-N-(4-(4-methyl-1-piperazinyl)phenyl)-2-chinazolinamin;
2-Methyl-N-(4-methyl-3-(2-((4-(4-methyl-1-piperazinyl)phenyl)amino)-6-chinazolinyl)phenyl)-3-(trifluormethyl)benzamid;
4-(1,1-Dimethylethyl)-N-(4-methyl-3-(2-((4-(4-methyl-1-piperazinyl)phenyl)amino)-6-chinazolinyl)phenyl)benzamid;
N-(4-Methyl-3-(2-((4-(4-methyl-1-piperazinyl)phenyl)amino)-6-chinazolinyl)phenyl)acetamid;
N-(4-Methyl-3-(2-((4-(4-methyl-1-piperazinyl)phenyl)amino)-6-chinazolinyl)phenyl)-3,5-bis(methyloxy)benzamid;
2-Fluor-N-(4-methyl-3-(2-((4-(4-methyl-1-piperazinyl)phenyl)amino)-6-chinazolinyl)phenyl)-3-(trifluormethyl)benzamid;
2,2-Difluor-N-(4-methyl-3-(2-((4-(4-methyl-1-piperazinyl)phenyl)amino)-6-chinazolinyl)phenyl)-1,3-benzodioxol-5-carboxamid;
N-(4-Methyl-3-(2-((4-(4-methyl-1-piperazinyl)phenyl)amino)-6-chinazolinyl)phenyl)-1-naphthalincarboxamid;
3-(1,1-Dimethylethyl)-1-methyl-N-(4-methyl-3-(2-((4-(4-methyl-1-piperazinyl)phenyl)amino)-6-chinazolinyl)phenyl)-1H-pyrazol-4-carboxamid;
6-(5-(1H-Benzimidazol-2-ylamino)-2-methylphenyl)-N-(4-(4-methyl-1-piperazinyl)phenyl)-2-chinazolinamin;
N-(4-(4-Methyl-1-piperazinyl)phenyl)-6-(2-methyl-5-((5-(trifluormethyl)-1H-benzimidazol-2-yl)amino)phenyl)-2-chinazolinamin;
N-(2-(2-(Dimethylamino)ethyl)-5-(trifluormethyl)phenyl)-4-methyl-3-(2-(methylamino)-6-chinazolinyl)benzamid;
N-Ethyl-N'-(4-methyl-3-(2-((4-(4-methyl-1-piperazinyl)phenyl)amino)-6-chinazolinyl)phenyl)harnstoff;
3-(2-((3-Hydroxypropyl)amino)-6-chinazolinyl)-4-methyl-N-(2-methyl-3-(trifluormethyl)phenyl)benzamid;
4-Methyl-N-(2-methyl-3-(trifluormethyl)phenyl)-3-(2-(3-pyridinylamino)-6-chinazolinyl)benzamid;
N-Methyl-6-(2-methyl-5-((5-(trifluormethyl)-1H-benzimidazol-2-yl)amino)phenyl)-2-chinazolinamin;
6-(5-(1H-Benzimidazol-2-ylamino)-2-methylphenyl)-N-methyl-2-chinazolinamin;
6-(5-((1-(2-(Dimethylamino)ethyl)-1H-benzimidazol-2-yl)amino)-2-methylphenyl)-N-methyl-2-chinazolinamin;
6-(5-((1-(3-(Dimethylamino)propyl)-1H-benzimidazol-2-yl)amino)-2-methylphenyl)-N-methyl-2-chinazolinamin;
6-(5-((7-Chlor-5-(trifluormethyl)-1H-benzimidazol-2-yl)amino)-2-methylphenyl)-N-methyl-2-chinazolinamin;
6-(5-(1H-Imidazo[4,5-b]pyridin-2-ylamino)-2-methylphenyl)-N-methyl-2-chinazolinamin;
N-Methyl-6-(2-methyl-5-((1-methyl-5-(trifluormethyl)-1H-benzimidazol-2-yl)amino)phenyl)-2-chinazolinamin;
6-(5-((5,6-Difluor-1H-benzimidazol-2-yl)amino)-2-methylphenyl)-N-methyl-2-chinazolinamin;
N-Methyl-6-(2-methyl-5-((6-(4-methyl-1-piperazinyl)-1H-benzimidazol-2-yl)amino)phenyl)-2-chinazolinamin;
N-Methyl-6-(6-methyl-5-phthalazinyl)-2-chinazolinamin;
6-Methyl-5-(2-(methylamino)-6-chinazolinyl)-1-phthalazinol;
N-(4-Chlor-3-(2-(methylamino)-6-chinazolinyl)phenyl)-3-(trifluormethyl)benzamid;
N-(4-Methyl-3-(2-(methylamino)-6-chinazolinyl)phenyl)-3-(trifluormethyl)benzamid;
N-(4-Chlor-3-(2-(methylamino)-6-chinazolinyl)phenyl)-2-fluor-5-(trifluormethyl)benzamid;
N-(4-Chlor-3-(2-(methylamino)-6-chinazolinyl)phenyl)-2-methyl-3-(trifluormethyl)benzamid;
3-(2-Amino-6-chinazolinyl)-N-(2,6-dimethylphenyl)-4-methylbenzamid;
3-(2-Amino-6-chinazolinyl)-N-(2-(ethyloxy)-5-(trifluormethyl)phenyl)-4-methylbenzamid;
3-(2-Amino-6-chinazolinyl)-4-methyl-N-(2-(methyloxy)-5-(trifluormethyl)phenyl)benzamid d_3_;
3-(2-Amino-6-chinazolinyl)-4-methyl-N-(2-methyl-5-nitrophenyl)benzamid;
3-(2-Amino-6-chinazolinyl)-N-(2,5-dimethylphenyl)-4-methylbenzamid;
N-(5-(Aminocarbonyl)-2-methylphenyl)-3-(2-amino-6-chinazolinyl)-4-methylbenzamid;
3-(2-Amino-6-chinazolinyl)-4-methyl-N-(2-(methyloxy)-5-nitrophenyl)benzamid;
3-(2-Amino-6-chinazolinyl)-N-(5-cyclohexyl-2-(methyloxy)phenyl)-4-methylbenzamid;
3-(2-Amino-6-chinazolinyl)-4-methyl-N-(2-methyl-5-(methyloxy)phenyl)benzamid;
4-(2-Amino-6-chinazolinyl)-N-(2-fluor-5-(trifluormethyl)phenyl)-3-methylbenzamid;
3-(2-Amino-6-chinazolinyl)-4-methyl-N-(2-(methyloxy)-5-((2-pyridinylamino)carbonyl)phenyl)benzamid;
3-(2-Amino-6-chinazolinyl)-4-methyl-N-(2-(methyloxy)-5-((3-pyridinylamino)carbonyl)phenyl)benzamid;
3-(2-Amino-6-chinazolinyl)-4-methyl-N-(2-(methyloxy)-5-((4-pyridinylamino)carbonyl)phenyl)benzamid;
4-(2-Amino-6-chinazolinyl)-3-methyl-N-(3-((4-methyl-1-piperazinyl)methyl)-5-(trifluormethyl)phenyl)benzamid;
4-Methyl-3-(2-(methylamino)-6-chinazolinyl)-N-(2-((2-(1-pyrrolidinyl)ethyl)oxy)-5-(trifluormethyl)phenyl)benzamid;
N-(4-Methyl-3-(2-(methylamino)-6-chinazolinyl)phenyl)-2-((2-(1-pyrrolidinyl)ethyl)amino)-5-(trifluormethyl)benzamid;
3-(2-Amino-6-chinazolinyl)-N-(2-chlor-4-(1,1-dimethylethyl)phenyl)-4-methylbenzamid;
3-(2-Amino-6-chinazolinyl)-N-(2-chlor-5-(trifluormethyl)phenyl)-4-methylbenzamid;
3-(2-Amino-6-chinazolinyl)-N-(2-brom-5-(trifluormethyl)phenyl)-4-methylbenzamid;
3-(2-Amino-6-chinazolinyl)-4-methyl-N-(4-(1-methylethyl)-2-(trifluormethyl)phenyl)benzamid;
3-(2-Amino-6-chinazolinyl)-N-(2-(1,1-dimethylethyl)imidazo[1,2-a]pyridin-6-yl)-4-methylbenzamid;
4-Methyl-3-(2-(methylamino)-6-chinazolinyl)-N-(4-((1-methyl-4-piperidinyl)oxy)phenyl)benzamid;
3-(2-(Cyclopropylamino)-6-chinazolinyl)-4-methyl-N-(4-((1-methyl-4-piperidinyl)oxy)phenyl)benzamid;
3-(2-Amino-6-chinazolinyl)-4-methyl-N-(3-methyl-4-((1-methyl-4-piperidinyl)oxy)phenyl)benzamid;
4-Methyl-3-(2-(methylamino)-6-chinazolinyl)-N-(3-methyl-4-((1-methyl-4-piperidinyl)oxy)phenyl)benzamid;
3-(2-(Cyclopropylamino)-6-chinazolinyl)-4-methyl-N-(3-methyl-4-((1-methyl-4-piperidinyl)oxy)phenyl)benzamid;
3-(2-Amino-6-chinazolinyl)-N-(4-((1-cyclopropylethyl)oxy)phenyl)-4-methylbenzamid;
N-(4-((1-Cyclopropylethyl)oxy)phenyl)-4-methyl-3-(2-(methylamino)-6-chinazolinyl)benzamid;
N-(4-((1-Cyclopropylethyl)oxy)-3-methylphenyl)-4-methyl-3-(2-(methylamino)-6-chinazolinyl)benzamid;
3-(2-Amino-6-chinazolinyl)-N-(4-((1-cyclopropylethyl)oxy)-3-methylphenyl)-4-methylbenzamid;
4-Methyl-3-(2-(methylamino)-6-chinazolinyl)-N-(4-(1-methylethyl)phenyl)benzamid;
3-(2-(Cyclopropylamino)-6-chinazolinyl)-4-methyl-N-(4-(1-methylethyl)phenyl)benzamid;
4-Methyl-N-(4-(1-methylethyl)phenyl)-3-(2-((1-methyl-4-piperidinyl)amino)-6-chinazolinyl)benzamid;
4-Methyl-3-(2-(methylamino)-6-chinazolinyl)-N-(2-((1-methyl-4-piperidinyl)oxy)-5-(trifluormethyl)phenyl)benzamid;
N-(4-((Cyclopropylmethyl)oxy)phenyl)-4-methyl-3-(2-(methylamino)-6-chinazolinyl)benzamid;
N-(4-((Cyclopropylmethyl)oxy)phenyl)-4-methyl-3-(2-((3-(4-morpholinyl)propyl)amino)-6-chinazolinyl)benzamid;
N-(3-(Acetylamino)-4-fluorphenyl)-4-methyl-3-(2-((3-(4-morpholinyl)propyl)amino)-6-chinazolinyl)benzamid;
N-(2-(((1R)-1-Cyclopropylethyl)oxy)-5-(trifluormethyl)phenyl)-4-methyl-3-(2-((3-(4-morpholinyl)propyl)amino)-6-chinazolinyl)benzamid;
N-(3-(Acetylamino)-4-(((1S)-1-cyclopropylethyl)oxy)phenyl)-4-methyl-3-(2-((3-(4-morpholinyl)propyl)amino)-6-chinazolinyl)benzamid;
N-(2-(((1R)-1-Cyclopropylethyl)oxy)-5-(trifluormethyl)phenyl)-4-methyl-3-(2-(methylamino)-6-chinazolinyl)benzamid;
4-Methyl-N-(4-(1-methylethyl)phenyl)-3-(2-((3-(4-morpholinyl)propyl)amino)-6-chinazolinyl)benzamid;
N-(2-(1,1-Dimethylethyl)imidazo[1,2-a]pyridin-6-yl)-4-methyl-3-(2-((3-(4-morpholinyl)propyl)amino)-6-chinazolinyl)benzamid;
N-(2-(1,1-Dimethylethyl)imidazo[1,2-a]pyridin-6-yl)-4-methyl-3-(2-((1-methyl-4-piperidinyl)amino)-6-chinazolinyl)benzamid;
3-(2-((3-(Diethylamino)propyl)amino)-6-chinazolinyl)-N-(2-(1,1-dimethylethyl)imidazo[1,2-a]pyridin-6-yl)-4-methylbenzamid;
N-(2-(1,1-Dimethylethyl)imidazo[1,2-a]pyridin-6-yl)-4-methyl-3-(2-(methylamino)-6-chinazolinyl)benzamid;
4-Methyl-N-(2-(1-methylethyl)-1H-benzimidazol-6-yl)-3-(2-((3-(4-morpholinyl)propyl)amino)-6-chinazolinyl)benzamid;
3-(2-((3-(Diethylamino)propyl)amino)-6-chinazolinyl)-4-methyl-N-(2-(1-methylethyl)-1H-benzimidazol-6-yl)benzamid;
4-Methyl-N-(2-(1-methylethyl)-1H-benzimidazol-6-yl)-3-(2-((1-methyl-4-piperidinyl)amino)-6-chinazolinyl)benzamid;
4-Methyl-3-(2-(methylamino)-6-chinazolinyl)-N-(2-(1-methylethyl)-1H-benzimidazol-6-yl)benzamid;
N-(2-((2-(Diethylamino)ethyl)oxy)-5-(trifluormethyl)phenyl)-4-methyl-3-(2-((3-(4-morpholinyl)propyl)amino)-6-chinazolinyl)benzamid;
N-(1-Acetyl-2,3-dihydro-1H-indol-5-yl)-4-methyl-3-(2-((1-methyl-4-piperidinyl)amino)-6-chinazolinyl)benzamid;
N-(1-Acetyl-2,3-dihydro-1H-indol-5-yl)-4-methyl-3-(2-((3-(4-morpholinyl)propyl)amino)-6-chinazolinyl)benzamid;
4-Methyl-3-(2-(methylamino)-6-chinazolinyl)-N-(2-((2-(4-morpholinyl)ethyl)oxy)-5-(trifluormethyl)phenyl)benzamid;
4-Methyl-3-(2-((1-methyl-4-piperidinyl)amino)-6-chinazolinyl)-N-(2-((2-(4-morpholinyl)ethyl)oxy)-5-(trifluormethyl)phenyl)benzamid;
4-Methyl-N-(2-((2-(4-morpholinyl)ethyl)oxy)-5-(trifluormethyl)phenyl)-3-(2-((3-(4-morpholinyl)propyl)amino)-6-chinazolinyl)benzamid;
N-Methyl-6-(2-methyl-5-(5-(trifluormethyl)-1,3-benzoxazol-2-yl)phenyl)-2-chinazolinamin;
N-(2-(1-Ethylpyrrolidin-3-yloxy)-5-(trifluormethyl)phenyl)-4-methyl-3-(2-(methylamino)chinazolin-6-yl)benzamid;
N-(2-(((3S)-1-Ethyl-3-piperidinyl)oxy)-5-(trifluormethyl)phenyl)-4-methyl-3-(2-(methylamino)-6-chinazolinyl)benzamid;
4-Methyl-N-(2-((1-methyl-4-piperidinyl)oxy)-5-(trifluormethyl)phenyl)-3-(2-((2-(1-piperidinyl)ethyl)amino)-6-chinazolinyl)benzamid;
4-Methyl-N-(2-((1-methyl-4-piperidinyl)oxy)-5-(trifluormethyl)phenyl)-3-(2-((2-(1-pyrrolidinyl)ethyl)amino)-6-chinazolinyl)benzamid;
N-(4-Methyl-3-(2-(methylamino)-6-chinazolinyl)phenyl)-2-((1-methyl-4-piperidinyl)oxy)-5-(trifluormethyl)benzamid;
2-(((3R)-1-Ethyl-3-piperidinyl)oxy)-N-(4-methyl-3-(2-(methylamino)-6-chinazolinyl)phenyl)-5-(trifluormethyl)benzamid;
2-((Cyclopentylmethyl)oxy)-N-(4-methyl-3-(2-(methylamino)-6-chinazolinyl)phenyl)-5-(trifluormethyl)benzamid;
2-((Cyclopropylmethyl)oxy)-N-(4-methyl-3-(2-(methylamino)-6-chinazolinyl)phenyl)-5-(trifluormethyl)benzamid;
2-((Cyclobutylmethyl)oxy)-N-(4-methyl-3-(2-(methylamino)-6-chinazolinyl)phenyl)-5-(trifluormethyl)benzamid;
N-(4-Methyl-3-(2-(methylamino)-6-chinazolinyl)phenyl)-2-((2-(4-morpholinyl)ethyl)oxy)-5-(trifluormethyl)benzamid;
2-((2-(Diethylamino)ethyl)oxy)-N-(4-methyl-3-(2-(methylamino)-6-chinazolinyl)phenyl)-5-(trifluormethyl)benzamid;
2-(((1S)-1-Cyclopentylethyl)oxy)-N-(4-methyl-3-(2-(methylamino)-6-chinazolinyl)phenyl)-5-(trifluormethyl)benzamid;
N-(4-Methyl-3-(2-(methylamino)-6-chinazolinyl)phenyl)-2-(4-methyl-1-piperazinyl)-5-(trifluormethyl)benzamid;
5-Methyl-N-(4-methyl-3-(2-(methylamino)-6-chinazolinyl)phenyl)-2-(methyloxy)benzamid;
N-(4-Methyl-3-(2-(methylamino)-6-chinazolinyl)phenyl)-2-((1-methyl-4-piperidinyl)amino)-5-(trifluormethyl)benzamid;
5-Fluor-N-(4-methyl-3-(2-(methylamino)-6-chinazolinyl)phenyl)-2-(methyloxy)benzamid;
6-(1,6-Dimethyl-1H-indazol-7-yl)-N-methyl-2-chinazolinamin;
4-Methyl-3-(2-(methylamino)-6-chinazolinyl)-N-(6-(trifluormethyl)-1,3-benzothiazol-2-yl)benzamid;
4-Methyl-3-(2-((2-(1-piperidinyl)ethyl)amino)-6-chinazolinyl)-N-(6-(trifluormethyl)-1,3-benzothiazol-2-yl)benzamid;
4-Methyl-3-(2-(methylamino)-6-chinazolinyl)-N-(6-((trifluormethyl)oxy)-1,3-benzothiazol-2-yl)benzamid;
4-Methyl-3-(2-(methylamino)-6-chinazolinyl)-N-(5-(trifluormethyl)-1,3-benzothiazol-2-yl)benzamid;
3-(2-Amino-6-chinazolinyl)-N-(5-(1,1-dimethylethyl)-2-((1-pyrrolidinylacetyl)amino)phenyl)-4-methylbenzamid;
3-(2-Amino-6-chinazolinyl)-N-(5-(1,1-dimethylethyl)-2-((2-(1-piperidinyl)ethyl)oxy)phenyl)-4-methylbenzamid;
3-(2-Amino-6-chinazolinyl)-N-(5-(1,1-dimethylethyl)-2-((1-piperidinylacetyl)amino)phenyl)-4-methylbenzamid;
3-(2-Amino-6-chinazolinyl)-N-(2-((N,N-diethylglycyl)amino)-5-(1,1-dimethylethyl)phenyl)-4-methylbenzamid;
3-(Trifluormethyl)-N-(2,4,6-trimethyl-3-(2-((3-(4-morpholinyl)propyl)amino)-6-chinazolinyl)phenyl)benzamid;
6-(2,6-Dichlorphenyl)-2-chinazolinamin;
N∼1∼-(6-(2,6-Dimethylphenyl)-2-chinazolinyl)-N∼4∼,N∼4∼-dimethyl-1,4-butandiamin;
6-(2-Chlor-6-methylphenyl)-2-chinazolinamin;
N∼1∼-(6-(2,6-Dichlorphenyl)-2-chinazolinyl)-N∼3∼,N∼3∼-diethyl-1,3-propandiamin;
N∼1∼-(6-(2,6-Dimethylphenyl)-2-chinazolinyl)-N∼3∼,N∼3∼-diethyl-1,3-propandiamin;
6-(2,6-Dichlorphenyl)-N-(2-(1-piperidinyl)ethyl)-2-chinazolinamin;
4-Methyl-3-(2-((2-(1-piperidinyl)ethyl)amino)-6-chinazolinyl)-N-(3-(trifluormethyl)phenyl)benzamid;
3-(2-((3-(Diethylamino)propyl)amino)-6-chinazolinyl)-4-methyl-N-(3-(trifluormethyl)phenyl)benzamid;
3-(2-((3-(Dimethylamino)propyl)(methyl)amino)-6-chinazolinyl)-4-methyl-N-(3-(trifluormethyl)phenyl)benzamid;
4-Methyl-3-(2-(methyl(2-(1-piperidinyl)ethyl)amino)-6-chinazolinyl)-N-(3-(trifluormethyl)phenyl)benzamid;
N-(2-Methyl-3-(2-((2-(1-piperidinyl)ethyl)amino)-6-chinazolinyl)phenyl)-3-(trifluormethyl)benzamid;
N∼1∼-(6-(2-Chlor-6-methylphenyl)-2-chinazolinyl)-N∼3∼,N∼3∼-diethyl-1,3-propandiamin;
N∼1∼-(6-(2-Chlor-6-methylphenyl)-2-chinazolinyl)-N∼2∼,N∼2∼-diethyl-1,2-ethandiamin;
6-(2-Chlor-6-methylphenyl)-N-(3-(1-pyrrolidinyl)propyl)-2-chinazolinamin;
N∼1∼,N∼1∼-Diethyl-N∼3∼-(6-(2-ethyl-6-methylphenyl)-2-chinazolinyl)-1,3-propandiamin;
N∼1∼-(6-(5-Amino-2-methylphenyl)-2-chinazolinyl)-N∼3∼,N∼3∼-diethyl-1,3-propandiamin;
N-(3-(2-((3-(Diethylamino)propyl)amino)-6-chinazolinyl)-2-methylphenyl)-3,5-bis(trifluormethyl)benzamid;
3-(2-((3-(Diethylamino)propyl)amino)pyrido[2,3-d]pyrimidin-6-yl)-4-methyl-N-(4-(trifluormethyl)phenyl)benzamid;
3-Fluor-N-(2-methyl-3-(2-(methylamino)-6-chinazolinyl)phenyl)-5-(trifluormethyl)benzamid;
N-(3-(2-((3-(Diethylamino)propyl)amino)-6-chinazolinyl)-4-methylphenyl)-2-fluor-5-(trifluormethyl)benzamid;
N-(3-(2-((3-(Diethylamino)propyl)amino)-6-chinazolinyl)-4-methylphenyl)-3-fluor-5-(trifluormethyl)benzamid;
N-(2-((N,N-Diethylglycyl)amino)-5-(trifluormethyl)phenyl)-4-methyl-3-(2-(methylamino)-6-chinazolinyl)benzamid;
3-(2-Amino-6-chinazolinyl)-N-(2-((N,N-diethylglycyl)amino)-5-(trifluormethyl)phenyl)-4-methylbenzamid;
4-Methyl-3-(2-(methylamino)-6-chinazolinyl)-N-(3-((trifluormethyl)oxy)phenyl)benzamid;
3-(2-((3-(Diethylamino)propyl)amino)-6-chinazolinyl)-4-methyl-N-(3-((trifluormethyl)oxy)phenyl)benzamid;
N-(3-(2-Amino-6-chinazolinyl)-4-methylphenyl)acetamid;
N-(4-Methyl-3-(2-(methylamino)-6-chinazolinyl)phenyl)acetamid;
N-(3-(2-((3-(Diethylamino)propyl)amino)-6-chinazolinyl)-4-methylphenyl)acetamid;
3-(2-Amino-6-chinazolinyl)-4-methyl-N-(5-methyl-2-((4-morpholinylacetyl)amino)phenyl)benzamid;
4-Methyl-3-(2-(methylamino)-6-chinazolinyl)-N-(5-methyl-2-((4-morpholinylacetyl)amino)phenyl)benzamid;
3-(2-Amino-6-chinazolinyl)-4-methyl-N-(5-methyl-2-((1-pyrrolidinylacetyl)amino)phenyl)benzamid;
4-Methyl-3-(2-(methylamino)-6-chinazolinyl)-N-(5-methyl-2-((1-pyrrolidinylacetyl)amino)phenyl)benzamid;
3-(2-Amino-6-chinazolinyl)-4-methyl-N-(5-methyl-2-((1-piperidinylacetyl)amino)phenyl)benzamid;
4-Methyl-3-(2-(methylamino)-6-chinazolinyl)-N-(5-methyl-2-((1-piperidinylacetyl)amino)phenyl)benzamid;
3-(2-Amino-6-chinazolinyl)-N-(2-((N,N-diethylglycyl)amino)-5-methylphenyl)-4-methylbenzamid;
N-(2-((N,N-Diethylglycyl)amino)-5-methylphenyl)-4-methyl-3-(2-(methylamino)-6-chinazolinyl)benzamid;
N-(2-Methyl-3-(2-((3-(1-piperidinyl)propyl)amino)-6-chinazolinyl)phenyl)-3-(trifluormethyl)benzamid;
N-(3-(2-((3-(diethylamino)propyl)amino)-6-chinazolinyl)-2-methylphenyl)-3-(trifluormethyl)benzamid;
4-Methyl-3-(2-(methylamino)-6-chinazolinyl)-N-(2-(1-pyrrolidinylcarbonyl)-5-(trifluormethyl)phenyl)benzamid;
3-(2-Amino-6-chinazolinyl)-4-methyl-N-(2-(1-pyrrolidinylcarbonyl)-5-(trifluormethyl)phenyl)benzamid;
4-Methyl-3-(2-((2-(1-piperidinyl)ethyl)amino)-6-chinazolinyl)-N-(2-(1-pyrrolidinylcarbonyl)-5-(trifluormethyl)phenyl)benzamid;
3-(2-Amino-6-chinazolinyl)-4-methyl-N-(2-(1-pyrrolidinylmethyl)-5-(trifluormethyl)phenyl)benzamid;
4-Methyl-3-(2-(methylamino)-6-chinazolinyl)-N-(2-(1-pyrrolidinylmethyl)-5-(trifluormethyl)phenyl)benzamid;
4-Methyl-3-(2-((2-(1-piperidinyl)ethyl)amino)-6-chinazolinyl)-N-(2-(1-pyrrolidinylmethyl)-5-(trifluormethyl)phenyl)benzamid;
N-(2-Methyl-3-(2-(methylamino)-6-chinazolinyl)phenyl)-3-((1,1,2,2-tetrafluorethyl)oxy)benzamid;
N-(2-Methyl-3-(2-((2-(1-piperidinyl)ethyl)amino)-6-chinazolinyl)phenyl)-3-((1,1,2,2-tetrafluorethyl)oxy)benzamid;
N-(2-(3,3-Dimethyl-2-oxo-1-azetidinyl)-5-(trifluormethyl)phenyl)-4-methyl-3-(2-(methylamino)-6-chinazolinyl)benzamid;
N-(2-(3,3-Dimethyl-2-oxo-1-azetidinyl)-5-(trifluormethyl)phenyl)-4-methyl-3-(2-((2-(1-piperidinyl)ethyl)amino)-6-chinazolinyl)benzamid;
N-Methyl-6-(2-methyl-5-(6-(trifluormethyl)-1H-benzimidazol-2-yl)phenyl)-2-chinazolinamin;
N-(2-Methyl-3-(2-(methylamino)-6-chinazolinyl)phenyl)-3-(trifluormethyl)benzamid;
N-(2-(Acetylamino)-5-(trifluormethyl)phenyl)-4-methyl-3-(2-((2-(1-piperidinyl)ethyl)amino)-6-chinazolinyl)benzamid;
N-(2-(Acetylamino)-5-(trifluormethyl)phenyl)-4-methyl-3-(2-((3-(1-piperidinyl)propyl)amino)-6-chinazolinyl)benzamid;
N-(2-(Acetylamino)-5-(trifluormethyl)phenyl)-3-(2-((3-(diethylamino)propyl)amino)-6-chinazolinyl)-4-methylbenzamid;
N-(4-Methyl-3-(2-((2-(1-piperidinyl)ethyl)amino)-6-chinazolinyl)phenyl)-3-(trifluormethyl)benzamid;
Methyl-4-methyl-3-(2-(methylamino)-6-chinazolinyl)benzoat;
4-Methyl-3-(2-(methylamino)-6-chinazolinyl)-N-(2-(((methyloxy)acetyl)amino)-5-(trifluormethyl)phenyl)benzamid;
Methyl-4-methyl-3-(2-((2-(1-piperidinyl)ethyl)amino)-6-chinazolinyl)benzoat;
4-Methyl-N-(2-(((methyloxy)acetyl)amino)-5-(trifluormethyl)phenyl)-3-(2-((2-(1-piperidinyl)ethyl)amino)-6-chinazolinyl)benzamid;
6-(5-(4-Chlor-6-(trifluormethyl)-1H-benzimidazol-2-yl)-2-methylphenyl)-N-methyl-2-chinazolinamin;
6-(5-(4,6-Bis(trifluormethyl)-1H-benzimidazol-2-yl)-2-methylphenyl)-N-methyl-2-chinazolinamin;
N-(2-(3,3-Dimethyl-2-oxo-1-azetidinyl)-5-(trifluormethyl)phenyl)-4-methyl-3-(2-((3-(4-morpholinyl)propyl)amino)-6-chinazolinyl)benzamid;
3-(2-((3-(Diethylamino)propyl)amino)-6-chinazolinyl)-N-(2-(3,3-dimethyl-2-oxo-1-azetidinyl)-5-(trifluormethyl)phenyl)-4-methylbenzamid;
N-(2-(3,3-Dimethyl-2-oxo-1-azetidinyl)-5-(trifluormethyl)phenyl)-4-methyl-3-(2-((3-(1-piperidinyl)propyl)amino)-6-chinazolinyl)benzamid;
N-(2-(Acetylamino)-5-(trifluormethyl)phenyl)-4-methyl-3-(2-(methylamino)-6-chinazolinyl)benzamid;
4-Methyl-3-(2-(methylamino)-6-chinazolinyl)-N-(2-((((2-(4-morpholinyl)ethyl)amino)carbonyl)amino)-5-(trifluormethyl)phenyl)benzamid;
4-Methyl-3-(2-(methylamino)-6-chinazolinyl)-N-(4-(2-oxo-1-azetidinyl)-3-(trifluormethyl)phenyl)benzamid;
4-Methyl-3-(2-(methylamino)-6-chinazolinyl)-N-(4-(2-oxo-1-pyrrolidinyl)-3-(trifluormethyl)phenyl)benzamid;
3-(2-((3-(Diethylamino)propyl)amino)-6-chinazolinyl)-4-methyl-N-(4-(2-oxo-1-azetidinyl)-3-(trifluormethyl)phenyl)benzamid;
3-(2-((3-(Diethylamino)propyl)amino)-6-chinazolinyl)-4-methyl-N-(4-(2-oxo-1-pyrrolidinyl)-3-(trifluormethyl)phenyl)benzamid;
4-Methyl-3-(2-(methylamino)-6-chinazolinyl)-N-(2-(2-oxo-1-pyrrolidinyl)-5-(trifluormethyl)phenyl)benzamid;
3-(2-((3-(Diethylamino)propyl)amino)-6-chinazolinyl)-4-methyl-N-(2-(2-oxo-1-pyrrolidinyl)-5-(trifluormethyl)phenyl)benzamid;
4-Methyl-3-(2-(methylamino)-6-chinazolinyl)-N-(2-(2-oxo-1-azetidinyl)-5-(trifluormethyl)phenyl)benzamid;
N-(2-(3-(1,1-Dimethylethyl)-2-oxo-1-imidazolidinyl)-5-(trifluormethyl)phenyl)-4-methyl-3-(2-(methylamino)-6-chinazolinyl)benzamid;
4-Methyl-3-(2-(methylamino)-6-chinazolinyl)-N-(5-(trifluormethyl)-2-(3,4,4-trimethyl-2,5-dioxo-1-imidazolidinyl)phenyl)benzamid;
3-(2-((3-(Diethylamino)propyl)amino)-6-chinazolinyl)-4-methyl-N-(5-(trifluormethyl)-2-(3,4,4-trimethyl-2,5-dioxo-1-imidazolidinyl)phenyl)benzamid;
3-(2-((3-(Diethylamino)propyl)amino)-6-chinazolinyl)-N-(2-(3-(1,1-dimethylethyl)-2-oxo-1-imidazolidinyl)-5-(trifluormethyl)phenyl)-4-methylbenzamid;
3-(2-((3-(Diethylamino)propyl)amino)-6-chinazolinyl)-4-methyl-N-(2-(2-oxo-1-azetidinyl)-5-(trifluormethyl)phenyl)benzamid;
4-Methyl-N-(2-(2-oxo-1-azetidinyl)-5-(trifluormethyl)phenyl)-3-(2-((2-(1-piperidinyl)ethyl)amino)-6-chinazolinyl)benzamid;
N-(2-(3-(1,1-Dimethylethyl)-2-oxo-1-imidazolidinyl)-5-(trifluormethyl)phenyl)-4-methyl-3-(2-((2-(1-piperidinyl)ethyl)amino)-6-chinazolinyl)benzamid;
4-Methyl-3-(2-((2-(1-piperidinyl)ethyl)amino)-6-chinazolinyl)-N-(5-(trifluormethyl)-2-(3,4,4-trimethyl-2,5-dioxo-1-imidazolidinyl)phenyl)benzamid;
4-Methyl-3-(2-(methylamino)-6-chinazolinyl)-N-(2-(3-methyl-2-oxo-1-imidazolidinyl)-5-(trifluormethyl)phenyl)benzamid;
4-Methyl-N-(2-(3-methyl-2-oxo-1-imidazolidinyl)-5-(trifluormethyl)phenyl)-3-(2-((1-methyl-4-piperidinyl)amino)-6-chinazolinyl)benzamid;
4-Methyl-N-(2-(3-methyl-2-oxo-1-imidazolidinyl)-5-(trifluormethyl)phenyl)-3-(2-((2-(1-piperidinyl)ethyl)amino)-6-chinazolinyl)benzamid;
3-(2-((3-(Diethylamino)propyl)amino)-6-chinazolinyl)-4-methyl-N-(2-(3-methyl-2-oxo-1-imidazolidinyl)-5-(trifluormethyl)phenyl)benzamid;
N-(2-((4-(2-(Dimethylamino)ethyl)phenyl)oxy)-5-(trifluormethyl)phenyl)-4-methyl-3-(2-(methylamino)-6-chinazolinyl)benzamid;
6-(5-(1H-Benzimidazol-2-yl)-2-methylphenyl)-N-methyl-2-chinazolinamin;
6-(5-(1H-Benzimidazol-2-yl)-2-methylphenyl)-N-(3-(4-morpholinyl)propyl)-2-chinazolinamin;
6-(2-Methyl-5-(6-(trifluormethyl)-1H-benzimidazol-2-yl)phenyl)-N-(3-(4-morpholinyl)propyl)-2-chinazolinamin;
N,N-Diethyl-N'-(6-(2-methyl-5-(6-(trifluormethyl)-1H-benzimidazol-2-yl)phenyl)-2-chinazolinyl)-1,3-propandiamin;
6-(2-Methyl-5-(6-(trifluormethyl)-1H-benzimidazol-2-yl)phenyl)-N-(2-(1-pyrrolidinyl)ethyl)-2-chinazolinamin;
4-Methyl-3-(2-(methylamino)-6-chinazolinyl)-N-(2-(3-oxo-1-piperazinyl)-5-(trifluormethyl)phenyl)benzamid;
N-(2-((3-(Dimethylamino)phenyl)oxy)-5-(trifluormethyl)phenyl)-4-methyl-3-(2-(methylamino)-6-chinazolinyl)benzamid;
4-Methyl-3-(2-((3-(4-morpholinyl)propyl)amino)-6-chinazolinyl)-N-(2-(3-oxo-1-piperazinyl)-5-(trifluormethyl)phenyl)benzamid;
4-Methyl-3-(2-(methylamino)-6-chinazolinyl)benzoesäure;
N-(2-(1-Azetidinyl)-5-(trifluormethyl)phenyl)-4-methyl-3-(2-(methylamino)-6-chinazolinyl)benzamid;
N-(2-(1-Azetidinyl)-5-(trifluormethyl)phenyl)-4-methyl-3-(2-((3-(4-morpholinyl)propyl)amino)-6-chinazolinyl)benzamid;
N-(2-(1-Azetidinyl)-5-(trifluormethyl)phenyl)-4-methyl-3-(2-((2-(1-pyrrolidinyl)ethyl)amino)-6-chinazolinyl)benzamid;
N-(2-(1-Azetidinyl)-5-(trifluormethyl)phenyl)-4-methyl-3-(2-((2-(1-piperidinyl)ethyl)amino)-6-chinazolinyl)benzamid;
4-(2-Amino-6-chinazolinyl)-N-cyclopropyl-1H-indol-6-carboxamid;
5-(2-Amino-6-chinazolinyl)-N-cyclopropyl-2-fluor-4-methylbenzamid;
N-Cyclopropyl-2-fluor-4-methyl-5-(2-((2-(4-morpholinyl)ethyl)amino)-6-chinazolinyl)benzamid;
N-Cyclopropyl-4-methyl-2-((2-(4-morpholinyl)ethyl)amino)-5-(2-((2-(4-morpholinyl)ethyl)amino)-6-chinazolinyl)benzamid;
4-(2-Amino-6-chinazolinyl)-N-cyclopropyl-5-methyl-2-pyridincarboxamid;
N-Cyclopropyl-5-methyl-4-(2-((2-(4-morpholinyl)ethyl)amino)-6-chinazolinyl)-2-pyridincarboxamid;
N-Cyclopropyl-5-methyl-4-(2-((3-((2R)-2-methyl-1-piperidinyl)propyl)amino)-6-chinazolinyl)-2-pyridincarboxamid;
N-Cyclopropyl-2-fluor-4-methyl-3-(2-((2-(4-morpholinyl)ethyl)amino)-6-chinazolinyl)benzamid;
1,1-Dimethylethyl-2-((6-(2-((cyclopropylamino)carbonyl)-5-methyl-4-pyridinyl)-2-chinazolinyl)amino)-2-methylpropylcarbamat;
4-(2-((2-Amino-1,1-dimethylethyl)amino)-6-chinazolinyl)-N-cyclopropyl-5-methyl-2-pyridincarboxamid;
N-Cyclopropyl-4-(2-((2-(dimethylamino)-1,1-dimethylethyl)amino)-6-chinazolinyl)-5-methyl-2-pyridincarboxamid;
N-Cyclopropyl-3-(2-((2-((2,2-dimethylpropanoyl)amino)-1,1-dimethylethyl)amino)-6-chinazolinyl)-4-methylbenzamid;
N-Cyclopropyl-3-(2-((2-((2,2-dimethylpropanoyl)amino)-2-methylpropyl)amino)-6-chinazolinyl)-4-methylbenzamid;
N-Cyclopropyl-4-(2-((1,1-dimethyl-2-((1-methylethyl)amino)ethyl)amino)-6-chinazolinyl)-5-methyl-2-pyridincarboxamid;
N-Cyclopropyl-3-(2-((2-(dimethylamino)-1,1-dimethylethyl)amino)-6-chinazolinyl)-4-methylbenzamid;
1,1-Dimethylethyl-2-((6-(5-((cyclopropylamino)carbonyl)-2-methylphenyl)-2-chinazolinyl)amino)-2-methylpropyl(1-methylethyl)carbamat;
N-Cyclopropyl-3-(2-((1,1-dimethyl-2-((1-methylethyl)amino)ethyl)amino)-6-chinazolinyl)-4-methylbenzamid;
N-Cyclopropyl-3-(2-((3-(dimethylamino)propyl)amino)-6-chinazolinyl)-4-methylbenzamid;
N-Cyclopropyl-4-methyl-3-(2-((3-(2-methyl-1-piperidinyl)propyl)amino)-6-chinazolinyl)benzamid;
N-Cyclopropyl-4-methyl-3-(2-((1-methyl-2-(methyloxy)ethyl)amino)-6-chinazolinyl)benzamid;
N-Cyclopropyl-4-methyl-3-(2-((3-((1-methylethyl)oxy)propyl)amino)-6-chinazolinyl)benzamid;
N-Cyclopropyl-3-(2-((4-(diethylamino)-1-methylbutyl)amino)-6-chinazolinyl)-4-methylbenzamid;
N-Cyclopropyl-4-methyl-3-(2-((3-(1-pyrrolidinyl)propyl)amino)-6-chinazolinyl)benzamid;
3-(2-((2-Amino-2-methylpropyl)amino)-6-chinazolinyl)-N-cyclopropyl-4-methylbenzamid;
N-(6-(2-Methylphenyl)pyrido[2,3-d]pyrimidin-2-yl)-1,4-cyclohexandiamin;
N-Cyclopropyl-4-methyl-3-(2-((tetrahydro-2-furanylmethyl)amino)pyrido[2,3-d]pyrimidin-6-yl)benzamid;
4-((6-(4-Methyl-3-pyridinyl)-2-chinazolinyl)amino)cyclohexanol;
trans-4-((6-(2-Methyl-3-pyridinyl)-2-chinazolinyl)amino)cyclohexanol;
trans-N-(6-(4-Methyl-3-pyridinyl)-2-chinazolinyl)-1,4-cyclohexandiamin;
N-(trans-4-((6-(4-Methyl-3-pyridinyl)-2-chinazolinyl)amino)cyclohexyl)acetamid;
N-(2-(1,1-Dioxido-4-thiomorpholinyl)ethyl)-6-(4-methyl-3-pyridinyl)-2-chinazolinamin;
4-((6-(3-Methyl-2-pyridinyl)-2-chinazolinyl)amino)cyclohexanol;
N∼1∼,N∼1∼-Dimethyl-N∼4∼-(6-(4-methyl-3-pyridinyl)-2-chinazolinyl)-1,4-cyclohexandiamin;
N∼1∼-(1-Methylethyl)-N∼4∼-(6-(4-methyl-3-pyridinyl)-2-chinazolinyl)-1,4-cyclohexandiamin;
6-(2-Fluorphenyl)-N-(2-(2-pyridinyl)ethyl)-2-chinazolinamin;
6-(4-Fluorphenyl)-N-(2-(2-pyridinyl)ethyl)-2-chinazolinamin;
6-(2-Chlorphenyl)-N-(2-(2-pyridinyl)ethyl)-2-chinazolinamin;
6-(2,3-Difluorphenyl)-N-(2-(2-pyridinyl)ethyl)-2-chinazolinamin;
6-(2,4-Difluorphenyl)-N-(2-(2-pyridinyl)ethyl)-2-chinazolinamin;
6-(2,5-Difluorphenyl)-N-(2-(2-pyridinyl)ethyl)-2-chinazolinamin;
6-(2,3-Dimethylphenyl)-N-(2-(2-pyridinyl)ethyl)-2-chinazolinamin;
6-(3,5-Difluorphenyl)-N-(2-(2-pyridinyl)ethyl)-2-chinazolinamin;
N-(2-(2-Pyridinyl)ethyl)-6-(2-(trifluormethyl)phenyl)-2-chinazolinamin;
4-((6-(2,5-Dimethylphenyl)-2-chinazolinyl)amino)cyclohexanol;
N-Cyclopropyl-4-methyl-3-(2-((2-(2-pyridinyl)ethyl)amino)-6-chinazolinyl)benzamid;
4-Methyl-3-(2-((2-(4-morpholinyl)ethyl)amino)-6-chinazolinyl)-N-(1,3-thiazol-2-yl)benzamid;
N-Cyclopropyl-4-methyl-3-(2-((3-(4-morpholinyl)propyl)amino)-6-chinazolinyl)benzamid;
N-Cyclopropyl-3-(2-((1,1-dimethyl-2-(4-morpholinyl)ethyl)amino)-6-chinazolinyl)-4-methylbenzamid;
3-(2-(((1S)-2-(3-(Aminomethyl)phenyl)-1-methylethyl)amino)-6-chinazolinyl)-N-cyclopropyl-4-methylbenzamid;
N-Cyclopropyl-4-methyl-3-(2-methyl-6-chinazolinyl)benzamid;
1,1-Dimethylethyl-(3-((2S)-2-((6-(5-((cyclopropylamino)carbonyl)-2-methylphenyl)-2-chinazolinyl)amino)propyl)phenyl)methylcarbamat;
N-Cyclopropyl-3-(2-(((1S)-2-(3-((dimethylamino)methyl)phenyl)-1-methylethyl)amino)-6-chinazolinyl)-4-methylbenzamid;
N-Cyclopropyl-4-methyl-3-(2-((2-methyl-2-(4-morpholinyl)propyl)amino)-6-chinazolinyl)benzamid;
1,1-Dimethylethyl-((3-((2S)-2-((6-(5-((ethylamino)carbonyl)-2-methylphenyl)-2-chinazolinyl)amino)propyl)phenyl)methyl)carbamat;
3-(2-(((1S)-2-(3-(Aminomethyl)phenyl)-1-methylethyl)amino)-6-chinazolinyl)-N-ethyl-4-methylbenzamid;
3-(2-((2-(Dimethylamino)ethyl)amino)-6-chinazolinyl)-4-methylbenzamid;
N-Cyclopropyl-3-(2-(((1S)-1-((dimethylamino)carbonyl)-2-methylpropyl)amino)-6-chinazolinyl)-4-methylbenzamid;
3-(2-((3-(Dimethylamino)-2,2-dimethylpropyl)amino)-6-chinazolinyl)-4-methylbenzamid;
3-(2-((3-(Dimethylamino)-2,2-dimethylpropyl)amino)-6-chinazolinyl)-N-ethyl-4-methylbenzamid;
4-Methyl-3-(2-((2-methyl-2-(4-morpholinyl)propyl)amino)-6-chinazolinyl)benzamid;
N,4-Dimethyl-3-(2-((2-methyl-2-(4-morpholinyl)propyl)amino)-6-chinazolinyl)benzamid;
N-Ethyl-4-methyl-3-(2-((2-methyl-2-(4-morpholinyl)propyl)amino)-6-chinazolinyl)benzamid;
N-Cyclopropyl-3-(2-((2-(3,5-dimethyl-4-morpholinyl)ethyl)amino)-6-chinazolinyl)-4-methylbenzamid;
4-Methyl-3-(2-((2-methyl-2-(4-morpholinyl)propyl)amino)-6-chinazolinyl)benzoesäure;
N-Cyclopropyl-4-methyl-3-(2-((2-((3S)-3-methyl-4-morpholinyl)ethyl)amino)-6-chinazolinyl)benzamid;
N-Cyclopropyl-4-methyl-3-(2-((2-(phenylamino)ethyl)amino)-6-chinazolinyl)benzamid;
N-(6-(5-((Cyclopropylamino)carbonyl)-2-methylphenyl)-2-chinazolinyl)-O-methyl-L-threonin;
N-(6-(5-((Cyclopropylamino)carbonyl)-2-methylphenyl)-2-chinazolinyl)-D-tryptophan;
N-(6-(5-((Cyclopropylamino)carbonyl)-2-methylphenyl)-2-chinazolinyl)-D-phenylalanin;
N-Cyclopropyl-3-(2-(((1S)-1-(4-fluorphenyl)ethyl)amino)-6-chinazolinyl)-4-methylbenzamid;
((6-(5-((Cyclopropylamino)carbonyl)-2-methylphenyl)-2-chinazolinyl)amino)(tetrahydro-2H-pyran-4-yl)essigsäure;
N-Cyclopropyl-4-methyl-3-(2-((2-(2-pyridinylamino)ethyl)amino)-6-chinazolinyl)benzamid;
3-(4-Brom-7-isochinolinyl)-4-methylbenzamid;
3-(4-(2-Chlor-4-fluorphenyl)-7-isochinolinyl)-4-methylbenzamid;
4-Methyl-3-(4-(4-morpholinyl)-7-isochinolinyl)benzamid;
4-Methyl-3-(4-(2-methyl-3-pyridinyl)-7-isochinolinyl)benzamid;
3-(4-(4-(Aminosulfonyl)-2-methylphenyl)-7-isochinolinyl)-N-cyclopropyl-4-methylbenzamid;
6-(2-Methylphenyl)-N-(3-(4-morpholinyl)propyl)-2-chinazolinamin;
N-(3-(1H-Imidazol-1-yl)propyl)-6-(2-methylphenyl)-2-chinazolinamin;
6-(2-Methylphenyl)-N-(2-(2-pyridinyl)ethyl)-2-chinazolinamin;
N-(6-(2-Methylphenyl)-2-chinazolinyl)-1,4-cyclohexandiamin;
6-(2-Methylphenyl)-N-(3-(2-methyl-1-piperidinyl)propyl)-2-chinazolinamin;
N-((1S)-2-(3-((1S)-1-Aminoethyl)phenyl)-1-methylethyl)-6-(2-methylphenyl)-2-chinazolinamin;
N∼1∼,N∼1∼-Diethyl-N∼3∼-(6-(2-methylphenyl)-2-chinazolinyl)-1,3-propandiamin;
4-((6-(2-Methylphenyl)-2-chinazolinyl)amino)cyclohexanol;
N-((1S)-2-(3-(1-Amino-1-methylethyl)phenyl)-1-methylethyl)-6-(2-methylphenyl)-2-chinazolinamin;
N-((1S)-2-(3-(1-Amino-1-methylethyl)phenyl)-1-methylethyl)-6-(4-methyl-3-thienyl)-2-chinazolinamin;
4-((6-(2-Aminophenyl)-2-chinazolinyl)amino)cyclohexanol;
6-(2-Methylphenyl)-N-(1-(phenylmethyl)-3-pyrrolidinyl)-2-chinazolinamin;
6-(2-Methylphenyl)-N-(1-(phenylmethyl)-4-piperidinyl)-2-chinazolinamin;
1,1-Dimethylethyl-2-(2-((6-(2-methylphenyl)-2-chinazolinyl)amino)ethyl)-1-piperidincarboxylat;
6-(2-Methylphenyl)-N-(2-(2-piperidinyl)ethyl)-2-chinazolinamin;
N-((1S)-2-(3-(1-Amino-1-methylethyl)phenyl)-1-methylethyl)-6-(4-methyl-3-pyridinyl)-2-chinazolinamin;
6-(3-Methyl-2-pyridinyl)-2-chinazolinamin;
N-((1S)-2-(3-(1-Amino-1-methylethyl)phenyl)-1-methylethyl)-6-(3-methyl-2-pyridinyl)-2-chinazolinamin;
N-((1S)-2-(3-((1S)-1-Aminoethyl)phenyl)-1-methylethyl)-6-(2-chlor-3-pyridinyl)-2-chinazolinamin;
N-((1S)-2-(3-((1S)-1-Aminoethyl)phenyl)-1-methylethyl)-6-(4-methyl-3-pyridinyl)-2-chinazolinamin;
N∼1∼,N∼1∼-Diethyl-N∼4∼-(6-(4-methyl-3-pyridinyl)-2-chinazolinyl)-1,4-pentandiamin;
N-(2-(3-Chlorphenyl)ethyl)-6-(4-methyl-3-pyridinyl)-2-chinazolinamin;
(5S)-5-((6-(4-Methyl-3-pyridinyl)-2-chinazolinyl)amino)-2-piperidinon;
((1R)-4-(Diethylamino)-1-methylbutyl)(6-(4-methyl-3-pyridinyl)-2-chinazolinyl)amin;
((1S)-4-(Diethylamino)-1-methylbutyl)(6-(4-methyl-3-pyridinyl)-2-chinazolinyl)amin;
N,N,2,2-Tetramethyl-N'-(6-(4-methyl-3-pyridinyl)-2-chinazolinyl)-1,3-propandiamin;
6-(4-Methyl-3-pyridinyl)-N-(4-(1-pyrrolidinyl)butyl)-2-chinazolinamin;
N-((1S)-1-(4-Fluorphenyl)ethyl)-6-(4-methyl-3-pyridinyl)-2-chinazolinamin;
4-Chlor-N-cyclopropyl-3-(2-((2-(dimethylamino)ethyl)amino)-6-chinazolinyl)benzamid;
N-Cyclopropyl-3-(2-((2-(dimethylamino)ethyl)amino)-6-chinazolinyl)-4-(trifluormethyl)benzamid;
Ethyl-5-(2-((2-(dimethylamino)ethyl)amino)-6-chinazolinyl)-4-methyl-2-thiophencarboxylat;
6-(2-Methylphenyl)-2-chinazolinamin;
6-(2-Methylphenyl)-N-(2-(3-pyridinyl)ethyl)-2-chinazolinamin;
N-((1S)-2-(3-(Aminomethyl)phenyl)-1-methylethyl)-6-(2-methylphenyl)-2-chinazolinamin;
N-(2,4-Dimethylphenyl)-6-(2-methylphenyl)-2-chinazolinamin;
6-(2-Methylphenyl)-N-(2-(4-morpholinyl)ethyl)-2-chinazolinamin;
6-(2-((Dimethylamino)methyl)phenyl)-N-(2-(4-morpholinyl)ethyl)-2-chinazolinamin;
6-(2-((Dimethylamino)methyl)phenyl)-N-(2-(2-pyridinyl)ethyl)-2-chinazolinamin;
6-(4-Fluor-2-methylphenyl)-N-(2-(2-pyridinyl)ethyl)-2-chinazolinamin;
6-(3-Chlor-2-methylphenyl)-N-(2-(2-pyridinyl)ethyl)-2-chinazolinamin;
6-(2,6-Dimethylphenyl)-N-(2-(2-pyridinyl)ethyl)-2-chinazolinamin;
(3-(2-((2-(2-Pyridinyl)ethyl)amino)-6-chinazolinyl)phenyl)methanol;
(2-(2-((2-(2-Pyridinyl)ethyl)amino)-6-chinazolinyl)phenyl)methanol;
6-(3-(Aminomethyl)phenyl)-N-(2-(2-pyridinyl)ethyl)-2-chinazolinamin;
N-Methyl-3-(2-((2-(2-pyridinyl)ethyl)amino)-6-chinazolinyl)benzamid;
N-Cyclopropyl-3-(2-((2-(2-pyridinyl)ethyl)amino)-6-chinazolinyl)benzamid;
6-Phenyl-N-(2-(2-pyridinyl)ethyl)-2-chinazolinamin;
6-(2-(Methyloxy)phenyl)-N-(2-(2-pyridinyl)ethyl)-2-chinazolinamin;
4-((6-(2,6-Dimethylphenyl)-2-chinazolinyl)amino)cyclohexanol;
N-(3-(2-Amino-6-chinazolinyl)-4-methylphenyl)cyclopropancarboxamid;
N-(4-Methyl-3-(2-((2-(4-morpholinyl)ethyl)amino)-6-chinazolinyl)phenyl)cyclopropancarboxamid;
N-(3-Methyl-4-(2-((2-(4-morpholinyl)ethyl)amino)-6-chinazolinyl)phenyl)cyclopropancarboxamid;
N-Cyclopropyl-3-(2-(3-(dimethylamino)-1-propinyl)-6-chinazolinyl)-4-methylbenzamid;
N-Cyclopropyl-3-(2-(3-(dimethylamino)propyl)-6-chinazolinyl)-4-methylbenzamid;
1,1-Dimethylethyl 1-(6-(5-((cyclopropylamino)carbonyl)-2-methylphenyl)-2-chinazolinyl)-3-pyrrolidinylcarbamat;
3-(2-((3S)-3-Amino-1-pyrrolidinyl)-6-chinazolinyl)-N-cyclopropyl-4-methylbenzamid;
N-Cyclopropyl-4-methyl-3-(2-(((3R)-6-oxo-3-piperidinyl)amino)-6-chinazolinyl)benzamid;
N-Cyclopropyl-3-(2-((2-((1,1-dimethylethyl)amino)ethyl)amino)-6-chinazolinyl)-4-methylbenzamid;
3-(2-(Cyclohexylamino)-6-chinazolinyl)-N-cyclopropyl-4-methylbenzamid;
N-Cyclopropyl-4-methyl-3-(2-(((3S)-6-oxo-3-piperidinyl)amino)-6-chinazolinyl)benzamid;
N-Ethyl-4-methyl-3-(2-(((3S)-6-oxo-3-piperidinyl)amino)-6-chinazolinyl)benzamid;
N-Cyclopropyl-3-(2-((2-(1,1-dioxido-4-thiomorpholinyl)ethyl)amino)-6-chinazolinyl)-4-methylbenzamid;
N-Cyclopropyl-4-methyl-3-(2-((2-(2-oxo-1-pyrrolidinyl)ethyl)amino)-6-chinazolinyl)benzamid;
3-(2-(4-Amino-1-piperidinyl)-6-chinazolinyl)-N-cyclopropyl-4-methylbenzamid;
3-(2-(4-(Acetylamino)-1-piperidinyl)-6-chinazolinyl)-N-cyclopropyl-4-methylbenzamid;
1,1-Dimethylethyl-(1-(6-(5-((cyclopropylamino)carbonyl)-2-methylphenyl)-2-chinazolinyl)-4-piperidinyl)carbamat;
1,1-Dimethylethyl-4-((6-(5-((cyclopropylamino)carbonyl)-2-methylphenyl)-2-chinazolinyl)amino)-1-piperidincarboxylat;
N-Cyclopropyl-4-methyl-3-(2-(4-piperidinylamino)-6-chinazolinyl)benzamid;
3-(2-((1-Acetyl-4-piperidinyl)amino)-6-chinazolinyl)-N-cyclopropyl-4-methylbenzamid;
N-Cyclopropyl-4-methyl-3-(4-(2-methylphenyl)-7-isochinolinyl)benzamid;
4-Methyl-3-(4-(2-methylphenyl)-7-isochinolinyl)benzamid;
Methyl-4-methyl-3-(4-((3S)-3-methyl-4-morpholinyl)-7-isochinolinyl)benzoat;
N-Cyclopropyl-4-methyl-3-(2-((2-(1-piperazinyl)ethyl)amino)-6-chinazolinyl)benzamid;
N-Cyclopropyl-4-methyl-3-(2-((3S)-3-piperidinylamino)-6-chinazolinyl)benzamid;
1,1-Dimethylethyl-2-((6-(5-((cyclopropylamino)carbonyl)-2-methylphenyl)-2-chinazolinyl)amino)-2-methylpropylcarbamat;
1,1-Dimethylethyl-(2S)-2-(((6-(5-((cyclopropylamino)carbonyl)-2-methylphenyl)-2-chinazolinyl)amino)methyl)-1-pyrrolidincarboxylat;
N-Cyclopropyl-4-methyl-3-(2-((2-(4-methyl-1-piperazinyl)ethyl)amino)-6-chinazolinyl)benzamid;
N-Cyclopropyl-4-methyl-3-(2-(((2S)-2-pyrrolidinylmethyl)amino)-6-chinazolinyl)benzamid;
1,1-Dimethylethyl-(2R)-2-(((6-(5-((cyclopropylamino)carbonyl)-2-methylphenyl)-2-chinazolinyl)amino)methyl)-1-pyrrolidincarboxylat;
N-Cyclopropyl-4-methyl-3-(2-(((2R)-2-pyrrolidinylmethyl)amino)-6-chinazolinyl)benzamid;
3-(2-((2-Amino-1,1-dimethylethyl)amino)-6-chinazolinyl)-N-cyclopropyl-4-methylbenzamid;
N-Cyclopropyl-4-methyl-3-(2-(((3R)-1-methyl-3-pyrrolidinyl)amino)-6-chinazolinyl)benzamid;
N-Cyclopropyl-4-methyl-3-(2-((3R)-3-pyrrolidinylamino)-6-chinazolinyl)benzamid;
N-Cyclopropyl-4-methyl-3-(2-((((2S)-1-methyl-2-pyrrolidinyl)methyl)amino)-6-chinazolinyl)benzamid;
N-Cyclopropyl-4-methyl-3-(2-((((2R)-1-(1-methylethyl)-2-pyrrolidinyl)methyl)amino)-6-chinazolinyl)benzamid;
N-Cyclopropyl-4-methyl-3-(2-((((2S)-1-(2-(methyloxy)ethyl)-2-pyrrolidinyl)methyl)amino)-6-chinazolinyl)benzamid;
3-(2-((2-Aminoethyl)amino)-6-chinazolinyl)-N-cyclopropyl-4-methylbenzamid;
N-Cyclopropyl-4-methyl-3-(2-((2-((1-methylethyl)amino)ethyl)amino)-6-chinazolinyl)benzamid
N-Cyclopropyl-3-(2-(((1R,2R)-2-(dimethylamino)cyclohexyl)amino)-6-chinazolinyl)-4-methylbenzamid
N-Cyclopropyl-3-(2-(((1S,2S)-2-(dimethylamino)cyclohexyl)amino)-6-chinazolinyl)-4-methylbenzamid
N-Cyclopropyl-4-methyl-3-(2-(((1R,2R)-2-((1-methylethyl)amino)cyclohexyl)amino)-6-chinazolinyl)benzamid
Methyl-2-((6-(5-((cyclopropylamino)carbonyl)-2-methylphenyl)-2-chinazolinyl)amino)ethylcarbamat
N-Cyclopropyl-4-methyl-3-(2-(((1S,2S)-2-((1-methylethyl)amino)cyclohexyl)amino)-6-chinazolinyl)benzamid;
1-((6-(5-((Cyclopropylamino)carbonyl)-2-methylphenyl)-2-chinazolinyl)amino)cyclopentancarbonsäure;
N-(6-(5-((Cyclopropylamino)carbonyl)-2-methylphenyl)-2-chinazolinyl)-3-(2-thienyl)alanin;
1-((6-(5-((Cyclopropylamino)carbonyl)-2-methylphenyl)-2-chinazolinyl)amino)cyclopropancarbonsäure;
Methyl-N-(6-(5-((cyclopropylamino)carbonyl)-2-methylphenyl)-2-chinazolinyl)-beta-alaninat;
N-(6-(5-((Cyclopropylamino)carbonyl)-2-methylphenyl)-2-chinazolinyl)-2-methylalanin;
N-(6-(5-((Cyclopropylamino)carbonyl)-2-methylphenyl)-2-chinazolinyl)-L-valin;
N,4-Dimethyl-3-(2-((2-(4-morpholinyl)ethyl)amino)-6-chinazolinyl)benzamid;
4-Methyl-3-(2-((2-(4-morpholinyl)ethyl)amino)-6-chinazolinyl)-N-(2,2,2-trifluorethyl)benzamid;
4-Methyl-N-(2-methylpropyl)-3-(2-((2-(4-morpholinyl)ethyl)amino)-6-chinazolinyl)benzamid;
N-Cyclopentyl-4-methyl-3-(2-((2-(4-morpholinyl)ethyl)amino)-6-chinazolinyl)benzamid;
N-Cyclopropyl-3-(2-((3-(dimethylamino)-2,2-dimethylpropyl)amino)-6-chinazolinyl)-4-methylbenzamid;
N-Cyclopropyl-3-(2-(((1S,2R)-2-(dimethylamino)cyclohexyl)amino)-6-chinazolinyl)-4-methylbenzamid;
N-Cyclopropyl-3-(2-((2-(diethylamino)ethyl)amino)-6-chinazolinyl)-4-methylbenzamid;
N-Cyclopropyl-4-methyl-3-(2-((2-(methyloxy)ethyl)amino)-6-chinazolinyl)benzamid;
3-(2-(((1S,2S)-2-Aminocyclohexyl)amino)-6-chinazolinyl)-N-cyclopropyl-4-methylbenzamid;
N-Cyclopropyl-3-(2-(((1R,2R)-2-(dimethylamino)cyclohexyl)amino)-6-chinazolinyl)-4-methylbenzamid;
N-Cyclopropyl-3-(2-((2-furanylmethyl)amino)-6-chinazolinyl)-4-methylbenzamid;
N-Cyclopropyl-4-methyl-3-(2-((tetrahydro-2-furanylmethyl)amino)-6-chinazolinyl)benzamid;
N∼5∼-(6-(4-Methyl-3-pyridinyl)-2-chinazolinyl)-2,5-pyridindiamin;
((1R)-4-(Diethylamino)-1-methylbutyl)(6-(6-fluor-2-methyl-3-pyridinyl)-2-chinazolinyl)amin;
5-(2-((4-(Diethylamino)-1-methylbutyl)amino)-6-chinazolinyl)-6-methyl-2(1H)-pyridinon;
3-(2-(((1R)-4-(Diethylamino)-1-methylbutyl)amino)-6-chinazolinyl)-4-methyl-2(1H)-pyridinon;
((1R)-4-(Diethylamino)-1-methylbutyl)(6-(2-fluor-5-methyl-4-pyridinyl)-2-chinazolinyl)amin;
4-(2-(((1R)-4-(Diethylamino)-1-methylbutyl)amino)-6-chinazolinyl)-5-methyl-2(1H)-pyridinon;
N-(6-(5-((Cyclopropylamino)carbonyl)-2-methylphenyl)-2-chinazolinyl)-D-valin;
N-(6-(5-((Cyclopropylamino)carbonyl)-2-methylphenyl)-2-chinazolinyl)-D-leucin;
N-(6-(5-((Cyclopropylamino)carbonyl)-2-methylphenyl)-2-chinazolinyl)glycin;
N-(6-(5-((Cyclopropylamino)carbonyl)-2-methylphenyl)-2-chinazolinyl)-L-alanin;
N-(6-(5-((Cyclopropylamino)carbonyl)-2-methylphenyl)-2-chinazolinyl)-D-alanin;
5-(2-Amino-6-chinazolinyl)-N-(2-(methyl((3R)-1-methyl-3-pyrrolidinyl)amino)-5-(trifluormethyl)phenyl)-2-(methyloxy)benzamid;
3-(2-Amino-6-chinazolinyl)-N-(2-((3-(dimethylamino)propyl)(methyl)amino)-5-(trifluormethyl)phenyl)-2-((phenylmethyl)amino)benzamid;
4-(2-Amino-6-chinazolinyl)-N-(2-((3-(dimethylamino)propyl)(methyl)amino)-5-(trifluormethyl)phenyl)-5-methyl-2-pyridincarboxamid;
4-(2-Amino-6-chinazolinyl)-N-(2-((3-(dimethylamino)propyl)(methyl)amino)-5-(trifluormethyl)phenyl)-1-(methyloxy)-2-naphthalincarboxamid;
4-(2-Amino-6-chinazolinyl)-1-(methyloxy)-N-(3-(trifluormethyl)phenyl)-2-naphthalincarboxamid;
4-(2-Amino-6-chinazolinyl)-N-(2-(methyl((3R)-1-methyl-3-pyrrolidinyl)amino)-5-(trifluormethyl)phenyl)-1-(methyloxy)-2-naphthalincarboxamid;
4-(2-Amino-6-chinazolinyl)-N-(2-(((3R)-3-(dimethylamino)-1-pyrrolidinyl)methyl)-5-(trifluormethyl)phenyl)-1-(methyloxy)-2-naphthalincarboxamid;
5-(2-Amino-6-chinazolinyl)-N-(2-(3,3-dimethyl-2-oxo-1-azetidinyl)-5-(trifluormethyl)phenyl)-2-(methyloxy)benzamid; und
5-(2-Amino-6-chinazolinyl)-N-(2-((3S)-3-(dimethylamino)-1-piperidinyl)-5-(trifluormethyl)phenyl)-2-fluorbenzamid;
6-(2-Phenylimidazo[1,2-a]pyridin-6-yl)-2-chinazolinamin
6-(2-(4-(Methyloxy)phenyl)imidazo[1,2-a]pyridin-6-yl)-2-chinazolinamin;
6-(2-(3-(Methyloxy)phenyl)imidazo[1,2-a]pyridin-6-yl)-2-chinazolinamin;
6-(2-(2-(Methyloxy)phenyl)imidazo[1,2-a]pyridin-6-yl)-2-chinazolinamin;
6-(2-(1,3-Benzodioxol-5-yl)imidazo[1,2-a]pyridin-6-yl)-2-chinazolinamin;
6-(2-(3-Thienyl)imidazo[1,2-a]pyridin-6-yl)-2-chinazolinamin;
6-(2-Ethyl-1-benzofuran-5-yl)-2-chinazolinamin; und
6-(2-(1,1-Dimethylethyl)imidazo[1,2-a]pyridin-6-yl)-2-chinazolinamin.

3. Pharmazeutische Zusammensetzung, umfassend einen pharmazeutisch verträglichen Träger und eine Verbindung nach einem der Ansprüche 1 oder 2.

4. Verbindung nach einem der Ansprüche 1 oder 2 zur Verwendung zum Modulieren eines Proteinkinase-Enzyms, ausgewählt aus der Gruppe bestehend aus KDR, Lck, p38, tie2 und eine Kombination davon, bei einem Probanden.

5. Verbindung nach einem der Ansprüche 1 oder 2 zur Verwendung beim Behandeln einer Störung, die mit wenigstens einem von Tie-2, Lck, KDR und P38 verbunden ist, bei einem Probanden.

6. Verbindung nach einem der Ansprüche 1 oder 2 zur Verwendung beim Behandeln einer mit der Proliferation verbundenen Störung bei einem Probanden.

7. Verbindung nach Anspruch 6, wobei die Störung ausgewählt ist aus der Gruppe bestehend aus einem Myokardinfarkt, einer Koronararterien-Erkrankung, einer peripheren Gefäßerkrankung, einem Schlaganfall, einer okularen Neovaskularisation, einer Retinopathie, einer diabetischen Retinopathie, einer altersbedingten Makuladegeneration, einer Psoriasis, einem Hämangioblastom, einem Hämangiom, einer Arteriosklerose, einer entzündlichen Erkrankung, rheumatoider Arthritis, Asthma, einer arteriellen oder nach einer Transplantation auftretenden Atherosklerose, einer Endometriose, einer Leukämie und Kombinationen davon.

8. Verbindung nach einem der Ansprüche 1 oder 2 zur Verwendung beim Behandeln von Krebs bei einem Probanden.

9. Verbindung zur Verwendung nach Anspruch 8, wobei das Verabreichen der wirksamen Menge der Verbindung an den Probanden das Verabreichen der Verbindung in Kombination mit einer oder mehreren Verbindungen, ausgewählt aus antineoplastischen Wirkstoffen, antiangiogenen Wirkstoffen, chemotherapeutischen Wirkstoffen und peptidischen Krebstherapie-Wirkstoffen, umfasst.

10. Verbindung zur Verwendung nach Anspruch 9, wobei die antineoplastischen Wirkstoffe ausgewählt sind aus Wirkstoffen vom Antibiotika-Typ, Alkylierungsmitteln, Antimetabolitenwirkstoffen, hormonellen Wirkstoffen, immunologischen Wirkstoffen, Wirkstoffen vom Interferon-Typ, Kinase-Inhibitoren, verschiedenartigen Wirkstoffen und Kombinationen davon.

11. Pharmazeutische Zusammensetzung nach Anspruch 3 zur Verwendung beim Behandeln von Krebs bei einem Probanden.

12. Verbindung nach einem der Ansprüche 1 oder 2 zur Verwendung beim Behandeln von Angiogenese bei einem Probanden.

13. Verbindung nach einem der Ansprüche 1 oder 2 zur Verwendung zum Verringern des Blutflusses in einem Tumor bei einem Probanden.

14. Verbindung nach einem der Ansprüche 1 oder 2 zur Verwendung zum Verringern der Tumorgröße bei einem Probanden.

15. Verbindung nach einem der Ansprüche 1 oder 2 zur Verwendung beim Behandeln einer Entzündung bei einem Probanden.

16. Verbindung nach einem der Ansprüche 1 oder 2 zur Verwendung zum Hemmen der T-Zell-Aktivierung und Proliferation bei einem Probanden.

17. Verbindung nach einem der Ansprüche 1 oder 2 zur Verwendung beim Behandeln von Arthritis, rheumatoider Arthritis, psoriatischer Arthritis oder Osteoarthritis bei einem Probanden.

18. Verbindung nach einem der Ansprüche 1 oder 2 zur Verwendung beim Behandeln einer Organtransplantat-, akuten Transplantat- oder Heterotransplantat- oder Homotransplantat-Abstoßung oder für eine Induktion von Transplantationstoleranz bei einem Probanden.

19. Verbindung nach einem der Ansprüche 1 oder 2 zur Verwendung beim Behandeln eines ischämischen oder Reperfusionsschadens, eines Myokardinfarkts oder eines Schlaganfalls bei einem Probanden.

20. Verbindung nach einem der Ansprüche 1 oder 2 zur Verwendung beim Behandeln von multipler Sklerose, einer entzündlichen Darmerkrankung, einschließlich Colitis ulcerosa, Morbus Crohn, Lupus, Kontaktüberempfindlichkeit, Überempfindlichkeit vom verzögerten Typ und Gluten-empfindlicher Enteropathie, Typ 1-Diabetes, Psoriasis, Kontaktdermatitis, Hashimoto-Thyreoiditis, Sjögren-Syndrom, Autoimmun-Hyperthyreoidismus, Addison-Krankheit, autoimmun-polyglandulärer Erkrankung, Autoimmun-Alopezie, perniziöser Anämie, Vitiligo, Autoimmun-Hypopituitarismus, Guillain-Barré-Syndrom, Glomerulonephritis, Serum-Krankheit, Urtikaria, Allergieerkrankungen, Asthma, Heufieber, allergischer Rhinitis, Skleracielma, Mycosis fungoides, Dermatomyositis, Alopecia areata, chronischer aktinischer Dermatitis, Ekzem, Behçet-Krankheit, Pustulosis palmoplantaris, Pyoderma gangrenosum, Sézary-Syndrom, atopischer Dermatitis, systemischer Sklerose, Morphea oder atopischer Dermatitis bei einem Probanden.

21. Verbindung nach einem der Ansprüche 1 oder 2 zur Verwendung beim Behandeln eines Dickdarmkarzinoms oder Thymoms bei einem Probanden.

22. Verbindung nach einem der Ansprüche 1 oder 2 zur Verwendung beim Herabsetzen des Spiegels von einem oder mehreren von TNF-α, IL-1β, IL-6 und IL-8 bei einem Probanden.

23. Verbindung nach einem der Ansprüche 1 oder 2 zur Verwendung beim Behandeln von rheumatoider Spondylitis, Gichtarthritis, Schocklunge (adult respiratory distress syndrome, ARDS), Anaphylaxie, Muskeldegeneration, Kachexie, Reiter-Syndrom, Typ II-Diabetes, Knochenresorptions-Erkrankungen, Transplantat-Wirt-Reaktion, Alzheimer-Krankheit, Atherosklerose, Hirntrauma, multipler Sklerose, zerebraler Malaria, Sepsis, septischem Schock, toxischem Schocksyndrom, Fieber und Myalgien aufgrund einer Infektion, oder wobei der Proband infiziert ist durch HIV-1, HIV-2, HIV-3, Cytomegalievirus (CMV), Influenza, Adenovirus, die Herpesviren (einschließlich HSV-1, HSV-2) oder Herpes zoster, bei einem Probanden.

24. Verwendung einer Verbindung nach einem der Ansprüche 1 oder 2 für die Herstellung eines Medikaments für die Behandlung von einer Entzündung oder Krebs bei einem Probanden.

25. Verwendung einer Verbindung nach einem der Ansprüche 1 oder 2 für die Herstellung eines Medikaments für die Hemmung der T-Zell-Aktivierung und -Proliferation bei einem Probanden.

26. Verwendung einer Verbindung nach einem der Ansprüche 1 oder 2 für die Herstellung eines Medikaments für die Behandlung von Arthritis, rheumatoider Arthritis, psoriatischer Arthritis oder Osteoarthritis bei einem Probanden.

27. Verwendung einer Verbindung nach einem der Ansprüche 1 oder 2 für die Herstellung eines Medikaments für die Behandlung einer Organtransplantat-, akuten Transplantat- oder Heterotransplantat- oder Homotransplantat-Abstoßung oder eine Induktion von Transplantationstoleranz bei einem Probanden.

28. Verwendung einer Verbindung nach einem der Ansprüche 1 oder 2 für die Herstellung eines Medikaments für die Behandlung eines ischämischen oder Reperfusionsschadens, eines Myokardinfarkts oder eines Schlaganfalls bei einem Probanden.

29. Verwendung einer Verbindung nach einem der Ansprüche 1 oder 2 für die Herstellung eines Medikaments für die Behandlung von multipler Sklerose, einer entzündlichen Darmerkrankung, einschließlich Colitis ulcerosa, Morbus Crohn, Lupus, Kontaktdermatitis, Hashimoto-Thyreoiditis, Sjögren-Syndrom, Autoimmun-Hyperthyreoidismus, Addison-Krankheit, autoimmun-polyglandulärer Erkrankung, Autoimmun-Alopezie, perniziöser Anämie, Vitiligo, Autoimmun-Hypopituitarismus, Guillain-Barré-Syndrom, Glomerulonephritis, Serum-Krankheit, Urtikaria, Allergieerkankungen, Asthma, Heufieber, allergischer Rhinitis, Skleracielma, Mycosis fungoides, Dermatomyositis, Alopecia areata, chronischer aktinischer Dermatitis, Ekzem, Behçet-Krankheit, Pustulosis palmoplantaris, Pyoderma gangrenosum, Sézary-Syndrom, atopischer Dermatitis, systemischer Sklerose, Morphea, atopischer Dermatitis, rheumatoider Spondylitis, Gichtarthritis, Schocklunge (adult respiratory distress syndrome, ARDS), Anaphylaxie, Muskeldegeneration, Kachexie, Reiter-Syndrom, Typ II-Diabetes, Knochenresorptions-Erkrankungen, Transplantat-Wirt-Reaktion, Alzheimer-Krankheit, Atherosklerose, Hirntrauma, multipler Sklerose, zerebraler Malaria, Sepsis, septischem Schock, toxischem Schocksyndrom, Fieber und Myalgien aufgrund einer infektion, oder wobei der Proband infiziert ist durch HIV-1, HIV-2, HIV-3, Cytomegalievirus (CMV), Influenza, Adenovirus, die Herpesviren (einschließlich HSV-1, HSV-2), oder Herpes zoster bei einem Probanden.

30. Verwendung einer Verbindung nach einem der Ansprüche 1 oder 2 für die Herstellung eines Medikaments für die Behandlung eines Dickdarmkarzinoms oder Thymoms bei einem Probanden.

## Revendications

1. Composé répondant à la formule II 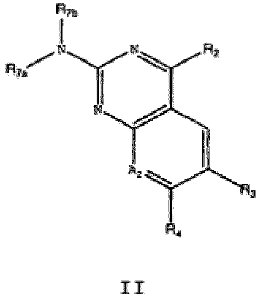
ou stéréoisomère, tautomère, solvate ou sel pharmaceutiquement acceptable de celui-ci, dans lequel
A² est CR⁵ ou N ;
R² est H, NO₂, CN, OR^{7b}, SR^{7b}, alkyle en C_{1 à 10} ou cycloalkyle en C_{3 à 10} ;
R³ est 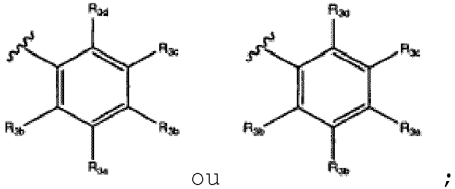 dans lequel
R^{3a} est C(O)R¹⁰, COOR¹⁰, C(O)R¹¹, COOR¹¹, C(O)SR¹⁰, C(O)SR¹¹, C(O)NR¹⁰R¹⁰, C(S)NR¹⁰R¹⁰, C(O)NR¹⁰R¹¹, C(S)NR¹⁰R¹¹, NR¹⁰C(O)R¹⁰, NR¹⁰C(S)R¹⁰, NR¹⁰C(O)R¹¹, NR¹⁰C(S)R¹¹, NR¹⁰C(O)NR¹⁰R¹⁰, NR¹⁰C(O)NR¹⁰R¹¹, NR¹⁰C(S)NR¹⁰R¹⁰, NR¹⁰C(S)NR¹⁰R¹¹, NR¹⁰(COOR¹⁰), NR¹⁰(COOR¹¹), S(O)₂R¹¹, S(O)₂NR¹⁰R¹⁰, S(O)₂NR¹⁰R¹¹, NR¹⁰S(O)₂NR¹⁰R¹¹, NR¹⁰S(O)₂R¹⁰ ou NR¹⁰S(O)₂R¹¹ ;
R^{3b} est H, F, Cl, Br, I, CF₃, CF₂CF₃, OCF₃, OCF₂CF₃, CN, NO₂, NH₂, méthyle, éthyle, propyle, isopropyle, n-butyle, sec-butyle, tert-butyle, acétylényle, cyclopropyle, cyclobutyle, cyclopentyle, cyclohexyle, OH, méthoxyle, éthoxyle, propoxyle, SH, thiométhyle ou thioéthyle ;
R^{3c} est H, F, Cl, Br, I, CF₃, CF₂CF₃, OCF₃, OCF₂CF₃, CN, NO₂, NH₂, méthyle, éthyle, propyle, isopropyle, n-butyle, sec-butyle, tert-butyle, acétylényle, cyclopropyle, cyclobutyle, cyclopentyle, cyclohexyle, OH, méthoxyle, éthoxyle, propoxyle, SH, thiométhyle ou thioéthyle ;
R^{3d} est H, F, Cl, Br, I, CF₃, CF₂CF₃, OCF₃, OCF₂CF₃, CN, NO₂, NH₂, méthyle, éthyle, propyle, isopropyle, n-butyle, sec-butyle, tert-butyle, acétylényle, cyclopropyle, cyclobutyle, cyclopentyle, cyclohexyle, OH, méthoxyle, éthoxyle, propoxyle, SH, thiométhyle ou thioéthyle ;
R⁴ est H, F, Cl, Br, I, CF₃, CF₂CF₃, OCF₃, CN, méthyle, éthyle, propyle, isopropyle, n-butyle, sec-butyle, tert-butyle, OH, méthoxyle, éthoxyle, propoxyle ;
R⁵ est H, F, Cl, Br, I, CF₃, CF₂CF₃, OCF₃, CN, méthyle, éthyle, propyle, isopropyle, n-butyle, sec-butyle, tert-butyle, OH, méthoxyle, éthoxyle, propoxyle ;
R⁶ est H, CN, méthyle, éthyle, propyle, isopropyle ou n-butyle ;
R^{7a} est H, C(O)R⁸, COOR⁸, C(O)R⁹, COOR⁹, C(O)NR⁸R⁹, C(O)NR⁹R⁹, S(O)₂R⁸, S(O)₂NR⁸R⁹, S(O)₂R⁹, S(O)₂NR⁹R⁹, alkyle en C_{1 à 10}, alcényle en C_{2 à 10}, alcynyle en C_{2 à 10} ou cycloalkyle en C_{3 à 10}, chacun parmi alkyle en C_{1 à 10}, alcényle en C_{2 à 10}, alcynyle en C_{2 à 10} et cycloalkyle en C_{3 à 10} optionnellement comprenant 1 à 4 hétéroatomes choisis parmi N, O et S et optionnellement substitué par un ou plusieurs substituants de NR⁸R⁹, NR⁹R⁹, OR⁸, SR⁸, OR⁹, SR⁹, C(O)R⁸, C(O)R⁹, C(O)NR⁸R⁹, C(O)NR⁹R⁹, NR⁹C(O)R⁸, NR⁹C(O)R⁹, NR⁹C(O)NR⁸R⁹, NR⁹C(O)NR⁹R⁹, NR⁹(COOR⁸), NR⁹(COOR⁹), S(O)₂R⁸, S(O)₂NR⁸R⁹, S(O)₂R⁹, S(O)₂NR⁹R⁹, NR⁹S(O)₂NR⁸R⁹, NR⁹S(O)₂NR⁹R⁹, NR⁹S(O)₂R⁸, NR⁹S(O)₂R⁹, R⁸ ou R⁹ ;
R^{7b} est H ;
R⁸ est un système de cycle choisi parmi phényle, naphtyle, pyridyle, pipérazinyle, triazinyle, quinoléinyle, isoquinoléinyle, quinazoléinyle, isoquinazoléinyle, thiophényle, furyle, pyrrolyle, imidazolyle, triazolyle, thiazolyle, oxazolyle, isoxazolyle, isothiazolyle, indolyle, isoindolyle, benzofuranyle, benzothiophényle, benzimidazolyle, tétrahydrofuranyle, pyrrolidinyle, oxazolinyle, isoxazolinyle, thiazolinyle, pyrazolinyle, morpholinyle, pipéridinyle, pipérazinyle, pyranyle, dioxozinyle, cyclopropyle, cyclobutyle, cyclopentyle, cyclohexyle et cycloheptyle, dans lequel le système de cycle est optionnellement substitué indépendamment par 1 à 3 substituants de R⁹, oxo, NR⁹R⁹, OR⁹ ; SR⁹, C(O)R⁹, phényle, pyridyle, pipéridyle, pipérazinyle ou morpholinyle ;
R⁹ est H, halogéno, halogénoalkyle, CN, OH, NO₂, NH₂, acétyle, alkyle en C_{1 à 10}, alcényle en C_{2 à 10}, alcynyle en C_{2 à 10}, cycloalkyle en C_{3 à 10}, alkylamino en C_{1 à 10}, dialkylamino en C_{1 à 10}, alcoxy en C_{1 à 10}, thioalkoxyle en C_{1 à 10} ou un système de cycle choisi parmi phényle, naphtyle, pyridyle, pipérazinyle, triazinyle, quinoléinyle, isoquinoléinyle, quinazoléinyle, isoquinazoléinyle, thiophényle, furyle, pyrrolyle, imidazolyle, triazolyle, thiazolyle, oxazolyle, isoxazolyle, isothiazolyle, indolyle, isoindolyle, benzofuranyle, benzothiophényle, benzimidazolyle, tétrahydrofuranyle, pyrrolidinyle, oxazolinyle, isoxazolinyle, thiazolinyle, pyrazolinyle, morpholinyle, pipéridinyle, pipérazinyle, pyranyle et dioxozinyle, chacun parmi alkyle en C_{1 à 10}, alcényle en C_{2 à 10}, alcynyle en C_{2 à 10}, cycloalkyle en C_{3 à 10}, alkylamino en C_{1 à 10}, dialkylamino en C_{1 à 10}, alcoxyle C_{1 à 10}, thioalcoxyle en C_{1 à 10} et système de cycle optionnellement substitué indépendamment par 1 à 3 substituants de halogéno, halogénoalkyle, CN, NO₂, NH₂, OH, oxo, méthyle, méthoxyle, éthyle, éthoxyle, propyle, propoxyle, isopropyle, cyclopropyle, butyle, isobutyle, tert-butyle, méthylamino, diméthylamino, éthylamino, diéthylamino, propylamine, isopropylamine, dipropylamine, diisopropylamine, benzyle ou phényle ;
R¹⁰ est H, méthyle, éthyle, propyle, isopropyle, n-butyle, sec-butyle, tert-butyle, acétylényle, cyclopropyle, cyclobutyle, cyclopentyle, cyclohexyle, OH, méthoxyle, éthoxyle, propoxyle, SH, thiométhyle ou thioéthyle ; dont chacun est optionnellement substitué par un ou plusieurs substituants de R¹¹, R¹² ou R¹⁶ ;
R¹¹ est phényle, naphtyle, pyridyle, quinoléinyle, tétrahydroquinoléinyle, oxo-tétrahydroquinoléinyle, isoquinoléinyle, oxo-tétrahydroisoquinoléinyle, tétrahydroisoquinoléinyle, thiophényle, tétrahydrofuranyle, thiéno-pyrazolyle, tétrahydropentapyrazolyle, imidazolyle, thiazolyle, thiadiazolyle, benzothiazolyle, oxazolyle, oxadiazolyle, benzoxazolyle, benzoxadiazolyle, isoxazolyle, isothiazolyle, indolyle, 2,3-dihydroindolyle, benzofuranyle, benzothiophényle, benzimidazolyle, 2,3-dihydro-1,4-benzoxazinyle, 1,3-benzodioxolyle, cyclopropyle, cyclobutyle, cyclopentyle et cyclohexyle, ledit système de cycle optionnellement substitué indépendamment par 1 à 3 substituants de R¹², R¹³, R¹⁴ ou R¹⁶ ;
R¹² est H, alkyle en C_{1 à 10}, alcényle en C_{2 à 10}, alcynyle en C_{2 à 10}, cycloalkyle en C₃₋₁₀, cycloalcényle en C_{4 à 10}, alkylamino en C_{1 à 10}, dialkylamino en C_{1 à 10} ou alcoxyle en C_{1 à 10}, dont chacun est optionnellement substitué indépendamment par 1 à 3 substituants de R¹³, R¹⁴, R¹⁵ ou R¹⁶ ;
R¹³ est NR¹⁴R¹⁵, NR¹⁵R¹⁵, OR¹⁴ ; SR¹⁴, OR¹⁵ ; SR¹⁵, C(O)R¹⁴, OC(O)R¹⁴, COOR¹⁴, C(O)R¹⁵, OC(O)R¹⁵, COOR¹⁵, C(O)NR¹⁴R¹⁵, C(O)NR¹⁵R¹⁵, NR¹⁴C(O)R¹⁴, NR¹⁵C(O)R¹⁴, NR¹⁴C(O)R¹⁵, NR¹⁵C(O)R¹⁵, NR¹⁵C(O)NR¹⁴R¹⁵, NR¹⁵C(O)NR¹⁵R¹⁵, NR¹⁵(COOR¹⁴), NR¹⁵(COOR¹⁵), OC(O)NR¹⁴R¹⁵, OC(O)NR¹⁵R¹⁵, S(O)₂R¹⁴, S(O)₂R¹⁵, S(O)₂NR¹⁴R¹⁵, S(O)₂NR¹⁵R¹⁵, NR¹⁴S(O)₂NR¹⁴R¹⁵, NR¹⁵S(O)₂NR¹⁵R¹⁵, NR¹⁴S(O)₂R¹⁴ ou NR¹⁵S(O)₂R¹⁵ ;
R¹⁴ est phényle, pyridyle, pyrimidinyle, thiophényle, furyle, tétrahydrofuryle, pyrrolyle, pyrazolyle, thiéno-pyrazolyle, imidazolyle, triazolyle, thiazolyle, thiadiazolyle, benzothiazolyle, oxazolyle, oxadiazolyle, benzoxazolyle, benzoxadiazolyle, isoxazolyle, isothiazolyle, indolyle, azaindolyle, isoindolyle, indazolyle, benzofuranyle, benzothiophényle, benzimidazolyle, pyrrolidinyle, pyrazolinyle, morpholinyle, pipéridinyle, pipérazinyle, 2,3-dihydro-1,4-benzoxazinyle, 1,3-benzodioxolyle, cyclopropyle, cyclobutyle, azétidinyle, cyclopentyle et cyclohexyle, dont chacun est optionnellement substitué indépendamment par 1 à 3 substituants de R¹⁵ ou R¹⁶ ;
R¹⁵ est H ou alkyle en C_{1 à 10}, alcényle en C_{2 à 10}, alcynyle en C_{2 à 10}, cycloalkyle en C_{3 à 10}, cycloalcényle en C_{4 à 10}, alkylamino en C_{1 à 10}, dialkylamino en C_{1 à 10}, alcoxyle en C_{1 à 10} ou thioalcoxyle en C_{1 à 10}, dont chacun est optionnellement substitué indépendamment par 1 à 3 substituants de R¹⁶ ; et
R¹⁶ est H, halogéno, halogénoalkyle, CN, OH, NO₂, NH₂, OH, méthyle, méthoxyle, éthyle, éthoxyle, propyle, propoxyle, isopropyle, cyclopropyle, butyle, isobutyle, tert-butyle, méthylamino, diméthylamino, éthylamino, diéthylamino, isopropylamino, oxo, acétyle, benzyle ou un système de cycle choisi parmi phényle, pyridyle, thiophényle, furyle, tétrahydrofuryle, pyrrolyle, pyrazolyle, thiéno-pyrazolyle, imidazolyle, triazolyle, thiazolyle, thiadiazolyle, benzothiazolyle, oxazolyle, oxadiazolyle, benzoxazolyle, benzoxadiazolyle, isoxazolyle, isothiazolyle, indolyle, azaindolyle, isoindolyle, indazolyle, benzofuranyle, benzothiophényle, benzimidazolyle, pyrrolidinyle, pyrazolinyle, morpholinyle, pipéridinyle, pipérazinyle, cyclopropyle, cyclobutyle, azétidinyle, cyclopentyle et cyclohexyle, ledit système de cycle optionnellement substitué indépendamment par 1 à 3 substituants de halogéno, halogénoalkyle, CN, NO₂, NH₂, OH, méthyle, méthoxyle, éthyle, éthoxyle, propyle, propoxyle, isopropyle, cyclopropyle, butyle, isobutyle, tert-butyle, méthylamino, diméthylamino, éthylamino, diéthylamino, isopropylamino, benzyle ou phényle ;
à condition que
(1) lorsque A² est N, alors R⁴ n'est pas halogéno ou hydroxyle.

2. Composé et sels pharmaceutiquement acceptables de celui-ci, choisis parmi :
le N-(3-(2-amino-6-quinazoléinyl)-4-méthylphényl)-2-((3-(diméthylamino)propyl)(méthyl)amino)-5-(trifluorométhyl)benzamide ;
le 5-(2-amino-6-quinazoléinyl)-N-(3-chloro-2-((3-(diméthylamino)propyl)(méthyl)amino)-5-(trifluorométhyl)phényl)-2-fluorobenzamide ;
le 5-(2-amino-6-quinazoléinyl)-N-(3-(1-méthyl-4-pipéridinyl)-5-(trifluorométhyl)phényl)-3-thiophènecarboxamide ;
le 3-(2-amino-6-quinazoléinyl)-4-méthyl-N-(2-méthyl-3-((4-méthyl-1-pipérazinyl)méthyl)phényl)benzamide ;
le 3-(2-amino-6-quinazoléinyl)-N-(4-(1,1-diméthyléthyl)-3-((N,N-diméthylglycyl)amino)phényl)-4-méthylbenzamide ;
le 3-(((3-(2-amino-6-quinazoléinyl)-4-méthylphényl)carbonyl)amino)-2,6-difluoro-N-méthylbenzamide ;
le 3-(2-amino-6-quinazoléinyl)-N-(2-fluoro-4-(1-pyrrolidinyl)-3-(1-pyrrolidinylcarbonyl)phényl)-4-méthylbenzamide ;
le 3-(2-amino-6-quinazoléinyl)-4-méthyl-N-(4-méthyl-3-((4-morpholinylacétyl)amino)phényl)benzamide ;
le 3-(2-amino-6-quinazoléinyl)-4-méthyl-N-(2-méthyl-3-((4-morpholinylacétyl)amino)phényl)benzamide ;
le 3-(2-amino-6-quinazoléinyl)-N-(3-((N,N-diéthylglycyl)amino)-2-méthylphényl)-4-méthylbenzamide ;
le 3-(2-amino-6-quinazoléinyl)-N-(1-(N,N-diéthylglycyl)-3,3-diméthyl-2,3-dihydro-1H-indol-6-yl)-4-méthylbenzamide ;
le 3-(2-amino-6-quinazoléinyl)-N-(3,3-diméthyl-1-(4-morpholinylacétyl)-2,3-dihydro-1H-indol-6-yl)-4-méthylbenzamide ;
le 3-(2-amino-6-quinazoléinyl)-4-méthyl-N-(2-méthyl-3-(trifluorométhyl)phényl)benzamide ;
le 3-(2-amino-6-quinazoléinyl)-4-méthyl-N-(3-((trifluorométhyl)oxy)phényl)benzamide ;
le 3-(2-amino-6-quinazoléinyl)-4-méthylbenzoate de phénylméthyle ;
le 3-(2-amino-6-quinazoléinyl)-N-(6-chloro-3-pyridinyl)-4-méthylbenzamide ;
le 3-(2-amino-6-quinazoléinyl)-N-(4-(cyclopropyléthynyl)-3-trifluorométhyl)phényl)-4-méthylbenzamide ;
le 3-(2-amino-6-quinazoléinyl)-N-(4-bromo-3-(trifluorométhyl)phényl)-4-méthylbenzamide ;
le 3-(2-amino-6-quinazoléinyl)-4-méthyl-N-(4-(phényléthynyl)-3-(trifluorométhyl)phényl)benzamide ;
le 3-(2-amino-6-quinazoléinyl)-4-chloro-N-(3-(trifluorométhyl)phényl)benzamide ;
le 3-(2-amino-6-quinazoléinyl)-4-méthyl-N-(2-((2,2,2-trifluoroéthyl)oxy)-5-(trifluorométhyl)phényl)benzamide ;
le 3-(2-amino-6-quinazoléinyl)-4-méthyl-N-(4-((3-(1-pipéridinyl)propyl)oxy)phényl)benzamide ;
le 3-(2-amino-6-quinazoléinyl)-4-méthyl-N-(3-((3-(1-pipéridinyl)propyl)oxy)phényl)benzamide ;
le 3-(2-amino-6-quinazoléinyl)-N-(4-((3-(diméthylamino)propyl)oxy)phényl)-4-méthylbenzamide ;
le 3-(2-amino-6-quinazoléinyl)-N-(4-((2-(diéthylamino)éthyl)oxy)-2-(méthyloxy)phényl)-4-méthylbenzamide ;
le 3-(2-amino-6-quinazoléinyl)-N-(5-((2-(diéthylamino)éthyl)oxy)-2-(méthyloxy)phényl)-4-méthylbenzamide ;
le 3-(2-amino-6-quinazoléinyl)-N-(2,4-bis(méthyloxy)-5-(trifluorométhyl)phényl)-4-méthylbenzamide ;
le 3-(2-amino-6-quinazoléinyl)-N-(5-((diéthylamino)sulfonyl)-2-(méthyloxy)phényl)-4-méthylbenzamide ;
le 3-(2-amino-6-quinazoléinyl)-N-(2,3-dichlorophényl)-4-méthylbenzamide ;
le 3-(2-amino-6-quinazoléinyl)-N-(2,6-bis(méthyloxy)-3-pyridinyl)-4-méthylbenzamide ;
le N-(2-(acétylamino)-5-(trifluorométhyl)phényl)-3-(2-amino-6-quinazoléinyl)-4-méthylbenzamide ;
le 3-(2-amino-6-quinazoléinyl)-4-méthyl-N-(2-(méthyloxy)-5-((3-(4-méthyl-1-pipérazinyl)propyl)oxy)phényl)benzamide ;
le 3-(2-amino-6-quinazoléinyl)-N-(2,2-difluoro-1,3-benzodioxol-4-yl)-4-méthylbenzamide ;
le 3-(2-amino-6-quinazoléinyl)-4-méthyl-N-(2,2,3,3-tétrafluoro-2,3-dihydro-1,4-benzodioxin-5-yl)benzamide ;
le 3-(7-isoquinoléinyl)-N-(3-(trifluorométhyl)phényl)benzamide ;
le 3-(1-hydroxy-7-isoquinoléinyl)-N-(3-(trifluorométhyl)phényl)benzamide ;
le 3-(2-amino-6-quinazoléinyl)-N-(3-(trifluorométhyl)phényl)benzamide ;
le 3-(7-isoquinoléinyl)-N-(2-(1-pipéridinyl)-5-(trifluorométhyl)phényl)benzamide ;
le 3-(2-amino-6-quinazoléinyl)-N-(2-(1-pipéridinyl)-5-(trifluorométhyl)phényl)benzamide ;
le 3-(2-(méthylamino)-6-quinazoléinyl)-N-(3-(trifluorométhyl)phényl)benzamide ;
le 4-(2-amino-6-quinazoléinyl)-N-(3-(trifluorométhyl)phényl)benzamide ;
le 3-(2-amino-6-quinazoléinyl)-4-méthyl-N-(2-(4-méthyl-1-pipérazinyl)-5-(trifluorométhyl)phényl)benzamide ;
le 3-(2-amino-6-quinazoléinyl)-N-(2-((3R)-3-(diméthylamino)-1-pyrrolidinyl)-5-(trifluorométhyl)phényl)-4-méthylbenzamide ;
le 3-(2-amino-6-quinazoléinyl)-4-méthyl-N-(2-(1-pipéridinyl)-5-(trifluorométhyl)phényl)benzamide ;
le 3-(2-amino-6-quinazoléinyl)-N-(2,5-bis(trifluorométhyl)phényl)-4-méthylbenzamide ;
le 3-(2-amino-6-quinazoléinyl)-N-(2,5-bis(1,1-diméthyléthyl)phényl)-4-méthylbenzamide ;
le 3-(2-amino-6-quinazoléinyl)-4-méthyl-N-(2-(4-morpholinyl)-5-(trifluorométhyl)phényl)benzamide ;
le 3-(2-amino-6-quinazoléinyl)-4-méthyl-N-(2-(1-pyrrolidinyl)-5-(trifluorométhyl)phényl)benzamide ;
le 3-(2-amino-6-quinazoléinyl)-N-(2-(diméthylamino)-5-(trifluorométhyl)phényl)-4-méthylbenzamide ;
le N-(3-(2-amino-6-quinazoléinyl)-5-(trifluorométhyl)phényl)-3-(trifluorométhyl)benzamide ;
le N-(3-(2-amino-6-quinazoléinyl)-5-(trifluorométhyl)phényl)-3-(1-méthyléthyl)benzamide ;
le 3-(2-amino-6-quinazoléinyl)-4-méthyl-N-(2-((3R)-1-méthyl-3-pipéridinyl)-5-(trifluorométhyl)phényl)benzamide ;
le 3-(2-amino-6-quinazoléinyl)-N-(3-(1-méthyléthyl)phényl)benzamide ;
le 3-(2-amino-6-quinazoléinyl)-N-(2-(2-(diméthylamino)-1,1-diméthyléthyl)-5-(trifluorométhyl)phényl)benzamide ;
le 3-(2-amino-6-quinazoléinyl)-N-(2-(2-(diméthylamino)-1,1-diméthyléthyl)-5-(trifluorométhyl)phényl)-4-méthylbenzamide ;
le 3-(2-amino-6-quinazoléinyl)-4-méthyl-N-(2-(méthyl((3S)-1-méthyl-3-pyrrolidinyl)amino)-5-(trifluorométhyl)phényl)benzamide ;
le N-(2-(2-(diméthylamino)-1,1-diméthyléthyl)-5-(trifluorométhyl)phényl)-3-(2-(méthylamino)-6-quinazoléinyl)benzamide ;
le 3-(2-amino-6-quinazoléinyl)-N-(2-(méthyl((3R)-1-méthyl-3-pyrrolidinyl)amino)-5-(trifluorométhyl)phényl)benzamide ;
le 3-(2-amino-6-quinazoléinyl)-N-(2-(4-méthyl-1-pipérazinyl)-5-(trifluorométhyl)phényl)benzamide ;
le 3-(2-amino-6-quinazoléinyl)-N-(2-((3-(diméthylamino)propyl)(méthyl)amino)-5-(trifluorométhyl)phényl)benzamide ;
le 3-(2-(méthylamino)-6-quinazoléinyl)-N-(2-((4-méthyl-1-pipérazinyl)méthyl)-5-(trifluorométhyl)phényl)benzamide ;
le 3-(2-amino-6-quinazoléinyl)-4-méthyl-N-(2-((4-méthyl-1-pipérazinyl)méthyl)-5-(trifluorométhyl)phényl)benzamide ;
le 3-(2-amino-6-quinazoléinyl)-N-(2-((3-(diméthylamino)propyl)(méthyl)amino)-5-(1,1-diméthyléthyl)phényl)-4-méthylbenzamide ;
le 3-(2,4-diaminopyrido[2,3-d]pyrimidin-6-yl)-4-méthyl-N-(3-(trifluorométhyl)phényl)benzamide ;
le 5-(2-amino-6-quinazoléinyl)-2-fluoro-N-(2-(1-pipéridinyl)-5-(trifluorométhyl)phényl)benzamide ;
le 5-(2-amino-6-quinazoléinyl)-N-(2-((3-(diméthylamino)propyl)(méthyl)amino)-5-(trifluorométhyl)phényl)-2-fluorobenzamide ;
le 3-(2-amino-6-quinazoléinyl)-N-(2-(2-(diméthylamino)éthyl)-5-(trifluorométhyl)phényl)-4-méthylbenzamide ;
le 5-(2-amino-6-quinazoléinyl)-2-fluoro-N-(2-(méthyl((3R)-1-méthyl-3-pyrrolidinyl)amino)-5-(trifluorométhyl)phényl)benzamide ;
le 3-(2-amino-6-quinazoléinyl)-4-méthyl-N-(2-((2-(1-pyrrolidinyl)éthyl)oxy)-5-(trifluorométhyl)phényl)benzamide ;
le 3-(2-amino-6-quinazoléinyl)-N-(2-((3-(diméthylamino)propyl)(méthyl)amino)-5-(1-méthyléthyl)phényl)benzamide ;
le 5-(2-amino-6-quinazoléinyl)-2-fluoro-N-(2-(4-méthyl-1-pipérazinyl)-5-(trifluorométhyl)phényl)benzamide ;
le 3-(2-amino-6-quinazoléinyl)-N-(2-((3S)-3-(diméthylamino)-1-pipéridinyl)-5-(trifluorométhyl)phényl)-4-méthylbenzamide ;
le 5-(2-amino-6-quinazoléinyl)-N-(2-((3R)-3-(diméthylamino)-1-pyrrolidinyl)-5-(trifluorométhyl)phényl)-2-fluorobenzamide ;
le 3-(2-amino-6-quinazoléinyl)-N-(2-(4-(diméthylamino)-1-pipéridinyl)-5-(trifluorométhyl)phényl)-4-méthylbenzamide ;
le 3-(2-amino-6-quinazoléinyl)-4-méthyl-N-(4-((trifluorométhyl)oxy)phényl)benzamide ;
le 3-(2-amino-6-quinazoléinyl)-N-(2-((3-(diméthylamino)propyl)(méthyl)amino)-5-(1,1-diméthyléthyl)phényl)benzamide ;
le 5-(2-amino-6-quinazoléinyl)-N-(2-((3-(diméthylamino)propyl)(méthyl)amino)-5-(1,1-diméthyléthyl)phényl)-2-fluorobenzamide ;
le 5-(2-amino-6-quinazoléinyl)-N-(2-((3-(diméthylamino)propyl)(méthyl)amino)-5-éthynylphényl)-2-fluorobenzamide ;
le 5-(2-amino-6-quinazoléinyl)-N-(2-((2-(diméthylamino)éthyl) (méthyl)amino)-5-(trifluorométhyl)phényl)-2-fluorobenzamide ;
le 5-(2-amino-6-quinazoléinyl)-N-(2-(diméthylamino)-5-(trifluorométhyl)phényl)-2-fluorobenzamide ;
le N-(2-((3-(diméthylamino)propyl)(méthyl)amino)-5-(trifluorométhyl)phényl)-2-fluoro-5-(2-(méthylamino)-6-quinazoléinyl)benzamide ;
le 5-(2-amino-6-quinazoléinyl)-N-(2-((3-(diméthylamino)propyl)(méthyl)amino)-5-(pentafluoroéthyl)phényl)-2-fluorobenzamide ;
la 6-(2,6-diméthylphényl)-2-quinazoléinamine ;
le 3-(2-amino-6-quinazoléinyl)-N-(2-(diméthylamino)-5-(trifluorométhyl)phényl)benzamide ;
le 3-(2-amino-6-quinazoléinyl)-N-(3-(1,1-diméthyléthyl)phényl)benzamide ;
la 6-(2,6-bis(méthyloxy)phényl)-2-quinazoléinamine ;
le N-(3-(1,1-diméthyléthyl)phényl)-3-(2-(méthylamino)-6-quinazoléinyl)benzamide ;
le 3-(2-amino-6-quinazoléinyl)-N-(3,5-bis(méthyloxy)phényl)benzamide ;
le 3-(2-amino-6-quinazoléinyl)-N-(5-(1,1-diméthyléthyl)-3-isoxazolyl)benzamide ;
le 3-(2-amino-6-quinazoléinyl)-N-(3-((1,1,2,2-tétrafluoroéthyl)oxy)phényl)benzamide ;
le 3-(2-amino-6-quinazoléinyl)-N-(5-(1,1-diméthyléthyl)-1H-pyrazol-3-yl)benzamide ;
le 3-(2-amino-6-quinazoléinyl)-N-(3-chlorophényl)benzamide ;
le 5-(2-amino-6-quinazoléinyl)-N-(5-bromo-2-((3-(diméthylamino)propyl)(méthyl)amino)phényl)-2-fluorobenzamide ;
le 5-(2-amino-6-quinazoléinyl)-N-(2-((3-(diméthylamino)propyl)oxy)-5-(trifluorométhyl)phényl)-2-fluorobenzamide ;
le 5-(2-amino-6-quinazoléinyl)-N-(3-(trifluorométhyl)phényl)-2-thiophènecarboxamide ;
le 3-(2-amino-6-quinazoléinyl)-4-méthyl-N-(3-(1-méthyl-4-pipéridinyl)-5-(trifluorométhyl)phényl)benzamide ;
le 5-(2-amino-6-quinazoléinyl)-N-(2-((3-(diméthylamino)propyl)(méthyl)amino)-5-(trifluorométhyl)phényl)-2-thiophènecarboxamide ;
le 5-(2-amino-6-quinazoléinyl)-N-(3-(trifluorométhyl)phényl)-3-thiophènecarboxamide ;
le 5-(2-amino-6-quinazoléinyl)-N-(2-(2-(diméthylamino)éthyl)-5-(trifluorométhyl)phényl)-2-fluorobenzamide ;
la N-(4-(2-amino-6-quinazoléinyl)-3-méthylphényl)-N'-(3-fluoro-5-(trifluorométhyl)phényl)urée ;
le 5-(2-amino-6-quinazoléinyl)-N-(5-chloro-2-((3-(diméthylamino)propyl)(méthyl)amino)phényl)-2-fluorobenzamide ;
le 4-méthyl-3-(2-(4-méthyl-1-pipérazinyl)-6-quinazoléinyl)-N-(3-(trifluorométhyl)phényl)benzamide ;
la N-(4-(2-amino-6-quinazoléinyl)-3-méthylphényl)-N'-(5-chloro-2-(méthyloxy)phényl)urée ;
le 4-(2-amino-6-quinazoléinyl)-N-(3-(trifluorométhyl)phényl)-2-pyridinecarboxamide ;
le 3-(2-amino-6-quinazoléinyl)-N-(2-(((3-(diméthylamino)propyl)(méthyl)amino)sulfonyl)-5-(trifluorométhyl)phényl)-4-méthylbenzamide ;
le 4-(2-amino-6-quinazoléinyl)-N-(2-((3-(diméthylamino)propyl)(méthyl)amino)-5-(trifluorométhyl)phényl)-2-pyridinecarboxamide ;
le 4-(2-amino-6-quinazoléinyl)-N-(2-((3-(diméthylamino)propyl)(méthyl)amino)-5-(trifluorométhyl)phényl)-2-thiophènecarboxamide ;
le 5-(2-amino-6-quinazoléinyl)-N-(2-((3-(diméthylamino)propyl)(méthyl)amino)-5-(trifluorométhyl)phényl)-3-thiophènecarboxamide ;
le 5-(2-amino-6-quinazoléinyl)-N-(2-((3-(diméthylamino)propyl)(méthyl)amino)-5-(trifluorométhyl)phényl)-2-(méthyloxy)benzamide ;
le 5-(2-amino-6-quinazoléinyl)-2-chloro-N-(2-((3-(diméthylamino)propyl)(méthyl)amino)-5-(trifluorométhyl)phényl)benzamide ;
le 3-(2-amino-6-quinazoléinyl)-5-bromo-N-(2-((3-(diméthylamino)propyl)(méthyl)amino)-5-(trifluorométhyl)phényl)benzamide ;
le 3-(2-amino-6-quinazoléinyl)-N-(5-chloro-2-((3-(diméthylamino)propyl)(méthyl)amino)phényl)-4-méthylbenzamide ;
le 5-(2-amino-6-quinazoléinyl)-N-(2-((3-(diméthylamino)propyl)(méthyl)amino)-5-(trifluorométhyl)phényl)-2,3-dihydro-1-benzofuran-7-carboxamide ;
le 5-(2-amino-6-quinazoléinyl)-2-bromo-N-(2-((3-(diméthylamino)propyl)(méthyl)amino)-5-(trifluorométhyl)phényl)benzamide ;
le 3-(2-amino-6-quinazoléinyl)-N-(3-((trifluorométhyl)oxy)phényl)benzamide ;
le 5-(2-amino-6-quinazoléinyl)-2,3-bis(méthyloxy)-N-(3-(trifluorométhyl)phényl)benzamide ;
le 5-(2-amino-6-quinazoléinyl)-2-(méthyloxy)-N-(3-(trifluorométhyl)phényl)benzamide ;
le 5-(2-amino-6-quinazoléinyl)-N-(5-cyclopropyl-2-((3-(diméthylamino)propyl) (méthyl)amino)phényl)-2-fluorobenzamide ;
le 5-(2-amino-6-quinazoléinyl)-N-(2-((3-(diméthylamino)propyl)(méthyl)amino)-5-(trifluorométhyl)phényl)-2,3-bis(méthyloxy)benzamide ;
le 5-(2-amino-6-quinazoléinyl)-N-(3-(trifluorométhyl)phényl)-2,3-dihydro-1-benzofuran-7-carboxamide ;
le 5-(2-amino-6-quinazoléinyl)-3-bromo-N-(2-((3-(diméthylamino)propyl)(méthyl)amino)-5-(trifluorométhyl)phényl)-2-(méthyloxy)benzamide ;
le 3-(2-amino-6-quinazoléinyl)-5-bromo-N-(2-((3-(diméthylamino)propyl)(méthyl)amino)-5-(trifluorométhyl)phényl)-2-(méthyloxy)benzamide ;
le 5-(2-amino-6-quinazoléinyl)-N-(2-((3-(diméthylamino)propyl)(méthyl)amino)-5-(trifluorométhyl)phényl)-1-méthyl-2-oxo-1,2-dihydro-3-pyridinecarboxamide ;
le 5-(2-amino-6-quinazoléinyl)-2-fluoro-N-(3-((4-méthyl-1-pipérazinyl)méthyl)-5-(trifluorométhyl)phényl)benzamide ;
le 5-(2-amino-6-quinazoléinyl)-2,3-bis(méthyloxy)-N-(3-((4-méthyl-1-pipérazinyl)méthyl)-5-(trifluorométhyl)phényl)benzamide ;
la 1-(4-(2-amino-6-quinazoléinyl)-3-méthylphényl)-3-(3-(trifluorométhyl)phényl)-2-imidazolidinone ;
le 5-(2-amino-6-quinazoléinyl)-2-(méthyloxy)-N-(3-((4-méthyl-1-pipérazinyl)méthyl)-5-(trifluorométhyl)phényl)benzamide ;
la N-(4-(2-amino-6-quinazoléinyl)phényl)-N'-(3-fluoro-5-(trifluorométhyl)phényl)urée ;
la N-(4-(2-amino-6-quinazoléinyl)phényl)-N'-(3-fluorophényl)urée ;
le 5-(2-amino-6-quinazoléinyl)-2-((2-(diméthylamino)éthyl)oxy)-N-(3-(trifluorométhyl)phényl)benzamide ;
le 5-(2-amino-6-quinazoléinyl)-2-(méthyloxy)-N-(3-((trifluorométhyl)oxy)phényl)benzamide ;
le 5-(2-amino-6-quinazoléinyl)-2,3,4-tris(méthyloxy)-N-(3-(trifluorométhyl)phényl)benzamide ;
le 5-(2-amino-6-quinazoléinyl)-N-(2-((3-(diméthylamino)propyl)(méthyl)amino)-5-(trifluorométhyl)phényl)-2,3,4-tris(méthyloxy)benzamide ;
le 5-(2-amino-6-quinazoléinyl)-N-(2-((3-(diméthylamino)propyl)(méthyl)amino)-5-(trifluorométhyl)phényl)-2-(éthyloxy)benzamide ;
le 3-(2-amino-6-quinazoléinyl)-2,6-bis(méthyloxy)-N-(3-(trifluorométhyl)phényl)benzamide ;
le 5-(2-amino-6-quinazoléinyl)-2-(diméthylamino)-N-(2-((3-(diméthylamino)propyl)(méthyl)amino)-5-(trifluorométhyl)phényl)benzamide ;
le N-(4-(2-amino-6-quinazoléinyl)-3-méthylphényl)-3-(trifluorométhyl)benzamide ;
le N-(4-(2-amino-6-quinazoléinyl)phényl)-3-(trifluorométhyl)benzamide ;
la N-(4-(4-amino-6-quinazoléinyl)phényl)-N'-(3-fluorophényl)urée ;
le 3-(2-amino-6-quinazoléinyl)-N-(2-((3-(diméthylamino)propyl)(méthyl)amino)-5-(trifluorométhyl)phényl)-2,6-bis(méthyloxy)benzamide ;
le 5-(2-amino-6-quinazoléinyl)-2-fluoro-N-(3-(trifluorométhyl)phényl)benzamide ;
le N-(3-(2-amino-6-quinazoléinyl)-4-méthylphényl)-3-(1-méthyléthyl)benzamide ;
le N-(5-(2-amino-6-quinazoléinyl)-2-fluorophényl)benzamide ;
le N-(5-(2-amino-6-quinazoléinyl)-2-fluorophényl)cyclopropanecarboxamide ;
le N-(5-(2-amino-6-quinazoléinyl)-2-fluorophényl)-2,3-dichlorobenzamide ;
le N-(5-(2-amino-6-quinazoléinyl)-2-fluorophényl)-4-(1,1-diméthyléthyl)benzamide ;
le N-(5-(2-amino-6-quinazoléinyl)-2-fluorophényl)-3-fluoro-5-(trifluorométhyl)benzamide ;
le N-(5-(2-amino-6-quinazoléinyl)-2-fluorophényl)-3,5-bis(méthyloxy)benzamide ;
le N-(5-(2-amino-6-quinazoléinyl)-2-fluorophényl)-3-(1-méthyléthyl)benzamide ;
le N-(5-(2-amino-6-quinazoléinyl)-2-fluorophényl)-2-fluoro-5-(trifluorométhyl)benzamide ;
le N-(5-(2-amino-6-quinazoléinyl)-2-fluorophényl)-2-(4-méthyl-1-pipérazinyl)-5-(trifluorométhyl)benzamide ;
le N-(5-(2-amino-6-quinazoléinyl)-2-fluorophényl)-2-((3-(diméthylamino)propyl)(méthyl)amino)-5-(trifluorométhyl)benzamide ;
le N-(5-(2-amino-6-quinazoléinyl)-2-fluorophényl)-2-((3R)-3-(diméthylamino)-1-pyrrolidinyl)-5-(trifluorométhyl)benzamide ;
le 3-(2-amino-6-quinazoléinyl)-N-(2-(1,1-dioxydo-4-thiomorpholinyl)-5-(trifluorométhyl)phényl)-4-méthylbenzamide ;
le 3-(2-amino-6-quinazoléinyl)-N-(2-((3-(diméthylamino)propyl)(méthyl)amino)-5-(trifluorométhyl)phényl)-4-méthylbenzamide ;
le 3-(2-amino-6-quinazoléinyl)-4-méthyl-N-phénylbenzamide ;
le 3-(2-amino-6-quinazoléinyl)-4-méthyl-N-(4-(phényloxy)phényl)benzamide ;
le 3-(2-amino-6-quinazoléinyl)-4-méthyl-N-(2-(méthyloxy)phényl)benzamide ;
le 3-(2-amino-6-quinazoléinyl)-N-(2,4-bis(méthyloxy)phényl)-4-méthylbenzamide ;
le 3-(2-amino-6-quinazoléinyl)-N-cyclohexyl-4-méthylbenzamide ;
le 3-(2-amino-6-quinazoléinyl)-4-méthyl-N-(2-(1-pipéridinyl)phényl)benzamide ;
le 3-(2-amino-6-quinazoléinyl)-N-(4-chloro-3-(trifluorométhyl)phényl)-4-méthylbenzamide ;
le 3-(2-amino-6-quinazoléinyl)-N-cyclopentyl-4-méthylbenzamide ;
le N-(2-((3-(diméthylamino)propyl)(méthyl)amino)-5-(trifluorométhyl)phényl)-4-méthyl-3-(2-(méthylamino)-6-quinazoléinyl)benzamide ;
le 3-(2-amino-6-quinazoléinyl)-N-(2-((3-(diméthylamino)propyl)(méthyl)amino)phényl)-4-méthylbenzamide ;
le 3-(2-amino-6-quinazoléinyl)-N-(2-((3-(diméthylamino)propyl)(méthyl)amino)-4-méthylphényl)-4-méthylbenzamide ;
le 3-(2-amino-6-quinazoléinyl)-4-méthyl-N-(4-(1-méthyl-4-pipéridinyl)phényl)benzamide ;
le 3-(2-amino-6-quinazoléinyl)-4-méthyl-N-(2-(4-méthyl-1-pipérazinyl)phényl)benzamide ;
le 3-(2-amino-6-quinazoléinyl)-4-méthyl-N-(2-méthyl-6-(méthyloxy)-3-(trifluorométhyl)phényl)benzamide ;
le 3-(2-amino-6-quinazoléinyl)-N-(3-chloro-4-((2-(1-pipéridinyl)éthyl)oxy)phényl)-4-méthylbenzamide ;
le 3-(2-amino-6-quinazoléinyl)-N-(2-fluoro-5-(trifluorométhyl)phényl)-4-méthylbenzamide ;
le 3-(2-amino-6-quinazoléinyl)-4-méthyl-N-(5-quinoléinyl)benzamide ;
le 3-(2-amino-6-quinazoléinyl)-4-méthyl-N-(8-quinoléinyl)benzamide ;
le 3-(2-amino-6-quinazoléinyl)-N-(5,6-bis(méthyloxy)-1,3-cyclohexadién-1-yl)-4-méthylbenzamide ;
le 3-(2-amino-6-quinazoléinyl)-N-(5-chloro-6-(méthyloxy)-1,3-cyclohexadién-1-yl)-4-méthylbenzamide ;
le 3-(2-amino-6-quinazoléinyl)-N-(2-((3-(diméthylamino)propyl)(méthyl)amino)-4,5-diméthylphényl)-4-méthylbenzamide ;
le 3-(2-amino-6-quinazoléinyl)-N-(6-((3-(diméthylamino)propyl)(méthyl)amino)-2-méthyl-3-(trifluorométhyl)phényl)-4-méthylbenzamide ;
le 3-(2-amino-6-quinazoléinyl)-N-(2-((3-(diméthylamino)propyl)(méthyl)amino)-4-(trifluorométhyl)phényl)-4-méthylbenzamide ;
le 3-(2-amino-6-quinazoléinyl)-4-méthyl-N-(4-(méthyloxy)-3-(trifluorométhyl)phényl)benzamide ;
le 4-méthyl-3-(2-(méthylamino)-6-quinazoléinyl)-N-(2-méthyl-3-(trifluorométhyl)phényl)benzamide ;
le N-(4-chloro-3-(trifluorométhyl)phényl)-4-méthyl-3-(2-(méthylamino)-6-quinazoléinyl)benzamide ;
le 3-(2-amino-6-quinazoléinyl)-N-(4-chloro-2-méthyl-3-(trifluorométhyl)phényl)-4-méthylbenzamide ;
le N-(4-chloro-2-méthyl-3-(trifluorométhyl)phényl)-4-méthyl-3-(2-(méthylamino)-6-quinazoléinyl)benzamide ;
le N-(2-fluoro-5-(trifluorométhyl)phényl)-4-méthyl-3-(2-(méthylamino)-6-quinazoléinyl)benzamide ;
le 3-(2-amino-6-quinazoléinyl)-4-méthyl-N-(3-(trifluorométhyl)phényl)benzamide ;
la N-(3-(2-amino-6-quinazoléinyl)-4-méthylphényl)-N'-(3-(trifluorométhyl)phényl)urée ;
le 3-(2-amino-6-quinazoléinyl)-4-méthyl-N-(4-(4-méthyl-1-pipérazinyl)phényl)benzamide ;
le 3-(2-amino-6-quinazoléinyl)-4-méthyl-N-(phénylméthyl)benzamide ;
le N-(1-acétyl-3,3-diméthyl-2,3-dihydro-1H-indol-6-yl)-3-(2-amino-6-quinazoléinyl)-4-méthylbenzamide ;
le N-(1-acétyl-2,3-dihydro-1H-indol-6-yl)-3-(2-amino-6-quinazoléinyl)-4-méthylbenzamide ;
la N-(3-(2-amino-6-quinazoléinyl)-4-méthylphényl)-N'-phénylurée ;
la N-(3-(2-amino-6-quinazoléinyl)-4-méthylphényl)-N'-(4-(phényloxy)phényl)urée ;
la N-(3-(2-amino-6-quinazoléinyl)-4-méthylphényl)-N'-(3,5-bis(méthyloxy)phényl)urée ;
le 3-(2-amino-6-quinazoléinyl)-4-méthyl-N-(3-pyridinylméthyl)benzamide ;
le 3-(2-amino-6-quinazoléinyl)-4-méthyl-N-(2-pyridinylméthyl)benzamide ;
le 3-(2-amino-6-quinazoléinyl)-4-méthyl-N-((1R)-1-phényléthyl)benzamide ;
le 3-(2-amino-6-quinazoléinyl)-4-méthyl-N-((3-(trifluorométhyl)phényl)méthyl)benzamide ;
le 3-(2-amino-6-quinazoléinyl)-N-(3,5-bis(trifluorométhyl)phényl)-4-méthylbenzamide ;
le 3-(2-amino-6-quinazoléinyl)-N-(2,3-dihydro-1H-indén-4-yl)-4-méthylbenzamide ;
le 3-(2-amino-6-quinazoléinyl)-4-méthyl-N-(2-(méthylsulfanyl)-3-(trifluorométhyl)phényl)benzamide ;
la N-(3-(2-amino-6-quinazoléinyl)-4-méthylphényl)-N'-(phénylméthyl)urée ;
la N-(3-(2-amino-6-quinazoléinyl)-4-méthylphényl)-N'-(3-(trifluorométhyl)phényl)thiourée ;
le 4-méthyl-3-(2-(méthylamino)-6-quinazoléinyl)-N-(3-(trifluorométhyl)phényl)benzamide ;
la N-(3-(2-amino-6-quinazoléinyl)-4-méthylphényl)-N'-phénylthiourée ;
le 3-(2-amino-6-quinazoléinyl)-4-méthyl-N-(4-(pentafluoroéthyl)phényl)benzamide ;
le 3-(trifluorométhyl)phényl-3-(2-amino-6-quinazoléinyl)-4-méthylphénylcarbamate ;
le N-(6-(2-méthyl-5-((phénylcarbonyl)amino)phényl)-2-quinazoléinyl)benzamide ;
le N-(3-(2-amino-6-quinazoléinyl)-4-méthylphényl)benzamide ;
le N-(3-(2-amino-6-quinazoléinyl)-4-méthylphényl)-3-(trifluorométhyl)benzamide ;
le 3-(2-amino-6-quinazoléinyl)-N-(2-fluoro-3-(trifluorométhyl)phényl)-4-méthylbenzamide ;
le 3-(2-amino-6-quinazoléinyl)-4-méthyl-N-((1S)-1-phényléthyl)benzamide ;
le 3-(2-amino-6-quinazoléinyl)-N-((1R)-2-(diméthylamino)-1-phényléthyl)-4-méthylbenzamide ;
le 3-(2-amino-6-quinazoléinyl)-4-méthyl-N-((1R)-2-(4-méthyl-1-pipérazinyl)-1-phényléthyl)benzamide ;
le N-(3-(2-amino-6-quinazoléinyl)-4-méthylphényl)-4-(1,1-diméthyléthyl)benzamide ;
le N-(3-(2-amino-6-quinazoléinyl)-4-méthylphényl)-2,3-dichlorobenzamide ;
le N-(3-(2-amino-6-quinazoléinyl)-4-méthylphényl)-3,5-bis(trifluorométhyl)benzamide ;
le N-(3-(2-amino-6-quinazoléinyl)-4-méthylphényl)-2-fluoro-3-(trifluorométhyl)benzamide ;
le N-(3-(2-amino-6-quinazoléinyl)-4-méthylphényl)-4-((trifluorométhyl)oxy)benzamide ;
le N-(3-(2-amino-6-quinazoléinyl)-4-méthylphényl)-3,5-dichlorobenzamide ;
le N-(3-(2-amino-6-quinazoléinyl)-4-méthylphényl)-3-fluoro-5-(trifluorométhyl)benzamide ;
le N-(3-(2-amino-6-quinazoléinyl)-4-méthylphényl)-4-(méthyloxy)-3-(trifluorométhyl)benzamide ;
le N-(3-(2-amino-6-quinazoléinyl)-4-méthylphényl)-2,3-dichlorobenzamide ;
le N-(3-(2-amino-6-quinazoléinyl)-4-méthylphényl)-2-méthyl-3-(trifluorométhyl)benzamide ;
le N-(3-(2-amino-6-quinazoléinyl)-4-méthylphényl)-2-naphtalènecarboxamide ;
le N-(3-(2-amino-6-quinazoléinyl)-4-méthylphényl)-1-naphtalènecarboxamide ;
le N-(3-(2-amino-6-quinazoléinyl)-4-méthylphényl)-2,2-difluoro-1,3-benzodioxole-4-carboxamide ;
le N-(3-(2-amino-6-quinazoléinyl)-4-méthylphényl)-5,5,8,8-tétraméthyl-5,6,7,8-tétrahydro-2-naphtalènecarboxamide ;
le N-(3-(2-amino-6-quinazoléinyl)-4-méthylphényl)-3-(1,1-diméthyléthyl)-1-méthyl-1H-pyrazole-5-carboxamide ;
le 4-méthyl-3-(2-((2-(4-morpholinyl)éthyl)amino)-6-quinazoléinyl)-N-(3-(trifluorométhyl)phényl)benzamide ;
le 2,3-dichloro-N-(4-méthyl-3-(2-(méthylamino)-6-quinazoléinyl)phényl)benzamide ;
la N-(4-méthyl-3-(2-(méthylamino)-6-quinazoléinyl)phényl)-N'-(3-(trifluorométhyl)phényl)urée ;
le N-(3-(2-amino-6-quinazoléinyl)-4-méthylphényl)-3-chloro-2-fluorobenzamide ;
le N-(3-(2-amino-6-quinazoléinyl)-4-méthylphényl)-3-fluoro-2-(trifluorométhyl)benzamide ;
le N-(3-(2-amino-6-quinazoléinyl)-4-méthylphényl)-1-benzofuran-2-carboxamide ;
le N-(3-(2-amino-6-quinazoléinyl)-4-méthylphényl)-1,3-diméthyl-1H-thiéno[2,3-c]pyrazole-5-carboxamide ;
le N-(3-(2-amino-6-quinazoléinyl)-4-(trifluorométhyl)phényl)-3-(trifluorométhyl)benzamide ;
la N-(3-(2-amino-6-quinazoléinyl)-4-(trifluorométhyl)phényl)-N'-(3-(trifluorométhyl)phényl)urée ;
le 2,3-dichloro-N-(4-méthyl-3-(2-((2-(4-morpholinyl)éthyl)amino)-6-quinazoléinyl)phényl)benzamide ;
le N-(2,3-dihydro-1H-indén-4-yl)-4-méthyl-3-(2-((2-(4-morpholinyl)éthyl)amino)-6-quinazoléinyl)benzamide ;
le N-(3-(2-amino-6-quinazoléinyl)-4-méthylphényl)-3-(trifluorométhyl)benzènesulfonamide ;
le 3-(2-amino-6-quinazoléinyl)-4-méthyl-N-((1S)-1-(1,3-thiazol-2-yl)éthyl)benzamide ;
le 3-(2-amino-6-quinazoléinyl)-N-(2,3-diméthyl-4-(méthyloxy)phényl)-4-méthylbenzamide ;
le N-(1-acétyl-2,3-dihydro-1H-indol-7-yl)-3-(2-amino-6-quinazoléinyl)-4-méthylbenzamide ;
le 3-(2-amino-6-quinazoléinyl)-4-méthyl-N-(1-méthyl-1H-indol-4-yl)benzamide ;
la N,N'-bis(3-(2-amino-6-quinazoléinyl)-4-méthylphényl)urée ;
le N-(2-fluoro-3-(trifluorométhyl)phényl)-4-méthyl-3-(2-((2-(4-morpholinyl)éthyl)amino)-6-quinazoléinyl)benzamide ;
le N-(2-fluoro-3-(trifluorométhyl)phényl)-4-méthyl-3-(2-((3-(4-morpholinyl)propyl)amino)-6-quinazoléinyl)benzamide ;
le N-(2-fluoro-3-(trifluorométhyl)phényl)-4-méthyl-3-(2-((2-(méthyloxy)éthyl)amino)-6-quinazoléinyl)benzamide ;
le N-(2-fluoro-3-(trifluorométhyl)phényl)-4-méthyl-3-(2-((1-méthyl-4-pipéridinyl)amino)-6-quinazoléinyl)benzamide ;
le N-(2-fluoro-3-(trifluorométhyl)phényl)-4-méthyl-3-(2-((2-(1-pyrrolidinyl)éthyl)amino)-6-quinazoléinyl)benzamide ;
le N-(2-fluoro-3-(trifluorométhyl)phényl)-4-méthyl-3-(2-(méthylamino)-6-quinazoléinyl)benzamide ;
le 3-(2-amino-6-quinazoléinyl)-N-(2,1,3-benzoxadiazol-4-yl)-4-méthylbenzamide ;
le 3-(2-amino-6-quinazoléinyl)-N-(2-(2-cyanoéthyl)-2,4,5,6-tétrahydrocyclopenta[c]pyrazol-3-yl)-4-méthylbenzamide ;
le 3-(2-amino-6-quinazoléinyl)-N-(4-((3-(diéthylamino)propyl)oxy)-3-(trifluorométhyl)phényl)-4-méthylbenzamide ;
le 3-(2-amino-6-quinazoléinyl)-4-méthyl-N-(4-((1-méthyl-4-pipéridinyl)oxy)-3-(trifluorométhyl)phényl)benzamide ;
le 3-(2-amino-6-quinazoléinyl)-N-(4-fluoro-3-(trifluorométhyl)phényl)-4-méthylbenzamide ;
le N-(4-fluoro-3-(trifluorométhyl)phényl)-4-méthyl-3-(2-(méthylamino)-6-quinazoléinyl)benzamide ;
le 3-(2-amino-6-quinazoléinyl)-N-(4-cyano-3-(trifluorométhyl)phényl)-4-méthylbenzamide ;
le 4-méthyl-N-(2-méthyl-3-(trifluorométhyl)phényl)-3-(2-((2-(4-morpholinyl)éthyl)amino)-6-quinazoléinyl)benzamide ;
le 3-(2-(cyclopropylamino)-6-quinazoléinyl)-4-méthyl-N-(3-(trifluorométhyl)phényl)benzamide ;
le N-(2-fluoro-3-(trifluorométhyl)phényl)-4-méthyl-3-(2-((4-(4-méthyl-1-pipérazinyl)phényl)amino)-6-quinazoléinyl)benzamide ;
le N-(2-fluoro-3-(trifluorométhyl)phényl)-4-méthyl-3-(2-((4-((3-(1-pipéridinyl)propyl)oxy)phényl)amino)-6-quinazoléinyl)benzamide ;
le 4-méthyl-N-(2-méthyl-3-(trifluorométhyl)phényl)-3-(2-((3-(4-morpholinyl)propyl)amino)-6-quinazoléinyl)benzamide ;
le 4-méthyl-N-(2-méthyl-3-(trifluorométhyl)phényl)-3-(2-((2-(1-pyrrolidinyl)éthyl)amino)-6-quinazoléinyl)benzamide ;
le 4-méthyl-N-(2-méthyl-3-(trifluorométhyl)phényl)-3-(2-((3-(2-oxo-1-pyrrolidinyl)propyl)amino)-6-quinazoléinyl)benzamide ;
le 3-(2-(((1-éthyl-4-pipéridinyl)méthyl)amino)-6-quinazoléinyl)-4-méthyl-N-(2-méthyl-3-(trifluorométhyl)phényl)benzamide ;
le 4-méthyl-3-(2-((1-méthyl-4-pipéridinyl)amino)-6-quinazoléinyl)-N-(2-méthyl-3-(trifluorométhyl)phényl)benzamide ;
le 3-(2-(cyclopropylamino)-6-quinazoléinyl)-4-méthyl-N-(2-méthyl-3-(trifluorométhyl)phényl)benzamide ;
la 6-(2,6-diméthylphényl)-N-(2-(4-morpholinyl)éthyl)-2-quinazoléinamine ;
la 6-(2,6-diméthylphényl)-N-(4-(4-méthyl-1-pipérazinyl)phényl)-2-quinazoléinamine ;
la 6-(2,6-diméthylphényl)-N-(4-((3-(1-pipéridinyl)propyl)oxy)phényl)-2-quinazoléinamine ;
le 5-(2-amino-6-quinazoléinyl)-2-fluoro-4-méthyl-N-(3-(trifluorométhyl)phényl)benzamide ;
le 2-fluoro-4-méthyl-5-(2-(méthylamino)-6-quinazoléinyl)-N-(3-(trifluorométhyl)phényl)benzamide ;
le 5-(2-(cyclopropylamino)-6-quinazoléinyl)-2-fluoro-4-méthyl-N-(3-(trifluorométhyl)phényl)benzamide ;
le 3-(2-(cyclopropylamino)-6-quinazoléinyl)-4-méthyl-N-(4-((1-méthyl-4-pipéridinyl)oxy)-3-(trifluorométhyl)phényl)benzamide ;
le 3-(2-amino-6-quinazoléinyl)-4-méthyl-N-(4-(trifluorométhyl)phényl)benzamide ;
le 3-(2-amino-6-quinazoléinyl)-N-(4-(1,1-diméthyléthyl)phényl)-4-méthylbenzamide ;
le 3-(2-amino-6-quinazoléinyl)-N-(4,4-diméthyl-2-oxo-1,2,3,4-tétrahydro-7-quinoléinyl)-4-méthylbenzamide ;
le 3-(2-amino-6-quinazoléinyl)-N-(4,4-diméthyl-1,2,3,4-tétrahydro-7-quinoléinyl)-4-méthylbenzamide ;
le 3-(2-amino-6-quinazoléinyl)-N-(4,4-diméthyl-1,2,3,4-tétrahydro-7-isoquinoléinyl)-4-méthylbenzamide ;
le 5-(2-amino-6-quinazoléinyl)-2-fluoro-N-(4-(trifluorométhyl)phényl)benzamide ;
le 5-(2-amino-6-quinazoléinyl)-N-(4,4-diméthyl-2-oxo-1,2,3,4-tétrahydro-7-quinoléinyl)-2-fluorobenzamide ;
le 5-(2-amino-6-quinazoléinyl)-N-(4-cyclohexylphényl)-2-fluorobenzamide ;
le 5-(2-amino-6-quinazoléinyl)-2-fluoro-N-(4-(4-morpholinyl)phényl)benzamide ;
le 5-(2-amino-6-quinazoléinyl)-N-(4-(1,1-diméthyléthyl)phényl)-2-fluorobenzamide ;
le 3-(2-amino-6-quinazoléinyl)-5-chloro-N-(4-(trifluorométhyl)phényl)benzamide ;
le 3-(2-amino-6-quinazoléinyl)-5-chloro-N-cyclopropylbenzamide ;
le 3-(2-amino-6-quinazoléinyl)-5-chloro-N-(1-méthyléthyl)benzamide ;
le 3-(2-amino-6-quinazoléinyl)-5-bromo-N-(4-(trifluorométhyl)phényl)benzamide ;
le 3-(2-amino-6-quinazoléinyl)-5-bromo-N-(4-chloro-3-(trifluorométhyl)phényl)benzamide ;
le 3-(2-amino-6-quinazoléinyl)-5-bromo-N-(3-méthyl-4-(1-méthyléthyl)phényl)benzamide ;
le 3-(2-amino-6-quinazoléinyl)-5-bromo-N-(4-((trifluorométhyl)oxy)phényl)benzamide ;
le 3-(2-amino-6-quinazoléinyl)-5-bromo-N-(3-chloro-4-méthylphényl)benzamide ;
le 3-(2-amino-6-quinazoléinyl)-5-bromo-N-cyclopropylbenzamide ;
le N-(3-(2-amino-6-quinazoléinyl)phényl)-5-(diméthylamino)-1-naphtalènesulfonamide ;
le N-(5-(2-amino-6-quinazoléinyl)-2-méthylphényl)-3-(trifluorométhyl)benzamide ;
le N-(5-(2-amino-6-quinazoléinyl)-2-méthylphényl)-4-(1,1-diméthyléthyl)benzamide ;
le N-(5-(2-amino-6-quinazoléinyl)-2-méthylphényl)-5-méthyl-3-isoxazolecarboxamide ;
le N-(5-(2-amino-6-quinazoléinyl)-2-méthylphényl)-3,4-difluorobenzamide ;
le N-(5-(2-amino-6-quinazoléinyl)-2-méthylphényl)cyclopropanecarboxamide ;
le N-(3-(2-amino-6-quinazoléinyl)-5-chlorophényl)-3-(trifluorométhyl)benzamide ;
la N'-(3-(2-amino-6-quinazoléinyl)-5-chlorophényl)-N,N-diméthylurée ;
le N-(3-(2-amino-6-quinazoléinyl)-5-chlorophényl)-4-(1,1-diméthyléthyl)benzamide ;
le N-(3-(2-amino-6-quinazoléinyl)-5-chlorophényl)-4-(diméthylamino)benzamide ;
le N-(3-(2-amino-6-quinazoléinyl)-5-chlorophényl)-3-((trifluorométhyl)oxy)benzamide ;
le N-(3-(2-amino-6-quinazoléinyl)-5-chloro-4-méthylphényl)-4-(trifluorométhyl)benzamide ;
le N-(3-(2-amino-6-quinazoléinyl)-5-chloro-4-méthylphényl)-3-(trifluorométhyl)benzamide ;
le N-(3-(2-amino-6-quinazoléinyl)-5-chloro-4-méthylphényl)-3-chlorobenzamide ;
le N-(3-(2-amino-6-quinazoléinyl)-5-chloro-4-méthylphényl)-4-chlorobenzamide ;
le N-(3-(2-amino-6-quinazoléinyl)-5-chloro-4-méthylphényl)-4-(méthyloxy)-3-(trifluorométhyl)benzamide ;
le 3-(2-amino-6-quinazoléinyl)-N-cyclopropyl-4-(méthyloxy)benzamide ;
le 3-(2-amino-6-quinazoléinyl)-2-méthyl-N-(3-(trifluorométhyl)phényl)benzamide ;
le 3-(2-amino-6-quinazoléinyl)-4-méthyl-N-(1-méthyléthyl)benzamide ;
le 7-(2-méthyl-5-(((3-(trifluorométhyl)phényl)amino)carbonyl)phényl)-3-isoquinoléinecarboxylate d'éthyle ;
le 3-(2-amino-6-quinazoléinyl)-N-(2-(diméthylamino)éthyl)-4-méthylbenzamide ;
l'acide 7-(2-méthyl-5-(((3-(trifluorométhyl)phényl)amino)carbonyl)phényl)-3-isoquinoléinecarboxylique ;
le N-(2-(diméthylamino)éthyl)-7-(2-méthyl-5-(((3-(trifluorométhyl)phényl)amino)carbonyl)phényl)-3-isoquinoléinecarboxamide ;
le 3-(2-amino-6-quinazoléinyl)-4-méthyl-N-(1-méthylpropyl)benzamide ;
le 7-(5-((cyclopropylamino)carbonyl)-2-méthylphényl)-3-isoquinoléinecarboxylate d'éthyle ;
l'acide 7-(5-((cyclopropylamino)carbonyl)-2-méthylphényl)-3-isoquinoléinecarboxylique ;
le N-cyclobutyl-4-méthyl-3-(2-((2-(4-morpholinyl)éthyl)amino)-6-quinazoléinyl)benzamide ;
le 7-(5-((cyclopropylamino)carbonyl)-2-méthylphényl)-N-(2-(diméthylamino)éthyl)-3-isoquinoléinecarboxamide ;
le 3-(2-aminopyrido[2,3-d]pyrimidin-6-yl)-N-(3-(trifluorométhyl)phényl)benzamide ;
le 4-méthyl-3-(2-(méthylamino)pyrido[2,3-d]pyrimidin-6-yl)-N-phénylbenzamide ;
le 3-(2-amino-6-quinazoléinyl)-N-(2-((3-(diméthylamino)propyl)(méthyl)amino)-5-éthynylphényl)-4-méthylbenzamide ;
le N-(2-((3-(diméthylamino)propyl)(méthyl)amino)-5-(trifluorométhyl)phényl)-4-méthyl-3-(2-(méthylamino)pyrido[2,3-d]pyrimidin-6-yl)benzamide ;
le 3-(2-amino-6-quinazoléinyl)-4-méthyl-N-(3-((((2S)-1-méthyl-2-pyrrolidinyl)méthyl)oxy)-5-(trifluorométhyl)phényl)benzamide ;
le N-(2-((3-(diméthylamino)propyl)(méthyl)amino)-5-(trifluorométhyl)phényl)-3-(2-(((éthylamino)carbonyl)amino)-6-quinazoléinyl)-4-méthylbenzamide ;
le 3-(2-(((éthylamino)carbonyl)amino)-6-quinazoléinyl)-4-méthyl-N-(3-(trifluorométhyl)phényl)benzamide ;
le 3-(2-amino-6-quinazoléinyl)-4-méthyl-N-(3-((1-méthyléthyl)oxy)phényl)benzamide ;
le 3-(2-amino-6-quinazoléinyl)-N-(3-(éthyloxy)phényl)-4-méthylbenzamide ;
le 3-(2-(diméthylamino)-6-quinazoléinyl)-4-méthyl-N-(3-(trifluorométhyl)phényl)benzamide ;
le 3-(2-(diméthylamino)-6-quinazoléinyl)-4-méthyl-N-(4-(trifluorométhyl)phényl)benzamide ;
le 2-fluoro-5-(2-(méthylamino)pyrido[2,3-d]pyrimidin-6-yl)-N-(3-(trifluorométhyl)phényl)benzamide ;
le N-(2-((3-(diméthylamino)propyl)(méthyl)amino)-5-(trifluorométhyl)phényl)-2-fluoro-5-(2-(méthylamino)pyrido[2,3-d]pyrimidin-6-yl)benzamide ;
le 3-(2-amino-6-quinazoléinyl)-N-(5-(1,1-diméthyléthyl)-2-(méthyloxy)phényl)-4-méthylbenzamide ;
le N-(3-(2-amino-6-quinazoléinyl)-4-méthylphényl)-2,5-bis(trifluorométhyl)benzamide ;
le N-(3-(2-amino-6-quinazoléinyl)-4-méthylphényl)-2-(trifluorométhyl)benzamide ;
le N-(3-(2-amino-6-quinazoléinyl)-4-méthylphényl)-2-fluoro-4-(trifluorométhyl)benzamide ;
le N-(3-(2-amino-6-quinazoléinyl)-4-méthylphényl)-1-méthyl-1H-indole-2-carboxamide ;
le N-(3-(2-amino-6-quinazoléinyl)-4-méthylphényl)-2,4-bis(trifluorométhyl)benzamide ;
le N-(3-(2-amino-6-quinazoléinyl)-4-méthylphényl)-2,3-difluorobenzamide ;
le N-(3-(2-amino-6-quinazoléinyl)-4-méthylphényl)-3,5-bis(1,1-diméthyléthyl)benzamide ;
le N-(3-(2-amino-6-quinazoléinyl)-4-méthylphényl)-3,5-bis(méthyloxy)benzamide ;
le N-(3-(2-amino-6-quinazoléinyl)-4-méthylphényl)-3,4-diméthylbenzamide ;
le N-(3-(2-amino-6-quinazoléinyl)-4-méthylphényl)-2-chloro-3-(trifluorométhyl)benzamide ;
le N-(3-(2-amino-6-quinazoléinyl)-4-méthylphényl)-2,3-diméthylbenzamide ;
le N-(3-(2-amino-6-quinazoléinyl)-4-méthylphényl)-4-chloro-2-(trifluorométhyl)benzamide ;
le N-(3-(2-amino-6-quinazoléinyl)-4-(méthyloxy)phényl)-1-méthyl-1H-indole-2-carboxamide ;
le N-(3-(2-amino-6-quinazoléinyl)-4-méthylphényl)-2-chloro-3-méthylbenzamide ;
le N-(3-(2-amino-6-quinazoléinyl)-4-méthylphényl)-2-chloro-3-fluorobenzamide ;
le N-(3-(2-amino-6-quinazoléinyl)-4-(méthyloxy)phényl)-2-fluoro-3-(trifluorométhyl)benzamide ;
le N-(3-(2-amino-6-quinazoléinyl)-4-méthylphényl)-3-chloro-2,4-difluorobenzamide ;
le N-(3-(2-amino-6-quinazoléinyl)-4-méthylphényl)-2,3-difluoro-4-(trifluorométhyl)benzamide ;
le N-(3-(2-amino-6-quinazoléinyl)-4-méthylphényl)-2,3-difluoro-4-méthylbenzamide ;
le N-(3-(2-amino-6-quinazoléinyl)phényl)-2-fluoro-3-(trifluorométhyl)benzamide ;
le N-(3-(2-amino-6-quinazoléinyl)phényl)-2,3-dichlorobenzamide ;
le N-(3-(2-amino-6-quinazoléinyl)-4-fluorophényl)-2-fluoro-3-(trifluorométhyl)benzamide ;
le N-(3-(2-amino-6-quinazoléinyl)-4-fluorophényl)-2,3-dichlorobenzamide ;
le N-(3-(2-amino-6-quinazoléinyl)-4-chlorophényl)-2-fluoro-3-(trifluorométhyl)benzamide ;
le N-(3-(2-amino-6-quinazoléinyl)-4-chlorophényl)-2,3-dichlorobenzamide ;
le 3-(2-amino-6-quinazoléinyl)-4-fluoro-N-(2-fluoro-3-(trifluorométhyl)phényl)benzamide ;
le 3-(2-amino-6-quinazoléinyl)-4-fluoro-N-(3-(trifluorométhyl)phényl)benzamide ;
le 3-(2-amino-6-quinazoléinyl)-N-(5-(1,1-diméthyléthyl)-2-(méthyloxy)phényl)-4-fluorobenzamide ;
le 3-(2-amino-6-quinazoléinyl)-4-chloro-N-(5-(1,1-diméthyléthyl)-2-(méthyloxy)phényl)benzamide ;
le 3-(2-amino-6-quinazoléinyl)-4-chloro-N-(2-fluoro-3-(trifluorométhyl)phényl)benzamide ;
le 3-(2-amino-6-quinazoléinyl)-4-chloro-N-(2-méthyl-3-(trifluorométhyl)phényl)benzamide ;
le 3-(2-amino-6-quinazoléinyl)-4-chloro-N-(3-((trifluorométhyl)oxy)phényl)benzamide ;
le 3-(4-amino-6-quinazoléinyl)-4-méthyl-N-(3-(trifluorométhyl)phényl)benzamide ;
le 3-(4-amino-6-quinazoléinyl)-4-méthyl-N-(4-(phényloxy)phényl)benzamide ;
le 3-(4-(((1E)-(diméthylamino)méthylidène)amino)-6-quinazoléinyl)-4-méthyl-N-(4-(phényloxy)phényl)benzamide ;
le 3-(4-amino-6-quinazoléinyl)-4-méthyl-N-phénylbenzamide ;
le 3-(4-amino-6-quinazoléinyl)-N-(3-bromophényl)-4-méthylbenzamide ;
le 3-(4-amino-6-quinazoléinyl)-N-(4-(1,1-diméthyléthyl)phényl)-4-méthylbenzamide ;
le 3-(4-amino-6-quinazoléinyl)-4-méthyl-N-(4-(4-morpholinyl)phényl)benzamide ;
le 3-(4-amino-6-quinazoléinyl)-4-méthyl-N-(2-((4-méthyl-1-pipérazinyl)méthyl)-5-(trifluorométhyl)phényl)benzamide ;
le 3-(4-amino-6-quinazoléinyl)-4-méthyl-N-(2-(4-morpholinyl)-5-(trifluorométhyl)phényl)benzamide ;
le 3-(4-amino-6-quinazoléinyl)-N-(2-(diméthylamino)-5-(trifluorométhyl)phényl)-4-méthylbenzamide ;
le 3-(4-amino-6-quinazoléinyl)-4-méthyl-N-(2-(1-pyrrolidinyl)-5-(trifluorométhyl)phényl)benzamide ;
le 3-(4-amino-6-quinazoléinyl)-N-(2-fluoro-5-(trifluorométhyl)phényl)-4-méthylbenzamide ;
le 3-(4-amino-6-quinazoléinyl)-4-méthyl-N-(2-(méthyloxy)-5-(trifluorométhyl)phényl)benzamide ;
le 3-(4-amino-6-quinazoléinyl)-N-cyclopropyl-4-méthylbenzamide ;
le 3-(4-amino-6-quinazoléinyl)-N-cyclopentyl-4-méthylbenzamide ;
le 3-(4-amino-6-quinazoléinyl)-N-(2,3-diméthylphényl)-4-méthylbenzamide ;
le 3-(4-amino-6-quinazoléinyl)-N-(2,5-bis(méthyloxy)phényl)-4-méthylbenzamide ;
le 3-(4-amino-6-quinazoléinyl)-N-(cyclopropylméthyl)-4-méthylbenzamide ;
le 3-(4-amino-6-quinazoléinyl)-4-méthyl-N-(3-((1-méthyléthyl)oxy)phényl)benzamide ;
le 3-(2-amino-6-quinazoléinyl)-N-(3,4-diméthyl-5-isoxazolyl)-4-méthylbenzamide ;
le 3-(4-amino-6-quinazoléinyl)-N-(3-(1,1-diméthyléthyl)phényl)-4-méthylbenzamide ;
le 3-(2-amino-6-quinazoléinyl)-N-(5-(1,1-diméthyléthyl)-3-isoxazolyl)-4-méthylbenzamide ;
le 3-(4-amino-6-quinazoléinyl)-N-(3,4-bis(méthyloxy)-5-((3-(4-méthyl-1-pipérazinyl)propyl)oxy)phényl)-4-méthylbenzamide ;
le 3-(4-amino-6-quinazoléinyl)-4-méthyl-N-(3-((trifluorométhyl)oxy)phényl)benzamide ;
le 3-(4-amino-6-quinazoléinyl)-N-(4,5-diméthyl-3-isoxazolyl)-4-méthylbenzamide ;
le 3-(4-amino-6-quinazoléinyl)-4-méthyl-N-(phénylméthyl)benzamide ;
le 3-(4-amino-6-quinazoléinyl)-4-méthyl-N-(2-méthyl-5-(1-méthyléthyl)phényl)benzamide ;
le 3-(4-amino-6-quinazoléinyl)-4-méthyl-N-(3-((phénylméthyl)oxy)phényl)benzamide ;
le 3-(2-amino-6-quinazoléinyl)-4-méthyl-N-(4-pyridinylméthyl)benzamide ;
le 3-(2-amino-6-quinazoléinyl)-4-méthyl-N-(2-pyridinylméthyl)benzamide ;
le 3-(4-amino-6-quinazoléinyl)-N-(1,1'-biphényl-3-yl)-4-méthylbenzamide ;
le 3-(4-amino-6-quinazoléinyl)-N-(5-(1,1-diméthyléthyl)-2-méthyloxy)phényl)-4-méthylbenzamide ;
le 3-(4-amino-6-quinazoléinyl)-N-(5-(1,1-diméthyléthyl)-3-isoxazolyl)-4-méthylbenzamide ;
le 3-(4-amino-6-quinazoléinyl)-4-méthyl-N-(3-méthyl-5-isoxazolyl)benzamide ;
le 3-(4-amino-6-quinazoléinyl)-4-méthyl-N-(3-((1,1,2,2-tétrafluoroéthyl)oxy)phényl)benzamide ;
le 3-(4-amino-6-quinazoléinyl)-N-(5-cyclohexyl-2-(méthyloxy)phényl)-4-méthylbenzamide ;
le 3-(4-amino-6-quinazoléinyl)-N-(3,5-bis(1,1-diméthyléthyl)phényl)-4-méthylbenzamide ;
le 3-(2-amino-6-quinazoléinyl)-4-méthyl-N-(3-méthyl-5-isoxazolyl)benzamide ;
le 3-(4-amino-6-quinazoléinyl)-4-méthyl-N-((2S)-tétrahydro-2-furanylméthyl)benzamide ;
le 3-(2-amino-6-quinazoléinyl)-N-(3,5-bis(1,1-diméthyléthyl)phényl)-4-méthylbenzamide ;
le 3-(2-amino-6-quinazoléinyl)-4-méthyl-N-(4-pyridinyl)benzamide ;
le 3-(2-amino-6-quinazoléinyl)-4-méthyl-N-(2-pyridinyl)benzamide ;
le 3-(2-amino-6-quinazoléinyl)-N-(2-chloro-5-(trifluorométhyl)-3-pyridinyl)-4-méthylbenzamide ;
le 3-(2-amino-6-quinazoléinyl)-4-méthyl-N-(1,3-thiazol-2-yl)benzamide ;
le 3-(2-amino-6-quinazoléinyl)-4-méthyl-N-(3-pyridinyl)benzamide ;
le 3-(2-amino-6-quinazoléinyl)-N-(5-bromo-2-pyridinyl)-4-méthylbenzamide ;
le 3-(2-amino-6-quinazoléinyl)-4-méthyl-N-(6-(4-morpholinyl)-3-pyridinyl)benzamide ;
le 3-(2-amino-6-quinazoléinyl)-4-méthyl-N-(3-quinoléinyl)benzamide ;
le 3-(2-amino-6-quinazoléinyl)-4-méthyl-N-(6-quinoléinyl)benzamide ;
le 3-(2-amino-6-quinazoléinyl)-N-(2-chloro-4-pyridinyl)-4-méthylbenzamide ;
le 3-(2-amino-6-quinazoléinyl)-4-méthyl-N-(2-naphtalényl)benzamide ;
le 3-(2-amino-6-quinazoléinyl)-N-(4,5-dihydro-1,3-thiazol-2-yl)-4-méthylbenzamide ;
le 3-(2-amino-6-quinazoléinyl)-4-méthylbenzènecarbothioate de S-(4,5-dihydro-1,3-thiazol-2-yle) ;
le 3-(2-amino-6-quinazoléinyl)-N-(5-éthyl-1,3,4-thiadiazol-2-yl)-4-méthylbenzamide ;
le 3-(2-amino-6-quinazoléinyl)-4-méthyl-N-(4-méthyl-2-pyridinyl)benzamide ;
le 3-(2-amino-6-quinazoléinyl)-N-(4-éthyl-2-pyridinyl)-4-méthylbenzamide ;
le 3-(2-amino-6-quinazoléinyl)-4-méthyl-N-(4-méthyl-1,3-thiazol-2-yl)benzamide ;
le 3-(2-amino-6-quinazoléinyl)-N-(5-chloro-1,3-benzoxazol-2-yl)-4-méthylbenzamide ;
le 3-(2-amino-6-quinazoléinyl)-4-méthyl-N-(5-(trifluorométhyl)-1,3,4-thiadiazol-2-yl)benzamide ;
le 3-(2-amino-6-quinazoléinyl)-4-méthyl-N-(2-(3-pyridinyl)éthyl)benzamide ;
le 3-(2-amino-6-quinazoléinyl)-4-méthyl-N-(5-méthyl-3-isothiazolyl)benzamide ;
le 3-(2-amino-6-quinazoléinyl)-4-méthyl-N-(4-(trifluorométhyl)-2-pyridinyl)benzamide ;
le 3-(2-amino-6-quinazoléinyl)-N-(3,5-dichloro-2-pyridinyl)-4-méthylbenzamide ;
le 3-(2-amino-6-quinazoléinyl)-4-méthyl-N-(2-(méthyloxy)-3-pyridinyl)benzamide ;
le 3-(2-amino-6-quinazoléinyl)-N-(4,5-diméthyl-1,3-thiazol-2-yl)-4-méthylbenzamide ;
le 3-(2-amino-6-quinazoléinyl)-4-méthyl-N-(4-méthyl-1,3-benzothiazol-2-yl)benzamide ;
le 3-(2-amino-6-quinazoléinyl)-N-(6-(éthyloxy)-1,3-benzothiazol-2-yl)-4-méthylbenzamide ;
le 3-(2-amino-6-quinazoléinyl)-4-méthyl-N-(5-nitro-1,3-thiazol-2-yl)benzamide ;
le 3-(2-amino-6-quinazoléinyl)-N-(6-fluoro-1,3-benzothiazol-2-yl)-4-méthylbenzamide ;
le 3-(2-amino-6-quinazoléinyl)-N-(4-(1,1-diméthyléthyl)-1,3-thiazol-2-yl)-4-méthylbenzamide ;
le 3-(2-amino-6-quinazoléinyl)-N-(5-iodo-2-pyridinyl)-4-méthylbenzamide ;
le 3-(2-amino-6-quinazoléinyl)-4-méthyl-N-(1-méthyl-1H-benzimidazol-2-yl)benzamide ;
le N-(2-acétyl-3-thiényl)-3-(2-amino-6-quinazoléinyl)-4-méthylbenzamide ;
le 3-(2-amino-6-quinazoléinyl)-N-(5-bromo-1,3-thiazol-2-yl)-4-méthylbenzamide ;
le 3-(2-amino-6-quinazoléinyl)-4-méthyl-N-((6-(trifluorométhyl)-3-pyridinyl)méthyl)benzamide ;
le 3-(2-amino-6-quinazoléinyl)-4-méthyl-N-(3-((phénylméthyl)oxy)-2-pyridinyl)benzamide ;
le 3-(2-amino-6-quinazoléinyl)-N-(2-fluoro-4-(1-pyrrolidinyl)-3-(1-pyrrolidinylcarbonyl)phényl)-4-méthylbenzamide ;
le 3-(2-amino-6-quinazoléinyl)-4-méthyl-N-(6-(trifluorométhyl)-2-pyridinyl)benzamide ;
le 3-(2-amino-6-quinazoléinyl)-4-méthyl-N-(5-(trifluorométhyl)-2-pyridinyl)benzamide ;
le 3-(2-amino-6-quinazoléinyl)-N-(3-hydroxy-2-pyridinyl)-4-méthylbenzamide ;
le 3-(2-amino-6-quinazoléinyl)-N-(2,4-difluorophényl)-4-méthylbenzamide ;
le 3-(2-amino-6-quinazoléinyl)-4-méthyl-N-(3-((3-(1-pipéridinyl)propyl)oxy)-2-pyridinyl)benzamide ;
le 3-(2-amino-6-quinazoléinyl)-N-(3-cyanophényl)-4-méthylbenzamide ;
le 3-(2-amino-6-quinazoléinyl)-N-(3-((2-(diéthylamino)éthyl)oxy)-2-pyridinyl)-4-méthylbenzamide ;
le 3-(2-amino-6-quinazoléinyl)-4-méthyl-N-(4-(méthyloxy)-1,1'-biphényl-3-yl)benzamide ;
le 3-(2-amino-6-quinazoléinyl)-4-méthyl-N-(2-méthyl-5-(trifluorométhyl)phényl)benzamide ;
le 3-(2-amino-6-quinazoléinyl)-N-(5-chloro-2-(méthyloxy)phényl)-4-méthylbenzamide ;
le 3-(2-amino-6-quinazoléinyl)-N-(2-((3-(diméthylamino)propyl)oxy)-5-(trifluorométhyl)phényl)-4-méthylbenzamide ;
le 3-(2-amino-6-quinazoléinyl)-4-méthyl-N-(2-(méthyloxy)-5-((phénylamino)carbonyl)phényl)benzamide ;
le 3-(2-amino-6-quinazoléinyl)-4-méthyl-N-(2-(méthylsulfanyl)-5-(trifluorométhyl)phényl)benzamide ;
le 3-(2-amino-6-quinazoléinyl)-N-(2-fluoro-5-méthylphényl)-4-méthylbenzamide ;
le 3-(2-amino-6-quinazoléinyl)-N-(2,5-bis(méthyloxy)phényl)-4-méthylbenzamide ;
le 5-(2-amino-6-quinazoléinyl)-2-chloro-N-(3-(trifluorométhyl)phényl)benzamide ;
le 3-(2-amino-6-quinazoléinyl)-5-fluoro-N-(3-(trifluorométhyl)phényl)benzamide ;
le 3-(2-amino-6-quinazoléinyl)-5-bromo-N-(4-(1,1-diméthyléthyl)phényl)benzamide ;
le 3-(2-amino-6-quinazoléinyl)-N-(3,5-bis(méthyloxy)phényl)-5-bromobenzamide ;
le 3-(2-amino-6-quinazoléinyl)-5-bromo-N-(3-(trifluorométhyl)phényl)benzamide ;
le 3-(2-amino-6-quinazoléinyl)-5-chloro-N-(3-(trifluorométhyl)phényl)benzamide ;
le 3-(2-amino-6-quinazoléinyl)-4-chloro-N-(2-(1-pyrrolidinyl)-5-(trifluorométhyl)phényl)benzamide ;
le 3-(2-amino-6-quinazoléinyl)-4-chloro-N-(2-(1-pipéridinyl)-5-(trifluorométhyl)phényl)benzamide ;
le 3-(2-amino-6-quinazoléinyl)-4-chloro-N-(2-(4-morpholinyl)-5-(trifluorométhyl)phényl)benzamide ;
le 3-(4-amino-6-quinazoléinyl)-4-méthyl-N-(3-(1-méthyléthyl)phényl)benzamide ;
le 3-(4-amino-6-quinazoléinyl)-N-(3-(1-méthyléthyl)phényl)benzamide ;
le 3-(4-amino-6-quinazoléinyl)-4-méthyl-N-(2-(méthyl((3S)-1-méthyl-3-pyrrolidinyl)amino)-5-(trifluorométhyl)phényl)benzamide ;
le 3-(4-amino-6-quinazoléinyl)-N-(2-((3-(diméthylamino)propyl)(méthyl)amino)-5-(trifluorométhyl)phényl)-4-méthylbenzamide ;
le 3-(4-amino-6-quinazoléinyl)-4-méthyl-N-(2-(1-pipéridinyl)-5-(trifluorométhyl)phényl)benzamide ;
le N-(3-(4-amino-6-quinazoléinyl)phényl)-3-(trifluorométhyl)benzamide ;
le N-(3-(4-amino-6-quinazoléinyl)phényl)benzamide ;
le N-(3-(4-amino-6-quinazoléinyl)phényl)-3-(méthyloxy)benzamide ;
le N-(3-(4-amino-6-quinazoléinyl)phényl)-4-(trifluorométhyl)benzamide ;
le 3-(2-amino-6-quinazoléinyl)-N-cyclopropyl-4-méthylbenzamide ;
le 3-(2-amino-6-quinazoléinyl)-4-méthyl-N-(2-(méthyloxy)-5-(trifluorométhyl)phényl)benzamide ;
le N-cyclopropyl-4-méthyl-3-(2-((2-(4-morpholinyl)éthyl)amino)-6-quinazoléinyl)benzamide ;
le 4-méthyl-N-(2-(méthyloxy)-5-(trifluorométhyl)phényl)-3-(2-((2-(4-morpholinyl)éthyl)amino)-6-quinazoléinyl)benzamide ;
le N-cyclopropyl-3-(2-((2-(diméthylamino)éthyl)amino)-6-quinazoléinyl)-4-méthylbenzamide ;
le N-cyclopropyl-4-méthyl-3-(2-((1-méthyl-4-pipéridinyl)amino)-6-quinazoléinyl)benzamide ;
le 2-(((6-(5-((cyclopropylamino)carbonyl)-2-méthylphényl)-2-quinazoléinyl)amino)méthyl)-1-pipéridinecarboxylate de 1,1-diméthyléthyle ;
le N-cyclopropyl-4-méthyl-3-(2-((2-(1-pipéridinyl)éthyl)amino)-6-quinazoléinyl)benzamide ;
le N-cyclopropyl-4-méthyl-3-(2-(4-méthyl-1-pipérazinyl)-6-quinazoléinyl)benzamide ;
le 4-(2-((6-(5-((cyclopropylamino)carbonyl)-2-méthylphényl)-2-quinazoléinyl)amino)éthyl)-1-pipérazinecarboxylate de 1,1-diméthyléthyle ;
le 4-méthyl-3-(2-(méthylamino)pyrido[2,3-d]pyrimidin-6-yl)-N-(3-(trifluorométhyl)phényl)benzamide ;
le N-(2,3-dihydro-1H-indén-4-yl)-4-méthyl-3-(2-(méthylamino)pyrido[2,3-d]pyrimidin-6-yl)benzamide ;
le N-(4-méthyl-3-(2-(méthylamino)pyrido[2,3-d]pyrimidin-6-yl)phényl)-3-(trifluorométhyl)benzamide ;
le N-(4-chloro-3-(trifluorométhyl)phényl)-4-méthyl-3-(2-(méthylamino)pyrido[2,3-d]pyrimidin-6-yl)benzamide ;
le 4-méthyl-3-(2-(méthylamino)pyrido[2,3-d]pyrimidin-6-yl)-N-(3-méthyl-4-(1-méthyléthyl)phényl)benzamide ;
le N-(4-méthyl-3-(2-(méthylamino)pyrido[2,3-d]pyrimidin-6-yl)phényl)-3,5-bis(trifluorométhyl)benzamide ;
le 2,3-dichloro-N-(4-méthyl-3-(2-(méthylamino)pyrido[2,3-d]pyrimidin-6-yl)phényl)benzamide ;
le 2-fluoro-N-(4-méthyl-3-(2-(méthylamino)pyrido[2,3-d]pyrimidin-6-yl)phényl)-3-(trifluorométhyl)benzamide ;
le 4-méthyl-3-(2-(méthylamino)pyrido[2,3-d]pyrimidin-6-yl)-N-(2-méthyl-3-(trifluorométhyl)phényl)benzamide ;
le 2-fluoro-N-(4-méthyl-3-(2-(méthylamino)pyrido[2,3-d]pyrimidin-6-yl)phényl)-5-(trifluorométhyl)benzamide ;
le 4-(1,1-diméthyléthyl)-N-(4-méthyl-3-(2-(méthylamino)pyrido[2,3-d]pyrimidin-6-yl)phényl)benzamide ;
le 4-méthyl-3-(2-(méthylamino)pyrido[2,3-d]pyrimidin-6-yl)-N-(4-(trifluorométhyl)phényl)benzamide ;
le N-(4-(1,1-diméthyléthyl)phényl)-4-méthyl-3-(2-(méthylamino)pyrido[2,3-d]pyrimidin-6-yl)benzamide ;
le N-(3-(1,1-diméthyléthyl)phényl)-4-méthyl-3-(2-(méthylamino)pyrido[2,3-d]pyrimidin-6-yl)benzamide ;
le N-(4-méthyl-3-(2-(méthylamino)pyrido[2,3-d]pyrimidin-6-yl)phényl)-2,4-bis(trifluorométhyl)benzamide ;
le N-(4-méthyl-3-(2-(méthylamino)pyrido[2,3-d]pyrimidin-6-yl)phényl)-2,3-dihydro-1-benzofuran-7-carboxamide ;
le 3-chloro-2-fluoro-N-(4-méthyl-3-(2-(méthylamino)pyrido[2,3-d]pyrimidin-6-yl)phényl)benzamide ;
le 3-fluoro-N-(4-méthyl-3-(2-(méthylamino)pyrido[2,3-d]pyrimidin-6-yl)phényl)-2-(trifluorométhyl)benzamide ;
le 2-chloro-3-fluoro-N-(4-méthyl-3-(2-(méthylamino)pyrido[2,3-d]pyrimidin-6-yl)phényl)benzamide ;
le 2-chloro-3-méthyl-N-(4-méthyl-3-(2-(méthylamino)pyrido[2,3-d]pyrimidin-6-yl)phényl)benzamide ;
le 2,3-difluoro-N-(4-méthyl-3-(2-(méthylamino)pyrido[2,3-d]pyrimidin-6-yl)phényl)-4-(trifluorométhyl)benzamide ;
le 2,3-difluoro-4-méthyl-N-(4-méthyl-3-(2-(méthylamino)pyrido[2,3-d]pyrimidin-6-yl)phényl)benzamide ;
le 4-méthyl-3-(2-(méthylamino)pyrido[2,3-d]pyrimidin-6-yl)-N-(3-((trifluorométhyl)oxy)phényl)benzamide ;
le 2-méthyl-3-(2-(méthylamino)pyrido[2,3-d]pyrimidin-6-yl)-N-(3-(trifluorométhyl)phényl)benzamide ;
le 2-méthyl-3-(2-(méthylamino)pyrido[2,3-d]pyrimidin-6-yl)-N-(2-méthyl-3-(trifluorométhyl)phényl)benzamide ;
le N-(2,3-dichlorophényl)-2-méthyl-3-(2-(méthylamino)pyrido[2,3-d]pyrimidin-6-yl)benzamide ;
le 2-méthyl-3-(2-(méthylamino)pyrido[2,3-d]pyrimidin-6-yl)-N-(4-(méthyloxy)-3-(trifluorométhyl)phényl)benzamide ;
le 2-méthyl-3-(2-(méthylamino)pyrido[2,3-d]pyrimidin-6-yl)-N-(3-((trifluorométhyl)oxy)phényl)benzamide ;
le N-(4-méthyl-3-(2-(méthylamino)pyrido[2,3-d]pyrimidin-6-yl)phényl)-4-(trifluorométhyl)benzamide ;
le 4-méthyl-3-(2-(méthylamino)pyrido[2,3-d]pyrimidin-6-yl)-N-(4-(méthyloxy)-3-(trifluorométhyl)phényl)benzamide ;
le 4-méthyl-3-(2-(méthylamino)pyrido[2,3-d]pyrimidin-6-yl)-N-(2-pyridinylméthyl)benzamide ;
le N-(2-méthyl-3-(2-(méthylamino)pyrido[2,3-d]pyrimidin-6-yl)phényl)-3-(trifluorométhyl)benzamide ;
le 2,2-difluoro-N-(4-méthyl-3-(2-(méthylamino)pyrido[2,3-d]pyrimidin-6-yl)phényl)-1,3-benzodioxole-4-carboxamide ;
le N-(5-(1,1-diméthyléthyl)-2-(méthyloxy)phényl)-4-méthyl-3-(2-(méthylamino)pyrido[2,3-d]pyrimidin-6-yl)benzamide ;
le 3-(2-amino-6-quinazoléinyl)-4-méthyl-N-(4-((((2-(4-morpholinyl)éthyl)amino)carbonyl)amino)-3-(trifluorométhyl)phényl)benzamide ;
le 3-(2-amino-6-quinazoléinyl)-N-(4-((((2-(diéthylamino)éthyl)amino)carbonyl)amino)-3-(trifluorométhyl)phényl)-4-méthylbenzamide ;
le 4-méthyl-3-(2-(méthylamino)pyrido[2,3-d]pyrimidin-6-yl)-N-(4-((((2-(4-morpholinyl)éthyl)amino)carbonyl)amino)-3-(trifluorométhyl)phényl)benzamide ;
le N-(4-((((2-(diéthylamino)éthyl)amino)carbonyl)amino)-3-(trifluorométhyl)phényl)-4-méthyl-3-(2-(méthylamino)pyrido[2,3-d]pyrimidin-6-yl)benzamide ;
le 3-(2-amino-6-quinazoléinyl)-4-méthyl-N-(3-(((2-(4-morpholinyl)éthyl)amino)carbonyl)-5-(trifluorométhyl)phényl)benzamide ;
le 4-méthyl-3-(2-(méthylamino)pyrido[2,3-d]pyrimidin-6-yl)-N-(3-(((2-(4-morpholinyl)éthyl)amino)carbonyl)-5-(trifluorométhyl)phényl)benzamide ;
le 3-(2-amino-6-quinazoléinyl)-N-(3-(((2-(diéthylamino)éthyl)amino)carbonyl)-5-(trifluorométhyl)phényl)-4-méthylbenzamide ;
le N-(3-(((2-(diéthylamino)éthyl)amino)carbonyl)-5-(trifluorométhyl)phényl)-4-méthyl-3-(2-(méthylamino)pyrido[2,3-d]pyrimidin-6-yl)benzamide ;
le N-(5-((diéthylamino)sulfonyl)-2-(méthyloxy)phényl)-4-méthyl-3-(2-(méthylamino)pyrido[2,3-d]pyrimidin-6-yl)benzamide ;
le N-(3-(2-amino-6-quinazoléinyl)-4-méthylphényl)-3,4,5-tris(méthyloxy)benzamide ;
le N-(4-méthyl-3-(2-(méthylamino)pyrido[2,3-d]pyrimidin-6-yl)phényl)-3,4,5-tris(méthyloxy)benzamide ;
le 3-fluoro-N-(4-méthyl-3-(2-(méthylamino)pyrido[2,3-d]pyrimidin-6-yl)phényl)-5-(trifluorométhyl)benzamide ;
le 3-(2-amino-6-quinazoléinyl)-4-méthyl-N-(3-((4-méthyl-1-pipérazinyl)carbonyl)-5-(trifluorométhyl)phényl)benzamide ;
le 3-(2-amino-6-quinazoléinyl)-4-méthyl-N-(3-((4-méthyl-1-pipérazinyl)méthyl)-5-(trifluorométhyl)phényl)benzamide ;
le N-(3-(2-amino-6-quinazoléinyl)-2-méthylphényl)-3-(trifluorométhyl)benzamide ;
le N-(5-(1,1-diméthyléthyl)-2-((4-morpholinylacétyl)amino)phényl)-4-méthyl-3-(2-(méthylamino)pyrido[2,3-d]pyrimidin-6-yl)benzamide ;
le 3-(2-amino-6-quinazoléinyl)-N-(5-(1,1-diméthyléthyl)-2-((4-morpholinylacétyl)amino)phényl)-4-méthylbenzamide ;
le N-(3-(2-amino-6-quinazoléinyl)-2-méthylphényl)-3-fluoro-5-(trifluorométhyl)benzamide ;
le 3-fluoro-N-(2-méthyl-3-(2-(méthylamino)pyrido[2,3-d]pyrimidin-6-yl)phényl)-5-(trifluorométhyl)benzamide ;
le N-(3-(2-amino-6-quinazoléinyl)-2-méthylphényl)-3,5-bis(trifluorométhyl)benzamide ;
le N-(2-méthyl-3-(2-(méthylamino)pyrido[2,3-d]pyrimidin-6-yl)phényl)-3,5-bis(trifluorométhyl)benzamide ;
le N-(3-(2-amino-6-quinazoléinyl)-2-méthylphényl)-2-fluoro-5-(trifluorométhyl)benzamide ;
le 2-fluoro-N-(2-méthyl-3-(2-(méthylamino)pyrido[2,3-d]pyrimidin-6-yl)phényl)-5-(trifluorométhyl)benzamide ;
le N-(3-(2-amino-6-quinazoléinyl)-2,4,6-triméthylphényl)-3-(trifluorométhyl)benzamide ;
la 4-((3-(2-amino-6-quinazoléinyl)-4-méthylphényl)carbonyl)-6-(1,1-diméthyléthyl)-3,4-dihydro-2(1H)-quinoxalinone ;
la N-(4-(2-amino-6-quinazoléinyl)phényl)-N'-phénylurée ;
le N-(4-(4-amino-6-quinazoléinyl)phényl)-3-(trifluorométhyl)benzamide ;
le N-(4-(2-((4-(4-méthyl-1-pipérazinyl)phényl)amino)-6-quinazoléinyl)phényl)-3-(trifluorométhyl)benzamide ;
le 4-(2-((4-(4-méthyl-1-pipérazinyl)phényl)amino)-6-quinazoléinyl)-N-(3-(trifluorométhyl)phényl)benzamide ;
le N-(4-(2-((3-(4-morpholinyl)propyl)amino)-6-quinazoléinyl)phényl)-3-(trifluorométhyl)benzamide ;
le 4-(2-((3-(4-morpholinyl)propyl)amino)-6-quinazoléinyl)-N-(3-(trifluorométhyl)phényl)benzamide ;
le N-(4-(2-((3-(diéthylamino)propyl)amino)-6-quinazoléinyl)phényl)-3-(trifluorométhyl)benzamide ;
le 4-(2-((3-(diéthylamino)propyl)amino)-6-quinazoléinyl)-N-(3-(trifluorométhyl)phényl)benzamide ;
le N-cyclopropyl-4-(2-((4-(4-méthyl-1-pipérazinyl)phényl)amino)-6-quinazoléinyl)benzamide ;
le 4-(2-((4-(4-méthyl-1-pipérazinyl)phényl)amino)-6-quinazoléinyl)-N-phénylbenzamide ;
le N-(4-chlorophényl)-4-(2-((4-(4-méthyl-1-pipérazinyl)phényl)amino)-6-quinazoléinyl)benzamide ;
le N-(4-(2-(méthylamino)-6-quinazoléinyl)phényl)-2-(phénylamino)benzamide ;
le N-(4-(4-amino-6-quinazoléinyl)phényl)-2-(phénylamino)benzamide ;
la 6-méthyl-5-(2-(méthylamino)-6-quinazoléinyl)-1(2H)-isoquinoléinone ;
la 2',7'-diméthyl-2-(méthylamino)-6,8'-biquinazoléin-4'(3'H)-one ;
le 2-fluoro-4-méthyl-3-(2-(méthylamino)-6-quinazoléinyl)-N-(3-((1-méthyléthyl)oxy)phényl)benzamide ;
le 3-fluoro-4-méthyl-2-(2-(méthylamino)-6-quinazoléinyl)-N-(3-((1-méthyléthyl)oxy)phényl)benzamide ;
le 2-fluoro-4-méthyl-3-(2-(méthylamino)-6-quinazoléinyl)-N-(3-((1-méthyléthyl)oxy)phényl)benzènecarbothioamide ;
le 2-fluoro-4-méthyl-3-(2-(méthylamino)-6-quinazoléinyl)-N-(4-(méthyloxy)-3-(trifluorométhyl)phényl)benzamide ;
l'acide 2-amino-4-méthyl-3-(2-(méthylamino)-6-quinazoléinyl)benzoïque ;
le 2-amino-4-méthyl-3-(2-(méthylamino)-6-quinazoléinyl)-N-(3-((1-méthyléthyl)oxy)phényl)benzamide ;
le 2-amino-4-méthyl-3-(2-(méthylamino)-6-quinazoléinyl)-N-(3-(1-méthyléthyl)phényl)benzamide ;
le 2-amino-N-(4-(1,1-diméthyléthyl)phényl)-4-méthyl-3-(2-(méthylamino)-6-quinazoléinyl)benzamide ;
le chlorhydrate de 4-méthyl-3-(quinazoléin-6-yl)-N-(3-(trifluorométhyl)phényl)benzamide ;
le 3-(2-amino-6-quinazoléinyl)-N-(3-((3-(diméthylamino)propyl)oxy)-5-(trifluorométhyl)phényl)-4-méthylbenzamide ;
la 6-(5-isothiocyanato-2-méthylphényl)-N-méthyl-2-quinazoléinamine ;
la 6-(2-phénylimidazo[1,2-a]pyridin-6-yl)-2-quinazoléinamine ;
la 6-(2-(4-(méthyloxy)phényl)imidazo[1,2-a]pyridin-6-yl)-2-quinazoléinamine ;
la 6-(2-(3-(méthyloxy)phényl)imidazo[1,2-a]pyridin-6-yl)-2-quinazoléinamine ;
la 6-(2-(2-(méthyloxy)phényl)imidazo[1,2-a]pyridin-6-yl)-2-quinazoléinamine ;
la 6-(2-(1,3-benzodioxol-5-yl)imidazo[1,2-a]pyridin-6-yl)-2-quinazoléinamine ;
la 6-(2-(3-thiényl)imidazo[1,2-a]pyridin-6-yl)-2-quinazoléinamine ;
la 6-(2-éthyl-1-benzofuran-5-yl)-2-quinazoléinamine ;
la 6-(2-(1,1-diméthyléthyl)imidazo[1,2-a]pyridin-6-yl)-2-quinazoléinamine ;
le 3-(2-amino-6-quinazoléinyl)-N-(3-(3-(diméthylamino)propyl)-5-(trifluorométhyl)phényl)-4-méthylbenzamide ;
la N-(4-(diéthylnitroryl)-1-méthylbutyl)-6-(4-méthyl-3-pyridinyl)-2-quinazoléinamine ;
le 4-méthyl-3-(6-quinazoléinyl)benzoate de méthyle ;
l'acide 4-méthyl-3-(6-quinazoléinyl)benzoïque ;
le N-cyclopropyl-4-méthyl-3-(2-((((2R)-1-(2,2,2-trifluoroéthyl)-2-pyrrolidinyl)méthyl)amino)-6-quinazoléinyl)benzamide ;
le 3-(2-amino-6-quinazoléinyl)-N-(3-((N,N-diéthylglycyl)amino)-4-(1,1-diméthyléthyl)phényl)-4-méthylbenzamide ;
le 3-(2-amino-6-quinazoléinyl)-N-(4-(1,1-diméthyléthyl)-3-((1-pyrrolidinylacétyl)amino)phényl)-4-méthylbenzamide ;
le N-(1-(N,N-diéthylglycyl)-3,3-diméthyl-2,3-dihydro-1H-indol-6-yl)-4-méthyl-3-(2-(méthylamino)-6-quinazoléinyl)benzamide ;
le 3-(2-(cyclopropylamino)-6-quinazoléinyl)-N-(1-(N,N-diéthylglycyl)-3,3-diméthyl-2,3-dihydro-1H-indol-6-yl)-4-méthylbenzamide ;
le N-(3-((N,N-diéthylglycyl)amino)-4-(1-pipéridinyl)phényl)-4-méthyl-3-(2-(méthylamino)-6-quinazoléinyl)benzamide ;
le N-(4-chloro-3-(((1-méthyl-4-pipéridinyl)méthyl)oxy)phényl)-4-méthyl-3-(2-(méthylamino)-6-quinazoléinyl)benzamide ;
le N-(3-fluoro-4-((3-(1-pipéridinyl)propyl)oxy)phényl)-4-méthyl-3-(2-(méthylamino)-6-quinazoléinyl)benzamide ;
le N-(3-((N,N-diéthylglycyl)amino)-4-méthylphényl)-4-méthyl-3-(2-(méthylamino)-6-quinazoléinyl)benzamide ;
le N-(3-((N,N-diéthylglycyl)amino)-4-fluorophényl)-4-méthyl-3-(2-(méthylamino)-6-quinazoléinyl)benzamide ;
le N-(2-((diéthylamino)méthyl)-4,5-bis(méthyloxy)phényl)-4-méthyl-3-(2-(méthylamino)-6-quinazoléinyl)benzamide ;
le N-(2-((diéthylamino)méthyl)-4,5-bis(méthyloxy)phényl)-4-méthyl-3-(2-((3-(4-morpholinyl)propyl)amino)-6-quinazoléinyl)benzamide ;
le N-(3-fluoro-4-(((1-méthyl-3-pipéridinyl)méthyl)oxy)phényl)-4-méthyl-3-(2-(méthylamino)-6-quinazoléinyl)benzamide ;
le 4-méthyl-3-(2-(méthylamino)-6-quinazoléinyl)-N-(3-(((méthylsulfonyl)amino)méthyl)phényl)benzamide ;
le 4-méthyl-N-(3-(((méthylsulfonyl)amino)méthyl)phényl)-3-(2-((3-(4-morpholinyl)propyl)amino)-6-quinazoléinyl)benzamide ;
le N-(4-fluoro-3-(((méthyloxy)acétyl)amino)phényl)-4-méthyl-3-(2-(méthylamino)-6-quinazoléinyl)benzamide ;
le 3-(2-((3-(diéthylamino)propyl)amino)-6-quinazoléinyl)-N-(4-fluoro-3-(((méthyloxy)acétyl)amino)phényl)-4-méthylbenzamide ;
le 4-méthyl-3-(2-(méthylamino)-6-quinazoléinyl)-N-(2-((4-méthyl-1-pipérazinyl)méthyl)-5-(trifluorométhyl)phényl)benzamide ;
le 4-méthyl-3-(2-(méthylamino)-6-quinazoléinyl)-N-(3-((((2S)-1-méthyl-2-pyrrolidinyl)méthyl)oxy)-4-(pentafluoroéthyl)phényl)benzamide ;
le N-(4-chloro-3-((((2S)-1-méthyl-2-pyrrolidinyl)méthyl)oxy)phényl)-4-méthyl-3-(2-(méthylamino)-6-quinazoléinyl)benzamide ;
le 3,3-diméthyl-6-(((4-méthyl-3-(2-(méthylamino)-6-quinazoléinyl)phényl)carbonyl)amino)-2,3-dihydro-1H-indole-1-carboxylate d'éthyle ;
le N-(3-((diéthylamino)méthyl)-4-(méthyloxy)phényl)-4-méthyl-3-(2-(méthylamino)-6-quinazoléinyl)benzamide ;
le 6-(((3-(2-((3-(diéthylamino)propyl)amino)-6-quinazoléinyl)-4-méthylphényl)carbonyl)amino)-3,3-diméthyl-2,3-dihydro-1H-indole-1-carboxylate d'éthyle ;
le 4-méthyl-3-(2-(méthylamino)-6-quinazoléinyl)-N-(3-(2-oxo-1-pyrrolidinyl)-5-(trifluorométhyl)phényl)benzamide ;
le 4-méthyl-3-(2-(méthylamino)-6-quinazoléinyl)-N-(2-(1-pipéridinyl)-5-(trifluorométhyl)phényl)benzamide ;
le 4-méthyl-3-(2-(méthylamino)-6-quinazoléinyl)-N-(1-méthyl-4-pipéridinyl)benzamide ;
le N-(2-((2,2-diméthylpropanoyl)amino)-5-(trifluorométhyl)phényl)-4-méthyl-3-(2-(méthylamino)-6-quinazoléinyl)benzamide ;
le 4-méthyl-3-(2-(méthylamino)-6-quinazoléinyl)-N-(2-(méthyl(1-méthyl-4-pipéridinyl)amino)-5-(trifluorométhyl)phényl)benzamide ;
le 4-méthyl-3-(2-(méthylamino)-6-quinazoléinyl)-N-(3-(2-méthyl-1-pyrrolidinyl)-2-pyridinyl)benzamide ;
le N-(2,2-difluoro-1,3-benzodioxol-4-yl)-4-méthyl-3-(2-((3-(4-morpholinyl)propyl)amino)-6-quinazoléinyl)benzamide ;
le N-(2,2-difluoro-1,3-benzodioxol-4-yl)-4-méthyl-3-(2-((1-méthyl-4-pipéridinyl)amino)-6-quinazoléinyl)benzamide ;
le N-(2,2-difluoro-1,3-benzodioxol-4-yl)-4-méthyl-3-(2-((2-(1-pipéridinyl)éthyl)amino)-6-quinazoléinyl)benzamide ;
le N-(2-((3-(diméthylamino)propyl)oxy)-5-(trifluorométhyl)phényl)-4-méthyl-3-(2-(méthylamino)-6-quinazoléinyl)benzamide ;
le 4-méthyl-3-(2-((3-(méthyloxy)propyl)amino)-6-quinazoléinyl)-N-(2-méthyl-3-(trifluorométhyl)phényl)benzamide ;
le 4-méthyl-N-(2-méthyl-3-(trifluorométhyl)phényl)-3-(2-(phénylamino)-6-quinazoléinyl)benzamide ;
le N-(1-acétyl-3,3-diméthyl-2,3-dihydro-1H-indol-6-yl)-4-méthyl-3-(2-((2-(4-morpholinyl)éthyl)amino)-6-quinazoléinyl)benzamide ;
le N-(1-acétyl-3,3-diméthyl-2,3-dihydro-1H-indol-6-yl)-4-méthyl-3-(2-((2-(1-pyrrolidinyl)éthyl)amino)-6-quinazoléinyl)benzamide ;
le N-(1-acétyl-3,3-diméthyl-2,3-dihydro-1H-indol-6-yl)-3-(2-(((1-éthyl-4-pipéridinyl)méthyl)amino)-6-quinazoléinyl)-4-méthylbenzamide ;
le N-(1-acétyl-3,3-diméthyl-2,3-dihydro-1H-indol-6-yl)-4-méthyl-3-(2-((1-méthyl-4-pipéridinyl)amino)-6-quinazoléinyl)benzamide ;
le N-(1-acétyl-3,3-diméthyl-2,3-dihydro-1H-indol-6-yl)-3-(2-(cyclopropylamino)-6-quinazoléinyl)-4-méthylbenzamide ;
le N-(1-acétyl-3,3-diméthyl-2,3-dihydro-1H-indol-6-yl)-4-méthyl-3-(2-((3-(2-oxo-1-pyrrolidinyl)propyl)amino)-6-quinazoléinyl)benzamide ;
le N-(1-acétyl-3,3-diméthyl-2,3-dihydro-1H-indol-6-yl)-4-méthyl-3-(2-(méthylamino)-6-quinazoléinyl)benzamide ;
le N-(1-acétyl-3,3-diméthyl-2,3-dihydro-1H-indol-6-yl)-4-méthyl-3-(2-((3-(4-morpholinyl)propyl)amino)-6-quinazoléinyl)benzamide ;
le 4-méthyl-3-(2-(méthyl(3-(4-morpholinyl)propyl)amino)-6-quinazoléinyl)-N-(2-méthyl-3-(trifluorométhyl)phényl)benzamide ;
le 2-fluoro-N-(2-méthyl-3-(2-(méthylamino)-6-quinazoléinyl)phényl)-5-(trifluorométhyl)benzamide ;
le 4-méthyl-3-(2-((1E)-3-(méthyloxy)-1-propényl)-6-quinazoléinyl)-N-(3-(trifluorométhyl)phényl)benzamide ;
le 4-méthyl-3-(2-((1E)-3-(méthyloxy)-1-propényl)-6-quinazoléinyl)-N-(2-méthyl-3-(trifluorométhyl)phényl)benzamide ;
la 6-(2,6-dichlorophényl)-N-(3-(4-morpholinyl)propyl)-2-quinazoléinamine ;
le 4-méthyl-3-(2-(méthylamino)-6-quinazoléinyl)benzamide ;
le 3-(2-(cyclopropylamino)-6-quinazoléinyl)-4-méthylbenzamide ;
le 4-méthyl-3-(2-((3-(4-morpholinyl)propyl)amino)-6-quinazoléinyl)benzamide ;
le 3-(2-((3-(diéthylamino)propyl)amino)-6-quinazoléinyl)-4-méthylbenzamide ;
le 3-(2-((3-fluoro-4-((4-(1H-imidazol-1-yl)butyl)oxy)phényl)amino)-6-quinazoléinyl)-4-méthylbenzamide ;
le 4-méthyl-3-(2-((4-((3-(1-pipéridinyl)propyl)oxy)phényl)amino)-6-quinazoléinyl)benzamide ;
la 6-(2,6-diméthylphényl)-N-(3-morpholinopropyl)quinazoléin-2-amine ;
le N-(2-fluoro-5-(trifluorométhyl)phényl)-4-méthyl-3-(2-((2-(4-morpholinyl)éthyl)amino)-6-quinazoléinyl)benzamide ;
le N-(2-fluoro-5-(trifluorométhyl)phényl)-4-méthyl-3-(2-((3-(4-morpholinyl)propyl)amino)-6-quinazoléinyl)benzamide ;
le N-(2-fluoro-5-(trifluorométhyl)phényl)-4-méthyl-3-(2-((2-(1-pipéridinyl)éthyl)amino)-6-quinazoléinyl)benzamide ;
le N-(2-fluoro-5-(trifluorométhyl)phényl)-4-méthyl-3-(2-((2-(1-pyrrolidinyl)éthyl)amino)-6-quinazoléinyl)benzamide ;
le 3-(2-((3-(diéthylamino)propyl)amino)-6-quinazoléinyl)-4-méthyl-N-(2-méthyl-3-(trifluorométhyl)phényl)benzamide ;
le 4-méthyl-N-(2-méthyl-3-(trifluorométhyl)phényl)-3-(2-((2-(1-(phénylméthyl)-4-pipéridinyl)éthyl)amino)-6-quinazoléinyl)benzamide ;
le 4-(2-((6-(2-méthyl-5-(((2-méthyl-3-(trifluorométhyl)phényl)amino)carbonyl)phényl)-2-quinazoléinyl)amino)éthyl)-1-pipérazinecarboxylate de 1,1-diméthyléthyle ;
le 3-(2-((((2R)-1-éthyl-2-pyrrolidinyl)méthyl)amino)-6-quinazoléinyl)-4-méthyl-N-(2-méthyl-3-(trifluorométhyl)phényl)benzamide ;
le 4-méthyl-N-(2-méthyl-3-(trifluorométhyl)phényl)-3-(2-((2-(1-pipéridinyl)éthyl)amino)-6-quinazoléinyl)benzamide ;
le 3-(2-(1,3-benzoxazol-2-ylamino)-6-quinazoléinyl)-4-méthyl-N-(2-méthyl-3-(trifluorométhyl)phényl)benzamide ;
le 4-méthyl-N-(2-méthyl-3-(trifluorométhyl)phényl)-3-(2-((2-(1-pipérazinyl)éthyl)amino)-6-quinazoléinyl)benzamide ;
le 4-méthyl-3-(2-((2-(1-pyrrolidinyl)éthyl)amino)-6-quinazoléinyl)-N-(3-(trifluorométhyl)phényl)benzamide ;
le N-(4-chloro-3-(trifluorométhyl)phényl)-4-méthyl-3-(2-((2-(1-pyrrolidinyl)éthyl)amino)-6-quinazoléinyl)benzamide ;
le 4-méthyl-N-(3-méthyl-4-(1-méthyléthyl)phényl)-3-(2-((2-(1-pyrrolidinyl)éthyl)amino)-6-quinazoléinyl)benzamide ;
le N-(4-fluoro-3-(trifluorométhyl)phényl)-4-méthyl-3-(2-((2-(1-pyrrolidinyl)éthyl)amino)-6-quinazoléinyl)benzamide ;
le N-(4-chloro-3-(trifluorométhyl)phényl)-4-méthyl-3-(2-((1-méthyl-4-pipéridinyl)amino)-6-quinazoléinyl)benzamide ;
le N-(4-fluoro-3-(trifluorométhyl)phényl)-4-méthyl-3-(2-((1-méthyl-4-pipéridinyl)amino)-6-quinazoléinyl)benzamide ;
le 4-méthyl-N-(3-méthyl-4-(1-méthyléthyl)phényl)-3-(2-((1-méthyl-4-pipéridinyl)amino)-6-quinazoléinyl)benzamide ;
le N-(4-chloro-3-(trifluorométhyl)phényl)-4-méthyl-3-(2-((2-(1-pipéridinyl)éthyl)amino)-6-quinazoléinyl)benzamide ;
le N-(4-fluoro-3-(trifluorométhyl)phényl)-4-méthyl-3-(2-((2-(1-pipéridinyl)éthyl)amino)-6-quinazoléinyl)benzamide ;
le 4-méthyl-N-(3-méthyl-4-(1-méthyléthyl)phényl)-3-(2-((2-(1-pipéridinyl)éthyl)amino)-6-quinazoléinyl)benzamide ;
le N-(1-acétyl-3,3-diméthyl-2,3-dihydro-1H-indol-6-yl)-4-méthyl-3-(2-((2-(1-pipéridinyl)éthyl)amino)-6-quinazoléinyl)benzamide ;
le 4-méthyl-N-(2-méthyl-3-(trifluorométhyl)phényl)-3-(2-((4-((3-(1-pipéridinyl)propyl)oxy)phényl)amino)-6-quinazoléinyl)benzamide ;
le 4-méthyl-3-(2-((3-(4-méthyl-1-pipérazinyl)phényl)amino)-6-quinazoléinyl)-N-(2-méthyl-3-(trifluorométhyl)phényl)benzamide ;
le N-(2-fluoro-3-(trifluorométhyl)phényl)-4-méthyl-3-(2-((2-(1-pipéridinyl)éthyl)amino)-6-quinazoléinyl)benzamide ;
le N-(2,3-dihydro-1H-indén-4-yl)-4-méthyl-3-(2-((3-(4-morpholinyl)propyl)amino)-6-quinazoléinyl)benzamide ;
le 3-(2-((3-(diéthylamino)propyl)amino)-6-quinazoléinyl)-N-(2,3-dihydro-1H-indén-4-yl)-4-méthylbenzamide ;
le N-(2,3-dihydro-1H-indén-4-yl)-4-méthyl-3-(2-(méthylamino)-6-quinazoléinyl)benzamide ;
le N-(2,3-dihydro-1H-indén-4-yl)-4-méthyl-3-(2-((4-((3-(1-pipéridinyl)propyl)oxy)phényl)amino)-6-quinazoléinyl)benzamide ;
le 3-(2-(((1S)-4-(diéthylamino)-1-méthylbutyl)amino)-6-quinazoléinyl)-4-méthyl-N-(2-méthyl-3-(trifluorométhyl)phényl)benzamide ;
la 6-(5-amino-2-méthylphényl)-N-(4-(4-méthyl-1-pipérazinyl)phényl)-2-quinazoléinamine ;
le 2-méthyl-N-(4-méthyl-3-(2-((4-(4-méthyl-1-pipérazinyl)phényl)amino)-6-quinazoléinyl)phényl)-3-(trifluorométhyl)benzamide ;
le 4-(1,1-diméthyléthyl)-N-(4-méthyl-3-(2-((4-(4-méthyl-1-pipérazinyl)phényl)amino)-6-quinazoléinyl)phényl)benzamide ;
le N-(4-méthyl-3-(2-((4-(4-méthyl-1-pipérazinyl)phényl)amino)-6-quinazoléinyl)phényl)acétamide ;
le N-(4-méthyl-3-(2-((4-(4-méthyl-1-pipérazinyl)phényl)amino)-6-quinazoléinyl)phényl)-3,5-bis(méthyloxy)benzamide ;
le 2-fluoro-N-(4-méthyl-3-(2-((4-(4-méthyl-1-pipérazinyl)phényl)amino)-6-quinazoléinyl)phényl)-3-(trifluorométhyl)benzamide ;
le 2,2-difluoro-N-(4-méthyl-3-(2-((4-(4-méthyl-1-pipérazinyl)phényl)amino)-6-quinazoléinyl)phényl)-1,3-benzodioxole-5-carboxamide ;
le N-(4-méthyl-3-(2-((4-(4-méthyl-1-pipérazinyl)phényl)amino)-6-quinazoléinyl)phényl)-1-naphtalènecarboxamide ;
le 3-(1,1-diméthyléthyl)-1-méthyl-N-(4-méthyl-3-(2-((4-(4-méthyl-1-pipérazinyl)phényl)amino)-6-quinazoléinyl)phényl)-1H-pyrazole-4-carboxamide ;
la 6-(5-(1H-benzimidazol-2-ylamino)-2-méthylphényl)-N-(4-(4-méthyl-1-pipérazinyl)phényl)-2-quinazoléinamine ;
la N-(4-(4-méthyl-1-pipérazinyl)phényl)-6-(2-méthyl-5-((5-(trifluorométhyl)-1H-benzimidazol-2-yl)amino)phényl)-2-quinazoléinamine ;
le N-(2-(2-(diméthylamino)éthyl)-5-(trifluorométhyl)phényl)-4-méthyl-3-(2-(méthylamino)-6-quinazoléinyl)benzamide ;
la N-éthyl-N'-(4-méthyl-3-(2-((4-(4-méthyl-1-pipérazinyl)phényl)amino)-6-quinazoléinyl)phényl)urée ;
le 3-(2-((3-hydroxypropyl)amino)-6-quinazoléinyl)-4-méthyl-N-(2-méthyl-3-(trifluorométhyl)phényl)benzamide ;
le 4-méthyl-N-(2-méthyl-3-(trifluorométhyl)phényl)-3-(2-(3-pyridinylamino)-6-quinazoléinyl)benzamide ;
la N-méthyl-6-(2-méthyl-5-((5-(trifluorométhyl)-1H-benzimidazol-2-yl)amino)phényl)-2-quinazoléinamine ;
la 6-(5-(1H-benzimidazol-2-ylamino)-2-méthylphényl)-N-méthyl-2-quinazoléinamine ;
la 6-(5-((1-(2-(diméthylamino)éthyl)-1H-benzimidazol-2-yl)amino)-2-méthylphényl)-N-méthyl-2-quinazoléinamine ;
la 6-(5-((1-(3-(diméthylamino)propyl)-1H-benzimidazol-2-yl)amino)-2-méthylphényl)-N-méthyl-2-quinazoléinamine ;
la 6-(5-((7-chloro-5-(trifluorométhyl)-1H-benzimidazol-2-yl)amino)-2-méthylphényl)-N-méthyl-2-quinazoléinamine ;
la 6-(5-(1H-imidazo[4,5-b]pyridin-2-ylamino)-2-méthylphényl)-N-méthyl-2-quinazoléinamine ;
la N-méthyl-6-(2-méthyl-5-((1-méthyl-5-(trifluorométhyl)-1H-benzimidazol-2-yl)amino)phényl)-2-quinazoléinamine ;
la 6-(5-((5,6-difluoro-1H-benzimidazol-2-yl)amino)-2-méthylphényl)-N-méthyl-2-quinazoléinamine ;
la N-méthyl-6-(2-méthyl-5-((6-(4-méthyl-1-pipérazinyl)-1H-benzimidazol-2-yl)amino)phényl)-2-quinazoléinamine ;
la N-méthyl-6-(6-méthyl-5-phtalazinyl)-2-quinazoléinamine ;
le 6-méthyl-5-(2-(méthylamino)-6-quinazoléinyl)-1-phtalazinol ;
le N-(4-chloro-3-(2-(méthylamino)-6-quinazoléinyl)phényl)-3-(trifluorométhyl)benzamide ;
le N-(4-méthyl-3-(2-(méthylamino)-6-quinazoléinyl)phényl)-3-(trifluorométhyl)benzamide ;
le N-(4-chloro-3-(2-(méthylamino)-6-quinazoléinyl)phényl)-2-fluoro-5-(trifluorométhyl)benzamide ;
le N-(4-chloro-3-(2-(méthylamino)-6-quinazoléinyl)phényl)-2-méthyl-3-(trifluorométhyl)benzamide ;
le 3-(2-amino-6-quinazoléinyl)-N-(2,6-diméthylphényl)-4-méthylbenzamide ;
le 3-(2-amino-6-quinazoléinyl)-N-(2-(éthyloxy)-5-(trifluorométhyl)phényl)-4-méthylbenzamide ;
le 3-(2-amino-6-quinazoléinyl)-4-méthyl-N-(2-(méthyloxy)-5-(trifluorométhyl)phényl)benzamide ;
le 3-(2-amino-6-quinazoléinyl)-4-méthyl-N-(2-méthyl-5-nitrophényl)benzamide ;
le 3-(2-amino-6-quinazoléinyl)-N-(2,5-diméthylphényl)-4-méthylbenzamide ;
le N-(5-(aminocarbonyl)-2-méthylphényl)-3-(2-amino-6-quinazoléinyl)-4-méthylbenzamide ;
le 3-(2-amino-6-quinazoléinyl)-4-méthyl-N-(2-(méthyloxy)-5-nitrophényl)benzamide ;
le 3-(2-amino-6-quinazoléinyl)-N-(5-cyclohexyl-2-(méthyloxy)phényl)-4-méthylbenzamide ;
le 3-(2-amino-6-quinazoléinyl)-4-méthyl-N-(2-méthyl-5-(méthyloxy)phényl)benzamide ;
le 4-(2-amino-6-quinazoléinyl)-N-(2-fluoro-5-(trifluorométhyl)phényl)-3-méthylbenzamide ;
le 3-(2-amino-6-quinazoléinyl)-4-méthyl-N-(2-(méthyloxy)-5-((2-pyridinylamino)carbonyl)phényl)benzamide ;
le 3-(2-amino-6-quinazoléinyl)-4-méthyl-N-(2-(méthyloxy)-5-((3-pyridinylamino)carbonyl)phényl)benzamide ;
le 3-(2-amino-6-quinazoléinyl)-4-méthyl-N-(2-(méthyloxy)-5-((4-pyridinylamino)carbonyl)phényl)benzamide ;
le 4-(2-amino-6-quinazoléinyl)-3-méthyl-N-(3-((4-méthyl-1-pipérazinyl)méthyl)-5-(trifluorométhyl)phényl)benzamide ;
le 4-méthyl-3-(2-(méthylamino)-6-quinazoléinyl)-N-(2-((2-(1-pyrrolidinyl)éthyl)oxy)-5-(trifluorométhyl)phényl)benzamide ;
le N-(4-méthyl-3-(2-(méthylamino)-6-quinazoléinyl)phényl)-2-((2-(1-pyrrolidinyl)éthyl)amino)-5-(trifluorométhyl)benzamide ;
le 3-(2-amino-6-quinazoléinyl)-N-(2-chloro-4-(1,1-diméthyléthyl)phényl)-4-méthylbenzamide ;
le 3-(2-amino-6-quinazoléinyl)-N-(2-chloro-5-(trifluorométhyl)phényl)-4-méthylbenzamide ;
le 3-(2-amino-6-quinazoléinyl)-N-(2-bromo-5-(trifluorométhyl)phényl)-4-méthylbenzamide ;
le 3-(2-amino-6-quinazoléinyl)-4-méthyl-N-(4-(1-méthyléthyl)-2-(trifluorométhyl)phényl)benzamide ;
le 3-(2-amino-6-quinazoléinyl)-N-(2-(1,1-diméthyléthyl)imidazo[1,2-a]pyridin-6-yl)-4-méthylbenzamide ;
le 4-méthyl-3-(2-(méthylamino)-6-quinazoléinyl)-N-(4-((1-méthyl-4-pipéridinyl)oxy)phényl)benzamide ;
le 3-(2-(cyclopropylamino)-6-quinazoléinyl)-4-méthyl-N-(4-((1-méthyl-4-pipéridinyl)oxy)phényl)benzamide ;
le 3-(2-amino-6-quinazoléinyl)-4-méthyl-N-(3-méthyl-4-((1-méthyl-4-pipéridinyl)oxy)phényl)benzamide ;
le 4-méthyl-3-(2-(méthylamino)-6-quinazoléinyl)-N-(3-méthyl-4-((1-méthyl-4-pipéridinyl)oxy)phényl)benzamide ;
le 3-(2-(cyclopropylamino)-6-quinazoléinyl)-4-méthyl-N-(3-méthyl-4-((1-méthyl-4-pipéridinyl)oxy)phényl)benzamide ;
le 3-(2-amino-6-quinazoléinyl)-N-(4-((1-cyclopropyléthyl)oxy)phényl)-4-méthylbenzamide ;
le N-(4-((1-cyclopropyléthyl)oxy)phényl)-4-méthyl-3-(2-(méthylamino)-6-quinazoléinyl)benzamide ;
le N-(4-((1-cyclopropyléthyl)oxy)-3-méthylphényl)-4-méthyl-3-(2-(méthylamino)-6-quinazoléinyl)benzamide ;
le 3-(2-amino-6-quinazoléinyl)-N-(4-((1-cyclopropyléthyl)oxy)-3-méthylphényl)-4-méthylbenzamide ;
le 4-méthyl-3-(2-(méthylamino)-6-quinazoléinyl)-N-(4-(1-méthyléthyl)phényl)benzamide ;
le 3-(2-(cyclopropylamino)-6-quinazoléinyl)-4-méthyl-N-(4-(1-méthyléthyl)phényl)benzamide ;
le 4-méthyl-N-(4-(1-méthyléthyl)phényl)-3-(2-((1-méthyl-4-pipéridinyl)amino)-6-quinazoléinyl)benzamide ;
le 4-méthyl-3-(2-(méthylamino)-6-quinazoléinyl)-N-(2-((1-méthyl-4-pipéridinyl)oxy)-5-(trifluorométhyl)phényl)benzamide ;
le N-(4-((cyclopropylméthyl)oxy)phényl)-4-méthyl-3-(2-(méthylamino)-6-quinazoléinyl)benzamide ;
le N-(4-((cyclopropylméthyl)oxy)phényl)-4-méthyl-3-(2-((3-(4-morpholinyl)propyl)amino)-6-quinazoléinyl)benzamide ;
le N-(3-(acétylamino)-4-fluorophényl)-4-méthyl-3-(2-((3-(4-morpholinyl)propyl)amino)-6-quinazoléinyl)benzamide ;
le N-(2-(((1R)-1-cyclopropyléthyl)oxy)-5-(trifluorométhyl)phényl)-4-méthyl-3-(2-((3-(4-morpholinyl)propyl)amino)-6-quinazoléinyl)benzamide ;
le N-(3-(acétylamino)-4-(((1S)-1-cyclopropyléthyl)oxy)phényl)-4-méthyl-3-(2-((3-(4-morpholinyl)propyl)amino)-6-quinazoléinyl)benzamide ;
le N-(2-(((1R)-1-cyclopropyléthyl)oxy)-5-(trifluorométhyl)phényl)-4-méthyl-3-(2-(méthylamino)-6-quinazoléinyl)benzamide ;
le 4-méthyl-N-(4-(1-méthyléthyl)phényl)-3-(2-((3-(4-morpholinyl)propyl)amino)-6-quinazoléinyl)benzamide ;
le N-(2-(1,1-diméthyléthyl)imidazo[1,2-a]pyridin-6-yl)-4-méthyl-3-(2-((3-(4-morpholinyl)propyl)amino)-6-quinazoléinyl)benzamide ;
le N-(2-(1,1-diméthyléthyl)imidazo(1,2-a]pyridin-6-yl)-4-méthyl-3-(2-((1-méthyl-4-pipéridinyl)amino)-6-quinazoléinyl)benzamide ;
le 3-(2-((3-(diéthylamino)propyl)amino)-6-quinazoléinyl)-N-(2-(1,1-diméthyléthyl)imidazo[1,2-a]pyridin-6-yl)-4-méthylbenzamide ;
le N-(2-(1,1-diméthyléthyl)imidazo[1,2-a]pyridin-6-yl)-4-méthyl-3-(2-(méthylamino)-6-quinazoléinyl)benzamide ;
le 4-méthyl-N-(2-(1-méthyléthyl)-1H-benzimidazol-6-yl)-3-(2-((3-(4-morpholinyl)propyl)amino)-6-quinazoléinyl)benzamide ;
le 3-(2-((3-(diéthylamino)propyl)amino)-6-quinazoléinyl)-4-méthyl-N-(2-(1-méthyléthyl)-1H-benzimidazol-6-yl)benzamide ;
le 4-méthyl-N-(2-(1-méthyléthyl)-1H-benzimidazol-6-yl)-3-(2-((1-méthyl-4-pipéridinyl)amino)-6-quinazoléinyl)benzamide ;
le 4-méthyl-3-(2-(méthylamino)-6-quinazoléinyl)-N-(2-(1-méthyléthyl)-1H-benzimidazol-6-yl)benzamide ;
le N-(2-((2-(diéthylamino)éthyl)oxy)-5-(trifluorométhyl)phényl)-4-méthyl-3-(2-((3-(4-morpholinyl)propyl)amino)-6-quinazoléinyl)benzamide ;
le N-(1-acétyl-2,3-dihydro-1H-indol-5-yl)-4-méthyl-3-(2-((1-méthyl-4-pipéridinyl)amino)-6-quinazoléinyl)benzamide ;
le N-(1-acétyl-2,3-dihydro-1H-indol-5-yl)-4-méthyl-3-(2-((3-(4-morpholinyl)propyl)amino)-6-quinazoléinyl)benzamide ;
le 4-méthyl-3-(2-(méthylamino)-6-quinazoléinyl)-N-(2-((2-(4-morpholinyl)éthyl)oxy)-5-(trifluorométhyl)phényl)benzamide ;
le 4-méthyl-3-(2-((1-méthyl-4-pipéridinyl)amino)-6-quinazoléinyl)-N-(2-((2-(4-morpholinyl)éthyl)oxy)-5-(trifluorométhyl)phényl)benzamide ;
le 4-méthyl-N-(2-((2-(4-morpholinyl)éthyl)oxy)-5-(trifluorométhyl)phényl)-3-(2-((3-(4-morpholinyl)propyl)amino)-6-quinazoléinyl)benzamide ;
la N-méthyl-6-(2-méthyl-5-(5-(trifluorométhyl)-1,3-benzoxazol-2-yl)phényl)-2-quinazoléinamine ;
le N-(2-(1-éthylpyrrolidin-3-yloxy)-5-(trifluorométhyl)phényl)-4-méthyl-3-(2-(méthylamino)quinazoléin-6-yl)benzamide ;
le N-(2-(((3S)-1-éthyl-3-pipéridinyl)oxy)-5-(trifluorométhyl)phényl)-4-méthyl-3-(2-(méthylamino)-6-quinazoléinyl)benzamide ;
le 4-méthyl-N-(2-((l-méthyl-4-pipéridinyl)oxy)-5-(trifluorométhyl)phényl)-3-(2-((2-(1-pipéridinyl)éthyl)amino)-6-quinazoléinyl)benzamide ;
le 4-méthyl-N-(2-((1-méthyl-4-pipéridinyl)oxy)-5-(trifluorométhyl)phényl)-3-(2-((2-(1-pyrrolidinyl)éthyl)amino)-6-quinazoléinyl)benzamide ;
le N-(4-méthyl-3-(2-(méthylamino)-6-quinazoléinyl)phényl)-2-((1-méthyl-4-pipéridinyl)oxy)-5-(trifluorométhyl)benzamide ;
le 2-(((3R)-1-éthyl-3-pipéridinyl)oxy)-N-(4-méthyl-3-(2-(méthylamino)-6-quinazoléinyl)phényl)-5-(trifluorométhyl)benzamide ;
le 2-((cyclopentylméthyl)oxy)-N-(4-méthyl-3-(2-(méthylamino)-6-quinazoléinyl)phényl)-5-(trifluorométhyl)benzamide ;
le 2-((cyclopropylméthyl)oxy)-N-(4-méthyl-3-(2-(méthylamino)-6-quinazoléinyl)phényl)-5-(trifluorométhyl)benzamide ;
le 2-((cyclobutylméthyl)oxy)-N-(4-méthyl-3-(2-(méthylamino)-6-quinazoléinyl)phényl)-5-(trifluorométhyl)benzamide ;
le N-(4-méthyl-3-(2-(méthylamino)-6-quinazoléinyl)phényl)-2-((2-(4-morpholinyl)éthyl)oxy)-5-(trifluorométhyl)benzamide ;
le 2-((2-(diéthylamino)éthyl)oxy)-N-(4-méthyl-3-(2-(méthylamino)-6-quinazoléinyl)phényl)-5-(trifluorométhyl)benzamide ;
le 2-(((1S)-1-cyclopentyléthyl)oxy)-N-(4-méthyl-3-(2-(méthylamino)-6-quinazoléinyl)phényl)-5-(trifluorométhyl)benzamide ;
le N-(4-méthyl-3-(2-(méthylamino)-6-quinazoléinyl)phényl)-2-(4-méthyl-1-pipérazinyl)-5-(trifluorométhyl)benzamide ;
le 5-méthyl-N-(4-méthyl-3-(2-(méthylamino)-6-quinazoléinyl)phényl)-2-(méthyloxy)benzamide ;
le N-(4-méthyl-3-(2-(méthylamino)-6-quinazoléinyl)phényl)-2-((1-méthyl-4-pipéridinyl)amino)-5-(trifluorométhyl)benzamide ;
le 5-fluoro-N-(4-méthyl-3-(2-(méthylamino)-6-quinazoléinyl)phényl)-2-(méthyloxy)benzamide ;
la 6-(1,6-diméthyl-1H-indazol-7-yl)-N-méthyl-2-quinazoléinamine ;
le 4-méthyl-3-(2-(méthylamino)-6-quinazoléinyl)-N-(6-(trifluorométhyl)-1,3-benzothiazol-2-yl)benzamide ;
le 4-méthyl-3-(2-((2-(1-pipéridinyl)éthyl)amino)-6-quinazoléinyl)-N-(6-(trifluorométhyl)-1,3-benzothiazol-2-yl)benzamide ;
le 4-méthyl-3-(2-(méthylamino)-6-quinazoléinyl)-N-(6-((trifluorométhyl)oxy)-1,3-benzothiazol-2-yl)benzamide ;
le 4-méthyl-3-(2-(méthylamino)-6-quinazoléinyl)-N-(5-(trifluorométhyl)-1,3-benzothiazol-2-yl)benzamide ;
le 3-(2-amino-6-quinazoléinyl)-N-(5-(1,1-diméthyléthyl)-2-((1-pyrrolidinylacétyl)amino)phényl)-4-méthylbenzamide ;
le 3-(2-amino-6-quinazoléinyl)-N-(5-(1,1-diméthyléthyl)-2-((2-(1-pipéridinyl)éthyl)oxy)phényl)-4-méthylbenzamide ;
le 3-(2-amino-6-quinazoléinyl)-N-(5-(1,1-diméthyléthyl)-2-((1-pipéridinylacétyl)amino)phényl)-4-méthylbenzamide ;
le 3-(2-amino-6-quinazoléinyl)-N-(2-((N,N-diéthylglycyl)amino)-5-(1,1-diméthyléthyl)phényl)-4-méthylbenzamide ;
le 3-(trifluorométhyl)-N-(2,4,6-triméthyl-3-(2-((3-(4-morpholinyl)propyl)amino)-6-quinazoléinyl)phényl)benzamide ;
la 6-(2,6-dichlorophényl)-2-quinazoléinamine ;
la N-1-(6-(2,6-diméthylphényl)-2-quinazoléinyl)-N-4-,N-4-diméthyl-1,4-butanediamine ;
la 6-(2-chloro-6-méthylphényl)-2-quinazoléinamine ;
la N-1-(6-(2,6-dichlorophényl)-2-quinazoléinyl)-N-3-,N-3-diéthyl-1,3-propanediamine ;
la N-1-(6-(2,6-diméthylphényl)-2-quinazoléinyl)-N-3-,N-3-diéthyl-1,3-propanediamine ;
la 6-(2,6-dichlorophényl)-N-(2-(1-pipéridinyl)éthyl)-2-quinazoléinamine ;
le 4-méthyl-3-(2-((2-(1-pipéridinyl)éthyl)amino)-6-quinazoléinyl)-N-(3-(trifluorométhyl)phényl)benzamide ;
le 3-(2-((3-(diéthylamino)propyl)amino)-6-quinazoléinyl)-4-méthyl-N-(3-(trifluorométhyl)phényl)benzamide ;
le 3-(2-((3-(diméthylamino)propyl)(méthyl)amino)-6-quinazoléinyl)-4-méthyl-N-(3-(trifluorométhyl)phényl)benzamide ;
le 4-méthyl-3-(2-(méthyl(2-(1-pipéridinyl)éthyl)amino)-6-quinazoléinyl)-N-(3-(trifluorométhyl)phényl)benzamide ;
le N-(2-méthyl-3-(2-((2-(1-pipéridinyl)éthyl)amino)-6-quinazoléinyl)phényl)-3-(trifluorométhyl)benzamide ;
la N-1-(6-(2-chloro-6-méthylphényl)-2-quinazoléinyl)-N-3-,N-3-diéthyl-1,3-propanediamine ;
la N-1-(6-(2-chloro-6-méthylphényl)-2-quinazoléinyl)-N-2-,N-2-diéthyl-1,2-éthanediamine ;
la 6-(2-chloro-6-méthylphényl)-N-(3-(1-pyrrolidinyl)propyl)-2-quinazoléinamine ;
la N-1-,N-1-diéthyl-N-3-(6-(2-éthyl-6-méthylphényl)-2-quinazoléinyl)-1,3-propanediamine ;
la N-1-(6-(5-amino-2-méthylphényl)-2-quinazoléinyl)-N-3-,N-3-diéthyl-1,3-propanediamine ;
le N-(3-(2-((3-(diéthylamino)propyl)amino)-6-quinazoléinyl)-2-méthylphényl)-3,5-bis(trifluorométhyl)benzamide ;
le 3-(2-((3-(diéthylamino)propyl)amino)pyrido[2,3-d]pyrimidin-6-yl)-4-méthyl-N-(4-(trifluorométhyl)phényl)benzamide ;
le 3-fluoro-N-(2-méthyl-3-(2-(méthylamino)-6-quinazoléinyl)phényl)-5-(trifluorométhyl)benzamide ;
le N-(3-(2-((3-(diéthylamino)propyl)amino)-6-quinazoléinyl)-4-méthylphényl)-2-fluoro-5-(trifluorométhyl)benzamide ;
le N-(3-(2-((3-(diéthylamino)propyl)amino)-6-quinazoléinyl)-4-méthylphényl)-3-fluoro-5-(trifluorométhyl)benzamide ;
le N-(2-((N,N-diéthylglycyl)amino)-5-(trifluorométhyl)phényl)-4-méthyl-3-(2-(méthylamino)-6-quinazoléinyl)benzamide ;
le 3-(2-amino-6-quinazoléinyl)-N-(2-((N,N-diéthylglycyl)amino)-5-(trifluorométhyl)phényl)-4-méthylbenzamide ;
le 4-méthyl-3-(2-(méthylamino)-6-quinazoléinyl)-N-(3-((trifluorométhyl)oxy)phényl)benzamide ;
le 3-(2-((3-(diéthylamino)propyl)amino)-6-quinazoléinyl)-4-méthyl-N-(3-((trifluorométhyl)oxy)phényl)benzamide ;
le N-(3-(2-amino-6-quinazoléinyl)-4-méthylphényl)acétamide ;
le N-(4-méthyl-3-(2-(méthylamino)-6-quinazoléinyl)phényl)acétamide ;
le N-(3-(2-((3-(diéthylamino)propyl)amino)-6-quinazoléinyl)-4-méthylphényl)acétamide ;
le 3-(2-amino-6-quinazoléinyl)-4-méthyl-N-(5-méthyl-2-((4-morpholinylacétyl)amino)phényl)benzamide ;
le 4-méthyl-3-(2-(méthylamino)-6-quinazoléinyl)-N-(5-méthyl-2-((4-morpholinylacétyl)amino)phényl)benzamide ;
le 3-(2-amino-6-quinazoléinyl)-4-méthyl-N-(5-méthyl-2-((1-pyrrolidinylacétyl)amino)phényl)benzamide ;
le 4-méthyl-3-(2-(méthylamino)-6-quinazoléinyl)-N-(5-méthyl-2-((1-pyrrolidinylacétyl)amino)phényl)benzamide ;
le 3-(2-amino-6-quinazoléinyl)-4-méthyl-N-(5-méthyl-2-((1-pipéridinylacétyl)amino)phényl)benzamide ;
le 4-méthyl-3-(2-(méthylamino)-6-quinazoléinyl)-N-(5-méthyl-2-((1-pipéridinylacétyl)amino)phényl)benzamide ;
le 3-(2-amino-6-quinazoléinyl)-N-(2-((N,N-diéthylglycyl)amino)-5-méthylphényl)-4-méthylbenzamide ;
le N-(2-((N,N-diéthylglycyl)amino)-5-méthylphényl)-4-méthyl-3-(2-(méthylamino)-6-quinazoléinyl)benzamide ;
le N-(2-méthyl-3-(2-((3-(1-pipéridinyl)propyl)amino)-6-quinazoléinyl)phényl)-3-(trifluorométhyl)benzamide ;
le N-(3-(2-((3-(diéthylamino)propyl)amino)-6-quinazoléinyl)-2-méthylphényl)-3-(trifluorométhyl)benzamide ;
le 4-méthyl-3-(2-(méthylamino)-6-quinazoléinyl)-N-(2-(1-pyrrolidinylcarbonyl)-5-(trifluorométhyl)phényl)benzamide ;
le 3-(2-amino-6-quinazoléinyl)-4-méthyl-N-(2-(1-pyrrolidinylcarbonyl)-5-(trifluorométhyl)phényl)benzamide ;
le 4-méthyl-3-(2-((2-(1-pipéridinyl)éthyl)amino)-6-quinazoléinyl)-N-(2-(1-pyrrolidinylcarbonyl)-5-(trifluorométhyl)phényl)benzamide ;
le 3-(2-amino-6-quinazoléinyl)-4-méthyl-N-(2-(1-pyrrolidinylméthyl)-5-(trifluorométhyl)phényl)benzamide ;
le 4-méthyl-3-(2-(méthylamino)-6-quinazoléinyl)-N-(2-(1-pyrrolidinylméthyl)-5-(trifluorométhyl)phényl)benzamide ;
le 4-méthyl-3-(2-((2-(1-pipéridinyl)éthyl)amino)-6-quinazoléinyl)-N-(2-(1-pyrrolidinylméthyl)-5-(trifluorométhyl)phényl)benzamide ;
le N-(2-méthyl-3-(2-(méthylamino)-6-quinazoléinyl)phényl)-3-((1,1,2,2-tétrafluoroéthyl)oxy)benzamide ;
le N-(2-méthyl-3-(2-((2-(1-pipéridinyl)éthyl)amino)-6-quinazoléinyl)phényl)-3-((1,1,2,2-tétrafluoroéthyl)oxy)benzamide ;
le N-(2-(3,3-diméthyl-2-oxo-1-azétidinyl)-5-(trifluorométhyl)phényl)-4-méthyl-3-(2-(méthylamino)-6-quinazoléinyl)benzamide ;
le N-(2-(3,3-diméthyl-2-oxo-1-azétidinyl)-5-(trifluorométhyl)phényl)-4-méthyl-3-(2-((2-(1-pipéridinyl)éthyl)amino)-6-quinazoléinyl)benzamide ;
la N-méthyl-6-(2-méthyl-5-(6-(trifluorométhyl)-1H-benzimidazol-2-yl)phényl)-2-quinazoléinamine ;
le N-(2-méthyl-3-(2-(méthylamino)-6-quinazoléinyl)phényl)-3-(trifluorométhyl)benzamide ;
le N-(2-(acétylamino)-5-(trifluorométhyl)phényl)-4-méthyl-3-(2-((2-(1-pipéridinyl)éthyl)amino)-6-quinazoléinyl)benzamide ;
le N-(2-(acétylamino)-5-(trifluorométhyl)phényl)-4-méthyl-3-(2-((3-(1-pipéridinyl)propyl)amino)-6-quinazoléinyl)benzamide ;
le N-(2-(acétylamino)-5-(trifluorométhyl)phényl)-3-(2-((3-(diéthylamino)propyl)amino)-6-quinazoléinyl)-4-méthylbenzamide ;
le N-(4-méthyl-3-(2-((2-(1-pipéridinyl)éthyl)amino)-6-quinazoléinyl)phényl)-3-(trifluorométhyl)benzamide ;
le 4-méthyl-3-(2-(méthylamino)-6-quinazoléinyl)benzoate de méthyle ;
le 4-méthyl-3-(2-(méthylamino)-6-quinazoléinyl)-N-(2-(((méthyloxy)acétyl)amino)-5-(trifluorométhyl)phényl)benzamide ;
le 4-méthyl-3-(2-((2-(1-pipéridinyl)éthyl)amino)-6-quinazoléinyl)benzoate de méthyle ;
le 4-méthyl-N-(2-(((méthyloxy)acétyl)amino)-5-(trifluorométhyl)phényl)-3-(2-((2-(1-pipéridinyl)éthyl)amino)-6-quinazoléinyl)benzamide ;
la 6-(5-(4-chloro-6-(trifluorométhyl)-1H-benzimidazol-2-yl)-2-méthylphényl)-N-méthyl-2-quinazoléinamine ;
la 6-(5-(4,6-bis(trifluorométhyl)-1H-benzimidazol-2-yl)-2-méthylphényl)-N-méthyl-2-quinazoléinamine ;
le N-(2-(3,3-diméthyl-2-oxo-1-azétidinyl)-5-(trifluorométhyl)phényl)-4-méthyl-3-(2-((3-(4-morpholinyl)propyl)amino)-6-quinazoléinyl)benzamide ;
le 3-(2-((3-(diéthylamino)propyl)amino)-6-quinazoléinyl)-N-(2-(3,3-diméthyl-2-oxo-1-azétidinyl)-5-(trifluorométhyl)phényl)-4-méthylbenzamide ;
le N-(2-(3,3-diméthyl-2-oxo-1-azétidinyl)-5-(trifluorométhyl)phényl)-4-méthyl-3-(2-((3-(1-pipéridinyl)propyl)amino)-6-quinazoléinyl)benzamide ;
le N-(2-(acétylamino)-5-(trifluorométhyl)phényl)-4-méthyl-3-(2-(méthylamino)-6-quinazoléinyl)benzamide ;
le 4-méthyl-3-(2-(méthylamino)-6-quinazoléinyl)-N-(2-((((2-(4-morpholinyl)éthyl)amino)carbonyl)amino)-5-(trifluorométhyl)phényl)benzamide ;
le 4-méthyl-3-(2-(méthylamino)-6-quinazoléinyl)-N-(4-(2-oxo-1-azétidinyl)-3-(trifluorométhyl)phényl)benzamide ;
le 4-méthyl-3-(2-(méthylamino)-6-quinazoléinyl)-N-(4-(2-oxo-1-pyrrolidinyl)-3-(trifluorométhyl)phényl)benzamide ;
le 3-(2-((3-(diéthylamino)propyl)amino)-6-quinazoléinyl)-4-méthyl-N-(4-(2-oxo-1-azétidinyl)-3-(trifluorométhyl)phényl)benzamide ;
le 3-(2-((3-(diéthylamino)propyl)amino)-6-quinazoléinyl)-4-méthyl-N-(4-(2-oxo-1-pyrrolidinyl)-3-(trifluorométhyl)phényl)benzamide ;
le 4-méthyl-3-(2-(méthylamino)-6-quinazoléinyl)-N-(2-(2-oxo-1-pyrrolidinyl)-5-(trifluorométhyl)phényl)benzamide ;
le 3-(2-((3-(diéthylamino)propyl)amino)-6-quinazoléinyl)-4-méthyl-N-(2-(2-oxo-1-pyrrolidinyl)-5-(trifluorométhyl)phényl)benzamide ;
le 4-méthyl-3-(2-(méthylamino)-6-quinazoléinyl)-N-(2-(2-oxo-1-azétidinyl)-5-(trifluorométhyl)phényl)benzamide ;
le N-(2-(3-(1,1-diméthyléthyl)-2-oxo-1-imidazolidinyl)-5-(trifluorométhyl)phényl)-4-méthyl-3-(2-(méthylamino)-6-quinazoléinyl)benzamide ;
le 4-méthyl-3-(2-(méthylamino)-6-quinazoléinyl)-N-(5-(trifluorométhyl)-2-(3,4,4-triméthyl-2,5-dioxo-1-imidazolidinyl)phényl)benzamide ;
le 3-(2-((3-(diéthylamino)propyl)amino)-6-quinazoléinyl)-4-méthyl-N-(5-(trifluorométhyl)-2-(3,4,4-triméthyl-2,5-dioxo-1-imidazolidinyl)phényl)benzamide ;
le 3-(2-((3-(diéthylamino)propyl)amino)-6-quinazoléinyl)-N-(2-(3-(1,1-diméthyléthyl)-2-oxo-1-imidazolidinyl)-5-(trifluorométhyl)phényl)-4-méthylbenzamide ;
le 3-(2-((3-(diéthylamino)propyl)amino)-6-quinazoléinyl)-4-méthyl-N-(2-(2-oxo-1-azétidinyl)-5-(trifluorométhyl)phényl)benzamide ;
le 4-méthyl-N-(2-(2-oxo-1-azétidinyl)-5-(trifluorométhyl)phényl)-3-(2-((2-(1-pipéridinyl)éthyl)amino)-6-quinazoléinyl)benzamide ;
le N-(2-(3-(1,1-diméthyléthyl)-2-oxo-1-imidazolidinyl)-5-(trifluorométhyl)phényl)-4-méthyl-3-(2-((2-(1-pipéridinyl)éthyl)amino)-6-quinazoléinyl)benzamide ;
le 4-méthyl-3-(2-((2-(1-pipéridinyl)éthyl)amino)-6-quinazoléinyl)-N-(5-(trifluorométhyl)-2-(3,4,4-triméthyl-2,5-dioxo-1-imidazolidinyl)phényl)benzamide ;
le 4-méthyl-3-(2-(méthylamino)-6-quinazoléinyl)-N-(2-(3-méthyl-2-oxo-1-imidazolidinyl)-5-(trifluorométhyl)phényl)benzamide ;
le 4-méthyl-N-(2-(3-méthyl-2-oxo-1-imidazolidinyl)-5-(trifluorométhyl)phényl)-3-(2-((1-méthyl-4-pipéridinyl)amino)-6-quinazoléinyl)benzamide ;
le 4-méthyl-N-(2-(3-méthyl-2-oxo-1-imidazolidinyl)-5-(trifluorométhyl)phényl)-3-(2-((2-(1-pipéridinyl)éthyl)amino)-6-quinazoléinyl)benzamide ;
le 3-(2-((3-(diéthylamino)propyl)amino)-6-quinazoléinyl)-4-méthyl-N-(2-(3-méthyl-2-oxo-1-imidazolidinyl)-5-(trifluorométhyl)phényl)benzamide ;
le N-(2-((4-(2-(diméthylamino)éthyl)phényl)oxy)-5-(trifluorométhyl)phényl)-4-méthyl-3-(2-(méthylamino)-6-quinazoléinyl)benzamide ;
la 6-(5-(1H-benzimidazol-2-yl)-2-méthylphényl)-N-méthyl-2-quinazoléinamine ;
la 6-(5-(1H-benzimidazol-2-yl)-2-méthylphényl)-N-(3-(4-morpholinyl)propyl)-2-quinazoléinamine ;
la 6-(2-méthyl-5-(6-(trifluorométhyl)-1H-benzimidazol-2-yl)phényl)-N-(3-(4-morpholinyl)propyl)-2-quinazoléinamine ;
la N,N-diéthyl-N'-(6-(2-méthyl-5-(6-(trifluorométhyl)-1H-benzimidazol-2-yl)phényl)-2-quinazoléinyl)-1,3-propanediamine ;
la 6-(2-méthyl-5-(6-(trifluorométhyl)-1H-benzimidazol-2-yl)phényl)-N-(2-(1-pyrrolidinyl)éthyl)-2-quinazoléinamine ;
le 4-méthyl-3-(2-(méthylamino)-6-quinazoléinyl)-N-(2-(3-oxo-1-pipérazinyl)-5-(trifluorométhyl)phényl)benzamide ;
le N-(2-((3-(diméthylamino)phényl)oxy)-5-(trifluorométhyl)phényl)-4-méthyl-3-(2-(méthylamino)-6-quinazoléinyl)benzamide ;
le 4-méthyl-3-(2-((3-(4-morpholinyl)propyl)amino)-6-quinazoléinyl)-N-(2-(3-oxo-1-pipérazinyl)-5-(trifluorométhyl)phényl)benzamide ;
l'acide 4-méthyl-3-(2-(méthylamino)-6-quinazoléinyl)benzoïque ;
le N-(2-(1-azétidinyl)-5-(trifluorométhyl)phényl)-4-méthyl-3-(2-(méthylamino)-6-quinazoléinyl)benzamide ;
le N-(2-(1-azétidinyl)-5-(trifluorométhyl)phényl)-4-méthyl-3-(2-((3-(4-morpholinyl)propyl)amino)-6-quinazoléinyl)benzamide ;
le N-(2-(1-azétidinyl)-5-(trifluorométhyl)phényl)-4-méthyl-3-(2-((2-(1-pyrrolidinyl)éthyl)amino)-6-quinazoléinyl)benzamide ;
le N-(2-(1-azétidinyl)-5-(trifluorométhyl)phényl)-4-méthyl-3-(2-((2-(1-pipéridinyl)éthyl)amino)-6-quinazoléinyl)benzamide ;
le 4-(2-amino-6-quinazoléinyl)-N-cyclopropyl-1H-indole-6-carboxamide ;
le 5-(2-amino-6-quinazoléinyl)-N-cyclopropyl-2-fluoro-4-méthylbenzamide ;
le N-cyclopropyl-2-fluoro-4-méthyl-5-(2-((2-(4-morpholinyl)éthyl)amino)-6-quinazoléinyl)benzamide ;
le N-cyclopropyl-4-méthyl-2-((2-(4-morpholinyl)éthyl)amino)-5-(2-((2-(4-morpholinyl)éthyl)amino)-6-quinazoléinyl)benzamide ;
le 4-(2-amino-6-quinazoléinyl)-N-cyclopropyl-5-méthyl-2-pyridinecarboxamide ;
le N-cyclopropyl-5-méthyl-4-(2-((2-(4-morpholinyl)éthyl)amino)-6-quinazoléinyl)-2-pyridinecarboxamide ;
le N-cyclopropyl-5-méthyl-4-(2-((3-((2R)-2-méthyl-1-pipéridinyl)propyl)amino)-6-quinazoléinyl)-2-pyridinecarboxamide ;
le N-cyclopropyl-2-fluoro-4-méthyl-3-(2-((2-(4-morpholinyl)éthyl)amino)-6-quinazoléinyl)benzamide ;
le 2-((6-(2-((cyclopropylamino)carbonyl)-5-méthyl-4-pyridinyl)-2-quinazoléinyl)amino)-2-méthylpropylcarbamate de 1,1-diméthyléthyle ;
le 4-(2-((2-amino-1,1-diméthyléthyl)amino)-6-quinazoléinyl)-N-cyclopropyl-5-méthyl-2-pyridinecarboxamide ;
le N-cyclopropyl-4-(2-((2-(diméthylamino)-1,1-diméthyléthyl)amino)-6-quinazoléinyl)-5-méthyl-2-pyridinecarboxamide ;
le N-cyclopropyl-3-(2-((2-((2,2-diméthylpropanoyl)amino)-1,1-diméthyléthyl)amino)-6-quinazoléinyl)-4-méthylbenzamide ;
le N-cyclopropyl-3-(2-((2-((2,2-diméthylpropanoyl)amino)-2-méthylpropyl)amino)-6-quinazoléinyl)-4-méthylbenzamide ;
le N-cyclopropyl-4-(2-((1,1-diméthyl-2-((1-méthyléthyl)amino)éthyl)amino)-6-quinazoléinyl)-5-méthyl-2-pyridinecarboxamide ;
le N-cyclopropyl-3-(2-((2-(diméthylamino)-1,1-diméthyléthyl)amino)-6-quinazoléinyl)-4-méthylbenzamide ;
le 2-((6-(5-((cyclopropylamino)carbonyl)-2-méthylphényl)-2-quinazoléinyl)amino)-2-méthylpropyl(1-méthyléthyl)carbamate de 1,1-diméthyléthyle ;
le N-cyclopropyl-3-(2-((1,1-diméthyl-2-((1-méthyléthyl)amino)éthyl)amino)-6-quinazoléinyl)-4-méthylbenzamide ;
le N-cyclopropyl-3-(2-((3-(diméthylamino)propyl)amino)-6-quinazoléinyl)-4-méthylbenzamide ;
le N-cyclopropyl-4-méthyl-3-(2-((3-(2-méthyl-1-pipéridinyl)propyl)amino)-6-quinazoléinyl)benzamide ;
le N-cyclopropyl-4-méthyl-3-(2-((1-méthyl-2-(méthyloxy)éthyl)amino)-6-quinazoléinyl)benzamide ;
le N-cyclopropyl-4-méthyl-3-(2-((3-((1-méthyléthyl)oxy)propyl)amino)-6-quinazoléinyl)benzamide ;
le N-cyclopropyl-3-(2-((4-(diéthylamino)-1-méthylbutyl)amino)-6-quinazoléinyl)-4-méthylbenzamide ;
le N-cyclopropyl-4-méthyl-3-(2-((3-(1-pyrrolidinyl)propyl)amino)-6-quinazoléinyl)benzamide ;
le 3-(2-((2-amino-2-méthylpropyl)amino)-6-quinazoléinyl)-N-cyclopropyl-4-méthylbenzamide ;
la N-(6-(2-méthylphényl)pyrido[2,3-d]pyrimidin-2-yl)-1,4-cyclohexanediamine ;
le N-cyclopropyl-4-méthyl-3-(2-((tétrahydro-2-furanylméthyl)amino)pyrido[2,3-d]pyrimidin-6-yl)benzamide ;
le 4-((6-(4-méthyl-3-pyridinyl)-2-quinazoléinyl)amino)cyclohexanol ;
le trans-4-((6-(2-méthyl-3-pyridinyl)-2-quinazoléinyl)amino)cyclohexanol ;
la trans-N-(6-(4-méthyl-3-pyridinyl)-2-quinazoléinyl)-1,4-cyclohexanediamine ;
le N-(trans-4-((6-(4-méthyl-3-pyridinyl)-2-quinazoléinyl)amino)cyclohexyl)acétamide ;
la N-(2-(1,1-dioxydo-4-thiomorpholinyl)éthyl)-6-(4-méthyl-3-pyridinyl)-2-quinazoléinamine ;
le 4-((6-(3-méthyl-2-pyridinyl)-2-quinazoléinyl)amino)cyclohexanol ;
la N-1,N-1-diméthyl-N-4-(6-(4-méthyl-3-pyridinyl)-2-quinazoléinyl)-1,4-cyclohexanediamine ;
la N-1-(1-méthyléthyl)-N-4-(6-(4-méthyl-3-pyridinyl)-2-quinazoléinyl)-1,4-cyclohexanediamine ;
la 6-(2-fluorophényl)-N-(2-(2-pyridinyl)éthyl)-2-quinazoléinamine ;
la 6-(4-fluorophényl)-N-(2-(2-pyridinyl)éthyl)-2-quinazoléinamine ;
la 6-(2-chlorophényl)-N-(2-(2-pyridinyl)éthyl)-2-quinazoléinamine ;
la 6-(2,3-difluorophényl)-N-(2-(2-pyridinyl)éthyl)-2-quinazoléinamine ;
la 6-(2,4-difluorophényl)-N-(2-(2-pyridinyl)éthyl)-2-quinazoléinamine ;
la 6-(2,5-difluorophényl)-N-(2-(2-pyridinyl)éthyl)-2-quinazoléinamine ;
la 6-(2,3-diméthylphényl)-N-(2-(2-pyridinyl)éthyl)-2-quinazoléinamine ;
la 6-(3,5-difluorophényl)-N-(2-(2-pyridinyl)éthyl)-2-quinazoléinamine ;
la N-(2-(2-pyridinyl)éthyl)-6-(2-(trifluorométhyl)phényl)-2-quinazoléinamine ;
le 4-((6-(2,5-diméthylphényl)-2-quinazoléinyl)amino)cyclohexanol ;
le N-cyclopropyl-4-méthyl-3-(2-((2-(2-pyridinyl)éthyl)amino)-6-quinazoléinyl)benzamide ;
le 4-méthyl-3-(2-((2-(4-morpholinyl)éthyl)amino)-6-quinazoléinyl)-N-(1,3-thiazol-2-yl)benzamide ;
le N-cyclopropyl-4-méthyl-3-(2-((3-(4-morpholinyl)propyl)amino)-6-quinazoléinyl)benzamide ;
le N-cyclopropyl-3-(2-((1,1-diméthyl-2-(4-morpholinyl)éthyl)amino)-6-quinazoléinyl)-4-méthylbenzamide ;
le 3-(2-(((1S)-2-(3-(aminométhyl)phényl)-1-méthyléthyl)amino)-6-quinazoléinyl)-N-cyclopropyl-4-méthylbenzamide ;
le N-cyclopropyl-4-méthyl-3-(2-méthyl-6-quinazoléinyl)benzamide ;
le (3-((2S)-2-((6-(5-((cyclopropylamino)carbonyl)-2-méthylphényl)-2-quinazoléinyl)amino)propyl)phényl)méthylcarbamate de 1,1-diméthyléthyle ;
le N-cyclopropyl-3-(2-(((1S)-2-(3-((diméthylamino)méthyl)phényl)-1-méthyléthyl)amino)-6-quinazoléinyl)-4-méthylbenzamide ;
le N-cyclopropyl-4-méthyl-3-(2-((2-méthyl-2-(4-morpholinyl)propyl)amino)-6-quinazoléinyl)benzamide ;
le ((3-((2S)-2-((6-(5-((éthylamino)carbonyl)-2-méthylphényl)-2-quinazoléinyl)amino)propyl)phényl)méthyl)carbamate de 1,1-diméthyléthyle ;
le 3-(2-(((1S)-2-(3-(aminométhyl)phényl)-1-méthyléthyl)amino)-6-quinazoléinyl)-N-éthyl-4-méthylbenzamide ;
le 3-(2-((2-(diméthylamino)éthyl)amino)-6-quinazoléinyl)-4-méthylbenzamide ;
le N-cyclopropyl-3-(2-(((1S)-1-((diméthylamino)carbonyl)-2-méthylpropyl)amino)-6-quinazoléinyl)-4-méthylbenzamide ;
le 3-(2-((3-(diméthylamino)-2,2-diméthylpropyl)amino)-6-quinazoléinyl)-4-méthylbenzamide ;
le 3-(2-((3-(diméthylamino)-2,2-diméthylpropyl)amino)-6-quinazoléinyl)-N-éthyl-4-méthylbenzamide ;
le 4-méthyl-3-(2-((2-méthyl-2-(4-morpholinyl)propyl)amino)-6-quinazoléinyl)benzamide ;
le N,4-diméthyl-3-(2-((2-méthyl-2-(4-morpholinyl)propyl)amino)-6-quinazoléinyl)benzamide ;
le N-éthyl-4-méthyl-3-(2-((2-méthyl-2-(4-morpholinyl)propyl)amino)-6-quinazoléinyl)benzamide ;
le N-cyclopropyl-3-(2-((2-(3,5-diméthyl-4-morpholinyl)éthyl)amino)-6-quinazoléinyl)-4-méthylbenzamide ;
l'acide 4-méthyl-3-(2-((2-méthyl-2-(4-morpholinyl)propyl)amino)-6-quinazoléinyl)benzoïque ;
le N-cyclopropyl-4-méthyl-3-(2-((2-((3S)-3-méthyl-4-morpholinyl)éthyl)amino)-6-quinazoléinyl)benzamide ;
le N-cyclopropyl-4-méthyl-3-(2-((2-(phénylamino)éthyl)amino)-6-quinazoléinyl)benzamide ;
la N-(6-(5-((cyclopropylamino)carbonyl)-2-méthylphényl)-2-quinazoléinyl)-O-méthyl-L-thréonine ;
le N-(6-(5-((cyclopropylamino)carbonyl)-2-méthylphényl)-2-quinazoléinyl)-D-tryptophane ;
la N-(6-(5-((cyclopropylamino)carbonyl)-2-méthylphényl)-2-quinazoléinyl)-D-phénylalanine ;
le N-cyclopropyl-3-(2-(((1S)-1-(4-fluorophényl)éthyl)amino)-6-quinazoléinyl)-4-méthylbenzamide ;
l'acide ((6-(5-((cyclopropylamino)carbonyl)-2-méthylphényl)-2-quinazoléinyl)amino) (tétrahydro-2H-pyran-4-yl)acétique ;
le N-cyclopropyl-4-méthyl-3-(2-((2-(2-pyridinylamino)éthyl)amino)-6-quinazoléinyl)benzamide ;
le 3-(4-bromo-7-isoquinoléinyl)-4-méthylbenzamide ;
le 3-(4-(2-chloro-4-fluorophényl)-7-isoquinoléinyl)-4-méthylbenzamide ;
le 4-méthyl-3-(4-(4-morpholinyl)-7-isoquinoléinyl)benzamide ;
le 4-méthyl-3-(4-(2-méthyl-3-pyridinyl)-7-isoquinoléinyl)benzamide ;
le 3-(4-(4-(aminosulfonyl)-2-méthylphényl)-7-isoquinoléinyl)-N-cyclopropyl-4-méthylbenzamide ;
la 6-(2-méthylphényl)-N-(3-(4-morpholinyl)propyl)-2-quinazoléinamine ;
la N-(3-(1H-imidazol-1-yl)propyl)-6-(2-méthylphényl)-2-quinazoléinamine ;
la 6-(2-méthylphényl)-N-(2-(2-pyridinyl)éthyl)-2-quinazoléinamine ;
la N-(6-(2-méthylphényl)-2-quinazoléinyl)-1,4-cyclohexanediamine ;
la 6-(2-méthylphényl)-N-(3-(2-méthyl-1-pipéridinyl)propyl)-2-quinazoléinamine ;
la N-((1S)-2-(3-((1S)-1-aminoéthyl)phényl)-1-méthyléthyl)-6-(2-méthylphényl)-2-quinazoléinamine ;
la N-1-,N-1-diéthyl-N-3-(6-(2-méthylphényl)-2-quinazoléinyl)-1,3-propanediamine ;
le 4-((6-(2-méthylphényl)-2-quinazoléinyl)amino)cyclohexanol ;
la N-((1S)-2-(3-(1-amino-1-méthyléthyl)phényl)-1-méthyléthyl)-6-(2-méthylphényl)-2-quinazoléinamine ;
la N-((1S)-2-(3-(1-amino-1-méthyléthyl)phényl)-1-méthyléthyl)-6-(4-méthyl-3-thiényl)-2-quinazoléinamine ;
le 4-((6-(2-aminophényl)-2-quinazoléinyl)amino)cyclohexanol ;
la 6-(2-méthylphényl)-N-(1-(phénylméthyl)-3-pyrrolidinyl)-2-quinazoléinamine ;
la 6-(2-méthylphényl)-N-(1-(phénylméthyl)-4-pipéridinyl)-2-quinazoléinamine ;
le 2-(2-((6-(2-méthylphényl)-2-quinazoléinyl)amino)éthyl)-1-pipéridinecarboxylate de 1,1-diméthyléthyle ;
la 6-(2-méthylphényl)-N-(2-(2-pipéridinyl)éthyl)-2-quinazoléinamine ;
la N-((1S)-2-(3-(1-amino-l-méthyléthyl)phényl)-1-méthyléthyl)-6-(4-méthyl-3-pyridinyl)-2-quinazoléinamine ;
la 6-(3-méthyl-2-pyridinyl)-2-quinazoléinamine ;
la N-((1S)-2-(3-(1-amino-1-méthyléthyl)phényl)-1-méthyléthyl)-6-(3-méthyl-2-pyridinyl)-2-quinazoléinamine ;
la N-((1S)-2-(3-((1S)-1-aminoéthyl)phényl)-1-méthyléthyl)-6-(2-chloro-3-pyridinyl)-2-quinazoléinamine ;
la N-((1S)-2-(3-((1S)-1-aminoéthyl)phényl)-1-méthyléthyl)-6-(4-méthyl-3-pyridinyl)-2-quinazoléinamine ;
la N-1-,N-1-diéthyl-N-4-(6-(4-méthyl-3-pyridinyl)-2-quinazoléinyl)-1,4-pentanediamine ;
la N-(2-(3-chlorophényl)éthyl)-6-(4-méthyl-3-pyridinyl)-2-quinazoléinamine ;
la (5S)-5-((6-(4-méthyl-3-pyridinyl)-2-quinazoléinyl)amino)-2-((1R)-4-(diéthylamino)-1-méthylbutyl)(6-(4-méthyl-3-pyridinyl)-2-quinazoléinyl)amine ;
la ((1S)-4-(diéthylamino)-1-méthylbutyl)(6-(4-méthyl-3-pyridinyl)-2-quinazoléinyl)amine ;
la N,N,2,2-tétraméthyl-N'-(6-(4-méthyl-3-pyridinyl)-2-quinazoléinyl)-1,3-propanediamine ;
la 6-(4-méthyl-3-pyridinyl)-N-(4-(1-pyrrolidinyl)butyl)-2-quinazoléinamine ;
la N-((1S)-1-(4-fluorophényl)éthyl)-6-(4-méthyl-3-pyridinyl)-2-quinazoléinamine ;
le 4-chloro-N-cyclopropyl-3-(2-((2-(diméthylamino)éthyl)amino)-6-quinazoléinyl)benzamide ;
le N-cyclopropyl-3-(2-((2-(diméthylamino)éthyl)amino)-6-quinazoléinyl)-4-(trifluorométhyl)benzamide ;
le 5-(2-((2-(diméthylamino)éthyl)amino)-6-quinazoléinyl)-4-méthyl-2-thiophènecarboxylate d'éthyle ;
la 6-(2-méthylphényl)-2-quinazoléinamine ;
la 6-(2-méthylphényl)-N-(2-(3-pyridinyl)éthyl)-2-quinazoléinamine ;
la N-((1S)-2-(3-(aminométhyl)phényl)-1-méthyléthyl)-6-(2-méthylphényl)-2-quinazoléinamine ;
la N-(2,4-diméthylphényl)-6-(2-méthylphényl)-2-quinazoléinamine ;
la 6-(2-méthylphényl)-N-(2-(4-morpholinyl)éthyl)-2-quinazoléinamine ;
la 6-(2-((diméthylamino)méthyl)phényl)-N-(2-(4-morpholinyl)éthyl)-2-quinazoléinamine ;
la 6-(2-((diméthylamino)méthyl)phényl)-N-(2-(2-pyridinyl)éthyl)-2-quinazoléinamine ;
la 6-(4-fluoro-2-méthylphényl)-N-(2-(2-pyridinyl)éthyl)-2-quinazoléinamine ;
la 6-(3-chloro-2-méthylphényl)-N-(2-(2-pyridinyl)éthyl)-2-quinazoléinamine ;
la 6-(2,6-diméthylphényl)-N-(2-(2-pyridinyl)éthyl)-2-quinazoléinamine ;
le (3-(2-((2-(2-pyridinyl)éthyl)amino)-6-quinazoléinyl)phényl)méthanol ;
le (2-(2-((2-(2-pyridinyl)éthyl)amino)-6-quinazoléinyl)phényl)méthanol ;
la 6-(3-(aminométhyl)phényl)-N-(2-(2-pyridinyl)éthyl)-2-quinazoléinamine ;
le N-méthyl-3-(2-((2-(2-pyridinyl)éthyl)amino)-6-quinazoléinyl)benzamide ;
le N-cyclopropyl-3-(2-((2-(2-pyridinyl)éthyl)amino)-6-quinazoléinyl)benzamide ;
la 6-phényl-N-(2-(2-pyridinyl)éthyl)-2-quinazoléinamine ;
la 6-(2-(méthyloxy)phényl)-N-(2-(2-pyridinyl)éthyl)-2-quinazoléinamine ;
le 4-((6-(2,6-diméthylphényl)-2-quinazoléinyl)amino)cyclohexanol ;
le N-(3-(2-amino-6-quinazoléinyl)-4-méthylphényl)cyclopropanecarboxamide ;
le N-(4-méthyl-3-(2-((2-(4-morpholinyl)éthyl)amino)-6-quinazoléinyl)phényl)cyclopropanecarboxamide ;
le N-(3-méthyl-4-(2-((2-(4-morpholinyl)éthyl)amino)-6-quinazoléinyl)phényl)cyclopropanecarboxamide ;
le N-cyclopropyl-3-(2-(3-(diméthylamino)-1-propynyl)-6-quinazoléinyl)-4-méthylbenzamide ;
le N-cyclopropyl-3-(2-(3-(diméthylamino)propyl)-6-quinazoléinyl)-4-méthylbenzamide ;
le 1-(6-(5-((cyclopropylamino)carbonyl)-2-méthylphényl)-2-quinazoléinyl)-3-pyrrolidinylcarbamate de 1,1-diméthyléthyle ;
le 3-(2-((3S)-3-amino-1-pyrrolidinyl)-6-quinazoléinyl)-N-cyclopropyl-4-méthylbenzamide ;
le N-cyclopropyl-4-méthyl-3-(2-(((3R)-6-oxo-3-pipéridinyl)amino)-6-quinazoléinyl)benzamide ;
le N-cyclopropyl-3-(2-((2-((1,1-diméthyléthyl)amino)éthyl)amino)-6-quinazoléinyl)-4-méthylbenzamide ;
le 3-(2-(cyclohexylamino)-6-quinazoléinyl)-N-cyclopropyl-4-méthylbenzamide ;
le N-cyclopropyl-4-méthyl-3-(2-(((3S)-6-oxo-3-pipéridinyl)amino)-6-quinazoléinyl)benzamide ;
le N-éthyl-4-méthyl-3-(2-(((3S)-6-oxo-3-pipéridinyl)amino)-6-quinazoléinyl)benzamide ;
le N-cyclopropyl-3-(2-((2-(1,1-dioxydo-4-thiomorpholinyl)éthyl)amino)-6-quinazoléinyl)-4-méthylbenzamide ;
le N-cyclopropyl-4-méthyl-3-(2-((2-(2-oxo-1-pyrrolidinyl)éthyl)amino)-6-quinazoléinyl)benzamide ;
le 3-(2-(4-amino-1-pipéridinyl)-6-quinazoléinyl)-N-cyclopropyl-4-méthylbenzamide ;
le 3-(2-(4-(acétylamino)-1-pipéridinyl)-6-quinazoléinyl)-N-cyclopropyl-4-méthylbenzamide ;
le (1-(6-(5-((cyclopropylamino)carbonyl)-2-méthylphényl)-2-quinazoléinyl)-4-pipéridinyl)carbamate de 1,1-diméthyléthyle ;
le 4-((6-(5-((cyclopropylamino)carbonyl)-2-méthylphényl)-2-quinazoléinyl)amino)-1-pipéridinecarboxylate de 1,1-diméthyléthyle ;
le N-cyclopropyl-4-méthyl-3-(2-(4-pipéridinylamino)-6-quinazoléinyl)benzamide ;
le 3-(2-((1-acétyl-4-pipéridinyl)amino)-6-quinazoléinyl)-N-cyclopropyl-4-méthylbenzamide ;
le N-cyclopropyl-4-méthyl-3-(4-(2-méthylphényl)-7-isoquinoléinyl)benzamide ;
le 4-méthyl-3-(4-(2-méthylphényl)-7-isoquinoléinyl)benzamide ;
le 4-méthyl-3-(4-((3S)-3-méthyl-4-morpholinyl)-7-isoquinoléinyl)benzoate de méthyle ;
le N-cyclopropyl-4-méthyl-3-(2-((2-(1-pipérazinyl)éthyl)amino)-6-quinazoléinyl)benzamide ;
le N-cyclopropyl-4-méthyl-3-(2-((3S)-3-pipéridinylamino)-6-quinazoléinyl)benzamide ;
le 2-((6-(5-((cyclopropylamino)carbonyl)-2-méthylphényl)-2-quinazoléinyl)amino)-2-méthylpropylcarbamate de 1,1-diméthyléthyle ;
le (2S)-2-(((6-(5-((cyclopropylamino)carbonyl)-2-méthylphényl)-2-quinazoléinyl)amino)méthyl)-1-pyrrolidinecarboxylate de 1,1-diméthyléthyle ;
le N-cyclopropyl-4-méthyl-3-(2-((2-(4-méthyl-1-pipérazinyl)éthyl)amino)-6-quinazoléinyl)benzamide ;
le N-cyclopropyl-4-méthyl-3-(2-(((2S)-2-pyrrolidinylméthyl)amino)-6-quinazoléinyl)benzamide ;
le (2R)-2-(((6-(5-((cyclopropylamino)carbonyl)-2-méthylphényl)-2-quinazoléinyl)amino)méthyl)-1-pyrrolidinecarboxylate de 1,1-diméthyléthyle ;
le N-cyclopropyl-4-méthyl-3-(2-(((2R)-2-pyrrolidinylméthyl)amino)-6-quinazoléinyl)benzamide ;
le 3-(2-((2-amino-1,1-diméthyléthyl)amino)-6-quinazoléinyl)-N-cyclopropyl-4-méthylbenzamide ;
le N-cyclopropyl-4-méthyl-3-(2-(((3R)-1-méthyl-3-pyrrolidinyl)amino)-6-quinazoléinyl)benzamide ;
le N-cyclopropyl-4-méthyl-3-(2-((3R)-3-pyrrolidinylamino)-6-quinazoléinyl)benzamide ;
le N-cyclopropyl-4-méthyl-3-(2-((((2S)-1-méthyl-2-pyrrolidinyl)méthyl)amino)-6-quinazoléinyl)benzamide ;
le N-cyclopropyl-4-méthyl-3-(2-((((2R)-1-(1-méthyléthyl)-2-pyrrolidinyl)méthyl)amino)-6-quinazoléinyl)benzamide ;
le N-cyclopropyl-4-méthyl-3-(2-((((2S)-1-(2-(méthyloxy)éthyl)-2-pyrrolidinyl)méthyl)amino)-6-quinazoléinyl)benzamide ;
le 3-(2-((2-aminoéthyl)amino)-6-quinazoléinyl)-N-cyclopropyl-4-méthylbenzamide ;
le N-cyclopropyl-4-méthyl-3-(2-((2-((1-méthyléthyl)amino)éthyl)amino)-6-quinazoléinyl)benzamide ;
le N-cyclopropyl-3-(2-(((1R,2R)-2-(diméthylamino)cyclohexyl)amino)-6-quinazoléinyl)-4-méthylbenzamide ;
le N-cyclopropyl-3-(2-(((1S,2S)-2-(diméthylamino)cyclohexyl)amino)-6-quinazoléinyl)-4-méthylbenzamide ;
le N-cyclopropyl-4-méthyl-3-(2-(((1R,2R)-2-((1-méthyléthyl)amino)cyclohexyl)amino)-6-quinazoléinyl)benzamide
le 2-((6-(5-((cyclopropylamino)carbonyl)-2-méthylphényl)-2-quinazoléinyl)amino)éthylcarbamate de méthyle ;
le N-cyclopropyl-4-méthyl-3-(2-(((1S,2S)-2-((1-méthyléthyl)amino)cyclohexyl)amino)-6-quinazoléinyl)benzamide ;
l'acide 1-((6-(5-((cyclopropylamino)carbonyl)-2-méthylphényl)-2-quinazoléinyl)amino)cyclopentanecarboxylique ;
la N-(6-(5-((cyclopropylamino)carbonyl)-2-méthylphényl)-2-quinazoléinyl)-3-(2-thiényl)alanine ;
l'acide 1-((6-(5-((cyclopropylamino)carbonyl)-2-méthylphényl)-2-quinazoléinyl)amino)cyclopropanecarboxylique ;
le N-(6-(5-((cyclopropylamino)carbonyl)-2-méthylphényl)-2-quinazoléinyl)-bêta-alaninate de méthyle ;
la N-(6-(5-((cyclopropylamino)carbonyl)-2-méthylphényl)-2-quinazoléinyl)-2-méthylalanine ;
la N-(6-(5-((cyclopropylamino)carbonyl)-2-méthylphényl)-2-quinazoléinyl)-L-valine ;
le N,4-diméthyl-3-(2-((2-(4-morpholinyl)éthyl)amino)-6-quinazoléinyl)benzamide ;
le 4-méthyl-3-(2-((2-(4-morpholinyl)éthyl)amino)-6-quinazoléinyl)-N-(2,2,2-trifluoroéthyl)benzamide ;
le 4-méthyl-N-(2-méthylpropyl)-3-(2-((2-(4-morpholinyl)éthyl)amino)-6-quinazoléinyl)benzamide ;
le N-cyclopentyl-4-méthyl-3-(2-((2-(4-morpholinyl)éthyl)amino)-6-quinazoléinyl)benzamide ;
le N-cyclopropyl-3-(2-((3-(diméthylamino)-2,2-diméthylpropyl)amino)-6-quinazoléinyl)-4-méthylbenzamide ;
le N-cyclopropyl-3-(2-(((1S,2R)-2-(diméthylamino)cyclohexyl)amino)-6-quinazoléinyl)-4-méthylbenzamide ;
le N-cyclopropyl-3-(2-((2-(diéthylamino)éthyl)amino)-6-quinazoléinyl)-4-méthylbenzamide ;
le N-cyclopropyl-4-méthyl-3-(2-((2-(méthyloxy)éthyl)amino)-6-quinazoléinyl)benzamide ;
le 3-(2-(((1S,2S)-2-aminocyclohexyl)amino)-6-quinazoléinyl)-N-cyclopropyl-4-méthylbenzamide ;
le N-cyclopropyl-3-(2-(((1R,2R)-2-(diméthylamino)cyclohexyl)amino)-6-quinazoléinyl)-4-méthylbenzamide ;
le N-cyclopropyl-3-(2-((2-furanylméthyl)amino)-6-quinazoléinyl)-4-méthylbenzamide ;
le N-cyclopropyl-4-méthyl-3-(2-((tétrahydro-2-furanylméthyl)amino)-6-quinazoléinyl)benzamide ;
la N-5-(6-(4-méthyl-3-pyridinyl)-2-quinazoléinyl)-2,5-pyridinediamine ;
la ((1R)-4-(diéthylamino)-1-méthylbutyl)(6-(6-fluoro-2-méthyl-3-pyridinyl)-2-quinazoléinyl)amine ;
la 5-(2-((4-(diéthylamino)-1-méthylbutyl)amino)-6-quinazoléinyl)-6-méthyl-2(1H)-pyridinone ;
la 3-(2-(((1R)-4-(diéthylamino)-1-méthylbutyl)amino)-6-quinazoléinyl)-4-méthyl-2(1H)-pyridinone ;
la ((1R)-4-(diéthylamino)-1-méthylbutyl)(6-(2-fluoro-5-méthyl-4-pyridinyl)-2-quinazoléinyl)amine ;
la 4-(2-(((1R)-4-(diéthylamino)-1-méthylbutyl)amino)-6-quinazoléinyl)-5-méthyl-2(1H)-pyridinone ;
la N-(6-(5-((cyclopropylamino)carbonyl)-2-méthylphényl)-2-quinazoléinyl)-D-valine ;
la N-(6-(5-((cyclopropylamino)carbonyl)-2-méthylphényl)-2-quinazoléinyl)-D-leucine ;
la N-(6-(5-((cyclopropylamino)carbonyl)-2-méthylphényl)-2-quinazoléinyl)glycine ;
la N-(6-(5-((cyclopropylamino)carbonyl)-2-méthylphényl)-2-quinazoléinyl)-1-alanine ;
la N-(6-(5-((cyclopropylamino)carbonyl)-2-méthylphényl)-2-quinazoléinyl)-D-alanine ;
le 5-(2-amino-6-quinazoléinyl)-N-(2-(méthyl((3R)-1-méthyl-3-pyrrolidinyl)amino)-5-(trifluorométhyl)phényl)-2-(méthyloxy)benzamide ;
le 3-(2-amino-6-quinazoléinyl)-N-(2-((3-(diméthylamino)propyl)(méthyl)amino)-5-(trifluorométhyl)phényl)-2-((phénylméthyl)amino)benzamide ;
le 4-(2-amino-6-quinazoléinyl)-N-(2-((3-(diméthylamino)propyl)(méthyl)amino)-5-(trifluorométhyl)phényl)-5-méthyl-2-pyridinecarboxamide ;
le 4-(2-amino-6-quinazoléinyl)-N-(2-((3-(diméthylamino)propyl)(méthyl)amino)-5-(trifluorométhyl)phényl)-1-(méthyloxy)-2-naphtalènecarboxamide ;
le 4-(2-amino-6-quinazoléinyl)-1-(méthyloxy)-N-(3-(trifluorométhyl)phényl)-2-naphtalènecarboxamide ;
le 4-(2-amino-6-quinazoléinyl)-N-(2-(méthyl((3R)-1-méthyl-3-pyrrolidinyl)amino)-5-(trifluorométhyl)phényl)-1-(méthyloxy)-2-naphtalènecarboxamide ;
le 4-(2-amino-6-quinazoléinyl)-N-(2-(((3R)-3-(diméthylamino)-1-pyrrolidinyl)méthyl)-5-(trifluorométhyl)phényl)-1-(méthyloxy)-2-naphtalènecarboxamide ;
le 5-(2-amino-6-quinazoléinyl)-N-(2-(3,3-diméthyl-2-oxo-1-azétidinyl)-5-(trifluorométhyl)phényl)-2-(méthyloxy)benzamide ; et
le 5-(2-amino-6-quinazoléinyl)-N-(2-((3S)-3-(diméthylamino)-1-pipéridinyl)-5-(trifluorométhyl)phényl)-2-fluorobenzamide ;
la 6-(2-phénylimidazo[1,2-a]pyridin-6-yl)-2-quinazoléinamine
la 6-(2-(4-(méthyloxy)phényl)imidazo[1,2-a]pyridin-6-yl)-2-quinazoléinamine ;
la 6-(2-(3-(méthyloxy)phényl)imidazo[1,2-a]pyridin-6-yl)-2-quinazoléinamine ;
la 6-(2-(2-(méthyloxy)phényl)imidazo[1,2-a]pyridin-6-yl)-2-quinazoléinamine ;
la 6-(2-(1,3-benzodioxol-5-yl)imidazo[1,2-a]pyridin-6-yl)-2-quinazoléinamine ;
la 6-(2-(3-thiényl)imidazo[1,2-a]pyridin-6-yl)-2-quinazoléinamine ;
la 6-(2-éthyl-1-benzofuran-5-yl)-2-quinazoléinamine ; et
la 6-(2-(1,1-diméthyléthyl)imidazo[1,2-a]pyridin-6-yl)-2-quinazoléinamine.

3. Composition pharmaceutique comprenant un support pharmaceutiquement acceptable et un composé selon la revendication 1 ou 2.

4. Composé selon la revendication 1 ou 2, à utiliser dans la modulation chez un sujet d'une enzyme protéine kinase choisie dans le groupe consistant en KDR, Lck, p38, tie2 et une leurs combinaisons.

5. Composé selon la revendication 1 ou 2, à utiliser dans le traitement d'un trouble relatif à au moins une parmi Tie-2 Lck, KDR et P38 chez un sujet.

6. Composé selon la revendication 1 ou 2, à utiliser dans le traitement d'un trouble lié à une prolifération chez un sujet.

7. Composé selon la revendication 6, dans lequel le trouble est choisi dans le groupe consistant en un infarctus du myocarde, une coronaropathie, un acrosyndrome, un accident vasculaire cérébral, une néovascularisation oculaire, une rétinopathie, une rétinopathie diabétique, la dégénérescence maculaire liée à l'âge, le psoriasis, un hémangioblastome, un hémangiome, une artériosclérose, une maladie inflammatoire, la polyarthrite rhumatoïde, l'asthme, une athérosclérose artérielle ou post-transplantation, l'endométriose, une leucémie et leurs combinaisons.

8. Composé selon la revendication 1 ou 2, à utiliser dans le traitement d'un cancer chez un sujet.

9. Composé selon la revendication 8, dans lequel l'administration de la quantité efficace du composé chez un sujet comprend l'administration du composé en combinaison avec un ou plusieurs composés choisis parmi les agents antinéoplasiques, les agents anti-angiogéniques, les agents chimiothérapeutiques, et des agents peptidiques de thérapie cancéreuse.

10. Composé selon la revendication 9, dans lequel les agents antinéoplasiques sont choisis parmi des agents de type antibiotique, des agents d'alkylation, des agents anti-métaboliques, des agents hormonaux, des agents immunologiques, des agents de type interféron, des inhibiteurs de kinase, des agents variés et leurs combinaisons.

11. Composition pharmaceutique selon la revendication 3, à utiliser dans le traitement du cancer chez un sujet.

12. Composé selon la revendication 1 ou 2, à utiliser dans le traitement d'une angiogenèse chez un sujet.

13. Composé selon la revendication 1 ou 2, à utiliser dans la réduction du flux sanguin dans une tumeur chez un sujet.

14. Composé selon la revendication 1 ou 2, à utiliser dans la réduction de la taille d'une tumeur chez un sujet.

15. Composé selon la revendication 1 ou 2, à utiliser dans le traitement d'une inflammation chez un sujet.

16. Composé selon la revendication 1 ou 2, à utiliser dans l'inhibition d'une activation et d'une prolifération de cellules T chez un sujet.

17. Composé selon la revendication 1 ou 2, à utiliser dans le traitement de l'arthrite, de la polyarthrite rhumatoïde, de l'arthrite psoriasique ou l'arthrose chez un sujet.

18. Composé selon la revendication 1 ou 2, à utiliser dans le traitement d'un transplant d'organe, d'un transplant aigu ou d'un rejet d'hétérogreffe ou d'homogreffe, ou d'une induction de tolérance à la transplantation chez un sujet.

19. Composé selon la revendication 1 ou 2, à utiliser dans le traitement d'une lésion ischémique ou de reperfusion, d'un infarctus du myocarde, ou d'un accident vasculaire cérébral chez un sujet.

20. Composé selon la revendication 1 ou 2, à utiliser dans le traitement de la sclérose en plaques, d'une affection abdominale inflammatoire, dont la rectocolite ulcérative, la maladie de Crohn, le lupus, l'hypersensibilité de contact, l'hypersensibilité de type retardé, et l'entéropathie sensible au gluten, le diabète de type 1, le psoriasis, la dermatite de contact, la thyroïdite d'Hashimoto, le syndrome de Sjögren, l'hyperthyroïdisme auto-immun, la maladie d'Addison, la maladie polyglandulaire auto-immune, l'alopécie auto-immune, l'anémie pernicieuse, le vitiligo, l'hypopituitarisme auto-immun, le syndrome de Guillain-Barré, la glomérulonéphrite, la maladie sérique, l'urticaire, les maladies allergiques, l'asthme, le rhume des foins, la rhinite allergique, la sclérodermie, la mycose fongoïde, la dermatomyosite, l'alopécie en aires, la dermatite actinique chronique, l'eczéma, la maladie de Behçet, la pustulose palmoplantaire, l'idiophagédénisme, le syndrome de Sézary, la dermatite atopique, la sclérodermie généralisée, la morphée ou la dermatite atopique chez un sujet.

21. Composé selon la revendication 1 ou 2, à utiliser dans le traitement d'un carcinome du côlon ou un thymome chez un sujet.

22. Composé selon la revendication 1 ou 2, à utiliser dans la diminution du niveau d'un ou plusieurs parmi TNF-α, IL-1β, IL-6 et IL-8 chez un sujet.

23. Composé selon la revendication 1 ou 2, à utiliser dans le traitement de la spondylarthrite ankylosante, l'arthrite goutteuse, le syndrome de détresse respiratoire de l'adulte (SDRA), l'anaphylaxie, la dégénérescence musculaire, la cachexie, le syndrome de Reiter, le diabète de type II, les maladies de résorption osseuse, la réaction de greffe contre hôte, la maladie d'Alzheimer, l'athérosclérose, un trauma du cerveau, la sclérose en plaques, l'accès pernicieux à forme cérébrale, la sepsie, le choc septique, le syndrome du choc toxique, la fièvre et les myalgies dues à une infection, ou dont le sujet est infecté par VIH-1, VIH-2, VIH-3, cytomégalovirus (CMV), grippe, adénovirus, les virus de l'herpès (dont HSV-1, HSV-2), ou le zona chez un sujet.

24. Utilisation d'un composé selon la revendication 1 ou 2, pour la préparation d'un médicament destiné au traitement d'une inflammation ou d'un cancer chez un sujet.

25. Utilisation d'un composé selon la revendication 1 ou 2, pour la préparation d'un médicament destiné à l'inhibition d'une activation et d'une prolifération de cellules T chez un sujet.

26. Utilisation d'un composé selon la revendication 1 ou 2, pour la préparation d'un médicament destiné au traitement de l'arthrite, de l'arthrite rhumatoïde, l'arthrite psoriasique, ou de l'arthrose chez un sujet.

27. Utilisation d'un composé selon la revendication 1 ou 2, pour la préparation d'un médicament destiné au traitement d'un transplant d'organe, d'un transplant aigu ou d'un rejet d'hétérogreffe ou d'homogreffe, d'une induction de tolérance à la transplantation chez un sujet.

28. Utilisation d'un composé selon la revendication 1 ou 2, pour la préparation d'un médicament destiné au traitement d'une lésion ischémique ou de reperfusion, d'un infarctus du myocarde ou d'un accident vasculaire cérébral chez un sujet.

29. Utilisation d'un composé selon la revendication 1 ou 2, pour la préparation d'un médicament destiné au traitement de la sclérose en plaques, d'une affection abdominale inflammatoire, dont la rectocolite ulcérative, la maladie de Crohn, le lupus, la dermatite de contact, la thyroïdite d'Hashimoto, le syndrome de Sjögren, l'hyperthyroïdisme auto-immun, la maladie d'Addison, la maladie polyglandulaire auto-immune, l'alopécie auto-immune, l'anémie pernicieuse, le vitiligo, l'hypopituitarisme auto-immun, le syndrome de Guillain-Barré, la glomérulonéphrite, la maladie sérique, l'urticaire, les maladies allergiques, l'asthme, le rhume des foins, la rhinite allergique, la sclérodermie, la mycose fongoïde, la dermatomyosite, l'alopécie en aires, la dermatite actinique chronique, l'eczéma, la maladie de Behçet, la pustulose palmoplantaire, l'idiophagédénisme, le syndrome de Sézary, la dermatite atopique, la sclérodermie généralisée, la morphée ou la dermatite atopique, la spondylarthrite ankylosante, l'arthrite goutteuse, le syndrome de détresse respiratoire de l'adulte (SDRA), l'anaphylaxie, la dégénérescence musculaire, la cachexie, le syndrome de Reiter, le diabète de type II, les maladies de résorption osseuse, la réaction de greffe contre hôte, la maladie d'Alzheimer, l'athérosclérose, un trauma du cerveau, la sclérose en plaques, l'accès pernicieux à forme cérébrale, la sepsie, le choc septique, le syndrome toxique, la fièvre et les myalgies dues à une infection, ou dont le sujet est infecté par VIH-1, VIH-2, VIH-3, cytomégalovirus (CMV), grippe, adénovirus, les virus de l'herpès (dont HSV-1, HSV-2), ou le zona chez un sujet.

30. Utilisation d'un composé selon la revendication 1 ou 2 pour la préparation d'un médicament destiné au traitement d'un carcinome du côlon ou d'un thymome chez un sujet.
